(19) Europäisches Patentamt
European Patent Office
Office européen des brevets

(11) EP 2 599 870 A2

(12) EUROPEAN PATENT APPLICATION

(43) Date of publication:
**05.06.2013 Bulletin 2013/23**

(51) Int Cl.:
***C12N 15/82** (2006.01)* ***C07K 14/415** (2006.01)*
*A01H 5/00 (2006.01)*

(21) Application number: **12191834.6**

(22) Date of filing: **15.06.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**15.06.2006 EP 06447081**
**15.06.2006 EP 06447082**
**26.06.2006 US 816444 P**
**26.06.2006 US 816447 P**
**19.01.2007 EP 07100862**
**31.01.2007 EP 07101535**
**31.01.2007 EP 07101534**
**15.02.2007 US 889957 P**
**22.02.2007 US 891045 P**
**22.02.2007 US 891047 P**

(62) Document number(s) of the earlier application(s) in accordance with Art. 76 EPC:
**07764668.5 / 2 032 704**

(71) Applicants:
- **CropDesign N.V.**
  **9052 Zwijnaarde (BE)**
- **Crop Functional Genomics Center**
  **Gwanak-Gu**
  **Seoul 151-921 (KR)**

(72) Inventors:
- **Choi, Yang Do**
  **137-909 Seoul (KR)**
- **Kim, Ju Kon**
  **463-846 Sungnam (KR)**
- **Nahm, Baek Hie**
  **463-500 Seongnam (KR)**
- **Jeong, Jin Seo**
  **449-706 Yongin (KR)**
- **Oh, Se-Jun**
  **121-875 Seoul (KR)**
- **Han, Chang-Deok**
  **660-770 Jinju (KR)**
- **Park, Sung Han**
  **443-727 Suwon (KR)**
- **Chung, Pil Joong**
  **151-921 Seoul (KR)**

(74) Representative: **Krieger, Stephan Gerhard**
**BASF SE**
**Global Intellectual Property**
**GVX/B - C 6**
**Carl-Bosch-Strasse 38**
**67056 Ludwigshafen (DE)**

Remarks:
This application was filed on 08-11-2012 as a divisional application to the application mentioned under INID code 62.

# (54) Plants having enhanced yield-related traits and a method for making the same

(57) The present invention concerns a method for enhancing yield-related traits in plants by modulating expression in a plant of a nucleic acid encoding an AP2-2 polypeptide. The present invention also concerns plants having modulated expression of a nucleic acid encoding an AP2-2 polypeptide, which plants have enhanced yield-related traits relative to control plants.

The present invention further concerns a method for enhancing yield-related traits in plants by modulating expression in a plant of a nucleic acid encoding an APETELA2-70-like (AP2-70-like) polypeptide. The present invention also concerns plants having modulated expression of a nucleic acid encoding an AP2-70-like polypeptide, which plants have enhanced yield-related traits relative to control plants. The invention also provides hitherto unknown NAC, AP2-2 and AP2-70-like-encoding nucleic acids, and constructs comprising the same, useful in performing the methods of the invention.

The invention also provides constructs useful in the methods of the invention.



Printed by Jouve, 75001 PARIS (FR)

## Description

The present invention relates generally to the field of molecular biology and concerns a method for enhancing various economically important yield-related traits in plants. More specifically,
the present invention concerns a method for enhancing yield-related traits in plants by modulating expression in a plant of a nucleic acid encoding a Apetala 2-2 (AP2-2) polypeptide. The present invention also concerns plants having modulated expression of a nucleic acid encoding an AP2-2 polypeptide, which plants have enhanced yield-related traits relative to control plants. The present invention also concerns a method for enhancing yield-related traits in plants by modulating expression in a plant of a nucleic acid encoding an APETALA2-70-like (AP2-70-like) polypeptide. The present invention also concerns plants having modulated expression of a nucleic acid encoding an AP2-70-like polypeptide, which plants have enhanced yield-related traits relative to control plants. The invention also provides hitherto unknown AP2-70-like-encoding nucleic acids, and constructs comprising the same, useful in performing the methods of the invention.

The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic and horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as, corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

A further trait of economic importance for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. Early vigour may also result from increased plant fitness due to, for example, the plants being better adapted to their environment (i.e. being more able to cope with various abiotic or biotic stress factors). Plants having early vigour also show better establishment of the crop (with the crop growing in a more uniform manner, i.e. with the majority of plants reaching the various stages of development at substantially the same time), and show better growth and often better yield.

A further important trait is improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

The ability to engineer abiotic stress tolerance or early vigour into plants would be of great importance in agriculture, as would the ability to increase plant seed yield, whether through seed number, seed biomass, seed development, seed filling, or any other seed-related trait. Aside form the many applications in agriculture including in the production of ornamental plants, arboriculture, horticulture and forestry, increasing yield would also have many non-agricultural uses, such as in the production of algae for use in bioreactors (for the biotechnological production of substances such as pharmaceuticals, antibodies or vaccines or for the bioconversion of organic waste) and other such areas.

Transcription factors are usually defined as proteins that show sequence-specific DNA binding and that are capable of activating and/or repressing transcription. The *Arabidopsis* genome codes for at least 1533 transcriptional regulators, which account for ~5.9% of its estimated total number of genes. About 45% of these transcription factors are reported to be from families specific to plants (Riechmann et al., 2000 (Science Vol. 290, 2105-2109)).

The present invention concerns the use of an Apetala type transcription factor, AP2-2, for enhancing yield-related traits in plants.

AP2 (APETALA2) and EREBPs (ethylene-responsive element binding proteins, or ERF, ethylene response factors) are the prototypic members of a family of transcription factors unique to plants, whose distinguishing characteristic is that they contain the so-called AP2 DNA-binding domain. AP2/EREBP genes form a large multigene family (the AP2/ERF superfamily), and they play a variety of roles throughout the plant life cycle: from being key regulators of several developmental processes, like floral organ identity determination or control of leaf epidermal cell identity, to forming part of the mechanisms used by plants to respond to various types of biotic and environmental stress. Within the AP2/ERF superfamily, 3 large families are discriminated: the AP2 family with two AP2/ERF domains, the ERF family with a single AP2/ERF domain and the RAV family comprising a B3-type DNA binding domain. Nakano et al. (Plant Physiology 140, 411-432, 2006) studied the *ERF* gene family in *Arabidopsis* and rice, and divided the *Arabidopsis ERF* gene family into 12 groups (designated Group I to X, and a Group VI-like and Group Xb-like), whereas in the case of rice 15 groups were discriminated. The *Arabidopsis* Group VII proteins are characterised by a conserved N-terminal motif, referred to as Conserved Motif VII-1 (CMVII-1). In rice, Group VII comprises more proteins than the *Arabidopsis* Group VII, and though many conserved motifs are in common between the rice and *Arabidopsis* Group VII, a separate rice Group VIIb was created for a sequence lacking this typical CMVII-1 motif. Functionally, members of Group VII are described to be involved in osmotic stress and disease responses (for example in WO 2003007699). Ectopic overexpression of tomato JERF3 in tobacco increased the salt tolerance of the transgenics (Wang et al., Plant Molecular Biology 58, 183-192, 2004), and pepper transcription factor CaPF1 overexpression resulted in increased osmotic tolerance in pine (Tang et al, Plant Cell Rep. 26, 115-124, 2007), but also increased pathogen resistance in *Arabidopsis* (Yi et al., Plant Physiol. 136, 2862-2874, 2004). A similar observation was made for barley HvRAF (Jung et al., Planta Epub 26 August 2006). Furthermore, a Group VII type ERF protein was used in a process for the production of Methionine (EP2005003297).

Surprisingly, it has now been found that modulating expression in a plant of a nucleic acid encoding a Group VII ERF protein (hereafter named AP2-2 polypeptide) gives plants having enhanced yield-related traits relative to control plants. These enhanced yield-related traits were not the result of increased stress resistance.

The ERF (Ethylene Response Factor) family is a large gene family of transcription factors and is part of the AP2/ERF superfamily, which also contains the AP2 and RAV families. The AP2/ERF superfamily is defined by the AP2/ERF domain, which consists of about 60 to 70 amino acids and is involved in DNA binding. These three families have been defined as follows. The AP2 family of proteins contain two repeated AP2/ERF domains, the ERF family of proteins contain a single AP2/ERF domain, and the RAV family proteins contain a B3 domain, which is a DNA-binding domain conserved in other plant-specific transcription factors, including VP1/AB13, in addition to the single AP2/ERF domain. The ERF family is sometimes further divided into two major subfamilies, the ERF subfamily and the CBF/DREB subfamily. 147 genes have been identified in the AP2/ERF superfamily in the Arabidopsis genome, including 122 genes in the ERF family. The AP2 domain was first identified as a repeated motif within the Arabidopsis (*Arabidopsis thaliana*) AP2 protein, which is involved in flower development. Nakano et al., 2006 (Plant Physiol. Vol. 140, pp.411-432).

Nakano *et al.,* 2006 further report that genes in the AP2 family have been shown to participate in the regulation of developmental processes, e.g. flower development, spikelet meristem determinacy, leaf epidermal cell identity, and embryo development. Several proteins in the ERF family have been identified and implicated in many diverse functions in cellular processes, such as hormonal signal transduction, response to biotic and abiotic stresses, and regulation of metabolism, and in developmental processes in various plant species.

Surprisingly, it has now been found that modulating expression in a plant of a nucleic acid encoding an AP2-70-like polypeptide gives plants having enhanced yield-related traits relative to control plants.

AP2-70-like polypeptides described herein as being useful in enhancing yield-related traits in plants are classified in Group Ib (A-6) according to the classification system of Nakano *et al*., 2006. Nakano reports that DBF1 from maize (Zea mays) - a member of Group Ib - has been shown to activate the drought-responsive element 2 (DRE2)-dependent transcription of ABA-responsive *rab17* in transiently transformed maize callus. In another Group Ib member, *Medicago truncatula* WXP1, overexpression of the same was reported to activate wax production in transgenic alfalfa (Medicago sativa).

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag-100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1**: Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu; Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.
The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1 °C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

a or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

<u>Operably linked</u>

The term "operably linked" as used herein refers to a functional linkage between the promoter sequence and the gene of interest, such that the promoter sequence is able to initiate transcription of the gene of interest.

<u>Constitutive promoter</u>

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a**: Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b**: Examples of root-specific promoters

| Gene Source | Reference |
|---|---|
| RCc3 | Plant Mol Biol. 1995 Jan;27(2):237-48 |
| Arabidopsis phosphate transporter PHT1 | Kovama et al., 2005 |
| Medicago phosphate transporter | Xiao et al., 2006 |
| Arabidopsis Pyk10 | Nitz et al. (2001) Plant Sci 161(2): 337-346 |

A seed-specific promoter is transcriptionally active predominantly in seed tissue, but not necessarily exclusively in seed tissue (in cases of leaky expression). The seed-specific promoter may be active during seed development and/or during germination.

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2c below.

**Table 2c**: Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Optionally, one or more terminator sequences may be used in the construct introduced into a plant. Additional regulatory elements may include transcriptional as well as translational enhancers. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Reduction or substantial elimination of expression may be achieved using routine tools and techniques known in the art.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per acre for a crop and year, which is determined by dividing total production (includes both harvested and appraised production) by planted acres. The term "yield" of a plant may relate to vegetative biomass (root and/or shoot biomass), to reproductive organs, and/or to propagules (such as seeds) of that plant.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Seed yield

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds; e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Increased growth rate

Since the transgenic plants according to the present invention have increased yield, it is likely that these plants exhibit an increased growth rate (during at least part of their life cycle), relative to the growth rate of corresponding wild type plants at a corresponding stage in their life cycle. Besides the increased yield capacity, an increased efficiency of nutrient uptake may also contribute to the increase in yield. It is observed that the plants according to the present invention show a higher efficiency in nutrient uptake. Increased efficiency of nutrient uptake allows better growth of the plant, whether the plant is under stress or non-stress conditions.

The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour (increased seedling vigor at emergence). The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of rice plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acacia* spp.,*Acer* spp., *Actinidia* spp., *Aesculus* spp., *Agathis australis, Albizia amara, Alsophila tricolor, Andropogon* spp., *Arachis* spp, *Areca catechu, Astelia fragrans, Astragalus cicer, Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Artocarpus* spp., *Asparagus officinalis, Avena* spp. *(e.g. Avena sativa, Avena fatua, Avena byzantina, Avena fatua var. sativa, Avena hybrida), Averrhoa carambola Baikiaea plurijuga, Betula* spp., *Bruguiera gymnorrhiza, Burkea africana, Butea frondosa, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Calliandra* spp, *Camellia sinensis, Canna indica, Capsicum* spp., *Cassia* spp., *Centroema pubescens, Chaenomeles* spp., *Cinnamomum cassia, Coffea arabica, Colophospermum mopane, Coronillia varia, Cotoneaster serotina, Crataegus* spp., *Cucumis* spp., *Cupressus* spp., *Cyathea dealbata, Cydonia oblonga, Cryptomeria japonica, Cymbopogon* spp., *Cynthea dealbata, Cydonia oblonga, Cadaba farinosa, Camellia sinensis, Cannabis*

*sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cynara* spp., *Dalbergia monetaria, Davallia divaricata, Desmodium* spp., *Dicksonia squarosa, Diheteropogon amplectens, Dioclea* spp, *Dolichos* spp., *Dorycnium rectum, Daucus carota, Dimocarpus longan, Dioscorea* spp., *Diospyros spp.,Echinochloa pyramidalis, Ehrartia* spp., *Eleusine coracana, Eragrestis* spp., *Erythrina* spp., *Eucalyptus* spp., *Euclea schimperi, Eulalia villosa, Elaeis (e.g. Elaeis guineensis, Elaeis oleifera)" Erianthus sp., Eriobotrya japonica" Eugenia uniflora, Fagopyrum* spp., *Feijoa sellowiana, Fragaria* spp., *Flemingia* spp, *Freycinetia banksii, Fagus* spp., *Festuca arundinacea, Ficus carica, Fortunella* spp., *Fragaria* spp., *Geranium thunbergii, Ginkgo biloba, Glycine* spp. *(e.g. Glycine max, Soja hispida or Soja max), Glycine javanica, Gliricidia* spp, *Gossypium hirsutum, Grevillea* spp., *Guibourtia coleosperma, Gossypium hirsutum, Helianthus* spp. *(e.g. Helianthus annuus), Hemerocallis fulva, Hedysarum* spp., *Hemarthia altissima, Heteropogon contortus, Hibiscus* spp., *Hordeum* spp. *(e.g. Hordeum vulgare), Hyparrhenia rufa, Hypericum erectum, Hyperthelia dissoluta, Indigo incarnata, Iris* spp., *Ipomoea batatas, Juglans* spp., *Leptarrhena pyrolifolia, Lespediza* spp., *Lettuca* spp., *Leucaena leucocephala, Loudetia simplex, Lotonus bainesii, Lotus* spp., *.Lactuca sativa, Lathyrus* spp., *Lens culinaris, Linum usitatissimum, Litchi chinensis, Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Macrotyloma axillare, Malus* spp., *Malus* spp., *Malpighia emarginata, Mammea americana, Mangifera indica, Manihot* spp., *Manihot esculenta, Medicago sativa, Metasequoia glyptostroboides, Manilkara zapota, Medicago sativa, Melilotus* spp., *Mentha* spp., *Miscanthus sinensis, Momordica* spp., *Morus nigra, Musa* spp., *Musa sapientum, Nicotianum* spp., *Onobrychis* spp., *Ornithopus* spp., *Peltophorum africanum, Pennisetum* spp., *Olea* spp., *Opuntia* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Persea gratissima, Petunia* spp., *Phaseolus* spp., *Phoenix canariensis, Phormium cookianum, Photinia* spp., *Picea glauca, Pinus* spp., *Pisum sativum, Podocarpus totara, Pogonarthria fleckii, Pogonarthria squarrosa, Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prosopis cineraria, Prunus* spp., *Psidium* spp., *Punica granatum, Pyrus communis, Pseudotsuga menziesii, Pterolobium stellatum, Pyrus communis, Quercus* spp., *Rhaphiolepsis umbellata, Rhopalostylis sapida, Rhus natalensis, Ribes grossularia, Ribes* spp., *Robinia pseudoacacia, Rosa* spp., *Raphanus sativus, Rheum rhabarbarum, Ricinus communis, Rubus* spp., *Salix* spp., *Schyzachyrium sanguineum, Sciadopitys verticillata, Sequoia sempervirens, Sequoiadendron giganteum, Saccharum* spp., *Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Sporobolus fimbriatus, Stiburus alopecuroides, Stylosanthos humilis, Syzygium* spp., *Tadehagi* spp, *Taxodium distichum, Themeda triandra, Trifolium* spp., *Triticum* spp. *(e.g. Triticum aestivum, Triticum durum, Triticum turgidum, Triticum hybernum, Triticum macha, Triticum sativum or Triticum vulgare), Tagetes* spp., *Tamarindus indica, Theobroma cacao, Triticosecale rimpaui, Tsuga heterophylla, Tropaeolum minus, Tropaeolum majus, Vaccinium* spp., *Vicia* spp., *Vitis vinifera, Vigna* spp., *Viola odorata, Vitis* spp., *VVatsonia pyramidata, Zantedeschia aethiopica, Zea mays, Zizania palustris, Ziziphus* spp., amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugarcane, sunflower, tomato, squash, tea and algae, amongst others.

Alignment of sequences

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole the sequence) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10; 4:29. MatGAT: an application that generates similarity/identity matrices using protein or DNA sequences). Minor manual editing may be performed to optimise alignment between conserved motifs, as would be apparent to a person skilled in the art. Furthermore, instead of using full-length sequences for the identification of homologues, specific domains may also be used. The sequence identity values, which are indicated below in Example 3 as a percentage were determined over the entire nucleic acid or amino acid sequence, and/or over selected domains or conserved motif(s), using the programs mentioned above using the default parameters.

Reciprocal blast search

Reciprocal blast search typically involves a first BLAST involving BLASTing a query sequence (for example using any of the sequences listed in Table 1, Table 2, Table 14 of Example 9 or Table 18 of Example 18) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is any of SEQ ID NO: 1 or SEQ ID NO: 2, or SEQ ID NO: 50 to SEQ ID NO: 59, or SEQ ID NO: 131 or SEQ ID NO: 132 or SEQ ID NO: 257 or SEQ ID NO: 258, the second BLAST would therefore be against *Oryza sativa* sequences). The results of the first and second BLASTs are then compared. A paralogue is identified if a high-ranking hit from the first blast is from the same species as from which the query sequence is derived, a BLAST back then ideally results in the query sequence as highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Amplification

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

**Detailed description of the invention**

The present invention provides a method for enhancing yield-related traits in plants relative to control plants, comprising modulating expression in a plant of a nucleic acid encoding an AP2-2 polypeptide. The present invention further provides a method for enhancing yield-related traits in plants relative to control plants, comprising modulating expression in a plant of a nucleic acid encoding an AP2-70-like polypeptide.

The present invention provides a method for enhancing yield-related traits in plants relative to control plants, comprising modulating expression in a plant of a nucleic acid encoding an AP2-2 polypeptide.

A preferred method for modulating (preferably, increasing) expression of a nucleic acid encoding an AP2-2 polypeptide is by introducing and expressing in a plant a nucleic acid encoding an AP2-2 polypeptide.

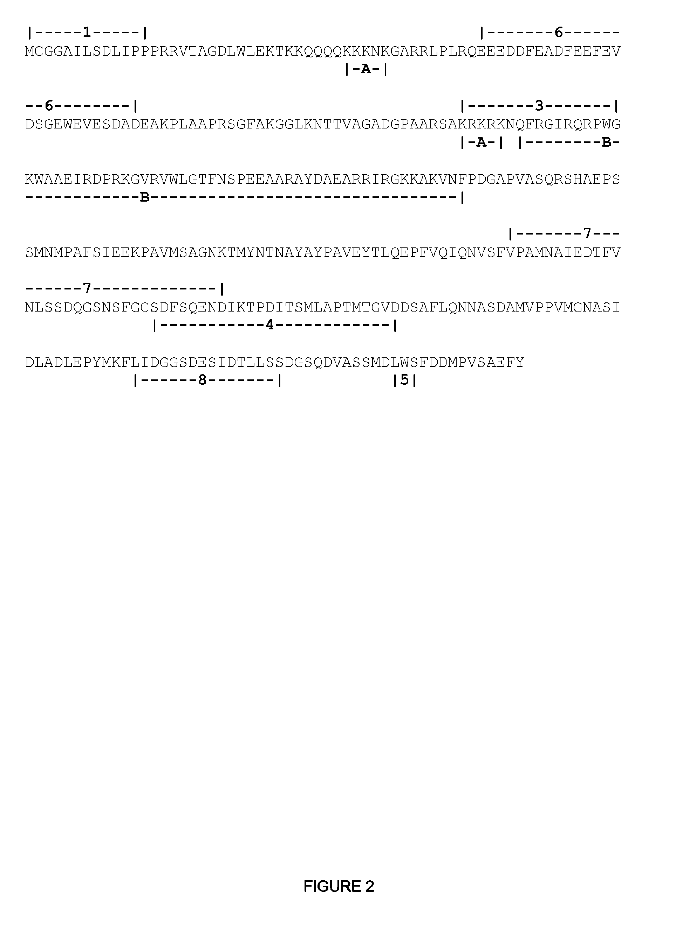

The term "AP2-2 polypeptide" as defined herein refers to a transcription factor that classifies as an ERF protein, and more particularly as a member of Group VII of the ERF proteins, according to the definition by Nakano et al. (2006). Group VII of ERF proteins is, besides the presence of a single AP2/ERF domain, which is part of all ERF proteins, also characterised by other motifs, such as CMVII-1 to CMVII-8 (Nakano et al., 2006). These CMVII motifs were delineated based on analysis of *Arabidopsis thaliana* and *Oryza sativa* sequences and are part of the *Arabidopsis* and/or rice sequences (Figure 2), but may also be present in Group VII ERF proteins of other species.

The AP2-2 polypeptide useful in the methods of the present invention comprises the conserved sequence Motif XII (SEQ ID NO: 133):

(R/I/V/L/K)(R/K/Q/D/E/A)(I/L)(R/Y/H)G(A/S/R/K/T/D/G/H/L/N)(K/T/R/N/G)A(K/R/E)(V/L/P/T) NF(P/V)

Preferably, motif XII is

(R/I/K)(R/K/Q/D/A)(I/L)(R/Y/H)G(A/S/R/K/T/D/E/G/H/L/N)(K/T/R/N/G)A(K/R/E)(V/L/P/T)NF(P /V/A)

Preferably, the AP2-2 polypeptide useful in the methods of the present invention furthermore also comprises one or more of the following motifs:

Motif XIII (SEQ ID NO: 134):
MCGG(A/S)(I/V/L)(I/L)(S/H/A/Y/G/P/E)(D/G/H/Y/E/Q/N)
Preferably, motif XIII is MCGG(A/S)I(I/L)(S/H/A/Y)(D/G/H/Y/E)
More preferably motif XIII is MCGG(A/S)I(I/L)(S/H)(D/G/H/E)
Motif XIV (SEQ ID NO: 135)
(K/N/R/S/H/M/Q/P/A/E)(R/K/H/A/G/E/V/P/M)(E/K/A/R/S/Q/T/V/G/H/P)(R/K/Q/S/G)(K/G/P/S/ R/T/A/N)(T/N/R/S/A/Y/H/G)(L/Q/H/V/R/K/A/P/G)(Y/F)(R/W/K/L/M)G(I/V)(R/Q/H)(R/Q/W/K)R( P/K/T)

Preferably, motif XIV is:
(K/N/R/S/H/M)(R/K/H/A/G/E/V/P)(E/K/A/R/S/Q)(R/K/Q/S)(K/G/P/S/R/T)(T/N/R/S/A/Y/H/G)(L/ Q/H/V/R/K/A/P/G/F/I)(Y/F/L)(R/W/K/L/M/H)G(I/V)(R/Q/H)(R/Q/W/K)R(P/K/T)

More preferably, motif XIV is:
(K/N/R/S)(R/K/H)(E/K/A/R)(R/K/Q)(K/G/P/S)(T/N/R/S/A)(L/Q/H/V/R/K)(Y/F)(R/W/K)G(I/V)R( R/Q) RP

Most preferably, motif XIV is:
(K/N)(R/H)KRKNQ(Y/F)RGI RQRP

Motif XV (SEQ ID NO: 136):
SD(Q/T/E/V)(G/S)SNSF(G/D/E/S/N)(C/S)S(D/E)(F/Y/L)(G/S)(W/Q/L)(E/G/S)(N/E/D)

Preferably, motif XV is:
SD(Q/T)(G/S/A)SNS(F/I)(G/D)(C/S)S(D/E)F(G/S)(W/Q/L)(E/S)(N/D)

Motif XVI (SEQ ID NO: 137):
(L/I/F/M)W(S/T/N/M)(F/Y/L/I)(D/E/Q/G)(N/D/H/E)(I/Y/F/S/M/L/V/D/H/N/E/G)

Preferably, motif XVI is
(L/I/M)W(S/T/M)(F/Y/L/I)(D/E/Q/G)(N/D/E)(I/Y/F/S/M/L/V/D)

More preferably, motif XVI is (L/I/M)W(S/M)(F/L/I)D(D/E)(I/M/L/V)

Motif XVII (SEQ ID NO: 138):
(D/E/S)(F/A/W/D)(E/A)(A/D/L)(D/A/G/E)(F/G/L)(N/E/R/G/Q/W)(E/G/V/D/R)F(E/K/V/Y/G/D/L/I )(V/R/D/S/N/A/E)(D/G/T/E/R/A/Y/L/F)

Preferably, motif XVII is
(D/E/S)(F/A/W/D)(E/A)(A/D)(D/A)(F/G)(E/R/Q/W)(E/G/D/R)F(E/K/Y/G/D/L/I)(V/R/D/S/N/A/E) (D/G/T/E/R)

Further preferably, motif XVII is
(D/E/S)(F/A)(E/A)(A/D)(D/A)F(E/R/Q/W)(E/G/D/R)F(E/K/Y/G/D)(V/R/D/S/N)(D/G/T/E)

More preferably, motif XVII is
(D/E/S)(F/A)(E/A)(A/D)DF(E/R/Q/W)(E/G)F(E/K/Y)(V/R/D/S)(D/G/T) Most preferably, motif XVII is (D/E)FEADF(E/R/Q) EF(E/K)(V/R/D)(D/G)

These motifs were derived mainly from *Arabidopsis* and rice sequences, therefore, one or more conserved substitutions are allowed in these motifs for AP2-2 sequences from other plant species.

Furthermore, AP2-2 polypeptides (at least in their native form) may have DNA-binding activity. Tools and techniques for measuring DNA-binding activity are well known in the art. The terms "domain" and "motif" are defined in the "definitions" section herein. Specialist databases exist for the identification of domains. Examples are given in the "definitions" section herein.

Analysis of the polypeptide sequence of SEQ ID NO: 132 in the SMART database, revealed there to be an AP2 domain (SMART entry SM00380, Fig. 2). This domain is plant specific and is known to play a role in protein-DNA interactions (binds to the GCC-box, essential for the response to ethylene).

The present invention is illustrated by transforming plants with the nucleic acid sequence represented by SEQ ID NO: 131, encoding the polypeptide sequence of SEQ ID NO: 132. However, performance of the invention is not restricted to these sequences; the methods of the invention may advantageously be performed using any AP2-2-encoding nucleic acid or AP2-2 polypeptides as defined herein.

Examples of nucleic acids encoding AP2-2 polypeptides are given in Table 14 of Example 9 herein. Such nucleic acids are useful in performing the methods of the invention. The amino acid sequences given in Table 14 of Example 9 are example sequences of orthologues and paralogues of the AP2-2 polypeptides represented by SEQ ID NO: 132, the terms "orthologues" and "paralogues" being as defined herein. Further orthologues and paralogues may readily be identified by performing a so-called reciprocal blast search.

The invention also provides hitherto unknown AP2-2-encoding nucleic acids and AP2-2 polypeptides.

According to a further embodiment of the present invention, there is therefore provided an isolated nucleic acid molecule comprising:

(i) a nucleic acid represented by one of SEQ ID NO: 341, SEQ ID NO: 343, or SEQ ID NO: 345;
(ii) the complement of a nucleic acid represented by one of SEQ ID NO: 341, SEQ ID NO: 343, or SEQ ID NO: 345;
(iii) a nucleic acid encoding a POI polypeptide having, in increasing order of preference, at least 50%, 55%, 60%, 65%, 70%, 75%, 80%, 85%, 90%, 95%, 96%, 97%, 98%, 99% or more sequence identity to the amino acid sequence represented by one of SEQ ID NO: 342, SEQ ID NO: 344, or SEQ ID NO: 346.

According to a further embodiment of the present invention, there is also provided an isolated polypeptide comprising:

(i) an amino acid sequence represented by one of SEQ ID NO: 342, SEQ ID NO: 344, or SEQ ID NO: 346;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence represented by one of SEQ ID NO: 342, SEQ ID NO: 344, or SEQ ID NO: 346;
(iii) derivatives of any of the amino acid sequences given in (i) or (ii) above.

Nucleic acid variants may also be useful in practising the methods of the invention. Examples of such variants include nucleic acids encoding homologues and derivatives of any one of the amino acid sequences given in Table 14 of Example 9, the terms "homologue" and "derivative" being as defined herein. Also useful in the methods of the invention are nucleic acids encoding homologues and derivatives of orthologues or paralogues of any one of the amino acid sequences given in Table 14 of Example 9. Homologues and derivatives useful in the methods of the present invention have substantially the same biological and functional activity as the unmodified protein from which they are derived.

Further nucleic acid variants useful in practising the methods of the invention include portions of nucleic acids encoding AP2-2 polypeptides, nucleic acids hybridising to nucleic acids encoding AP2-2 polypeptides, splice variants of nucleic acids encoding AP2-2 polypeptides, allelic variants of nucleic acids encoding AP2-2 polypeptides and variants of nucleic acids encoding AP2-2 polypeptides obtained by gene shuffling. The terms hybridising sequence, splice variant, allelic variant and gene shuffling are as described herein.

Nucleic acids encoding AP2-2 polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table 14 of Example 9, or a portion of a nucleic acid encoding an orthologue, paralogue or homologue of any of the amino acid sequences given in Table 14 of Example 9.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode an AP2-2 polypeptide as defined herein, and have substantially the same biological activity as the amino acid sequences given in Table 14 of Example 9. Preferably, the portion is a portion of any one of the nucleic acids given in Table 14 of Example 9, or is a portion of a nucleic acid encoding an orthologue or paralogue of any one of the amino acid sequences given in Table 14 of Example 9. Preferably the portion is, in increasing order of preference at least 600, 800, 900 or 1000 consecutive nucleotides in length, the consecutive nucleotides being of any one of the nucleic acid sequences given in Table 14 of Example 9, or of a nucleic acid encoding an orthologue or paralogue of any one of the amino acid sequences given in Table 14 of Example 9. Most preferably the portion is a portion of the nucleic acid of SEQ ID NO: 131. Preferably, the portion encodes an amino acid sequence comprising (any one or more of the domains or motifs defined herein). Preferably, the portion encodes an amino acid sequence which, when used in the construction of a phylogenetic tree, tends to cluster with the group of AP2-2 polypeptides comprising the amino acid sequence represented by SEQ ID NO: 132 rather than with any other group.

Another nucleic acid variant useful in the methods of the invention is a nucleic acid capable of hybridising, under reduced stringency conditions, preferably under stringent conditions, with a nucleic acid encoding an AP2-2 polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table 14 of Example 9, or comprising introducing and expressing in a plant a nucleic acid capable of hybridising to a nucleic acid encoding an orthologue, paralogue or homologue of any of the nucleic acid sequences given in Table 14 of Example 9.

Hybridising sequences useful in the methods of the invention encode an AP2-2 polypeptide as defined herein, and have substantially the same biological activity as the amino acid sequences given in Table 14 of Example 9. Preferably, the hybridising sequence is capable of hybridising to any one of the nucleic acids given in Table 14 of Example 9, or to a portion of any of these sequences, a portion being as defined above, or wherein the hybridising sequence is capable of hybridising to a nucleic acid encoding an orthologue or paralogue of any one of the amino acid sequences given in Table 14 of Example 9. Most preferably, the hybridising sequence is capable of hybridising to a nucleic acid as represented by SEQ ID NO: 131 or to a portion thereof. Preferably, the hybridising sequence encodes an amino acid sequence comprising any one or more of the motifs or domains as defined herein. Preferably, the hybridising sequence encodes an amino acid sequence which, when used in the construction of a phylogenetic tree, tends to cluster with the group of AP2-2 polypeptides comprising the amino acid sequence represented by SEQ ID NO: 132 rather than with any other group.

Another nucleic acid variant useful in the methods of the invention is a splice variant encoding an AP2-2 polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table 14 of Example 9, or a splice variant of a nucleic acid encoding an orthologue, paralogue or homologue of any of the amino acid sequences given in Table 14 of Example 9.

Preferred splice variants are splice variants of a nucleic acid represented by SEQ ID NO: 131, or a splice variant of a nucleic acid encoding an orthologue or paralogue of SEQ ID NO: 132. Preferably, the amino acid sequence encoded by the splice variant comprises any one or more of the motifs or domains as defined herein. Preferably, the amino acid sequence encoded by the splice variant, when used in the construction of a phylogenetic tree, tends to cluster with the group of AP2-2 polypeptides comprising the amino acid sequence represented by SEQ ID NO: 132 rather than with any other group.

Another nucleic acid variant useful in performing the methods of the invention is an allelic variant of a nucleic acid encoding an AP2-2 polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table 14 of Example 9, or comprising introducing and expressing in a plant an allelic variant of a nucleic acid encoding an orthologue, paralogue or homologue of any of the amino acid sequences given in Table 14 of Example 9.

The allelic variants useful in the methods of the present invention have substantially the same biological activity as the AP2-2 polypeptide of SEQ ID NO: 132 and any of the amino acids depicted in Table 14 of Example 9. Allelic variants exist in nature, and encompassed within the methods of the present invention is the use of these natural alleles. Preferably, the allelic variant is an allelic variant of SEQ ID NO: 131 or an allelic variant of a nucleic acid encoding an orthologue or paralogue of SEQ ID NO: 132. Preferably, the amino acid encoded by the allelic variant comprises any one or more of the motifs or domains as defined herein. Preferably, the amino acid sequence encoded by the allelic variant, when used in the construction of a phylogenetic tree, tends to cluster with the group of AP2-2 polypeptides comprising the amino acid sequence represented by SEQ ID NO: 132 rather than with any other group.

Gene shuffling or directed evolution may also be used to generate variants of nucleic acids encoding AP2-2 polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table 14 of Example 9, or comprising introducing and expressing in a plant a variant of a nucleic acid encoding an orthologue, paralogue or homologue of any of the amino acid sequences given in Table 14 of Example 9, which variant nucleic acid is obtained by gene shuffling.

Preferably, the variant nucleic acid obtained by gene shuffling encodes an amino acid sequence comprising any one or more of the motifs or domains as defined herein. Preferably, the amino acid sequence encoded by the variant nucleic acid obtained by gene shuffling, when used in the construction of a phylogenetic tree, tends to cluster with the group of AP2-2 polypeptides comprising the amino acid sequence represented by SEQ ID NO: 132 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding AP2-2 polypeptides may be derived from any natural or artificial source. The nucleic acid may be modified from its native form in composition and/or genomic environment through deliberate human manipulation. Preferably the AP2-2 polypeptide-encoding nucleic acid is from a plant, further preferably from a dicotyledonous plant, more preferably from the family Poaceae, more preferably from the genus *Oryza,* most preferably from *Oryza sativa.*

Performance of the methods of the invention gives plants having enhanced yield-related traits. In particular, performance of the methods of the invention gives plants having increased yield, especially increased seed yield relative to control plants. The terms "yield" and "seed yield" are described in more detail in the "definitions" section herein. However it should be noted that the term "yield-related traits" does not encompass the metabolite content of plant cells and that the enhanced yield-related traits are not the result of increased stress resistance.

The present invention provides a method for increasing yield, especially seed yield of plants, relative to control plants, which method comprises modulating expression, preferably increasing expression, in a plant of a nucleic acid encoding an AP2-2 polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding an AP2-2 polypeptide as defined herein.

Performance of the methods of the invention gives plants grown under non-stress conditions or under mild drought conditions increased yield relative to suitable control plants grown under comparable conditions. Therefore, according to the present invention, there is provided a method for increasing yield in plants grown under non-stress conditions, which method comprises increasing expression in a plant of a nucleic acid encoding an AP2-2 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a POI polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding an AP2-2 polypeptide as defined above.

The invention also provides genetic constructs and vectors to facilitate introduction and/or expression in plants of nucleic acids encoding AP2-2 polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an AP2-2 polypeptide as defined above;
(b) one or more control sequences capable of driving expression of the nucleic acid sequence of (a); and optionally
(c) a transcription termination sequence.

The term "control sequence" and "termination sequence" are as defined herein.

Plants are transformed with a vector comprising any of the nucleic acids described above.

The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter may be used to drive expression of the nucleic acid sequence. A constitutive promoter is particularly useful in the methods of the invention, preferably the constitutive promoter is a strong constitutive promoters. It should be clear that the applicability of the present invention is not restricted to the AP2-2 polypeptide-encoding nucleic acid represented by SEQ ID NO: 131, nor is the applicability of the invention restricted to expression of an AP2-2 polypeptide-encoding nucleic acid when driven by a constitutive promoter.

The constitutive promoter is preferably a GOS2 promoter, more preferably the rice GOS2 promoter. See Table 2a in the "definitions" section herein for further examples of constitutive promoters.

Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding an AP2-2 polypeptide as defined hereinabove.

More specifically, the present invention provides a method for the production of transgenic plants having increased yield, which method comprises:

(i) introducing and expressing in a plant or plant cell an AP2-2 polypeptide-encoding nucleic acid; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding an AP2-2 polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art. Examples of methods for increasing expression are given in the "definitions" section.

As mentioned above, a preferred method for modulating (preferably, increasing) expression of a nucleic acid encoding an AP2-2 polypeptide is by introducing and expressing in a plant a nucleic acid encoding an AP2-2 polypeptide; however the effects of performing the method, i.e. enhancing yield-related traits may also be achieved using other well known techniques. A description of some of these techniques will now follow.

The effects of the invention may also be reproduced using the technique of T-DNA activation or TILLING (Targeted Induced Local Lesions In Genomes); for a description of the same see the "definitions" section.

The effects of the invention may also be reproduced using homologous recombination; for a description of the same see the "definitions" section.

The present invention also encompasses use of nucleic acids encoding AP2-2 polypeptides as described herein and use of this AP2-2 polypeptide in enhancing any of the aforementioned yield-related traits in plants.

Nucleic acids encoding AP2-2 polypeptide described herein, or the AP2-2 polypeptides themselves, may find use in breeding programmes in which a DNA marker is identified which may be genetically linked to an AP2-2 polypeptide-encoding gene. The nucleic acids/genes, or the AP2-2 polypeptides themselves may be used to define a molecular marker. This DNA or protein marker may then be used in breeding programmes to select plants having enhanced yield-related traits as defined hereinabove in the methods of the invention.

Allelic variants of an AP2-2 polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding AP2-2 polypeptides may also be used as probes for genetically and physically mapping the genes that they are a part of, and as markers for traits linked to those genes. Such information may be useful in plant breeding in order to develop lines with desired phenotypes. Such use of AP2-2 polypeptide-encoding nucleic acids requires only a nucleic acid sequence of at least 15 nucleotides in length. The AP2-2 polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the AP2-2-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the AP2-2 polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples can be found in the "definitions" section herein.

The present invention further provides a method for enhancing yield-related traits in plants relative to control plants, comprising modulating expression in a plant of a nucleic acid encoding an AP2-70-like polypeptide.

The invention also provides hitherto unknown AP2-70-like-encoding nucleic acids and AP2-70-like polypeptides.

According to a further embodiment of the present invention, there is therefore provided an isolated nucleic acid molecule comprising:

(i) a nucleic acid represented by SEQ ID NO: 257;

(ii) the complement of a nucleic acid represented by SEQ ID NO: 257;

(iii) a nucleic acid encoding an AP2-70-like polypeptide having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 258, and having in increasing order of preference at least 50%, 55%, 60%, 65%, 70%, 75%, 80%, 85%, 90%, 95%, 96%, 97%, 98%, 99% or more sequence identity to SEQ ID NO: 331:

PFLMQWLNLLPLPVLDSSSWCPEHFHNSESDALP (which represents the C-terminal region of SEQ ID NO:

258).

According to a further embodiment of the present invention, there is also provided an isolated polypeptide comprising:

(i) an amino acid sequence represented by SEQ ID NO: 258;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 258, and having in increasing order of preference at least 50%, 55%, 60%, 65%, 70%, 75%, 80%, 85%, 90%, 95%, 96%, 97%, 98%, 99% or more sequence identity to SEQ ID NO: 331: PFLMQWLNLLPLPVLDSSSWCPEHFHNSESDALP (which represents the C-terminal region of SEQ ID NO: 258);
(iii) derivatives of any of the amino acid sequences given in (i) or (ii) above.

A preferred method for modulating (preferably, increasing) expression of a nucleic acid encoding an AP2-70-like polypeptide is by introducing and expressing in a plant a nucleic acid encoding an AP2-70-like polypeptide.
Any reference hereinafter to a "protein useful in the methods of the invention" is taken to mean an AP2-70-like polypeptide as defined herein. Any reference hereinafter to a "nucleic acid useful in the methods of the invention" is taken to mean a nucleic acid capable of encoding such an AP2-70-like polypeptide. The nucleic acid to be introduced into a plant (and therefore useful in performing the methods of the invention) is any nucleic acid encoding the type of protein which will now be described, hereafter also named "AP2-70-like nucleic acid" or "AP2-70-like gene".
AP2-70-like polypeptides as defined below fall within Group Ib (A-6) according to the classification of Nakano *et al.,* 2006.
An "AP2-70-like polypeptide" as defined herein refers to any polypeptide comprising the following:

(i) An AP2 DNA-binding domain as represented by SEQ ID NO: 332: YRGVRQRHWGKWVAEIRLPRNRTRL WLGTFDTAEEAALAYDSAAFRLRGESARLNF , or a domain having in increasing order of preference at least 50%, 55%, 60%, 65%, 70%, 75%, 80%, 85%, 90%, 95% or more sequence identity to the domain represented by SEQ ID NO: 332; and
(ii) Having in increasing order of preference at least 50%, 55%, 60%, 65%, 70%, 75%, 80%, 85%, 90%, 95% or more sequence identity to the (*Oryza sativa*) polypeptide sequence represented by SEQ ID NO: 258; and
(iii) Comprising Motif XVIII represented by SEQ ID NO: 333: RLPXNX$^1$RXRXWLGT F/Y D/E T/S, where X is any amino acid and X$^1$ is zero, one or more, and up to 30, gaps; and
(iv) Comprising Motif XIX represented by SEQ ID NO: 334: RG D/E.

In the AP2 DNA-binding domain described above under (i), YRG and LAYD, as highlighted below, are preferred specific boxes for DNA-binding.
SEQ ID NO: 332:
<u>**YRG**</u>VRQRHWGKWVAEIRLPRNRTRLWLGTFDTAEEAA<u>**LAYD**</u>SAAFRLRGESARLNF.
Preferably, the AP2 DNA-binding domain described above under (i) comprises at least residues LPRNRTRLWLGTFDT.
Motif XVIII (SEQ ID NO: 333) is preferably RLP K/R/Q NX$^1$R T/V/M R L/V WLGT F/Y D/E T/S, where X$^1$ is zero, one or more, and up to 30, gaps.
More preferably, Motif XVIII (SEQ ID NO: 333) is RLPRNX$^1$RTRLWLGTFDT, wherein X$^1$ is zero, one or more, and up to 30, gaps.
AP2-70 polypeptides as defined herein may also comprise one or more of the following motifs:
<u>Motif XX/SEQ ID NO: 335</u>: WDESESFLLHKYPSLEIDWDAILS, or a motif having in increasing order of preference at least 50%, 55%, 60%, 65%, 70%, 75%, 80%, 85%, 90%, 95% or more sequence identity to Motif XX. Adapted from Nakano *et al.,* 2006 (CMI-1).
<u>Motif XXI/SEQ ID NO: 336:</u> GPPLHAAVDAKLHAICH, or a motif having in increasing order of preference at least 50%, 55%, 60%, 65%, 70%, 75%, 80%, 85%, 90%, 95% or more sequence identity to Motif XXI. Adapted from Nakano *et al.,* 2006 (CMI-2).
<u>Motif XXII/SEQ ID NO: 337:</u> GANYLTPAQVLHVQAQLQRLRRP, or a motif having in increasing order of preference at least 50%, 55%, 60%, 65%, 70%, 75%, 80%, 85%, 90%, 95% or more sequence identity to Motif XXII. Adapted from Nakano *et al.,* 2006 (CMI-3).
<u>Motif XXIII/SEQ ID NO: 338:</u> VDSKELMGALAPSMVSFSYPCSEQSASS, or a motif having in increasing order of preference at least 50%, 55%, 60%, 65%, 70%, 75%, 80%, 85%, 90%, 95% or more sequence identity to Motif XXIII. Adapted from Nakano *et al.,* 2006 (CMI-4).
Any of Motifs XVIII and XX to XXIII described above may comprise one, two or three conservative and/or non-conservative change and/or deletion at any position.

The term "domain" and "motif" is defined in the "definitions" section herein. Specialist databases exist for the identification of domains. Examples are given in the "definitions" section herein.

Furthermore, AP2-70-like polypeptides (at least in their native form) typically have DNA-binding activity. Tools and techniques for measuring DNA-binding activity are well known in the art.

The present invention is illustrated by transforming plants with the nucleic acid sequence represented by SEQ ID NO: 257, encoding the polypeptide sequence of SEQ ID NO: 258. However, performance of the invention is not restricted to these sequences; the methods of the invention may advantageously be performed using any AP2-70-like-encoding nucleic acid or AP2-70-like polypeptide as defined herein.

Examples of nucleic acids encoding AP2-70-like polypeptides are given in Table 18 of Example 18 herein. Such nucleic acids are useful in performing the methods of the invention. The amino acid sequences given in Table 18 of Example 18 are example sequences of orthologues and paralogues of the AP2-70-like polypeptide represented by SEQ ID NO: 258, the terms "orthologues" and "paralogues" being as defined herein.

Nucleic acid variants may also be useful in practising the methods of the invention. Examples of such variants include nucleic acids encoding homologues and derivatives of any one of the amino acid sequences given in Table 18 of Example 18, the terms "homologue" and "derivative" being as defined herein. Also useful in the methods of the invention are nucleic acids encoding homologues and derivatives of orthologues or paralogues of any one of the amino acid sequences given in Table 18 of Example 18. Homologues and derivatives useful in the methods of the present invention have substantially the same biological and functional activity as the unmodified protein from which they are derived.

Further nucleic acid variants useful in practising the methods of the invention include portions of nucleic acids encoding AP2-70-like polypeptides, nucleic acids hybridising to nucleic acids encoding AP2-70-like polypeptides, splice variants of nucleic acids encoding AP2-70-like polypeptides, allelic variants of nucleic acids encoding AP2-70-like polypeptides and variants of nucleic acids encoding AP2-70-like polypeptides obtained by gene shuffling. The terms hybridising sequence, splice variant, allelic variant and gene shuffling are as described herein.

Nucleic acids encoding AP2-70-like polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table 18 of Example 18, or a portion of a nucleic acid encoding an orthologue, paralogue or homologue of any of the amino acid sequences given in Table 18 of Example 18.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

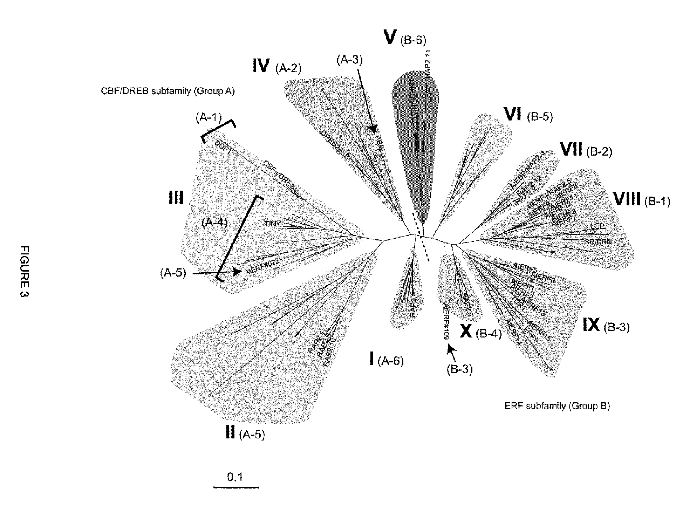

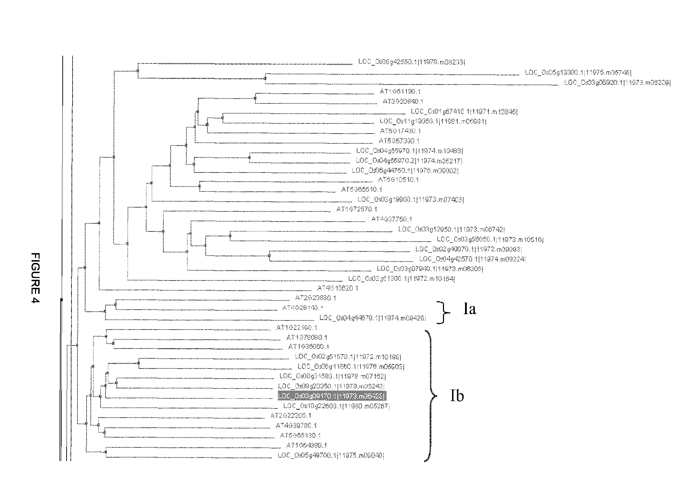

Portions useful in the methods of the invention, encode an AP2-70-like polypeptide as defined herein, and have substantially the same biological activity as the amino acid sequences given in Table 18 of Example 18. Preferably, the portion is a portion of any one of the nucleic acids given in Table 18 of Example 18, or is a portion of a nucleic acid encoding an orthologue or paralogue of any one of the amino acid sequences given in Table 18 of Example 18. Preferably the portion is, in increasing order of preference at least 600, 650, 700, 750 consecutive nucleotides in length, the consecutive nucleotides being of any one of the nucleic acid sequences given in Table 18 of Example 18, or of a nucleic acid encoding an orthologue or paralogue of any one of the amino acid sequences given in Table 18 of Example 18. Most preferably the portion is a portion of the nucleic acid of SEQ ID NO: 257. Preferably, the portion encodes an amino acid sequence which when used in the construction of a phylogenetic tree, such as the one depicted in Figures. 3 and 4, tends to cluster with the group of AP2-70-like polypeptides (Group I(A-6), specifically Group Ib: see Nakanao et al., 2006 for classification) comprising the amino acid sequence represented by SEQ ID NO: 258 rather than with any other group.

Another nucleic acid variant useful in the methods of the invention is a nucleic acid capable of hybridising, under reduced stringency conditions, preferably under stringent conditions, with a nucleic acid encoding an AP2-70-like polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table 18 of Example 18, or comprising introducing and expressing in a plant a nucleic acid capable of hybridising to a nucleic acid encoding an orthologue, paralogue or homologue of any of the nucleic acid sequences given in Table 18 of Example 18.

Hybridising sequences useful in the methods of the invention encode an AP2-70-like polypeptide as defined herein, and have substantially the same biological activity as the amino acid sequences given in Table 18 of Example 18. Preferably, the hybridising sequence is capable of hybridising to any one of the nucleic acids given in Table 18 of Example 18, or to a portion of any of these sequences, a portion being as defined above, or wherein the hybridising sequence is capable of hybridising to a nucleic acid encoding an orthologue or paralogue of any one of the amino acid sequences given in Table 18 of Example 18. Most preferably, the hybridising sequence is capable of hybridising to a nucleic acid as represented by SEQ ID NO: 257 or to a portion thereof.

Preferably, the hybridising sequence encodes an amino acid sequence which when used in the construction of a phylogenetic tree, such as the one depicted in Figures 3 and 4, tends to cluster with the group of AP2-70-like polypeptides (Group I(A-6), specifically Group Ib: see Nakanao *et al.,* 2006 for classification) comprising the amino acid sequence represented by SEQ ID NO: 258 rather than with any other group.

Another nucleic acid variant useful in the methods of the invention is a splice variant encoding an AP2-70-like polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table 18 of Example 18, or a splice variant of a nucleic acid encoding an orthologue, paralogue or homologue of any of the amino acid sequences given in Table 18 of Example 18.

Preferred splice variants are splice variants of a nucleic acid represented by SEQ ID NO: 257, or a splice variant of a nucleic acid encoding an orthologue or paralogue of SEQ ID NO: 258. Preferably, the amino acid sequence encoded by the splice variant, when used in the construction of a phylogenetic tree, such as the one depicted in Figures 3 and 4, tends to cluster with the group of AP2-70-like polypeptides (Group I(A-6), specifically Group Ib: see Nakanao *et al.,* 2006 for classification) comprising the amino acid sequence represented by SEQ ID NO: 258 rather than with any other group.

Another nucleic acid variant useful in performing the methods of the invention is an allelic variant of a nucleic acid encoding an AP2-70-like polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table 18 of Example 18, or comprising introducing and expressing in a plant an allelic variant of a nucleic acid encoding an orthologue, paralogue or homologue of any of the amino acid sequences given in Table 18 of Example 18.

The allelic variants useful in the methods of the present invention have substantially the same biological activity as the AP2-70-like polypeptide of SEQ ID NO: 258 and any of the amino acids depicted in Table 18 of Example 18. Allelic variants exist in nature, and encompassed within the methods of the present invention is the use of these natural alleles. Preferably, the allelic variant is an allelic variant of SEQ ID NO: 257 or an allelic variant of a nucleic acid encoding an orthologue or paralogue of SEQ ID NO: 258. Preferably, the amino acid sequence encoded by the allelic variant, when used in the construction of a phylogenetic tree, such as the one depicted in Figures 3 and 4, tends to cluster with the AP2-70-like polypeptides (Group I(A-6), specifically Group Ib: see Nakanao *et al.,* 2006 for classification) comprising the amino acid sequence represented by SEQ ID NO: 258 rather than with any other group.

Gene shuffling or directed evolution may also be used to generate variants of nucleic acids encoding AP2-70-like polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table 18 of Example 18, or comprising introducing and expressing in a plant a variant of a nucleic acid encoding an orthologue, paralogue or homologue of any of the amino acid sequences given in Table 18 of Example 18, which variant nucleic acid is obtained by gene shuffling.

Preferably, the amino acid sequence encoded by the variant nucleic acid obtained by gene shuffling, when used in the construction of a phylogenetic tree such as the one depicted in Figures 3 and 4, tends to cluster with the group of AP2-70-like polypeptides (Group I(A-6), specifically Group Ib: see Nakanao *et al.,* 2006 for classification) comprising the amino acid sequence represented by SEQ ID NO: 258 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding AP2-70-like polypeptides may be derived from any natural or artificial source. The nucleic acid may be modified from its native form in composition and/or genomic environment through deliberate human manipulation. Preferably the AP2-70-like polypeptide-encoding nucleic acid is from a plant, further preferably from a monocotyledonous plant, more preferably from the family poaceae, most preferably the nucleic acid is from *Oryza sativa.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

The present invention provides a method for increasing yield, especially seed yield of plants, relative to control plants, which method comprises modulating expression, preferably increasing expression, in a plant of a nucleic acid encoding an AP2-70-like polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding an AP2-70-like polypeptide as defined herein.

Performance of the methods of the invention gives plants grown under non-stress conditions or under mild drought conditions increased yield relative to suitable control plants grown under comparable conditions. Therefore, according to the present invention, there is provided a method for increasing yield in plants grown under non-stress conditions or under mild drought conditions, which method comprises increasing expression in a plant of a nucleic acid encoding an AP2-70-like polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding an AP2-70-like polypeptide. Nutrient deficiency may result from a lack or excess of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding an AP2-70-like polypeptide as defined above.

The invention also provides genetic constructs and vectors to facilitate introduction and/or expression in plants of nucleic acids encoding AP2-70-like polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(d) a nucleic acid encoding an AP2-70-like polypeptide as defined above;
(e) one or more control sequences capable of driving expression of the nucleic acid sequence of (a); and optionally
(f) a transcription termination sequence.

Preferably, the nucleic acid encoding an AP2-70-like polypeptide is:

(i) a nucleic acid represented by SEQ ID NO: 257;
(ii) the complement of a nucleic acid represented by SEQ ID NO: 257;
(iv) a nucleic acid encoding an AP2-70-like polypeptide having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 258, and having in increasing order of preference at least 50%, 55%, 60%, 65%, 70%, 75%, 80%, 85%, 90%, 95%, 96%, 97%, 98%, 99% or more sequence identity to SEQ ID NO: 331: PFLMQWLNLLPLPVLDSSSWCPEHFHNSESDALP (which represents the C-terminal region of SEQ ID NO: 258).

The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. See the "Definitions" section herein for definitions of the various promoter types. Also useful in the methods of the invention is a root-specific promoter.

It should be clear that the applicability of the present invention is not restricted to the AP2-70-like polypeptide-encoding nucleic acid represented by SEQ ID NO: 257, nor is the applicability of the invention restricted to expression of an AP2-70-like polypeptide-encoding nucleic acid when driven by a constitutive promoter, or when driven by a root-specific promoter.

The constitutive promoter is preferably a GOS2 promoter, preferably a GOS2 promoter from rice. Further preferably the constitutive promoter is represented by a nucleic acid sequence substantially similar to SEQ ID NO: 39 or SEQ ID NO: 339 most preferably the constitutive promoter is as represented by SEQ ID NO: 39 or SEQ ID NO: 339. See Table 2a in the "Definitions" section herein for further examples of constitutive promoters.

According to another preferred feature of the invention, the nucleic acid encoding an AP2-70-like polypeptide is operably linked to a root-specific promoter. The root-specific promoter is preferably an RCc3 promoter (Plant Mol Biol. 1995 Jan;27(2):237-48), more preferably the RCc3 promoter is from rice, further preferably the RCc3 promoter is represented by a nucleic acid sequence substantially similar to SEQ ID NO: 110 or SEQ ID NO: 340, most preferably the promoter is as represented by SEQ ID NO: 110. Examples of other root-specific promoters which may also be used to perform the methods of the invention are shown in Table 2b herein.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein.

The invention also provides a method for the production of transgenic plants having enhanced yield-related traits relative to control plants, comprising introduction and expression in a plant of any nucleic acid encoding an AP2-70-like polypeptide as defined hereinabove.

More specifically, the present invention provides a method for the production of transgenic plants having increased enhanced yield-related traits, particularly increased (seed) yield, which method comprises:

(iii) introducing and expressing in a plant or plant cell an AP2-70-like polypeptide-encoding nucleic acid; and
(iv) cultivating the plant cell under conditions promoting plant growth and development.

The nucleic acid of (i) may be any of the nucleic acids capable of encoding an AP2-70-like polypeptide as defined herein. Preferably, the nucleic acid is:

(i) a nucleic acid represented by SEQ ID NO: 257;
(ii) the complement of a nucleic acid represented by SEQ ID NO: 257;
(iii) a nucleic acid encoding an AP2-70-like polypeptide having, in increasing order of preference, at least 50%, 55%, 60%, 65%, 70%, 75%, 80%, 85%, 90%, 95%, 96%, 97%, 98%, 99% or more sequence identity to the amino acid sequence represented by SEQ ID NO: 258, and having in increasing order of preference at least 50%, 55%, 60%, 65%, 70%, 75%, 80%, 85%, 90%, 95%, 96%, 97%, 98%, 99% or more sequence identity to SEQ ID NO: 331: PFLMQWLNLLPLPVLDSSSWCPEHFHNSESDALP (which represents the C-terminal region of SEQ ID NO: 258).

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a AP2-70-like polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art. Examples are given in the "definitions" section herein.

As mentioned above, a preferred method for modulating (preferably, increasing) expression of a nucleic acid encoding an AP2-70-like polypeptide is by introducing and expressing in a plant a nucleic acid encoding an AP2-70-like polypeptide; however the effects of performing the method, i.e. enhancing yield-related traits may also be achieved using other well known techniques. A description of some of these techniques can be found in the "definitions" section.

The effects of the invention may also be reproduced using the technique of T-DNA activation or TILLING (Targeted Induced Local Lesions In Genomes); for a description of the same see the "definitions" section.

The effects of the invention may also be reproduced using homologous recombination; for a description of the same see the "definitions" section.

The present invention also encompasses use of nucleic acids encoding AP2-70-like polypeptides as described herein and use of these AP2-70-like polypeptide in enhancing any of the aforementioned yield-related traits in plants.

Nucleic acids encoding AP2-70-like polypeptide described herein, or the AP2-70-like polypeptides themselves, may find use in breeding programmes in which a DNA marker is identified which may be genetically linked to an AP2-70-like polypeptide-encoding gene. The nucleic acids/genes, or the AP2-70-like polypeptides themselves may be used to define a molecular marker. This DNA or protein marker may then be used in breeding programmes to select plants having enhanced yield-related traits as defined hereinabove in the methods of the invention.

Allelic variants of an AP2-70-like polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding AP2-70-like polypeptides may also be used as probes for genetically and physically mapping the genes that they are a part of, and as markers for traits linked to those genes. Such information may be useful in plant breeding in order to develop lines with desired phenotypes. Such use of AP2-70-like polypeptide-encoding nucleic acids requires only a nucleic acid sequence of at least 15 nucleotides in length. The AP2-70-like polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the AP2-70-like-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the AP2-70-like polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping.

Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

**Description of figures**

The present invention will now be described with reference to the following figures in which:

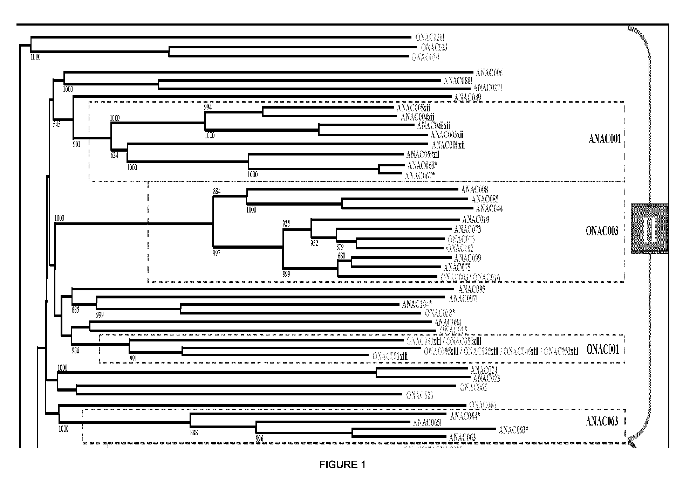

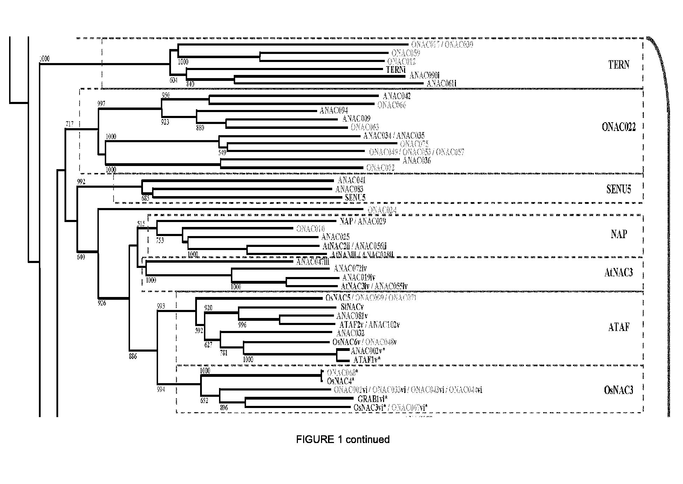

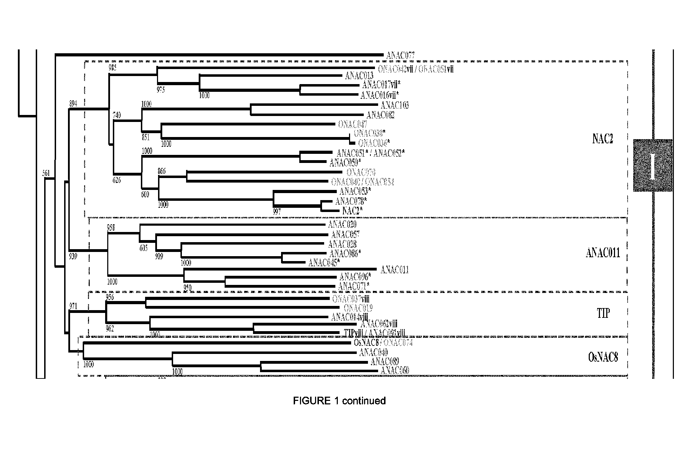

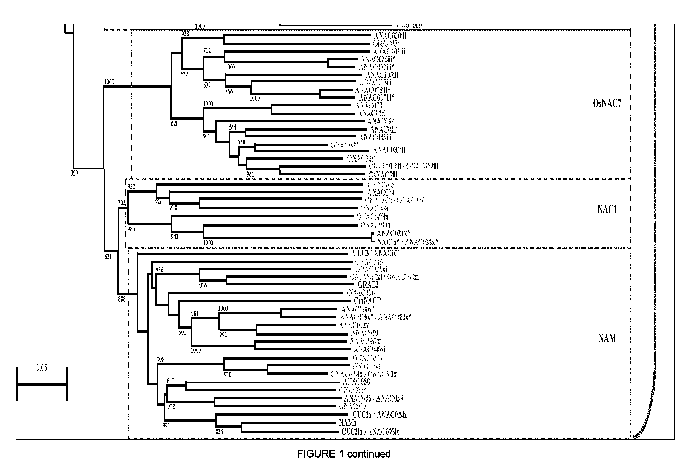

**Fig. 1** shows a phylogenetic tree taken from Ooka et al., 2003 (DNA Research 10, 239-247). The dashed box 3$^{rd}$ from the bottom, marked "OsNAC7", is the group comprising the sequence of SEQ ID NO: 2. The cluster of NACs starting from ONAC022 until OsNAC3, represent the cluster comprising the sequences of SEQ ID NO: 51, SEQ ID NO: 53, SEQ ID NO: 55, SEQ ID NO: 57 and SEQ ID NO: 59. Amino acid sequences clustering in these groups (the amino acid sequences given in Tables 3 and 4, for example) are considered to be useful in the methods of the invention.

**Fig. 2** shows the sequence of SEQ ID NO: 132 with (A): Putative nuclear localisation signal, (B): AP2/ERF DNA binding domain, (1): CMVII-1 motif, (3): CMVII-3 motif, (4): CMVII-4 motif, (5): CMVII-5 motif, (6): CMVII-6 motif, (7): CMVII-7 motif, (8): CMVII-8 motif. The CMVII motifs were identified according to Nakano et al. (2006).

**Fig. 3** shows a phylogenetic tree taken from Nakano *et al.* (Plant Physiol. Vol. 140, 2006) in which the group indicated as I(A-6) includes the group of AP2-70-like polypeptides defined herein.

**Fig. 4** shows a section of a phylogenetic tree comprising AP2-70-like polypeptide sequences defined herein and expanding the group indicated as Group I(A-6) in Nakano *et al.,* 2006. The phylogenetic tree was constructed using a neighbour-joining clustering algorithm as provided in the AlignX programme from the Vector NTI (Invitrogen).

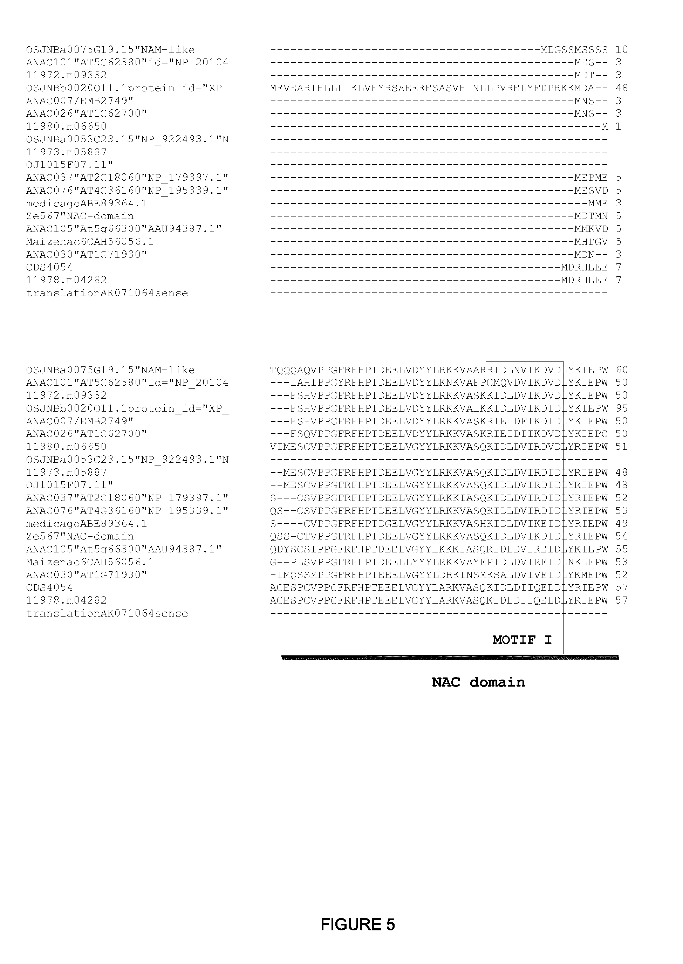

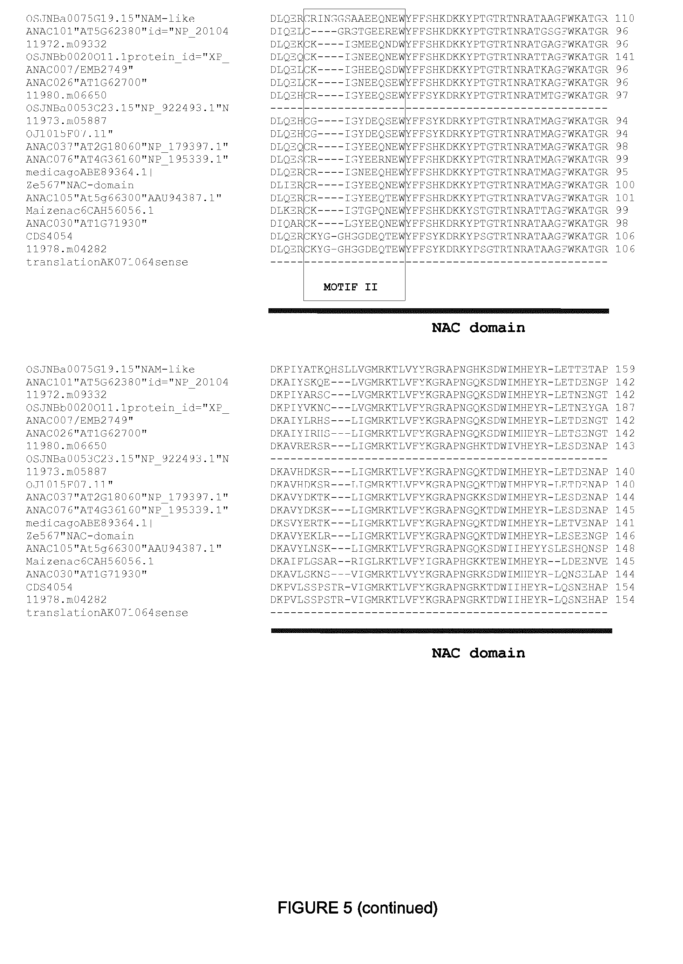

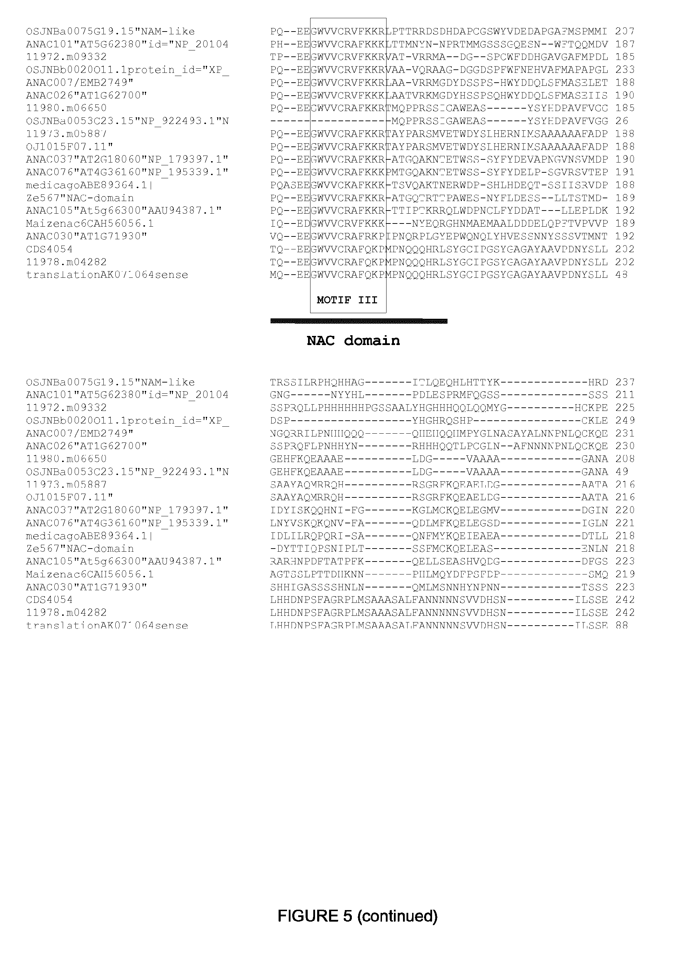

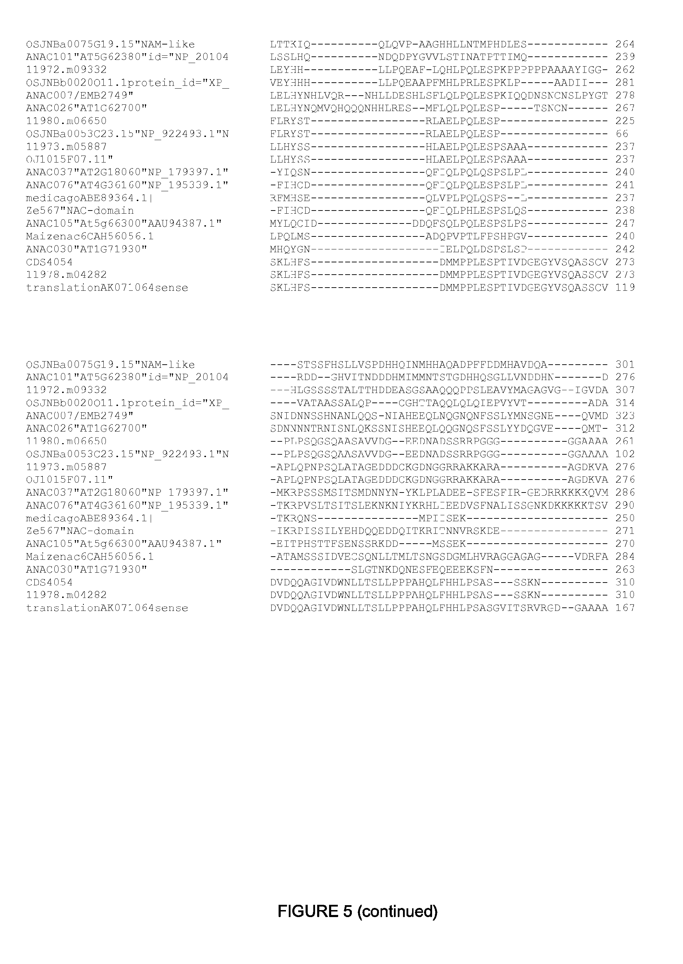

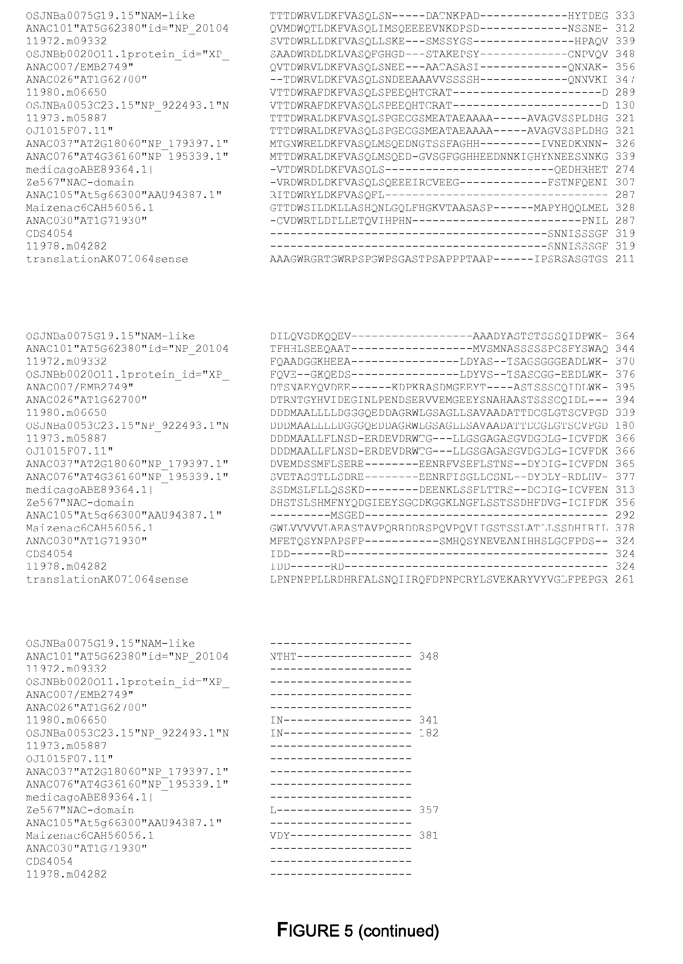

**Fig. 5** shows an alignment of NAC transcription factors as defined hereinabove. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. The NAC domain and Motifs I to III are indicated.

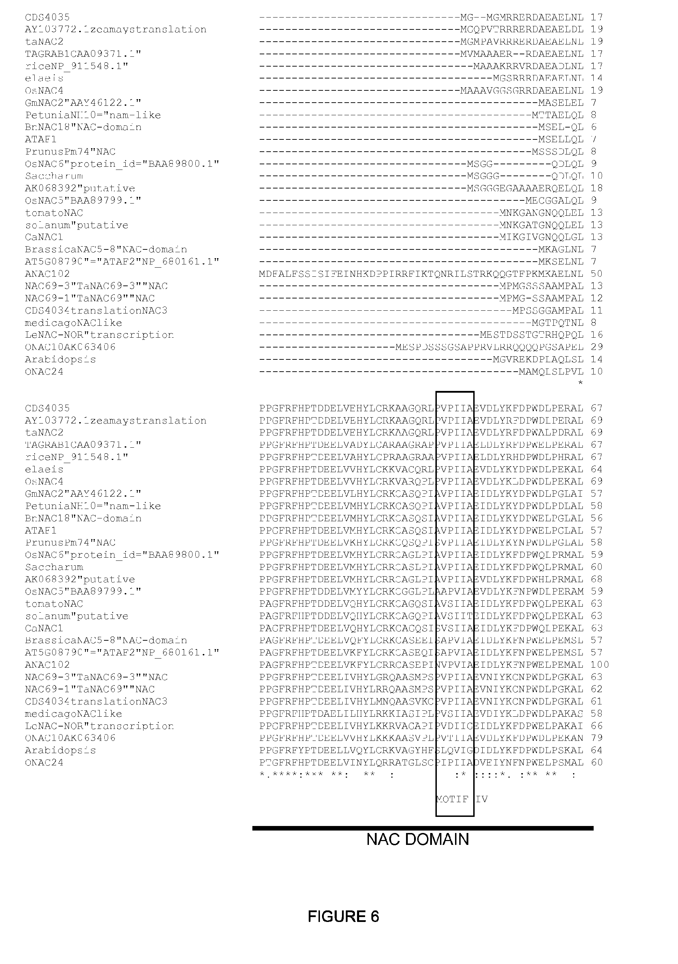

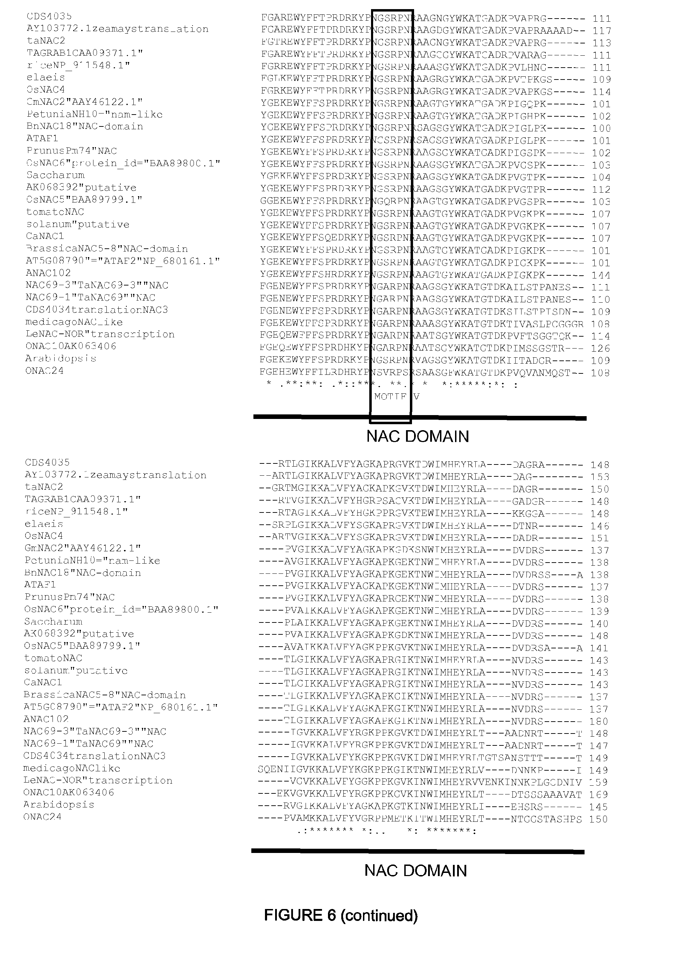

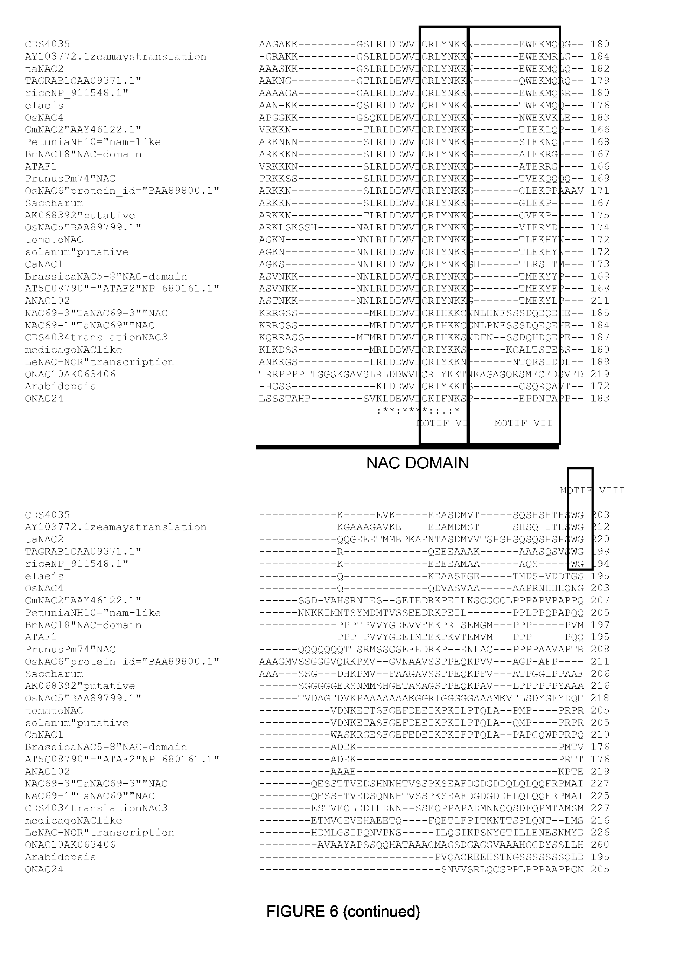

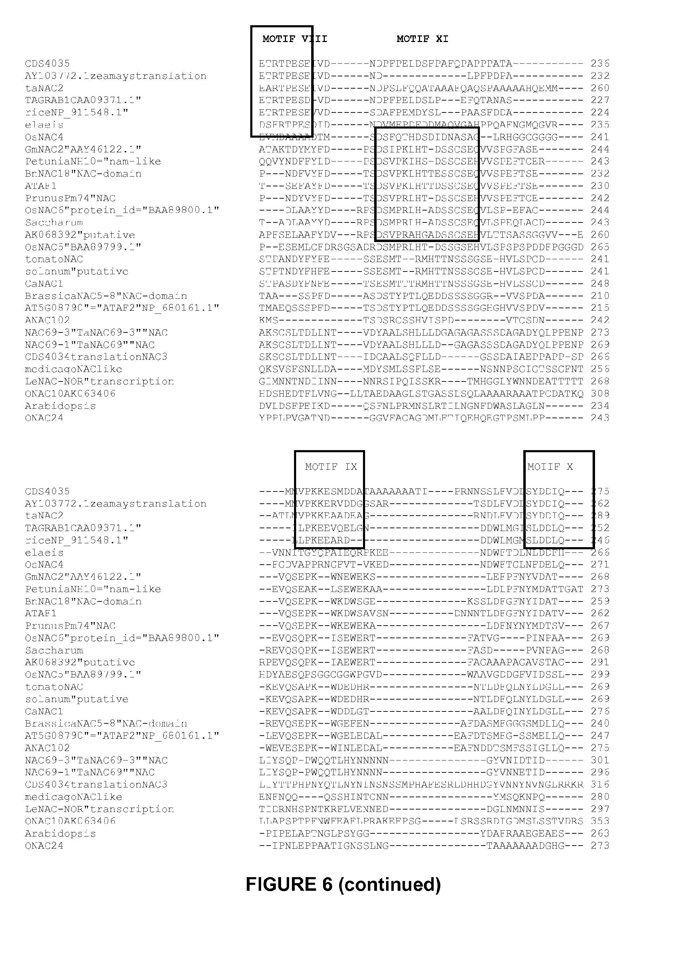

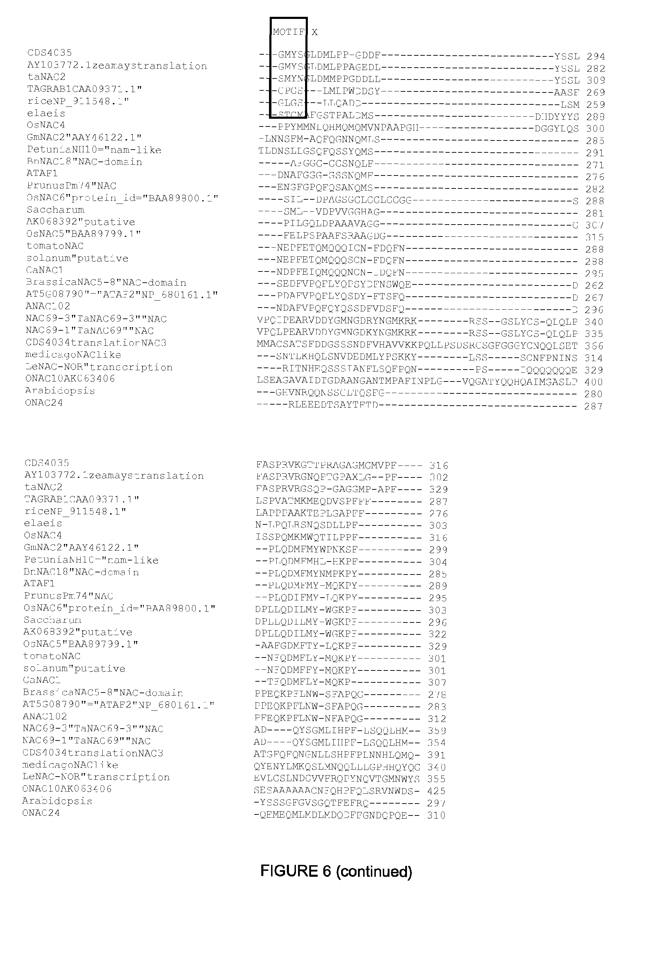

**Fig. 6** shows an alignment of NAC transcription factors as defined hereinabove. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. The NAC domain and Motifs IV to XI are indicated.

**Fig. 7** shows a CLUSTAL W multiple sequence alignment of AP2-2 polypeptides from *Arabidopsis* and rice. SEQ ID NO: 132 (Os06g09390) is indicated in bold and the conserved regions (motifs XII to XVII, SEQ ID NO: 133 to SEQ ID NO: 138) are underlined.

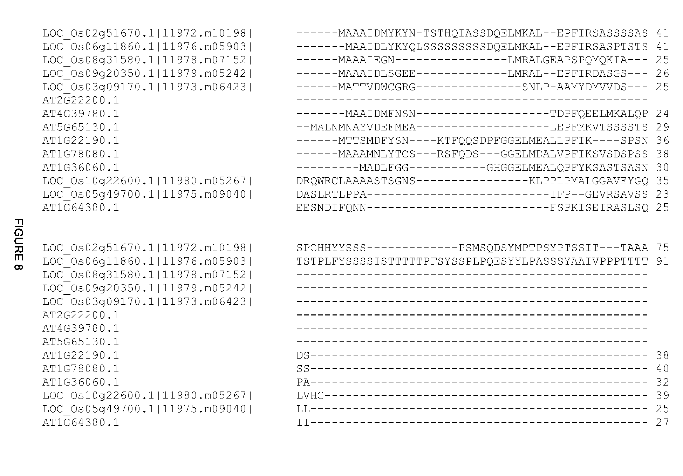

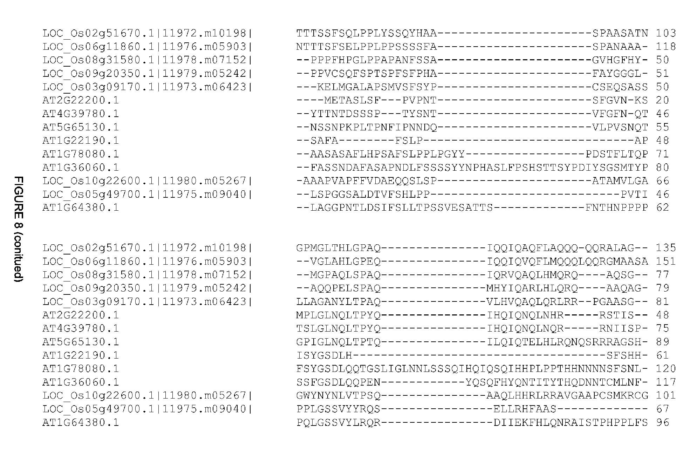

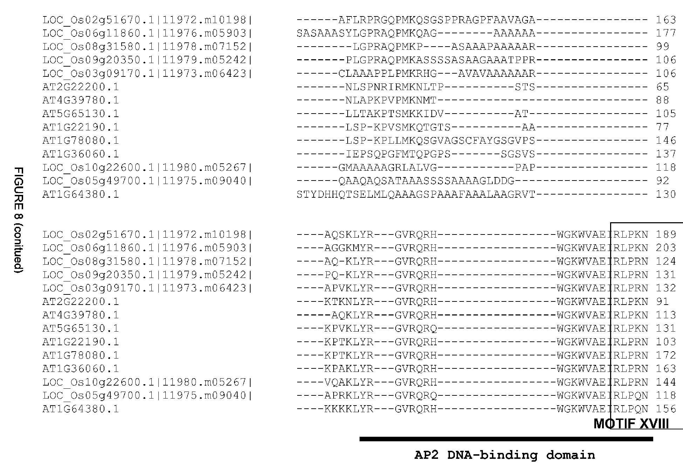

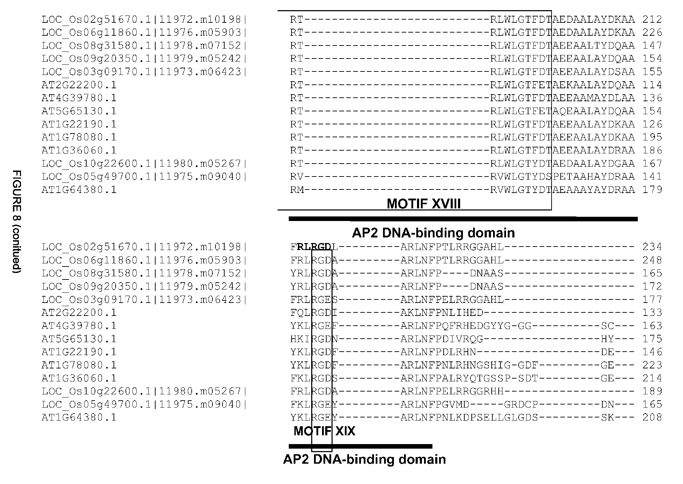

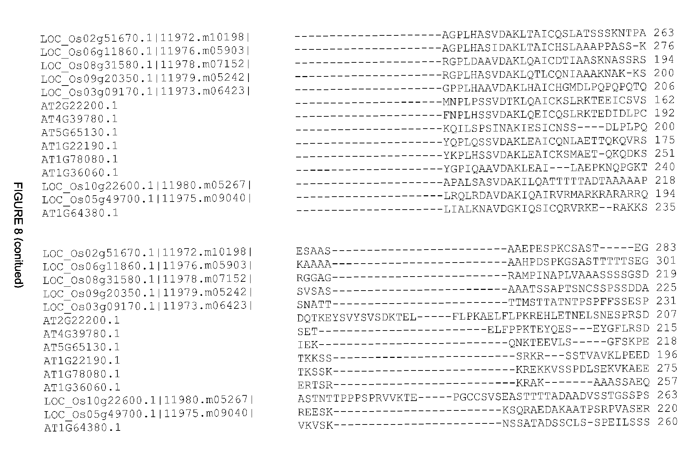

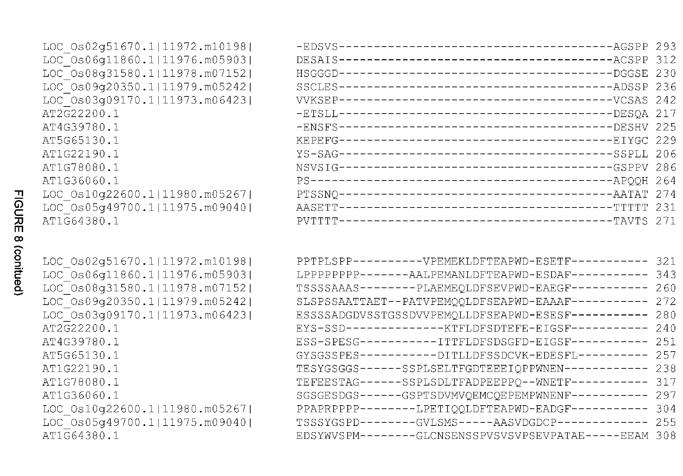

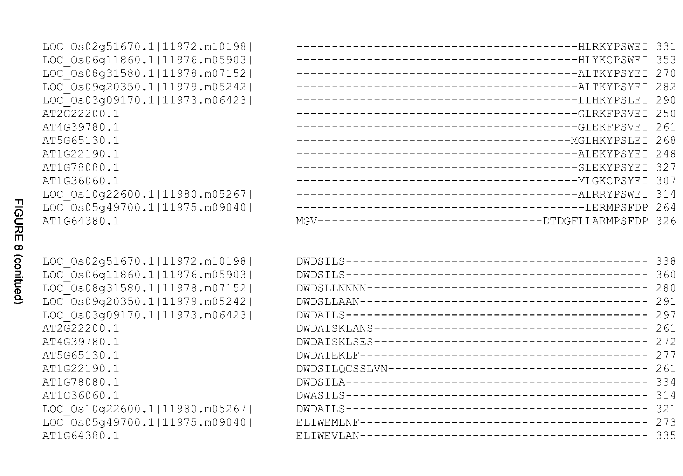

**Fig. 8** shows an alignment of AP2-70-like polypeptides with Motifs XVIII and XIX indicated and with the AP2 DNA-binding domain also indicated.

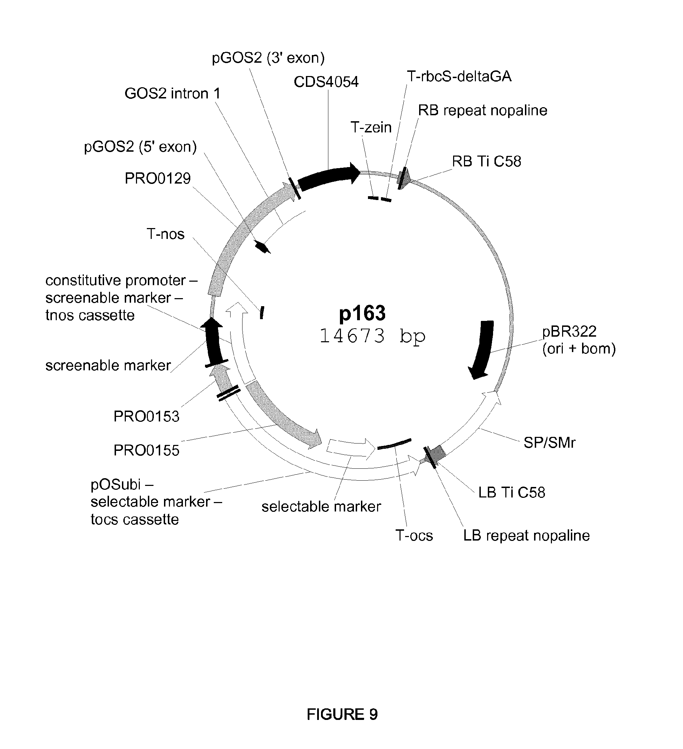

**Fig. 9** shows a binary vector p163, for increased expression in *Oryza sativa* of an *Oryza sativa* NAC transcription factor-encoding nucleic acid under the control of a GOS2 promoter (internal reference PR00129).

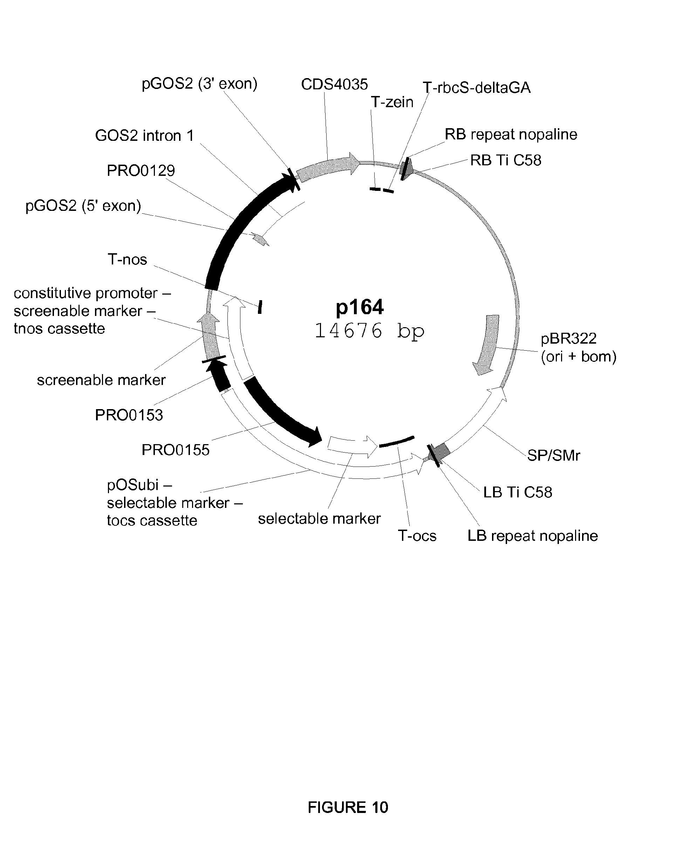

**Fig. 10** shows a binary vector p164, for increased expression in *Oryza sativa* of an *Oryza sativa* NAC1 (SEQ ID NO: 50) transcription factor-encoding nucleic acid under the control of a GOS2 promoter (internal reference PRO012) for constitutive expression.

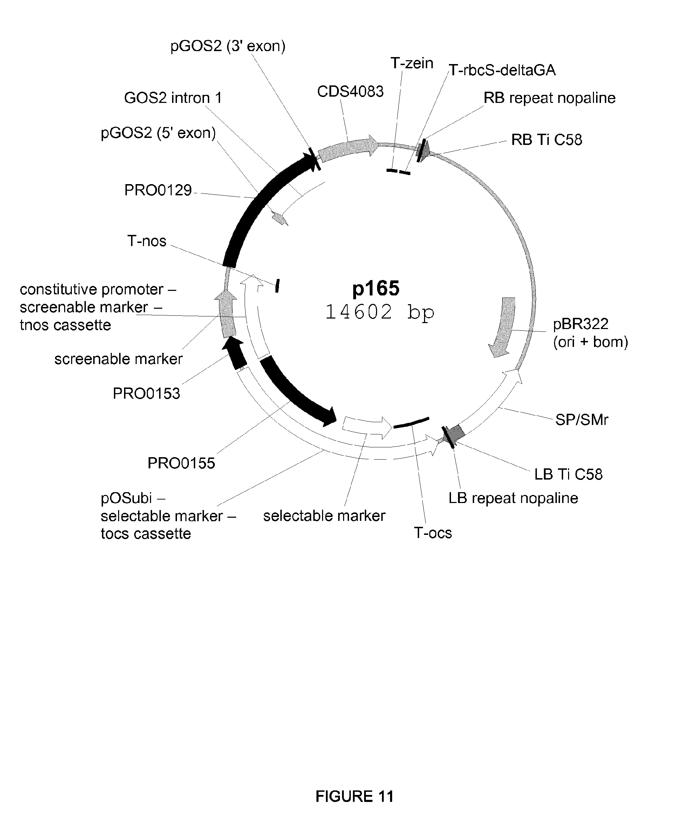

**Fig. 11** shows a binary vector p165, for increased expression in *Oryza sativa* of an *Oryza sativa* NAC4 (SEQ ID NO: 52) transcription factor-encoding nucleic acid under the control of a GOS2 promoter (internal reference PRO012) for constitutive expression.

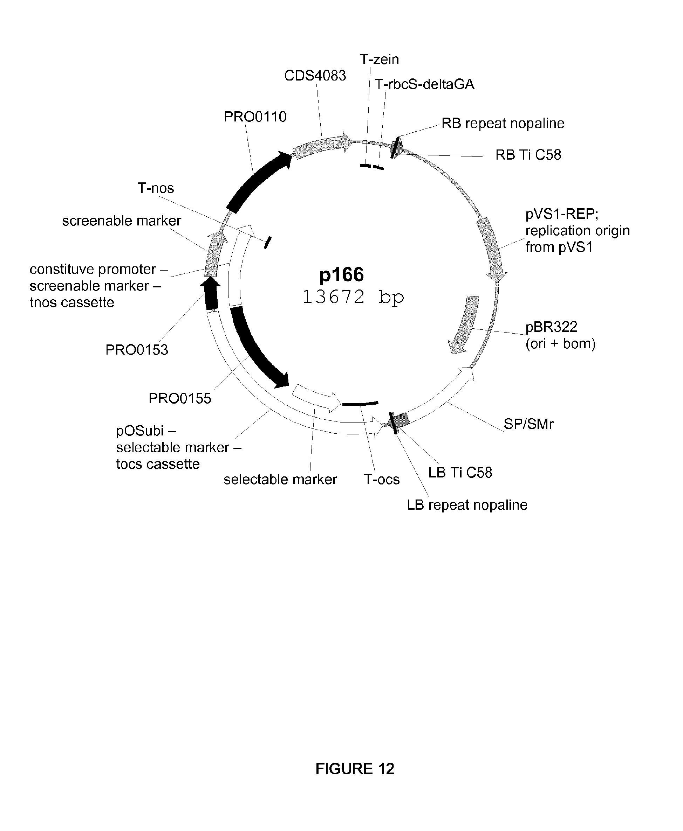

**Fig. 12** shows a binary vector p166, for increased expression in *Oryza sativa* of an *Oryza sativa* NAC4 (SEQ ID NO: 52) transcription factor-encoding nucleic acid under the control of an RCc3 promoter (internal reference PRO0110) for root-specific expression.

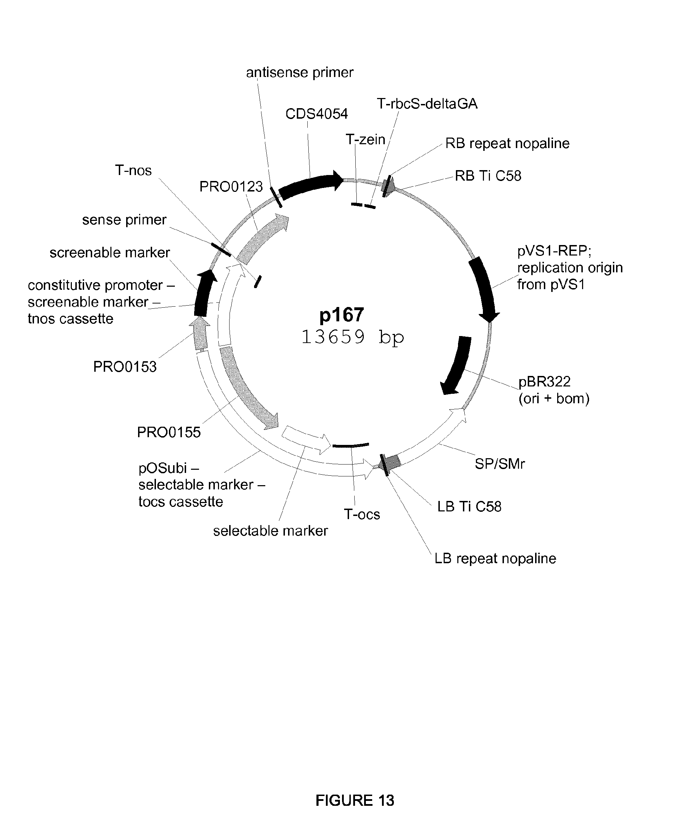

**Fig. 13** shows a binary vector p167, for increased expression in *Oryza sativa* of an *Oryza sativa* NAC transcription factor-encoding nucleic acid under the control of a protochlorophylid reductase promoter (internal reference PR00123).

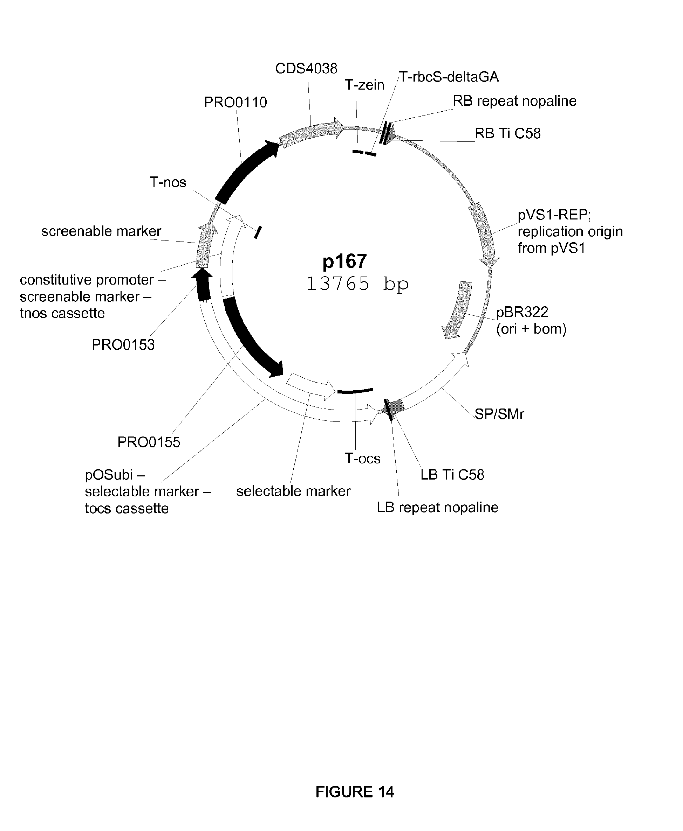

**Fig. 14** shows a binary vector p167, for increased expression in *Oryza sativa* of an *Oryza sativa* NAC6 (SEQ ID NO: 54) transcription factor-encoding nucleic acid under the control of an RCc3 promoter (internal reference PRO0110) for root-specific expression.

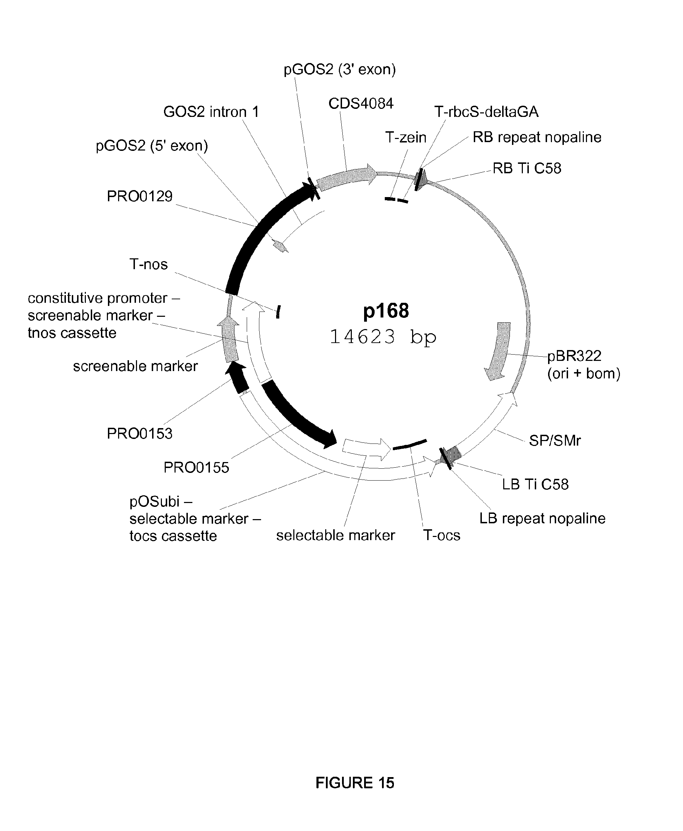

**Fig. 15** shows a binary vector p168, for increased expression in *Oryza sativa* of an *Oryza sativa* NAC7 (SEQ ID NO: 56) transcription factor-encoding nucleic acid under the control of a GOS2 promoter (internal reference PRO0129) for constitutive expression.

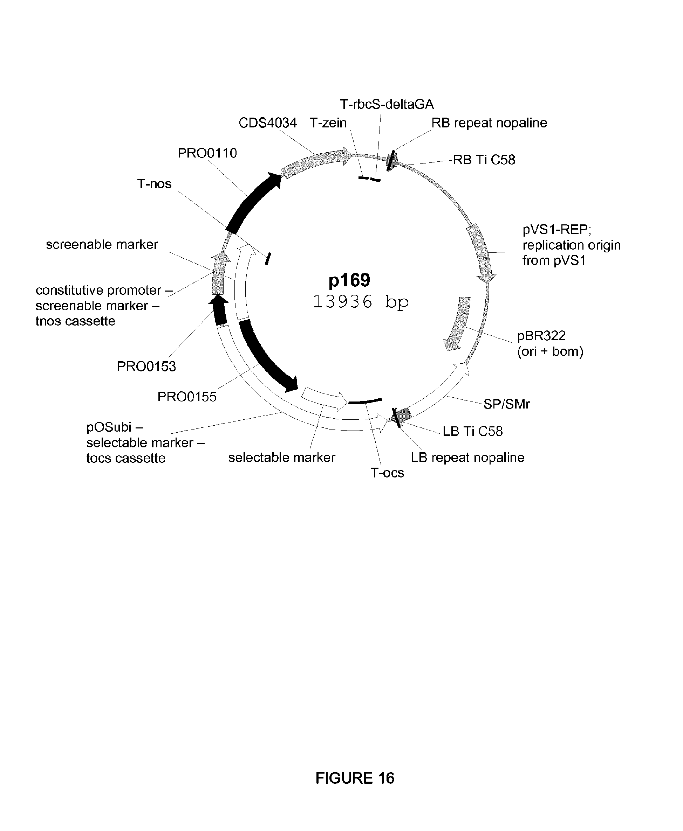

**Fig. 16** shows a binary vector p169, for increased expression in *Oryza sativa* of an *Oryza sativa* NAC3 (SEQ ID NO: 58) transcription factor-encoding nucleic acid under the control of an RCc3 promoter (internal reference PRO0110) for root-specific expression.

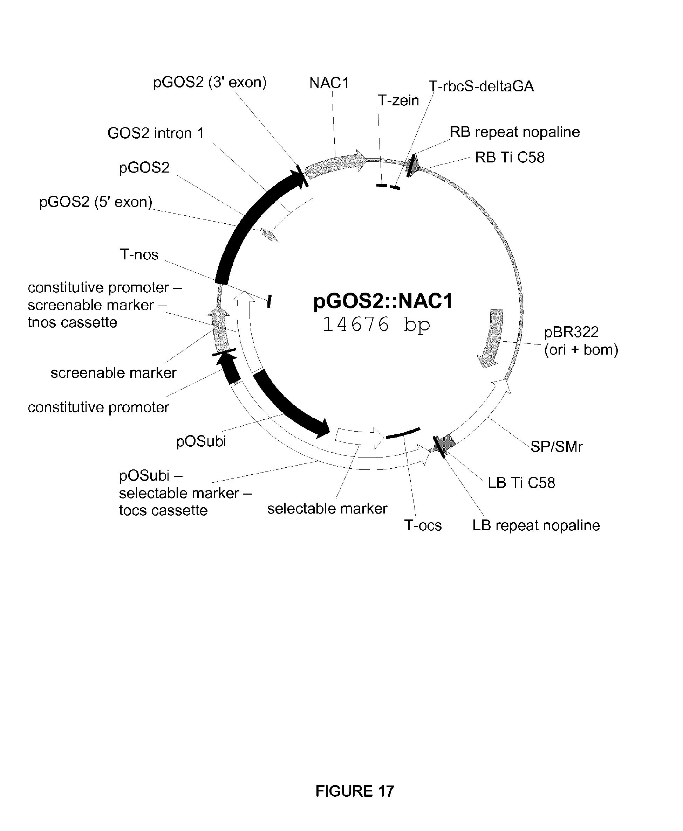

**Fig. 17** shows a binary vector pGOS2::NAC1, for increased expression in *Oryza sativa* of an *Oryza sativa* NAC1 (SEQ ID NO: 50) transcription factor-encoding nucleic acid under the control of a GOS2 promoter for constitutive expression.

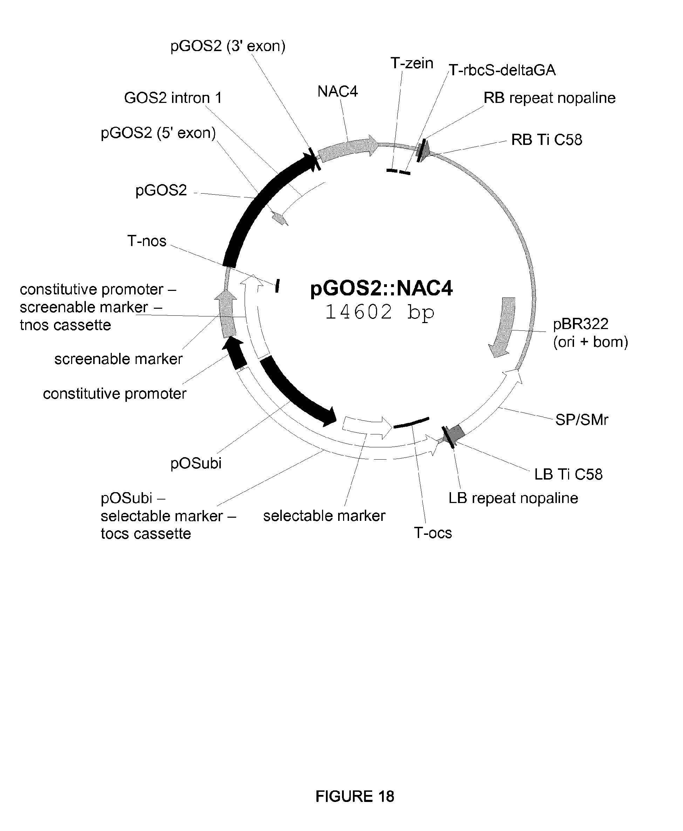

**Fig. 18** shows a binary vector pGOS2::NAC4, for increased expression in *Oryza sativa* of an *Oryza sativa* NAC4 (SEQ ID NO: 52) transcription factor-encoding nucleic acid under the control of a GOS2 promoter for constitutive expression.

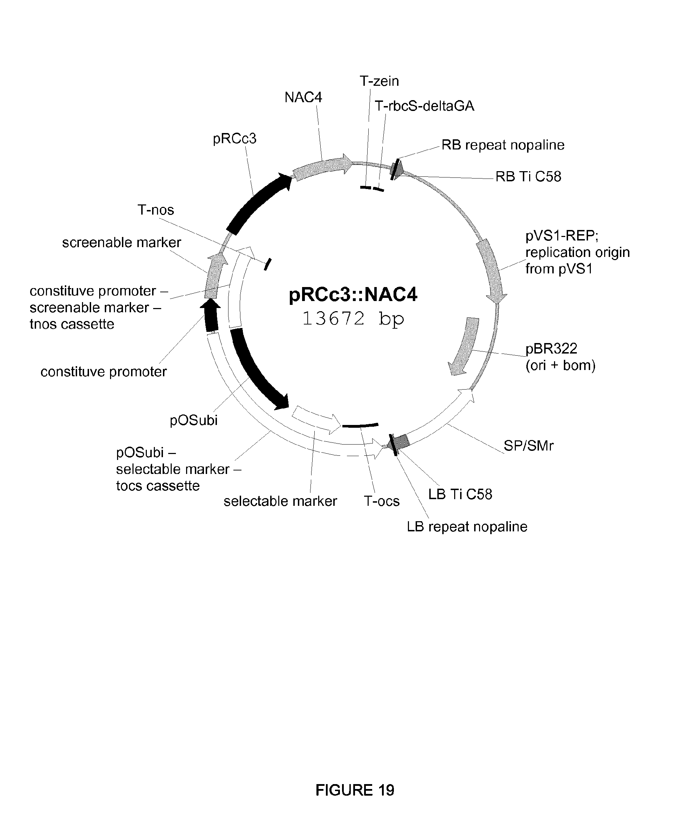

**Fig. 19** shows a binary vector pRCc3::NAC4, for increased expression in *Oryza sativa* of an *Oryza sativa* NAC4 (SEQ ID NO: 52) transcription factor-encoding nucleic acid under the control of an RCc3 promoter for root-specific expression.

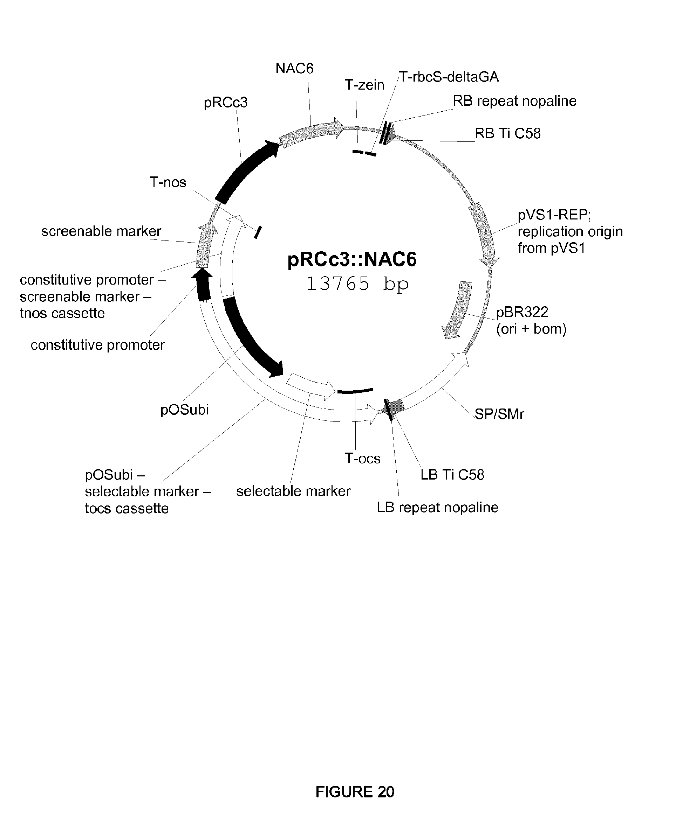

**Fig. 20** shows a binary vector pRCc3::NAC6, for increased expression in *Oryza sativa* of an *Oryza sativa* NAC6 (SEQ ID NO: 54) transcription factor-encoding nucleic acid under the control of an RCc3 promoter for root-specific expression.

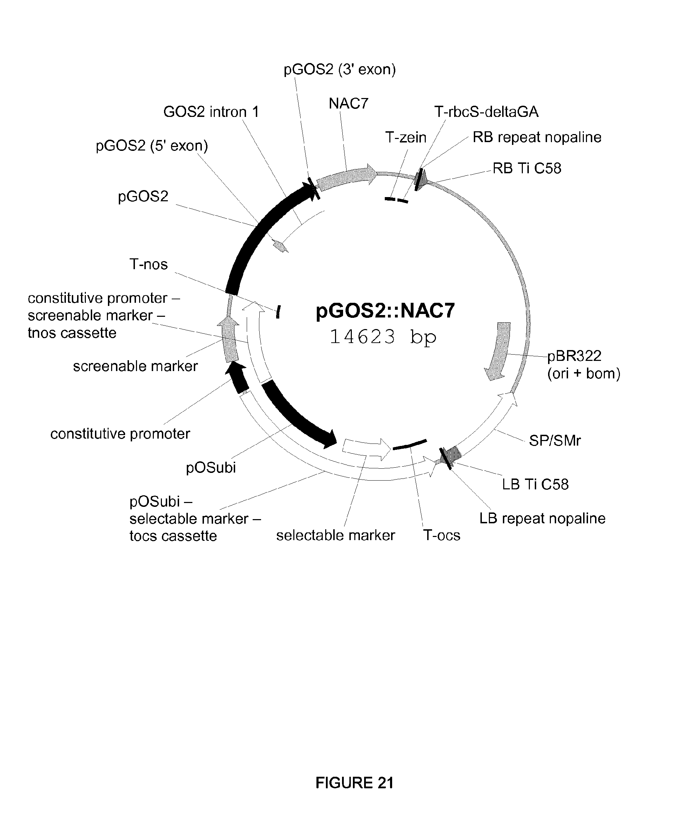

**Fig. 21** shows a binary vector pGOS2::NAC7, for increased expression in *Oryza sativa* of an *Oryza sativa* NAC7 (SEQ ID NO: 56) transcription factor-encoding nucleic acid under the control of a GOS2 promoter for constitutive expression.

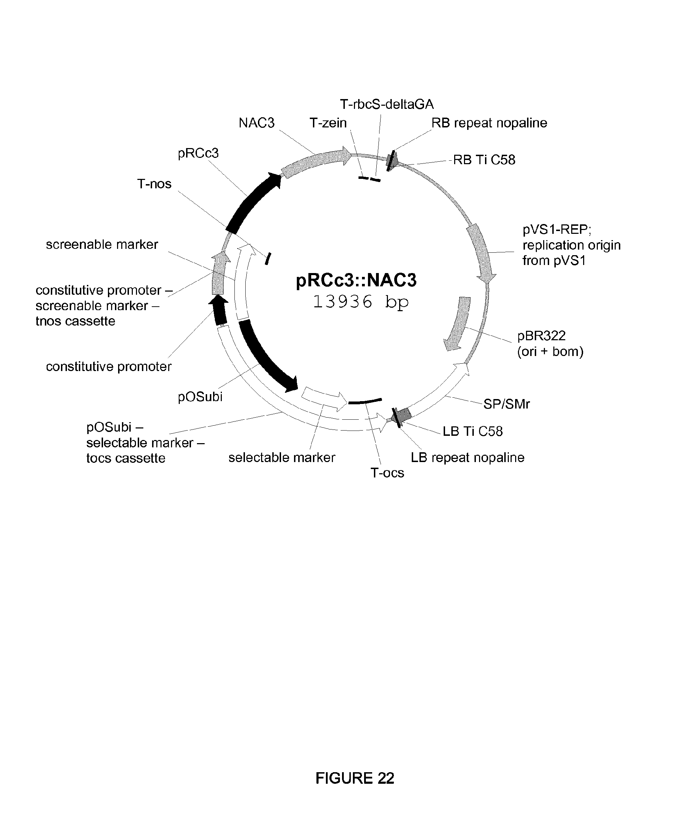

**Fig. 22** shows a binary vector pRCc3::NAC3, for increased expression in *Oryza sativa* of an *Oryza sativa* NAC3 (SEQ ID NO: 58) transcription factor-encoding nucleic acid under the control of an RCc3 promoter for root-specific expression.

**Fig. 23** shows the binary vector for increased expression in *Oryza sativa* of a rice AP2-2 protein-encoding nucleic acid under the control of a GOS2 promoter.

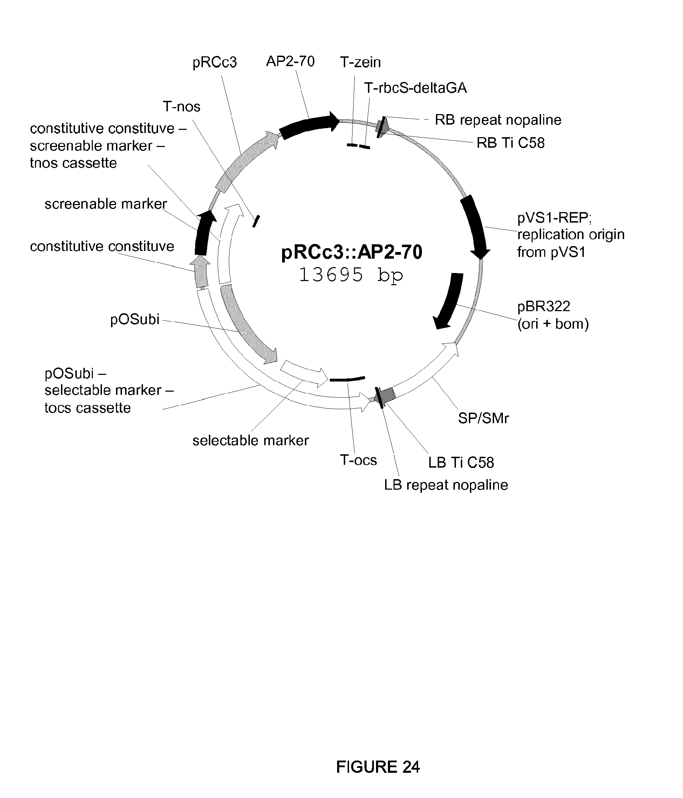

**Fig. 24** shows the binary vector for increased expression in *Oryza sativa* of an *Oryza sativa* AP2-70-like protein-encoding nucleic acid under the control of a rice RCC3 promoter (pRCC3).

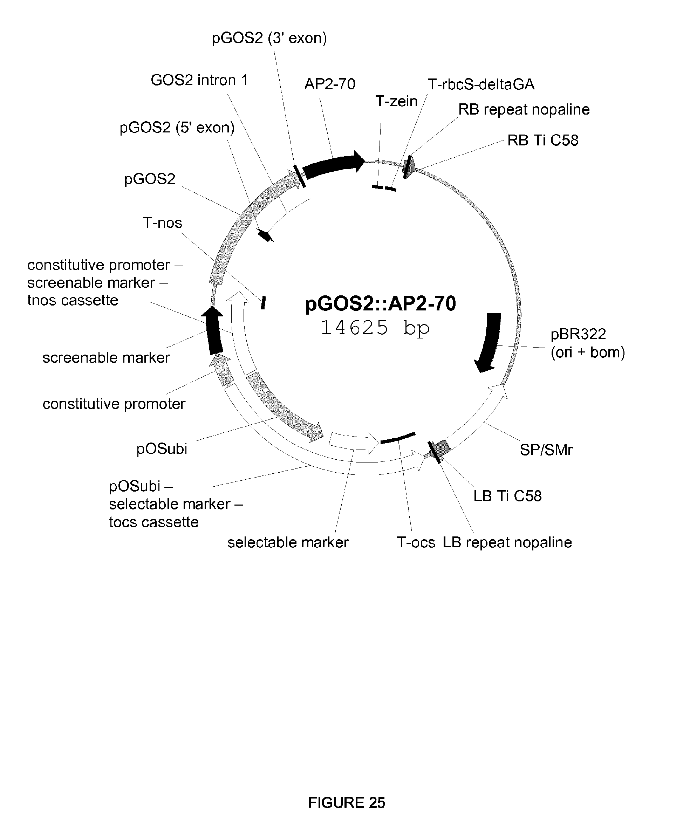

**Fig. 25** shows the binary vector for increased expression in *Oryza sativa* of an *Oryza sativa* AP2-70-like protein-encoding nucleic acid under the control of a rice GOS2 promoter (pGOS2).

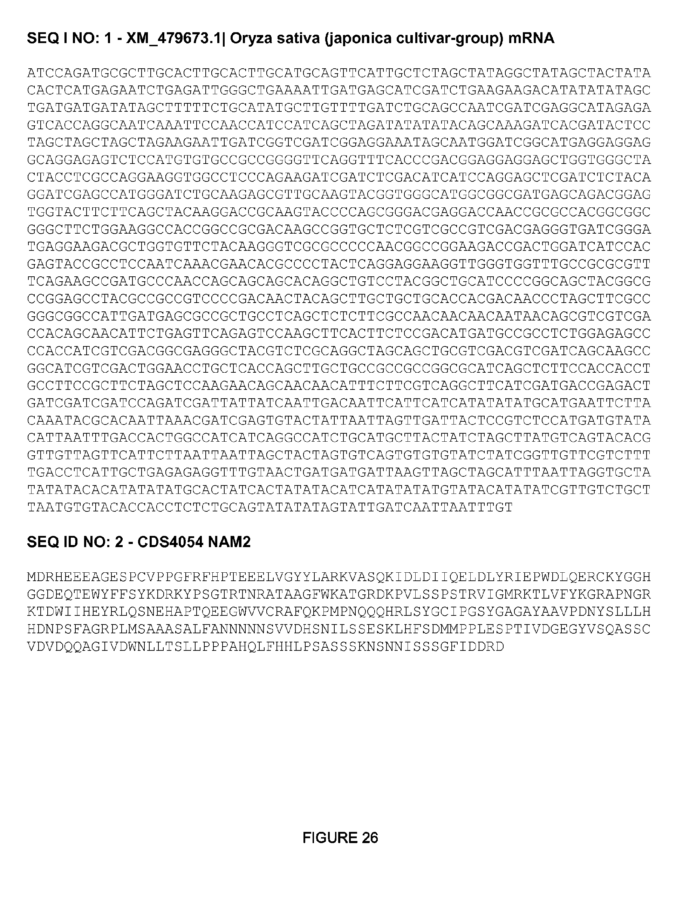

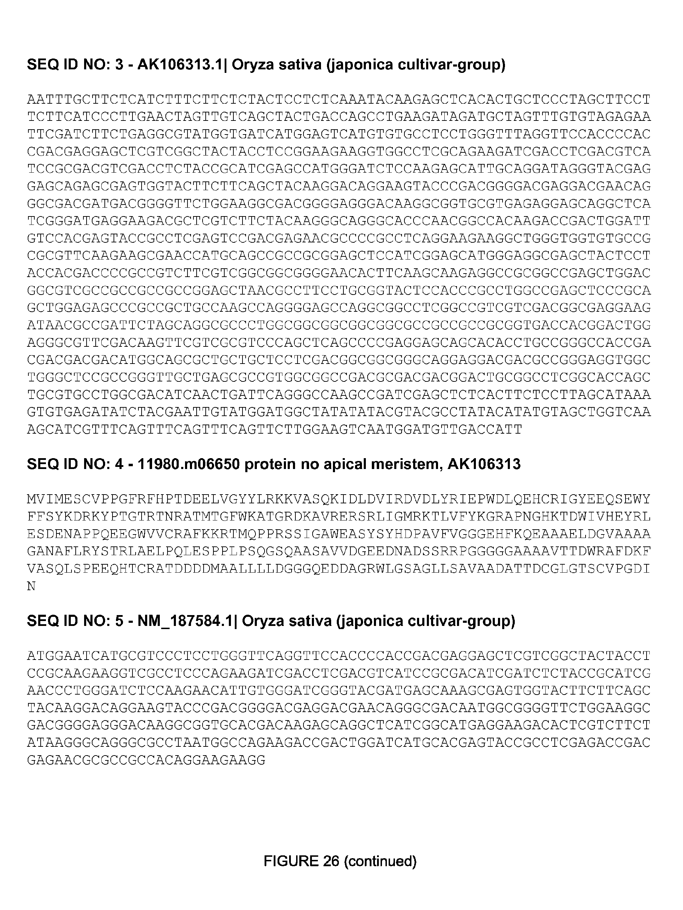

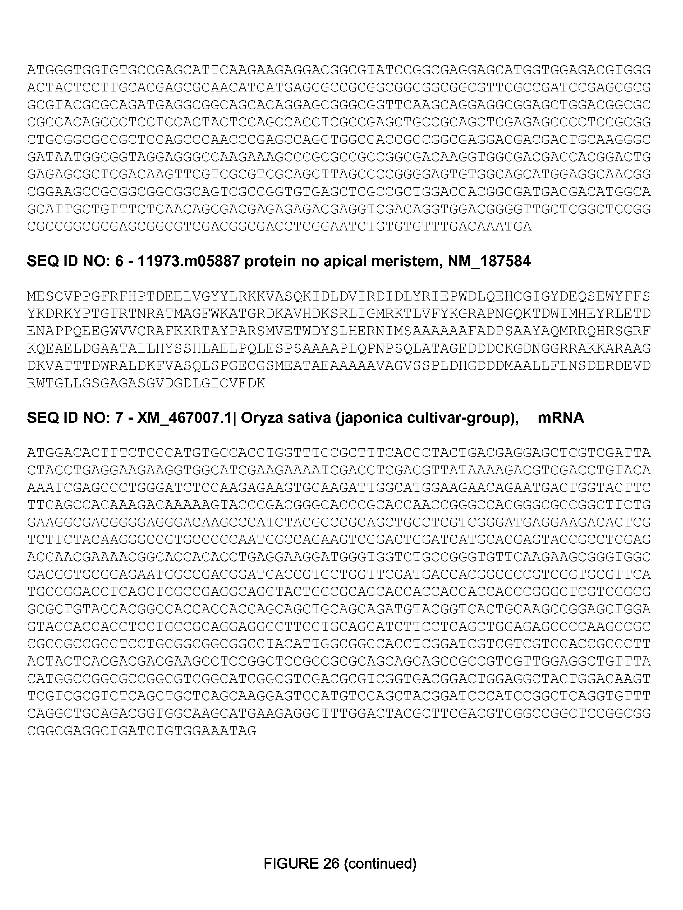

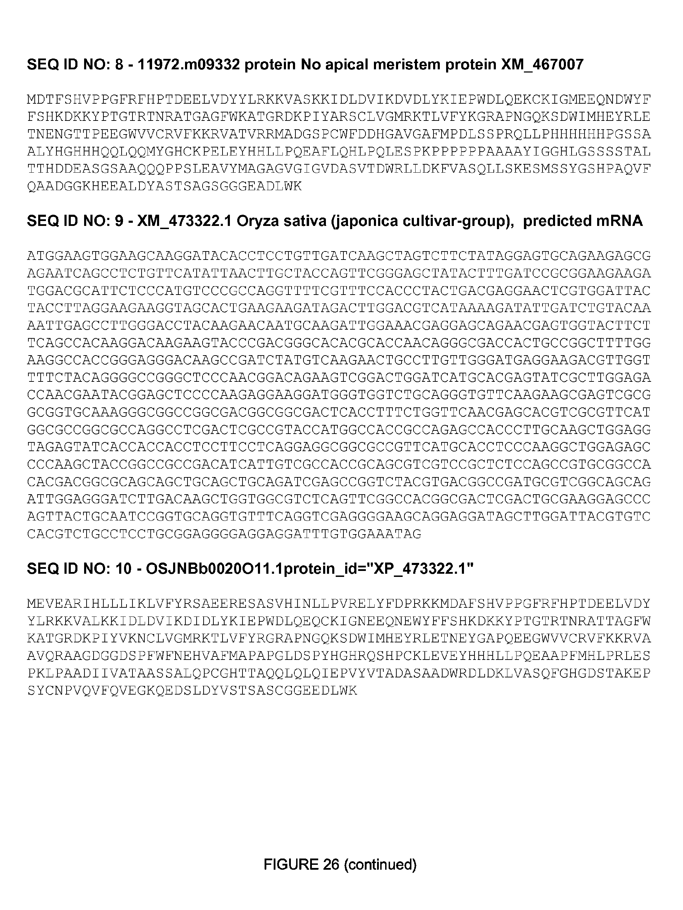

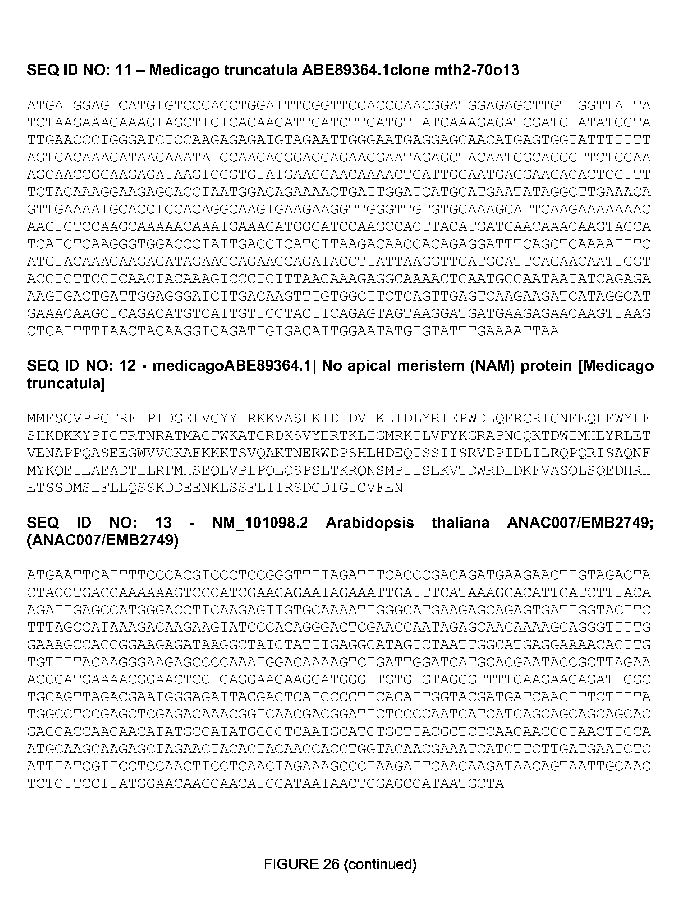

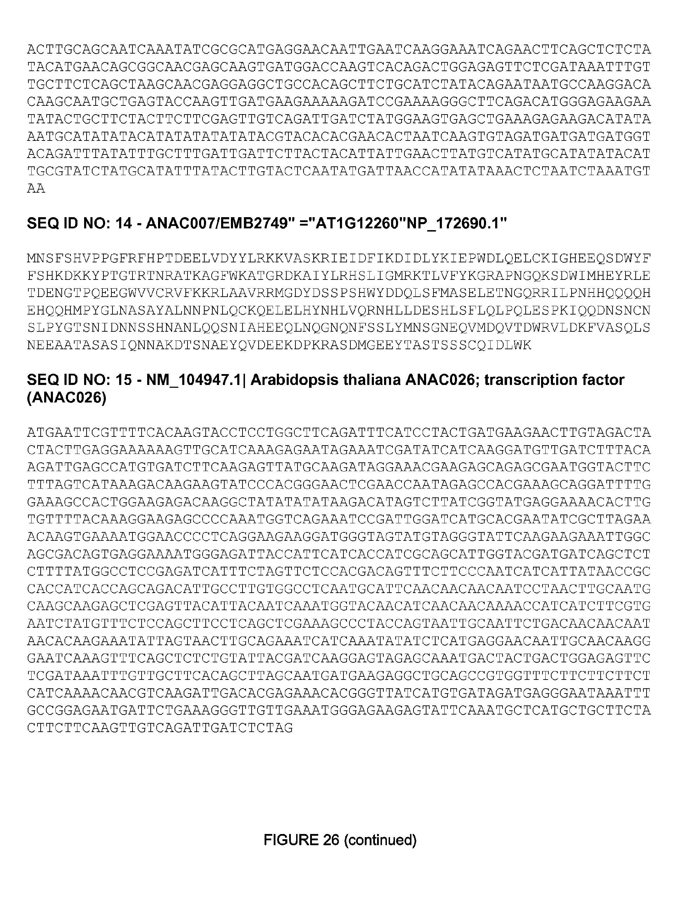

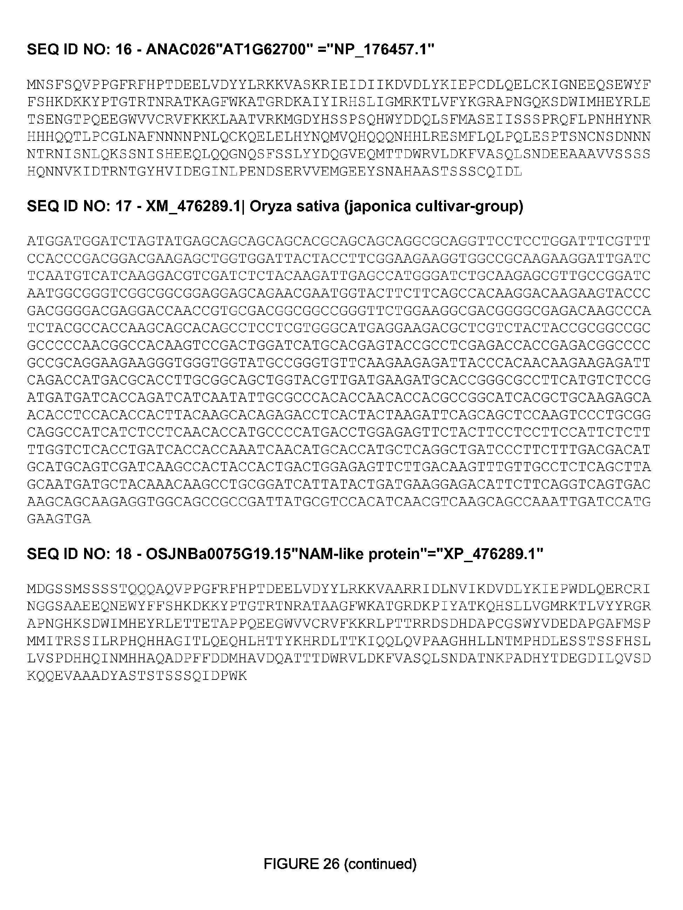

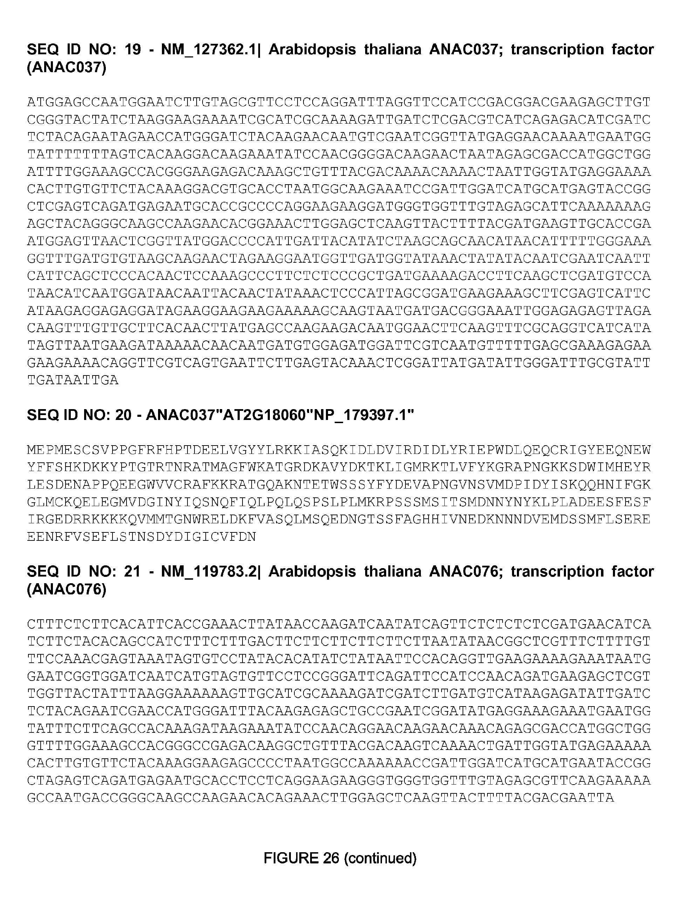

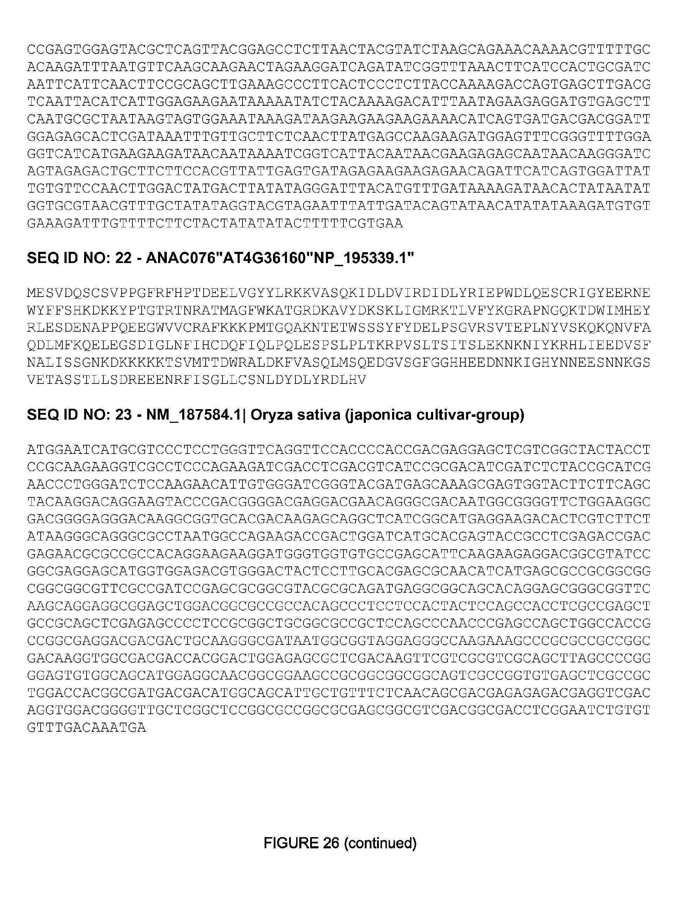

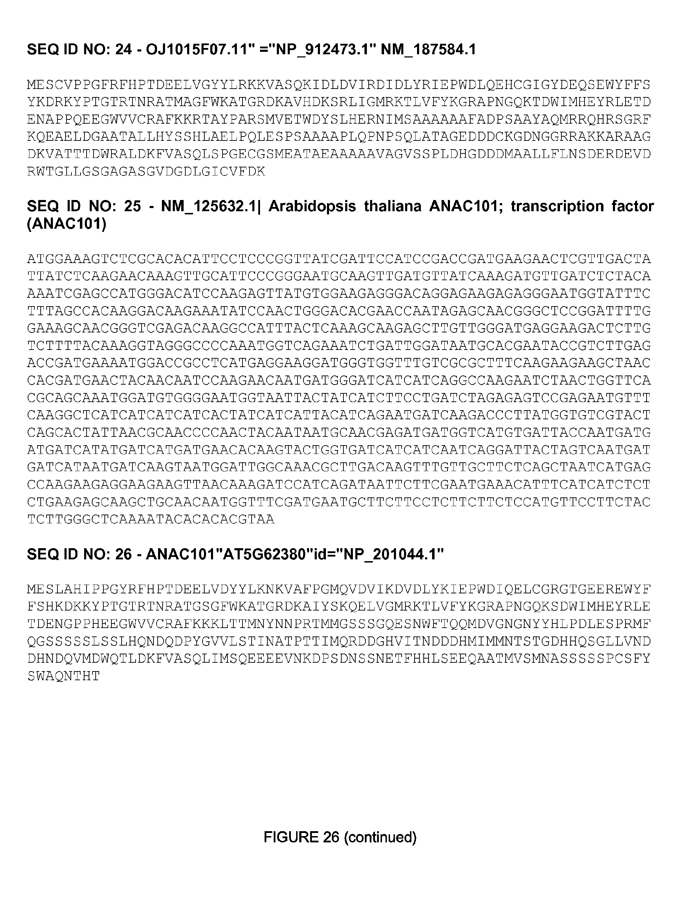

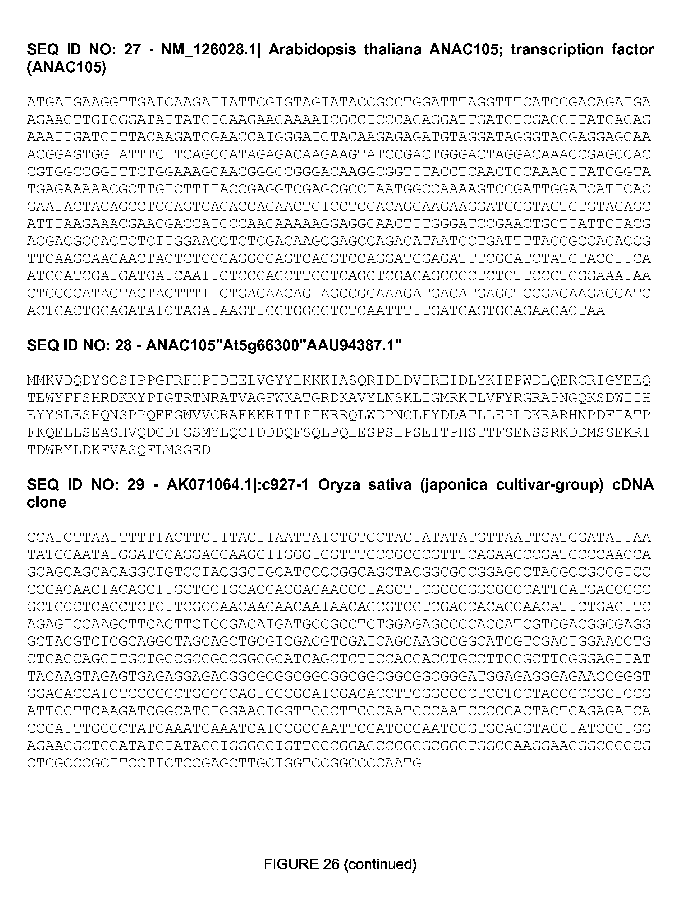

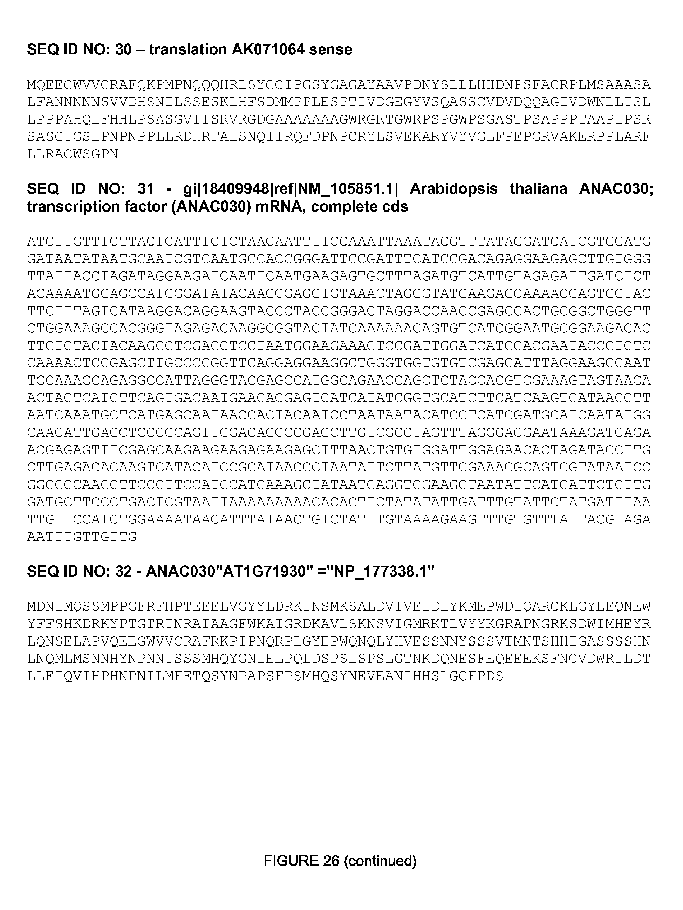

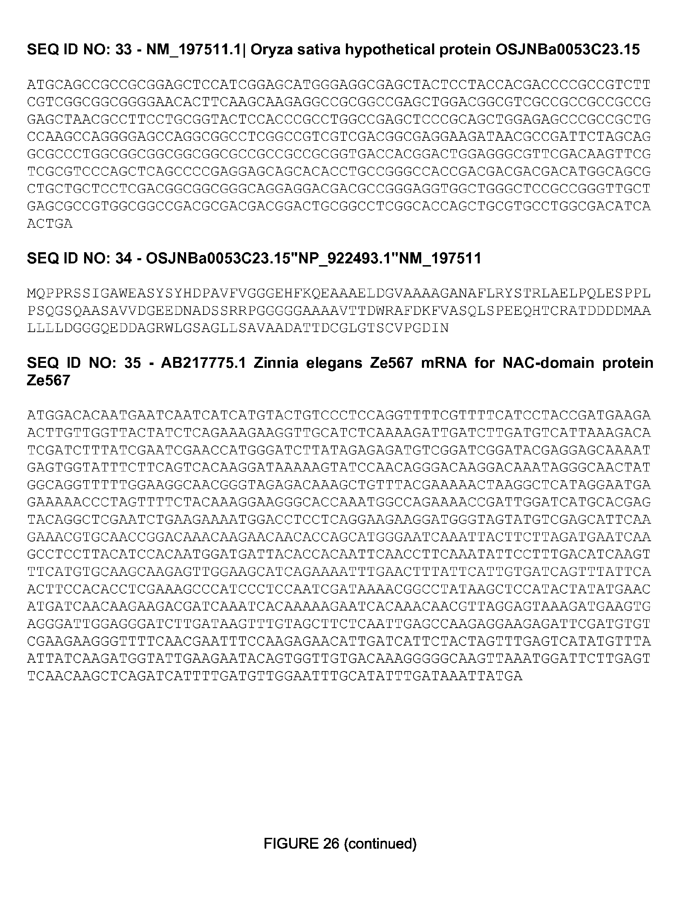

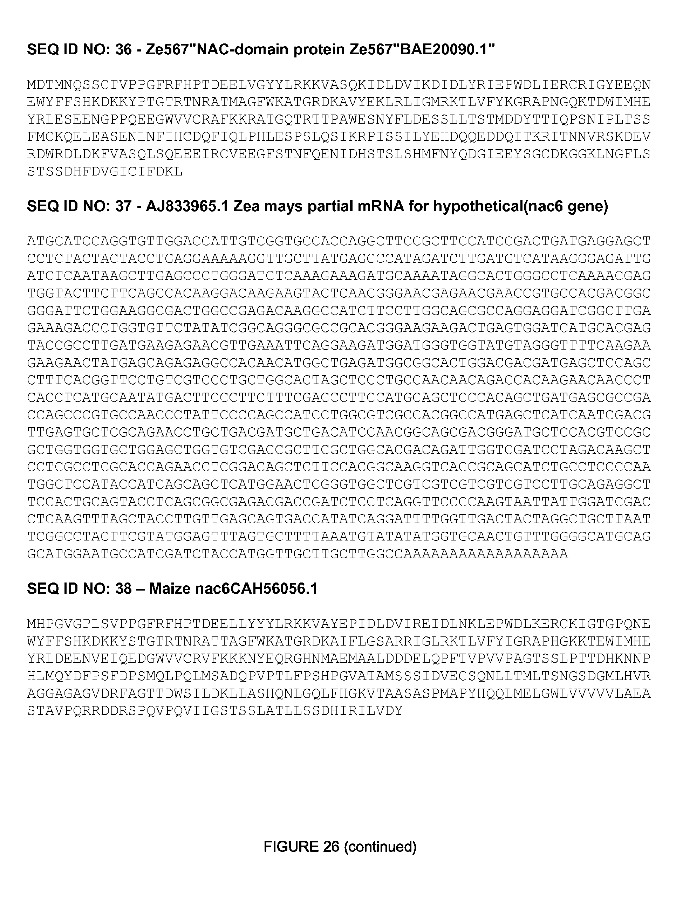

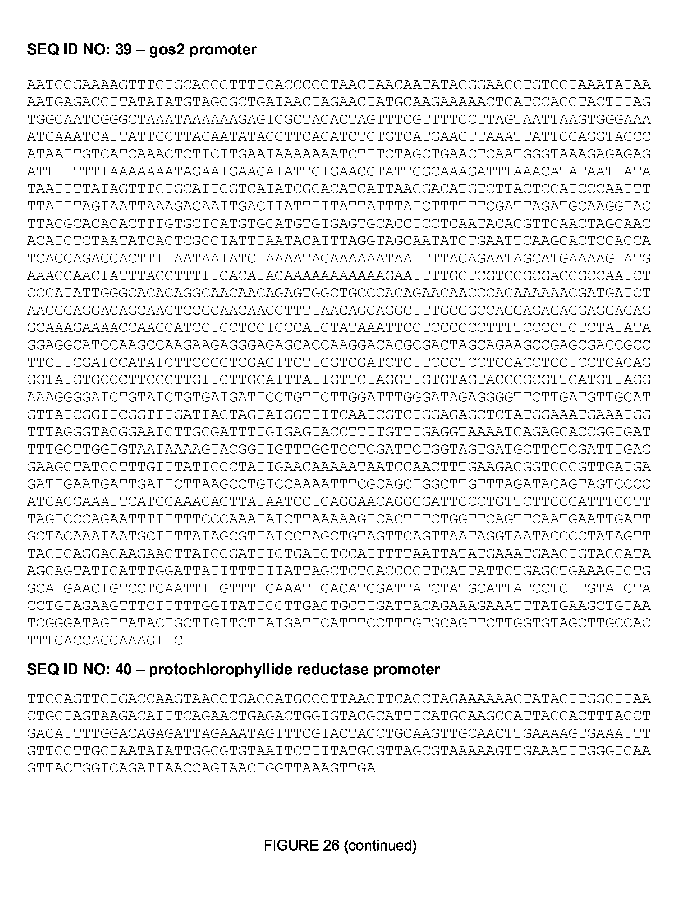

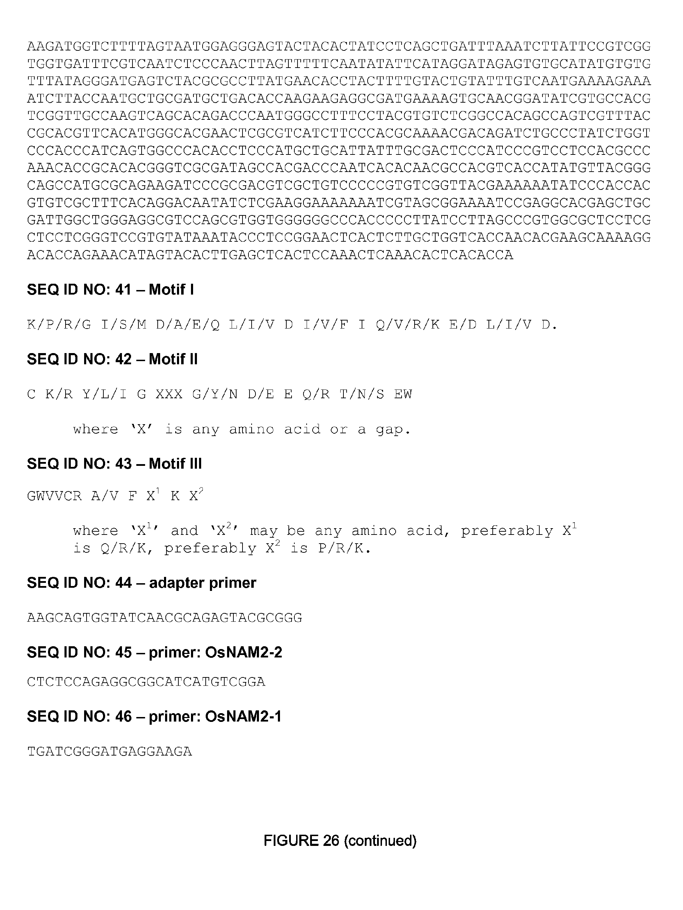

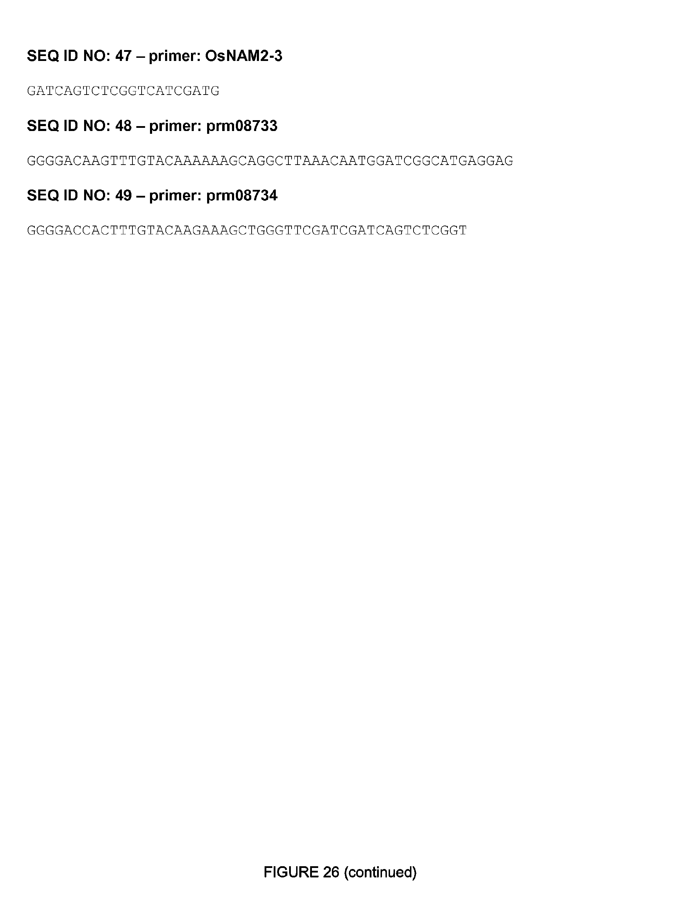

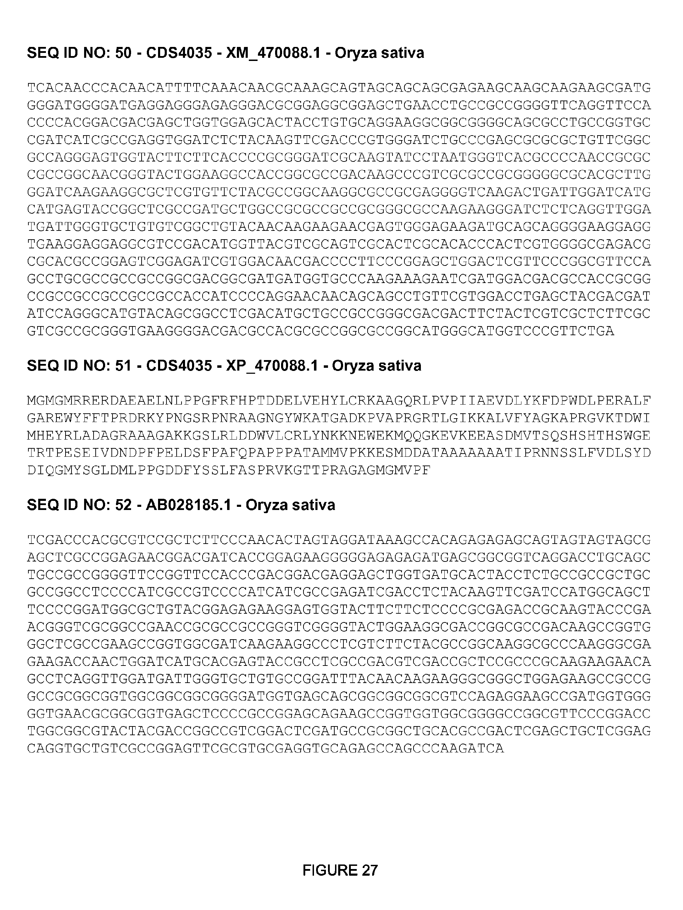

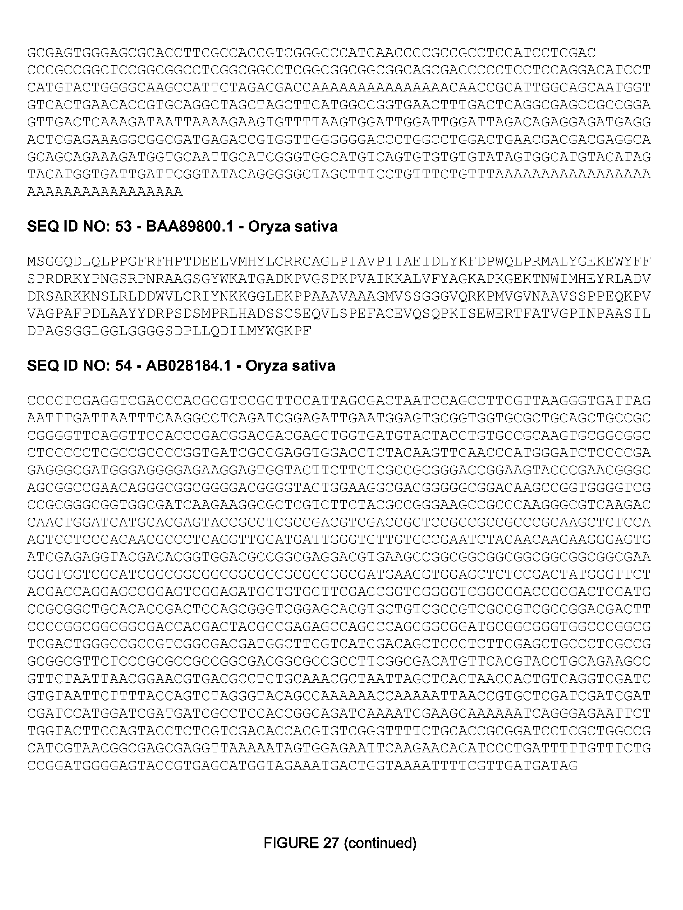

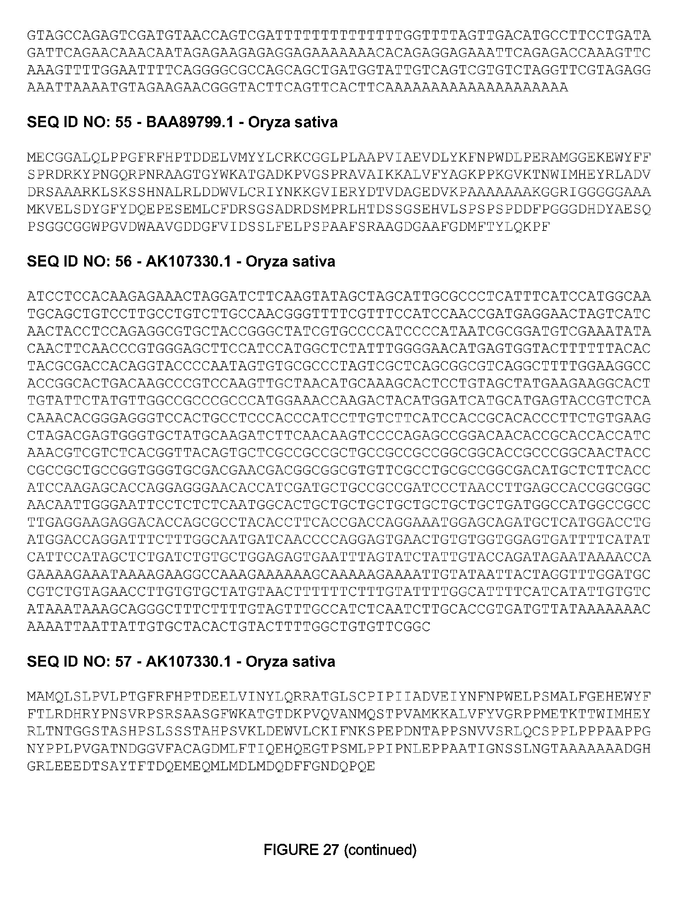

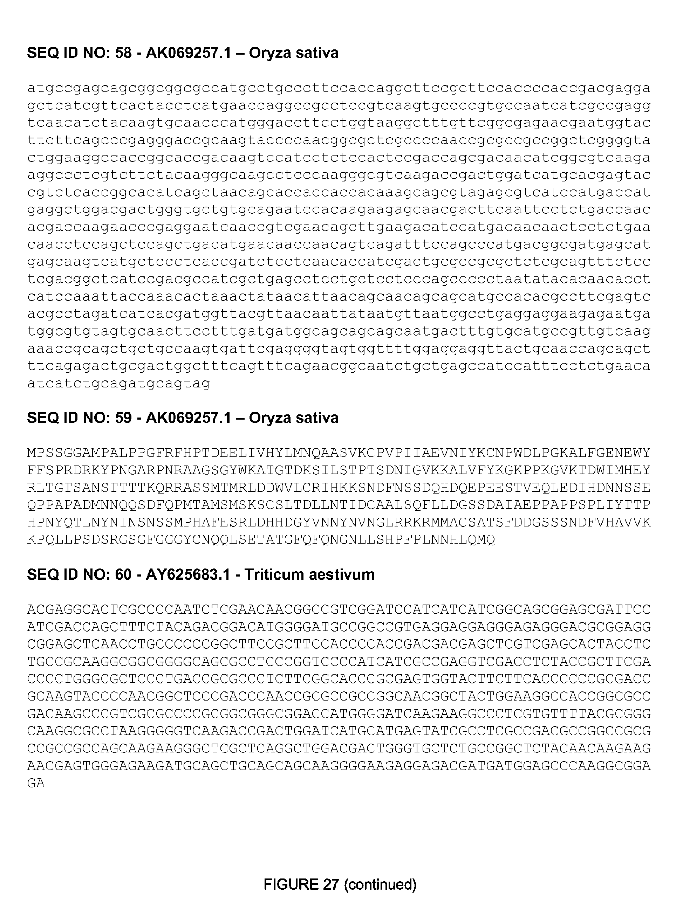

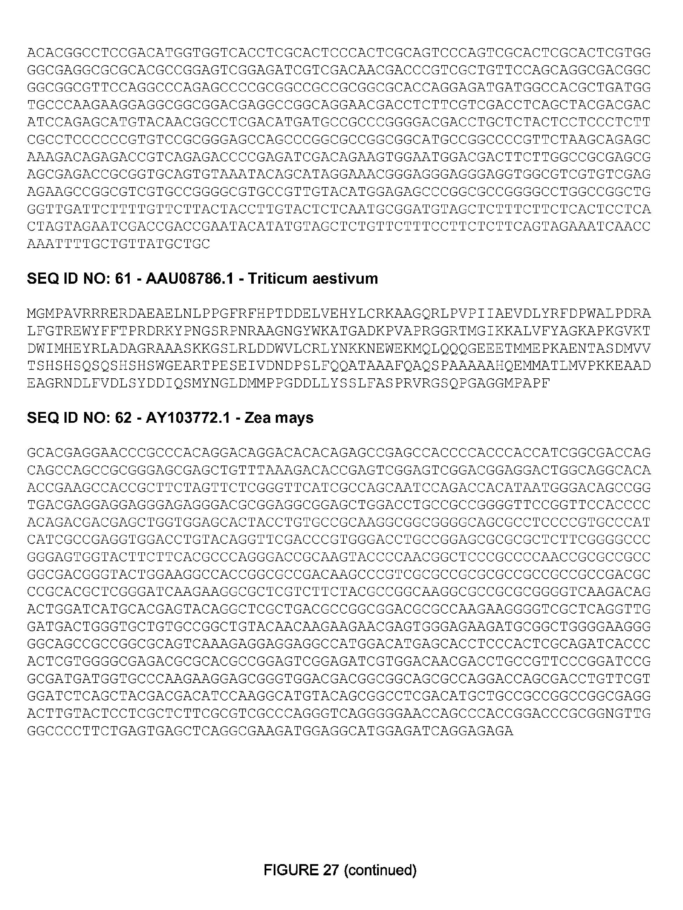

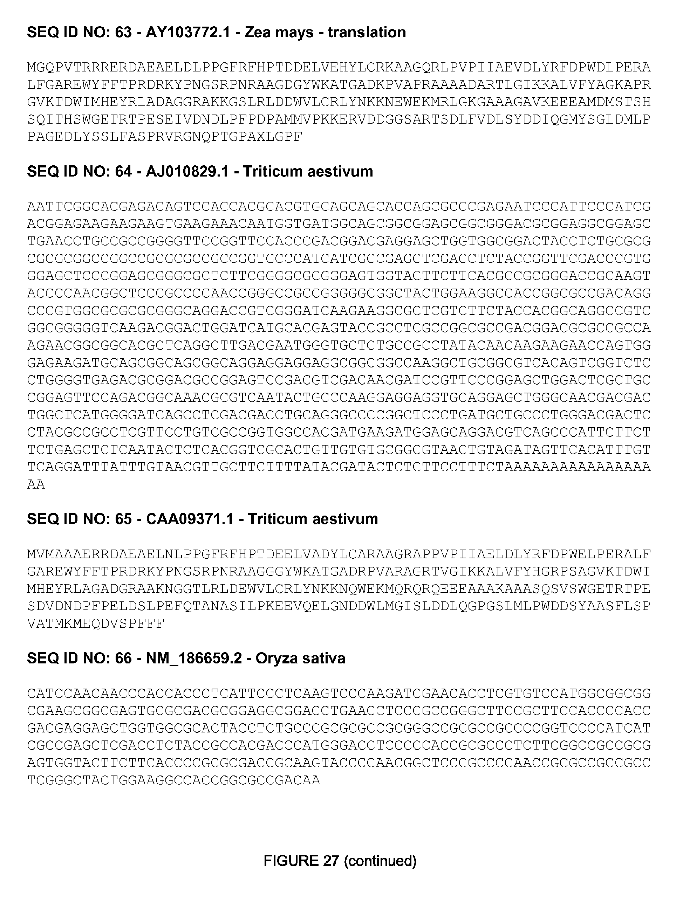

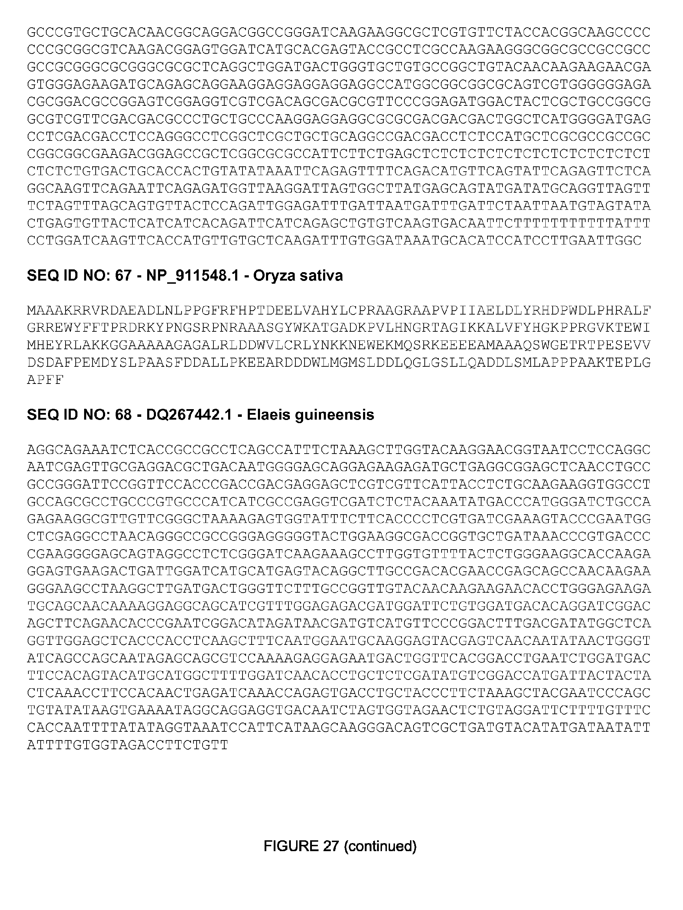

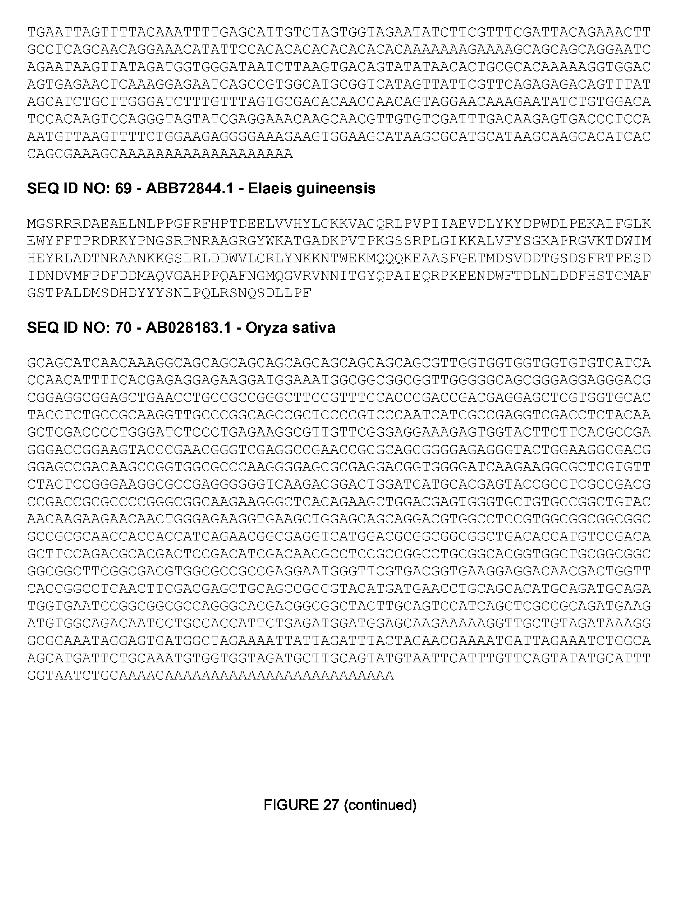

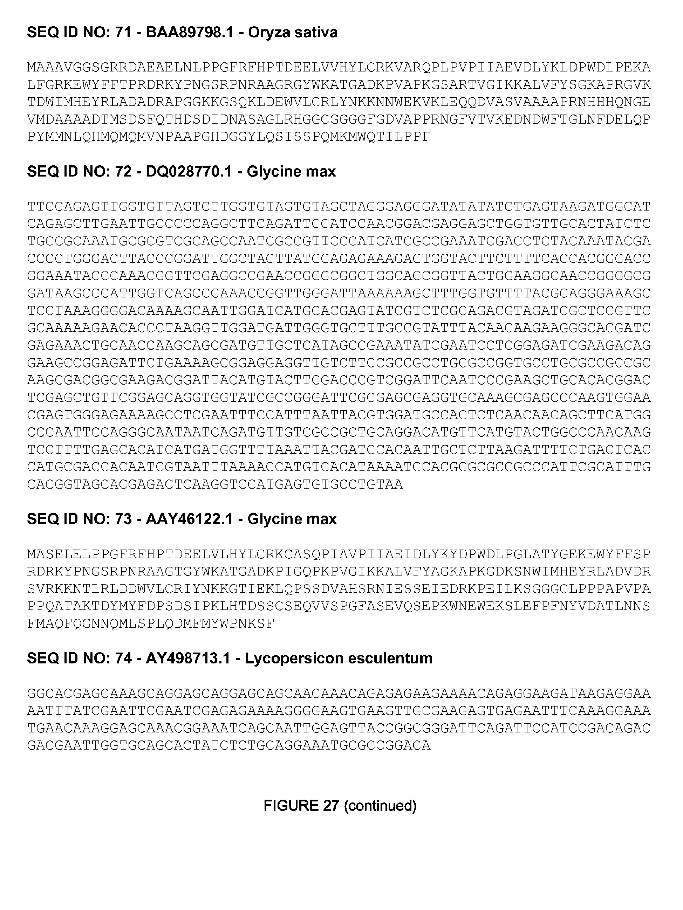

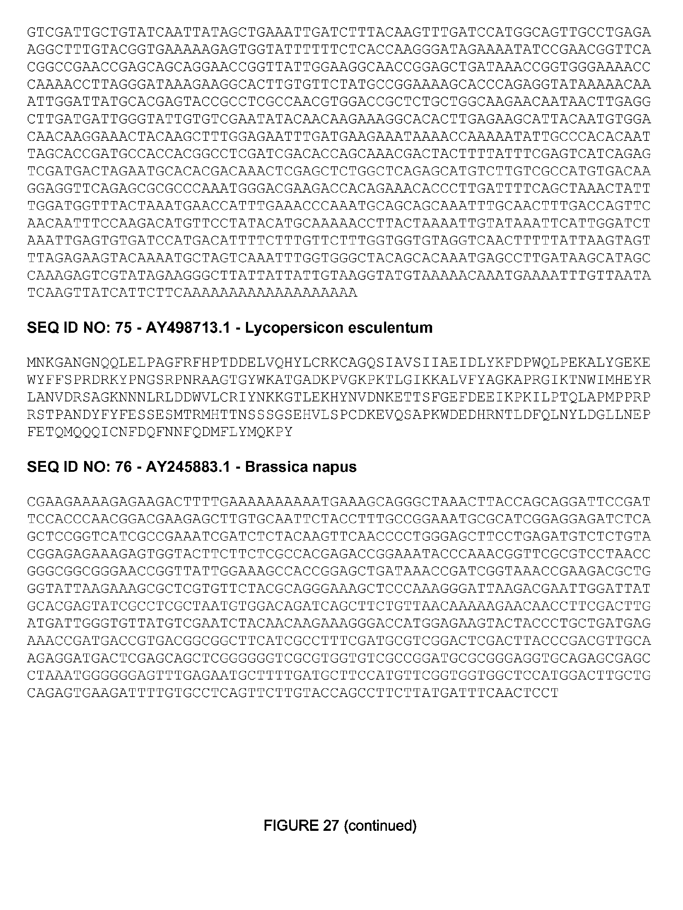

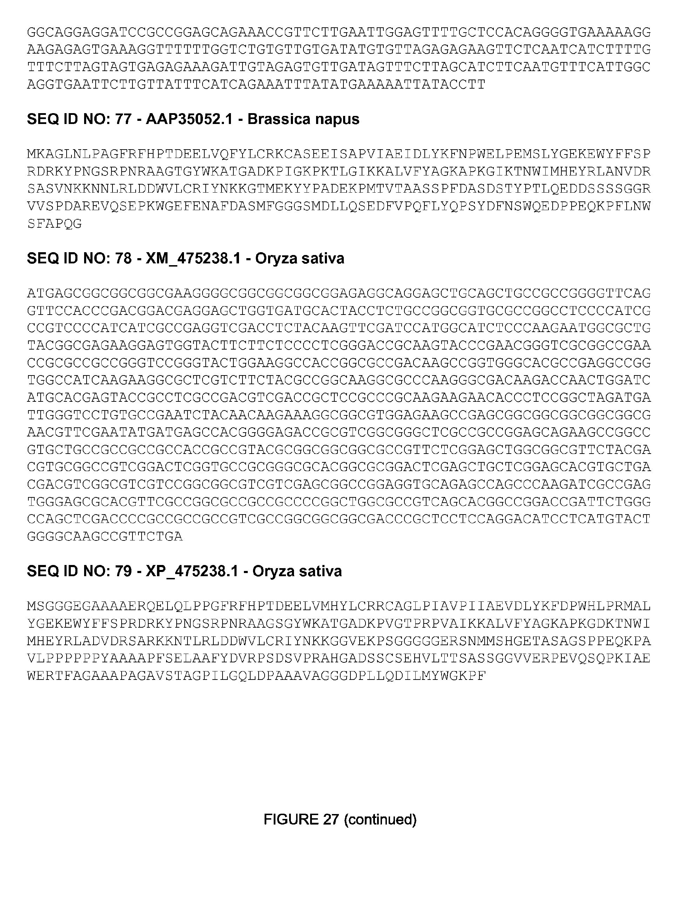

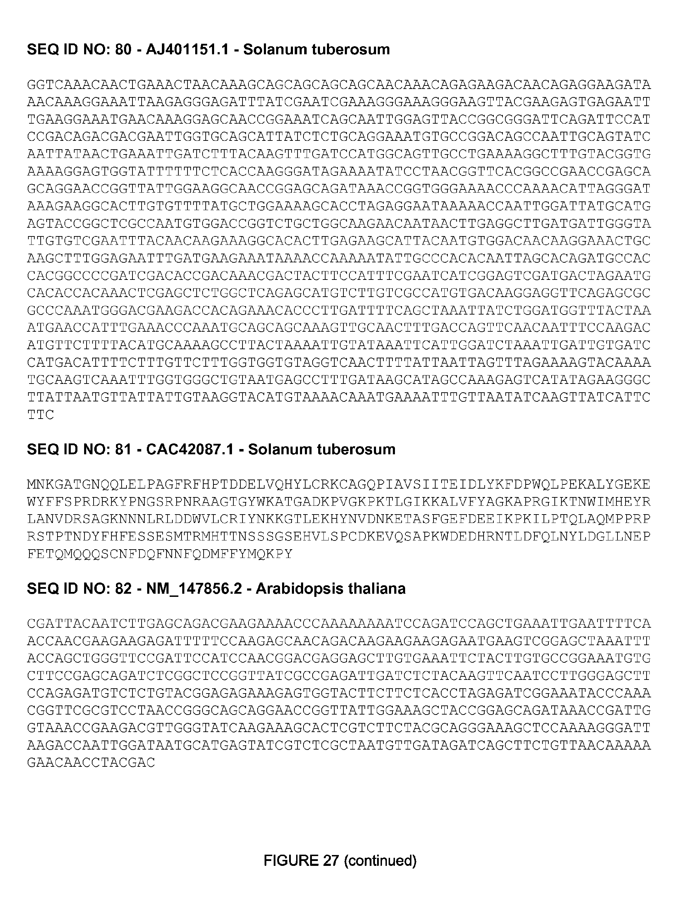

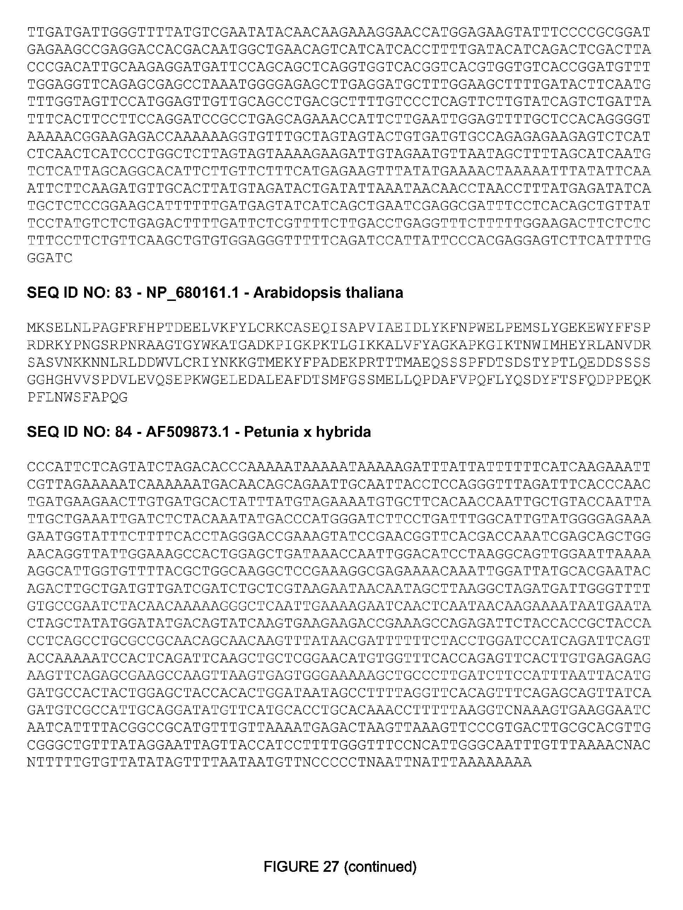

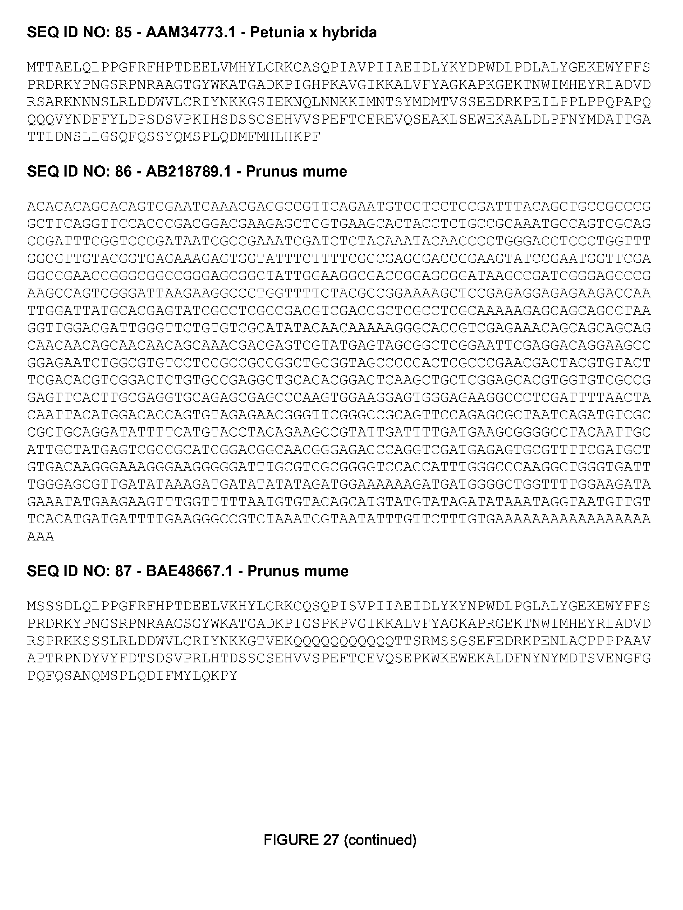

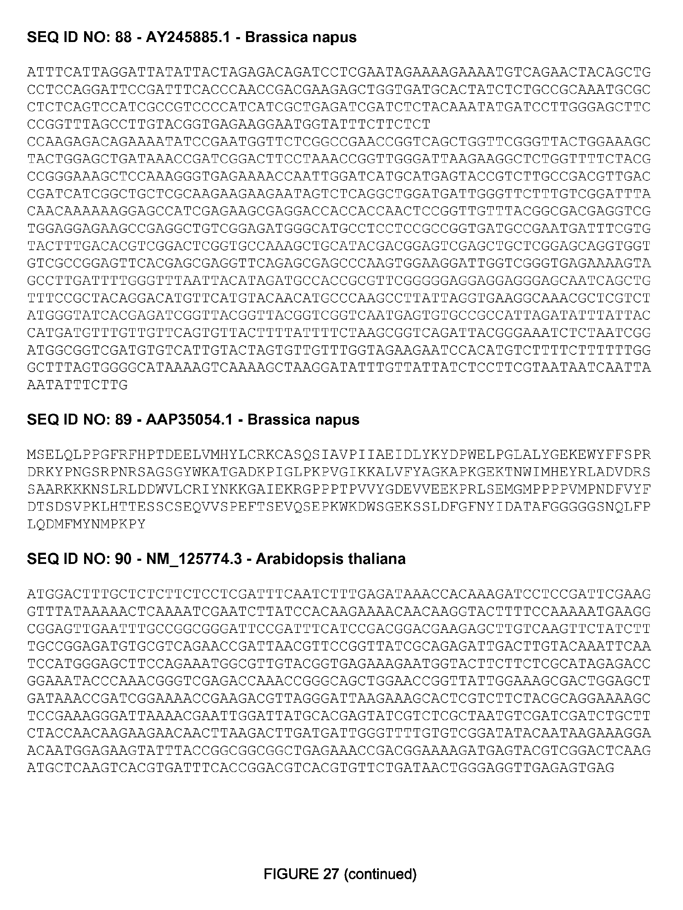

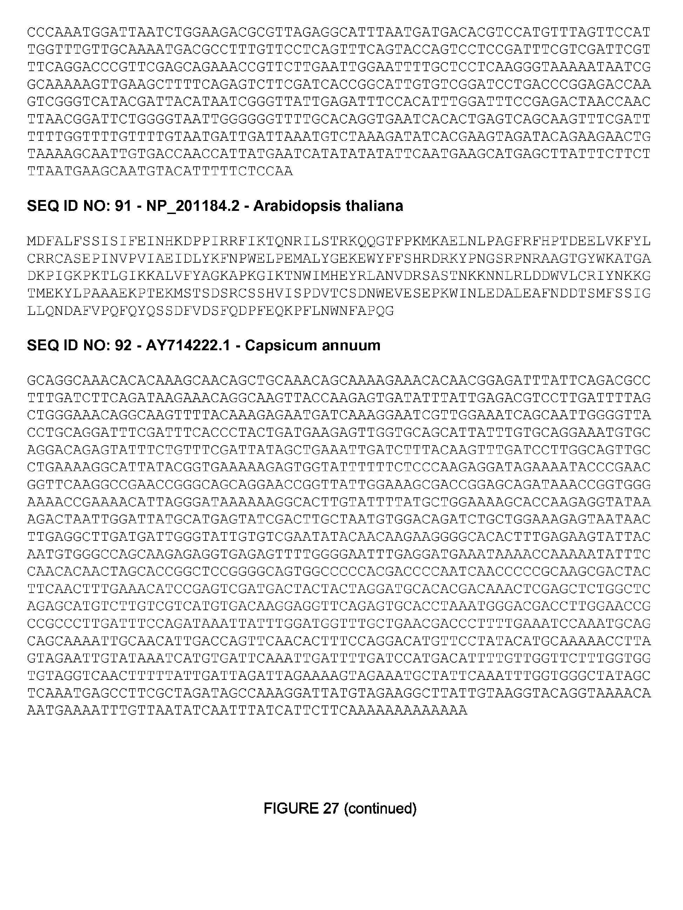

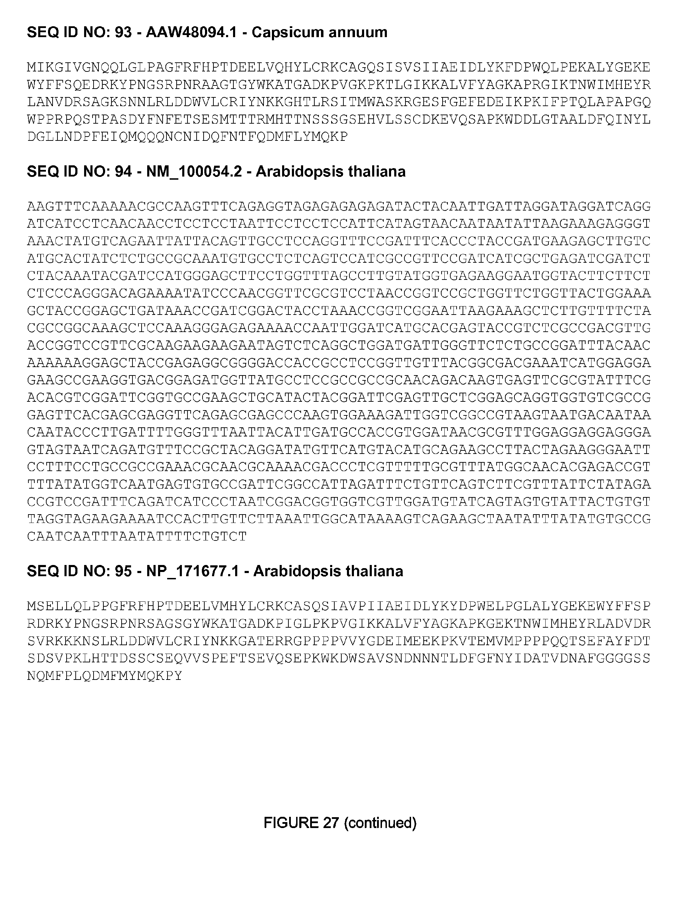

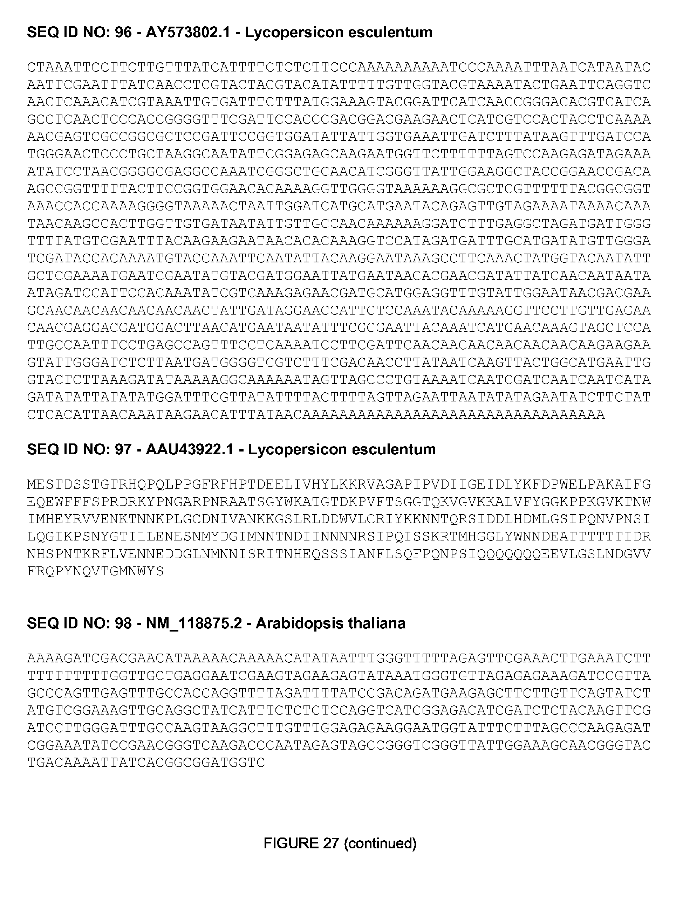

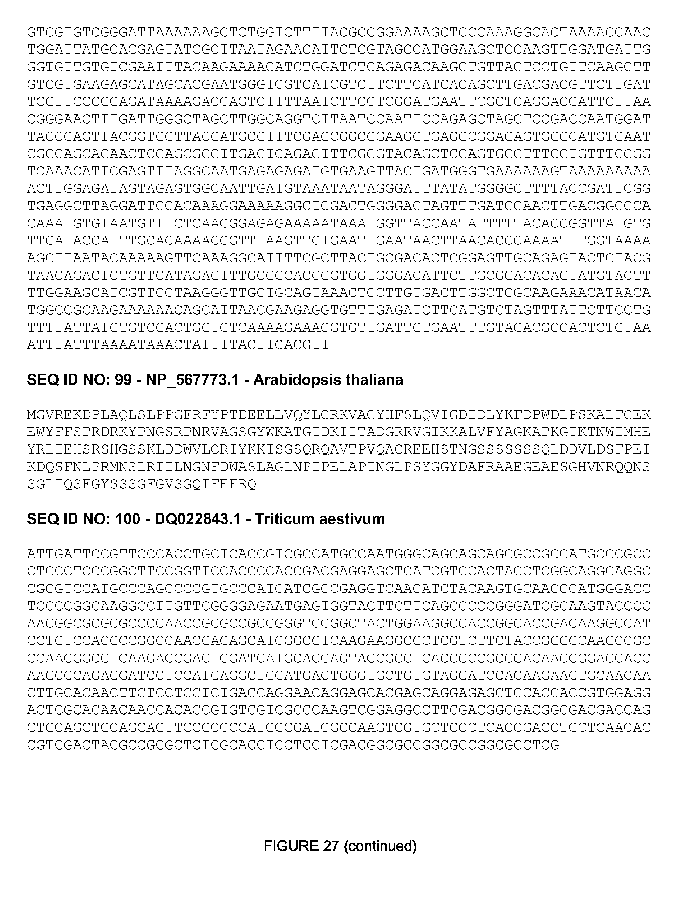

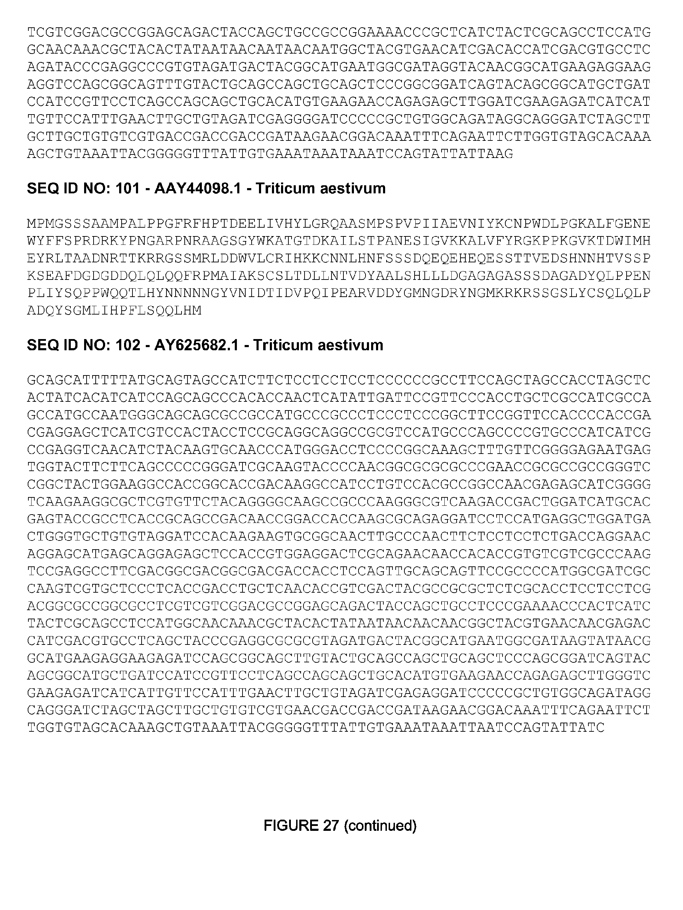

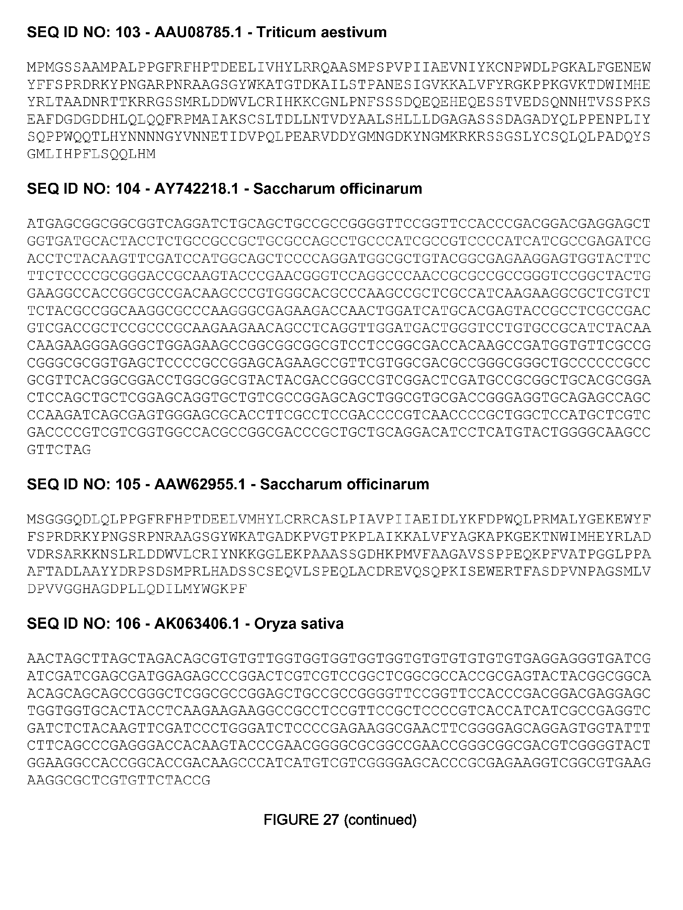

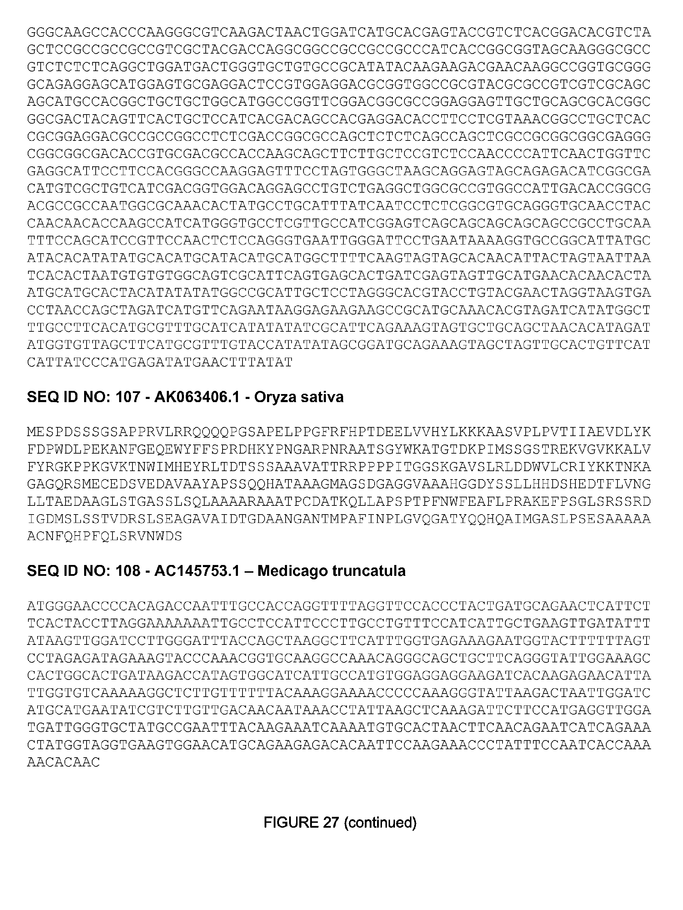

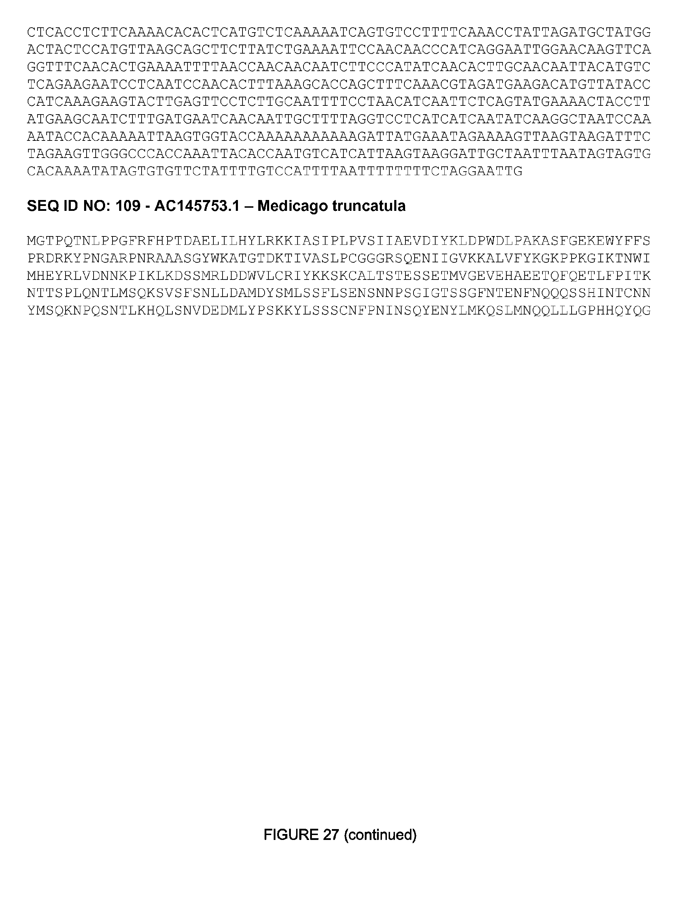

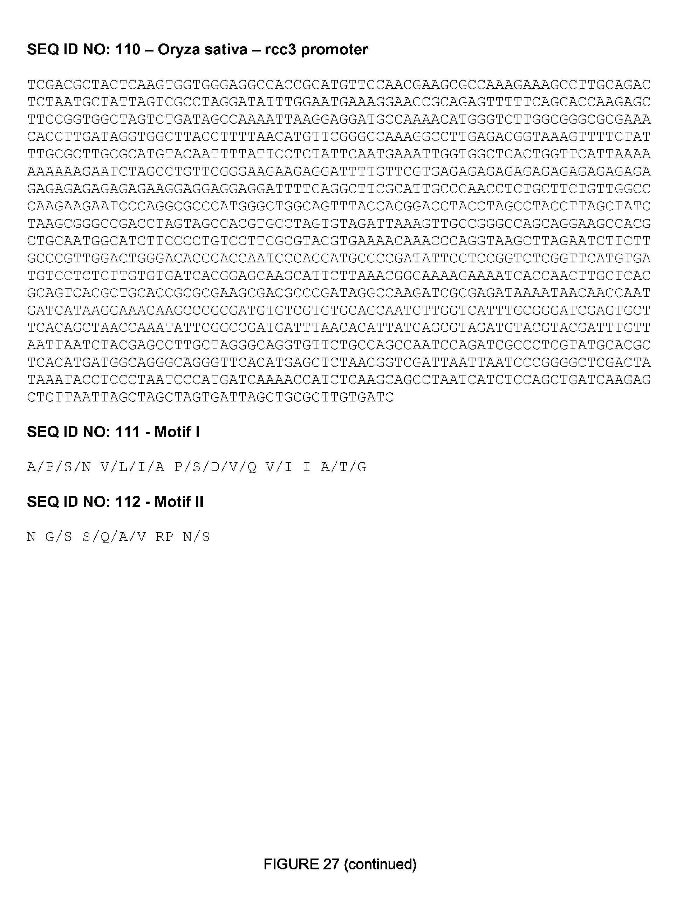

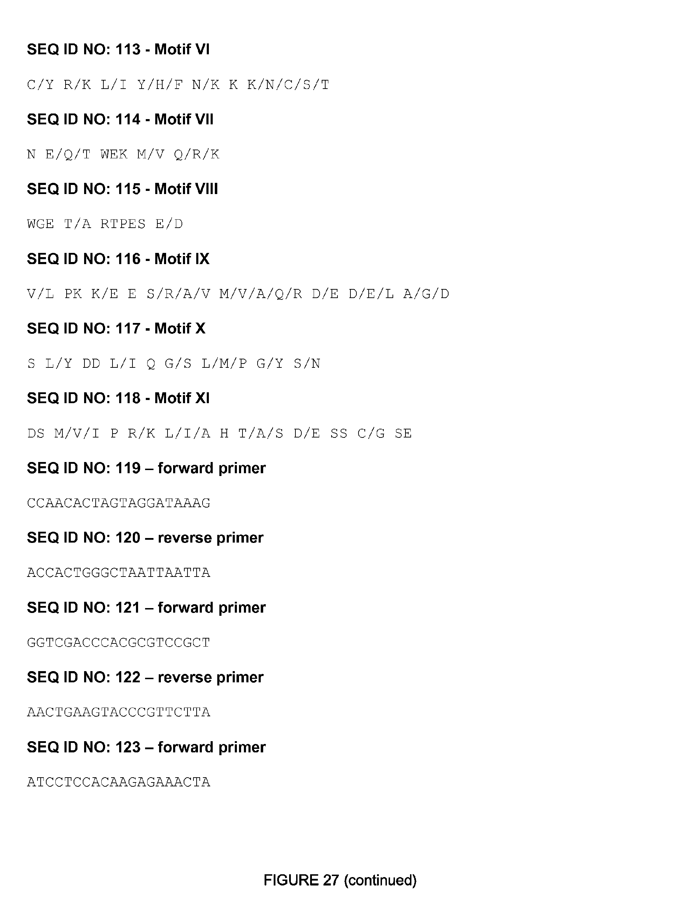

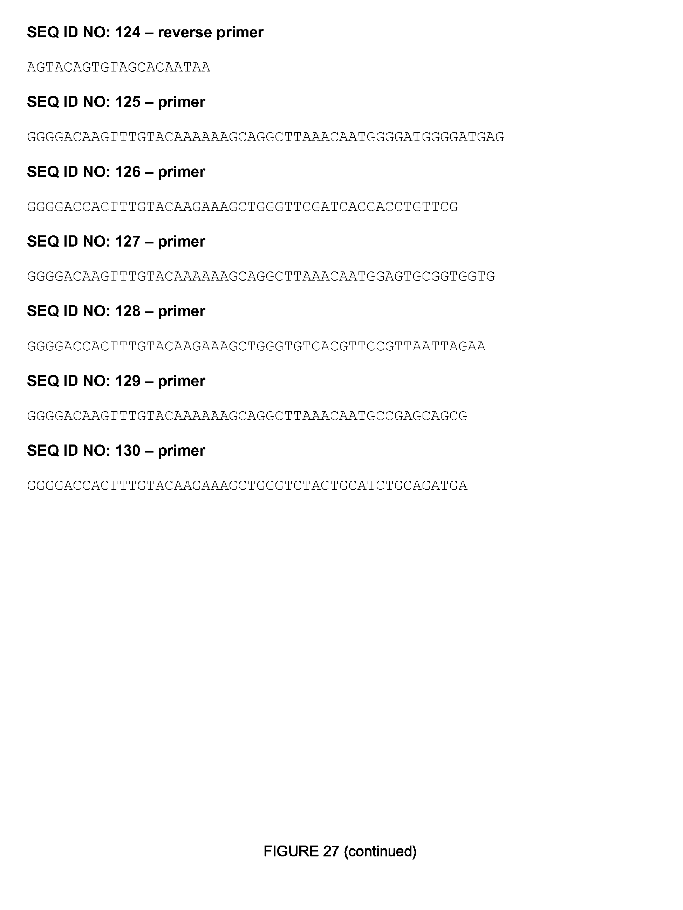

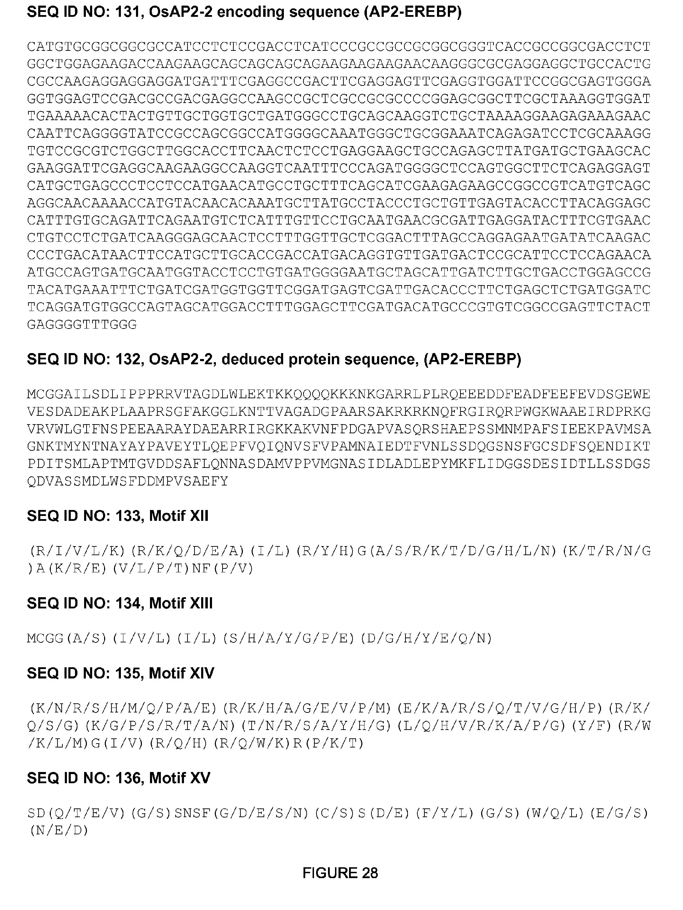

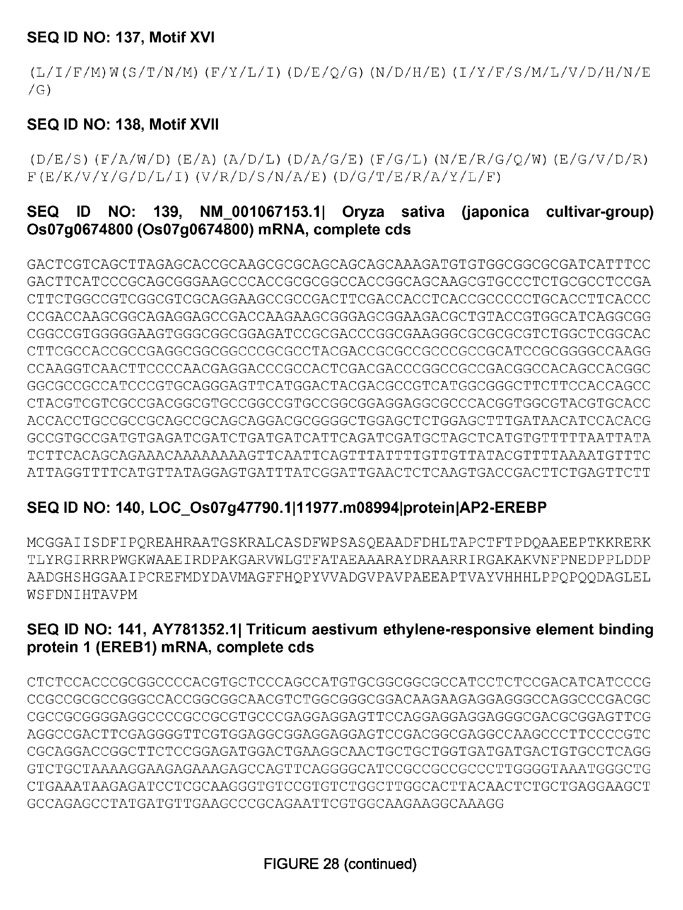

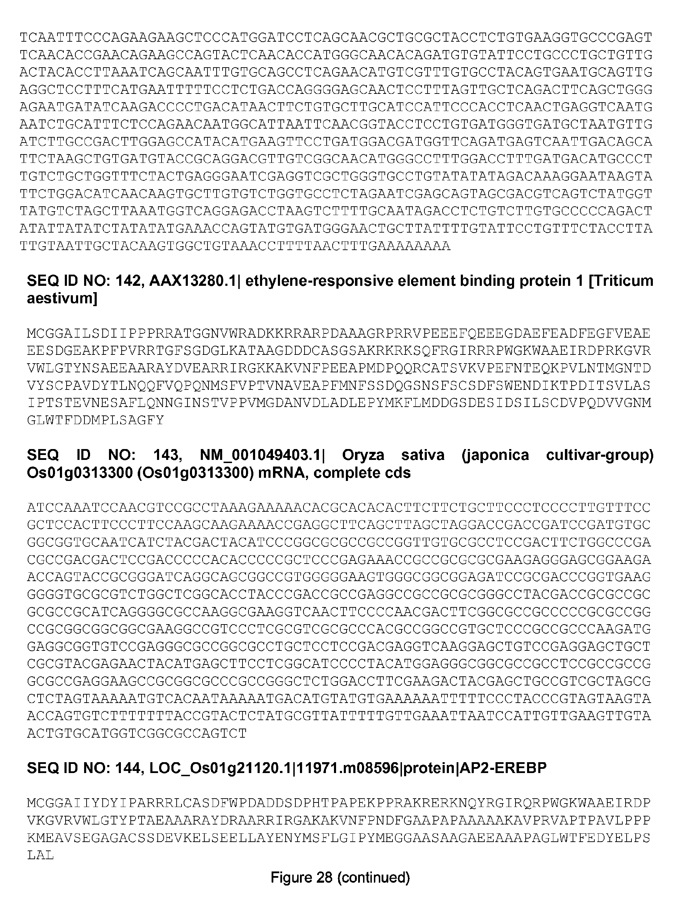

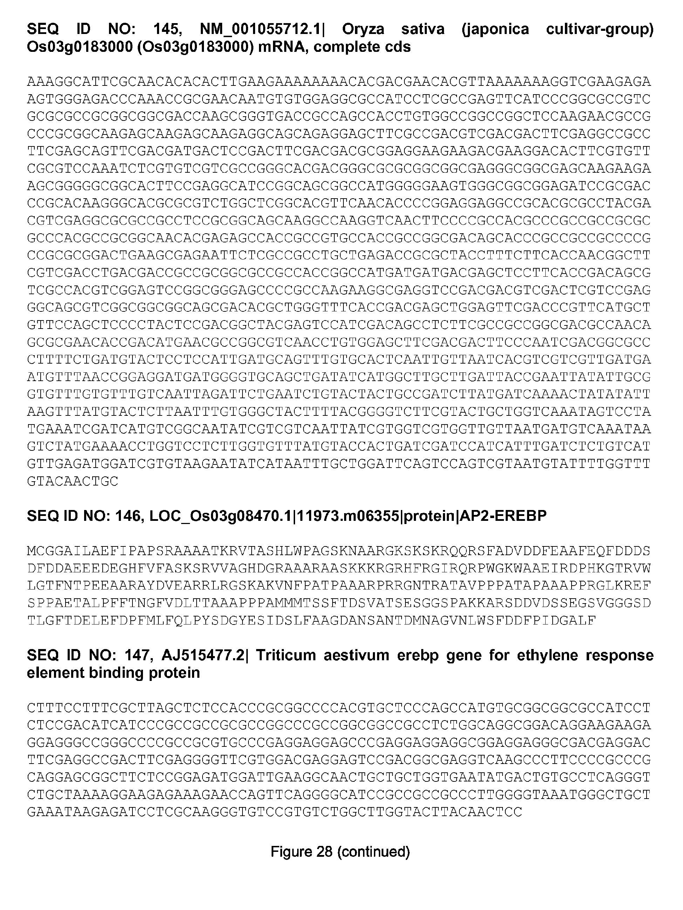

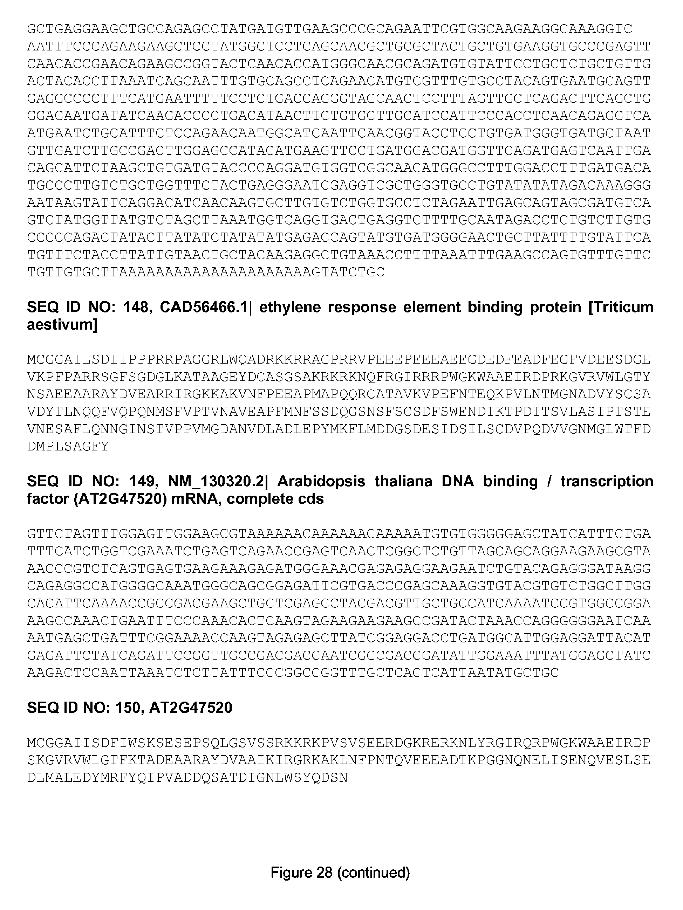

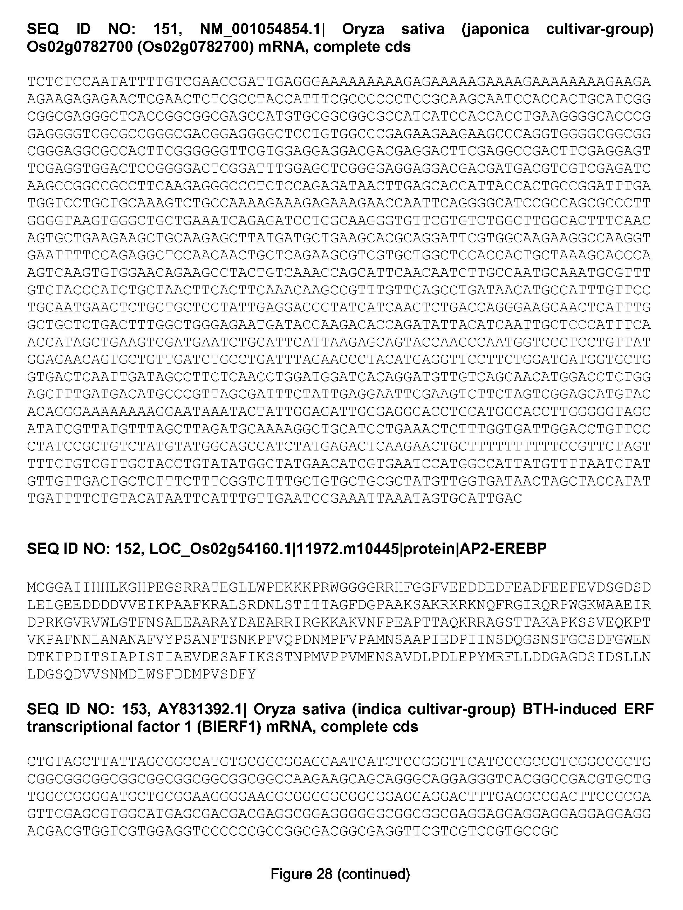

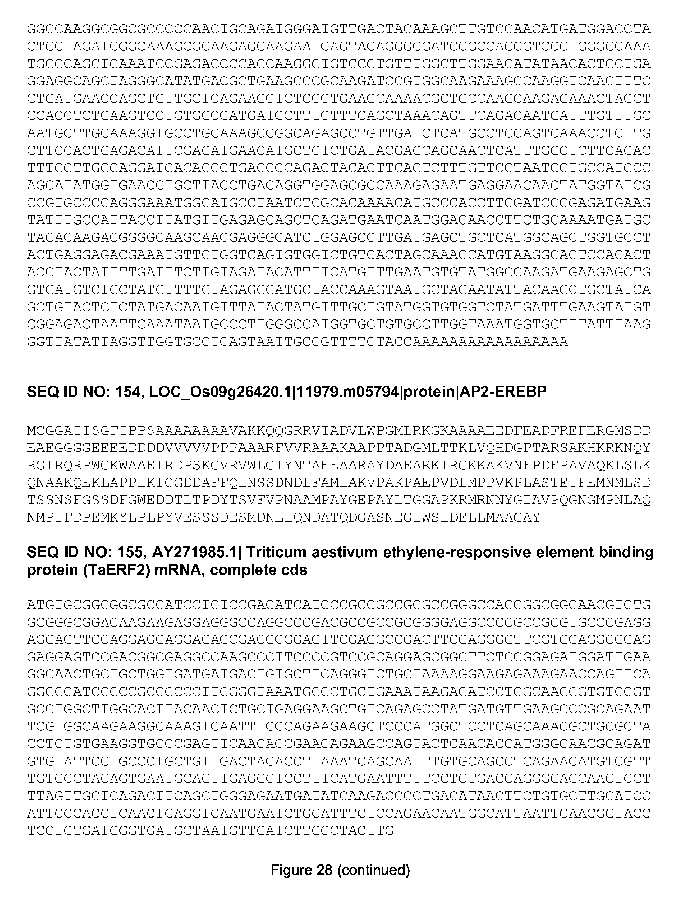

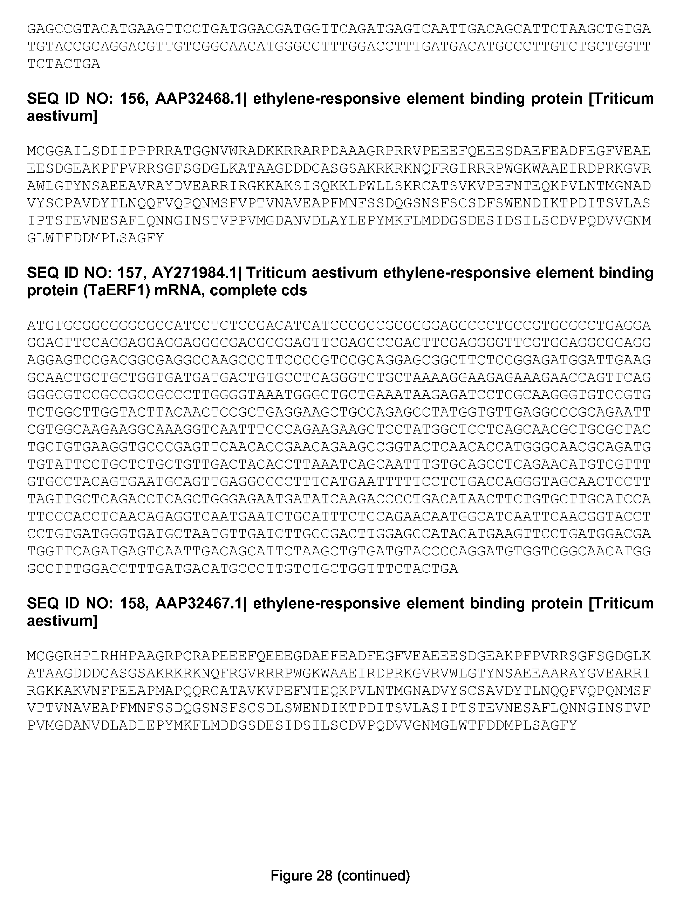

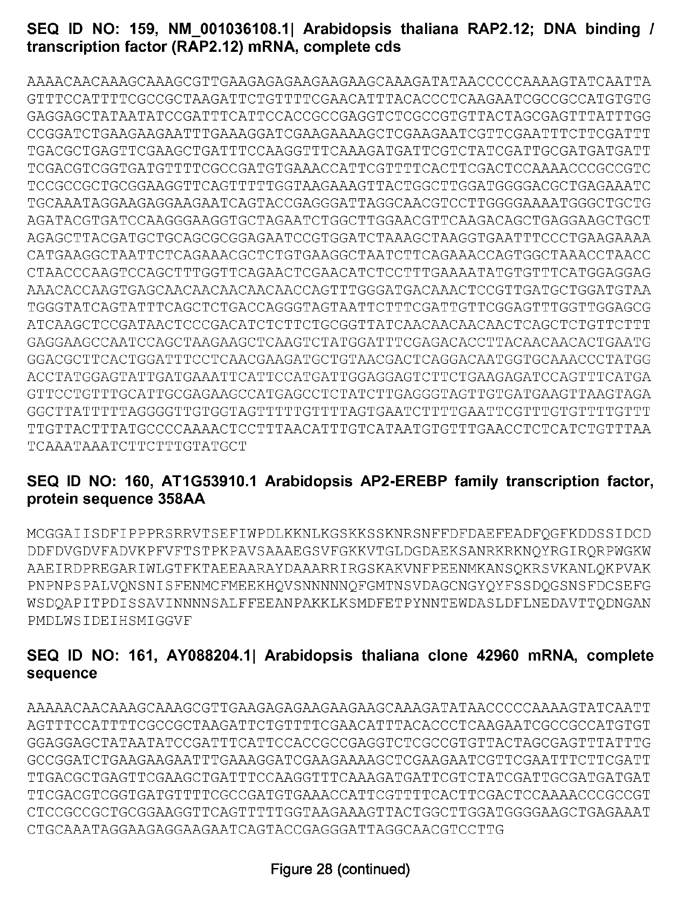

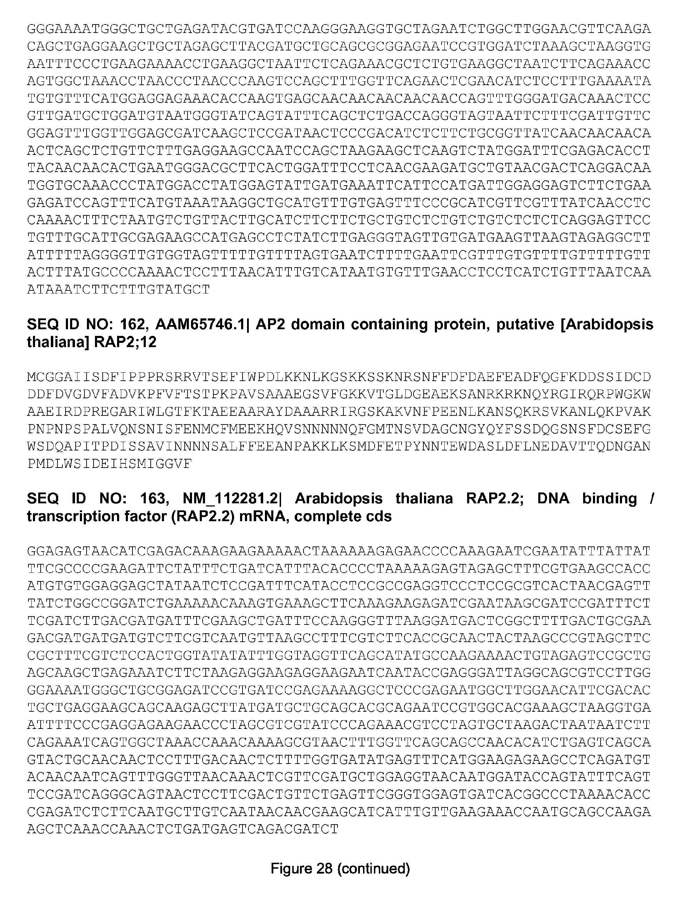

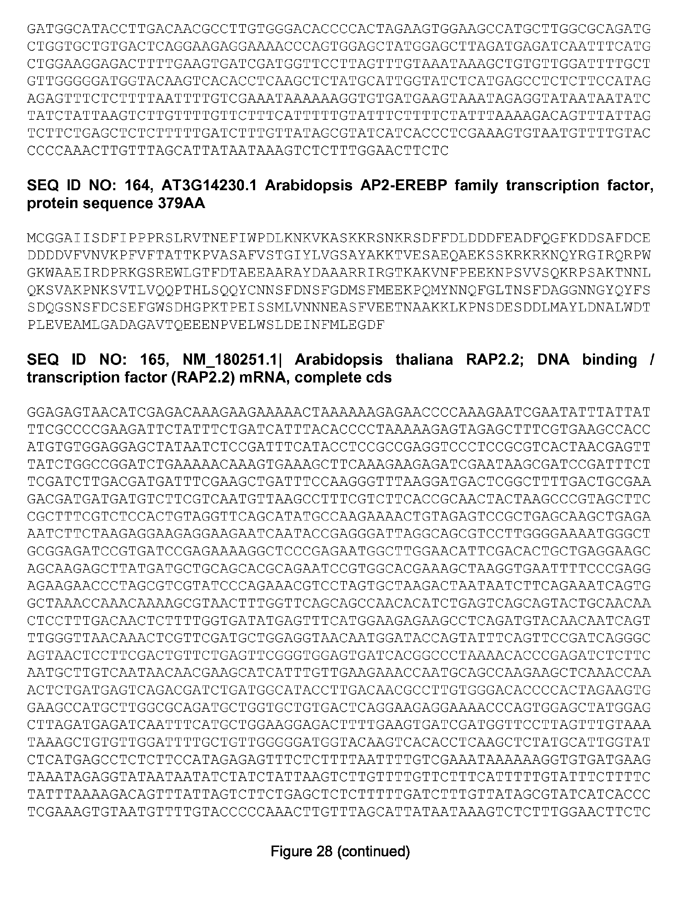

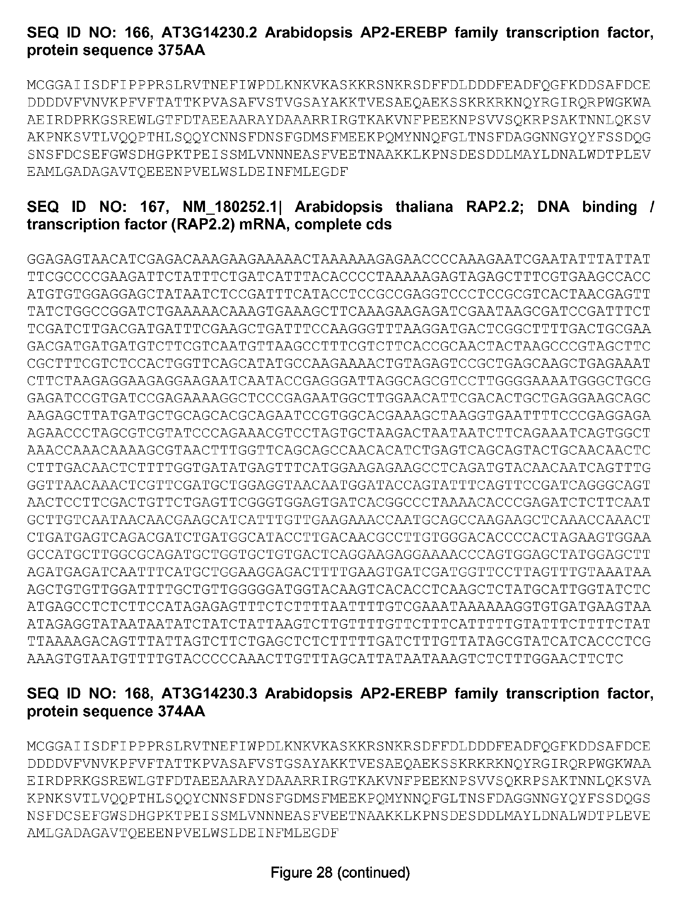

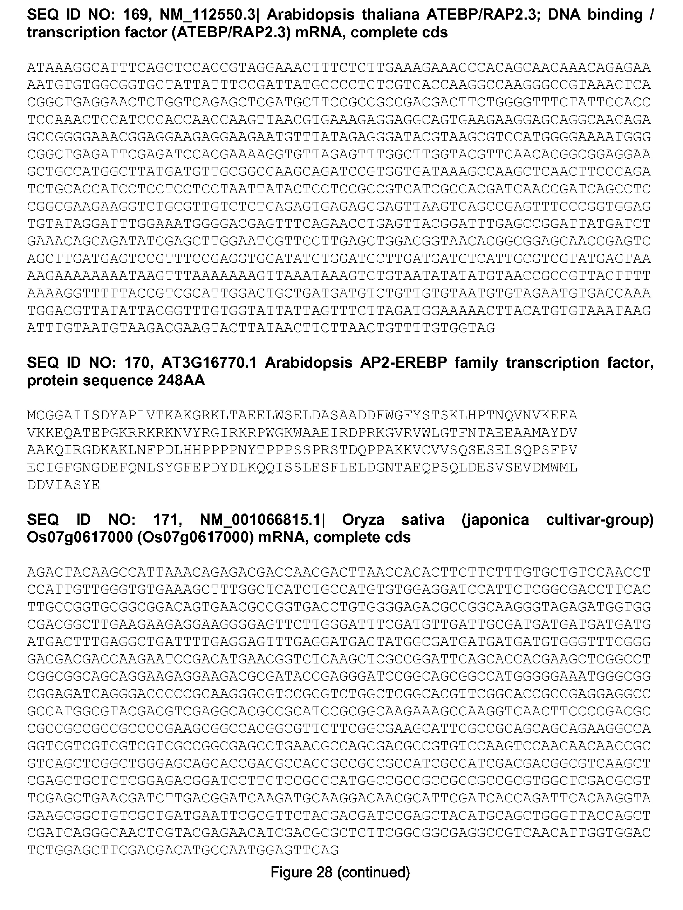

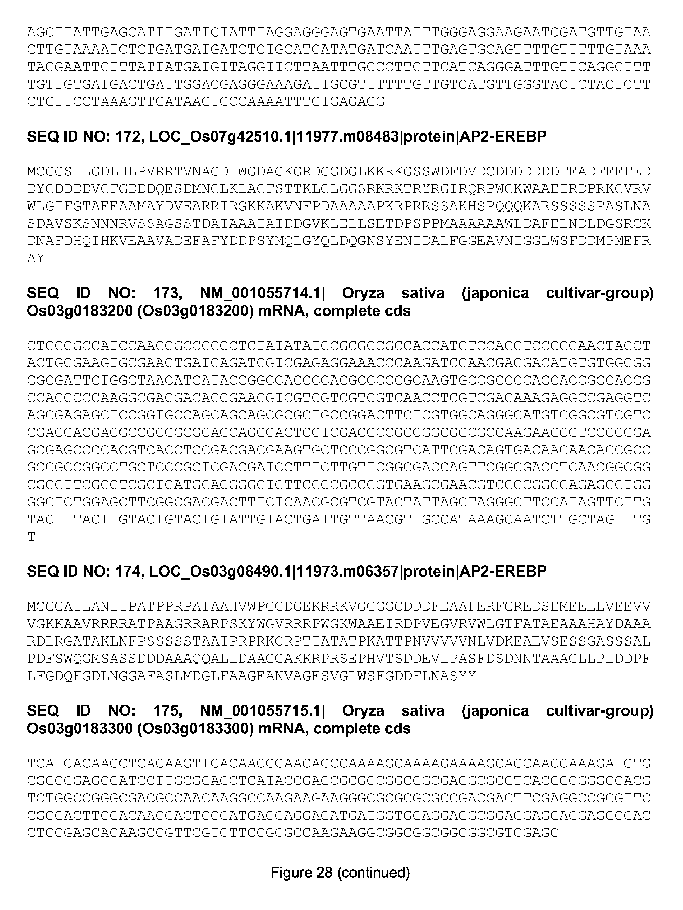

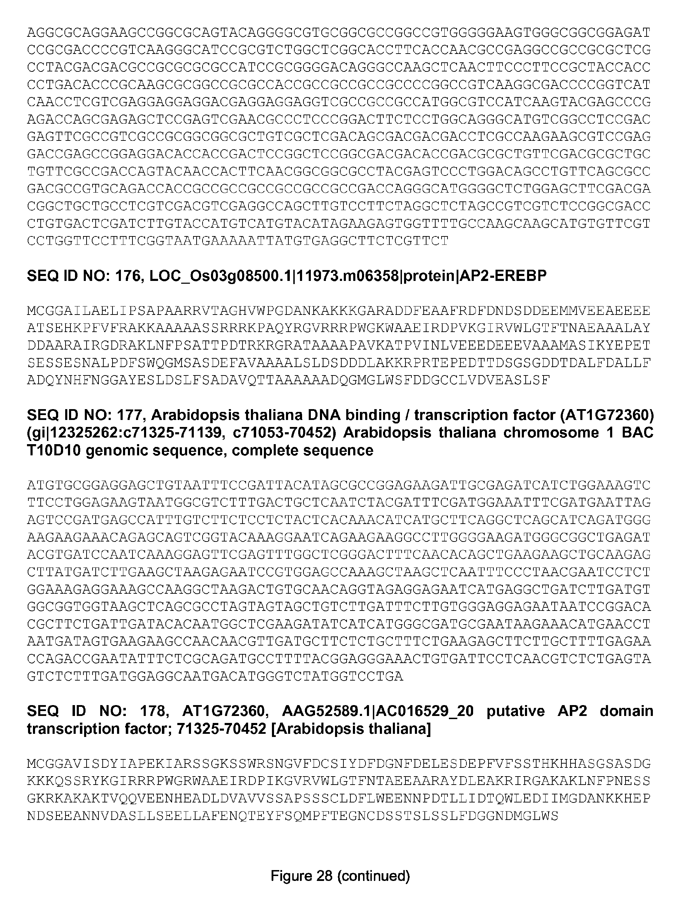

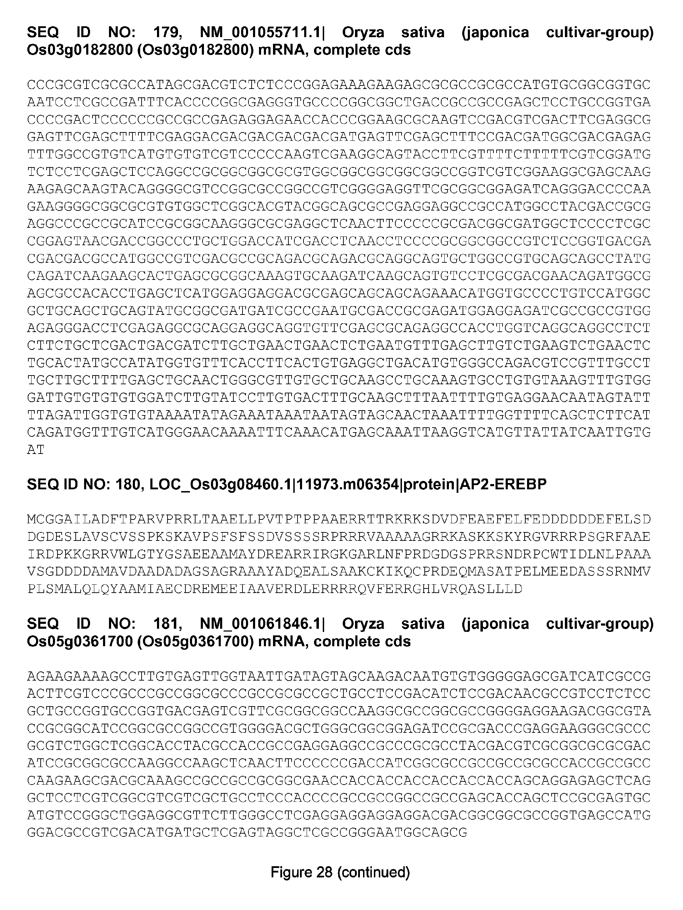

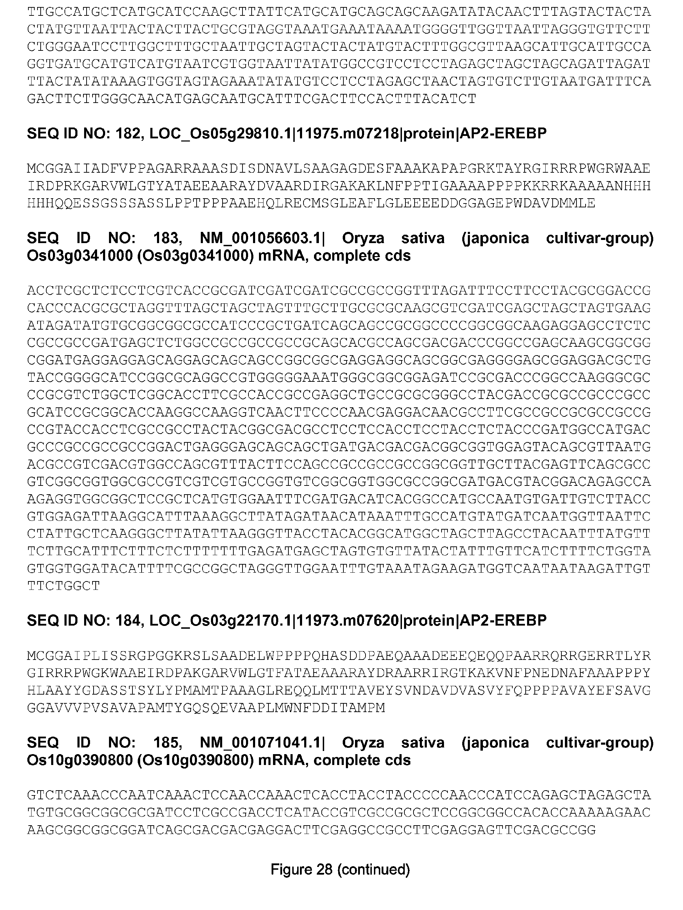

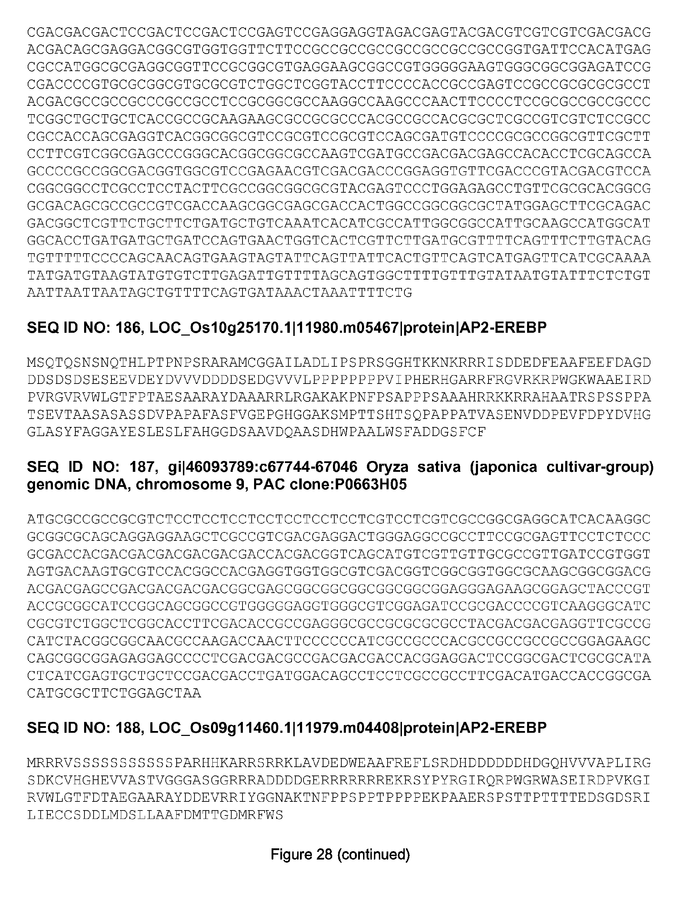

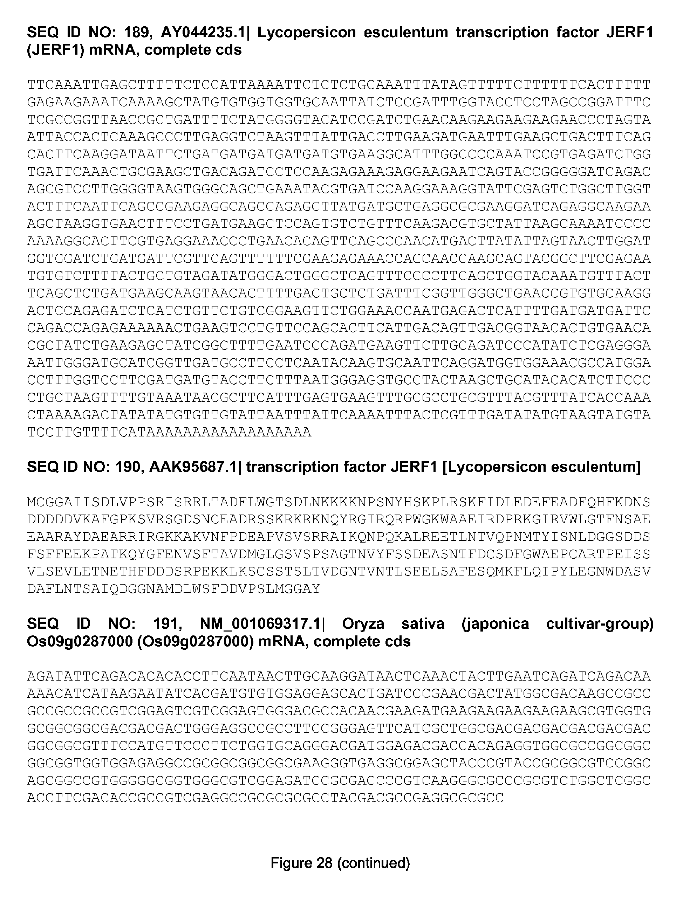

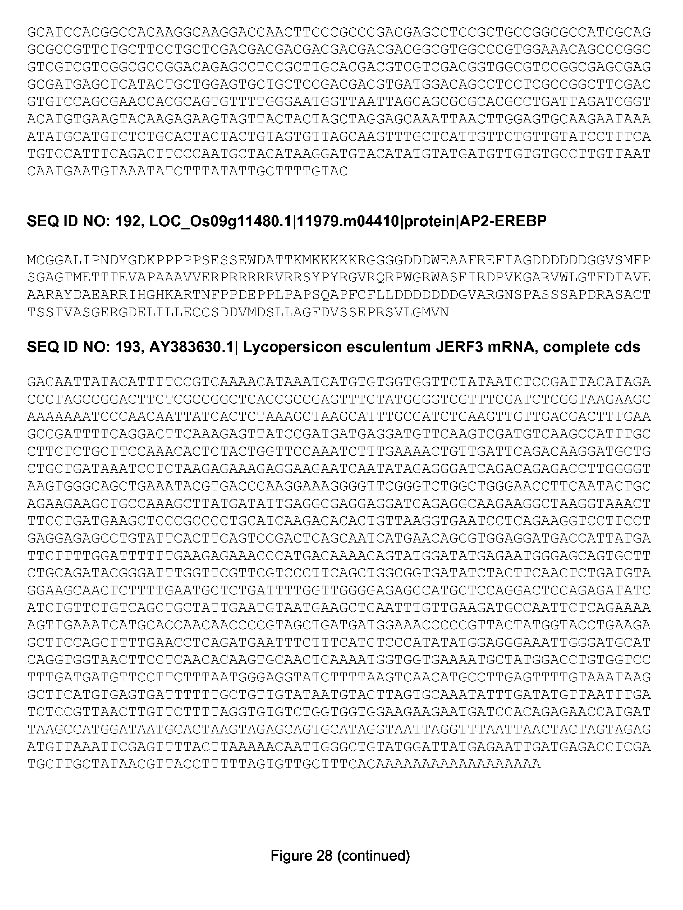

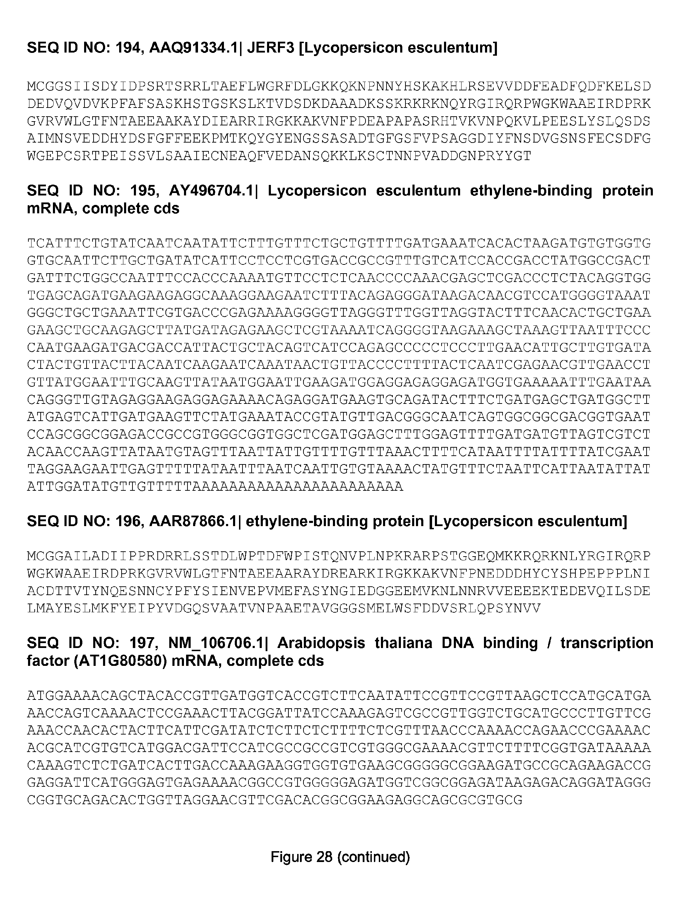

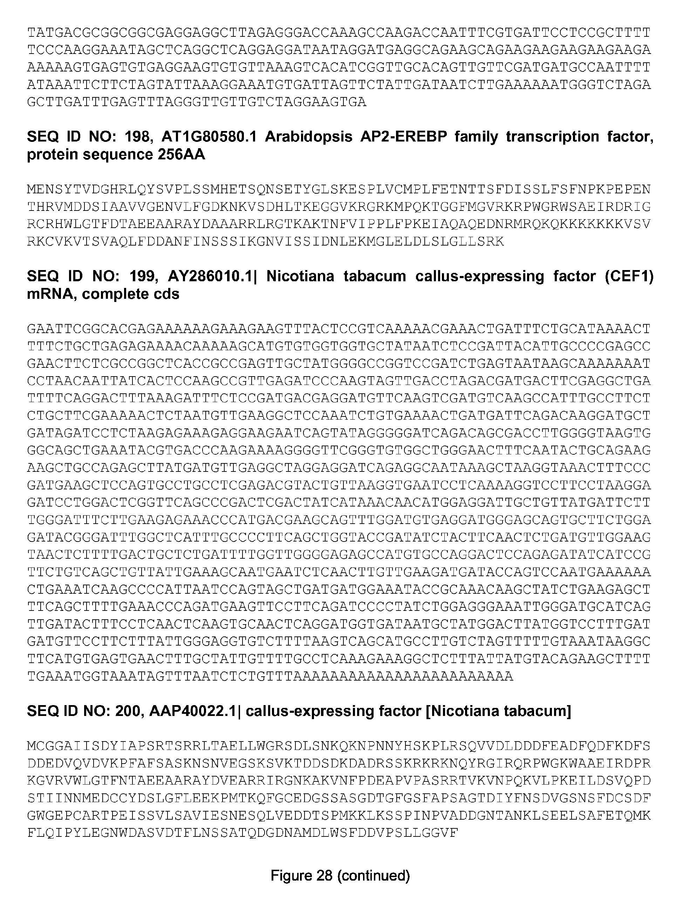

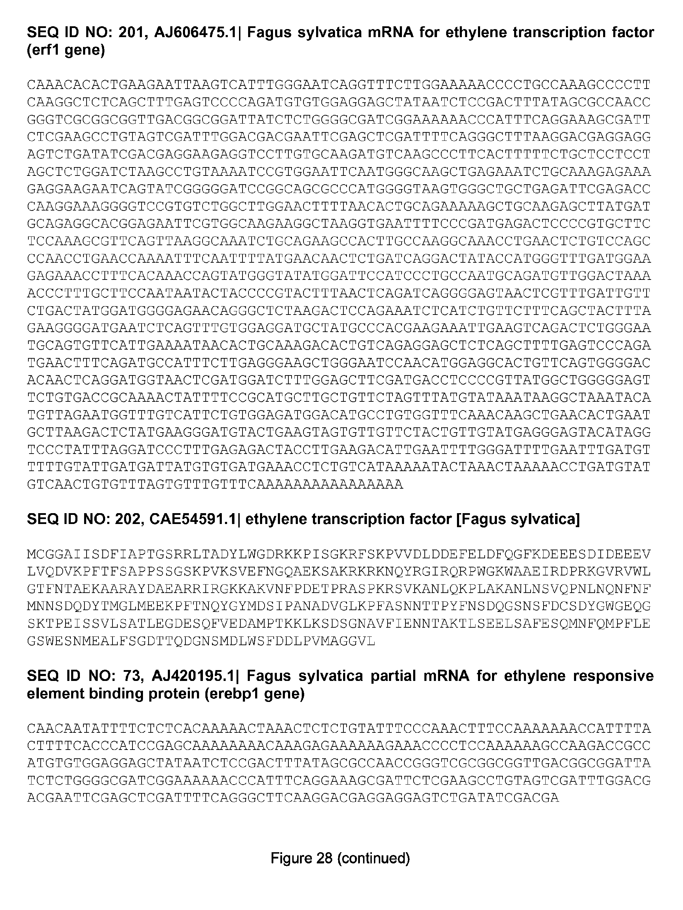

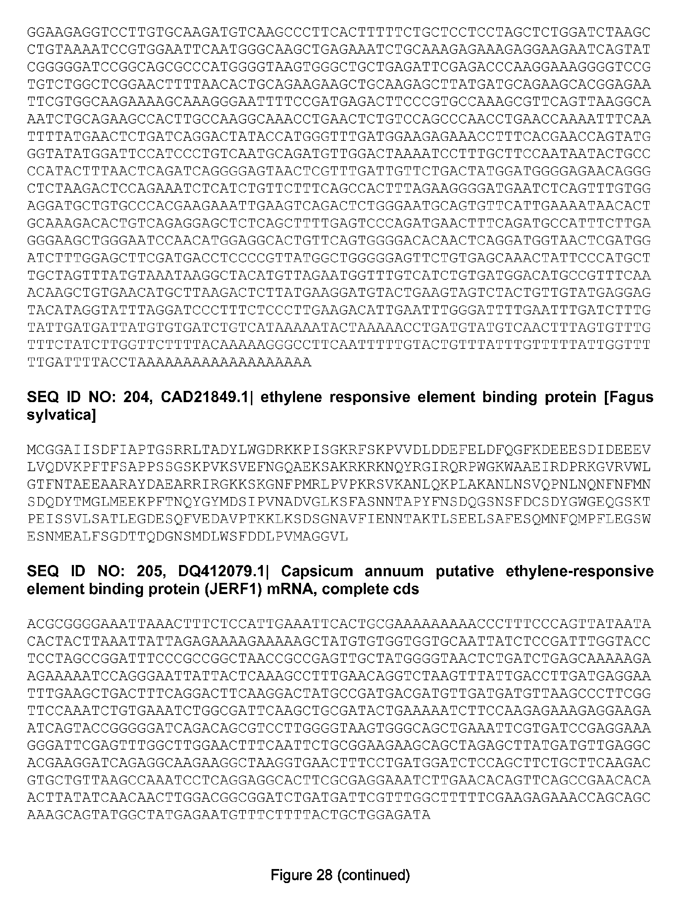

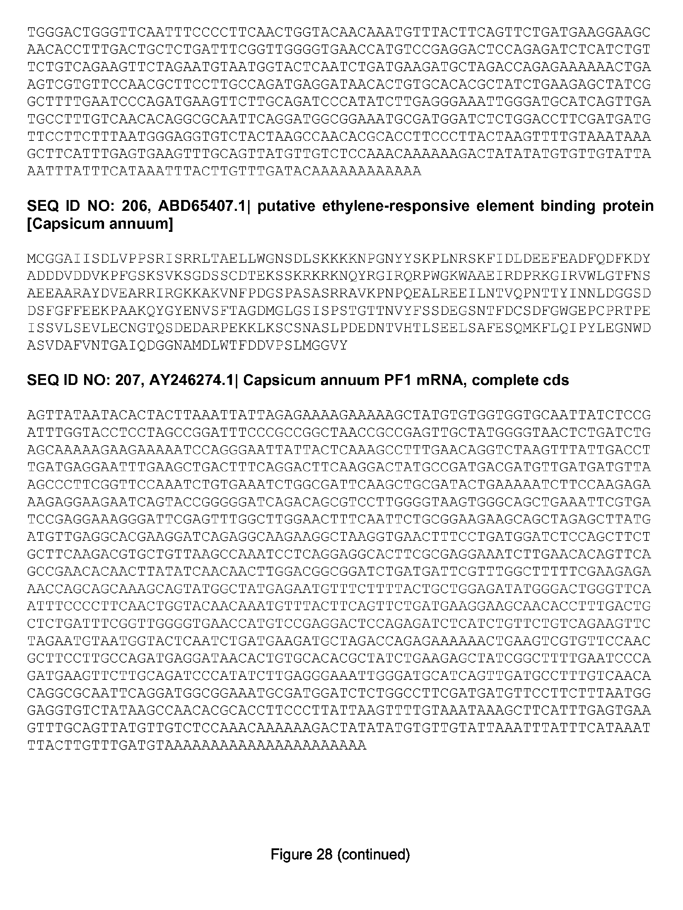

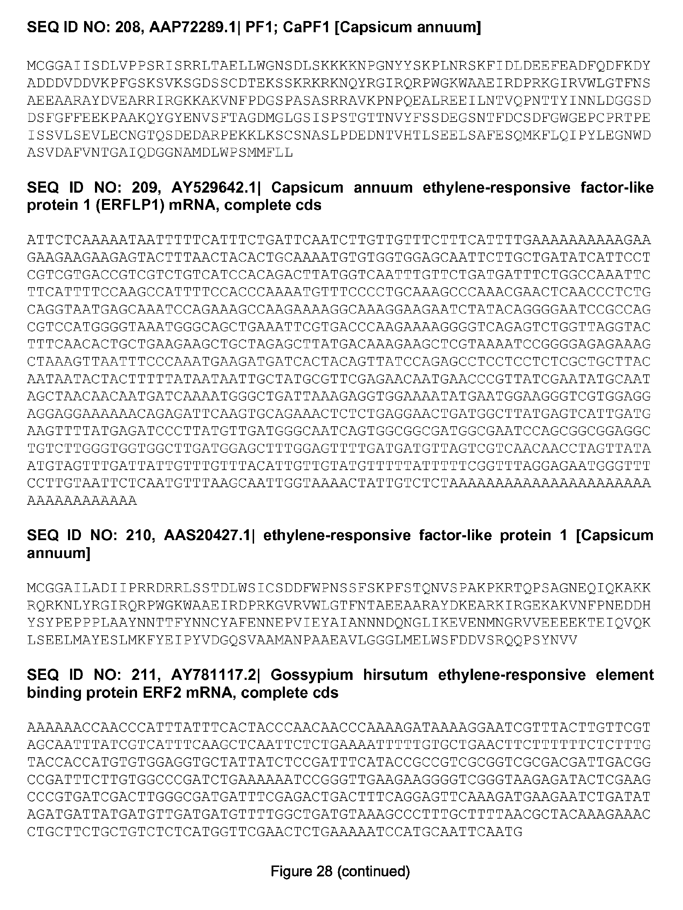

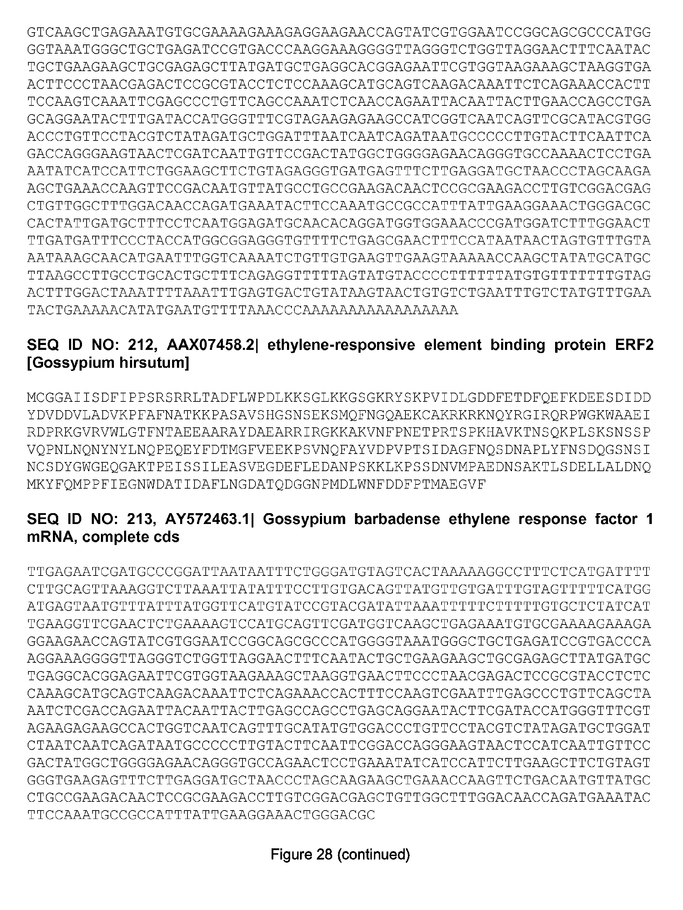

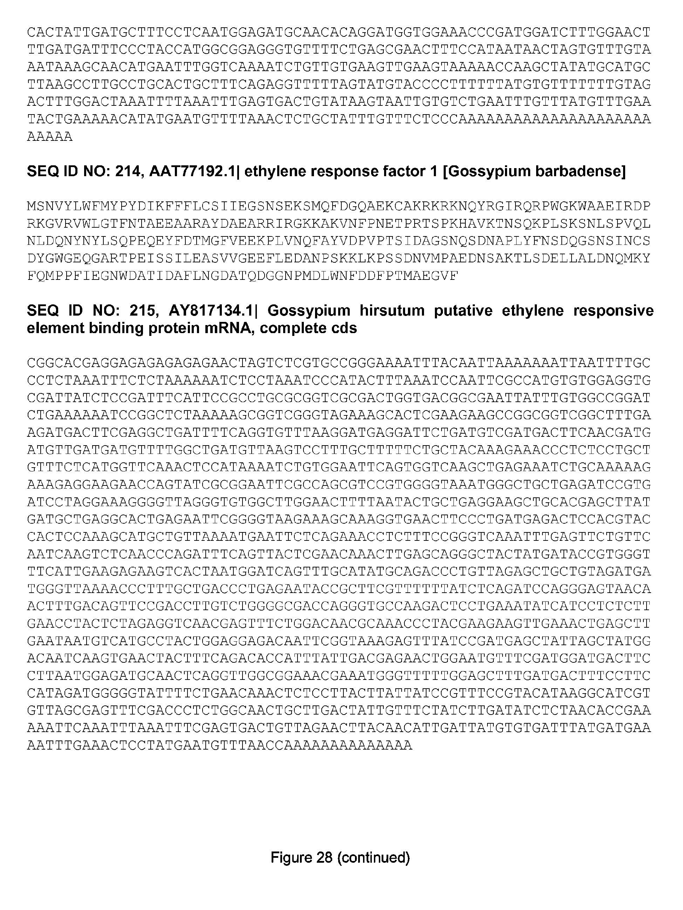

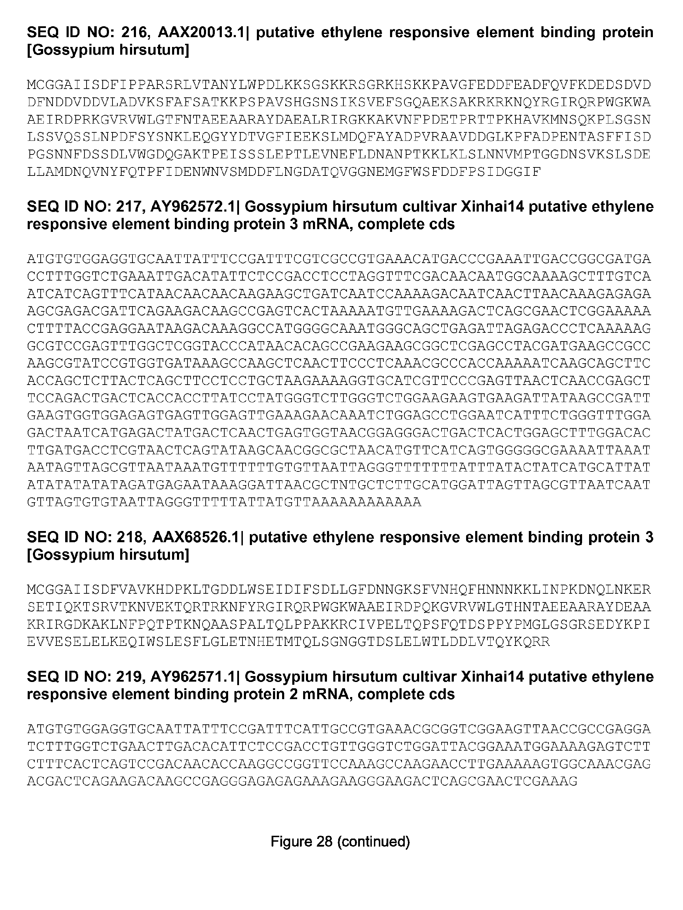

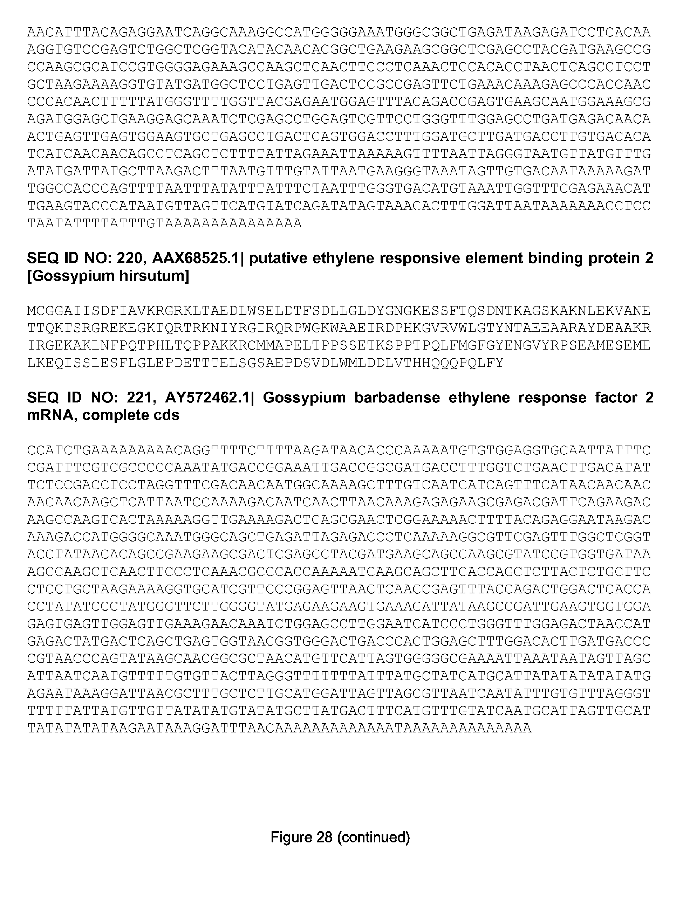

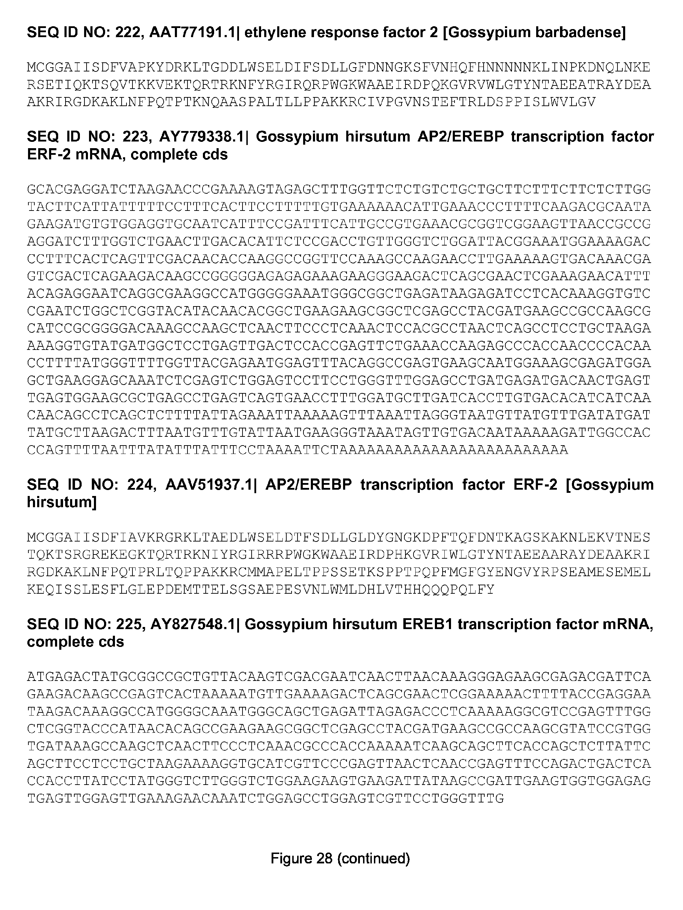

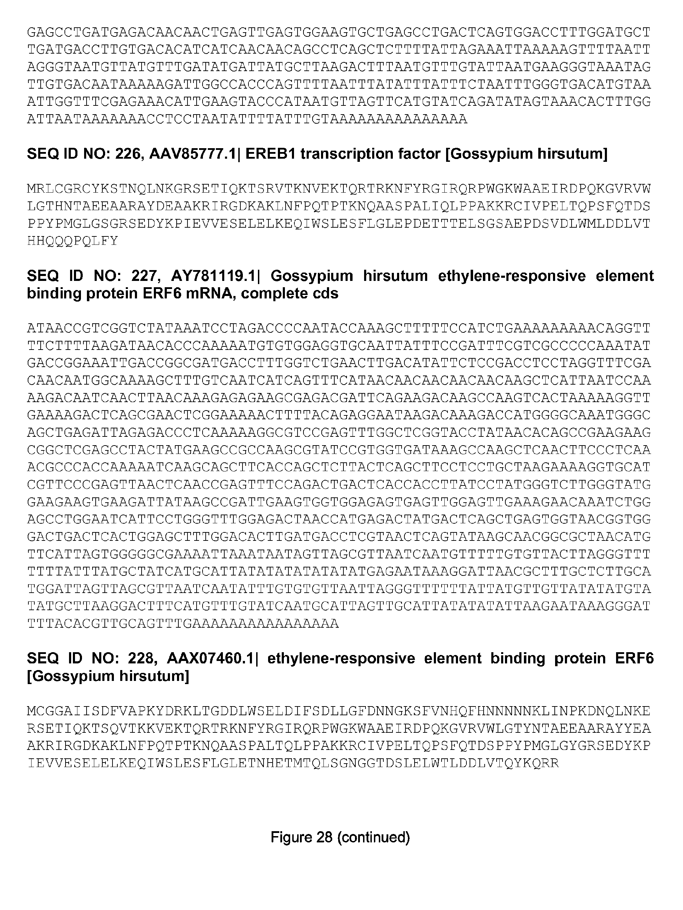

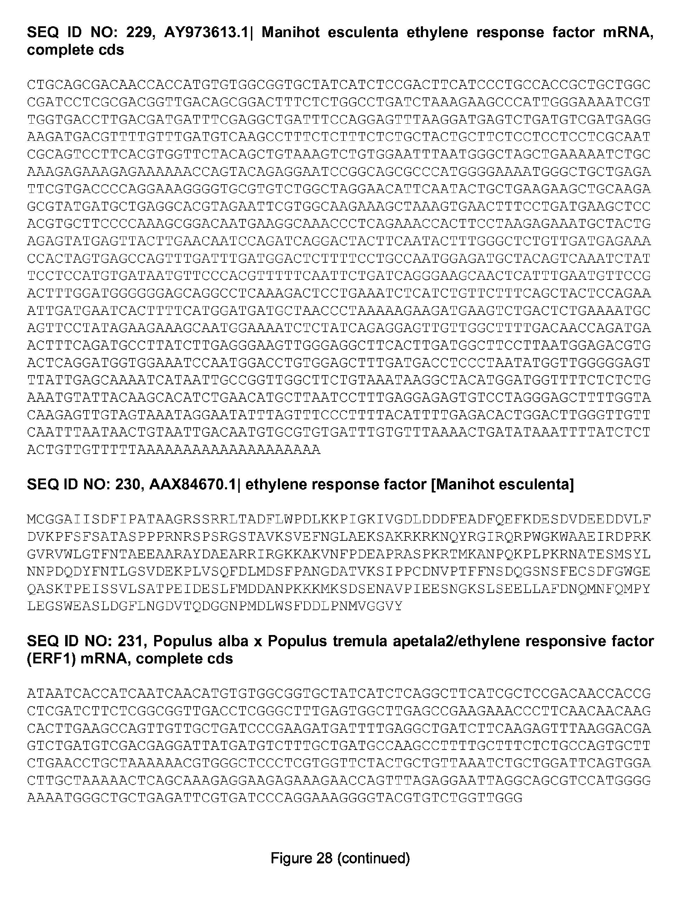

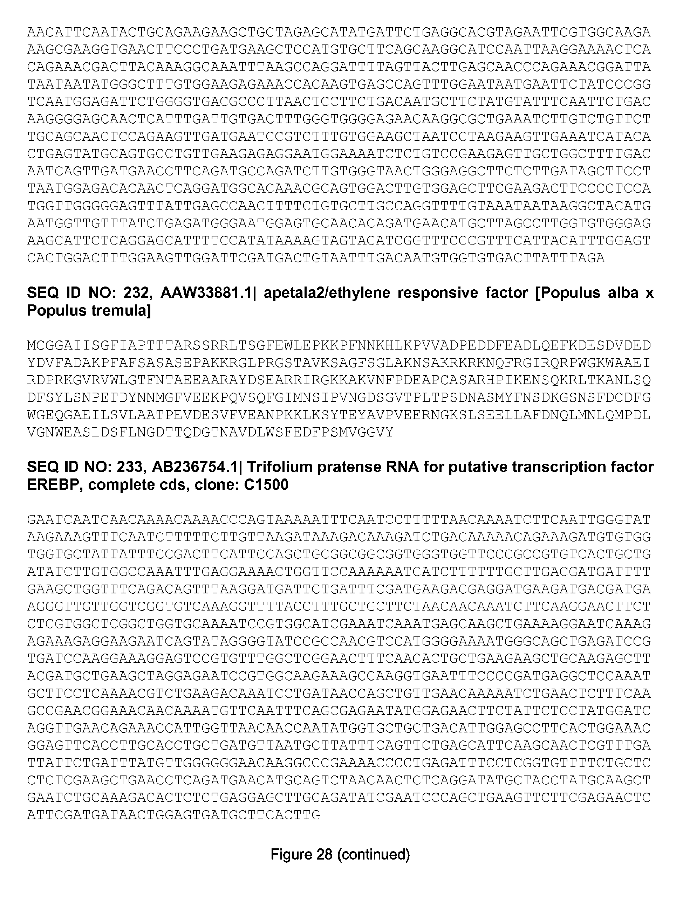

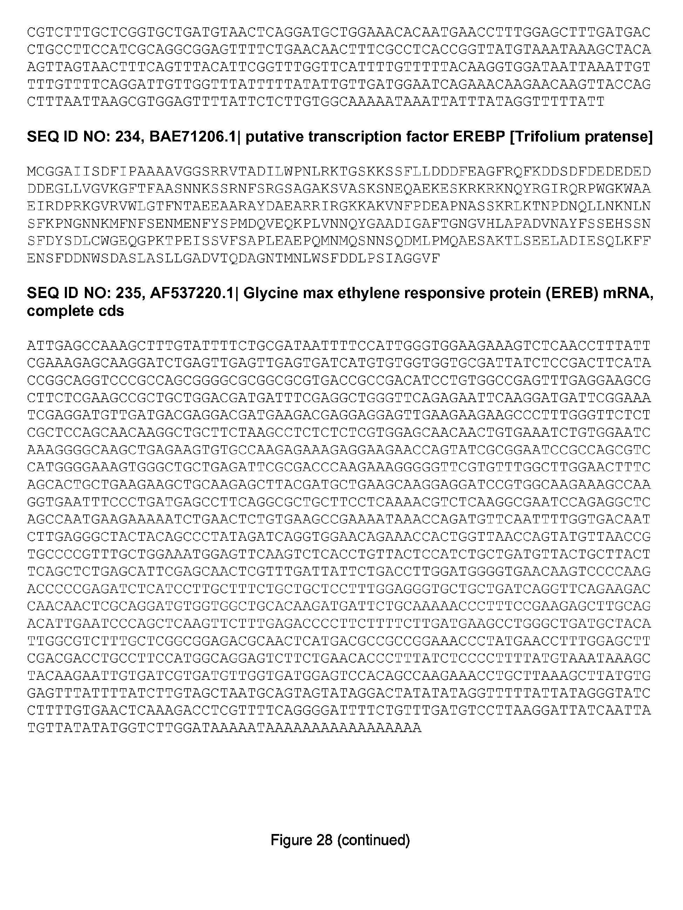

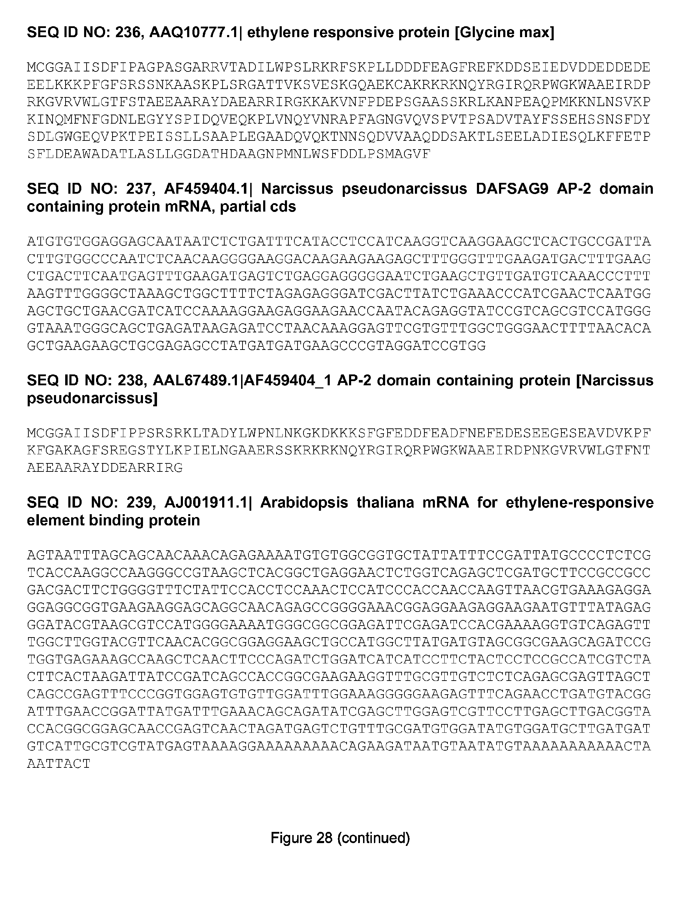

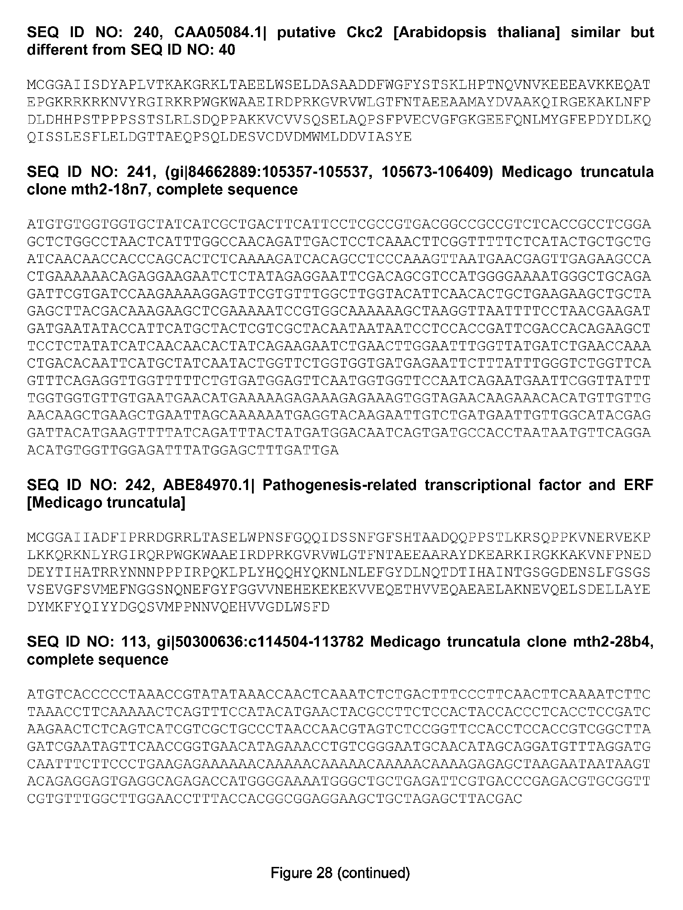

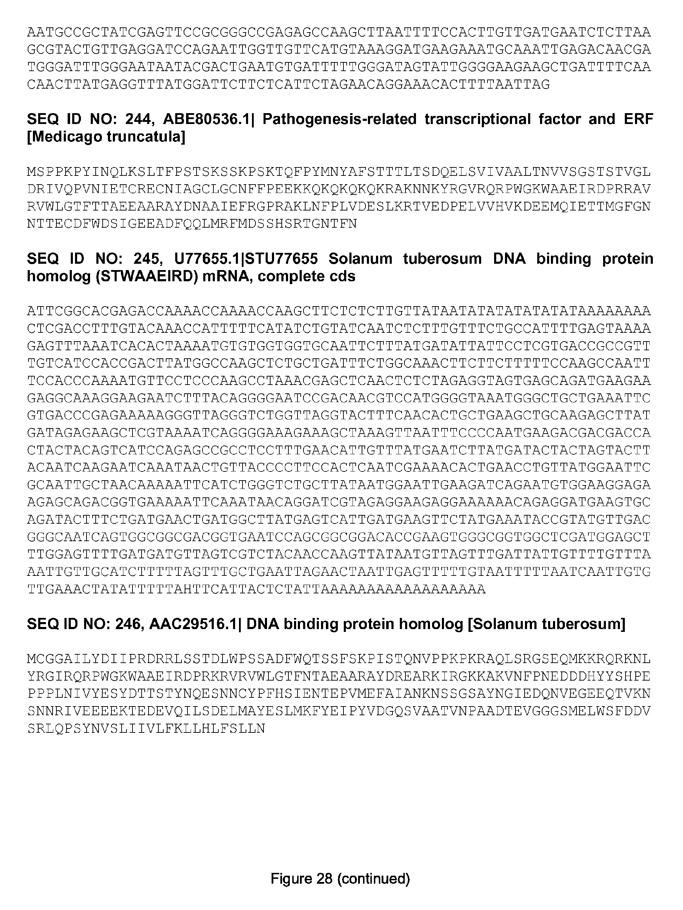

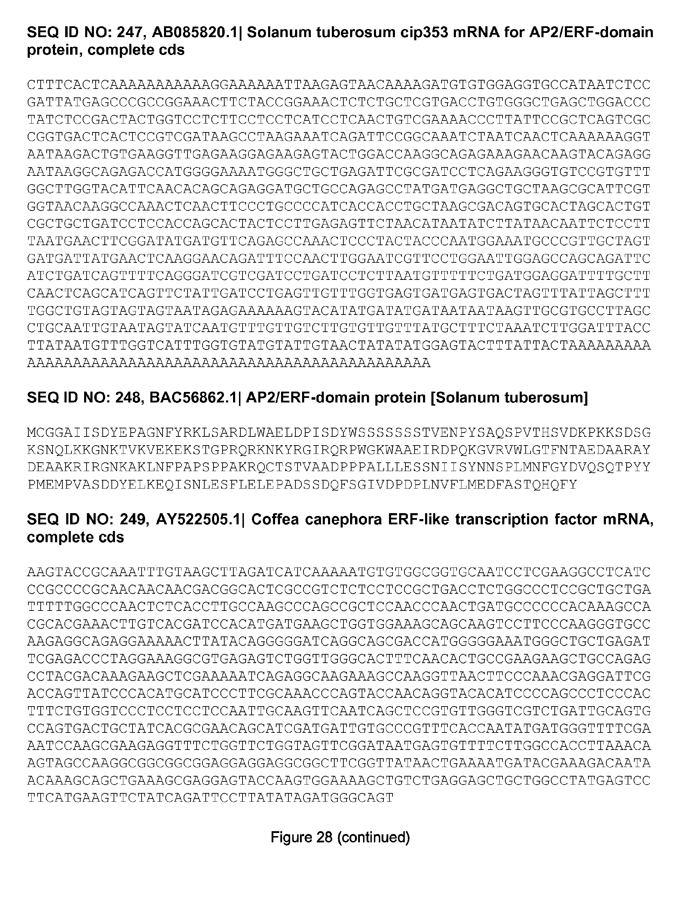

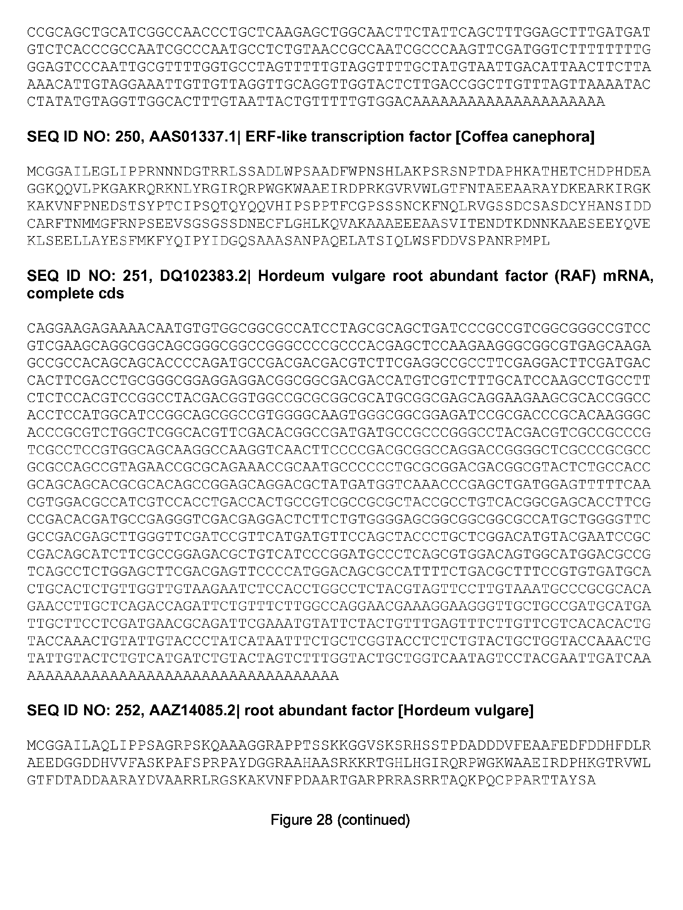

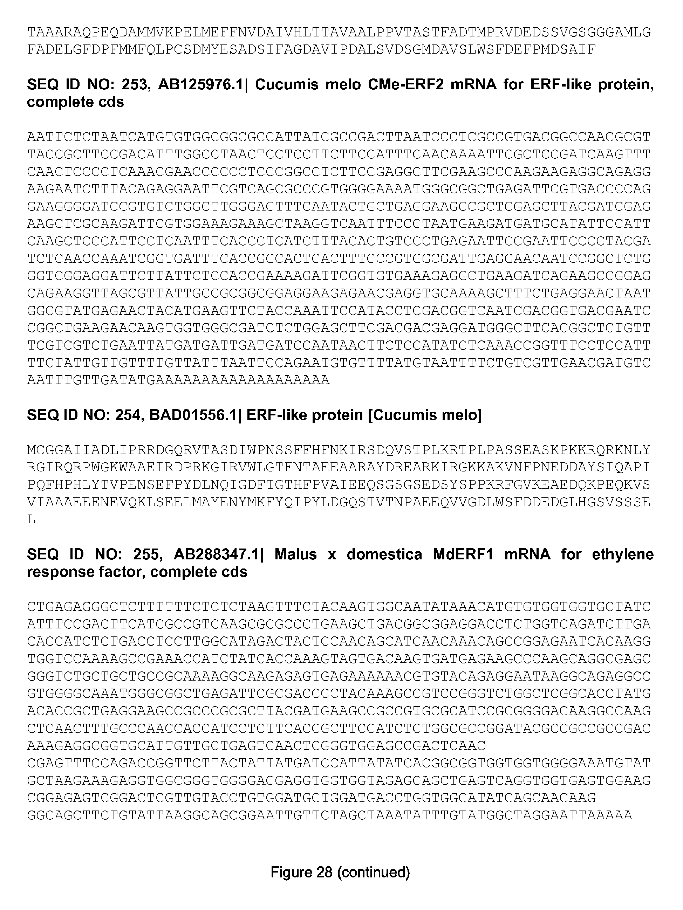

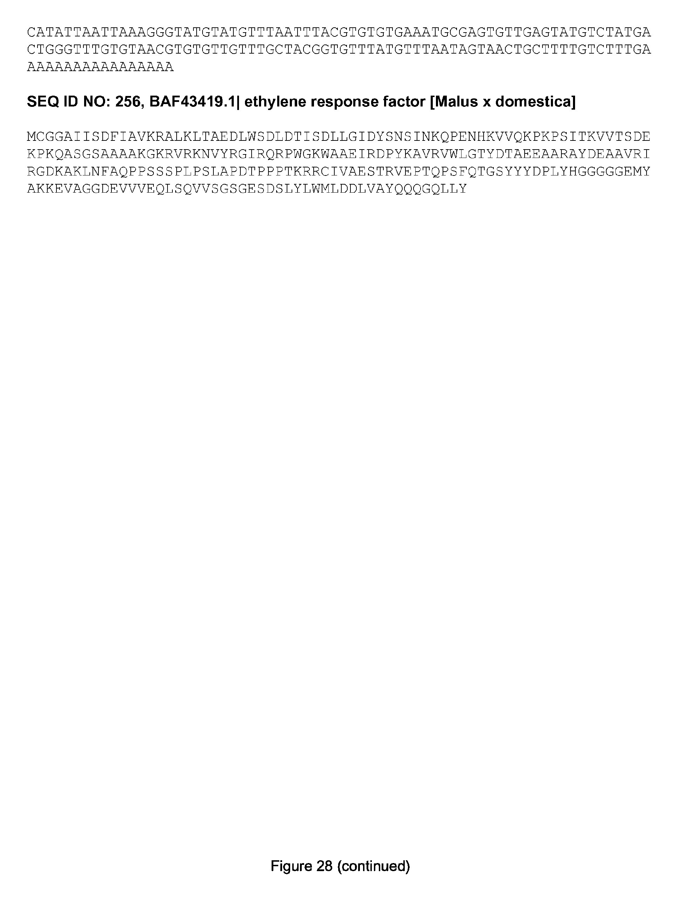

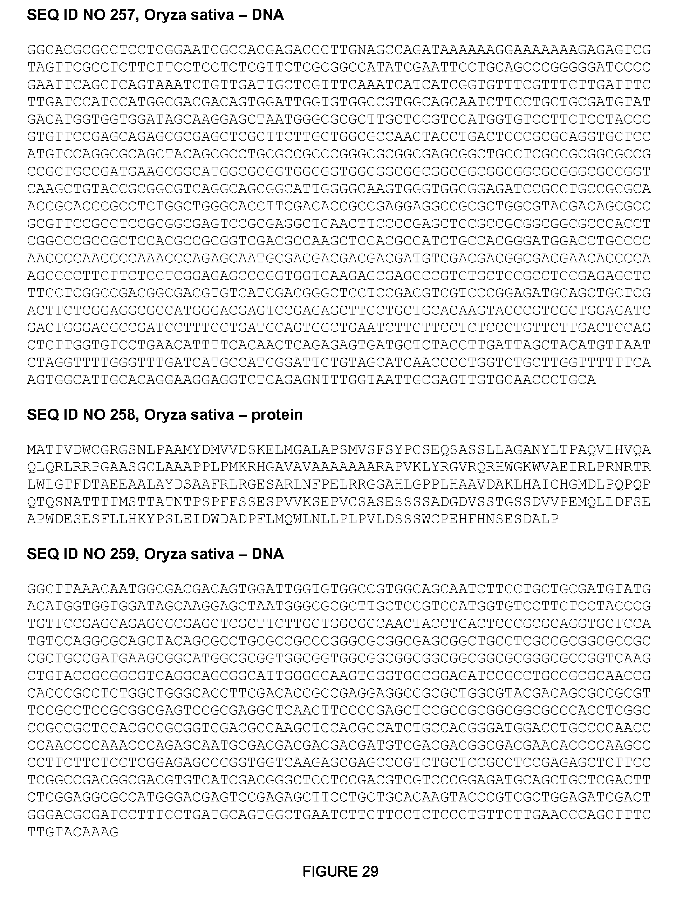

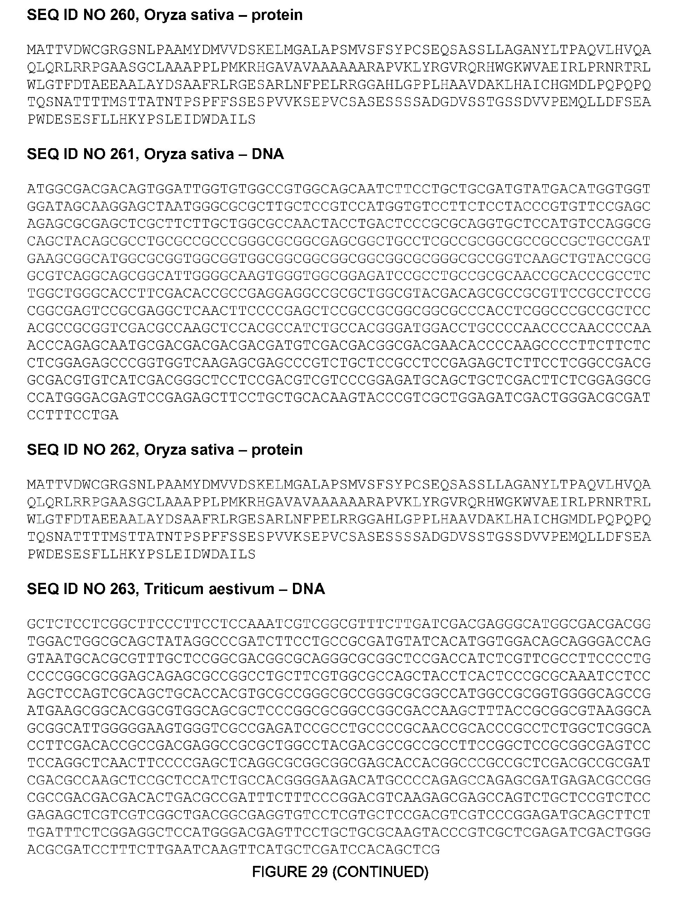

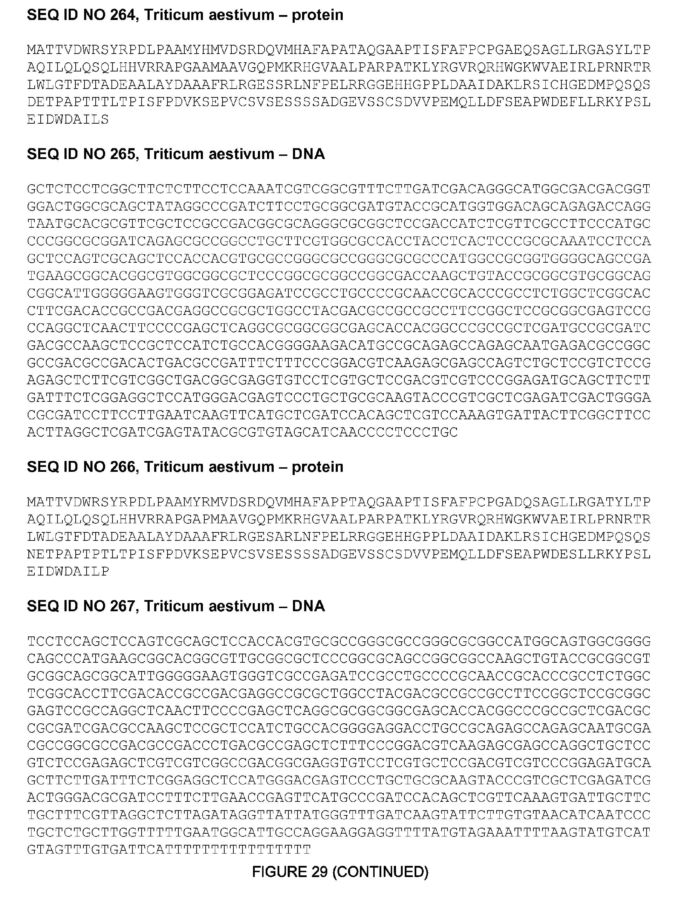

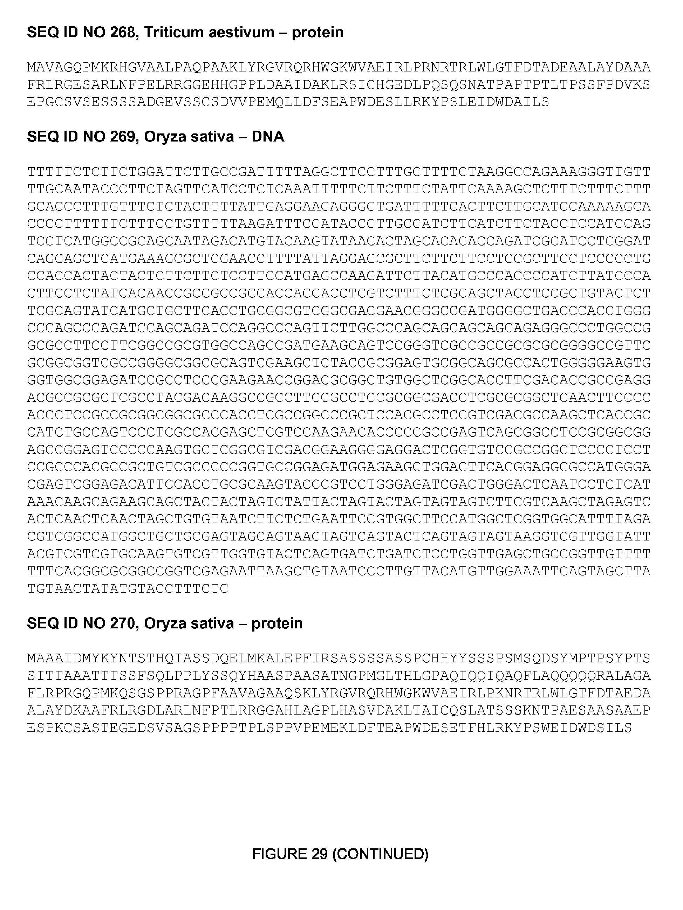

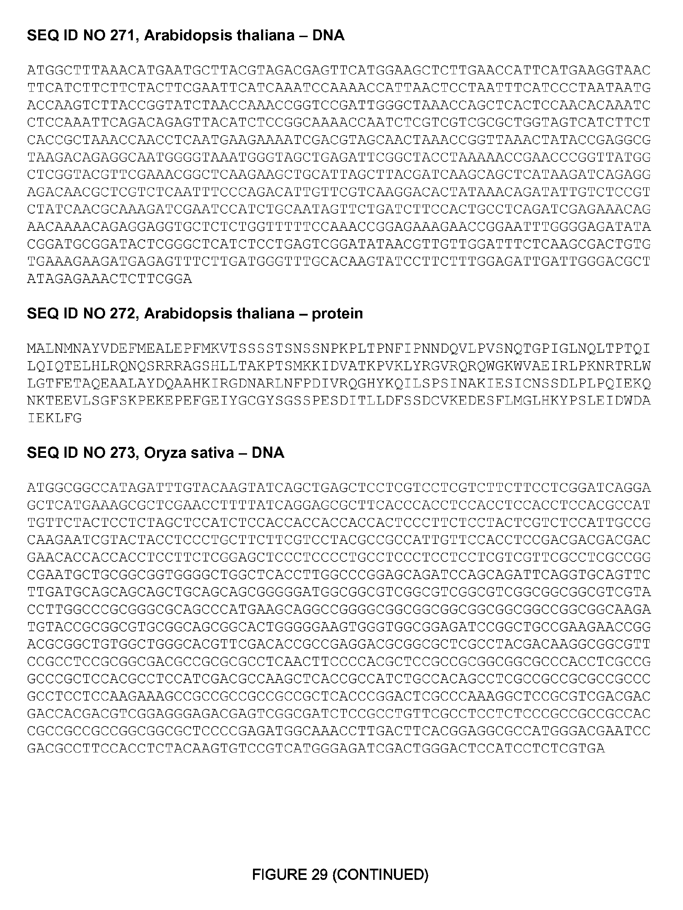

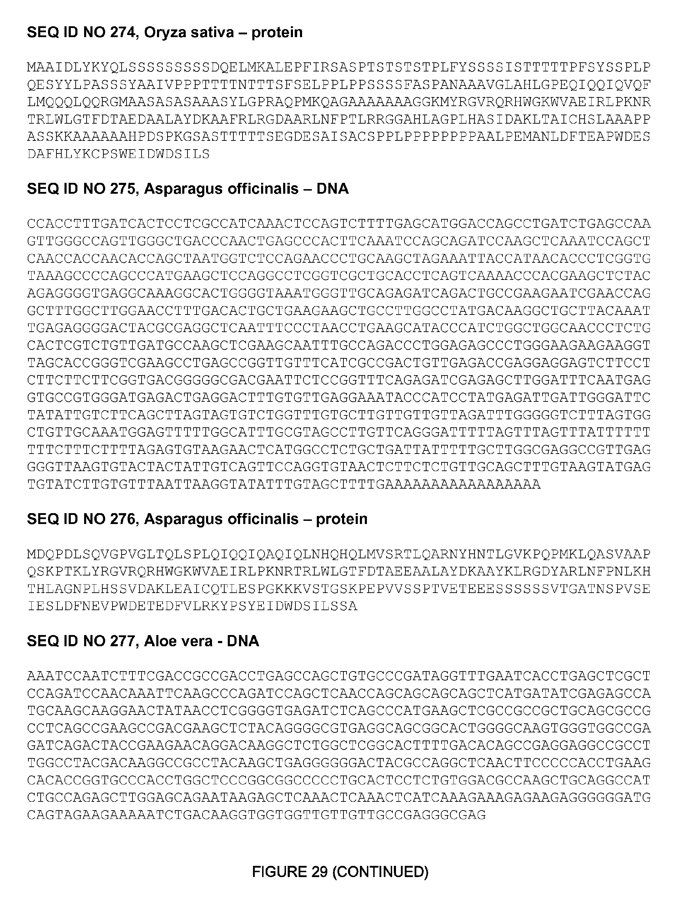

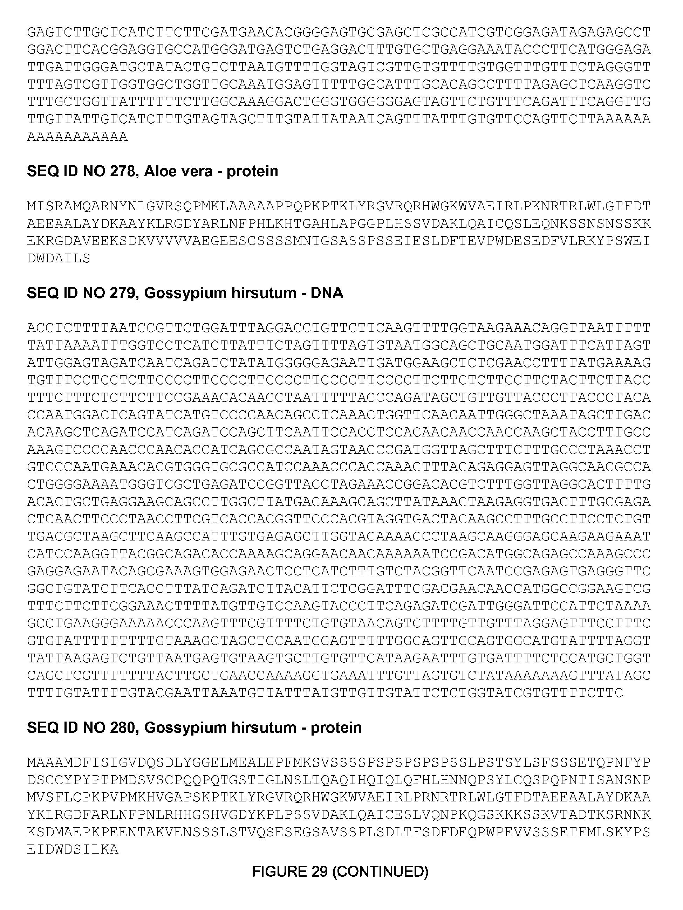

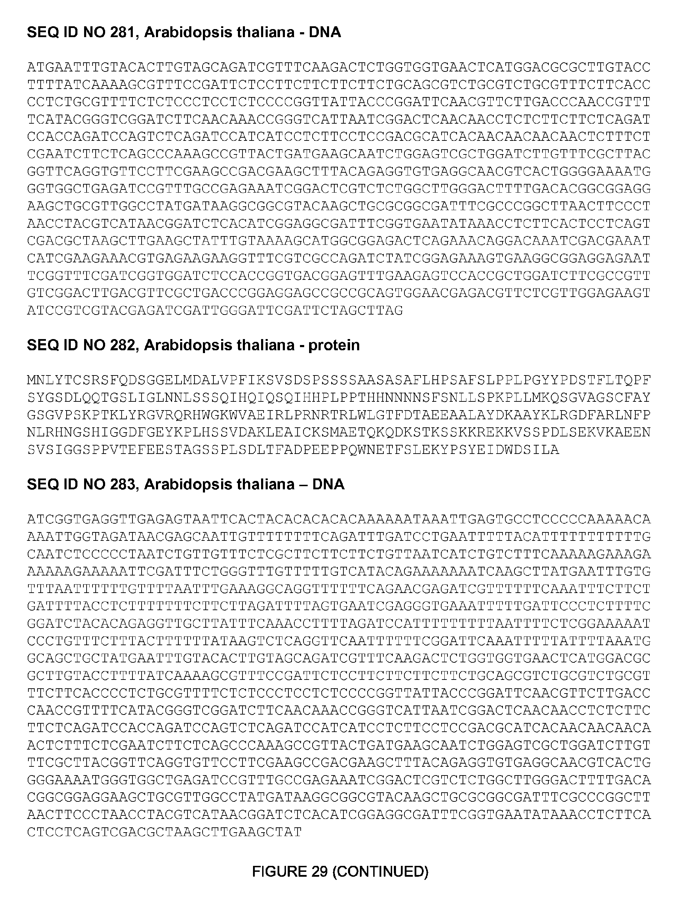

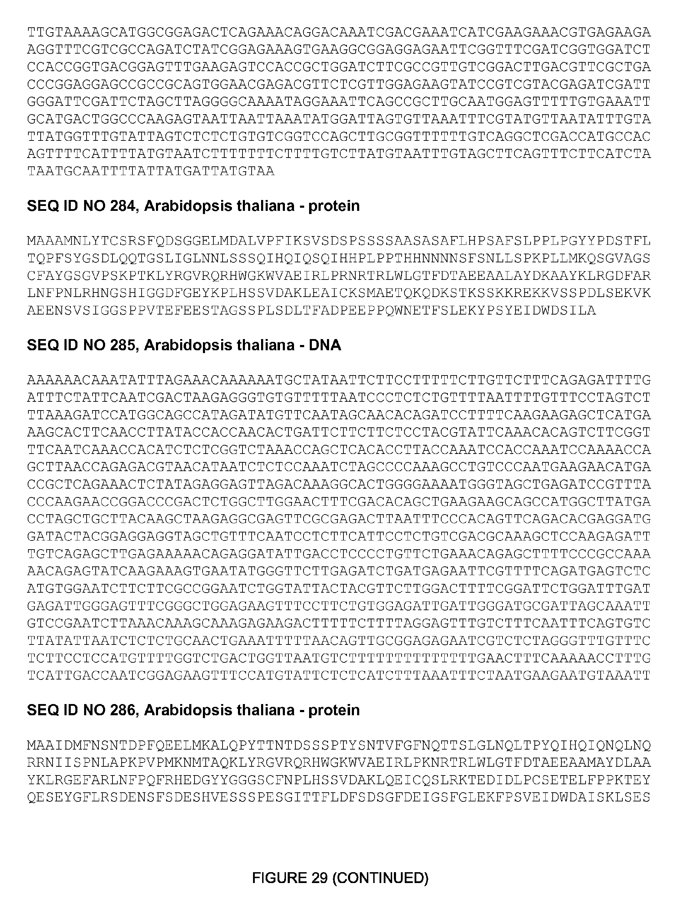

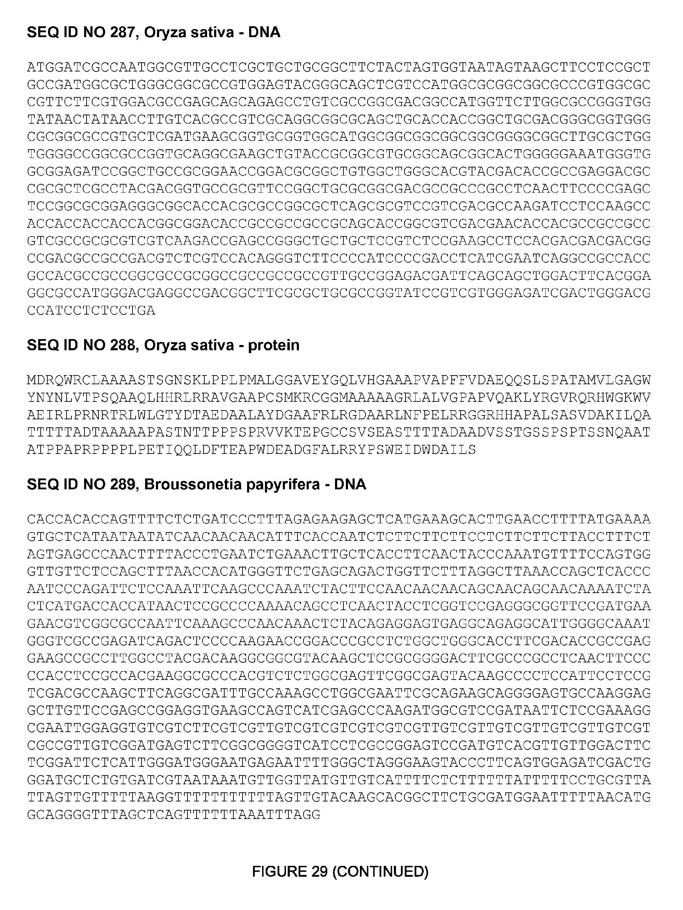

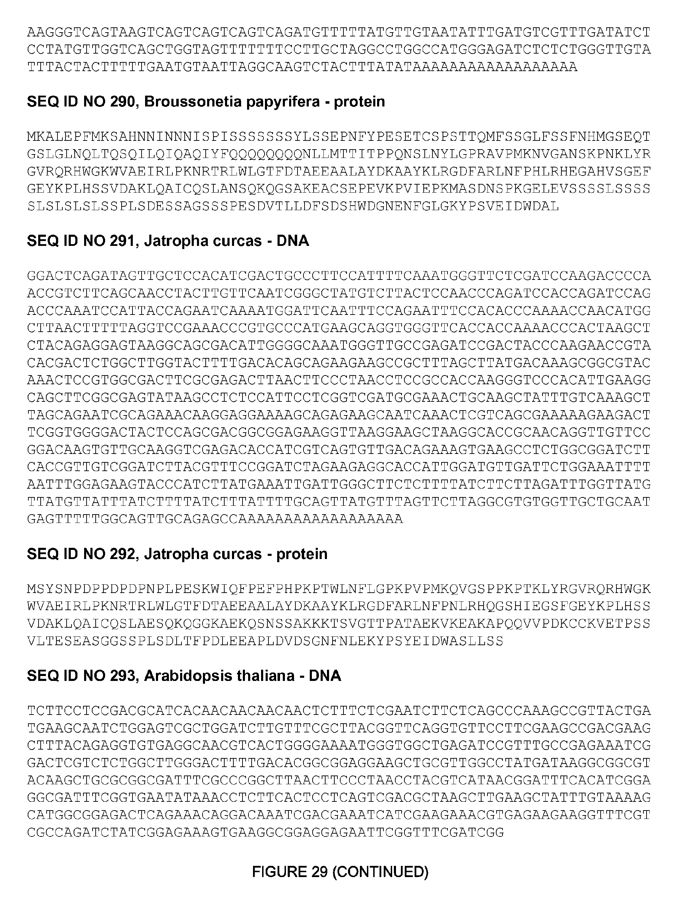

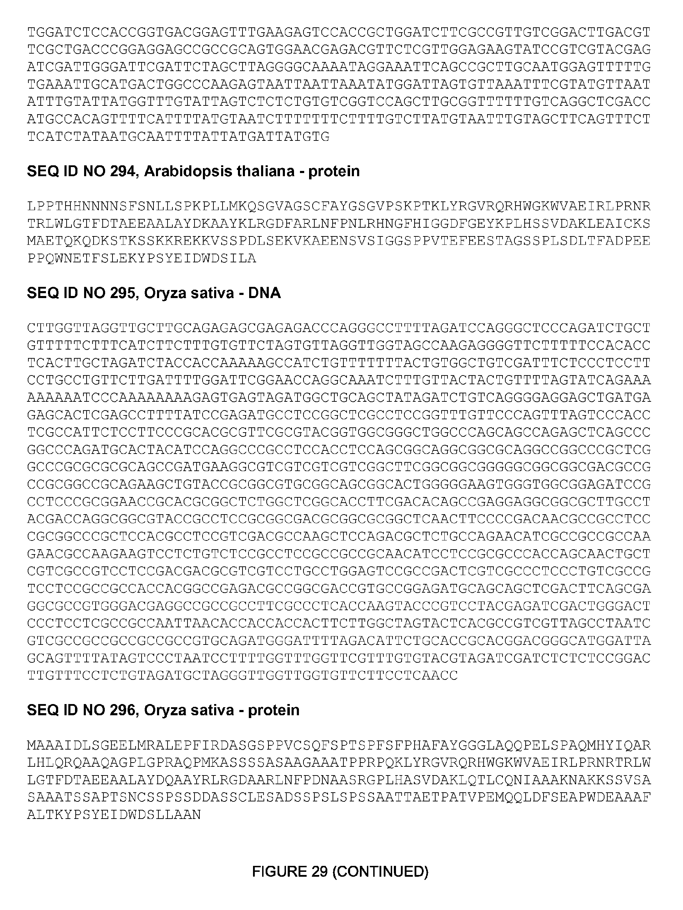

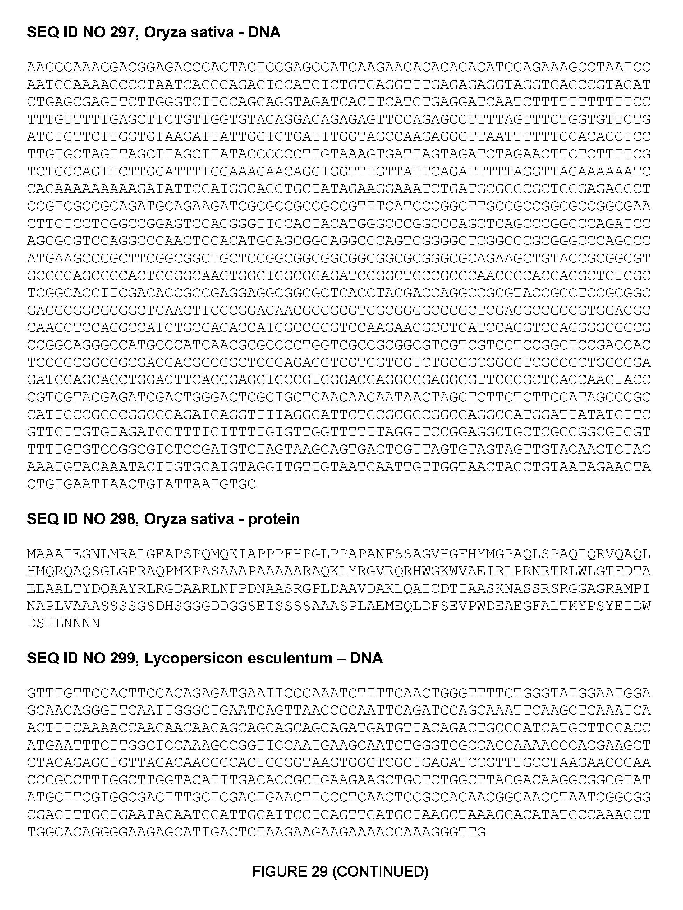

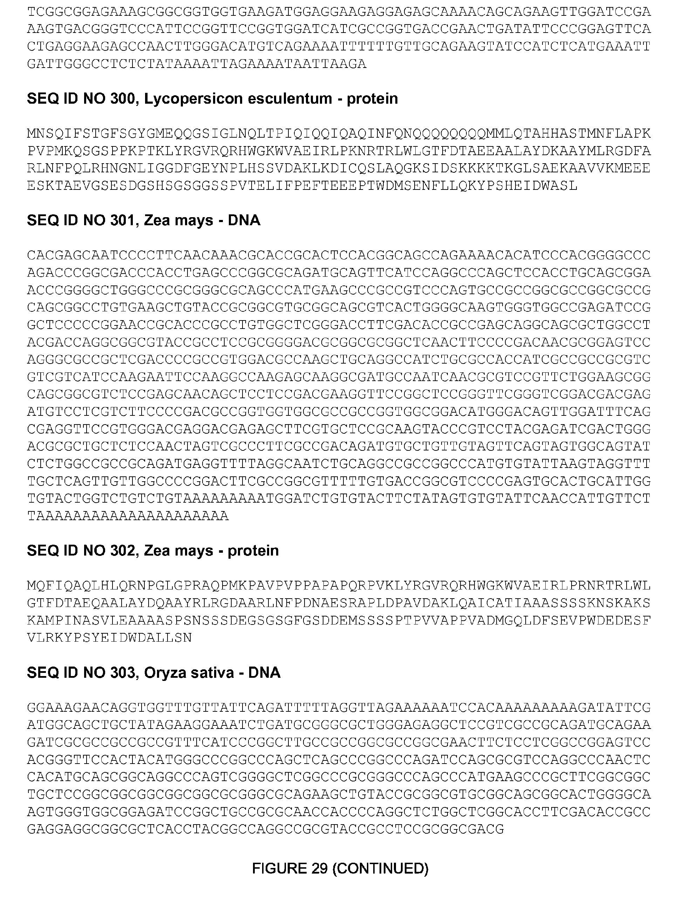

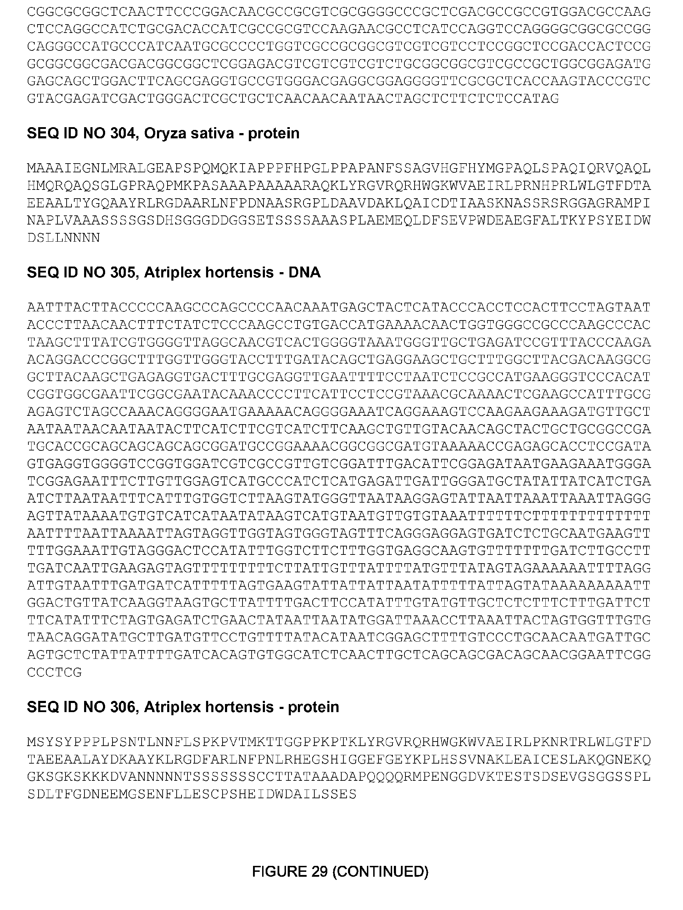

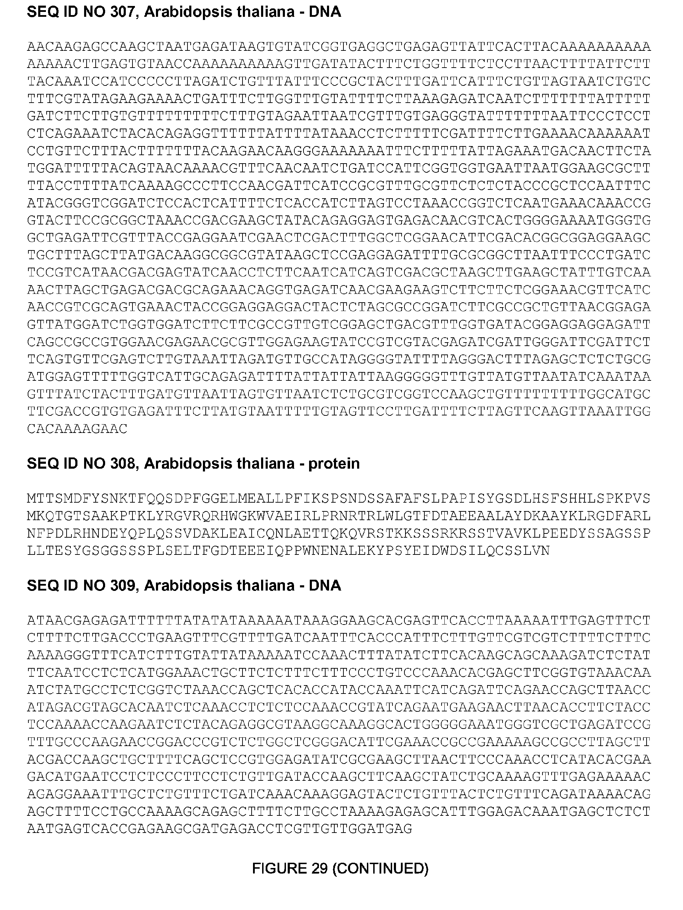

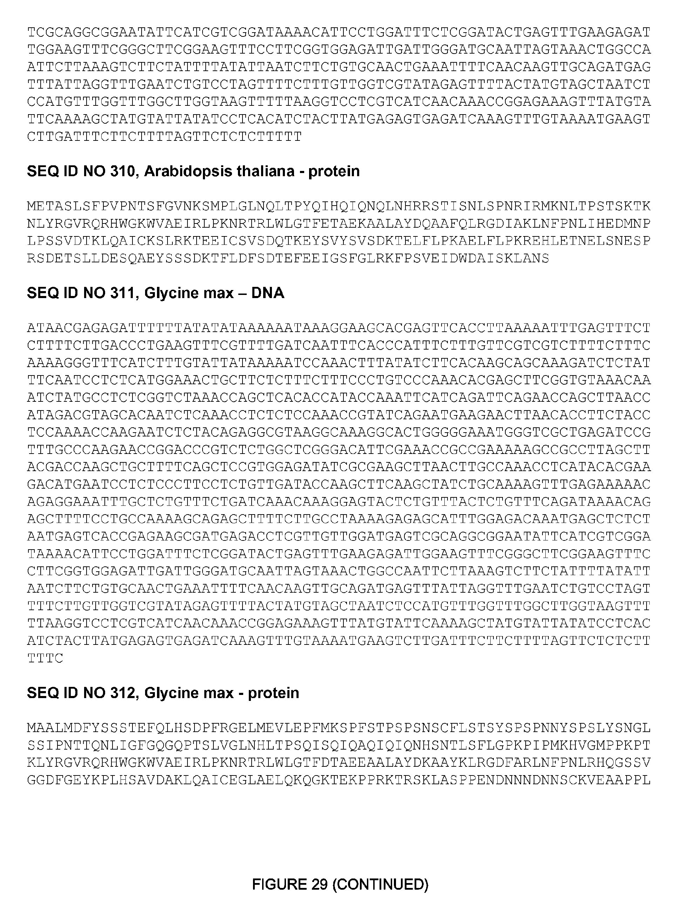

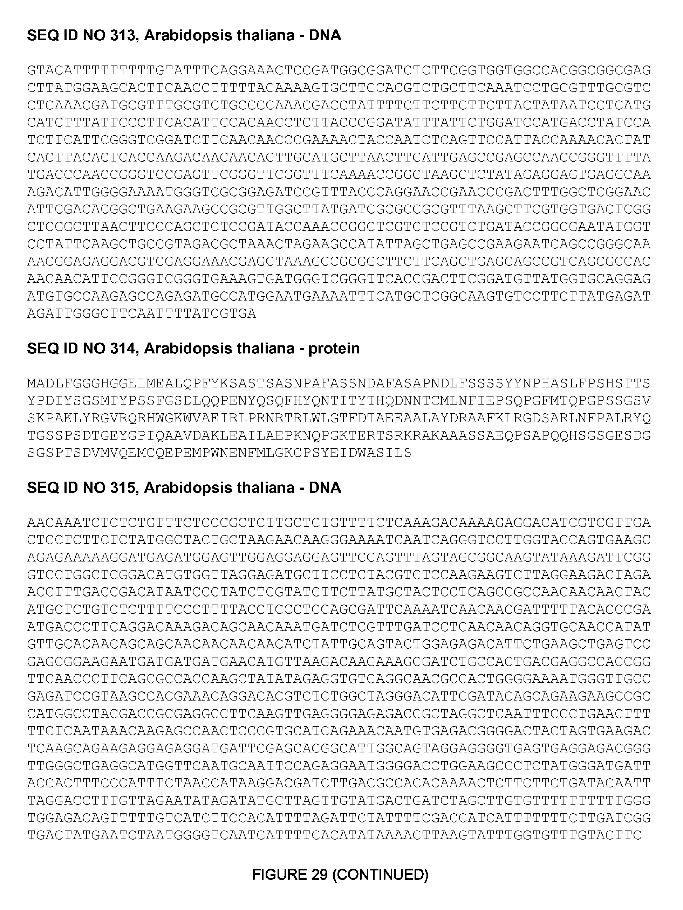

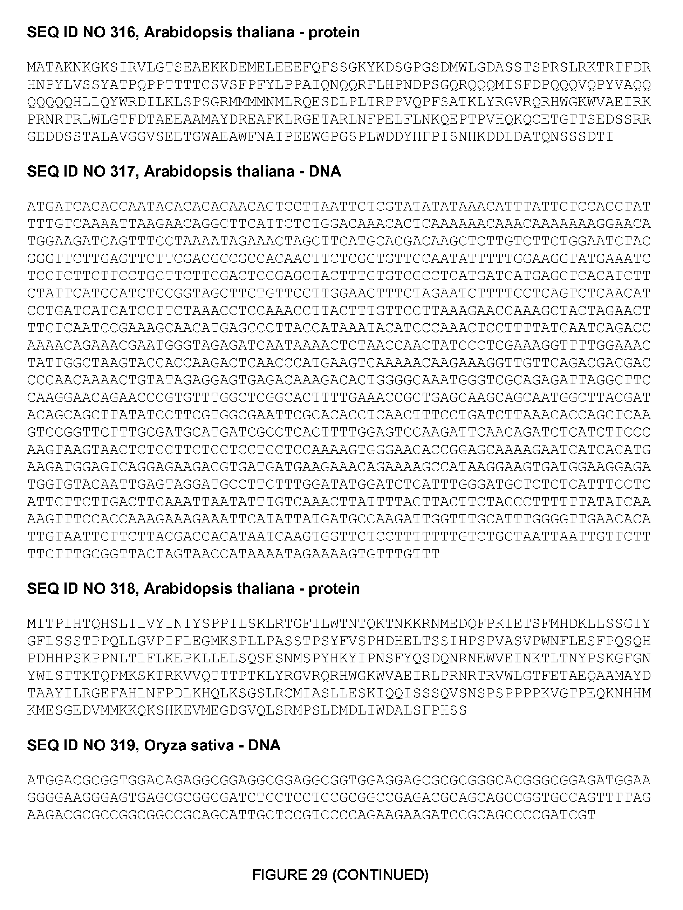

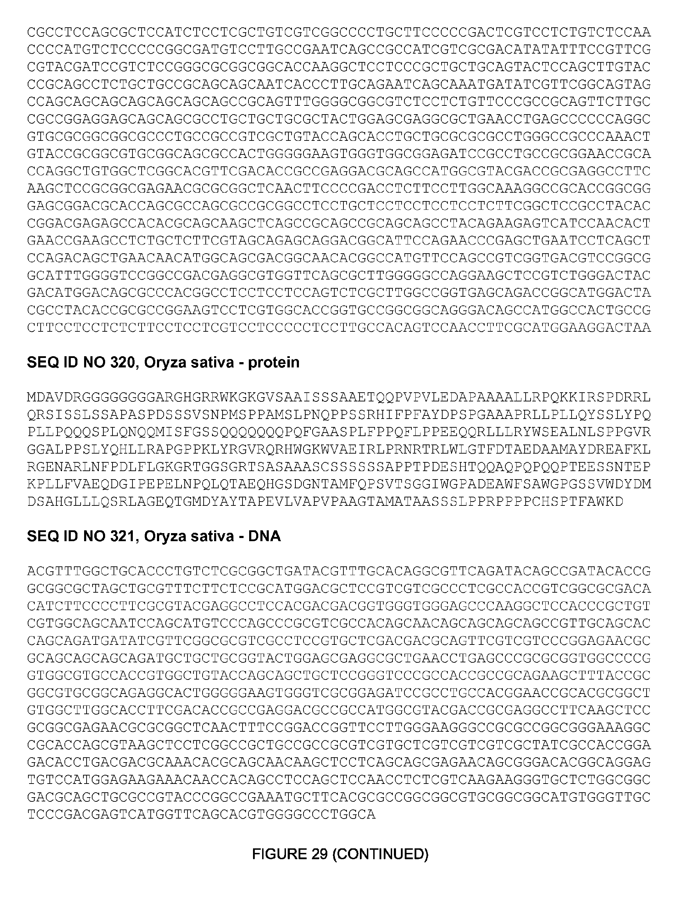

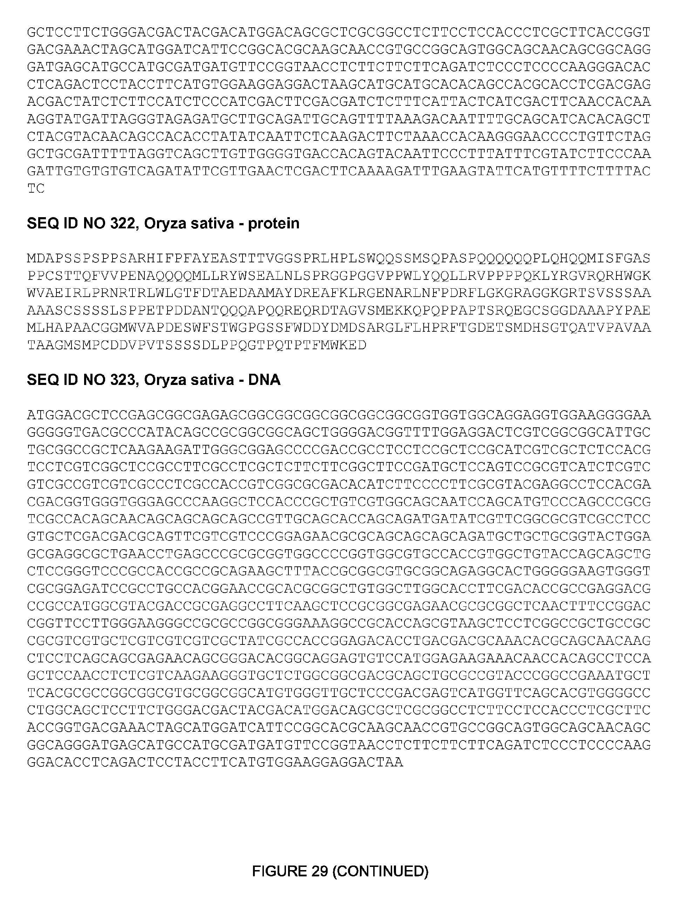

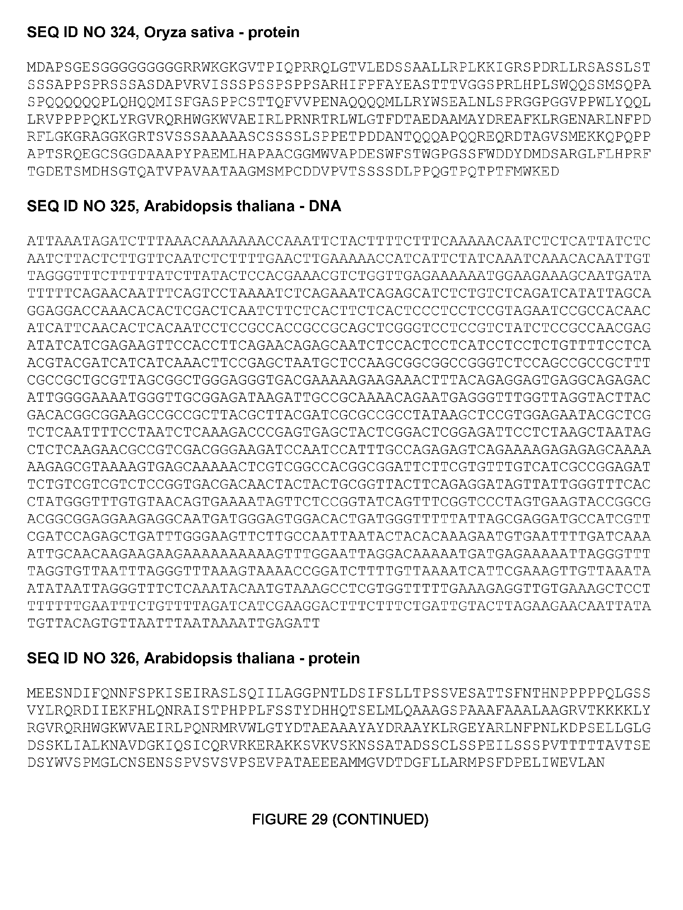

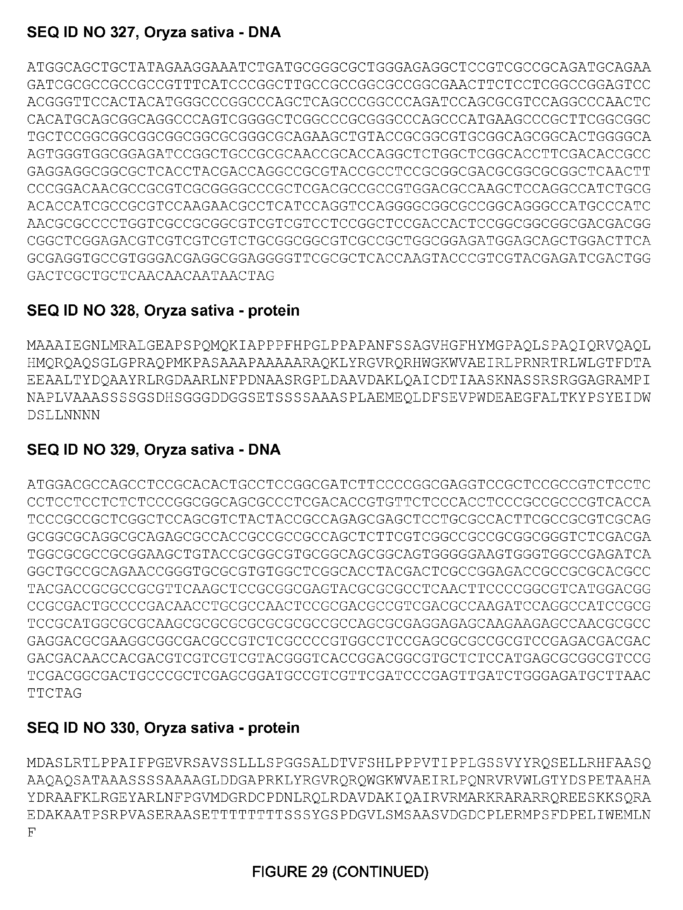

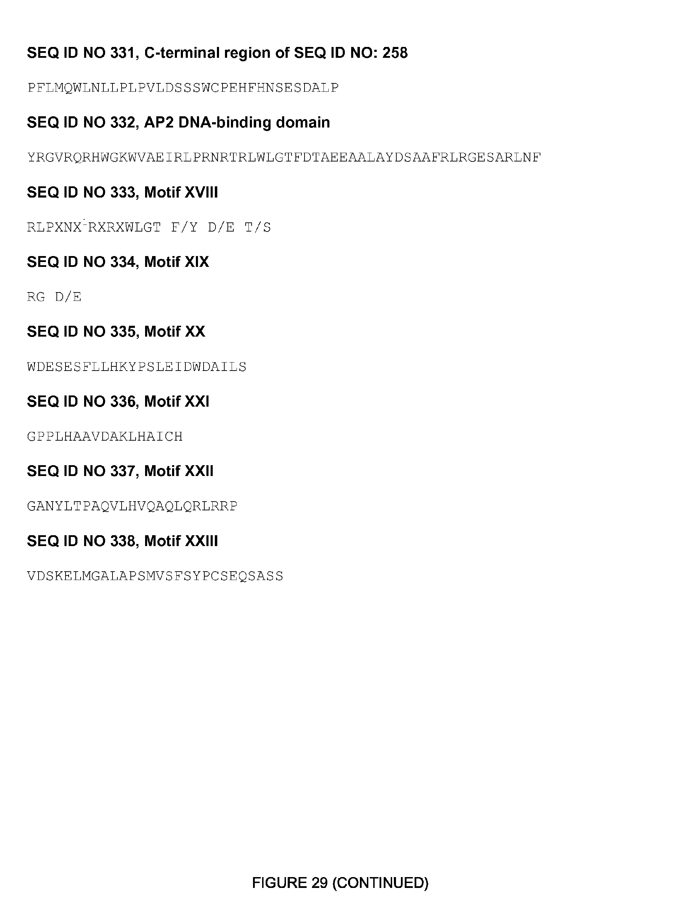

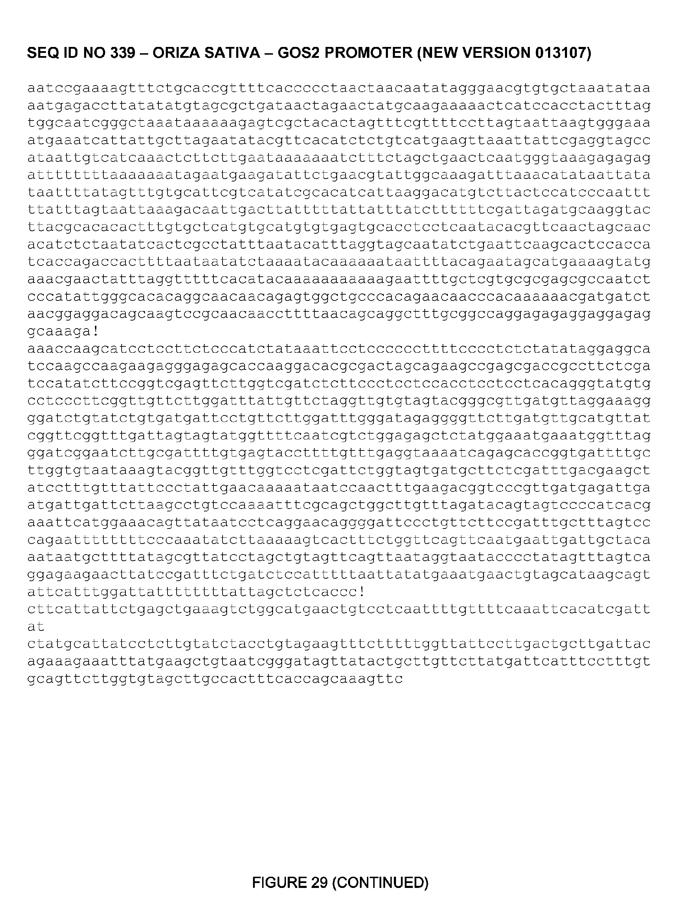

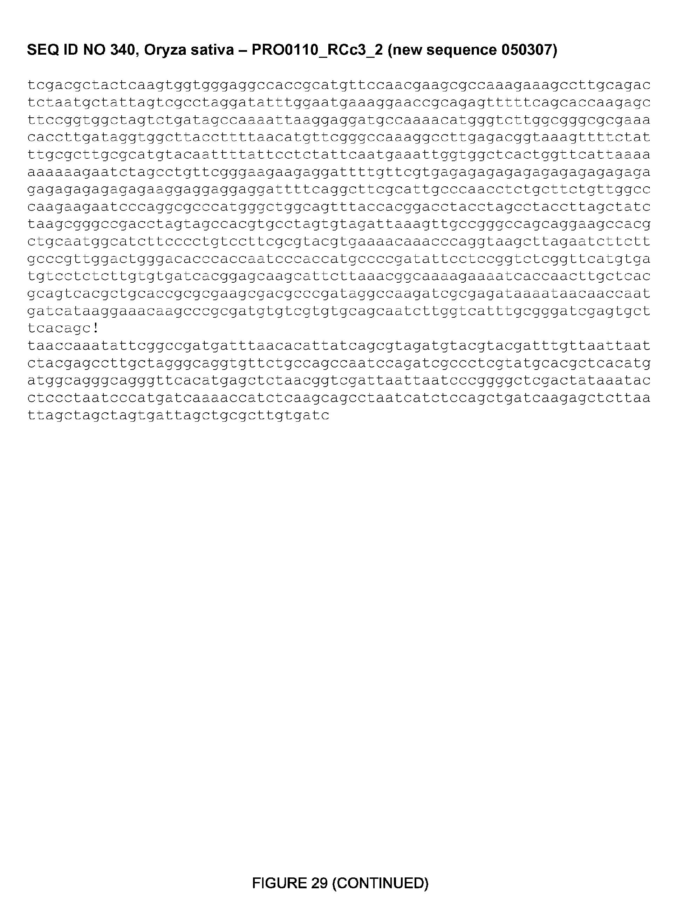

**Figs. 26 to 29** details examples of sequences useful in performing the methods according to the present invention.

**Examples**

The present invention will now be described with reference to the following examples, which are by way of illustration alone. The following examples are not intended to completely define or otherwise limit the scope of the invention.

**C) AP2-2 polypeptide**

***Example 9: Identification of sequences related to SEQ ID NO: 131 and SEQ ID NO: 132***

Sequences (full length cDNA, ESTs or genomic) related to SEQ ID NO: 131 and/or protein sequences related to SEQ ID NO: 132 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program was used to find regions of local similarity between sequences by comparing nucleic acid or polypeptide sequences to sequence databases and by calculating the statistical significance of matches. The polypeptide encoded by SEQ ID NO: 131 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search.

Table 14 provides a list of nucleic acid and protein sequences related to the nucleic acid sequence as represented by SEQ ID NO: 131 and the protein sequence represented by SEO ID NO: 132.

**Table 14**: Nucleic acid sequences encoding AP2-2 polypeptides and AP2-2 polypeptides.

| Name/identifier | Source organism | Nucleic acid SEQ ID NO: | Poly-peptide SEQ ID NO: |
|---|---|---|---|
| AP2-EREBP | *Oryza sativa* | 131 | 132 |
| Os07g47790 | *Oryza sativa* | 139 | 140 |
| AAX13280 | *Triticum aestivum* | 141 | 142 |
| Os01g21120 | *Oryza sativa* | 143 | 144 |
| Os03g08470 | *Oryza sativa* | 145 | 146 |
| CAD56466 | *Triticum aestivum* | 147 | 148 |
| AT2G47520.1 | *Arabidopsis thaliana* | 149 | 150 |
| Os02g54160 | *Oryza sativa* | 151 | 152 |
| Os09g26420 | *Oryza sativa* | 153 | 154 |
| AAP32468 | *Triticum aestivum* | 155 | 156 |
| AAP32467 | *Triticum aestivum* | 157 | 158 |
| AT1G53910.1 | *Arabidopsis thaliana* | 159 | 160 |
| AAM65746 | *Arabidopsis thaliana* | 161 | 162 |
| AT3G14230.1 | *Arabidopsis thaliana* | 163 | 164 |
| AT3G14230.2 | *Arabidopsis thaliana* | 165 | 166 |
| AT3G14230.3 | *Arabidopsis thaliana* | 167 | 168 |
| AT3G16770 | *Arabidopsis thaliana* | 169 | 170 |
| Os07g42510 | *Oryza sativa* | 171 | 172 |
| Os03g08490 | *Oryza sativa* | 173 | 174 |
| Os03g08500 | *Oryza sativa* | 175 | 176 |

(continued)

| Name/identifier | Source organism | Nucleic acid SEQ ID NO: | Poly-peptide SEQ ID NO: |
|---|---|---|---|
| AT1G72360 | *Arabidopsis thaliana* | 177 | 178 |
| Os03g08460 | *Oryza sativa* | 179 | 180 |
| Os05g29810 | *Oryza sativa* | 181 | 182 |
| Os03g22170 | *Oryza sativa* | 183 | 184 |
| Os10g25170 | *Oryza sativa* | 185 | 186 |
| Os09g11460 | *Oryza sativa* | 187 | 188 |
| AAK95687 | *Lycopersicon esculentum* | 189 | 190 |
| Os09g11480 | *Oryza sativa* | 191 | 192 |
| AAQ91334 | *Lycopersicon esculentum* | 193 | 194 |
| AAR87866 | *Lycopersicon esculentum* | 195 | 196 |
| AT1G80580 | *Arabidopsis thaliana* | 197 | 198 |
| AAP40022 | *Nicotiana tabacum* | 199 | 200 |
| CAE54591 | *Fagus sylvatica* | 201 | 202 |
| CAD21849 | *Fagus sylvatica* | 203 | 204 |
| ABD65407 | *Capsicum annuum* | 205 | 206 |
| AAP72289 | *Capsicum annuum* | 207 | 208 |
| AAS20427 | *Capsicum annuum* | 209 | 210 |
| AAX07458 | *Gossypium hirsutum* | 211 | 212 |
| AAT77192 | *Gossypium barbadense* | 213 | 214 |
| AAX20013 | *Gossypium hirsutum* | 215 | 216 |
| AAX68526 | *Gossypium hirsutum* | 217 | 218 |
| AAX68525 | *Gossypium hirsutum* | 219 | 220 |
| AAT77191 | *Gossypium barbadense* | 221 | 222 |
| AAV51937 | *Gossypium hirsutum* | 223 | 224 |
| AAV85777 | *Gossypium hirsutum* | 225 | 226 |
| AAX07460 | *Gossypium hirsutum* | 227 | 228 |
| AAX84670 | *Manihot esculenta* | 229 | 230 |
| AAW33881 | *Populus alba x Populus tremula* | 231 | 232 |
| BAE71206 | *Trifolium pratense* | 233 | 234 |
| AAQ10777 | *Glycine max* | 235 | 236 |
| AAL67489 | *Narcissus pseudonarcissus* | 237 | 238 |
| CAA05084 | *Arabidopsis thaliana* | 239 | 240 |
| ABE84970 | *Medicago truncatula* | 241 | 242 |
| ABE80536 | *Medicago truncatula* | 243 | 244 |
| AAC29516 | *Solanum tuberosum* | 245 | 246 |
| BAC56862 | *Solanum tuberosum* | 247 | 248 |
| AAS01337 | *Coffea canephora* | 249 | 250 |
| AAZ14085 | *Hordeum vulgare* | 251 | 252 |

(continued)

| Name/identifier | Source organism | Nucleic acid SEQ ID NO: | Poly-peptide SEQ ID NO: |
|---|---|---|---|
| BAD01556 | *Cucumis melo* | 253 | 254 |
| BAF43419 | *Malus x domestica* | 255 | 256 |

***Example 10: Alignment ofAP2-2 polypeptide sequences***

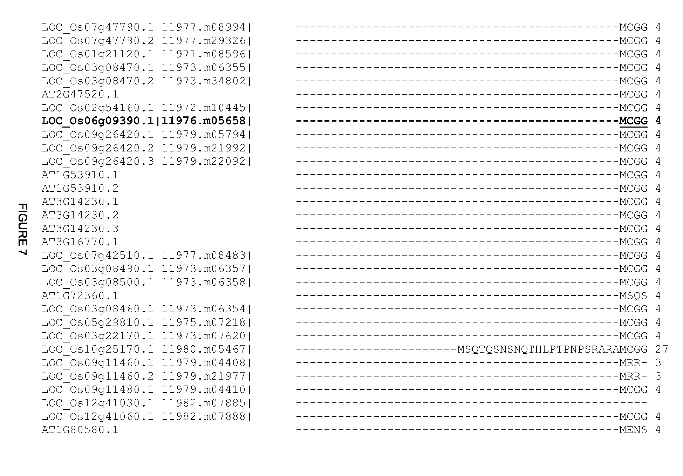

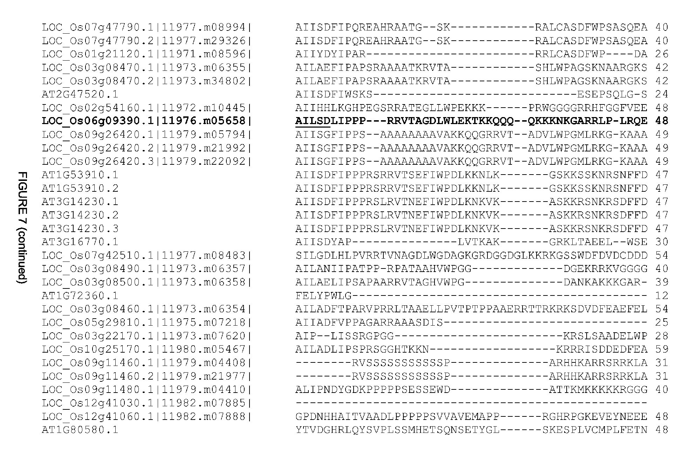

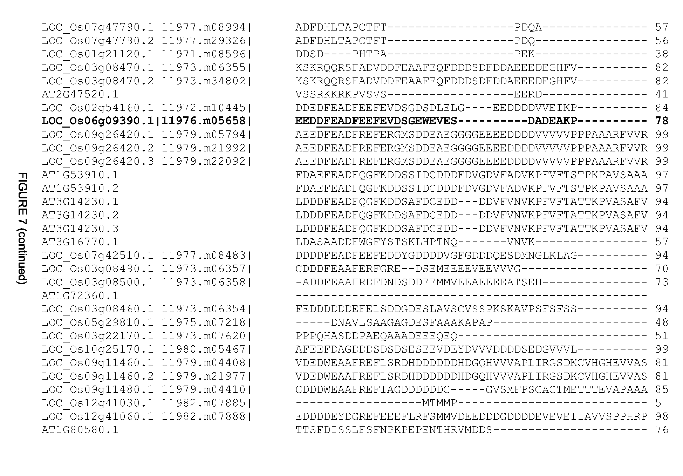

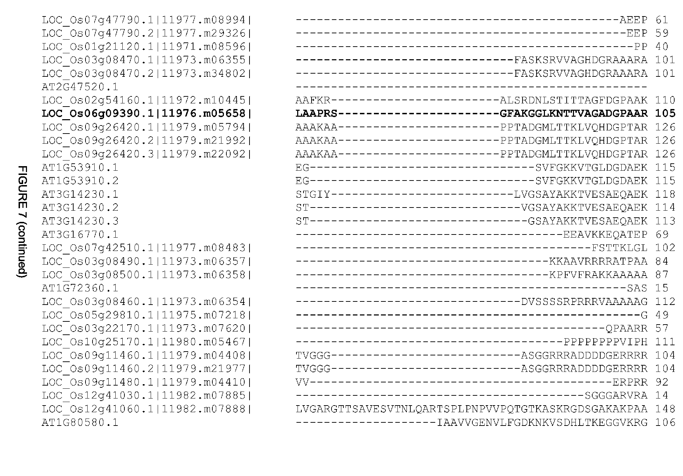

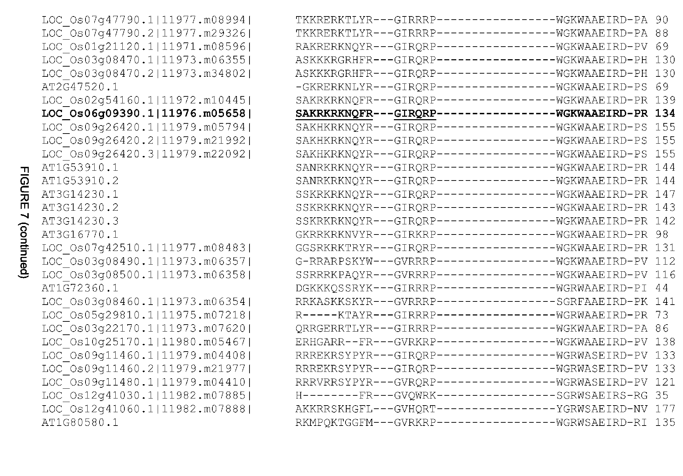

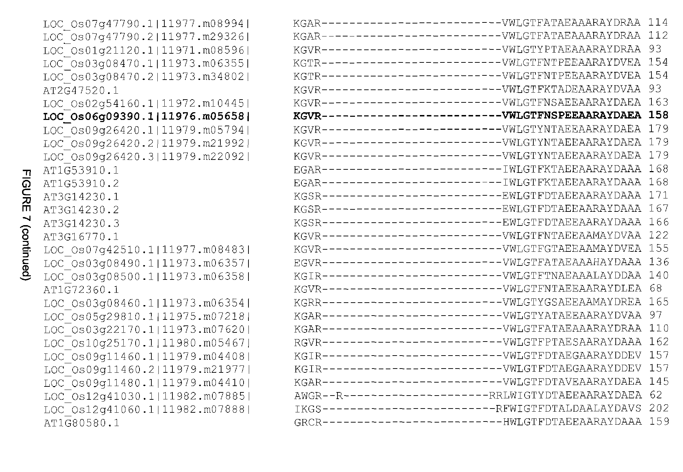

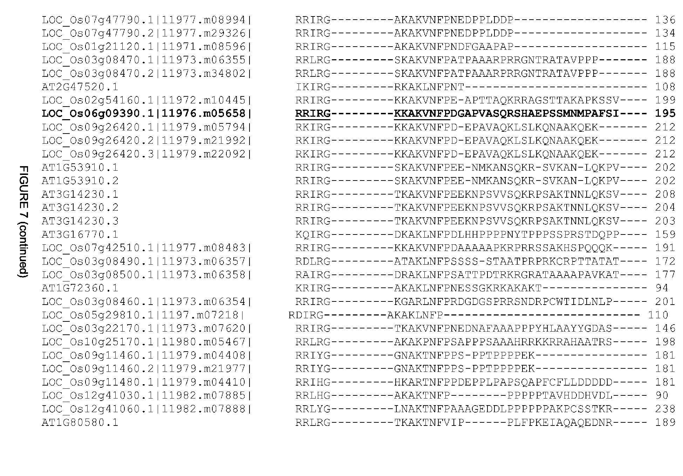

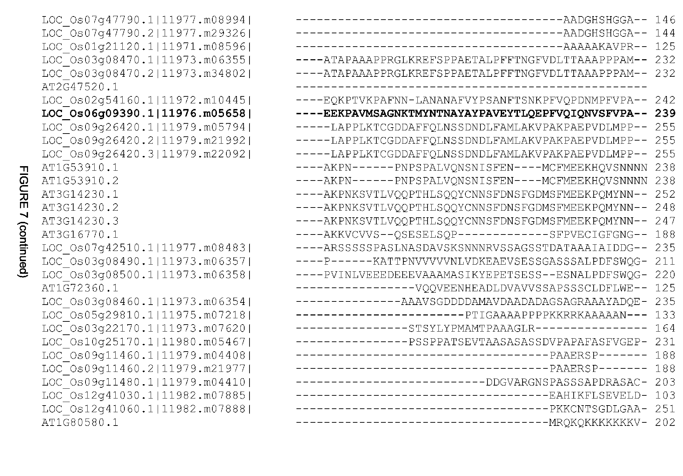

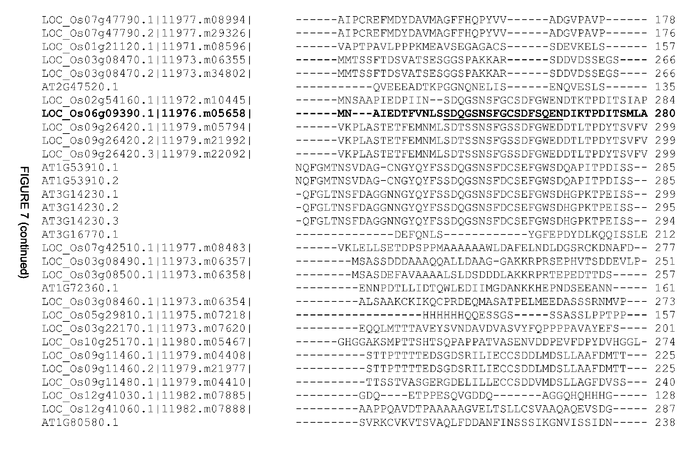

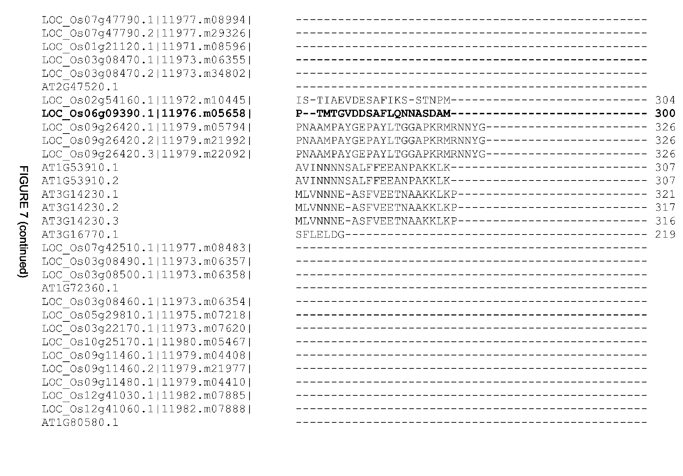

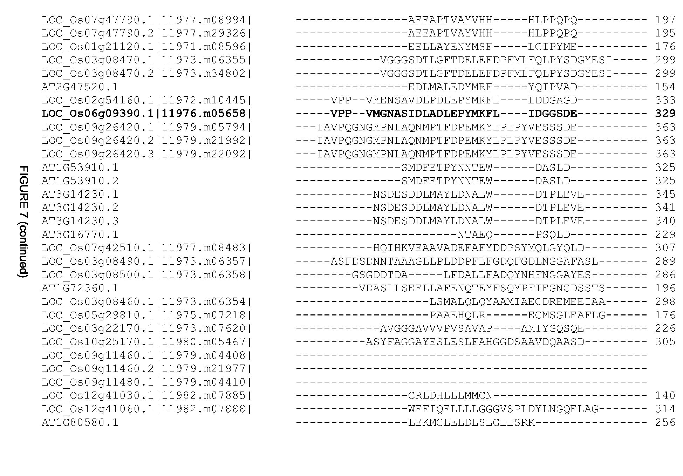

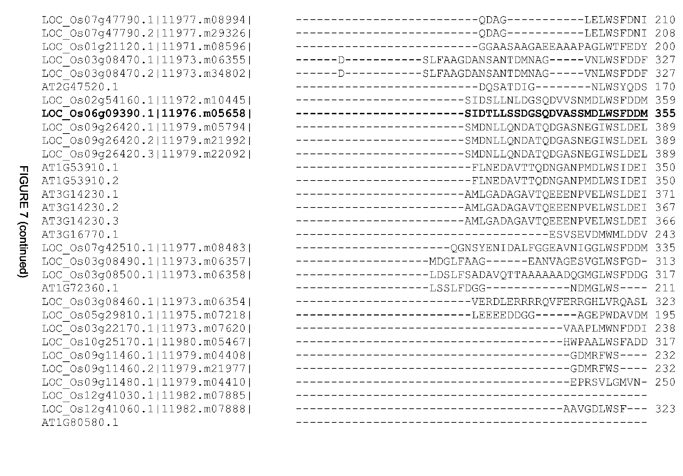

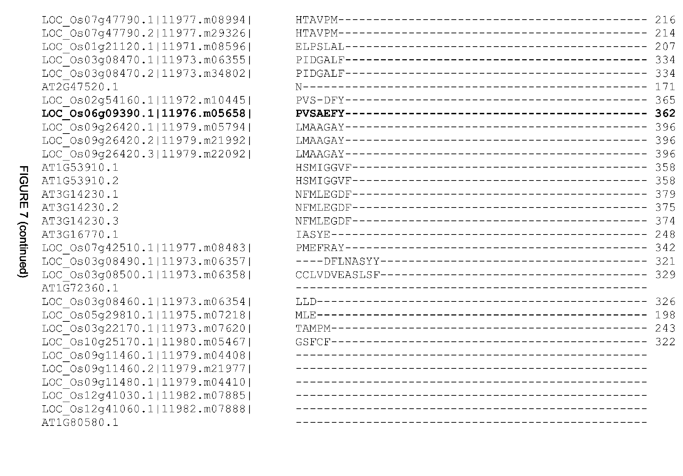

Alignment of polypeptide sequences was performed using the AlignX programme from the Vector NTI (Invitrogen) which is based on the popular Clustal algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Results in Figure 7 show that AP2-2 polypeptides share regions of high sequence conservation. Motifs XII to XVII are underlined for Os06g09390 (SEQ ID NO: 132).

***Example 11: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

**Scoring matrix: Blosum62**

First Gap: 12

Extending gap: 2

Results of the software analysis are shown in Table 15 for the global similarity and identity over the full length of the polypeptide sequences (excluding the partial polypeptide sequences). Percentage identity is given above the diagonal and percentage similarity is given below the diagonal.

The percentage identity between the full length polypeptide sequences useful in performing the methods of the invention can be as low as 20 % amino acid identity compared to SEQ ID NO: 132. The sequence identity will be higher when specific domains, such as the AP2 domain are compared.

**Table 15**: MatGAT results for global similarity and identity over the full length of the polypeptide sequences.

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| 1. SEQID 132 | | 27.7 | 63.3 | 29.8 | 38.3 | 62.4 | 24 | 59.5 | 40.8 | 61.1 | 58.2 | 37.2 | 37.2 | 37.1 | 37 |
| 2. SEQID 140 | 37.8 | | 27.2 | 43.9 | 29.7 | 26.6 | 36.4 | 27.5 | 26.5 | 26.2 | 25.7 | 29.1 | 29.1 | 27.4 | 27.7 |
| 3. SEQID 142 | 76.5 | 35.8 | | 29.9 | 33.1 | 90.8 | 24.5 | 55.9 | 39 | 94.6 | 88.5 | 35.9 | 35.9 | 36.7 | 37.3 |
| 4. SEQID 144 | 37.6 | 55.6 | 35.5 | | 31.1 | 30.3 | 41.1 | 27.6 | 25 | 28.7 | 29.2 | 27.7 | 27.9 | 27.2 | 27.5 |
| 5. SEQID 146 | 50.6 | 37.7 | 48.2 | 39.5 | | 32 | 24.9 | 36.1 | 33.2 | 30.7 | 30.2 | 34.3 | 34.3 | 32.1 | 32.4 |
| 6. SEQID 148 | 74.9 | 35.2 | 94.4 | 35.2 | 47 | | 25.2 | 56.5 | 38.6 | 87.4 | 88 | 36.2 | 36.2 | 37.6 | 37.9 |
| 7. SEQID 150 | 31.8 | 48.1 | 32.1 | 52.7 | 34.1 | 32.1 | | 23.6 | 22.2 | 23.9 | 24.7 | 26.3 | 26.3 | 24.8 | 25.3 |
| 8. SEQID 152 | 74.8 | 35.1 | 69.9 | 34.2 | 47.1 | 69 | 31.2 | | 42.7 | 53.5 | 53.6 | 39.9 | 39.9 | 36.1 | 36.3 |
| 9. SEQID 154 | 54 | 33.6 | 51.5 | 32.6 | 45.7 | 51 | 28 | 56.1 | | 37.3 | 36.1 | 36.8 | 36.8 | 35 | 35.3 |
| 10. SEQID 156 | 73.2 | 33.8 | 96.1 | 34.6 | 46.5 | 91.3 | 31.5 | 67.1 | 50 | | 85.1 | 34.8 | 35.1 | 35.9 | 36.3 |
| 11. SEQID 158 | 70.7 | 34.6 | 89.9 | 36.1 | 45.8 | 90 | 34 | 66.6 | 48 | 86.8 | | 32.5 | 32.5 | 34 | 34.4 |
| 12. SEQID 160 | 53.3 | 38.5 | 54.2 | 35.8 | 48 | 52.5 | 33 | 53.7 | 50 | 53.6 | 49.2 | | 99.4 | 62.2 | 62.8 |
| 13. SEQID 162 | 53.3 | 38.5 | 54.2 | 35.8 | 48 | 52.5 | 33 | 53.7 | 50 | 53.6 | 49.2 | 100 | | 62.2 | 62.8 |
| 14. SEQID 164 | 56.7 | 34.3 | 54.4 | 33.5 | 47.2 | 54.6 | 29.6 | 55.4 | 49.7 | 54.1 | 50.1 | 70.7 | 71 | | 98.9 |
| 15. SEQID 166 | 57.1 | 34.7 | 54.7 | 33.9 | 47.7 | 54.9 | 30.4 | 55.7 | 49.7 | 54.7 | 50.7 | 71.7 | 72 | 98.9 | |
| 16. SEQID 168 | 57.2 | 34.8 | 54.8 | 34 | 47.9 | 55.1 | 30.2 | 55.6 | 51 | 54.8 | 50.8 | 71.9 | 72.2 | 98.7 | 99.7 |
| 17. SEQID 170 | 41.2 | 47.2 | 38.9 | 46.4 | 41.3 | 38.4 | 46.8 | 38.6 | 36.9 | 37.5 | 38.3 | 40.8 | 40.8 | 39.6 | 40 |
| 18. SEQID 172 | 51.9 | 34.8 | 52.1 | 38.9 | 52 | 54.4 | 32.2 | 50.4 | 43.9 | 49.9 | 52.9 | 46.6 | 46.6 | 44.3 | 43.7 |
| 19. SEQID 174 | 46.4 | 34.9 | 43.4 | 40.8 | 51.2 | 46.7 | 31.5 | 48.2 | 40.9 | 42.8 | 44 | 43.3 | 43.6 | 42.2 | 42.7 |
| 20. SEQID 176 | 50 | 38.6 | 49.6 | 40.1 | 52.7 | 49 | 32.8 | 48.2 | 44.7 | 47.6 | 45.5 | 48 | 48 | 44.6 | 45.1 |
| 21. SEQID 178 | 41.7 | 46.9 | 44.5 | 47.7 | 47 | 45 | 44.3 | 42.5 | 39.6 | 42.5 | 44.6 | 45 | 45.3 | 39.6 | 40.3 |
| 22. SEQID 180 | 47.5 | 36.5 | 40.8 | 35.9 | 44.9 | 42.1 | 32.8 | 44.1 | 38.1 | 39.4 | 42.8 | 46.6 | 46.6 | 42.7 | 42.4 |
| 23. SEQID 182 | 32.3 | 50 | 32.7 | 50.7 | 36.8 | 33.2 | 47 | 32.1 | 31.1 | 31.5 | 32.2 | 31.8 | 31.8 | 32.5 | 32.8 |
| 24. SEQID 184 | 39 | 55.1 | 37.2 | 53.5 | 43.1 | 39.8 | 44.9 | 37.3 | 36.9 | 36.6 | 39.8 | 36.3 | 36.3 | 34.3 | 34.7 |
| 25. SEQID 186 | 40.6 | 35.7 | 42 | 41.3 | 50.6 | 42.1 | 32 | 44.4 | 42.7 | 42.5 | 40.7 | 39.7 | 39.7 | 40.1 | 39.5 |

(continued)

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| **26. SEQID 188** | 31.8 | 37.9 | 34.1 | 41.4 | 39.2 | 33.8 | 38.4 | 32.1 | 33.1 | 33 | 35.8 | 34.1 | 34.1 | 33 | 33.6 |
| **27. SEQID 190** | 60.2 | 34.1 | 59.1 | 36.8 | 48.1 | 59.4 | 33.6 | 58.9 | 54.3 | 57.5 | 54.6 | 59.7 | 59.7 | 60.7 | 61.3 |
| **28. SEQID 192** | 39.5 | 41.2 | 38 | 42 | 38.6 | 39.8 | 38 | 38.9 | 37.4 | 36.9 | 39.5 | 39.4 | 39.4 | 38 | 39.5 |
| **29. SEQID 194** | 53.9 | 37 | 54.6 | 38.2 | 47.9 | 55.3 | 34.9 | 50.1 | 46.7 | 53.2 | 53.6 | 57.5 | 57.5 | 58.3 | 57.6 |
| **30. SEQID 196** | 44.2 | 48.8 | 44.5 | 51.2 | 41.3 | 43.8 | 49.2 | 40.5 | 37.6 | 41.4 | 41.9 | 39.1 | 39.1 | 41.2 | 40 |
| **31. SEQID 198** | 29.6 | 35.5 | 33.5 | 34 | 33.2 | 32.7 | 32 | 32.3 | 29.5 | 34.1 | 35.2 | 33.8 | 33.8 | 31.1 | 31.5 |
| **32. SEQID 200** | 56.6 | 32.8 | 55.6 | 37 | 49.9 | 55.3 | 33.3 | 56.1 | 52.3 | 54 | 51.4 | 61.5 | 61.5 | 62.8 | 62.8 |
| **33. SEQID 202** | 56.6 | 34.9 | 57.4 | 37.6 | 49.7 | 57.9 | 31 | 60.8 | 56.8 | 55.3 | 53.4 | 63.8 | 64 | 64.1 | 64.3 |
| **34. SEQID 204** | 56.5 | 34.1 | 57.6 | 37.6 | 49.3 | 57.6 | 30.7 | 60.5 | 55.1 | 56.3 | 53.3 | 62.7 | 62.9 | 62.8 | 63.5 |
| **35. SEQID 206** | 60 | 34.1 | 57.9 | 36.5 | 50.4 | 58.9 | 32.5 | 57.6 | 52.5 | 56.5 | 53.3 | 59.5 | 59.5 | 59.6 | 60.3 |
| **36. SEQID 208** | 59.1 | 32.8 | 56.6 | 35.2 | 49.3 | 57.7 | 32 | 56.6 | 51.3 | 55.3 | 52 | 58.5 | 58.5 | 58.6 | 59.2 |
| **37. SEQID 210** | 44.2 | 48.5 | 43.4 | 52.3 | 42.8 | 43.3 | 45.8 | 42.7 | 39.6 | 40.8 | 42.5 | 43.6 | 43.6 | 41.7 | 41.3 |
| **38. SEQID 212** | 55.4 | 34.9 | 51.8 | 34.6 | 50 | 53.3 | 33.1 | 57.4 | 57.6 | 50 | 47.7 | 64.6 | 64.9 | 65.6 | 65.4 |
| **39. SEQID 214** | 50.3 | 37 | 49.6 | 38.6 | 46.1 | 51.3 | 37 | 48.8 | 44.9 | 47.3 | 52.1 | 54.2 | 54.5 | 53.6 | 54.1 |
| **40. SEQID 216** | 53.5 | 30.8 | 50.8 | 34.1 | 49 | 51.8 | 33.1 | 52.8 | 55.6 | 49.2 | 48.2 | 59.8 | 60.1 | 59.8 | 59.6 |
| **41. SEQID 218** | 39 | 43.3 | 40 | 45.6 | 41.6 | 37.2 | 42.1 | 40 | 37.9 | 37.7 | 41 | 39.9 | 39.7 | 39.3 | 38.4 |
| **42. SEQID 220** | 38.7 | 45.3 | 42 | 47.3 | 42.5 | 40.4 | 46.5 | 37.5 | 40.7 | 37.2 | 40.4 | 40.5 | 40.5 | 40.4 | 40 |
| **43. SEQID 222** | 32.6 | 47.7 | 32.7 | 49.3 | 35.3 | 33 | 51.5 | 32.9 | 31.3 | 30.4 | 31.3 | 33.2 | 33.2 | 33.5 | 34.7 |
| **44. SEQID 224** | 39.2 | 45.9 | 40.8 | 45.1 | 40.4 | 39.5 | 45.9 | 38.9 | 39.4 | 39.4 | 37.7 | 41.1 | 41.1 | 38.8 | 38.9 |
| **45. SEQID 228** | 40.6 | 43.5 | 39.4 | 45.4 | 41.9 | 40.1 | 42.4 | 40.8 | 38.9 | 37.5 | 39.8 | 39.1 | 39.1 | 39.8 | 40.5 |
| **46. SEQID 230** | 58 | 34.1 | 53.5 | 38.1 | 48 | 57 | 32 | 59.3 | 59.3 | 51.7 | 53.5 | 64.8 | 64.8 | 64.6 | 64.6 |
| **47. SEQID 232** | 56.6 | 33.9 | 51.3 | 36.1 | 47.4 | 51.6 | 31.8 | 56.1 | 53.8 | 52.1 | 49.2 | 60.3 | 60 | 61.3 | 61.1 |
| **48. SEQID 234** | 56.9 | 35.1 | 52.7 | 35.3 | 48.1 | 53.2 | 31.7 | 53.8 | 54.5 | 50.9 | 50.6 | 57.4 | 57.4 | 61 | 61.3 |
| **49. SEQID 236** | 55.7 | 35.2 | 52.9 | 35.4 | 45.8 | 53.1 | 33.3 | 57 | 56.8 | 49.7 | 50.8 | 55.7 | 56 | 59.4 | 59.1 |
| **50. SEQID 240** | 40.6 | 46.3 | 42.5 | 47.2 | 40.4 | 41.3 | 46.3 | 39.5 | 36.9 | 39.4 | 41.3 | 39.7 | 39.7 | 39.8 | 40.3 |

(continued)

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| **51. SEQID 242** | 42.5 | 40.7 | 44.8 | 43.9 | 40.7 | 44.7 | 43 | 42.2 | 37.6 | 43.1 | 43.4 | 44.1 | 44.1 | 43 | 43.7 |
| **52. SEQID 244** | 33.4 | 40.8 | 29.6 | 38.3 | 32.9 | 32.1 | 41.7 | 32.9 | 27.8 | 28.7 | 31.9 | 35.2 | 35.5 | 32.5 | 32.8 |
| **53. SEQID 246** | 46.1 | 42.6 | 41.4 | 47.3 | 42.8 | 41.3 | 41.6 | 41.6 | 39.4 | 41.7 | 40.4 | 41.6 | 41.1 | 40.6 | 40.8 |
| **54. SEQID 248** | 42.3 | 46.6 | 40.6 | 45.1 | 42.2 | 40.7 | 43.6 | 37.8 | 37.9 | 39.2 | 39.8 | 42.2 | 42.2 | 41.2 | 41.1 |
| **55. SEQID 250** | 43.4 | 40.1 | 44.8 | 43.2 | 48.5 | 45.6 | 37.7 | 45.5 | 42.7 | 43.7 | 44.3 | 43 | 43 | 42.5 | 42.9 |
| **56. SEQID 252** | 46.7 | 38.4 | 47.3 | 39.6 | 60.5 | 47.9 | 34.1 | 47.9 | 42.4 | 45.9 | 48.5 | 44.7 | 45.8 | 42.5 | 42.9 |
| **57. SEQID 254** | 43.4 | 49.5 | 44.2 | 51.6 | 44.9 | 43.8 | 46.2 | 40.5 | 40.2 | 42.5 | 44 | 43 | 43 | 40.1 | 40 |
| **58. SEQID 256** | 36.2 | 44.8 | 38.3 | 45.6 | 40.4 | 39.5 | 45.2 | 36.4 | 36.6 | 36.3 | 38.9 | 38.3 | 38.3 | 38 | 38.7 |

| | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|
| **1. SEQID 132** | **37.6** | **29.6** | **38.3** | **30.1** | **34** | **26.5** | **29.6** | **26** | **27.2** | **27.9** | **23.7** | **41.4** | **27.7** | **38.4** | **32.2** |
| **2. SEQID 140** | **27.8** | **34.1** | **28.7** | **27.7** | **27.6** | **34.1** | **26.1** | **40.3** | **45.8** | **28.5** | **26.6** | **26.9** | **32.5** | **28.7** | **36.7** |
| **3. SEQID 142** | **37.4** | **28.5** | **35.2** | **30.7** | **32.4** | **29.1** | **26.6** | **25.8** | **28.2** | **27.8** | **23.5** | **40.1** | **25.3** | **36.7** | **32.8** |
| **4. SEQID 144** | **27.5** | **35.9** | **30.6** | **32** | **31.3** | **33** | **29.8** | **41.6** | **39.3** | **31.4** | **28.8** | **29.4** | **30.2** | **31.2** | **43.5** |
| **5. SEQID 146** | **32.5** | **29.3** | **36.3** | **35.7** | **37.5** | **29.5** | **30.2** | **27.5** | **30.3** | **35.9** | **27.6** | **35.4** | **29.7** | **35.4** | **30.3** |
| **6. SEQID 148** | **38** | **28.4** | **36.9** | **31.6** | **32** | **30.3** | **26.9** | **26.9** | **28.7** | **29.3** | **23.5** | **40.9** | **26.5** | **38** | **33.3** |
| **7. SEQID 150** | **25.7** | **33.3** | **25.1** | **24** | **24.6** | **31.7** | **21.8** | **36.7** | **31** | **24.2** | **26.9** | **28** | **28** | **25.4** | **38.1** |
| **8. SEQID 152** | **36.4** | **28.3** | **37.2** | **32** | **31.6** | **30.1** | **27.9** | **25.8** | **28.6** | **32.2** | **23.4** | **42** | **28.7** | **37.5** | **31.1** |
| **9. SEQID 154** | **35.7** | **25.3** | **31.7** | **30.1** | **31** | **26.2** | **23.8** | **24.7** | **28.8** | **28.1** | **23.6** | **37.8** | **26.2** | **33.6** | **27.5** |
| **10. SEQID 156** | **36.4** | **27.3** | **33.2** | **30.2** | **31.4** | **27.7** | **24.6** | **24.7** | **24.5** | **26.2** | **22.4** | **39** | **24.2** | **35.3** | **30.5** |
| **11. SEQID 158** | **34.5** | **28** | **33.7** | **29.3** | **28.3** | **29.1** | **25.3** | **25.8** | **27.8** | **26.6** | **23.6** | **37.1** | **25.9** | **34.9** | **30.1** |
| **12. SEQID 160** | **63** | **28.5** | **32.4** | **28** | **30.2** | **31.3** | **27.3** | **24.9** | **25.7** | **27.2** | **24.9** | **43.4** | **29.1** | **42.9** | **28.9** |
| **13. SEQID 162** | **63** | **28.5** | **32.4** | **28.2** | **30.2** | **31** | **27.3** | **24.9** | **25.7** | **27.2** | **24.7** | **43.7** | **29.1** | **42.9** | **28.9** |
| **14. SEQID 164** | **98.7** | **29.3** | **32.6** | **27.9** | **29.9** | **27.7** | **26.1** | **24.5** | **26.3** | **27.1** | **24.1** | **42.5** | **27.9** | **44** | **29.1** |

(continued)

| | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|
| **15. SEQID 166** | **99.7** | **29.6** | **31.9** | **28.2** | **30.2** | **28.3** | **26.4** | **24.8** | **26.6** | **27.9** | **24.9** | **42.9** | **29.6** | **43.9** | **28.3** |
| **16. SEQID 168** | | **29.7** | **31.9** | **28.3** | **30.3** | **28.1** | **26.7** | **24.9** | **26.7** | **28.5** | **23.9** | **43** | **29.4** | **44.8** | **29.5** |
| **17. SEQID 170** | 40.1 | | **30.7** | **29.6** | **29.3** | **31.6** | **24.8** | **36.1** | **35.6,** | **28.5** | **26.5** | **28.9** | **30.4** | **31.6** | **34.9** |
| **18. SEQID 172** | 43.6 | 40.6 | | **31.4** | **32.6** | **30.1** | **28.3** | **28.8** | **28.5** | **32.5** | **24.4** | **33.8** | **26.4** | **32.5** | **28.1** |
| **19. SEQID 174** | 42.8 | 42.7 | 46.2 | | **47.1** | **30.5** | **30.9** | **30.5** | **28.9** | **35.3** | **24.5** | **29.3** | **28.8** | **27.4** | **28.7** |
| **20. SEQID 176** | 45.2 | 41.3 | 50.3 | 59.3 | | **28.9** | **30.3** | **27.9** | **29.2** | **34.3** | **24.6** | **29.8** | **28.5** | **29.9** | **29.7** |
| **21. SEQID 178** | 40.1 | 45 | 45.6 | 44.5 | 45.9 | | **27** | **30.2** | **28.6** | **26.1** | **27.9** | **31.4** | **28.4** | **31.2** | **33.7** |
| **22. SEQID 180** | 42.8 | 38 | 42.1 | 47.2 | 47.1 | 41.4 | | **25.8** | **25** | **26.5** | **26.1** | **28** | **27.5** | **30** | **24.7** |
| **23. SEQID 182** | 32.9 | 46.8 | 39.2 | 38 | 37.7 | 42.7 | 35 | | **37.4** | **27.6** | **28.6** | **23.9** | **33.5** | **26.6** | **31.3** |
| **24. SEQID 184** | 34.5 | 48.8 | 38.6 | 40.5 | 38.6 | 43.1 | 37.1 | 44.4 | | **30.6** | **27.2** | **24.6** | **30.6** | **28.3** | **35.4** |
| **25. SEQID 186** | 39.6 | 40.7 | 45 | 51.2 | 50.2 | 39.8 | 38 | 33.9 | 37.9 | | **26.9** | **29.2** | **28.5** | **27.3** | **26.6** |
| **26. SEQID 188** | 33.7 | 44 | 33 | 37.4 | 36.8 | 41.6 | 35.3 | 37.9 | 39.5 | 39.8 | | **23.3** | **41.3** | **25.7** | **25.2** |
| **27. SEQID 190** | 61.5 | 39.2 | 49.7 | 44.9 | 45.2 | 46.2 | 45.4 | 32.5 | 34.7 | 43 | 33.9 | | **27.1** | **52.8** | **32.8** |
| **28. SEQID 192** | 39.6 | 43.2 | 37.1 | 39.9 | 39.8 | 47.7 | 41.4 | 40.8 | 43.6 | 37.6 | 56.4 | 37.4 | | **29.5** | **26.5** |
| **29. SEQID 194** | 59.1 | 42.2 | 45.3 | 47.1 | 46.5 | 45.3 | 45 | 36.4 | 38.8 | 43.1 | 37 | 66.9 | 41.9 | | **31** |
| **30. SEQID 196** | 41.4 | 51.2 | 39.8 | 41.4 | 42.2 | 53.1 | 39.6 | 41.5 | 50.8 | 38.2 | 42.7 | 45.4 | 44.6 | 43.4 | |
| **31. SEQID 198** | 31.6 | 43.8 | 33.9 | 35.2 | 30.4 | 42 | 34 | 36.3 | 40.6 | 33.5 | 39.5 | 34.4 | 41 | 32.1 | 41.5 |
| **32. SEQID 200** | 62 | 40.8 | 47.8 | 41.3 | 44.4 | 44.2 | 45 | 32.6 | 32.6 | 40.3 | 33.9 | 80.4 | 37.5 | 73.9 | 42.4 |
| **33. SEQID 202** | 64.3 | 38.4 | 47.9 | 45.8 | 47.9 | 44.7 | 46.6 | 32.5 | 36.8 | 41.3 | 35.2 | 71.7 | 35.2 | 62.2 | 44.7 |
| **34. SEQID 204** | 63.5 | 38.1 | 48.3 | 45.6 | 47.5 | 43.5 | 45.9 | 31.7 | 36 | 41.1 | 33.6 | 71.7 | 35.7 | 61.3 | 45.1 |
| **35. SEQID 206** | 60.3 | 41.9 | 50.7 | 44 | 46.4 | 45.1 | 44.8 | 33.9 | 36 | 39.7 | 36.3 | 90.7 | 38.4 | 66.7 | 46.1 |
| **36. SEQID 208** | 59.4 | 41.7 | 49.6 | 43.9 | 47.4 | 45.5 | 46.1 | 34.4 | 36.3 | 40.1 | 36.6 | 89.2 | 38.8 | 67.8 | 46.1 |
| **37. SEQID 210** | 42.2 | 55.7 | 43 | 43.6 | 43.2 | 50.8 | 40.5 | 41.3 | 51.1 | 37.9 | 40.5 | 45.7 | 46.2 | 43.4 | 81.4 |
| **38. SEQID 212** | 65.9 | 40.3 | 47.4 | 43.1 | 45.9 | 43.6 | 43.1 | 33.3 | 33.8 | 41.3 | 33.3 | 67.2 | 35.4 | 57.4 | 44.4 |
| **39. SEQID 214** | 54.3 | 40.8 | 44.7 | 43.9 | 44.1 | 47.6 | 41.1 | 34.5 | 37 | 41.9 | 32.9 | 58.9 | 36.7 | 53.5 | 48.6 |

(continued)

| | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|
| **40. SEQID 216** | 59.8 | 35.6 | 49.2 | 43.4 | 45.7 | 42.7 | 42.2 | 30.3 | 32.6 | 39.9 | 33.1 | 63.9 | 35.9 | 55.1 | 41.4 |
| **41. SEQID 218** | 38.2 | 62.5 | 39.2 | 39.9 | 41.6 | 50.8 | 44.5 | 44.4 | 40.6 | 37.9 | 41.8 | 40.1 | 42.5 | 44.3 | 50.6 |
| **42. SEQID 220** | 40.6 | 59.4 | 38 | 38.6 | 40.1 | 48.1 | 40.5 | 47.3 | 45.3 | 38.5 | 44.1 | 38.4 | 43 | 43.4 | 50.8 |
| **43. SEQID 222** | 34.5 | 50.8 | 32.5 | 30.8 | 35 | 42.7 | 35.9 | 51 | 40.7 | 34.5 | 44.4 | 32.5 | 41.2 | 38.5 | 41.9 |
| **44. SEQID 224** | 38.8 | 60.4 | 38 | 39.6 | 40.4 | 47.3 | 41.4 | 45.9 | 45.9 | 38.2 | 42 | 38.2 | 43.9 | 42.5 | 50 |
| **45. SEQID 228** | 40.4 | 62.6 | 40.1 | 39.6 | 41.3 | 50.4 | 42.3 | 44.3 | 42 | 39.8 | 43.5 | 41.1 | 42.7 | 45 | 51.1 |
| **46. SEQID 230** | 64.3 | 39.9 | 50.4 | 45.1 | 46.5 | 45.9 | 44.4 | 33.3 | 34.9 | 42 | 36 | 69.3 | 36.2 | 61.2 | 43.6 |
| **47. SEQID 232** | 61.8 | 39.2 | 46.3 | 40.8 | 43.4 | 43.7 | 43.4 | 31.3 | 35.8 | 38.4 | 32.6 | 67.4 | 36.8 | 56.8 | 42.1 |
| **48. SEQID 234** | 61.3 | 39 | 43.9 | 42.3 | 47 | 44.2 | 47.3 | 32.5 | 36.1 | 39 | 33 | 63.4 | 37.7 | 54.3 | 42.3 |
| **49. SEQID 236** | 59.4 | 39.6 | 45.6 | 44.5 | 46.9 | 43.2 | 41.9 | 33.3 | 37 | 39.1 | 33.6 | 63.5 | 35.4 | 53.1 | 41.9 |
| **50. SEQID 240** | 40.4 | 94.4 | 39.8 | 40.2 | 42.9 | 44.7 | 40.5 | 45.9 | 50 | 37.6 | 43.1 | 41.1 | 42 | 43.4 | 51.9 |
| **51. SEQID 242** | 43.6 | 47.2 | 43.6 | 42.1 | 41.3 | 44.9 | 43.3 | 37.4 | 42.3 | 37.9 | 34.1 | 48.4 | 39 | 48.3 | 58 |
| **52. SEQID 244** | 31.8 | 47.6 | 29.5 | 33.3 | 33.1 | 45 | 33.1 | 35.4 | 39.1 | 32.3 | 42.9 | 33.6 | 39.6 | 36.4 | 41.2 |
| **53. SEQID-246** | 41.2 | 46 | 41.8 | 41.7 | 41.9 | 47.7 | 44.5 | 35.2 | 43.6 | 37.6 | 37.6 | 48.4 | 41.6 | 45 | 81.2 |
| **54. SEQID 248** | 40.4 | 62.1 | 43 | 39.6 | 42.2 | 51.1 | 43.6 | 46.2 | 47 | 41.3 | 42.4 | 41.9 | 47 | 47.1 | 53.8 |
| **55. SEQID 250** | 43 | 45.9 | 47.4 | 45.3 | 45 | 42.6 | 44.4 | 37.7 | 43.8 | 44.7 | 35.6 | 50.5 | 40.4 | 48 | 55.3 |
| **56. SEQID 252** | 43.9 | 40.9 | 47.7 | 45.4 | 51.4 | 46 | 43.9 | 36.6 | 40.2 | 45.7 | 35.4 | 46.8 | 39.9 | 46.6 | 41.8 |
| **57. SEQID 154** | 40.4 | 55.7 | 41.8 | 44.2 | 43.2 | 52 | 41.1 | 41 | 46.9 | 38.2 | 38.5 | 45.2 | 42.9 | 42.8 | 63.7 |
| **58. SEQID-156** | 39.3 | 58.3 | 37.4 | 38.6 | 41.3 | 46.2 | 40.8 | 44 | 46 | 40.4 | 40.9 | 36.8 | 42.5 | 42.8 | 50 |
| | | | | | | | | | | | | | | | |
| | **31** | **32** | **33** | **34** | **35** | **36** | **37** | **38** | **39** | **40** | **41** | **42** | **43** | **44** | **45** |
| **1. SEQID 132** | **20.3** | **41.3** | **41.2** | **41** | **40.3** | **39.4** | **31.4** | **42** | **34.5** | **38.4** | **26.8** | **29.5** | **24.2** | **28.2** | **27** |
| **2. SEQID 140** | **24** | **25.8** | **27** | **26.4** | **25.1** | **26.3** | **36.6** | **26.9** | **26.8** | **25.5** | **33.1** | **33.3** | **35.4** | **33.1** | **33.7** |
| **3. SEQID 142** | **21.5** | **40.3** | **41.2** | **40.5** | **38.7** | **37** | **29.9** | **38.4** | **34.3** | **35.7** | **26.3** | **28** | **24** | **27.9** | **26.2** |

(continued)

| | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|
| 4. SEQID 144 | 24.1 | 30.1 | 30.4 | 30.2 | 28.9 | 28.5 | 42.8 | 27.8 | 29.4 | 27.3 | 34.2 | 37.5 | 33.9 | 37 | 34.8 |
| 5. SEQID 146 | 21.5 | 36.8 | 34.5 | 33.8 | 36.9 | 36.2 | 29.7 | 35 | 29.4 | 34.9 | 29.3 | 29.1 | 25.1 | 28.3 | 27.8 |
| 6. SEQID 148 | 21.2 | 41.3 | 43.1 | 42 | 40.6 | 38.9 | 31.2 | 39.9 | 35.6 | 37.2 | 27.4 | 27.8 | 24.4 | 27.7 | 27.2 |
| 7. SEQID 150 | 23 | 26.9 | 26.1 | 25.8 | 26.7 | 26.3 | 39.4 | 25.6 | 26.3 | 25.3 | 33.7 | 34.8 | 35.8 | 34.8 | 32.8 |
| 8. SEQID 152 | 21 | 41.1 | 43 | 43 | 41.1 | 40.1 | 29.8 | 43.2 | 35.9 | 39.7 | 27 | 29.1 | 25.2 | 28 | 27.2 |
| 9. SEQID 154 | 20.8 | 37.1 | 40 | 38.7 | 35.9 | 35.5 | 28.3 | 38.8 | 30.8 | 38.1 | 27.2 | 29.4 | 23.5 | 27.9 | 26.7 |
| 10. SEQID156 | 21.3 | 39.5 | 39.9 | 39.5 | 38.5 | 36.8 | 28 | 36.9 | 32.5 | 34.6 | 25.8 | 26.2 | 22.6 | 27.6 | 25.1 |
| 11. SEQID 158 | 23.7 | 38.1 | 40 | 38.8 | 36.8 | 35.1 | 29.6 | 35.4 | 35.7 | 34.2 | 25.7 | 26.8 | 22.7 | 26.5 | 26.5 |
| 12. SEQID 160 | 23.2 | 47.2 | 48.8 | 47.9 | 44.3 | 43.6 | 32.2 | 52.1 | 39.1 | 46.6 | 28.4 | 30.7 | 25.4 | 30.1 | 28.4 |
| 13. SEQID 162 | 23.2 | 46.9 | 48.8 | 47.6 | 44.1 | 43.3 | 32.2 | 52.1 | 39.1 | 46.6 | 28.4 | 30.9 | 25.4 | 30.4 | 28.4 |
| 14. SEQID 164 | 21.8 | 45.8 | 47 | 45.3 | 43.9 | 43.9 | 29.8 | 50.9 | 38.2 | 45.8 | 28.5 | 30.3 | 24.8 | 30 | 29 |
| 15. SEQID166 | 21.5 | 46 | 47.3 | 45.5 | 43.8 | 43.8 | 30.1 | 51.1 | 38.8 | 46 | 28 | 30.1 | 24.3 | 29.8 | 28.3 |
| 16. SEQID 168 | 21.3 | 45.9 | 47.6 | 45.9 | 44.4 | 44.4 | 30.2 | 52.5 | 39.2 | 46.6 | 28.3 | 31 | 24.3 | 29.9 | 28.3 |
| 17. SEQID 170 | 24.5 | 30.8 | 28.1 | 28.3 | 30.8 | 30.2 | 36.9 | 28.5 | 27.3 | 26.1 | 45 | 44.1 | 39.2 | 44.3 | 45.2 |
| 18. SEQID 172 | 21.6 | 34.3 | 32.2 | 32.2 | 34 | 33.2 | 28.9 | 34.3 | 28.8 | 36.2 | 27 | 28.4 | 25.6 | 25.4 | 27.5 |
| 19. SEQID 174 | 23.5 | 26.9 | 30.2 | 29.9 | 28.3 | 27.8 | 28.4 | 28 | 24.9 | 27.8 | 25 | 25.9 | 22 | 27.9 | 24.9 |
| 20. SEQID 176 | 19 | 30.4 | 31.2 | 31.3 | 29.7 | 29.8 | 28.5 | 30.1 | 25.9 | 30.3 | 25.2 | 26.7 | 23.1 | 28 | 25.8 |
| 21. SEQID178 | 23.9 | 32.5 | 33.2 | 33.2 | 31.4 | 31.9 | 32.7 | 32 | 31.5 | 31.5 | 33.9 | 31 | 28.6 | 31.4 | 33.1 |
| 22. SEQID 180 | 20.5 | 27.9 | 29.8 | 29.2 | 27.6 | 28.3 | 24.8 | 27.3 | 22.1 | 25.2 | 27.2 | 26.4 | 23 | 26.1 | 25.8 |
| 23. SEQID 182 | 24.2 | 24 | 24.3 | 24.5 | 24.8 | 25.1 | 32 | 24.4 | 24.5 | 23.7 | 35.2 | 37.5 | 33.2 | 37.4 | 35.5 |
| 24. SEQID 184 | 24.9 | 24.5 | 27.1 | 26.5 | 24.9 | 25.6 | 36 | 25.4 | 25.4 | 24.4 | 29.6 | 30.5 | 26.8 | 31 | 29.4 |
| 25. SEQID 186 | 23.1 | 27.6 | 28 | 27.7 | 27.3 | 26.2 | 25.2 | 28.5 | 24.2 | 27.9 | 25.8 | 24.8 | 23.1 | 25.5 | 25.5 |
| 26. SEQID 188 | 23.8 | 24.7 | 23.3 | 22 | 24.9 | 25.3 | 25.1 | 24.3 | 20.9 | 23.6 | 28.2 | 27.8 | 26.9 | 25.6 | 27 |
| 27. SEQID 190 | 21 | 69.5 | 54.9 | 54.5 | 84.3 | 82.1 | 33.6 | 51.3 | 44.6 | 49.5 | 28.7 | 27.6 | 23.9 | 28.2 | 29 |
| 28. SEQID 192 | 26.7 | 26.7 | 25.2 | 25.2 | 26.5 | 27.2 | 27.1 | 26.1 | 23.5 | 27 | 27.4 | 29.1 | 27.9 | 28.4 | 27 |

(continued)

| | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|
| 29. SEQID 194 | 21.3 | 68.5 | 48.7 | 48.3 | 54.5 | 55.4 | 31.7 | 44.9 | 35.9 | 44.2 | 30.4 | 31.6 | 28.4 | 31.3 | 30.7 |
| 30. SEQID 196 | 21.9 | 32.3 | 32.5 | 32.7 | 35.2 | 34 | 72.7 | 32.3 | 34.2 | 28.9 | 32.3 | 33.9 | 28.7 | 33 | 32.9 |
| 31. SEQID 198 | | 20.9 | 18.9 | 18.8 | 20.2 | 20.5 | 23 | 22.1 | 21 | 20.9 | 24.3 | 24.4 | 26.5 | 24.9 | 25.3 |
| 32. SEQID 200 | 31.5 | | 59.9 | 59.5 | 69.4 | 67.4 | 32.3 | 55.1 | 45.4 | 52.9 | 28.1 | 28.8 | 23.5 | 28.5 | 27.3 |
| 33. SEQID202 | 30.7 | 72.9 | | 96.3 | 56.1 | 55.1 | 33.2 | 66.1 | 54 | 58.6 | 30.5 | 28.5 | 24.1 | 28.9 | 30.7 |
| 34. SEQID204 | 30.7 | 71.8 | 97.1 | | 55.8 | 54.7 | 33.5 | 65.1 | 53 | 57.1 | 29.7 | 28.9 | 24.3 | 27.7 | 30.4 |
| 35. SEQID 206 | 32 | 81.1 | 72.2 | 72 | | 97.3 | 34.4 | 53.5 | 45.8 | 49.5 | 28.8 | 28.9 | 25.3 | 27.2 | 28.8 |
| 36. SEQID-208 | 32.8 | 79.1 | 70.6 | 70.4 | 97.9 | | 33.5 | 52.5 | 44.7 | 48 | 28.4 | 28.1 | 24.7 | 27.1 | 28.4 |
| 37. SEQID 210 | 43.6 | 43.2 | 43.7 | 43.2 | 45.9 | 46.6 | | 32.1 | 32 | 30.6 | 35.8 | 36.5 | 32.5 | 32.4 | 36 |
| 38. SEQID 212 | 31.8 | 69 | 79.7 | 78.7 | 68.7 | 67.4 | 42.8 | | 75.1 | 69.5 | 27.9 | 29 | 25.4 | 29.2 | 29.5 |
| 39. SEQID 214 | 33.5 | 56.1 | 66.9 | 66.1 | 58.7 | 58.3 | 46.4 | 77.7 | | 52.9 | 25.4 | 26.9 | 23.4 | 25.9 | 26.7 |
| 40. SEQID 216 | 32.3 | 64.9 | 72.5 | 70.7 | 63.9 | 61.6 | 40.9 | 83.1 | 64.4 | | 27 | 29.5 | 24 | 29.2 | 27.5 |
| 41. SEQID 218 | 43.7 | 42.6 | 41.3 | 40 | 40.5 | 39.3 | 51.1 | 40.3 | 40.1 | 37.6 | | 62.8 | 65.8 | 61.9 | 95.8 |
| 42. SEQID 220 | 41.8 | 40.6 | 37.8 | 38.1 | 41.1 | 39 | 54.5 | 36.9 | 39.2 | 36.4 | 74.3 | | 47.3 | 94.1 | 64.2 |
| 43. SEQID 222 | 38.7 | 35.1 | 33.1 | 33.3 | 33.1 | 33.3 | 44.3 | 34.1 | 33.2 | 32.8 | 69 | 54.3 | | 46.5 | 68.8 |
| 44. SEQID 224 | 40.6 | 40.3 | 39.4 | 38.1 | 36.8 | 37.4 | 49.2 | 37.9 | 38.9 | 36.9 | 72.8 | 96.9 | 54.1 | | 62.4 |
| 45. SEQID 228 | 42.4 | 43.2 | 42.9 | 41.9 | 40.3 | 38.8 | 52.3 | 41.8 | 41.1 | 38.6 | 97.3 | 74.8 | 70.2 | 73.3 | |
| 46. SEQID 230 | 31.5 | 72.1 | 79.5 | 76.9 | 70.1 | 68 | 44.4 | 77.7 | 65.1 | 70.5 | 39.6 | 39.1 | 34.4 | 36.5 | 40.9 |
| 47. SEQID 232 | 30.5 | 68.2 | 74.5 | 72.9 | 68.2 | 66.3 | 42.4 | 74.1 | 62.6 | 65.4 | 38.4 | 40.5 | 31.6 | 38.9 | 40.5 |
| 48. SEQID 234 | 28.8 | 65.6 | 68.3 | 66.5 | 63.4 | 61.3 | 43.9 | 65.9 | 54.5 | 64.4 | 39.7 | 41.6 | 33 | 38.7 | 39.7 |
| 49. SEQID 236 | 28.6 | 63.8 | 69.8 | 67.2 | 65.4 | 63.5 | 44.5 | 70.3 | 57.8 | 64.6 | 40.4 | 40.1 | 34.4 | 39.6 | 41.9 |
| 50. SEQID 240 | 42.6 | 39.5 | 38.4 | 38.7 | 42.4 | 42.3 | 54.5 | 37.2 | 40.4 | 36.4 | 61.3 | 58.2 | 52 | 60.8 | 63.4 |
| 51. SEQID 242 | 36.1 | 47.3 | 45.5 | 45.3 | 46.7 | 46.6 | 59 | 46.9 | 46.7 | 43.4 | 45.6 | 45.9 | 37 | 45.2 | 45.6 |
| 52. SEQID 244 | 40.2 | 32.6 | 31 | 31.2 | 33.9 | 33.9 | 42.8 | 31.5 | 35.1 | 31.1 | 42.9 | 43.8 | 40.8 | 42.4 | 40.8 |
| 53. SEQID 246 | 39.3 | 42.9 | 44.4 | 44.5 | 47.5 | 47.4 | 75.5 | 46.7 | 51.4 | 42.9 | 43.3 | 47 | 37.9 | 46.3 | 45 |

(continued)

| | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|
| **54. SEQID248** | 40.2 | 42.9 | 40.2 | 39.5 | 41.3 | 40.7 | 55.3 | 40.3 | 44.2 | 37.4 | 56.1 | 62.1 | 49.2 | 61.4 | 58 |
| **55. SEQID 250** | 34.3 | 46.8 | 45.2 | 46.9 | 46.9 | 46.3 | 55.6 | 46.7 | 47.4 | 43.4 | 43.2 | 43.8 | 34 | 41.9 | 42.6 |
| **56. SEQID 252** | 36 | 43.7 | 45.8 | 43.2 | 45.1 | 45 | 40.9 | 41.5 | 43 | 43.4 | 38.7 | 39 | 29.9 | 39.9 | 36.6 |
| **57. SEQID254** | 39.9 | 42.9 | 45.5 | 45.1 | 46.1 | 45.5 | 64.1 | 44.4 | 47 | 43.2 | 48 | 49.1 | 38.5 | 48 | 48.7 |
| **58. SEQID 256** | 41.8 | 39.3 | 41.3 | 41.3 | 37.3 | 37.4 | 51.9 | 40 | 39.8 | 38.1 | 62.5 | 66 | 50.4 | 67.5 | 61.1 |

| | 46 | 47 | 48 | 49 | 50 | 51 | 52 | 53 | 54 | 55 | 56 | 57 | 58 |
|---|---|---|---|---|---|---|---|---|---|---|---|---|---|
| **1. SEQID 132** | **41** | **41.4** | **40.8** | **41.6** | **28.6** | **29.8** | **21.5** | **33.4** | **27.9** | **32.1** | **33.3** | **31.5** | **26.6** |
| **2. SEQID 140** | **28.1** | **27.1** | **26.8** | **27** | **36.4** | **31.3** | **26.8** | **31.8** | **36.4** | **31.1** | **28.4** | **37.8** | **33.2** |
| **3. SEQID 142** | **38.8** | **38.3** | **38.6** | **36.2** | **30.3** | **30.5** | **20.2** | **27.2** | **29** | **30.2** | **31.3** | **32.4** | **26.8** |
| **4. SEQID 144** | **31.3** | **28.4** | **29.2** | **31.1** | **35.1** | **33.7** | **23.5** | **37.8** | **34.6** | **35** | **30.5** | **43.8** | **36.2** |
| **5. SEQID 146** | **34.8** | **34.4** | **35.8** | **34.5** | **27.8** | **30.4** | **21.5** | **28.1** | **28.1** | **31.9** | **49.7** | **31.5** | **29.6** |
| **6. SEQID 148** | **43.4** | **39.7** | **39.8** | **37.9** | **29.5** | **32.2** | **21.5** | **29.4** | **28.9** | **30.3** | **31** | **34.5** | **26.7** |
| **7. SEQID 150** | **26.2** | **25** | **24.9** | **25.3** | **33.2** | **33.7** | **29.1** | **34.1** | **35.1** | **31** | **25.5** | **37.7** | **35.7** |
| **8. SEQID 152** | **45.3** | **42.4** | **40.8** | **39.5** | **28.3** | **30.4** | **22.1** | **28.6** | **28.1** | **30.7** | **35.7** | **31** | **25.4** |
| **9. SEQID 154** | **43.1** | **37.4** | **36.8** | **39.7** | **26** | **27.8** | **19.8** | **27.1** | **27.3** | **30.6** | **30.2** | **28.7** | **27.8** |
| **10. SEQID 156** | **39.2** | **37.9** | **37.5** | **34.5** | **28.7** | **27.7** | **19.9** | **27.4** | **27** | **29.1** | **28.8** | **30.8** | **26.8** |
| **11. SEQID 158** | **40.9** | **37** | **38.6** | **37** | **29.5** | **29.8** | **21.1** | **27** | **27.4** | **29.2** | **31** | **32.9** | **26.5** |
| **12. SEQID160** | **49.3** | **46.3** | **44.8** | **44.4** | **28.3** | **30.8** | **24.1** | **29.9** | **29.7** | **29.3** | **31.1** | **32.2** | **27** |
| **13. SEQID 162** | **49.3** | **46** | **45.1** | **44.4** | **28.3** | **30.8** | **24.4** | **29.1** | **30** | **29.3** | **31.6** | **32.5** | **27** |
| **14. SEQID 164** | **46.6** | **42.3** | **43.2** | **42.6** | **29.7** | **31** | **22.6** | **28.3** | **29.2** | **29.9** | **30.6** | **29.8** | **28.8** |
| **15. SEQID 166** | **47.1** | **42.7** | **43.6** | **42.8** | **30.1** | **31.8** | **21.8** | **28.8** | **29.3** | **30.2** | **30.8** | **29.6** | **28.8** |
| **16. SEQID 168** | **46.7** | **42.7** | **44** | **43.1** | **30.1** | **31.4** | **21.7** | **28.6** | **28.8** | **30.3** | **31.9** | **30.2** | **28.6** |
| **17. SEQID 170** | **30.2** | **29.1** | **29.1** | **28.4** | **90.8** | **32.5** | **29** | **34.3** | **41.2** | **31.4** | **27.5** | **35.4** | **42.8** |

(continued)

| | 46 | 47 | 48 | 49 | 50 | 51 | 52 | 53 | 54 | 55 | 56 | 57 | 58 |
|---|---|---|---|---|---|---|---|---|---|---|---|---|---|
| 18. SEQID 172 | 34.9 | 31.7 | 31.5 | 33.5 | 29.8 | 28.7 | 19.5 | 26.9 | 29.7 | 31.5 | 36 | 30.1 | 27.9 |
| 19. SEQID 174 | 31.1 | 28 | 28 | 30.8 | 29 | 28.3 | 21.1 | 26.9 | 29.4 | 28.8 | 32.4 | 25.5 | 28.6 |
| 20. SEQID 176 | 31.6 | 29.9 | 32.2 | 33 | 28.7 | 26.5 | 22.5 | 28 | 25.5 | 27.1 | 35.9 | 30.8 | 27.7 |
| 21. SEQID 178 | 35.5 | 33.3 | 31.4 | 32.2 | 31.2 | 28.9 | 26.6 | 30.1 | 31.4 | 29.3 | 30 | 35 | 30.6 |
| 22. SEQID 180 | 28.2 | 26.4 | 29.6 | 29 | 26.7 | 27.1 | 19.6 | 24.1 | 25.7 | 24.1 | 27.4 | 26.8 | 27.5 |
| 23. SEQID 182 | 24.1 | 24.5 | 25.7 | 26.8 | 36.2 | 28.9 | 23.6 | 27 | 33 | 28 | 27.7 | 33.3 | 35.2 |
| 24. SEQID184 | 27.4 | 25.7 | 25.6 | 26.2 | 34.9 | 31.8 | 23.7 | 32.8 | 33.6 | 30.6 | 29.8 | 34.2 | 33 |
| 25. SEQID 186 | 30 | 28.3 | 27 | 29 | 28.2 | 25.4 | 22.6 | 25.6 | 25.8 | 28 | 32.4 | 26.9 | 28.3 |
| 26. SEQID 188 | 23.6 | 21.3 | 22.1 | 24.8 | 27.5 | 23.1 | 27.9 | 23 | 29.1 | 23.2 | 25.8 | 23.4 | 28.3 |
| 27. SEQID 190 | 52 | 49.7 | 46.2 | 45.1 | 28.6 | 37.1 | 22.2 | 32.2 | 28.9 | 36.1 | 32.2 | 35.1 | 27.2 |
| 28. SEQID 192 | 27.5 | 26.1 | 27.8 | 26.9 | 29.1 | 26.4 | 24.7 | 24.6 | 30.4 | 24.9 | 28.7 | 27.4 | 29.2 |
| 29. SEQID194 | 46.2 | 45.3 | 41.8 | 40.1 | 31.5 | 32.8 | 26.6 | 31.3 | 33.2 | 30.9 | 32.6 | 31.4 | 30.7 |
| 30. SEQID 196 | 34.1 | 31.4 | 31 | 30.9 | 37.3 | 44.6 | 26.2 | 77.7 | 33.2 | 46.2 | 28.8 | 51.1 | 34.9 |
| 31. SEQID 198 | 18.9 | 19.2 | 19.9 | 19.9 | 23.7 | 20 | 24.9 | 21.4 | 22.5 | 23.7 | 22.8 | 22.1 | 25.1 |
| 32. SEQID 200 | 57.4 | 55.4 | 48.4 | 46.9 | 30.1 | 35.5 | 22.1 | 32.4 | 31.5 | 34.5 | 32 | 34.5 | 29.7 |
| 33. SEQID 202 | 67.1 | 59.2 | 53.5 | 54.4 | 28.6 | 33.8 | 20.4 | 31.5 | 30.3 | 33.2 | 31.1 | 35 | 31 |
| 34. SEQID 204 | 66 | 58 | 51.1 | 52.5 | 28.8 | 31.1 | 20.3 | 31.2 | 28.6 | 34 | 30.5 | 34.6 | 31 |
| 35. SEQID 206 | 51.8 | 49 | 45.6 | 45.3 | 30.2 | 34.7 | 22.3 | 33.5 | 29.5 | 34.5 | 30.7 | 36.8 | 28.3 |
| 36. SEQID 208 | 50.3 | 47.8 | 44.2 | 44.2 | 29.6 | 34.8 | 22.4 | 32.7 | 28.9 | 33.4 | 30.6 | 36 | 28.7 |
| 37. SEQID 210 | 33.6 | 31.4 | 33.9 | 34 | 38.2 | 44.3 | 26.8 | 67.9 | 37.6 | 46.3 | 29.5 | 48.6 | 33.9 |
| 38. SEQID 212 | 63.7 | 57 | 49.9 | 52.2 | 28 | 34 | 22.3 | 31.6 | 28.4 | 33.8 | 31.2 | 34.3 | 29.6 |
| 39. SEQID 214 | 50.8 | 48.2 | 40.3 | 43.5 | 26.1 | 32.4 | 20.4 | 30.6 | 28.4 | 31 | 25.6 | 34 | 25.4 |
| 40. SEQID 216 | 57.9 | 53.2 | 49.5 | 48.5 | 27.3 | 32.3 | 19.9 | 29.9 | 27.7 | 31.8 | 31.6 | 31 | 27.4 |
| 41. SEQID 218 | 28.6 | 26.6 | 28.8 | 30.1 | 44.4 | 31.5 | 26.3 | 29.1 | 44.6 | 29.4 | 25.3 | 32.2 | 50 |
| 42. SEQID 220 | 30.4 | 30.2 | 30.1 | 31.2 | 45.8 | 32.3 | 25 | 31.5 | 48 | 30.6 | 27.9 | 32.4 | 52.1 |

(continued)

| | 46 | 47 | 48 | 49 | 50 | 51 | 52 | 53 | 54 | 55 | 56 | 57 | 58 |
|---|---|---|---|---|---|---|---|---|---|---|---|---|---|
| **43. SEQID 222** | **26.5** | **24.5** | **24.9** | **25.6** | **37.7** | **26.9** | **28.8** | **25.3** | **37.5** | **25.6** | **21** | **27.1** | **41.8** |
| **44. SEQID 224** | **28.1** | **29.1** | **29.3** | **29.6** | **44.5** | **31.1** | **24.7** | **29.9** | **46.9** | **29.5** | **27.5** | **30.5** | **51.3** |
| **45. SEQID 228** | **29.4** | **27.6** | **29.3** | **30.7** | **45.3** | **31** | **24.8** | **29.7** | **45** | **30.3** | **24.9** | **31.8** | **49.4** |
| **46. SEQID 230** | | **68.5** | **52.9** | **51.9** | **30.8** | **33.3** | **22.1** | **31.9** | **31.4** | **35.1** | **32.9** | **36** | **29.1** |
| **47. SEQID 232** | 80.1 | | **46** | **47.6** | **27.8** | **29.5** | **20.5** | **30** | **29.7** | **35.6** | **32.9** | **32.8** | **27.4** |
| **48. SEQID 234** | 68.3 | 62.6 | | **69.4** | **27.5** | **34** | **20.3** | **31.7** | **28.4** | **32.5** | **29.7** | **32.9** | **26** |
| **49. SEQID 236** | 69.8 | 66.4 | 80.5 | | **28.6** | **31.9** | **21.2** | **30.8** | **29.4** | **33.9** | **30.8** | **33.1** | **27.8** |
| **50. SEQID 240** | 39.9 | 37.9 | 37.4 | 41.1 | | **31.3** | **28.6** | **33.9** | **42** | **32.3** | **28.4** | **36.6** | **40.4** |
| **51. SEQID 242** | 45.4 | 42.4 | 46 | 43.8 | 45.9 | | **22.3** | **41.3** | **33.2** | **41.7** | **25.4** | **52.8** | **31.3** |
| **52. SEQID 244** | 31.8 | 31.1 | 31.9 | 31.3 | 47.2 | 38 | | **23.3** | **27** | **23.3** | **22.2** | **23.2** | **28** |
| **53. SEQID 246** | 45.9 | 41.1 | 44.2 | 43 | 49.3 | 60.7 | 38.9 | | **32.8** | **43.1** | **26** | **47.5** | **31.1** |
| **54. SEQID 248** | 44.6 | 40 | 39.5 | 40.9 | 60.2 | 47.2 | 39.4 | 48.7 | | **30.5** | **29.3** | **33.2** | **39.9** |
| **55. SEQID 250** | 44.9 | 48.7 | 44.9 | 47.1 | 44.4 | 53.8 | 33.7 | 56.5 | 42.9 | | **29.2** | **46.7** | **30.2** |
| **56. SEQID 252** | 47.2 | 45.8 | 39.5 | 43.5 | 40.2 | 40.9 | 32.6 | 42.4 | 41.8 | 44.4 | | **26.5** | **26.9** |
| **57. SEQID 254** | 45.4 | 41.6 | 43.1 | 44.5 | 57.9 | 63.6 | 39.6 | 61.1 | 49.8 | 56.5 | 38.4 | | **31.9** |
| **58. SEQID 256** | 39.6 | 36.6 | 37.1 | 35.9 | 58.3 | 46.9 | 42.1 | 45 | 54.2 | 41.9 | 39.9 | 46.5 | |

***Example 12: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 132 resented in Table **16.**

**Table 16:** InterPro scan results of the polypeptide sequence as represented by SEQ ID NO: 132

| Database | Accession number | Accession name |
|---|---|---|
| PRODOM | PD001423 | Q9SP16_ORYSA_Q9SP16; |
| PRINTS | PR00367 | ETHRSPELEMNT |
| GENE3D | G3DSA:3.30.730.10 | no description |
| PFAM | PF00847 | AP2 |
| SMART | SM00380 | AP2 |
| PROFILE | PS51032 | AP2_ERF |
| SUPERFAMILY | SSF54171 | DNA binding domain |

***Example 13: Topology prediction of the polypeptide sequences useful in performing the methods of the invention (subcellular localization, transmembrane...)***

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 132 are presented Table 17. The "plant" organism group has been selected, no cutoffs defined, and the predicted length of the transit peptide requested. The subcellular localization of the polypeptide sequence as represented by SEQ ID NO: 132 may be the cytoplasm or nucleus, no transit peptide is predicted.

**Table 17:** TargetP 1.1 analysis of the polypeptide sequence as represented by SEQ ID NO: 132

| | |
|---|---|
| Length (AA) | 130 |
| Chloroplastic transit peptide | 0.098 |
| Mitochondrial transit peptide | 0.339 |
| Secretory pathway signal peptide | 0.035 |
| Other subcellular targeting | 0.797 |
| Predicted Location | / |
| Reliability class | 3 |
| Predicted transit peptide length | / |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

***Example 14: Expression vector construction using the nucleic acid sequence as represented by SEQ ID NO: 131***

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The *Oryza sativa AP2-2* gene was amplified by PCR from a rice cDNA library. The PCR fragment of the expected length was purified and subsequently cloned in a Gateway® vector using standard technology. The entry clone comprising SEQ ID NO: 131 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 39) for root specific expression was located upstream of this Gateway cassette.

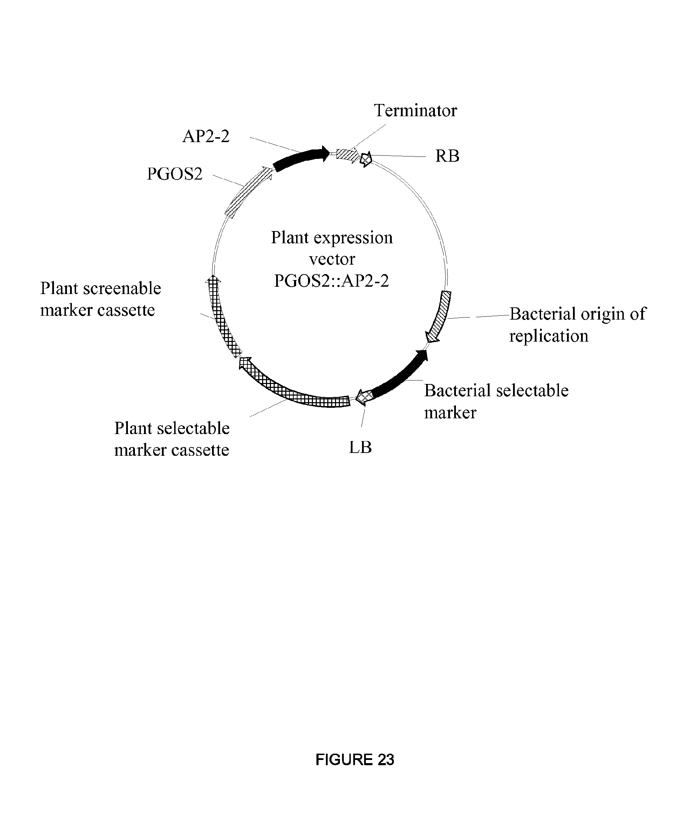

After the LR recombination step, the resulting expression vector pGOS2::AP2-2 (Figure 23) was transformed into *Agrobacterium* strain LBA4044 according to methods well known in the art.

***Example 15: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges 1996, Chan *et al.* 1993, Hiei *et al.* 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58Cl pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 16: Phenotypic evaluation procedure***

<u>**16.1** Evaluation setup</u>

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

<u>**16.2** Statistical analysis: F-test</u>

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

<u>**16.3** Parameters measured</u>

Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor 106. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 17: Results of the phenotypic evaluation of the transgenic plants***

The results of the evaluation of transgenic rice plants expressing the AP2-2 nucleic acid are as follows.

There was an increase in yield compared to corresponding nullizygotes (controls), in particular seed yield, expressed as Thousand Kernel Weight and Harvest Index, was statistically significant increased compared to the control plants. For the TKW, an overall increase of 2% in T1 and of 3.4% in T2 was observed compared to control plants. In the case of harvest index, the overall increase compared to control plants was respectively 9.2% (T1) and 36.2% (T2).

**Claims**

1. A method for enhancing yield-related traits in plants relative to control plants, comprising modulating expression in a plant of a nucleic acid encoding an AP2-2 polypeptide, which AP2-2 polypeptide comprises motif XII (SEQ ID NO: 133) and preferably also one or more of: motif XIII (SEQ ID NO: 134), motif XIV (SEQ ID NO: 135), motif XV (SEQ ID NO: 136), motif XVI (SEQ ID NO: 137), and motif XVII (SEQ ID NO: 138).

2. Method according to claim 1, wherein said AP2-2 polypeptide has, in increasing order of preference, at least 20, 25, 30, 35, 40, 45%, 50%, 55%, 60%, 65%, 70%, 75%, 80%, 85%, 90%, 95% or more sequence identity to the AP2-2 polypeptide represented by SEQ ID NO: 132.

3. Method according to claim 1 or 2, wherein said nucleic acid encoding an AP2-2 polypeptide is represented by any one of the nucleic acid SEQ ID NOs given in Table 14 or a portion thereof, or a sequence capable of hybridising with any one of the nucleic acids SEQ ID NOs given in Table 14.

4. Method according to any of claims 1 to 3, wherein said nucleic acid sequence encodes an orthologue or paralogue of any of the SEQ ID NOs given in Table 14.

5. Method according to any of claims 1 to 4, wherein said modulated expression is effected by introducing and expressing in a plant a nucleic acid encoding an AP2-2 polypeptide.

6. Method according to any of claims 1 to 5, wherein said enhanced yield-related trait is increased yield, preferably increased seed yield relative to control plants.

7. Method according to claim 5 or 6, wherein said nucleic acid is operably linked to a constitutive promoter, preferably to a GOS2 promoter.

8. Method according to any of claims 1 to 7, wherein said nucleic acid encoding an AP2-2 polypeptide is of plant origin, preferably from a dicotyledonous plant, further preferably from the family Poaceae, more preferably from the genus *Oryza,* most preferably from *Oryza sativa.*

9. Plant or part thereof, including seeds, obtainable by a method according to any of claims 1 to 8, wherein said plant or part thereof comprises a recombinant nucleic acid encoding an AP2-2 polypeptide.

**10.** Construct comprising:

(a) nucleic acid encoding an AP2-2 polypeptide as defined in any one of claims 1 to 4;
(b) one or more control sequences capable of driving expression of the nucleic acid sequence of (a); and optionally
(c) a transcription termination sequence.

**11.** Construct according to claim 10, wherein said one or more control sequences is a constitutive promoter, preferably a GOS2 promoter.

**12.** Use of a construct according to claims 10 or 11 in a method for making plants having increased yield, particularly increased seed yield relative to control plants.

**13.** Plant, plant part or plant cell transformed with a construct according to claim 10 or 11.

**14.** Method for the production of a transgenic plant having increased yield, particularly increased seed yield relative to control plants, comprising:

(i) introducing and expressing in a plant a nucleic acid encoding an AP2-2 polypeptide as defined in any one of claims 1 to 4; and
(ii) cultivating the plant cell under conditions promoting plant growth and development.

**15.** Transgenic plant having increased yield, particularly increased seed yield, relative to control plants, resulting from increased expression of a nucleic acid encoding an AP2-2 polypeptide as defined in any one of claims 1 to 4, or a transgenic plant cell derived from said transgenic plant.

**16.** Transgenic plant according to claim 9, 13 or 15, wherein said plant is a crop plant or a monocot or a cereal, such as rice, maize, wheat, barley, millet, rye, sorghum and oats, or a transgenic plant cell derived from said transgenic plant.

**17.** Harvestable parts of a plant according to claim 16, wherein said harvestable parts are preferably seeds.

**18.** Products derived from a plant according to claim 16 and/or from harvestable parts of a plant according to claim 17.

**19.** Use of a nucleic acid encoding an AP2-2 polypeptide as defined in any one of claims 1 to 4 in increasing yield, particularly in increasing seed yield, in plants relative to control plants.

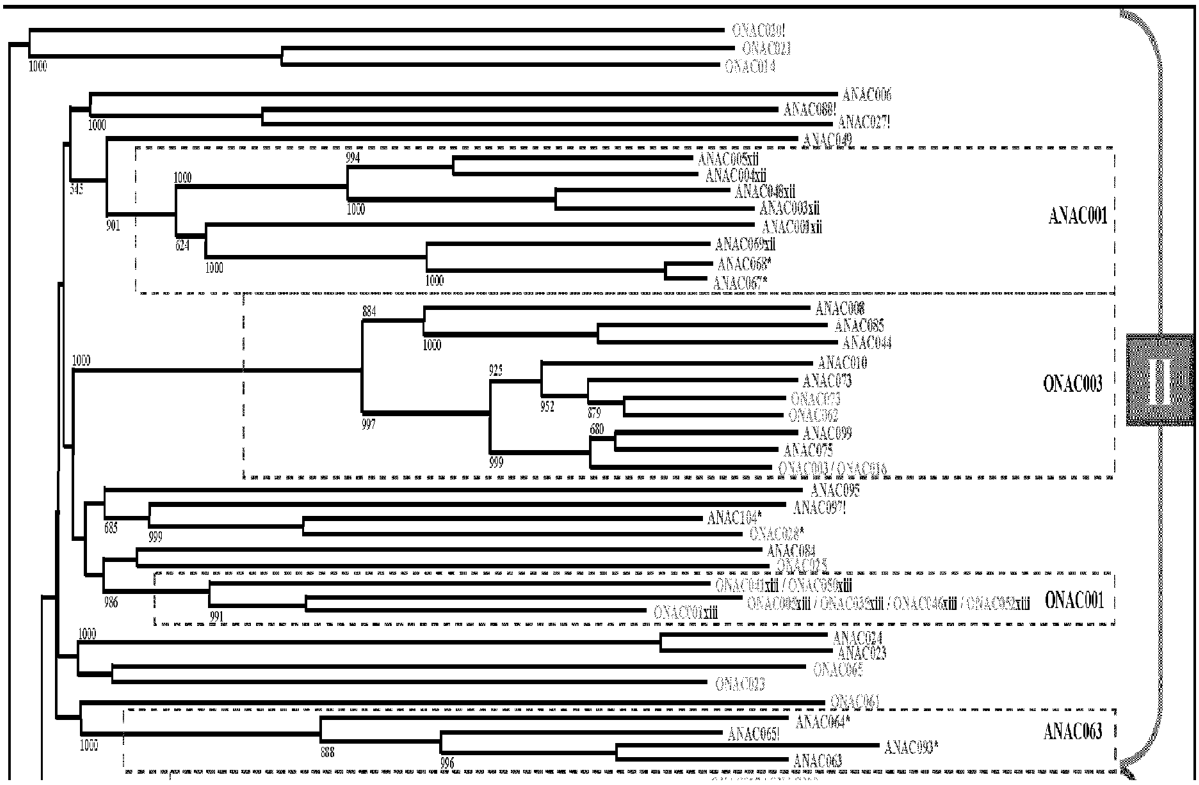


FIGURE 1

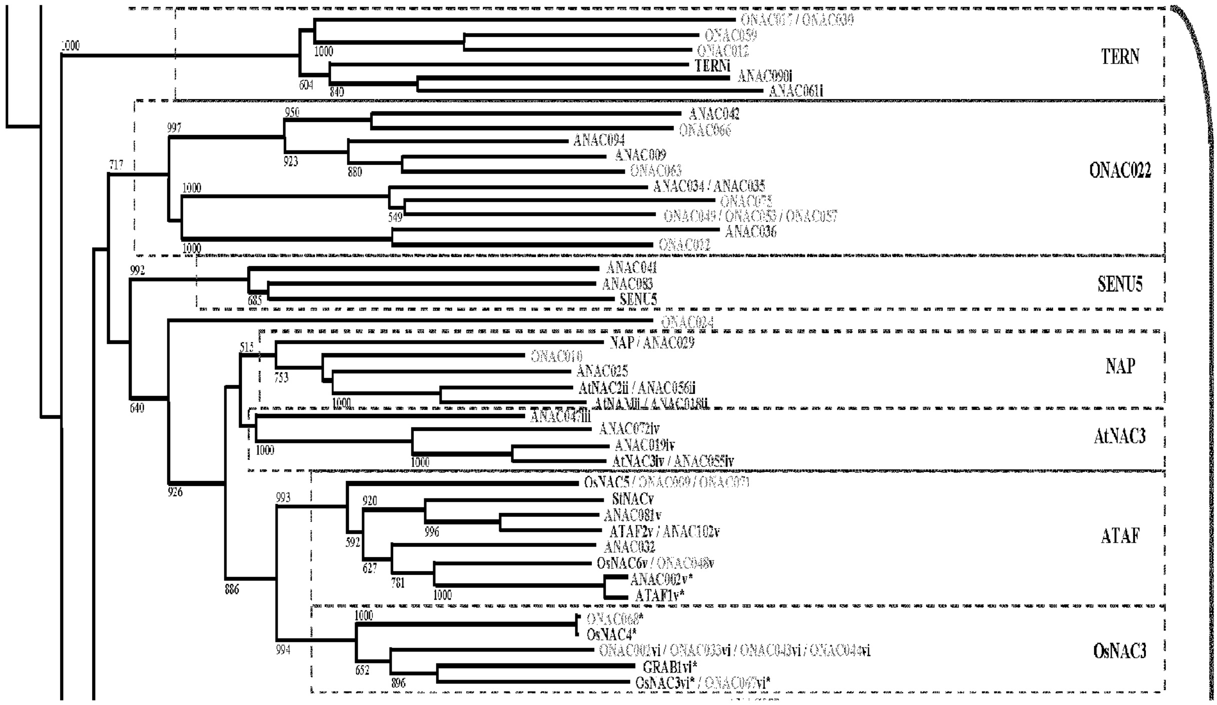

FIGURE 1 continued

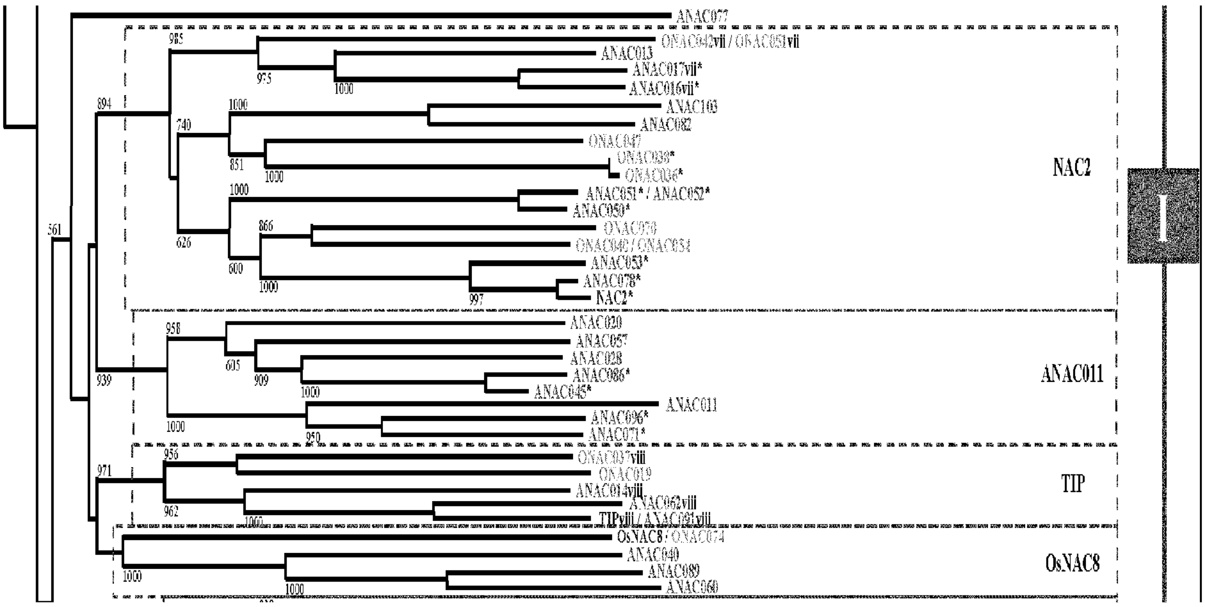

FIGURE 1 continued

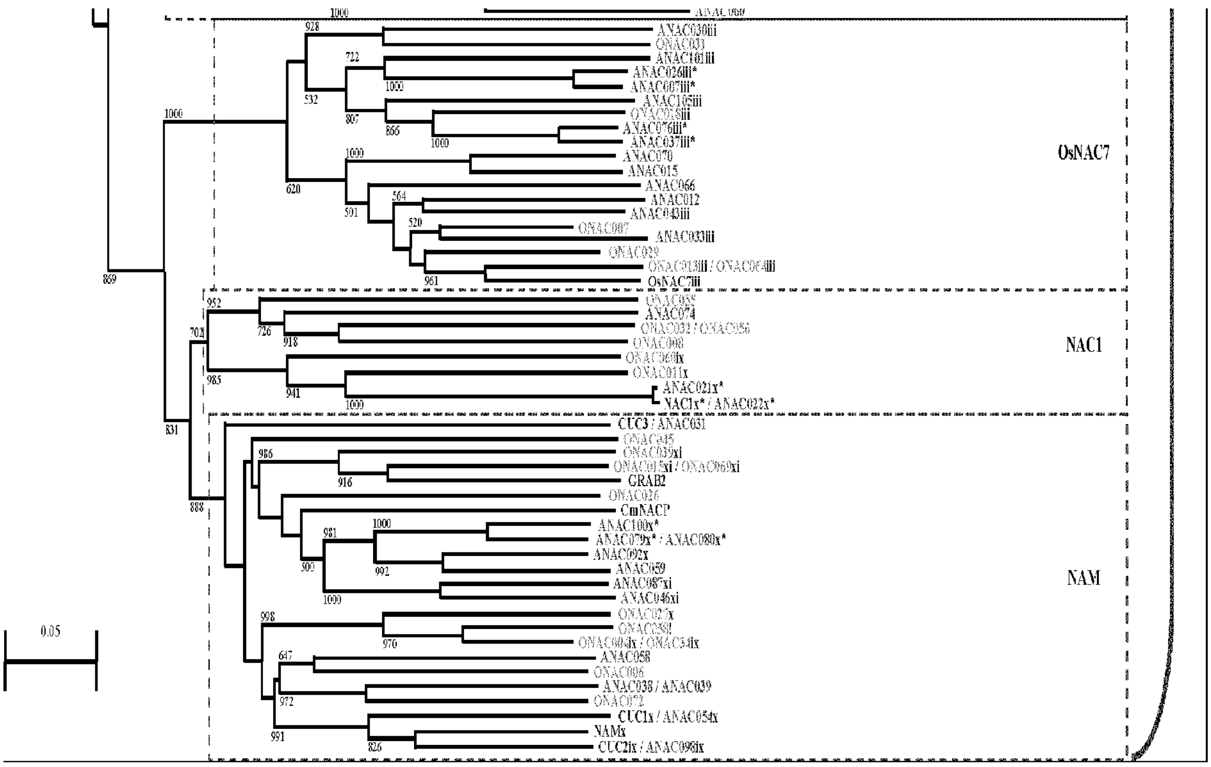


FIGURE 1 continued

```
|-----1-----|                                  |-------6------
MCGGAILSDLIPPPRRVTAGDLWLEKTKKQQQQKKKNKGARRLPLRQEEEDDFEADFEEFEV
                                 |-A-|

--6--------|                                 |-------3-------|
DSGEWEVESDADEAKPLAAPRSGFAKGGLKNTTVAGADGPAARSAKRKRKNQFRGIRQRPWG
                                             |-A-| |--------B-

KWAAEIRDPRKGVRVWLGTFNSPEEAARAYDAEARRIRGKKAKVNFPDGAPVASQRSHAEPS
------------B--------------------------------|

                                                  |-------7---
SMNMPAFSIEEKPAVMSAGNKTMYNTNAYAYPAVEYTLQEPFVQIQNVSFVPAMNAIEDTFV

------7-------------|
NLSSDQGSNSFGCSDFSQENDIKTPDITSMLAPTMTGVDDSAFLQNNASDAMVPPVMGNASI
             |-----------4------------|

DLADLEPYMKFLIDGGSDESIDTLLSSDGSQDVASSMDLWSFDDMPVSAEFY
           |------8-------|           |5|
```

FIGURE 2

FIGURE 3

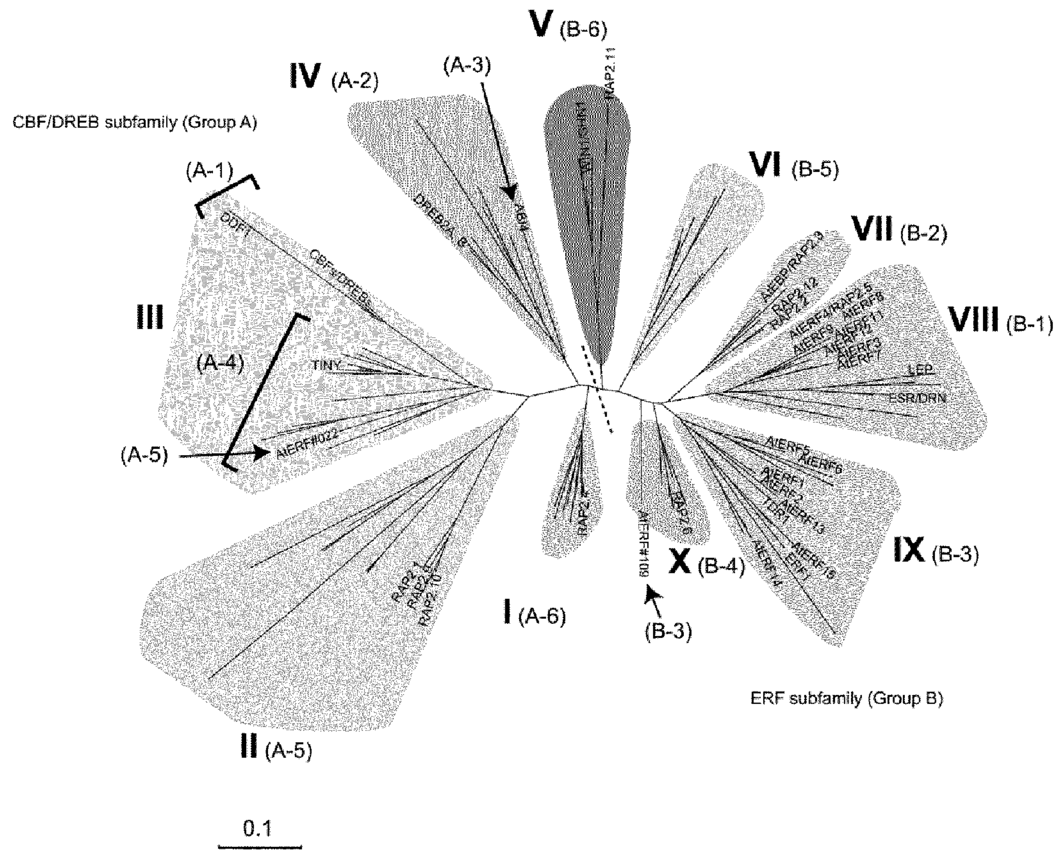

V (B-6)
IV (A-2)
(A-3)
CBF/DREB subfamily (Group A)
(A-1)
III
(A-4)
(A-5)
VI (B-5)
VII (B-2)
VIII (B-1)
IX (B-3)
X (B-4)
(B-3)
I (A-6)
II (A-5)
ERF subfamily (Group B)
DDF1
CBFs/DREBs
TINY
AtERF#022
DREB2A, B
ABI4
RAP2.11
RAP2
RAP2.6
AtERF#109
RAP2.1
RAP2.10
LEP
ESR/DRN
AtERF5
AtERF6
AtERF1
AtERF2
AtERF13
TDR1
AtERF15
ERF1
AtERF14
AtEBP/RAP2.3
RAP2.12
RAP2.2
AtERF4/RAP2.5
AtERF8
AtERF9
AtERF11
AtERF3
AtERF7
0.1

FIGURE 4

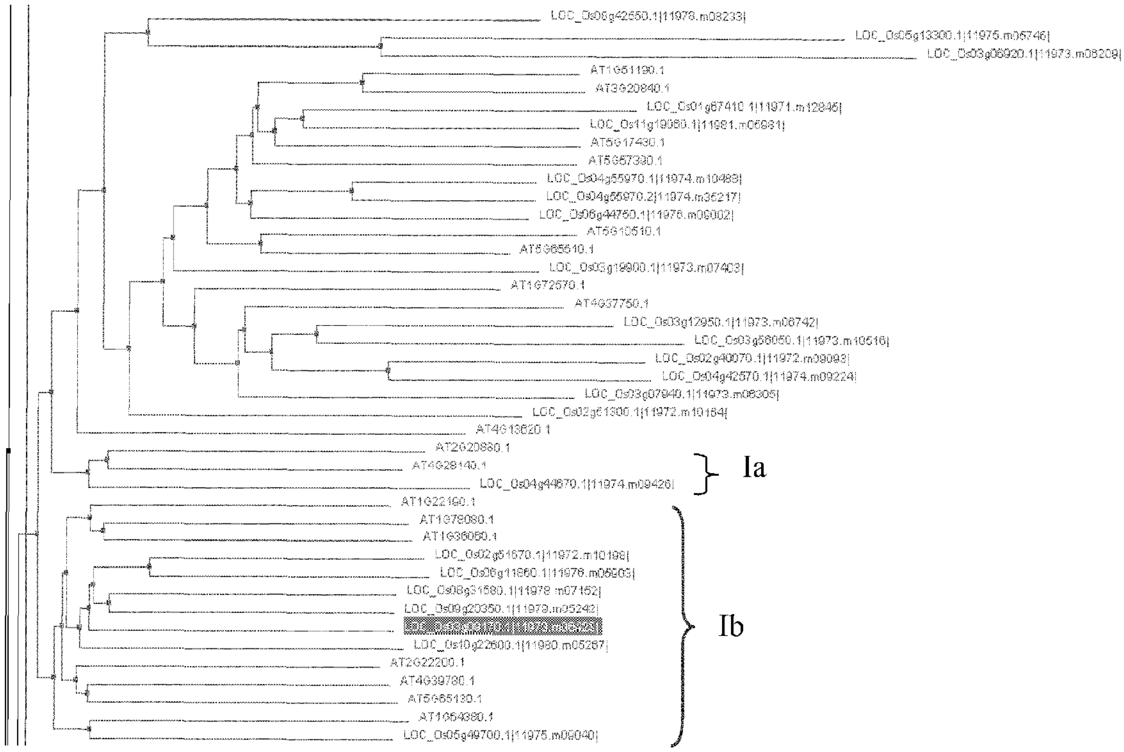
LOC_Os06g42550.1|11976.m08233|
LOC_Os05g13300.1|11975.m05746|
LOC_Os03g06920.1|11973.m06209|
AT1G51190.1
AT3G20840.1
LOC_Os01g67410.1|11971.m12846|
LOC_Os11g19060.1|11981.m05981|
AT5G17430.1
AT5G57390.1
LOC_Os04g55970.1|11974.m10489|
LOC_Os04g55970.2|11974.m35217|
LOC_Os06g44750.1|11976.m09002|
AT5G10510.1
AT5G65510.1
LOC_Os03g19900.1|11973.m07403|
AT1G72570.1
AT4G37750.1
LOC_Os03g12950.1|11973.m06742|
LOC_Os03g56050.1|11973.m10516|
LOC_Os02g40070.1|11972.m09093|
LOC_Os04g42570.1|11974.m09224|
LOC_Os03g07940.1|11973.m06305|
LOC_Os02g61300.1|11972.m10164|
AT4G13620.1
AT2G20880.1
AT4G28140.1
LOC_Os04g44670.1|11974.m09426|
Ia
AT1G22190.1
AT1G78080.1
AT1G36060.1
LOC_Os02g51670.1|11972.m10198|
LOC_Os06g11860.1|11976.m05903|
LOC_Os08g31580.1|11978.m07152|
LOC_Os09g20350.1|11979.m05242|
LOC_Os10g22600.1|11980.m05267|
AT2G22200.1
AT4G39780.1
AT5G65130.1
AT1G64380.1
LOC_Os05g49700.1|11975.m09040|
Ib

```
OSJNBa0075G19.15"NAM-like               ----------------------------------------MDGSSMSSSS 10
ANAC101"AT5G62380"id="NP_20104          ---------------------------------------------MES-- 3
11972.m09332                            ---------------------------------------------MDT-- 3
OSJNBb0020O11.1protein_id-"XP_          MEVEARIHLLLIKLVFYRSAEERESASVHINLLPVRELYFDPRKKMDA-- 48
ANAC007/EMB2749"                        ---------------------------------------------MNS-- 3
ANAC026"AT1G62700"                      ---------------------------------------------MNS-- 3
11980.m06650                            -------------------------------------------------M 1
OSJNBa0053C23.15"NP_922493.1"N          --------------------------------------------------
11973.m05887                            --------------------------------------------------
OJ1015F07.11"                           --------------------------------------------------
ANAC037"AT2G18060"NP_179397.1"          ---------------------------------------------MEPME 5
ANAC076"AT4G36160"NP_195339.1"          ---------------------------------------------MESVD 5
medicagoABE89364.1|                     -----------------------------------------------MME 3
Ze567"NAC-domain                        ---------------------------------------------MDTMN 5
ANAC105"At5g66300"AAU94387.1"           ---------------------------------------------MMKVD 5
Maizenac6CAH56056.1                     ---------------------------------------------MHPGV 5
ANAC030"AT1G71930"                      ---------------------------------------------MDN-- 3
CDS4054                                 -------------------------------------------MDRHEEE 7
11978.m04282                            -------------------------------------------MDRHEEE 7
translationAK071064sense                --------------------------------------------------
```

```
OSJNBa0075G19.15"NAM-like               TQQQAQVPPGFRFHPTDEELVDYYLRKKVAARRIDLNVIKDVDLYKIEPW 60
ANAC101"AT5G62380"id="NP_20104          ---LAHIPPGYRFHPTDEELVDYYLKNKVAFPGMQVDVIKDVDLYKIEPW 50
11972.m09332                            ---FSHVPPGFRFHPTDEELVDYYLRKKVASKKIDLDVIKDVDLYKIEPW 50
OSJNBb0020O11.1protein_id="XP_          ---FSHVPPGFRFHPTDEELVDYYLRKKVALKKIDLDVIKDIDLYKIEPW 95
ANAC007/EMB2749"                        ---FSHVPPGFRFHPTDEELVDYYLRKKVASKRIEIDFIKDIDLYKIEPW 50
ANAC026"AT1G62700"                      ---FSQVPPGFRFHPTDEELVDYYLRKKVASKRIEIDIIKDVDLYKIEPC 50
11980.m06650                            VIMESCVPPGFRFHPTDEELVGYYLRKKVASQKIDLDVIRDVDLYRIEPW 51
OSJNBa0053C23.15"NP_922493.1"N          --------------------------------------------------
11973.m05887                            --MESCVPPGFRFHPTDEELVGYYLRKKVASQKIDLDVIRDIDLYRIEPW 48
OJ1015F07.11"                           --MESCVPPGFRFHPTDEELVGYYLRKKVASQKIDLDVIRDIDLYRIEPW 48
ANAC037"AT2G18060"NP_179397.1"          S---CSVPPGFRFHPTDEELVGYYLRKKIASQKIDLDVIRDIDLYRIEPW 52
ANAC076"AT4G36160"NP_195339.1"          QS--CSVPPGFRFHPTDEELVGYYLRKKVASQKIDLDVIRDIDLYRIEPW 53
medicagoABE89364.1|                     S----CVPPGFRFHPTDGELVGYYLRKKVASHKIDLDVIKEIDLYRIEPW 49
Ze567"NAC-domain                        QSS-CTVPPGFRFHPTDEELVGYYLRKKVASQKIDLDVIKDIDLYRIEPW 54
ANAC105"At5g66300"AAU94387.1"           QDYSCSIPPGFRFHPTDEELVGYYLKKKIASQRIDLDVIREIDLYKIEPW 55
Maizenac6CAH56056.1                     G--PLSVPPGFRFHPTDEELLYYYLRKKVAYEPIDLDVIREIDLNKLEPW 53
ANAC030"AT1G71930"                      -IMQSSMPPGFRFHPTEEELVGYYLDRKINSMKSALDVIVEIDLYKMEPW 52
CDS4054                                 AGESPCVPPGFRFHPTEEELVGYYLARKVASQKIDLDIIQELDLYRIEPW 57
11978.m04282                            AGESPCVPPGFRFHPTEEELVGYYLARKVASQKIDLDIIQELDLYRIEPW 57
translationAK071064sense                --------------------------------------------------
```

**MOTIF I**

**NAC domain**

FIGURE 5

```
OSJNBa0075G19.15"NAM-like             DLQERCRINGGSAAEEQNEWYFFSHKDKKYPTGTRTNRATAAGFWKATGR 110
ANAC101"AT5G62380"id="NP_20104        DIQELC----GRGTGEEREWYFFSHKDKKYPTGTRTNRATGSGFWKATGR 96
11972.m09332                          DLQEKCK----IGMEEQNDWYFFSHKDKKYPTGTRTNRATGAGFWKATGR 96
OSJNBb0020O11.1protein_id="XP_        DLQEQCK----IGNEEQNEWYFFSHKDKKYPTGTRTNRATTAGFWKATGR 141
ANAC007/EMB2749"                      DLQELCK----IGHEEQSDWYFFSHKDKKYPTGTRTNRATKAGFWKATGR 96
ANAC026"AT1G62700"                    DLQELCK----IGNEEQSEWYFFSHKDKKYPTGTRTNRATKAGFWKATGR 96
11980.m06650                          DLQEHCR----IGYEEQSEWYFFSYKDRKYPTGTRTNRATMTGFWKATGR 97
OSJNBa0053C23.15"NP_922493.1"N        --------------------------------------------------
11973.m05887                          DLQEHCG----IGYDEQSEWYFFSYKDRKYPTGTRTNRATMAGFWKATGR 94
OJ1015F07.11"                         DLQEHCG----IGYDEQSEWYFFSYKDRKYPTGTRTNRATMAGFWKATGR 94
ANAC037"AT2G18060"NP_179397.1"        DLQEQCR----IGYEEQNEWYFFSHKDKKYPTGTRTNRATMAGFWKATGR 98
ANAC076"AT4G36160"NP_195339.1"        DLQESCR----IGYEERNEWYFFSHKDKKYPTGTRTNRATMAGFWKATGR 99
medicagoABE89364.1|                   DLQERCR----IGNEEQHEWYFFSHKDKKYPTGTRTNRATMAGFWKATGR 95
Ze567"NAC-domain                      DLIERCR----IGYEEQNEWYFFSHKDKKYPTGTRTNRATMAGFWKATGR 100
ANAC105"At5g66300"AAU94387.1"         DLQERCR----IGYEEQTEWYFFSHRDKKYPTGTRTNRATVAGFWKATGR 101
Maizenac6CAH56056.1                   DLKERCK----IGTGPQNEWYFFSHKDKKYSTGTRTNRATTAGFWKATGR 99
ANAC030"AT1G71930"                    DIQARCK----LGYEEQNEWYFFSHKDRKYPTGTRTNRATAAGFWKATGR 98
CDS4054                               DLQERCKYG-GHGGDEQTEWYFFSYKDRKYPSGTRTNRATAAGFWKATGR 106
11978.m04282                          DLQERCKYG-GHGGDEQTEWYFFSYKDRKYPSGTRTNRATAAGFWKATGR 106
translationAK071064sense              --------------------------------------------------

                                             MOTIF II
```

**NAC domain**

```
OSJNBa0075G19.15"NAM-like             DKPIYATKQHSLLVGMRKTLVYYRGRAPNGHKSDWIMHEYR-LETTETAP 159
ANAC101"AT5G62380"id="NP_20104        DKAIYSKQE---LVGMRKTLVFYKGRAPNGQKSDWIMHEYR-LETDENGP 142
11972.m09332                          DKPIYARSC---LVGMRKTLVFYKGRAPNGQKSDWIMHEYR-LETNENGT 142
OSJNBb0020O11.1protein_id="XP_        DKPIYVKNC---LVGMRKTLVFYRGRAPNGQKSDWIMHEYR-LETNEYGA 187
ANAC007/EMB2749"                      DKAIYLRHS---LIGMRKTLVFYKGRAPNGQKSDWIMHEYR-LETDENGT 142
ANAC026"AT1G62700"                    DKAIYIRHS---LIGMRKTLVFYKGRAPNGQKSDWIMHEYR-LETSENGT 142
11980.m06650                          DKAVRERSR---LIGMRKTLVFYKGRAPNGHKTDWIVHEYR-LESDENAP 143
OSJNBa0053C23.15"NP_922493.1"N        --------------------------------------------------
11973.m05887                          DKAVHDKSR---LIGMRKTLVFYKGRAPNGQKTDWIMHEYR-LETDENAP 140
OJ1015F07.11"                         DKAVHDKSR---LIGMRKTLVFYKGRAPNGQKTDWIMHEYR-LETDENAP 140
ANAC037"AT2G18060"NP_179397.1"        DKAVYDKTK---LIGMRKTLVFYKGRAPNGKKSDWIMHEYR-LESDENAP 144
ANAC076"AT4G36160"NP_195339.1"        DKAVYDKSK---LIGMRKTLVFYKGRAPNGQKTDWIMHEYR-LESDENAP 145
medicagoABE89364.1|                   DKSVYERTK---LIGMRKTLVFYKGRAPNGQKTDWIMHEYR-LETVENAP 141
Ze567"NAC-domain                      DKAVYEKLR---LIGMRKTLVFYKGRAPNGQKTDWIMHEYR-LESEENGP 146
ANAC105"At5g66300"AAU94387.1"         DKAVYLNSK---LIGMRKTLVFYRGRAPNGQKSDWIIHEYYSLESHQNSP 148
Maizenac6CAH56056.1                   DKAIFLGSAR--RIGLRKTLVFYIGRAPHGKKTEWIMHEYR--LDEENVE 145
ANAC030"AT1G71930"                    DKAVLSKNS---VIGMRKTLVYYKGRAPNGRKSDWIMHEYR-LQNSELAP 144
CDS4054                               DKPVLSSPSTR-VIGMRKTLVFYKGRAPNGRKTDWIIHEYR-LQSNEHAP 154
11978.m04282                          DKPVLSSPSTR-VIGMRKTLVFYKGRAPNGRKTDWIIHEYR-LQSNEHAP 154
translationAK071064sense              --------------------------------------------------
```

**NAC domain**

FIGURE 5 (continued)

```
OSJNBa0075G19.15"NAM-like              PQ--EEGWVVCRVFKKRLPTTRRDSDHDAPCGSWYVDEDAPGAFMSPMMI 207
ANAC101"AT5G62380"id="NP_20104         PH--EEGWVVCRAFKKKLTTMNYN-NPRTMMGSSSGQESN--WFTQQMDV 187
11972.m09332                           TP--EEGWVVCRVFKKRVAT-VRRMA--DG--SPCWFDDHGAVGAFMPDL 185
OSJNBb0020O11.1protein_id="XP_         PQ--EEGWVVCRVFKKRVAA-VQRAAG-DGGDSPFWFNEHVAFMAPAPGL 233
ANAC007/EMB2749"                       PQ--EEGWVVCRVFKKRLAA-VRRMGDYDSSPS-HWYDDQLSFMASELET 188
ANAC026"AT1G62700"                     PQ--EEGWVVCRVFKKKLAATVRKMGDYHSSPSQHWYDDQLSFMASEIIS 190
11980.m06650                           PQ--EEGWVVCRAFKKRTMQPPRSSIGAWEAS------YSYHDPAVFVGG 185
OSJNBa0053C23.15"NP_922493.1"N         ------------------MQPPRSSIGAWEAS------YSYHDPAVFVGG 26
11973.m05887                           PQ--EEGWVVCRAFKKRTAYPARSMVETWDYSLHERNIMSAAAAAAFADP 188
OJ1015F07.11"                          PQ--EEGWVVCRAFKKRTAYPARSMVETWDYSLHERNIMSAAAAAAFADP 188
ANAC037"AT2G18060"NP_179397.1"         PQ--EEGWVVCRAFKKR-ATGQAKNTETWSS-SYFYDEVAPNGVNSVMDP 190
ANAC076"AT4G36160"NP_195339.1"         PQ--EEGWVVCRAFKKKPMTGQAKNTETWSS-SYFYDELP-SGVRSVTEP 191
medicagoABE89364.1|                    PQASEEGWVVCKAFKKK-TSVQAKTNERWDP-SHLHDEQT-SSIISRVDP 188
Ze567"NAC-domain                       PQ--EEGWVVCRAFKKR-ATGQTRTTPAWES-NYFLDESS--LLTSTMD- 189
ANAC105"At5g66300"AAU94387.1"          PQ--EEGWVVCRAFKKR-TTIPTKRRQLWDPNCLFYDDAT---LLEPLDK 192
Maizenac6CAH56056.1                    IQ--EDGWVVCRVFKKK----NYEQRGHNMAEMAALDDDELQPFTVPVVP 189
ANAC030"AT1G71930"                     VQ--EEGWVVCRAFRKPIPNQRPLGYEPWQNQLYHVESSNNYSSSVTMNT 192
CDS4054                                TQ--EEGWVVCRAFQKPMPNQQQHRLSYGCIPGSYGAGAYAAVPDNYSLL 202
11978.m04282                           TQ--EEGWVVCRAFQKPMPNQQQHRLSYGCIPGSYGAGAYAAVPDNYSLL 202
translationAK071064sense               MQ--EEGWVVCRAFQKPMPNQQQHRLSYGCIPGSYGAGAYAAVPDNYSLL 48

                                              MOTIF III

                                             NAC domain

OSJNBa0075G19.15"NAM-like              TRSSILRPHQHHAG-------ITLQEQHLHTTYK-------------HRD 237
ANAC101"AT5G62380"id="NP_20104         GNG------NYYHL-------PDLESPRMFQGSS-------------SSS 211
11972.m09332                           SSPRQLLPHHHHHHPGSSAALYHGHHHQQLQQMYG----------HCKPE 225
OSJNBb0020O11.1protein_id="XP_         DSP------------------YHGHRQSHP----------------CKLE 249
ANAC007/EMB2749"                       NGQRRILPNHHQQQ-------QHEHQQHMPYGLNASAYALNNPNLQCKQE 231
ANAC026"AT1G62700"                     SSPRQFLPNHHYN--------RHHHQQTLPCGLN--AFNNNNPNLQCKQE 230
11980.m06650                           GEHFKQEAAAE----------LDG-----VAAAA------------GANA 208
OSJNBa0053C23.15"NP_922493.1"N         GEHFKQEAAAE----------LDG-----VAAAA------------GANA 49
11973.m05887                           SAAYAQMRRQH----------RSGRFKQEAELDG------------AATA 216
OJ1015F07.11"                          SAAYAQMRRQH----------RSGRFKQEAELDG------------AATA 216
ANAC037"AT2G18060"NP_179397.1"         IDYISKQQHNI-FG-------KGLMCKQELEGMV------------DGIN 220
ANAC076"AT4G36160"NP_195339.1"         LNYVSKQKQNV-FA-------QDLMFKQELEGSD------------IGLN 221
medicagoABE89364.1|                    IDLILRQPQRI-SA-------QNFMYKQEIEAEA------------DTLL 218
Ze567"NAC-domain                       -DYTTIQPSNIPLT-------SSFMCKQELEAS-------------ENLN 218
ANAC105"At5g66300"AAU94387.1"          RARHNPDFTATPFK-------QELLSEASHVQDG------------DFGS 223
Maizenac6CAH56056.1                    AGTSSLPTTDHKNN-------PHLMQYDFPSFDP-------------SMQ 219
ANAC030"AT1G71930"                     SHHIGASSSSHNLN-------QMLMSNNHYNPNN------------TSSS 223
CDS4054                                LHHDNPSFAGRPLMSAAASALFANNNNNSVVDHSN----------ILSSE 242
11978.m04282                           LHHDNPSFAGRPLMSAAASALFANNNNNSVVDHSN----------ILSSE 242
translationAK071064sense               LHHDNPSFAGRPLMSAAASALFANNNNNSVVDHSN----------ILSSE 88
```

FIGURE 5 (continued)

```
OSJNBa0075G19.15"NAM-like           LTTKIQ----------QLQVP-AAGHHLLNTMPHDLES------------ 264
ANAC101"AT5G62380"id="NP_20104      LSSLHQ----------NDQDPYGVVLSTINATPTTIMQ------------ 239
11972.m09332                        LEYHH-----------LLPQEAF-LQHLPQLESPKPPPPPPAAAAYIGG- 262
OSJNBb0020O11.1protein_id="XP_      VEYHHH----------LLPQEAAPFMHLPRLESPKLP-----AADII--- 281
ANAC007/EMB2749"                    LELHYNHLVQR---NHLLDESHLSFLQLPQLESPKIQQDNSNCNSLPYGT 278
ANAC026"AT1G62700"                  LELHYNQMVQHQQQNHHLRES--MFLQLPQLESP-----TSNCN------ 267
11980.m06650                        FLRYST-----------------RLAELPQLESP---------------- 225
OSJNBa0053C23.15"NP_922493.1"N      FLRYST-----------------RLAELPQLESP---------------- 66
11973.m05887                        LLHYSS-----------------HLAELPQLESPSAAA------------ 237
OJ1015F07.11"                       LLHYSS-----------------HLAELPQLESPSAAA------------ 237
ANAC037"AT2G18060"NP_179397.1"      -YIQSN-----------------QFIQLPQLQSPSLPL------------ 240
ANAC076"AT4G36160"NP_195339.1"      -FIHCD-----------------QFIQLPQLESPSLPL------------ 241
medicagoABE89364.1|                 RFMHSE-----------------QLVPLPQLQSPS--L------------ 237
Ze567"NAC-domain                    -FIHCD-----------------QFIQLPHLESPSLQS------------ 238
ANAC105"At5g66300"AAU94387.1"       MYLQCID--------------DDQFSQLPQLESPSLPS------------ 247
Maizenac6CAH56056.1                 LPQLMS-----------------ADQPVPTLFPSHPGV------------ 240
ANAC030"AT1G71930"                  MHQYGN-------------------IELPQLDSPSLSP------------ 242
CDS4054                             SKLHFS-------------------DMMPPLESPTIVDGEGYVSQASSCV 273
11978.m04282                        SKLHFS-------------------DMMPPLESPTIVDGEGYVSQASSCV 273
translationAK071064sense            SKLHFS-------------------DMMPPLESPTIVDGEGYVSQASSCV 119


OSJNBa0075G19.15"NAM-like           ----STSSFHSLLVSPDHHQINMHHAQADPFFDDMHAVDQA--------- 301
ANAC101"AT5G62380"id="NP_20104      ----RDD--GHVITNDDDHMIMMNTSTGDHHQSGLLVNDDHN-------D 276
11972.m09332                        ---HLGSSSSTALTTHDDEASGSAAQQQPPSLEAVYMAGAGVG--IGVDA 307
OSJNBb0020O11.1protein_id="XP_      ----VATAASSALQP----CGHTTAQQLQLQIEPVYVT---------ADA 314
ANAC007/EMB2749"                    SNIDNNSSHNANLQQS-NIAHEEQLNQGNQNFSSLYMNSGNE----QVMD 323
ANAC026"AT1G62700"                  SDNNNNTRNISNLQKSSNISHEEQLQQGNQSFSSLYYDQGVE----QMT- 312
11980.m06650                        --PLPSQGSQAASAVVDG--EEDNADSSRRPGGG----------GGAAAA 261
OSJNBa0053C23.15"NP_922493.1"N      --PLPSQGSQAASAVVDG--EEDNADSSRRPGGG----------GGAAAA 102
11973.m05887                        -APLQPNPSQLATAGEDDDCKGDNGGRRAKKARA----------AGDKVA 276
OJ1015F07.11"                       -APLQPNPSQLATAGEDDDCKGDNGGRRAKKARA----------AGDKVA 276
ANAC037"AT2G18060"NP 179397.1"      -MKRPSSSMSITSMDNNYN-YKLPLADEE-SFESFIR-GEDRRKKKKQVM 286
ANAC076"AT4G36160"NP_195339.1"      -TKRPVSLTSITSLEKNKNIYKRHLIEEDVSFNALISSGNKDKKKKKTSV 290
medicagoABE89364.1|                 -TKRQNS----------------MPIISEK-------------------- 250
Ze567"NAC-domain                    -IKRPISSILYEHDQQEDDQITKRITNNVRSKDE---------------- 271
ANAC105"At5g66300"AAU94387.1"       -EITPHSTTFSENSSRKDD-----MSSEK--------------------- 270
Maizenac6CAH56056.1                 -ATAMSSSIDVECSQNLLTMLTSNGSDGMLHVRAGGAGAG-----VDRFA 284
ANAC030"AT1G71930"                  ------------SLGTNKDQNESFEQEEEKSFN----------------- 263
CDS4054                             DVDQQAGIVDWNLLTSLLPPPAHQLFHHLPSAS---SSKN---------- 310
11978.m04282                        DVDQQAGIVDWNLLTSLLPPPAHQLFHHLPSAS---SSKN---------- 310
translationAK071064sense            DVDQQAGIVDWNLLTSLLPPPAHQLFHHLPSASGVITSRVRGD--GAAAA 167
```

FIGURE 5 (continued)

```
OSJNBa0075G19.15"NAM-like          TTTDWRVLDKFVASQLSN-----DATNKPAD-------------HYTDEG 333
ANAC101"AT5G62380"id="NP_20104     QVMDWQTLDKFVASQLIMSQEEEEVNKDPSD-------------NSSNE- 312
11972.m09332                       SVTDWRLLDKFVASQLLSKE---SMSSYGS---------------HPAQV 339
OSJNBb0020O11.1protein_id="XP_     SAADWRDLDKLVASQFGHGD---STAKEPSY-------------CNPVQV 348
ANAC007/EMB2749"                   QVTDWRVLDKFVASQLSNEE---AATASASI-------------QNNAK- 356
ANAC026"AT1G62700"                 --TDWRVLDKFVASQLSNDEEAAAVVSSSSH-------------QNNVKI 347
11980.m06650                       VTTDWRAFDKFVASQLSPEEQHTCRAT----------------------D 289
OSJNBa0053C23.15"NP_922493.1"N     VTTDWRAFDKFVASQLSPEEQHTCRAT----------------------D 130
11973.m05887                       TTTDWRALDKFVASQLSPGECGSMEATAEAAAA-----AVAGVSSPLDHG 321
OJ1015F07.11"                      TTTDWRALDKFVASQLSPGECGSMEATAEAAAA-----AVAGVSSPLDHG 321
ANAC037"AT2G18060"NP_179397.1"     MTGNWRELDKFVASQLMSQEDNGTSSFAGHH---------IVNEDKNNN- 326
ANAC076"AT4G36160"NP_195339.1"     MTTDWRALDKFVASQLMSQED-GVSGFGGHHEEDNNKIGHYNNEESNNKG 339
medicagoABE89364.1|                -VTDWRDLDKFVASQLS-------------------------QEDHRHET 274
Ze567"NAC-domain                   -VRDWRDLDKFVASQLSQEEEIRCVEEG-------------FSTNFQENI 307
ANAC105"At5g66300"AAU94387.1"      RITDWRYLDKFVASQFL--------------------------------- 287
Maizenac6CAH56056.1                GTTDWSILDKLLASHQNLGQLFHGKVTAASASP------MAPYHQQLMEL 328
ANAC030"AT1G71930"                 -CVDWRTLDTLLETQVIHPHN-------------------------PNIL 287
CDS4054                            -----------------------------------------SNNISSSGF 319
11978.m04282                       -----------------------------------------SNNISSSGF 319
translationAK071064sense           AAAGWRGRTGWRPSPGWPSGASTPSAPPPTAAP------IPSRSASGTGS 211


OSJNBa0075G19.15"NAM-like          DILQVSDKQQEV------------------AAADYASTSTSSSQIDPWK- 364
ANAC101"AT5G62380"id="NP_20104     TFHHLSEEQAAT------------------MVSMNASSSSSPCSFYSWAQ 344
11972.m09332                       FQAADGGKHEEA----------------LDYAS--TSAGSGGGEADLWK- 370
OSJNBb0020O11.1protein_id="XP_     FQVE--GKQEDS----------------LDYVS--TSASCGG-EEDLWK- 376
ANAC007/EMB2749"                   DTSNAEYQVDEE------KDPKRASDMGEEYT----ASTSSSCQIDLWK- 395
ANAC026"AT1G62700"                 DTRNTGYHVIDEGINLPENDSERVVEMGEEYSNAHAASTSSSCQIDL--- 394
11980.m06650                       DDDMAALLLLDGGGQEDDAGRWLGSAGLLSAVAADATTDCGLGTSCVPGD 339
OSJNBa0053C23.15"NP_922493.1"N     DDDMAALLLLDGGGQEDDAGRWLGSAGLLSAVAADATTDCGLGTSCVPGD 180
11973.m05887                       DDDMAALLFLNSD-ERDEVDRWTG---LLGSGAGASGVDGDLG-ICVFDK 366
OJ1015F07.11"                      DDDMAALLFLNSD-ERDEVDRWTG---LLGSGAGASGVDGDLG-ICVFDK 366
ANAC037"AT2G18060"NP_179397.1"     DVEMDSSMFLSERE--------EENRFVSEFLSTNS--DYDIG-ICVFDN 365
ANAC076"AT4G36160"NP_195339.1"     SVETASSTLLSDRE--------EENRFISGLLCSNL--DYDLY-RDLHV- 377
medicagoABE89364.1|                SSDMSLFLLQSSKD--------DEENKLSSFLTTRS--DCDIG-ICVFEN 313
Ze567"NAC-domain                   DHSTSLSHMFNYQDGIEEYSGCDKGGKLNGFLSSTSSDHFDVG-ICIFDK 356
ANAC105"At5g66300"AAU94387.1"      ---------MSGED------------------------------------ 292
Maizenac6CAH56056.1                GWLVVVVVLAEASTAVPQRRDDRSPQVPQVTTGSTSSLATTLSSDHTRTL 378
ANAC030"AT1G71930"                 MFETQSYNPAPSFP-----------SMHQSYNEVEANIHHSLGCFPDS-- 324
CDS4054                            IDD------RD--------------------------------------- 324
11978.m04282                       IDD------RD--------------------------------------- 324
translationAK071064sense           LPNPNPPLLRDHRFALSNQIIRQFDPNPCRYLSVEKARYVYVGLFPEPGR 261


OSJNBa0075G19.15"NAM-like          ---------------------
ANAC101"AT5G62380"id="NP_20104     NTHT----------------- 348
11972.m09332                       ---------------------
OSJNBb0020O11.1protein_id="XP_     ---------------------
ANAC007/EMB2749"                   ---------------------
ANAC026"AT1G62700"                 ---------------------
11980.m06650                       IN------------------- 341
OSJNBa0053C23.15"NP_922493.1"N     IN------------------- 182
11973.m05887                       ---------------------
OJ1015F07.11"                      ---------------------
ANAC037"AT2G18060"NP_179397.1"     ---------------------
ANAC076"AT4G36160"NP_195339.1"     ---------------------
medicagoABE89364.1|                ---------------------
Ze567"NAC-domain                   L-------------------- 357
ANAC105"At5g66300"AAU94387.1"      ---------------------
Maizenac6CAH56056.1                VDY------------------ 381
ANAC030"AT1G71930"                 ---------------------
CDS4054                            ---------------------
11978.m04282                       ---------------------
```

FIGURE 5 (continued)

```
CDS4035                             -------------------------------MG--MGMRRERDAEAELNL 17
AY103772.1zeamaystranslation        -------------------------------MCQPVTRRRERDAEAELDL 19
taNAC2                              -------------------------------MGMPAVRRRERDAEAELNL 19
TAGRAB1CAA09371.1"                  -------------------------------MVMAAAER--RDAEAELNL 17
riceNP_911548.1"                    ---------------------------------MAAAKRRVRDAEADLNL 17
elaeis                              ------------------------------------MGSRRRDAEAELNL 14
OsNAC4                              -------------------------------MAAAVGGSGRRDAEAELNL 19
GmNAC2"AAY46122.1"                  -------------------------------------------MASELEL 7
PetuniaNH10="nam-like               ------------------------------------------MTTAELQL 8
BrNAC18"NAC-domain                  -------------------------------------------MSEL-QL 6
ATAF1                               -------------------------------------------MSELLQL 7
PrunusPm74"NAC                      ------------------------------------------MSSSDLQL 8
OsNAC6"protein_id="BAA89800.1"      --------------------------------MSGG---------QDLQL 9
Saccharum                           --------------------------------MSGGG--------QDLQL 10
AK068392"putative                   --------------------------------MSGGGEGAAAAERQELQL 18
OsNAC5"BAA89799.1"                  -----------------------------------------MECGGALQL 9
tomatoNAC                           -------------------------------------MNKGANGNQQLEL 13
solanum"putative                    -------------------------------------MNKGATGNQQLEL 13
CaNAC1                              -------------------------------------MIKGIVGNQQLGL 13
BrassicaNAC5-8"NAC-domain           -------------------------------------------MKAGLNL 7
AT5G08790"="ATAF2"NP_680161.1"      -------------------------------------------MKSELNL 7
ANAC102                             MDFALFSSISIFEINHKDPPIRRFIKTQNRILSTRKQQGTFPKMKAELNL 50
NAC69-3"TaNAC69-3""NAC              -------------------------------------MPMGSSSAAMPAL 13
NAC69-1"TaNAC69""NAC                -------------------------------------MPMG-SSAAMPAL 12
CDS4034translationNAC3              ---------------------------------------MPSSGGAMPAL 11
medicagoNAClike                     ------------------------------------------MGTPQTNL 8
LeNAC-NOR"transcription             ----------------------------------MESTDSSTGTRHQPQL 16
ONAC10AKC63406                      ---------------------MESPDSSSGSAPPRVLRRQQQQPGSAPEL 29
Arabidopsis                         ------------------------------------MGVREKDPLAQLSL 14
ONAC24                              ----------------------------------------MAMQLSLPVL 10
                                                                                     *

CDS4035                             PPGFRFHPTDDELVEHYLCRKAAGQRLPVPIIAEVDLYKFDPWDLPERAL 67
AY103772.1zeamaystranslation        PPGFRFHPTDDELVEHYLCRKAAGQRLPVPIIAEVDLYRFDPWDLPERAL 69
taNAC2                              PPGFRFHPTDDELVEHYLCRKAAGQRLPVPIIAEVDLYRFDPWALPDRAL 69
TAGRAB1CAA09371.1"                  PPGFRFHPTDEELVADYLCARAAGRAPPVPIIAELDLYRFDPWELPERAL 67
riceNP_911548.1"                    PPGFRFHPTDEELVAHYLCPRAAGRAAPVPIIAELDLYRHDPWDLPHRAL 67
elaeis                              PPGFRFHPTDEELVVHYLCKKVACQRLPVPIIAEVDLYKYDPWDLPEKAL 64
OsNAC4                              PPGFRFHPTDEELVVHYLCRKVARQPLPVPIIAEVDLYKLDPWDLPEKAL 69
GmNAC2"AAY46122.1"                  PPGFRFHPTDEELVLHYLCRKCASQPIAVPIIAEIDLYKYDPWDLPGLAI 57
PetuniaNH10="nam-like               PPGFRFHPTDEELVMHYLCRKCASQPIAVPIIAEIDLYKYDPWDLPDLAL 58
BrNAC18"NAC-domain                  PPGFRFHPTDEELVMHYLCRKCASQSIAVPIIAEIDLYKYDPWELPGLAL 56
ATAF1                               PPGFRFHPTDEELVMHYLCRKCASQSIAVPIIAEIDLYKYDPWELPGLAL 57
PrunusPm74"NAC                      PPGFRFHPTDEELVKHYLCRKCQSQPISVPIIAEIDLYKYNPWDLPGLAL 58
OsNAC6"protein_id="BAA89800.1"      PPGFRFHPTDEELVMHYLCRRCAGLPIAVPIIAEIDLYKFDPWQLPRMAL 59
Saccharum                           PPGFRFHPTDEELVMHYLCRRCASLPIAVPIIAEIDLYKFDPWQLPRMAL 60
AK068392"putative                   PPGFRFHPTDEELVMHYLCRRCAGLPIAVPIIAEVDLYKFDPWHLPRMAL 68
OsNAC5"BAA89799.1"                  PPGFRFHPTDDELVMYYLCRKCGGLPLAAPVIAEVDLYKFNPWDLPERAM 59
tomatoNAC                           PAGFRFHPTDDELVQHYLCRKCAGQSIAVSIIAEIDLYKFDPWQLPEKAL 63
solanum"putative                    PAGFRFHPTDDELVQHYLCRKCAGQPIAVSIITEIDLYKFDPWQLPEKAL 63
CaNAC1                              PAGFRFHPTDEELVQHYLCRKCAGQSISVSIIAEIDLYKFDPWQLPEKAL 63
BrassicaNAC5-8"NAC-domain           PAGFRFHPTDEELVQFYLCRKCASEEISAPVIAEIDLYKFNPWELPEMSL 57
AT5G08790"="ATAF2"NP_680161.1"      PAGFRFHPTDEELVKFYLCRKCASEQISAPVIAEIDLYKFNPWELPEMSL 57
ANAC102                             PAGFRFHPTDEELVKFYLCRRCASEPINVPVIAEIDLYKFNPWELPEMAL 100
NAC69-3"TaNAC69-3""NAC              PPGFRFHPTDEELIVHYLGRQAASMPSPVPIIAEVNIYKCNPWDLPGKAL 63
NAC69-1"TaNAC69""NAC                PPGFRFHPTDEELIVHYLRRQAASMPSPVPIIAEVNIYKCNPWDLPGKAL 62
CDS4034translationNAC3              PPGFRFHPTDEELIVHYLMNQAASVKCPVPIIAEVNIYKCNPWDLPGKAL 61
medicagoNAClike                     PPGFRFHPTDAELILHYLRKKIASIPLPVSIIAEVDIYKLDPWDLPAKAS 58
LeNAC-NOR"transcription             PPGFRFHPTDEELIVHYLKKRVACAPIPVDIICEIDLYKFDPWELPAKAI 66
ONAC10AKC63406                      PPGFRFHPTDEELVVHYLKKKAASVPLPVTIIAEVDLYKFDPWDLPEKAN 79
Arabidopsis                         PPGFRFYPTDEELLVQYLCRKVAGYHFSLQVIGDIDLYKFDPWDLPSKAL 64
ONAC24                              PTGFRFHPTDEELVINYLQRRATGLSCPIPIIADVEIYNFNPWELPSMAL 60
                                    *.****:*** **:  **  :         :* ::::*. :** **  :

                                                                MOTIF IV
```

NAC DOMAIN

FIGURE 6

```
CDS4035                                  FGAREWYFFTPRDRKYPNGSRPNRAAGNGYWKATGADKPVAPRG------ 111
AY103772.1zeamaystranslation             FGAREWYFFTPRDRKYPNGSRPNRAAGDGYWKATGADKPVAPRAAAAD-- 117
taNAC2                                   FGTREWYFFTPRDRKYPNGSRPNRAAGNGYWKATGADKPVAPRG------ 113
TAGRAB1CAA09371.1"                       FGAREWYFFTPRDRKYPNGSRPNRAAGCGYWKATCADRPVARAG------ 111
riceNP_911548.1"                         FGRREWYFFTPRDRKYPNGSRPNRAAASGYWKATGADKPVLHNG------ 111
elaeis                                   FGLKEWYFFTPRDRKYPNGSRPNRAAGRGYWKATGADKPVTPKGS----- 109
OsNAC4                                   FGRKEWYFFTPRDRKYPNGSRPNRAAGRGYWKATGADKPVAPKGS----- 114
GmNAC2"AAY46122.1"                       YGEKEWYFFSPRDRKYPNGSRPNRAAGTGYWKATGADKPIGQPK------ 101
PetuniaNH10="nam-like                    YGEKEWYFFSPRDRKYPNGSRPNRAAGTGYWKATGADKPTGHPK------ 102
BnNAC18"NAC-domain                       YGEKEWYFFSPRDRKYPNGSRPNRSAGSGYWKATGADKPIGLPK------ 100
ATAF1                                    YGEKEWYFFSPRDRKYPNCSRPNRSAGSGYWKATGADKPIGLPK------ 101
PrunusPm74"NAC                           YGEKEWYFFSPRDRKYPNGSRPNRAAGSGYWKATGADKPIGSPK------ 102
OsNAC6"protein_id="BAA89800.1"           YGEKEWYFFSPRDRKYPNGSRPNRAAGSGYWKATGADKPVGSPK------ 103
Saccharum                                YGEKEWYFFSPRDRKYPNGSRPNRAAGSGYWKATGADKPVGTPK------ 104
AK068392"putative                        YGEKEWYFFSPRDRKYPNGSRPNRAAGSGYWKATGADKPVGTPR------ 112
OsNAC5"BAA89799.1"                       GGEKEWYFFSPRDRKYPNGQRPNRAAGTGYWKATGADKPVGSPR------ 103
tomatoNAC                                YGEKEWYFFSPRDRKYPNGSRPNRAAGTGYWKATGADKPVGKPK------ 107
solanum"putative                         YGEKEWYFFSPRDRKYPNGSRPNRAAGTGYWKATGADKPVGKPK------ 107
CaNAC1                                   YGEKEWYFFSQEDRKYPNGSRPNRAAGTGYWKATGADKPVGKPK------ 107
BrassicaNAC5-8"NAC-domain                YGEKEWYFFSPRDRKYPNGSRPNRAAGTGYWKATGADKPIGKPK------ 101
AT5G08790"="ATAF2"NP_680161.1"           YGEKEWYFFSPRDRKYPNGSRPNRAAGTGYWKATGADKPIGKPK------ 101
ANAC102                                  YGEKEWYFFSHRDRKYPNGSRPNRAAGTGYWKATGADKPIGKPK------ 144
NAC69-3"TaNAC69-3""NAC                   FGENEWYFFSPRDRKYPNGARPNRAAGSGYWKATGTDKAILSTPANES-- 111
NAC69-1"TaNAC69""NAC                     FGENEWYFFSPRDRKYPNGARPNRAAGSGYWKATGTDKAILSTPANES-- 110
CDS4034translationNAC3                   FGENEWYFFSPRDRKYPNGARPNRAAGSGYWKATGTDKSILSTPTSDN-- 109
medicagoNAClike                          FGEKEWYFFSPRDRKYPNGARPNRAAASGYWKATGTDKTIVASLPCGGGR 108
LeNAC-NOR"transcription                  FGEQEWYFFSPRDRKYPNGARPNRAATSGYWKATGTDKPVFTSGGTQK-- 114
ONAC10AK063406                           FGEQEWYFFSPRDHKYPNGARPNRAATSGYWKATGTDKPIMSSGSTR--- 126
Arabidopsis                              FGEKEWYFFSPRDRKYPNGSRPNRVAGSGYWKATGTDKIITADGR----- 109
ONAC24                                   FGEHEWYFFILRDHRYPNSVRPSRSAASGFWKATGTDKPVQVANMQST-- 108
                                          *  .**:**:  .*::**. **.  *   *:*****:*: :
                                                           MOTIF V
```

NAC DOMAIN

```
CDS4035                                  ---RTLGIKKALVFYAGKAPRGVKTDWIMHEYRLA----DAGRA------ 148
AY103772.1zeamaystranslation             --ARTLGIKKALVFYAGKAPRGVKTDWIMHEYRLA----DAG-------- 153
taNAC2                                   --GRTMGIKKALVFYAGKAPKGVKTDWIMHEYRLA----DAGR------- 150
TAGRAB1CAA09371.1"                       ---RTVGIKKALVFYHGRPSAGVKTDWIMHEYRLA----GADGR------ 148
riceNP_911548.1"                         ---RTAGIKKALVFYHGKPPRGVKTEWIMHEYRLA----KKGGA------ 148
elaeis                                   --SRPLGIKKALVFYSGKAPRGVKTDWIMHEYRLA----DTNR------- 146
OsNAC4                                   --ARTVGIKKALVFYSGKAPRGVKTDWIMHEYRLA----DADR------- 151
GmNAC2"AAY46122.1"                       ----PVGIKKALVFYAGKAPKGDKSNWIMHEYRLA----DVDRS------ 137
PetuniaNH10="nam-like                    ----AVGIKKALVFYAGKAPKGEKTNWIMHEYRLA----DVDRS------ 138
BnNAC18"NAC-domain                       ----PVGIKKALVFYAGKAPKGEKTNWIMHEYRLA----DVDRSS----A 138
ATAF1                                    ----PVGIKKALVFYAGKAPKGEKTNWIMHEYRLA----DVDRS------ 137
PrunusPm74"NAC                           ----PVGIKKALVFYAGKAPRGEKTNWIMHEYRLA----DVDRS------ 138
OsNAC6"protein_id="BAA89800.1"           ----PVAIKKALVFYAGKAPKGEKTNWIMHEYRLA----DVDRS------ 139
Saccharum                                ----PLAIKKALVFYAGKAPKGEKTNWIMHEYRLA----DVDRS------ 140
AK068392"putative                        ----PVAIKKALVFYAGKAPKGDKTNWIMHEYRLA----DVDRS------ 148
OsNAC5"BAA89799.1"                       ----AVATKKALVFYAGKPPKGVKTNWIMHEYRLA----DVDRSA----A 141
tomatoNAC                                ----TLGIKKALVFYAGKAPRGIKTNWIMHEYRLA----NVDRS------ 143
solanum"putative                         ----TLGIKKALVFYAGKAPRGIKTNWIMHEYRLA----NVDRS------ 143
CaNAC1                                   ----TLGIKKALVFYAGKAPRGIKTNWIMHEYRLA----NVDRS------ 143
BrassicaNAC5-8"NAC-domain                ----TLGIKKALVFYAGKAPKGIKTNWIMHEYRLA----NVDRS------ 137
AT5G08790"="ATAF2"NP_680161.1"           ----TLGIKKALVFYAGKAPKGIKTNWIMHEYRLA----NVDRS------ 137
ANAC102                                  ----TLGIKKALVFYAGKAPKGIKTNWIMHEYRLA----NVDRS------ 180
NAC69-3"TaNAC69-3""NAC                   -----TGVKKALVFYRGKPPKGVKTDWIMHEYRLT---AADNRT-----T 148
NAC69-1"TaNAC69""NAC                     -----IGVKKALVFYRGKPPKGVKTDWIMHEYRLT---AADNRT-----T 147
CDS4034translationNAC3                   -----IGVKKALVFYKGKPPKGVKIDWIMHEYRLTGTSANSTTT-----T 149
medicagoNAClike                          SQENIIGVKKALVFYKGKPPKGIKTNWIMHEYRLV----DNNKP-----I 149
LeNAC-NOR"transcription                  -----VGVKKALVFYGGKPPKGVKINWIMHEYRVVENKINNKPLGCDNIV 159
ONAC10AK063406                           ---EKVGVKKALVFYRGKPPKGVKINWIMHEYRLT----DTSSSAAAVAT 169
Arabidopsis                              ----RVGIKKALVFYAGKAPKGTKINWIMHEYRLI----EHSRS------ 145
ONAC24                                   ----PVAMKKALVFYVGRPPMETKITWIMHEYRLT----NTCCSTASHPS 150
                                                .:******* *:..    *: *******:
```

NAC DOMAIN

FIGURE 6 (continued)

```
CDS4035                             AAGAKK---------GSLRLDDWVICRLYNKKN-------EWEKMQQG-- 180
AY103772.1zeamaystranslation        -GRAKK---------GSLRLDDWVICRLYNKKN-------EWEKMRLG-- 184
taNAC2                              AAASKK---------GSLRLDDWVICRLYNKKN-------EWEKMQLQ-- 182
TAGRAB1CAA09371.1"                  AAKNG----------GTLRLDEWVICRLYNKKN-------QWEKMQRQ-- 179
riceNP_911548.1"                    AAAACA---------CALRLDDWVICRLYNKKN-------EWEKMQSR-- 180
elaeis                              AAN-KK---------GSLRLDDWVICRLYNKKN-------TWEKMQQ--- 176
OsNAC4                              APGGKK---------GSQKLDEWVICRLYNKKN-------NWEKVKLE-- 183
GmNAC2"AAY46122.1"                  VRKKN-----------TLRLDDWVICRIYNKKG-------TIEKLQP--- 166
PetuniaNH10="nam-like               ARKNNN----------SLRLDDWVICRIYNKKG-------SIEKNQL--- 168
BrNAC18"NAC-domain                  ARKKKN----------SLRLDDWVICRIYNKKG-------AIEKRG---- 167
ATAF1                               VRKKKN----------SLRLDDWVICRIYNKKG-------ATERRG---- 166
PrunusPm74"NAC                      PRKKSS----------SLRLDDWVICRIYNKKG-------TVEKQQQQ-- 169
OsNAC6"protein_id="BAA89800.1"      ARKKN-----------SLRLDDWVICRIYNKKG-------CLEKPPAAAV 171
Saccharum                           ARKKN-----------SLRLDDWVICRIYNKKG-------GLEKP----- 167
AK068392"putative                   ARKKN-----------TLRLDDWVICRIYNKKG-------GVEKP----- 175
OsNAC5"BAA89799.1"                  ARKLSKSSH------NALRLDDWVICRIYNKKG-------VIERYD---- 174
tomatoNAC                           AGKN-----------NNLRLDDWVICRIYNKKG-------TLEKHYN--- 172
solanum"putative                    AGKN-----------NNLRLDDWVICRIYNKKG-------TLEKHYN--- 172
CaNAC1                              AGKS-----------NNLRLDDWVICRIYNKKGH------TLRSITM--- 173
BrassicaNAC5-8"NAC-domain           ASVNKK---------NNLRLDDWVICRIYNKKG-------TMEKYYP--- 168
AT5G08790"="ATAF2"NP_680161.1"      ASVNKK---------NNLRLDDWVICRIYNKKG-------TMEKYFP--- 168
ANAC102                             ASTNKK---------NNLRLDDWVICRIYNKKG-------TMEKYLP--- 211
NAC69-3"TaNAC69-3""NAC              KRRGSS-----------MRLDDWVICRIHKKCNNLHNFSSSDQEQEHE-- 185
NAC69-1"TaNAC69""NAC                KRRGSS-----------MRLDDWVICRIHKKCGNLPNFSSSDQEQEHE-- 184
CDS4034translationNAC3              KQRRASS--------MTMRLDDWVICRIHKKSNDFN--SSDQHDQEPE-- 187
medicagoNAClike                     KLKDSS-----------MRLDDWVICRIYKKS------KCALTSTESS-- 180
LeNAC-NOR"transcription             ANKKGS-----------LRLDDWVICRIYKKN-------NTQRSIDDL-- 189
ONAC10AKC63406                      TRRPPPPITGGSKGAVSLRLDDWVICRIYKKTNKAGAGQRSMECEDSVED 219
Arabidopsis                         -HCSS-------------KLDDWVICRIYKKTG-------CSQRQAVT-- 172
ONAC24                              LSSSTAHP--------SVKLDEWVICKIFNKSP-------EPDNTAPP-- 183
                                                      :**:****:::.:*
                                                            MOTIF VI    MOTIF VII
```

NAC DOMAIN

```
                                                                                  MOTIF VIII

CDS4035                             ------------K-----EVK-----EEASDMVT-----SQSHSHTHSWG 203
AY103772.1zeamaystranslation        ------------KGAAAGAVKE----EEAMDMST-----SHSQ-ITHSWG 212
taNAC2                              ------------QQGEEETMMEPKAENTASDMVVTSHSHSQSQSHSHSWG 220
TAGRAB1CAA09371.1"                  ------------R-------------QEEEAAAK------AAASQSVSWG 198
riceNP_911548.1"                    ------------K-------------EEEEAMAA------AQS-----WG 194
elaeis                              ------------Q-------------KEAASFGE-----TMDS-VDDTGS 195
OsNAC4                              ------------Q-------------QDVASVAA-----AAPRNHHHQNG 203
GmNAC2"AAY46122.1"                  ------SSD-VAHSRNTES--SETEDRKPETLKSGGGCLPPPAPVPAPPQ 207
PetuniaNH10-"nam-like               ------NNKKIMNTSYMDMTVSSEEDRKPEIL-------PPLPPQPAPQQ 205
BrNAC18"NAC-domain                  ------------PPPTPVVYGDEVVEEKPRLSEMGM---PPP-----PVM 197
ATAF1                               ------------PPP-PVVYGDEIMEEKPKVTEMVM---PPP-----PQQ 195
PrunusPm74"NAC                      ------QQQQQQTTSRMSSCSEFEDRKP--ENLAC---PPPPAAVAPTR 208
OsNAC6"protein_id="BAA89800.1"      AAAGMVSSGGGVQRKPMV--GVNAAVSSPPEQKPVV---AGP-AEP---- 211
Saccharum                           AAA---SSG---DHKPMV--FAAGAVSSPPEQKPFV---ATPGGLPPAAF 206
AK068392"putative                   ------SGGGGGERSNMMSHGETASAGSPPEQKPAV---LPPPPPPYAAA 216
OsNAC5"BAA89799.1"                  ------TVDAGEDVKPAAAAAAAKGGRTGGGGGAAAMKVELSDYGFYDQF 218
tomatoNAC                           -----------VDNKETTSFGEFDEEIKPKILPTQLA--PMP----PRPR 205
solanum"putative                    -----------VDNKETASFGEFDEEIKPKILPTQLA--QMP----PRPR 205
CaNAC1                              -----------WASKRGESFGEFEDEIKPKIFPTQLA--PAPGQWPPRPQ 210
BrassicaNAC5-8"NAC-domain           -----------ADEK-------------------------------PMTV 176
AT5G08790"="ATAF2"NP_680161.1"      -----------ADEK-------------------------------PRTT 176
ANAC102                             -----------AAAE-------------------------------KPTE 219
NAC69-3"TaNAC69-3""NAC              --------QESSTTVEDSHNNHTVSSPKSEAFDGDGDDQLQLQQFRPMAI 227
NAC69-1"TaNAC69""NAC                --------QESS-TVEDSQNNHTVSSPKSEAFDGDGDDHLQLQQFRPMAT 225
CDS4034translationNAC3              --------ESTVEQLEDIHDNN--SSEQPPAPADMNNQQSDFQPMTAMSM 227
medicagoNAClike                     --------ETMVGEVEHAEETQ----FQETLFPITKNTTSPLQNT--LMS 216
LeNAC-NOR"transcription             --------HDMLGSIPQNVPNS-----ILQGIKPSNYGTILLENESNMYD 226
ONAC10AKC63406                      ---------AVAAYAPSSQQHATAAACMACSDCACCVAAAHCCDYSSLLE 260
Arabidopsis                         ---------------------------PVQACREEESTNGSSSSSSSQLD 195
ONAC24                              ----------------------------SNVVSRLQCSPPLPPPAAPPGN 205
```

FIGURE 6 (continued)

```
                                          MOTIF VIII          MOTIF XI

CDS4035                                   ETRTPESEIVD------NDPFPELDSFPAFQPAPPPATA------------ 236
AY103772.1zeamaystranslation              ETRTPESEIVD------ND--------------LPFPDPA----------- 232
taNAC2                                    EARTPESEIVD------NDPSLFQQATAAAFQAQSPAAAAAHQEMM----- 260
TAGRAB1CAA09371.1"                        ETRTPESD-VD------NDPFPELDSLP---EFQTANAS------------ 227
riceNP_911548.1"                          ETRTPESEVVD------SDAFPEMDYSL---PAASFDDA------------ 224
elaeis                                    DSERTPESDID------NDVMEPDEDDMAQVGAHPPQAFNGMQGVR----- 235
OsNAC4                                    EVMDAAAADTM------SDSFQTHDSDIDNASAG--LRHGGCGGGG----- 241
GmNAC2"AAY46122.1"                        ATAKTDYMYFD-----PSDSIPKLHT-DSSCSEQVVSPGFASE-------- 244
PetuniaNH10="nam-like                     QQVYNDFFYLD-----PSDSVPKIHS-DSSCSEHVVSPEFTCER------- 243
BrNAC18"NAC-domain                        P---NDFVYFD-----TSDSVPKLHTTESSCSEQVVSPEFTSE-------- 232
ATAF1                                     T---SEFAYFD-----TSDSVPKLHTTDSSCSEQVVSPEFTSE-------- 230
PrunusPm74"NAC                            P---NDYVYFD-----TSDSVPRLHT-DSSCSEHVVSPEFTCE-------- 242
OsNAC6"protein_id="BAA89800.1"            ----DLAAYYD----RPSDSMPRLH-ADSSCSEQVLSP-EFAC-------- 244
Saccharum                                 T--ADLAAYYD----RPSDSMPRLH-ADSSCSEQVLSPEQLACD------- 243
AK068392"putative                         APFSELAAFYDV---RPSDSVPRAHGADSSCSEHVLTTSASSGGVV---E 260
OsNAC5"BAA89799.1"                        P--ESEMLCFDRSGSADRDSMPRLHT-DSSGSEHVLSPSPSPDDFPGGGD 265
tomatoNAC                                 STPANDYFYFE-----SSESMT--RMHTTNSSSGSE-HVLSPCD------- 241
solanum"putative                          STPTNDYFHFE-----SSESMT--RMHTTNSSSGSE-HVLSPCD------- 241
CaNAC1                                    STPASDYFNFE-----TSESMTTTRMHTTNSSSGSE-HVLSSCD------- 248
BrassicaNAC5-8"NAC-domain                 TAA---SSPFD-----ASDSTYPTLQEDDSSSSGGR--VVSPDA------- 210
AT5G08790"="ATAF2"NP_680161.1"            TMAEQSSSPFD-----TSDSTYPTLQEDDSSSSGGEGHVVSPDV------- 215
ANAC102                                   KMS-------------TSDSRCSSHVTSPD--------VTCSDN------- 242
NAC69-3"TaNAC69-3""NAC                    AKSCSLTDLLNT----VDYAALSHLLLDGAGAGASSSDAGADYQLPPENP 273
NAC69-1"TaNAC69""NAC                      AKSCSLTDLLNT----VDYAALSHLLLD--GAGASSSDAGADYQLPPENP 269
CDS4034translationNAC3                    SKSCSLTDLLNT----IDCAALSQFLLD------GSSDAIAEPPAPP-SP 266
medicagoNAClike                           QKSVSFSNLLDA----MDYSMLSSFLSE------NSNNPSCICTSSCFNT 256
LeNAC-NOR"transcription                   GIMNNTNDIINN----NNRSIPQISSKR----TMHGGLYWNNDEATTTTT 268
ONAC10AK063406                            EDSHEDTFLVNG--LLTAEDAAGLSTGASSLSQLAAAARAAATPCDATKQ 308
Arabidopsis                               DVLDSFPEIKD-----QSFNLPRMNSLRTILNGNFDWASLAGLN------- 234
ONAC24                                    YPPLPVGATND------GGVFACAGDMLFTTQEHQEGTPSMLPP------- 243


                                                 MOTIF IX                      MOTIF X

CDS4035                                   ----MMVPKKESMDDATAAAAAAATI----PRNNSSLFVDLSYDDIQ--- 275
AY103772.1zeamaystranslation              ----MMVPKKERVDDGGSAR-------------TSDLFVDLSYDDIQ--- 262
taNAC2                                    --ATLMVPKKEAADEAG----------------RNDLFVDLSYDDIQ--- 289
TAGRAB1CAA09371.1"                        -----ILPKEEVQELGN------------------DDWLMGISLDDLQ--- 252
riceNP_911548.1"                          -----LLPKEEARD--------------------DDWLMGMSLDDLQ--- 246
elaeis                                    --VNNITGYQPAIEQRPKEE--------------NDWFTDLNLDDFH--- 266
OsNAC4                                    --FCDVAPPRNCFVT-VKED--------------NDWFTCLNFDELQ--- 271
GmNAC2"AAY46122.1"                        ---VQSEPK--WNEWEKS-----------------LEFPFNYVDAT---- 268
PetuniaNH10="nam-like                     --EVQSEAK--LSEWEKAA----------------LDLPFNYMDATTGAT 273
BrNAC18"NAC-domain                        ---VQSEPK--WKDWSGE--------------KSSLDFGFNYIDAT---- 259
ATAF1                                     ---VQSEPK--WKDWSAVSN----------DNNNTLDFGFNYIDATV--- 262
PrunusPm74"NAC                            ---VQSEPK--WKEWEKA-----------------LDFNYNYMDTSV--- 267
OsNAC6"protein_id="BAA89800.1"            --EVQSQPK--ISEWERT---------------FATVG----PINPAA--- 269
Saccharum                                 -REVQSQPK--ISEWERT---------------FASD-----PVNPAG--- 268
AK068392"putative                         RPEVQSQPK--IAEWERT---------------FACAAAPACAVSTAC--- 291
OsNAC5"BAA89799.1"                        EDYAESQPSGGCGGWPGVD-------------WAAVGDDGFVIDSSL--- 299
tomatoNAC                                 -KEVQSAPK--WDEDHR----------------NTLDFQLNYLDGLL--- 269
solanum"putative                          -KEVQSAPK--WDEDHR----------------NTLDFQLNYLDGLL--- 269
CaNAC1                                    -KEVQSAPK--WDDLGT----------------AALDFQINYLDGLL--- 276
BrassicaNAC5-8"NAC-domain                 -REVQSEPK--WGEFEN---------------AFDASMFGGGSMDLLQ--- 240
AT5G08790"="ATAF2"NP_680161.1"            -LEVQSEPK--WGELEDAL-----------EAFDTSMFG-SSMELLQ--- 247
ANAC102                                   -WEVESEPK--WINLEDAL-----------EAFNDDTSMFSSIGLLQ--- 275
NAC69-3"TaNAC69-3""NAC                    LIYSQP-PWQQTLHYNNNNN---------------GYVNIDTID------ 301
NAC69-1"TaNAC69""NAC                      LIYSQP-PWQQTLHYNNNN-----------------GYVNNETID------ 296
CDS4034translationNAC3                    LIYTTPHPNYQTLNYNINSNSSMPHAFESRLDHHDGYVNNYNVNGLRRKR 316
medicagoNAClike                           ENFNQQ----QSSHINTCNN----------------YMSQKNPQ------ 280
LeNAC-NOR"transcription                   TIDRNHSPNTKRFLVENNED---------------DGLNMNNIS------ 297
ONAC10AK063406                            LLAPSPTPFNWFEAFLPRAKEFPSG-----LSRSSRDTGDMSLSSTVDRS 353
Arabidopsis                               -PIPELAPTNGLPSYGG------------------YDAFRAAEGEAES--- 263
ONAC24                                    --IPNLEPPAATIGNSSLNG---------------TAAAAAAADGHG--- 273
```

## FIGURE 6 (continued)

```
                                      MOTIF X
CDS4035                               ---GMYSGLDMLPP-GDDF----------------------------YSSL 294
AY103772.1zeamaystranslation          ---GMYSGLDMLPPAGEDL----------------------------YSSL 282
taNAC2                                ---SMYNGLDMMPPGDDLL----------------------------YSSL 309
TAGRAB1CAA09371.1"                    ---CPGS---LMLPWDDSY----------------------------AASF 269
riceNP_911548.1"                      ---GLGS---LLQADD--------------------------------LSM 259
elaeis                                ---STCMAFGSTPALDMS---------------------------DHDYYYS 283
OsNAC4                                ---PPYMMNLQHMQMQMVNPAAPGH-------------------DGGYLQS 300
GmNAC2"AAY46122.1"                    -LNNSFM-AQFQGNNQMLS-------------------------------- 285
PetuniaNH10="nam-like                 TLDNSLLGSQFQSSYQMS--------------------------------- 291
BnNAC18"NAC-domain                    -----AEGGG-GGSNQLF--------------------------------- 271
ATAF1                                 ---DNAFGGG-GSSNQMF---------------------------------- 276
PrunusPm74"NAC                        ---ENGFGPQFQSANQMS---------------------------------- 282
OsNAC6"protein_id="BAA89800.1"        ----SIL--DPAGSGGLGGLGGGG--------------------------S 288
Saccharum                             ----SML--VDPVVGGHAG-------------------------------- 281
AK068392"putative                     ----PILGQLDPAAAVAGG-------------------------------G 307
OsNAC5"BAA89799.1"                    ----FELPSPAAFSRAAGDG------------------------------- 315
tomatoNAC                             ---NEPFETQMQQQICN-FDQFN---------------------------- 288
solanum"putative                      ---NEPFETQMQQQSCN-FDQFN---------------------------- 288
CaNAC1                                ---NDPFEIQMQQQNCN-LDQFN---------------------------- 295
BrassicaNAC5-8"NAC-domain             ---SEDFVPQFLYQPSYDFNSWQE--------------------------D 262
AT5G08790"="ATAF2"NP_680161.1"        ---PDAFVPQFLYQSDY-FTSFQ---------------------------D 267
ANAC102                               ---NDAFVPQFQYQSSDFVDSFQ---------------------------D 296
NAC69-3"TaNAC69-3""NAC                VPQIPEARVDDYGMNGDRYNGMKRK---------RSS--GSLYCS-QLQLP 340
NAC69-1"TaNAC69""NAC                  VPQLPEARVDDYGMNGDKYNGMKRK---------RSS--GSLYCS-QLQLP 335
CDS4034translationNAC3                MMACSATSFDDGSSSNDFVHAVVKKPQLLPSDSRCSGFGGGYCNQQLSET 366
medicagoNAClike                       ---SNTLKHQLSNVDEDMLYPSKKY---------LSS-----SCNFPNINS 314
LeNAC-NOR"transcription               ----RITNHEQSSSTANFLSQFPQN----------PS-----IQQQQQQE 329
ONAC10AK063406                        LSEAGAVAIDTGDAANGANTMPAFINPLG---VQGATYQQHQAIMGASLP 400
Arabidopsis                           ---GEVNRQQNSSCLTQSFG------------------------------- 280
ONAC24                                -----RLEEEDTSAYTFTD-------------------------------- 287


CDS4035                               FASPRVKGTTPRAGAGMGMVPF---- 316
AY103772.1zeamaystranslation          FASPRVRGNQPTGPAXLG--PF---- 302
taNAC2                                FASPRVRGSQP-GAGGMP-APF---- 329
TAGRAB1CAA09371.1"                    LSPVATMKMEQDVSPFFF-------- 287
riceNP_911548.1"                      LAPPPAAKTEPLGAPFF--------- 276
elaeis                                N-LPQLRSNQSDLLPF---------- 303
OsNAC4                                ISSPQMKMWQTILPPF---------- 316
GmNAC2"AAY46122.1"                    --PLQDMFMYWPNKSF---------- 299
PetuniaNH10="nam-like                 --PLQDMFMHL-EKPF---------- 304
BnNAC18"NAC-domain                    --PLQDMFMYNMPKPY---------- 285
ATAF1                                 --PLQDMFMY-MQKPY---------- 289
PrunusPm74"NAC                        --PLQDIFMY-LQKPY---------- 295
OsNAC6"protein_id="BAA89800.1"        DPLLQDILMY-WGKPF---------- 303
Saccharum                             DPLLQDILMY-WGKPF---------- 296
AK068392"putative                     DPLLQDILMY-WGKPF---------- 322
OsNAC5"BAA89799.1"                    -AAFGDMFTY-LQKPF---------- 329
tomatoNAC                             --NFQDMFLY-MQKPY---------- 301
solanum"putative                      --NFQDMFFY-MQKPY---------- 301
CaNAC1                                --TFQDMFLY-MQKP----------- 307
BrassicaNAC5-8"NAC-domain             PPEQKPFLNW-SFAPQG--------- 278
AT5G08790"="ATAF2"NP_680161.1"        PPEQKPFLNW-SFAPQG--------- 283
ANAC102                               PFEQKPFLNW-NFAPQG--------- 312
NAC69-3"TaNAC69-3""NAC                AD----QYSGMLIHPF-LSQQLHM-- 359
NAC69-1"TaNAC69""NAC                  AD----QYSGMLIHPF-LSQQLHM-- 354
CDS4034translationNAC3                ATGFQFQNGNLLSHPFPLNNHLQMQ- 391
medicagoNAClike                       QYENYLMKQSLMNQQLLLGPHHQYQQ 340
LeNAC-NOR"transcription               EVLGSLNDGVVFRQPYNQVTGMNWYS 355
ONAC10AK063406                        SESAAAAAACNFQHPFQLSRVNWDS- 425
Arabidopsis                           -YSSSGFGVSGQTFEFRQ-------- 297
ONAC24                                -QEMEQMLMDLMDQDFFGNDQPQE-- 310
```

FIGURE 6 (continued)

FIGURE 7

```
LOC_Os07g47790.1|11977.m08994|     ----------------------------------------------MCGG 4
LOC_Os07g47790.2|11977.m29326|     ----------------------------------------------MCGG 4
LOC_Os01g21120.1|11971.m08596|     ----------------------------------------------MCGG 4
LOC_Os03g08470.1|11973.m06355|     ----------------------------------------------MCGG 4
LOC_Os03g08470.2|11973.m34802|     ----------------------------------------------MCGG 4
AT2G47520.1                        ----------------------------------------------MCGG 4
LOC_Os02g54160.1|11972.m10445|     ----------------------------------------------MCGG 4
LOC_Os06g09390.1|11976.m05658|     ----------------------------------------------MCGG 4
LOC_Os09g26420.1|11979.m05794|     ----------------------------------------------MCGG 4
LOC_Os09g26420.2|11979.m21992|     ----------------------------------------------MCGG 4
LOC_Os09g26420.3|11979.m22092|     ----------------------------------------------MCGG 4
AT1G53910.1                        ----------------------------------------------MCGG 4
AT1G53910.2                        ----------------------------------------------MCGG 4
AT3G14230.1                        ----------------------------------------------MCGG 4
AT3G14230.2                        ----------------------------------------------MCGG 4
AT3G14230.3                        ----------------------------------------------MCGG 4
AT3G16770.1                        ----------------------------------------------MCGG 4
LOC_Os07g42510.1|11977.m08483|     ----------------------------------------------MCGG 4
LOC_Os03g08490.1|11973.m06357|     ----------------------------------------------MCGG 4
LOC_Os03g08500.1|11973.m06358|     ----------------------------------------------MCGG 4
AT1G72360.1                        ----------------------------------------------MSQS 4
LOC_Os03g08460.1|11973.m06354|     ----------------------------------------------MCGG 4
LOC_Os05g29810.1|11975.m07218|     ----------------------------------------------MCGG 4
LOC_Os03g22170.1|11973.m07620|     ----------------------------------------------MCGG 4
LOC_Os10g25170.1|11980.m05467|     -----------------------MSQTQSNSNQTHLPTPNPSRARAMCGG 27
LOC_Os09g11460.1|11979.m04408|     ----------------------------------------------MRR- 3
LOC_Os09g11460.2|11979.m21977|     ----------------------------------------------MRR- 3
LOC_Os09g11480.1|11979.m04410|     ----------------------------------------------MCGG 4
LOC_Os12g41030.1|11982.m07885|     --------------------------------------------------
LOC_Os12g41060.1|11982.m07888|     ----------------------------------------------MCGG 4
AT1G80580.1                        ----------------------------------------------MENS 4
```

FIGURE 7 (continued)

```
LOC_Os07g47790.1|11977.m08994|      AIISDFIPQREAHRAATG--SK------------RALCASDFWPSASQEA 40
LOC_Os07g47790.2|11977.m29326|      AIISDFIPQREAHRAATG--SK------------RALCASDFWPSASQEA 40
LOC_Os01g21120.1|11971.m08596|      AIIYDYIPAR------------------------RRLCASDFWP----DA 26
LOC_Os03g08470.1|11973.m06355|      AILAEFIPAPSRAAAATKRVTA------------SHLWPAGSKNAARGKS 42
LOC_Os03g08470.2|11973.m34802|      AILAEFIPAPSRAAAATKRVTA------------SHLWPAGSKNAARGKS 42
AT2G47520.1                         AIISDFIWSKS----------------------------ESEPSQLG-S 24
LOC_Os02g54160.1|11972.m10445|      AIIHHLKGHPEGSRRATEGLLWPEKKK------PRWGGGGRRHFGGFVEE 48
LOC_Os06g09390.1|11976.m05658|      AILSDLIPPP---RRVTAGDLWLEKTKKQQQ--QKKKNKGARRLP-LRQE 48
LOC_Os09g26420.1|11979.m05794|      AIISGFIPPS--AAAAAAAAVAKKQQGRRVT--ADVLWPGMLRKG-KAAA 49
LOC_Os09g26420.2|11979.m21992|      AIISGFIPPS--AAAAAAAAVAKKQQGRRVT--ADVLWPGMLRKG-KAAA 49
LOC_Os09g26420.3|11979.m22092|      AIISGFIPPS--AAAAAAAAVAKKQQGRRVT--ADVLWPGMLRKG-KAAA 49
AT1G53910.1                         AIISDFIPPPRSRRVTSEFIWPDLKKNLK-------GSKKSSKNRSNFFD 47
AT1G53910.2                         AIISDFIPPPRSRRVTSEFIWPDLKKNLK-------GSKKSSKNRSNFFD 47
AT3G14230.1                         AIISDFIPPPRSLRVTNEFIWPDLKNKVK-------ASKKRSNKRSDFFD 47
AT3G14230.2                         AIISDFIPPPRSLRVTNEFIWPDLKNKVK-------ASKKRSNKRSDFFD 47
AT3G14230.3                         AIISDFIPPPRSLRVTNEFIWPDLKNKVK-------ASKKRSNKRSDFFD 47
AT3G16770.1                         AIISDYAP---------------LVTKAK-------GRKLTAEEL--WSE 30
LOC_Os07g42510.1|11977.m08483|      SILGDLHLPVRRTVNAGDLWGDAGKGRDGGDGLKKRKGSSWDFDVDCDDD 54
LOC_Os03g08490.1|11973.m06357|      AILANIIPATPP-RPATAAHVWPGG-------------DGEKRRKVGGGG 40
LOC_Os03g08500.1|11973.m06358|      AILAELIPSAPAARRVTAGHVWPG--------------DANKAKKKGAR- 39
AT1G72360.1                         FELYPWLG------------------------------------------ 12
LOC_Os03g08460.1|11973.m06354|      AILADFTPARVPRRLTAAELLPVTPTPPAAERRTTRKRKSDVDFEAEFEL 54
LOC_Os05g29810.1|11975.m07218|      AIIADFVPPAGARRAAASDIS----------------------------- 25
LOC_Os03g22170.1|11973.m07620|      AIP--LISSRGPGG------------------------KRSLSAADELWP 28
LOC_Os10g25170.1|11980.m05467|      AILADLIPSPRSGGHTKKN-------------------KRRRISDDEDFEA 59
LOC_Os09g11460.1|11979.m04408|      --------RVSSSSSSSSSSP--------------ARHHKARRSRRKLA 31
LOC_Os09g11460.2|11979.m21977|      --------RVSSSSSSSSSSP--------------ARHHKARRSRRKLA 31
LOC_Os09g11480.1|11979.m04410|      ALIPNDYGDKPPPPPSESSEWD--------------ATTKMKKKKRGGG 40
LOC_Os12g41030.1|11982.m07885|      --------------------------------------------------
LOC_Os12g41060.1|11982.m07888|      GPDNHHAITVAADLPPPPPSVVAVEMAPP------RGHRPGKEVEYNEEE 48
AT1G80580.1                         YTVDGHRLQYSVPLSSMHETSQNSETYGL------SKESPLVCMPLFETN 48
```

```
LOC_Os07g47790.1|11977.m08994|      ADFDHLTAPCTFT------------------PDQA--------------- 57
LOC_Os07g47790.2|11977.m29326|      ADFDHLTAPCTFT------------------PDQ---------------- 56
LOC_Os01g21120.1|11971.m08596|      DDSD----PHTPA------------------PEK---------------- 38
LOC_Os03g08470.1|11973.m06355|      KSKRQQRSFADVDDFEAAFEQFDDDSDFDDAEEEDEGHFV---------- 82
LOC_Os03g08470.2|11973.m34802|      KSKRQQRSFADVDDFEAAFEQFDDDSDFDDAEEEDEGHFV---------- 82
AT2G47520.1                         VSSRKKRKPVSVS------------------EERD--------------- 41
LOC_Os02g54160.1|11972.m10445|      DDEDFEADFEEFEVDSGDSDLELG----EEDDDDVVEIKP---------- 84
LOC_Os06g09390.1|11976.m05658|      EEDDFEADFEEFEVDSGEWEVES----------DADEAKP---------- 78
LOC_Os09g26420.1|11979.m05794|      AEEDFEADFREFERGMSDDEAEGGGGEEEEDDDDVVVVVPPPAAARFVVR 99
LOC_Os09g26420.2|11979.m21992|      AEEDFEADFREFERGMSDDEAEGGGGEEEEDDDDVVVVVPPPAAARFVVR 99
LOC_Os09g26420.3|11979.m22092|      AEEDFEADFREFERGMSDDEAEGGGGEEEEDDDDVVVVVPPPAAARFVVR 99
AT1G53910.1                         FDAEFEADFQGFKDDSSIDCDDDFDVGDVFADVKPFVFTSTPKPAVSAAA 97
AT1G53910.2                         FDAEFEADFQGFKDDSSIDCDDDFDVGDVFADVKPFVFTSTPKPAVSAAA 97
AT3G14230.1                         LDDDFEADFQGFKDDSAFDCEDD---DDVFVNVKPFVFTATTKPVASAFV 94
AT3G14230.2                         LDDDFEADFQGFKDDSAFDCEDD---DDVFVNVKPFVFTATTKPVASAFV 94
AT3G14230.3                         LDDDFEADFQGFKDDSAFDCEDD---DDVFVNVKPFVFTATTKPVASAFV 94
AT3G16770.1                         LDASAADDFWGFYSTSKLHPTNQ-------VNVK---------------- 57
LOC_Os07g42510.1|11977.m08483|      DDDDFEADFEEFEDDYGDDDDVGFGDDDQESDMNGLKLAG---------- 94
LOC_Os03g08490.1|11973.m06357|      CDDDFEAAFERFGRE--DSEMEEEEVEEVVVG------------------ 70
LOC_Os03g08500.1|11973.m06358|      -ADDFEAAFRDFDNDSDDEEMMVEEAEEEEATSEH--------------- 73
AT1G72360.1                         --------------------------------------------------
LOC_Os03g08460.1|11973.m06354|      FEDDDDDDEFELSDDGDESLAVSCVSSPKSKAVPSFSFSS---------- 94
LOC_Os05g29810.1|11975.m07218|      -----DNAVLSAAGAGDESFAAAKAPAP---------------------- 48
LOC_Os03g22170.1|11973.m07620|      PPPQHASDDPAEQAAADEEEQEQ--------------------------- 51
LOC_Os10g25170.1|11980.m05467|      AFEEFDAGDDDSDSDSESEEVDEYDVVVDDDDSEDGVVVL---------- 99
LOC_Os09g11460.1|11979.m04408|      VDEDWEAAFREFLSRDHDDDDDDHDGQHVVVAPLIRGSDKCVHGHEVVAS 81
LOC_Os09g11460.2|11979.m21977|      VDEDWEAAFREFLSRDHDDDDDDHDGQHVVVAPLIRGSDKCVHGHEVVAS 81
LOC_Os09g11480.1|11979.m04410|      GDDDWEAAFREFIAGDDDDDDG-----GVSMFPSGAGTMETTTEVAPAAA 85
LOC_Os12g41030.1|11982.m07885|      ------------------MTMMP--------------------------- 5
LOC_Os12g41060.1|11982.m07888|      EDDDDEYDGREFEEEFLRFSMMVDEEDDDGDDDDEVEVEIIAVVSPPHRP 98
AT1G80580.1                         TTSFDISSLFSFNPKPEPENTHRVMDDS---------------------- 76
```

FIGURE 7 (continued)

FIGURE 7 (continued)

```
LOC_Os07g47790.1|11977.m08994|    ----------------------------------------------AEEP 61
LOC_Os07g47790.2|11977.m29326|    -----------------------------------------------EEP 59
LOC_Os01g21120.1|11971.m08596|    ------------------------------------------------PP 40
LOC_Os03g08470.1|11973.m06355|    -------------------------------FASKSRVVAGHDGRAAARA 101
LOC_Os03g08470.2|11973.m34802|    -------------------------------FASKSRVVAGHDGRAAARA 101
AT2G47520.1                       --------------------------------------------------
LOC_Os02g54160.1|11972.m10445|    AAFKR------------------------ALSRDNLSTITTAGFDGPAAK 110
LOC_Os06g09390.1|11976.m05658|    LAAPRS-----------------------GFAKGGLKNTTVAGADGPAAR 105
LOC_Os09g26420.1|11979.m05794|    AAAKAA-----------------------PPTADGMLTTKLVQHDGPTAR 126
LOC_Os09g26420.2|11979.m21992|    AAAKAA-----------------------PPTADGMLTTKLVQHDGPTAR 126
LOC_Os09g26420.3|11979.m22092|    AAAKAA-----------------------PPTADGMLTTKLVQHDGPTAR 126
AT1G53910.1                       EG--------------------------------SVFGKKVTGLDGDAEK 115
AT1G53910.2                       EG--------------------------------SVFGKKVTGLDGDAEK 115
AT3G14230.1                       STGIY--------------------------LVGSAYAKKTVESAEQAEK 118
AT3G14230.2                       ST------------------------------VGSAYAKKTVESAEQAEK 114
AT3G14230.3                       ST-------------------------------GSAYAKKTVESAEQAEK 113
AT3G16770.1                       --------------------------------------EEAVKKEQATEP 69
LOC_Os07g42510.1|11977.m08483|    ------------------------------------------FSTTKLGL 102
LOC_Os03g08490.1|11973.m06357|    ------------------------------------KKAAVRRRRATPAA 84
LOC_Os03g08500.1|11973.m06358|    ------------------------------------KPFVFRAKKAAAAA 87
AT1G72360.1                       -----------------------------------------------SAS 15
LOC_Os03g08460.1|11973.m06354|    --------------------------------DVSSSSRPRRRVAAAAAG 112
LOC_Os05g29810.1|11975.m07218|    -------------------------------------------------G 49
LOC_Os03g22170.1|11973.m07620|    --------------------------------------------QPAARR 57
LOC_Os10g25170.1|11980.m05467|    --------------------------------------PPPPPPPPVIPH 111
LOC_Os09g11460.1|11979.m04408|    TVGGG---------------------------ASGGRRRADDDDGERRRR 104
LOC_Os09g11460.2|11979.m21977|    TVGGG---------------------------ASGGRRRADDDDGERRRR 104
LOC_Os09g11480.1|11979.m04410|    VV-------------------------------------------ERPRR 92
LOC_Os12g41030.1|11982.m07885|    -----------------------------------------SGGGARVRA 14
LOC_Os12g41060.1|11982.m07888|    LVGARGTTSAVESVTNLQARTSPLPNPVVPQTGTKASKRGDSGAKAKPAA 148
AT1G80580.1                       --------------------IAAVVGENVLFGDKNKVSDHLTKEGGVKRG 106
```

FIGURE 7 (continued)

| | | |
|---|---|---|
| LOC_Os07g47790.1\|11977.m08994\| | TKKRERKTLYR---GIRRRP-----------------WGKWAAEIRD-PA | 90 |
| LOC_Os07g47790.2\|11977.m29326\| | TKKRERKTLYR---GIRRRP-----------------WGKWAAEIRD-PA | 88 |
| LOC_Os01g21120.1\|11971.m08596\| | RAKRERKNQYR---GIRQRP-----------------WGKWAAEIRD-PV | 69 |
| LOC_Os03g08470.1\|11973.m06355\| | ASKKKRGRHFR---GIRQRP-----------------WGKWAAEIRD-PH | 130 |
| LOC_Os03g08470.2\|11973.m34802\| | ASKKKRGRHFR---GIRQRP-----------------WGKWAAEIRD-PH | 130 |
| AT2G47520.1 | -GKRERKNLYR---GIRQRP-----------------WGKWAAEIRD-PS | 69 |
| LOC_Os02g54160.1\|11972.m10445\| | SAKRKRKNQFR---GIRQRP-----------------WGKWAAEIRD-PR | 139 |
| **LOC_Os06g09390.1\|11976.m05658\|** | **SAKRKRKNQFR---GIRQRP-----------------WGKWAAEIRD-PR** | **134** |
| LOC_Os09g26420.1\|11979.m05794\| | SAKHKRKNQYR---GIRQRP-----------------WGKWAAEIRD-PS | 155 |
| LOC_Os09g26420.2\|11979.m21992\| | SAKHKRKNQYR---GIRQRP-----------------WGKWAAEIRD-PS | 155 |
| LOC_Os09g26420.3\|11979.m22092\| | SAKHKRKNQYR---GIRQRP-----------------WGKWAAEIRD-PS | 155 |
| AT1G53910.1 | SANRKRKNQYR---GIRQRP-----------------WGKWAAEIRD-PR | 144 |
| AT1G53910.2 | SANRKRKNQYR---GIRQRP-----------------WGKWAAEIRD-PR | 144 |
| AT3G14230.1 | SSKRKRKNQYR---GIRQRP-----------------WGKWAAEIRD-PR | 147 |
| AT3G14230.2 | SSKRKRKNQYR---GIRQRP-----------------WGKWAAEIRD-PR | 143 |
| AT3G14230.3 | SSKRKRKNQYR---GIRQRP-----------------WGKWAAEIRD-PR | 142 |
| AT3G16770.1 | GKRRKRKNVYR---GIRKRP-----------------WGKWAAEIRD-PR | 98 |
| LOC_Os07g42510.1\|11977.m08483\| | GGSRKRKTRYR---GIRQRP-----------------WGKWAAEIRD-PR | 131 |
| LOC_Os03g08490.1\|11973.m06357\| | G-RRARPSKYW---GVRRRP-----------------WGKWAAEIRD-PV | 112 |
| LOC_Os03g08500.1\|11973.m06358\| | SSRRRKPAQYR---GVRRRP-----------------WGKWAAEIRD-PV | 116 |
| AT1G72360.1 | DGKKKQSSRYK---GIRRRP-----------------WGRWAAEIRD-PI | 44 |
| LOC_Os03g08460.1\|11973.m06354\| | RRKASKKSKYR---GVRRRP-----------------SGRFAAEIRD-PK | 141 |
| LOC_Os05g29810.1\|11975.m07218\| | R-----KTAYR---GIRRRP-----------------WGRWAAEIRD-PR | 73 |
| LOC_Os03g22170.1\|11973.m07620\| | QRRGERRTLYR---GIRRRP-----------------WGKWAAEIRD-PA | 86 |
| LOC_Os10g25170.1\|11980.m05467\| | ERHGARR--FR---GVRKRP-----------------WGKWAAEIRD-PV | 138 |
| LOC_Os09g11460.1\|11979.m04408\| | RRREKRSYPYR---GIRQRP-----------------WGRWASEIRD-PV | 133 |
| LOC_Os09g11460.2\|11979.m21977\| | RRREKRSYPYR---GIRQRP-----------------WGRWASEIRD-PV | 133 |
| LOC_Os09g11480.1\|11979.m04410\| | RRRVRRSYPYR---GVRQRP-----------------WGRWASEIRD-PV | 121 |
| LOC_Os12g41030.1\|11982.m07885\| | H--------FR---GVQWRK-----------------SGRWSAEIRS-RG | 35 |
| LOC_Os12g41060.1\|11982.m07888\| | AKKRRSKHGFL---GVHQRT-----------------YGRWSAEIRD-NV | 177 |
| AT1G80580.1 | RKMPQKTGGFM---GVRKRP-----------------WGRWSAEIRD-RI | 135 |

FIGURE 7 (continued)

| | | |
|---|---|---|
| LOC_Os07g47790.1\|11977.m08994\| | KGAR--------------------------VWLGTFATAEAAARAYDRAA | 114 |
| LOC_Os07g47790.2\|11977.m29326\| | KGAR--------------------------VWLGTFATAEAAARAYDRAA | 112 |
| LOC_Os01g21120.1\|11971.m08596\| | KGVR--------------------------VWLGTYPTAEAAARAYDRAA | 93 |
| LOC_Os03g08470.1\|11973.m06355\| | KGTR--------------------------VWLGTFNTPEEAARAYDVEA | 154 |
| LOC_Os03g08470.2\|11973.m34802\| | KGTR--------------------------VWLGTFNTPEEAARAYDVEA | 154 |
| AT2G47520.1 | KGVR--------------------------VWLGTFKTADEAARAYDVAA | 93 |
| LOC_Os02g54160.1\|11972.m10445\| | KGVR--------------------------VWLGTFNSAEEAARAYDAEA | 163 |
| **LOC_Os06g09390.1\|11976.m05658\|** | **KGVR--------------------------VWLGTFNSPEEAARAYDAEA** | **158** |
| LOC_Os09g26420.1\|11979.m05794\| | KGVR--------------------------VWLGTYNTAEEAARAYDAEA | 179 |
| LOC_Os09g26420.2\|11979.m21992\| | KGVR--------------------------VWLGTYNTAEEAARAYDAEA | 179 |
| LOC_Os09g26420.3\|11979.m22092\| | KGVR--------------------------VWLGTYNTAEEAARAYDAEA | 179 |
| AT1G53910.1 | EGAR--------------------------IWLGTFKTAEEAARAYDAAA | 168 |
| AT1G53910.2 | EGAR--------------------------IWLGTFKTAEEAARAYDAAA | 168 |
| AT3G14230.1 | KGSR--------------------------EWLGTFDTAEEAARAYDAAA | 171 |
| AT3G14230.2 | KGSR--------------------------EWLGTFDTAEEAARAYDAAA | 167 |
| AT3G14230.3 | KGSR--------------------------EWLGTFDTAEEAARAYDAAA | 166 |
| AT3G16770.1 | KGVR--------------------------VWLGTFNTAEEAAMAYDVAA | 122 |
| LOC_Os07g42510.1\|11977.m08483\| | KGVR--------------------------VWLGTFGTAEEAAMAYDVEA | 155 |
| LOC_Os03g08490.1\|11973.m06357\| | EGVR--------------------------VWLGTFATAEAAAHAYDAAA | 136 |
| LOC_Os03g08500.1\|11973.m06358\| | KGIR--------------------------VWLGTFTNAEAAALAYDDAA | 140 |
| AT1G72360.1 | KGVR--------------------------VWLGTFNTAEEAARAYDLEA | 68 |
| LOC_Os03g08460.1\|11973.m06354\| | KGRR--------------------------VWLGTYGSAEEAAMAYDREA | 165 |
| LOC_Os05g29810.1\|11975.m07218\| | KGAR--------------------------VWLGTYATAEEAARAYDVAA | 97 |
| LOC_Os03g22170.1\|11973.m07620\| | KGAR--------------------------VWLGTFATAEAAARAYDRAA | 110 |
| LOC_Os10g25170.1\|11980.m05467\| | RGVR--------------------------VWLGTFPTAESAARAYDAAA | 162 |
| LOC_Os09g11460.1\|11979.m04408\| | KGIR--------------------------VWLGTFDTAEGAARAYDDEV | 157 |
| LOC_Os09g11460.2\|11979.m21977\| | KGIR--------------------------VWLGTFDTAEGAARAYDDEV | 157 |
| LOC_Os09g11480.1\|11979.m04410\| | KGAR--------------------------VWLGTFDTAVEAARAYDAEA | 145 |
| LOC_Os12g41030.1\|11982.m07885\| | AWGR--R---------------------RRLWIGTYDTAEEAARAYDAEA | 62 |
| LOC_Os12g41060.1\|11982.m07888\| | IKGS-------------------------RFWIGTFDTALDAALAYDAVS | 202 |
| AT1G80580.1 | GRCR--------------------------HWLGTFDTAEEAARAYDAAA | 159 |

FIGURE 7 (continued)

| | | |
|---|---|---|
| LOC_Os07g47790.1\|11977.m08994\| | RRIRG---------AKAKVNFPNEDPPLDDP------------------- | 136 |
| LOC_Os07g47790.2\|11977.m29326\| | RRIRG---------AKAKVNFPNEDPPLDDP------------------- | 134 |
| LOC_Os01g21120.1\|11971.m08596\| | RRIRG---------AKAKVNFPNDFGAAPAP------------------- | 115 |
| LOC_Os03g08470.1\|11973.m06355\| | RRLRG---------SKAKVNFPATPAAARPRRGNTRATAVPPP------- | 188 |
| LOC_Os03g08470.2\|11973.m34802\| | RRLRG---------SKAKVNFPATPAAARPRRGNTRATAVPPP------- | 188 |
| AT2G47520.1 | IKIRG---------RKAKLNFPNT-------------------------- | 108 |
| LOC_Os02g54160.1\|11972.m10445\| | RRIRG---------KKAKVNFPE-APTTAQKRRAGSTTAKAPKSSV---- | 199 |
| **LOC_Os06g09390.1\|11976.m05658\|** | **RRIRG---------KKAKVNFPDGAPVASQRSHAEPSSMNMPAFSI----** | **195** |
| LOC_Os09g26420.1\|11979.m05794\| | RKIRG---------KKAKVNFPD-EPAVAQKLSLKQNAAKQEK------- | 212 |
| LOC_Os09g26420.2\|11979.m21992\| | RKIRG---------KKAKVNFPD-EPAVAQKLSLKQNAAKQEK------- | 212 |
| LOC_Os09g26420.3\|11979.m22092\| | RKIRG---------KKAKVNFPD-EPAVAQKLSLKQNAAKQEK------- | 212 |
| AT1G53910.1 | RRIRG---------SKAKVNFPEE-NMKANSQKR-SVKAN-LQKPV---- | 202 |
| AT1G53910.2 | RRIRG---------SKAKVNFPEE-NMKANSQKR-SVKAN-LQKPV---- | 202 |
| AT3G14230.1 | RRIRG---------TKAKVNFPEEKNPSVVSQKRPSAKTNNLQKSV---- | 208 |
| AT3G14230.2 | RRIRG---------TKAKVNFPEEKNPSVVSQKRPSAKTNNLQKSV---- | 204 |
| AT3G14230.3 | RRIRG---------TKAKVNFPEEKNPSVVSQKRPSAKTNNLQKSV---- | 203 |
| AT3G16770.1 | KQIRG---------DKAKLNFPDLHHPPPPNYTPPPSSPRSTDQPP---- | 159 |
| LOC_Os07g42510.1\|11977.m08483\| | RRIRG---------KKAKVNFPDAAAAAPKRPRRSSAKHSPQQQK----- | 191 |
| LOC_Os03g08490.1\|11973.m06357\| | RDLRG---------ATAKLNFPSSSS-STAATPRPRKCRPTTATAT---- | 172 |
| LOC_Os03g08500.1\|11973.m06358\| | RAIRG---------DRAKLNFPSATTPDTRKRGRATAAAAPAVKAT---- | 177 |
| AT1G72360.1 | KRIRG---------AKAKLNFPNESSGKRKAKAKT--------------- | 94 |
| LOC_Os03g08460.1\|11973.m06354\| | RRIRG---------KGARLNFPRDGDGSPRRSNDRPCWTIDLNLP----- | 201 |
| LOC_Os05g29810.1\|1197.m07218\| | RDIRG---------AKAKLNFP---------------------------- | 110 |
| LOC_Os03g22170.1\|11973.m07620\| | RRIRG---------TKAKVNFPNEDNAFAAAPPPYHLAAYYGDAS----- | 146 |
| LOC_Os10g25170.1\|11980.m05467\| | RRLRG---------AKAKPNFPSAPPPSAAAHRRKKRRAHAATRS----- | 198 |
| LOC_Os09g11460.1\|11979.m04408\| | RRIYG---------GNAKTNFPPS-PPTPPPPEK---------------- | 181 |
| LOC_Os09g11460.2\|11979.m21977\| | RRIYG---------GNAKTNFPPS-PPTPPPPEK---------------- | 181 |
| LOC_Os09g11480.1\|11979.m04410\| | RRIHG---------HKARTNFPPDEPPLPAPSQAPFCFLLDDDDD----- | 181 |
| LOC_Os12g41030.1\|11982.m07885\| | RRLHG---------AKAKTNFP--------PPPPPTAVHDDHVDL----- | 90 |
| LOC_Os12g41060.1\|11982.m07888\| | RRLYG---------LNAKTNFPAAAGEDDLPPPPPPAKPCSSTKR----- | 238 |
| AT1G80580.1 | RRLRG---------TKAKTNFVIP------PLFPKEIAQAQEDNR----- | 189 |

FIGURE 7 (continued)

```
LOC_Os07g47790.1|11977.m08994|     -------------------------------------AADGHSHGGA-- 146
LOC_Os07g47790.2|11977.m29326|     -------------------------------------AADGHSHGGA-- 144
LOC_Os01g21120.1|11971.m08596|     -------------------------------------AAAAAKAVPR-- 125
LOC_Os03g08470.1|11973.m06355|     ----ATAPAAAPPRGLKREFSPPAETALPFFTNGFVDLTTAAAPPPAM-- 232
LOC_Os03g08470.2|11973.m34802|     ----ATAPAAAPPRGLKREFSPPAETALPFFTNGFVDLTTAAAPPPAM-- 232
AT2G47520.1                        ------------------------------------------------
LOC_Os02g54160.1|11972.m10445|     ----EQKPTVKPAFNN-LANANAFVYPSANFTSNKPFVQPDNMPFVPA-- 242
LOC_Os06g09390.1|11976.m05658|     ----EEKPAVMSAGNKTMYNTNAYAYPAVEYTLQEPFVQIQNVSFVPA-- 239
LOC_Os09g26420.1|11979.m05794|     -----LAPPLKTCGDDAFFQLNSSDNDLFAMLAKVPAKPAEPVDLMPP-- 255
LOC_Os09g26420.2|11979.m21992|     -----LAPPLKTCGDDAFFQLNSSDNDLFAMLAKVPAKPAEPVDLMPP-- 255
LOC_Os09g26420.3|11979.m22092|     -----LAPPLKTCGDDAFFQLNSSDNDLFAMLAKVPAKPAEPVDLMPP-- 255
AT1G53910.1                        ----AKPN------PNPSPALVQNSNISFEN----MCFMEEKHQVSNNNN 238
AT1G53910.2                        ----AKPN------PNPSPALVQNSNISFEN----MCFMEEKHQVSNNNN 238
AT3G14230.1                        ----AKPNKSVTLVQQPTHLSQQYCNNSFDNSFGDMSFMEEKPQMYNN-- 252
AT3G14230.2                        ----AKPNKSVTLVQQPTHLSQQYCNNSFDNSFGDMSFMEEKPQMYNN-- 248
AT3G14230.3                        ----AKPNKSVTLVQQPTHLSQQYCNNSFDNSFGDMSFMEEKPQMYNN-- 247
AT3G16770.1                        ----AKKVCVVS--QSESELSQP-------------SFPVECIGFGNG-- 188
LOC_Os07g42510.1|11977.m08483|     ----ARSSSSSPASLNASDAVSKSNNNRVSSAGSSTDATAAAIAIDDG-- 235
LOC_Os03g08490.1|11973.m06357|     ----P------KATTPNVVVVVNLVDKEAEVSESSGASSSALPDFSWQG- 211
LOC_Os03g08500.1|11973.m06358|     ----PVINLVEEEDEEEVAAAMASIKYEPETSESS--ESNALPDFSWQG- 220
AT1G72360.1                        -----------------VQQVEENHEADLDVAVVSSAPSSSCLDFLWE-- 125
LOC_Os03g08460.1|11973.m06354|     ---------------AAAVSGDDDDAMAVDAADADAGSAGRAAAYADQE- 235
LOC_Os05g29810.1|11975.m07218|     -------------------------PTIGAAAAPPPPKKRRKAAAAAN--- 133
LOC_Os03g22170.1|11973.m07620|     ----------------STSYLYPMAMTPAAAGLR---------------- 164
LOC_Os10g25170.1|11980.m05467|     ----------------PSSPPATSEVTAASASASSDVPAPAFASFVGEP- 231
LOC_Os09g11460.1|11979.m04408|     ------------------------------------PAAERSP------- 188
LOC_Os09g11460.2|11979.m21977|     ------------------------------------PAAERSP------- 188
LOC_Os09g11480.1|11979.m04410|     ---------------------------DDGVARGNSPASSSAPDRASAC- 203
LOC_Os12g41030.1|11982.m07885|     -------------------------------------EAHIKFLSEVELD- 103
LOC_Os12g41060.1|11982.m07888|     ------------------------------------PKKCNTSGDLGAA- 251
AT1G80580.1                        ------------------------------------MRQKQKKKKKKKV- 202
```

FIGURE 7 (continued)

LOC_Os07g47790.1|11977.m08994| ------AIPCREFMDYDAVMAGFFHQPYVV------ADGVPAVP------ 178
LOC_Os07g47790.2|11977.m29326| ------AIPCREFMDYDAVMAGFFHQPYVV------ADGVPAVP------ 176
LOC_Os01g21120.1|11971.m08596| ------VAPTPAVLPPPKMEAVSEGAGACS------SDEVKELS------ 157
LOC_Os03g08470.1|11973.m06355| ------MMTSSFTDSVATSESGGSPAKKAR------SDDVDSSEGS---- 266
LOC_Os03g08470.2|11973.m34802| ------MMTSSFTDSVATSESGGSPAKKAR------SDDVDSSEGS---- 266
AT2G47520.1 -----------QVEEEADTKPGGNQNELIS------ENQVESLS------ 135
LOC_Os02g54160.1|11972.m10445| ------MNSAAPIEDPIIN--SDQGSNSFGCSDFGWENDTKTPDITSIAP 284
**LOC_Os06g09390.1|11976.m05658| ------MN---AIEDTFVNLSSDQGSNSFGCSDFSQENDIKTPDITSMLA 280**
LOC_Os09g26420.1|11979.m05794| ------VKPLASTETFEMNMLSDTSSNSFGSSDFGWEDDTLTPDYTSVFV 299
LOC_Os09g26420.2|11979.m21992| ------VKPLASTETFEMNMLSDTSSNSFGSSDFGWEDDTLTPDYTSVFV 299
LOC_Os09g26420.3|11979.m22092| ------VKPLASTETFEMNMLSDTSSNSFGSSDFGWEDDTLTPDYTSVFV 299
AT1G53910.1 NQFGMTNSVDAG-CNGYQYFSSDQGSNSFDCSEFGWSDQAPITPDISS-- 285
AT1G53910.2 NQFGMTNSVDAG-CNGYQYFSSDQGSNSFDCSEFGWSDQAPITPDISS-- 285
AT3G14230.1 -QFGLTNSFDAGGNNGYQYFSSDQGSNSFDCSEFGWSDHGPKTPEISS-- 299
AT3G14230.2 -QFGLTNSFDAGGNNGYQYFSSDQGSNSFDCSEFGWSDHGPKTPEISS-- 295
AT3G14230.3 -QFGLTNSFDAGGNNGYQYFSSDQGSNSFDCSEFGWSDHGPKTPEISS-- 294
AT3G16770.1 --------------DEFQNLS------------YGFEPDYDLKQQISSLE 212
LOC_Os07g42510.1|11977.m08483| ------VKLELLSETDPSPPMAAAAAAWLDAFELNDLDGSRCKDNAFD-- 277
LOC_Os03g08490.1|11973.m06357| --------MSASSDDDAAAQQALLDAAG-GAKKRPRSEPHVTSDDEVLP- 251
LOC_Os03g08500.1|11973.m06358| --------MSASDEFAVAAAALSLDSDDDLAKKRPRTEPEDTTDS----- 257
AT1G72360.1 ---------ENNPDTLLIDTQWLEDIIMGDANKKHEPNDSEEANN----- 161
LOC_Os03g08460.1|11973.m06354| ---------ALSAAKCKIKQCPRDEQMASATPELMEEDASSSRNMVP--- 273
LOC_Os05g29810.1|11975.m07218| ------------------HHHHHHQQESSGS-----SSASSLPPTPP--- 157
LOC_Os03g22170.1|11973.m07620| ---------EQQLMTTTAVEYSVNDAVDVASVYFQPPPPAVAYEFS---- 201
LOC_Os10g25170.1|11980.m05467| ------GHGGAKSMPTTSHTSQPAPPATVASENVDDPEVFDPYDVHGGL- 274
LOC_Os09g11460.1|11979.m04408| ----------STTPTTTTEDSGDSRILIECCSDDLMDSLLAAFDMTT--- 225
LOC_Os09g11460.2|11979.m21977| ----------STTPTTTTEDSGDSRILIECCSDDLMDSLLAAFDMTT--- 225
LOC_Os09g11480.1|11979.m04410| ----------TTSSTVASGERGDELILLECCSDDVMDSLLAGFDVSS--- 240
LOC_Os12g41030.1|11982.m07885| ---------GDQ----ETPPESQVGDDQ-------AGGQHQHHHG----- 128
LOC_Os12g41060.1|11982.m07888| ---------AAPPQAVDTPAAAAAGVELTSLLCSVAAQAQEVSDG----- 287
AT1G80580.1 ---------SVRKCVKVTSVAQLFDDANFINSSSIKGNVISSIDN----- 238

FIGURE 7 (continued)

```
LOC_Os07g47790.1|11977.m08994|      --------------------------------------------------
LOC_Os07g47790.2|11977.m29326|      --------------------------------------------------
LOC_Os01g21120.1|11971.m08596|      --------------------------------------------------
LOC_Os03g08470.1|11973.m06355|      --------------------------------------------------
LOC_Os03g08470.2|11973.m34802|      --------------------------------------------------
AT2G47520.1                         --------------------------------------------------
LOC_Os02g54160.1|11972.m10445|      IS-TIAEVDESAFIKS-STNPM---------------------------- 304
LOC_Os06g09390.1|11976.m05658|      P--TMTGVDDSAFLQNNASDAM---------------------------- 300
LOC_Os09g26420.1|11979.m05794|      PNAAMPAYGEPAYLTGGAPKRMRNNYG----------------------- 326
LOC_Os09g26420.2|11979.m21992|      PNAAMPAYGEPAYLTGGAPKRMRNNYG----------------------- 326
LOC_Os09g26420.3|11979.m22092|      PNAAMPAYGEPAYLTGGAPKRMRNNYG----------------------- 326
AT1G53910.1                         AVINNNNSALFFEEANPAKKLK---------------------------- 307
AT1G53910.2                         AVINNNNSALFFEEANPAKKLK---------------------------- 307
AT3G14230.1                         MLVNNNE-ASFVEETNAAKKLKP--------------------------- 321
AT3G14230.2                         MLVNNNE-ASFVEETNAAKKLKP--------------------------- 317
AT3G14230.3                         MLVNNNE-ASFVEETNAAKKLKP--------------------------- 316
AT3G16770.1                         SFLELDG------------------------------------------- 219
LOC_Os07g42510.1|11977.m08483|      --------------------------------------------------
LOC_Os03g08490.1|11973.m06357|      --------------------------------------------------
LOC_Os03g08500.1|11973.m06358|      --------------------------------------------------
AT1G72360.1                         --------------------------------------------------
LOC_Os03g08460.1|11973.m06354|      --------------------------------------------------
LOC_Os05g29810.1|11975.m07218|      --------------------------------------------------
LOC_Os03g22170.1|11973.m07620|      --------------------------------------------------
LOC_Os10g25170.1|11980.m05467|      --------------------------------------------------
LOC_Os09g11460.1|11979.m04408|      --------------------------------------------------
LOC_Os09g11460.2|11979.m21977|      --------------------------------------------------
LOC_Os09g11480.1|11979.m04410|      --------------------------------------------------
LOC_Os12g41030.1|11982.m07885|      --------------------------------------------------
LOC_Os12g41060.1|11982.m07888|      --------------------------------------------------
AT1G80580.1                         --------------------------------------------------
```

FIGURE 7 (continued)

| | | |
|---|---|---|
| LOC_Os07g47790.1\|11977.m08994\| | ----------------AEEAPTVAYVHH-----HLPPQPQ---------- | 197 |
| LOC_Os07g47790.2\|11977.m29326\| | ----------------AEEAPTVAYVHH-----HLPPQPQ---------- | 195 |
| LOC_Os01g21120.1\|11971.m08596\| | ----------------EELLAYENYMSF-----LGIPYME---------- | 176 |
| LOC_Os03g08470.1\|11973.m06355\| | ------------VGGGSDTLGFTDELEFDPFMLFQLPYSDGYESI----- | 299 |
| LOC_Os03g08470.2\|11973.m34802\| | ------------VGGGSDTLGFTDELEFDPFMLFQLPYSDGYESI----- | 299 |
| AT2G47520.1 | ----------------EDLMALEDYMRF-----YQIPVAD---------- | 154 |
| LOC_Os02g54160.1\|11972.m10445\| | -----VPP--VMENSAVDLPDLEPYMRFL----LDDGAGD---------- | 333 |
| **LOC_Os06g09390.1\|11976.m05658\|** | **-----VPP--VMGNASIDLADLEPYMKFL----IDGGSDE----------** | **329** |
| LOC_Os09g26420.1\|11979.m05794\| | ---IAVPQGNGMPNLAQNMPTFDPEMKYLPLPYVESSSDE---------- | 363 |
| LOC_Os09g26420.2\|11979.m21992\| | ---IAVPQGNGMPNLAQNMPTFDPEMKYLPLPYVESSSDE---------- | 363 |
| LOC_Os09g26420.3\|11979.m22092\| | ---IAVPQGNGMPNLAQNMPTFDPEMKYLPLPYVESSSDE---------- | 363 |
| AT1G53910.1 | ---------------SMDFETPYNNTEW------DASLD----------- | 325 |
| AT1G53910.2 | ---------------SMDFETPYNNTEW------DASLD----------- | 325 |
| AT3G14230.1 | -----------NSDESDDLMAYLDNALW------DTPLEVE--------- | 345 |
| AT3G14230.2 | -----------NSDESDDLMAYLDNALW------DTPLEVE--------- | 341 |
| AT3G14230.3 | -----------NSDESDDLMAYLDNALW------DTPLEVE--------- | 340 |
| AT3G16770.1 | -----------------------NTAEQ------PSQLD----------- | 229 |
| LOC_Os07g42510.1\|11977.m08483\| | -----------HQIHKVEAAVADEFAFYDDPSYMQLGYQLD--------- | 307 |
| LOC_Os03g08490.1\|11973.m06357\| | -----ASFDSDNNTAAAGLLPLDDPFLFGDQFGDLNGGAFASL------- | 289 |
| LOC_Os03g08500.1\|11973.m06358\| | --------GSGDDTDA-----LFDALLFADQYNHFNGGAYES-------- | 286 |
| AT1G72360.1 | ---------VDASLLSEELLAFENQTEYFSQMPFTEGNCDSSTS------ | 196 |
| LOC_Os03g08460.1\|11973.m06354\| | -------------------LSMALQLQYAAMIAECDREMEEIAA------ | 298 |
| LOC_Os05g29810.1\|11975.m07218\| | -------------------PAAEHQLR------ECMSGLEAFLG------ | 176 |
| LOC_Os03g22170.1\|11973.m07620\| | ------------AVGGGAVVVPVSAVAP----AMTYGQSQE--------- | 226 |
| LOC_Os10g25170.1\|11980.m05467\| | ----------ASYFAGGAYESLESLFAHGGDSAAVDQAASD--------- | 305 |
| LOC_Os09g11460.1\|11979.m04408\| | -------------------------------------------------- | |
| LOC_Os09g11460.2\|11979.m21977\| | -------------------------------------------------- | |
| LOC_Os09g11480.1\|11979.m04410\| | -------------------------------------------------- | |
| LOC_Os12g41030.1\|11982.m07885\| | ----------------CRLDHLLLMMCN---------------------- | 140 |
| LOC_Os12g41060.1\|11982.m07888\| | ----------------WEFIQELLLLGGGVSPLDYLNGQELAG------- | 314 |
| AT1G80580.1 | ----------------LEKMGLELDLSLGLLSRK---------------- | 256 |

FIGURE 7 (continued)

```
LOC_Os07g47790.1|11977.m08994|      --------------------------QDAG-----------LELWSFDNI 210
LOC_Os07g47790.2|11977.m29326|      --------------------------QDAG-----------LELWSFDNI 208
LOC_Os01g21120.1|11971.m08596|      --------------------------GGAASAAGAEEAAAPAGLWTFEDY 200
LOC_Os03g08470.1|11973.m06355|      ------D-----------SLFAAGDANSANTDMNAG-----VNLWSFDDF 327
LOC_Os03g08470.2|11973.m34802|      ------D-----------SLFAAGDANSANTDMNAG-----VNLWSFDDF 327
AT2G47520.1                         --------------------------DQSATDIG--------NLWSYQDS 170
LOC_Os02g54160.1|11972.m10445|      ------------------------SIDSLLNLDGSQDVVSNMDLWSFDDM 359
LOC_Os06g09390.1|11976.m05658|      ------------------------SIDTLLSSDGSQDVASSMDLWSFDDM 355
LOC_Os09g26420.1|11979.m05794|      ------------------------SMDNLLQNDATQDGASNEGIWSLDEL 389
LOC_Os09g26420.2|11979.m21992|      ------------------------SMDNLLQNDATQDGASNEGIWSLDEL 389
LOC_Os09g26420.3|11979.m22092|      ------------------------SMDNLLQNDATQDGASNEGIWSLDEL 389
AT1G53910.1                         -------------------------FLNEDAVTTQDNGANPMDLWSIDEI 350
AT1G53910.2                         -------------------------FLNEDAVTTQDNGANPMDLWSIDEI 350
AT3G14230.1                         ------------------------AMLGADAGAVTQEEENPVELWSLDEI 371
AT3G14230.2                         ------------------------AMLGADAGAVTQEEENPVELWSLDEI 367
AT3G14230.3                         ------------------------AMLGADAGAVTQEEENPVELWSLDEI 366
AT3G16770.1                         ------------------------------------ESVSEVDMWMLDDV 243
LOC_Os07g42510.1|11977.m08483|      ----------------------QGNSYENIDALFGGEAVNIGGLWSFDDM 335
LOC_Os03g08490.1|11973.m06357|      -------------------MDGLFAAG------EANVAGESVGLWSFGD- 313
LOC_Os03g08500.1|11973.m06358|      -------------------LDSLFSADAVQTTAAAAAADQGMGLWSFDDG 317
AT1G72360.1                         -------------------LSSLFDGG------------NDMGLWS---- 211
LOC_Os03g08460.1|11973.m06354|      -------------------------VERDLERRRRQVFERRGHLVRQASL 323
LOC_Os05g29810.1|11975.m07218|      -------------------------LEEEDDGG------AGEPWDAVDM 195
LOC_Os03g22170.1|11973.m07620|      --------------------------------------VAAPLMWNFDDI 238
LOC_Os10g25170.1|11980.m05467|      --------------------------------------HWPAALWSFADD 317
LOC_Os09g11460.1|11979.m04408|      ---------------------------------------GDMRFWS---- 232
LOC_Os09g11460.2|11979.m21977|      ---------------------------------------GDMRFWS---- 232
LOC_Os09g11480.1|11979.m04410|      ---------------------------------------EPRSVLGMVN- 250
LOC_Os12g41030.1|11982.m07885|      --------------------------------------------------
LOC_Os12g41060.1|11982.m07888|      --------------------------------------AAVGDLWSF--- 323
AT1G80580.1                         --------------------------------------------------
```

FIGURE 7 (continued)

```
LOC_Os07g47790.1|11977.m08994|     HTAVPM-------------------------------------------- 216
LOC_Os07g47790.2|11977.m29326|     HTAVPM-------------------------------------------- 214
LOC_Os01g21120.1|11971.m08596|     ELPSLAL------------------------------------------- 207
LOC_Os03g08470.1|11973.m06355|     PIDGALF------------------------------------------- 334
LOC_Os03g08470.2|11973.m34802|     PIDGALF------------------------------------------- 334
AT2G47520.1                        N------------------------------------------------- 171
LOC_Os02g54160.1|11972.m10445|     PVS-DFY------------------------------------------- 365
LOC_Os06g09390.1|11976.m05658|     PVSAEFY------------------------------------------- 362
LOC_Os09g26420.1|11979.m05794|     LMAAGAY------------------------------------------- 396
LOC_Os09g26420.2|11979.m21992|     LMAAGAY------------------------------------------- 396
LOC_Os09g26420.3|11979.m22092|     LMAAGAY------------------------------------------- 396
AT1G53910.1                        HSMIGGVF------------------------------------------ 358
AT1G53910.2                        HSMIGGVF------------------------------------------ 358
AT3G14230.1                        NFMLEGDF------------------------------------------ 379
AT3G14230.2                        NFMLEGDF------------------------------------------ 375
AT3G14230.3                        NFMLEGDF------------------------------------------ 374
AT3G16770.1                        IASYE--------------------------------------------- 248
LOC_Os07g42510.1|11977.m08483|     PMEFRAY------------------------------------------- 342
LOC_Os03g08490.1|11973.m06357|     ----DFLNASYY-------------------------------------- 321
LOC_Os03g08500.1|11973.m06358|     CCLVDVEASLSF-------------------------------------- 329
AT1G72360.1                        --------------------------------------------------
LOC_Os03g08460.1|11973.m06354|     LLD----------------------------------------------- 326
LOC_Os05g29810.1|11975.m07218|     MLE----------------------------------------------- 198
LOC_Os03g22170.1|11973.m07620|     TAMPM--------------------------------------------- 243
LOC_Os10g25170.1|11980.m05467|     GSFCF--------------------------------------------- 322
LOC_Os09g11460.1|11979.m04408|     --------------------------------------------------
LOC_Os09g11460.2|11979.m21977|     --------------------------------------------------
LOC_Os09g11480.1|11979.m04410|     --------------------------------------------------
LOC_Os12g41030.1|11982.m07885|     --------------------------------------------------
LOC_Os12g41060.1|11982.m07888|     --------------------------------------------------
AT1G80580.1                        --------------------------------------------------
```

FIGURE 8

```
LOC_Os02g51670.1|11972.m10198|      ------MAAAIDMYKYN-TSTHQIASSDQELMKAL--EPFIRSASSSSAS 41
LOC_Os06g11860.1|11976.m05903|      -------MAAIDLYKYQLSSSSSSSSSDQELMKAL--EPFIRSASPTSTS 41
LOC_Os08g31580.1|11978.m07152|      ------MAAAIEGN----------------LMRALGEAPSPQMQKIA--- 25
LOC_Os09g20350.1|11979.m05242|      ------MAAAIDLSGEE-------------LMRAL--EPFIRDASGS--- 26
LOC_Os03g09170.1|11973.m06423|      ------MATTVDWCGRG---------------SNLP-AAMYDMVVDS--- 25
AT2G22200.1                         --------------------------------------------------
AT4G39780.1                         -------MAAIDMFNSN-------------------TDPFQEELMKALQP 24
AT5G65130.1                         --MALNMNAYVDEFMEA-------------------LEPFMKVTSSSSTS 29
AT1G22190.1                         ------MTTSMDFYSN----KTFQQSDPFGGELMEALLPFIK----SPSN 36
AT1G78080.1                         ------MAAAMNLYTCS---RSFQDS---GGELMDALVPFIKSVSDSPSS 38
AT1G36060.1                         ---------MADLFGG-----------GHGGELMEALQPFYKSASTSASN 30
LOC_Os10g22600.1|11980.m05267|      DRQWRCLAAAASTSGNS----------------KLPPLPMALGGAVEYGQ 35
LOC_Os05g49700.1|11975.m09040|      DASLRTLPPA--------------------------IFP--GEVRSAVSS 23
AT1G64380.1                         EESNDIFQNN--------------------------FSPKISEIRASLSQ 25

LOC_Os02g51670.1|11972.m10198|      SPCHHYYSSS-------------PSMSQDSYMPTPSYPTSSIT---TAAA 75
LOC_Os06g11860.1|11976.m05903|      TSTPLFYSSSSISTTTTTPFSYSSPLPQESYYLPASSSYAAIVPPPTTTT 91
LOC_Os08g31580.1|11978.m07152|      --------------------------------------------------
LOC_Os09g20350.1|11979.m05242|      --------------------------------------------------
LOC_Os03g09170.1|11973.m06423|      --------------------------------------------------
AT2G22200.1                         --------------------------------------------------
AT4G39780.1                         --------------------------------------------------
AT5G65130.1                         --------------------------------------------------
AT1G22190.1                         DS------------------------------------------------ 38
AT1G78080.1                         SS------------------------------------------------ 40
AT1G36060.1                         PA------------------------------------------------ 32
LOC_Os10g22600.1|11980.m05267|      LVHG---------------------------------------------- 39
LOC_Os05g49700.1|11975.m09040|      LL------------------------------------------------ 25
AT1G64380.1                         II------------------------------------------------ 27
```

FIGURE 8 (conitued)

```
LOC_Os02g51670.1|11972.m10198|      TTTSSFSQLPPLYSSQYHAA----------------------SPAASATN 103
LOC_Os06g11860.1|11976.m05903|      NTTTSFSELPPLPPSSSSFA----------------------SPANAAA- 118
LOC_Os08g31580.1|11978.m07152|      --PPPFHPGLPPAPANFSSA----------------------GVHGFHY- 50
LOC_Os09g20350.1|11979.m05242|      --PPVCSQFSPTSPFSFPHA----------------------FAYGGGL- 51
LOC_Os03g09170.1|11973.m06423|      ---KELMGALAPSMVSFSYP----------------------CSEQSASS 50
AT2G22200.1                         ----METASLSF---PVPNT----------------------SFGVN-KS 20
AT4G39780.1                         --YTTNTDSSSP---TYSNT----------------------VFGFN-QT 46
AT5G65130.1                         --NSSNPKPLTPNFIPNNDQ----------------------VLPVSNQT 55
AT1G22190.1                         --SAFA--------FSLP------------------------------AP 48
AT1G78080.1                         --AASASAFLHPSAFSLPPLPGYY-----------------PDSTFLTQP 71
AT1G36060.1                         --FASSNDAFASAPNDLFSSSSYYNPHASLFPSHSTTSYPDIYSGSMTYP 80
LOC_Os10g22600.1|11980.m05267|      -AAAPVAPFFVDAEQQSLSP----------------------ATAMVLGA 66
LOC_Os05g49700.1|11975.m09040|      --LSPGGSALDTVFSHLPP---------------------------PVTI 46
AT1G64380.1                         --LAGGPNTLDSIFSLLTPSSVESATTS-------------FNTHNPPPP 62

LOC_Os02g51670.1|11972.m10198|      GPMGLTHLGPAQ---------------IQQIQAQFLAQQQ-QQRALAG-- 135
LOC_Os06g11860.1|11976.m05903|      --VGLAHLGPEQ---------------IQQIQVQFLMQQQLQQRGMAASA 151
LOC_Os08g31580.1|11978.m07152|      --MGPAQLSPAQ---------------IQRVQAQLHMQRQ----AQSG-- 77
LOC_Os09g20350.1|11979.m05242|      --AQQPELSPAQ---------------MHYIQARLHLQRQ----AAQAG- 79
LOC_Os03g09170.1|11973.m06423|      LLAGANYLTPAQ---------------VLHVQAQLQRLRR--PGAASG-- 81
AT2G22200.1                         MPLGLNQLTPYQ---------------IHQIQNQLNHR-----RSTIS-- 48
AT4G39780.1                         TSLGLNQLTPYQ---------------IHQIQNQLNQR-----RNIISP- 75
AT5G65130.1                         GPIGLNQLTPTQ---------------ILQIQTELHLRQNQSRRRAGSH- 89
AT1G22190.1                         ISYGSDLH------------------------------------SFSHH- 61
AT1G78080.1                         FSYGSDLQQTGSLIGLNNLSSSQIHQIQSQIHHPLPPTHHNNNNSFSNL- 120
AT1G36060.1                         SSFGSDLQQPEN------------YQSQFHYQNTITYTHQDNNTCMLNF- 117
LOC_Os10g22600.1|11980.m05267|      GWYNYNLVTPSQ---------------AAQLHHRLRRAVGAAPCSMKRCG 101
LOC_Os05g49700.1|11975.m09040|      PPLGSSVYYRQS----------------ELLRHFAAS------------- 67
AT1G64380.1                         PQLGSSVYLRQR----------------DIIEKFHLQNRAISTPHPPLFS 96
```

FIGURE 8 (conitued)

```
LOC_Os02g51670.1|11972.m10198|      ------AFLRPRGQPMKQSGSPPRAGPFAAVAGA---------------- 163
LOC_Os06g11860.1|11976.m05903|      SASAAASYLGPRAQPMKQAG--------AAAAAA---------------- 177
LOC_Os08g31580.1|11978.m07152|      --------LGPRAQPMKP----ASAAAPAAAAAR---------------- 99
LOC_Os09g20350.1|11979.m05242|      -------PLGPRAQPMKASSSSASAAGAAATPPR---------------- 106
LOC_Os03g09170.1|11973.m06423|      ------CLAAAPPLPMKRHG---AVAVAAAAAAR---------------- 106
AT2G22200.1                         -------NLSPNRIRMKNLTP----------STS---------------- 65
AT4G39780.1                         -------NLAPKPVPMKNMT------------------------------ 88
AT5G65130.1                         -------LLTAKPTSMKKIDV----------AT----------------- 105
AT1G22190.1                         -------LSP-KPVSMKQTGTS----------AA---------------- 77
AT1G78080.1                         -------LSP-KPLLMKQSGVAGSCFAYGSGVPS---------------- 146
AT1G36060.1                         -------IEPSQPGFMTQPGPS-------SGSVS---------------- 137
LOC_Os10g22600.1|11980.m05267|      ------GMAAAAAGRLALVG-----------PAP---------------- 118
LOC_Os05g49700.1|11975.m09040|      ------QAAQAQSATAAASSSSAAAAGLDDG------------------- 92
AT1G64380.1                         STYDHHQTSELMLQAAAGSPAAAFAAALAAGRVT---------------- 130

LOC_Os02g51670.1|11972.m10198|      ----AQSKLYR---GVRQRH-----------------WGKWVAEIRLPKN 189
LOC_Os06g11860.1|11976.m05903|      ----AGGKMYR---GVRQRH-----------------WGKWVAEIRLPKN 203
LOC_Os08g31580.1|11978.m07152|      ----AQ-KLYR---GVRQRH-----------------WGKWVAEIRLPRN 124
LOC_Os09g20350.1|11979.m05242|      ----PQ-KLYR---GVRQRH-----------------WGKWVAEIRLPRN 131
LOC_Os03g09170.1|11973.m06423|      ----APVKLYR---GVRQRH-----------------WGKWVAEIRLPRN 132
AT2G22200.1                         ----KTKNLYR---GVRQRH-----------------WGKWVAEIRLPKN 91
AT4G39780.1                         ------AQKLYR---GVRQRH----------------WGKWVAEIRLPKN 113
AT5G65130.1                         ----KPVKLYR---GVRQRQ-----------------WGKWVAEIRLPKN 131
AT1G22190.1                         ----KPTKLYR---GVRQRH-----------------WGKWVAEIRLPRN 103
AT1G78080.1                         ----KPTKLYR---GVRQRH-----------------WGKWVAEIRLPRN 172
AT1G36060.1                         ----KPAKLYR---GVRQRH-----------------WGKWVAEIRLPRN 163
LOC_Os10g22600.1|11980.m05267|      ----VQAKLYR---GVRQRH-----------------WGKWVAEIRLPRN 144
LOC_Os05g49700.1|11975.m09040|      ----APRKLYR---GVRQRQ-----------------WGKWVAEIRLPQN 118
AT1G64380.1                         ----KKKKLYR---GVRQRH-----------------WGKWVAEIRLPQN 156
```

MOTIF XVIII

AP2 DNA-binding domain

FIGURE 8 (conitued)

```
LOC_Os02g51670.1|11972.m10198|    RT---------------------------RLWLGTFDTAEDAALAYDKAA 212
LOC_Os06g11860.1|11976.m05903|    RT---------------------------RLWLGTFDTAEDAALAYDKAA 226
LOC_Os08g31580.1|11978.m07152|    RT---------------------------RLWLGTFDTAEEAALTYDQAA 147
LOC_Os09g20350.1|11979.m05242|    RT---------------------------RLWLGTFDTAEEAALAYDQAA 154
LOC_Os03g09170.1|11973.m06423|    RT---------------------------RLWLGTFDTAEEAALAYDSAA 155
AT2G22200.1                       RT---------------------------RLWLGTFETAEKAALAYDQAA 114
AT4G39780.1                       RT---------------------------RLWLGTFDTAEEAAMAYDLAA 136
AT5G65130.1                       RT---------------------------RLWLGTFETAQEAALAYDQAA 154
AT1G22190.1                       RT---------------------------RLWLGTFDTAEEAALAYDKAA 126
AT1G78080.1                       RT---------------------------RLWLGTFDTAEEAALAYDKAA 195
AT1G36060.1                       RT---------------------------RLWLGTFDTAEEAALAYDRAA 186
LOC_Os10g22600.1|11980.m05267|    RT---------------------------RLWLGTYDTAEDAALAYDGAA 167
LOC_Os05g49700.1|11975.m09040|    RV---------------------------RVWLGTYDSPETAAHAYDRAA 141
AT1G64380.1                       RM---------------------------RVWLGTYDTAEAAAYAYDRAA 179
```

**MOTIF XVIII**

**AP2 DNA-binding domain**

```
LOC_Os02g51670.1|11972.m10198|    FRLRGDL---------ARLNFPTLRRGGAHL------------------- 234
LOC_Os06g11860.1|11976.m05903|    FRLRGDA---------ARLNFPTLRRGGAHL------------------- 248
LOC_Os08g31580.1|11978.m07152|    YRLRGDA---------ARLNFP----DNAAS------------------- 165
LOC_Os09g20350.1|11979.m05242|    YRLRGDA---------ARLNFP----DNAAS------------------- 172
LOC_Os03g09170.1|11973.m06423|    FRLRGES---------ARLNFPELRRGGAHL------------------- 177
AT2G22200.1                       FQLRGDI---------AKLNFPNLIHED---------------------- 133
AT4G39780.1                       YKLRGEF---------ARLNFPQFRHEDGYYG-GG----------SC--- 163
AT5G65130.1                       HKIRGDN---------ARLNFPDIVRQG-----------------HY--- 175
AT1G22190.1                       YKLRGDF---------ARLNFPDLRHN------------------DE--- 146
AT1G78080.1                       YKLRGDF---------ARLNFPNLRHNGSHIG-GDF---------GE--- 223
AT1G36060.1                       FKLRGDS---------ARLNFPALRYQTGSSP-SDT---------GE--- 214
LOC_Os10g22600.1|11980.m05267|    FRLRGDA---------ARLNFPELRRGGRHH------------------- 189
LOC_Os05g49700.1|11975.m09040|    FKLRGEY---------ARLNFPGVMD-----GRDCP---------DN--- 165
AT1G64380.1                       YKLRGEY---------ARLNFPNLKDPSELLGLGDS---------SK--- 208
```

**MOTIF XIX**

**AP2 DNA-binding domain**

FIGURE 8 (conitued)

```
LOC_Os02g51670.1|11972.m10198|     ---------------------AGPLHASVDAKLTAICQSLATSSSKNTPA 263
LOC_Os06g11860.1|11976.m05903|     ---------------------AGPLHASIDAKLTAICHSLAAAPPASS-K 276
LOC_Os08g31580.1|11978.m07152|     ---------------------RGPLDAAVDAKLQAICDTIAASKNASSRS 194
LOC_Os09g20350.1|11979.m05242|     ---------------------RGPLHASVDAKLQTLCQNIAAAKNAK-KS 200
LOC_Os03g09170.1|11973.m06423|     ---------------------GPPLHAAVDAKLHAICHGMDLPQPQPQTQ 206
AT2G22200.1                        ---------------------MNPLPSSVDTKLQAICKSLRKTEEICSVS 162
AT4G39780.1                        ---------------------FNPLHSSVDAKLQEICQSLRKTEDIDLPC 192
AT5G65130.1                        ---------------------KQILSPSINAKIESICNSS----DLPLPQ 200
AT1G22190.1                        ---------------------YQPLQSSVDAKLEAICQNLAETTQKQVRS 175
AT1G78080.1                        ---------------------YKPLHSSVDAKLEAICKSMAET-QKQDKS 251
AT1G36060.1                        ---------------------YGPIQAAVDAKLEAI---LAEPKNQPGKT 240
LOC_Os10g22600.1|11980.m05267|     ---------------------APALSASVDAKILQATTTTTADTAAAAAP 218
LOC_Os05g49700.1|11975.m09040|     ---------------------LRQLRDAVDAKIQAIRVRMARKRARARRQ 194
AT1G64380.1                        ---------------------LIALKNAVDGKIQSICQRVRKE--RAKKS 235

LOC_Os02g51670.1|11972.m10198|     ESAAS-------------------------AAEPESPKCSAST-----EG 283
LOC_Os06g11860.1|11976.m05903|     KAAAA-------------------------AAHPDSPKGSASTTTTTSEG 301
LOC_Os08g31580.1|11978.m07152|     RGGAG-------------------------RAMPINAPLVAAASSSSGSD 219
LOC_Os09g20350.1|11979.m05242|     SVSAS-------------------------AAATSSAPTSNCSSPSSDDA 225
LOC_Os03g09170.1|11973.m06423|     SNATT-------------------------TTMSTTATNTPSPFFSSESP 231
AT2G22200.1                        DQTKEYSVYSVSDKTEL-----FLPKAELFLPKREHLETNELSNESPRSD 207
AT4G39780.1                        SET------------------------ELFPPKTEYQES---EYGFLRSD 215
AT5G65130.1                        IEK---------------------------QNKTEEVLS-----GFSKPE 218
AT1G22190.1                        TKKSS--------------------------SRKR---SSTVAVKLPEED 196
AT1G78080.1                        TKSSK--------------------------KREKKVSSPDLSEKVKAEE 275
AT1G36060.1                        ERTSR--------------------------KRAK-------AAASSAEQ 257
LOC_Os10g22600.1|11980.m05267|     ASTNTTPPPSPRVVKTE-----PGCCSVSEASTTTTADAADVSSTGSSPS 263
LOC_Os05g49700.1|11975.m09040|     REESK------------------------KSQRAEDAKAATPSRPVASER 220
AT1G64380.1                        VKVSK------------------------NSSATADSSCLS-SPEILSSS 260
```

FIGURE 8 (conitued)

```
LOC_Os02g51670.1|11972.m10198|    -EDSVS---------------------------------------AGSPP 293
LOC_Os06g11860.1|11976.m05903|    DESAIS---------------------------------------ACSPP 312
LOC_Os08g31580.1|11978.m07152|    HSGGGD---------------------------------------DGGSE 230
LOC_Os09g20350.1|11979.m05242|    SSCLES---------------------------------------ADSSP 236
LOC_Os03g09170.1|11973.m06423|    VVKSEP---------------------------------------VCSAS 242
AT2G22200.1                       -ETSLL---------------------------------------DESQA 217
AT4G39780.1                       -ENSFS---------------------------------------DESHV 225
AT5G65130.1                       KEPEFG---------------------------------------EIYGC 229
AT1G22190.1                       YS-SAG---------------------------------------SSPLL 206
AT1G78080.1                       NSVSIG---------------------------------------GSPPV 286
AT1G36060.1                       PS-------------------------------------------APQQH 264
LOC_Os10g22600.1|11980.m05267|    PTSSNQ---------------------------------------AATAT 274
LOC_Os05g49700.1|11975.m09040|    AASETT---------------------------------------TTTTT 231
AT1G64380.1                       PVTTTT---------------------------------------TAVTS 271

LOC_Os02g51670.1|11972.m10198|    PPTPLSPP----------VPEMEKLDFTEAPWD-ESETF----------- 321
LOC_Os06g11860.1|11976.m05903|    LPPPPPPPP-------AALPEMANLDFTEAPWD-ESDAF----------- 343
LOC_Os08g31580.1|11978.m07152|    TSSSSAAAS--------PLAEMEQLDFSEVPWD-EAEGF----------- 260
LOC_Os09g20350.1|11979.m05242|    SLSPSSAATTAET--PATVPEMQQLDFSEAPWD-EAAAF----------- 272
LOC_Os03g09170.1|11973.m06423|    ESSSSADGDVSSTGSSDVVPEMQLLDFSEAPWD-ESESF----------- 280
AT2G22200.1                       EYS-SSD--------------KTFLDFSDTEFE-EIGSF----------- 240
AT4G39780.1                       ESS-SPESG-----------ITTFLDFSDSGFD-EIGSF----------- 251
AT5G65130.1                       GYSGSSPES-----------DITLLDFSSDCVK-EDESFL---------- 257
AT1G22190.1                       TESYGSGGS------SSPLSELTFGDTEEEIQPPWNEN------------ 238
AT1G78080.1                       TEFEESTAG------SSPLSDLTFADPEEPPQ--WNETF----------- 317
AT1G36060.1                       SGSGESDGS------GSPTSDVMVQEMCQEPEMPWNENF----------- 297
LOC_Os10g22600.1|11980.m05267|    PPAPRPPPP--------LPETIQQLDFTEAPWD-EADGF----------- 304
LOC_Os05g49700.1|11975.m09040|    TSSSYGSPD--------GVLSMS-----AASVDGDCP------------- 255
AT1G64380.1                       EDSYWVSPM--------GLCNSENSSPVSVSVPSEVPATAE-----EEAM 308
```

FIGURE 8 (conitued)

```
LOC_Os02g51670.1|11972.m10198|      ----------------------------------------HLRKYPSWEI 331
LOC_Os06g11860.1|11976.m05903|      ----------------------------------------HLYKCPSWEI 353
LOC_Os08g31580.1|11978.m07152|      ----------------------------------------ALTKYPSYEI 270
LOC_Os09g20350.1|11979.m05242|      ----------------------------------------ALTKYPSYEI 282
LOC_Os03g09170.1|11973.m06423|      ----------------------------------------LLHKYPSLEI 290
AT2G22200.1                         ----------------------------------------GLRKFPSVEI 250
AT4G39780.1                         ----------------------------------------GLEKFPSVEI 261
AT5G65130.1                         ---------------------------------------MGLHKYPSLEI 268
AT1G22190.1                         ----------------------------------------ALEKYPSYEI 248
AT1G78080.1                         ----------------------------------------SLEKYPSYEI 327
AT1G36060.1                         ----------------------------------------MLGKCPSYEI 307
LOC_Os10g22600.1|11980.m05267|      ----------------------------------------ALRRYPSWEI 314
LOC_Os05g49700.1|11975.m09040|      -----------------------------------------LERMPSFDP 264
AT1G64380.1                         MGV--------------------------------DTDGFLLARMPSFDP 326

LOC_Os02g51670.1|11972.m10198|      DWDSILS------------------------------------------- 338
LOC_Os06g11860.1|11976.m05903|      DWDSILS------------------------------------------- 360
LOC_Os08g31580.1|11978.m07152|      DWDSLLNNNN---------------------------------------- 280
LOC_Os09g20350.1|11979.m05242|      DWDSLLAAN----------------------------------------- 291
LOC_Os03g09170.1|11973.m06423|      DWDAILS------------------------------------------- 297
AT2G22200.1                         DWDAISKLANS--------------------------------------- 261
AT4G39780.1                         DWDAISKLSES--------------------------------------- 272
AT5G65130.1                         DWDAIEKLF----------------------------------------- 277
AT1G22190.1                         DWDSILQCSSLVN------------------------------------- 261
AT1G78080.1                         DWDSILA------------------------------------------- 334
AT1G36060.1                         DWASILS------------------------------------------- 314
LOC_Os10g22600.1|11980.m05267|      DWDAILS------------------------------------------- 321
LOC_Os05g49700.1|11975.m09040|      ELIWEMLNF----------------------------------------- 273
AT1G64380.1                         ELIWEVLAN----------------------------------------- 335
```

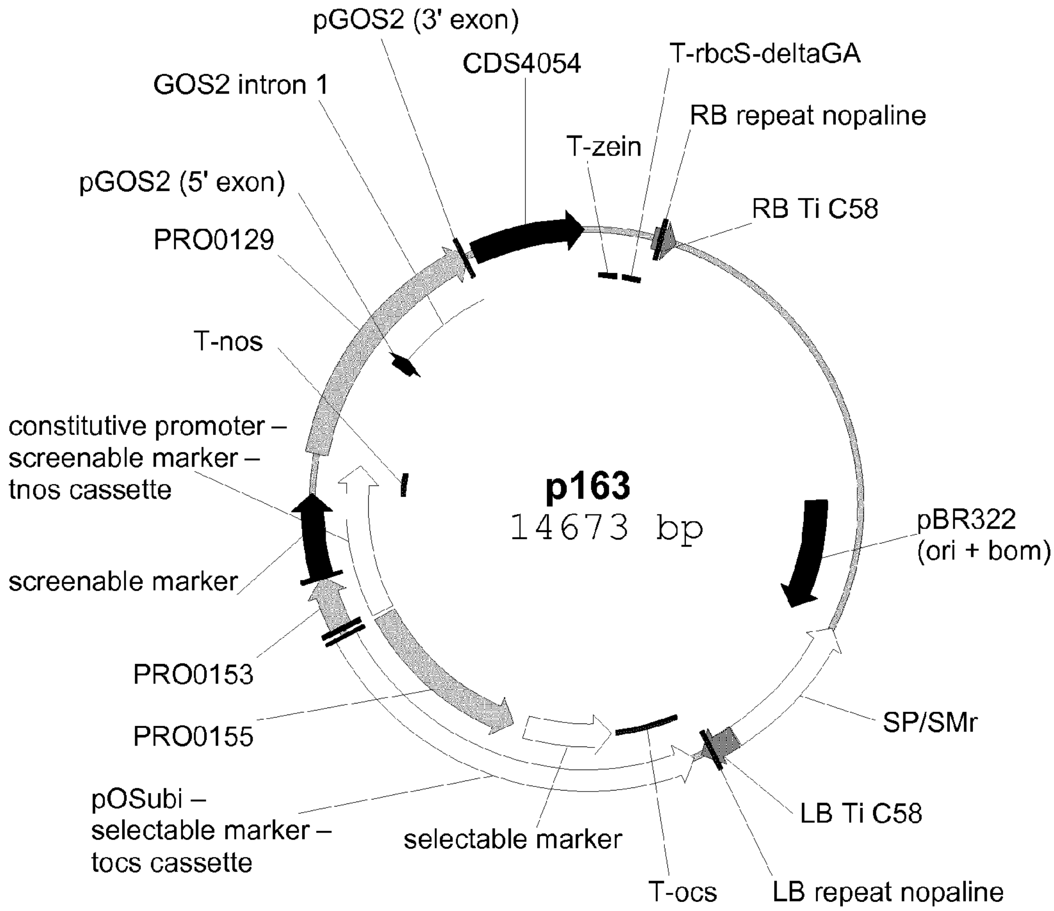
pGOS2 (3' exon)
CDS4054
T-rbcS-deltaGA
GOS2 intron 1
RB repeat nopaline
T-zein
pGOS2 (5' exon)
RB Ti C58
PRO0129
T-nos
constitutive promoter – screenable marker – tnos cassette
p163
14673 bp
pBR322 (ori + bom)
screenable marker
PRO0153
SP/SMr
PRO0155
LB Ti C58
pOSubi – selectable marker – tocs cassette
selectable marker
T-ocs
LB repeat nopaline

FIGURE 9

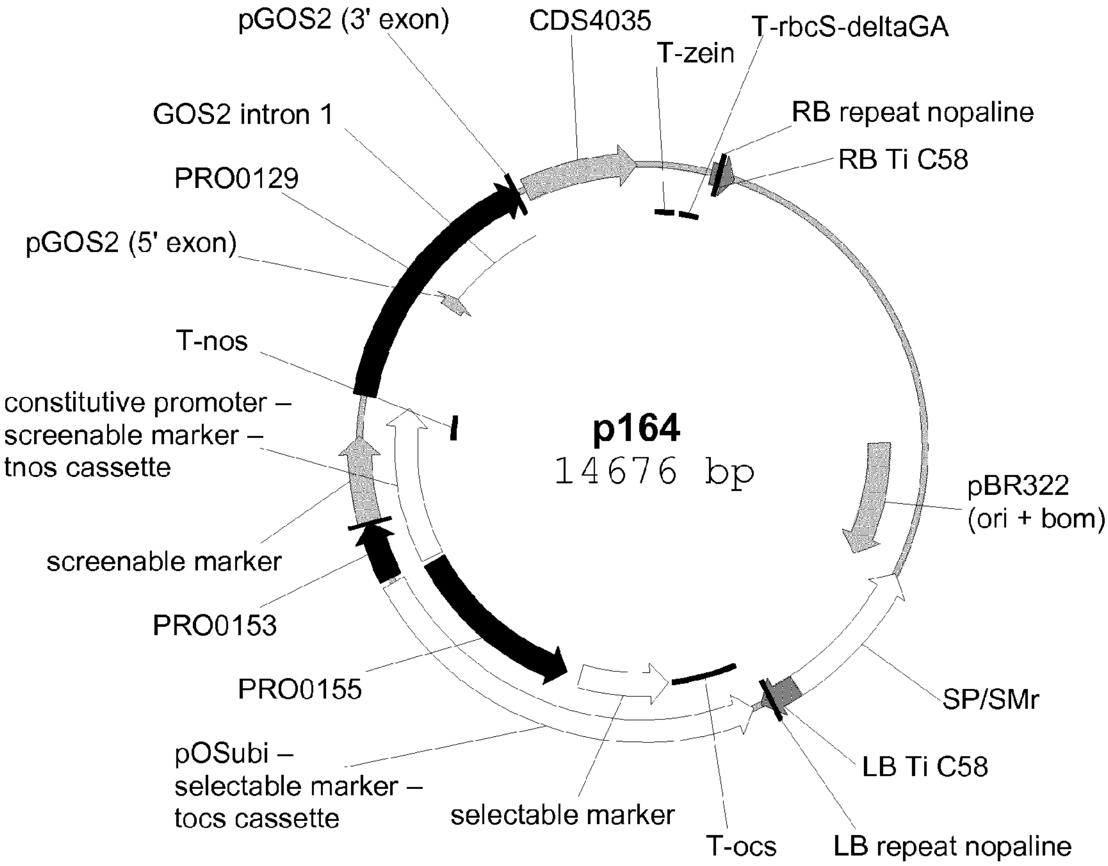
pGOS2 (3' exon)
CDS4035
T-zein
T-rbcS-deltaGA
GOS2 intron 1
RB repeat nopaline
RB Ti C58
PRO0129
pGOS2 (5' exon)
T-nos
constitutive promoter – screenable marker – tnos cassette
p164
14676 bp
pBR322 (ori + bom)
screenable marker
PRO0153
PRO0155
SP/SMr
pOSubi – selectable marker – tocs cassette
LB Ti C58
selectable marker
T-ocs
LB repeat nopaline

FIGURE 10

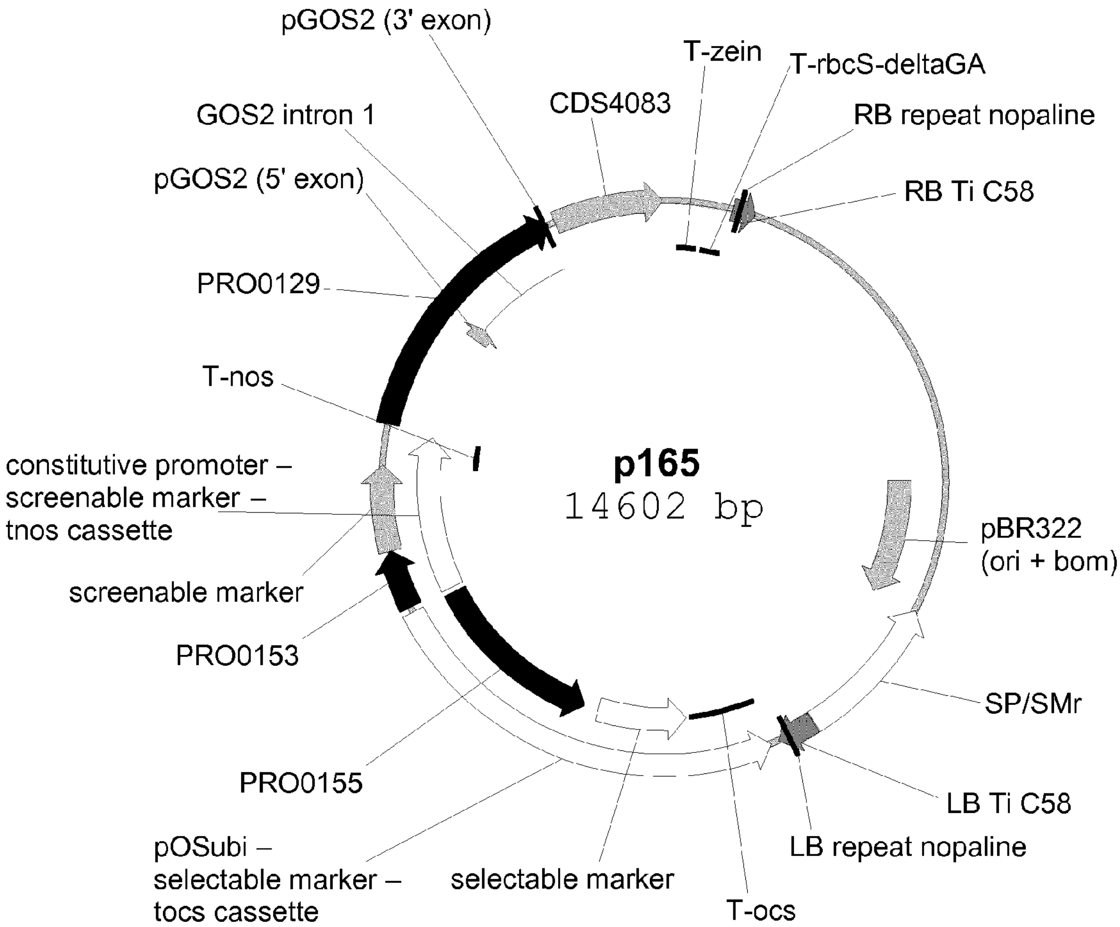
pGOS2 (3' exon)
T-zein
T-rbcS-deltaGA
GOS2 intron 1
CDS4083
RB repeat nopaline
pGOS2 (5' exon)
RB Ti C58
PRO0129
T-nos
p165
14602 bp
constitutive promoter – screenable marker – tnos cassette
pBR322 (ori + bom)
screenable marker
PRO0153
SP/SMr
PRO0155
LB Ti C58
LB repeat nopaline
pOSubi – selectable marker – tocs cassette
selectable marker
T-ocs

FIGURE 11

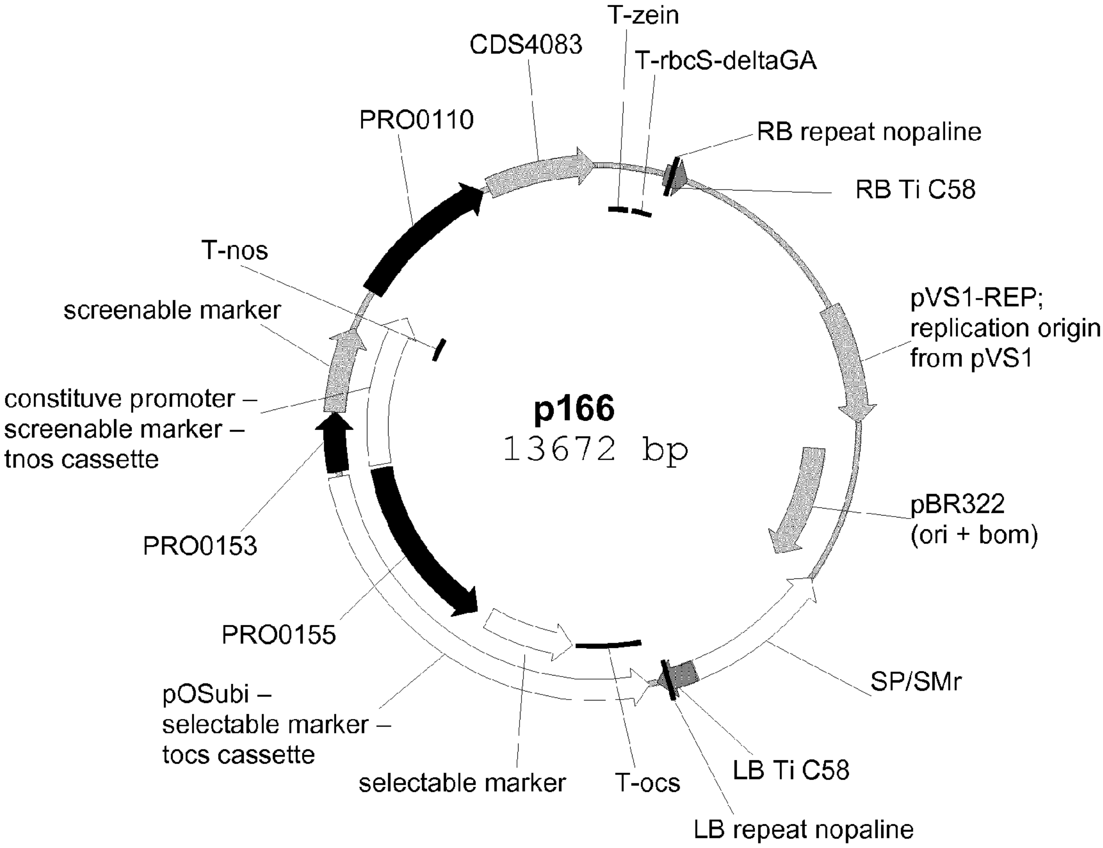
T-zein
CDS4083
T-rbcS-deltaGA
PRO0110
RB repeat nopaline
RB Ti C58
T-nos
screenable marker
pVS1-REP;
replication origin
from pVS1
constituve promoter –
screenable marker –
tnos cassette
p166
13672 bp
pBR322
(ori + bom)
PRO0153
PRO0155
SP/SMr
pOSubi –
selectable marker –
tocs cassette
LB Ti C58
selectable marker
T-ocs
LB repeat nopaline

FIGURE 12

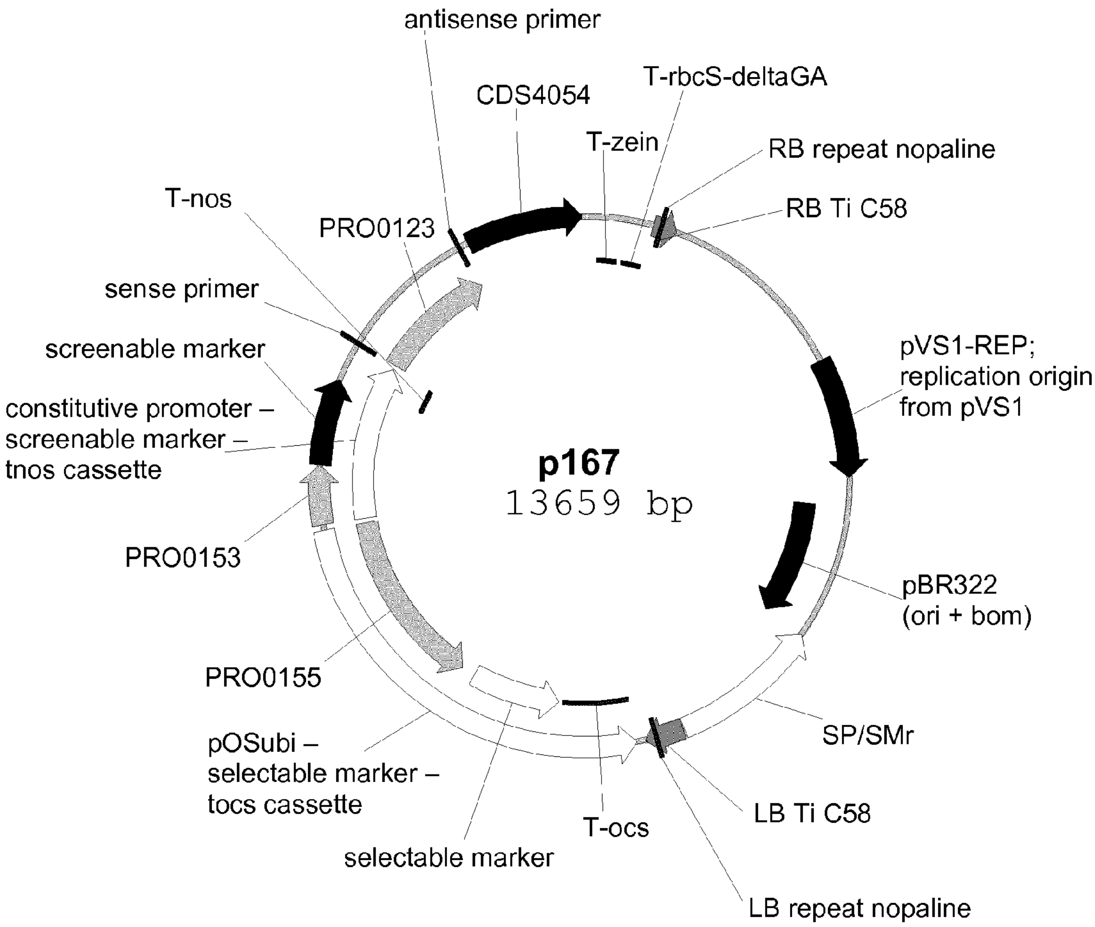
antisense primer
CDS4054
T-rbcS-deltaGA
T-zein
RB repeat nopaline
RB Ti C58
T-nos
PRO0123
sense primer
screenable marker
pVS1-REP;
replication origin
from pVS1
constitutive promoter –
screenable marker –
tnos cassette
p167
13659 bp
PRO0153
pBR322
(ori + bom)
PRO0155
SP/SMr
pOSubi –
selectable marker –
tocs cassette
LB Ti C58
T-ocs
selectable marker
LB repeat nopaline

FIGURE 13

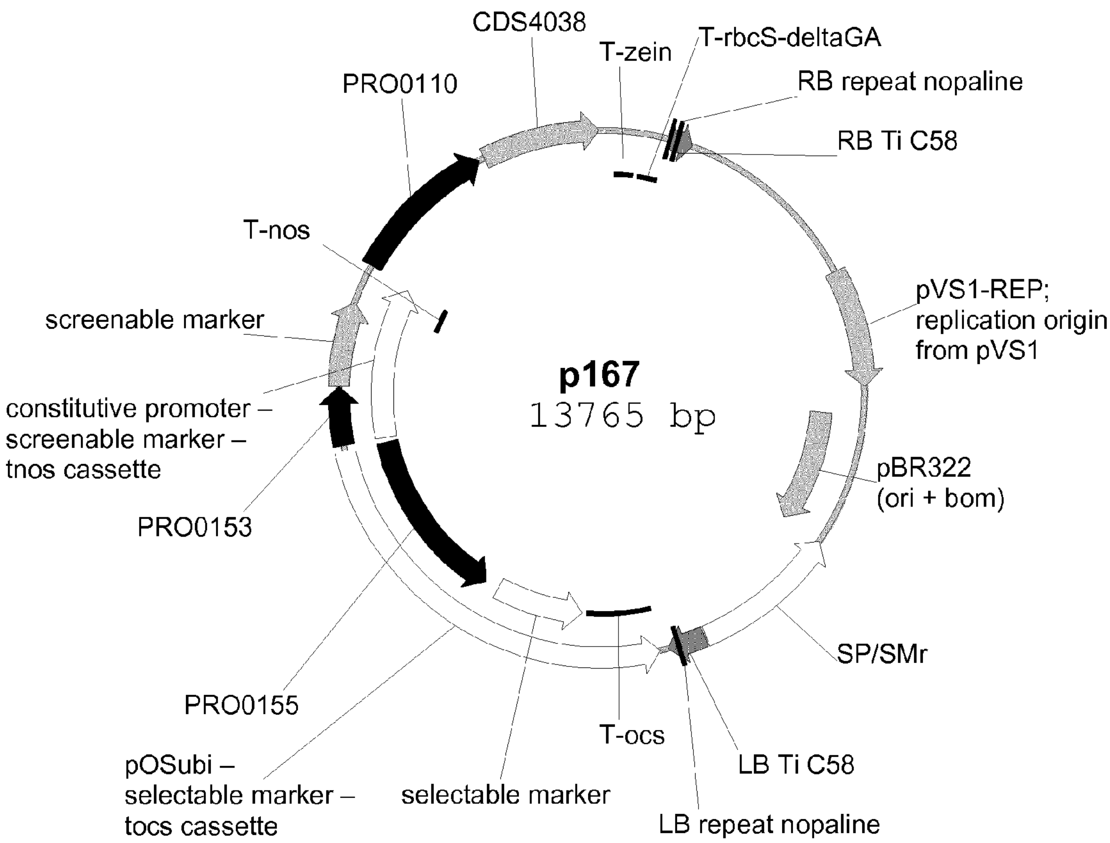
CDS4038
T-zein
T-rbcS-deltaGA
RB repeat nopaline
RB Ti C58
PRO0110
T-nos
pVS1-REP;
replication origin
from pVS1
screenable marker
p167
13765 bp
constitutive promoter –
screenable marker –
tnos cassette
pBR322
(ori + bom)
PRO0153
SP/SMr
PRO0155
T-ocs
LB Ti C58
pOSubi –
selectable marker –
tocs cassette
selectable marker
LB repeat nopaline

FIGURE 14

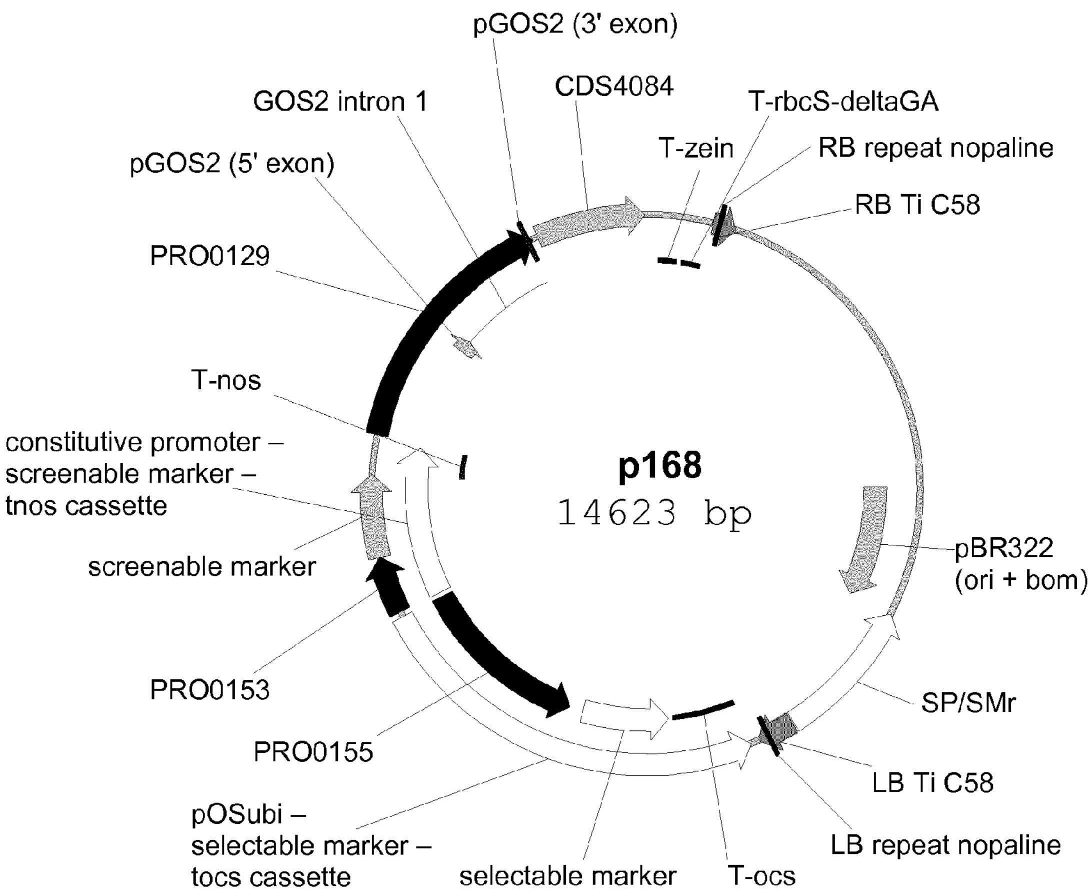
pGOS2 (3' exon)
CDS4084
GOS2 intron 1
T-rbcS-deltaGA
T-zein
RB repeat nopaline
pGOS2 (5' exon)
RB Ti C58
PRO0129
T-nos
constitutive promoter – screenable marker – tnos cassette
p168
14623 bp
pBR322 (ori + bom)
screenable marker
SP/SMr
PRO0153
PRO0155
LB Ti C58
pOSubi – selectable marker – tocs cassette
LB repeat nopaline
selectable marker
T-ocs

FIGURE 15

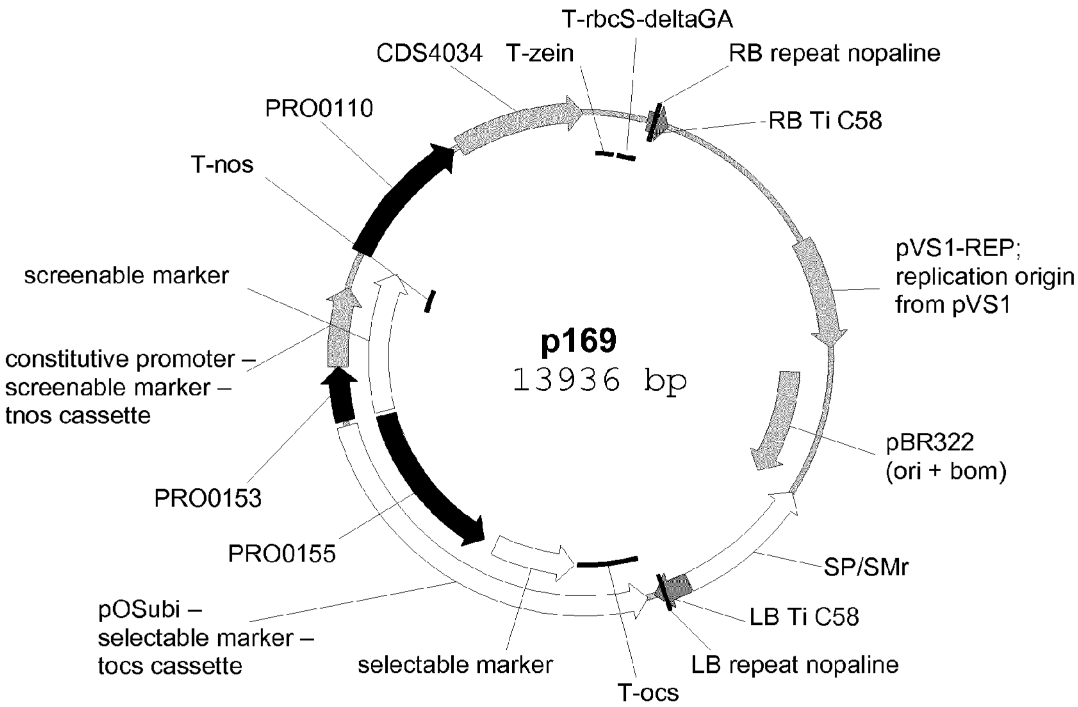
T-rbcS-deltaGA
CDS4034
T-zein
RB repeat nopaline
PRO0110
RB Ti C58
T-nos
pVS1-REP;
replication origin
from pVS1
screenable marker
constitutive promoter –
screenable marker –
tnos cassette
p169
13936 bp
pBR322
(ori + bom)
PRO0153
PRO0155
SP/SMr
pOSubi –
selectable marker –
tocs cassette
LB Ti C58
selectable marker
LB repeat nopaline
T-ocs

FIGURE 16

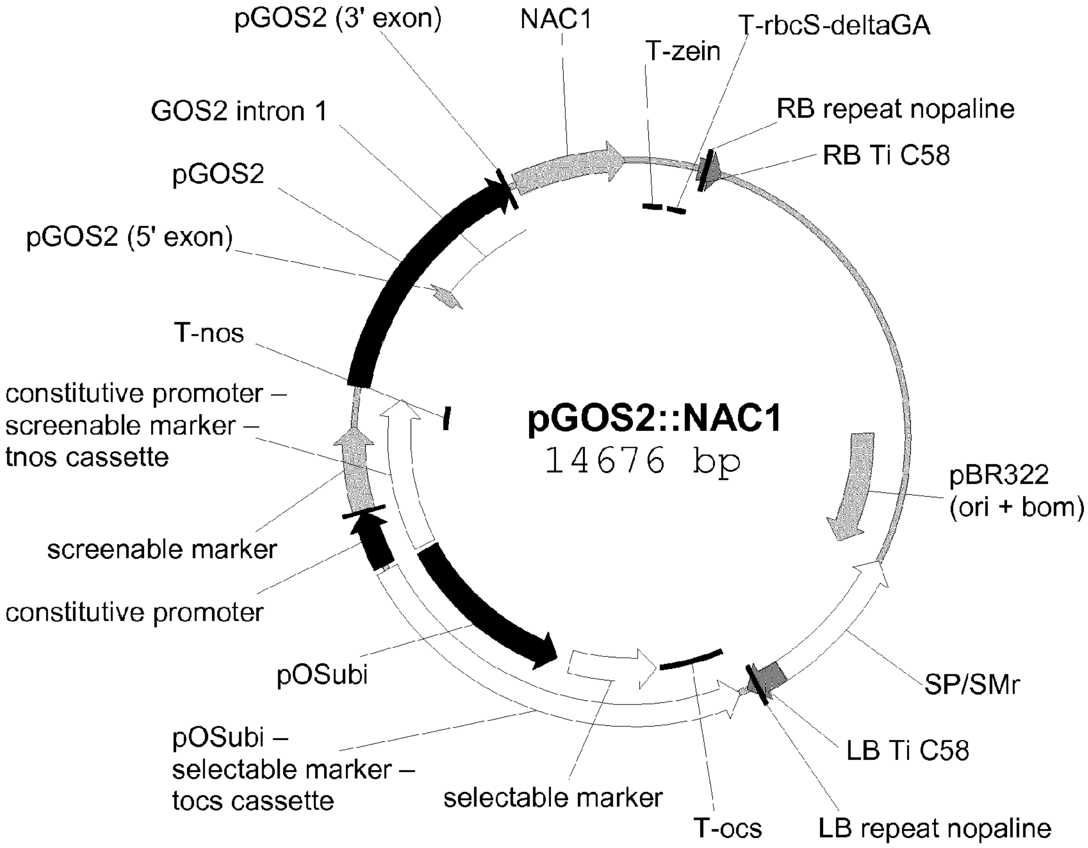
pGOS2 (3' exon)
NAC1
T-zein
T-rbcS-deltaGA
RB repeat nopaline
RB Ti C58
GOS2 intron 1
pGOS2
pGOS2 (5' exon)
T-nos
constitutive promoter – screenable marker – tnos cassette
pGOS2::NAC1
14676 bp
pBR322 (ori + bom)
screenable marker
constitutive promoter
pOSubi
SP/SMr
pOSubi – selectable marker – tocs cassette
selectable marker
LB Ti C58
T-ocs
LB repeat nopaline

FIGURE 17

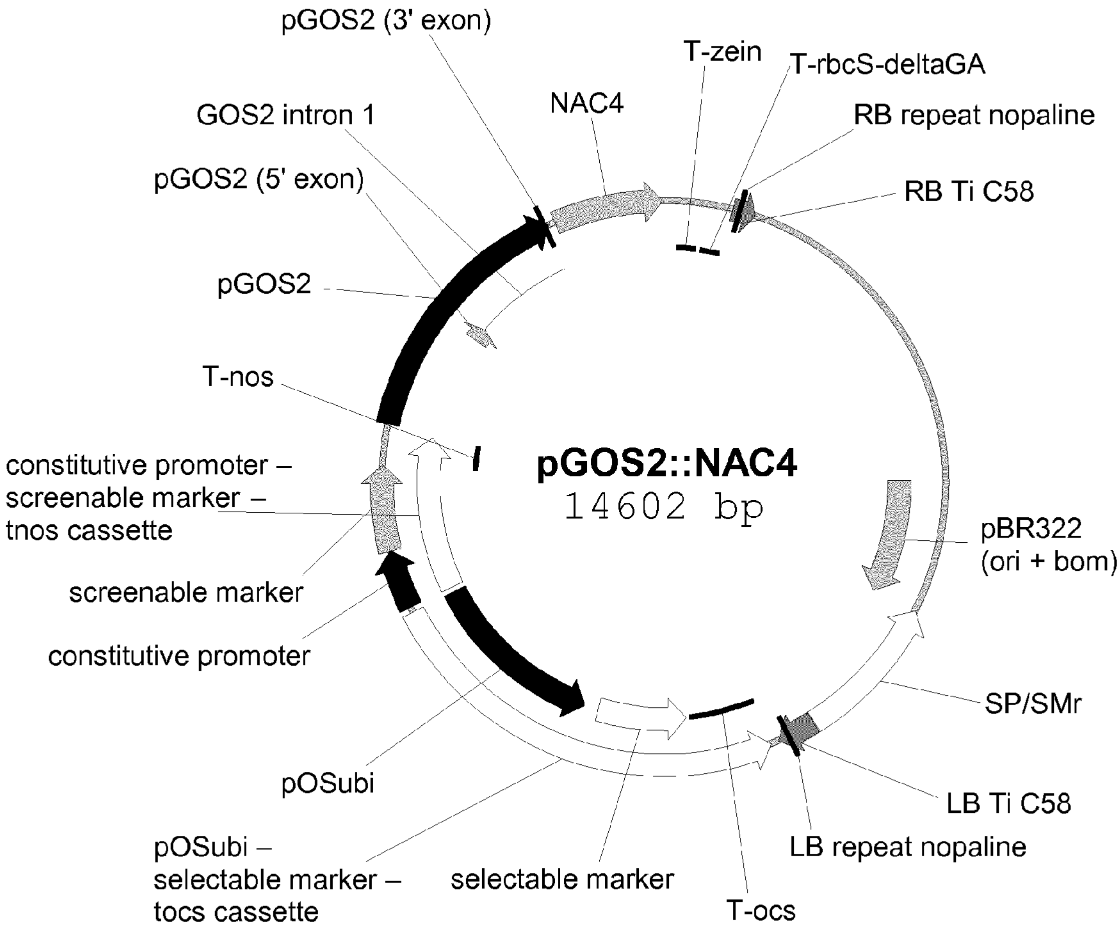
pGOS2 (3' exon)
T-zein
T-rbcS-deltaGA
GOS2 intron 1
NAC4
RB repeat nopaline
pGOS2 (5' exon)
RB Ti C58
pGOS2
T-nos
pGOS2::NAC4
14602 bp
constitutive promoter – screenable marker – tnos cassette
pBR322 (ori + bom)
screenable marker
constitutive promoter
SP/SMr
pOSubi
LB Ti C58
LB repeat nopaline
pOSubi – selectable marker – tocs cassette
selectable marker
T-ocs

FIGURE 18

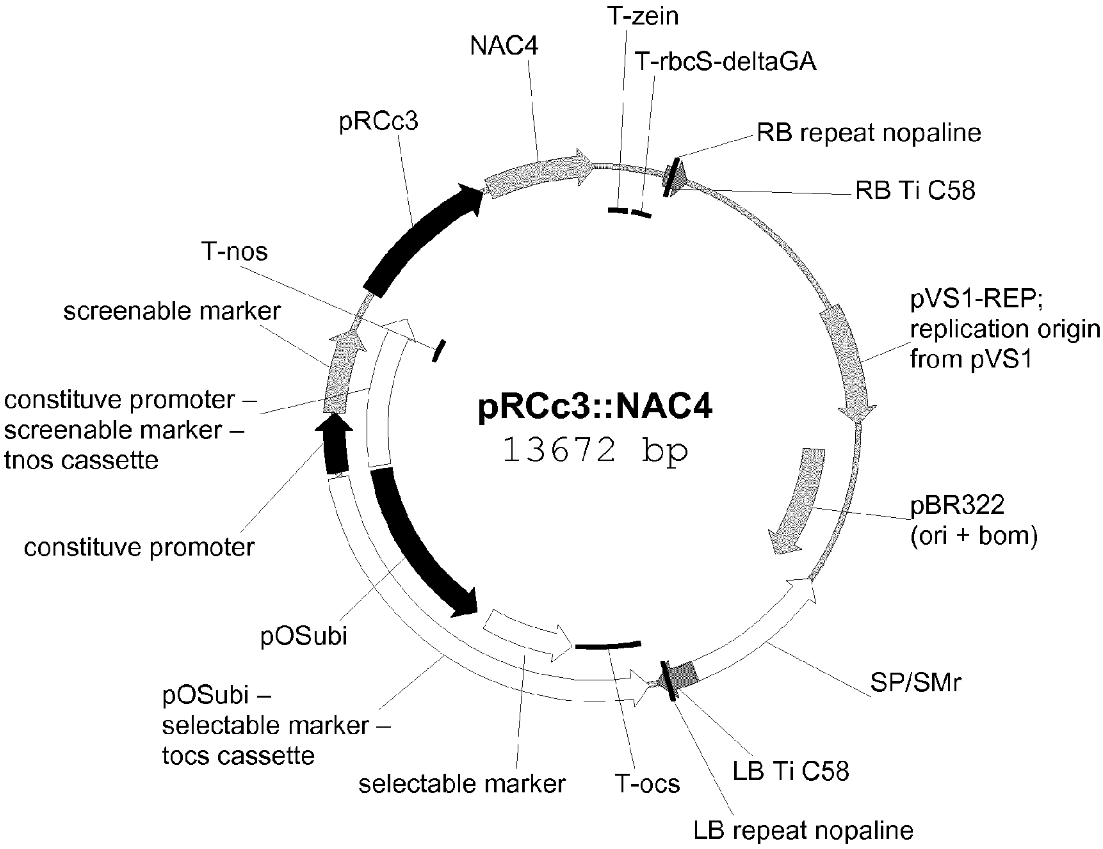
T-zein
NAC4
T-rbcS-deltaGA
pRCc3
RB repeat nopaline
RB Ti C58
T-nos
screenable marker
pVS1-REP;
replication origin
from pVS1
constituve promoter –
screenable marker –
tnos cassette
pRCc3::NAC4
13672 bp
pBR322
(ori + bom)
constituve promoter
pOSubi
SP/SMr
pOSubi –
selectable marker –
tocs cassette
selectable marker
T-ocs
LB Ti C58
LB repeat nopaline

FIGURE 19

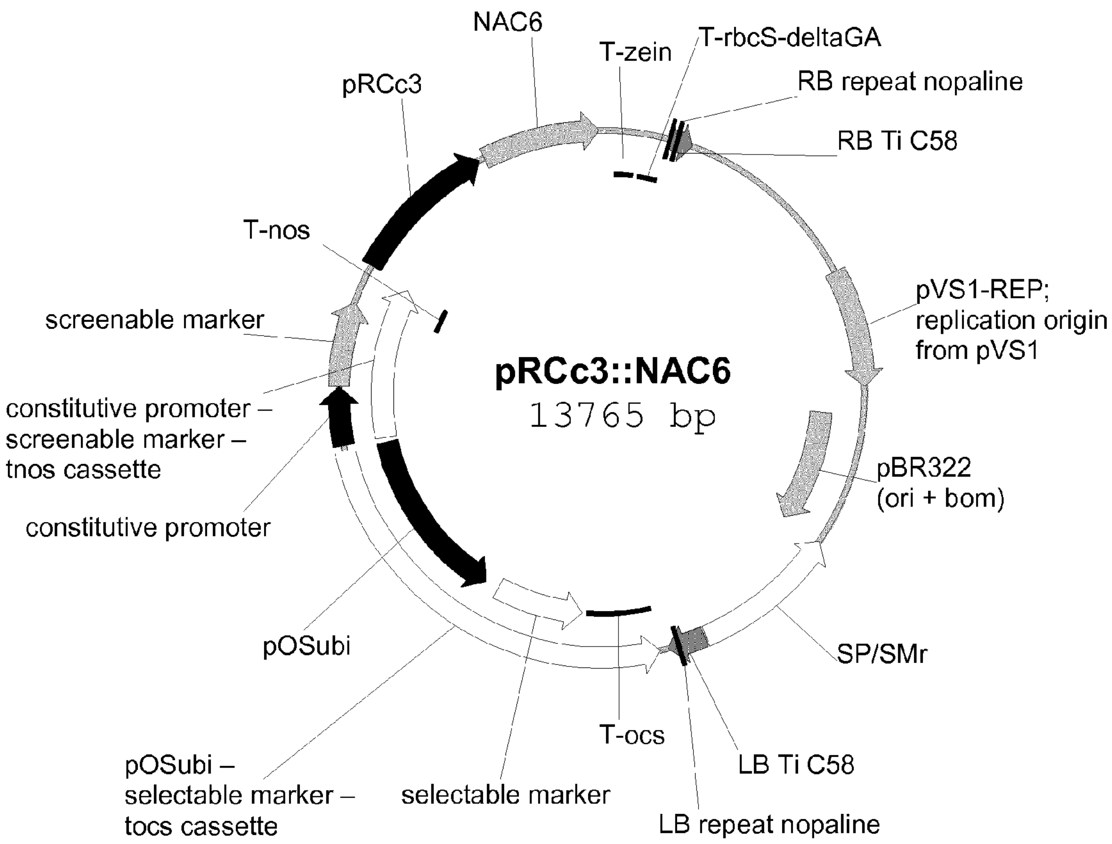
NAC6
T-zein
T-rbcS-deltaGA
pRCc3
RB repeat nopaline
RB Ti C58
T-nos
pVS1-REP;
replication origin
from pVS1
screenable marker
pRCc3::NAC6
13765 bp
constitutive promoter –
screenable marker –
tnos cassette
pBR322
(ori + bom)
constitutive promoter
pOSubi
SP/SMr
T-ocs
pOSubi –
selectable marker –
tocs cassette
LB Ti C58
selectable marker
LB repeat nopaline

FIGURE 20

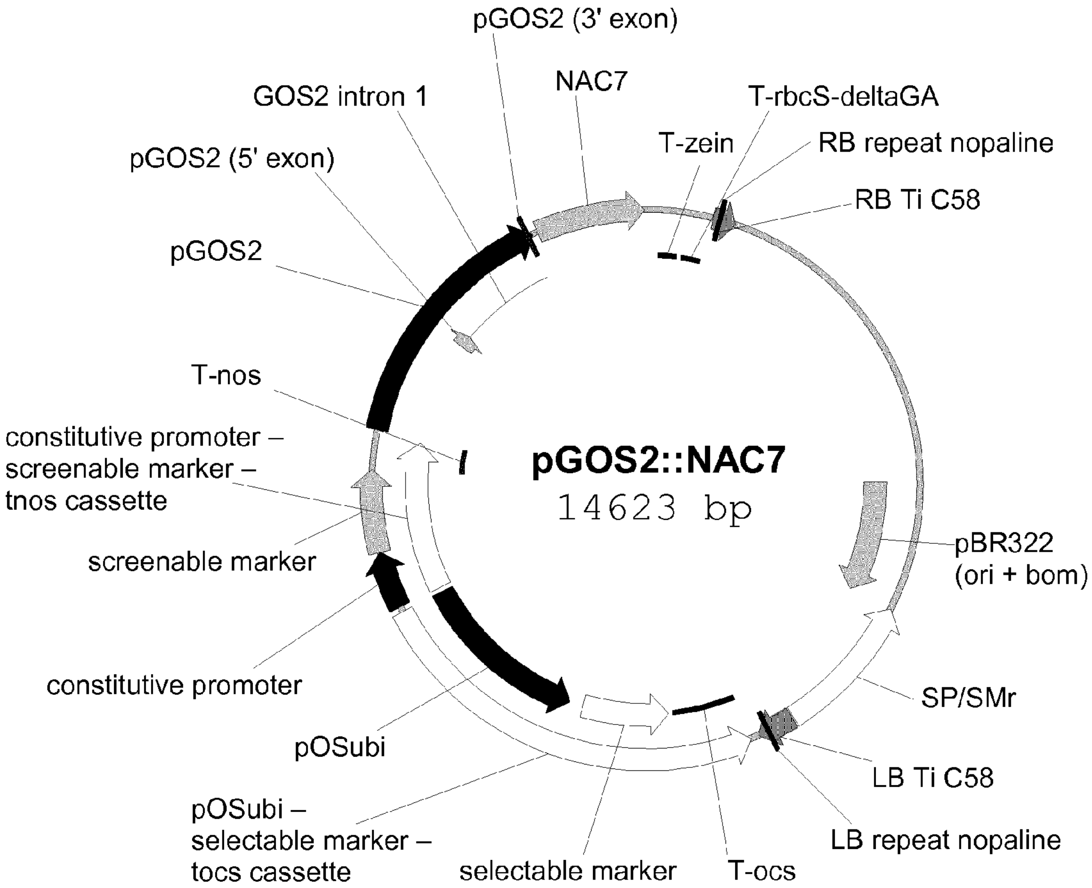
pGOS2 (3' exon)
NAC7
GOS2 intron 1
T-rbcS-deltaGA
T-zein
RB repeat nopaline
pGOS2 (5' exon)
RB Ti C58
pGOS2
T-nos
pGOS2::NAC7
14623 bp
constitutive promoter –
screenable marker –
tnos cassette
pBR322
(ori + bom)
screenable marker
SP/SMr
constitutive promoter
LB Ti C58
pOSubi
pOSubi –
selectable marker –
tocs cassette
LB repeat nopaline
selectable marker
T-ocs

FIGURE 21

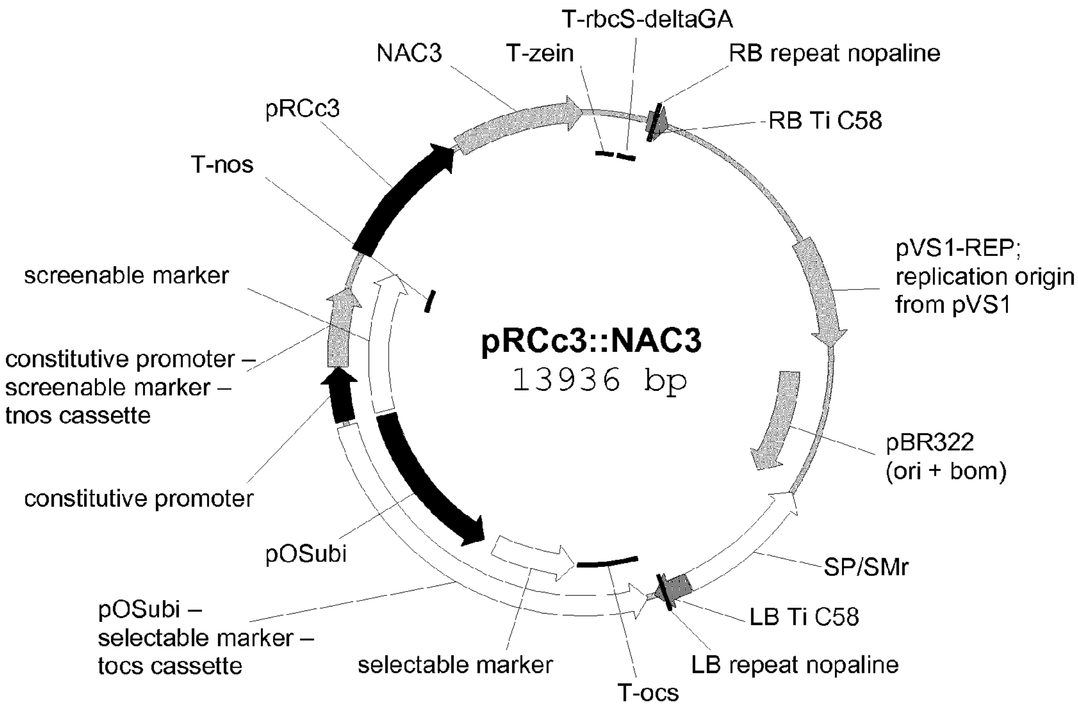
T-rbcS-deltaGA
NAC3
T-zein
RB repeat nopaline
pRCc3
RB Ti C58
T-nos
pVS1-REP; replication origin from pVS1
screenable marker
pRCc3::NAC3
13936 bp
constitutive promoter – screenable marker – tnos cassette
pBR322 (ori + bom)
constitutive promoter
pOSubi
SP/SMr
pOSubi – selectable marker – tocs cassette
LB Ti C58
selectable marker
LB repeat nopaline
T-ocs

FIGURE 22

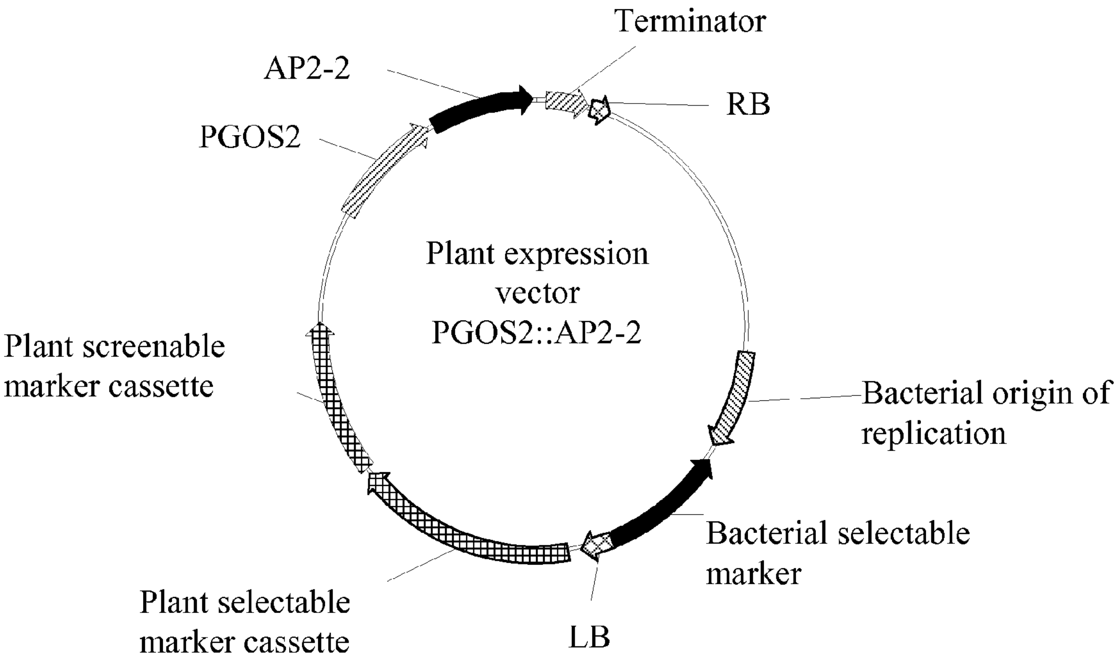
Terminator
AP2-2
RB
PGOS2
Plant expression vector PGOS2::AP2-2
Plant screenable marker cassette
Bacterial origin of replication
Bacterial selectable marker
Plant selectable marker cassette
LB

FIGURE 23

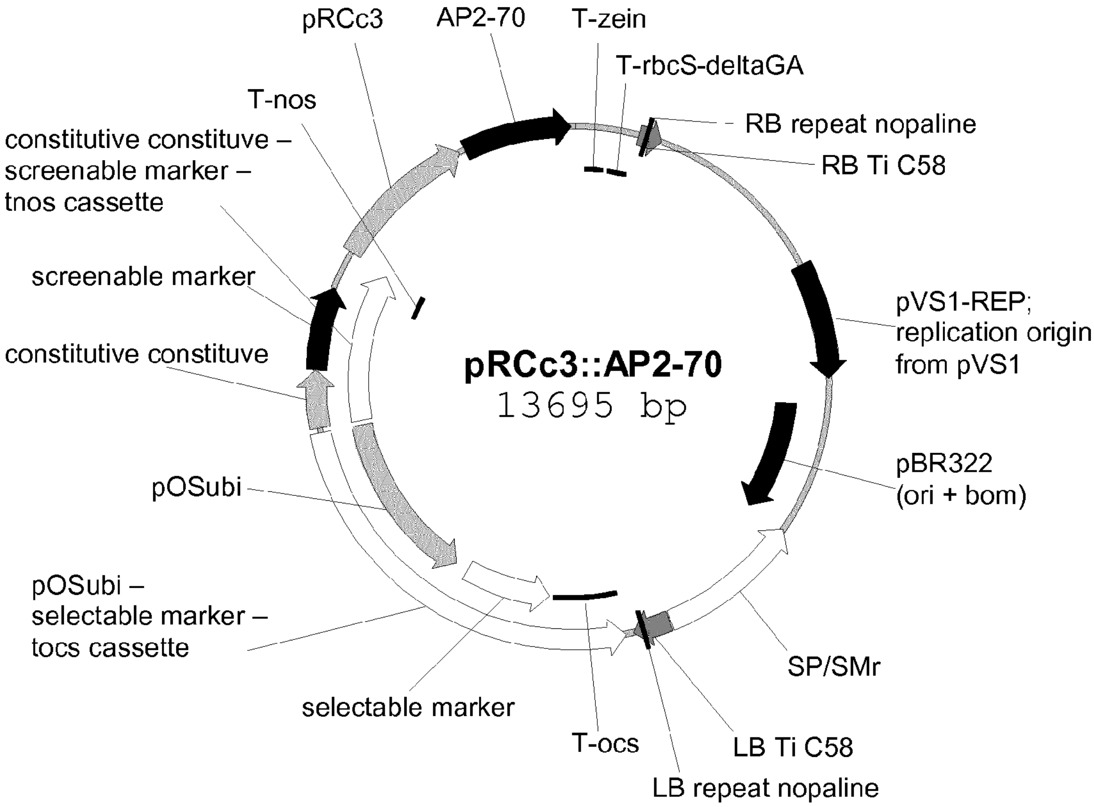
pRCc3
AP2-70
T-zein
T-rbcS-deltaGA
T-nos
RB repeat nopaline
constitutive constituve –
screenable marker –
tnos cassette
RB Ti C58
screenable marker
pVS1-REP;
replication origin
from pVS1
constitutive constituve
pRCc3::AP2-70
13695 bp
pBR322
(ori + bom)
pOSubi
pOSubi –
selectable marker –
tocs cassette
SP/SMr
selectable marker
T-ocs
LB Ti C58
LB repeat nopaline

FIGURE 24

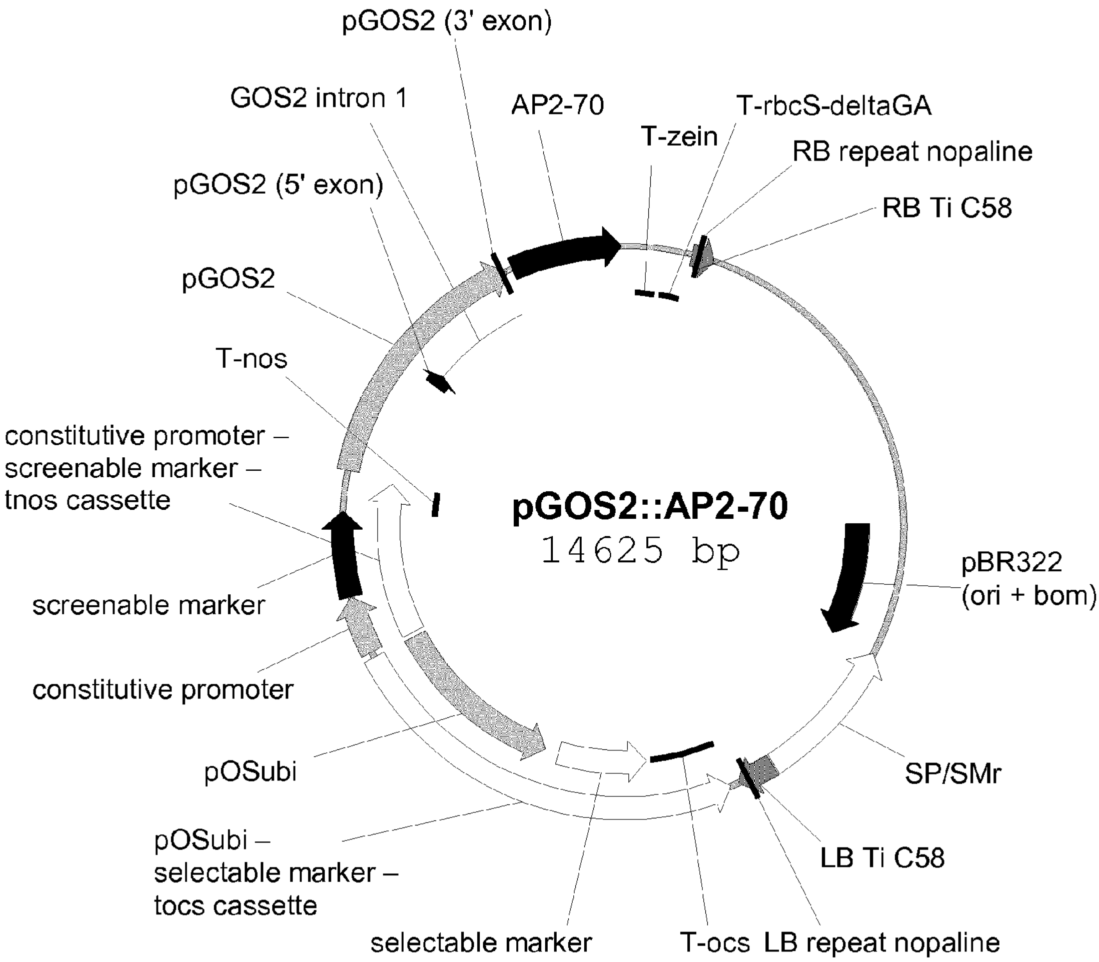
pGOS2 (3' exon)
GOS2 intron 1
AP2-70
T-rbcS-deltaGA
T-zein
RB repeat nopaline
pGOS2 (5' exon)
RB Ti C58
pGOS2
T-nos
constitutive promoter – screenable marker – tnos cassette
pGOS2::AP2-70
14625 bp
pBR322 (ori + bom)
screenable marker
constitutive promoter
pOSubi
SP/SMr
pOSubi – selectable marker – tocs cassette
LB Ti C58
selectable marker
T-ocs
LB repeat nopaline

FIGURE 25

## SEQ I NO: 1 - XM_479673.1| Oryza sativa (japonica cultivar-group) mRNA

ATCCAGATGCGCTTGCACTTGCACTTGCATGCAGTTCATTGCTCTAGCTATAGGCTATAGCTACTATA
CACTCATGAGAATCTGAGATTGGGCTGAAAATTGATGAGCATCGATCTGAAGAAGACATATATATAGC
TGATGATGATATAGCTTTTTCTGCATATGCTTGTTTTGATCTGCAGCCAATCGATCGAGGCATAGAGA
GTCACCAGGCAATCAAATTCCAACCATCCATCAGCTAGATATATATACAGCAAAGATCACGATACTCC
TAGCTAGCTAGCTAGAAGAATTGATCGGTCGATCGGAGGAAATAGCAATGGATCGGCATGAGGAGGAG
GCAGGAGAGTCTCCATGTGTGCCGCCGGGGTTCAGGTTTCACCCGACGGAGGAGGAGCTGGTGGGCTA
CTACCTCGCCAGGAAGGTGGCCTCCCAGAAGATCGATCTCGACATCATCCAGGAGCTCGATCTCTACA
GGATCGAGCCATGGGATCTGCAAGAGCGTTGCAAGTACGGTGGGCATGGCGGCGATGAGCAGACGGAG
TGGTACTTCTTCAGCTACAAGGACCGCAAGTACCCCAGCGGGACGAGGACCAACCGCGCCACGGCGGC
GGGCTTCTGGAAGGCCACCGGCCGCGACAAGCCGGTGCTCTCGTCGCCGTCGACGAGGGTGATCGGGA
TGAGGAAGACGCTGGTGTTCTACAAGGGTCGCGCCCCCAACGGCCGGAAGACCGACTGGATCATCCAC
GAGTACCGCCTCCAATCAAACGAACACGCCCCTACTCAGGAGGAAGGTTGGGTGGTTTGCCGCGCGTT
TCAGAAGCCGATGCCCAACCAGCAGCAGCACAGGCTGTCCTACGGCTGCATCCCCGGCAGCTACGGCG
CCGGAGCCTACGCCGCCGTCCCCGACAACTACAGCTTGCTGCTGCACCACGACAACCCTAGCTTCGCC
GGGCGGCCATTGATGAGCGCCGCTGCCTCAGCTCTCTTCGCCAACAACAACAATAACAGCGTCGTCGA
CCACAGCAACATTCTGAGTTCAGAGTCCAAGCTTCACTTCTCCGACATGATGCCGCCTCTGGAGAGCC
CCACCATCGTCGACGGCGAGGGCTACGTCTCGCAGGCTAGCAGCTGCGTCGACGTCGATCAGCAAGCC
GGCATCGTCGACTGGAACCTGCTCACCAGCTTGCTGCCGCCGCCGGCGCATCAGCTCTTCCACCACCT
GCCTTCCGCTTCTAGCTCCAAGAACAGCAACAACATTTCTTCGTCAGGCTTCATCGATGACCGAGACT
GATCGATCGATCCAGATCGATTATTATCAATTGACAATTCATTCATCATATATATGCATGAATTCTTA
CAAATACGCACAATTAAACGATCGAGTGTACTATTAATTAGTTGATTACTCCGTCTCCATGATGTATA
CATTAATTTGACCACTGGCCATCATCAGGCCATCTGCATGCTTACTATCTAGCTTATGTCAGTACACG
GTTGTTAGTTCATTCTTAATTAATTAGCTACTAGTGTCAGTGTGTGTATCTATCGGTTGTTCGTCTTT
TGACCTCATTGCTGAGAGAGGTTTGTAACTGATGATGATTAAGTTAGCTAGCATTTAATTAGGTGCTA
TATATACACATATATATGCACTATCACTATATACATCATATATATGTATACATATATCGTTGTCTGCT
TAATGTGTACACCACCTCTCTGCAGTATATATAGTATTGATCAATTAATTTGT

## SEQ ID NO: 2 - CDS4054 NAM2

MDRHEEEAGESPCVPPGFRFHPTEEELVGYYLARKVASQKIDLDIIQELDLYRIEPWDLQERCKYGGH
GGDEQTEWYFFSYKDRKYPSGTRTNRATAAGFWKATGRDKPVLSSPSTRVIGMRKTLVFYKGRAPNGR
KTDWIIHEYRLQSNEHAPTQEEGWVVCRAFQKPMPNQQQHRLSYGCIPGSYGAGAYAAVPDNYSLLLH
HDNPSFAGRPLMSAAASALFANNNNNSVVDHSNILSSESKLHFSDMMPPLESPTIVDGEGYVSQASSC
VDVDQQAGIVDWNLLTSLLPPPAHQLFHHLPSASSSKNSNNISSSGFIDDRD

FIGURE 26

## SEQ ID NO: 3 - AK106313.1| Oryza sativa (japonica cultivar-group)

AATTTGCTTCTCATCTTTCTTCTCTACTCCTCTCAAATACAAGAGCTCACACTGCTCCCTAGCTTCCT
TCTTCATCCCTTGAACTAGTTGTCAGCTACTGACCAGCCTGAAGATAGATGCTAGTTTGTGTAGAGAA
TTCGATCTTCTGAGGCGTATGGTGATCATGGAGTCATGTGTGCCTCCTGGGTTTAGGTTCCACCCCAC
CGACGAGGAGCTCGTCGGCTACTACCTCCGGAAGAAGGTGGCCTCGCAGAAGATCGACCTCGACGTCA
TCCGCGACGTCGACCTCTACCGCATCGAGCCATGGGATCTCCAAGAGCATTGCAGGATAGGGTACGAG
GAGCAGAGCGAGTGGTACTTCTTCAGCTACAAGGACAGGAAGTACCCGACGGGGACGAGGACGAACAG
GGCGACGATGACGGGGTTCTGGAAGGCGACGGGGAGGGACAAGGCGGTGCGTGAGAGGAGCAGGCTCA
TCGGGATGAGGAAGACGCTCGTCTTCTACAAGGGCAGGGCACCCAACGGCCACAAGACCGACTGGATT
GTCCACGAGTACCGCCTCGAGTCCGACGAGAACGCCCCGCCTCAGGAAGAAGGCTGGGTGGTGTGCCG
CGCGTTCAAGAAGCGAACCATGCAGCCGCCGCGGAGCTCCATCGGAGCATGGGAGGCGAGCTACTCCT
ACCACGACCCCGCCGTCTTCGTCGGCGGCGGGGAACACTTCAAGCAAGAGGCCGCGGCCGAGCTGGAC
GGCGTCGCCGCCGCCGCCGGAGCTAACGCCTTCCTGCGGTACTCCACCCGCCTGGCCGAGCTCCCGCA
GCTGGAGAGCCCGCCGCTGCCAAGCCAGGGGAGCCAGGCGGCCTCGGCCGTCGTCGACGGCGAGGAAG
ATAACGCCGATTCTAGCAGGCGCCCTGGCGGCGGCGGCGGCGCCGCCGCCGCGGTGACCACGGACTGG
AGGGCGTTCGACAAGTTCGTCGCGTCCCAGCTCAGCCCCGAGGAGCAGCACACCTGCCGGGCCACCGA
CGACGACGACATGGCAGCGCTGCTGCTCCTCGACGGCGGCGGGCAGGAGGACGACGCCGGGAGGTGGC
TGGGCTCCGCCGGGTTGCTGAGCGCCGTGGCGGCCGACGCGACGACGGACTGCGGCCTCGGCACCAGC
TGCGTGCCTGGCGACATCAACTGATTCAGGGCCAAGCCGATCGAGCTCTCACTTCTCCTTAGCATAAA
GTGTGAGATATCTACGAATTGTATGGATGGCTATATATACGTACGCCTATACATATGTAGCTGGTCAA
AGCATCGTTTCAGTTTCAGTTTCAGTTCTTGGAAGTCAATGGATGTTGACCATT

## SEQ ID NO: 4 - 11980.m06650 protein no apical meristem, AK106313

MVIMESCVPPGFRFHPTDEELVGYYLRKKVASQKIDLDVIRDVDLYRIEPWDLQEHCRIGYEEQSEWY
FFSYKDRKYPTGTRTNRATMTGFWKATGRDKAVRERSRLIGMRKTLVFYKGRAPNGHKTDWIVHEYRL
ESDENAPPQEEGWVVCRAFKKRTMQPPRSSIGAWEASYSYHDPAVFVGGGEHFKQEAAAELDGVAAAA
GANAFLRYSTRLAELPQLESPPLPSQGSQAASAVVDGEEDNADSSRRPGGGGAAAAVTTDWRAFDKF
VASQLSPEEQHTCRATDDDDMAALLLLDGGGQEDDAGRWLGSAGLLSAVAADATTDCGLGTSCVPGDI
N

## SEQ ID NO: 5 - NM_187584.1| Oryza sativa (japonica cultivar-group)

ATGGAATCATGCGTCCCTCCTGGGTTCAGGTTCCACCCCACCGACGAGGAGCTCGTCGGCTACTACCT
CCGCAAGAAGGTCGCCTCCCAGAAGATCGACCTCGACGTCATCCGCGACATCGATCTCTACCGCATCG
AACCCTGGGATCTCCAAGAACATTGTGGATCGGGTACGATGAGCAAAGCGAGTGGTACTTCTTCAGC
TACAAGGACAGGAAGTACCCGACGGGGACGAGGACGAACAGGGCGACAATGGCGGGGTTCTGGAAGGC
GACGGGGAGGGACAAGGCGGTGCACGACAAGAGCAGGCTCATCGGCATGAGGAAGACACTCGTCTTCT
ATAAGGGCAGGGCGCCTAATGGCCAGAAGACCGACTGGATCATGCACGAGTACCGCCTCGAGACCGAC
GAGAACGCGCCGCCACAGGAAGAAGG

FIGURE 26 (continued)

ATGGGTGGTGTGCCGAGCATTCAAGAAGAGGACGGCGTATCCGGCGAGGAGCATGGTGGAGACGTGGG
ACTACTCCTTGCACGAGCGCAACATCATGAGCGCCGCGGCGGCGGCGGCGTTCGCCGATCCGAGCGCG
GCGTACGCGCAGATGAGGCGGCAGCACAGGAGCGGGCGGTTCAAGCAGGAGGCGGAGCTGGACGGCGC
CGCCACAGCCCTCCTCCACTACTCCAGCCACCTCGCCGAGCTGCCGCAGCTCGAGAGCCCCTCCGCGG
CTGCGGCGCCGCTCCAGCCCAACCCGAGCCAGCTGGCCACCGCCGGCGAGGACGACGACTGCAAGGGC
GATAATGGCGGTAGGAGGGCCAAGAAAGCCCGCGCCGCCGGCGACAAGGTGGCGACGACCACGGACTG
GAGAGCGCTCGACAAGTTCGTCGCGTCGCAGCTTAGCCCCGGGGAGTGTGGCAGCATGGAGGCAACGG
CGGAAGCCGCGGCGGCGGCAGTCGCCGGTGTGAGCTCGCCGCTGGACCACGGCGATGACGACATGGCA
GCATTGCTGTTTCTCAACAGCGACGAGAGAGACGAGGTCGACAGGTGGACGGGGTTGCTCGGCTCCGG
CGCCGGCGCGAGCGGCGTCGACGGCGACCTCGGAATCTGTGTGTTTGACAAATGA

**SEQ ID NO: 6 - 11973.m05887 protein no apical meristem, NM_187584**

MESCVPPGFRFHPTDEELVGYYLRKKVASQKIDLDVIRDIDLYRIEPWDLQEHCGIGYDEQSEWYFFS
YKDRKYPTGTRTNRATMAGFWKATGRDKAVHDKSRLIGMRKTLVFYKGRAPNGQKTDWIMHEYRLETD
ENAPPQEEGWVVCRAFKKRTAYPARSMVETWDYSLHERNIMSAAAAAAFADPSAAYAQMRRQHRSGRF
KQEAELDGAATALLHYSSHLAELPQLESPSAAAAPLQPNPSQLATAGEDDDCKGDNGGRRAKKARAAG
DKVATTTDWRALDKFVASQLSPGECGSMEATAEAAAAAVAGVSSPLDHGDDDMAALLFLNSDERDEVD
RWTGLLGSGAGASGVDGDLGICVFDK

**SEQ ID NO: 7 - XM_467007.1| Oryza sativa (japonica cultivar-group), mRNA**

ATGGACACTTTCTCCCATGTGCCACCTGGTTTCCGCTTTCACCCTACTGACGAGGAGCTCGTCGATTA
CTACCTGAGGAAGAAGGTGGCATCGAAGAAAATCGACCTCGACGTTATAAAAGACGTCGACCTGTACA
AAATCGAGCCCTGGGATCTCCAAGAGAAGTGCAAGATTGGCATGGAAGAACAGAATGACTGGTACTTC
TTCAGCCACAAAGACAAAAAGTACCCGACGGGCACCCGCACCAACCGGGCCACGGGCGCCGGCTTCTG
GAAGGCGACGGGGAGGGACAAGCCCATCTACGCCCGCAGCTGCCTCGTCGGGATGAGGAAGACACTCG
TCTTCTACAAGGGCCGTGCCCCCAATGGCCAGAAGTCGGACTGGATCATGCACGAGTACCGCCTCGAG
ACCAACGAAAACGGCACCACACCTGAGGAAGGATGGGTGGTCTGCCGGGTGTTCAAGAAGCGGGTGGC
GACGGTGCGGAGAATGGCCGACGGATCACCGTGCTGGTTCGATGACCACGGCGCCGTCGGTGCGTTCA
TGCCGGACCTCAGCTCGCCGAGGCAGCTACTGCCGCACCACCACCACCACCACCCGGGCTCGTCGGCG
GCGCTGTACCACGGCCACCACCACCAGCAGCTGCAGCAGATGTACGGTCACTGCAAGCCGGAGCTGGA
GTACCACCACCTCCTGCCGCAGGAGGCCTTCCTGCAGCATCTTCCTCAGCTGGAGAGCCCCAAGCCGC
CGCCGCCGCCTCCTGCGGCGGCGGCCTACATTGGCGGCCACCTCGGATCGTCGTCGTCCACCGCCCTT
ACTACTCACGACGACGAAGCCTCCGGCTCCGCCGCGCAGCAGCAGCCGCCGTCGTTGGAGGCTGTTTA
CATGGCCGGCGCCGGCGTCGGCATCGGCGTCGACGCGTCGGTGACGGACTGGAGGCTACTGGACAAGT
TCGTCGCGTCTCAGCTGCTCAGCAAGGAGTCCATGTCCAGCTACGGATCCCATCCGGCTCAGGTGTTT
CAGGCTGCAGACGGTGGCAAGCATGAAGAGGCTTTGGACTACGCTTCGACGTCGGCCGGCTCCGGCGG
CGGCGAGGCTGATCTGTGGAAATAG

FIGURE 26 (continued)

**SEQ ID NO: 8 - 11972.m09332 protein No apical meristem protein XM_467007**

MDTFSHVPPGFRFHPTDEELVDYYLRKKVASKKIDLDVIKDVDLYKIEPWDLQEKCKIGMEEQNDWYF
FSHKDKKYPTGTRTNRATGAGFWKATGRDKPIYARSCLVGMRKTLVFYKGRAPNGQKSDWIMHEYRLE
TNENGTTPEEGWVVCRVFKKRVATVRRMADGSPCWFDDHGAVGAFMPDLSSPRQLLPHHHHHHPGSSA
ALYHGHHHQQLQQMYGHCKPELEYHHLLPQEAFLQHLPQLESPKPPPPPPAAAAYIGGHLGSSSSTAL
TTHDDEASGSAAQQQPPSLEAVYMAGAGVGIGVDASVTDWRLLDKFVASQLLSKESMSSYGSHPAQVF
QAADGGKHEEALDYASTSAGSGGGEADLWK

**SEQ ID NO: 9 - XM_473322.1 Oryza sativa (japonica cultivar-group), predicted mRNA**

ATGGAAGTGGAAGCAAGGATACACCTCCTGTTGATCAAGCTAGTCTTCTATAGGAGTGCAGAAGAGCG
AGAATCAGCCTCTGTTCATATTAACTTGCTACCAGTTCGGGAGCTATACTTTGATCCGCGGAAGAAGA
TGGACGCATTCTCCCATGTCCCGCCAGGTTTTCGTTTCCACCCTACTGACGAGGAACTCGTGGATTAC
TACCTTAGGAAGAAGGTAGCACTGAAGAAGATAGACTTGGACGTCATAAAAGATATTGATCTGTACAA
AATTGAGCCTTGGGACCTACAAGAACAATGCAAGATTGGAAACGAGGAGCAGAACGAGTGGTACTTCT
TCAGCCACAAGGACAAGAAGTACCCGACGGGCACACGCACCAACAGGGCGACCACTGCCGGCTTTTGG
AAGGCCACCGGGAGGGACAAGCCGATCTATGTCAAGAACTGCCTTGTTGGGATGAGGAAGACGTTGGT
TTTCTACAGGGGCCGGGCTCCCAACGGACAGAAGTCGGACTGGATCATGCACGAGTATCGCTTGGAGA
CCAACGAATACGGAGCTCCCCAAGAGGAAGGATGGGTGGTCTGCAGGGTGTTCAAGAAGCGAGTCGCG
GCGGTGCAAAGGGCGGCCGGCGACGGCGGCGACTCACCTTTCTGGTTCAACGAGCACGTCGCGTTCAT
GGCGCCGGCGCCAGGCCTCGACTCGCCGTACCATGGCCACCGCCAGAGCCACCCTTGCAAGCTGGAGG
TAGAGTATCACCACCACCTCCTTCCTCAGGAGGCGGCGCCGTTCATGCACCTCCCAAGGCTGGAGAGC
CCCAAGCTACCGGCCGCCGACATCATTGTCGCCACCGCAGCGTCGTCCGCTCTCCAGCCGTGCGGCCA
CACGACGGCGCAGCAGCTGCAGCTGCAGATCGAGCCGGTCTACGTGACGGCCGATGCGTCGGCAGCAG
ATTGGAGGGATCTTGACAAGCTGGTGGCGTCTCAGTTCGGCCACGGCGACTCGACTGCGAAGGAGCCC
AGTTACTGCAATCCGGTGCAGGTGTTTCAGGTCGAGGGGAAGCAGGAGGATAGCTTGGATTACGTGTC
CACGTCTGCCTCCTGCGGAGGGGAGGAGGATTTGTGGAAATAG

**SEQ ID NO: 10 - OSJNBb0020O11.1protein_id="XP_473322.1"**

MEVEARIHLLLIKLVFYRSAEERESASVHINLLPVRELYFDPRKKMDAFSHVPPGFRFHPTDEELVDY
YLRKKVALKKIDLDVIKDIDLYKIEPWDLQEQCKIGNEEQNEWYFFSHKDKKYPTGTRTNRATTAGFW
KATGRDKPIYVKNCLVGMRKTLVFYRGRAPNGQKSDWIMHEYRLETNEYGAPQEEGWVVCRVFKKRVA
AVQRAAGDGGDSPFWFNEHVAFMAPAPGLDSPYHGHRQSHPCKLEVEYHHHLLPQEAAPFMHLPRLES
PKLPAADIIVATAASSALQPCGHTTAQQLQLQIEPVYVTADASAADWRDLDKLVASQFGHGDSTAKEP
SYCNPVQVFQVEGKQEDSLDYVSTSASCGGEEDLWK

FIGURE 26 (continued)

**SEQ ID NO: 11 – Medicago truncatula ABE89364.1clone mth2-70o13**

ATGATGGAGTCATGTGTCCCACCTGGATTTCGGTTCCACCCAACGGATGGAGAGCTTGTTGGTTATTA
TCTAAGAAAGAAAGTAGCTTCTCACAAGATTGATCTTGATGTTATCAAAGAGATCGATCTATATCGTA
TTGAACCCTGGGATCTCCAAGAGAGATGTAGAATTGGGAATGAGGAGCAACATGAGTGGTATTTTTTT
AGTCACAAAGATAAGAAATATCCAACAGGGACGAGAACGAATAGAGCTACAATGGCAGGGTTCTGGAA
AGCAACCGGAAGAGATAAGTCGGTGTATGAACGAACAAAACTGATTGGAATGAGGAAGACACTCGTTT
TCTACAAAGGAAGAGCACCTAATGGACAGAAAACTGATTGGATCATGCATGAATATAGGCTTGAAACA
GTTGAAAATGCACCTCCACAGGCAAGTGAAGAAGGTTGGGTTGTGTGCAAAGCATTCAAGAAAAAAAC
AAGTGTCCAAGCAAAAACAAATGAAAGATGGGATCCAAGCCACTTACATGATGAACAAACAAGTAGCA
TCATCTCAAGGGTGGACCCTATTGACCTCATCTTAAGACAACCACAGAGGATTTCAGCTCAAAATTTC
ATGTACAAACAAGAGATAGAAGCAGAAGCAGATACCTTATTAAGGTTCATGCATTCAGAACAATTGGT
ACCTCTTCCTCAACTACAAAGTCCCTCTTTAACAAAGAGGCAAAACTCAATGCCAATAATATCAGAGA
AAGTGACTGATTGGAGGGATCTTGACAAGTTTGTGGCTTCTCAGTTGAGTCAAGAAGATCATAGGCAT
GAAACAAGCTCAGACATGTCATTGTTCCTACTTCAGAGTAGTAAGGATGATGAAGAGAACAAGTTAAG
CTCATTTTTAACTACAAGGTCAGATTGTGACATTGGAATATGTGTATTTGAAAATTAA

**SEQ ID NO: 12 - medicagoABE89364.1| No apical meristem (NAM) protein [Medicago truncatula]**

MMESCVPPGFRFHPTDGELVGYYLRKKVASHKIDLDVIKEIDLYRIEPWDLQERCRIGNEEQHEWYFF
SHKDKKYPTGTRTNRATMAGFWKATGRDKSVYERTKLIGMRKTLVFYKGRAPNGQKTDWIMHEYRLET
VENAPPQASEEGWVVCKAFKKKTSVQAKTNERWDPSHLHDEQTSSIISRVDPIDLILRQPQRISAQNF
MYKQEIEAEADTLLRFMHSEQLVPLPQLQSPSLTKRQNSMPIISEKVTDWRDLDKFVASQLSQEDHRH
ETSSDMSLFLLQSSKDDEENKLSSFLTTRSDCDIGICVFEN

**SEQ ID NO: 13 - NM_101098.2 Arabidopsis thaliana ANAC007/EMB2749; (ANAC007/EMB2749)**

ATGAATTCATTTTCCCACGTCCCTCCGGGTTTAGATTTCACCCGACAGATGAAGAACTTGTAGACTA
CTACCTGAGGAAAAAAGTCGCATCGAAGAGAATAGAAATTGATTTCATAAAGGACATTGATCTTTACA
AGATTGAGCCATGGGACCTTCAAGAGTTGTGCAAAATTGGGCATGAAGAGCAGAGTGATTGGTACTTC
TTTAGCCATAAAGACAAGAAGTATCCCACAGGGACTCGAACCAATAGAGCAACAAAAGCAGGGTTTTG
GAAAGCCACCGGAAGAGATAAGGCTATCTATTTGAGGCATAGTCTAATTGGCATGAGGAAAACACTTG
TGTTTTACAAGGGAAGAGCCCCAAATGGACAAAAGTCTGATTGGATCATGCACGAATACCGCTTAGAA
ACCGATGAAAACGGAACTCCTCAGGAAGAAGGATGGGTTGTGTGTAGGGTTTTCAAGAAGAGATTGGC
TGCAGTTAGACGAATGGGAGATTACGACTCATCCCCTTCACATTGGTACGATGATCAACTTTCTTTTA
TGGCCTCCGAGCTCGAGACAAACGGTCAACGACGGATTCTCCCCAATCATCATCAGCAGCAGCAGCAC
GAGCACCAACAACATATGCCATATGGCCTCAATGCATCTGCTTACGCTCTCAACAACCCTAACTTGCA
ATGCAAGCAAGAGCTAGAACTACACTACAACCACCTGGTACAACGAAATCATCTTCTTGATGAATCTC
ATTTATCGTTCCTCCAACTTCCTCAACTAGAAAGCCCTAAGATTCAACAAGATAACAGTAATTGCAAC
TCTCTTCCTTATGGAACAAGCAACATCGATAATAACTCGAGCCATAATGCTA

FIGURE 26 (continued)

ACTTGCAGCAATCAAATATCGCGCATGAGGAACAATTGAATCAAGGAAATCAGAACTTCAGCTCTCTA
TACATGAACAGCGGCAACGAGCAAGTGATGGACCAAGTCACAGACTGGAGAGTTCTCGATAAATTTGT
TGCTTCTCAGCTAAGCAACGAGGAGGCTGCCACAGCTTCTGCATCTATACAGAATAATGCCAAGGACA
CAAGCAATGCTGAGTACCAAGTTGATGAAGAAAAAGATCCGAAAAGGGCTTCAGACATGGGAGAAGAA
TATACTGCTTCTACTTCTTCGAGTTGTCAGATTGATCTATGGAAGTGAGCTGAAAGAGAAGACATATA
AATGCATATATACATATATATATATACGTACACACGAACACTAATCAAGTGTAGATGATGATGATGGT
ACAGATTTATATTTGCTTTGATTGATTCTTACTACATTATTGAACTTATGTCATATGCATATATACAT
TGCGTATCTATGCATATTTATACTTGTACTCAATATGATTAACCATATATAAACTCTAATCTAAATGT
AA

## SEQ ID NO: 14 - ANAC007/EMB2749" ="AT1G12260"NP_172690.1"

MNSFSHVPPGFRFHPTDEELVDYYLRKKVASKRIEIDFIKDIDLYKIEPWDLQELCKIGHEEQSDWYF
FSHKDKKYPTGTRTNRATKAGFWKATGRDKAIYLRHSLIGMRKTLVFYKGRAPNGQKSDWIMHEYRLE
TDENGTPQEEGWVVCRVFKKRLAAVRRMGDYDSSPSHWYDDQLSFMASELETNGQRRILPNHHQQQQH
EHQQHMPYGLNASAYALNNPNLQCKQELELHYNHLVQRNHLLDESHLSFLQLPQLESPKIQQDNSNCN
SLPYGTSNIDNNSSHNANLQQSNIAHEEQLNQGNQNFSSLYMNSGNEQVMDQVTDWRVLDKFVASQLS
NEEAATASASIQNNAKDTSNAEYQVDEEKDPKRASDMGEEYTASTSSSCQIDLWK

## SEQ ID NO: 15 - NM_104947.1| Arabidopsis thaliana ANAC026; transcription factor (ANAC026)

ATGAATTCGTTTTCACAAGTACCTCCTGGCTTCAGATTTCATCCTACTGATGAAGAACTTGTAGACTA
CTACTTGAGGAAAAAAGTTGCATCAAAGAGAATAGAAATCGATATCATCAAGGATGTTGATCTTTACA
AGATTGAGCCATGTGATCTTCAAGAGTTATGCAAGATAGGAAACGAAGAGCAGAGCGAATGGTACTTC
TTTAGTCATAAAGACAAGAAGTATCCCACGGGAACTCGAACCAATAGAGCCACGAAAGCAGGATTTTG
GAAAGCCACTGGAAGAGACAAGGCTATATATATAAGACATAGTCTTATCGGTATGAGGAAAACACTTG
TGTTTTACAAAGGAAGAGCCCCAAATGGTCAGAAATCCGATTGGATCATGCACGAATATCGCTTAGAA
ACAAGTGAAAATGGAACCCCTCAGGAAGAAGGATGGGTAGTATGTAGGGTATTCAAGAAGAAATTGGC
AGCGACAGTGAGGAAAATGGGAGATTACCATTCATCACCATCGCAGCATTGGTACGATGATCAGCTCT
CTTTTATGGCCTCCGAGATCATTTCTAGTTCTCCACGACAGTTTCTTCCCAATCATCATTATAACCGC
CACCATCACCAGCAGACATTGCCTTGTGGCCTCAATGCATTCAACAACAACAATCCTAACTTGCAATG
CAAGCAAGAGCTCGAGTTACATTACAATCAAATGGTACAACATCAACAACAAAACCATCATCTTCGTG
AATCTATGTTTCTCCAGCTTCCTCAGCTCGAAAGCCCTACCAGTAATTGCAATTCTGACAACAACAAT
AACACAAGAAATATTAGTAACTTGCAGAAATCATCAAATATATCTCATGAGGAACAATTGCAACAAGG
GAATCAAAGTTTCAGCTCTCTGTATTACGATCAAGGAGTAGAGCAAATGACTACTGACTGGAGAGTTC
TCGATAAATTTGTTGCTTCACAGCTTAGCAATGATGAAGAGGCTGCAGCCGTGGTTTCTTCTTCTTCT
CATCAAAACAACGTCAAGATTGACACGAGAAACACGGGTTATCATGTGATAGATGAGGGAATAAATTT
GCCGGAGAATGATTCTGAAAGGGTTGTTGAAATGGGAGAAGAGTATTCAAATGCTCATGCTGCTTCTA
CTTCTTCAAGTTGTCAGATTGATCTCTAG

FIGURE 26 (continued)

**SEQ ID NO: 16 - ANAC026"AT1G62700" ="NP_176457.1"**

MNSFSQVPPGFRFHPTDEELVDYYLRKKVASKRIEIDIIKDVDLYKIEPCDLQELCKIGNEEQSEWYF
FSHKDKKYPTGTRTNRATKAGFWKATGRDKAIYIRHSLIGMRKTLVFYKGRAPNGQKSDWIMHEYRLE
TSENGTPQEEGWVVCRVFKKKLAATVRKMGDYHSSPSQHWYDDQLSFMASEIISSSPRQFLPNHHYNR
HHHQQTLPCGLNAFNNNNPNLQCKQELELHYNQMVQHQQQNHHLRESMFLQLPQLESPTSNCNSDNNN
NTRNISNLQKSSNISHEEQLQQGNQSFSSLYYDQGVEQMTTDWRVLDKFVASQLSNDEEAAAVVSSSS
HQNNVKIDTRNTGYHVIDEGINLPENDSERVVEMGEEYSNAHAASTSSSCQIDL

**SEQ ID NO: 17 - XM_476289.1| Oryza sativa (japonica cultivar-group)**

ATGGATGGATCTAGTATGAGCAGCAGCAGCACGCAGCAGCAGGCGCAGGTTCCTCCTGGATTTCGTTT
CCACCCGACGGACGAAGAGCTGGTGGATTACTACCTTCGGAAGAAGGTGGCCGCAAGAAGGATTGATC
TCAATGTCATCAAGGACGTCGATCTCTACAAGATTGAGCCATGGGATCTGCAAGAGCGTTGCCGGATC
AATGGCGGGTCGGCGGCGGAGGAGCAGAACGAATGGTACTTCTTCAGCCACAAGGACAAGAAGTACCC
GACGGGGACGAGGACCAACCGTGCGACGGCGGCCGGGTTCTGGAAGGCGACGGGGCGAGACAAGCCCA
TCTACGCCACCAAGCAGCACAGCCTCCTCGTGGGCATGAGGAAGACGCTCGTCTACTACCGCGGCCGC
GCCCCCAACGGCCACAAGTCCGACTGGATCATGCACGAGTACCGCCTCGAGACCACCGAGACGGCCCC
GCCGCAGGAAGAAGGGTGGGTGGTATGCCGGGTGTTCAAGAAGAGATTACCCACAACAAGAAGAGATT
CAGACCATGACGCACCTTGCGGCAGCTGGTACGTTGATGAAGATGCACCGGGCGCCTTCATGTCTCCG
ATGATGATCACCAGATCATCAATATTGCGCCCACACCAACACCACGCCGGCATCACGCTGCAAGAGCA
ACACCTCCACACCACTTACAAGCACAGAGACCTCACTACTAAGATTCAGCAGCTCCAAGTCCCTGCGG
CAGGCCATCATCTCCTCAACACCATGCCCCATGACCTGGAGAGTTCTACTTCCTCCTTCCATTCTCTT
TTGGTCTCACCTGATCACCACCAAATCAACATGCACCATGCTCAGGCTGATCCCTTCTTTGACGACAT
GCATGCAGTCGATCAAGCCACTACCACTGACTGGAGAGTTCTTGACAAGTTTGTTGCCTCTCAGCTTA
GCAATGATGCTACAAACAAGCCTGCGGATCATTATACTGATGAAGGAGACATTCTTCAGGTCAGTGAC
AAGCAGCAAGAGGTGGCAGCCGCCGATTATGCGTCCACATCAACGTCAAGCAGCCAAATTGATCCATG
GAAGTGA

**SEQ ID NO: 18 - OSJNBa0075G19.15"NAM-like protein"="XP_476289.1"**

MDGSSMSSSSTQQQAQVPPGFRFHPTDEELVDYYLRKKVAARRIDLNVIKDVDLYKIEPWDLQERCRI
NGGSAAEEQNEWYFFSHKDKKYPTGTRTNRATAAGFWKATGRDKPIYATKQHSLLVGMRKTLVYYRGR
APNGHKSDWIMHEYRLETTETAPPQEEGWVVCRVFKKRLPTTRRDSDHDAPCGSWYVDEDAPGAFMSP
MMITRSSILRPHQHHAGITLQEQHLHTTYKHRDLTTKIQQLQVPAAGHHLLNTMPHDLESSTSSFHSL
LVSPDHHQINMHHAQADPFFDDMHAVDQATTDWRVLDKFVASQLSNDATNKPADHYTDEGDILQVSD
KQQEVAAADYASTSTSSSQIDPWK

FIGURE 26 (continued)

**SEQ ID NO: 19 - NM_127362.1| Arabidopsis thaliana ANAC037; transcription factor (ANAC037)**

ATGGAGCCAATGGAATCTTGTAGCGTTCCTCCAGGATTTAGGTTCCATCCGACGGACGAAGAGCTTGT
CGGGTACTATCTAAGGAAGAAAATCGCATCGCAAAAGATTGATCTCGACGTCATCAGAGACATCGATC
TCTACAGAATAGAACCATGGGATCTACAAGAACAATGTCGAATCGGTTATGAGGAACAAAATGAATGG
TATTTTTTTAGTCACAAGGACAAGAAATATCCAACGGGGACAAGAACTAATAGAGCGACCATGGCTGG
ATTTTGGAAAGCCACGGGAAGAGACAAAGCTGTTTACGACAAAACAAAACTAATTGGTATGAGGAAAA
CACTTGTGTTCTACAAAGGACGTGCACCTAATGGCAAGAAATCCGATTGGATCATGCATGAGTACCGG
CTCGAGTCAGATGAGAATGCACCGCCCCAGGAAGAAGGATGGGTGGTTTGTAGAGCATTCAAAAAAAG
AGCTACAGGGCAAGCCAAGAACACGGAAACTTGGAGCTCAAGTTACTTTTACGATGAAGTTGCACCGA
ATGGAGTTAACTCGGTTATGGACCCCATTGATTACATATCTAAGCAGCAACATAACATTTTTGGGAAA
GGTTTGATGTGTAAGCAAGAACTAGAAGGAATGGTTGATGGTATAAACTATATACAATCGAATCAATT
CATTCAGCTCCCACAACTCCAAAGCCCTTCTCTCCCGCTGATGAAAAGACCTTCAAGCTCGATGTCCA
TAACATCAATGGATAACAATTACAACTATAAACTCCCATTAGCGGATGAAGAAAGCTTCGAGTCATTC
ATAAGAGGAGAGGATAGAAGGAAGAAGAAAAAGCAAGTAATGATGACGGGAAATTGGAGAGAGTTAGA
CAAGTTTGTTGCTTCACAACTTATGAGCCAAGAAGACAATGGAACTTCAAGTTTCGCAGGTCATCATA
TAGTTAATGAAGATAAAAACAACAATGATGTGGAGATGGATTCGTCAATGTTTTTGAGCGAAAGAGAA
GAAGAAAACAGGTTCGTCAGTGAATTCTTGAGTACAAACTCGGATTATGATATTGGGATTTGCGTATT
TGATAATTGA

**SEQ ID NO: 20 - ANAC037"AT2G18060"NP_179397.1"**

MEPMESCSVPPGFRFHPTDEELVGYYLRKKIASQKIDLDVIRDIDLYRIEPWDLQEQCRIGYEEQNEW
YFFSHKDKKYPTGTRTNRATMAGFWKATGRDKAVYDKTKLIGMRKTLVFYKGRAPNGKKSDWIMHEYR
LESDENAPPQEEGWVVCRAFKKRATGQAKNTETWSSSYFYDEVAPNGVNSVMDPIDYISKQQHNIFGK
GLMCKQELEGMVDGINYIQSNQFIQLPQLQSPSLPLMKRPSSSMSITSMDNNYNYKLPLADEESFESF
IRGEDRRKKKKQVMMTGNWRELDKFVASQLMSQEDNGTSSFAGHHIVNEDKNNNDVEMDSSMFLSERE
EENRFVSEFLSTNSDYDIGICVFDN

**SEQ ID NO: 21 - NM_119783.2| Arabidopsis thaliana ANAC076; transcription factor (ANAC076)**

CTTTCTCTTCACATTCACCGAAACTTATAACCAAGATCAATATCAGTTCTCTCTCTCGATGAACATCA
TCTTCTACACAGCCATCTTTCTTTGACTTCTTCTTCTTCTTCTTAATATAACGGCTCGTTTCTTTTGT
TTCCAAACGAGTAAATAGTGTCCTATACACATATCTATAATTCCACAGGTTGAAGAAAAGAAATAATG
GAATCGGTGGATCAATCATGTAGTGTTCCTCCGGGATTCAGATTCCATCCAACAGATGAAGAGCTCGT
TGGTTACTATTTAAGGAAAAAAGTTGCATCGCAAAAGATCGATCTTGATGTCATAAGAGATATTGATC
TCTACAGAATCGAACCATGGGATTTACAAGAGAGCTGCCGAATCGGATATGAGGAAAGAAATGAATGG
TATTTCTTCAGCCACAAAGATAAGAAATATCCAACAGGAACAAGAACAAACAGAGCGACCATGGCTGG
GTTTTGGAAAGCCACGGGCCGAGACAAGGCTGTTTACGACAAGTCAAAACTGATTGGTATGAGAAAAA
CACTTGTGTTCTACAAAGGAAGAGCCCCTAATGGCCAAAAAACCGATTGGATCATGCATGAATACCGG
CTAGAGTCAGATGAGAATGCACCTCCTCAGGAAGAAGGGTGGGTGGTTTGTAGAGCGTTCAAGAAAAA
GCCAATGACCGGGCAAGCCAAGAACACAGAAACTTGGAGCTCAAGTTACTTTTACGACGAATTA

FIGURE 26 (continued)

CCGAGTGGAGTACGCTCAGTTACGGAGCCTCTTAACTACGTATCTAAGCAGAAACAAAACGTTTTTGC
ACAAGATTTAATGTTCAAGCAAGAACTAGAAGGATCAGATATCGGTTTAAACTTCATCCACTGCGATC
AATTCATTCAACTTCCGCAGCTTGAAAGCCCTTCACTCCCTCTTACCAAAAGACCAGTGAGCTTGACG
TCAATTACATCATTGGAGAAGAATAAAAATATCTACAAAAGACATTTAATAGAAGAGGATGTGAGCTT
CAATGCGCTAATAAGTAGTGGAAATAAAGATAAGAAGAAGAAGAAAACATCAGTGATGACGACGGATT
GGAGAGCACTCGATAAATTTGTTGCTTCTCAACTTATGAGCCAAGAAGATGGAGTTTCGGGTTTTGGA
GGTCATCATGAAGAAGATAACAATAAAATCGGTCATTACAATAACGAAGAGAGCAATAACAAGGGATC
AGTAGAGACTGCTTCTTCCACGTTATTGAGTGATAGAGAAGAAGAGAACAGATTCATCAGTGGATTAT
TGTGTTCCAACTTGGACTATGACTTATATAGGGATTTACATGTTTGATAAAAGATAACACTATAATAT
GGTGCGTAACGTTTGCTATATAGGTACGTAGAATTTATTGATACAGTATAACATATATAAAGATGTGT
GAAAGATTTGTTTTCTTCTACTATATATACTTTTTCGTGAA

**SEQ ID NO: 22 - ANAC076"AT4G36160"NP_195339.1"**

MESVDQSCSVPPGFRFHPTDEELVGYYLRKKVASQKIDLDVIRDIDLYRIEPWDLQESCRIGYEERNE
WYFFSHKDKKYPTGTRTNRATMAGFWKATGRDKAVYDKSKLIGMRKTLVFYKGRAPNGQKTDWIMHEY
RLESDENAPPQEEGWVVCRAFKKKPMTGQAKNTETWSSSYFYDELPSGVRSVTEPLNYVSKQKQNVFA
QDLMFKQELEGSDIGLNFIHCDQFIQLPQLESPSLPLTKRPVSLTSITSLEKNKNIYKRHLIEEDVSF
NALISSGNKDKKKKKTSVMTTDWRALDKFVASQLMSQEDGVSGFGGHHEEDNNKIGHYNNEESNNKGS
VETASSTLLSDREEENRFISGLLCSNLDYDLYRDLHV

**SEQ ID NO: 23 - NM_187584.1| Oryza sativa (japonica cultivar-group)**

ATGGAATCATGCGTCCCTCCTGGGTTCAGGTTCCACCCCACCGACGAGGAGCTCGTCGGCTACTACCT
CCGCAAGAAGGTCGCCTCCCAGAAGATCGACCTCGACGTCATCCGCGACATCGATCTCTACCGCATCG
AACCCTGGGATCTCCAAGAACATTGTGGGATCGGGTACGATGAGCAAAGCGAGTGGTACTTCTTCAGC
TACAAGGACAGGAAGTACCCGACGGGGACGAGGACGAACAGGGCGACAATGGCGGGGTTCTGGAAGGC
GACGGGGAGGGACAAGGCGGTGCACGACAAGAGCAGGCTCATCGGCATGAGGAAGACACTCGTCTTCT
ATAAGGGCAGGGCGCCTAATGGCCAGAAGACCGACTGGATCATGCACGAGTACCGCCTCGAGACCGAC
GAGAACGCGCCGCCACAGGAAGAAGGATGGGTGGTGTGCCGAGCATTCAAGAAGAGGACGGCGTATCC
GGCGAGGAGCATGGTGGAGACGTGGGACTACTCCTTGCACGAGCGCAACATCATGAGCGCCGCGGCGG
CGGCGGCGTTCGCCGATCCGAGCGCGGCGTACGCGCAGATGAGGCGGCAGCACAGGAGCGGGCGGTTC
AAGCAGGAGGCGGAGCTGGACGGCGCCGCCACAGCCCTCCTCCACTACTCCAGCCACCTCGCCGAGCT
GCCGCAGCTCGAGAGCCCCTCCGCGGCTGCGGCGCCGCTCCAGCCCAACCCGAGCCAGCTGGCCACCG
CCGGCGAGGACGACGACTGCAAGGGCGATAATGGCGGTAGGAGGGCCAAGAAAGCCCGCGCCGCCGGC
GACAAGGTGGCGACGACCACGGACTGGAGAGCGCTCGACAAGTTCGTCGCGTCGCAGCTTAGCCCCGG
GGAGTGTGGCAGCATGGAGGCAACGGCGGAAGCCGCGGCGGCGGCAGTCGCCGGTGTGAGCTCGCCGC
TGGACCACGGCGATGACGACATGGCAGCATTGCTGTTTCTCAACAGCGACGAGAGAGACGAGGTCGAC
AGGTGGACGGGGTTGCTCGGCTCCGGCGCCGGCGCGAGCGGCGTCGACGGCGACCTCGGAATCTGTGT
GTTTGACAAATGA

FIGURE 26 (continued)

**SEQ ID NO: 24 - OJ1015F07.11" ="NP_912473.1" NM_187584.1**

MESCVPPGFRFHPTDEELVGYYLRKKVASQKIDLDVIRDIDLYRIEPWDLQEHCGIGYDEQSEWYFFS
YKDRKYPTGTRTNRATMAGFWKATGRDKAVHDKSRLIGMRKTLVFYKGRAPNGQKTDWIMHEYRLETD
ENAPPQEEGWVVCRAFKKRTAYPARSMVETWDYSLHERNIMSAAAAAAFADPSAAYAQMRRQHRSGRF
KQEAELDGAATALLHYSSHLAELPQLESPSAAAAPLQPNPSQLATAGEDDDCKGDNGGRRAKKARAAG
DKVATTTDWRALDKFVASQLSPGECGSMEATAEAAAAAVAGVSSPLDHGDDDMAALLFLNSDERDEVD
RWTGLLGSGAGASGVDGDLGICVFDK

**SEQ ID NO: 25 - NM_125632.1| Arabidopsis thaliana ANAC101; transcription factor (ANAC101)**

ATGGAAAGTCTCGCACACATTCCTCCCGGTTATCGATTCCATCCGACCGATGAAGAACTCGTTGACTA
TTATCTCAAGAACAAAGTTGCATTCCCGGGAATGCAAGTTGATGTTATCAAAGATGTTGATCTCTACA
AAATCGAGCCATGGGACATCCAAGAGTTATGTGGAAGAGGGACAGGAGAAGAGAGGGAATGGTATTTC
TTTAGCCACAAGGACAAGAAATATCCAACTGGGACACGAACCAATAGAGCAACGGGCTCCGGATTTTG
GAAAGCAACGGGTCGAGACAAGGCCATTTACTCAAAGCAAGAGCTTGTTGGGATGAGGAAGACTCTTG
TCTTTTACAAAGGTAGGGCCCCAAATGGTCAGAAATCTGATTGGATAATGCACGAATACCGTCTTGAG
ACCGATGAAAATGGACCGCCTCATGAGGAAGGATGGGTGGTTTGTCGCGCTTTCAAGAAGAAGCTAAC
CACGATGAACTACAACAATCCAAGAACAATGATGGGATCATCATCAGGCCAAGAATCTAACTGGTTCA
CGCAGCAAATGGATGTGGGGAATGGTAATTACTATCATCTTCCTGATCTAGAGAGTCCGAGAATGTTT
CAAGGCTCATCATCATCATCACTATCATCATTACATCAGAATGATCAAGACCCTTATGGTGTCGTACT
CAGCACTATTAACGCAACCCCAACTACAATAATGCAACGAGATGATGGTCATGTGATTACCAATGATG
ATGATCATATGATCATGATGAACACAAGTACTGGTGATCATCATCAATCAGGATTACTAGTCAATGAT
GATCATAATGATCAAGTAATGGATTGGCAAACGCTTGACAAGTTTGTTGCTTCTCAGCTAATCATGAG
CCAAGAAGAGGAAGAAGTTAACAAAGATCCATCAGATAATTCTTCGAATGAAACATTTCATCATCTCT
CTGAAGAGCAAGCTGCAACAATGGTTTCGATGAATGCTTCTTCCTCTTCTTCTCCATGTTCCTTCTAC
TCTTGGGCTCAAAATACACACACGTAA

**SEQ ID NO: 26 - ANAC101"AT5G62380"id="NP_201044.1"**

MESLAHIPPGYRFHPTDEELVDYYLKNKVAFPGMQVDVIKDVDLYKIEPWDIQELCGRGTGEEREWYF
FSHKDKKYPTGTRTNRATGSGFWKATGRDKAIYSKQELVGMRKTLVFYKGRAPNGQKSDWIMHEYRLE
TDENGPPHEEGWVVCRAFKKKLTTMNYNNPRTMMGSSSGQESNWFTQQMDVGNGNYYHLPDLESPRMF
QGSSSSSLSSLHQNDQDPYGVVLSTINATPTTIMQRDDGHVITNDDDHMIMMNTSTGDHHQSGLLVND
DHNDQVMDWQTLDKFVASQLIMSQEEEEVNKDPSDNSSNETFHHLSEEQAATMVSMNASSSSSPCSFY
SWAQNTHT

FIGURE 26 (continued)

## SEQ ID NO: 27 - NM_126028.1| Arabidopsis thaliana ANAC105; transcription factor (ANAC105)

ATGATGAAGGTTGATCAAGATTATTCGTGTAGTATACCGCCTGGATTTAGGTTTCATCCGACAGATGA
AGAACTTGTCGGATATTATCTCAAGAAGAAAATCGCCTCCCAGAGGATTGATCTCGACGTTATCAGAG
AAATTGATCTTTACAAGATCGAACCATGGGATCTACAAGAGAGATGTAGGATAGGGTACGAGGAGCAA
ACGGAGTGGTATTTCTTCAGCCATAGAGACAAGAAGTATCCGACTGGGACTAGGACAAACCGAGCCAC
CGTGGCCGGTTTCTGGAAAGCAACGGGCCGGGACAAGGCGGTTTACCTCAACTCCAAACTTATCGGTA
TGAGAAAAACGCTTGTCTTTTACCGAGGTCGAGCGCCTAATGGCCAAAAGTCCGATTGGATCATTCAC
GAATACTACAGCCTCGAGTCACACCAGAACTCTCCTCCACAGGAAGAAGGATGGGTAGTGTGTAGAGC
ATTTAAGAAACGAACGACCATCCCAACAAAAAGGAGGCAACTTTGGGATCCGAACTGCTTATTCTACG
ACGACGCCACTCTCTTGGAACCTCTCGACAAGCGAGCCAGACATAATCCTGATTTTACCGCCACACCG
TTCAAGCAAGAACTACTCTCCGAGGCCAGTCACGTCCAGGATGGAGATTTCGGATCTATGTACCTTCA
ATGCATCGATGATGATCAATTCTCCCAGCTTCCTCAGCTCGAGAGCCCCTCTCTTCCGTCGGAAATAA
CTCCCCATAGTACTACTTTTTCTGAGAACAGTAGCCGGAAAGATGACATGAGCTCCGAGAAGAGGATC
ACTGACTGGAGATATCTAGATAAGTTCGTGGCGTCTCAATTTTTGATGAGTGGAGAAGACTAA

## SEQ ID NO: 28 - ANAC105"At5g66300"AAU94387.1"

MMKVDQDYSCSIPPGFRFHPTDEELVGYYLKKKIASQRIDLDVIREIDLYKIEPWDLQERCRIGYEEQ
TEWYFFSHRDKKYPTGTRTNRATVAGFWKATGRDKAVYLNSKLIGMRKTLVFYRGRAPNGQKSDWIIH
EYYSLESHQNSPPQEEGWVVCRAFKKRTTIPTKRRQLWDPNCLFYDDATLLEPLDKRARHNPDFTATP
FKQELLSEASHVQDGDFGSMYLQCIDDDQFSQLPQLESPSLPSEITPHSTTFSENSSRKDDMSSEKRI
TDWRYLDKFVASQFLMSGED

## SEQ ID NO: 29 - AK071064.1|:c927-1 Oryza sativa (japonica cultivar-group) cDNA clone

CCATCTTAATTTTTTACTTCTTTACTTAATTATCTGTCCTACTATATATGTTAATTCATGGATATTAA
TATGGAATATGGATGCAGGAGGAAGGTTGGGTGGTTTGCCGCGCGTTTCAGAAGCCGATGCCCAACCA
GCAGCAGCACAGGCTGTCCTACGGCTGCATCCCCGGCAGCTACGGCGCCGGAGCCTACGCCGCCGTCC
CCGACAACTACAGCTTGCTGCTGCACCACGACAACCCTAGCTTCGCCGGGCGGCCATTGATGAGCGCC
GCTGCCTCAGCTCTCTTCGCCAACAACAACAATAACAGCGTCGTCGACCACAGCAACATTCTGAGTTC
AGAGTCCAAGCTTCACTTCTCCGACATGATGCCGCCTCTGGAGAGCCCCACCATCGTCGACGGCGAGG
GCTACGTCTCGCAGGCTAGCAGCTGCGTCGACGTCGATCAGCAAGCCGGCATCGTCGACTGGAACCTG
CTCACCAGCTTGCTGCCGCCGCCGGCGCATCAGCTCTTCCACCACCTGCCTTCCGCTTCGGGAGTTAT
TACAAGTAGAGTGAGAGGAGACGGCGCGGCGGCGGCGGCGGCGGCGGGATGGAGAGGGAGAACCGGGT
GGAGACCATCTCCCGGCTGGCCCAGTGGCGCATCGACACCTTCGGCCCCTCCTCCTACCGCCGCTCCG
ATTCCTTCAAGATCGGCATCTGGAACTGGTTCCCTTCCCAATCCCAATCCCCCACTACTCAGAGATCA
CCGATTTGCCCTATCAAATCAAATCATCCGCCAATTCGATCCGAATCCGTGCAGGTACCTATCGGTGG
AGAAGGCTCGATATGTATACGTGGGGCTGTTCCCGGAGCCCGGGCGGGTGGCCAAGGAACGGCCCCCG
CTCGCCCGCTTCCTTCTCCGAGCTTGCTGGTCCGGCCCCAATG

FIGURE 26 (continued)

**SEQ ID NO: 30 – translation AK071064 sense**

MQEEGWVVCRAFQKPMPNQQQHRLSYGCIPGSYGAGAYAAVPDNYSLLLHHDNPSFAGRPLMSAAASA
LFANNNNNSVVDHSNILSSESKLHFSDMMPPLESPTIVDGEGYVSQASSCVDVDQQAGIVDWNLLTSL
LPPPAHQLFHHLPSASGVITSRVRGDGAAAAAAAGWRGRTGWRPSPGWPSGASTPSAPPPTAAPIPSR
SASGTGSLPNPNPPLLRDHRFALSNQIIRQFDPNPCRYLSVEKARYVYVGLFPEPGRVAKERPPLARF
LLRACWSGPN

**SEQ ID NO: 31 - gi|18409948|ref|NM_105851.1| Arabidopsis thaliana ANAC030; transcription factor (ANAC030) mRNA, complete cds**

ATCTTGTTTCTTACTCATTTCTCTAACAATTTTCCAAATTAAATACGTTTATAGGATCATCGTGGATG
GATAATATAATGCAATCGTCAATGCCACCGGGATTCCGATTTCATCCGACAGAGGAAGAGCTTGTGGG
TTATTACCTAGATAGGAAGATCAATTCAATGAAGAGTGCTTTAGATGTCATTGTAGAGATTGATCTCT
ACAAAATGGAGCCATGGGATATACAAGCGAGGTGTAAACTAGGGTATGAAGAGCAAAACGAGTGGTAC
TTCTTTAGTCATAAGGACAGGAAGTACCCTACCGGGACTAGGACCAACCGAGCCACTGCGGCTGGGTT
CTGGAAAGCCACGGGTAGAGACAAGGCGGTACTATCAAAAAACAGTGTCATCGGAATGCGGAAGACAC
TTGTCTACTACAAGGGTCGAGCTCCTAATGGAAGAAAGTCCGATTGGATCATGCACGAATACCGTCTC
CAAAACTCCGAGCTTGCCCCGGTTCAGGAGGAAGGCTGGGTGGTGTGTCGAGCATTTAGGAAGCCAAT
TCCAAACCAGAGGCCATTAGGGTACGAGCCATGGCAGAACCAGCTCTACCACGTCGAAAGTAGTAACA
ACTACTCATCTTCAGTGACAATGAACACGAGTCATCATATCGGTGCATCTTCATCAAGTCATAACCTT
AATCAAATGCTCATGAGCAATAACCACTACAATCCTAATAATACATCCTCATCGATGCATCAATATGG
CAACATTGAGCTCCCGCAGTTGGACAGCCCGAGCTTGTCGCCTAGTTTAGGGACGAATAAAGATCAGA
ACGAGAGTTTCGAGCAAGAAGAAGAGAAGAGCTTTAACTGTGTGGATTGGAGAACACTAGATACCTTG
CTTGAGACACAAGTCATACATCCGCATAACCCTAATATTCTTATGTTCGAAACGCAGTCGTATAATCC
GGCGCCAAGCTTCCCTTCCATGCATCAAAGCTATAATGAGGTCGAAGCTAATATTCATCATTCTCTTG
GATGCTTCCCTGACTCGTAATTAAAAAAAAACACACTTCTATATATTGATTTGTATTCTATGATTTAA
TTGTTCCATCTGGAAAATAACATTTATAACTGTCTATTTGTAAAAGAAGTTTGTGTTTATTACGTAGA
AATTTGTTGTTG

**SEQ ID NO: 32 - ANAC030"AT1G71930" ="NP_177338.1"**

MDNIMQSSMPPGFRFHPTEEELVGYYLDRKINSMKSALDVIVEIDLYKMEPWDIQARCKLGYEEQNEW
YFFSHKDRKYPTGTRTNRATAAGFWKATGRDKAVLSKNSVIGMRKTLVYYKGRAPNGRKSDWIMHEYR
LQNSELAPVQEEGWVVCRAFRKPIPNQRPLGYEPWQNQLYHVESSNNYSSSVTMNTSHHIGASSSSHN
LNQMLMSNNHYNPNNTSSSMHQYGNIELPQLDSPSLSPSLGTNKDQNESFEQEEEKSFNCVDWRTLDT
LLETQVIHPHNPNILMFETQSYNPAPSFPSMHQSYNEVEANIHHSLGCFPDS

FIGURE 26 (continued)

**SEQ ID NO: 33 - NM_197511.1| Oryza sativa hypothetical protein OSJNBa0053C23.15**

ATGCAGCCGCCGCGGAGCTCCATCGGAGCATGGGAGGCGAGCTACTCCTACCACGACCCCGCCGTCTT
CGTCGGCGGCGGGGAACACTTCAAGCAAGAGGCCGCGGCCGAGCTGGACGGCGTCGCCGCCGCCGCCG
GAGCTAACGCCTTCCTGCGGTACTCCACCCGCCTGGCCGAGCTCCCGCAGCTGGAGAGCCCGCCGCTG
CCAAGCCAGGGGAGCCAGGCGGCCTCGGCCGTCGTCGACGGCGAGGAAGATAACGCCGATTCTAGCAG
GCGCCCTGGCGGCGGCGGCGGCGCCGCCGCCGCGGTGACCACGGACTGGAGGGCGTTCGACAAGTTCG
TCGCGTCCCAGCTCAGCCCCGAGGAGCAGCACACCTGCCGGGCCACCGACGACGACGACATGGCAGCG
CTGCTGCTCCTCGACGGCGGCGGGCAGGAGGACGACGCCGGGAGGTGGCTGGGCTCCGCCGGGTTGCT
GAGCGCCGTGGCGGCCGACGCGACGACGGACTGCGGCCTCGGCACCAGCTGCGTGCCTGGCGACATCA
ACTGA

**SEQ ID NO: 34 - OSJNBa0053C23.15"NP_922493.1"NM_197511**

MQPPRSSIGAWEASYSYHDPAVFVGGGEHFKQEAAAELDGVAAAAGANAFLRYSTRLAELPQLESPPL
PSQGSQAASAVVDGEEDNADSSRRPGGGGAAAAVTTDWRAFDKFVASQLSPEEQHTCRATDDDDMAA
LLLLDGGGQEDDAGRWLGSAGLLSAVAADATTDCGLGTSCVPGDIN

**SEQ ID NO: 35 - AB217775.1 Zinnia elegans Ze567 mRNA for NAC-domain protein Ze567**

ATGGACACAATGAATCAATCATCATGTACTGTCCCTCCAGGTTTTCGTTTTCATCCTACCGATGAAGA
ACTTGTTGGTTACTATCTCAGAAAGAAGGTTGCATCTCAAAAGATTGATCTTGATGTCATTAAAGACA
TCGATCTTTATCGAATCGAACCATGGGATCTTATAGAGAGATGTCGGATCGGATACGAGGAGCAAAAT
GAGTGGTATTTCTTCAGTCACAAGGATAAAAAGTATCCAACAGGGACAAGGACAAATAGGGCAACTAT
GGCAGGTTTTTGGAAGGCAACGGGTAGAGACAAAGCTGTTTACGAAAAACTAAGGCTCATAGGAATGA
GAAAAACCCTAGTTTTCTACAAAGGAAGGGCACCAAATGGCCAGAAAACCGATTGGATCATGCACGAG
TACAGGCTCGAATCTGAAGAAAATGGACCTCCTCAGGAAGAAGGATGGGTAGTATGTCGAGCATTCAA
GAAACGTGCAACCGGACAAACAAGAACAACACCAGCATGGGAATCAAATTACTTCTTAGATGAATCAA
GCCTCCTTACATCCACAATGGATGATTACACCACAATTCAACCTTCAAATATTCCTTTGACATCAAGT
TTCATGTGCAAGCAAGAGTTGGAAGCATCAGAAAATTTGAACTTTATTCATTGTGATCAGTTTATTCA
ACTTCCACACCTCGAAAGCCCATCCCTCCAATCGATAAAACGGCCTATAAGCTCCATACTATATGAAC
ATGATCAACAAGAAGACGATCAAATCACAAAAAGAATCACAAACAACGTTAGGAGTAAAGATGAAGTG
AGGGATTGGAGGGATCTTGATAAGTTTGTAGCTTCTCAATTGAGCCAAGAGGAAGAGATTCGATGTGT
CGAAGAAGGGTTTTCAACGAATTTCCAAGAGAACATTGATCATTCTACTAGTTTGAGTCATATGTTTA
ATTATCAAGATGGTATTGAAGAATACAGTGGTTGTGACAAAGGGGGCAAGTTAAATGGATTCTTGAGT
TCAACAAGCTCAGATCATTTTGATGTTGGAATTTGCATATTTGATAAATTATGA

FIGURE 26 (continued)

**SEQ ID NO: 36 - Ze567"NAC-domain protein Ze567"BAE20090.1"**

MDTMNQSSCTVPPGFRFHPTDEELVGYYLRKKVASQKIDLDVIKDIDLYRIEPWDLIERCRIGYEEQN
EWYFFSHKDKKYPTGTRTNRATMAGFWKATGRDKAVYEKLRLIGMRKTLVFYKGRAPNGQKTDWIMHE
YRLESEENGPPQEEGWVVCRAFKKRATGQTRTTPAWESNYFLDESSLLTSTMDDYTTIQPSNIPLTSS
FMCKQELEASENLNFIHCDQFIQLPHLESPSLQSIKRPISSILYEHDQQEDDQITKRITNNVRSKDEV
RDWRDLDKFVASQLSQEEEIRCVEEGFSTNFQENIDHSTSLSHMFNYQDGIEEYSGCDKGGKLNGFLS
STSSDHFDVGICIFDKL

**SEQ ID NO: 37 - AJ833965.1 Zea mays partial mRNA for hypothetical(nac6 gene)**

ATGCATCCAGGTGTTGGACCATTGTCGGTGCCACCAGGCTTCCGCTTCCATCCGACTGATGAGGAGCT
CCTCTACTACTACCTGAGGAAAAAGGTTGCTTATGAGCCCATAGATCTTGATGTCATAAGGGAGATTG
ATCTCAATAAGCTTGAGCCCTGGGATCTCAAAGAAAGATGCAAAATAGGCACTGGGCCTCAAAACGAG
TGGTACTTCTTCAGCCACAAGGACAAGAAGTACTCAACGGGAACGAGAACGAACCGTGCCACGACGGC
GGGATTCTGGAAGGCGACTGGCCGAGACAAGGCCATCTTCCTTGGCAGCGCCAGGAGGATCGGCTTGA
GAAAGACCCTGGTGTTCTATATCGGCAGGGCGCCGCACGGGAAGAAGACTGAGTGGATCATGCACGAG
TACCGCCTTGATGAAGAGAACGTTGAAATTCAGGAAGATGGATGGGTGGTATGTAGGGTTTTCAAGAA
GAAGAACTATGAGCAGAGAGGCCACAACATGGCTGAGATGGCGGCACTGGACGACGATGAGCTCCAGC
CTTTCACGGTTCCTGTCGTCCCTGCTGGCACTAGCTCCCTGCCAACAACAGACCACAAGAACAACCCT
CACCTCATGCAATATGACTTCCCTTCTTTCGACCCTTCCATGCAGCTCCCACAGCTGATGAGCGCCGA
CCAGCCCGTGCCAACCCTATTCCCCAGCCATCCTGGCGTCGCCACGGCCATGAGCTCATCAATCGACG
TTGAGTGCTCGCAGAACCTGCTGACGATGCTGACATCCAACGGCAGCGACGGGATGCTCCACGTCCGC
GCTGGTGGTGCTGGAGCTGGTGTCGACCGCTTCGCTGGCACGACAGATTGGTCGATCCTAGACAAGCT
CCTCGCCTCGCACCAGAACCTCGGACAGCTCTTCCACGGCAAGGTCACCGCAGCATCTGCCTCCCCAA
TGGCTCCATACCATCAGCAGCTCATGGAACTCGGGTGGCTCGTCGTCGTCGTCGTCCTTGCAGAGGCT
TCCACTGCAGTACCTCAGCGGCGAGACGACCGATCTCCTCAGGTTCCCCAAGTAATTATTGGATCGAC
CTCAAGTTTAGCTACCTTGTTGAGCAGTGACCATATCAGGATTTTGGTTGACTACTAGGCTGCTTAAT
TCGGCCTACTTCGTATGGAGTTTAGTGCTTTTAAATGTATATATGGTGCAACTGTTTGGGGCATGCAG
GCATGGAATGCCATCGATCTACCATGGTTGCTTGCTTGGCCAAAAAAAAAAAAAAAAAA

**SEQ ID NO: 38 – Maize nac6CAH56056.1**

MHPGVGPLSVPPGFRFHPTDEELLYYYLRKKVAYEPIDLDVIREIDLNKLEPWDLKERCKIGTGPQNE
WYFFSHKDKKYSTGTRTNRATTAGFWKATGRDKAIFLGSARRIGLRKTLVFYIGRAPHGKKTEWIMHE
YRLDEENVEIQEDGWVVCRVFKKKNYEQRGHNMAEMAALDDDELQPFTVPVVPAGTSSLPTTDHKNNP
HLMQYDFPSFDPSMQLPQLMSADQPVPTLFPSHPGVATAMSSSIDVECSQNLLTMLTSNGSDGMLHVR
AGGAGAGVDRFAGTTDWSILDKLLASHQNLGQLFHGKVTAASASPMAPYHQQLMELGWLVVVVVLAEA
STAVPQRRDDRSPQVPQVIIGSTSSLATLLSSDHIRILVDY

FIGURE 26 (continued)

## SEQ ID NO: 39 – gos2 promoter

AATCCGAAAAGTTTCTGCACCGTTTTCACCCCCTAACTAACAATATAGGGAACGTGTGCTAAATATAA
AATGAGACCTTATATATGTAGCGCTGATAACTAGAACTATGCAAGAAAAACTCATCCACCTACTTTAG
TGGCAATCGGGCTAAATAAAAAAGAGTCGCTACACTAGTTTCGTTTTCCTTAGTAATTAAGTGGGAAA
ATGAAATCATTATTGCTTAGAATATACGTTCACATCTCTGTCATGAAGTTAAATTATTCGAGGTAGCC
ATAATTGTCATCAAACTCTTCTTGAATAAAAAAATCTTTCTAGCTGAACTCAATGGGTAAAGAGAGAG
ATTTTTTTTAAAAAAATAGAATGAAGATATTCTGAACGTATTGGCAAAGATTTAAACATATAATTATA
TAATTTTATAGTTTGTGCATTCGTCATATCGCACATCATTAAGGACATGTCTTACTCCATCCCAATTT
TTATTTAGTAATTAAAGACAATTGACTTATTTTTATTATTTATCTTTTTTCGATTAGATGCAAGGTAC
TTACGCACACACTTTGTGCTCATGTGCATGTGTGAGTGCACCTCCTCAATACACGTTCAACTAGCAAC
ACATCTCTAATATCACTCGCCTATTTAATACATTTAGGTAGCAATATCTGAATTCAAGCACTCCACCA
TCACCAGACCACTTTTAATAATATCTAAAATACAAAAAATAATTTTACAGAATAGCATGAAAAGTATG
AAACGAACTATTTAGGTTTTTCACATACAAAAAAAAAAAAGAATTTTGCTCGTGCGCGAGCGCCAATCT
CCCATATTGGGCACACAGGCAACAACAGAGTGGCTGCCCACAGAACAACCCACAAAAAACGATGATCT
AACGGAGGACAGCAAGTCCGCAACAACCTTTTAACAGCAGGCTTTGCGGCCAGGAGAGAGGAGGAGAG
GCAAAGAAAACCAAGCATCCTCCTCCTCCCATCTATAAATTCCTCCCCCCTTTTCCCCTCTCTATATA
GGAGGCATCCAAGCCAAGAAGAGGGAGAGCACCAAGGACACGCGACTAGCAGAAGCCGAGCGACCGCC
TTCTTCGATCCATATCTTCCGGTCGAGTTCTTGGTCGATCTCTTCCCTCCTCCACCTCCTCCTCACAG
GGTATGTGCCCTTCGGTTGTTCTTGGATTTATTGTTCTAGGTTGTGTAGTACGGGCGTTGATGTTAGG
AAAGGGGATCTGTATCTGTGATGATTCCTGTTCTTGGATTTGGGATAGAGGGGTTCTTGATGTTGCAT
GTTATCGGTTCGGTTTGATTAGTAGTATGGTTTTCAATCGTCTGGAGAGCTCTATGGAAATGAAATGG
TTTAGGGTACGGAATCTTGCGATTTTGTGAGTACCTTTTGTTTGAGGTAAAATCAGAGCACCGGTGAT
TTTGCTTGGTGTAATAAAAGTACGGTTGTTTGGTCCTCGATTCTGGTAGTGATGCTTCTCGATTTGAC
GAAGCTATCCTTTGTTTATTCCCTATTGAACAAAAATAATCCAACTTTGAAGACGGTCCCGTTGATGA
GATTGAATGATTGATTCTTAAGCCTGTCCAAAATTTCGCAGCTGGCTTGTTTAGATACAGTAGTCCCC
ATCACGAAATTCATGGAAACAGTTATAATCCTCAGGAACAGGGGATTCCCTGTTCTTCCGATTTGCTT
TAGTCCCAGAATTTTTTTTCCCAAATATCTTAAAAAGTCACTTTCTGGTTCAGTTCAATGAATTGATT
GCTACAAATAATGCTTTTATAGCGTTATCCTAGCTGTAGTTCAGTTAATAGGTAATACCCCTATAGTT
TAGTCAGGAGAAGAACTTATCCGATTTCTGATCTCCATTTTTAATTATATGAAATGAACTGTAGCATA
AGCAGTATTCATTTGGATTATTTTTTTTATTAGCTCTCACCCCTTCATTATTCTGAGCTGAAAGTCTG
GCATGAACTGTCCTCAATTTTGTTTTCAAATTCACATCGATTATCTATGCATTATCCTCTTGTATCTA
CCTGTAGAAGTTTCTTTTTGGTTATTCCTTGACTGCTTGATTACAGAAAGAAATTTATGAAGCTGTAA
TCGGGATAGTTATACTGCTTGTTCTTATGATTCATTTCCTTTGTGCAGTTCTTGGTGTAGCTTGCCAC
TTTCACCAGCAAAGTTC

## SEQ ID NO: 40 – protochlorophyllide reductase promoter

TTGCAGTTGTGACCAAGTAAGCTGAGCATGCCCTTAACTTCACCTAGAAAAAAGTATACTTGGCTTAA
CTGCTAGTAAGACATTTCAGAACTGAGACTGGTGTACGCATTTCATGCAAGCCATTACCACTTTACCT
GACATTTTGGACAGAGATTAGAAATAGTTTCGTACTACCTGCAAGTTGCAACTTGAAAAGTGAAATTT
GTTCCTTGCTAATATATTGGCGTGTAATTCTTTTATGCGTTAGCGTAAAAAGTTGAAATTTGGGTCAA
GTTACTGGTCAGATTAACCAGTAACTGGTTAAAGTTGA

FIGURE 26 (continued)

AAGATGGTCTTTTAGTAATGGAGGGAGTACTACACTATCCTCAGCTGATTTAAATCTTATTCCGTCGG
TGGTGATTTCGTCAATCTCCCAACTTAGTTTTTCAATATATTCATAGGATAGAGTGTGCATATGTGTG
TTTATAGGGATGAGTCTACGCGCCTTATGAACACCTACTTTTGTACTGTATTTGTCAATGAAAAGAAA
ATCTTACCAATGCTGCGATGCTGACACCAAGAAGAGGCGATGAAAAGTGCAACGGATATCGTGCCACG
TCGGTTGCCAAGTCAGCACAGACCCAATGGGCCTTTCCTACGTGTCTCGGCCACAGCCAGTCGTTTAC
CGCACGTTCACATGGGCACGAACTCGCGTCATCTTCCCACGCAAAACGACAGATCTGCCCTATCTGGT
CCCACCCATCAGTGGCCCACACCTCCCATGCTGCATTATTTGCGACTCCCATCCCGTCCTCCACGCCC
AAACACCGCACACGGGTCGCGATAGCCACGACCCAATCACACAACGCCACGTCACCATATGTTACGGG
CAGCCATGCGCAGAAGATCCCGCGACGTCGCTGTCCCCCGTGTCGGTTACGAAAAAATATCCCACCAC
GTGTCGCTTTCACAGGACAATATCTCGAAGGAAAAAAATCGTAGCGGAAAATCCGAGGCACGAGCTGC
GATTGGCTGGGAGGCGTCCAGCGTGGTGGGGGGCCCACCCCCTTATCCTTAGCCCGTGGCGCTCCTCG
CTCCTCGGGTCCGTGTATAAATACCCTCCGGAACTCACTCTTGCTGGTCACCAACACGAAGCAAAAGG
ACACCAGAAACATAGTACACTTGAGCTCACTCCAAACTCAAACACTCACACCA

**SEQ ID NO: 41 – Motif I**

K/P/R/G I/S/M D/A/E/Q L/I/V D I/V/F I Q/V/R/K E/D L/I/V D.

**SEQ ID NO: 42 – Motif II**

C K/R Y/L/I G XXX G/Y/N D/E E Q/R T/N/S EW

where 'X' is any amino acid or a gap.

**SEQ ID NO: 43 – Motif III**

GWVVCR A/V F $X^1$ K $X^2$

where '$X^1$' and '$X^2$' may be any amino acid, preferably $X^1$ is Q/R/K, preferably $X^2$ is P/R/K.

**SEQ ID NO: 44 – adapter primer**

AAGCAGTGGTATCAACGCAGAGTACGCGGG

**SEQ ID NO: 45 – primer: OsNAM2-2**

CTCTCCAGAGGCGGCATCATGTCGGA

**SEQ ID NO: 46 – primer: OsNAM2-1**

TGATCGGGATGAGGAAGA

FIGURE 26 (continued)

**SEQ ID NO: 47 – primer: OsNAM2-3**

GATCAGTCTCGGTCATCGATG

**SEQ ID NO: 48 – primer: prm08733**

GGGGACAAGTTTGTACAAAAAAGCAGGCTTAAACAATGGATCGGCATGAGGAG

**SEQ ID NO: 49 – primer: prm08734**

GGGGACCACTTTGTACAAGAAAGCTGGGTTCGATCGATCAGTCTCGGT

FIGURE 26 (continued)

**SEQ ID NO: 50 - CDS4035 - XM_470088.1 - Oryza sativa**

TCACAACCCACAACATTTTCAAACAACGCAAAGCAGTAGCAGCAGCGAGAAGCAAGCAAGAAGCGATG
GGGATGGGGATGAGGAGGGAGAGGGACGCGGAGGCGGAGCTGAACCTGCCGCCGGGGTTCAGGTTCCA
CCCCACGGACGACGAGCTGGTGGAGCACTACCTGTGCAGGAAGGCGGCGGGGCAGCGCCTGCCGGTGC
CGATCATCGCCGAGGTGGATCTCTACAAGTTCGACCCGTGGGATCTGCCCGAGCGCGCGCTGTTCGGC
GCCAGGGAGTGGTACTTCTTCACCCCGCGGGATCGCAAGTATCCTAATGGGTCACGCCCCAACCGCGC
CGCCGGCAACGGGTACTGGAAGGCCACCGGCGCCGACAAGCCCGTCGCGCCGCGGGGGCGCACGCTTG
GGATCAAGAAGGCGCTCGTGTTCTACGCCGGCAAGGCGCCGCGAGGGGTCAAGACTGATTGGATCATG
CATGAGTACCGGCTCGCCGATGCTGGCCGCGCCGCCGCGGGCGCCAAGAAGGGATCTCTCAGGTTGGA
TGATTGGGTGCTGTGTCGGCTGTACAACAAGAAGAACGAGTGGGAGAAGATGCAGCAGGGGAAGGAGG
TGAAGGAGGAGGCGTCCGACATGGTTACGTCGCAGTCGCACTCGCACACCCACTCGTGGGGCGAGACG
CGCACGCCGGAGTCGGAGATCGTGGACAACGACCCCTTCCCGGAGCTGGACTCGTTCCCGGCGTTCCA
GCCTGCGCCGCCGCCGGCGACGGCGATGATGGTGCCCAAGAAAGAATCGATGGACGACGCCACCGCGG
CCGCCGCCGCCGCCGCCACCATCCCCAGGAACAACAGCAGCCTGTTCGTGGACCTGAGCTACGACGAT
ATCCAGGGCATGTACAGCGGCCTCGACATGCTGCCGCCGGGCGACGACTTCTACTCGTCGCTCTTCGC
GTCGCCGCGGGTGAAGGGGACGACGCCACGCGCCGGCGCCGGCATGGGCATGGTCCCGTTCTGA

**SEQ ID NO: 51 - CDS4035 - XP_470088.1 - Oryza sativa**

MGMGMRRERDAEAELNLPPGFRFHPTDDELVEHYLCRKAAGQRLPVPIIAEVDLYKFDPWDLPERALF
GAREWYFFTPRDRKYPNGSRPNRAAGNGYWKATGADKPVAPRGRTLGIKKALVFYAGKAPRGVKTDWI
MHEYRLADAGRAAAGAKKGSLRLDDWVLCRLYNKKNEWEKMQQGKEVKEEASDMVTSQSHSHTHSWGE
TRTPESEIVDNDPFPELDSFPAFQPAPPPATAMMVPKKESMDDATAAAAAAATIPRNNSSLFVDLSYD
DIQGMYSGLDMLPPGDDFYSSLFASPRVKGTTPRAGAGMGMVPF

**SEQ ID NO: 52 - AB028185.1 - Oryza sativa**

TCGACCCACGCGTCCGCTCTTCCCAACACTAGTAGGATAAAGCCACAGAGAGAGCAGTAGTAGTAGCG
AGCTCGCCGGAGAACGGACGATCACCGGAGAAGGGGGAGAGAGATGAGCGGCGGTCAGGACCTGCAGC
TGCCGCCGGGGTTCCGGTTCCACCCGACGGACGAGGAGCTGGTGATGCACTACCTCTGCCGCCGCTGC
GCCGGCCTCCCCATCGCCGTCCCCATCATCGCCGAGATCGACCTCTACAAGTTCGATCCATGGCAGCT
TCCCCGGATGGCGCTGTACGGAGAGAAGGAGTGGTACTTCTTCTCCCCGCGAGACCGCAAGTACCCGA
ACGGGTCGCGGCCGAACCGCGCCGCCGGGTCGGGGTACTGGAAGGCGACCGGCGCCGACAAGCCGGTG
GGCTCGCCGAAGCCGGTGGCGATCAAGAAGGCCCTCGTCTTCTACGCCGGCAAGGCGCCCAAGGGCGA
GAAGACCAACTGGATCATGCACGAGTACCGCCTCGCCGACGTCGACCGCTCCGCCCGCAAGAAGAACA
GCCTCAGGTTGGATGATTGGGTGCTGTGCCGGATTTACAACAAGAAGGGCGGGCTGGAGAAGCCGCCG
GCCGCGGCGGTGGCGGCGGCGGGGATGGTGAGCAGCGGCGGCGGCGTCCAGAGGAAGCCGATGGTGGG
GGTGAACGCGGCGGTGAGCTCCCCGCCGGAGCAGAAGCCGGTGGTGGCGGGGCCGGCGTTCCCGGACC
TGGCGGCGTACTACGACCGGCCGTCGGACTCGATGCCGCGGCTGCACGCCGACTCGAGCTGCTCGGAG
CAGGTGCTGTCGCCGGAGTTCGCGTGCGAGGTGCAGAGCCAGCCCAAGATCA

FIGURE 27

GCGAGTGGGAGCGCACCTTCGCCACCGTCGGGCCCATCAACCCCGCCGCCTCCATCCTCGAC
CCCGCCGGCTCCGGCGGCCTCGGCGGCCTCGGCGGCGGCGGCAGCGACCCCCTCCTCCAGGACATCCT
CATGTACTGGGGCAAGCCATTCTAGACGACCAAAAAAAAAAAAAAAACAACCGCATTGGCAGCAATGGT
GTCACTGAACACCGTGCAGGCTAGCTAGCTTCATGGCCGGTGAACTTTGACTCAGGCGAGCCGCCGGA
GTTGACTCAAAGATAATTAAAAGAAGTGTTTTAAGTGGATTGGATTGGATTAGACAGAGGAGATGAGG
ACTCGAGAAAGGCGGCGATGAGACCGTGGTTGGGGGGACCCTGGCCTGGACTGAACGACGACGAGGCA
GCAGCAGAAAGATGGTGCAATTGCATCGGGTGGCATGTCAGTGTGTGTGTATAGTGGCATGTACATAG
TACATGGTGATTGATTCGGTATACAGGGGGCTAGCTTTCCTGTTTCTGTTTAAAAAAAAAAAAAAAAA
AAAAAAAAAAAAAAAAA

**SEQ ID NO: 53 - BAA89800.1 - Oryza sativa**

MSGGQDLQLPPGFRFHPTDEELVMHYLCRRCAGLPIAVPIIAEIDLYKFDPWQLPRMALYGEKEWYFF
SPRDRKYPNGSRPNRAAGSGYWKATGADKPVGSPKPVAIKKALVFYAGKAPKGEKTNWIMHEYRLADV
DRSARKKNSLRLDDWVLCRIYNKKGGLEKPPAAAVAAAGMVSSGGGVQRKPMVGVNAAVSSPPEQKPV
VAGPAFPDLAAYYDRPSDSMPRLHADSSCSEQVLSPEFACEVQSQPKISEWERTFATVGPINPAASIL
DPAGSGGLGGLGGGGSDPLLQDILMYWGKPF

**SEQ ID NO: 54 - AB028184.1 - Oryza sativa**

CCCCTCGAGGTCGACCCACGCGTCCGCTTCCATTAGCGACTAATCCAGCCTTCGTTAAGGGTGATTAG
AATTTGATTAATTTCAAGGCCTCAGATCGGAGATTGAATGGAGTGCGGTGGTGCGCTGCAGCTGCCGC
CGGGGTTCAGGTTCCACCCGACGGACGACGAGCTGGTGATGTACTACCTGTGCCGCAAGTGCGGCGGC
CTCCCCCTCGCCGCCCCGGTGATCGCCGAGGTGGACCTCTACAAGTTCAACCCATGGGATCTCCCCGA
GAGGGCGATGGGAGGGGAGAAGGAGTGGTACTTCTTCTCGCCGCGGGACCGGAAGTACCCGAACGGGC
AGCGGCCGAACAGGGCGGCGGGGACGGGGTACTGGAAGGCGACGGGGGCGGACAAGCCGGTGGGGTCG
CCGCGGGCGGTGGCGATCAAGAAGGCGCTCGTCTTCTACGCCGGGAAGCCGCCCAAGGGCGTCAAGAC
CAACTGGATCATGCACGAGTACCGCCTCGCCGACGTCGACCGCTCCGCCGCCGCCCGCAAGCTCTCCA
AGTCCTCCCACAACGCCCTCAGGTTGGATGATTGGGTGTTGTGCCGAATCTACAACAAGAAGGGAGTG
ATCGAGAGGTACGACACGGTGGACGCCGGCGAGGACGTGAAGCCGGCGGCGGCGGCGGCGGCGGCGAA
GGGTGGTCGCATCGGCGGCGGCGGCGGCGCGGCGGCGATGAAGGTGGAGCTCTCCGACTATGGGTTCT
ACGACCAGGAGCCGGAGTCGGAGATGCTGTGCTTCGACCGGTCGGGGTCGGCGGACCGCGACTCGATG
CCGCGGCTGCACACCGACTCCAGCGGGTCGGAGCACGTGCTGTCGCCGTCGCCGTCGCCGGACGACTT
CCCCGGCGGCGGCGACCACGACTACGCCGAGAGCCAGCCCAGCGGCGGATGCGGCGGGTGGCCCGGCG
TCGACTGGGCCGCCGTCGGCGACGATGGCTTCGTCATCGACAGCTCCCTCTTCGAGCTGCCCTCGCCG
GCGGCGTTCTCCCGCGCCGCCGGCGACGGCGCCGCCTTCGGCGACATGTTCACGTACCTGCAGAAGCC
GTTCTAATTAACGGAACGTGACGCCTCTGCAAACGCTAATTAGCTCACTAACCACTGTCAGGTCGATC
GTGTAATTCTTTTACCAGTCTAGGGTACAGCCAAAAAACCAAAAATTAACCGTGCTCGATCGATCGAT
CGATCCATGGATCGATGATCGCCTCCACCGGCAGATCAAAATCGAAGCAAAAAATCAGGGAGAATTCT
TGGTACTTCCAGTACCTCTCGTCGACACCACGTGTCGGGTTTTCTGCACCGCGGATCCTCGCTGGCCG
CATCGTAACGGCGAGCGAGGTTAAAAATAGTGGAGAATTCAAGAACACATCCCTGATTTTTGTTTCTG
CCGGATGGGGAGTACCGTGAGCATGGTAGAAATGACTGGTAAAATTTTCGTTGATGATAG

FIGURE 27 (continued)

GTAGCCAGAGTCGATGTAACCAGTCGATTTTTTTTTTTTTTGGTTTTAGTTGACATGCCTTCCTGATA
GATTCAGAACAAACAATAGAGAAGAGAGGAGAAAAAAACACAGAGGAGAAATTCAGAGACCAAAGTTC
AAAGTTTTGGAATTTTCAGGGGCGCCAGCAGCTGATGGTATTGTCAGTCGTGTCTAGGTTCGTAGAGG
AAATTAAAATGTAGAAGAACGGGTACTTCAGTTCACTTCAAAAAAAAAAAAAAAAAAAA

**SEQ ID NO: 55 - BAA89799.1 - Oryza sativa**

MECGGALQLPPGFRFHPTDDELVMYYLCRKCGGLPLAAPVIAEVDLYKFNPWDLPERAMGGEKEWYFF
SPRDRKYPNGQRPNRAAGTGYWKATGADKPVGSPRAVAIKKALVFYAGKPPKGVKTNWIMHEYRLADV
DRSAAARKLSKSSHNALRLDDWVLCRIYNKKGVIERYDTVDAGEDVKPAAAAAAAKGGRIGGGGGAAA
MKVELSDYGFYDQEPESEMLCFDRSGSADRDSMPRLHTDSSGSEHVLSPSPSPDDFPGGGDHDYAESQ
PSGGCGGWPGVDWAAVGDDGFVIDSSLFELPSPAAFSRAAGDGAAFGDMFTYLQKPF

**SEQ ID NO: 56 - AK107330.1 - Oryza sativa**

ATCCTCCACAAGAGAAACTAGGATCTTCAAGTATAGCTAGCATTGCGCCCTCATTTCATCCATGGCAA
TGCAGCTGTCCTTGCCTGTCTTGCCAACGGGTTTTCGTTTCCATCCAACCGATGAGGAACTAGTCATC
AACTACCTCCAGAGGCGTGCTACCGGGCTATCGTGCCCCATCCCCATAATCGCGGATGTCGAAATATA
CAACTTCAACCCGTGGGAGCTTCCATCCATGGCTCTATTTGGGGAACATGAGTGGTACTTTTTTACAC
TACGCGACCACAGGTACCCCAATAGTGTGCGCCCTAGTCGCTCAGCGGCGTCAGGCTTTTGGAAGGCC
ACCGGCACTGACAAGCCCGTCCAAGTTGCTAACATGCAAAGCACTCCTGTAGCTATGAAGAAGGCACT
TGTATTCTATGTTGGCCGCCCGCCCATGGAAACCAAGACTACATGGATCATGCATGAGTACCGTCTCA
CAAACACGGGAGGGTCCACTGCCTCCCACCCATCCTTGTCTTCATCCACCGCACACCCTTCTGTGAAG
CTAGACGAGTGGGTGCTATGCAAGATCTTCAACAAGTCCCCAGAGCCGGACAACACCGCACCACCATC
AAACGTCGTCTCACGGTTACAGTGCTCGCCGCCGCTGCCGCCGCCGGCGGCACCGCCCGGCAACTACC
CGCCGCTGCCGGTGGGTGCGACGAACGACGGCGGCGTGTTCGCCTGCGCCGGCGACATGCTCTTCACC
ATCCAAGAGCACCAGGAGGGAACACCATCGATGCTGCCGCCGATCCCTAACCTTGAGCCACCGGCGGC
AACAATTGGGAATTCCTCTCTCAATGGCACTGCTGCTGCTGCTGCTGCTGCTGATGGCCATGGCCGCC
TTGAGGAAGAGGACACCAGCGCCTACACCTTCACCGACCAGGAAATGGAGCAGATGCTCATGGACCTG
ATGGACCAGGATTTCTTTGGCAATGATCAACCCCAGGAGTGAACTGTGTGGTGGAGTGATTTTCATAT
CATTCCATAGCTCTGATCTGTGCTGGAGAGTGAATTTAGTATCTATTGTACCAGATAGAATAAAACCA
GAAAAGAAATAAAAGAAGGCCAAAGAAAAAAGCAAAAAGAAAATTGTATAATTACTAGGTTTGGATGC
CGTCTGTAGAACCTTGTGTGCTATGTAACTTTTTTCTTTGTATTTTGGCATTTTCATCATATTGTGTC
ATAAATAAAGCAGGGCTTTCTTTTGTAGTTTGCCATCTCAATCTTGCACCGTGATGTTATAAAAAAAC
AAAATTAATTATTGTGCTACACTGTACTTTTGGCTGTGTTCGGC

**SEQ ID NO: 57 - AK107330.1 - Oryza sativa**

MAMQLSLPVLPTGFRFHPTDEELVINYLQRRATGLSCPIPIIADVEIYNFNPWELPSMALFGEHEWYF
FTLRDHRYPNSVRPSRSAASGFWKATGTDKPVQVANMQSTPVAMKKALVFYVGRPPMETKTTWIMHEY
RLTNTGGSTASHPSLSSSTAHPSVKLDEWVLCKIFNKSPEPDNTAPPSNVVSRLQCSPPLPPPAAPPG
NYPPLPVGATNDGGVFACAGDMLFTIQEHQEGTPSMLPPIPNLEPPAATIGNSSLNGTAAAAAAADGH
GRLEEEDTSAYTFTDQEMEQMLMDLMDQDFFGNDQPQE

FIGURE 27 (continued)

**SEQ ID NO: 58 - AK069257.1 – Oryza sativa**

atgccgagcagcggcggcgccatgcctgcccttccaccaggcttccgcttccaccccaccgacgagga
gctcatcgttcactacctcatgaaccaggccgcctccgtcaagtgccccgtgccaatcatcgccgagg
tcaacatctacaagtgcaacccatgggaccttcctggtaaggctttgttcggcgagaacgaatggtac
ttcttcagcccgagggaccgcaagtaccccaacggcgctcgccccaaccgcgccgccggctcggggta
ctggaaggccaccggcaccgacaagtccatcctctccactccgaccagcgacaacatcggcgtcaaga
aggccctcgtcttctacaagggcaagcctcccaagggcgtcaagaccgactggatcatgcacgagtac
cgtctcaccggcacatcagctaacagcaccaccaccacaaagcagcgtagagcgtcatccatgaccat
gaggctggacgactgggtgctgtgcagaatccacaagaagagcaacgacttcaattcctctgaccaac
acgaccaagaacccgaggaatcaaccgtcgaacagcttgaagacatccatgacaacaactcctctgaa
caacctccagctccagctgacatgaacaaccaacagtcagatttccagcccatgacggcgatgagcat
gagcaagtcatgctccctcaccgatctcctcaacaccatcgactgcgccgcgctctcgcagtttctcc
tcgacggctcatccgacgccatcgctgagcctcctgctcctcccagcccccctaatatacacaacacct
catccaaattaccaaacactaaactataacattaacagcaacagcagcatgccacacgccttcgagtc
acgcctagatcatcacgatggttacgttaacaattataatgttaatggcctgaggaggaagagaatga
tggcgtgtagtgcaacttcctttgatgatggcagcagcagcaatgactttgtgcatgccgttgtcaag
aaaccgcagctgctgccaagtgattcgaggggtagtggttttggaggaggttactgcaaccagcagct
ttcagagactgcgactggctttcagtttcagaacggcaatctgctgagccatccatttcctctgaaca
atcatctgcagatgcagtag

**SEQ ID NO: 59 - AK069257.1 – Oryza sativa**

MPSSGGAMPALPPGFRFHPTDEELIVHYLMNQAASVKCPVPIIAEVNIYKCNPWDLPGKALFGENEWY
FFSPRDRKYPNGARPNRAAGSGYWKATGTDKSILSTPTSDNIGVKKALVFYKGKPPKGVKTDWIMHEY
RLTGTSANSTTTTKQRRASSMTMRLDDWVLCRIHKKSNDFNSSDQHDQEPEESTVEQLEDIHDNNSSE
QPPAPADMNNQQSDFQPMTAMSMSKSCSLTDLLNTIDCAALSQFLLDGSSDAIAEPPAPPSPLIYTTP
HPNYQTLNYNINSNSSMPHAFESRLDHHDGYVNNYNVNGLRRKRMMACSATSFDDGSSSNDFVHAVVK
KPQLLPSDSRGSGFGGGYCNQQLSETATGFQFQNGNLLSHPFPLNNHLQMQ

**SEQ ID NO: 60 - AY625683.1 - Triticum aestivum**

ACGAGGCACTCGCCCCAATCTCGAACAACGGCCGTCGGATCCATCATCATCGGCAGCGGAGCGATTCC
ATCGACCAGCTTTCTACAGACGGACATGGGGATGCCGGCCGTGAGGAGGAGGGAGAGGGACGCGGAGG
CGGAGCTCAACCTGCCCCCCGGCTTCCGCTTCCACCCCACCGACGACGAGCTCGTCGAGCACTACCTC
TGCCGCAAGGCGGCGGGGCAGCGCCTCCCGGTCCCCATCATCGCCGAGGTCGACCTCTACCGCTTCGA
CCCCTGGGCGCTCCCTGACCGCGCCCTCTTCGGCACCCGCGAGTGGTACTTCTTCACCCCCCGCGACC
GCAAGTACCCCAACGGCTCCCGACCCAACCGCGCCGCCGGCAACGGCTACTGGAAGGCCACCGGCGCC
GACAAGCCCGTCGCGCCCCGCGGCGGGCGGACCATGGGGATCAAGAAGGCCCTCGTGTTTTACGCGGG
CAAGGCGCCTAAGGGGGTCAAGACCGACTGGATCATGCATGAGTATCGCCTCGCCGACGCCGGCCGCG
CCGCCGCCAGCAAGAAGGGCTCGCTCAGGCTGGACGACTGGGTGCTCTGCCGGCTCTACAACAAGAAG
AACGAGTGGGAGAAGATGCAGCTGCAGCAGCAAGGGGAAGAGGAGACGATGATGGAGCCCAAGGCGGA
GA

FIGURE 27 (continued)

ACACGGCCTCCGACATGGTGGTCACCTCGCACTCCCACTCGCAGTCCCAGTCGCACTCGCACTCGTGG
GGCGAGGCGCGCACGCCGGAGTCGGAGATCGTCGACAACGACCCGTCGCTGTTCCAGCAGGCGACGGC
GGCGGCGTTCCAGGCCCAGAGCCCCGCGGCCGCCGCGGCGCACCAGGAGATGATGGCCACGCTGATGG
TGCCCAAGAAGGAGGCGGCGGACGAGGCCGGCAGGAACGACCTCTTCGTCGACCTCAGCTACGACGAC
ATCCAGAGCATGTACAACGGCCTCGACATGATGCCGCCCGGGGACGACCTGCTCTACTCCTCCCTCTT
CGCCTCCCCCCGTGTCCGCGGGAGCCAGCCCGGCGCCGGCGGCATGCCGGCCCCGTTCTAAGCAGAGC
AAAGACAGAGACCGTCAGAGACCCCGAGATCGACAGAAGTGGAATGGACGACTTCTTGGCCGCGAGCG
AGCGAGACCGCGGTGCAGTGTAAATACAGCATAGGAAACGGGAGGGAGGGAGGTGGCGTCGTGTCGAG
AGAAGCCGGCGTCGTGCCGGGGCGTGCCGTTGTACATGGAGAGCCCGGCGCCGGGGCCTGGCCGGCTG
GGTTGATTCTTTTGTTCTTACTACCTTGTACTCTCAATGCGGATGTAGCTCTTTCTTCTCACTCCTCA
CTAGTAGAATCGACCGACCGAATACATATGTAGCTCTGTTCTTTCCTTCTCTTCAGTAGAAATCAACC
AAATTTTGCTGTTATGCTGC

**SEQ ID NO: 61 - AAU08786.1 - Triticum aestivum**

MGMPAVRRRERDAEAELNLPPGFRFHPTDDELVEHYLCRKAAGQRLPVPIIAEVDLYRFDPWALPDRA
LFGTREWYFFTPRDRKYPNGSRPNRAAGNGYWKATGADKPVAPRGGRTMGIKKALVFYAGKAPKGVKT
DWIMHEYRLADAGRAAASKKGSLRLDDWVLCRLYNKKNEWEKMQLQQQGEEETMMEPKAENTASDMVV
TSHSHSQSQSHSHSWGEARTPESEIVDNDPSLFQQATAAAFQAQSPAAAAAHQEMMATLMVPKKEAAD
EAGRNDLFVDLSYDDIQSMYNGLDMMPPGDDLLYSSLFASPRVRGSQPGAGGMPAPF

**SEQ ID NO: 62 - AY103772.1 - Zea mays**

GCACGAGGAACCCGCCCACAGGACAGGACACACAGAGCCGAGCCACCCCACCCACCATCGGCGACCAG
CAGCCAGCCGCGGGAGCGAGCTGTTTAAAGACACCGAGTCGGAGTCGGACGGAGGACTGGCAGGCACA
ACCGAAGCCACCGCTTCTAGTTCTCGGGTTCATCGCCAGCAATCCAGACCACATAATGGGACAGCCGG
TGACGAGGAGGAGGGAGAGGGACGCGGAGGCGGAGCTGGACCTGCCGCCGGGGTTCCGGTTCCACCCC
ACAGACGACGAGCTGGTGGAGCACTACCTGTGCCGCAAGGCGGCGGGGCAGCGCCTCCCCGTGCCCAT
CATCGCCGAGGTGGACCTGTACAGGTTCGACCCGTGGGACCTGCCGGAGCGCGCGCTCTTCGGGGCCC
GGGAGTGGTACTTCTTCACGCCCAGGGACCGCAAGTACCCCAACGGCTCCCGCCCCAACCGCGCCGCC
GGCGACGGGTACTGGAAGGCCACCGGCGCCGACAAGCCCGTCGCGCCGCGCGCCGCCGCCGCCGACGC
CCGCACGCTCGGGATCAAGAAGGCGCTCGTCTTCTACGCCGGCAAGGCGCCGCGCGGGGTCAAGACAG
ACTGGATCATGCACGAGTACAGGCTCGCTGACGCCGGCGGACGCGCCAAGAAGGGGTCGCTCAGGTTG
GATGACTGGGTGCTGTGCCGGCTGTACAACAAGAAGAACGAGTGGGAGAAGATGCGGCTGGGGAAGGG
GGCAGCCGCCGGCGCAGTCAAAGAGGAGGAGGCCATGGACATGAGCACCTCCCACTCGCAGATCACCC
ACTCGTGGGGCGAGACGCGCACGCCGGAGTCGGAGATCGTGGACAACGACCTGCCGTTCCCGGATCCG
GCGATGATGGTGCCCAAGAAGGAGCGGGTGGACGACGGCGGCAGCGCCAGGACCAGCGACCTGTTCGT
GGATCTCAGCTACGACGACATCCAAGGCATGTACAGCGGCCTCGACATGCTGCCGCCGGCCGGCGAGG
ACTTGTACTCCTCGCTCTTCGCGTCGCCCAGGGTCAGGGGGAACCAGCCCACCGGACCCGCGGNGTTG
GGCCCCTTCTGAGTGAGCTCAGGCGAAGATGGAGGCATGGAGATCAGGAGAGA

FIGURE 27 (continued)

**SEQ ID NO: 63 - AY103772.1 - Zea mays - translation**

MGQPVTRRRERDAEAELDLPPGFRFHPTDDELVEHYLCRKAAGQRLPVPIIAEVDLYRFDPWDLPERA
LFGAREWYFFTPRDRKYPNGSRPNRAAGDGYWKATGADKPVAPRAAAADARTLGIKKALVFYAGKAPR
GVKTDWIMHEYRLADAGGRAKKGSLRLDDWVLCRLYNKKNEWEKMRLGKGAAAGAVKEEEAMDMSTSH
SQITHSWGETRTPESEIVDNDLPFPDPAMMVPKKERVDDGGSARTSDLFVDLSYDDIQGMYSGLDMLP
PAGEDLYSSLFASPRVRGNQPTGPAXLGPF

**SEQ ID NO: 64 - AJ010829.1 - Triticum aestivum**

AATTCGGCACGAGACAGTCCACCACGCACGTGCAGCAGCACCAGCGCCCGAGAATCCCATTCCCATCG
ACGGAGAAGAAGAAGTGAAGAAACAATGGTGATGGCAGCGGCGGAGCGGCGGGACGCGGAGGCGGAGC
TGAACCTGCCGCCGGGGTTCCGGTTCCACCCGACGGACGAGGAGCTGGTGGCGGACTACCTCTGCGCG
CGCGCGGCCGGCCGCGCGCCGCCGGTGCCCATCATCGCCGAGCTCGACCTCTACCGGTTCGACCCGTG
GGAGCTCCCGGAGCGGGCGCTCTTCGGGGCGCGGGAGTGGTACTTCTTCACGCCGCGGGACCGCAAGT
ACCCCAACGGCTCCCGCCCCAACCGGGCCGCCGGGGGCGGCTACTGGAAGGCCACCGGCGCCGACAGG
CCCGTGGCGCGCGCGGGCAGGACCGTCGGGATCAAGAAGGCGCTCGTCTTCTACCACGGCAGGCCGTC
GGCGGGGGTCAAGACGGACTGGATCATGCACGAGTACCGCCTCGCCGGCGCCGACGGACGCGCCGCCA
AGAACGGCGGCACGCTCAGGCTTGACGAATGGGTGCTCTGCCGCCTATACAACAAGAAGAACCAGTGG
GAGAAGATGCAGCGGCAGCGGCAGGAGGAGGAGGCGGCGGCCAAGGCTGCGGCGTCACAGTCGGTCTC
CTGGGGTGAGACGCGGACGCCGGAGTCCGACGTCGACAACGATCCGTTCCCGGAGCTGGACTCGCTGC
CGGAGTTCCAGACGGCAAACGCGTCAATACTGCCCAAGGAGGAGGTGCAGGAGCTGGGCAACGACGAC
TGGCTCATGGGGATCAGCCTCGACGACCTGCAGGGCCCCGGCTCCCTGATGCTGCCCTGGGACGACTC
CTACGCCGCCTCGTTCCTGTCGCCGGTGGCCACGATGAAGATGGAGCAGGACGTCAGCCCATTCTTCT
TCTGAGCTCTCAATACTCTCACGGTCGCACTGTTGTGTGCGGCGTAACTGTAGATAGTTCACATTTGT
TCAGGATTTATTTGTAACGTTGCTTCTTTTATACGATACTCTCTTCCTTTCTAAAAAAAAAAAAAAAA
AA

**SEQ ID NO: 65 - CAA09371.1 - Triticum aestivum**

MVMAAAERRDAEAELNLPPGFRFHPTDEELVADYLCARAAGRAPPVPIIAELDLYRFDPWELPERALF
GAREWYFFTPRDRKYPNGSRPNRAAGGGYWKATGADRPVARAGRTVGIKKALVFYHGRPSAGVKTDWI
MHEYRLAGADGRAAKNGGTLRLDEWVLCRLYNKKNQWEKMQRQRQEEEAAAKAAASQSVSWGETRTPE
SDVDNDPFPELDSLPEFQTANASILPKEEVQELGNDDWLMGISLDDLQGPGSLMLPWDDSYAASFLSP
VATMKMEQDVSPFFF

**SEQ ID NO: 66 - NM_186659.2 - Oryza sativa**

CATCCAACAACCCACCACCCTCATTCCCTCAAGTCCCAAGATCGAACACCTCGTGTCCATGGCGGCGG
CGAAGCGGCGAGTGCGCGACGCGGAGGCGGACCTGAACCTCCCGCCGGGCTTCCGCTTCCACCCCACC
GACGAGGAGCTGGTGGCGCACTACCTCTGCCCGCGCGCCGCGGGCCGCGCCGCCCCGGTCCCCATCAT
CGCCGAGCTCGACCTCTACCGCCACGACCCATGGGACCTCCCCCACCGCGCCCTCTTCGGCCGCCGCG
AGTGGTACTTCTTCACCCCGCGCGACCGCAAGTACCCCAACGGCTCCCGCCCCAACCGCGCCGCCGCC
TCGGGCTACTGGAAGGCCACCGGCGCCGACAA

FIGURE 27 (continued)

GCCCGTGCTGCACAACGGCAGGACGGCCGGGATCAAGAAGGCGCTCGTGTTCTACCACGGCAAGCCCC
CCCGCGGCGTCAAGACGGAGTGGATCATGCACGAGTACCGCCTCGCCAAGAAGGGCGGCGCCGCCGCC
GCCGCGGGCGCGGGCGCGCTCAGGCTGGATGACTGGGTGCTGTGCCGGCTGTACAACAAGAAGAACGA
GTGGGAGAAGATGCAGAGCAGGAAGGAGGAGGAGGAGGCCATGGCGGCGGCGCAGTCGTGGGGGGAGA
CGCGGACGCCGGAGTCGGAGGTCGTCGACAGCGACGCGTTCCCGGAGATGGACTACTCGCTGCCGGCG
GCGTCGTTCGACGACGCCCTGCTGCCCAAGGAGGAGGCGCGCGACGACGACTGGCTCATGGGGATGAG
CCTCGACGACCTCCAGGGCCTCGGCTCGCTGCTGCAGGCCGACGACCTCTCCATGCTCGCGCCGCCGC
CGGCGGCGAAGACGGAGCCGCTCGGCGCGCCATTCTTCTGAGCTCTCTCTCTCTCTCTCTCTCTCTCT
CTCTCTGTGACTGCACCACTGTATATAAATTCAGAGTTTTCAGACATGTTCAGTATTCAGAGTTCTCA
GGCAAGTTCAGAATTCAGAGATGGTTAAGGATTAGTGGCTTATGAGCAGTATGATATGCAGGTTAGTT
TCTAGTTTAGCAGTGTTACTCCAGATTGGAGATTTGATTAATGATTTGATTCTAATTAATGTAGTATA
CTGAGTGTTACTCATCATCACAGATTCATCAGAGCTGTGTCAAGTGACAATTCTTTTTTTTTTTATTT
CCTGGATCAAGTTCACCATGTTGTGCTCAAGATTTGTGGATAAATGCACATCCATCCTTGAATTGGC

## SEQ ID NO: 67 - NP_911548.1 - Oryza sativa

MAAAKRRVRDAEADLNLPPGFRFHPTDEELVAHYLCPRAAGRAAPVPIIAELDLYRHDPWDLPHRALF
GRREWYFFTPRDRKYPNGSRPNRAAASGYWKATGADKPVLHNGRTAGIKKALVFYHGKPPRGVKTEWI
MHEYRLAKKGGAAAAAGAGALRLDDWVLCRLYNKKNEWEKMQSRKEEEEAMAAAQSWGETRTPESEVV
DSDAFPEMDYSLPAASFDDALLPKEEARDDDWLMGMSLDDLQGLGSLLQADDLSMLAPPPAAKTEPLG
APFF

## SEQ ID NO: 68 - DQ267442.1 - Elaeis guineensis

AGGCAGAAATCTCACCGCCGCCTCAGCCATTTCTAAAGCTTGGTACAAGGAACGGTAATCCTCCAGGC
AATCGAGTTGCGAGGACGCTGACAATGGGGAGCAGGAGAAGAGATGCTGAGGCGGAGCTCAACCTGCC
GCCGGGATTCCGGTTCCACCCGACCGACGAGGAGCTCGTCGTTCATTACCTCTGCAAGAAGGTGGCCT
GCCAGCGCCTGCCCGTGCCCATCATCGCCGAGGTCGATCTCTACAAATATGACCCATGGGATCTGCCA
GAGAAGGCGTTGTTCGGGCTAAAAGAGTGGTATTTCTTCACCCCTCGTGATCGAAAGTACCCGAATGG
CTCGAGGCCTAACAGGGCCGCCGGGAGGGGGTACTGGAAGGCGACCGGTGCTGATAAACCCGTGACCC
CGAAGGGGAGCAGTAGGCCTCTCGGGATCAAGAAAGCCTTGGTGTTTTACTCTGGGAAGGCACCAAGA
GGAGTGAAGACTGATTGGATCATGCATGAGTACAGGCTTGCCGACACGAACCGAGCAGCCAACAAGAA
GGGAAGCCTAAGGCTTGATGACTGGGTTCTTTGCCGGTTGTACAACAAGAAGAACACCTGGGAGAAGA
TGCAGCAACAAAAGGAGGCAGCATCGTTTGGAGAGACGATGGATTCTGTGGATGACACAGGATCGGAC
AGCTTCAGAACACCCGAATCGGACATAGATAACGATGTCATGTTCCCGGACTTTGACGATATGGCTCA
GGTTGGAGCTCACCCACCTCAAGCTTTCAATGGAATGCAAGGAGTACGAGTCAACAATATAACTGGGT
ATCAGCCAGCAATAGAGCAGCGTCCAAAAGAGGAGAATGACTGGTTCACGGACCTGAATCTGGATGAC
TTCCACAGTACATGCATGGCTTTTGGATCAACACCTGCTCTCGATATGTCGGACCATGATTACTACTA
CTCAAACCTTCCACAACTGAGATCAAACCAGAGTGACCTGCTACCCTTCTAAAGCTACGAATCCCAGC
TGTATATAAGTGAAAATAGGCAGGAGGTGACAATCTAGTGGTAGAACTCTGTAGGATTCTTTTGTTTC
CACCAATTTTATATAGGTAAATCCATTCATAAGCAAGGGACAGTCGCTGATGTACATATGATAATATT
ATTTTGTGGTAGACCTTCTGTT

FIGURE 27 (continued)

TGAATTAGTTTTACAAATTTTGAGCATTGTCTAGTGGTAGAATATCTTCGTTTCGATTACAGAAACTT
GCCTCAGCAACAGGAAACATATTCCACACACACACACACACAAAAAAAGAAAAGCAGCAGCAGGAATC
AGAATAAGTTATAGATGGTGGGATAATCTTAAGTGACAGTATATAACACTGCGCACAAAAAGGTGGAC
AGTGAGAACTCAAAGGAGAATCAGCCGTGGCATGCGGTCATAGTTATTCGTTCAGAGAGACAGTTTAT
AGCATCTGCTTGGGATCTTTGTTTAGTGCGACACAACCAACAGTAGGAACAAAGAATATCTGTGGACA
TCCACAAGTCCAGGGTAGTATCGAGGAAACAAGCAACGTTGTGTCGATTTGACAAGAGTGACCCTCCA
AATGTTAAGTTTTCTGGAAGAGGGGAAAGAAGTGGAAGCATAAGCGCATGCATAAGCAAGCACATCAC
CAGCGAAAGCAAAAAAAAAAAAAAAAAAA

## SEQ ID NO: 69 - ABB72844.1 - Elaeis guineensis

MGSRRRDAEAELNLPPGFRFHPTDEELVVHYLCKKVACQRLPVPIIAEVDLYKYDPWDLPEKALFGLK
EWYFFTPRDRKYPNGSRPNRAAGRGYWKATGADKPVTPKGSSRPLGIKKALVFYSGKAPRGVKTDWIM
HEYRLADTNRAANKKGSLRLDDWVLCRLYNKKNTWEKMQQQKEAASFGETMDSVDDTGSDSFRTPESD
IDNDVMFPDFDDMAQVGAHPPQAFNGMQGVRVNNITGYQPAIEQRPKEENDWFTDLNLDDFHSTCMAF
GSTPALDMSDHDYYYSNLPQLRSNQSDLLPF

## SEQ ID NO: 70 - AB028183.1 - Oryza sativa

GCAGCATCAACAAAGGCAGCAGCAGCAGCAGCAGCAGCAGCAGCGTTGGTGGTGGTGGTGTGTCATCA
CCAACATTTTCACGAGAGGAGAAGGATGGAAATGGCGGCGGCGGTTGGGGGCAGCGGGAGGAGGGACG
CGGAGGCGGAGCTGAACCTGCCGCCGGGCTTCCGTTTCCACCCGACCGACGAGGAGCTCGTGGTGCAC
TACCTCTGCCGCAAGGTTGCCCGGCAGCCGCTCCCCGTCCCAATCATCGCCGAGGTCGACCTCTACAA
GCTCGACCCCTGGGATCTCCCTGAGAAGGCGTTGTTCGGGAGGAAAGAGTGGTACTTCTTCACGCCGA
GGGACCGGAAGTACCCGAACGGGTCGAGGCCGAACCGCGCAGCGGGGAGAGGGTACTGGAAGGCGACG
GGAGCCGACAAGCCGGTGGCGCCCAAGGGGAGCGCGAGGACGGTGGGGATCAAGAAGGCGCTCGTGTT
CTACTCCGGGAAGGCGCCGAGGGGGGTCAAGACGGACTGGATCATGCACGAGTACCGCCTCGCCGACG
CCGACCGCGCCCCGGGCGGCAAGAAGGGCTCACAGAAGCTGGACGAGTGGGTGCTGTGCCGGCTGTAC
AACAAGAAGAACAACTGGGAGAAGGTGAAGCTGGAGCAGCAGGACGTGGCCTCCGTGGCGGCGGCGGC
GCCGCGCAACCACCACCATCAGAACGGCGAGGTCATGGACGCGGCGGCGGCTGACACCATGTCCGACA
GCTTCCAGACGCACGACTCCGACATCGACAACGCCTCCGCCGGCCTGCGGCACGGTGGCTGCGGCGGC
GGCGGCTTCGGCGACGTGGCGCCGCCGAGGAATGGGTTCGTGACGGTGAAGGAGGACAACGACTGGTT
CACCGGCCTCAACTTCGACGAGCTGCAGCCGCCGTACATGATGAACCTGCAGCACATGCAGATGCAGA
TGGTGAATCCGGCGGCGCCAGGGCACGACGGCGGCTACTTGCAGTCCATCAGCTCGCCGCAGATGAAG
ATGTGGCAGACAATCCTGCCACCATTCTGAGATGGATGGAGCAAGAAAAAGGTTGCTGTAGATAAAGG
GCGGAAATAGGAGTGATGGCTAGAAAATTATTAGATTTACTAGAACGAAAATGATTAGAAATCTGGCA
AGCATGATTCTGCAAATGTGGTGGTAGATGCTTGCAGTATGTAATTCATTTGTTCAGTATATGCATTT
GGTAATCTGCAAAACAAAAAAAAAAAAAAAAAAAAAAAAA

FIGURE 27 (continued)

**SEQ ID NO: 71 - BAA89798.1 - Oryza sativa**

MAAAVGGSGRRDAEAELNLPPGFRFHPTDEELVVHYLCRKVARQPLPVPIIAEVDLYKLDPWDLPEKA
LFGRKEWYFFTPRDRKYPNGSRPNRAAGRGYWKATGADKPVAPKGSARTVGIKKALVFYSGKAPRGVK
TDWIMHEYRLADADRAPGGKKGSQKLDEWVLCRLYNKKNNWEKVKLEQQDVASVAAAAPRNHHHQNGE
VMDAAAADTMSDSFQTHDSDIDNASAGLRHGGCGGGGFGDVAPPRNGFVTVKEDNDWFTGLNFDELQP
PYMMNLQHMQMQMVNPAAPGHDGGYLQSISSPQMKMWQTILPPF

**SEQ ID NO: 72 - DQ028770.1 - Glycine max**

TTCCAGAGTTGGTGTTAGTCTTGGTGTAGTGTAGCTAGGGAGGGATATATATCTGAGTAAGATGGCAT
CAGAGCTTGAATTGCCCCCAGGCTTCAGATTCCATCCAACGGACGAGGAGCTGGTGTTGCACTATCTC
TGCCGCAAATGCGCGTCGCAGCCAATCGCCGTTCCCATCATCGCCGAAATCGACCTCTACAAATACGA
CCCCTGGGACTTACCCGGATTGGCTACTTATGGAGAGAAAGAGTGGTACTTCTTTTCACCACGGGACC
GGAAATACCCAAACGGTTCGAGGCCGAACCGGGCGGCTGGCACCGGTTACTGGAAGGCAACCGGGGCG
GATAAGCCCATTGGTCAGCCCAAACCGGTTGGGATTAAAAAAGCTTTGGTGTTTTACGCAGGGAAAGC
TCCTAAAGGGGACAAAAGCAATTGGATCATGCACGAGTATCGTCTCGCAGACGTAGATCGCTCCGTTC
GCAAAAAGAACACCCTAAGGTTGGATGATTGGGTGCTTTGCCGTATTTACAACAAGAAGGGCACGATC
GAGAAACTGCAACCAAGCAGCGATGTTGCTCATAGCCGAAATATCGAATCCTCGGAGATCGAAGACAG
GAAGCCGGAGATTCTGAAAAGCGGAGGAGGTTGTCTTCCGCCGCCTGCGCCGGTGCCTGCGCCGCCGC
AAGCGACGGCGAAGACGGATTACATGTACTTCGACCCGTCGGATTCAATCCCGAAGCTGCACACGGAC
TCGAGCTGTTCGGAGCAGGTGGTATCGCCGGGATTCGCGAGCGAGGTGCAAAGCGAGCCCAAGTGGAA
CGAGTGGGAGAAAAGCCTCGAATTTCCATTTAATTACGTGGATGCCACTCTCAACAACAGCTTCATGG
CCCAATTCCAGGGCAATAATCAGATGTTGTCGCCGCTGCAGGACATGTTCATGTACTGGCCCAACAAG
TCCTTTTGAGCACATCATGATGGTTTTAAATTACGATCCACAATTGCTCTTAAGATTTTCTGACTCAC
CATGCGACCACAATCGTAATTTAAAACCATGTCACATAAAATCCACGCGCGCCGCCCATTCGCATTTG
CACGGTAGCACGAGACTCAAGGTCCATGAGTGTGCCTGTAA

**SEQ ID NO: 73 - AAY46122.1 - Glycine max**

MASELELPPGFRFHPTDEELVLHYLCRKCASQPIAVPIIAEIDLYKYDPWDLPGLATYGEKEWYFFSP
RDRKYPNGSRPNRAAGTGYWKATGADKPIGQPKPVGIKKALVFYAGKAPKGDKSNWIMHEYRLADVDR
SVRKKNTLRLDDWVLCRIYNKKGTIEKLQPSSDVAHSRNIESSEIEDRKPEILKSGGGCLPPPAPVPA
PPQATAKTDYMYFDPSDSIPKLHTDSSCSEQVVSPGFASEVQSEPKWNEWEKSLEFPFNYVDATLNNS
FMAQFQGNNQMLSPLQDMFMYWPNKSF

**SEQ ID NO: 74 - AY498713.1 - Lycopersicon esculentum**

GGCACGAGCAAAGCAGGAGCAGGAGCAGCAACAAACAGAGAGAAGAAAACAGAGGAAGATAAGAGGAA
AATTTATCGAATTCGAATCGAGAGAAAAGGGGAAGTGAAGTTGCGAAGAGTGAGAATTTCAAAGGAAA
TGAACAAAGGAGCAAACGGAAATCAGCAATTGGAGTTACCGGCGGGATTCAGATTCCATCCGACAGAC
GACGAATTGGTGCAGCACTATCTCTGCAGGAAATGCGCCGGACA

FIGURE 27 (continued)

GTCGATTGCTGTATCAATTATAGCTGAAATTGATCTTTACAAGTTTGATCCATGGCAGTTGCCTGAGA
AGGCTTTGTACGGTGAAAAAGAGTGGTATTTTTTCTCACCAAGGGATAGAAAATATCCGAACGGTTCA
CGGCCGAACCGAGCAGCAGGAACCGGTTATTGGAAGGCAACCGGAGCTGATAAACCGGTGGGAAAACC
CAAAACCTTAGGGATAAAGAAGGCACTTGTGTTCTATGCCGGAAAAGCACCCAGAGGTATAAAAACAA
ATTGGATTATGCACGAGTACCGCCTCGCCAACGTGGACCGCTCTGCTGGCAAGAACAATAACTTGAGG
CTTGATGATTGGGTATTGTGTCGAATATACAACAAGAAAGGCACACTTGAGAAGCATTACAATGTGGA
CAACAAGGAAACTACAAGCTTTGGAGAATTTGATGAAGAAATAAAACCAAAAATATTGCCCACACAAT
TAGCACCGATGCCACCACGGCCTCGATCGACACCAGCAAACGACTACTTTTATTTCGAGTCATCAGAG
TCGATGACTAGAATGCACACGACAAACTCGAGCTCTGGCTCAGAGCATGTCTTGTCGCCATGTGACAA
GGAGGTTCAGAGCGCGCCCAAATGGGACGAAGACCACAGAAACACCCTTGATTTTCAGCTAAACTATT
TGGATGGTTTACTAAATGAACCATTTGAAACCCAAATGCAGCAGCAAATTTGCAACTTTGACCAGTTC
AACAATTTCCAAGACATGTTCCTATACATGCAAAAACCTTACTAAAATTGTATAAATTCATTGGATCT
AAATTGAGTGTGATCCATGACATTTTCTTTGTTCTTTGGTGGTGTAGGTCAACTTTTTATTAAGTAGT
TTAGAGAAGTACAAAATGCTAGTCAAATTTGGTGGGCTACAGCACAAATGAGCCTTGATAAGCATAGC
CAAAGAGTCGTATAGAAGGGCTTATTATTATTGTAAGGTATGTAAAAACAAATGAAAATTTGTTAATA
TCAAGTTATCATTCTTCAAAAAAAAAAAAAAAAAAA

**SEQ ID NO: 75 - AY498713.1 - Lycopersicon esculentum**

MNKGANGNQQLELPAGFRFHPTDDELVQHYLCRKCAGQSIAVSIIAEIDLYKFDPWQLPEKALYGEKE
WYFFSPRDRKYPNGSRPNRAAGTGYWKATGADKPVGKPKTLGIKKALVFYAGKAPRGIKTNWIMHEYR
LANVDRSAGKNNNLRLDDWVLCRIYNKKGTLEKHYNVDNKETTSFGEFDEEIKPKILPTQLAPMPPRP
RSTPANDYFYFESSESMTRMHTTNSSSGSEHVLSPCDKEVQSAPKWDEDHRNTLDFQLNYLDGLLNEP
FETQMQQQICNFDQFNNFQDMFLYMQKPY

**SEQ ID NO: 76 - AY245883.1 - Brassica napus**

CGAAGAAAAGAGAAGACTTTTGAAAAAAAAAAATGAAAGCAGGGCTAAACTTACCAGCAGGATTCCGAT
TCCACCCAACGGACGAAGAGCTTGTGCAATTCTACCTTTGCCGGAAATGCGCATCGGAGGAGATCTCA
GCTCCGGTCATCGCCGAAATCGATCTCTACAAGTTCAACCCCTGGGAGCTTCCTGAGATGTCTCTGTA
CGGAGAGAAAGAGTGGTACTTCTTCTCGCCACGAGACCGGAAATACCCAAACGGTTCGCGTCCTAACC
GGGCGGCGGGAACCGGTTATTGGAAAGCCACCGGAGCTGATAAACCGATCGGTAAACCGAAGACGCTG
GGTATTAAGAAAGCGCTCGTGTTCTACGCAGGGAAAGCTCCCAAAGGGATTAAGACGAATTGGATTAT
GCACGAGTATCGCCTCGCTAATGTGGACAGATCAGCTTCTGTTAACAAAAAGAACAACCTTCGACTTG
ATGATTGGGTGTTATGTCGAATCTACAACAAGAAAGGGACCATGGAGAAGTACTACCCTGCTGATGAG
AAACCGATGACCGTGACGGCGGCTTCATCGCCTTTCGATGCGTCGGACTCGACTTACCCGACGTTGCA
AGAGGATGACTCGAGCAGCTCGGGGGGTCGCGTGGTGTCGCCGGATGCGCGGGAGGTGCAGAGCGAGC
CTAAATGGGGGGAGTTTGAGAATGCTTTTGATGCTTCCATGTTCGGTGGTGGCTCCATGGACTTGCTG
CAGAGTGAAGATTTTGTGCCTCAGTTCTTGTACCAGCCTTCTTATGATTTCAACTCCT

FIGURE 27 (continued)

GGCAGGAGGATCCGCCGGAGCAGAAACCGTTCTTGAATTGGAGTTTTGCTCCACAGGGGTGAAAAAGG
AAGAGAGTGAAAGGTTTTTTGGTCTGTGTTGTGATATGTGTTAGAGAGAAGTTCTCAATCATCTTTTG
TTTCTTAGTAGTGAGAGAAAGATTGTAGAGTGTTGATAGTTTCTTAGCATCTTCAATGTTTCATTGGC
AGGTGAATTCTTGTTATTTCATCAGAAATTTATATGAAAAATTATACCTT

## SEQ ID NO: 77 - AAP35052.1 - Brassica napus

MKAGLNLPAGFRFHPTDEELVQFYLCRKCASEEISAPVIAEIDLYKFNPWELPEMSLYGEKEWYFFSP
RDRKYPNGSRPNRAAGTGYWKATGADKPIGKPKTLGIKKALVFYAGKAPKGIKTNWIMHEYRLANVDR
SASVNKKNNLRLDDWVLCRIYNKKGTMEKYYPADEKPMTVTAASSPFDASDSTYPTLQEDDSSSSGGR
VVSPDAREVQSEPKWGEFENAFDASMFGGGSMDLLQSEDFVPQFLYQPSYDFNSWQEDPPEQKPFLNW
SFAPQG

## SEQ ID NO: 78 - XM_475238.1 - Oryza sativa

ATGAGCGGCGGCGGCGAAGGGGCGGCGGCGGCGGAGAGGCAGGAGCTGCAGCTGCCGCCGGGGTTCAG
GTTCCACCCGACGGACGAGGAGCTGGTGATGCACTACCTCTGCCGGCGGTGCGCCGGCCTCCCCATCG
CCGTCCCCATCATCGCCGAGGTCGACCTCTACAAGTTCGATCCATGGCATCTCCCAAGAATGGCGCTG
TACGGCGAGAAGGAGTGGTACTTCTTCTCCCCTCGGGACCGCAAGTACCCGAACGGGTCGCGGCCGAA
CCGCGCCGCCGGGTCCGGGTACTGGAAGGCCACCGGCGCCGACAAGCCGGTGGGCACGCCGAGGCCGG
TGGCCATCAAGAAGGCGCTCGTCTTCTACGCCGGCAAGGCGCCCAAGGGCGACAAGACCAACTGGATC
ATGCACGAGTACCGCCTCGCCGACGTCGACCGCTCCGCCCGCAAGAAGAACACCCTCCGGCTAGATGA
TTGGGTCCTGTGCCGAATCTACAACAAGAAAGGCGGCGTGGAGAAGCCGAGCGGCGGCGGCGGCGGCG
AACGTTCGAATATGATGAGCCACGGGGAGACCGCGTCGGCGGGCTCGCCGCCGGAGCAGAAGCCGGCC
GTGCTGCCGCCGCCGCCACCGCCGTACGCGGCGGCGGCGCCGTTCTCGGAGCTGGCGGCGTTCTACGA
CGTGCGGCCGTCGGACTCGGTGCCGCGGGCGCACGGCGCGGACTCGAGCTGCTCGGAGCACGTGCTGA
CGACGTCGGCGTCGTCCGGCGGCGTCGTCGAGCGGCCGGAGGTGCAGAGCCAGCCCAAGATCGCCGAG
TGGGAGCGCACGTTCGCCGGCGCCGCCGCCCCGGCTGGCGCCGTCAGCACGGCCGGACCGATTCTGGG
CCAGCTCGACCCCGCCGCCGCCGTCGCCGGCGGCGGCGACCCGCTCCTCCAGGACATCCTCATGTACT
GGGGCAAGCCGTTCTGA

## SEQ ID NO: 79 - XP_475238.1 - Oryza sativa

MSGGGEGAAAAERQELQLPPGFRFHPTDEELVMHYLCRRCAGLPIAVPIIAEVDLYKFDPWHLPRMAL
YGEKEWYFFSPRDRKYPNGSRPNRAAGSGYWKATGADKPVGTPRPVAIKKALVFYAGKAPKGDKTNWI
MHEYRLADVDRSARKKNTLRLDDWVLCRIYNKKGGVEKPSGGGGGERSNMMSHGETASAGSPPEQKPA
VLPPPPPPYAAAAPFSELAAFYDVRPSDSVPRAHGADSSCSEHVLTTSASSGGVVERPEVQSQPKIAE
WERTFAGAAAPAGAVSTAGPILGQLDPAAAVAGGGDPLLQDILMYWGKPF

FIGURE 27 (continued)

## SEQ ID NO: 80 - AJ401151.1 - Solanum tuberosum

GGTCAAACAACTGAAACTAACAAAGCAGCAGCAGCAGCAACAAACAGAGAAGACAACAGAGGAAGATA
AACAAAGGAAATTAAGAGGGAGATTTATCGAATCGAAAGGGAAAGGGAAGTTACGAAGAGTGAGAATT
TGAAGGAAATGAACAAAGGAGCAACCGGAAATCAGCAATTGGAGTTACCGGCGGGATTCAGATTCCAT
CCGACAGACGACGAATTGGTGCAGCATTATCTCTGCAGGAAATGTGCCGGACAGCCAATTGCAGTATC
AATTATAACTGAAATTGATCTTTACAAGTTTGATCCATGGCAGTTGCCTGAAAAGGCTTTGTACGGTG
AAAAGGAGTGGTATTTTTTCTCACCAAGGGATAGAAAATATCCTAACGGTTCACGGCCGAACCGAGCA
GCAGGAACCGGTTATTGGAAGGCAACCGGAGCAGATAAACCGGTGGGAAAACCCAAAACATTAGGGAT
AAAGAAGGCACTTGTGTTTTATGCTGGAAAAGCACCTAGAGGAATAAAAACCAATTGGATTATGCATG
AGTACCGGCTCGCCAATGTGGACCGGTCTGCTGGCAAGAACAATAACTTGAGGCTTGATGATTGGGTA
TTGTGTCGAATTTACAACAAGAAAGGCACACTTGAGAAGCATTACAATGTGGACAACAAGGAAACTGC
AAGCTTTGGAGAATTTGATGAAGAAATAAAACCAAAAATATTGCCCACACAATTAGCACAGATGCCAC
CACGGCCCCGATCGACACCGACAAACGACTACTTCCATTTCGAATCATCGGAGTCGATGACTAGAATG
CACACCACAAACTCGAGCTCTGGCTCAGAGCATGTCTTGTCGCCATGTGACAAGGAGGTTCAGAGCGC
GCCCAAATGGGACGAAGACCACAGAAACACCCTTGATTTTCAGCTAAATTATCTGGATGGTTTACTAA
ATGAACCATTTGAAACCCAAATGCAGCAGCAAAGTTGCAACTTTGACCAGTTCAACAATTTCCAAGAC
ATGTTCTTTTACATGCAAAAGCCTTACTAAAATTGTATAAATTCATTGGATCTAAATTGATTGTGATC
CATGACATTTTCTTTGTTCTTTGGTGGTGTAGGTCAACTTTTATTAATTAGTTTAGAAAAGTACAAAA
TGCAAGTCAAATTTGGTGGGCTGTAATGAGCCTTTGATAAGCATAGCCAAAGAGTCATATAGAAGGGC
TTATTAATGTTATTATTGTAAGGTACATGTAAAACAAATGAAAATTTGTTAATATCAAGTTATCATTC
TTC

## SEQ ID NO: 81 - CAC42087.1 - Solanum tuberosum

MNKGATGNQQLELPAGFRFHPTDDELVQHYLCRKCAGQPIAVSIITEIDLYKFDPWQLPEKALYGEKE
WYFFSPRDRKYPNGSRPNRAAGTGYWKATGADKPVGKPKTLGIKKALVFYAGKAPRGIKTNWIMHEYR
LANVDRSAGKNNNLRLDDWVLCRIYNKKGTLEKHYNVDNKETASFGEFDEEIKPKILPTQLAQMPPRP
RSTPTNDYFHFESSESMTRMHTTNSSSGSEHVLSPCDKEVQSAPKWDEDHRNTLDFQLNYLDGLLNEP
FETQMQQQSCNFDQFNNFQDMFFYMQKPY

## SEQ ID NO: 82 - NM_147856.2 - Arabidopsis thaliana

CGATTACAATCTTGAGCAGACGAAGAAAACCCAAAAAAAATCCAGATCCAGCTGAAATTGAATTTTCA
ACCAACGAAGAAGAGATTTTTCCAAGAGCAACAGACAAGAAGAAGAGAATGAAGTCGGAGCTAAATTT
ACCAGCTGGGTTCCGATTCCATCCAACGGACGAGGAGCTTGTGAAATTCTACTTGTGCCGGAAATGTG
CTTCCGAGCAGATCTCGGCTCCGGTTATCGCCGAGATTGATCTCTACAAGTTCAATCCTTGGGAGCTT
CCAGAGATGTCTCTGTACGGAGAGAAAGAGTGGTACTTCTTCTCACCTAGAGATCGGAAATACCCAAA
CGGTTCGCGTCCTAACCGGGCAGCAGGAACCGGTTATTGGAAAGCTACCGGAGCAGATAAACCGATTG
GTAAACCGAAGACGTTGGGTATCAAGAAAGCACTCGTCTTCTACGCAGGGAAAGCTCCAAAAGGGATT
AAGACCAATTGGATAATGCATGAGTATCGTCTCGCTAATGTTGATAGATCAGCTTCTGTTAACAAAAA
GAACAACCTACGAC

FIGURE 27 (continued)

TTGATGATTGGGTTTTATGTCGAATATACAACAAGAAAGGAACCATGGAGAAGTATTTCCCCGCGGAT
GAGAAGCCGAGGACCACGACAATGGCTGAACAGTCATCATCACCTTTTGATACATCAGACTCGACTTA
CCCGACATTGCAAGAGGATGATTCCAGCAGCTCAGGTGGTCACGGTCACGTGGTGTCACCGGATGTTT
TGGAGGTTCAGAGCGAGCCTAAATGGGGAGAGCTTGAGGATGCTTTGGAAGCTTTTGATACTTCAATG
TTTGGTAGTTCCATGGAGTTGTTGCAGCCTGACGCTTTTGTCCCTCAGTTCTTGTATCAGTCTGATTA
TTTCACTTCCTTCCAGGATCCGCCTGAGCAGAAACCATTCTTGAATTGGAGTTTTGCTCCACAGGGGT
AAAAACGGAAGAGACCAAAAAAGGTGTTTGCTAGTAGTACTGTGATGTGCCAGAGAGAAGAGTCTCAT
CTCAACTCATCCCTGGCTCTTAGTAGTAAAAGAAGATTGTAGAATGTTAATAGCTTTTAGCATCAATG
TCTCATTAGCAGGCACATTCTTGTTCTTTCATGAGAAGTTTATATGAAAACTAAAAATTTATATTCAA
ATTCTTCAAGATGTTGCACTTATGTAGATACTGATATTAAATAACAACCTAACCTTTATGAGATATCA
TGCTCTCCGGAAGCATTTTTTGATGAGTATCATCAGCTGAATCGAGGCGATTTCCTCACAGCTGTTAT
TCCTATGTCTCTGAGACTTTTGATTCTCGTTTCTTGACCTGAGGTTTCTTTTTGGAAGACTTCTCTC
TTTCCTTCTGTTCAAGCTGTGTGGAGGGTTTTTCAGATCCATTATTCCCACGAGGAGTCTTCATTTTG
GGATC

**SEQ ID NO: 83 - NP_680161.1 - Arabidopsis thaliana**

MKSELNLPAGFRFHPTDEELVKFYLCRKCASEQISAPVIAEIDLYKFNPWELPEMSLYGEKEWYFFSP
RDRKYPNGSRPNRAAGTGYWKATGADKPIGKPKTLGIKKALVFYAGKAPKGIKTNWIMHEYRLANVDR
SASVNKKNNLRLDDWVLCRIYNKKGTMEKYFPADEKPRTTTMAEQSSSPFDTSDSTYPTLQEDDSSSS
GGHGHVVSPDVLEVQSEPKWGELEDALEAFDTSMFGSSMELLQPDAFVPQFLYQSDYFTSFQDPPEQK
PFLNWSFAPQG

**SEQ ID NO: 84 - AF509873.1 - Petunia x hybrida**

CCCATTCTCAGTATCTAGACACCCAAAAATAAAAATAAAAAGATTTATTATTTTTTCATCAAGAAATT
CGTTAGAAAAATCAAAAAATGACAACAGCAGAATTGCAATTACCTCCAGGGTTTAGATTTCACCCAAC
TGATGAAGAACTTGTGATGCACTATTTATGTAGAAAATGTGCTTCACAACCAATTGCTGTACCAATTA
TTGCTGAAATTGATCTCTACAAATATGACCCATGGGATCTTCCTGATTTGGCATTGTATGGGGAGAAA
GAATGGTATTTCTTTTCACCTAGGGACCGAAAGTATCCGAACGGTTCACGACCAAATCGAGCAGCTGG
AACAGGTTATTGGAAAGCCACTGGAGCTGATAAACCAATTGGACATCCTAAGGCAGTTGGAATTAAAA
AGGCATTGGTGTTTTACGCTGGCAAGGCTCCGAAAGGCGAGAAAACAAATTGGATTATGCACGAATAC
AGACTTGCTGATGTTGATCGATCTGCTCGTAAGAATAACAATAGCTTAAGGCTAGATGATTGGGTTTT
GTGCCGAATCTACAACAAAAAGGGCTCAATTGAAAAGAATCAACTCAATAACAAGAAAATAATGAATA
CTAGCTATATGGATATGACAGTATCAAGTGAAGAAGACCGAAAGCCAGAGATTCTACCACCGCTACCA
CCTCAGCCTGCGCCGCAACAGCAACAAGTTTATAACGATTTTTTCTACCTGGATCCATCAGATTCAGT
ACCAAAAATCCACTCAGATTCAAGCTGCTCGGAACATGTGGTTTCACCAGAGTTCACTTGTGAGAGAG
AAGTTCAGAGCGAAGCCAAGTTAAGTGAGTGGGAAAAAGCTGCCCTTGATCTTCCATTTAATTACATG
GATGCCACTACTGGAGCTACCACACTGGATAATAGCCTTTTAGGTTCACAGTTTCAGAGCAGTTATCA
GATGTCGCCATTGCAGGATATGTTCATGCACCTGCACAAACCTTTTTAAGGTCNAAAGTGAAGGAATC
AATCATTTTACGGCCGCATGTTTGTTAAAATGAGACTAAGTTAAAGTTCCCGTGACTTGCGCACGTTG
CGGGCTGTTTATAGGAATTAGTTACCATCCTTTTGGGTTTCCNCATTGGGCAATTTGTTTAAAACNAC
NTTTTTGTGTTATATAGTTTTAATAATGTTNCCCCCTNAATTNATTTAAAAAAAA

FIGURE 27 (continued)

**SEQ ID NO: 85 - AAM34773.1 - Petunia x hybrida**

MTTAELQLPPGFRFHPTDEELVMHYLCRKCASQPIAVPIIAEIDLYKYDPWDLPDLALYGEKEWYFFS
PRDRKYPNGSRPNRAAGTGYWKATGADKPIGHPKAVGIKKALVFYAGKAPKGEKTNWIMHEYRLADVD
RSARKNNNSLRLDDWVLCRIYNKKGSIEKNQLNNKKIMNTSYMDMTVSSEEDRKPEILPPLPPQPAPQ
QQQVYNDFFYLDPSDSVPKIHSDSSCSEHVVSPEFTCEREVQSEAKLSEWEKAALDLPFNYMDATTGA
TTLDNSLLGSQFQSSYQMSPLQDMFMHLHKPF

**SEQ ID NO: 86 - AB218789.1 - Prunus mume**

ACACACAGCACAGTCGAATCAAACGACGCCGTTCAGAATGTCCTCCTCCGATTTACAGCTGCCGCCCG
GCTTCAGGTTCCACCCGACGGACGAAGAGCTCGTGAAGCACTACCTCTGCCGCAAATGCCAGTCGCAG
CCGATTTCGGTCCCGATAATCGCCGAAATCGATCTCTACAAATACAACCCCTGGGACCTCCCTGGTTT
GGCGTTGTACGGTGAGAAAGAGTGGTATTTCTTTTCGCCGAGGGACCGGAAGTATCCGAATGGTTCGA
GGCCGAACCGGGCGGCCGGGAGCGGCTATTGGAAGGCGACCGGAGCGGATAAGCCGATCGGGAGCCCG
AAGCCAGTCGGGATTAAGAAGGCCCTGGTTTTCTACGCCGGAAAAGCTCCGAGAGGAGAGAAGACCAA
TTGGATTATGCACGAGTATCGCCTCGCCGACGTCGACCGCTCGCCTCGCAAAAAGAGCAGCAGCCTAA
GGTTGGACGATTGGGTTCTGTGTCGCATATACAACAAAAAGGGCACCGTCGAGAAACAGCAGCAGCAG
CAACAACAGCAACAACAGCAAACGACGAGTCGTATGAGTAGCGGCTCGGAATTCGAGGACAGGAAGCC
GGAGAATCTGGCGTGTCCTCCGCCGCCGGCTGCGGTAGCCCCCACTCGCCCGAACGACTACGTGTACT
TCGACACGTCGGACTCTGTGCCGAGGCTGCACACGGACTCAAGCTGCTCGGAGCACGTGGTGTCGCCG
GAGTTCACTTGCGAGGTGCAGAGCGAGCCCAAGTGGAAGGAGTGGGAGAAGGCCCTCGATTTTAACTA
CAATTACATGGACACCAGTGTAGAGAACGGGTTCGGGCCGCAGTTCCAGAGCGCTAATCAGATGTCGC
CGCTGCAGGATATTTTCATGTACCTACAGAAGCCGTATTGATTTTGATGAAGCGGGGCCTACAATTGC
ATTGCTATGAGTCGCCGCATCGGACGGCAACGGGAGACCCAGGTCGATGAGAGTGCGTTTTCGATGCT
GTGACAAGGGAAAGGGAAGGGGGATTTGCGTCGCGGGGTCCACCATTTGGGCCCAAGGCTGGGTGATT
TGGGAGCGTTGATATAAAGATGATATATATAGATGGAAAAAAGATGATGGGGCTGGTTTTGGAAGATA
GAAATATGAAGAAGTTTGGTTTTTAATGTGTACAGCATGTATGTATAGATATAAATAGGTAATGTTGT
TCACATGATGATTTTGAAGGGCCGTCTAAATCGTAATATTTGTTCTTTGTGAAAAAAAAAAAAAAAAA
AAA

**SEQ ID NO: 87 - BAE48667.1 - Prunus mume**

MSSSDLQLPPGFRFHPTDEELVKHYLCRKCQSQPISVPIIAEIDLYKYNPWDLPGLALYGEKEWYFFS
PRDRKYPNGSRPNRAAGSGYWKATGADKPIGSPKPVGIKKALVFYAGKAPRGEKTNWIMHEYRLADVD
RSPRKKSSSLRLDDWVLCRIYNKKGTVEKQQQQQQQQQQTTSRMSSGSEFEDRKPENLACPPPPAAV
APTRPNDYVYFDTSDSVPRLHTDSSCSEHVVSPEFTCEVQSEPKWKEWEKALDFNYNYMDTSVENGFG
PQFQSANQMSPLQDIFMYLQKPY

FIGURE 27 (continued)

**SEQ ID NO: 88 - AY245885.1 - Brassica napus**

ATTTCATTAGGATTATATTACTAGAGACAGATCCTCGAATAGAAAAGAAAATGTCAGAACTACAGCTG
CCTCCAGGATTCCGATTTCACCCAACCGACGAAGAGCTGGTGATGCACTATCTCTGCCGCAAATGCGC
CTCTCAGTCCATCGCCGTCCCCATCATCGCTGAGATCGATCTCTACAAATATGATCCTTGGGAGCTTC
CCGGTTTAGCCTTGTACGGTGAGAAGGAATGGTATTTCTTCTCT
CCAAGAGACAGAAAATATCCGAATGGTTCTCGGCCGAACCGGTCAGCTGGTTCGGGTTACTGGAAAGC
TACTGGAGCTGATAAACCGATCGGACTTCCTAAACCGGTTGGGATTAAGAAGGCTCTGGTTTTCTACG
CCGGGAAAGCTCCAAAGGGTGAGAAAACCAATTGGATCATGCATGAGTACCGTCTTGCCGACGTTGAC
CGATCATCGGCTGCTCGCAAGAAGAAGAATAGTCTCAGGCTGGATGATTGGGTTCTTTGTCGGATTTA
CAACAAAAAAGGAGCCATCGAGAAGCGAGGACCACCACCAACTCCGGTTGTTTACGGCGACGAGGTCG
TGGAGGAGAAGCCGAGGCTGTCGGAGATGGGCATGCCTCCTCCGCCGGTGATGCCGAATGATTTCGTG
TACTTTGACACGTCGGACTCGGTGCCAAAGCTGCATACGACGGAGTCGAGCTGCTCGGAGCAGGTGGT
GTCGCCGGAGTTCACGAGCGAGGTTCAGAGCGAGCCCAAGTGGAAGGATTGGTCGGGTGAGAAAAGTA
GCCTTGATTTTGGGTTTAATTACATAGATGCCACCGCGTTCGGGGGAGGAGGAGGGAGCAATCAGCTG
TTTCCGCTACAGGACATGTTCATGTACAACATGCCCAAGCCTTATTAGGTGAAGGCAAACGCTCGTCT
ATGGGTATCACGAGATCGGTTACGGTTACGGTCGGTCAATGAGTGTGCCGCCATTAGATATTTATTAC
CATGATGTTTGTTGTTCAGTGTTACTTTTATTTTCTAAGCGGTCAGATTACGGGAAATCTCTAATCGG
ATGGCGGTCGATGTGTCATTGTACTAGTGTTGTTTGGTAGAAGAATCCACATGTCTTTTCTTTTTTGG
GCTTTAGTGGGGCATAAAAGTCAAAAGCTAAGGATATTTGTTATTATCTCCTTCGTAATAATCAATTA
AATATTTCTTG

**SEQ ID NO: 89 - AAP35054.1 - Brassica napus**

MSELQLPPGFRFHPTDEELVMHYLCRKCASQSIAVPIIAEIDLYKYDPWELPGLALYGEKEWYFFSPR
DRKYPNGSRPNRSAGSGYWKATGADKPIGLPKPVGIKKALVFYAGKAPKGEKTNWIMHEYRLADVDRS
SAARKKKNSLRLDDWVLCRIYNKKGAIEKRGPPPTPVVYGDEVVEEKPRLSEMGMPPPPVMPNDFVYF
DTSDSVPKLHTTESSCSEQVVSPEFTSEVQSEPKWKDWSGEKSSLDFGFNYIDATAFGGGGGSNQLFP
LQDMFMYNMPKPY

**SEQ ID NO: 90 - NM_125774.3 - Arabidopsis thaliana**

ATGGACTTTGCTCTCTTCTCCTCGATTTCAATCTTTGAGATAAACCACAAAGATCCTCCGATTCGAAG
GTTTATAAAAACTCAAAATCGAATCTTATCCACAAGAAAACAACAAGGTACTTTTCCAAAAATGAAGG
CGGAGTTGAATTTGCCGGCGGGATTCCGATTTCATCCGACGGACGAAGAGCTTGTCAAGTTCTATCTT
TGCCGGAGATGTGCGTCAGAACCGATTAACGTTCCGGTTATCGCAGAGATTGACTTGTACAAATTCAA
TCCATGGGAGCTTCCAGAAATGGCGTTGTACGGTGAGAAAGAATGGTACTTCTTCTCGCATAGAGACC
GGAAATACCCAAACGGGTCGAGACCAAACCGGGCAGCTGGAACCGGTTATTGGAAAGCGACTGGAGCT
GATAAACCGATCGGAAAACCGAAGACGTTAGGGATTAAGAAAGCACTCGTCTTCTACGCAGGAAAAGC
TCCGAAAGGGATTAAAACGAATTGGATTATGCACGAGTATCGTCTCGCTAATGTCGATCGATCTGCTT
CTACCAACAAGAAGAACAACTTAAGACTTGATGATTGGGTTTTGTGTCGGATATACAATAAGAAAGGA
ACAATGGAGAAGTATTTACCGGCGGCGGCTGAGAAACCGACGGAAAAGATGAGTACGTCGGACTCAAG
ATGCTCAAGTCACGTGATTTCACCGGACGTCACGTGTTCTGATAACTGGGAGGTTGAGAGTGAG

FIGURE 27 (continued)

CCCAAATGGATTAATCTGGAAGACGCGTTAGAGGCATTTAATGATGACACGTCCATGTTTAGTTCCAT
TGGTTTGTTGCAAAATGACGCCTTTGTTCCTCAGTTTCAGTACCAGTCCTCCGATTTCGTCGATTCGT
TTCAGGACCCGTTCGAGCAGAAACCGTTCTTGAATTGGAATTTTGCTCCTCAAGGGTAAAAATAATCG
GCAAAAAGTTGAAGCTTTTCAGAGTCTTCGATCACCGGCATTGTGTCGGATCCTGACCCGGAGACCAA
GTCGGGTCATACGATTACATAATCGGGTTATTGAGATTTCCACATTTGGATTTCCGAGACTAACCAAC
TTAACGGATTCTGGGGTAATTGGGGGGTTTTGCACAGGTGAATCACACTGAGTCAGCAAGTTTCGATT
TTTTGGTTTTGTTTTGTAATGATTGATTAAATGTCTAAAGATATCACGAAGTAGATACAGAAGAACTG
TAAAAGCAATTGTGACCAACCATTATGAATCATATATATATTCAATGAAGCATGAGCTTATTTCTTCT
TTAATGAAGCAATGTACATTTTTCTCCAA

**SEQ ID NO: 91 - NP_201184.2 - Arabidopsis thaliana**

MDFALFSSISIFEINHKDPPIRRFIKTQNRILSTRKQQGTFPKMKAELNLPAGFRFHPTDEELVKFYL
CRRCASEPINVPVIAEIDLYKFNPWELPEMALYGEKEWYFFSHRDRKYPNGSRPNRAAGTGYWKATGA
DKPIGKPKTLGIKKALVFYAGKAPKGIKTNWIMHEYRLANVDRSASTNKKNNLRLDDWVLCRIYNKKG
TMEKYLPAAAEKPTEKMSTSDSRCSSHVISPDVTCSDNWEVESEPKWINLEDALEAFNDDTSMFSSIG
LLQNDAFVPQFQYQSSDFVDSFQDPFEQKPFLNWNFAPQG

**SEQ ID NO: 92 - AY714222.1 - Capsicum annuum**

GCAGGCAAACACACAAAGCAACAGCTGCAAACAGCAAAAGAAACACAACGGAGATTTATTCAGACGCC
TTTGATCTTCAGATAAGAAACAGGCAAGTTACCAAGAGTGATATTTATTGAGACGTCCTTGATTTTAG
CTGGGAAACAGGCAAGTTTTACAAAGAGAATGATCAAAGGAATCGTTGGAAATCAGCAATTGGGGTTA
CCTGCAGGATTTCGATTTCACCCTACTGATGAAGAGTTGGTGCAGCATTATTTGTGCAGGAAATGTGC
AGGACAGAGTATTTCTGTTTCGATTATAGCTGAAATTGATCTTTACAAGTTTGATCCTTGGCAGTTGC
CTGAAAAGGCATTATACGGTGAAAAAGAGTGGTATTTTTTCTCCCAAGAGGATAGAAAATACCCGAAC
GGTTCAAGGCCGAACCGGGCAGCAGGAACCGGTTATTGGAAAGCGACCGGAGCAGATAAACCGGTGGG
AAAACCGAAAACATTAGGGATAAAAAAGGCACTTGTATTTTATGCTGGAAAAGCACCAAGAGGTATAA
AGACTAATTGGATTATGCATGAGTATCGACTTGCTAATGTGGACAGATCTGCTGGAAAGAGTAATAAC
TTGAGGCTTGATGATTGGGTATTGTGTCGAATATACAACAAGAAGGGGCACACTTTGAGAAGTATTAC
AATGTGGGCCAGCAAGAGAGGTGAGAGTTTTGGGGAATTTGAGGATGAAATAAAACCAAAAATATTTC
CAACACAACTAGCACCGGCTCCGGGGCAGTGGCCCCCACGACCCCAATCAACCCCCGCAAGCGACTAC
TTCAACTTTGAAACATCCGAGTCGATGACTACTACTAGGATGCACACGACAAACTCGAGCTCTGGCTC
AGAGCATGTCTTGTCGTCATGTGACAAGGAGGTTCAGAGTGCACCTAAATGGGACGACCTTGGAACCG
CCGCCCTTGATTTCCAGATAAATTATTTGGATGGTTTGCTGAACGACCCTTTTGAAATCCAAATGCAG
CAGCAAAATTGCAACATTGACCAGTTCAACACTTTCCAGGACATGTTCCTATACATGCAAAAACCTTA
GTAGAATTGTATAAATCATGTGATTCAAATTGATTTTGATCCATGACATTTTGTTGGTTCTTTGGTGG
TGTAGGTCAACTTTTTATTGATTAGATTAGAAAAGTAGAAATGCTATTCAAATTTGGTGGGCTATAGC
TCAAATGAGCCTTCGCTAGATAGCCAAAGGATTATGTAGAAGGCTTATTGTAAGGTACAGGTAAAACA
AATGAAAATTTGTTAATATCAATTTATCATTCTTCAAAAAAAAAAAAA

FIGURE 27 (continued)

**SEQ ID NO: 93 - AAW48094.1 - Capsicum annuum**

MIKGIVGNQQLGLPAGFRFHPTDEELVQHYLCRKCAGQSISVSIIAEIDLYKFDPWQLPEKALYGEKE
WYFFSQEDRKYPNGSRPNRAAGTGYWKATGADKPVGKPKTLGIKKALVFYAGKAPRGIKTNWIMHEYR
LANVDRSAGKSNNLRLDDWVLCRIYNKKGHTLRSITMWASKRGESFGEFEDEIKPKIFPTQLAPAPGQ
WPPRPQSTPASDYFNFETSESMTTTRMHTTNSSSGSEHVLSSCDKEVQSAPKWDDLGTAALDFQINYL
DGLLNDPFEIQMQQQNCNIDQFNTFQDMFLYMQKP

**SEQ ID NO: 94 - NM_100054.2 - Arabidopsis thaliana**

AAGTTTCAAAAACGCCAAGTTTCAGAGGTAGAGAGAGAGATACTACAATTGATTAGGATAGGATCAGG
ATCATCCTCAACAACCTCCTCCTAATTCCTCCTCCATTCATAGTAACAATAATATTAAGAAAGAGGGT
AAACTATGTCAGAATTATTACAGTTGCCTCCAGGTTTCCGATTTCACCCTACCGATGAAGAGCTTGTC
ATGCACTATCTCTGCCGCAAATGTGCCTCTCAGTCCATCGCCGTTCCGATCATCGCTGAGATCGATCT
CTACAAATACGATCCATGGGAGCTTCCTGGTTTAGCCTTGTATGGTGAGAAGGAATGGTACTTCTTCT
CTCCCAGGGACAGAAAATATCCCAACGGTTCGCGTCCTAACCGGTCCGCTGGTTCTGGTTACTGGAAA
GCTACCGGAGCTGATAAACCGATCGGACTACCTAAACCGGTCGGAATTAAGAAAGCTCTTGTTTTCTA
CGCCGGCAAAGCTCCAAAGGGAGAGAAAACCAATTGGATCATGCACGAGTACCGTCTCGCCGACGTTG
ACCGGTCCGTTCGCAAGAAGAAGAATAGTCTCAGGCTGGATGATTGGGTTCTCTGCCGGATTTACAAC
AAAAAAGGAGCTACCGAGAGGCGGGGACCACCGCCTCCGGTTGTTTACGGCGACGAAATCATGGAGGA
GAAGCCGAAGGTGACGGAGATGGTTATGCCTCCGCCGCCGCAACAGACAAGTGAGTTCGCGTATTTCG
ACACGTCGGATTCGGTGCCGAAGCTGCATACTACGGATTCGAGTTGCTCGGAGCAGGTGGTGTCGCCG
GAGTTCACGAGCGAGGTTCAGAGCGAGCCCAAGTGGAAAGATTGGTCGGCCGTAAGTAATGACAATAA
CAATACCCTTGATTTTGGGTTTAATTACATTGATGCCACCGTGGATAACGCGTTTGGAGGAGGAGGGA
GTAGTAATCAGATGTTTCCGCTACAGGATATGTTCATGTACATGCAGAAGCCTTACTAGAAGGGAATT
CCTTTCCTGCCGCCGAAACGCAACGCAAAACGACCCTCGTTTTTGCGTTTATGGCAACACGAGACCGT
TTTATATGGTCAATGAGTGTGCCGATTCGGCCATTAGATTTCTGTTCAGTCTTCGTTTATTCTATAGA
CCGTCCGATTTCAGATCATCCCTAATCGGACGGTGGTCGTTGGATGTATCAGTAGTGTATTACTGTGT
TAGGTAGAAGAAAATCCACTTGTTCTTAAATTGGCATAAAAGTCAGAAGCTAATATTTATATGTGCCG
CAATCAATTTAATATTTTCTGTCT

**SEQ ID NO: 95 - NP_171677.1 - Arabidopsis thaliana**

MSELLQLPPGFRFHPTDEELVMHYLCRKCASQSIAVPIIAEIDLYKYDPWELPGLALYGEKEWYFFSP
RDRKYPNGSRPNRSAGSGYWKATGADKPIGLPKPVGIKKALVFYAGKAPKGEKTNWIMHEYRLADVDR
SVRKKKNSLRLDDWVLCRIYNKKGATERRGPPPPVVYGDEIMEEKPKVTEMVMPPPPQQTSEFAYFDT
SDSVPKLHTTDSSCSEQVVSPEFTSEVQSEPKWKDWSAVSNDNNNTLDFGFNYIDATVDNAFGGGGSS
NQMFPLQDMFMYMQKPY

FIGURE 27 (continued)

**SEQ ID NO: 96 - AY573802.1 - Lycopersicon esculentum**

CTAAATTCCTTCTTGTTTATCATTTTCTCTCTTCCCAAAAAAAAAATCCCAAAATTTAATCATAATAC
AATTCGAATTTATCAACCTCGTACTACGTACATATTTTTGTTGGTACGTAAAATACTGAATTCAGGTC
AACTCAAACATCGTAAATTGTGATTTCTTTATGGAAAGTACGGATTCATCAACCGGGACACGTCATCA
GCCTCAACTCCCACCGGGGTTTCGATTCCACCCGACGGACGAAGAACTCATCGTCCACTACCTCAAAA
AACGAGTCGCCGGCGCTCCGATTCCGGTGGATATTATTGGTGAAATTGATCTTTATAAGTTTGATCCA
TGGGAACTCCCTGCTAAGGCAATATTCGGAGAGCAAGAATGGTTCTTTTTTAGTCCAAGAGATAGAAA
ATATCCTAACGGGGCGAGGCCAAATCGGGCTGCAACATCGGGTTATTGGAAGGCTACCGGAACCGACA
AGCCGGTTTTTACTTCCGGTGGAACACAAAAGGTTGGGGTAAAAAAGGCGCTCGTTTTTTACGGCGGT
AAACCACCAAAAGGGGTAAAAACTAATTGGATCATGCATGAATACAGAGTTGTAGAAAATAAAACAAA
TAACAAGCCACTTGGTTGTGATAATATTGTTGCCAACAAAAAAGGATCTTTGAGGCTAGATGATTGGG
TTTTATGTCGAATTTACAAGAAGAATAACACACAAAGGTCCATAGATGATTTGCATGATATGTTGGGA
TCGATACCACAAAATGTACCAAATTCAATATTACAAGGAATAAAGCCTTCAAACTATGGTACAATATT
GCTCGAAAATGAATCGAATATGTACGATGGAATTATGAATAACACGAACGATATTATCAACAATAATA
ATAGATCCATTCCACAAATATCGTCAAAGAGAACGATGCATGGAGGTTTGTATTGGAATAACGACGAA
GCAACAACAACAACAACAACTATTGATAGGAACCATTCTCCAAATACAAAAAGGTTCCTTGTTGAGAA
CAACGAGGACGATGGACTTAACATGAATAATATTTCGCGAATTACAAATCATGAACAAAGTAGCTCCA
TTGCCAATTTCCTGAGCCAGTTTCCTCAAAATCCTTCGATTCAACAACAACAACAACAACAAGAAGAA
GTATTGGGATCTCTTAATGATGGGGTCGTCTTTCGACAACCTTATAATCAAGTTACTGGCATGAATTG
GTACTCTTAAAGATATAAAAAGGCAAAAAATAGTTAGCCCTGTAAAATCAATCGATCAATCAATCATA
GATATATTATATATGGATTTCGTTATATTTTACTTTTAGTTAGAATTAATATATAGAATATCTTCTAT
CTCACATTAACAAATAAGAACATTTATAACAAAAAAAAAAAAAAAAAAAAAAAAAAAAAAAAA

**SEQ ID NO: 97 - AAU43922.1 - Lycopersicon esculentum**

MESTDSSTGTRHQPQLPPGFRFHPTDEELIVHYLKKRVAGAPIPVDIIGEIDLYKFDPWELPAKAIFG
EQEWFFFSPRDRKYPNGARPNRAATSGYWKATGTDKPVFTSGGTQKVGVKKALVFYGGKPPKGVKTNW
IMHEYRVVENKTNNKPLGCDNIVANKKGSLRLDDWVLCRIYKKNNTQRSIDDLHDMLGSIPQNVPNSI
LQGIKPSNYGTILLENESNMYDGIMNNTNDIINNNNRSIPQISSKRTMHGGLYWNNDEATTTTTTIDR
NHSPNTKRFLVENNEDDGLNMNNISRITNHEQSSSIANFLSQFPQNPSIQQQQQQEEVLGSLNDGVV
FRQPYNQVTGMNWYS

**SEQ ID NO: 98 - NM_118875.2 - Arabidopsis thaliana**

AAAAGATCGACGAACATAAAAACAAAAACATATAATTTGGGTTTTTAGAGTTCGAAACTTGAAATCTT
TTTTTTTTTGGTTGCTGAGGAATCGAAGTAGAAGAGTATAAATGGGTGTTAGAGAGAAAGATCCGTTA
GCCCAGTTGAGTTTGCCACCAGGTTTTAGATTTTATCCGACAGATGAAGAGCTTCTTGTTCAGTATCT
ATGTCGGAAAGTTGCAGGCTATCATTTCTCTCTCCAGGTCATCGGAGACATCGATCTCTACAAGTTCG
ATCCTTGGGATTTGCCAAGTAAGGCTTTGTTTGGAGAGAAGGAATGGTATTTCTTTAGCCCAAGAGAT
CGGAAATATCCGAACGGGTCAAGACCCAATAGAGTAGCCGGGTCGGGTTATTGGAAAGCAACGGGTAC
TGACAAAATTATCACGGCGGATGGTC

FIGURE 27 (continued)

GTCGTGTCGGGATTAAAAAAGCTCTGGTCTTTTACGCCGGAAAAGCTCCCAAAGGCACTAAAACCAAC
TGGATTATGCACGAGTATCGCTTAATAGAACATTCTCGTAGCCATGGAAGCTCCAAGTTGGATGATTG
GGTGTTGTGTCGAATTTACAAGAAAACATCTGGATCTCAGAGACAAGCTGTTACTCCTGTTCAAGCTT
GTCGTGAAGAGCATAGCACGAATGGGTCGTCATCGTCTTCTTCATCACAGCTTGACGACGTTCTTGAT
TCGTTCCCGGAGATAAAAGACCAGTCTTTTAATCTTCCTCGGATGAATTCGCTCAGGACGATTCTTAA
CGGGAACTTTGATTGGGCTAGCTTGGCAGGTCTTAATCCAATTCCAGAGCTAGCTCCGACCAATGGAT
TACCGAGTTACGGTGGTTACGATGCGTTTCGAGCGGCGGAAGGTGAGGCGGAGAGTGGGCATGTGAAT
CGGCAGCAGAACTCGAGCGGGTTGACTCAGAGTTTCGGGTACAGCTCGAGTGGGTTTGGTGTTTCGGG
TCAAACATTCGAGTTTAGGCAATGAGAGAGATGTGAAGTTACTGATGGGTGAAAAAAGTAAAAAAAAA
ACTTGGAGATAGTAGAGTGGCAATTGATGTAAATAATAGGGATTTATATGGGGCTTTTACCGATTCGG
TGAGGCTTAGGATTCCACAAAGGAAAAAGGCTCGACTGGGGACTAGTTTGATCCAACTTGACGGCCCA
CAAATGTGTAATGTTTCTCAACGGAGAGAAAAATAAATGGTTACCAATATTTTTACACCGGTTATGTG
TTGATACCATTTGCACAAAACGGTTTAAGTTCTGAATTGAATAACTTAACACCCAAAATTTGGTAAAA
AGCTTAATACAAAAAGTTCAAAGGCATTTTCGCTTACTGCGACACTCGGAGTTGCAGAGTACTCTACG
TAACAGACTCTGTTCATAGAGTTTGCGGCACCGGTGGTGGGACATTCTTGCGGACACAGTATGTACTT
TTGGAAGCATCGTTCCTAAGGGTTGCTGCAGTAAACTCCTTGTGACTTGGCTCGCAAGAAACATAACA
TGGCCGCAAGAAAAAAACAGCATTAACGAAGAGGTGTTTGAGATCTTCATGTCTAGTTTATTCTTCCTG
TTTTATTATGTGTCGACTGGTGTCAAAAGAAACGTGTTGATTGTGAATTTGTAGACGCCACTCTGTAA
ATTTATTTAAAATAAACTATTTTACTTCACGTT

**SEQ ID NO: 99 - NP_567773.1 - Arabidopsis thaliana**

MGVREKDPLAQLSLPPGFRFYPTDEELLVQYLCRKVAGYHFSLQVIGDIDLYKFDPWDLPSKALFGEK
EWYFFSPRDRKYPNGSRPNRVAGSGYWKATGTDKIITADGRRVGIKKALVFYAGKAPKGTKTNWIMHE
YRLIEHSRSHGSSKLDDWVLCRIYKKTSGSQRQAVTPVQACREEHSTNGSSSSSSSQLDDVLDSFPEI
KDQSFNLPRMNSLRTILNGNFDWASLAGLNPIPELAPTNGLPSYGGYDAFRAAEGEAESGHVNRQQNS
SGLTQSFGYSSSGFGVSGQTFEFRQ

**SEQ ID NO: 100 - DQ022843.1 - Triticum aestivum**

ATTGATTCCGTTCCCACCTGCTCACCGTCGCCATGCCAATGGGCAGCAGCAGCGCCGCCATGCCCGCC
CTCCCTCCCGGCTTCCGGTTCCACCCCACCGACGAGGAGCTCATCGTCCACTACCTCGGCAGGCAGGC
CGCGTCCATGCCCAGCCCCGTGCCCATCATCGCCGAGGTCAACATCTACAAGTGCAACCCATGGGACC
TCCCCGGCAAGGCCTTGTTCGGGGAGAATGAGTGGTACTTCTTCAGCCCCCGGGATCGCAAGTACCCC
AACGGCGCGCGCCCCAACCGCGCCGCCGGGTCCGGCTACTGGAAGGCCACCGGCACCGACAAGGCCAT
CCTGTCCACGCCGGCCAACGAGAGCATCGGCGTCAAGAAGGCGCTCGTCTTCTACCGGGGCAAGCCGC
CCAAGGGCGTCAAGACCGACTGGATCATGCACGAGTACCGCCTCACCGCCGCCGACAACCGGACCACC
AAGCGCAGAGGATCCTCCATGAGGCTGGATGACTGGGTGCTGTGTAGGATCCACAAGAAGTGCAACAA
CTTGCACAACTTCTCCTCCTCTGACCAGGAACAGGAGCACGAGCAGGAGAGCTCCACCACCGTGGAGG
ACTCGCACAACAACCACACCGTGTCGTCGCCCAAGTCGGAGGCCTTCGACGGCGACGGCGACGACCAG
CTGCAGCTGCAGCAGTTCCGCCCCATGGCGATCGCCAAGTCGTGCTCCCTCACCGACCTGCTCAACAC
CGTCGACTACGCCGCGCTCTCGCACCTCCTCCTCGACGGCGCCGGCGCCGGCGCCTCG

FIGURE 27 (continued)

TCGTCGGACGCCGGAGCAGACTACCAGCTGCCGCCGGAAAACCCGCTCATCTACTCGCAGCCTCCATG
GCAACAAACGCTACACTATAATAACAATAACAATGGCTACGTGAACATCGACACCATCGACGTGCCTC
AGATACCCGAGGCCCGTGTAGATGACTACGGCATGAATGGCGATAGGTACAACGGCATGAAGAGGAAG
AGGTCCAGCGGCAGTTTGTACTGCAGCCAGCTGCAGCTCCCGGCGGATCAGTACAGCGGCATGCTGAT
CCATCCGTTCCTCAGCCAGCAGCTGCACATGTGAAGAACCAGAGAGCTTGGATCGAAGAGATCATCAT
TGTTCCATTTGAACTTGCTGTAGATCGAGGGGATCCCCCGCTGTGGCAGATAGGCAGGGATCTAGCTT
GCTTGCTGTGTCGTGACCGACCGACCGATAAGAACGGACAAATTTCAGAATTCTTGGTGTAGCACAAA
AGCTGTAAATTACGGGGGTTTATTGTGAAATAAATAAATCCAGTATTATTAAG

**SEQ ID NO: 101 - AAY44098.1 - Triticum aestivum**

MPMGSSSAAMPALPPGFRFHPTDEELIVHYLGRQAASMPSPVPIIAEVNIYKCNPWDLPGKALFGENE
WYFFSPRDRKYPNGARPNRAAGSGYWKATGTDKAILSTPANESIGVKKALVFYRGKPPKGVKTDWIMH
EYRLTAADNRTTKRRGSSMRLDDWVLCRIHKKCNNLHNFSSSDQEQEHEQESSTTVEDSHNNHTVSSP
KSEAFDGDGDDQLQLQQFRPMAIAKSCSLTDLLNTVDYAALSHLLLDGAGAGASSSDAGADYQLPPEN
PLIYSQPPWQQTLHYNNNNNGYVNIDTIDVPQIPEARVDDYGMNGDRYNGMKRKRSSGSLYCSQLQLP
ADQYSGMLIHPFLSQQLHM

**SEQ ID NO: 102 - AY625682.1 - Triticum aestivum**

GCAGCATTTTTATGCAGTAGCCATCTTCTCCTCCTCCTCCCCCCGCCTTCCAGCTAGCCACCTAGCTC
ACTATCACATCATCCAGCAGCCCACACCAACTCATATTGATTCCGTTCCCACCTGCTCGCCATCGCCA
GCCATGCCAATGGGCAGCAGCGCCGCCATGCCCGCCCTCCCTCCCGGCTTCCGGTTCCACCCCACCGA
CGAGGAGCTCATCGTCCACTACCTCCGCAGGCAGGCCGCGTCCATGCCCAGCCCCGTGCCCATCATCG
CCGAGGTCAACATCTACAAGTGCAACCCATGGGACCTCCCCGGCAAAGCTTTGTTCGGGGAGAATGAG
TGGTACTTCTTCAGCCCCCGGGATCGCAAGTACCCCAACGGCGCGCGCCCGAACCGCGCCGCCGGGTC
CGGCTACTGGAAGGCCACCGGCACCGACAAGGCCATCCTGTCCACGCCGGCCAACGAGAGCATCGGGG
TCAAGAAGGCGCTCGTGTTCTACAGGGGCAAGCCGCCCAAGGGCGTCAAGACCGACTGGATCATGCAC
GAGTACCGCCTCACCGCAGCCGACAACCGGACCACCAAGCGCAGAGGATCCTCCATGAGGCTGGATGA
CTGGGTGCTGTGTAGGATCCACAAGAAGTGCGGCAACTTGCCCAACTTCTCCTCCTCTGACCAGGAAC
AGGAGCATGAGCAGGAGAGCTCCACCGTGGAGGACTCGCAGAACAACCACACCGTGTCGTCGCCCAAG
TCCGAGGCCTTCGACGGCGACGGCGACGACCACCTCCAGTTGCAGCAGTTCCGCCCCATGGCGATCGC
CAAGTCGTGCTCCCTCACCGACCTGCTCAACACCGTCGACTACGCCGCGCTCTCGCACCTCCTCCTCG
ACGGCGCCGGCGCCTCGTCGTCGGACGCCGGAGCAGACTACCAGCTGCCTCCCGAAAACCCACTCATC
TACTCGCAGCCTCCATGGCAACAAACGCTACACTATAATAACAACAACGGCTACGTGAACAACGAGAC
CATCGACGTGCCTCAGCTACCCGAGGCGCGCGTAGATGACTACGGCATGAATGGCGATAAGTATAACG
GCATGAAGAGGAAGAGATCCAGCGGCAGCTTGTACTGCAGCCAGCTGCAGCTCCCAGCGGATCAGTAC
AGCGGCATGCTGATCCATCCGTTCCTCAGCCAGCAGCTGCACATGTGAAGAACCAGAGAGCTTGGGTC
GAAGAGATCATCATTGTTCCATTTGAACTTGCTGTAGATCGAGAGGATCCCCCGCTGTGGCAGATAGG
CAGGGATCTAGCTAGCTTGCTGTGTCGTGAACGACCGACCGATAAGAACGGACAAATTTCAGAATTCT
TGGTGTAGCACAAAGCTGTAAATTACGGGGGTTTATTGTGAAATAAATTAATCCAGTATTATC

FIGURE 27 (continued)

## SEQ ID NO: 103 - AAU08785.1 - Triticum aestivum

MPMGSSAAMPALPPGFRFHPTDEELIVHYLRRQAASMPSPVPIIAEVNIYKCNPWDLPGKALFGENEW
YFFSPRDRKYPNGARPNRAAGSGYWKATGTDKAILSTPANESIGVKKALVFYRGKPPKGVKTDWIMHE
YRLTAADNRTTKRRGSSMRLDDWVLCRIHKKCGNLPNFSSSDQEQEHEQESSTVEDSQNNHTVSSPKS
EAFDGDGDDHLQLQQFRPMAIAKSCSLTDLLNTVDYAALSHLLLDGAGASSSDAGADYQLPPENPLIY
SQPPWQQTLHYNNNNGYVNNETIDVPQLPEARVDDYGMNGDKYNGMKRKRSSGSLYCSQLQLPADQYS
GMLIHPFLSQQLHM

## SEQ ID NO: 104 - AY742218.1 - Saccharum officinarum

ATGAGCGGCGGCGGTCAGGATCTGCAGCTGCCGCCGGGGTTCCGGTTCCACCCGACGGACGAGGAGCT
GGTGATGCACTACCTCTGCCGCCGCTGCGCCAGCCTGCCCATCGCCGTCCCCATCATCGCCGAGATCG
ACCTCTACAAGTTCGATCCATGGCAGCTCCCCAGGATGGCGCTGTACGGCGAGAAGGAGTGGTACTTC
TTCTCCCCGCGGGACCGCAAGTACCCGAACGGGTCCAGGCCCAACCGCGCCGCCGGGTCCGGCTACTG
GAAGGCCACCGGCGCCGACAAGCCCGTGGGCACGCCCAAGCCGCTCGCCATCAAGAAGGCGCTCGTCT
TCTACGCCGGCAAGGCGCCCAAGGGCGAGAAGACCAACTGGATCATGCACGAGTACCGCCTCGCCGAC
GTCGACCGCTCCGCCCGCAAGAAGAACAGCCTCAGGTTGGATGACTGGGTCCTGTGCCGCATCTACAA
CAAGAAGGGAGGGCTGGAGAAGCCGGCGGCGGCGTCCTCCGGCGACCACAAGCCGATGGTGTTCGCCG
CGGGCGCGGTGAGCTCCCCGCCGGAGCAGAAGCCGTTCGTGGCGACGCCGGGCGGGCTGCCCCCCGCC
GCGTTCACGGCGGACCTGGCGGCGTACTACGACCGGCCGTCGGACTCGATGCCGCGGCTGCACGCGGA
CTCCAGCTGCTCGGAGCAGGTGCTGTCGCCGGAGCAGCTGGCGTGCGACCGGGAGGTGCAGAGCCAGC
CCAAGATCAGCGAGTGGGAGCGCACCTTCGCCTCCGACCCCGTCAACCCCGCTGGCTCCATGCTCGTC
GACCCCGTCGTCGGTGGCCACGCCGGCGACCCGCTGCTGCAGGACATCCTCATGTACTGGGGCAAGCC
GTTCTAG

## SEQ ID NO: 105 - AAW62955.1 - Saccharum officinarum

MSGGGQDLQLPPGFRFHPTDEELVMHYLCRRCASLPIAVPIIAEIDLYKFDPWQLPRMALYGEKEWYF
FSPRDRKYPNGSRPNRAAGSGYWKATGADKPVGTPKPLAIKKALVFYAGKAPKGEKTNWIMHEYRLAD
VDRSARKKNSLRLDDWVLCRIYNKKGGLEKPAAASSGDHKPMVFAAGAVSSPPEQKPFVATPGGLPPA
AFTADLAAYYDRPSDSMPRLHADSSCSEQVLSPEQLACDREVQSQPKISEWERTFASDPVNPAGSMLV
DPVVGGHAGDPLLQDILMYWGKPF

## SEQ ID NO: 106 - AK063406.1 - Oryza sativa

AACTAGCTTAGCTAGACAGCGTGTGTTGGTGGTGGTGGTGGTGTGTGTGTGTGTGAGGAGGGTGATCG
ATCGATCGAGCGATGGAGAGCCCGGACTCGTCGTCCGGCTCGGCGCCACCGCGAGTACTACGGCGGCA
ACAGCAGCAGCCGGGCTCGGCGCCGGAGCTGCCGCCGGGGTTCCGGTTCCACCCGACGGACGAGGAGC
TGGTGGTGCACTACCTCAAGAAGAAGGCCGCCTCCGTTCCGCTCCCCGTCACCATCATCGCCGAGGTC
GATCTCTACAAGTTCGATCCCTGGGATCTCCCCGAGAAGGCGAACTTCGGGGAGCAGGAGTGGTATTT
CTTCAGCCCGAGGGACCACAAGTACCCGAACGGGGCGCGGCCGAACCGGGCGGCGACGTCGGGGTACT
GGAAGGCCACCGGCACCGACAAGCCCATCATGTCGTCGGGGAGCACCCGCGAGAAGGTCGGCGTGAAG
AAGGCGCTCGTGTTCTACCG

FIGURE 27 (continued)

GGGCAAGCCACCCAAGGGCGTCAAGACTAACTGGATCATGCACGAGTACCGTCTCACGGACACGTCTA
GCTCCGCCGCCGCCGTCGCTACGACCAGGCGGCCGCCGCCGCCCATCACCGGCGGTAGCAAGGGCGCC
GTCTCTCTCAGGCTGGATGACTGGGTGCTGTGCCGCATATACAAGAAGACGAACAAGGCCGGTGCGGG
GCAGAGGAGCATGGAGTGCGAGGACTCCGTGGAGGACGCGGTGGCCGCGTACGCGCCGTCGTCGCAGC
AGCATGCCACGGCTGCTGCTGGCATGGCCGGTTCGGACGGCGCCGGAGGAGTTGCTGCAGCGCACGGC
GGCGACTACAGTTCACTGCTCCATCACGACAGCCACGAGGACACCTTCCTCGTAAACGGCCTGCTCAC
CGCGGAGGACGCCGCCGGCCTCTCGACCGGCGCCAGCTCTCTCAGCCAGCTCGCCGCGGCGGCGAGGG
CGGCGGCGACACCGTGCGACGCCACCAAGCAGCTTCTTGCTCCGTCTCCAACCCCATTCAACTGGTTC
GAGGCATTCCTTCCACGGGCCAAGGAGTTTCCTAGTGGGCTAAGCAGGAGTAGCAGAGACATCGGCGA
CATGTCGCTGTCATCGACGGTGGACAGGAGCCTGTCTGAGGCTGGCGCCGTGGCCATTGACACCGGCG
ACGCCGCCAATGGCGCAAACACTATGCCTGCATTTATCAATCCTCTCGGCGTGCAGGGTGCAACCTAC
CAACAACACCAAGCCATCATGGGTGCCTCGTTGCCATCGGAGTCAGCAGCAGCAGCAGCCGCCTGCAA
TTTCCAGCATCCGTTCCAACTCTCCAGGGTGAATTGGGATTCCTGAATAAAAGGTGCCGGCATTATGC
ATACACATATATGCACATGCATACATGCATGGCTTTTCAAGTAGTAGCACAACATTACTAGTAATTAA
TCACACTAATGTGTGTGGCAGTCGCATTCAGTGAGCACTGATCGAGTAGTTGCATGAACACAACACTA
ATGCATGCACTACATATATATGGCCGCATTGCTCCTAGGGCACGTACCTGTACGAACTAGGTAAGTGA
CCTAACCAGCTAGATCATGTTCAGAATAAGGAGAAGAAGCCGCATGCAAACACGTAGATCATATGGCT
TTGCCTTCACATGCGTTTGCATCATATATATCGCATTCAGAAAGTAGTGCTGCAGCTAACACATAGAT
ATGGTGTTAGCTTCATGCGTTTGTACCATATATAGCGGATGCAGAAAGTAGCTAGTTGCACTGTTCAT
CATTATCCCATGAGATATGAACTTTATAT

**SEQ ID NO: 107 - AK063406.1 - Oryza sativa**

MESPDSSSGSAPPRVLRRQQQQPGSAPELPPGFRFHPTDEELVVHYLKKKAASVPLPVTIIAEVDLYK
FDPWDLPEKANFGEQEWYFFSPRDHKYPNGARPNRAATSGYWKATGTDKPIMSSGSTREKVGVKKALV
FYRGKPPKGVKTNWIMHEYRLTDTSSSAAAVATTRRPPPPITGGSKGAVSLRLDDWVLCRIYKKTNKA
GAGQRSMECEDSVEDAVAAYAPSSQQHATAAAGMAGSDGAGGVAAAHGGDYSSLLHHDSHEDTFLVNG
LLTAEDAAGLSTGASSLSQLAAAARAAATPCDATKQLLAPSPTPFNWFEAFLPRAKEFPSGLSRSSRD
IGDMSLSSTVDRSLSEAGAVAIDTGDAANGANTMPAFINPLGVQGATYQQHQAIMGASLPSESAAAAA
ACNFQHPFQLSRVNWDS

**SEQ ID NO: 108 - AC145753.1 – Medicago truncatula**

ATGGGAACCCCACAGACCAATTTGCCACCAGGTTTTAGGTTCCACCCTACTGATGCAGAACTCATTCT
TCACTACCTTAGGAAAAAAATTGCCTCCATTCCCTTGCCTGTTTCCATCATTGCTGAAGTTGATATTT
ATAAGTTGGATCCTTGGGATTTACCAGCTAAGGCTTCATTTGGTGAGAAAGAATGGTACTTTTTTAGT
CCTAGAGATAGAAAGTACCCAAACGGTGCAAGGCCAAACAGGGCAGCTGCTTCAGGGTATTGGAAAGC
CACTGGCACTGATAAGACCATAGTGGCATCATTGCCATGTGGAGGAGGAAGATCACAAGAGAACATTA
TTGGTGTCAAAAAGGCTCTTGTTTTTTACAAAGGAAAACCCCCAAAGGGTATTAAGACTAATTGGATC
ATGCATGAATATCGTCTTGTTGACAACAATAAACCTATTAAGCTCAAAGATTCTTCCATGAGGTTGGA
TGATTGGGTGCTATGCCGAATTTACAAGAAATCAAAATGTGCACTAACTTCAACAGAATCATCAGAAA
CTATGGTAGGTGAAGTGGAACATGCAGAAGAGACACAATTCCAAGAAACCCTATTTCCAATCACCAAA
AACACAAC

FIGURE 27 (continued)

CTCACCTCTTCAAAACACACTCATGTCTCAAAAATCAGTGTCCTTTTCAAACCTATTAGATGCTATGG
ACTACTCCATGTTAAGCAGCTTCTTATCTGAAAATTCCAACAACCCATCAGGAATTGGAACAAGTTCA
GGTTTCAACACTGAAAATTTTAACCAACAACAATCTTCCCATATCAACACTTGCAACAATTACATGTC
TCAGAAGAATCCTCAATCCAACACTTTAAAGCACCAGCTTTCAAACGTAGATGAAGACATGTTATACC
CATCAAAGAAGTACTTGAGTTCCTCTTGCAATTTTCCTAACATCAATTCTCAGTATGAAAACTACCTT
ATGAAGCAATCTTTGATGAATCAACAATTGCTTTTAGGTCCTCATCATCAATATCAAGGCTAATCCAA
AATACCACAAAAATTAAGTGGTACCAAAAAAAAAAAAGATTATGAAATAGAAAAGTTAAGTAAGATTTC
TAGAAGTTGGGCCCACCAAATTACACCAATGTCATCATTAAGTAAGGATTGCTAATTTAATAGTAGTG
CACAAAATATAGTGTGTTCTATTTTGTCCATTTTAATTTTTTTTCTAGGAATTG

**SEQ ID NO: 109 - AC145753.1 – Medicago truncatula**

MGTPQTNLPPGFRFHPTDAELILHYLRKKIASIPLPVSIIAEVDIYKLDPWDLPAKASFGEKEWYFFS
PRDRKYPNGARPNRAAASGYWKATGTDKTIVASLPCGGGRSQENIIGVKKALVFYKGKPPKGIKTNWI
MHEYRLVDNNKPIKLKDSSMRLDDWVLCRIYKKSKCALTSTESSETMVGEVEHAEETQFQETLFPITK
NTTSPLQNTLMSQKSVSFSNLLDAMDYSMLSSFLSENSNNPSGIGTSSGFNTENFNQQQSSHINTCNN
YMSQKNPQSNTLKHQLSNVDEDMLYPSKKYLSSSCNFPNINSQYENYLMKQSLMNQQLLLGPHHQYQG

FIGURE 27 (continued)

**SEQ ID NO: 110 – Oryza sativa – rcc3 promoter**

TCGACGCTACTCAAGTGGTGGGAGGCCACCGCATGTTCCAACGAAGCGCCAAAGAAAGCCTTGCAGAC
TCTAATGCTATTAGTCGCCTAGGATATTTGGAATGAAAGGAACCGCAGAGTTTTTCAGCACCAAGAGC
TTCCGGTGGCTAGTCTGATAGCCAAAATTAAGGAGGATGCCAAAACATGGGTCTTGGCGGGCGCGAAA
CACCTTGATAGGTGGCTTACCTTTTAACATGTTCGGGCCAAAGGCCTTGAGACGGTAAAGTTTTCTAT
TTGCGCTTGCGCATGTACAATTTTATTCCTCTATTCAATGAAATTGGTGGCTCACTGGTTCATTAAAA
AAAAAAGAATCTAGCCTGTTCGGGAAGAAGAGGATTTTGTTCGTGAGAGAGAGAGAGAGAGAGAGAGA
GAGAGAGAGAGAGAAGGAGGAGGAGGATTTTCAGGCTTCGCATTGCCCAACCTCTGCTTCTGTTGGCC
CAAGAAGAATCCCAGGCGCCCATGGGCTGGCAGTTTACCACGGACCTACCTAGCCTACCTTAGCTATC
TAAGCGGGCCGACCTAGTAGCCACGTGCCTAGTGTAGATTAAAGTTGCCGGGCCAGCAGGAAGCCACG
CTGCAATGGCATCTTCCCCTGTCCTTCGCGTACGTGAAAACAAACCCAGGTAAGCTTAGAATCTTCTT
GCCCGTTGGACTGGGACACCCACCAATCCCACCATGCCCCGATATTCCTCCGGTCTCGGTTCATGTGA
TGTCCTCTCTTGTGTGATCACGGAGCAAGCATTCTTAAACGGCAAAAGAAAATCACCAACTTGCTCAC
GCAGTCACGCTGCACCGCGCGAAGCGACGCCCGATAGGCCAAGATCGCGAGATAAAATAACAACCAAT
GATCATAAGGAAACAAGCCCGCGATGTGTCGTGTGCAGCAATCTTGGTCATTTGCGGGATCGAGTGCT
TCACAGCTAACCAAATATTCGGCCGATGATTTAACACATTATCAGCGTAGATGTACGTACGATTTGTT
AATTAATCTACGAGCCTTGCTAGGGCAGGTGTTCTGCCAGCCAATCCAGATCGCCCTCGTATGCACGC
TCACATGATGGCAGGGCAGGGTTCACATGAGCTCTAACGGTCGATTAATTAATCCCGGGGCTCGACTA
TAAATACCTCCCTAATCCCATGATCAAAACCATCTCAAGCAGCCTAATCATCTCCAGCTGATCAAGAG
CTCTTAATTAGCTAGCTAGTGATTAGCTGCGCTTGTGATC

**SEQ ID NO: 111 - Motif I**

A/P/S/N V/L/I/A P/S/D/V/Q V/I I A/T/G

**SEQ ID NO: 112 - Motif II**

N G/S S/Q/A/V RP N/S

FIGURE 27 (continued)

**SEQ ID NO: 113 - Motif VI**

C/Y R/K L/I Y/H/F N/K K K/N/C/S/T

**SEQ ID NO: 114 - Motif VII**

N E/Q/T WEK M/V Q/R/K

**SEQ ID NO: 115 - Motif VIII**

WGE T/A RTPES E/D

**SEQ ID NO: 116 - Motif IX**

V/L PK K/E E S/R/A/V M/V/A/Q/R D/E D/E/L A/G/D

**SEQ ID NO: 117 - Motif X**

S L/Y DD L/I Q G/S L/M/P G/Y S/N

**SEQ ID NO: 118 - Motif XI**

DS M/V/I P R/K L/I/A H T/A/S D/E SS C/G SE

**SEQ ID NO: 119 – forward primer**

CCAACACTAGTAGGATAAAG

**SEQ ID NO: 120 – reverse primer**

ACCACTGGGCTAATTAATTA

**SEQ ID NO: 121 – forward primer**

GGTCGACCCACGCGTCCGCT

**SEQ ID NO: 122 – reverse primer**

AACTGAAGTACCCGTTCTTA

**SEQ ID NO: 123 – forward primer**

ATCCTCCACAAGAGAAACTA

FIGURE 27 (continued)

**SEQ ID NO: 124 – reverse primer**

AGTACAGTGTAGCACAATAA

**SEQ ID NO: 125 – primer**

GGGGACAAGTTTGTACAAAAAAGCAGGCTTAAACAATGGGGATGGGGATGAG

**SEQ ID NO: 126 – primer**

GGGGACCACTTTGTACAAGAAAGCTGGGTTCGATCACCACCTGTTCG

**SEQ ID NO: 127 – primer**

GGGGACAAGTTTGTACAAAAAAGCAGGCTTAAACAATGGAGTGCGGTGGTG

**SEQ ID NO: 128 – primer**

GGGGACCACTTTGTACAAGAAAGCTGGGTGTCACGTTCCGTTAATTAGAA

**SEQ ID NO: 129 – primer**

GGGGACAAGTTTGTACAAAAAAGCAGGCTTAAACAATGCCGAGCAGCG

**SEQ ID NO: 130 – primer**

GGGGACCACTTTGTACAAGAAAGCTGGGTCTACTGCATCTGCAGATGA

FIGURE 27 (continued)

**SEQ ID NO: 131, OsAP2-2 encoding sequence (AP2-EREBP)**

CATGTGCGGCGGCGCCATCCTCTCCGACCTCATCCCGCCGCCGCGGCGGGTCACCGCCGGCGACCTCT
GGCTGGAGAAGACCAAGAAGCAGCAGCAGCAGAAGAAGAAGAACAAGGGCGCGAGGAGGCTGCCACTG
CGCCAAGAGGAGGAGGATGATTTCGAGGCCGACTTCGAGGAGTTCGAGGTGGATTCCGGCGAGTGGGA
GGTGGAGTCCGACGCCGACGAGGCCAAGCCGCTCGCCGCGCCCCGGAGCGGCTTCGCTAAAGGTGGAT
TGAAAAACACTACTGTTGCTGGTGCTGATGGGCCTGCAGCAAGGTCTGCTAAAAGGAAGAGAAAGAAC
CAATTCAGGGGTATCCGCCAGCGGCCATGGGGCAAATGGGCTGCGGAAATCAGAGATCCTCGCAAAGG
TGTCCGCGTCTGGCTTGGCACCTTCAACTCTCCTGAGGAAGCTGCCAGAGCTTATGATGCTGAAGCAC
GAAGGATTCGAGGCAAGAAGGCCAAGGTCAATTTCCCAGATGGGGCTCCAGTGGCTTCTCAGAGGAGT
CATGCTGAGCCCTCCTCCATGAACATGCCTGCTTTCAGCATCGAAGAGAAGCCGGCCGTCATGTCAGC
AGGCAACAAAACCATGTACAACACAAATGCTTATGCCTACCCTGCTGTTGAGTACACCTTACAGGAGC
CATTTGTGCAGATTCAGAATGTCTCATTTGTTCCTGCAATGAACGCGATTGAGGATACTTTCGTGAAC
CTGTCCTCTGATCAAGGGAGCAACTCCTTTGGTTGCTCGGACTTTAGCCAGGAGAATGATATCAAGAC
CCCTGACATAACTTCCATGCTTGCACCGACCATGACAGGTGTTGATGACTCCGCATTCCTCCAGAACA
ATGCCAGTGATGCAATGGTACCTCCTGTGATGGGGAATGCTAGCATTGATCTTGCTGACCTGGAGCCG
TACATGAAATTTCTGATCGATGGTGGTTCGGATGAGTCGATTGACACCCTTCTGAGCTCTGATGGATC
TCAGGATGTGGCCAGTAGCATGGACCTTTGGAGCTTCGATGACATGCCCGTGTCGGCCGAGTTCTACT
GAGGGGTTTGGG

**SEQ ID NO: 132, OsAP2-2, deduced protein sequence, (AP2-EREBP)**

MCGGAILSDLIPPPRRVTAGDLWLEKTKKQQQQKKKNKGARRLPLRQEEEDDFEADFEEFEVDSGEWE
VESDADEAKPLAAPRSGFAKGGLKNTTVAGADGPAARSAKRKRKNQFRGIRQRPWGKWAAEIRDPRKG
VRVWLGTFNSPEEAARAYDAEARRIRGKKAKVNFPDGAPVASQRSHAEPSSMNMPAFSIEEKPAVMSA
GNKTMYNTNAYAYPAVEYTLQEPFVQIQNVSFVPAMNAIEDTFVNLSSDQGSNSFGCSDFSQENDIKT
PDITSMLAPTMTGVDDSAFLQNNASDAMVPPVMGNASIDLADLEPYMKFLIDGGSDESIDTLLSSDGS
QDVASSMDLWSFDDMPVSAEFY

**SEQ ID NO: 133, Motif XII**

(R/I/V/L/K)(R/K/Q/D/E/A)(I/L)(R/Y/H)G(A/S/R/K/T/D/G/H/L/N)(K/T/R/N/G
)A(K/R/E)(V/L/P/T)NF(P/V)

**SEQ ID NO: 134, Motif XIII**

MCGG(A/S)(I/V/L)(I/L)(S/H/A/Y/G/P/E)(D/G/H/Y/E/Q/N)

**SEQ ID NO: 135, Motif XIV**

(K/N/R/S/H/M/Q/P/A/E)(R/K/H/A/G/E/V/P/M)(E/K/A/R/S/Q/T/V/G/H/P)(R/K/
Q/S/G)(K/G/P/S/R/T/A/N)(T/N/R/S/A/Y/H/G)(L/Q/H/V/R/K/A/P/G)(Y/F)(R/W
/K/L/M)G(I/V)(R/Q/H)(R/Q/W/K)R(P/K/T)

**SEQ ID NO: 136, Motif XV**

SD(Q/T/E/V)(G/S)SNSF(G/D/E/S/N)(C/S)S(D/E)(F/Y/L)(G/S)(W/Q/L)(E/G/S)
(N/E/D)

FIGURE 28

**SEQ ID NO: 137, Motif XVI**

(L/I/F/M)W(S/T/N/M)(F/Y/L/I)(D/E/Q/G)(N/D/H/E)(I/Y/F/S/M/L/V/D/H/N/E
/G)

**SEQ ID NO: 138, Motif XVII**

(D/E/S)(F/A/W/D)(E/A)(A/D/L)(D/A/G/E)(F/G/L)(N/E/R/G/Q/W)(E/G/V/D/R)
F(E/K/V/Y/G/D/L/I)(V/R/D/S/N/A/E)(D/G/T/E/R/A/Y/L/F)

**SEQ ID NO: 139, NM_001067153.1| Oryza sativa (japonica cultivar-group) Os07g0674800 (Os07g0674800) mRNA, complete cds**

GACTCGTCAGCTTAGAGCACCGCAAGCGCGCAGCAGCAGCAAAGATGTGTGGCGGCGCGATCATTTCC
GACTTCATCCCGCAGCGGGAAGCCCACCGCGCGGCCACCGGCAGCAAGCGTGCCCTCTGCGCCTCCGA
CTTCTGGCCGTCGGCGTCGCAGGAAGCCGCCGACTTCGACCACCTCACCGCCCCCTGCACCTTCACCC
CCGACCAAGCGGCAGAGGAGCCGACCAAGAAGCGGGAGCGGAAGACGCTGTACCGTGGCATCAGGCGG
CGGCCGTGGGGGAAGTGGGCGGCGGAGATCCGCGACCCGGCGAAGGGCGCGCGCGTCTGGCTCGGCAC
CTTCGCCACCGCCGAGGCGGCGGCCCGCGCCTACGACCGCGCCGCCCGCCGCATCCGCGGGGCCAAGG
CCAAGGTCAACTTCCCCAACGAGGACCCGCCACTCGACGACCCGGCCGCCGACGGCCACAGCCACGGC
GGCGCCGCCATCCCGTGCAGGGAGTTCATGGACTACGACGCCGTCATGGCGGGCTTCTTCCACCAGCC
CTACGTCGTCGCCGACGGCGTGCCGGCCGTGCCGGCGGAGGAGGCGCCCACGGTGGCGTACGTGCACC
ACCACCTGCCGCCGCAGCCGCAGCAGGACGCGGGGCTGGAGCTCTGGAGCTTTGATAACATCCACACG
GCCGTGCCGATGTGAGATCGATCTGATGATCATTCAGATCGATGCTAGCTCATGTGTTTTTAATTATA
TCTTCACAGCAGAAACAAAAAAAAAGTTCAATTCAGTTTATTTTGTTGTTATACGTTTTAAAATGTTTC
ATTAGGTTTTCATGTTATAGGAGTGATTTATCGGATTGAACTCTCAAGTGACCGACTTCTGAGTTCTT

**SEQ ID NO: 140, LOC_Os07g47790.1|11977.m08994|protein|AP2-EREBP**

MCGGAIISDFIPQREAHRAATGSKRALCASDFWPSASQEAADFDHLTAPCTFTPDQAAEEPTKKRERK
TLYRGIRRRPWGKWAAEIRDPAKGARVWLGTFATAEAAARAYDRAARRIRGAKAKVNFPNEDPPLDDP
AADGHSHGGAAIPCREFMDYDAVMAGFFHQPYVVADGVPAVPAEEAPTVAYVHHHLPPQPQQDAGLEL
WSFDNIHTAVPM

**SEQ ID NO: 141, AY781352.1| Triticum aestivum ethylene-responsive element binding protein 1 (EREB1) mRNA, complete cds**

CTCTCCACCCGCGGCCCCACGTGCTCCCAGCCATGTGCGGCGGCGCCATCCTCTCCGACATCATCCCG
CCGCCGCGCCGGGCCACCGGCGGCAACGTCTGGCGGGCGGACAAGAAGAGGAGGGCCAGGCCCGACGC
CGCCGCGGGGAGGCCCCGCCGCGTGCCCGAGGAGGAGTTCCAGGAGGAGGAGGGCGACGCGGAGTTCG
AGGCCGACTTCGAGGGGTTCGTGGAGGCGGAGGAGGAGTCCGACGGCGAGGCCAAGCCCTTCCCCGTC
CGCAGGACCGGCTTCTCCGGAGATGGACTGAAGGCAACTGCTGCTGGTGATGATGACTGTGCCTCAGG
GTCTGCTAAAAGGAAGAGAAAGAGCCAGTTCAGGGGCATCCGCCGCCGCCCTTGGGGTAAATGGGCTG
CTGAAATAAGAGATCCTCGCAAGGGTGTCCGTGTCTGGCTTGGCACTTACAACTCTGCTGAGGAAGCT
GCCAGAGCCTATGATGTTGAAGCCCGCAGAATTCGTGGCAAGAAGGCAAAGG

FIGURE 28 (continued)

TCAATTTCCCAGAAGAAGCTCCCATGGATCCTCAGCAACGCTGCGCTACCTCTGTGAAGGTGCCCGAGT
TCAACACCGAACAGAAGCCAGTACTCAACACCATGGGCAACACAGATGTGTATTCCTGCCCTGCTGTTG
ACTACACCTTAAATCAGCAATTTGTGCAGCCTCAGAACATGTCGTTTGTGCCTACAGTGAATGCAGTTG
AGGCTCCTTTCATGAATTTTTCCTCTGACCAGGGGAGCAACTCCTTTAGTTGCTCAGACTTCAGCTGGG
AGAATGATATCAAGACCCCTGACATAACTTCTGTGCTTGCATCCATTCCCACCTCAACTGAGGTCAATG
AATCTGCATTTCTCCAGAACAATGGCATTAATTCAACGGTACCTCCTGTGATGGGTGATGCTAATGTTG
ATCTTGCCGACTTGGAGCCATACATGAAGTTCCTGATGGACGATGGTTCAGATGAGTCAATTGACAGCA
TTCTAAGCTGTGATGTACCGCAGGACGTTGTCGGCAACATGGGCCTTTGGACCTTTGATGACATGCCCT
TGTCTGCTGGTTTCTACTGAGGGAATCGAGGTCGCTGGGTGCCTGTATATATAGACAAAGGAATAAGTA
TTCTGGACATCAACAAGTGCTTGTGTCTGGTGCCTCTAGAATCGAGCAGTAGCGACGTCAGTCTATGGT
TATGTCTAGCTTAAATGGTCAGGAGACCTAAGTCTTTTGCAATAGACCTCTGTCTTGTGCCCCCAGACT
ATATTATATCTATATATGAAACCAGTATGTGATGGGAACTGCTTATTTTGTATTCCTGTTTCTACCTTA
TTGTAATTGCTACAAGTGGCTGTAAACCTTTTAACTTTGAAAAAAAA

**SEQ ID NO: 142, AAX13280.1| ethylene-responsive element binding protein 1 [Triticum aestivum]**

MCGGAILSDIIPPPRRATGGNVWRADKKRRARPDAAAGRPRRVPEEEFQEEEGDAEFEADFEGFVEAE
EESDGEAKPFPVRRTGFSGDGLKATAAGDDDCASGSAKRKRKSQFRGIRRRPWGKWAAEIRDPRKGVR
VWLGTYNSAEEAARAYDVEARRIRGKKAKVNFPEEAPMDPQQRCATSVKVPEFNTEQKPVLNTMGNTD
VYSCPAVDYTLNQQFVQPQNMSFVPTVNAVEAPFMNFSSDQGSNSFSCSDFSWENDIKTPDITSVLAS
IPTSTEVNESAFLQNNGINSTVPPVMGDANVDLADLEPYMKFLMDDGSDESIDSILSCDVPQDVVGNM
GLWTFDDMPLSAGFY

**SEQ ID NO: 143, NM_001049403.1| Oryza sativa (japonica cultivar-group) Os01g0313300 (Os01g0313300) mRNA, complete cds**

ATCCAAATCCAACGTCCGCCTAAAGAAAAACACGCACACACTTCTTCTGCTTCCCTCCCCTTGTTTCC
GCTCCACTTCCCTTCCAAGCAAGAAAACCGAGGCTTCAGCTTAGCTAGGACCGACCGATCCGATGTGC
GGCGGTGCAATCATCTACGACTACATCCCGGCGCGCCGCCGGTTGTGCGCCTCCGACTTCTGGCCCGA
CGCCGACGACTCCGACCCCCACACCCCCGCTCCCGAGAAACCGCCGCGCGCGAAGAGGGAGCGGAAGA
ACCAGTACCGCGGGATCAGGCAGCGGCCGTGGGGGAAGTGGGCGGCGGAGATCCGCGACCCGGTGAAG
GGGGTGCGCGTCTGGCTCGGCACCTACCCGACCGCCGAGGCCGCCGCGCGGGCCTACGACCGCGCCGC
GCGCCGCATCAGGGGCGCCAAGGCGAAGGTCAACTTCCCCAACGACTTCGGCGCCGCCCCCGCGCCGG
CCGCGGCGGCGGCGAAGGCCGTCCCTCGCGTCGCGCCCACGCCGGCCGTGCTCCCGCCGCCCAAGATG
GAGGCGGTGTCCGAGGGCGCCGGCGCCTGCTCCTCCGACGAGGTCAAGGAGCTGTCCGAGGAGCTGCT
CGCGTACGAGAACTACATGAGCTTCCTCGGCATCCCCTACATGGAGGGCGGCGCCGCCTCCGCCGCCG
GCGCCGAGGAAGCCGCGGCGCCCGCCGGGCTCTGGACCTTCGAAGACTACGAGCTGCCGTCGCTAGCG
CTCTAGTAAAAATGTCACAATAAAAATGACATGTATGTGAAAAAATTTTTCCCTACCCGTAGTAAGTA
ACCAGTGTCTTTTTTTACCGTACTCTATGCGTTATTTTTGTTGAAATTAATCCATTGTTGAAGTTGTA
ACTGTGCATGGTCGGCGCCAGTCT

**SEQ ID NO: 144, LOC_Os01g21120.1|11971.m08596|protein|AP2-EREBP**

MCGGAIIYDYIPARRRLCASDFWPDADDSDPHTPAPEKPPRAKRERKNQYRGIRQRPWGKWAAEIRDP
VKGVRVWLGTYPTAEAAARAYDRAARRIRGAKAKVNFPNDFGAAPAPAAAAAKAVPRVAPTPAVLPPP
KMEAVSEGAGACSSDEVKELSEELLAYENYMSFLGIPYMEGGAASAAGAEEAAAPAGLWTFEDYELPS
LAL

Figure 28 (continued)

**SEQ ID NO: 145, NM_001055712.1| Oryza sativa (japonica cultivar-group) Os03g0183000 (Os03g0183000) mRNA, complete cds**

AAAGGCATTCGCAACACACACTTGAAGAAAAAAAACACGACGAACACGTTAAAAAAAGGTCGAAGAGA
AGTGGGAGACCCAAACCGCGAACAATGTGTGGAGGCGCCATCCTCGCCGAGTTCATCCCGGCGCCGTC
GCGCGCCGCGGCGGCGACCAAGCGGGTGACCGCCAGCCACCTGTGGCCGGCCGGCTCCAAGAACGCCG
CCCGCGGCAAGAGCAAGAGCAAGAGGCAGCAGAGGAGCTTCGCCGACGTCGACGACTTCGAGGCCGCC
TTCGAGCAGTTCGACGATGACTCCGACTTCGACGACGCGGAGGAAGAAGACGAAGGACACTTCGTGTT
CGCGTCCAAATCTCGTGTCGTCGCCGGGCACGACGGGCGCGCGGCGGCGAGGGCGGCGAGCAAGAAGA
AGCGGGGGCGGCACTTCCGAGGCATCCGGCAGCGGCCATGGGGGAAGTGGGCGGCGGAGATCCGCGAC
CCGCACAAGGGCACGCGCGTCTGGCTCGGCACGTTCAACACCCCGGAGGAGGCCGCACGCGCCTACGA
CGTCGAGGCGCGCCGCCTCCGCGGCAGCAAGGCCAAGGTCAACTTCCCCGCCACGCCCGCCGCCGCGC
GCCCACGCCGCGGCAACACGAGAGCCACCGCCGTGCCACCGCCGGCGACAGCACCCGCCGCCGCCCCG
CCGCGCGGACTGAAGCGAGAATTCTCGCCGCCTGCTGAGACCGCGCTACCTTTCTTCACCAACGGCTT
CGTCGACCTGACGACCGCCGCGGCGCCGCCACCGGCCATGATGATGACGAGCTCCTTCACCGACAGCG
TCGCCACGTCGGAGTCCGGCGGGAGCCCCGCCAAGAAGGCGAGGTCCGACGACGTCGACTCGTCCGAG
GGCAGCGTCGGCGGCGGCAGCGACACGCTGGGTTTCACCGACGAGCTGGAGTTCGACCCGTTCATGCT
GTTCCAGCTCCCCTACTCCGACGGCTACGAGTCCATCGACAGCCTCTTCGCCGCCGGCGACGCCAACA
GCGCGAACACCGACATGAACGCCGGCGTCAACCTGTGGAGCTTCGACGACTTCCCAATCGACGGCGCC
CTTTTCTGATGTACTCCTCCATTGATGCAGTTTGTGCACTCAATTGTTAATCACGTCGTCGTTGATGA
ATGTTTAACCGGAGGATGATGGGGTGCAGCTGATATCATGGCTTGCTTGATTACCGAATTATATTGCG
GTGTTTGTGTTTGTCAATTAGATTCTGAATCTGTACTACTGCCGATCTTATGATCAAAACTATATATT
AAGTTTATGTACTCTTAATTTGTGGGCTACTTTTACGGGGTCTTCGTACTGCTGGTCAAATAGTCCTA
TGAAATCGATCATGTCGGCAATATCGTCGTCAATTATCGTGGTCGTGGTTGTTAATGATGTCAAATAA
GTCTATGAAAACCTGGTCCTCTTGGTGTTTATGTACCACTGATCGATCCATCATTTGATCTCTGTCAT
GTTGAGATGGATCGTGTAAGAATATCATAATTTGCTGGATTCAGTCCAGTCGTAATGTATTTTGGTTT
GTACAACTGC

**SEQ ID NO: 146, LOC_Os03g08470.1|11973.m06355|protein|AP2-EREBP**

MCGGAILAEFIPAPSRAAAATKRVTASHLWPAGSKNAARGKSKSKRQQRSFADVDDFEAAFEQFDDDS
DFDDAEEEDEGHFVFASKSRVVAGHDGRAAARAASKKKRGRHFRGIRQRPWGKWAAEIRDPHKGTRVW
LGTFNTPEEAARAYDVEARRLRGSKAKVNFPATPAAARPRRGNTRATAVPPPATAPAAAPPRGLKREF
SPPAETALPFFTNGFVDLTTAAAPPPAMMMTSSFTDSVATSESGGSPAKKARSDDVDSSEGSVGGGSD
TLGFTDELEFDPFMLFQLPYSDGYESIDSLFAAGDANSANTDMNAGVNLWSFDDFPIDGALF

**SEQ ID NO: 147, AJ515477.2| Triticum aestivum erebp gene for ethylene response element binding protein**

CTTTCCTTTCGCTTAGCTCTCCACCCGCGGCCCCACGTGCTCCCAGCCATGTGCGGCGGCGCCATCCT
CTCCGACATCATCCCGCCGCCGCGCCGGCCCGCCGGCGGCCGCCTCTGGCAGGCGGACAGGAAGAAGA
GGAGGGCCGGGCCCCGCCGCGTGCCCGAGGAGGAGCCCGAGGAGGAGGCGGAGGAGGGCGACGAGGAC
TTCGAGGCCGACTTCGAGGGGTTCGTGGACGAGGAGTCCGACGGCGAGGTCAAGCCCTTCCCCGCCCG
CAGGAGCGGCTTCTCCGGAGATGGATTGAAGGCAACTGCTGCTGGTGAATATGACTGTGCCTCAGGGT
CTGCTAAAAGGAAGAGAAAGAACCAGTTCAGGGGCATCCGCCGCCGCCCTTGGGGTAAATGGGCTGCT
GAAATAAGAGATCCTCGCAAGGGTGTCCGTGTCTGGCTTGGTACTTACAACTCC

Figure 28 (continued)

GCTGAGGAAGCTGCCAGAGCCTATGATGTTGAAGCCCGCAGAATTCGTGGCAAGAAGGCAAAGGTC
AATTTCCCAGAAGAAGCTCCTATGGCTCCTCAGCAACGCTGCGCTACTGCTGTGAAGGTGCCCGAGTT
CAACACCGAACAGAAGCCGGTACTCAACACCATGGGCAACGCAGATGTGTATTCCTGCTCTGCTGTTG
ACTACACCTTAAATCAGCAATTTGTGCAGCCTCAGAACATGTCGTTTGTGCCTACAGTGAATGCAGTT
GAGGCCCCTTTCATGAATTTTTCCTCTGACCAGGGTAGCAACTCCTTTAGTTGCTCAGACTTCAGCTG
GGAGAATGATATCAAGACCCCTGACATAACTTCTGTGCTTGCATCCATTCCCACCTCAACAGAGGTCA
ATGAATCTGCATTTCTCCAGAACAATGGCATCAATTCAACGGTACCTCCTGTGATGGGTGATGCTAAT
GTTGATCTTGCCGACTTGGAGCCATACATGAAGTTCCTGATGGACGATGGTTCAGATGAGTCAATTGA
CAGCATTCTAAGCTGTGATGTACCCCAGGATGTGGTCGGCAACATGGGCCTTTGGACCTTTGATGACA
TGCCCTTGTCTGCTGGTTTCTACTGAGGGAATCGAGGTCGCTGGGTGCCTGTATATATAGACAAAGGG
AATAAGTATTCAGGACATCAACAAGTGCTTGTGTCTGGTGCCTCTAGAATTGAGCAGTAGCGATGTCA
GTCTATGGTTATGTCTAGCTTAAATGGTCAGGTGACTGAGGTCTTTTGCAATAGACCTCTGTCTTGTG
CCCCCAGACTATACTTATATCTATATATGAGACCAGTATGTGATGGGGAACTGCTTATTTTGTATTCA
TGTTTCTACCTTATTGTAACTGCTACAAGAGGCTGTAAACCTTTTAAATTTGAAGCCAGTGTTTGTTC
TGTTGTGCTTAAAAAAAAAAAAAAAAAAAAAAGTATCTGC

**SEQ ID NO: 148, CAD56466.1| ethylene response element binding protein [Triticum aestivum]**

MCGGAILSDIIPPPRRPAGGRLWQADRKKRRAGPRRVPEEEPEEEAEEGDEDFEADFEGFVDEESDGE
VKPFPARRSGFSGDGLKATAAGEYDCASGSAKRKRKNQFRGIRRRPWGKWAAEIRDPRKGVRVWLGTY
NSAEEAARAYDVEARRIRGKKAKVNFPEEAPMAPQQRCATAVKVPEFNTEQKPVLNTMGNADVYSCSA
VDYTLNQQFVQPQNMSFVPTVNAVEAPFMNFSSDQGSNSFSCSDFSWENDIKTPDITSVLASIPTSTE
VNESAFLQNNGINSTVPPVMGDANVDLADLEPYMKFLMDDGSDESIDSILSCDVPQDVVGNMGLWTFD
DMPLSAGFY

**SEQ ID NO: 149, NM_130320.2| Arabidopsis thaliana DNA binding / transcription factor (AT2G47520) mRNA, complete cds**

GTTCTAGTTTGGAGTTGGAAGCGTAAAAAACAAAAAACAAAAATGTGTGGGGGAGCTATCATTTCTGA
TTTCATCTGGTCGAAATCTGAGTCAGAACCGAGTCAACTCGGCTCTGTTAGCAGCAGGAAGAAGCGTA
AACCCGTCTCAGTGAGTGAAGAAAGAGATGGGAAACGAGAGAGGAAGAATCTGTACAGAGGGATAAGG
CAGAGGCCATGGGGCAAATGGGCAGCGGAGATTCGTGACCCGAGCAAAGGTGTACGTGTCTGGCTTGG
CACATTCAAAACCGCCGACGAAGCTGCTCGAGCCTACGACGTTGCTGCCATCAAAATCCGTGGCCGGA
AAGCCAAACTGAATTTCCCAAACACTCAAGTAGAAGAAGAAGCCGATACTAAACCAGGGGGGAATCAA
AATGAGCTGATTTCGGAAAACCAAGTAGAGAGCTTATCGGAGGACCTGATGGCATTGGAGGATTACAT
GAGATTCTATCAGATTCCGGTTGCCGACGACCAATCGGCGACCGATATTGGAAATTTATGGAGCTATC
AAGACTCCAATTAAATCTCTTATTTCCCGGCCGGTTTGCTCACTCATTAATATGCTGC

**SEQ ID NO: 150, AT2G47520**

MCGGAIISDFIWSKSESEPSQLGSVSSRKKRKPVSVSEERDGKRERKNLYRGIRQRPWGKWAAEIRDP
SKGVRVWLGTFKTADEAARAYDVAAIKIRGRKAKLNFPNTQVEEEADTKPGGNQNELISENQVESLSE
DLMALEDYMRFYQIPVADDQSATDIGNLWSYQDSN

Figure 28 (continued)

**SEQ ID NO: 151, NM_001054854.1| Oryza sativa (japonica cultivar-group) Os02g0782700 (Os02g0782700) mRNA, complete cds**

TCTCTCCAATATTTTGTCGAACCGATTGAGGGAAAAAAAAAGAGAAAAAGAAAAGAAAAAAAAGAAGA
AGAAGAGAGAACTCGAACTCTCGCCTACCATTTCGCCCCCCTCCGCAAGCAATCCACCACTGCATCGG
CGGCGAGGGCTCACCGGCGGCGAGCCATGTGCGGCGGCGCCATCATCCACCACCTGAAGGGGCACCCG
GAGGGGTCGCGCCGGGCGACGGAGGGGCTCCTGTGGCCCGAGAAGAAGAAGCCCAGGTGGGGCGGCGG
CGGGAGGCGCCACTTCGGGGGGTTCGTGGAGGAGGACGACGAGGACTTCGAGGCCGACTTCGAGGAGT
TCGAGGTGGACTCCGGGGACTCGGATTTGGAGCTCGGGGAGGAGGACGACGATGACGTCGTCGAGATC
AAGCCGGCCGCCTTCAAGAGGGCCCTCTCCAGAGATAACTTGAGCACCATTACCACTGCCGGATTTGA
TGGTCCTGCTGCAAAGTCTGCCAAAAGAAAGAGAAAGAACCAATTCAGGGGCATCCGCCAGCGCCCTT
GGGGTAAGTGGGCTGCTGAAATCAGAGATCCTCGCAAGGGTGTTCGTGTCTGGCTTGGCACTTTCAAC
AGTGCTGAAGAAGCTGCAAGAGCTTATGATGCTGAAGCACGCAGGATTCGTGGCAAGAAGGCCAAGGT
GAATTTTCCAGAGGCTCCAACAACTGCTCAGAAGCGTCGTGCTGGCTCCACCACTGCTAAAGCACCCA
AGTCAAGTGTGGAACAGAAGCCTACTGTCAAACCAGCATTCAACAATCTTGCCAATGCAAATGCGTTT
GTCTACCCATCTGCTAACTTCACTTCAAACAAGCCGTTTGTTCAGCCTGATAACATGCCATTTGTTCC
TGCAATGAACTCTGCTGCTCCTATTGAGGACCCTATCATCAACTCTGACCAGGGAAGCAACTCATTTG
GCTGCTCTGACTTTGGCTGGGAGAATGATACCAAGACACCAGATATTACATCAATTGCTCCCATTTCA
ACCATAGCTGAAGTCGATGAATCTGCATTCATTAAGAGCAGTACCAACCCAATGGTCCCTCCTGTTAT
GGAGAACAGTGCTGTTGATCTGCCTGATTTAGAACCCTACATGAGGTTCCTTCTGGATGATGGTGCTG
GTGACTCAATTGATAGCCTTCTCAACCTGGATGGATCACAGGATGTTGTCAGCAACATGGACCTCTGG
AGCTTTGATGACATGCCCGTTAGCGATTTCTATTGAGGAATTCGAAGTCTTCTAGTCGGAGCATGTAC
ACAGGGAAAAAAAAGGAATAAATACTATTGGAGATTGGGAGGCACCTGCATGGCACCTTGGGGGTAGC
ATATCGTTATGTTTAGCTTAGATGCAAAAGGCTGCATCCTGAAACTCTTTGGTGATTGGACCTGTTCC
CTATCCGCTGTCTATGTATGGCAGCCATCTATGAGACTCAAGAACTGCTTTTTTTTTTCCGTTCTAGT
TTTCTGTCGTTGCTACCTGTATATGGCTATGAACATCGTGAATCCATGGCCATTATGTTTTAATCTAT
GTTGTTGACTGCTCTTTCTTTCGGTCTTTGCTGTGCTGCGCTATGTTGGTGATAACTAGCTACCATAT
TGATTTTCTGTACATAATTCATTTGTTGAATCCGAAATTAAATAGTGCATTGAC

**SEQ ID NO: 152, LOC_Os02g54160.1|11972.m10445|protein|AP2-EREBP**

MCGGAIIHHLKGHPEGSRRATEGLLWPEKKKPRWGGGGRRHFGGFVEEDDEDFEADFEEFEVDSGDSD
LELGEEDDDDVVEIKPAAFKRALSRDNLSTITTAGFDGPAAKSAKRKRKNQFRGIRQRPWGKWAAEIR
DPRKGVRVWLGTFNSAEEAARAYDAEARRIRGKKAKVNFPEAPTTAQKRRAGSTTAKAPKSSVEQKPT
VKPAFNNLANANAFVYPSANFTSNKPFVQPDNMPFVPAMNSAAPIEDPIINSDQGSNSFGCSDFGWEN
DTKTPDITSIAPISTIAEVDESAFIKSSTNPMVPPVMENSAVDLPDLEPYMRFLLDDGAGDSIDSLLN
LDGSQDVVSNMDLWSFDDMPVSDFY

**SEQ ID NO: 153, AY831392.1| Oryza sativa (indica cultivar-group) BTH-induced ERF transcriptional factor 1 (BIERF1) mRNA, complete cds**

CTGTAGCTTATTAGCGGCCATGTGCGGCGGAGCAATCATCTCCGGGTTCATCCCGCCGTCGGCCGCTG
CGGCGGCGGCGGCGGCGGCGGCGGCGGCCAAGAAGCAGCAGGGCAGGAGGGTCACGGCCGACGTGCTG
TGGCCGGGGATGCTGCGGAAGGGGAAGGCGGGGGCGGCGGAGGAGGACTTTGAGGCCGACTTCCGCGA
GTTCGAGCGTGGCATGAGCGACGACGAGGCGGAGGGGGGCGGCGGCGAGGAGGAGGAGGAGGAGGAGG
ACGACGTGGTCGTGGAGGTCCCCCCGCCGGCGACGGCGAGGTTCGTCGTCCGTGCCGC

Figure 28 (continued)

GGCCAAGGCGGCGCCCCCAACTGCAGATGGGATGTTGACTACAAAGCTTGTCCAACATGATGGACCTA
CTGCTAGATCGGCAAAGCGCAAGAGGAAGAATCAGTACAGGGGGATCCGCCAGCGTCCCTGGGGCAAA
TGGGCAGCTGAAATCCGAGACCCCAGCAAGGGTGTCCGTGTTTGGCTTGGAACATATAACACTGCTGA
GGAGGCAGCTAGGGCATATGACGCTGAAGCCCGCAAGATCCGTGGCAAGAAAGCCAAGGTCAACTTTC
CTGATGAACCAGCTGTTGCTCAGAAGCTCTCCCTGAAGCAAAACGCTGCCAAGCAAGAGAAACTAGCT
CCACCTCTGAAGTCCTGTGGCGATGATGCTTTCTTTCAGCTAAACAGTTCAGACAATGATTTGTTTGC
AATGCTTGCAAAGGTGCCTGCAAAGCCGGCAGAGCCTGTTGATCTCATGCCTCCAGTCAAACCTCTTG
CTTCCACTGAGACATTCGAGATGAACATGCTCTCTGATACGAGCAGCAACTCATTTGGCTCTTCAGAC
TTTGGTTGGGAGGATGACACCCTGACCCCAGACTACACTTCAGTCTTTGTTCCTAATGCTGCCATGCC
AGCATATGGTGAACCTGCTTACCTGACAGGTGGAGCGCCAAAGAGAATGAGGAACAACTATGGTATCG
CCGTGCCCCAGGGAAATGGCATGCCTAATCTCGCACAAAACATGCCCACCTTCGATCCCGAGATGAAG
TATTTGCCATTACCTTATGTTGAGAGCAGCTCAGATGAATCAATGGACAACCTTCTGCAAAATGATGC
TACACAAGACGGGGCAAGCAACGAGGGCATCTGGAGCCTTGATGAGCTGCTCATGGCAGCTGGTGCCT
ACTGAGGAGACGAAATGTTCTGGTCAGTGTGGTCTGTCACTAGCAAACCATGTAAGGCACTCCACACT
ACCTACTATTTTGATTTCTTGTAGATACATTTTCATGTTTGAATGTGTATGGCCAAGATGAAGAGCTG
GTGATGTCTGCTATGTTTTGTAGAGGGATGCTACCAAAGTAATGCTAGAATATTACAAGCTGCTATCA
GCTGTACTCTCTATGACAATGTTTATACTATGTTTGCTGTATGGTGTGGTCTATGATTTGAAGTATGT
CGGAGACTAATTCAAATAATGCCCTTGGGCCATGGTGCTGTGCCTTGGTAAATGGTGCTTTATTTAAG
GGTTATATTAGGTTGGTGCCTCAGTAATTGCCGTTTTCTACCAAAAAAAAAAAAAAAAA

**SEQ ID NO: 154, LOC_Os09g26420.1|11979.m05794|protein|AP2-EREBP**

MCGGAIISGFIPPSAAAAAAAAVAKKQQGRRVTADVLWPGMLRKGKAAAAEEDFEADFREFERGMSDD
EAEGGGGEEEEDDDDVVVVVPPPAAARFVVRAAAKAAPPTADGMLTTKLVQHDGPTARSAKHKRKNQY
RGIRQRPWGKWAAEIRDPSKGVRVWLGTYNTAEEAARAYDAEARKIRGKKAKVNFPDEPAVAQKLSLK
QNAAKQEKLAPPLKTCGDDAFFQLNSSDNDLFAMLAKVPAKPAEPVDLMPPVKPLASTETFEMNMLSD
TSSNSFGSSDFGWEDDTLTPDYTSVFVPNAAMPAYGEPAYLTGGAPKRMRNNYGIAVPQGNGMPNLAQ
NMPTFDPEMKYLPLPYVESSSDESMDNLLQNDATQDGASNEGIWSLDELLMAAGAY

**SEQ ID NO: 155, AY271985.1| Triticum aestivum ethylene-responsive element binding protein (TaERF2) mRNA, complete cds**

ATGTGCGGCGGCGCCATCCTCTCCGACATCATCCCGCCGCCGCGCCGGGCCACCGGCGGCAACGTCTG
GCGGGCGGACAAGAAGAGGAGGGCCAGGCCCGACGCCGCCGCGGGGAGGCCCCGCCGCGTGCCCGAGG
AGGAGTTCCAGGAGGAGGAGAGCGACGCGGAGTTCGAGGCCGACTTCGAGGGGTTCGTGGAGGCGGAG
GAGGAGTCCGACGGCGAGGCCAAGCCCTTCCCCGTCCGCAGGAGCGGCTTCTCCGGAGATGGATTGAA
GGCAACTGCTGCTGGTGATGATGACTGTGCTTCAGGGTCTGCTAAAAGGAAGAGAAAGAACCAGTTCA
GGGGCATCCGCCGCCGCCCTTGGGGTAAATGGGCTGCTGAAATAAGAGATCCTCGCAAGGGTGTCCGT
GCCTGGCTTGGCACTTACAACTCTGCTGAGGAAGCTGTCAGAGCCTATGATGTTGAAGCCCGCAGAAT
TCGTGGCAAGAAGGCAAAGTCAATTTCCCAGAAGAAGCTCCCATGGCTCCTCAGCAAACGCTGCGCTA
CCTCTGTGAAGGTGCCCGAGTTCAACACCGAACAGAAGCCAGTACTCAACACCATGGGCAACGCAGAT
GTGTATTCCTGCCCTGCTGTTGACTACACCTTAAATCAGCAATTTGTGCAGCCTCAGAACATGTCGTT
TGTGCCTACAGTGAATGCAGTTGAGGCTCCTTTCATGAATTTTTCCTCTGACCAGGGGAGCAACTCCT
TTAGTTGCTCAGACTTCAGCTGGGAGAATGATATCAAGACCCCTGACATAACTTCTGTGCTTGCATCC
ATTCCCACCTCAACTGAGGTCAATGAATCTGCATTTCTCCAGAACAATGGCATTAATTCAACGGTACC
TCCTGTGATGGGTGATGCTAATGTTGATCTTGCCTACTTG

Figure 28 (continued)

GAGCCGTACATGAAGTTCCTGATGGACGATGGTTCAGATGAGTCAATTGACAGCATTCTAAGCTGTGA
TGTACCGCAGGACGTTGTCGGCAACATGGGCCTTTGGACCTTTGATGACATGCCCTTGTCTGCTGGTT
TCTACTGA

**SEQ ID NO: 156, AAP32468.1| ethylene-responsive element binding protein [Triticum aestivum]**

MCGGAILSDIIPPPRRATGGNVWRADKKRRARPDAAAGRPRRVPEEEFQEEESDAEFEADFEGFVEAE
EESDGEAKPFPVRRSGFSGDGLKATAAGDDDCASGSAKRKRKNQFRGIRRRPWGKWAAEIRDPRKGVR
AWLGTYNSAEEAVRAYDVEARRIRGKKAKSISQKKLPWLLSKRCATSVKVPEFNTEQKPVLNTMGNAD
VYSCPAVDYTLNQQFVQPQNMSFVPTVNAVEAPFMNFSSDQGSNSFSCSDFSWENDIKTPDITSVLAS
IPTSTEVNESAFLQNNGINSTVPPVMGDANVDLAYLEPYMKFLMDDGSDESIDSILSCDVPQDVVGNM
GLWTFDDMPLSAGFY

**SEQ ID NO: 157, AY271984.1| Triticum aestivum ethylene-responsive element binding protein (TaERF1) mRNA, complete cds**

ATGTGCGGCGGGCGCCATCCTCTCCGACATCATCCCGCCGCGGGGAGGCCCTGCCGTGCGCCTGAGGA
GGAGTTCCAGGAGGAGGAGGGCGACGCGGAGTTCGAGGCCGACTTCGAGGGGTTCGTGGAGGCGGAGG
AGGAGTCCGACGGCGAGGCCAAGCCCTTCCCCGTCCGCAGGAGCGGCTTCTCCGGAGATGGATTGAAG
GCAACTGCTGCTGGTGATGATGACTGTGCCTCAGGGTCTGCTAAAAGGAAGAGAAAGAACCAGTTCAG
GGGCGTCCGCCGCCGCCCTTGGGGTAAATGGGCTGCTGAAATAAGAGATCCTCGCAAGGGTGTCCGTG
TCTGGCTTGGTACTTACAACTCCGCTGAGGAAGCTGCCAGAGCCTATGGTGTTGAGGCCCGCAGAATT
CGTGGCAAGAAGGCAAAGGTCAATTTCCCAGAAGAAGCTCCTATGGCTCCTCAGCAACGCTGCGCTAC
TGCTGTGAAGGTGCCCGAGTTCAACACCGAACAGAAGCCGGTACTCAACACCATGGGCAACGCAGATG
TGTATTCCTGCTCTGCTGTTGACTACACCTTAAATCAGCAATTTGTGCAGCCTCAGAACATGTCGTTT
GTGCCTACAGTGAATGCAGTTGAGGCCCCTTTCATGAATTTTTCCTCTGACCAGGGTAGCAACTCCTT
TAGTTGCTCAGACCTCAGCTGGGAGAATGATATCAAGACCCCTGACATAACTTCTGTGCTTGCATCCA
TTCCCACCTCAACAGAGGTCAATGAATCTGCATTTCTCCAGAACAATGGCATCAATTCAACGGTACCT
CCTGTGATGGGTGATGCTAATGTTGATCTTGCCGACTTGGAGCCATACATGAAGTTCCTGATGGACGA
TGGTTCAGATGAGTCAATTGACAGCATTCTAAGCTGTGATGTACCCCAGGATGTGGTCGGCAACATGG
GCCTTTGGACCTTTGATGACATGCCCTTGTCTGCTGGTTTCTACTGA

**SEQ ID NO: 158, AAP32467.1| ethylene-responsive element binding protein [Triticum aestivum]**

MCGGRHPLRHHPAAGRPCRAPEEEFQEEEGDAEFEADFEGFVEAEEESDGEAKPFPVRRSGFSGDGLK
ATAAGDDDCASGSAKRKRKNQFRGVRRRPWGKWAAEIRDPRKGVRVWLGTYNSAEEAARAYGVEARRI
RGKKAKVNFPEEAPMAPQQRCATAVKVPEFNTEQKPVLNTMGNADVYSCSAVDYTLNQQFVQPQNMSF
VPTVNAVEAPFMNFSSDQGSNSFSCSDLSWENDIKTPDITSVLASIPTSTEVNESAFLQNNGINSTVP
PVMGDANVDLADLEPYMKFLMDDGSDESIDSILSCDVPQDVVGNMGLWTFDDMPLSAGFY

Figure 28 (continued)

**SEQ ID NO: 159, NM_001036108.1| Arabidopsis thaliana RAP2.12; DNA binding / transcription factor (RAP2.12) mRNA, complete cds**

AAAACAACAAAGCAAAGCGTTGAAGAGAGAAGAAGAAGCAAAGATATAACCCCCAAAAGTATCAATTA
GTTTCCATTTTCGCCGCTAAGATTCTGTTTTCGAACATTTACACCCTCAAGAATCGCCGCCATGTGTG
GAGGAGCTATAATATCCGATTTCATTCCACCGCCGAGGTCTCGCCGTGTTACTAGCGAGTTTATTTGG
CCGGATCTGAAGAAGAATTTGAAAGGATCGAAGAAAAGCTCGAAGAATCGTTCGAATTTCTTCGATTT
TGACGCTGAGTTCGAAGCTGATTTCCAAGGTTTCAAAGATGATTCGTCTATCGATTGCGATGATGATT
TCGACGTCGGTGATGTTTTCGCCGATGTGAAACCATTCGTTTTCACTTCGACTCCAAAACCCGCCGTC
TCCGCCGCTGCGGAAGGTTCAGTTTTTGGTAAGAAAGTTACTGGCTTGGATGGGGACGCTGAGAAATC
TGCAAATAGGAAGAGGAAGAATCAGTACCGAGGGATTAGGCAACGTCCTTGGGGAAAATGGGCTGCTG
AGATACGTGATCCAAGGGAAGGTGCTAGAATCTGGCTTGGAACGTTCAAGACAGCTGAGGAAGCTGCT
AGAGCTTACGATGCTGCAGCGCGGAGAATCCGTGGATCTAAAGCTAAGGTGAATTTCCCTGAAGAAAA
CATGAAGGCTAATTCTCAGAAACGCTCTGTGAAGGCTAATCTTCAGAAACCAGTGGCTAAACCTAACC
CTAACCCAAGTCCAGCTTTGGTTCAGAACTCGAACATCTCCTTTGAAAATATGTGTTTCATGGAGGAG
AAACACCAAGTGAGCAACAACAACAACAACCAGTTTGGGATGACAAACTCCGTTGATGCTGGATGTAA
TGGGTATCAGTATTTCAGCTCTGACCAGGGTAGTAATTCTTTCGATTGTTCGGAGTTTGGTTGGAGCG
ATCAAGCTCCGATAACTCCCGACATCTCTTCTGCGGTTATCAACAACAACAACTCAGCTCTGTTCTTT
GAGGAAGCCAATCCAGCTAAGAAGCTCAAGTCTATGGATTTCGAGACACCTTACAACAACACTGAATG
GGACGCTTCACTGGATTTCCTCAACGAAGATGCTGTAACGACTCAGGACAATGGTGCAAACCCTATGG
ACCTATGGAGTATTGATGAAATTCATTCCATGATTGGAGGAGTCTTCTGAAGAGATCCAGTTTCATGA
GTTCCTGTTTGCATTGCGAGAAGCCATGAGCCTCTATCTTGAGGGTAGTTGTGATGAAGTTAAGTAGA
GGCTTATTTTTAGGGGTTGTGGTAGTTTTTGTTTTAGTGAATCTTTTGAATTCGTTTGTGTTTTGTTT
TTGTTACTTTATGCCCCAAAACTCCTTTAACATTTGTCATAATGTGTTTGAACCTCTCATCTGTTTAA
TCAAATAAATCTTCTTTGTATGCT

**SEQ ID NO: 160, AT1G53910.1 Arabidopsis AP2-EREBP family transcription factor, protein sequence 358AA**

MCGGAIISDFIPPPRSRRVTSEFIWPDLKKNLKGSKKSSKNRSNFFDFDAEFEADFQGFKDDSSIDCD
DDFDVGDVFADVKPFVFTSTPKPAVSAAAEGSVFGKKVTGLDGDAEKSANRKRKNQYRGIRQRPWGKW
AAEIRDPREGARIWLGTFKTAEEAARAYDAAARRIRGSKAKVNFPEENMKANSQKRSVKANLQKPVAK
PNPNPSPALVQNSNISFENMCFMEEKHQVSNNNNNQFGMTNSVDAGCNGYQYFSSDQGSNSFDCSEFG
WSDQAPITPDISSAVINNNNSALFFEEANPAKKLKSMDFETPYNNTEWDASLDFLNEDAVTTQDNGAN
PMDLWSIDEIHSMIGGVF

**SEQ ID NO: 161, AY088204.1| Arabidopsis thaliana clone 42960 mRNA, complete sequence**

AAAAACAACAAAGCAAAGCGTTGAAGAGAGAAGAAGAAGCAAAGATATAACCCCCAAAAGTATCAATT
AGTTTCCATTTTCGCCGCTAAGATTCTGTTTTCGAACATTTACACCCTCAAGAATCGCCGCCATGTGT
GGAGGAGCTATAATATCCGATTTCATTCCACCGCCGAGGTCTCGCCGTGTTACTAGCGAGTTTATTTG
GCCGGATCTGAAGAAGAATTTGAAAGGATCGAAGAAAAGCTCGAAGAATCGTTCGAATTTCTTCGATT
TTGACGCTGAGTTCGAAGCTGATTTCCAAGGTTTCAAAGATGATTCGTCTATCGATTGCGATGATGAT
TTCGACGTCGGTGATGTTTTCGCCGATGTGAAACCATTCGTTTTCACTTCGACTCCAAAACCCGCCGT
CTCCGCCGCTGCGGAAGGTTCAGTTTTTGGTAAGAAAGTTACTGGCTTGGATGGGGAAGCTGAGAAAT
CTGCAAATAGGAAGAGGAAGAATCAGTACCGAGGGATTAGGCAACGTCCTTG

Figure 28 (continued)

GGGAAAATGGGCTGCTGAGATACGTGATCCAAGGGAAGGTGCTAGAATCTGGCTTGGAACGTTCAAGA
CAGCTGAGGAAGCTGCTAGAGCTTACGATGCTGCAGCGCGGAGAATCCGTGGATCTAAAGCTAAGGTG
AATTTCCCTGAAGAAAACCTGAAGGCTAATTCTCAGAAACGCTCTGTGAAGGCTAATCTTCAGAAACC
AGTGGCTAAACCTAACCCTAACCCAAGTCCAGCTTTGGTTCAGAACTCGAACATCTCCTTTGAAAATA
TGTGTTTCATGGAGGAGAAACACCAAGTGAGCAACAACAACAACAACCAGTTTGGGATGACAAACTCC
GTTGATGCTGGATGTAATGGGTATCAGTATTTCAGCTCTGACCAGGGTAGTAATTCTTTCGATTGTTC
GGAGTTTGGTTGGAGCGATCAAGCTCCGATAACTCCCGACATCTCTTCTGCGGTTATCAACAACAACA
ACTCAGCTCTGTTCTTTGAGGAAGCCAATCCAGCTAAGAAGCTCAAGTCTATGGATTTCGAGACACCT
TACAACAACACTGAATGGGACGCTTCACTGGATTTCCTCAACGAAGATGCTGTAACGACTCAGGACAA
TGGTGCAAACCCTATGGACCTATGGAGTATTGATGAAATTCATTCCATGATTGGAGGAGTCTTCTGAA
GAGATCCAGTTTCATGTAAATAAGGCTGCATGTTTGTGAGTTTCCCGCATCGTTCGTTTATCAACCTC
CAAAACTTTCTAATGTCTGTTACTTGCATCTTCTTCTGCTGTCTCTGTCTGTCTCTCTCAGGAGTTCC
TGTTTGCATTGCGAGAAGCCATGAGCCTCTATCTTGAGGGTAGTTGTGATGAAGTTAAGTAGAGGCTT
ATTTTTAGGGGTTGTGGTAGTTTTTGTTTTAGTGAATCTTTTGAATTCGTTTGTGTTTTGTTTTTGTT
ACTTTATGCCCCAAAACTCCTTTAACATTTGTCATAATGTGTTTGAACCTCCTCATCTGTTTAATCAA
ATAAATCTTCTTTGTATGCT

**SEQ ID NO: 162, AAM65746.1| AP2 domain containing protein, putative [Arabidopsis thaliana] RAP2;12**

MCGGAIISDFIPPPRSRRVTSEFIWPDLKKNLKGSKKSSKNRSNFFDFDAEFEADFQGFKDDSSIDCD
DDFDVGDVFADVKPFVFTSTPKPAVSAAAEGSVFGKKVTGLDGEAEKSANRKRKNQYRGIRQRPWGKW
AAEIRDPREGARIWLGTFKTAEEAARAYDAAARRIRGSKAKVNFPEENLKANSQKRSVKANLQKPVAK
PNPNPSPALVQNSNISFENMCFMEEKHQVSNNNNNQFGMTNSVDAGCNGYQYFSSDQGSNSFDCSEFG
WSDQAPITPDISSAVINNNNSALFFEEANPAKKLKSMDFETPYNNTEWDASLDFLNEDAVTTQDNGAN
PMDLWSIDEIHSMIGGVF

**SEQ ID NO: 163, NM_112281.2| Arabidopsis thaliana RAP2.2; DNA binding / transcription factor (RAP2.2) mRNA, complete cds**

GGAGAGTAACATCGAGACAAAGAAGAAAAACTAAAAAAGAGAACCCCAAAGAATCGAATATTTATTAT
TTCGCCCCGAAGATTCTATTTCTGATCATTTACACCCCTAAAAAGAGTAGAGCTTTCGTGAAGCCACC
ATGTGTGGAGGAGCTATAATCTCCGATTTCATACCTCCGCCGAGGTCCCTCCGCGTCACTAACGAGTT
TATCTGGCCGGATCTGAAAAACAAAGTGAAAGCTTCAAAGAAGAGATCGAATAAGCGATCCGATTTCT
TCGATCTTGACGATGATTTCGAAGCTGATTTCCAAGGGTTTAAGGATGACTCGGCTTTTGACTGCGAA
GACGATGATGATGTCTTCGTCAATGTTAAGCCTTTCGTCTTCACCGCAACTACTAAGCCCGTAGCTTC
CGCTTTCGTCTCCACTGGTATATATTTGGTAGGTTCAGCATATGCCAAGAAAACTGTAGAGTCCGCTG
AGCAAGCTGAGAAATCTTCTAAGAGGAAGAGGAAGAATCAATACCGAGGGATTAGGCAGCGTCCTTGG
GGAAAATGGGCTGCGGAGATCCGTGATCCGAGAAAAGGCTCCCGAGAATGGCTTGGAACATTCGACAC
TGCTGAGGAAGCAGCAAGAGCTTATGATGCTGCAGCACGCAGAATCCGTGGCACGAAAGCTAAGGTGA
ATTTTCCCGAGGAGAAGAACCCTAGCGTCGTATCCCAGAAACGTCCTAGTGCTAAGACTAATAATCTT
CAGAAATCAGTGGCTAAACCAAACAAAAGCGTAACTTTGGTTCAGCAGCCAACACATCTGAGTCAGCA
GTACTGCAACAACTCCTTTGACAACTCTTTTGGTGATATGAGTTTCATGGAAGAGAAGCCTCAGATGT
ACAACAATCAGTTTGGGTTAACAAACTCGTTCGATGCTGGAGGTAACAATGGATACCAGTATTTCAGT
TCCGATCAGGGCAGTAACTCCTTCGACTGTTCTGAGTTCGGGTGGAGTGATCACGGCCCTAAAACACC
CGAGATCTCTTCAATGCTTGTCAATAACAACGAAGCATCATTTGTTGAAGAAACCAATGCAGCCAAGA
AGCTCAAACCAAACTCTGATGAGTCAGACGATCT

Figure 28 (continued)

GATGGCATACCTTGACAACGCCTTGTGGGACACCCCACTAGAAGTGGAAGCCATGCTTGGCGCAGATG
CTGGTGCTGTGACTCAGGAAGAGGAAAACCCAGTGGAGCTATGGAGCTTAGATGAGATCAATTTCATG
CTGGAAGGAGACTTTTGAAGTGATCGATGGTTCCTTAGTTTGTAAATAAAGCTGTGTTGGATTTTGCT
GTTGGGGGATGGTACAAGTCACACCTCAAGCTCTATGCATTGGTATCTCATGAGCCTCTCTTCCATAG
AGAGTTTCTCTTTTAATTTTGTCGAAATAAAAAAGGTGTGATGAAGTAAATAGAGGTATAATAATATC
TATCTATTAAGTCTTGTTTTGTTCTTTCATTTTTGTATTTCTTTTCTATTTAAAAGACAGTTTATTAG
TCTTCTGAGCTCTCTTTTTGATCTTTGTTATAGCGTATCATCACCCTCGAAAGTGTAATGTTTTGTAC
CCCCAAACTTGTTTAGCATTATAATAAAGTCTCTTTGGAACTTCTC

**SEQ ID NO: 164, AT3G14230.1 Arabidopsis AP2-EREBP family transcription factor, protein sequence 379AA**

MCGGAIISDFIPPPRSLRVTNEFIWPDLKNKVKASKKRSNKRSDFFDLDDDFEADFQGFKDDSAFDCE
DDDDVFVNVKPFVFTATTKPVASAFVSTGIYLVGSAYAKKTVESAEQAEKSSKRKRKNQYRGIRQRPW
GKWAAEIRDPRKGSREWLGTFDTAEEAARAYDAAARRIRGTKAKVNFPEEKNPSVVSQKRPSAKTNNL
QKSVAKPNKSVTLVQQPTHLSQQYCNNSFDNSFGDMSFMEEKPQMYNNQFGLTNSFDAGGNNGYQYFS
SDQGSNSFDCSEFGWSDHGPKTPEISSMLVNNNEASFVEETNAAKKLKPNSDESDDLMAYLDNALWDT
PLEVEAMLGADAGAVTQEEENPVELWSLDEINFMLEGDF

**SEQ ID NO: 165, NM_180251.1| Arabidopsis thaliana RAP2.2; DNA binding / transcription factor (RAP2.2) mRNA, complete cds**

GGAGAGTAACATCGAGACAAAGAAGAAAAACTAAAAAAGAGAACCCCAAAGAATCGAATATTTATTAT
TTCGCCCCGAAGATTCTATTTCTGATCATTTACACCCCTAAAAAGAGTAGAGCTTTCGTGAAGCCACC
ATGTGTGGAGGAGCTATAATCTCCGATTTCATACCTCCGCCGAGGTCCCTCCGCGTCACTAACGAGTT
TATCTGGCCGGATCTGAAAAACAAAGTGAAAGCTTCAAAGAAGAGATCGAATAAGCGATCCGATTTCT
TCGATCTTGACGATGATTTCGAAGCTGATTTCCAAGGGTTTAAGGATGACTCGGCTTTTGACTGCGAA
GACGATGATGATGTCTTCGTCAATGTTAAGCCTTTCGTCTTCACCGCAACTACTAAGCCCGTAGCTTC
CGCTTTCGTCTCCACTGTAGGTTCAGCATATGCCAAGAAAACTGTAGAGTCCGCTGAGCAAGCTGAGA
AATCTTCTAAGAGGAAGAGGAAGAATCAATACCGAGGGATTAGGCAGCGTCCTTGGGGAAAATGGGCT
GCGGAGATCCGTGATCCGAGAAAAGGCTCCCGAGAATGGCTTGGAACATTCGACACTGCTGAGGAAGC
AGCAAGAGCTTATGATGCTGCAGCACGCAGAATCCGTGGCACGAAAGCTAAGGTGAATTTTCCCGAGG
AGAAGAACCCTAGCGTCGTATCCCAGAAACGTCCTAGTGCTAAGACTAATAATCTTCAGAAATCAGTG
GCTAAACCAAACAAAAGCGTAACTTTGGTTCAGCAGCCAACACATCTGAGTCAGCAGTACTGCAACAA
CTCCTTTGACAACTCTTTTGGTGATATGAGTTTCATGGAAGAGAAGCCTCAGATGTACAACAATCAGT
TTGGGTTAACAAACTCGTTCGATGCTGGAGGTAACAATGGATACCAGTATTTCAGTTCCGATCAGGGC
AGTAACTCCTTCGACTGTTCTGAGTTCGGGTGGAGTGATCACGGCCCTAAAACACCCGAGATCTCTTC
AATGCTTGTCAATAACAACGAAGCATCATTTGTTGAAGAAACCAATGCAGCCAAGAAGCTCAAACCAA
ACTCTGATGAGTCAGACGATCTGATGGCATACCTTGACAACGCCTTGTGGGACACCCCACTAGAAGTG
GAAGCCATGCTTGGCGCAGATGCTGGTGCTGTGACTCAGGAAGAGGAAAACCCAGTGGAGCTATGGAG
CTTAGATGAGATCAATTTCATGCTGGAAGGAGACTTTTGAAGTGATCGATGGTTCCTTAGTTTGTAAA
TAAAGCTGTGTTGGATTTTGCTGTTGGGGGATGGTACAAGTCACACCTCAAGCTCTATGCATTGGTAT
CTCATGAGCCTCTCTTCCATAGAGAGTTTCTCTTTTAATTTTGTCGAAATAAAAAAGGTGTGATGAAG
TAAATAGAGGTATAATAATATCTATCTATTAAGTCTTGTTTTGTTCTTTCATTTTTGTATTTCTTTTC
TATTTAAAAGACAGTTTATTAGTCTTCTGAGCTCTCTTTTTGATCTTTGTTATAGCGTATCATCACCC
TCGAAAGTGTAATGTTTTGTACCCCCAAACTTGTTTAGCATTATAATAAAGTCTCTTTGGAACTTCTC

Figure 28 (continued)

**SEQ ID NO: 166, AT3G14230.2 Arabidopsis AP2-EREBP family transcription factor, protein sequence 375AA**

MCGGAIISDFIPPPRSLRVTNEFIWPDLKNKVKASKKRSNKRSDFFDLDDDFEADFQGFKDDSAFDCE
DDDDVFVNVKPFVFTATTKPVASAFVSTVGSAYAKKTVESAEQAEKSSKRKRKNQYRGIRQRPWGKWA
AEIRDPRKGSREWLGTFDTAEEAARAYDAAARRIRGTKAKVNFPEEKNPSVVSQKRPSAKTNNLQKSV
AKPNKSVTLVQQPTHLSQQYCNNSFDNSFGDMSFMEEKPQMYNNQFGLTNSFDAGGNNGYQYFSSDQG
SNSFDCSEFGWSDHGPKTPEISSMLVNNNEASFVEETNAAKKLKPNSDESDDLMAYLDNALWDTPLEV
EAMLGADAGAVTQEEENPVELWSLDEINFMLEGDF

**SEQ ID NO: 167, NM_180252.1| Arabidopsis thaliana RAP2.2; DNA binding / transcription factor (RAP2.2) mRNA, complete cds**

GGAGAGTAACATCGAGACAAAGAAGAAAAACTAAAAAAGAGAACCCCAAAGAATCGAATATTTATTAT
TTCGCCCCGAAGATTCTATTTCTGATCATTTACACCCCTAAAAAGAGTAGAGCTTTCGTGAAGCCACC
ATGTGTGGAGGAGCTATAATCTCCGATTTCATACCTCCGCCGAGGTCCCTCCGCGTCACTAACGAGTT
TATCTGGCCGGATCTGAAAAACAAAGTGAAAGCTTCAAAGAAGAGATCGAATAAGCGATCCGATTTCT
TCGATCTTGACGATGATTTCGAAGCTGATTTCCAAGGGTTTAAGGATGACTCGGCTTTTGACTGCGAA
GACGATGATGATGTCTTCGTCAATGTTAAGCCTTTCGTCTTCACCGCAACTACTAAGCCCGTAGCTTC
CGCTTTCGTCTCCACTGGTTCAGCATATGCCAAGAAAACTGTAGAGTCCGCTGAGCAAGCTGAGAAAT
CTTCTAAGAGGAAGAGGAAGAATCAATACCGAGGGATTAGGCAGCGTCCTTGGGGAAAATGGGCTGCG
GAGATCCGTGATCCGAGAAAAGGCTCCCGAGAATGGCTTGGAACATTCGACACTGCTGAGGAAGCAGC
AAGAGCTTATGATGCTGCAGCACGCAGAATCCGTGGCACGAAAGCTAAGGTGAATTTTCCCGAGGAGA
AGAACCCTAGCGTCGTATCCCAGAAACGTCCTAGTGCTAAGACTAATAATCTTCAGAAATCAGTGGCT
AAACCAAACAAAAGCGTAACTTTGGTTCAGCAGCCAACACATCTGAGTCAGCAGTACTGCAACAACTC
CTTTGACAACTCTTTTGGTGATATGAGTTTCATGGAAGAGAAGCCTCAGATGTACAACAATCAGTTTG
GGTTAACAAACTCGTTCGATGCTGGAGGTAACAATGGATACCAGTATTTCAGTTCCGATCAGGGCAGT
AACTCCTTCGACTGTTCTGAGTTCGGGTGGAGTGATCACGGCCCTAAAACACCCGAGATCTCTTCAAT
GCTTGTCAATAACAACGAAGCATCATTTGTTGAAGAAACCAATGCAGCCAAGAAGCTCAAACCAAACT
CTGATGAGTCAGACGATCTGATGGCATACCTTGACAACGCCTTGTGGGACACCCCACTAGAAGTGGAA
GCCATGCTTGGCGCAGATGCTGGTGCTGTGACTCAGGAAGAGGAAAACCCAGTGGAGCTATGGAGCTT
AGATGAGATCAATTTCATGCTGGAAGGAGACTTTTGAAGTGATCGATGGTTCCTTAGTTTGTAAATAA
AGCTGTGTTGGATTTTGCTGTTGGGGGATGGTACAAGTCACACCTCAAGCTCTATGCATTGGTATCTC
ATGAGCCTCTCTTCCATAGAGAGTTTCTCTTTTAATTTTGTCGAAATAAAAAAGGTGTGATGAAGTAA
ATAGAGGTATAATAATATCTATCTATTAAGTCTTGTTTTGTTCTTTCATTTTTGTATTTCTTTTCTAT
TTAAAAGACAGTTTATTAGTCTTCTGAGCTCTCTTTTTGATCTTTGTTATAGCGTATCATCACCCTCG
AAAGTGTAATGTTTTGTACCCCCAAACTTGTTTAGCATTATAATAAAGTCTCTTTGGAACTTCTC

**SEQ ID NO: 168, AT3G14230.3 Arabidopsis AP2-EREBP family transcription factor, protein sequence 374AA**

MCGGAIISDFIPPPRSLRVTNEFIWPDLKNKVKASKKRSNKRSDFFDLDDDFEADFQGFKDDSAFDCE
DDDDVFVNVKPFVFTATTKPVASAFVSTGSAYAKKTVESAEQAEKSSKRKRKNQYRGIRQRPWGKWAA
EIRDPRKGSREWLGTFDTAEEAARAYDAAARRIRGTKAKVNFPEEKNPSVVSQKRPSAKTNNLQKSVA
KPNKSVTLVQQPTHLSQQYCNNSFDNSFGDMSFMEEKPQMYNNQFGLTNSFDAGGNNGYQYFSSDQGS
NSFDCSEFGWSDHGPKTPEISSMLVNNNEASFVEETNAAKKLKPNSDESDDLMAYLDNALWDTPLEVE
AMLGADAGAVTQEEENPVELWSLDEINFMLEGDF

Figure 28 (continued)

**SEQ ID NO: 169, NM_112550.3| Arabidopsis thaliana ATEBP/RAP2.3; DNA binding / transcription factor (ATEBP/RAP2.3) mRNA, complete cds**

ATAAAGGCATTTCAGCTCCACCGTAGGAAACTTTCTCTTGAAAGAAACCCACAGCAACAAACAGAGAA
AATGTGTGGCGGTGCTATTATTTCCGATTATGCCCCTCTCGTCACCAAGGCCAAGGGCCGTAAACTCA
CGGCTGAGGAACTCTGGTCAGAGCTCGATGCTTCCGCCGCCGACGACTTCTGGGGTTTCTATTCCACC
TCCAAACTCCATCCCACCAACCAAGTTAACGTGAAAGAGGAGGCAGTGAAGAAGGAGCAGGCAACAGA
GCCGGGGAAACGGAGGAAGAGGAAGAATGTTTATAGAGGGATACGTAAGCGTCCATGGGGAAAATGGG
CGGCTGAGATTCGAGATCCACGAAAAGGTGTTAGAGTTTGGCTTGGTACGTTCAACACGGCGGAGGAA
GCTGCCATGGCTTATGATGTTGCGGCCAAGCAGATCCGTGGTGATAAAGCCAAGCTCAACTTCCCAGA
TCTGCACCATCCTCCTCCTCCTAATTATACTCCTCCGCCGTCATCGCCACGATCAACCGATCAGCCTC
CGGCGAAGAAGGTCTGCGTTGTCTCTCAGAGTGAGAGCGAGTTAAGTCAGCCGAGTTTCCCGGTGGAG
TGTATAGGATTTGGAAATGGGGACGAGTTTCAGAACCTGAGTTACGGATTTGAGCCGGATTATGATCT
GAAACAGCAGATATCGAGCTTGGAATCGTTCCTTGAGCTGGACGGTAACACGGCGGAGCAACCGAGTC
AGCTTGATGAGTCCGTTTCCGAGGTGGATATGTGGATGCTTGATGATGTCATTGCGTCGTATGAGTAA
AAGAAAAAAAATAAGTTTAAAAAAAGTTAAATAAAGTCTGTAATATATATGTAACCGCCGTTACTTTT
AAAAGGTTTTTACCGTCGCATTGGACTGCTGATGATGTCTGTTGTGTAATGTGTAGAATGTGACCAAA
TGGACGTTATATTACGGTTTGTGGTATTATTAGTTTCTTAGATGGAAAAACTTACATGTGTAAATAAG
ATTTGTAATGTAAGACGAAGTACTTATAACTTCTTAACTGTTTTGTGGTAG

**SEQ ID NO: 170, AT3G16770.1 Arabidopsis AP2-EREBP family transcription factor, protein sequence 248AA**

MCGGAIISDYAPLVTKAKGRKLTAEELWSELDASAADDFWGFYSTSKLHPTNQVNVKEEA
VKKEQATEPGKRRKRKNVYRGIRKRPWGKWAAEIRDPRKGVRVWLGTFNTAEEAAMAYDV
AAKQIRGDKAKLNFPDLHHPPPPNYTPPPSSPRSTDQPPAKKVCVVSQSESELSQPSFPV
ECIGFGNGDEFQNLSYGFEPDYDLKQQISSLESFLELDGNTAEQPSQLDESVSEVDMWML
DDVIASYE

**SEQ ID NO: 171, NM_001066815.1| Oryza sativa (japonica cultivar-group) Os07g0617000 (Os07g0617000) mRNA, complete cds**

AGACTACAAGCCATTAAACAGAGACGACCAACGACTTAACCACACTTCTTCTTTGTGCTGTCCAACCT
CCATTGTTGGGTGTGAAAGCTTTGGCTCATCTGCCATGTGTGGAGGATCCATTCTCGGCGACCTTCAC
TTGCCGGTGCGGCGGACAGTGAACGCCGGTGACCTGTGGGGAGACGCCGGCAAGGGTAGAGATGGTGG
CGACGGCTTGAAGAAGAGGAAGGGGAGTTCTTGGGATTTCGATGTTGATTGCGATGATGATGATGATG
ATGACTTTGAGGCTGATTTTGAGGAGTTTGAGGATGACTATGGCGATGATGATGATGTGGGTTTCGGG
GACGACGACCAAGAATCCGACATGAACGGTCTCAAGCTCGCCGGATTCAGCACCACGAAGCTCGGCCT
CGGCGGCAGCAGGAAGAGGAAGACGCGATACCGAGGGATCCGGCAGCGGCCATGGGGAAATGGGCGG
CGGAGATCAGGGACCCCCGCAAGGGCGTCCGCGTCTGGCTCGGCACGTTCGGCACCGCCGAGGAGGCC
GCCATGGCGTACGACGTCGAGGCACGCCGCATCCGCGGCAAGAAAGCCAAGGTCAACTTCCCCGACGC
CGCCGCCGCCGCCCCGAAGCGGCCACGGCGTTCTTCGGCGAAGCATTCGCCGCAGCAGCAGAAGGCCA
GGTCGTCGTCGTCGTCGCCGGCGAGCCTGAACGCCAGCGACGCCGTGTCCAAGTCCAACAACAACCGC
GTCAGCTCGGCTGGGAGCAGCACCGACGCCACCGCCGCCGCCATCGCCATCGACGACGGCGTCAAGCT
CGAGCTGCTCTCGGAGACGGATCCTTCTCCGCCCATGGCCGCCGCCGCCGCCGCGTGGCTCGACGCGT
TCGAGCTGAACGATCTTGACGGATCAAGATGCAAGGACAACGCATTCGATCACCAGATTCACAAGGTA
GAAGCGGCTGTCGCTGATGAATTCGCGTTCTACGACGATCCGAGCTACATGCAGCTGGGTTACCAGCT
CGATCAGGGCAACTCGTACGAGAACATCGACGCGCTCTTCGGCGGCGAGGCCGTCAACATTGGTGGAC
TCTGGAGCTTCGACGACATGCCAATGGAGTTCAG

Figure 28 (continued)

AGCTTATTGAGCATTTGATTCTATTTAGGAGGGAGTGAATTATTTGGGAGGAAGAATCGATGTTGTAA
CTTGTAAAATCTCTGATGATGATCTCTGCATCATATGATCAATTTGAGTGCAGTTTTGTTTTTGTAAA
TACGAATTCTTTATTATGATGTTAGGTTCTTAATTTGCCCTTCTTCATCAGGGATTTGTTCAGGCTTT
TGTTGTGATGACTGATTGGACGAGGGAAAGATTGCGTTTTTTGTTGTCATGTTGGGTACTCTACTCTT
CTGTTCCTAAAGTTGATAAGTGCCAAAATTTGTGAGAGG

**SEQ ID NO: 172, LOC_Os07g42510.1|11977.m08483|protein|AP2-EREBP**

MCGGSILGDLHLPVRRTVNAGDLWGDAGKGRDGGDGLKKRKGSSWDFDVDCDDDDDDDFEADFEEFED
DYGDDDDVGFGDDDQESDMNGLKLAGFSTTKLGLGGSRKRKTRYRGIRQRPWGKWAAEIRDPRKGVRV
WLGTFGTAEEAAMAYDVEARRIRGKKAKVNFPDAAAAAPKRPRRSSAKHSPQQQKARSSSSSPASLNA
SDAVSKSNNNRVSSAGSSTDATAAAIAIDDGVKLELLSETDPSPPMAAAAAAWLDAFELNDLDGSRCK
DNAFDHQIHKVEAAVADEFAFYDDPSYMQLGYQLDQGNSYENIDALFGGEAVNIGGLWSFDDMPMEFR
AY

**SEQ ID NO: 173, NM_001055714.1| Oryza sativa (japonica cultivar-group) Os03g0183200 (Os03g0183200) mRNA, complete cds**

CTCGCGCCATCCAAGCGCCCGCCTCTATATATGCGCGCCGCCACCATGTCCAGCTCCGGCAACTAGCT
ACTGCGAAGTGCGAACTGATCAGATCGTCGAGAGGAAACCCAAGATCCAACGACGACATGTGTGGCGG
CGCGATTCTGGCTAACATCATACCGGCCACCCCACGCCCCCGCAAGTGCCGCCCCACCACCGCCACCG
CCACCCCCAAGGCGACGACACCGAACGTCGTCGTCGTCGTCAACCTCGTCGACAAAGAGGCCGAGGTC
AGCGAGAGCTCCGGTGCCAGCAGCAGCGCGCTGCCGGACTTCTCGTGGCAGGGCATGTCGGCGTCGTC
CGACGACGACGCCGCGGCGCAGCAGGCACTCCTCGACGCCGCCGGCGGCGCCAAGAAGCGTCCCCGGA
GCGAGCCCCACGTCACCTCCGACGACGAAGTGCTCCCGGCGTCATTCGACAGTGACAACAACACCGCC
GCCGCCGGCCTGCTCCCGCTCGACGATCCTTTCTTGTTCGGCGACCAGTTCGGCGACCTCAACGGCGG
CGCGTTCGCCTCGCTCATGGACGGGCTGTTCGCCGCCGGTGAAGCGAACGTCGCCGGCGAGAGCGTGG
GGCTCTGGAGCTTCGGCGACGACTTTCTCAACGCGTCGTACTATTAGCTAGGGCTTCCATAGTTCTTG
TACTTTACTTGTACTGTACTGTATTGTACTGATTGTTAACGTTGCCATAAAGCAATCTTGCTAGTTTG
T

**SEQ ID NO: 174, LOC_Os03g08490.1|11973.m06357|protein|AP2-EREBP**

MCGGAILANIIPATPPRPATAAHVWPGGDGEKRRKVGGGGCDDDFEAAFERFGREDSEMEEEEVEEVV
VGKKAAVRRRRATPAAGRRARPSKYWGVRRRPWGKWAAEIRDPVEGVRVWLGTFATAEAAAHAYDAAA
RDLRGATAKLNFPSSSSSTAATPRPRKCRPTTATATPKATTPNVVVVVNLVDKEAEVSESSGASSSAL
PDFSWQGMSASSDDDAAAQQALLDAAGGAKKRPRSEPHVTSDDEVLPASFDSDNNTAAAGLLPLDDPF
LFGDQFGDLNGGAFASLMDGLFAAGEANVAGESVGLWSFGDDFLNASYY

**SEQ ID NO: 175, NM_001055715.1| Oryza sativa (japonica cultivar-group) Os03g0183300 (Os03g0183300) mRNA, complete cds**

TCATCACAAGCTCACAAGTTCACAACCCAACACCCAAAAGCAAAAGAAAAGCAGCAACCAAAGATGTG
CGGCGGAGCGATCCTTGCGGAGCTCATACCGAGCGCGCCGGCGGCGAGGCGCGTCACGGCGGGCCACG
TCTGGCCGGGCGACGCCAACAAGGCCAAGAAGAAGGGCGCGCGCGCCGACGACTTCGAGGCCGCGTTC
CGCGACTTCGACAACGACTCCGATGACGAGGAGATGATGGTGGAGGAGGCGGAGGAGGAGGAGGCGAC
CTCCGAGCACAAGCCGTTCGTCTTCCGCGCCAAGAAGGCGGCGGCGGCGGCGTCGAGC

Figure 28 (continued)

AGGCGCAGGAAGCCGGCGCAGTACAGGGGCGTGCGGCGCCGGCCGTGGGGGAAGTGGGCGGCGGAGAT
CCGCGACCCCGTCAAGGGCATCCGCGTCTGGCTCGGCACCTTCACCAACGCCGAGGCCGCCGCGCTCG
CCTACGACGACGCCGCGCGCGCCATCCGCGGGGACAGGGCCAAGCTCAACTTCCCTTCCGCTACCACC
CCTGACACCCGCAAGCGCGGCCGCGCCACCGCCGCCGCCGCCCCGGCCGTCAAGGCGACCCCGGTCAT
CAACCTCGTCGAGGAGGAGGACGAGGAGGAGGTCGCCGCCGCCATGGCGTCCATCAAGTACGAGCCCG
AGACCAGCGAGAGCTCCGAGTCGAACGCCCTCCCGGACTTCTCCTGGCAGGGCATGTCGGCCTCCGAC
GAGTTCGCCGTCGCCGCGGCGGCGCTGTCGCTCGACAGCGACGACGACCTCGCCAAGAAGCGTCCGAG
GACCGAGCCGGAGGACACCACCGACTCCGGCTCCGGCGACGACACCGACGCGCTGTTCGACGCGCTGC
TGTTCGCCGACCAGTACAACCACTTCAACGGCGGCGCCTACGAGTCCCTGGACAGCCTGTTCAGCGCC
GACGCCGTGCAGACCACCGCCGCCGCCGCCGCCGCCGACCAGGGCATGGGGCTCTGGAGCTTCGACGA
CGGCTGCTGCCTCGTCGACGTCGAGGCCAGCTTGTCCTTCTAGGCTCTAGCCGTCGTCTCCGGCGACC
CTGTGACTCGATCTTGTACCATGTCATGTACATAGAAGAGTGGTTTTGCCAAGCAAGCATGTGTTCGT
CCTGGTTCCTTTCGGTAATGAAAAATTATGTGAGGCTTCTCGTTCT

**SEQ ID NO: 176, LOC_Os03g08500.1|11973.m06358|protein|AP2-EREBP**

MCGGAILAELIPSAPAARRVTAGHVWPGDANKAKKKGARADDFEAAFRDFDNDSDDEEMMVEEAEEEE
ATSEHKPFVFRAKKAAAAASSRRRKPAQYRGVRRRPWGKWAAEIRDPVKGIRVWLGTFTNAEAAALAY
DDAARAIRGDRAKLNFPSATTPDTRKRGRATAAAAPAVKATPVINLVEEEDEEEVAAAMASIKYEPET
SESSESNALPDFSWQGMSASDEFAVAAAALSLDSDDDLAKKRPRTEPEDTTDSGSGDDTDALFDALLF
ADQYNHFNGGAYESLDSLFSADAVQTTAAAAAADQGMGLWSFDDGCCLVDVEASLSF

**SEQ ID NO: 177, Arabidopsis thaliana DNA binding / transcription factor (AT1G72360) (gi|12325262:c71325-71139, c71053-70452) Arabidopsis thaliana chromosome 1 BAC T10D10 genomic sequence, complete sequence**

ATGTGCGGAGGAGCTGTAATTTCCGATTACATAGCGCCGGAGAAGATTGCGAGATCATCTGGAAAGTC
TTCCTGGAGAAGTAATGGCGTCTTTGACTGCTCAATCTACGATTTCGATGGAAATTTCGATGAATTAG
AGTCCGATGAGCCATTTGTCTTCTCCTCTACTCACAAACATCATGCTTCAGGCTCAGCATCAGATGGG
AAGAAGAAACAGAGCAGTCGGTACAAAGGAATCAGAAGAAGGCCTTGGGGAAGATGGGCGGCTGAGAT
ACGTGATCCAATCAAAGGAGTTCGAGTTTGGCTCGGGACTTTCAACACAGCTGAAGAAGCTGCAAGAG
CTTATGATCTTGAAGCTAAGAGAATCCGTGGAGCCAAAGCTAAGCTCAATTTCCCTAACGAATCCTCT
GGAAAGAGGAAAGCCAAGGCTAAGACTGTGCAACAGGTAGAGGAGAATCATGAGGCTGATCTTGATGT
GGCGGTGGTAAGCTCAGCGCCTAGTAGTAGCTGTCTTGATTTCTTGTGGGAGGAGAATAATCCGGACA
CGCTTCTGATTGATACACAATGGCTCGAAGATATCATCATGGGCGATGCGAATAAGAAACATGAACCT
AATGATAGTGAAGAAGCCAACAACGTTGATGCTTCTCTGCTTTCTGAAGAGCTTCTTGCTTTTGAGAA
CCAGACCGAATATTTCTCGCAGATGCCTTTTACGGAGGGAAACTGTGATTCCTCAACGTCTCTGAGTA
GTCTCTTTGATGGAGGCAATGACATGGGTCTATGGTCCTGA

**SEQ ID NO: 178, AT1G72360, AAG52589.1|AC016529_20 putative AP2 domain transcription factor; 71325-70452 [Arabidopsis thaliana]**

MCGGAVISDYIAPEKIARSSGKSSWRSNGVFDCSIYDFDGNFDELESDEPFVFSSTHKHHASGSASDG
KKKQSSRYKGIRRRPWGRWAAEIRDPIKGVRVWLGTFNTAEEAARAYDLEAKRIRGAKAKLNFPNESS
GKRKAKAKTVQQVEENHEADLDVAVVSSAPSSSCLDFLWEENNPDTLLIDTQWLEDIIMGDANKKHEP
NDSEEANNVDASLLSEELLAFENQTEYFSQMPFTEGNCDSSTSLSSLFDGGNDMGLWS

Figure 28 (continued)

**SEQ ID NO: 179, NM_001055711.1| Oryza sativa (japonica cultivar-group) Os03g0182800 (Os03g0182800) mRNA, complete cds**

CCCGCGTCGCGCCATAGCGACGTCTCTCCCGGAGAAAGAAGAGCGCGCCGCGCCATGTGCGGCGGTGC
AATCCTCGCCGATTTCACCCCGGCGAGGGTGCCCCGGCGGCTGACCGCCGCCGAGCTCCTGCCGGTGA
CCCCGACTCCCCCCGCCGCCGAGAGGAGAACCACCCGGAAGCGCAAGTCCGACGTCGACTTCGAGGCG
GAGTTCGAGCTTTTCGAGGACGACGACGACGACGATGAGTTCGAGCTTTCCGACGATGGCGACGAGAG
TTTGGCCGTGTCATGTGTGTCGTCCCCCAAGTCGAAGGCAGTACCTTCGTTTTCTTTTTCGTCGGATG
TCTCCTCGAGCTCCAGGCCGCGGCGGCGCGTGGCGGCGGCGGCGGCCGGTCGTCGGAAGGCGAGCAAG
AAGAGCAAGTACAGGGGCGTCCGGCGCCGGCCGTCGGGGAGGTTCGCGGCGGAGATCAGGGACCCCAA
GAAGGGGCGGCGCGTGTGGCTCGGCACGTACGGCAGCGCCGAGGAGGCCGCCATGGCCTACGACCGCG
AGGCCCGCCGCATCCGCGGCAAGGGCGCGAGGCTCAACTTCCCCCGCGACGGCGATGGCTCCCCTCGC
CGGAGTAACGACCGGCCCTGCTGGACCATCGACCTCAACCTCCCCGCGGCGGCCGTCTCCGGTGACGA
CGACGACGCCATGGCCGTCGACGCCGCAGACGCAGACGCAGGCAGTGCTGGCCGTGCAGCAGCCTATG
CAGATCAAGAAGCACTGAGCGCGGCAAAGTGCAAGATCAAGCAGTGTCCTCGCGACGAACAGATGGCG
AGCGCCACACCTGAGCTCATGGAGGAGGACGCGAGCAGCAGCAGAAACATGGTGCCCCTGTCCATGGC
GCTGCAGCTGCAGTATGCGGCGATGATCGCCGAATGCGACCGCGAGATGGAGGAGATCGCCGCCGTGG
AGAGGGACCTCGAGAGGCGCAGGAGGCAGGTGTTCGAGCGCAGAGGCCACCTGGTCAGGCAGGCCTCT
CTTCTGCTCGACTGACGATCTTGCTGAACTGAACTCTGAATGTTTGAGCTTGTCTGAAGTCTGAACTC
TGCACTATGCCATATGGTGTTTCACCTTCACTGTGAGGCTGACATGTGGGCCAGACGTCCGTTTGCCT
TGCTTGCTTTTGAGCTGCAACTGGGCGTTGTGCTGCAAGCCTGCAAAGTGCCTGTGTAAAGTTTGTGG
GATTGTGTGTGTGGATCTTGTATCCTTGTGACTTTGCAAGCTTTAATTTTGTGAGGAACAATAGTATT
TTAGATTGGTGTGTAAAATATAGAAATAAATAATAGTAGCAACTAAATTTTGGTTTTCAGCTCTTCAT
CAGATGGTTTGTCATGGGAACAAAATTTCAAACATGAGCAAATTAAGGTCATGTTATTATCAATTGTG
AT

**SEQ ID NO: 180, LOC_Os03g08460.1|11973.m06354|protein|AP2-EREBP**

MCGGAILADFTPARVPRRLTAAELLPVTPTPPAAERRTTRKRKSDVDFEAEFELFEDDDDDDEFELSD
DGDESLAVSCVSSPKSKAVPSFSFSSDVSSSSRPRRRVAAAAAGRRKASKKSKYRGVRRRPSGRFAAE
IRDPKKGRRVWLGTYGSAEEAAMAYDREARRIRGKGARLNFPRDGDGSPRRSNDRPCWTIDLNLPAAA
VSGDDDDAMAVDAADADAGSAGRAAAYADQEALSAAKCKIKQCPRDEQMASATPELMEEDASSSRNMV
PLSMALQLQYAAMIAECDREMEEIAAVERDLERRRRQVFERRGHLVRQASLLLD

**SEQ ID NO: 181, NM_001061846.1| Oryza sativa (japonica cultivar-group) Os05g0361700 (Os05g0361700) mRNA, complete cds**

AGAAGAAAAGCCTTGTGAGTTGGTAATTGATAGTAGCAAGACAATGTGTGGGGGAGCGATCATCGCCG
ACTTCGTCCCGCCCGCCGGCGCCCGCCGCGCCGCTGCCTCCGACATCTCCGACAACGCCGTCCTCTCC
GCTGCCGGTGCCGGTGACGAGTCGTTCGCGGCGGCCAAGGCGCCGGCGCCGGGGAGGAAGACGGCGTA
CCGCGGCATCCGGCGCCGGCCGTGGGGACGCTGGGCGGCGGAGATCCGCGACCCGAGGAAGGGCGCCC
GCGTCTGGCTCGGCACCTACGCCACCGCCGAGGAGGCCGCCCGCGCCTACGACGTCGCGGCGCGCGAC
ATCCGCGGCGCCAAGGCCAAGCTCAACTTCCCCCCGACCATCGGCGCCGCCGCCGCGCCACCGCCGCC
CAAGAAGCGACGCAAAGCCGCCGCCGCGGCGAACCACCACCACCACCACCACCAGCAGGAGAGCTCAG
GCTCCTCGTCGGCGTCGTCGCTGCCTCCCACCCCGCCGCCGGCCGCCGAGCACCAGCTCCGCGAGTGC
ATGTCCGGGCTGGAGGCGTTCTTGGGCCTCGAGGAGGAGGAGGACGACGGCGGCGCCGGTGAGCCATG
GGACGCCGTCGACATGATGCTCGAGTAGGCTCGCCGGGAATGGCAGCG

Figure 28 (continued)

TTGCCATGCTCATGCATCCAAGCTTATTCATGCATGCAGCAGCAAGATATACAACTTTAGTACTACTA
CTATGTTAATTACTACTTACTGCGTAGGTAAATGAAATAAAATGGGGTTGGTTAATTAGGGTGTTCTT
CTGGGAATCCTTGGCTTTGCTAATTGCTAGTACTACTATGTACTTTGGCGTTAAGCATTGCATTGCCA
GGTGATGCATGTCATGTAATCGTGGTAATTATATGGCCGTCCTCCTAGAGCTAGCTAGCAGATTAGAT
TTACTATATAAAGTGGTAGTAGAAATATATGTCCTCCTAGAGCTAACTAGTGTCTTGTAATGATTTCA
GACTTCTTGGGCAACATGAGCAATGCATTTCGACTTCCACTTTACATCT

**SEQ ID NO: 182, LOC_Os05g29810.1|11975.m07218|protein|AP2-EREBP**

MCGGAIIADFVPPAGARRAAASDISDNAVLSAAGAGDESFAAAKAPAPGRKTAYRGIRRRPWGRWAAE
IRDPRKGARVWLGTYATAEEAARAYDVAARDIRGAKAKLNFPPTIGAAAAPPPPKKRRKAAAAANHHH
HHHQQESSGSSSASSLPPTPPPAAEHQLRECMSGLEAFLGLEEEEDDGGAGEPWDAVDMMLE

**SEQ ID NO: 183, NM_001056603.1| Oryza sativa (japonica cultivar-group) Os03g0341000 (Os03g0341000) mRNA, complete cds**

ACCTCGCTCTCCTCGTCACCGCGATCGATCGATCGCCGCCGGTTTAGATTTCCTTCCTACGCGGACCG
CACCCACGCGCTAGGTTTAGCTAGCTAGTTTGCTTGCGCGCAAGCGTCGATCGAGCTAGCTAGTGAAG
ATAGATATGTGCGGCGGCGCCATCCCGCTGATCAGCAGCCGCGGCCCCGGCGGCAAGAGGAGCCTCTC
CGCCGCCGATGAGCTCTGGCCGCCGCCGCCGCAGCACGCCAGCGACGACCCGGCCGAGCAAGCGGCGG
CGGATGAGGAGGAGCAGGAGCAGCAGCCGGCGGCGAGGAGGCAGCGGCGAGGGGAGCGGAGGACGCTG
TACCGGGGCATCCGGCGCAGGCCGTGGGGGAAATGGGCGGCGGAGATCCGCGACCCGGCCAAGGGCGC
CCGCGTCTGGCTCGGCACCTTCGCCACCGCCGAGGCTGCCGCGCGGGCCTACGACCGCGCCGCCCGCC
GCATCCGCGGCACCAAGGCCAAGGTCAACTTCCCCAACGAGGACAACGCCTTCGCCGCCGCGCCGCCG
CCGTACCACCTCGCCGCCTACTACGGCGACGCCTCCTCCACCTCCTACCTCTACCCGATGGCCATGAC
GCCCGCCGCCGCCGGACTGAGGGAGCAGCAGCTGATGACGACGACGGCGGTGGAGTACAGCGTTAATG
ACGCCGTCGACGTGGCCAGCGTTTACTTCCAGCCGCCGCCGCCGGCGGTTGCTTACGAGTTCAGCGCC
GTCGGCGGTGGCGCCGTCGTCGTGCCGGTGTCGGCGGTGGCGCCGGCGATGACGTACGGACAGAGCCA
AGAGGTGGCGGCTCCGCTCATGTGGAATTTCGATGACATCACGGCCATGCCAATGTGATTGTCTTACC
GTGGAGATTAAGGCATTTAAAGGCTTATAGATAACATAAATTTGCCATGTATGATCAATGGTTAATTC
CTATTGCTCAAGGGCTTATATTAAGGGTTACCTACACGGCATGGCTAGCTTAGCCTACAATTTATGTT
TCTTGCATTTCTTTCTCTTTTTTTGAGATGAGCTAGTGTGTTATACTATTTGTTCATCTTTTCTGGTA
GTGGTGGATACATTTTCGCCGGCTAGGGTTGGAATTTGTAAATAGAAGATGGTCAATAATAAGATTGT
TTCTGGCT

**SEQ ID NO: 184, LOC_Os03g22170.1|11973.m07620|protein|AP2-EREBP**

MCGGAIPLISSRGPGGKRSLSAADELWPPPPQHASDDPAEQAAADEEEQEQQPAARRQRRGERRTLYR
GIRRRPWGKWAAEIRDPAKGARVWLGTFATAEAAARAYDRAARRIRGTKAKVNFPNEDNAFAAAPPPY
HLAAYYGDASSTSYLYPMAMTPAAAGLREQQLMTTTAVEYSVNDAVDVASVYFQPPPPAVAYEFSAVG
GGAVVVPVSAVAPAMTYGQSQEVAAPLMWNFDDITAMPM

**SEQ ID NO: 185, NM_001071041.1| Oryza sativa (japonica cultivar-group) Os10g0390800 (Os10g0390800) mRNA, complete cds**

GTCTCAAACCCAATCAAACTCCAACCAAACTCACCTACCTACCCCCAACCCATCCAGAGCTAGAGCTA
TGTGCGGCGGCGCGATCCTCGCCGACCTCATACCGTCGCCGCGCTCCGGCGGCCACACCAAAAAGAAC
AAGCGGCGGCGGATCAGCGACGACGAGGACTTCGAGGCCGCCTTCGAGGAGTTCGACGCCGG

Figure 28 (continued)

CGACGACGACTCCGACTCCGACTCCGAGTCCGAGGAGGTAGACGAGTACGACGTCGTCGTCGACGACG
ACGACAGCGAGGACGGCGTGGTGGTTCTTCCGCCGCCGCCGCCGCCGCCGCCGGTGATTCCACATGAG
CGCCATGGCGCGAGGCGGTTCCGCGGCGTGAGGAAGCGGCCGTGGGGGAAGTGGGCGGCGGAGATCCG
CGACCCCGTGCGCGGCGTGCGCGTCTGGCTCGGTACCTTCCCCACCGCCGAGTCCGCCGCGCGCGCCT
ACGACGCCGCCGCCCGCCGCCTCCGCGGCGCCAAGGCCAAGCCCAACTTCCCCTCCGCGCCGCCGCCC
TCGGCTGCTGCTCACCGCCGCAAGAAGCGCCGCGCCCACGCCGCCACGCGCTCGCCGTCGTCTCCGCC
CGCCACCAGCGAGGTCACGGCGGCGTCCGCGTCCGCGTCCAGCGATGTCCCCGCGCCGGCGTTCGCTT
CCTTCGTCGGCGAGCCCGGGCACGGCGGCGCCAAGTCGATGCCGACGACGAGCCACACCTCGCAGCCA
GCCCCGCCGGCGACGGTGGCGTCCGAGAACGTCGACGACCCGGAGGTGTTCGACCCGTACGACGTCCA
CGGCGGCCTCGCCTCCTACTTCGCCGGCGGCGCGTACGAGTCCCTGGAGAGCCTGTTCGCGCACGGCG
GCGACAGCGCCGCCGTCGACCAAGCGGCGAGCGACCACTGGCCGGCGGCGCTATGGAGCTTCGCAGAC
GACGGCTCGTTCTGCTTCTGATGCTGTCAAATCACATCGCCATTGGCGGCCATTGCAAGCCATGGCAT
GGCACCTGATGATGCTGATCCAGTGAACTGGTCACTCGTTCTTGATGCGTTTTCAGTTTCTTGTACAG
TGTTTTTCCCCAGCAACAGTGAAGTAGTATTCAGTTATTCACTGTTCAGTCATGAGTTCATCGCAAAA
TATGATGTAAGTATGTGTCTTGAGATTGTTTTAGCAGTGGCTTTTGTTTGTATAATGTATTTCTCTGT
AATTAATTAATAGCTGTTTTCAGTGATAAACTAAATTTTCTG

**SEQ ID NO: 186, LOC_Os10g25170.1|11980.m05467|protein|AP2-EREBP**

MSQTQSNSNQTHLPTPNPSRARAMCGGAILADLIPSPRSGGHTKKNKRRRISDDEDFEAAFEEFDAGD
DDSDSDSESEEVDEYDVVVDDDDSEDGVVVLPPPPPPPPVIPHERHGARRFRGVRKRPWGKWAAEIRD
PVRGVRVWLGTFPTAESAARAYDAAARRLRGAKAKPNFPSAPPPSAAAHRRKKRRAHAATRSPSSPPA
TSEVTAASASASSDVPAPAFASFVGEPGHGGAKSMPTTSHTSQPAPPATVASENVDDPEVFDPYDVHG
GLASYFAGGAYESLESLFAHGGDSAAVDQAASDHWPAALWSFADDGSFCF

**SEQ ID NO: 187, gi|46093789:c67744-67046 Oryza sativa (japonica cultivar-group) genomic DNA, chromosome 9, PAC clone:P0663H05**

ATGCGCCGCCGCGTCTCCTCCTCCTCCTCCTCCTCCTCGTCCTCGTCGCCGGCGAGGCATCACAAGGC
GCGGCGCAGCAGGAGGAAGCTCGCCGTCGACGAGGACTGGGAGGCCGCCTTCCGCGAGTTCCTCTCCC
GCGACCACGACGACGACGACGACGACCACGACGGTCAGCATGTCGTTGTTGCGCCGTTGATCCGTGGT
AGTGACAAGTGCGTCCACGGCCACGAGGTGGTGGCGTCGACGGTCGGCGGTGGCGCAAGCGGCGGACG
ACGACGAGCCGACGACGACGACGGCGAGCGGCGGCGGCGGCGGCGGAGGGAGAAGCGGAGCTACCCGT
ACCGCGGCATCCGGCAGCGGCCGTGGGGGAGGTGGGCGTCGGAGATCCGCGACCCCGTCAAGGGCATC
CGCGTCTGGCTCGGCACCTTCGACACCGCCGAGGGCGCCGCGCGCGCCTACGACGACGAGGTTCGCCG
CATCTACGGCGGCAACGCCAAGACCAACTTCCCCCCATCGCCGCCCACGCCGCCGCCGCCGGAGAAGC
CAGCGGCGGAGAGGAGCCCCTCGACGACGCCGACGACGACCACGGAGGACTCCGGCGACTCGCGCATA
CTCATCGAGTGCTGCTCCGACGACCTGATGGACAGCCTCCTCGCCGCCTTCGACATGACCACCGGCGA
CATGCGCTTCTGGAGCTAA

**SEQ ID NO: 188, LOC_Os09g11460.1|11979.m04408|protein|AP2-EREBP**

MRRRVSSSSSSSSSSSPARHHKARRSRRKLAVDEDWEAAFREFLSRDHDDDDDDHDGQHVVVAPLIRG
SDKCVHGHEVVASTVGGGASGGRRRADDDGERRRRRRREKRSYPYRGIRQRPWGRWASEIRDPVKGI
RVWLGTFDTAEGAARAYDDEVRRIYGGNAKTNFPPSPPTPPPPEKPAAERSPSTTPTTTTEDSGDSRI
LIECCSDDLMDSLLAAFDMTTGDMRFWS

Figure 28 (continued)

**SEQ ID NO: 189, AY044235.1| Lycopersicon esculentum transcription factor JERF1 (JERF1) mRNA, complete cds**

TTCAAATTGAGCTTTTTCTCCATTAAAATTCTCTCTGCAAATTTATAGTTTTTCTTTTTTCACTTTTT
GAGAAGAAATCAAAAGCTATGTGTGGTGGTGCAATTATCTCCGATTTGGTACCTCCTAGCCGGATTTC
TCGCCGGTTAACCGCTGATTTTCTATGGGGTACATCCGATCTGAACAAGAAGAAGAAGAACCCTAGTA
ATTACCACTCAAAGCCCTTGAGGTCTAAGTTTATTGACCTTGAAGATGAATTTGAAGCTGACTTTCAG
CACTTCAAGGATAATTCTGATGATGATGATGATGTGAAGGCATTTGGCCCCAAATCCGTGAGATCTGG
TGATTCAAACTGCGAAGCTGACAGATCCTCCAAGAGAAAGAGGAAGAATCAGTACCGGGGGATCAGAC
AGCGTCCTTGGGGTAAGTGGGCAGCTGAAATACGTGATCCAAGGAAAGGTATTCGAGTCTGGCTTGGT
ACTTTCAATTCAGCCGAAGAGGCAGCCAGAGCTTATGATGCTGAGGCGCGAAGGATCAGAGGCAAGAA
AGCTAAGGTGAACTTTCCTGATGAAGCTCCAGTGTCTGTTTCAAGACGTGCTATTAAGCAAAATCCCC
AAAAGGCACTTCGTGAGGAAACCCTGAACACAGTTCAGCCCAACATGACTTATATTAGTAACTTGGAT
GGTGGATCTGATGATTCGTTCAGTTTTTTCGAAGAGAAACCAGCAACCAAGCAGTACGGCTTCGAGAA
TGTGTCTTTTACTGCTGTAGATATGGGACTGGGCTCAGTTTCCCCTTCAGCTGGTACAAATGTTTACT
TCAGCTCTGATGAAGCAAGTAACACTTTTGACTGCTCTGATTTCGGTTGGGCTGAACCGTGTGCAAGG
ACTCCAGAGATCTCATCTGTTCTGTCGGAAGTTCTGGAAACCAATGAGACTCATTTTGATGATGATTC
CAGACCAGAGAAAAAACTGAAGTCCTGTTCCAGCACTTCATTGACAGTTGACGGTAACACTGTGAACA
CGCTATCTGAAGAGCTATCGGCTTTTGAATCCCAGATGAAGTTCTTGCAGATCCCATATCTCGAGGGA
AATTGGGATGCATCGGTTGATGCCTTCCTCAATACAAGTGCAATTCAGGATGGTGGAAACGCCATGGA
CCTTTGGTCCTTCGATGATGTACCTTCTTTAATGGGAGGTGCCTACTAAGCTGCATACACATCTTCCC
CTGCTAAGTTTTGTAAATAACGCTTCATTTGAGTGAAGTTTGCGCCTGCGTTTACGTTTATCACCAAA
CTAAAAGACTATATATGTGTTGTATTAATTTATTCAAAATTTACTCGTTTGATATATGTAAGTATGTA
TCCTTGTTTTCATAAAAAAAAAAAAAAAAAAA

**SEQ ID NO: 190, AAK95687.1| transcription factor JERF1 [Lycopersicon esculentum]**

MCGGAIISDLVPPSRISRRLTADFLWGTSDLNKKKKNPSNYHSKPLRSKFIDLEDEFEADFQHFKDNS
DDDDDVKAFGPKSVRSGDSNCEADRSSKRKRKNQYRGIRQRPWGKWAAEIRDPRKGIRVWLGTFNSAE
EAARAYDAEARRIRGKKAKVNFPDEAPVSVSRRAIKQNPQKALREETLNTVQPNMTYISNLDGGSDDS
FSFFEEKPATKQYGFENVSFTAVDMGLGSVSPSAGTNVYFSSDEASNTFDCSDFGWAEPCARTPEISS
VLSEVLETNETHFDDDSRPEKKLKSCSSTSLTVDGNTVNTLSEELSAFESQMKFLQIPYLEGNWDASV
DAFLNTSAIQDGGNAMDLWSFDDVPSLMGGAY

**SEQ ID NO: 191, NM_001069317.1| Oryza sativa (japonica cultivar-group) Os09g0287000 (Os09g0287000) mRNA, complete cds**

AGATATTCAGACACACACCTTCAATAACTTGCAAGGATAACTCAAACTACTTGAATCAGATCAGACAA
AAACATCATAAGAATATCACGATGTGTGGAGGAGCACTGATCCCGAACGACTATGGCGACAAGCCGCC
GCCGCCGCCGTCGGAGTCGTCGGAGTGGGACGCCACAACGAAGATGAAGAAGAAGAAGCGTGGTG
GCGGCGGCGACGACGACTGGGAGGCCGCCTTCCGGGAGTTCATCGCTGGCGACGACGACGACGACGAC
GGCGGCGTTTCCATGTTCCCTTCTGGTGCAGGGACGATGGAGACGACCACAGAGGTGGCGCCGGCGGC
GGCGGTGGTGGAGAGGCCGCGGCGGCGGCGAAGGGTGAGGCGGAGCTACCCGTACCGCGGCGTCCGGC
AGCGGCCGTGGGGGCGGTGGGCGTCGGAGATCCGCGACCCCGTCAAGGGCGCCCGCGTCTGGCTCGGC
ACCTTCGACACCGCCGTCGAGGCCGCGCGCGCCTACGACGCCGAGGCGCGCC

Figure 28 (continued)

GCATCCACGGCCACAAGGCAAGGACCAACTTCCCGCCCGACGAGCCTCCGCTGCCGGCGCCATCGCAG
GCGCCGTTCTGCTTCCTGCTCGACGACGACGACGACGACGACGGCGTGGCCCGTGGAAACAGCCCGGC
GTCGTCGTCGGCGCCGGACAGAGCCTCCGCTTGCACGACGTCGTCGACGGTGGCGTCCGGCGAGCGAG
GCGATGAGCTCATACTGCTGGAGTGCTGCTCCGACGACGTGATGGACAGCCTCCTCGCCGGCTTCGAC
GTGTCCAGCGAACCACGCAGTGTTTTGGGAATGGTTAATTAGCAGCGCGCACGCCTGATTAGATCGGT
ACATGTGAAGTACAAGAGAAGTAGTTACTACTAGCTAGGAGCAAATTAACTTGGAGTGCAAGAATAAA
ATATGCATGTCTCTGCACTACTACTGTAGTGTTAGCAAGTTTGCTCATTGTTCTGTTGTATCCTTTCA
TGTCCATTTCAGACTTCCCAATGCTACATAAGGATGTACATATGTATGATGTTGTGTGCCTTGTTAAT
CAATGAATGTAAATATCTTTATATTGCTTTTGTAC

**SEQ ID NO: 192, LOC_Os09g11480.1|11979.m04410|protein|AP2-EREBP**

MCGGALIPNDYGDKPPPPPSESSEWDATTKMKKKKRGGGDDDWEAAFREFIAGDDDDDDGGVSMFP
SGAGTMETTTEVAPAAAVVERPRRRRRVRRSYPYRGVRQRPWGRWASEIRDPVKGARVWLGTFDTAVE
AARAYDAEARRIHGHKARTNFPPDEPPLPAPSQAPFCFLLDDDDDDDGVARGNSPASSSAPDRASACT
TSSTVASGERGDELILLECCSDDVMDSLLAGFDVSSEPRSVLGMVN

**SEQ ID NO: 193, AY383630.1| Lycopersicon esculentum JERF3 mRNA, complete cds**

GACAATTATACATTTTCCGTCAAAACATAAATCATGTGTGGTGGTTCTATAATCTCCGATTACATAGA
CCCTAGCCGGACTTCTCGCCGGCTCACCGCCGAGTTTCTATGGGGTCGTTTCGATCTCGGTAAGAAGC
AAAAAAATCCCAACAATTATCACTCTAAAGCTAAGCATTTGCGATCTGAAGTTGTTGACGACTTTGAA
GCCGATTTTCAGGACTTCAAAGAGTTATCCGATGATGAGGATGTTCAAGTCGATGTCAAGCCATTTGC
CTTCTCTGCTTCCAAACACTCTACTGGTTCCAAATCTTTGAAAACTGTTGATTCAGACAAGGATGCTG
CTGCTGATAAATCCTCTAAGAGAAAGAGGAAGAATCAATATAGAGGGATCAGACAGAGACCTTGGGGT
AAGTGGGCAGCTGAAATACGTGACCCAAGGAAAGGGGTTCGGGTCTGGCTGGGAACCTTCAATACTGC
AGAAGAAGCTGCCAAAGCTTATGATATTGAGGCGAGGAGGATCAGAGGCAAGAAGGCTAAGGTAAACT
TTCCTGATGAAGCTCCCGCCCCTGCATCAAGACACACTGTTAAGGTGAATCCTCAGAAGGTCCTTCCT
GAGGAGAGCCTGTATTCACTTCAGTCCGACTCAGCAATCATGAACAGCGTGGAGGATGACCATTATGA
TTCTTTTGGATTTTTTGAAGAGAAACCCATGACAAAACAGTATGGATATGAGAATGGGAGCAGTGCTT
CTGCAGATACGGGATTTGGTTCGTTCGTCCCTTCAGCTGGCGGTGATATCTACTTCAACTCTGATGTA
GGAAGCAACTCTTTTGAATGCTCTGATTTTGGTTGGGGAGAGCCATGCTCCAGGACTCCAGAGATATC
ATCTGTTCTGTCAGCTGCTATTGAATGTAATGAAGCTCAATTTGTTGAAGATGCCAATTCTCAGAAAA
AGTTGAAATCATGCACCAACAACCCCGTAGCTGATGATGGAAACCCCCGTTACTATGGTACCTGAAGA
GCTTCCAGCTTTTGAACCTCAGATGAATTTCTTTCATCTCCCATATATGGAGGGAAATTGGGATGCAT
CAGGTGGTAACTTCCTCAACACAAGTGCAACTCAAAATGGTGGTGAAAATGCTATGGACCTGTGGTCC
TTTGATGATGTTCCTTCTTTAATGGGAGGTATCTTTTAAGTCAACATGCCTTGAGTTTTGTAAATAAG
GCTTCATGTGAGTGATTTTTTGCTGTTGTATAATGTACTTAGTGCAAATATTTGATATGTTAATTTGA
TCTCCGTTAACTTGTTCTTTTAGGTGTGTCTGGTGGTGGAAGAAGAATGATCCACAGAGAACCATGAT
TAAGCCATGGATAATGCACTAAGTAGAGCAGTGCATAGGTAATTAGGTTTAATTAACTACTAGTAGAG
ATGTTAAATTCGAGTTTTACTTAAAAACAATTGGGCTGTATGGATTATGAGAATTGATGAGACCTCGA
TGCTTGCTATAACGTTACCTTTTTAGTGTTGCTTTCACAAAAAAAAAAAAAAAAAA

Figure 28 (continued)

**SEQ ID NO: 194, AAQ91334.1| JERF3 [Lycopersicon esculentum]**

MCGGSIISDYIDPSRTSRRLTAEFLWGRFDLGKKQKNPNNYHSKAKHLRSEVVDDFEADFQDFKELSD
DEDVQVDVKPFAFSASKHSTGSKSLKTVDSDKDAAADKSSKRKRKNQYRGIRQRPWGKWAAEIRDPRK
GVRVWLGTFNTAEEAAKAYDIEARRIRGKKAKVNFPDEAPAPASRHTVKVNPQKVLPEESLYSLQSDS
AIMNSVEDDHYDSFGFFEEKPMTKQYGYENGSSASADTGFGSFVPSAGGDIYFNSDVGSNSFECSDFG
WGEPCSRTPEISSVLSAAIECNEAQFVEDANSQKKLKSCTNNPVADDGNPRYYGT

**SEQ ID NO: 195, AY496704.1| Lycopersicon esculentum ethylene-binding protein mRNA, complete cds**

TCATTTCTGTATCAATCAATATTCTTTGTTTCTGCTGTTTTGATGAAATCACACTAAGATGTGTGGTG
GTGCAATTCTTGCTGATATCATTCCTCCTCGTGACCGCCGTTTGTCATCCACCGACCTATGGCCGACT
GATTTCTGGCCAATTTCCACCCAAAATGTTCCTCTCAACCCCAAACGAGCTCGACCCTCTACAGGTGG
TGAGCAGATGAAGAAGAGGCAAAGGAAGAATCTTTACAGAGGGATAAGACAACGTCCATGGGGTAAAT
GGGCTGCTGAAATTCGTGACCCGAGAAAAGGGGTTAGGGTTTGGTTAGGTACTTTCAACACTGCTGAA
GAAGCTGCAAGAGCTTATGATAGAGAAGCTCGTAAAATCAGGGGTAAGAAAGCTAAAGTTAATTTCCC
CAATGAAGATGACGACCATTACTGCTACAGTCATCCAGAGCCCCCTCCCTTGAACATTGCTTGTGATA
CTACTGTTACTTACAATCAAGAATCAAATAACTGTTACCCCTTTTACTCAATCGAGAACGTTGAACCT
GTTATGGAATTTGCAAGTTATAATGGAATTGAAGATGGAGGAGAGGAGATGGTGAAAAATTTGAATAA
CAGGGTTGTAGAGGAAGAGGAGAAAACAGAGGATGAAGTGCAGATACTTTCTGATGAGCTGATGGCTT
ATGAGTCATTGATGAAGTTCTATGAAATACCGTATGTTGACGGGCAATCAGTGGCGGCGACGGTGAAT
CCAGCGGCGGAGACCGCCGTGGGCGGTGGCTCGATGGAGCTTTGGAGTTTTGATGATGTTAGTCGTCT
ACAACCAAGTTATAATGTAGTTTAATTATTGTTTTGTTTAAACTTTTCATAATTTTATTTTATCGAAT
TAGGAAGAATTGAGTTTTTATAATTTAATCAATTGTGTAAAACTATGTTTCTAATTCATTAATATTAT
ATTGGATATGTTGTTTTTAAAAAAAAAAAAAAAAAAAAAAA

**SEQ ID NO: 196, AAR87866.1| ethylene-binding protein [Lycopersicon esculentum]**

MCGGAILADIIPPRDRRLSSTDLWPTDFWPISTQNVPLNPKRARPSTGGEQMKKRQRKNLYRGIRQRP
WGKWAAEIRDPRKGVRVWLGTFNTAEEAARAYDREARKIRGKKAKVNFPNEDDDHYCYSHPEPPPLNI
ACDTTVTYNQESNNCYPFYSIENVEPVMEFASYNGIEDGGEEMVKNLNNRVVEEEEKTEDEVQILSDE
LMAYESLMKFYEIPYVDGQSVAATVNPAAETAVGGGSMELWSFDDVSRLQPSYNVV

**SEQ ID NO: 197, NM_106706.1| Arabidopsis thaliana DNA binding / transcription factor (AT1G80580) mRNA, complete cds**

ATGGAAAACAGCTACACCGTTGATGGTCACCGTCTTCAATATTCCGTTCCGTTAAGCTCCATGCATGA
AACCAGTCAAAACTCCGAAACTTACGGATTATCCAAAGAGTCGCCGTTGGTCTGCATGCCCTTGTTCG
AAACCAACACTACTTCATTCGATATCTCTTCTCTTTTCTCGTTTAACCCAAAACCAGAACCCGAAAAC
ACGCATCGTGTCATGGACGATTCCATCGCCGCCGTCGTGGGCGAAAACGTTCTTTTCGGTGATAAAAA
CAAAGTCTCTGATCACTTGACCAAAGAAGGTGGTGTGAAGCGGGGGCGGAAGATGCCGCAGAAGACCG
GAGGATTCATGGGAGTGAGAAAACGGCCGTGGGGGAGATGGTCGGCGGAGATAAGAGACAGGATAGGG
CGGTGCAGACACTGGTTAGGAACGTTCGACACGGCGGAAGAGGCAGCGCGTGCG

Figure 28 (continued)

TATGACGCGGCGGCGAGGAGGCTTAGAGGGACCAAAGCCAAGACCAATTTCGTGATTCCTCCGCTTTT
TCCCAAGGAAATAGCTCAGGCTCAGGAGGATAATAGGATGAGGCAGAAGCAGAAGAAGAAGAAGAAGA
AAAAAGTGAGTGTGAGGAAGTGTGTTAAAGTCACATCGGTTGCACAGTTGTTCGATGATGCCAATTTT
ATAAATTCTTCTAGTATTAAAGGAAATGTGATTAGTTCTATTGATAATCTTGAAAAAATGGGTCTAGA
GCTTGATTTGAGTTTAGGGTTGTTGTCTAGGAAGTGA

**SEQ ID NO: 198, AT1G80580.1 Arabidopsis AP2-EREBP family transcription factor, protein sequence 256AA**

MENSYTVDGHRLQYSVPLSSMHETSQNSETYGLSKESPLVCMPLFETNTTSFDISSLFSFNPKPEPEN
THRVMDDSIAAVVGENVLFGDKNKVSDHLTKEGGVKRGRKMPQKTGGFMGVRKRPWGRWSAEIRDRIG
RCRHWLGTFDTAEEAARAYDAAARRLRGTKAKTNFVIPPLFPKEIAQAQEDNRMRQKQKKKKKKKVSV
RKCVKVTSVAQLFDDANFINSSSIKGNVISSIDNLEKMGLELDLSLGLLSRK

**SEQ ID NO: 199, AY286010.1| Nicotiana tabacum callus-expressing factor (CEF1) mRNA, complete cds**

GAATTCGGCACGAGAAAAAAGAAAGAAGTTTACTCCGTCAAAAACGAAACTGATTTCTGCATAAAACT
TTTCTGCTGAGAGAAAACAAAAAGCATGTGTGGTGGTGCTATAATCTCCGATTACATTGCCCCGAGCC
GAACTTCTCGCCGGCTCACCGCCGAGTTGCTATGGGGCCGGTCCGATCTGAGTAATAAGCAAAAAAAT
CCTAACAATTATCACTCCAAGCCGTTGAGATCCCAAGTAGTTGACCTAGACGATGACTTCGAGGCTGA
TTTTCAGGACTTTAAAGATTTCTCCGATGACGAGGATGTTCAAGTCGATGTCAAGCCATTTGCCTTCT
CTGCTTCGAAAAACTCTAATGTTGAAGGCTCCAAATCTGTGAAAACTGATGATTCAGACAAGGATGCT
GATAGATCCTCTAAGAGAAAGAGGAAGAATCAGTATAGGGGGATCAGACAGCGACCTTGGGGTAAGTG
GGCAGCTGAAATACGTGACCCAAGAAAAGGGGTTCGGGTGTGGCTGGGAACTTTCAATACTGCAGAAG
AAGCTGCCAGAGCTTATGATGTTGAGGCTAGGAGGATCAGAGGCAATAAAGCTAAGGTAAACTTTCCC
GATGAAGCTCCAGTGCCTGCCTCGAGACGTACTGTTAAGGTGAATCCTCAAAAGGTCCTTCCTAAGGA
GATCCTGGACTCGGTTCAGCCCGACTCGACTATCATAAACAACATGGAGGATTGCTGTTATGATTCTT
TGGGATTTCTTGAAGAGAAACCCATGACGAAGCAGTTTGGATGTGAGGATGGGAGCAGTGCTTCTGGA
GATACGGGATTTGGCTCATTTGCCCCTTCAGCTGGTACCGATATCTACTTCAACTCTGATGTTGGAAG
TAACTCTTTTGACTGCTCTGATTTTGGTTGGGGAGAGCCATGTGCCAGGACTCCAGAGATATCATCCG
TTCTGTCAGCTGTTATTGAAAGCAATGAATCTCAACTTGTTGAAGATGATACCAGTCCAATGAAAAAA
CTGAAATCAAGCCCCATTAATCCAGTAGCTGATGATGGAAATACCGCAAACAAGCTATCTGAAGAGCT
TTCAGCTTTTGAAACCCAGATGAAGTTCCTTCAGATCCCCTATCTGGAGGGAAATTGGGATGCATCAG
TTGATACTTTCCTCAACTCAAGTGCAACTCAGGATGGTGATAATGCTATGGACTTATGGTCCTTTGAT
GATGTTCCTTCTTTATTGGGAGGTGTCTTTTAAGTCAGCATGCCTTGTCTAGTTTTTGTAAATAAGGC
TTCATGTGAGTGAACTTTGCTATTGTTTTGCCTCAAAGAAAGGCTCTTTATTATGTACAGAAGCTTTT
TGAAATGGTAAATAGTTTAATCTCTGTTTAAAAAAAAAAAAAAAAAAAAAAAA

**SEQ ID NO: 200, AAP40022.1| callus-expressing factor [Nicotiana tabacum]**

MCGGAIISDYIAPSRTSRRLTAELLWGRSDLSNKQKNPNNYHSKPLRSQVVDLDDDFEADFQDFKDFS
DDEDVQVDVKPFAFSASKNSNVEGSKSVKTDDSDKDADRSSKRKRKNQYRGIRQRPWGKWAAEIRDPR
KGVRVWLGTFNTAEEAARAYDVEARRIRGNKAKVNFPDEAPVPASRRTVKVNPQKVLPKEILDSVQPD
STIINNMEDCCYDSLGFLEEKPMTKQFGCEDGSSASGDTGFGSFAPSAGTDIYFNSDVGSNSFDCSDF
GWGEPCARTPEISSVLSAVIESNESQLVEDDTSPMKKLKSSPINPVADDGNTANKLSEELSAFETQMK
FLQIPYLEGNWDASVDTFLNSSATQDGDNAMDLWSFDDVPSLLGGVF

Figure 28 (continued)

**SEQ ID NO: 201, AJ606475.1| Fagus sylvatica mRNA for ethylene transcription factor (erf1 gene)**

CAAACACACTGAAGAATTAAGTCATTTGGGAATCAGGTTTCTTGGAAAAACCCCTGCCAAAGCCCCTT
CAAGGCTCTCAGCTTTGAGTCCCCAGATGTGTGGAGGAGCTATAATCTCCGACTTTATAGCGCCAACC
GGGTCGCGGCGGTTGACGGCGGATTATCTCTGGGGCGATCGGAAAAAACCCATTTCAGGAAAGCGATT
CTCGAAGCCTGTAGTCGATTTGGACGACGAATTCGAGCTCGATTTTCAGGGCTTTAAGGACGAGGAGG
AGTCTGATATCGACGAGGAAGAGGTCCTTGTGCAAGATGTCAAGCCCTTCACTTTTTCTGCTCCTCCT
AGCTCTGGATCTAAGCCTGTAAAATCCGTGGAATTCAATGGGCAAGCTGAGAAATCTGCAAAGAGAAA
GAGGAAGAATCAGTATCGGGGGATCCGGCAGCGCCCATGGGGTAAGTGGGCTGCTGAGATTCGAGACC
CAAGGAAAGGGGTCCGTGTCTGGCTTGGAACTTTTAACACTGCAGAAAAAGCTGCAAGAGCTTATGAT
GCAGAGGCACGGAGAATTCGTGGCAAGAAGGCTAAGGTGAATTTTCCCGATGAGACTCCCCGTGCTTC
TCCAAAGCGTTCAGTTAAGGCAAATCTGCAGAAGCCACTTGCCAAGGCAAACCTGAACTCTGTCCAGC
CCAACCTGAACCAAAATTTCAATTTTATGAACAACTCTGATCAGGACTATACCATGGGTTTGATGGAA
GAGAAACCTTTCACAAACCAGTATGGGTATATGGATTCCATCCCTGCCAATGCAGATGTTGGACTAAA
ACCCTTTGCTTCCAATAATACTACCCCGTACTTTAACTCAGATCAGGGGAGTAACTCGTTTGATTGTT
CTGACTATGGATGGGGAGAACAGGGCTCTAAGACTCCAGAAATCTCATCTGTTCTTTCAGCTACTTTA
GAAGGGGATGAATCTCAGTTTGTGGAGGATGCTATGCCCACGAAGAAATTGAAGTCAGACTCTGGGAA
TGCAGTGTTCATTGAAAATAACACTGCAAAGACACTGTCAGAGGAGCTCTCAGCTTTTGAGTCCCAGA
TGAACTTTCAGATGCCATTTCTTGAGGGAAGCTGGGAATCCAACATGGAGGCACTGTTCAGTGGGGAC
ACAACTCAGGATGGTAACTCGATGGATCTTTGGAGCTTCGATGACCTCCCCGTTATGGCTGGGGGAGT
TCTGTGACCGCAAAACTATTTTCCGCATGCTTGCTGTTCTAGTTTATGTATAAATAAGGCTAAATACA
TGTTAGAATGGTTTGTCATTCTGTGGAGATGGACATGCCTGTGGTTTCAAACAAGCTGAACACTGAAT
GCTTAAGACTCTATGAAGGGATGTACTGAAGTAGTGTTGTTCTACTGTTGTATGAGGGAGTACATAGG
TCCCTATTTAGGATCCCTTTGAGAGACTACCTTGAAGACATTGAATTTTGGGATTTTGAATTTGATGT
TTTTGTATTGATGATTATGTGTGATGAAACCTCTGTCATAAAAATACTAAACTAAAAACCTGATGTAT
GTCAACTGTGTTTAGTGTTTGTTTCAAAAAAAAAAAAAAAA

**SEQ ID NO: 202, CAE54591.1| ethylene transcription factor [Fagus sylvatica]**

MCGGAIISDFIAPTGSRRLTADYLWGDRKKPISGKRFSKPVVDLDDEFELDFQGFKDEEESDIDEEEV
LVQDVKPFTFSAPPSSGSKPVKSVEFNGQAEKSAKRKRKNQYRGIRQRPWGKWAAEIRDPRKGVRVWL
GTFNTAEKAARAYDAEARRIRGKKAKVNFPDETPRASPKRSVKANLQKPLAKANLNSVQPNLNQNFNF
MNNSDQDYTMGLMEEKPFTNQYGYMDSIPANADVGLKPFASNNTTPYFNSDQGSNSFDCSDYGWGEQG
SKTPEISSVLSATLEGDESQFVEDAMPTKKLKSDSGNAVFIENNTAKTLSEELSAFESQMNFQMPFLE
GSWESNMEALFSGDTTQDGNSMDLWSFDDLPVMAGGVL

**SEQ ID NO: 73, AJ420195.1| Fagus sylvatica partial mRNA for ethylene responsive element binding protein (erebp1 gene)**

CAACAATATTTTCTCTCACAAAAACTAAACTCTCTGTATTTCCCAAACTTTCCAAAAAAACCATTTTA
CTTTTCACCCATCCGAGCAAAAAAAACAAAGAGAAAAAAGAAACCCCTCCAAAAAAGCCAAGACCGCC
ATGTGTGGAGGAGCTATAATCTCCGACTTTATAGCGCCAACCGGGTCGCGGCGGTTGACGGCGGATTA
TCTCTGGGGCGATCGGAAAAAACCCATTTCAGGAAAGCGATTCTCGAAGCCTGTAGTCGATTTGGACG
ACGAATTCGAGCTCGATTTTCAGGGCTTCAAGGACGAGGAGGAGTCTGATATCGACGA

Figure 28 (continued)

GGAAGAGGTCCTTGTGCAAGATGTCAAGCCCTTCACTTTTTCTGCTCCTCCTAGCTCTGGATCTAAGC
CTGTAAAATCCGTGGAATTCAATGGGCAAGCTGAGAAATCTGCAAAGAGAAAGAGGAAGAATCAGTAT
CGGGGGATCCGGCAGCGCCCATGGGGTAAGTGGGCTGCTGAGATTCGAGACCCAAGGAAAGGGGTCCG
TGTCTGGCTCGGAACTTTTAACACTGCAGAAGAAGCTGCAAGAGCTTATGATGCAGAAGCACGGAGAA
TTCGTGGCAAGAAAAGCAAAGGGAATTTTCCGATGAGACTTCCCGTGCCAAAGCGTTCAGTTAAGGCA
AATCTGCAGAAGCCACTTGCCAAGGCAAACCTGAACTCTGTCCAGCCCAACCTGAACCAAAATTTCAA
TTTTATGAACTCTGATCAGGACTATACCATGGGTTTGATGGAAGAGAAACCTTTCACGAACCAGTATG
GGTATATGGATTCCATCCCTGTCAATGCAGATGTTGGACTAAAATCCTTTGCTTCCAATAATACTGCC
CCATACTTTAACTCAGATCAGGGGAGTAACTCGTTTGATTGTTCTGACTATGGATGGGGAGAACAGGG
CTCTAAGACTCCAGAAATCTCATCTGTTCTTTCAGCCACTTTAGAAGGGGATGAATCTCAGTTTGTGG
AGGATGCTGTGCCCACGAAGAAATTGAAGTCAGACTCTGGGAATGCAGTGTTCATTGAAAATAACACT
GCAAAGACACTGTCAGAGGAGCTCTCAGCTTTTGAGTCCCAGATGAACTTTCAGATGCCATTTCTTGA
GGGAAGCTGGGAATCCAACATGGAGGCACTGTTCAGTGGGGACACAACTCAGGATGGTAACTCGATGG
ATCTTTGGAGCTTCGATGACCTCCCCGTTATGGCTGGGGGAGTTCTGTGAGCAAACTATTCCCATGCT
TGCTAGTTTATGTAAATAAGGCTACATGTTAGAATGGTTTGTCATCTGTGATGGACATGCCGTTTCAA
ACAAGCTGTGAACATGCTTAAGACTCTTATGAAGGATGTACTGAAGTAGTCTACTGTTGTATGAGGAG
TACATAGGTATTTAGGATCCCTTTCTCCCTTGAAGACATTGAATTTGGGATTTTGAATTTGATCTTTG
TATTGATGATTATGTGTGATCTGTCATAAAAATACTAAAAACCTGATGTATGTCAACTTTAGTGTTTG
TTTCTATCTTGGTTCTTTTACAAAAAGGGCCTTCAATTTTTGTACTGTTTATTTGTTTTTATTGGTTT
TTGATTTTACCTAAAAAAAAAAAAAAAAAAA

**SEQ ID NO: 204, CAD21849.1| ethylene responsive element binding protein [Fagus sylvatica]**

MCGGAIISDFIAPTGSRRLTADYLWGDRKKPISGKRFSKPVVDLDDEFELDFQGFKDEEESDIDEEEV
LVQDVKPFTFSAPPSSGSKPVKSVEFNGQAEKSAKRKRKNQYRGIRQRPWGKWAAEIRDPRKGVRVWL
GTFNTAEEAARAYDAEARRIRGKKSKGNFPMRLPVPKRSVKANLQKPLAKANLNSVQPNLNQNFNFMN
SDQDYTMGLMEEKPFTNQYGYMDSIPVNADVGLKSFASNNTAPYFNSDQGSNSFDCSDYGWGEQGSKT
PEISSVLSATLEGDESQFVEDAVPTKKLKSDSGNAVFIENNTAKTLSEELSAFESQMNFQMPFLEGSW
ESNMEALFSGDTTQDGNSMDLWSFDDLPVMAGGVL

**SEQ ID NO: 205, DQ412079.1| Capsicum annuum putative ethylene-responsive element binding protein (JERF1) mRNA, complete cds**

ACGCGGGGAAATTAAACTTTCTCCATTGAAATTCACTGCGAAAAAAAAACCCTTTCCCAGTTATAATA
CACTACTTAAATTATTAGAGAAAAGAAAAAGCTATGTGTGGTGGTGCAATTATCTCCGATTTGGTACC
TCCTAGCCGGATTTCCCGCCGGCTAACCGCCGAGTTGCTATGGGGTAACTCTGATCTGAGCAAAAAGA
AGAAAAATCCAGGGAATTATTACTCAAAGCCTTTGAACAGGTCTAAGTTTATTGACCTTGATGAGGAA
TTTGAAGCTGACTTTCAGGACTTCAAGGACTATGCCGATGACGATGTTGATGATGTTAAGCCCTTCGG
TTCCAAATCTGTGAAATCTGGCGATTCAAGCTGCGATACTGAAAAATCTTCCAAGAGAAAGAGGAAGA
ATCAGTACCGGGGGATCAGACAGCGTCCTTGGGGTAAGTGGGCAGCTGAAATTCGTGATCCGAGGAAA
GGGATTCGAGTTTGGCTTGGAACTTTCAATTCTGCGGAAGAAGCAGCTAGAGCTTATGATGTTGAGGC
ACGAAGGATCAGAGGCAAGAAGGCTAAGGTGAACTTTCCTGATGGATCTCCAGCTTCTGCTTCAAGAC
GTGCTGTTAAGCCAAATCCTCAGGAGGCACTTCGCGAGGAAATCTTGAACACAGTTCAGCCGAACACA
ACTTATATCAACAACTTGGACGGCGGATCTGATGATTCGTTTGGCTTTTTCGAAGAGAAACCAGCAGC
AAAGCAGTATGGCTATGAGAATGTTTCTTTTACTGCTGGAGATA

Figure 28 (continued)

TGGGACTGGGTTCAATTTCCCCTTCAACTGGTACAACAAATGTTTACTTCAGTTCTGATGAAGGAAGC
AACACCTTTGACTGCTCTGATTTCGGTTGGGGTGAACCATGTCCGAGGACTCCAGAGATCTCATCTGT
TCTGTCAGAAGTTCTAGAATGTAATGGTACTCAATCTGATGAAGATGCTAGACCAGAGAAAAAACTGA
AGTCGTGTTCCAACGCTTCCTTGCCAGATGAGGATAACACTGTGCACACGCTATCTGAAGAGCTATCG
GCTTTTGAATCCCAGATGAAGTTCTTGCAGATCCCATATCTTGAGGGAAATTGGGATGCATCAGTTGA
TGCCTTTGTCAACACAGGCGCAATTCAGGATGGCGGAAATGCGATGGATCTCTGGACCTTCGATGATG
TTCCTTCTTTAATGGGAGGTGTCTACTAAGCCAACACGCACCTTCCCTTACTAAGTTTTGTAAATAAA
GCTTCATTTGAGTGAAGTTTGCAGTTATGTTGTCTCCAAACAAAAAAGACTATATATGTGTTGTATTA
AATTTATTTCATAAATTTACTTGTTTGATACAAAAAAAAAAAAA

**SEQ ID NO: 206, ABD65407.1| putative ethylene-responsive element binding protein [Capsicum annuum]**

MCGGAIISDLVPPSRISRRLTAELLWGNSDLSKKKKNPGNYYSKPLNRSKFIDLDEEFEADFQDFKDY
ADDDVDDVKPFGSKSVKSGDSSCDTEKSSKRKRKNQYRGIRQRPWGKWAAEIRDPRKGIRVWLGTFNS
AEEAARAYDVEARRIRGKKAKVNFPDGSPASASRRAVKPNPQEALREEILNTVQPNTTYINNLDGGSD
DSFGFFEEKPAAKQYGYENVSFTAGDMGLGSISPSTGTTNVYFSSDEGSNTFDCSDFGWGEPCPRTPE
ISSVLSEVLECNGTQSDEDARPEKKLKSCSNASLPDEDNTVHTLSEELSAFESQMKFLQIPYLEGNWD
ASVDAFVNTGAIQDGGNAMDLWTFDDVPSLMGGVY

**SEQ ID NO: 207, AY246274.1| Capsicum annuum PF1 mRNA, complete cds**

AGTTATAATACACTACTTAAATTATTAGAGAAAAGAAAAAGCTATGTGTGGTGGTGCAATTATCTCCG
ATTTGGTACCTCCTAGCCGGATTTCCCGCCGGCTAACCGCCGAGTTGCTATGGGGTAACTCTGATCTG
AGCAAAAAGAAGAAAAATCCAGGGAATTATTACTCAAAGCCTTTGAACAGGTCTAAGTTTATTGACCT
TGATGAGGAATTTGAAGCTGACTTTCAGGACTTCAAGGACTATGCCGATGACGATGTTGATGATGTTA
AGCCCTTCGGTTCCAAATCTGTGAAATCTGGCGATTCAAGCTGCGATACTGAAAAATCTTCCAAGAGA
AAGAGGAAGAATCAGTACCGGGGGATCAGACAGCGTCCTTGGGGTAAGTGGGCAGCTGAAATTCGTGA
TCCGAGGAAAGGGATTCGAGTTTGGCTTGGAACTTTCAATTCTGCGGAAGAAGCAGCTAGAGCTTATG
ATGTTGAGGCACGAAGGATCAGAGGCAAGAAGGCTAAGGTGAACTTTCCTGATGGATCTCCAGCTTCT
GCTTCAAGACGTGCTGTTAAGCCAAATCCTCAGGAGGCACTTCGCGAGGAAATCTTGAACACAGTTCA
GCCGAACACAACTTATATCAACAACTTGGACGGCGGATCTGATGATTCGTTTGGCTTTTTCGAAGAGA
AACCAGCAGCAAAGCAGTATGGCTATGAGAATGTTTCTTTTACTGCTGGAGATATGGGACTGGGTTCA
ATTTCCCCTTCAACTGGTACAACAAATGTTTACTTCAGTTCTGATGAAGGAAGCAACACCTTTGACTG
CTCTGATTTCGGTTGGGGTGAACCATGTCCGAGGACTCCAGAGATCTCATCTGTTCTGTCAGAAGTTC
TAGAATGTAATGGTACTCAATCTGATGAAGATGCTAGACCAGAGAAAAAACTGAAGTCGTGTTCCAAC
GCTTCCTTGCCAGATGAGGATAACACTGTGCACACGCTATCTGAAGAGCTATCGGCTTTTGAATCCCA
GATGAAGTTCTTGCAGATCCCATATCTTGAGGGAAATTGGGATGCATCAGTTGATGCCTTTGTCAACA
CAGGCGCAATTCAGGATGGCGGAAATGCGATGGATCTCTGGCCTTCGATGATGTTCCTTCTTTAATGG
GAGGTGTCTATAAGCCAACACGCACCTTCCCTTATTAAGTTTTGTAAATAAAGCTTCATTTGAGTGAA
GTTTGCAGTTATGTTGTCTCCAAACAAAAAAGACTATATATGTGTTGTATTAAATTTATTTCATAAAT
TTACTTGTTTGATGTAAAAAAAAAAAAAAAAAAAAA

Figure 28 (continued)

**SEQ ID NO: 208, AAP72289.1| PF1; CaPF1 [Capsicum annuum]**

MCGGAIISDLVPPSRISRRLTAELLWGNSDLSKKKKNPGNYYSKPLNRSKFIDLDEEFEADFQDFKDY
ADDDVDDVKPFGSKSVKSGDSSCDTEKSSKRKRKNQYRGIRQRPWGKWAAEIRDPRKGIRVWLGTFNS
AEEAARAYDVEARRIRGKKAKVNFPDGSPASASRRAVKPNPQEALREEILNTVQPNTTYINNLDGGSD
DSFGFFEEKPAAKQYGYENVSFTAGDMGLGSISPSTGTTNVYFSSDEGSNTFDCSDFGWGEPCPRTPE
ISSVLSEVLECNGTQSDEDARPEKKLKSCSNASLPDEDNTVHTLSEELSAFESQMKFLQIPYLEGNWD
ASVDAFVNTGAIQDGGNAMDLWPSMMFLL

**SEQ ID NO: 209, AY529642.1| Capsicum annuum ethylene-responsive factor-like protein 1 (ERFLP1) mRNA, complete cds**

ATTCTCAAAAATAATTTTTCATTTCTGATTCAATCTTGTTGTTTCTTTCATTTTGAAAAAAAAAAGAA
GAAGAAGAAGAGTACTTTAACTACACTGCAAAATGTGTGGTGGAGCAATTCTTGCTGATATCATTCCT
CGTCGTGACCGTCGTCTGTCATCCACAGACTTATGGTCAATTTGTTCTGATGATTTCTGGCCAAATTC
TTCATTTTCCAAGCCATTTTCCACCCAAAATGTTTCCCCTGCAAAGCCCAAACGAACTCAACCCTCTG
CAGGTAATGAGCAAATCCAGAAAGCCAAGAAAAGGCAAAGGAAGAATCTATACAGGGGAATCCGCCAG
CGTCCATGGGGTAAATGGGCAGCTGAAATTCGTGACCCAAGAAAAGGGGTCAGAGTCTGGTTAGGTAC
TTTCAACACTGCTGAAGAAGCTGCTAGAGCTTATGACAAAGAAGCTCGTAAAATCCGGGGAGAGAAAG
CTAAAGTTAATTTCCCAAATGAAGATGATCACTACAGTTATCCAGAGCCTCCTCCTCTCGCTGCTTAC
AATAATACTACTTTTTATAATAATTGCTATGCGTTCGAGAACAATGAACCCGTTATCGAATATGCAAT
AGCTAACAACAATGATCAAAATGGGCTGATTAAAGAGGTGGAAAATATGAATGGAAGGGTCGTGGAGG
AGGAGGAAAAAACAGAGATTCAAGTGCAGAAACTCTCTGAGGAACTGATGGCTTATGAGTCATTGATG
AAGTTTTATGAGATCCCTTATGTTGATGGGCAATCAGTGGCGGCGATGGCGAATCCAGCGGCGGAGGC
TGTCTTGGGTGGTGGCTTGATGGAGCTTTGGAGTTTTGATGATGTTAGTCGTCAACAACCTAGTTATA
ATGTAGTTTGATTATTGTTTGTTTACATTGTTGTATGTTTTTATTTTTCGGTTTAGGAGAATGGGTTT
CCTTGTAATTCTCAATGTTTAAGCAATTGGTAAAACTATTGTCTCTAAAAAAAAAAAAAAAAAAAAAA
AAAAAAAAAAAA

**SEQ ID NO: 210, AAS20427.1| ethylene-responsive factor-like protein 1 [Capsicum annuum]**

MCGGAILADIIPRRDRRLSSTDLWSICSDDFWPNSSFSKPFSTQNVSPAKPKRTQPSAGNEQIQKAKK
RQRKNLYRGIRQRPWGKWAAEIRDPRKGVRVWLGTFNTAEEAARAYDKEARKIRGEKAKVNFPNEDDH
YSYPEPPPLAAYNNTTFYNNCYAFENNEPVIEYAIANNNDQNGLIKEVENMNGRVVEEEEKTEIQVQK
LSEELMAYESLMKFYEIPYVDGQSVAAMANPAAEAVLGGGLMELWSFDDVSRQQPSYNVV

**SEQ ID NO: 211, AY781117.2| Gossypium hirsutum ethylene-responsive element binding protein ERF2 mRNA, complete cds**

AAAAAACCAACCCATTTATTTCACTACCCAACAACCCAAAAGATAAAAGGAATCGTTTACTTGTTCGT
AGCAATTTATCGTCATTTCAAGCTCAATTCTCTGAAAATTTTTGTGCTGAACTTCTTTTTTCTCTTTG
TACCACCATGTGTGGAGGTGCTATTATCTCCGATTTCATACCGCCGTCGCGGTCGCGACGATTGACGG
CCGATTTCTTGTGGCCCGATCTGAAAAAATCCGGGTTGAAGAAGGGGTCGGGTAAGAGATACTCGAAG
CCCGTGATCGACTTGGGCGATGATTTCGAGACTGACTTTCAGGAGTTCAAAGATGAAGAATCTGATAT
AGATGATTATGATGTTGATGATGTTTTGGCTGATGTAAAGCCCTTTGCTTTTAACGCTACAAAGAAAC
CTGCTTCTGCTGTCTCTCATGGTTCGAACTCTGAAAAATCCATGCAATTCAATG

Figure 28 (continued)

GTCAAGCTGAGAAATGTGCGAAAAGAAAGAGGAAGAACCAGTATCGTGGAATCCGGCAGCGCCCATGG
GGTAAATGGGCTGCTGAGATCCGTGACCCAAGGAAAGGGGTTAGGGTCTGGTTAGGAACTTTCAATAC
TGCTGAAGAAGCTGCGAGAGCTTATGATGCTGAGGCACGGAGAATTCGTGGTAAGAAAGCTAAGGTGA
ACTTCCCTAACGAGACTCCGCGTACCTCTCCAAAGCATGCAGTCAAGACAAATTCTCAGAAACCACTT
TCCAAGTCAAATTCGAGCCCTGTTCAGCCAAATCTCAACCAGAATTACAATTACTTGAACCAGCCTGA
GCAGGAATACTTTGATACCATGGGTTTCGTAGAAGAGAAGCCATCGGTCAATCAGTTCGCATACGTGG
ACCCTGTTCCTACGTCTATAGATGCTGGATTTAATCAATCAGATAATGCCCCCTTGTACTTCAATTCA
GACCAGGGAAGTAACTCGATCAATTGTTCCGACTATGGCTGGGGAGAACAGGGTGCCAAAACTCCTGA
AATATCATCCATTCTGGAAGCTTCTGTAGAGGGTGATGAGTTTCTTGAGGATGCTAACCCTAGCAAGA
AGCTGAAACCAAGTTCCGACAATGTTATGCCTGCCGAAGACAACTCCGCGAAGACCTTGTCGGACGAG
CTGTTGGCTTTGGACAACCAGATGAAATACTTCCAAATGCCGCCATTTATTGAAGGAAACTGGGACGC
CACTATTGATGCTTTCCTCAATGGAGATGCAACACAGGATGGTGGAAACCCGATGGATCTTTGGAACT
TTGATGATTTCCCTACCATGGCGGAGGGTGTTTTCTGAGCGAACTTTCCATAATAACTAGTGTTTGTA
AATAAAGCAACATGAATTTGGTCAAAATCTGTTGTGAAGTTGAAGTAAAAACCAAGCTATATGCATGC
TTAAGCCTTGCCTGCACTGCTTTCAGAGGTTTTTAGTATGTACCCCTTTTTTATGTGTTTTTTTGTAG
ACTTTGGACTAAATTTTAAATTTGAGTGACTGTATAAGTAACTGTGTCTGAATTTGTCTATGTTTGAA
TACTGAAAAACATATGAATGTTTTAAACCCAAAAAAAAAAAAAAAAA

**SEQ ID NO: 212, AAX07458.2| ethylene-responsive element binding protein ERF2 [Gossypium hirsutum]**

MCGGAIISDFIPPSRSRRLTADFLWPDLKKSGLKKGSGKRYSKPVIDLGDDFETDFQEFKDEESDIDD
YDVDDVLADVKPFAFNATKKPASAVSHGSNSEKSMQFNGQAEKCAKRKRKNQYRGIRQRPWGKWAAEI
RDPRKGVRVWLGTFNTAEEAARAYDAEARRIRGKKAKVNFPNETPRTSPKHAVKTNSQKPLSKSNSSP
VQPNLNQNYNYLNQPEQEYFDTMGFVEEKPSVNQFAYVDPVPTSIDAGFNQSDNAPLYFNSDQGSNSI
NCSDYGWGEQGAKTPEISSILEASVEGDEFLEDANPSKKLKPSSDNVMPAEDNSAKTLSDELLALDNQ
MKYFQMPPFIEGNWDATIDAFLNGDATQDGGNPMDLWNFDDFPTMAEGVF

**SEQ ID NO: 213, AY572463.1| Gossypium barbadense ethylene response factor 1 mRNA, complete cds**

TTGAGAATCGATGCCCGGATTAATAATTTCTGGGATGTAGTCACTAAAAAGGCCTTTCTCATGATTTT
CTTGCAGTTAAAGGTCTTAAATTATATTTCCTTGTGACAGTTATGTTGTGATTTGTAGTTTTTCATGG
ATGAGTAATGTTTATTTATGGTTCATGTATCCGTACGATATTAAATTTTTCTTTTTGTGCTCTATCAT
TGAAGGTTCGAACTCTGAAAAGTCCATGCAGTTCGATGGTCAAGCTGAGAAATGTGCGAAAAGAAAGA
GGAAGAACCAGTATCGTGGAATCCGGCAGCGCCCATGGGGTAAATGGGCTGCTGAGATCCGTGACCCA
AGGAAAGGGGTTAGGGTCTGGTTAGGAACTTTCAATACTGCTGAAGAAGCTGCGAGAGCTTATGATGC
TGAGGCACGGAGAATTCGTGGTAAGAAAGCTAAGGTGAACTTCCCTAACGAGACTCCGCGTACCTCTC
CAAAGCATGCAGTCAAGACAAATTCTCAGAAACCACTTTCCAAGTCGAATTTGAGCCCTGTTCAGCTA
AATCTCGACCAGAATTACAATTACTTGAGCCAGCCTGAGCAGGAATACTTCGATACCATGGGTTTCGT
AGAAGAGAAGCCACTGGTCAATCAGTTTGCATATGTGGACCCTGTTCCTACGTCTATAGATGCTGGAT
CTAATCAATCAGATAATGCCCCCTTGTACTTCAATTCGGACCAGGGAAGTAACTCCATCAATTGTTCC
GACTATGGCTGGGGAGAACAGGGTGCCAGAACTCCTGAAATATCATCCATTCTTGAAGCTTCTGTAGT
GGGTGAAGAGTTTCTTGAGGATGCTAACCCTAGCAAGAAGCTGAAACCAAGTTCTGACAATGTTATGC
CTGCCGAAGACAACTCCGCGAAGACCTTGTCGGACGAGCTGTTGGCTTTGGACAACCAGATGAAATAC
TTCCAAATGCCGCCATTTATTGAAGGAAACTGGGACGC

Figure 28 (continued)

CACTATTGATGCTTTCCTCAATGGAGATGCAACACAGGATGGTGGAAACCCGATGGATCTTTGGAACT
TTGATGATTTCCCTACCATGGCGGAGGGTGTTTTCTGAGCGAACTTTCCATAATAACTAGTGTTTGTA
AATAAAGCAACATGAATTTGGTCAAAATCTGTTGTGAAGTTGAAGTAAAAACCAAGCTATATGCATGC
TTAAGCCTTGCCTGCACTGCTTTCAGAGGTTTTAGTATGTACCCCTTTTTTATGTGTTTTTTTGTAG
ACTTTGGACTAAATTTTAAATTTGAGTGACTGTATAAGTAATTGTGTCTGAATTTGTTTATGTTTGAA
TACTGAAAAACATATGAATGTTTTAAACTCTGCTATTTGTTTCTCCCAAAAAAAAAAAAAAAAAAAAA
AAAAA

**SEQ ID NO: 214, AAT77192.1| ethylene response factor 1 [Gossypium barbadense]**

MSNVYLWFMYPYDIKFFFLCSIIEGSNSEKSMQFDGQAEKCAKRKRKNQYRGIRQRPWGKWAAEIRDP
RKGVRVWLGTFNTAEEAARAYDAEARRIRGKKAKVNFPNETPRTSPKHAVKTNSQKPLSKSNLSPVQL
NLDQNYNYLSQPEQEYFDTMGFVEEKPLVNQFAYVDPVPTSIDAGSNQSDNAPLYFNSDQGSNSINCS
DYGWGEQGARTPEISSILEASVVGEEFLEDANPSKKLKPSSDNVMPAEDNSAKTLSDELLALDNQMKY
FQMPPFIEGNWDATIDAFLNGDATQDGGNPMDLWNFDDFPTMAEGVF

**SEQ ID NO: 215, AY817134.1| Gossypium hirsutum putative ethylene responsive element binding protein mRNA, complete cds**

CGGCACGAGGAGAGAGAGAGAACTAGTCTCGTGCCGGGAAAATTTACAATTAAAAAAATTAATTTTGC
CCTCTAAATTTCTCTAAAAAATCTCCTAAATCCCATACTTTAAATCCAATTCGCCATGTGTGGAGGTG
CGATTATCTCCGATTTCATTCCGCCTGCGCGGTCGCGACTGGTGACGGCGAATTATTTGTGGCCGGAT
CTGAAAAAAATCCGGCTCTAAAAAGCGGTCGGGTAGAAAGCACTCGAAGAAGCCGGCGGTCGGCTTTGA
AGATGACTTCGAGGCTGATTTTCAGGTGTTTAAGGATGAGGATTCTGATGTCGATGACTTCAACGATG
ATGTTGATGATGTTTTGGCTGATGTTAAGTCCTTTGCTTTTTCTGCTACAAAGAAACCCTCTCCTGCT
GTTTCTCATGGTTCAAACTCCATAAAATCTGTGGAATTCAGTGGTCAAGCTGAGAAATCTGCAAAAAG
AAAGAGGAAGAACCAGTATCGCGGAATTCGCCAGCGTCCGTGGGGTAAATGGGCTGCTGAGATCCGTG
ATCCTAGGAAAGGGGTTAGGGTGTGGCTTGGAACTTTTAATACTGCTGAGGAAGCTGCACGAGCTTAT
GATGCTGAGGCACTGAGAATTCGGGGTAAGAAAGCAAAGGTGAACTTCCCTGATGAGACTCCACGTAC
CACTCCAAAGCATGCTGTTAAAATGAATTCTCAGAAACCTCTTTCCGGGTCAAATTTGAGTTCTGTTC
AATCAAGTCTCAACCCAGATTTCAGTTACTCGAACAAACTTGAGCAGGGCTACTATGATACCGTGGGT
TTCATTGAAGAGAAGTCACTAATGGATCAGTTTGCATATGCAGACCCTGTTAGAGCTGCTGTAGATGA
TGGGTTAAAACCCTTTGCTGACCCTGAGAATACCGCTTCGTTTTTTATCTCAGATCCAGGGAGTAACA
ACTTTGACAGTTCCGACCTTGTCTGGGGCGACCAGGGTGCCAAGACTCCTGAAATATCATCCTCTCTT
GAACCTACTCTAGAGGTCAACGAGTTTCTGGACAACGCAAACCCTACGAAGAAGTTGAAACTGAGCTT
GAATAATGTCATGCCTACTGGAGGAGACAATTCGGTAAAGAGTTTATCCGATGAGCTATTAGCTATGG
ACAATCAAGTGAACTACTTTCAGACACCATTTATTGACGAGAACTGGAATGTTTCGATGGATGACTTC
CTTAATGGAGATGCAACTCAGGTTGGCGGAAACGAAATGGGTTTTTGGAGCTTTGATGACTTTCCTTC
CATAGATGGGGGTATTTTCTGAACAAACTCTCCTTACTTATTATCCGTTTCCGTACATAAGGCATCGT
GTTAGCGAGTTTCGACCCTCTGGCAACTGCTTGACTATTGTTTCTATCTTGATATCTCTAACACCGAA
AAATTCAAATTTAAATTTCGAGTGACTGTTAGAACTTACAACATTGATTATGTGTGATTTATGATGAA
AATTTGAAACTCCTATGAATGTTTAACCAAAAAAAAAAAAAA

Figure 28 (continued)

**SEQ ID NO: 216, AAX20013.1| putative ethylene responsive element binding protein [Gossypium hirsutum]**

MCGGAIISDFIPPARSRLVTANYLWPDLKKSGSKKRSGRKHSKKPAVGFEDDFEADFQVFKDEDSDVD
DFNDDVDDVLADVKSFAFSATKKPSPAVSHGSNSIKSVEFSGQAEKSAKRKRKNQYRGIRQRPWGKWA
AEIRDPRKGVRVWLGTFNTAEEAARAYDAEALRIRGKKAKVNFPDETPRTTPKHAVKMNSQKPLSGSN
LSSVQSSLNPDFSYSNKLEQGYYDTVGFIEEKSLMDQFAYADPVRAAVDDGLKPFADPENTASFFISD
PGSNNFDSSDLVWGDQGAKTPEISSSLEPTLEVNEFLDNANPTKKLKLSLNNVMPTGGDNSVKSLSDE
LLAMDNQVNYFQTPFIDENWNVSMDDFLNGDATQVGGNEMGFWSFDDFPSIDGGIF

**SEQ ID NO: 217, AY962572.1| Gossypium hirsutum cultivar Xinhai14 putative ethylene responsive element binding protein 3 mRNA, complete cds**

ATGTGTGGAGGTGCAATTATTTCCGATTTCGTCGCCGTGAAACATGACCCGAAATTGACCGGCGATGA
CCTTTGGTCTGAAATTGACATATTCTCCGACCTCCTAGGTTTCGACAACAATGGCAAAAGCTTTGTCA
ATCATCAGTTTCATAACAACAACAAGAAGCTGATCAATCCAAAAGACAATCAACTTAACAAAGAGAGA
AGCGAGACGATTCAGAAGACAAGCCGAGTCACTAAAAATGTTGAAAAGACTCAGCGAACTCGGAAAAA
CTTTTACCGAGGAATAAGACAAAGGCCATGGGGCAAATGGGCAGCTGAGATTAGAGACCCTCAAAAAG
GCGTCCGAGTTTGGCTCGGTACCCATAACACAGCCGAAGAAGCGGCTCGAGCCTACGATGAAGCCGCC
AAGCGTATCCGTGGTGATAAAGCCAAGCTCAACTTCCCTCAAACGCCCACCAAAAATCAAGCAGCTTC
ACCAGCTCTTACTCAGCTTCCTCCTGCTAAGAAAAGGTGCATCGTTCCCGAGTTAACTCAACCGAGCT
TCCAGACTGACTCACCACCTTATCCTATGGGTCTTGGGTCTGGAAGAAGTGAAGATTATAAGCCGATT
GAAGTGGTGGAGAGTGAGTTGGAGTTGAAAGAACAAATCTGGAGCCTGGAATCATTTCTGGGTTTGGA
GACTAATCATGAGACTATGACTCAACTGAGTGGTAACGGAGGGACTGACTCACTGGAGCTTTGGACAC
TTGATGACCTCGTAACTCAGTATAAGCAACGGCGCTAACATGTTCATCAGTGGGGGCGAAAATTAAAT
AATAGTTAGCGTTAATAAATGTTTTTTGTGTTAATTAGGGTTTTTTTATTTATACTATCATGCATTAT
ATATATATATAGATGAGAATAAAGGATTAACGCTNTGCTCTTGCATGGATTAGTTAGCGTTAATCAAT
GTTAGTGTGTAATTAGGGTTTTTATTATGTTAAAAAAAAAAAA

**SEQ ID NO: 218, AAX68526.1| putative ethylene responsive element binding protein 3 [Gossypium hirsutum]**

MCGGAIISDFVAVKHDPKLTGDDLWSEIDIFSDLLGFDNNGKSFVNHQFHNNNKKLINPKDNQLNKER
SETIQKTSRVTKNVEKTQRTRKNFYRGIRQRPWGKWAAEIRDPQKGVRVWLGTHNTAEEAARAYDEAA
KRIRGDKAKLNFPQTPTKNQAASPALTQLPPAKKRCIVPELTQPSFQTDSPPYPMGLGSGRSEDYKPI
EVVESELELKEQIWSLESFLGLETNHETMTQLSGNGGTDSLELWTLDDLVTQYKQRR

**SEQ ID NO: 219, AY962571.1| Gossypium hirsutum cultivar Xinhai14 putative ethylene responsive element binding protein 2 mRNA, complete cds**

ATGTGTGGAGGTGCAATTATTTCCGATTTCATTGCCGTGAAACGCGGTCGGAAGTTAACCGCCGAGGA
TCTTTGGTCTGAACTTGACACATTCTCCGACCTGTTGGGTCTGGATTACGGAAATGGAAAAGAGTCTT
CTTTCACTCAGTCCGACAACACCAAGGCCGGTTCCAAAGCCAAGAACCTTGAAAAAGTGGCAAACGAG
ACGACTCAGAAGACAAGCCGAGGGAGAGAGAAAGAAGGGAAGACTCAGCGAACTCGAAAG

Figure 28 (continued)

AACATTTACAGAGGAATCAGGCAAAGGCCATGGGGGAAATGGGCGGCTGAGATAAGAGATCCTCACAA
AGGTGTCCGAGTCTGGCTCGGTACATACAACACGGCTGAAGAAGCGGCTCGAGCCTACGATGAAGCCG
CCAAGCGCATCCGTGGGGAGAAAGCCAAGCTCAACTTCCCTCAAACTCCACACCTAACTCAGCCTCCT
GCTAAGAAAAGGTGTATGATGGCTCCTGAGTTGACTCCGCCGAGTTCTGAAACAAAGAGCCCACCAAC
CCCACAACTTTTTATGGGTTTTGGTTACGAGAATGGAGTTTACAGACCGAGTGAAGCAATGGAAAGCG
AGATGGAGCTGAAGGAGCAAATCTCGAGCCTGGAGTCGTTCCTGGGTTTGGAGCCTGATGAGACAACA
ACTGAGTTGAGTGGAAGTGCTGAGCCTGACTCAGTGGACCTTTGGATGCTTGATGACCTTGTGACACA
TCATCAACAACAGCCTCAGCTCTTTTATTAGAAATTAAAAAGTTTTAATTAGGGTAATGTTATGTTTG
ATATGATTATGCTTAAGACTTTAATGTTTGTATTAATGAAGGGTAAATAGTTGTGACAATAAAAAGAT
TGGCCACCCAGTTTTAATTTATATTTATTTCTAATTTGGGTGACATGTAAATTGGTTTCGAGAAACAT
TGAAGTACCCATAATGTTAGTTCATGTATCAGATATAGTAAACACTTTGGATTAATAAAAAAACCTCC
TAATATTTTATTTGTAAAAAAAAAAAAAAAA

**SEQ ID NO: 220, AAX68525.1| putative ethylene responsive element binding protein 2 [Gossypium hirsutum]**

MCGGAIISDFIAVKRGRKLTAEDLWSELDTFSDLLGLDYGNGKESSFTQSDNTKAGSKAKNLEKVANE
TTQKTSRGREKEGKTQRTRKNIYRGIRQRPWGKWAAEIRDPHKGVRVWLGTYNTAEEAARAYDEAAKR
IRGEKAKLNFPQTPHLTQPPAKKRCMMAPELTPPSSETKSPPTPQLFMGFGYENGVYRPSEAMESEME
LKEQISSLESFLGLEPDETTTELSGSAEPDSVDLWMLDDLVTHHQQQPQLFY

**SEQ ID NO: 221, AY572462.1| Gossypium barbadense ethylene response factor 2 mRNA, complete cds**

CCATCTGAAAAAAAAACAGGTTTTCTTTTAAGATAACACCCAAAAATGTGTGGAGGTGCAATTATTTC
CGATTTCGTCGCCCCCAAATATGACCGGAAATTGACCGGCGATGACCTTTGGTCTGAACTTGACATAT
TCTCCGACCTCCTAGGTTTCGACAACAATGGCAAAAGCTTTGTCAATCATCAGTTTCATAACAACAAC
AACAACAAGCTCATTAATCCAAAAGACAATCAACTTAACAAAGAGAGAAGCGAGACGATTCAGAAGAC
AAGCCAAGTCACTAAAAAGGTTGAAAAGACTCAGCGAACTCGGAAAAAACTTTTACAGAGGAATAAGAC
AAAGACCATGGGGCAAATGGGCAGCTGAGATTAGAGACCCTCAAAAAGGCGTTCGAGTTTGGCTCGGT
ACCTATAACACAGCCGAAGAAGCGACTCGAGCCTACGATGAAGCAGCCAAGCGTATCCGTGGTGATAA
AGCCAAGCTCAACTTCCCTCAAACGCCCACCAAAAATCAAGCAGCTTCACCAGCTCTTACTCTGCTTC
CTCCTGCTAAGAAAAGGTGCATCGTTCCCGGAGTTAACTCAACCGAGTTTACCAGACTGGACTCACCA
CCTATATCCCTATGGGTTCTTGGGGTATGAGAAGAAGTGAAAGATTATAAGCCGATTGAAGTGGTGGA
GAGTGAGTTGGAGTTGAAAGAACAAATCTGGAGCCTTGGAATCATCCCTGGGTTTGGAGACTAACCAT
GAGACTATGACTCAGCTGAGTGGTAACGGTGGGACTGACCCACTGGAGCTTTGGACACTTGATGACCC
CGTAACCCAGTATAAGCAACGGCGCTAACATGTTCATTAGTGGGGGCGAAAATTAAATAATAGTTAGC
ATTAATCAATGTTTTTGTGTTACTTAGGGTTTTTTATTTATGCTATCATGCATTATATATATATATG
AGAATAAAGGATTAACGCTTTGCTCTTGCATGGATTAGTTAGCGTTAATCAATATTTGTGTTTAGGGT
TTTTTATTATGTTGTTATATATGTATATGCTTATGACTTTCATGTTTGTATCAATGCATTAGTTGCAT
TATATATATAAGAATAAAGGATTTAACAAAAAAAAAAAAAATAAAAAAAAAAAAAA

Figure 28 (continued)

**SEQ ID NO: 222, AAT77191.1| ethylene response factor 2 [Gossypium barbadense]**

MCGGAIISDFVAPKYDRKLTGDDLWSELDIFSDLLGFDNNGKSFVNHQFHNNNNNKLINPKDNQLNKE
RSETIQKTSQVTKKVEKTQRTRKNFYRGIRQRPWGKWAAEIRDPQKGVRVWLGTYNTAEEATRAYDEA
AKRIRGDKAKLNFPQTPTKNQAASPALTLLPPAKKRCIVPGVNSTEFTRLDSPPISLWVLGV

**SEQ ID NO: 223, AY779338.1| Gossypium hirsutum AP2/EREBP transcription factor ERF-2 mRNA, complete cds**

GCACGAGGATCTAAGAACCCGAAAAGTAGAGCTTTGGTTCTCTGTCTGCTGCTTCTTTCTTCTCTTGG
TACTTCATTATTTTTCCTTTCACTTCCTTTTTGTGAAAAAACATTGAAACCCTTTTCAAGACGCAATA
GAAGATGTGTGGAGGTGCAATCATTTCCGATTTCATTGCCGTGAAACGCGGTCGGAAGTTAACCGCCG
AGGATCTTTGGTCTGAACTTGACACATTCTCCGACCTGTTGGGTCTGGATTACGGAAATGGAAAAGAC
CCTTTCACTCAGTTCGACAACACCAAGGCCGGTTCCAAAGCCAAGAACCTTGAAAAAGTGACAAACGA
GTCGACTCAGAAGACAAGCCGGGGGAGAGAGAAAGAAGGGAAGACTCAGCGAACTCGAAAGAACATTT
ACAGAGGAATCAGGCGAAGGCCATGGGGGAAATGGGCGGCTGAGATAAGAGATCCTCACAAAGGTGTC
CGAATCTGGCTCGGTACATACAACACGGCTGAAGAAGCGGCTCGAGCCTACGATGAAGCCGCCAAGCG
CATCCGCGGGGACAAAGCCAAGCTCAACTTCCCTCAAACTCCACGCCTAACTCAGCCTCCTGCTAAGA
AAAGGTGTATGATGGCTCCTGAGTTGACTCCACCGAGTTCTGAAACCAAGAGCCCACCAACCCCACAA
CCTTTTATGGGTTTTGGTTACGAGAATGGAGTTTACAGGCCGAGTGAAGCAATGGAAAGCGAGATGGA
GCTGAAGGAGCAAATCTCGAGTCTGGAGTCCTTCCTGGGTTTGGAGCCTGATGAGATGACAACTGAGT
TGAGTGGAAGCGCTGAGCCTGAGTCAGTGAACCTTTGGATGCTTGATCACCTTGTGACACATCATCAA
CAACAGCCTCAGCTCTTTTATTAGAAATTAAAAAGTTTAAATTAGGGTAATGTTATGTTTGATATGAT
TATGCTTAAGACTTTAATGTTTGTATTAATGAAGGGTAAATAGTTGTGACAATAAAAAGATTGGCCAC
CCAGTTTTAATTTATATTTATTTCCTAAAATTCTAAAAAAAAAAAAAAAAAAAAAAAAA

**SEQ ID NO: 224, AAV51937.1| AP2/EREBP transcription factor ERF-2 [Gossypium hirsutum]**

MCGGAIISDFIAVKRGRKLTAEDLWSELDTFSDLLGLDYGNGKDPFTQFDNTKAGSKAKNLEKVTNES
TQKTSRGREKEGKTQRTRKNIYRGIRRRPWGKWAAEIRDPHKGVRIWLGTYNTAEEAARAYDEAAKRI
RGDKAKLNFPQTPRLTQPPAKKRCMMAPELTPPSSETKSPPTPQPFMGFGYENGVYRPSEAMESEMEL
KEQISSLESFLGLEPDEMTTELSGSAEPESVNLWMLDHLVTHHQQQPQLFY

**SEQ ID NO: 225, AY827548.1| Gossypium hirsutum EREB1 transcription factor mRNA, complete cds**

ATGAGACTATGCGGCCGCTGTTACAAGTCGACGAATCAACTTAACAAAGGGAGAAGCGAGACGATTCA
GAAGACAAGCCGAGTCACTAAAAATGTTGAAAAGACTCAGCGAACTCGGAAAAACTTTTACCGAGGAA
TAAGACAAAGGCCATGGGGCAAATGGGCAGCTGAGATTAGAGACCCTCAAAAAGGCGTCCGAGTTTGG
CTCGGTACCCATAACACAGCCGAAGAAGCGGCTCGAGCCTACGATGAAGCCGCCAAGCGTATCCGTGG
TGATAAAGCCAAGCTCAACTTCCCTCAAACGCCCACCAAAAATCAAGCAGCTTCACCAGCTCTTATTC
AGCTTCCTCCTGCTAAGAAAAGGTGCATCGTTCCCGAGTTAACTCAACCGAGTTTCCAGACTGACTCA
CCACCTTATCCTATGGGTCTTGGGTCTGGAAGAAGTGAAGATTATAAGCCGATTGAAGTGGTGGAGAG
TGAGTTGGAGTTGAAAGAACAAATCTGGAGCCTGGAGTCGTTCCTGGGTTTG

Figure 28 (continued)

GAGCCTGATGAGACAACAACTGAGTTGAGTGGAAGTGCTGAGCCTGACTCAGTGGACCTTTGGATGCT
TGATGACCTTGTGACACATCATCAACAACAGCCTCAGCTCTTTTATTAGAAATTAAAAAGTTTTAATT
AGGGTAATGTTATGTTTGATATGATTATGCTTAAGACTTTAATGTTTGTATTAATGAAGGGTAAATAG
TTGTGACAATAAAAAGATTGGCCACCCAGTTTTAATTTATATTTATTTCTAATTTGGGTGACATGTAA
ATTGGTTTCGAGAAACATTGAAGTACCCATAATGTTAGTTCATGTATCAGATATAGTAAACACTTTGG
ATTAATAAAAAAACCTCCTAATATTTTATTTGTAAAAAAAAAAAAAAA

**SEQ ID NO: 226, AAV85777.1| EREB1 transcription factor [Gossypium hirsutum]**

MRLCGRCYKSTNQLNKGRSETIQKTSRVTKNVEKTQRTRKNFYRGIRQRPWGKWAAEIRDPQKGVRVW
LGTHNTAEEAARAYDEAAKRIRGDKAKLNFPQTPTKNQAASPALIQLPPAKKRCIVPELTQPSFQTDS
PPYPMGLGSGRSEDYKPIEVVESELELKEQIWSLESFLGLEPDETTTELSGSAEPDSVDLWMLDDLVT
HHQQQPQLFY

**SEQ ID NO: 227, AY781119.1| Gossypium hirsutum ethylene-responsive element binding protein ERF6 mRNA, complete cds**

ATAACCGTCGGTCTATAAATCCTAGACCCCAATACCAAAGCTTTTTCCATCTGAAAAAAAAACAGGTT
TTCTTTTAAGATAACACCCAAAAATGTGTGGAGGTGCAATTATTTCCGATTTCGTCGCCCCCAAATAT
GACCGGAAATTGACCGGCGATGACCTTTGGTCTGAACTTGACATATTCTCCGACCTCCTAGGTTTCGA
CAACAATGGCAAAAGCTTTGTCAATCATCAGTTTCATAACAACAACAACAACAAGCTCATTAATCCAA
AAGACAATCAACTTAACAAAGAGAGAAGCGAGACGATTCAGAAGACAAGCCAAGTCACTAAAAAGGTT
GAAAAGACTCAGCGAACTCGGAAAAACTTTTACAGAGGAATAAGACAAAGACCATGGGGCAAATGGGC
AGCTGAGATTAGAGACCCTCAAAAAGGCGTCCGAGTTTGGCTCGGTACCTATAACACAGCCGAAGAAG
CGGCTCGAGCCTACTATGAAGCCGCCAAGCGTATCCGTGGTGATAAAGCCAAGCTCAACTTCCCTCAA
ACGCCCACCAAAAATCAAGCAGCTTCACCAGCTCTTACTCAGCTTCCTCCTGCTAAGAAAAGGTGCAT
CGTTCCCGAGTTAACTCAACCGAGTTTCCAGACTGACTCACCACCTTATCCTATGGGTCTTGGGTATG
GAAGAAGTGAAGATTATAAGCCGATTGAAGTGGTGGAGAGTGAGTTGGAGTTGAAAGAACAAATCTGG
AGCCTGGAATCATTCCTGGGTTTGGAGACTAACCATGAGACTATGACTCAGCTGAGTGGTAACGGTGG
GACTGACTCACTGGAGCTTTGGACACTTGATGACCTCGTAACTCAGTATAAGCAACGGCGCTAACATG
TTCATTAGTGGGGGCGAAAATTAAATAATAGTTAGCGTTAATCAATGTTTTTGTGTTACTTAGGGTTT
TTTTATTTATGCTATCATGCATTATATATATATATATGAGAATAAAGGATTAACGCTTTGCTCTTGCA
TGGATTAGTTAGCGTTAATCAATATTTGTGTGTTAATTAGGGTTTTTTATTATGTTGTTATATATGTA
TATGCTTAAGGACTTTCATGTTTGTATCAATGCATTAGTTGCATTATATATATTAAGAATAAAGGGAT
TTTACACGTTGCAGTTTGAAAAAAAAAAAAAAAA

**SEQ ID NO: 228, AAX07460.1| ethylene-responsive element binding protein ERF6 [Gossypium hirsutum]**

MCGGAIISDFVAPKYDRKLTGDDLWSELDIFSDLLGFDNNGKSFVNHQFHNNNNNKLINPKDNQLNKE
RSETIQKTSQVTKKVEKTQRTRKNFYRGIRQRPWGKWAAEIRDPQKGVRVWLGTYNTAEEAARAYYEA
AKRIRGDKAKLNFPQTPTKNQAASPALTQLPPAKKRCIVPELTQPSFQTDSPPYPMGLGYGRSEDYKP
IEVVESELELKEQIWSLESFLGLETNHETMTQLSGNGGTDSLELWTLDDLVTQYKQRR

Figure 28 (continued)

**SEQ ID NO: 229, AY973613.1| Manihot esculenta ethylene response factor mRNA, complete cds**

CTGCAGCGACAACCACCATGTGTGGCGGTGCTATCATCTCCGACTTCATCCCTGCCACCGCTGCTGGC
CGATCCTCGCGACGGTTGACAGCGGACTTTCTCTGGCCTGATCTAAAGAAGCCCATTGGGAAAATCGT
TGGTGACCTTGACGATGATTTCGAGGCTGATTTCCAGGAGTTTAAGGATGAGTCTGATGTCGATGAGG
AAGATGACGTTTTGTTTGATGTCAAGCCTTTCTCTTTCTCTGCTACTGCTTCTCCTCCTCCTCGCAAT
CGCAGTCCTTCACGTGGTTCTACAGCTGTAAAGTCTGTGGAATTTAATGGGCTAGCTGAAAAATCTGC
AAAGAGAAAGAGAAAAAACCAGTACAGAGGAATCCGGCAGCGCCCATGGGGAAAATGGGCTGCTGAGA
TTCGTGACCCCAGGAAAGGGGTGCGTGTCTGGCTAGGAACATTCAATACTGCTGAAGAAGCTGCAAGA
GCGTATGATGCTGAGGCACGTAGAATTCGTGGCAAGAAAGCTAAAGTGAACTTTCCTGATGAAGCTCC
ACGTGCTTCCCCAAAGCGGACAATGAAGGCAAACCCTCAGAAACCACTTCCTAAGAGAAATGCTACTG
AGAGTATGAGTTACTTGAACAATCCAGATCAGGACTACTTCAATACTTTGGGCTCTGTTGATGAGAAA
CCACTAGTGAGCCAGTTTGATTTGATGGACTCTTTTCCTGCCAATGGAGATGCTACAGTCAAATCTAT
TCCTCCATGTGATAATGTTCCCACGTTTTTCAATTCTGATCAGGGAAGCAACTCATTTGAATGTTCCG
ACTTTGGATGGGGGGAGCAGGCCTCAAAGACTCCTGAAATCTCATCTGTTCTTTCAGCTACTCCAGAA
ATTGATGAATCACTTTTCATGGATGATGCTAACCCTAAAAAGAAGATGAAGTCTGACTCTGAAAATGC
AGTTCCTATAGAAGAAAGCAATGGAAAATCTCTATCAGAGGAGTTGTTGGCTTTTGACAACCAGATGA
ACTTTCAGATGCCTTATCTTGAGGGAAGTTGGGAGGCTTCACTTGATGGCTTCCTTAATGGAGACGTG
ACTCAGGATGGTGGAAATCCAATGGACCTGTGGAGCTTTGATGACCTCCCTAATATGGTTGGGGGAGT
TTATTGAGCAAAATCATAATTGCCGGTTGGCTTCTGTAAATAAGGCTACATGGATGGTTTTCTCTCTG
AAATGTATTACAAGCACATCTGAACATGCTTAATCCTTTGAGGAGAGTGTCCTAGGGAGCTTTTGGTA
CAAGAGTTGTAGTAAATAGGAATATTTAGTTTCCCTTTTACATTTTGAGACACTGGACTTGGGTTGTT
CAATTTAATAACTGTAATTGACAATGTGCGTGTGATTTGTGTTTAAAACTGATATAAATTTTATCTCT
ACTGTTGTTTTTAAAAAAAAAAAAAAAAAAAA

**SEQ ID NO: 230, AAX84670.1| ethylene response factor [Manihot esculenta]**

MCGGAIISDFIPATAAGRSSRRLTADFLWPDLKKPIGKIVGDLDDDFEADFQEFKDESDVDEEDDVLF
DVKPFSFSATASPPPRNRSPSRGSTAVKSVEFNGLAEKSAKRKRKNQYRGIRQRPWGKWAAEIRDPRK
GVRVWLGTFNTAEEAARAYDAEARRIRGKKAKVNFPDEAPRASPKRTMKANPQKPLPKRNATESMSYL
NNPDQDYFNTLGSVDEKPLVSQFDLMDSFPANGDATVKSIPPCDNVPTFFNSDQGSNSFECSDFGWGE
QASKTPEISSVLSATPEIDESLFMDDANPKKKMKSDSENAVPIEESNGKSLSEELLAFDNQMNFQMPY
LEGSWEASLDGFLNGDVTQDGGNPMDLWSFDDLPNMVGGVY

**SEQ ID NO: 231, Populus alba x Populus tremula apetala2/ethylene responsive factor (ERF1) mRNA, complete cds**

ATAATCACCATCAATCAACATGTGTGGCGGTGCTATCATCTCAGGCTTCATCGCTCCGACAACCACCG
CTCGATCTTCTCGGCGGTTGACCTCGGGCTTTGAGTGGCTTGAGCCGAAGAAACCCTTCAACAACAAG
CACTTGAAGCCAGTTGTTGCTGATCCCGAAGATGATTTTGAGGCTGATCTTCAAGAGTTTAAGGACGA
GTCTGATGTCGACGAGGATTATGATGTCTTTGCTGATGCCAAGCCTTTTGCTTTCTCTGCCAGTGCTT
CTGAACCTGCTAAAAAACGTGGGCTCCCTCGTGGTTCTACTGCTGTTAAATCTGCTGGATTCAGTGGA
CTTGCTAAAAACTCAGCAAAGAGGAAGAGAAAGAACCAGTTTAGAGGAATTAGGCAGCGTCCATGGGG
AAAATGGGCTGCTGAGATTCGTGATCCCAGGAAAGGGGTACGTGTCTGGTTGGG

Figure 28 (continued)

AACATTCAATACTGCAGAAGAAGCTGCTAGAGCATATGATTCTGAGGCACGTAGAATTCGTGGCAAGA
AAGCGAAGGTGAACTTCCCTGATGAAGCTCCATGTGCTTCAGCAAGGCATCCAATTAAGGAAAACTCA
CAGAAACGACTTACAAAGGCAAATTTAAGCCAGGATTTTAGTTACTTGAGCAACCCAGAAACGGATTA
TAATAATATGGGCTTTGTGGAAGAGAAACCACAAGTGAGCCAGTTTGGAATAATGAATTCTATCCCGG
TCAATGGAGATTCTGGGGTGACGCCCTTAACTCCTTCTGACAATGCTTCTATGTATTTCAATTCTGAC
AAGGGGAGCAACTCATTTGATTGTGACTTTGGGTGGGGAGAACAAGGCGCTGAAATCTTGTCTGTTCT
TGCAGCAACTCCAGAAGTTGATGAATCCGTCTTTGTGGAAGCTAATCCTAAGAAGTTGAAATCATACA
CTGAGTATGCAGTGCCTGTTGAAGAGAGGAATGGAAAATCTCTGTCCGAAGAGTTGCTGGCTTTTGAC
AATCAGTTGATGAACCTTCAGATGCCAGATCTTGTGGGTAACTGGGAGGCTTCTCTTGATAGCTTCCT
TAATGGAGACACAACTCAGGATGGCACAAACGCAGTGGACTTGTGGAGCTTCGAAGACTTCCCCTCCA
TGGTTGGGGGAGTTTATTGAGCCAACTTTTCTGTGCTTGCCAGGTTTTGTAAATAATAAGGCTACATG
AATGGTTGTTTATCTGAGATGGGAATGGAGTGCAACACAGATGAACATGCTTAGCCTTGGTGTGGGAG
AAGCATTCTCAGGAGCATTTTCCATATAAAAGTAGTACATCGGTTTCCCGTTTCATTACATTTGGAGT
CACTGGACTTTGGAAGTTGGATTCGATGACTGTAATTTGACAATGTGGTGTGACTTATTTAGA

**SEQ ID NO: 232, AAW33881.1| apetala2/ethylene responsive factor [Populus alba x Populus tremula]**

MCGGAIISGFIAPTTTARSSRRLTSGFEWLEPKKPFNNKHLKPVVADPEDDFEADLQEFKDESDVDED
YDVFADAKPFAFSASASEPAKKRGLPRGSTAVKSAGFSGLAKNSAKRKRKNQFRGIRQRPWGKWAAEI
RDPRKGVRVWLGTFNTAEEAARAYDSEARRIRGKKAKVNFPDEAPCASARHPIKENSQKRLTKANLSQ
DFSYLSNPETDYNNMGFVEEKPQVSQFGIMNSIPVNGDSGVTPLTPSDNASMYFNSDKGSNSFDCDFG
WGEQGAEILSVLAATPEVDESVFVEANPKKLKSYTEYAVPVEERNGKSLSEELLAFDNQLMNLQMPDL
VGNWEASLDSFLNGDTTQDGTNAVDLWSFEDFPSMVGGVY

**SEQ ID NO: 233, AB236754.1| Trifolium pratense RNA for putative transcription factor EREBP, complete cds, clone: C1500**

GAATCAATCAACAAAACAAAACCCAGTAAAAATTTCAATCCTTTTTAACAAAATCTTCAATTGGGTAT
AAGAAAGTTTCAATCTTTTTCTTGTTAAGATAAAGACAAAGATCTGACAAAAACAGAAAGATGTGTGG
TGGTGCTATTATTTCCGACTTCATTCCAGCTGCGGCGGCGGTGGGTGGTTCCCGCCGTGTCACTGCTG
ATATCTTGTGGCCAAATTTGAGGAAAACTGGTTCCAAAAAATCATCTTTTTTGCTTGACGATGATTTT
GAAGCTGGTTTCAGACAGTTTAAGGATGATTCTGATTTCGATGAAGACGAGGATGAAGATGACGATGA
AGGGTTGTTGGTCGGTGTCAAAGGTTTTACCTTTGCTGCTTCTAACAACAAATCTTCAAGGAACTTCT
CTCGTGGCTCGGCTGGTGCAAAATCCGTGGCATCGAAATCAAATGAGCAAGCTGAAAAGGAATCAAAG
AGAAAGAGGAAGAATCAGTATAGGGGTATCCGCCAACGTCCATGGGGAAAATGGGCAGCTGAGATCCG
TGATCCAAGGAAAGGAGTCCGTGTTTGGCTCGGAACTTTCAACACTGCTGAAGAAGCTGCAAGAGCTT
ACGATGCTGAAGCTAGGAGAATCCGTGGCAAGAAAGCCAAGGTGAATTTCCCCGATGAGGCTCCAAAT
GCTTCCTCAAAACGTCTGAAGACAAATCCTGATAACCAGCTGTTGAACAAAAATCTGAACTCTTTCAA
GCCGAACGGAAACAACAAAATGTTCAATTTCAGCGAGAATATGGAGAACTTCTATTCTCCTATGGATC
AGGTTGAACAGAAACCATTGGTTAACAACCAATATGGTGCTGCTGACATTGGAGCCTTCACTGGAAAC
GGAGTTCACCTTGCACCTGCTGATGTTAATGCTTATTTCAGTTCTGAGCATTCAAGCAACTCGTTTGA
TTATTCTGATTTATGTTGGGGGGAACAAGGCCCGAAAACCCCTGAGATTTCCTCGGTGTTTTCTGCTC
CTCTCGAAGCTGAACCTCAGATGAACATGCAGTCTAACAACTCTCAGGATATGCTACCTATGCAAGCT
GAATCTGCAAAGACACTCTCTGAGGAGCTTGCAGATATCGAATCCCAGCTGAAGTTCTTCGAGAACTC
ATTCGATGATAACTGGAGTGATGCTTCACTTG

Figure 28 (continued)

CGTCTTTGCTCGGTGCTGATGTAACTCAGGATGCTGGAAACACAATGAACCTTTGGAGCTTTGATGAC
CTGCCTTCCATCGCAGGCGGAGTTTTCTGAACAACTTTCGCCTCACCGGTTATGTAAATAAAGCTACA
AGTTAGTAACTTTCAGTTTACATTCGGTTTGGTTCATTTTGTTTTTACAAGGTGGATAATTAAATTGT
TTTGTTTTCAGGATTGTTGGTTTATTTTTATATTGTTGATGGAATCAGAAACAAGAACAAGTTACCAG
CTTTAATTAAGCGTGGAGTTTTATTCTCTTGTGGCAAAAATAAATTATTTATAGGTTTTTATT

**SEQ ID NO: 234, BAE71206.1| putative transcription factor EREBP [Trifolium pratense]**

MCGGAIISDFIPAAAAVGGSRRVTADILWPNLRKTGSKKSSFLLDDDFEAGFRQFKDDSDFDEDEDED
DDEGLLVGVKGFTFAASNNKSSRNFSRGSAGAKSVASKSNEQAEKESKRKRKNQYRGIRQRPWGKWAA
EIRDPRKGVRVWLGTFNTAEEAARAYDAEARRIRGKKAKVNFPDEAPNASSKRLKTNPDNQLLNKNLN
SFKPNGNNKMFNFSENMENFYSPMDQVEQKPLVNNQYGAADIGAFTGNGVHLAPADVNAYFSSEHSSN
SFDYSDLCWGEQGPKTPEISSVFSAPLEAEPQMNMQSNNSQDMLPMQAESAKTLSEELADIESQLKFF
ENSFDDNWSDASLASLLGADVTQDAGNTMNLWSFDDLPSIAGGVF

**SEQ ID NO: 235, AF537220.1| Glycine max ethylene responsive protein (EREB) mRNA, complete cds**

ATTGAGCCAAAGCTTTGTATTTTCTGCGATAATTTTCCATTGGGTGGAAGAAAGTCTCAACCTTTATT
CGAAAGAGCAAGGATCTGAGTTGAGTTGAGTGATCATGTGTGGTGGTGCGATTATCTCCGACTTCATA
CCGGCAGGTCCCGCCAGCGGGGCGCGGCGCGTGACCGCCGACATCCTGTGGCCGAGTTTGAGGAAGCG
CTTCTCGAAGCCGCTGCTGGACGATGATTTCGAGGCTGGGTTCAGAGAATTCAAGGATGATTCGGAAA
TCGAGGATGTTGATGACGAGGACGATGAAGACGAGGAGGAGTTGAAGAAGAAGCCCTTTGGGTTCTCT
CGCTCCAGCAACAAGGCTGCTTCTAAGCCTCTCTCTCGTGGAGCAACAACTGTGAAATCTGTGGAATC
AAAGGGGCAAGCTGAGAAGTGTGCCAAGAGAAAGAGGAAGAACCAGTATCGCGGAATCCGCCAGCGTC
CATGGGGAAAGTGGGCTGCTGAGATTCGCGACCCAAGAAAGGGGGTTCGTGTTTGGCTTGGAACTTTC
AGCACTGCTGAAGAAGCTGCAAGAGCTTACGATGCTGAAGCAAGGAGGATCCGTGGCAAGAAAGCCAA
GGTGAATTTCCCTGATGAGCCTTCAGGCGCTGCTTCCTCAAAACGTCTCAAGGCGAATCCAGAGGCTC
AGCCAATGAAGAAAAATCTGAACTCTGTGAAGCCGAAAATAAACCAGATGTTCAATTTTGGTGACAAT
CTTGAGGGCTACTACAGCCCTATAGATCAGGTGGAACAGAAACCACTGGTTAACCAGTATGTTAACCG
TGCCCCGTTTGCTGGAAATGGAGTTCAAGTCTCACCTGTTACTCCATCTGCTGATGTTACTGCTTACT
TCAGCTCTGAGCATTCGAGCAACTCGTTTGATTATTCTGACCTTGGATGGGGTGAACAAGTCCCCAAG
ACCCCCGAGATCTCATCCTTGCTTTCTGCTGCTCCTTTGGAGGGTGCTGCTGATCAGGTTCAGAAGAC
CAACAACTCGCAGGATGTGGTGGCTGCACAAGATGATTCTGCAAAAACCCTTTCCGAAGAGCTTGCAG
ACATTGAATCCCAGCTCAAGTTCTTTGAGACCCCTTCTTTTCTTGATGAAGCCTGGGCTGATGCTACA
TTGGCGTCTTTGCTCGGCGGAGACGCAACTCATGACGCCGCCGGAAACCCTATGAACCTTTGGAGCTT
CGACGACCTGCCTTCCATGGCAGGAGTCTTCTGAACACCCTTTATCTCCCCTTTTATGTAAATAAAGC
TACAAGAATTGTGATCGTGATGTTGGTGATGGAGTCCACAGCCAAGAAACCTGCTTAAAGCTTATGTG
GAGTTTATTTTATCTTGTAGCTAATGCAGTAGTATAGGACTATATATAGGTTTTTATTATAGGGTATC
CTTTTGTGAACTCAAAGACCTCGTTTTCAGGGGATTTTCTGTTTGATGTCCTTAAGGATTATCAATTA
TGTTATATATGGTCTTGGATAAAAATAAAAAAAAAAAAAAAA

Figure 28 (continued)

**SEQ ID NO: 236, AAQ10777.1| ethylene responsive protein [Glycine max]**

MCGGAIISDFIPAGPASGARRVTADILWPSLRKRFSKPLLDDDFEAGFREFKDDSEIEDVDDEDDEDE
EELKKKPFGFSRSSNKAASKPLSRGATTVKSVESKGQAEKCAKRKRKNQYRGIRQRPWGKWAAEIRDP
RKGVRVWLGTFSTAEEAARAYDAEARRIRGKKAKVNFPDEPSGAASSKRLKANPEAQPMKKNLNSVKP
KINQMFNFGDNLEGYYSPIDQVEQKPLVNQYVNRAPFAGNGVQVSPVTPSADVTAYFSSEHSSNSFDY
SDLGWGEQVPKTPEISSLLSAAPLEGAADQVQKTNNSQDVVAAQDDSAKTLSEELADIESQLKFFETP
SFLDEAWADATLASLLGGDATHDAAGNPMNLWSFDDLPSMAGVF

**SEQ ID NO: 237, AF459404.1| Narcissus pseudonarcissus DAFSAG9 AP-2 domain containing protein mRNA, partial cds**

ATGTGTGGAGGAGCAATAATCTCTGATTTCATACCTCCATCAAGGTCAAGGAAGCTCACTGCCGATTA
CTTGTGGCCCAATCTCAACAAGGGGAAGGACAAGAAGAAGAGCTTTGGGTTTGAAGATGACTTTGAAG
CTGACTTCAATGAGTTTGAAGATGAGTCTGAGGAGGGGGAATCTGAAGCTGTTGATGTCAAACCCTTT
AAGTTTGGGGCTAAAGCTGGCTTTTCTAGAGAGGGATCGACTTATCTGAAACCCATCGAACTCAATGG
AGCTGCTGAACGATCATCCAAAAGGAAGAGGAAGAACCAATACAGAGGTATCCGTCAGCGTCCATGGG
GTAAATGGGCAGCTGAGATAAGAGATCCTAACAAAGGAGTTCGTGTTTGGCTGGGAACTTTTAACACA
GCTGAAGAAGCTGCGAGAGCCTATGATGATGAAGCCCGTAGGATCCGTGG

**SEQ ID NO: 238, AAL67489.1|AF459404_1 AP-2 domain containing protein [Narcissus pseudonarcissus]**

MCGGAIISDFIPPSRSRKLTADYLWPNLNKGKDKKKSFGFEDDFEADFNEFEDESEEGESEAVDVKPF
KFGAKAGFSREGSTYLKPIELNGAAERSSKRKRKNQYRGIRQRPWGKWAAEIRDPNKGVRVWLGTFNT
AEEAARAYDDEARRIRG

**SEQ ID NO: 239, AJ001911.1| Arabidopsis thaliana mRNA for ethylene-responsive element binding protein**

AGTAATTTAGCAGCAACAAACAGAGAAAATGTGTGGCGGTGCTATTATTTCCGATTATGCCCCTCTCG
TCACCAAGGCCAAGGGCCGTAAGCTCACGGCTGAGGAACTCTGGTCAGAGCTCGATGCTTCCGCCGCC
GACGACTTCTGGGGTTTCTATTCCACCTCCAAACTCCATCCCACCAACCAAGTTAACGTGAAAGAGGA
GGAGGCGGTGAAGAAGGAGCAGGCAACAGAGCCGGGGAAACGGAGGAAGAGGAAGAATGTTTATAGAG
GGATACGTAAGCGTCCATGGGGAAAATGGGCGGCGGAGATTCGAGATCCACGAAAAGGTGTCAGAGTT
TGGCTTGGTACGTTCAACACGGCGGAGGAAGCTGCCATGGCTTATGATGTAGCGGCGAAGCAGATCCG
TGGTGAGAAAGCCAAGCTCAACTTCCCAGATCTGGATCATCATCCTTCTACTCCTCCGCCATCGTCTA
CTTCACTAAGATTATCCGATCAGCCACCGGCGAAGAAGGTTTGCGTTGTCTCTCAGAGCGAGTTAGCT
CAGCCGAGTTTCCCGGTGGAGTGTGTTGGATTTGGAAAGGGGGAAGAGTTTCAGAACCTGATGTACGG
ATTTGAACCGGATTATGATTTGAAACAGCAGATATCGAGCTTGGAGTCGTTCCTTGAGCTTGACGGTA
CCACGGCGGAGCAACCGAGTCAACTAGATGAGTCTGTTTGCGATGTGGATATGTGGATGCTTGATGAT
GTCATTGCGTCGTATGAGTAAAAGGAAAAAAAAACAGAAGATAATGTAATATGTAAAAAAAAAAACTA
AATTACT

Figure 28 (continued)

**SEQ ID NO: 240, CAA05084.1| putative Ckc2 [Arabidopsis thaliana] similar but different from SEQ ID NO: 40**

MCGGAIISDYAPLVTKAKGRKLTAEELWSELDASAADDFWGFYSTSKLHPTNQVNVKEEEAVKKEQAT
EPGKRRKRKNVYRGIRKRPWGKWAAEIRDPRKGVRVWLGTFNTAEEAAMAYDVAAKQIRGEKAKLNFP
DLDHHPSTPPPSSTSLRLSDQPPAKKVCVVSQSELAQPSFPVECVGFGKGEEFQNLMYGFEPDYDLKQ
QISSLESFLELDGTTAEQPSQLDESVCDVDMWMLDDVIASYE

**SEQ ID NO: 241, (gi|84662889:105357-105537, 105673-106409) Medicago truncatula clone mth2-18n7, complete sequence**

ATGTGTGGTGGTGCTATCATCGCTGACTTCATTCCTCGCCGTGACGGCCGCCGTCTCACCGCCTCGGA
GCTCTGGCCTAACTCATTTGGCCAACAGATTGACTCCTCAAACTTCGGTTTTTCTCATACTGCTGCTG
ATCAACAACCACCCAGCACTCTCAAAAGATCACAGCCTCCCAAAGTTAATGAACGAGTTGAGAAGCCA
CTGAAAAAACAGAGGAAGAATCTCTATAGAGGAATTCGACAGCGTCCATGGGGAAAATGGGCTGCAGA
GATTCGTGATCCAAGAAAAGGAGTTCGTGTTTGGCTTGGTACATTCAACACTGCTGAAGAAGCTGCTA
GAGCTTACGACAAAGAAGCTCGAAAAATCCGTGGCAAAAAAGCTAAGGTTAATTTTCCTAACGAAGAT
GATGAATATACCATTCATGCTACTCGTCGCTACAATAATAATCCTCCACCGATTCGACCACAGAAGCT
TCCTCTATATCATCAACAACACTATCAGAAGAATCTGAACTTGGAATTTGGTTATGATCTGAACCAAA
CTGACACAATTCATGCTATCAATACTGGTTCTGGTGGTGATGAGAATTCTTTATTTGGGTCTGGTTCA
GTTTCAGAGGTTGGTTTTTCTGTGATGGAGTTCAATGGTGGTTCCAATCAGAATGAATTCGGTTATTT
TGGTGGTGTTGTGAATGAACATGAAAAAGAGAAAGAGAAAGTGGTAGAACAAGAAACACATGTTGTTG
AACAAGCTGAAGCTGAATTAGCAAAAAATGAGGTACAAGAATTGTCTGATGAATTGTTGGCATACGAG
GATTACATGAAGTTTTATCAGATTTACTATGATGGACAATCAGTGATGCCACCTAATAATGTTCAGGA
ACATGTGGTTGGAGATTTATGGAGCTTTGATTGA

**SEQ ID NO: 242, ABE84970.1| Pathogenesis-related transcriptional factor and ERF [Medicago truncatula]**

MCGGAIIADFIPRRDGRRLTASELWPNSFGQQIDSSNFGFSHTAADQQPPSTLKRSQPPKVNERVEKP
LKKQRKNLYRGIRQRPWGKWAAEIRDPRKGVRVWLGTFNTAEEAARAYDKEARKIRGKKAKVNFPNED
DEYTIHATRRYNNNPPPIRPQKLPLYHQQHYQKNLNLEFGYDLNQTDTIHAINTGSGGDENSLFGSGS
VSEVGFSVMEFNGGSNQNEFGYFGGVVNEHEKEKEKVVEQETHVVEQAEAELAKNEVQELSDELLAYE
DYMKFYQIYYDGQSVMPPNNVQEHVVGDLWSFD

**SEQ ID NO: 113, gi|50300636:c114504-113782 Medicago truncatula clone mth2-28b4, complete sequence**

ATGTCACCCCCTAAACCGTATATAAACCAACTCAAATCTCTGACTTTCCCTTCAACTTCAAAATCTTC
TAAACCTTCAAAAACTCAGTTTCCATACATGAACTACGCCTTCTCCACTACCACCCTCACCTCCGATC
AAGAACTCTCAGTCATCGTCGCTGCCCTAACCAACGTAGTCTCCGGTTCCACCTCCACCGTCGGCTTA
GATCGAATAGTTCAACCGGTGAACATAGAAACCTGTCGGGAATGCAACATAGCAGGATGTTTAGGATG
CAATTTCTTCCCTGAAGAGAAAAAACAAAAACAAAAACAAAAACAAAAGAGAGCTAAGAATAATAAGT
ACAGAGGAGTGAGGCAGAGACCATGGGGAAAATGGGCTGCTGAGATTCGTGACCCGAGACGTGCGGTT
CGTGTTTGGCTTGGAACCTTTACCACGGCGGAGGAAGCTGCTAGAGCTTACGAC

Figure 28 (continued)

AATGCCGCTATCGAGTTCCGCGGGCCGAGAGCCAAGCTTAATTTTCCACTTGTTGATGAATCTCTTAA
GCGTACTGTTGAGGATCCAGAATTGGTTGTTCATGTAAAGGATGAAGAAATGCAAATTGAGACAACGA
TGGGATTTGGGAATAATACGACTGAATGTGATTTTTGGGATAGTATTGGGGAAGAAGCTGATTTTCAA
CAACTTATGAGGTTTATGGATTCTTCTCATTCTAGAACAGGAAACACTTTTAATTAG

**SEQ ID NO: 244, ABE80536.1| Pathogenesis-related transcriptional factor and ERF [Medicago truncatula]**

MSPPKPYINQLKSLTFPSTSKSSKPSKTQFPYMNYAFSTTTLTSDQELSVIVAALTNVVSGSTSTVGL
DRIVQPVNIETCRECNIAGCLGCNFFPEEKKQKQKQKQKRAKNNKYRGVRQRPWGKWAAEIRDPRRAV
RVWLGTFTTAEEAARAYDNAAIEFRGPRAKLNFPLVDESLKRTVEDPELVVHVKDEEMQIETTMGFGN
NTTECDFWDSIGEEADFQQLMRFMDSSHSRTGNTFN

**SEQ ID NO: 245, U77655.1|STU77655 Solanum tuberosum DNA binding protein homolog (STWAAEIRD) mRNA, complete cds**

ATTCGGCACGAGACCAAAACCAAAACCAAGCTTCTCTCTTGTTATAATATATATATATATAAAAAAAA
CTCGACCTTTGTACAAACCATTTTTCATATCTGTATCAATCTCTTTGTTTCTGCCATTTTGAGTAAAA
GAGTTTAAATCACACTAAAATGTGTGGTGGTGCAATTCTTTATGATATTATTCCTCGTGACCGCCGTT
TGTCATCCACCGACTTATGGCCAAGCTCTGCTGATTTCTGGCAAACTTCTTCTTTTTCCAAGCCAATT
TCCACCCAAAATGTTCCTCCCAAGCCTAAACGAGCTCAACTCTCTAGAGGTAGTGAGCAGATGAAGAA
GAGGCAAAGGAAGAATCTTTACAGGGGAATCCGACAACGTCCATGGGGTAAATGGGCTGCTGAAATTC
GTGACCCGAGAAAAAGGGTTAGGGTCTGGTTAGGTACTTTCAACACTGCTGAAGCTGCAAGAGCTTAT
GATAGAGAAGCTCGTAAAATCAGGGGAAAGAAAGCTAAAGTTAATTTCCCCAATGAAGACGACGACCA
CTACTACAGTCATCCAGAGCCGCCTCCTTTGAACATTGTTTATGAATCTTATGATACTACTAGTACTT
ACAATCAAGAATCAAATAACTGTTACCCCTTCCACTCAATCGAAAACACTGAACCTGTTATGGAATTC
GCAATTGCTAACAAAAATTCATCTGGGTCTGCTTATAATGGAATTGAAGATCAGAATGTGGAAGGAGA
AGAGCAGACGGTGAAAAATTCAAATAACAGGATCGTAGAGGAAGAGGAAAAAACAGAGGATGAAGTGC
AGATACTTTCTGATGAACTGATGGCTTATGAGTCATTGATGAAGTTCTATGAAATACCGTATGTTGAC
GGGCAATCAGTGGCGGCGACGGTGAATCCAGCGGCGGACACCGAAGTGGGCGGTGGCTCGATGGAGCT
TTGGAGTTTTGATGATGTTAGTCGTCTACAACCAAGTTATAATGTTAGTTTGATTATTGTTTTGTTTA
AATTGTTGCATCTTTTTAGTTTGCTGAATTAGAACTAATTGAGTTTTTGTAATTTTTAATCAATTGTG
TTGAAACTATATTTTTAHTTCATTACTCTATTAAAAAAAAAAAAAAAAAA

**SEQ ID NO: 246, AAC29516.1| DNA binding protein homolog [Solanum tuberosum]**

MCGGAILYDIIPRDRRLSSTDLWPSSADFWQTSSFSKPISTQNVPPKPKRAQLSRGSEQMKKRQRKNL
YRGIRQRPWGKWAAEIRDPRKRVRVWLGTFNTAEAARAYDREARKIRGKKAKVNFPNEDDDHYYSHPE
PPPLNIVYESYDTTSTYNQESNNCYPFHSIENTEPVMEFAIANKNSSGSAYNGIEDQNVEGEEQTVKN
SNNRIVEEEEKTEDEVQILSDELMAYESLMKFYEIPYVDGQSVAATVNPAADTEVGGGSMELWSFDDV
SRLQPSYNVSLIIVLFKLLHLFSLLN

Figure 28 (continued)

**SEQ ID NO: 247, AB085820.1| Solanum tuberosum cip353 mRNA for AP2/ERF-domain protein, complete cds**

CTTTCACTCAAAAAAAAAAAGGAAAAAATTAAGAGTAACAAAAGATGTGTGGAGGTGCCATAATCTCC
GATTATGAGCCCGCCGGAAACTTCTACCGGAAACTCTCTGCTCGTGACCTGTGGGCTGAGCTGGACCC
TATCTCCGACTACTGGTCCTCTTCCTCCTCATCCTCAACTGTCGAAAACCCTTATTCCGCTCAGTCGC
CGGTGACTCACTCCGTCGATAAGCCTAAGAAATCAGATTCCGGCAAATCTAATCAACTCAAAAAAGGT
AATAAGACTGTGAAGGTTGAGAAGGAGAAGAGTACTGGACCAAGGCAGAGAAAGAACAAGTACAGAGG
AATAAGGCAGAGACCATGGGGAAAATGGGCTGCTGAGATTCGCGATCCTCAGAAGGGTGTCCGTGTTT
GGCTTGGTACATTCAACACAGCAGAGGATGCTGCCAGAGCCTATGATGAGGCTGCTAAGCGCATTCGT
GGTAACAAGGCCAAACTCAACTTCCCTGCCCCATCACCACCTGCTAAGCGACAGTGCACTAGCACTGT
CGCTGCTGATCCTCCACCAGCACTACTCCTTGAGAGTTCTAACATAATATCTTATAACAATTCTCCTT
TAATGAACTTCGGATATGATGTTCAGAGCCAAACTCCCTACTACCCAATGGAAATGCCCGTTGCTAGT
GATGATTATGAACTCAAGGAACAGATTTCCAACTTGGAATCGTTCCTGGAATTGGAGCCAGCAGATTC
ATCTGATCAGTTTTCAGGGATCGTCGATCCTGATCCTCTTAATGTTTTTCTGATGGAGGATTTTGCTT
CAACTCAGCATCAGTTCTATTGATCCTGAGTTGTTTGGTGAGTGATGAGTGACTAGTTTATTAGCTTT
TGGCTGTAGTAGTAGTAATAGAGAAAAAAGTACATATGATATGATAATAATAAGTTGCGTGCCTTAGC
CTGCAATTGTAATAGTATCAATGTTTGTTGTCTTGTGTTGTTTATGCTTTCTAAATCTTGGATTTACC
TTATAATGTTTGGTCATTTGGTGTATGTATTGTAACTATATATGGAGTACTTTATTACTAAAAAAAAA
AAAAAAAAAAAAAAAAAAAAAAAAAAAAAAAAAAAAAAAAAAAA

**SEQ ID NO: 248, BAC56862.1| AP2/ERF-domain protein [Solanum tuberosum]**

MCGGAIISDYEPAGNFYRKLSARDLWAELDPISDYWSSSSSSSTVENPYSAQSPVTHSVDKPKKSDSG
KSNQLKKGNKTVKVEKEKSTGPRQRKNKYRGIRQRPWGKWAAEIRDPQKGVRVWLGTFNTAEDAARAY
DEAAKRIRGNKAKLNFPAPSPPAKRQCTSTVAADPPPALLLESSNIISYNNSPLMNFGYDVQSQTPYY
PMEMPVASDDYELKEQISNLESFLELEPADSSDQFSGIVDPDPLNVFLMEDFASTQHQFY

**SEQ ID NO: 249, AY522505.1| Coffea canephora ERF-like transcription factor mRNA, complete cds**

AAGTACCGCAAATTTGTAAGCTTAGATCATCAAAAATGTGTGGCGGTGCAATCCTCGAAGGCCTCATC
CCGCCCCGCAACAACAACGACGGCACTCGCCGTCTCTCCTCCGCTGACCTCTGGCCCTCCGCTGCTGA
TTTTTGGCCCAACTCTCACCTTGCCAAGCCCAGCCGCTCCAACCCAACTGATGCCCCCCACAAAGCCA
CGCACGAAACTTGTCACGATCCACATGATGAAGCTGGTGGAAAGCAGCAAGTCCTTCCCAAGGGTGCC
AAGAGGCAGAGGAAAAACTTATACAGGGGGATCAGGCAGCGACCATGGGGGAAATGGGCTGCTGAGAT
TCGAGACCCTAGGAAAGGCGTGAGAGTCTGGTTGGGCACTTTCAACACTGCCGAAGAAGCTGCCAGAG
CCTACGACAAAGAAGCTCGAAAAATCAGAGGCAAGAAAGCCAAGGTTAACTTCCCAAACGAGGATTCG
ACCAGTTATCCCACATGCATCCCTTCGCAAACCCAGTACCAACAGGTACACATCCCCAGCCCTCCCAC
TTTCTGTGGTCCCTCCTCCTCCAATTGCAAGTTCAATCAGCTCCGTGTTGGGTCGTCTGATTGCAGTG
CCAGTGACTGCTATCACGCGAACAGCATCGATGATTGTGCCCGTTTCACCAATATGATGGGTTTTCGA
AATCCAAGCGAAGAGGTTTCTGGTTCTGGTAGTTCGGATAATGAGTGTTTTCTTGGCCACCTTAAACA
AGTAGCCAAGGCGGCGGCGGAGGAGGAGGCGGCTTCGGTTATAACTGAAAATGATACGAAAGACAATA
ACAAAGCAGCTGAAAGCGAGGAGTACCAAGTGGAAAAGCTGTCTGAGGAGCTGCTGGCCTATGAGTCC
TTCATGAAGTTCTATCAGATTCCTTATATAGATGGGCAGT

Figure 28 (continued)

CCGCAGCTGCATCGGCCAACCCTGCTCAAGAGCTGGCAACTTCTATTCAGCTTTGGAGCTTTGATGAT
GTCTCACCCGCCAATCGCCCAATGCCTCTGTAACCGCCAATCGCCCAAGTTCGATGGTCTTTTTTTG
GGAGTCCCAATTGCGTTTTGGTGCCTAGTTTTTGTAGGTTTTGCTATGTAATTGACATTAACTTCTTA
AAACATTGTAGGAAATTGTTGTTAGGTTGCAGGTTGGTACTCTTGACCGGCTTGTTTAGTTAAAATAC
CTATATGTAGGTTGGCACTTTGTAATTACTGTTTTTGTGGACAAAAAAAAAAAAAAAAAAAA

**SEQ ID NO: 250, AAS01337.1| ERF-like transcription factor [Coffea canephora]**

MCGGAILEGLIPPRNNNDGTRRLSSADLWPSAADFWPNSHLAKPSRSNPTDAPHKATHETCHDPHDEA
GGKQQVLPKGAKRQRKNLYRGIRQRPWGKWAAEIRDPRKGVRVWLGTFNTAEEAARAYDKEARKIRGK
KAKVNFPNEDSTSYPTCIPSQTQYQQVHIPSPPTFCGPSSSNCKFNQLRVGSSDCSASDCYHANSIDD
CARFTNMMGFRNPSEEVSGSGSSDNECFLGHLKQVAKAAAEEEAASVITENDTKDNNKAAESEEYQVE
KLSEELLAYESFMKFYQIPYIDGQSAAASANPAQELATSIQLWSFDDVSPANRPMPL

**SEQ ID NO: 251, DQ102383.2| Hordeum vulgare root abundant factor (RAF) mRNA, complete cds**

CAGGAAGAGAAAACAATGTGTGGCGGCGCCATCCTAGCGCAGCTGATCCCGCCGTCGGCGGGCCGTCC
GTCGAAGCAGGCGGCAGCGGGCGGCCGGGCCCCGCCCACGAGCTCCAAGAAGGGCGGCGTGAGCAAGA
GCCGCCACAGCAGCACCCCAGATGCCGACGACGACGTCTTCGAGGCCGCCTTCGAGGACTTCGATGAC
CACTTCGACCTGCGGGCGGAGGAGGACGGCGGCGACGACCATGTCGTCTTTGCATCCAAGCCTGCCTT
CTCTCCACGTCCGGCCTACGACGGTGGCCGCGCGGCGCATGCGGCGAGCAGGAAGAAGCGCACCGGCC
ACCTCCATGGCATCCGGCAGCGGCCGTGGGGCAAGTGGGCGGCGGAGATCCGCGACCCGCACAAGGGC
ACCCGCGTCTGGCTCGGCACGTTCGACACGGCCGATGATGCCGCCCGGGCCTACGACGTCGCCGCCCG
TCGCCTCCGTGGCAGCAAGGCCAAGGTCAACTTCCCCGACGCGGCCAGGACCGGGGCTCGCCCGCGCC
GCGCCAGCCGTAGAACCGCGCAGAAACCGCAATGCCCCCCTGCGCGGACGACGGCGTACTCTGCCACC
GCAGCAGCACGCGCACAGCCGGAGCAGGACGCTATGATGGTCAAACCCGAGCTGATGGAGTTTTTCAA
CGTGGACGCCATCGTCCACCTGACCACTGCCGTCGCCGCGCTACCGCCTGTCACGGCGAGCACCTTCG
CCGACACGATGCCGAGGGTCGACGAGGACTCTTCTGTGGGGAGCGGCGGCGGCGCCATGCTGGGGTTC
GCCGACGAGCTTGGGTTCGATCCGTTCATGATGTTCCAGCTACCCTGCTCGGACATGTACGAATCCGC
CGACAGCATCTTCGCCGGAGACGCTGTCATCCCGGATGCCCTCAGCGTGGACAGTGGCATGGACGCCG
TCAGCCTCTGGAGCTTCGACGAGTTCCCCATGGACAGCGCCATTTTCTGACGCTTTCCGTGTGATGCA
CTGCACTCTGTTGGTTGTAAGAATCTCCACCTGGCCTCTACGTAGTTCCTTGTAAATGCCCGCGCACA
GAACCTTGCTCAGACCAGATTCTGTTTCTTGGCCAGGAACGAAAGGAAGGGTTGCTGCCGATGCATGA
TTGCTTCCTCGATGAACGCAGATTCGAAATGTATTCTACTGTTTGAGTTTCTTGTTCGTCACACACTG
TACCAAACTGTATTGTACCCTATCATAATTTCTGCTCGGTACCTCTCTGTACTGCTGGTACCAAACTG
TATTGTACTCTGTCATGATCTGTACTAGTCTTTGGTACTGCTGGTCAATAGTCCTACGAATTGATCAA
AAAAAAAAAAAAAAAAAAAAAAAAAAAAAAAAAAAA

**SEQ ID NO: 252, AAZ14085.2| root abundant factor [Hordeum vulgare]**

MCGGAILAQLIPPSAGRPSKQAAAGGRAPPTSSKKGGVSKSRHSSTPDADDDVFEAAFEDFDDHFDLR
AEEDGGDDHVVFASKPAFSPRPAYDGGRAAHAASRKKRTGHLHGIRQRPWGKWAAEIRDPHKGTRVWL
GTFDTADDAARAYDVAARRLRGSKAKVNFPDAARTGARPRRASRRTAQKPQCPPARTTAYSA

Figure 28 (continued)

TAAARAQPEQDAMMVKPELMEFFNVDAIVHLTTAVAALPPVTASTFADTMPRVDEDSSVGSGGGAMLG
FADELGFDPFMMFQLPCSDMYESADSIFAGDAVIPDALSVDSGMDAVSLWSFDEFPMDSAIF

**SEQ ID NO: 253, AB125976.1| Cucumis melo CMe-ERF2 mRNA for ERF-like protein, complete cds**

AATTCTCTAATCATGTGTGGCGGCGCCATTATCGCCGACTTAATCCCTCGCCGTGACGGCCAACGCGT
TACCGCTTCCGACATTTGGCCTAACTCCTCCTTCTTCCATTTCAACAAAATTCGCTCCGATCAAGTTT
CAACTCCCCTCAAACGAACCCCCCTCCCGGCCTCTTCCGAGGCTTCGAAGCCCAAGAAGAGGCAGAGG
AAGAATCTTTACAGAGGAATTCGTCAGCGCCCGTGGGGAAAATGGGCGGCTGAGATTCGTGACCCCAG
GAAGGGGATCCGTGTCTGGCTTGGGACTTTCAATACTGCTGAGGAAGCCGCTCGAGCTTACGATCGAG
AAGCTCGCAAGATTCGTGGAAAGAAAGCTAAGGTCAATTTCCCTAATGAAGATGATGCATATTCCATT
CAAGCTCCCATTCCTCAATTTCACCCTCATCTTTACACTGTCCCTGAGAATTCCGAATTCCCCTACGA
TCTCAACCAAATCGGTGATTTCACCGGCACTCACTTTCCCGTGGCGATTGAGGAACAATCCGGCTCTG
GGTCGGAGGATTCTTATTCTCCACCGAAAAGATTCGGTGTGAAAGAGGCTGAAGATCAGAAGCCGGAG
CAGAAGGTTAGCGTTATTGCCGCGGCGGAGGAAGAGAACGAGGTGCAAAAGCTTTCTGAGGAACTAAT
GGCGTATGAGAACTACATGAAGTTCTACCAAATTCCATACCTCGACGGTCAATCGACGGTGACGAATC
CGGCTGAAGAACAAGTGGTGGGCGATCTCTGGAGCTTCGACGACGAGGATGGGCTTCACGGCTCTGTT
TCGTCGTCTGAATTATGATGATTGATGATCCAATAACTTCTCCATATCTCAAACCGGTTTCCTCCATT
TTCTATTGTTGTTTTGTTATTTAATTCCAGAATGTGTTTTATGTAATTTTCTGTCGTTGAACGATGTC
AATTTGTTGATATGAAAAAAAAAAAAAAAAAAA

**SEQ ID NO: 254, BAD01556.1| ERF-like protein [Cucumis melo]**

MCGGAIIADLIPRRDGQRVTASDIWPNSSFFHFNKIRSDQVSTPLKRTPLPASSEASKPKKRQRKNLY
RGIRQRPWGKWAAEIRDPRKGIRVWLGTFNTAEEAARAYDREARKIRGKKAKVNFPNEDDAYSIQAPI
PQFHPHLYTVPENSEFPYDLNQIGDFTGTHFPVAIEEQSGSGSEDSYSPPKRFGVKEAEDQKPEQKVS
VIAAAEEENEVQKLSEELMAYENYMKFYQIPYLDGQSTVTNPAEEQVVGDLWSFDDEDGLHGSVSSSE
L

**SEQ ID NO: 255, AB288347.1| Malus x domestica MdERF1 mRNA for ethylene response factor, complete cds**

CTGAGAGGGCTCTTTTTTCTCTCTAAGTTTCTACAAGTGGCAATATAAACATGTGTGGTGGTGCTATC
ATTTCCGACTTCATCGCCGTCAAGCGCGCCCTGAAGCTGACGGCGGAGGACCTCTGGTCAGATCTTGA
CACCATCTCTGACCTCCTTGGCATAGACTACTCCAACAGCATCAACAAACAGCCGGAGAATCACAAGG
TGGTCCAAAAGCCGAAACCATCTATCACCAAAGTAGTGACAAGTGATGAGAAGCCCAAGCAGGCGAGC
GGGTCTGCTGCTGCCGCAAAAGGCAAGAGAGTGAGAAAAAAACGTGTACAGAGGAATAAGGCAGAGGCC
GTGGGGCAAATGGGCGGCTGAGATTCGCGACCCCTACAAAGCCGTCCGGGTCTGGCTCGGCACCTATG
ACACCGCTGAGGAAGCCGCCCGCGCTTACGATGAAGCCGCCGTGCGCATCCGCGGGGACAAGGCCAAG
CTCAACTTTGCCCAACCACCATCCTCTTCACCGCTTCCATCTCTGGCGCCGGATACGCCGCCGCCGAC
AAAGAGGCGGTGCATTGTTGCTGAGTCAACTCGGGTGGAGCCGACTCAAC
CGAGTTTCCAGACCGGTTCTTACTATTATGATCCATTATATCACGGCGGTGGTGGTGGGGAAATGTAT
GCTAAGAAAGAGGTGGCGGGTGGGGACGAGGTGGTGGTAGAGCAGCTGAGTCAGGTGGTGAGTGGAAG
CGGAGAGTCGGACTCGTTGTACCTGTGGATGCTGGATGACCTGGTGGCATATCAGCAACAAG
GGCAGCTTCTGTATTAAGGCAGCGGAATTGTTCTAGCTAAATATTTGTATGGCTAGGAATTAAAAA

Figure 28 (continued)

CATATTAATTAAAGGGTATGTATGTTTAATTTACGTGTGTGAAATGCGAGTGTTGAGTATGTCTATGA
CTGGGTTTGTGTAACGTGTGTTGTTTGCTACGGTGTTTATGTTTAATAGTAACTGCTTTTGTCTTTGA
AAAAAAAAAAAAAAAA

**SEQ ID NO: 256, BAF43419.1| ethylene response factor [Malus x domestica]**

MCGGAIISDFIAVKRALKLTAEDLWSDLDTISDLLGIDYSNSINKQPENHKVVQKPKPSITKVVTSDE
KPKQASGSAAAAKGKRVRKNVYRGIRQRPWGKWAAEIRDPYKAVRVWLGTYDTAEEAARAYDEAAVRI
RGDKAKLNFAQPPSSSPLPSLAPDTPPPTKRRCIVAESTRVEPTQPSFQTGSYYYDPLYHGGGGGEMY
AKKEVAGGDEVVVEQLSQVVSGSGESDSLYLWMLDDLVAYQQQGQLLY

Figure 28 (continued)

**SEQ ID NO 257, Oryza sativa – DNA**

GGCACGCGCCTCCTCGGAATCGCCACGAGACCCTTGNAGCCAGATAAAAAAGGAAAAAAAGAGAGTCG
TAGTTCGCCTCTTCTTCCTCCTCTCGTTCTCGCGGCCATATCGAATTCCTGCAGCCCGGGGGATCCCC
GAATTCAGCTCAGTAAATCTGTTGATTGCTCGTTTCAAATCATCATCGGTGTTTCGTTTCTTGATTTC
TTGATCCATCCATGGCGACGACAGTGGATTGGTGTGGCCGTGGCAGCAATCTTCCTGCTGCGATGTAT
GACATGGTGGTGGATAGCAAGGAGCTAATGGGCGCGCTTGCTCCGTCCATGGTGTCCTTCTCCTACCC
GTGTTCCGAGCAGAGCGCGAGCTCGCTTCTTGCTGGCGCCAACTACCTGACTCCCGCGCAGGTGCTCC
ATGTCCAGGCGCAGCTACAGCGCCTGCGCCGCCCGGGCGCGGCGAGCGGCTGCCTCGCCGCGGCGCCG
CCGCTGCCGATGAAGCGGCATGGCGCGGTGGCGGTGGCGGCGGCGGCGGCGGCGCGGGCGCCGGT
CAAGCTGTACCGCGGCGTCAGGCAGCGGCATTGGGGCAAGTGGGTGGCGGAGATCCGCCTGCCGCGCA
ACCGCACCCGCCTCTGGCTGGGCACCTTCGACACCGCCGAGGAGGCCGCGCTGGCGTACGACAGCGCC
GCGTTCCGCCTCCGCGGCGAGTCCGCGAGGCTCAACTTCCCCGAGCTCCGCCGCGGCGGCGCCCACCT
CGGCCCGCCGCTCCACGCCGCGGTCGACGCCAAGCTCCACGCCATCTGCCACGGGATGGACCTGCCCC
AACCCCAACCCCAAACCCAGAGCAATGCGACGACGACGACGATGTCGACGACGGCGACGAACACCCCA
AGCCCCTTCTTCTCCTCGGAGAGCCCGGTGGTCAAGAGCGAGCCCGTCTGCTCCGCCTCCGAGAGCTC
TTCCTCGGCCGACGGCGACGTGTCATCGACGGGCTCCTCCGACGTCGTCCCGGAGATGCAGCTGCTCG
ACTTCTCGGAGGCGCCATGGGACGAGTCCGAGAGCTTCCTGCTGCACAAGTACCCGTCGCTGGAGATC
GACTGGGACGCCGATCCTTTCCTGATGCAGTGGCTGAATCTTCTTCCTCTCCCTGTTCTTGACTCCAG
CTCTTGGTGTCCTGAACATTTTCACAACTCAGAGAGTGATGCTCTACCTTGATTAGCTACATGTTAAT
CTAGGTTTTGGGTTTGATCATGCCATCGGATTCTGTAGCATCAACCCCTGGTCTGCTTGGTTTTTTCA
AGTGGCATTGCACAGGAAGGAGGTCTCAGAGNTTTGGTAATTGCGAGTTGTGCAACCCTGCA

**SEQ ID NO 258, Oryza sativa – protein**

MATTVDWCGRGSNLPAAMYDMVVDSKELMGALAPSMVSFSYPCSEQSASSLLAGANYLTPAQVLHVQA
QLQRLRRPGAASGCLAAAPPLPMKRHGAVAVAAAAAAARAPVKLYRGVRQRHWGKWVAEIRLPRNRTR
LWLGTFDTAEEAALAYDSAAFRLRGESARLNFPELRRGGAHLGPPLHAAVDAKLHAICHGMDLPQPQP
QTQSNATTTTMSTTATNTPSPFFSSESPVVKSEPVCSASESSSSADGDVSSTGSSDVVPEMQLLDFSE
APWDESESFLLHKYPSLEIDWDADPFLMQWLNLLPLPVLDSSSWCPEHFHNSESDALP

**SEQ ID NO 259, Oryza sativa – DNA**

GGCTTAAACAATGGCGACGACAGTGGATTGGTGTGGCCGTGGCAGCAATCTTCCTGCTGCGATGTATG
ACATGGTGGTGGATAGCAAGGAGCTAATGGGCGCGCTTGCTCCGTCCATGGTGTCCTTCTCCTACCCG
TGTTCCGAGCAGAGCGCGAGCTCGCTTCTTGCTGGCGCCAACTACCTGACTCCCGCGCAGGTGCTCCA
TGTCCAGGCGCAGCTACAGCGCCTGCGCCGCCCGGGCGCGGCGAGCGGCTGCCTCGCCGCGGCGCCGC
CGCTGCCGATGAAGCGGCATGGCGCGGTGGCGGTGGCGGCGGCGGCGGCGGCGCGGGCGCCGGTCAAG
CTGTACCGCGGCGTCAGGCAGCGGCATTGGGGCAAGTGGGTGGCGGAGATCCGCCTGCCGCGCAACCG
CACCCGCCTCTGGCTGGGCACCTTCGACACCGCCGAGGAGGCCGCGCTGGCGTACGACAGCGCCGCGT
TCCGCCTCCGCGGCGAGTCCGCGAGGCTCAACTTCCCCGAGCTCCGCCGCGGCGGCGCCCACCTCGGC
CCGCCGCTCCACGCCGCGGTCGACGCCAAGCTCCACGCCATCTGCCACGGGATGGACCTGCCCCAACC
CCAACCCCAAACCCAGAGCAATGCGACGACGACGACGATGTCGACGACGGCGACGAACACCCCAAGCC
CCTTCTTCTCCTCGGAGAGCCCGGTGGTCAAGAGCGAGCCCGTCTGCTCCGCCTCCGAGAGCTCTTCC
TCGGCCGACGGCGACGTGTCATCGACGGGCTCCTCCGACGTCGTCCCGGAGATGCAGCTGCTCGACTT
CTCGGAGGCGCCATGGGACGAGTCCGAGAGCTTCCTGCTGCACAAGTACCCGTCGCTGGAGATCGACT
GGGACGCGATCCTTTCCTGATGCAGTGGCTGAATCTTCTTCCTCTCCCTGTTCTTGAACCCAGCTTTC
TTGTACAAAG

FIGURE 29

**SEQ ID NO 260, Oryza sativa – protein**

MATTVDWCGRGSNLPAAMYDMVVDSKELMGALAPSMVSFSYPCSEQSASSLLAGANYLTPAQVLHVQA
QLQRLRRPGAASGCLAAAPPLPMKRHGAVAVAAAAAARAPVKLYRGVRQRHWGKWVAEIRLPRNRTRL
WLGTFDTAEEAALAYDSAAFRLRGESARLNFPELRRGGAHLGPPLHAAVDAKLHAICHGMDLPQPQPQ
TQSNATTTTMSTTATNTPSPFFSSESPVVKSEPVCSASESSSSADGDVSSTGSSDVVPEMQLLDFSEA
PWDESESFLLHKYPSLEIDWDAILS

**SEQ ID NO 261, Oryza sativa – DNA**

ATGGCGACGACAGTGGATTGGTGTGGCCGTGGCAGCAATCTTCCTGCTGCGATGTATGACATGGTGGT
GGATAGCAAGGAGCTAATGGGCGCGCTTGCTCCGTCCATGGTGTCCTTCTCCTACCCGTGTTCCGAGC
AGAGCGCGAGCTCGCTTCTTGCTGGCGCCAACTACCTGACTCCCGCGCAGGTGCTCCATGTCCAGGCG
CAGCTACAGCGCCTGCGCCGCCCGGGCGCGGCGAGCGGCTGCCTCGCCGCGGCGCCGCCGCTGCCGAT
GAAGCGGCATGGCGCGGTGGCGGTGGCGGCGGCGGCGGCGGCGCGGGCGCCGGTCAAGCTGTACCGCG
GCGTCAGGCAGCGGCATTGGGGCAAGTGGGTGGCGGAGATCCGCCTGCCGCGCAACCGCACCCGCCTC
TGGCTGGGCACCTTCGACACCGCCGAGGAGGCCGCGCTGGCGTACGACAGCGCCGCGTTCCGCCTCCG
CGGCGAGTCCGCGAGGCTCAACTTCCCCGAGCTCCGCCGCGGCGGCGCCCACCTCGGCCCGCCGCTCC
ACGCCGCGGTCGACGCCAAGCTCCACGCCATCTGCCACGGGATGGACCTGCCCCAACCCCAACCCCAA
ACCCAGAGCAATGCGACGACGACGACGATGTCGACGACGGCGACGAACACCCCAAGCCCCTTCTTCTC
CTCGGAGAGCCCGGTGGTCAAGAGCGAGCCCGTCTGCTCCGCCTCCGAGAGCTCTTCCTCGGCCGACG
GCGACGTGTCATCGACGGGCTCCTCCGACGTCGTCCCGGAGATGCAGCTGCTCGACTTCTCGGAGGCG
CCATGGGACGAGTCCGAGAGCTTCCTGCTGCACAAGTACCCGTCGCTGGAGATCGACTGGGACGCGAT
CCTTTCCTGA

**SEQ ID NO 262, Oryza sativa – protein**

MATTVDWCGRGSNLPAAMYDMVVDSKELMGALAPSMVSFSYPCSEQSASSLLAGANYLTPAQVLHVQA
QLQRLRRPGAASGCLAAAPPLPMKRHGAVAVAAAAAARAPVKLYRGVRQRHWGKWVAEIRLPRNRTRL
WLGTFDTAEEAALAYDSAAFRLRGESARLNFPELRRGGAHLGPPLHAAVDAKLHAICHGMDLPQPQPQ
TQSNATTTTMSTTATNTPSPFFSSESPVVKSEPVCSASESSSSADGDVSSTGSSDVVPEMQLLDFSEA
PWDESESFLLHKYPSLEIDWDAILS

**SEQ ID NO 263, Triticum aestivum – DNA**

GCTCTCCTCGGCTTCCCTTCCTCCAAATCGTCGGCGTTTCTTGATCGACGAGGGCATGGCGACGACGG
TGGACTGGCGCAGCTATAGGCCCGATCTTCCTGCCGCGATGTATCACATGGTGGACAGCAGGGACCAG
GTAATGCACGCGTTTGCTCCGGCGACGGCGCAGGGCGCGGCTCCGACCATCTCGTTCGCCTTCCCCTG
CCCCGGCGCGGAGCAGAGCGCCGGCCTGCTTCGTGGCGCCAGCTACCTCACTCCCGCGCAAATCCTCC
AGCTCCAGTCGCAGCTGCACCACGTGCGCCGGGCGCCGGGCGCGGCCATGGCCGCGGTGGGGCAGCCG
ATGAAGCGGCACGGCGTGGCAGCGCTCCCGGCGCGGCCGGCGACCAAGCTTTACCGCGGCGTAAGGCA
GCGGCATTGGGGGAAGTGGGTCGCCGAGATCCGCCTGCCCCGCAACCGCACCCGCCTCTGGCTCGGCA
CCTTCGACACCGCCGACGAGGCCGCGCTGGCCTACGACGCCGCCGCCTTCCGGCTCCGCGGCGAGTCC
TCCAGGCTCAACTTCCCCGAGCTCAGGCGCGGCGGCGAGCACCACGGCCCGCCGCTCGACGCCGCGAT
CGACGCCAAGCTCCGCTCCATCTGCCACGGGGAAGACATGCCCCAGAGCCAGAGCGATGAGACGCCGG
CGCCGACGACGACACTGACGCCGATTTCTTTCCCGGACGTCAAGAGCGAGCCAGTCTGCTCCGTCTCC
GAGAGCTCGTCGTCGGCTGACGGCGAGGTGTCCTCGTGCTCCGACGTCGTCCCGGAGATGCAGCTTCT
TGATTTCTCGGAGGCTCCATGGGACGAGTTCCTGCTGCGCAAGTACCCGTCGCTCGAGATCGACTGGG
ACGCGATCCTTTCTTGAATCAAGTTCATGCTCGATCCACAGCTCG

FIGURE 29 (CONTINUED)

**SEQ ID NO 264, Triticum aestivum – protein**

MATTVDWRSYRPDLPAAMYHMVDSRDQVMHAFAPATAQGAAPTISFAFPCPGAEQSAGLLRGASYLTP
AQILQLQSQLHHVRRAPGAAMAAVGQPMKRHGVAALPARPATKLYRGVRQRHWGKWVAEIRLPRNRTR
LWLGTFDTADEAALAYDAAAFRLRGESSRLNFPELRRGGEHHGPPLDAAIDAKLRSICHGEDMPQSQS
DETPAPTTTLTPISFPDVKSEPVCSVSESSSSADGEVSSCSDVVPEMQLLDFSEAPWDEFLLRKYPSL
EIDWDAILS

**SEQ ID NO 265, Triticum aestivum – DNA**

GCTCTCCTCGGCTTCTCTTCCTCCAAATCGTCGGCGTTTCTTGATCGACAGGGCATGGCGACGACGGT
GGACTGGCGCAGCTATAGGCCCGATCTTCCTGCGGCGATGTACCGCATGGTGGACAGCAGAGACCAGG
TAATGCACGCGTTCGCTCCGCCGACGGCGCAGGGCGCGGCTCCGACCATCTCGTTCGCCTTCCCATGC
CCCGGCGCGGATCAGAGCGCCGGCCTGCTTCGTGGCGCCACCTACCTCACTCCCGCGCAAATCCTCCA
GCTCCAGTCGCAGCTCCACCACGTGCGCCGGGCGCCGGGCGCGCCCATGGCCGCGGTGGGGCAGCCGA
TGAAGCGGCACGGCGTGGCGGCGCTCCCGGCGCGGCCGGCGACCAAGCTGTACCGCGGCGTGCGGCAG
CGGCATTGGGGGAAGTGGGTCGCGGAGATCCGCCTGCCCCGCAACCGCACCCGCCTCTGGCTCGGCAC
CTTCGACACCGCCGACGAGGCCGCGCTGGCCTACGACGCCGCCGCCTTCCGGCTCCGCGGCGAGTCCG
CCAGGCTCAACTTCCCCGAGCTCAGGCGCGGCGGCGAGCACCACGGCCCGCCGCTCGATGCCGCGATC
GACGCCAAGCTCCGCTCCATCTGCCACGGGGAAGACATGCCGCAGAGCCAGAGCAATGAGACGCCGGC
GCCGACGCCGACACTGACGCCGATTTCTTTCCCGGACGTCAAGAGCGAGCCAGTCTGCTCCGTCTCCG
AGAGCTCTTCGTCGGCTGACGGCGAGGTGTCCTCGTGCTCCGACGTCGTCCCGGAGATGCAGCTTCTT
GATTTCTCGGAGGCTCCATGGGACGAGTCCCTGCTGCGCAAGTACCCGTCGCTCGAGATCGACTGGGA
CGCGATCCTTCCTTGAATCAAGTTCATGCTCGATCCACAGCTCGTCCAAAGTGATTACTTCGGCTTCC
ACTTAGGCTCGATCGAGTATACGCGTGTAGCATCAACCCCTCCCTGC

**SEQ ID NO 266, Triticum aestivum – protein**

MATTVDWRSYRPDLPAAMYRMVDSRDQVMHAFAPPTAQGAAPTISFAFPCPGADQSAGLLRGATYLTP
AQILQLQSQLHHVRRAPGAPMAAVGQPMKRHGVAALPARPATKLYRGVRQRHWGKWVAEIRLPRNRTR
LWLGTFDTADEAALAYDAAAFRLRGESARLNFPELRRGGEHHGPPLDAAIDAKLRSICHGEDMPQSQS
NETPAPTPTLTPISFPDVKSEPVCSVSESSSSADGEVSSCSDVVPEMQLLDFSEAPWDESLLRKYPSL
EIDWDAILP

**SEQ ID NO 267, Triticum aestivum – DNA**

TCCTCCAGCTCCAGTCGCAGCTCCACCACGTGCGCCGGGCGCCGGGCGCGGCCATGGCAGTGGCGGGG
CAGCCCATGAAGCGGCACGGCGTTGCGGCGCTCCCGGCGCAGCCGGCGGCCAAGCTGTACCGCGGCGT
GCGGCAGCGGCATTGGGGGAAGTGGGTCGCCGAGATCCGCCTGCCCCGCAACCGCACCCGCCTCTGGC
TCGGCACCTTCGACACCGCCGACGAGGCCGCGCTGGCCTACGACGCCGCCGCCTTCCGGCTCCGCGGC
GAGTCCGCCAGGCTCAACTTCCCCGAGCTCAGGCGCGGCGGCGAGCACCACGGCCCGCCGCTCGACGC
CGCGATCGACGCCAAGCTCCGCTCCATCTGCCACGGGGAGGACCTGCCGCAGAGCCAGAGCAATGCGA
CGCCGGCGCCGACGCCGACCCTGACGCCGAGCTCTTTCCCGGACGTCAAGAGCGAGCCAGGCTGCTCC
GTCTCCGAGAGCTCGTCGTCGGCCGACGGCGAGGTGTCCTCGTGCTCCGACGTCGTCCCGGAGATGCA
GCTTCTTGATTTCTCGGAGGCTCCATGGGACGAGTCCCTGCTGCGCAAGTACCCGTCGCTCGAGATCG
ACTGGGACGCGATCCTTTCTTGAACCGAGTTCATGCCCGATCCACAGCTCGTTCAAAGTGATTGCTTC
TGCTTTCGTTAGGCTCTTAGATAGGTTATTATGGGTTTGATCAAGTATTCTTGTGTAACATCAATCCC
TGCTCTGCTTGGTTTTTGAATGGCATTGCCAGGAAGGAGGTTTTATGTAGAAATTTTAAGTATGTCAT
GTAGTTTGTGATTCATTTTTTTTTTTTTTT

FIGURE 29 (CONTINUED)

**SEQ ID NO 268, Triticum aestivum – protein**

MAVAGQPMKRHGVAALPAQPAAKLYRGVRQRHWGKWVAEIRLPRNRTRLWLGTFDTADEAALAYDAAA
FRLRGESARLNFPELRRGGEHHGPPLDAAIDAKLRSICHGEDLPQSQSNATPAPTPTLTPSSFPDVKS
EPGCSVSESSSSADGEVSSCSDVVPEMQLLDFSEAPWDESLLRKYPSLEIDWDAILS

**SEQ ID NO 269, Oryza sativa – DNA**

TTTTTCTCTTCTGGATTCTTGCCGATTTTTAGGCTTCCTTTGCTTTTCTAAGGCCAGAAAGGGTTGTT
TTGCAATACCCTTCTAGTTCATCCTCTCAAATTTTTCTTCTTTCTATTCAAAAGCTCTTTCTTTCTTT
GCACCCTTTGTTTCTCTACTTTTATTGAGGAACAGGGCTGATTTTTCACTTCTTGCATCCAAAAAGCA
CCCCTTTTTTCTTTCCTGTTTTTAAGATTTCCATACCCTTGCCATCTTCATCTTCTACCTCCATCCAG
TCCTCATGGCCGCAGCAATAGACATGTACAAGTATAACACTAGCACACACCAGATCGCATCCTCGGAT
CAGGAGCTCATGAAAGCGCTCGAACCTTTTATTAGGAGCGCTTCTTCTTCCTCCGCTTCCTCCCCCTG
CCACCACTACTACTCTTCTTCTCCTTCCATGAGCCAAGATTCTTACATGCCCACCCCATCTTATCCCA
CTTCCTCTATCACAACCGCCGCCGCCACCACCACCTCGTCTTTCTCGCAGCTACCTCCGCTGTACTCT
TCGCAGTATCATGCTGCTTCACCTGCGGCGTCGGCGACGAACGGGCCGATGGGGCTGACCCACCTGGG
CCCAGCCCAGATCCAGCAGATCCAGGCCCAGTTCTTGGCCCAGCAGCAGCAGCAGAGGGCCCTGGCCG
GCGCCTTCCTTCGGCCGCGTGGCCAGCCGATGAAGCAGTCCGGGTCGCCGCCGCGCGCGGGGCCGTTC
GCGGCGGTCGCCGGGGCGGCGCAGTCGAAGCTCTACCGCGGAGTGCGGCAGCGCCACTGGGGGAAGTG
GGTGGCGGAGATCCGCCTCCCGAAGAACCGGACGCGGCTGTGGCTCGGCACCTTCGACACCGCCGAGG
ACGCCGCGCTCGCCTACGACAAGGCCGCCTTCCGCCTCCGCGGCGACCTCGCGCGGCTCAACTTCCCC
ACCCTCCGCCGCGGCGGCGCCCACCTCGCCGGCCCGCTCCACGCCTCCGTCGACGCCAAGCTCACCGC
CATCTGCCAGTCCCTCGCCACGAGCTCGTCCAAGAACACCCCCGCCGAGTCAGCGGCCTCCGCGGCGG
AGCCGGAGTCCCCCAAGTGCTCGGCGTCGACGGAAGGGGAGGACTCGGTGTCCGCCGGCTCCCCTCCT
CCGCCCACGCCGCTGTCGCCCCCGGTGCCGGAGATGGAGAAGCTGGACTTCACGGAGGCGCCATGGGA
CGAGTCGGAGACATTCCACCTGCGCAAGTACCCGTCCTGGGAGATCGACTGGGACTCAATCCTCTCAT
AAACAAGCAGAAGCAGCTACTACTAGTCTATTACTAGTACTAGTAGTAGTCTTCGTCAAGCTAGAGTC
ACTCAACTCAACTAGCTGTGTAATCTTCTCTGAATTCCGTGGCTTCCATGGCTCGGTGGCATTTTAGA
CGTCGGCCATGGCTGCTGCGAGTAGCAGTAACTAGTCAGTACTCAGTAGTAGTAAGGTCGTTGGTATT
ACGTCGTCGTGCAAGTGTCGTTGGTGTACTCAGTGATCTGATCTCCTGGTTGAGCTGCCGGTTGTTTT
TTTCACGGCGCGGCCGGTCGAGAATTAAGCTGTAATCCCTTGTTACATGTTGGAAATTCAGTAGCTTA
TGTAACTATATGTACCTTTCTC

**SEQ ID NO 270, Oryza sativa – protein**

MAAAIDMYKYNTSTHQIASSDQELMKALEPFIRSASSSSASSPCHHYYSSSPSMSQDSYMPTPSYPTS
SITTAAATTTSSFSQLPPLYSSQYHAASPAASATNGPMGLTHLGPAQIQQIQAQFLAQQQQRALAGA
FLRPRGQPMKQSGSPPRAGPFAAVAGAAQSKLYRGVRQRHWGKWVAEIRLPKNRTRLWLGTFDTAEDA
ALAYDKAAFRLRGDLARLNFPTLRRGGAHLAGPLHASVDAKLTAICQSLATSSSKNTPAESAASAAEP
ESPKCSASTEGEDSVSAGSPPPPTPLSPPVPEMEKLDFTEAPWDESETFHLRKYPSWEIDWDSILS

FIGURE 29 (CONTINUED)

**SEQ ID NO 271, Arabidopsis thaliana – DNA**

ATGGCTTTAAACATGAATGCTTACGTAGACGAGTTCATGGAAGCTCTTGAACCATTCATGAAGGTAAC
TTCATCTTCTTCTACTTCGAATTCATCAAATCCAAAACCATTAACTCCTAATTTCATCCCTAATAATG
ACCAAGTCTTACCGGTATCTAACCAAACCGGTCCGATTGGGCTAAACCAGCTCACTCCAACACAAATC
CTCCAAATTCAGACAGAGTTACATCTCCGGCAAAACCAATCTCGTCGTCGCGCTGGTAGTCATCTTCT
CACCGCTAAACCAACCTCAATGAAGAAAATCGACGTAGCAACTAAACCGGTTAAACTATACCGAGGCG
TAAGACAGAGGCAATGGGGTAAATGGGTAGCTGAGATTCGGCTACCTAAAAACCGAACCCGGTTATGG
CTCGGTACGTTCGAAACGGCTCAAGAAGCTGCATTAGCTTACGATCAAGCAGCTCATAAGATCAGAGG
AGACAACGCTCGTCTCAATTTCCCAGACATTGTTCGTCAAGGACACTATAAACAGATATTGTCTCCGT
CTATCAACGCAAAGATCGAATCCATCTGCAATAGTTCTGATCTTCCACTGCCTCAGATCGAGAAACAG
AACAAAAACAGAGGAGGTGCTCTCTGGTTTTTCCAAACCGGAGAAAGAACCGGAATTTGGGGAGATATA
CGGATGCGGATACTCGGGCTCATCTCCTGAGTCGGATATAACGTTGTTGGATTTCTCAAGCGACTGTG
TGAAAGAAGATGAGAGTTTCTTGATGGGTTTGCACAAGTATCCTTCTTTGGAGATTGATTGGGACGCT
ATAGAGAAACTCTTCGGA

**SEQ ID NO 272, Arabidopsis thaliana – protein**

MALNMNAYVDEFMEALEPFMKVTSSSSTSNSSNPKPLTPNFIPNNDQVLPVSNQTGPIGLNQLTPTQI
LQIQTELHLRQNQSRRRAGSHLLTAKPTSMKKIDVATKPVKLYRGVRQRQWGKWVAEIRLPKNRTRLW
LGTFETAQEAALAYDQAAHKIRGDNARLNFPDIVRQGHYKQILSPSINAKIESICNSSDLPLPQIEKQ
NKTEEVLSGFSKPEKEPEFGEIYGCGYSGSSPESDITLLDFSSDCVKEDESFLMGLHKYPSLEIDWDA
IEKLFG

**SEQ ID NO 273, Oryza sativa – DNA**

ATGGCGGCCATAGATTTGTACAAGTATCAGCTGAGCTCCTCGTCCTCGTCTTCTTCCTCGGATCAGGA
GCTCATGAAAGCGCTCGAACCTTTTATCAGGAGCGCTTCACCCACCTCCACCTCCACCTCCACGCCAT
TGTTCTACTCCTCTAGCTCCATCTCCACCACCACCACCACTCCCTTCTCCTACTCGTCTCCATTGCCG
CAAGAATCGTACTACCTCCCTGCTTCTTCGTCCTACGCCGCCATTGTTCCACCTCCGACGACGACGAC
GAACACCACCACCTCCTTCTCGGAGCTCCCTCCCCTGCCTCCCTCCTCCTCGTCGTTCGCCTCGCCGG
CGAATGCTGCGGCGGTGGGGCTGGCTCACCTTGGCCCGGAGCAGATCCAGCAGATTCAGGTGCAGTTC
TTGATGCAGCAGCAGCTGCAGCAGCGGGGGATGGCGGCGTCGGCGTCGGCGTCGGCGGCGGCGTCGTA
CCTTGGCCCGCGGGCGCAGCCCATGAAGCAGGCCGGGGCGGCGGCGGCGGCGGCGGCCGGCGGCAAGA
TGTACCGCGGCGTGCGGCAGCGGCACTGGGGGAAGTGGGTGGCGGAGATCCGGCTGCCGAAGAACCGG
ACGCGGCTGTGGCTGGGCACGTTCGACACCGCCGAGGACGCGGCGCTCGCCTACGACAAGGCGGCGTT
CCGCCTCCGCGGCGACGCCGCGCGCCTCAACTTCCCCACGCTCCGCCGCGGCGGCGCCCACCTCGCCG
GCCCGCTCCACGCCTCCATCGACGCCAAGCTCACCGCCATCTGCCACAGCCTCGCCGCCGCGCCGCCC
GCCTCCTCCAAGAAAGCCGCCGCCGCCGCCGCTCACCCGGACTCGCCCAAAGGCTCCGCGTCGACGAC
GACCACGACGTCGGAGGGAGACGAGTCGGCGATCTCCGCCTGTTCGCCTCCTCTCCCGCCGCCGCCAC
CGCCGCCGCCGGCGGCGCTCCCCGAGATGGCAAACCTTGACTTCACGGAGGCGCCATGGGACGAATCC
GACGCCTTCCACCTCTACAAGTGTCCGTCATGGAGATCGACTGGGACTCCATCCTCTCGTGA

FIGURE 29 (CONTINUED)

**SEQ ID NO 274, Oryza sativa – protein**

MAAIDLYKYQLSSSSSSSSSDQELMKALEPFIRSASPTSTSTSTPLFYSSSSISTTTTTPFSYSSPLP
QESYYLPASSSYAAIVPPPTTTTNTTTSFSELPPLPPSSSSFASPANAAAVGLAHLGPEQIQQIQVQF
LMQQQLQQRGMAASASASASAAASYLGPRAQPMKQAGAAAAAAAAGGKMYRGVRQRHWGKWVAEIRLPKNR
TRLWLGTFDTAEDAALAYDKAAFRLRGDAARLNFPTLRRGGAHLAGPLHASIDAKLTAICHSLAAAPP
ASSKKAAAAAAAHPDSPKGSASTTTTTSEGDESAISACSPPLPPPPPPPPAALPEMANLDFTEAPWDES
DAFHLYKCPSWEIDWDSILS

**SEQ ID NO 275, Asparagus officinalis – DNA**

CCACCTTTGATCACTCCTCGCCATCAAACTCCAGTCTTTTGAGCATGGACCAGCCTGATCTGAGCCAA
GTTGGGCCAGTTGGGCTGACCCAACTGAGCCCACTTCAAATCCAGCAGATCCAAGCTCAAATCCAGCT
CAACCACCAACACCAGCTAATGGTCTCCAGAACCCTGCAAGCTAGAAATTACCATAACACCCTCGGTG
TAAAGCCCCAGCCCATGAAGCTCCAGGCCTCGGTCGCTGCACCTCAGTCAAAACCCACGAAGCTCTAC
AGAGGGGTGAGGCAAAGGCACTGGGGTAAATGGGTTGCAGAGATCAGACTGCCGAAGAATCGAACCAG
GCTTTGGCTTGGAACCTTTGACACTGCTGAAGAAGCTGCCTTGGCCTATGACAAGGCTGCTTACAAAT
TGAGAGGGGACTACGCGAGGCTCAATTTCCCTAACCTGAAGCATACCCATCTGGCTGGCAACCCTCTG
CACTCGTCTGTTGATGCCAAGCTCGAAGCAATTTGCCAGACCCTGGAGAGCCCTGGGAAGAAGAAGGT
TAGCACCGGGTCGAAGCCTGAGCCGGTTGTTTCATCGCCGACTGTTGAGACCGAGGAGGAGTCTTCCT
CTTCTTCTTCGGTGACGGGGGCGACGAATTCTCCGGTTTCAGAGATCGAGAGCTTGGATTTCAATGAG
GTGCCGTGGGATGAGACTGAGGACTTTGTGTTGAGGAAATACCCATCCTATGAGATTGATTGGGATTC
TATATTGTCTTCAGCTTAGTAGTGTCTGGTTTGTGCTTGTTGTTGTTAGATTTGGGGGTCTTTAGTGG
CTGTTGCAAATGGAGTTTTTGGCATTTGCGTAGCCTTGTTCAGGGATTTTTAGTTTAGTTTATTTTTT
TTTCTTTCTTTTAGAGTGTAAGAACTCATGGCCTCTGCTGATTATTTTTGCTTGGCGAGGCCGTTGAG
GGGTTAAGTGTACTACTATTGTCAGTTCCAGGTGTAACTCTTCTCTGTTGCAGCTTTGTAAGTATGAG
TGTATCTTGTGTTTAATTAAGGTATATTTGTAGCTTTTGAAAAAAAAAAAAAAAAA

**SEQ ID NO 276, Asparagus officinalis – protein**

MDQPDLSQVGPVGLTQLSPLQIQQIQAQIQLNHQHQLMVSRTLQARNYHNTLGVKPQPMKLQASVAAP
QSKPTKLYRGVRQRHWGKWVAEIRLPKNRTRLWLGTFDTAEEAALAYDKAAYKLRGDYARLNFPNLKH
THLAGNPLHSSVDAKLEAICQTLESPGKKKVSTGSKPEPVVSSPTVETEEESSSSSVTGATNSPVSE
IESLDFNEVPWDETEDFVLRKYPSYEIDWDSILSSA

**SEQ ID NO 277, Aloe vera - DNA**

AAATCCAATCTTTCGACCGCCGACCTGAGCCAGCTGTGCCCGATAGGTTTGAATCACCTGAGCTCGCT
CCAGATCCAACAAATTCAAGCCCAGATCCAGCTCAACCAGCAGCAGCAGCTCATGATATCGAGAGCCA
TGCAAGCAAGGAACTATAACCTCGGGGTGAGATCTCAGCCCATGAAGCTCGCCGCCGCTGCAGCGCCG
CCTCAGCCGAAGCCGACGAAGCTCTACAGGGGCGTGAGGCAGCGGCACTGGGGCAAGTGGGTGGCCGA
GATCAGACTACCGAAGAACAGGACAAGGCTCTGGCTCGGCACTTTTGACACAGCCGAGGAGGCCGCCT
TGGCCTACGACAAGGCCGCCTACAAGCTGAGGGGGGACTACGCCAGGCTCAACTTCCCCCACCTGAAG
CACACCGGTGCCCACCTGGCTCCCGGCGGCCCCCTGCACTCCTCTGTGGACGCCAAGCTGCAGGCCAT
CTGCCAGAGCTTGGAGCAGAATAAGAGCTCAAACTCAAACTCATCAAAGAAAGAGAAGAGGGGGGATG
CAGTAGAAGAAAAATCTGACAAGGTGGTGGTTGTTGTTGCCGAGGGCGAG

FIGURE 29 (CONTINUED)

GAGTCTTGCTCATCTTCTTCGATGAACACGGGGAGTGCGAGCTCGCCATCGTCGGAGATAGAGAGCCT
GGACTTCACGGAGGTGCCATGGGATGAGTCTGAGGACTTTGTGCTGAGGAAATACCCTTCATGGGAGA
TTGATTGGGATGCTATACTGTCTTAATGTTTTGGTAGTCGTTGTGTTTTGTGGTTTGTTTCTAGGGTT
TTTAGTCGTTGGTGGCTGGTTGCAAATGGAGTTTTTGGCATTTGCACAGCCTTTTAGAGCTCAAGGTC
TTTGCTGGTTATTTTTTCTTGGCAAAGGACTGGGTGGGGGGAGTAGTTCTGTTTCAGATTTCAGGTTG
TTGTTATTGTCATCTTTGTAGTAGCTTTGTATTATAATCAGTTTATTTGTGTTCCAGTTCTTAAAAAA
AAAAAAAAAAA

**SEQ ID NO 278, Aloe vera - protein**

MISRAMQARNYNLGVRSQPMKLAAAAAPPQPKPTKLYRGVRQRHWGKWVAEIRLPKNRTRLWLGTFDT
AEEAALAYDKAAYKLRGDYARLNFPHLKHTGAHLAPGGPLHSSVDAKLQAICQSLEQNKSSNSNSSKK
EKRGDAVEEKSDKVVVVVAEGEESCSSSSMNTGSASSPSSEIESLDFTEVPWDESEDFVLRKYPSWEI
DWDAILS

**SEQ ID NO 279, Gossypium hirsutum - DNA**

ACCTCTTTTAATCCGTTCTGGATTTAGGACCTGTTCTTCAAGTTTTGGTAAGAAACAGGTTAATTTTT
TATTAAAATTTGGTCCTCATCTTATTTCTAGTTTTAGTGTAATGGCAGCTGCAATGGATTTCATTAGT
ATTGGAGTAGATCAATCAGATCTATATGGGGGAGAATTGATGGAAGCTCTCGAACCTTTTATGAAAAG
TGTTTCCTCCTCTTCCCCTTCCCCTTCCCCTTCCCCTTCCCCTTCTTCTCTTCCTTCTACTTCTTACC
TTTCTTTCTCTTCTTCCGAAACACAACCTAATTTTTACCCAGATAGCTGTTGTTACCCTTACCCTACA
CCAATGGACTCAGTATCATGTCCCCAACAGCCTCAAACTGGTTCAACAATTGGGCTAAATAGCTTGAC
ACAAGCTCAGATCCATCAGATCCAGCTTCAATTCCACCTCCACAACAACCAACCAAGCTACCTTTGCC
AAAGTCCCCAACCCAACACCATCAGCGCCAATAGTAACCCGATGGTTAGCTTTCTTTGCCCTAAACCT
GTCCCAATGAAACACGTGGGTGCGCCATCCAAACCCACCAAACTTTACAGAGGAGTTAGGCAACGCCA
CTGGGGAAAATGGGTCGCTGAGATCCGGTTACCTAGAAACCGGACACGTCTTTGGTTAGGCACTTTTG
ACACTGCTGAGGAAGCAGCCTTGGCTTATGACAAAGCAGCTTATAAACTAAGAGGTGACTTTGCGAGA
CTCAACTTCCCTAACCTTCGTCACCACGGTTCCCACGTAGGTGACTACAAGCCTTTGCCTTCCTCTGT
TGACGCTAAGCTTCAAGCCATTTGTGAGAGCTTGGTACAAAACCCTAAGCAAGGGAGCAAGAAGAAAT
CATCCAAGGTTACGGCAGACACCAAAAGCAGGAACAACAAAAAATCCGACATGGCAGAGCCAAAGCCC
GAGGAGAATACAGCGAAAGTGGAGAACTCCTCATCTTTGTCTACGGTTCAATCCGAGAGTGAGGGTTC
GGCTGTATCTTCACCTTTATCAGATCTTACATTCTCGGATTTCGACGAACAACCATGGCCGGAAGTCG
TTTCTTCTTCGGAAACTTTTATGTTGTCCAAGTACCCTTCAGAGATCGATTGGGATTCCATTCTAAAA
GCCTGAAGGGAAAAACCCAAGTTTCGTTTTCTGTGTAACAGTCTTTTGTTGTTTAGGAGTTTCCTTTC
GTGTATTTTTTTTTGTAAAGCTAGCTGCAATGGAGTTTTTGGCAGTTGCAGTGGCATGTATTTTAGGT
TATTAAGAGTCTGTTAATGAGTGTAAGTGCTTGTGTTCATAAGAATTTGTGATTTTCTCCATGCTGGT
CAGCTCGTTTTTTTACTTGCTGAACCAAAAGGTGAAATTTGTTAGTGTCTATAAAAAAAGTTTATAGC
TTTTGTATTTTGTACGAATTAAATGTTATTTATGTTGTTGTATTCTCTGGTATCGTGTTTTCTTC

**SEQ ID NO 280, Gossypium hirsutum - protein**

MAAAMDFISIGVDQSDLYGGELMEALEPFMKSVSSSSPSPSPSPSPSSLPSTSYLSFSSSETQPNFYP
DSCCYPYPTPMDSVSCPQQPQTGSTIGLNSLTQAQIHQIQLQFHLHNNQPSYLCQSPQPNTISANSNP
MVSFLCPKPVPMKHVGAPSKPTKLYRGVRQRHWGKWVAEIRLPRNRTRLWLGTFDTAEEAALAYDKAA
YKLRGDFARLNFPNLRHHGSHVGDYKPLPSSVDAKLQAICESLVQNPKQGSKKKSSKVTADTKSRNNK
KSDMAEPKPEENTAKVENSSSLSTVQSESEGSAVSSPLSDLTFSDFDEQPWPEVVSSSETFMLSKYPS
EIDWDSILKA

FIGURE 29 (CONTINUED)

**SEQ ID NO 281, Arabidopsis thaliana - DNA**

ATGAATTTGTACACTTGTAGCAGATCGTTTCAAGACTCTGGTGGTGAACTCATGGACGCGCTTGTACC
TTTTATCAAAAGCGTTTCCGATTCTCCTTCTTCTTCTTCTGCAGCGTCTGCGTCTGCGTTTCTTCACC
CCTCTGCGTTTTCTCTCCCTCCTCTCCCCGGTTATTACCCGGATTCAACGTTCTTGACCCAACCGTTT
TCATACGGGTCGGATCTTCAACAAACCGGGTCATTAATCGGACTCAACAACCTCTCTTCTTCTCAGAT
CCACCAGATCCAGTCTCAGATCCATCATCCTCTTCCTCCGACGCATCACAACAACAACAACTCTTTCT
CGAATCTTCTCAGCCCAAAGCCGTTACTGATGAAGCAATCTGGAGTCGCTGGATCTTGTTTCGCTTAC
GGTTCAGGTGTTCCTTCGAAGCCGACGAAGCTTTACAGAGGTGTGAGGCAACGTCACTGGGGAAAATG
GGTGGCTGAGATCCGTTTGCCGAGAAATCGGACTCGTCTCTGGCTTGGGACTTTTGACACGGCGGAGG
AAGCTGCGTTGGCCTATGATAAGGCGGCGTACAAGCTGCGCGGCGATTTCGCCCGGCTTAACTTCCCT
AACCTACGTCATAACGGATCTCACATCGGAGGCGATTTCGGTGAATATAAACCTCTTCACTCCTCAGT
CGACGCTAAGCTTGAAGCTATTTGTAAAAGCATGGCGGAGACTCAGAAACAGGACAAATCGACGAAAT
CATCGAAGAAACGTGAGAAGAAGGTTTCGTCGCCAGATCTATCGGAGAAAGTGAAGGCGGAGGAGAAT
TCGGTTTCGATCGGTGGATCTCCACCGGTGACGGAGTTTGAAGAGTCCACCGCTGGATCTTCGCCGTT
GTCGGACTTGACGTTCGCTGACCCGGAGGAGCCGCCGCAGTGGAACGAGACGTTCTCGTTGGAGAAGT
ATCCGTCGTACGAGATCGATTGGGATTCGATTCTAGCTTAG

**SEQ ID NO 282, Arabidopsis thaliana - protein**

MNLYTCSRSFQDSGGELMDALVPFIKSVSDSPSSSSAASASAFLHPSAFSLPPLPGYYPDSTFLTQPF
SYGSDLQQTGSLIGLNNLSSSQIHQIQSQIHHPLPPTHHNNNNSFSNLLSPKPLLMKQSGVAGSCFAY
GSGVPSKPTKLYRGVRQRHWGKWVAEIRLPRNRTRLWLGTFDTAEEAALAYDKAAYKLRGDFARLNFP
NLRHNGSHIGGDFGEYKPLHSSVDAKLEAICKSMAETQKQDKSTKSSKKREKKVSSPDLSEKVKAEEN
SVSIGGSPPVTEFEESTAGSSPLSDLTFADPEEPPQWNETFSLEKYPSYEIDWDSILA

**SEQ ID NO 283, Arabidopsis thaliana – DNA**

ATCGGTGAGGTTGAGAGTAATTCACTACACACACACAAAAAAATAAATTGAGTGCCTCCCCCAAAAACA
AAATTGGTAGATAACGAGCAATTGTTTTTTTTCAGATTTGATCCTGAATTTTTACATTTTTTTTTTTG
CAATCTCCCCCTAATCTGTTGTTTCTCGCTTCTTCTTCTGTTAATCATCTGTCTTTCAAAAAGAAAGA
AAAAAGAAAAAATTCGATTTCTGGGTTTGTTTTTGTCATACAGAAAAAAAATCAAGCTTATGAATTTGTG
TTTAATTTTTTGTTTTAATTTGAAAGGCAGGTTTTTTCAGAACGAGATCGTTTTTTCAAATTTCTTCT
GATTTTACCTCTTTTTTTCTTCTTAGATTTTAGTGAATCGAGGGTGAAATTTTTGATTCCCTCTTTTC
GGATCTACACAGAGGTTGCTTATTTCAAACCTTTTAGATCCATTTTTTTTTAATTTTCTCGGAAAAAT
CCCTGTTTCTTTACTTTTTTATAAGTCTCAGGTTCAATTTTTTCGGATTCAAATTTTTATTTTAAATG
GCAGCTGCTATGAATTTGTACACTTGTAGCAGATCGTTTCAAGACTCTGGTGGTGAACTCATGGACGC
GCTTGTACCTTTTATCAAAAGCGTTTCCGATTCTCCTTCTTCTTCTTCTGCAGCGTCTGCGTCTGCGT
TTCTTCACCCCTCTGCGTTTTCTCTCCCTCCTCTCCCCGGTTATTACCCGGATTCAACGTTCTTGACC
CAACCGTTTTCATACGGGTCGGATCTTCAACAAACCGGGTCATTAATCGGACTCAACAACCTCTCTTC
TTCTCAGATCCACCAGATCCAGTCTCAGATCCATCATCCTCTTCCTCCGACGCATCACAACAACAACA
ACTCTTTCTCGAATCTTCTCAGCCCAAAGCCGTTACTGATGAAGCAATCTGGAGTCGCTGGATCTTGT
TTCGCTTACGGTTCAGGTGTTCCTTCGAAGCCGACGAAGCTTTACAGAGGTGTGAGGCAACGTCACTG
GGGAAAATGGGTGGCTGAGATCCGTTTGCCGAGAAATCGGACTCGTCTCTGGCTTGGGACTTTTGACA
CGGCGGAGGAAGCTGCGTTGGCCTATGATAAGGCGGCGTACAAGCTGCGCGGCGATTTCGCCCGGCTT
AACTTCCCTAACCTACGTCATAACGGATCTCACATCGGAGGCGATTTCGGTGAATATAAACCTCTTCA
CTCCTCAGTCGACGCTAAGCTTGAAGCTAT

FIGURE 29 (CONTINUED)

TTGTAAAAGCATGGCGGAGACTCAGAAACAGGACAAATCGACGAAATCATCGAAGAAACGTGAGAAGA
AGGTTTCGTCGCCAGATCTATCGGAGAAAGTGAAGGCGGAGGAGAATTCGGTTTCGATCGGTGGATCT
CCACCGGTGACGGAGTTTGAAGAGTCCACCGCTGGATCTTCGCCGTTGTCGGACTTGACGTTCGCTGA
CCCGGAGGAGCCGCCGCAGTGGAACGAGACGTTCTCGTTGGAGAAGTATCCGTCGTACGAGATCGATT
GGGATTCGATTCTAGCTTAGGGGCAAAATAGGAAATTCAGCCGCTTGCAATGGAGTTTTTGTGAAATT
GCATGACTGGCCCAAGAGTAATTAATTAAATATGGATTAGTGTTAAATTTCGTATGTTAATATTTGTA
TTATGGTTTGTATTAGTCTCTCTGTGTCGGTCCAGCTTGCGGTTTTTTGTCAGGCTCGACCATGCCAC
AGTTTTCATTTTATGTAATCTTTTTTTCTTTTGTCTTATGTAATTTGTAGCTTCAGTTTCTTCATCTA
TAATGCAATTTTATTATGATTATGTAA

**SEQ ID NO 284, Arabidopsis thaliana - protein**

MAAAMNLYTCSRSFQDSGGELMDALVPFIKSVSDSPSSSSAASASAFLHPSAFSLPPLPGYYPDSTFL
TQPFSYGSDLQQTGSLIGLNNLSSSQIHQIQSQIHHPLPPTHHNNNNSFSNLLSPKPLLMKQSGVAGS
CFAYGSGVPSKPTKLYRGVRQRHWGKWVAEIRLPRNRTRLWLGTFDTAEEAALAYDKAAYKLRGDFAR
LNFPNLRHNGSHIGGDFGEYKPLHSSVDAKLEAICKSMAETQKQDKSTKSSKKREKKVSSPDLSEKVK
AEENSVSIGGSPPVTEFEESTAGSSPLSDLTFADPEEPPQWNETFSLEKYPSYEIDWDSILA

**SEQ ID NO 285, Arabidopsis thaliana - DNA**

AAAAAACAAATATTTAGAAACAAAAAATGCTATAATTCTTCCTTTTTCTTGTTCTTTCAGAGATTTTG
ATTTCTATTCAATCGACTAAGAGGGTGTGTTTTTAATCCCTCTCTGTTTTAATTTTGTTTCCTAGTCT
TTAAAGATCCATGGCAGCCATAGATATGTTCAATAGCAACACAGATCCTTTTCAAGAAGAGCTCATGA
AAGCACTTCAACCTTATACCACCAACACTGATTCTTCTTCTCCTACGTATTCAAACACAGTCTTCGGT
TTCAATCAAACCACATCTCTCGGTCTAAACCAGCTCACACCTTACCAAATCCACCAAATCCAAAACCA
GCTTAACCAGAGACGTAACATAATCTCTCCAAATCTAGCCCCAAAGCCTGTCCCAATGAAGAACATGA
CCGCTCAGAAACTCTATAGAGGAGTTAGACAAAGGCACTGGGGAAAATGGGTAGCTGAGATCCGTTTA
CCCAAGAACCGGACCCGACTCTGGCTTGGAACTTTCGACACAGCTGAAGAAGCAGCCATGGCTTATGA
CCTAGCTGCTTACAAGCTAAGAGGCGAGTTCGCGAGACTTAATTTCCCACAGTTCAGACACGAGGATG
GATACTACGGAGGAGGTAGCTGTTTCAATCCTCTTCATTCCTCTGTCGACGCAAAGCTCCAAGAGATT
TGTCAGAGCTTGAGAAAAACAGAGGATATTGACCTCCCCTGTTCTGAAACAGAGCTTTTCCCGCCAAA
AACAGAGTATCAAGAAAGTGAATATGGGTTCTTGAGATCTGATGAGAATTCGTTTTCAGATGAGTCTC
ATGTGGAATCTTCTTCGCCGGAATCTGGTATTACTACGTTCTTGGACTTTTCGGATTCTGGATTTGAT
GAGATTGGGAGTTTCGGGCTGGAGAAGTTTCCTTCTGTGGAGATTGATTGGGATGCGATTAGCAAATT
GTCCGAATCTTAAACAAAGCAAAGAGAAGACTTTTTCTTTTAGGAGTTTGTCTTTCAATTTCAGTGTC
TTATATTAATCTCTCTGCAACTGAAATTTTTAACAGTTGCGGAGAGAATCGTCTCTAGGGTTTGTTTC
TCTTCCTCCATGTTTTGGTCTGACTGGTTAATGTCTTTTTTTTTTTTTTGAACTTTCAAAAACCTTTG
TCATTGACCAATCGGAGAAGTTTCCATGTATTCTCTCATCTTTAAATTTCTAATGAAGAATGTAAATT

**SEQ ID NO 286, Arabidopsis thaliana - protein**

MAAIDMFNSNTDPFQEELMKALQPYTTNTDSSSPTYSNTVFGFNQTTSLGLNQLTPYQIHQIQNQLNQ
RRNIISPNLAPKPVPMKNMTAQKLYRGVRQRHWGKWVAEIRLPKNRTRLWLGTFDTAEEAAMAYDLAA
YKLRGEFARLNFPQFRHEDGYYGGGSCFNPLHSSVDAKLQEICQSLRKTEDIDLPCSETELFPPKTEY
QESEYGFLRSDENSFSDESHVESSSPESGITTFLDFSDSGFDEIGSFGLEKFPSVEIDWDAISKLSES

FIGURE 29 (CONTINUED)

**SEQ ID NO 287, Oryza sativa - DNA**

ATGGATCGCCAATGGCGTTGCCTCGCTGCTGCGGCTTCTACTAGTGGTAATAGTAAGCTTCCTCCGCT
GCCGATGGCGCTGGGCGGCGCCGTGGAGTACGGGCAGCTCGTCCATGGCGCGGCGGCGCCCGTGGCGC
CGTTCTTCGTGGACGCCGAGCAGCAGAGCCTGTCGCCGGCGACGGCCATGGTTCTTGGCGCCGGGTGG
TATAACTATAACCTTGTCACGCCGTCGCAGGCGGCGCAGCTGCACCACCGGCTGCGACGGGCGGTGGG
CGCGGCGCCGTGCTCGATGAAGCGGTGCGGTGGCATGGCGGCGGCGGCGGCGGGGCGGCTTGCGCTGG
TGGGGCCGGCGCCGGTGCAGGCGAAGCTGTACCGCGGCGTGCGGCAGCGGCACTGGGGGAAATGGGTG
GCGGAGATCCGGCTGCCGCGGAACCGGACGCGGCTGTGGCTGGGCACGTACGACACCGCCGAGGACGC
CGCGCTCGCCTACGACGGTGCCGCGTTCCGGCTGCGCGGCGACGCCGCCCGCCTCAACTTCCCCGAGC
TCCGGCGCGGAGGGCGGCACCACGCGCCGGCGCTCAGCGCGTCCGTCGACGCCAAGATCCTCCAAGCC
ACCACCACCACCACGGCGGACACCGCCGCCGCCGCAGCACCGGCGTCGACGAACACCACGCCGCCGCC
GTCGCCGCGCGTCGTCAAGACCGAGCCGGGCTGCTGCTCCGTCTCCGAAGCCTCCACGACGACGACGG
CCGACGCCGCCGACGTCTCGTCCACAGGGTCTTCCCCATCCCCGACCTCATCGAATCAGGCCGCCACC
GCCACGCCGCCGGCGCCGCGGCCGCCGCCGCCGTTGCCGGAGACGATTCAGCAGCTGGACTTCACGGA
GGCGCCATGGGACGAGGCCGACGGCTTCGCGCTGCGCCGGTATCCGTCGTGGGAGATCGACTGGGACG
CCATCCTCTCCTGA

**SEQ ID NO 288, Oryza sativa - protein**

MDRQWRCLAAAASTSGNSKLPPLPMALGGAVEYGQLVHGAAAPVAPFFVDAEQQSLSPATAMVLGAGW
YNYNLVTPSQAAQLHHRLRRAVGAAPCSMKRCGGMAAAAAGRLALVGPAPVQAKLYRGVRQRHWGKWV
AEIRLPRNRTRLWLGTYDTAEDAALAYDGAAFRLRGDAARLNFPELRRGGRHHAPALSASVDAKILQA
TTTTTADTAAAAAPASTNTTPPPSPRVVKTEPGCCSVSEASTTTTADAADVSSTGSSPSPTSSNQAAT
ATPPAPRPPPPLPETIQQLDFTEAPWDEADGFALRRYPSWEIDWDAILS

**SEQ ID NO 289, Broussonetia papyrifera - DNA**

CACCACACCAGTTTTCTCTGATCCCTTTAGAGAAGAGCTCATGAAAGCACTTGAACCTTTTATGAAAA
GTGCTCATAATAATATCAACAACAACATTTCACCAATCTCTTCTTCTTCCTCTTCTTCTTACCTTTCT
AGTGAGCCCAACTTTTACCCTGAATCTGAAACTTGCTCACCTTCAACTACCCAAATGTTTTCCAGTGG
GTTGTTCTCCAGCTTTAACCACATGGGTTCTGAGCAGACTGGTTCTTTAGGCTTAAACCAGCTCACCC
AATCCCAGATTCTCCAAATTCAAGCCCAAATCTACTTCCAACAACAACAGCAACAGCAACAAAATCTA
CTCATGACCACCATAACTCCGCCCCAAAACAGCCTCAACTACCTCGGTCCGAGGGCGGTTCCGATGAA
GAACGTCGGCGCCAATTCAAAGCCCAACAAACTCTACAGAGGAGTGAGGCAGAGGCATTGGGGCAAAT
GGGTCGCCGAGATCAGACTCCCCAAGAACCGGACCCGCCTCTGGCTGGGCACCTTCGACACCGCCGAG
GAAGCCGCCTTGGCCTACGACAAGGCGGCGTACAAGCTCCGCGGGGACTTCGCCCGCCTCAACTTCCC
CCACCTCCGCCACGAAGGCGCCCACGTCTCTGGCGAGTTCGGCGAGTACAAGCCCCTCCATTCCTCCG
TCGACGCCAAGCTTCAGGCGATTTGCCAAAGCCTGGCGAATTCGCAGAAGCAGGGGAGTGCCAAGGAG
GCTTGTTCCGAGCCGGAGGTGAAGCCAGTCATCGAGCCCAAGATGGCGTCCGATAATTCTCCGAAAGG
CGAATTGGAGGTGTCGTCTTCGTCGTTGTCGTCGTCGTCGTCGTTGTCGTTGTCGTTGTCGTTGTCGT
CGCCGTTGTCGGATGAGTCTTCGGCGGGGTCATCCTCGCCGGAGTCCGATGTCACGTTGTTGGACTTC
TCGGATTCTCATTGGGATGGGAATGAGAATTTTGGGCTAGGGAAGTACCCTTCAGTGGAGATCGACTG
GGATGCTCTGTGATCGTAATAAATGTTGGTTATGTTGTCATTTTCTCTTTTTTATTTTTCCTGCGTTA
TTAGTTGTTTTTAAGGTTTTTTTTTTTAGTTGTACAAGCACGGCTTCTGCGATGGAATTTTTAACATG
GCAGGGGTTTAGCTCAGTTTTTTAAATTTAGG

FIGURE 29 (CONTINUED)

AAGGGTCAGTAAGTCAGTCAGTCAGTCAGATGTTTTTATGTTGTAATATTTGATGTCGTTTGATATCT
CCTATGTTGGTCAGCTGGTAGTTTTTTTCCTTGCTAGGCCTGGCCATGGGAGATCTCTCTGGGTTGTA
TTTACTACTTTTTGAATGTAATTAGGCAAGTCTACTTTATATAAAAAAAAAAAAAAAAAA

**SEQ ID NO 290, Broussonetia papyrifera - protein**

MKALEPFMKSAHNNINNNISPISSSSSSSYLSSEPNFYPESETCSPSTTQMFSSGLFSSFNHMGSEQT
GSLGLNQLTQSQILQIQAQIYFQQQQQQQNLLMTTITPPQNSLNYLGPRAVPMKNVGANSKPNKLYR
GVRQRHWGKWVAEIRLPKNRTRLWLGTFDTAEEAALAYDKAAYKLRGDFARLNFPHLRHEGAHVSGEF
GEYKPLHSSVDAKLQAICQSLANSQKQGSAKEACSEPEVKPVIEPKMASDNSPKGELEVSSSSLSSSS
SLSLSLSLSSPLSDESSAGSSSPESDVTLLDFSDSHWDGNENFGLGKYPSVEIDWDAL

**SEQ ID NO 291, Jatropha curcas - DNA**

GGACTCAGATAGTTGCTCCACATCGACTGCCCTTCCATTTTCAAATGGGTTCTCGATCCAAGACCCCA
ACCGTCTTCAGCAACCTACTTGTTCAATCGGGCTATGTCTTACTCCAACCCAGATCCACCAGATCCAG
ACCCAAATCCATTACCAGAATCAAAATGGATTCAATTTCCAGAATTTCCACACCCAAAACCAACATGG
CTTAACTTTTTAGGTCCGAAACCCGTGCCCATGAAGCAGGTGGGTTCACCACCAAAACCCACTAAGCT
CTACAGAGGAGTAAGGCAGCGACATTGGGGCAAATGGGTTGCCGAGATCCGACTACCCAAGAACCGTA
CACGACTCTGGCTTGGTACTTTTGACACAGCAGAAGAAGCCGCTTTAGCTTATGACAAAGCGGCGTAC
AAACTCCGTGGCGACTTCGCGAGACTTAACTTCCCTAACCTCCGCCACCAAGGGTCCCACATTGAAGG
CAGCTTCGGCGAGTATAAGCCTCTCCATTCCTCGGTCGATGCGAAACTGCAAGCTATTTGTCAAAGCT
TAGCAGAATCGCAGAAACAAGGAGGAAAAGCAGAGAAGCAATCAAACTCGTCAGCGAAAAAGAAGACT
TCGGTGGGGACTACTCCAGCGACGGCGGAGAAGGTTAAGGAAGCTAAGGCACCGCAACAGGTTGTTCC
GGACAAGTGTTGCAAGGTCGAGACACCATCGTCAGTGTTGACAGAAAGTGAAGCCTCTGGCGGATCTT
CACCGTTGTCGGATCTTACGTTTCCGGATCTAGAAGAGGCACCATTGGATGTTGATTCTGGAAATTTT
AATTTGGAGAAGTACCCATCTTATGAAATTGATTGGGCTTCTCTTTTATCTTCTTAGATTTGGTTATG
TTATGTTATTTATCTTTTATCTTTATTTTGCAGTTATGTTTAGTTCTTAGGCGTGTGGTTGCTGCAAT
GAGTTTTTGGCAGTTGCAGAGCCAAAAAAAAAAAAAAAAAA

**SEQ ID NO 292, Jatropha curcas - protein**

MSYSNPDPPDPDPNPLPESKWIQFPEFPHPKPTWLNFLGPKPVPMKQVGSPPKPTKLYRGVRQRHWGK
WVAEIRLPKNRTRLWLGTFDTAEEAALAYDKAAYKLRGDFARLNFPNLRHQGSHIEGSFGEYKPLHSS
VDAKLQAICQSLAESQKQGGKAEKQSNSSAKKKTSVGTTPATAEKVKEAKAPQQVVPDKCCKVETPSS
VLTESEASGGSSPLSDLTFPDLEEAPLDVDSGNFNLEKYPSYEIDWASLLSS

**SEQ ID NO 293, Arabidopsis thaliana - DNA**

TCTTCCTCCGACGCATCACAACAACAACAACTCTTTCTCGAATCTTCTCAGCCCAAAGCCGTTACTGA
TGAAGCAATCTGGAGTCGCTGGATCTTGTTTCGCTTACGGTTCAGGTGTTCCTTCGAAGCCGACGAAG
CTTTACAGAGGTGTGAGGCAACGTCACTGGGGAAAATGGGTGGCTGAGATCCGTTTGCCGAGAAATCG
GACTCGTCTCTGGCTTGGGACTTTTGACACGGCGGAGGAAGCTGCGTTGGCCTATGATAAGGCGGCGT
ACAAGCTGCGCGGCGATTTCGCCCGGCTTAACTTCCCTAACCTACGTCATAACGGATTTCACATCGGA
GGCGATTTCGGTGAATATAAACCTCTTCACTCCTCAGTCGACGCTAAGCTTGAAGCTATTTGTAAAAG
CATGGCGGAGACTCAGAAACAGGACAAATCGACGAAATCATCGAAGAAACGTGAGAAGAAGGTTTCGT
CGCCAGATCTATCGGAGAAAGTGAAGGCGGAGGAGAATTCGGTTTCGATCGG

FIGURE 29 (CONTINUED)

TGGATCTCCACCGGTGACGGAGTTTGAAGAGTCCACCGCTGGATCTTCGCCGTTGTCGGACTTGACGT
TCGCTGACCCGGAGGAGCCGCCGCAGTGGAACGAGACGTTCTCGTTGGAGAAGTATCCGTCGTACGAG
ATCGATTGGGATTCGATTCTAGCTTAGGGGCAAAATAGGAAATTCAGCCGCTTGCAATGGAGTTTTTG
TGAAATTGCATGACTGGCCCAAGAGTAATTAATTAAATATGGATTAGTGTTAAATTTCGTATGTTAAT
ATTTGTATTATGGTTTGTATTAGTCTCTCTGTGTCGGTCCAGCTTGCGGTTTTTTGTCAGGCTCGACC
ATGCCACAGTTTTCATTTTATGTAATCTTTTTTTCTTTTGTCTTATGTAATTTGTAGCTTCAGTTTCT
TCATCTATAATGCAATTTTATTATGATTATGTG

**SEQ ID NO 294, Arabidopsis thaliana - protein**

LPPTHHNNNNSFSNLLSPKPLLMKQSGVAGSCFAYGSGVPSKPTKLYRGVRQRHWGKWVAEIRLPRNR
TRLWLGTFDTAEEAALAYDKAAYKLRGDFARLNFPNLRHNGFHIGGDFGEYKPLHSSVDAKLEAICKS
MAETQKQDKSTKSSKKREKKVSSPDLSEKVKAEENSVSIGGSPPVTEFEESTAGSSPLSDLTFADPEE
PPQWNETFSLEKYPSYEIDWDSILA

**SEQ ID NO 295, Oryza sativa - DNA**

CTTGGTTAGGTTGCTTGCAGAGAGCGAGAGACCCAGGGCCTTTTAGATCCAGGGCTCCCAGATCTGCT
GTTTTTCTTTCATCTTCTTTGTGTTCTAGTGTTAGGTTGGTAGCCAAGAGGGGTTCTTTTTCCACACC
TCACTTGCTAGATCTACCACCAAAAAGCCATCTGTTTTTTTACTGTGGCTGTCGATTTCTCCCTCCTT
CCTGCCTGTTCTTGATTTTGGATTCGGAACCAGGCAAATCTTTGTTACTACTGTTTTAGTATCAGAAA
AAAAAATCCCAAAAAAAAGAGTGAGTAGATGGCTGCAGCTATAGATCTGTCAGGGGAGGAGCTGATGA
GAGCACTCGAGCCTTTTATCCGAGATGCCTCCGGCTCGCCTCCGGTTTGTTCCCAGTTTAGTCCCACC
TCGCCATTCTCCTTCCCGCACGCGTTCGCGTACGGTGGCGGGCTGGCCCAGCAGCCAGAGCTCAGCCC
GGCCCAGATGCACTACATCCAGGCCCGCCTCCACCTCCAGCGGCAGGCGGCGCAGGCCGGCCCGCTCG
GCCCGCGCGCGCAGCCGATGAAGGCGTCGTCGTCGTCGGCTTCGGCGGCGGGGGCGGCGGCGACGCCG
CCGCGGCCGCAGAAGCTGTACCGCGGCGTGCGGCAGCGGCACTGGGGGAAGTGGGTGGCGGAGATCCG
CCTCCCGCGGAACCGCACGCGGCTCTGGCTCGGCACCTTCGACACAGCCGAGGAGGCGGCGCTTGCCT
ACGACCAGGCGGCGTACCGCCTCCGCGGCGACGCGGCGCGGCTCAACTTCCCCGACAACGCCGCCTCC
CGCGGCCCGCTCCACGCCTCCGTCGACGCCAAGCTCCAGACGCTCTGCCAGAACATCGCCGCCGCCAA
GAACGCCAAGAAGTCCTCTGTCTCCGCCTCCGCCGCCGCAACATCCTCCGCGCCCACCAGCAACTGCT
CGTCGCCGTCCTCCGACGACGCGTCGTCCTGCCTGGAGTCCGCCGACTCGTCGCCCTCCCTGTCGCCG
TCCTCCGCCGCCACCACGGCCGAGACGCCGGCGACCGTGCCGGAGATGCAGCAGCTCGACTTCAGCGA
GGCGCCGTGGGACGAGGCCGCCGCCTTCGCCCTCACCAAGTACCCGTCCTACGAGATCGACTGGGACT
CCCTCCTCGCCGCCAATTAACACCACCACCACTTCTTGGCTAGTACTCACGCCGTCGTTAGCCTAATC
GTCGCCGCCGCCGCCGCCGTGCAGATGGGATTTTAGACATTCTGCACCGCACGGACGGGCATGGATTA
GCAGTTTTATAGTCCCTAATCCTTTTGGTTTGGTTCGTTTGTGTACGTAGATCGATCTCTCTCCGGAC
TTGTTTCCTCTGTAGATGCTAGGGTTGGTTGGTGTTCTTCCTCAACC

**SEQ ID NO 296, Oryza sativa - protein**

MAAAIDLSGEELMRALEPFIRDASGSPPVCSQFSPTSPFSFPHAFAYGGGLAQQPELSPAQMHYIQAR
LHLQRQAAQAGPLGPRAQPMKASSSSASAAGAAATPPRPQKLYRGVRQRHWGKWVAEIRLPRNRTRLW
LGTFDTAEEAALAYDQAAYRLRGDAARLNFPDNAASRGPLHASVDAKLQTLCQNIAAAKNAKKSSVSA
SAAATSSAPTSNCSSPSSDDASSCLESADSSPSLSPSSAATTAETPATVPEMQQLDFSEAPWDEAAAF
ALTKYPSYEIDWDSLLAAN

FIGURE 29 (CONTINUED)

**SEQ ID NO 297, Oryza sativa - DNA**

AACCCAAACGACGGAGACCCACTACTCCGAGCCATCAAGAACACACACACATCCAGAAAGCCTAATCC
AATCCAAAAGCCCTAATCACCCAGACTCCATCTCTGTGAGGTTTGAGAGAGGTAGGTGAGCCGTAGAT
CTGAGCGAGTTCTTGGGTCTTCCAGCAGGTAGATCACTTCATCTGAGGATCAATCTTTTTTTTTTCC
TTTGTTTTTGAGCTTCTGTTGGTGTACAGGACAGAGAGTTCCAGAGCCTTTTAGTTTCTGGTGTTCTG
ATCTGTTCTTGGTGTAAGATTATTGGTCTGATTTGGTAGCCAAGAGGGTTAATTTTTTCCACACCTCC
TTGTGCTAGTTAGCTTAGCTTATACCCCCCTTGTAAAGTGATTAGTAGATCTAGAACTTCTCTTTTCG
TCTGCCAGTTCTTGGATTTTGGAAAGAACAGGTGGTTTGTTATTCAGATTTTTAGGTTAGAAAAAATC
CACAAAAAAAAAGATATTCGATGGCAGCTGCTATAGAAGGAAATCTGATGCGGGCGCTGGGAGAGGCT
CCGTCGCCGCAGATGCAGAAGATCGCGCCGCCGCCGTTTCATCCCGGCTTGCCGCCGGCGCCGGCGAA
CTTCTCCTCGGCCGGAGTCCACGGGTTCCACTACATGGGCCCGGCCCAGCTCAGCCCGGCCCAGATCC
AGCGCGTCCAGGCCCAACTCCACATGCAGCGGCAGGCCCAGTCGGGGCTCGGCCCGCGGGCCCAGCCC
ATGAAGCCCGCTTCGGCGGCTGCTCCGGCGGCGGCGGCGGCGCGGGCGCAGAAGCTGTACCGCGGCGT
GCGGCAGCGGCACTGGGGCAAGTGGGTGGCGGAGATCCGGCTGCCGCGCAACCGCACCAGGCTCTGGC
TCGGCACCTTCGACACCGCCGAGGAGGCGGCGCTCACCTACGACCAGGCCGCGTACCGCCTCCGCGGC
GACGCGGCGCGGCTCAACTTCCCGGACAACGCCGCGTCGCGGGGCCCGCTCGACGCCGCCGTGGACGC
CAAGCTCCAGGCCATCTGCGACACCATCGCCGCGTCCAAGAACGCCTCATCCAGGTCCAGGGGCGGCG
CCGGCAGGGCCATGCCCATCAACGCGCCCCTGGTCGCCGCGGCGTCGTCGTCCTCCGGCTCCGACCAC
TCCGGCGGCGGCGACGACGGCGGCTCGGAGACGTCGTCGTCGTCTGCGGCGGCGTCGCCGCTGGCGGA
GATGGAGCAGCTGGACTTCAGCGAGGTGCCGTGGGACGAGGCGGAGGGGTTCGCGCTCACCAAGTACC
CGTCGTACGAGATCGACTGGGACTCGCTGCTCAACAACAATAACTAGCTCTTCTCTTCCATAGCCCGC
CATTGCCGGCCGGCGCAGATGAGGTTTTAGGCATTCTGCGCGGCGGCGAGGCGATGGATTATATGTTC
GTTCTTGTGTAGATCCTTTTCTTTTTGTGTTGGTTTTTTAGGTTCCGGAGGCTGCTCGCCGGCGTCGT
TTTTGTGTCCGGCGTCTCCGATGTCTAGTAAGCAGTGACTCGTTAGTGTAGTAGTTGTACAACTCTAC
AAATGTACAAATACTTGTGCATGTAGGTTGTTGTAATCAATTGTTGGTAACTACCTGTAATAGAACTA
CTGTGAATTAACTGTATTAATGTGC

**SEQ ID NO 298, Oryza sativa - protein**

MAAAIEGNLMRALGEAPSPQMQKIAPPPFHPGLPPAPANFSSAGVHGFHYMGPAQLSPAQIQRVQAQL
HMQRQAQSGLGPRAQPMKPASAAAPAAAAARAQKLYRGVRQRHWGKWVAEIRLPRNRTRLWLGTFDTA
EEAALTYDQAAYRLRGDAARLNFPDNAASRGPLDAAVDAKLQAICDTIAASKNASSRSRGGAGRAMPI
NAPLVAAASSSSGSDHSGGGDDGGSETSSSSAAASPLAEMEQLDFSEVPWDEAEGFALTKYPSYEIDW
DSLLNNNN

**SEQ ID NO 299, Lycopersicon esculentum – DNA**

GTTTGTTCCACTTCCACAGAGATGAATTCCCAAATCTTTTCAACTGGGTTTTCTGGGTATGGAATGGA
GCAACAGGGTTCAATTGGGCTGAATCAGTTAACCCCAATTCAGATCCAGCAAATTCAAGCTCAAATCA
ACTTTCAAAACCAACAACAACAGCAGCAGCAGCAGATGATGTTACAGACTGCCCATCATGCTTCCACC
ATGAATTTCTTGGCTCCAAAGCCGGTTCCAATGAAGCAATCTGGGTCGCCACCAAAACCCACGAAGCT
CTACAGAGGTGTTAGACAACGCCACTGGGGTAAGTGGGTCGCTGAGATCCGTTTGCCTAAGAACCGAA
CCCGCCTTTGGCTTGGTACATTTGACACCGCTGAAGAAGCTGCTCTGGCTTACGACAAGGCGGCGTAT
ATGCTTCGTGGCGACTTTGCTCGACTGAACTTCCCTCAACTCCGCCACAACGGCAACCTAATCGGCGG
CGACTTTGGTGAATACAATCCATTGCATTCCTCAGTTGATGCTAAGCTAAAGGACATATGCCAAAGCT
TGGCACAGGGGAAGAGCATTGACTCTAAGAAGAAGAAAACCAAAGGGTTG

FIGURE 29 (CONTINUED)

TCGGCGGAGAAAGCGGCGGTGGTGAAGATGGAGGAAGAGGAGAGCAAAACAGCAGAAGTTGGATCCGA
AAGTGACGGGTCCCATTCCGGTTCCGGTGGATCATCGCCGGTGACCGAACTGATATTCCCGGAGTTCA
CTGAGGAAGAGCCAACTTGGGACATGTCAGAAAATTTTTTGTTGCAGAAGTATCCATCTCATGAAATT
GATTGGGCCTCTCTATAAAATTAGAAAATAATTAAGA

**SEQ ID NO 300, Lycopersicon esculentum - protein**

MNSQIFSTGFSGYGMEQQGSIGLNQLTPIQIQQIQAQINFQNQQQQQQQMMLQTAHHASTMNFLAPK
PVPMKQSGSPPKPTKLYRGVRQRHWGKWVAEIRLPKNRTRLWLGTFDTAEEAALAYDKAAYMLRGDFA
RLNFPQLRHNGNLIGGDFGEYNPLHSSVDAKLKDICQSLAQGKSIDSKKKKTKGLSAEKAAVVKMEEE
ESKTAEVGSESDGSHSGSGGSSPVTELIFPEFTEEEPTWDMSENFLLQKYPSHEIDWASL

**SEQ ID NO 301, Zea mays - DNA**

CACGAGCAATCCCCTTCAACAAACGCACCGCACTCCACGGCAGCCAGAAAACACATCCCACGGGGCCC
AGACCCGGCGACCCACCTGAGCCCGGCGCAGATGCAGTTCATCCAGGCCCAGCTCCACCTGCAGCGGA
ACCCGGGGCTGGGCCCGCGGGCGCAGCCCATGAAGCCCGCCGTCCCAGTGCCGCCGGCGCCGGCGCCG
CAGCGGCCTGTGAAGCTGTACCGCGGCGTGCGGCAGCGTCACTGGGGCAAGTGGGTGGCCGAGATCCG
GCTCCCCCGGAACCGCACCCGCCTGTGGCTCGGGACCTTCGACACCGCCGAGCAGGCAGCGCTGGCCT
ACGACCAGGCGGCGTACCGCCTCCGCGGGGACGCGGCGCGGCTCAACTTCCCCGACAACGCGGAGTCC
AGGGCGCCGCTCGACCCCGCCGTGGACGCCAAGCTGCAGGCCATCTGCGCCACCATCGCCGCCGCGTC
GTCGTCATCCAAGAATTCCAAGGCCAAGAGCAAGGCGATGCCAATCAACGCGTCCGTTCTGGAAGCGG
CAGCGGCGTCTCCGAGCAACAGCTCCTCCGACGAAGGTTCCGGCTCCGGGTTCGGGTCGGACGACGAG
ATGTCCTCGTCTTCCCCGACGCCGGTGGTGGCGCCGCCGGTGGCGGACATGGGACAGTTGGATTTCAG
CGAGGTTCCGTGGGACGAGGACGAGAGCTTCGTGCTCCGCAAGTACCCGTCCTACGAGATCGACTGGG
ACGCGCTGCTCTCCAACTAGTCGCCCTTCGCCGACAGATGTGCTGTTGTAGTTCAGTAGTGGCAGTAT
CTCTGGCCGCCGCAGATGAGGTTTTAGGCAATCTGCAGGCCGCCGGCCCATGTGTATTAAGTAGGTTT
TGCTCAGTTGTTGGCCCCGGACTTCGCCGGCGTTTTTGTGACCGGCGTCCCCGAGTGCACTGCATTGG
TGTACTGGTCTGTCTGTAAAAAAAAAATGGATCTGTGTACTTCTATAGTGTGTATTCAACCATTGTTCT
TAAAAAAAAAAAAAAAAAAAA

**SEQ ID NO 302, Zea mays - protein**

MQFIQAQLHLQRNPGLGPRAQPMKPAVPVPPAPAPQRPVKLYRGVRQRHWGKWVAEIRLPRNRTRLWL
GTFDTAEQAALAYDQAAYRLRGDAARLNFPDNAESRAPLDPAVDAKLQAICATIAAASSSSKNSKAKS
KAMPINASVLEAAAASPSNSSSDEGSGSGFGSDDEMSSSSPTPVVAPPVADMGQLDFSEVPWDEDESF
VLRKYPSYEIDWDALLSN

**SEQ ID NO 303, Oryza sativa - DNA**

GGAAAGAACAGGTGGTTTGTTATTCAGATTTTTAGGTTAGAAAAAATCCACAAAAAAAAAAGATATTCG
ATGGCAGCTGCTATAGAAGGAAATCTGATGCGGGCGCTGGGAGAGGCTCCGTCGCCGCAGATGCAGAA
GATCGCGCCGCCGCCGTTTCATCCCGGCTTGCCGCCGGCGCCGGCGAACTTCTCCTCGGCCGGAGTCC
ACGGGTTCCACTACATGGGCCCGGCCCAGCTCAGCCCGGCCCAGATCCAGCGCGTCCAGGCCCAACTC
CACATGCAGCGGCAGGCCCAGTCGGGGCTCGGCCCGCGGGCCCAGCCCATGAAGCCCGCTTCGGCGGC
TGCTCCGGCGGCGGCGGCGGCGCGGGCGCAGAAGCTGTACCGCGGCGTGCGGCAGCGGCACTGGGGCA
AGTGGGTGGCGGAGATCCGGCTGCCGCGCAACCACCCCAGGCTCTGGCTCGGCACCTTCGACACCGCC
GAGGAGGCGGCGCTCACCTACGGCCAGGCCGCGTACCGCCTCCGCGGCGACG

FIGURE 29 (CONTINUED)

CGGCGCGGCTCAACTTCCCGGACAACGCCGCGTCGCGGGGCCCGCTCGACGCCGCCGTGGACGCCAAG
CTCCAGGCCATCTGCGACACCATCGCCGCGTCCAAGAACGCCTCATCCAGGTCCAGGGGCGGCGCCGG
CAGGGCCATGCCCATCAATGCGCCCCTGGTCGCCGCGGCGTCGTCGTCCTCCGGCTCCGACCACTCCG
GCGGCGGCGACGACGGCGGCTCGGAGACGTCGTCGTCGTCTGCGGCGGCGTCGCCGCTGGCGGAGATG
GAGCAGCTGGACTTCAGCGAGGTGCCGTGGGACGAGGCGGAGGGGTTCGCGCTCACCAAGTACCCGTC
GTACGAGATCGACTGGGACTCGCTGCTCAACAACAATAACTAGCTCTTCTCTCCATAG

## SEQ ID NO 304, Oryza sativa - protein

MAAAIEGNLMRALGEAPSPQMQKIAPPPFHPGLPPAPANFSSAGVHGFHYMGPAQLSPAQIQRVQAQL
HMQRQAQSGLGPRAQPMKPASAAAPAAAAARAQKLYRGVRQRHWGKWVAEIRLPRNHPRLWLGTFDTA
EEAALTYGQAAYRLRGDAARLNFPDNAASRGPLDAAVDAKLQAICDTIAASKNASSRSRGGAGRAMPI
NAPLVAAASSSSGSDHSGGGDDGGSETSSSSAAASPLAEMEQLDFSEVPWDEAEGFALTKYPSYEIDW
DSLLNNNN

## SEQ ID NO 305, Atriplex hortensis - DNA

AATTTACTTACCCCCAAGCCCAGCCCCAACAAATGAGCTACTCATACCCACCTCCACTTCCTAGTAAT
ACCCTTAACAACTTTCTATCTCCCAAGCCTGTGACCATGAAAACAACTGGTGGGCCGCCCAAGCCCAC
TAAGCTTTATCGTGGGGTTAGGCAACGTCACTGGGGTAAATGGGTTGCTGAGATCCGTTTACCCAAGA
ACAGGACCCGGCTTTGGTTGGGTACCTTTGATACAGCTGAGGAAGCTGCTTTGGCTTACGACAAGGCG
GCTTACAAGCTGAGAGGTGACTTTGCGAGGTTGAATTTTCCTAATCTCCGCCATGAAGGGTCCCACAT
CGGTGGCGAATTCGGCGAATACAAACCCCTTCATTCCTCCGTAAACGCAAAACTCGAAGCCATTTGCG
AGAGTCTAGCCAAACAGGGGAATGAAAAACAGGGGAAATCAGGAAAGTCCAAGAAGAAAGATGTTGCT
AATAATAACAATAATACTTCATCTTCGTCATCTTCAAGCTGTTGTACAACAGCTACTGCTGCGGCCGA
TGCACCGCAGCAGCAGCAGCGGATGCCGGAAAACGGCGGCGATGTAAAAACCGAGAGCACCTCCGATA
GTGAGGTGGGGTCCGGTGGATCGTCGCCGTTGTCGGATTTGACATTCGGAGATAATGAAGAAATGGGA
TCGGAGAATTTCTTGTTGGAGTCATGCCCATCTCATGAGATTGATTGGGATGCTATATTATCATCTGA
ATCTTAATAATTTCATTTGTGGTCTTAAGTATGGGTTAATAAGGAGTATTAATTAAATTAAATTAGGG
AGTTATAAAATGTGTCATCATAATATAAGTCATGTAATGTTGTGTAAATTTTTTCTTTTTTTTTTTTT
AATTTTAATTAAAATTAGTAGGTTGGTAGTGGGTAGTTTCAGGGAGGAGTGATCTCTGCAATGAAGTT
TTTGGAAATTGTAGGGACTCCATATTTGGTCTTCTTTGGTGAGGCAAGTGTTTTTTTGATCTTGCCTT
TGATCAATTGAAGAGTAGTTTTTTTTTCTTATTGTTTATTTTATGTTTATAGTAGAAAAAATTTTAGG
ATTGTAATTTGATGATCATTTTTAGTGAAGTATTATTATTAATATTTTTATTAGTATAAAAAAAAATT
GGACTGTTATCAAGGTAAGTGCTTATTTTGACTTCCATATTTGTATGTTGCTCTCTTTCTTTGATTCT
TTCATATTTCTAGTGAGATCTGAACTATAATTAATATGGATTAAACCTTAAATTACTAGTGGTTTGTG
TAACAGGATATGCTTGATGTTCCTGTTTTATACATAATCGGAGCTTTTGTCCCTGCAACAATGATTGC
AGTGCTCTATTATTTTGATCACAGTGTGGCATCTCAACTTGCTCAGCAGCGACAGCAACGGAATTCGG
CCCTCG

## SEQ ID NO 306, Atriplex hortensis - protein

MSYSYPPPLPSNTLNNFLSPKPVTMKTTGGPPKPTKLYRGVRQRHWGKWVAEIRLPKNRTRLWLGTFD
TAEEAALAYDKAAYKLRGDFARLNFPNLRHEGSHIGGEFGEYKPLHSSVNAKLEAICESLAKQGNEKQ
GKSGKSKKKDVANNNNNTSSSSSSSCCTTATAAADAPQQQQRMPENGGDVKTESTSDSEVGSGGSSPL
SDLTFGDNEEMGSENFLLESCPSHEIDWDAILSSES

FIGURE 29 (CONTINUED)

**SEQ ID NO 307, Arabidopsis thaliana - DNA**

AACAAGAGCCAAGCTAATGAGATAAGTGTATCGGTGAGGCTGAGAGTTATTCACTTACAAAAAAAAAA
AAAAACTTGAGTGTAACCAAAAAAAAAAAGTTGATATACTTTCTGGTTTTCTCCTTAACTTTTATTCTT
TACAAATCCATCCCCCTTAGATCTGTTTATTTCCCGCTACTTTGATTCATTTCTGTTAGTAATCTGTC
TTTCGTATAGAAGAAAACTGATTTCTTGGTTTGTATTTTCTTAAAGAGATCAATCTTTTTTTATTTTT
GATCTTCTTGTGTTTTTTTTTCTTTGTAGAATTAATCGTTTGTGAGGGTATTTTTTTAATTCCCTCCT
CTCAGAAATCTACACAGAGGTTTTTTATTTTATAAACCTCTTTTTCGATTTTCTTGAAAACAAAAAAT
CCTGTTCTTTACTTTTTTTACAAGAACAAGGGAAAAAAATTTCTTTTTATTAGAAATGACAACTTCTA
TGGATTTTTACAGTAACAAAACGTTTCAACAATCTGATCCATTCGGTGGTGAATTAATGGAAGCGCTT
TTACCTTTTATCAAAAGCCCTTCCAACGATTCATCCGCGTTTGCGTTCTCTCTACCCGCTCCAATTTC
ATACGGGTCGGATCTCCACTCATTTTCTCACCATCTTAGTCCTAAACCGGTCTCAATGAAACAAACCG
GTACTTCCGCGGCTAAACCGACGAAGCTATACAGAGGAGTGAGACAACGTCACTGGGGAAAATGGGTG
GCTGAGATTCGTTTACCGAGGAATCGAACTCGACTTTGGCTCGGAACATTCGACACGGCGGAGGAAGC
TGCTTTAGCTTATGACAAGGCGGCGTATAAGCTCCGAGGAGATTTTGCGCGGCTTAATTTCCCTGATC
TCCGTCATAACGACGAGTATCAACCTCTTCAATCATCAGTCGACGCTAAGCTTGAAGCTATTTGTCAA
AACTTAGCTGAGACGACGCAGAAACAGGTGAGATCAACGAAGAAGTCTTCTTCTCGGAAACGTTCATC
AACCGTCGCAGTGAAACTACCGGAGGAGGACTACTCTAGCGCCGGATCTTCGCCGCTGTTAACGGAGA
GTTATGGATCTGGTGGATCTTCTTCGCCGTTGTCGGAGCTGACGTTTGGTGATACGGAGGAGGAGATT
CAGCCGCCGTGGAACGAGAACGCGTTGGAGAAGTATCCGTCGTACGAGATCGATTGGGATTCGATTCT
TCAGTGTTCGAGTCTTGTAAATTAGATGTTGCCATAGGGGTATTTTAGGGACTTTAGAGCTCTCTGCG
ATGGAGTTTTTGGTCATTGCAGAGATTTTATTATTATTAAGGGGGGTTTGTTATGTTAATATCAAATAA
GTTTATCTACTTTGATGTTAATTAGTGTTAATCTCTGCGTCGGTCCAAGCTGTTTTTTTTTGGCATGC
TTCGACCGTGTGAGATTTCTTATGTAATTTTTGTAGTTCCTTGATTTTCTTAGTTCAAGTTAAATTGG
CACAAAAGAAC

**SEQ ID NO 308, Arabidopsis thaliana - protein**

MTTSMDFYSNKTFQQSDPFGGELMEALLPFIKSPSNDSSAFAFSLPAPISYGSDLHSFSHHLSPKPVS
MKQTGTSAAKPTKLYRGVRQRHWGKWVAEIRLPRNRTRLWLGTFDTAEEAALAYDKAAYKLRGDFARL
NFPDLRHNDEYQPLQSSVDAKLEAICQNLAETTQKQVRSTKKSSSRKRSSTVAVKLPEEDYSSAGSSP
LLTESYGSGGSSSPLSELTFGDTEEEIQPPWNENALEKYPSYEIDWDSILQCSSLVN

**SEQ ID NO 309, Arabidopsis thaliana - DNA**

ATAACGAGAGATTTTTTATATATAAAAAATAAAGGAAGCACGAGTTCACCTTAAAAATTTGAGTTTCT
CTTTTCTTGACCCTGAAGTTTCGTTTTGATCAATTTCACCCATTTCTTTGTTCGTCGTCTTTTCTTTC
AAAAGGGTTTCATCTTTGTATTATAAAAATCCAAACTTTATATCTTCACAAGCAGCAAAGATCTCTAT
TTCAATCCTCTCATGGAAACTGCTTCTCTTTCTTTCCCTGTCCCAAACACGAGCTTCGGTGTAAACAA
ATCTATGCCTCTCGGTCTAAACCAGCTCACACCATACCAAATTCATCAGATTCAGAACCAGCTTAACC
ATAGACGTAGCACAATCTCAAACCTCTCTCCAAACCGTATCAGAATGAAGAACTTAACACCTTCTACC
TCCAAAACCAAGAATCTCTACAGAGGCGTAAGGCAAAGGCACTGGGGGAAATGGGTCGCTGAGATCCG
TTTGCCCAAGAACCGGACCCGTCTCTGGCTCGGGACATTCGAAACCGCCGAAAAAGCCGCCTTAGCTT
ACGACCAAGCTGCTTTTCAGCTCCGTGGAGATATCGCGAAGCTTAACTTCCCAAACCTCATACACGAA
GACATGAATCCTCTCCCTTCCTCTGTTGATACCAAGCTTCAAGCTATCTGCAAAAGTTTGAGAAAAAC
AGAGGAAATTTGCTCTGTTTCTGATCAAACAAAGGAGTACTCTGTTTACTCTGTTTCAGATAAAACAG
AGCTTTTCCTGCCAAAAGCAGAGCTTTTCTTGCCTAAAAGAGAGCATTTGGAGACAAATGAGCTCTCT
AATGAGTCACCGAGAAGCGATGAGACCTCGTTGTTGGATGAG

FIGURE 29 (CONTINUED)

TCGCAGGCGGAATATTCATCGTCGGATAAAACATTCCTGGATTTCTCGGATACTGAGTTTGAAGAGAT
TGGAAGTTTCGGGCTTCGGAAGTTTCCTTCGGTGGAGATTGATTGGGATGCAATTAGTAAACTGGCCA
ATTCTTAAAGTCTTCTATTTTATATTAATCTTCTGTGCAACTGAAATTTTCAACAAGTTGCAGATGAG
TTTATTAGGTTTGAATCTGTCCTAGTTTTCTTTGTTGGTCGTATAGAGTTTTACTATGTAGCTAATCT
CCATGTTTGGTTTGGCTTGGTAAGTTTTTAAGGTCCTCGTCATCAACAAACCGGAGAAAGTTTATGTA
TTCAAAAGCTATGTATTATATCCTCACATCTACTTATGAGAGTGAGATCAAAGTTTGTAAAATGAAGT
CTTGATTTCTTCTTTTAGTTCTCTCTTTTT

## SEQ ID NO 310, Arabidopsis thaliana - protein

METASLSFPVPNTSFGVNKSMPLGLNQLTPYQIHQIQNQLNHRRSTISNLSPNRIRMKNLTPSTSKTK
NLYRGVRQRHWGKWVAEIRLPKNRTRLWLGTFETAEKAALAYDQAAFQLRGDIAKLNFPNLIHEDMNP
LPSSVDTKLQAICKSLRKTEEICSVSDQTKEYSVYSVSDKTELFLPKAELFLPKREHLETNELSNESP
RSDETSLLDESQAEYSSSDKTFLDFSDTEFEEIGSFGLRKFPSVEIDWDAISKLANS

## SEQ ID NO 311, Glycine max – DNA

ATAACGAGAGATTTTTTATATATAAAAAATAAAGGAAGCACGAGTTCACCTTAAAAATTTGAGTTTCT
CTTTTCTTGACCCTGAAGTTTCGTTTTGATCAATTTCACCCATTTCTTTGTTCGTCGTCTTTTCTTTC
AAAAGGGTTTCATCTTTGTATTATAAAAATCCAAACTTTATATCTTCACAAGCAGCAAAGATCTCTAT
TTCAATCCTCTCATGGAAACTGCTTCTCTTTCTTTCCCTGTCCCAAACACGAGCTTCGGTGTAAACAA
ATCTATGCCTCTCGGTCTAAACCAGCTCACACCATACCAAATTCATCAGATTCAGAACCAGCTTAACC
ATAGACGTAGCACAATCTCAAACCTCTCTCCAAACCGTATCAGAATGAAGAACTTAACACCTTCTACC
TCCAAAACCAAGAATCTCTACAGAGGCGTAAGGCAAAGGCACTGGGGGAAATGGGTCGCTGAGATCCG
TTTGCCCAAGAACCGGACCCGTCTCTGGCTCGGGACATTCGAAACCGCCGAAAAAGCCGCCTTAGCTT
ACGACCAAGCTGCTTTTCAGCTCCGTGGAGATATCGCGAAGCTTAACTTGCCAAACCTCATACACGAA
GACATGAATCCTCTCCCTTCCTCTGTTGATACCAAGCTTCAAGCTATCTGCAAAAGTTTGAGAAAAAC
AGAGGAAATTTGCTCTGTTTCTGATCAAACAAAGGAGTACTCTGTTTACTCTGTTTCAGATAAAACAG
AGCTTTTCCTGCCAAAAGCAGAGCTTTTCTTGCCTAAAAGAGAGCATTTGGAGACAAATGAGCTCTCT
AATGAGTCACCGAGAAGCGATGAGACCTCGTTGTTGGATGAGTCGCAGGCGGAATATTCATCGTCGGA
TAAAACATTCCTGGATTTCTCGGATACTGAGTTTGAAGAGATTGGAAGTTTCGGGCTTCGGAAGTTTC
CTTCGGTGGAGATTGATTGGGATGCAATTAGTAAACTGGCCAATTCTTAAAGTCTTCTATTTTATATT
AATCTTCTGTGCAACTGAAATTTTCAACAAGTTGCAGATGAGTTTATTAGGTTTGAATCTGTCCTAGT
TTTCTTGTTGGTCGTATAGAGTTTTACTATGTAGCTAATCTCCATGTTTGGTTTGGCTTGGTAAGTTT
TTAAGGTCCTCGTCATCAACAAACCGGAGAAAGTTTATGTATTCAAAAGCTATGTATTATATCCTCAC
ATCTACTTATGAGAGTGAGATCAAAGTTTGTAAAATGAAGTCTTGATTTCTTCTTTTAGTTCTCTCTT
TTTC

## SEQ ID NO 312, Glycine max - protein

MAALMDFYSSSTEFQLHSDPFRGELMEVLEPFMKSPFSTPSPSNSCFLSTSYSPSPNNYSPSLYSNGL
SSIPNTTQNLIGFGQGQPTSLVGLNHLTPSQISQIQAQIQIQNHSNTLSFLGPKPIPMKHVGMPPKPT
KLYRGVRQRHWGKWVAEIRLPKNRTRLWLGTFDTAEEAALAYDKAAYKLRGDFARLNFPNLRHQGSSV
GGDFGEYKPLHSAVDAKLQAICEGLAELQKQGKTEKPPRKTRSKLASPPENDNNNDNNSCKVEAAPPL

FIGURE 29 (CONTINUED)

**SEQ ID NO 313, Arabidopsis thaliana - DNA**

GTACATTTTTTTTTGTATTTCAGGAAACTCCGATGGCGGATCTCTTCGGTGGTGGCCACGGCGGCGAG
CTTATGGAAGCACTTCAACCTTTTTACAAAAGTGCTTCCACGTCTGCTTCAAATCCTGCGTTTGCGTC
CTCAAACGATGCGTTTGCGTCTGCCCCAAACGACCTATTTCTTCTTCTTCTTACTATAATCCTCATG
CATCTTTATTCCCTTCACATTCCACAACCTCTTACCCGGATATTTATTCTGGATCCATGACCTATCCA
TCTTCATTCGGGTCGGATCTTCAACAACCCGAAAACTACCAATCTCAGTTCCATTACCAAAACACTAT
CACTTACACTCACCAAGACAACAACACTTGCATGCTTAACTTCATTGAGCCGAGCCAACCGGGTTTTA
TGACCCAACCGGGTCCGAGTTCGGGTTCGGTTTCAAAACCGGCTAAGCTCTATAGAGGAGTGAGGCAA
AGACATTGGGGAAAATGGGTCGCGGAGATCCGTTTACCCAGGAACCGAACCCGACTTTGGCTCGGAAC
ATTCGACACGGCTGAAGAAGCCGCGTTGGCTTATGATCGCGCCGCGTTTAAGCTTCGTGGTGACTCGG
CTCGGCTTAACTTCCCAGCTCTCCGATACCAAACCGGCTCGTCTCCGTCTGATACCGGCGAATATGGT
CCTATTCAAGCTGCCGTAGACGCTAAACTAGAAGCCATATTAGCTGAGCCGAAGAATCAGCCGGGCAA
AACGGAGAGGACGTCGAGGAAACGAGCTAAAGCCGCGGCTTCTTCAGCTGAGCAGCCGTCAGCGCCAC
AACAACATTCCGGGTCGGGTGAAAGTGATGGGTCGGGTTCACCGACTTCGGATGTTATGGTGCAGGAG
ATGTGCCAAGAGCCAGAGATGCCATGGAATGAAAATTTCATGCTCGGCAAGTGTCCTTCTTATGAGAT
AGATTGGGCTTCAATTTTATCGTGA

**SEQ ID NO 314, Arabidopsis thaliana - protein**

MADLFGGGHGGELMEALQPFYKSASTSASNPAFASSNDAFASAPNDLFSSSSYYNPHASLFPSHSTTS
YPDIYSGSMTYPSSFGSDLQQPENYQSQFHYQNTITYTHQDNNTCMLNFIEPSQPGFMTQPGPSSGSV
SKPAKLYRGVRQRHWGKWVAEIRLPRNRTRLWLGTFDTAEEAALAYDRAAFKLRGDSARLNFPALRYQ
TGSSPSDTGEYGPIQAAVDAKLEAILAEPKNQPGKTERTSRKRAKAAASSAEQPSAPQQHSGSGESDG
SGSPTSDVMVQEMCQEPEMPWNENFMLGKCPSYEIDWASILS

**SEQ ID NO 315, Arabidopsis thaliana - DNA**

AACAAATCTCTCTGTTTCTCCCGCTCTTGCTCTGTTTTCTCAAAGACAAAAGAGGACATCGTCGTTGA
CTCCTCTTCTCTATGGCTACTGCTAAGAACAAGGGAAAATCAATCAGGGTCCTTGGTACCAGTGAAGC
AGAGAAAAAGGATGAGATGGAGTTGGAGGAGGAGTTCCAGTTTAGTAGCGGCAAGTATAAAGATTCGG
GTCCTGGCTCGGACATGTGGTTAGGAGATGCTTCCTCTACGTCTCCAAGAAGTCTTAGGAAGACTAGA
ACCTTTGACCGACATAATCCCTATCTCGTATCTTCTTATGCTACTCCTCAGCCGCCAACAACAACTAC
ATGCTCTGTCTCTTTTCCCTTTTACCTCCCTCCAGCGATTCAAAATCAACAACGATTTTTACACCCGA
ATGACCCTTCAGGACAAAGACAGCAACAAATGATCTCGTTTGATCCTCAACAACAGGTGCAACCATAT
GTTGCACAACAGCAGCAACAACAACAACATCTATTGCAGTACTGGAGAGACATTCTGAAGCTGAGTCC
GAGCGGAAGAATGATGATGATGAACATGTTAAGACAAGAAAGCGATCTGCCACTGACGAGGCCACCGG
TTCAACCCTTCAGCGCCACCAAGCTATATAGAGGTGTCAGGCAACGCCACTGGGGAAAATGGGTTGCC
GAGATCCGTAAGCCACGAAACAGGACACGTCTCTGGCTAGGGACATTCGATACAGCAGAAGAAGCCGC
CATGGCCTACGACCGCGAGGCCTTCAAGTTGAGGGGAGAGACCGCTAGGCTCAATTTCCCTGAACTTT
TTCTCAATAAACAAGAGCCAACTCCCGTGCATCAGAAACAATGTGAGACGGGGACTACTAGTGAAGAC
TCAAGCAGAAGAGGAGAGGATGATTCGAGCACGGCATTGGCAGTAGGAGGGGTGAGTGAGGAGACGGG
TTGGGCTGAGGCATGGTTCAATGCAATTCCAGAGGAATGGGGACCTGGAAGCCCTCTATGGGATGATT
ACCACTTTCCCATTTCTAACCATAAGGACGATCTTGACGCCACACAAAACTCTTCTTCTGATACAATT
TAGGACCTTTGTTAGAATATAGATATGCTTAGTTGTATGACTGATCTAGCTTGTGTTTTTTTTTTGGG
TGGAGACAGTTTTTGTCATCTTCCACATTTTAGATTCTATTTTCGACCATCATTTTTTTCTTGATCGG
TGACTATGAATCTAATGGGGTCAATCATTTTCACATATAAAACTTAAGTATTTGGTGTTTGTACTTC

FIGURE 29 (CONTINUED)

**SEQ ID NO 316, Arabidopsis thaliana - protein**

MATAKNKGKSIRVLGTSEAEKKDEMELEEEFQFSSGKYKDSGPGSDMWLGDASSTSPRSLRKTRTFDR
HNPYLVSSYATPQPPTTTTCSVSFPFYLPPAIQNQQRFLHPNDPSGQRQQQMISFDPQQQVQPYVAQQ
QQQQQHLLQYWRDILKLSPSGRMMMMNMLRQESDLPLTRPPVQPFSATKLYRGVRQRHWGKWVAEIRK
PRNRTRLWLGTFDTAEEAAMAYDREAFKLRGETARLNFPELFLNKQEPTPVHQKQCETGTTSEDSSRR
GEDDSSTALAVGGVSEETGWAEAWFNAIPEEWGPGSPLWDDYHFPISNHKDDLDATQNSSSDTI

**SEQ ID NO 317, Arabidopsis thaliana - DNA**

ATGATCACACCAATACACACACAACACTCCTTAATTCTCGTATATATAAACATTTATTCTCCACCTAT
TTTGTCAAAATTAAGAACAGGCTTCATTCTCTGGACAAACACTCAAAAAACAAACAAAAAAAGGAACA
TGGAAGATCAGTTTCCTAAAATAGAAACTAGCTTCATGCACGACAAGCTCTTGTCTTCTGGAATCTAC
GGGTTCTTGAGTTCTTCGACGCCGCCACAACTTCTCGGTGTTCCAATATTTTTGGAAGGTATGAAATC
TCCTCTTCTTCCTGCTTCTTCGACTCCGAGCTACTTTGTGTCGCCTCATGATCATGAGCTCACATCTT
CTATTCATCCATCTCCGGTAGCTTCTGTTCCTTGGAACTTTCTAGAATCTTTTCCTCAGTCTCAACAT
CCTGATCATCATCCTTCTAAACCTCCAAACCTTACTTTGTTCCTTAAAGAACCAAAGCTACTAGAACT
TTCTCAATCCGAAAGCAACATGAGCCCTTACCATAAATACATCCCAAACTCCTTTTATCAATCAGACC
AAAACAGAAACGAATGGGTAGAGATCAATAAAACTCTAACCAACTATCCCTCGAAAGGTTTTGGAAAC
TATTGGCTAAGTACCACCAAGACTCAACCCATGAAGTCAAAAACAAGAAAGGTTGTTCAGACGACGAC
CCCAACAAAACTGTATAGAGGAGTGAGACAAAGACACTGGGGCAAATGGGTCGCAGAGATTAGGCTTC
CAAGGAACAGAACCCGTGTTTGGCTCGGCACTTTTGAAACCGCTGAGCAAGCAGCAATGGCTTACGAT
ACAGCAGCTTATATCCTTCGTGGCGAATTCGCACACCTCAACTTTCCTGATCTTAAACACCAGCTCAA
GTCCGGTTCTTTGCGATGCATGATCGCCTCACTTTTGGAGTCCAAGATTCAACAGATCTCATCTTCCC
AAGTAAGTAACTCTCCTTCTCCTCCTCCTCCAAAAGTGGGAACACCGGAGCAAAAGAATCATCACATG
AAGATGGAGTCAGGAGAAGACGTGATGATGAAGAAACAGAAAAGCCATAAGGAAGTGATGGAAGGAGA
TGGTGTACAATTGAGTAGGATGCCTTCTTTGGATATGGATCTCATTTGGGATGCTCTCTCATTTCCTC
ATTCTTCTTGACTTCAAATTAATATTTGTCAAACTTATTTTACTTACTTCTACCCTTTTTTATATCAA
AAGTTTCCACCAAAGAAAGAAATTCATATTATGATGCCAAGATTGGTTTGCATTTGGGGTTGAACACA
TTGTAATTCTTCTTACGACCACATAATCAAGTGGTTCTCCTTTTTTTGTCTGCTAATTAATTGTTCTT
TTCTTTGCGGTTACTAGTAACCATAAAATAGAAAAGTGTTTGTTT

**SEQ ID NO 318, Arabidopsis thaliana - protein**

MITPIHTQHSLILVYINIYSPPILSKLRTGFILWTNTQKTNKKRNMEDQFPKIETSFMHDKLLSSGIY
GFLSSSTPPQLLGVPIFLEGMKSPLLPASSTPSYFVSPHDHELTSSIHPSPVASVPWNFLESFPQSQH
PDHHPSKPPNLTLFLKEPKLLELSQSESNMSPYHKYIPNSFYQSDQNRNEWVEINKTLTNYPSKGFGN
YWLSTTKTQPMKSKTRKVVQTTTPTKLYRGVRQRHWGKWVAEIRLPRNRTRVWLGTFETAEQAAMAYD
TAAYILRGEFAHLNFPDLKHQLKSGSLRCMIASLLESKIQQISSSQVSNSPSPPPPKVGTPEQKNHHM
KMESGEDVMMKKQKSHKEVMEGDGVQLSRMPSLDMDLIWDALSFPHSS

**SEQ ID NO 319, Oryza sativa - DNA**

ATGGACGCGGTGGACAGAGGCGGAGGCGGAGGCGGTGGAGGAGCGCGCGGGCACGGGCGGAGATGGAA
GGGGAAGGGAGTGAGCGCGGCGATCTCCTCCTCCGCGGCCGAGACGCAGCAGCCGGTGCCAGTTTTAG
AAGACGCGCCGGCGGCCGCAGCATTGCTCCGTCCCCAGAAGAAGATCCGCAGCCCCGATCGT

FIGURE 29 (CONTINUED)

CGCCTCCAGCGCTCCATCTCCTCGCTGTCGTCGGCCCCTGCTTCCCCCGACTCGTCCTCTGTCTCCAA
CCCCATGTCTCCCCCGGCGATGTCCTTGCCGAATCAGCCGCCATCGTCGCGACATATATTTCCGTTCG
CGTACGATCCGTCTCCGGGCGCGGCGGCACCAAGGCTCCTCCCGCTGCTGCAGTACTCCAGCTTGTAC
CCGCAGCCTCTGCTGCCGCAGCAGCAATCACCCTTGCAGAATCAGCAAATGATATCGTTCGGCAGTAG
CCAGCAGCAGCAGCAGCAGCAGCCGCAGTTTGGGGCGGCGTCTCCTCTGTTCCCGCCGCAGTTCTTGC
CGCCGGAGGAGCAGCAGCGCCTGCTGCTGCGCTACTGGAGCGAGGCGCTGAACCTGAGCCCCCCAGGC
GTGCGCGGCGGCGCCCTGCCGCCGTCGCTGTACCAGCACCTGCTGCGCGCGCCTGGGCCGCCCAAACT
GTACCGCGGCGTGCGGCAGCGCCACTGGGGGAAGTGGGTGGCGGAGATCCGCCTGCCGCGGAACCGCA
CCAGGCTGTGGCTCGGCACGTTCGACACCGCCGAGGACGCAGCCATGGCGTACGACCGCGAGGCCTTC
AAGCTCCGCGGCGAGAACGCGCGGCTCAACTTCCCCGACCTCTTCCTTGGCAAAGGCCGCACCGGCGG
GAGCGGACGCACCAGCGCCAGCGCCGCGGCCTCCTGCTCCTCCTCCTCCTCTTCGGCTCCGCCTACAC
CGGACGAGAGCCACACGCAGCAAGCTCAGCCGCAGCCGCAGCAGCCTACAGAAGAGTCATCCAACACT
GAACCGAAGCCTCTGCTCTTCGTAGCAGAGCAGGACGGCATTCCAGAACCCGAGCTGAATCCTCAGCT
CCAGACAGCTGAACAACATGGCAGCGACGGCAACACGGCCATGTTCCAGCCGTCGGTGACGTCCGGCG
GCATTTGGGGTCCGGCCGACGAGGCGTGGTTCAGCGCTTGGGGGCCAGGAAGCTCCGTCTGGGACTAC
GACATGGACAGCGCCCACGGCCTCCTCCTCCAGTCTCGCTTGGCCGGTGAGCAGACCGGCATGGACTA
CGCCTACACCGCGCCGGAAGTCCTCGTGGCACCGGTGCCGGCGGCAGGGACAGCCATGGCCACTGCCG
CTTCCTCCTCTCTTCCTCCTCGTCCTCCCCCTCCTTGCCACAGTCCAACCTTCGCATGGAAGGACTAA

**SEQ ID NO 320, Oryza sativa - protein**

MDAVDRGGGGGGGGARGHGRRWKGKGVSAAISSSAAETQQPVPVLEDAPAAAALLRPQKKIRSPDRRL
QRSISSLSSAPASPDSSSVSNPMSPPAMSLPNQPPSSRHIFPFAYDPSPGAAAPRLLPLLQYSSLYPQ
PLLPQQQSPLQNQQMISFGSSQQQQQQPQFGAASPLFPPQFLPPEEQQRLLLRYWSEALNLSPPGVR
GGALPPSLYQHLLRAPGPPKLYRGVRQRHWGKWVAEIRLPRNRTRLWLGTFDTAEDAAMAYDREAFKL
RGENARLNFPDLFLGKGRTGGSGRTSASAAASCSSSSSSAPPTPDESHTQQAQPQPQQPTEESSNTEP
KPLLFVAEQDGIPEPELNPQLQTAEQHGSDGNTAMFQPSVTSGGIWGPADEAWFSAWGPGSSVWDYDM
DSAHGLLLQSRLAGEQTGMDYAYTAPEVLVAPVPAAGTAMATAASSSLPPRPPPPCHSPTFAWKD

**SEQ ID NO 321, Oryza sativa - DNA**

ACGTTTGGCTGCACCCTGTCTCGCGGCTGATACGTTTGCACAGGCGTTCAGATACAGCCGATACACCG
GCGGCGCTAGCTGCGTTTCTTCTCCGCATGGACGCTCCGTCGTCGCCCTCGCCACCGTCGGCGCGACA
CATCTTCCCCTTCGCGTACGAGGCCTCCACGACGACGGTGGGTGGGAGCCCAAGGCTCCACCCGCTGT
CGTGGCAGCAATCCAGCATGTCCCAGCCCGCGTCGCCACAGCAACAGCAGCAGCAGCCGTTGCAGCAC
CAGCAGATGATATCGTTCGGCGCGTCGCCTCCGTGCTCGACGACGCAGTTCGTCGTCCCGGAGAACGC
GCAGCAGCAGCAGATGCTGCTGCGGTACTGGAGCGAGGCGCTGAACCTGAGCCCGCGCGGTGGCCCCG
GTGGCGTGCCACCGTGGCTGTACCAGCAGCTGCTCCGGGTCCCGCCACCGCCGCAGAAGCTTTACCGC
GGCGTGCGGCAGAGGCACTGGGGGAAGTGGGTCGCGGAGATCCGCCTGCCACGGAACCGCACGCGGCT
GTGGCTTGGCACCTTCGACACCGCCGAGGACGCCGCCATGGCGTACGACCGCGAGGCCTTCAAGCTCC
GCGGCGAGAACGCGCGGCTCAACTTTCCGGACCGGTTCCTTGGGAAGGGCCGCGCCGGCGGGAAAGGC
CGCACCAGCGTAAGCTCCTCGGCCGCTGCCGCCGCGTCGTGCTCGTCGTCGTCGCTATCGCCACCGGA
GACACCTGACGACGCAAACACGCAGCAACAAGCTCCTCAGCAGCGAGAACAGCGGGACACGGCAGGAG
TGTCCATGGAGAAGAAACAACCACAGCCTCCAGCTCCAACCTCTCGTCAAGAAGGGTGCTCTGGCGGC
GACGCAGCTGCGCCGTACCCGGCCGAAATGCTTCACGCGCCGGCGGCGTGCGGCGGCATGTGGGTTGC
TCCCGACGAGTCATGGTTCAGCACGTGGGGCCCTGGCA

FIGURE 29 (CONTINUED)

GCTCCTTCTGGGACGACTACGACATGGACAGCGCTCGCGGCCTCTTCCTCCACCCTCGCTTCACCGGT
GACGAAACTAGCATGGATCATTCCGGCACGCAAGCAACCGTGCCGGCAGTGGCAGCAACAGCGGCAGG
GATGAGCATGCCATGCGATGATGTTCCGGTAACCTCTTCTTCTTCAGATCTCCCTCCCCAAGGGACAC
CTCAGACTCCTACCTTCATGTGGAAGGAGGACTAAGCATGCATGCACACAGCCACGCACCTCGACGAG
ACGACTATCTCTTCCATCTCCCATCGACTTCGACGATCTCTTTCATTACTCATCGACTTCAACCACAA
AGGTATGATTAGGGTAGAGATGCTTGCAGATTGCAGTTTTAAAGACAATTTTGCAGCATCACACAGCT
CTACGTACAACAGCCACACCTATATCAATTCTCAAGACTTCTAAACCACAAGGGAACCCCTGTTCTAG
GCTGCGATTTTTAGGTCAGCTTGTTGGGGTGACCACAGTACAATTCCCTTTATTTCGTATCTTCCCAA
GATTGTGTGTGTCAGATATTCGTTGAACTCGACTTCAAAAGATTTGAAGTATTCATGTTTTCTTTTAC
TC

**SEQ ID NO 322, Oryza sativa - protein**

MDAPSSPSPPSARHIFPFAYEASTTTVGGSPRLHPLSWQQSSMSQPASPQQQQQQPLQHQQMISFGAS
PPCSTTQFVVPENAQQQQMLLRYWSEALNLSPRGGPGGVPPWLYQQLLRVPPPPQKLYRGVRQRHWGK
WVAEIRLPRNRTRLWLGTFDTAEDAAMAYDREAFKLRGENARLNFPDRFLGKGRAGGKGRTSVSSSAA
AAASCSSSSLSPPETPDDANTQQQAPQQREQRDTAGVSMEKKQPQPPAPTSRQEGCSGGDAAAPYPAE
MLHAPAACGGMWVAPDESWFSTWGPGSSFWDDYDMDSARGLFLHPRFTGDETSMDHSGTQATVPAVAA
TAAGMSMPCDDVPVTSSSSDLPPQGTPQTPTFMWKED

**SEQ ID NO 323, Oryza sativa - DNA**

ATGGACGCTCCGAGCGGCGAGAGCGGCGGCGGCGGCGGCGGCGGTGGTGGCAGGAGGTGGAAGGGGAA
GGGGGTGACGCCCATACAGCCGCGGCGGCAGCTGGGGACGGTTTTGGAGGACTCGTCGGCGGCATTGC
TGCGGCCGCTCAAGAAGATTGGGCGGAGCCCCGACCGCCTCCTCCGCTCCGCATCGTCGCTCTCCACG
TCCTCGTCGGCTCCGCCTTCGCCTCGCTCTTCTTCGGCTTCCGATGCTCCAGTCCGCGTCATCTCGTC
GTCGCCGTCGTCGCCCTCGCCACCGTCGGCGCGACACATCTTCCCCTTCGCGTACGAGGCCTCCACGA
CGACGGTGGGTGGGAGCCCAAGGCTCCACCCGCTGTCGTGGCAGCAATCCAGCATGTCCCAGCCCGCG
TCGCCACAGCAACAGCAGCAGCAGCCGTTGCAGCACCAGCAGATGATATCGTTCGGCGCGTCGCCTCC
GTGCTCGACGACGCAGTTCGTCGTCCCGGAGAACGCGCAGCAGCAGCAGATGCTGCTGCGGTACTGGA
GCGAGGCGCTGAACCTGAGCCCGCGCGGTGGCCCCGGTGGCGTGCCACCGTGGCTGTACCAGCAGCTG
CTCCGGGTCCCGCCACCGCCGCAGAAGCTTTACCGCGGCGTGCGGCAGAGGCACTGGGGGAAGTGGGT
CGCGGAGATCCGCCTGCCACGGAACCGCACGCGGCTGTGGCTTGGCACCTTCGACACCGCCGAGGACG
CCGCCATGGCGTACGACCGCGAGGCCTTCAAGCTCCGCGGCGAGAACGCGCGGCTCAACTTTCCGGAC
CGGTTCCTTGGGAAGGGCCGCGCCGGCGGGAAAGGCCGCACCAGCGTAAGCTCCTCGGCCGCTGCCGC
CGCGTCGTGCTCGTCGTCGTCGCTATCGCCACCGGAGACACCTGACGACGCAAACACGCAGCAACAAG
CTCCTCAGCAGCGAGAACAGCGGGACACGGCAGGAGTGTCCATGGAGAAGAAACAACCACAGCCTCCA
GCTCCAACCTCTCGTCAAGAAGGGTGCTCTGGCGGCGACGCAGCTGCGCCGTACCCGGCCGAAATGCT
TCACGCGCCGGCGGCGTGCGGCGGCATGTGGGTTGCTCCCGACGAGTCATGGTTCAGCACGTGGGGCC
CTGGCAGCTCCTTCTGGGACGACTACGACATGGACAGCGCTCGCGGCCTCTTCCTCCACCCTCGCTTC
ACCGGTGACGAAACTAGCATGGATCATTCCGGCACGCAAGCAACCGTGCCGGCAGTGGCAGCAACAGC
GGCAGGGATGAGCATGCCATGCGATGATGTTCCGGTAACCTCTTCTTCTTCAGATCTCCCTCCCCAAG
GGACACCTCAGACTCCTACCTTCATGTGGAAGGAGGACTAA

FIGURE 29 (CONTINUED)

**SEQ ID NO 324, Oryza sativa - protein**

MDAPSGESGGGGGGGGGRRWKGKGVTPIQPRRQLGTVLEDSSAALLRPLKKIGRSPDRLLRSASSLST
SSSAPPSPRSSSASDAPVRVISSSPSSPSPPSARHIFPFAYEASTTTVGGSPRLHPLSWQQSSMSQPA
SPQQQQQQPLQHQQMISFGASPPCSTTQFVVPENAQQQQMLLRYWSEALNLSPRGGPGGVPPWLYQQL
LRVPPPPQKLYRGVRQRHWGKWVAEIRLPRNRTRLWLGTFDTAEDAAMAYDREAFKLRGENARLNFPD
RFLGKGRAGGKGRTSVSSSAAAAASCSSSSLSPPETPDDANTQQQAPQQREQRDTAGVSMEKKQPQPP
APTSRQEGCSGGDAAAPYPAEMLHAPAACGGMWVAPDESWFSTWGPGSSFWDDYDMDSARGLFLHPRF
TGDETSMDHSGTQATVPAVAATAAGMSMPCDDVPVTSSSSDLPPQGTPQTPTFMWKED

**SEQ ID NO 325, Arabidopsis thaliana - DNA**

ATTAAATAGATCTTTAAACAAAAAAACCAAATTCTACTTTTCTTTCAAAAACAATCTCTCATTATCTC
AATCTTACTCTTGTTCAATCTCTTTTGAACTTGAAAAACCATCATTCTATCAAATCAAACACAATTGT
TAGGGTTTCTTTTTATCTTATACTCCACGAAACGTCTGGTTGAGAAAAAATGGAAGAAAGCAATGATA
TTTTTCAGAACAATTTCAGTCCTAAAATCTCAGAAATCAGAGCATCTCTGTCTCAGATCATATTAGCA
GGAGGACCAAACACACTCGACTCAATCTTCTCACTTCTCACTCCCTCCTCCGTAGAATCCGCCACAAC
ATCATTCAACACTCACAATCCTCCGCCACCGCCGCAGCTCGGGTCCTCCGTCTATCTCCGCCAACGAG
ATATCATCGAGAAGTTCCACCTTCAGAACAGAGCAATCTCCACTCCTCATCCTCCTCTGTTTTCCTCA
ACGTACGATCATCATCAAACTTCCGAGCTAATGCTCCAAGCGGCGGCCGGGTCTCCAGCCGCCGCTTT
CGCCGCTGCGTTAGCGGCTGGGAGGGTGACGAAAAAGAAGAAACTTTACAGAGGAGTGAGGCAGAGAC
ATTGGGGAAAATGGGTTGCGGAGATAAGATTGCCGCAAAACAGAATGAGGGTTTGGTTAGGTACTTAC
GACACGGCGGAAGCCGCCGCTTACGCTTACGATCGCGCCGCCTATAAGCTCCGTGGAGAATACGCTCG
TCTCAATTTTCCTAATCTCAAAGACCCGAGTGAGCTACTCGGACTCGGAGATTCCTCTAAGCTAATAG
CTCTCAAGAACGCCGTCGACGGGAAGATCCAATCCATTTGCCAGAGAGTCAGAAAAGAGAGAGCAAAA
AAGAGCGTAAAAGTGAGCAAAAACTCGTCGGCCACGGCGGATTCTTCGTGTTTGTCATCGCCGGAGAT
TCTGTCGTCGTCTCCGGTGACGACAACTACTACTGCGGTTACTTCAGAGGATAGTTATTGGGTTTCAC
CTATGGGTTTGTGTAACAGTGAAAATAGTTCTCCGGTATCAGTTTCGGTCCCTAGTGAAGTACCGGCG
ACGGCGGAGGAAGAGGCAATGATGGGAGTGGACACTGATGGGTTTTTATTAGCGAGGATGCCATCGTT
CGATCCAGAGCTGATTTGGGAAGTTCTTGCCAATTAATACTACACAAAGAATGTGAATTTTGATCAAA
ATTGCAACAAGAAGAAGAAAAAAAAAAAGTTTGGAATTAGGACAAAAATGATGAGAAAAATTAGGGTTT
TAGGTGTTAATTTAGGGTTTAAAGTAAAACCGGATCTTTTGTTAAAATCATTCGAAAGTTGTTAAATA
ATATAATTAGGGTTTCTCAAATACAATGTAAAGCCTCGTGGTTTTTGAAAGAGGTTGTGAAAGCTCCT
TTTTTTGAATTTCTGTTTTAGATCATCGAAGGACTTTCTTTCTGATTGTACTTAGAAGAACAATTATA
TGTTACAGTGTTAATTTAATAAAATTGAGATT

**SEQ ID NO 326, Arabidopsis thaliana - protein**

MEESNDIFQNNFSPKISEIRASLSQIILAGGPNTLDSIFSLLTPSSVESATTSFNTHNPPPPPQLGSS
VYLRQRDIIEKFHLQNRAISTPHPPLFSSTYDHHQTSELMLQAAAGSPAAAFAAALAAGRVTKKKKLY
RGVRQRHWGKWVAEIRLPQNRMRVWLGTYDTAEAAAYAYDRAAYKLRGEYARLNFPNLKDPSELLGLG
DSSKLIALKNAVDGKIQSICQRVRKERAKKSVKVSKNSSATADSSCLSSPEILSSSPVTTTTTAVTSE
DSYWVSPMGLCNSENSSPVSVSVPSEVPATAEEEAMMGVDTDGFLLARMPSFDPELIWEVLAN

FIGURE 29 (CONTINUED)

**SEQ ID NO 327, Oryza sativa - DNA**

ATGGCAGCTGCTATAGAAGGAAATCTGATGCGGGCGCTGGGAGAGGCTCCGTCGCCGCAGATGCAGAA
GATCGCGCCGCCGCCGTTTCATCCCGGCTTGCCGCCGGCGCCGGCGAACTTCTCCTCGGCCGGAGTCC
ACGGGTTCCACTACATGGGCCCGGCCCAGCTCAGCCCGGCCCAGATCCAGCGCGTCCAGGCCCAACTC
CACATGCAGCGGCAGGCCCAGTCGGGGCTCGGCCCGCGGGCCCAGCCCATGAAGCCCGCTTCGGCGGC
TGCTCCGGCGGCGGCGGCGGCGCGGGCGCAGAAGCTGTACCGCGGCGTGCGGCAGCGGCACTGGGGCA
AGTGGGTGGCGGAGATCCGGCTGCCGCGCAACCGCACCAGGCTCTGGCTCGGCACCTTCGACACCGCC
GAGGAGGCGGCGCTCACCTACGACCAGGCCGCGTACCGCCTCCGCGGCGACGCGGCGCGGCTCAACTT
CCCGGACAACGCCGCGTCGCGGGGCCCGCTCGACGCCGCCGTGGACGCCAAGCTCCAGGCCATCTGCG
ACACCATCGCCGCGTCCAAGAACGCCTCATCCAGGTCCAGGGGCGGCGCCGGCAGGGCCATGCCCATC
AACGCGCCCCTGGTCGCCGCGGCGTCGTCGTCCTCCGGCTCCGACCACTCCGGCGGCGGCGACGACGG
CGGCTCGGAGACGTCGTCGTCGTCTGCGGCGGCGTCGCCGCTGGCGGAGATGGAGCAGCTGGACTTCA
GCGAGGTGCCGTGGGACGAGGCGGAGGGGTTCGCGCTCACCAAGTACCCGTCGTACGAGATCGACTGG
GACTCGCTGCTCAACAACAATAACTAG

**SEQ ID NO 328, Oryza sativa - protein**

MAAAIEGNLMRALGEAPSPQMQKIAPPPFHPGLPPAPANFSSAGVHGFHYMGPAQLSPAQIQRVQAQL
HMQRQAQSGLGPRAQPMKPASAAAPAAAAARAQKLYRGVRQRHWGKWVAEIRLPRNRTRLWLGTFDTA
EEAALTYDQAAYRLRGDAARLNFPDNAASRGPLDAAVDAKLQAICDTIAASKNASSRSRGGAGRAMPI
NAPLVAAASSSSGSDHSGGGDDGGSETSSSSAAASPLAEMEQLDFSEVPWDEAEGFALTKYPSYEIDW
DSLLNNNN

**SEQ ID NO 329, Oryza sativa - DNA**

ATGGACGCCAGCCTCCGCACACTGCCTCCGGCGATCTTCCCCGGCGAGGTCCGCTCCGCCGTCTCCTC
CCTCCTCCTCTCTCCCGGCGGCAGCGCCCTCGACACCGTGTTCTCCCACCTCCCGCCGCCCGTCACCA
TCCCGCCGCTCGGCTCCAGCGTCTACTACCGCCAGAGCGAGCTCCTGCGCCACTTCGCCGCGTCGCAG
GCGGCGCAGGCGCAGAGCGCCACCGCCGCCGCCAGCTCTTCGTCGGCCGCCGCGGCGGGTCTCGACGA
TGGCGCGCCGCGGAAGCTGTACCGCGGCGTGCGGCAGCGGCAGTGGGGGAAGTGGGTGGCCGAGATCA
GGCTGCCGCAGAACCGGGTGCGCGTGTGGCTCGGCACCTACGACTCGCCGGAGACCGCCGCGCACGCC
TACGACCGCGCCGCGTTCAAGCTCCGCGGCGAGTACGCGCGCCTCAACTTCCCCGGCGTCATGGACGG
CCGCGACTGCCCCGACAACCTGCGCCAACTCCGCGACGCCGTCGACGCCAAGATCCAGGCCATCCGCG
TCCGCATGGCGCGCAAGCGCGCGCGCGCGCGCCGCCAGCGCGAGGAGAGCAAGAAGAGCCAACGCGCC
GAGGACGCGAAGGCGGCGACGCCGTCTCGCCCCGTGGCCTCCGAGCGCGCCGCGTCCGAGACGACGAC
GACGACAACCACGACGTCGTCGTCGTACGGGTCACCGGACGGCGTGCTCTCCATGAGCGCGGCGTCCG
TCGACGGCGACTGCCCGCTCGAGCGGATGCCGTCGTTCGATCCCGAGTTGATCTGGGAGATGCTTAAC
TTCTAG

**SEQ ID NO 330, Oryza sativa - protein**

MDASLRTLPPAIFPGEVRSAVSSLLLSPGGSALDTVFSHLPPPVTIPPLGSSVYYRQSELLRHFAASQ
AAQAQSATAAASSSSAAAAGLDDGAPRKLYRGVRQRQWGKWVAEIRLPQNRVRVWLGTYDSPETAAHA
YDRAAFKLRGEYARLNFPGVMDGRDCPDNLRQLRDAVDAKIQAIRVRMARKRARARRQREESKKSQRA
EDAKAATPSRPVASERAASETTTTTTTSSSYGSPDGVLSMSAASVDGDCPLERMPSFDPELIWEMLN
F

FIGURE 29 (CONTINUED)

**SEQ ID NO 331, C-terminal region of SEQ ID NO: 258**

PFLMQWLNLLPLPVLDSSSWCPEHFHNSESDALP

**SEQ ID NO 332, AP2 DNA-binding domain**

YRGVRQRHWGKWVAEIRLPRNRTRLWLGTFDTAEEAALAYDSAAFRLRGESARLNF

**SEQ ID NO 333, Motif XVIII**

RLPXNX-RXRXWLGT F/Y D/E T/S

**SEQ ID NO 334, Motif XIX**

RG D/E

**SEQ ID NO 335, Motif XX**

WDESESFLLHKYPSLEIDWDAILS

**SEQ ID NO 336, Motif XXI**

GPPLHAAVDAKLHAICH

**SEQ ID NO 337, Motif XXII**

GANYLTPAQVLHVQAQLQRLRRP

**SEQ ID NO 338, Motif XXIII**

VDSKELMGALAPSMVSFSYPCSEQSASS

FIGURE 29 (CONTINUED)

**SEQ ID NO 339 – ORIZA SATIVA – GOS2 PROMOTER (NEW VERSION 013107)**

aatccgaaaagtttctgcaccgttttcacccccctaactaacaatatagggaacgtgtgctaaatataa
aatgagaccttatatatgtagcgctgataactagaactatgcaagaaaaactcatccacctactttag
tggcaatcgggctaaataaaaaagagtcgctacactagtttcgttttccttagtaattaagtgggaaa
atgaaatcattattgcttagaatatacgttcacatctctgtcatgaagttaaattattcgaggtagcc
ataattgtcatcaaactcttcttgaataaaaaaatctttctagctgaactcaatgggtaaagagagag
atttttttaaaaaaatagaatgaagatattctgaacgtattggcaaagatttaaacatataattata
taattttatagtttgtgcattcgtcatatcgcacatcattaaggacatgtcttactccatcccaattt
ttatttagtaattaaagacaattgacttattttattatttatctttttttcgattagatgcaaggtac
ttacgcacacactttgtgctcatgtgcatgtgtgagtgcacctcctcaatacacgttcaactagcaac
acatctctaatatcactcgcctatttaatacatttaggtagcaatatctgaattcaagcactccacca
tcaccagaccacttttaataatatctaaaatacaaaaaataattttacagaatagcatgaaaagtatg
aaacgaactatttaggtttttcacatacaaaaaaaaaaaagaattttgctcgtgcgcgagcgccaatct
cccatattgggcacacaggcaacaacagagtggctgcccacagaacaacccacaaaaaacgatgatct
aacggaggacagcaagtccgcaacaacctttaacagcaggctttgcggccaggagagaggaggagag
gcaaaga!
aaaccaagcatcctccttctcccatctataaattcctccccccttttcccctctctatataggaggca
tccaagccaagaagagggagagcaccaaggacacgcgactagcagaagccgagcgaccgccttctcga
tccatatcttccggtcgagttcttggtcgatctcttccctcctccacctcctcctcacagggtatgtg
cctcccttcggttgttcttggatttattgttctaggttgtgtagtacgggcgttgatgttaggaaagg
ggatctgtatctgtgatgattcctgttcttggatttgggatagaggggttcttgatgttgcatgttat
cggttcggtttgattagtagtatggttttcaatcgtctggagagctctatggaaatgaaatggtttag
ggatcggaatcttgcgattttgtgagtaccttttgtttgaggtaaaatcagagcaccggtgattttgc
ttggtgtaataaagtacggttgtttggtcctcgattctggtagtgatgcttctcgatttgacgaagct
atcctttgtttattccctattgaacaaaaataatccaactttgaagacggtcccgttgatgagattga
atgattgattcttaagcctgtccaaaatttcgcagctggcttgtttagatacagtagtccccatcacg
aaattcatggaaacagttataatcctcaggaacaggggattccctgttcttccgatttgctttagtcc
cagaatttttttcccaaatatcttaaaaagtcactttctggttcagttcaatgaattgattgctaca
aataatgcttttatagcgttatcctagctgtagttcagttaataggtaataccccctatagtttagtca
ggagaagaacttatccgatttctgatctccatttttaattatatgaaatgaactgtagcataagcagt
attcatttggattattttttttattagctctcaccc!
cttcattattctgagctgaaagtctggcatgaactgtcctcaattttgttttcaaattcacatcgatt
at
ctatgcattatcctcttgtatctacctgtagaagtttctttttggttattccttgactgcttgattac
agaaagaaatttatgaagctgtaatcgggatagttatactgcttgttcttatgattcatttcctttgt
gcagttcttggtgtagcttgccactttcaccagcaaagttc

FIGURE 29 (CONTINUED)

**SEQ ID NO 340, Oryza sativa – PRO0110_RCc3_2 (new sequence 050307)**

tcgacgctactcaagtggtgggaggccaccgcatgttccaacgaagcgccaaagaaagccttgcagac
tctaatgctattagtcgcctaggatatttggaatgaaaggaaccgcagagtttttcagcaccaagagc
ttccggtggctagtctgatagccaaaattaaggaggatgccaaaacatgggtcttggcgggcgcgaaa
caccttgataggtggcttacctttttaacatgttcgggccaaaggccttgagacggtaaagttttctat
ttgcgcttgcgcatgtacaattttattcctctattcaatgaaattggtggctcactggttcattaaaa
aaaaaagaatctagcctgttcgggaagaagaggattttgttcgtgagagagagagagagagagagaga
gagagagagagagaaggaggaggaggattttcaggcttcgcattgcccaacctctgcttctgttggcc
caagaagaatcccaggcgcccatgggctggcagtttaccacggacctacctagcctaccttagctatc
taagcgggccgacctagtagccacgtgcctagtgtagattaaagttgccgggccagcaggaagccacg
ctgcaatggcatcttcccctgtccttcgcgtacgtgaaaacaaacccaggtaagcttagaatcttctt
gcccgttggactgggacacccaccaatcccaccatgccccgatattcctccggtctcggttcatgtga
tgtcctctcttgtgtgatcacggagcaagcattcttaaacggcaaaagaaaatcaccaacttgctcac
gcagtcacgctgcaccgcgcgaagcgacgcccgataggccaagatcgcgagataaaataacaaccaat
gatcataaggaaacaagcccgcgatgtgtcgtgtgcagcaatcttggtcatttgcgggatcgagtgct
tcacagc!
taaccaaatattcggccgatgatttaacacattatcagcgtagatgtacgtacgatttgttaattaat
ctacgagccttgctagggcaggtgttctgccagccaatccagatcgccctcgtatgcacgctcacatg
atggcagggcagggttcacatgagctctaacggtcgattaattaatcccggggctcgactataaatac
ctccctaatcccatgatcaaaaccatctcaagcagcctaatcatctccagctgatcaagagctcttaa
ttagctagctagtgattagctgcgcttgtgatc

FIGURE 29 (CONTINUED)

**SEQ ID NO: 341; HVAP2-2 Hordeum vulgare c62815824hv270303@7626**

CCGCTTCATTAAAAACTGCCCATTTTTCCAGTTCCGACGAGGCGCCTCCCCCCCTCCCCG
GCGACGACTAGGACGAGGCACCGCCGGCAACAACCATGTGCGGCGGCGCGATCCTCAAGG
ACCTCAAGGTCCCCGCGGTGACGCGGAAGGTGACGGAGGCGGCGCTGTGGCCCGAGAAGA
AGAAGCCCAGGCAGGCCGACGGCGGGGCCCGGCGCCTCGGGCTGGTCGACGGCGAGGAGG
ACTTCGAGGCCGACTTCGAGGAGTTCGAGGCCGACTCCGGGGACTCCGACCTGGAGCTCG
GGCGCGGCCGGGCGGCTGAGAAGGACGACGACGAGGTCGTCGAGATCAAGCCCTACGCCG
CCGTCAAGAGGCCCCTCTCCCAAGATGACTTCAGCATCATTACTACTGCTGGTTGTGATG
GTCCTGCACAAAGGTCAGCAAAAAGGAAGAGAAAGAACCAATTCAGGGGTATCCGTCAGC
GCCCCTGGGGTAAGTGGGCTGCTGAAATCAGAGATCCTAGCAAAGGTGTCCGTGTTTGGC
TTGGTACTTTCAACAGTGCTGAAGAAGCTGCAAGAGCCTACGATGTTGAAGCACGCAGGA
TCCGTGGCAAGAAGGCCAAGGTAAACTTTCCAGAGGAACCAACAGTTCCTCAGAAGCGCC
GTGTTCGCCCTGCTCCTCTTAAAGCACCCAAGCTAAGCGCATCACAGGAACCTACCGTCA
TACCAGCAGTCAACAACCTTGCCAACCCAAATGCTTTCGTCTACCCATCTGCTGACTTTG
CATCAAACCAGCCACCTGTTCAGCCTGATAACGTGCCATTTGTTCCTGCAATGAAGTTTG
CTGCCCCTGTTGAAGCTCCTGTTATGAATATGTACTCTGACCAGGGAAGCAACTCTTTTG
GCTGTTCTGACTTGGGCTGGGACTATGAGACCAAGACTCCAGATATATCATCCGTTGCTC
CCATTTCCACCATTGCTGAAGGAGCAGAATCTGCGCTTATCCAGAGCAACACCTACAACT
CAGTGGTGCCTCCTGTTATGGAGAACAATGCTGTCGATTTTGAACCTTGGACGAGGTTTC
TTATGGATGATGGCGTGGATGAGCCGATTGACAGCCTTCTGAACTTTGGTGTGCCTCAGG
ATGTCATTAGCAACATGGACCTTTGGAGCTTCGATGACATGCCCATCTGTGGAGAATTAT
GCTGAGGGATTCAAACCCCTGTATATAAAGACAAAGGGAATAAGACTATGGGAGATTGGG
AAGCACCCTGTTGTCAACTTCGGCTAGCATATGCTTATGTCCAAGCTTAGATGCAAAAAA
GTTGCATCCTGAGTCTCTTTTGTAATCAACCCTTTTCCTAGCTGACTGTCGATGAACCGT
TGTCATTTATGAGTCTGATGAACCGTTGTCTTTATCTTTTGTCAACTTATATGTCGTCGC
TACCATTTCTGAATGTGAATGGCATGGATCTATGTCCAGTTTGCTTTAAAAAAAAAAAAA
AAAAAAAAAAAAAAAAAAAAAAAAAAAAAAAAAAAAAAAAA

**SEQ ID NO: 342; HVAP2-2 Hordeum vulgare c62815824hv270303@7626, deduced protein sequence**

MCGGAILKDLKVPAVTRKVTEAALWPEKKKPRQADGGARRLGLVDGEEDFEADFEEFEAD
SGDSDLELGRGRAAEKDDDEVVEIKPYAAVKRPLSQDDFSIITTAGCDGPAQRSAKRKRK
NQFRGIRQRPWGKWAAEIRDPSKGVRVWLGTFNSAEEAARAYDVEARRIRGKKAKVNFPE
EPTVPQKRRVRPAPLKAPKLSASQEPTVIPAVNNLANPNAFVYPSADFASNQPPVQPDNV
PFVPAMKFAAPVEAPVMNMYSDQGSNSFGCSDLGWDYETKTPDISSVAPISTIAEGAESA
LIQSNTYNSVVPPVMENNAVDFEPWTRFLMDDGVDEPIDSLLNFGVPQDVISNMDLWSFD
DMPICGELC

**SEQ ID NO: 343; ZmAP2-2 Zea mays AP2-2 homologue c65144673gm030403@12827**

CACCATTTCGCTCCCGCAAGCACCGATTTGTTTACTGCTCTCTCCGACGCGCGGCGGCGG
AGCTCCCTACGACGACTGAACCATGTGCGGCGGCGCGATCCTTGCCAACCTCCGCGAGCC
GGCGCCGCGCCGGCTCACAGAGCGGGACATCTGGCAGCAGAAGAAGAAGCCCAAGAGGGG
CGGCGCCGGCGGGAGGCGCTCGTTCGCGGCGGAAGACGATGAGGACTTCGAGGCCGACTT

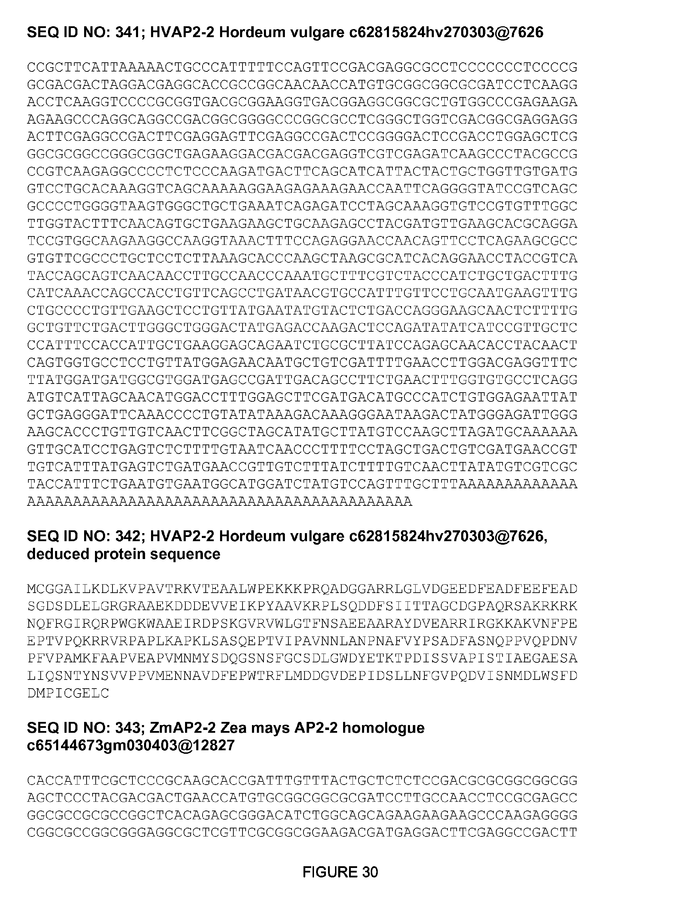

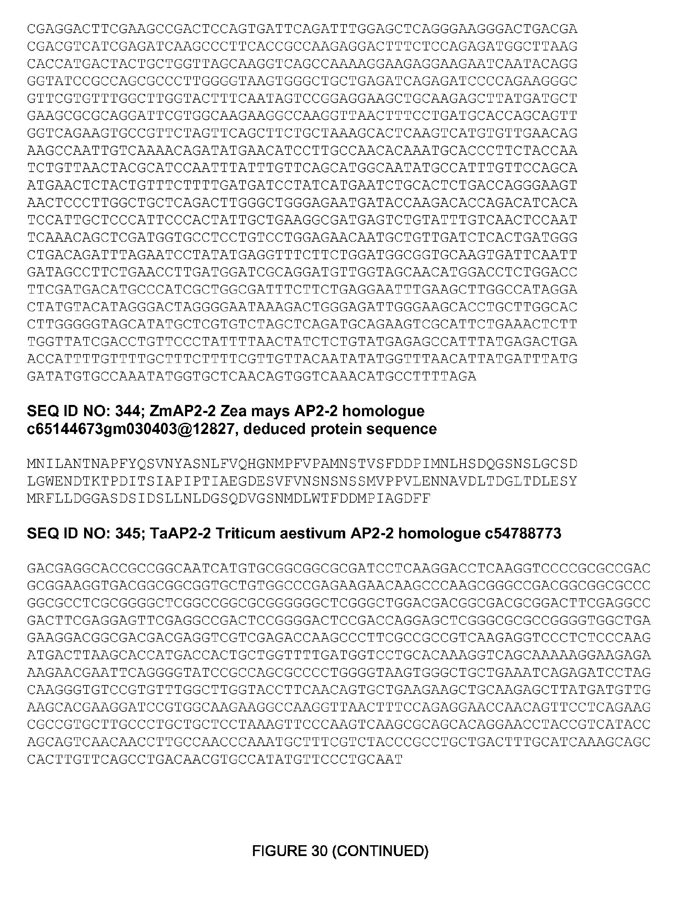

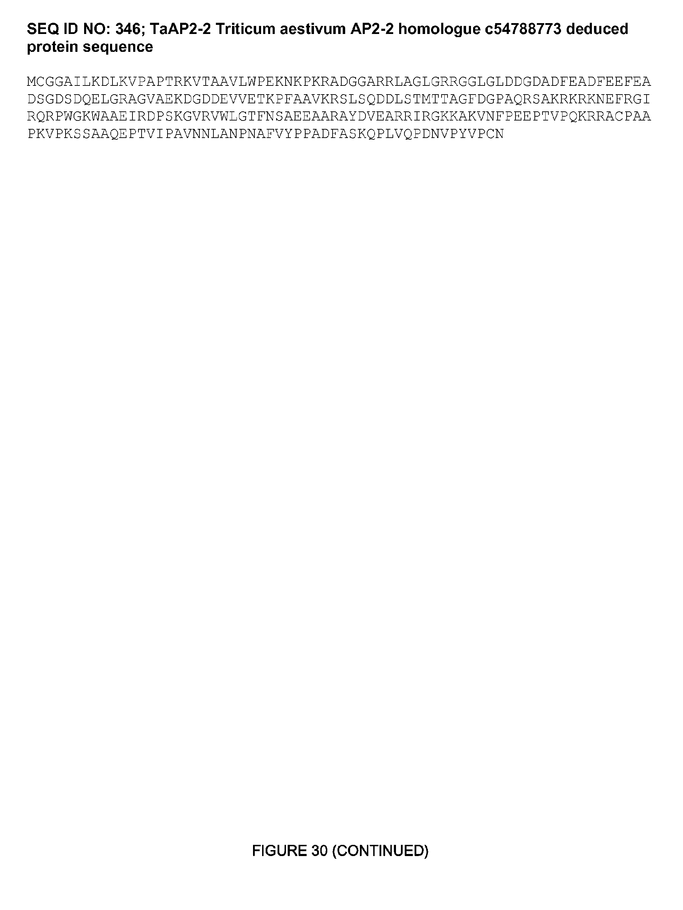

FIGURE 30

CGAGGACTTCGAAGCCGACTCCAGTGATTCAGATTTGGAGCTCAGGGAAGGGACTGACGA
CGACGTCATCGAGATCAAGCCCTTCACCGCCAAGAGGACTTTCTCCAGAGATGGCTTAAG
CACCATGACTACTGCTGGTTAGCAAGGTCAGCCAAAAGGAAGAGGAAGAATCAATACAGG
GGTATCCGCCAGCGCCCTTGGGGTAAGTGGGCTGCTGAGATCAGAGATCCCCAGAAGGGC
GTTCGTGTTTGGCTTGGTACTTTCAATAGTCCGGAGGAAGCTGCAAGAGCTTATGATGCT
GAAGCGCGCAGGATTCGTGGCAAGAAGGCCAAGGTTAACTTTCCTGATGCACCAGCAGTT
GGTCAGAAGTGCCGTTCTAGTTCAGCTTCTGCTAAAGCACTCAAGTCATGTGTTGAACAG
AAGCCAATTGTCAAAACAGATATGAACATCCTTGCCAACACAAATGCACCCTTCTACCAA
TCTGTTAACTACGCATCCAATTTATTTGTTCAGCATGGCAATATGCCATTTGTTCCAGCA
ATGAACTCTACTGTTTCTTTTGATGATCCTATCATGAATCTGCACTCTGACCAGGGAAGT
AACTCCCTTGGCTGCTCAGACTTGGGCTGGGAGAATGATACCAAGACACCAGACATCACA
TCCATTGCTCCCATTCCCACTATTGCTGAAGGCGATGAGTCTGTATTTGTCAACTCCAAT
TCAAACAGCTCGATGGTGCCTCCTGTCCTGGAGAACAATGCTGTTGATCTCACTGATGGG
CTGACAGATTTAGAATCCTATATGAGGTTTCTTCTGGATGGCGGTGCAAGTGATTCAATT
GATAGCCTTCTGAACCTTGATGGATCGCAGGATGTTGGTAGCAACATGGACCTCTGGACC
TTCGATGACATGCCCATCGCTGGCGATTTCTTCTGAGGAATTTGAAGCTTGGCCATAGGA
CTATGTACATAGGGACTAGGGGAATAAAGACTGGGAGATTGGGAAGCACCTGCTTGGCAC
CTTGGGGGTAGCATATGCTCGTGTCTAGCTCAGATGCAGAAGTCGCATTCTGAAACTCTT
TGGTTATCGACCTGTTCCCTATTTTAACTATCTCTGTATGAGAGCCATTTATGAGACTGA
ACCATTTTGTTTTGCTTTCTTTTCGTTGTTACAATATATGGTTTAACATTATGATTTATG
GATATGTGCCAAATATGGTGCTCAACAGTGGTCAAACATGCCTTTTAGA

**SEQ ID NO: 344; ZmAP2-2 Zea mays AP2-2 homologue c65144673gm030403@12827, deduced protein sequence**

MNILANTNAPFYQSVNYASNLFVQHGNMPFVPAMNSTVSFDDPIMNLHSDQGSNSLGCSD
LGWENDTKTPDITSIAPIPTIAEGDESVFVNSNSNSSMVPPVLENNAVDLTDGLTDLESY
MRFLLDGGASDSIDSLLNLDGSQDVGSNMDLWTFDDMPIAGDFF

**SEQ ID NO: 345; TaAP2-2 Triticum aestivum AP2-2 homologue c54788773**

GACGAGGCACCGCCGGCAATCATGTGCGGCGGCGCGATCCTCAAGGACCTCAAGGTCCCCGCGCCGAC
GCGGAAGGTGACGGCGGCGGTGCTGTGGCCCGAGAAGAACAAGCCCAAGCGGGCCGACGGCGGCGCCC
GGCGCCTCGCGGGGCTCGGCCGGCGCGGGGGGCTCGGGCTGGACGACGGCGACGCGGACTTCGAGGCC
GACTTCGAGGAGTTCGAGGCCGACTCCGGGGACTCCGACCAGGAGCTCGGGCGCGCCGGGGTGGCTGA
GAAGGACGGCGACGACGAGGTCGTCGAGACCAAGCCCTTCGCCGCCGTCAAGAGGTCCCTCTCCCAAG
ATGACTTAAGCACCATGACCACTGCTGGTTTTGATGGTCCTGCACAAAGGTCAGCAAAAAGGAAGAGA
AAGAACGAATTCAGGGGTATCCGCCAGCGCCCCTGGGGTAAGTGGGCTGCTGAAATCAGAGATCCTAG
CAAGGGTGTCCGTGTTTGGCTTGGTACCTTCAACAGTGCTGAAGAAGCTGCAAGAGCTTATGATGTTG
AAGCACGAAGGATCCGTGGCAAGAAGGCCAAGGTTAACTTTCCAGAGGAACCAACAGTTCCTCAGAAG
CGCCGTGCTTGCCCTGCTGCTCCTAAAGTTCCCAAGTCAAGCGCAGCACAGGAACCTACCGTCATACC
AGCAGTCAACAACCTTGCCAACCCAAATGCTTTCGTCTACCCGCCTGCTGACTTTGCATCAAAGCAGC
CACTTGTTCAGCCTGACAACGTGCCATATGTTCCCTGCAAT

FIGURE 30 (CONTINUED)

**SEQ ID NO: 346; TaAP2-2 Triticum aestivum AP2-2 homologue c54788773 deduced protein sequence**

MCGGAILKDLKVPAPTRKVTAAVLWPEKNKPKRADGGARRLAGLGRRGGLGLDDGDADFEADFEEFEA
DSGDSDQELGRAGVAEKDGDDEVVETKPFAAVKRSLSQDDLSTMTTAGFDGPAQRSAKRKRKNEFRGI
RQRPWGKWAAEIRDPSKGVRVWLGTFNSAEEAARAYDVEARRIRGKKAKVNFPEEPTVPQKRRACPAA
PKVPKSSAAQEPTVIPAVNNLANPNAFVYPPADFASKQPLVQPDNVPYVPCN

FIGURE 30 (CONTINUED)

**SEQ ID NO: 347; ZmNAC1 Zea mays NAC1 homologue c58553294gm030403@3368**

GGGACACACACGCAGATCCGAGCTAACCACCATCGACGAGCGCCAGCGCCAGCAGCCGAG
CCGGATCGACCTTTTCTTTTTTCTTTTACACAGCGGGACAGAGAAAGGAGTCAGGCAGCC
AAAGCCACCCACCGCTTTTACCAACCGATCGTCCGATCGGCGTTGCCGCCACCGCTAGCA
TTGTCGGCTTCAGCTCCATCCAAATCCACCGCCAGCAAGCAAGCCGGCGCCATGGGTCTG
CCGATGAGGAGGGAGAGGGACGCGGAGGCGGAGCTCAACCTGCCGCCGGGGTTCCGGTTC
CACCCGACGGACGAGGAGCTGGTGGCGCACTACCTCTGCGCGCGCGCCGCGGGGCGCCGC
CCGCCCGTGCCCATCATCGCCGAGGTCGACCTGTACCGCTTCGACCCCTGGGACCTGCCG
GAGCGCGCCCTCTTCGGGCGCCGCGAGTGGTACTTCTTCACGCCGCGGGACCGCAAGTAC
CCCAACGGCTCCCGCCCCAACCGCGCCGCCGGGTCGGGCTACTGGAAGGCCACGGGCGCG
GACAAGCCCGTGGAGCACGGGGGCCGGACGGCGGGGATCAAGAAGGCGCTCGTGTTCTAC
CACGGCAAGCCGCCGCGCGGGGTCAAGACGGAGTGGATCATGCACGAGTACCGCCTCGCC
GAAGCCGGCGGCGCCCGCGCCAAGAAGTCCGGCGCCGGCACGCTCAGGCTGGATGACTGG
GTGCTGTGCCGCCTGTACAACAAGAAGAACGAGTGGGAGAAGATGCAGCAGCAGAAGGAG
AAGGAGAGAAGAAGGCGATGGAGTCGGAGGCGTCGCTCTCGCACTCCCACTCCGACACGC
GGACGCCGGAATCGGAGATCGACGACGACCCGTTCCCGGAGCTGGGCTCTCTGCCGGCGT
TCGACGACATGGGCCCAGCCCCAGCAGCAGCAGCCCCTGCCGGCGTCGTCCTGCCCAAGG
AGGAGGTGGAGGACTTCGGCGACCTCGGCGGCGACGACTGGCTCGCGGGCATCAACCTCG
ACGACCTGCAGATGCCGGGGGACGCCGCCGATTTCTACGGCTCCATGCTCGTCTCGCCGA
TGGCCGCCAAGATGGAGCCGGACGGCGGCTTCCCCTTCTTCTGAGATCCTTCGGCGACTC
CGGGGACGACCGGATGCAGCGCAACGCAAACTGTGTATAGTTCAGATTTTCTTCATGGAA
CCAACAACAACAACAAAAAAAGTTCTGCTAATATCTTGTTCTAGTGGTGTAGTGCAGAAG
TAGCAGCAGGCATGATTGAAGTTCCATTGTAGTACTGATGTTCATCGCCACTTGATCTGT
GACAAGTGACGGCATTCTTTCGTTTCTTGGCTGCAAATTCTTCGTTGTTCAGAATATAAT
TTCAGATGGGTAATGCCAA

**SEQ ID NO: 348; ZmNAC1 Zea mays c58553294gm030403@3368 deduced protein sequence**

MGLPMRRERDAEAELNLPPGFRFHPTDEELVAHYLCARAAGRRPPVPIIAEVDLYRFDPW
DLPERALFGRREWYFFTPRDRKYPNGSRPNRAAGSGYWKATGADKPVEHGGRTAGIKKAL
VFYHGKPPRGVKTEWIMHEYRLAEAGGARAKKSGAGTLRLDDWVLCRLYNKKNEWEKMQQ
QKEKERRRRWSRRRRSRTPTPTRGRRNRRSTTTRSRSWALCRRSTTWAQPQQQQPLPASS
CPRRRWRTSATSAATTGSRASTSTTCRCRGTPPISTAPCSSRRWPPRWSRTAASPSSEIL
RRLRGRPDAAQRKLCIVQIFFMEPTTTTKKVLLISCSSGVVQK

**SEQ ID NO: 349; Hordeum vulgare NAC3 homologue c62818592hv270303@4035 revcomp**

TTTCATATGCAGTAGCCGTCTTCTCCTCCTCCTCCTCCTGCCTTCCAGCTAGCTCAC
TATCGCATCATCCACACCACACCTACTACTCATACCGATTCCGTTCCCACCCGCTCGCCA
TCGCCATGCCAATGGGCAGCAGCAGCAGCAGCGCCGCCATGCCGGCCCTCCCTCCCGGCT
TCCGGGTCCACCCCACCGACGAGGAGCTCATCGTCCACTACCTCGGCAGGCAGGCCGCGT
CCATGCCCAGCCCCGTGCCCATCATCGCCGAGGTCAACATCTACAAGTGCAACCCATGGG

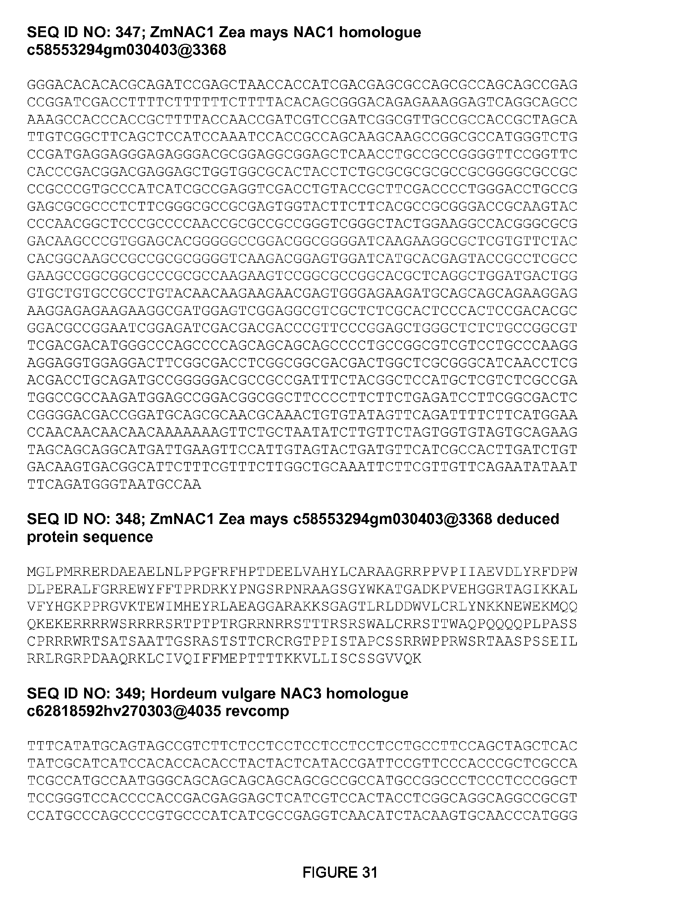

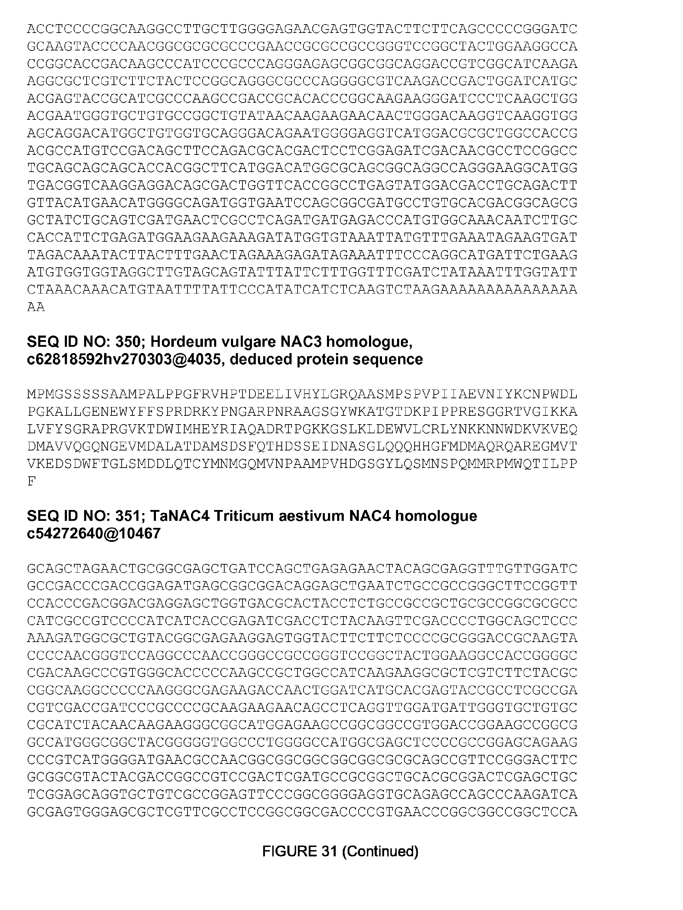

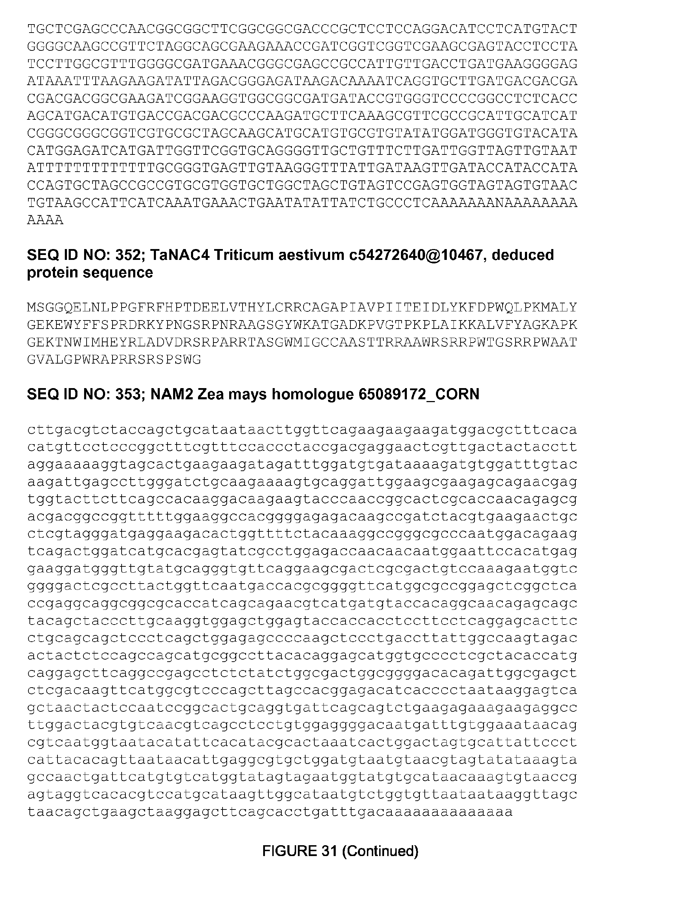

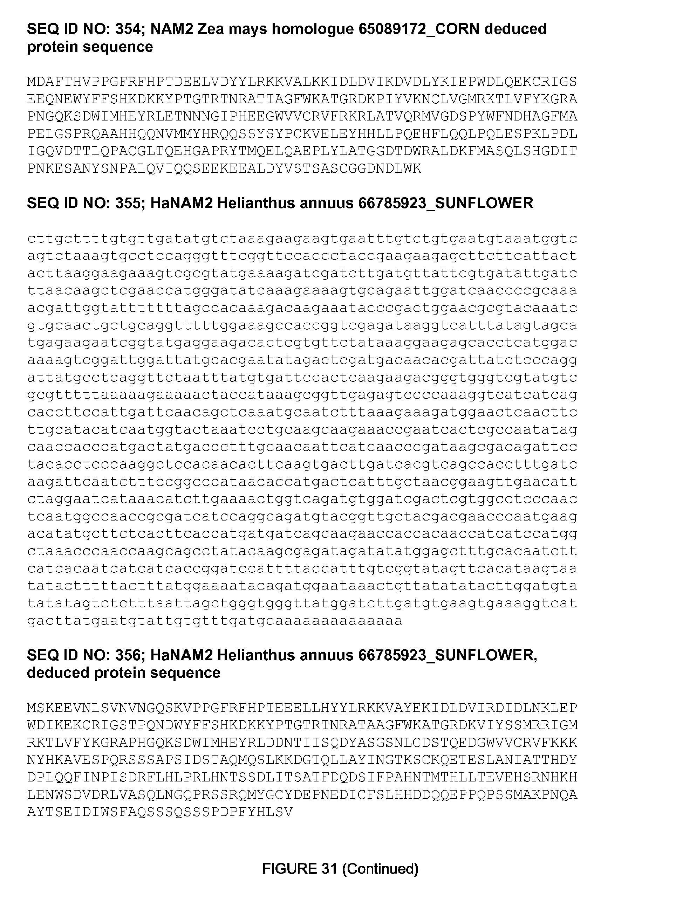

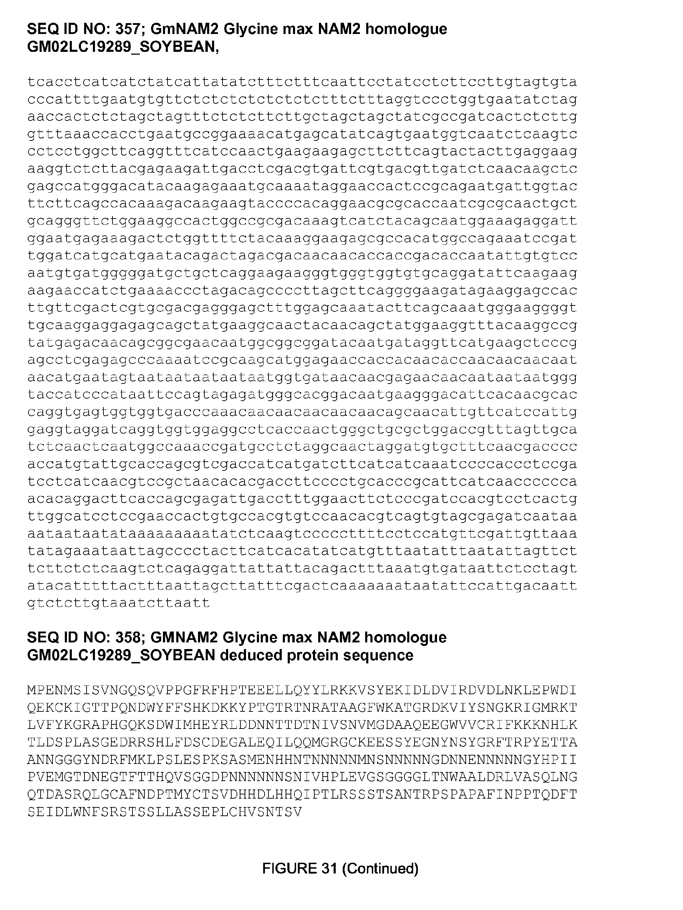

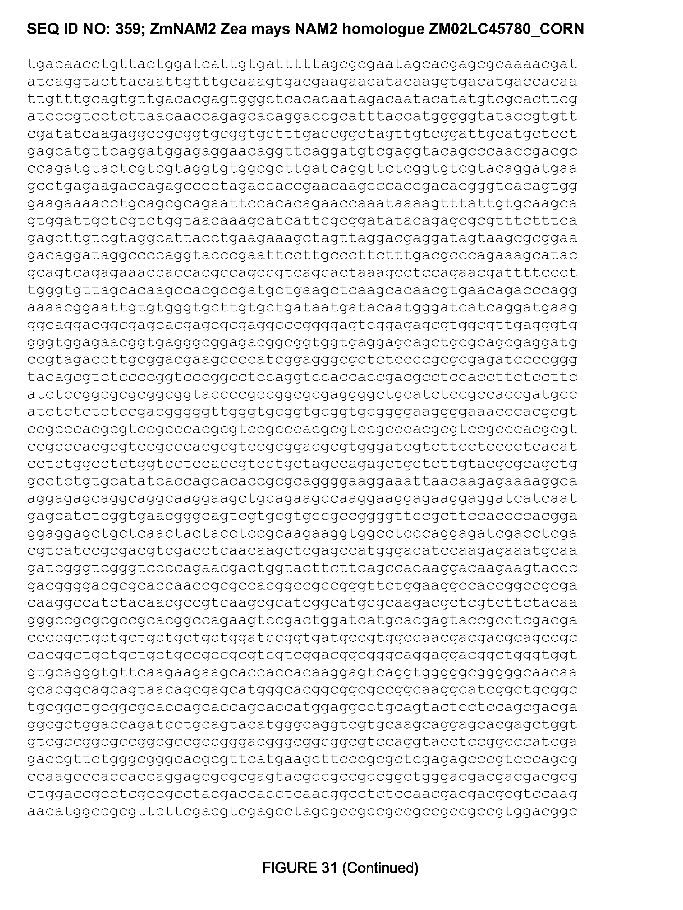

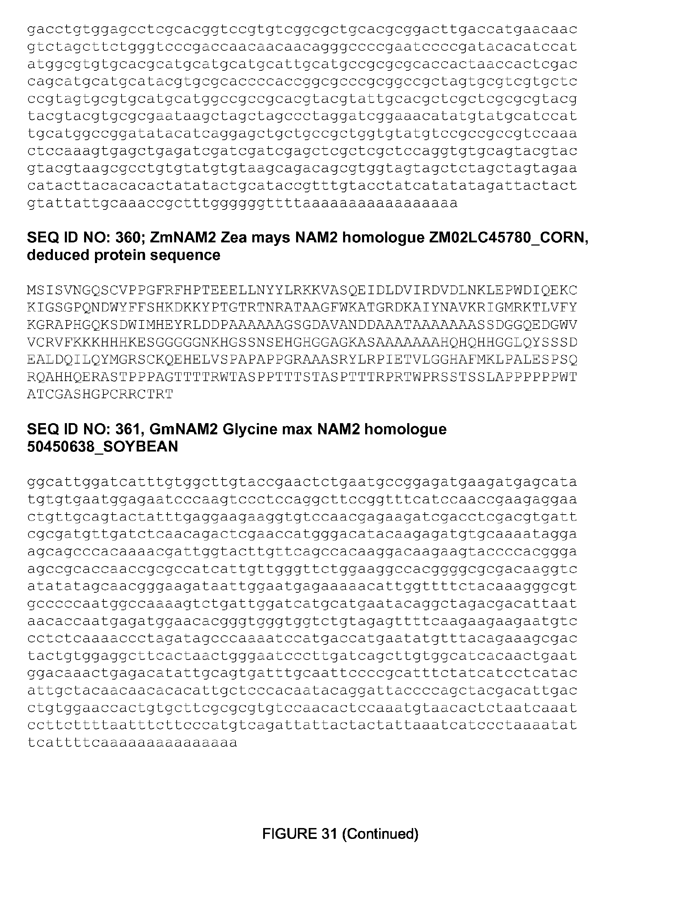

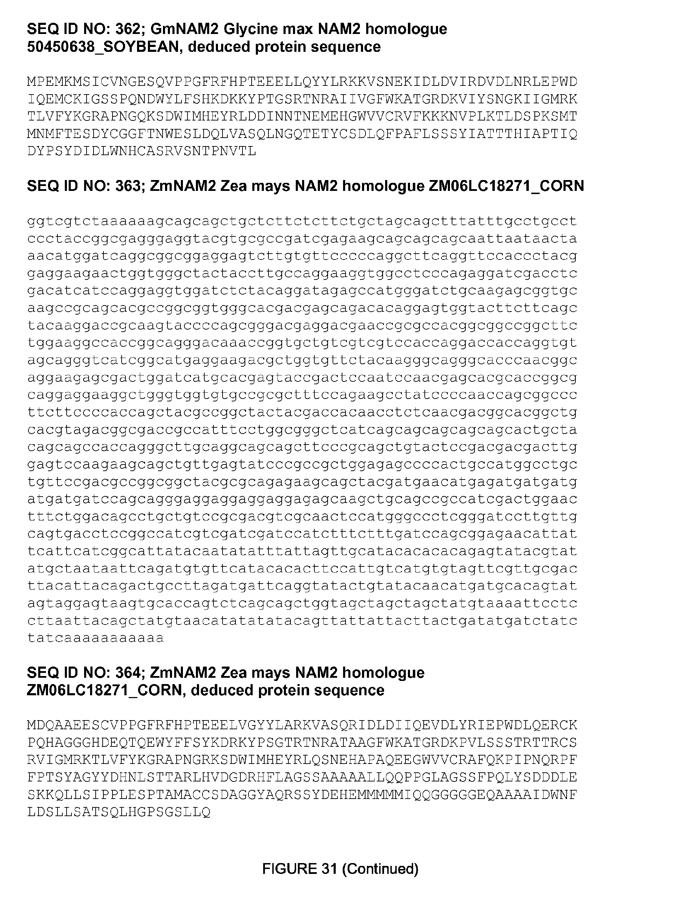

FIGURE 31

ACCTCCCCGGCAAGGCCTTGCTTGGGGAGAACGAGTGGTACTTCTTCAGCCCCCGGGATC
GCAAGTACCCCAACGGCGCGCGCCCGAACCGCGCCGCCGGGTCCGGCTACTGGAAGGCCA
CCGGCACCGACAAGCCCATCCCGCCCAGGGAGAGCGGCGGCAGGACCGTCGGCATCAAGA
AGGCGCTCGTCTTCTACTCCGGCAGGGCGCCCAGGGGCGTCAAGACCGACTGGATCATGC
ACGAGTACCGCATCGCCCAAGCCGACCGCACACCCGGCAAGAAGGGATCCCTCAAGCTGG
ACGAATGGGTGCTGTGCCGGCTGTATAACAAGAAGAACAACTGGGACAAGGTCAAGGTGG
AGCAGGACATGGCTGTGGTGCAGGGACAGAATGGGGAGGTCATGGACGCGCTGGCCACCG
ACGCCATGTCCGACAGCTTCCAGACGCACGACTCCTCGGAGATCGACAACGCCTCCGGCC
TGCAGCAGCAGCACCACGGCTTCATGGACATGGCGCAGCGGCAGGCCAGGGAAGGCATGG
TGACGGTCAAGGAGGACAGCGACTGGTTCACCGGCCTGAGTATGGACGACCTGCAGACTT
GTTACATGAACATGGGGCAGATGGTGAATCCAGCGGCGATGCCTGTGCACGACGGCAGCG
GCTATCTGCAGTCGATGAACTCGCCTCAGATGATGAGACCCATGTGGCAAACAATCTTGC
CACCATTCTGAGATGGAAGAAGAAAGATATGGTGTAAATTATGTTTGAAATAGAAGTGAT
TAGACAAATACTTACTTTGAACTAGAAAGAGATAGAAATTTCCCAGGCATGATTCTGAAG
ATGTGGTGGTAGGCTTGTAGCAGTATTTATTCTTTGGTTTCGATCTATAAATTTGGTATT
CTAAACAAACATGTAATTTTATTCCCATATCATCTCAAGTCTAAGAAAAAAAAAAAAAAA
AA

**SEQ ID NO: 350; Hordeum vulgare NAC3 homologue, c62818592hv270303@4035, deduced protein sequence**

MPMGSSSSSAAMPALPPGFRVHPTDEELIVHYLGRQAASMPSPVPIIAEVNIYKCNPWDL
PGKALLGENEWYFFSPRDRKYPNGARPNRAAGSGYWKATGTDKPIPPRESGGRTVGIKKA
LVFYSGRAPRGVKTDWIMHEYRIAQADRTPGKKGSLKLDEWVLCRLYNKKNNWDKVKVEQ
DMAVVQGQNGEVMDALATDAMSDSFQTHDSSEIDNASGLQQQHHGFMDMAQRQAREGMVT
VKEDSDWFTGLSMDDLQTCYMNMGQMVNPAAMPVHDGSGYLQSMNSPQMMRPMWQTILPP
F

**SEQ ID NO: 351; TaNAC4 Triticum aestivum NAC4 homologue c54272640@10467**

GCAGCTAGAACTGCGGCGAGCTGATCCAGCTGAGAGAACTACAGCGAGGTTTGTTGGATC
GCCGACCCGACCGGAGATGAGCGGCGGACAGGAGCTGAATCTGCCGCCGGGCTTCCGGTT
CCACCCGACGGACGAGGAGCTGGTGACGCACTACCTCTGCCGCCGCTGCGCCGGCGCGCC
CATCGCCGTCCCCATCATCACCGAGATCGACCTCTACAAGTTCGACCCCTGGCAGCTCCC
AAAGATGGCGCTGTACGGCGAGAAGGAGTGGTACTTCTTCTCCCCGCGGGACCGCAAGTA
CCCCAACGGGTCCAGGCCCAACCGGGCCGCCGGGTCCGGCTACTGGAAGGCCACCGGGGC
CGACAAGCCCGTGGGCACCCCCAAGCCGCTGGCCATCAAGAAGGCGCTCGTCTTCTACGC
CGGCAAGGCCCCCAAGGGCGAGAAGACCAACTGGATCATGCACGAGTACCGCCTCGCCGA
CGTCGACCGATCCCGCCCCGCAAGAAGAACAGCCTCAGGTTGGATGATTGGGTGCTGTGC
CGCATCTACAACAAGAAGGGCGGCATGGAGAAGCCGGCGGCCGTGGACCGGAAGCCGGCG
GCCATGGGCGGCTACGGGGGTGGCCCTGGGGCCATGGCGAGCTCCCCGCCGGAGCAGAAG
CCCGTCATGGGGATGAACGCCAACGGCGGCGGCGGCGGCGCGCAGCCGTTCCGGGACTTC
GCGGCGTACTACGACCGGCCGTCCGACTCGATGCCGCGGCTGCACGCGGACTCGAGCTGC
TCGGAGCAGGTGCTGTCGCCGGAGTTCCCGGCGGGGAGGTGCAGAGCCAGCCCAAGATCA
GCGAGTGGGAGCGCTCGTTCGCCTCCGGCGGCGACCCCGTGAACCCGGCGGCCGGCTCCA

FIGURE 31 (Continued)

TGCTCGAGCCCAACGGCGGCTTCGGCGGCGACCCGCTCCTCCAGGACATCCTCATGTACT
GGGGCAAGCCGTTCTAGGCAGCGAAGAAACCGATCGGTCGGTCGAAGCGAGTACCTCCTA
TCCTTGGCGTTTGGGGCGATGAAACGGGCGAGCCGCCATTGTTGACCTGATGAAGGGGAG
ATAAATTTAAGAAGATATTAGACGGGAGATAAGACAAAATCAGGTGCTTGATGACGACGA
CGACGACGGCGAAGATCGGAAGGTGGCGGCGATGATACCGTGGGTCCCCGGCCTCTCACC
AGCATGACATGTGACCGACGACGCCCAAGATGCTTCAAAGCGTTCGCCGCATTGCATCAT
CGGGCGGGCGGTCGTGCGCTAGCAAGCATGCATGTGCGTGTATATGGATGGGTGTACATA
CATGGAGATCATGATTGGTTCGGTGCAGGGGTTGCTGTTTCTTGATTGGTTAGTTGTAAT
ATTTTTTTTTTTTTGCGGGTGAGTTGTAAGGGTTTATTGATAAGTTGATACCATACCATA
CCAGTGCTAGCCGCCGTGCGTGGTGCTGGCTAGCTGTAGTCCGAGTGGTAGTAGTGTAAC
TGTAAGCCATTCATCAAATGAAACTGAATATATTATCTGCCCTCAAAAAAANAAAAAAAA
AAAA

## SEQ ID NO: 352; TaNAC4 Triticum aestivum c54272640@10467, deduced protein sequence

MSGGQELNLPPGFRFHPTDEELVTHYLCRRCAGAPIAVPIITEIDLYKFDPWQLPKMALY
GEKEWYFFSPRDRKYPNGSRPNRAAGSGYWKATGADKPVGTPKPLAIKKALVFYAGKAPK
GEKTNWIMHEYRLADVDRSRPARRTASGWMIGCCAASTTRRAAWRSRRPWTGSRRPWAAT
GVALGPWRAPRRSRSPSWG

## SEQ ID NO: 353; NAM2 Zea mays homologue 65089172_CORN

cttgacgtctaccagctgcataataacttggttcagaagaagaagatggacgctttcaca
catgttcctcccggctttcgtttccaccctaccgacgaggaactcgttgactactacctt
aggaaaaaggtagcactgaagaagatagatttggatgtgataaaagatgtggatttgtac
aagattgagccttgggatctgcaagaaaagtgcaggattggaagcgaagagcagaacgag
tggtacttcttcagccacaaggacaagaagtacccaaccggcactcgcaccaacagagcg
acgacggccggtttttggaaggccacggggagagacaagccgatctacgtgaagaactgc
ctcgtagggatgaggaagacactggttttctacaaaggccgggcgcccaatggacagaag
tcagactggatcatgcacgagtatcgcctggagaccaacaacaatggaattccacatgag
gaaggatgggttgtatgcagggtgttcaggaagcgactcgcgactgtccaaagaatggtc
ggggactcgccttactggttcaatgaccacgcggggttcatggcgccggagctcggctca
ccgaggcaggcggcgcaccatcagcagaacgtcatgatgtaccacaggcaacagagcagc
tacagctacccttgcaaggtggagctggagtaccaccacctccttcctcaggagcacttc
ctgcagcagctccctcagctggagagccccaagctccctgaccttattggccaagtagac
actactctccagccagcatgcggccttacacaggagcatggtgcccctcgctacaccatg
caggagcttcaggccgagcctctctatctggcgactggcggggacacagattggcgagct
ctcgacaagttcatggcgtcccagcttagccacggagacatcaccccctaataaggagtca
gctaactactccaatccggcactgcaggtgattcagcagtctgaagagaaagaagaggcc
ttggactacgtgtcaacgtcagcctcctgtggaggggacaatgatttgtggaaataacag
cgtcaatggtaatacatattcacatacgcactaaatcactggactagtgcattattccct
cattacacagttaataacattgaggcgtgctggatgtaatgtaacgtagtatataaagta
gccaactgattcatgtgtcatggtatagtagaatggtatgtgcataacaaagtgtaaccg
agtaggtcacacgtccatgcataagttggcataatgtctggtgttaataataaggttagc
taacagctgaagctaaggagcttcagcacctgatttgacaaaaaaaaaaaaaa

FIGURE 31 (Continued)

**SEQ ID NO: 354; NAM2 Zea mays homologue 65089172_CORN deduced protein sequence**

MDAFTHVPPGFRFHPTDEELVDYYLRKKVALKKIDLDVIKDVDLYKIEPWDLQEKCRIGS
EEQNEWYFFSHKDKKYPTGTRTNRATTAGFWKATGRDKPIYVKNCLVGMRKTLVFYKGRA
PNGQKSDWIMHEYRLETNNNGIPHEEGWVVCRVFRKRLATVQRMVGDSPYWFNDHAGFMA
PELGSPRQAAHHQQNVMMYHRQQSSYSYPCKVELEYHHLLPQEHFLQQLPQLESPKLPDL
IGQVDTTLQPACGLTQEHGAPRYTMQELQAEPLYLATGGDTDWRALDKFMASQLSHGDIT
PNKESANYSNPALQVIQQSEEKEEALDYVSTSASCGGDNDLWK

**SEQ ID NO: 355; HaNAM2 Helianthus annuus 66785923_SUNFLOWER**

cttgcttttgtgttgatatgtctaaagaagaagtgaatttgtctgtgaatgtaaatggtc
agtctaaagtgcctccagggtttcggttccaccctaccgaagaagagcttcttcattact
acttaaggaagaaagtcgcgtatgaaaagatcgatcttgatgttattcgtgatattgatc
ttaacaagctcgaaccatgggatatcaaagaaaagtgcagaattggatcaaccccgcaaa
acgattggtatttttttagccacaaagacaagaaatacccgactggaacgcgtacaaatc
gtgcaactgctgcaggtttttggaaagccaccggtcgagataaggtcatttatagtagca
tgagaagaatcggtatgaggaagacactcgtgttctataaaggaagagcacctcatggac
aaaagtcggattggattatgcacgaatatagactcgatgacaacacgattatctcccagg
attatgcctcaggttctaatttatgtgattccactcaagaagacgggtgggtcgtatgtc
gcgtttttaaaaagaaaaactaccataaagcggttgagagtccccaaaggtcatcatcag
caccttccattgattcaacagctcaaatgcaatctttaaagaaagatggaactcaacttc
ttgcatacatcaatggtactaaatcctgcaagcaagaaaccgaatcactcgccaatatag
caaccacccatgactatgaccctttgcaacaattcatcaacccgataagcgacagattcc
tacacctcccaaggctccacaacacttcaagtgacttgatcacgtcagccacctttgatc
aagattcaatctttccggcccataacaccatgactcatttgctaacggaagttgaacatt
ctaggaatcataaacatcttgaaaactggtcagatgtggatcgactcgtggcctcccaac
tcaatggccaaccgcgatcatccaggcagatgtacggttgctacgacgaacccaatgaag
acatatgcttctcacttcaccatgatgatcagcaagaaccaccacaaccatcatccatgg
ctaaacccaaccaagcagcctatacaagcgagatagatatatggagctttgcacaatctt
catcacaatcatcatcaccggatccattttaccatttgtcggtatagttcacataagtaa
tatactttttactttatggaaaatacagatggaataaactgttatatatacttggatgta
tatatagtctctttaattagctgggtgggttatggatcttgatgtgaagtgaaaggtcat
gacttatgaatgtattgtgtttgatgcaaaaaaaaaaaaaa

**SEQ ID NO: 356; HaNAM2 Helianthus annuus 66785923_SUNFLOWER, deduced protein sequence**

MSKEEVNLSVNVNGQSKVPPGFRFHPTEEELLHYYLRKKVAYEKIDLDVIRDIDLNKLEP
WDIKEKCRIGSTPQNDWYFFSHKDKKYPTGTRTNRATAAGFWKATGRDKVIYSSMRRIGM
RKTLVFYKGRAPHGQKSDWIMHEYRLDDNTIISQDYASGSNLCDSTQEDGWVVCRVFKKK
NYHKAVESPQRSSSAPSIDSTAQMQSLKKDGTQLLAYINGTKSCKQETESLANIATTHDY
DPLQQFINPISDRFLHLPRLHNTSSDLITSATFDQDSIFPAHNTMTHLLTEVEHSRNHKH
LENWSDVDRLVASQLNGQPRSSRQMYGCYDEPNEDICFSLHHDDQQEPPQPSSMAKPNQA
AYTSEIDIWSFAQSSSQSSSPDPFYHLSV

FIGURE 31 (Continued)

**SEQ ID NO: 357; GmNAM2 Glycine max NAM2 homologue GM02LC19289_SOYBEAN,**

tcacctcatcatctatcattatatctttctttcaattcctatcctcttccttgtagtgta
cccattttgaatgtgttctctctctctctctctttctttaggtccctggtgaatatctag
aaccactctctagctagtttctctcttcttgctagctagctatcgccgatcactctcttg
gtttaaaccacctgaatgccggaaaacatgagcatatcagtgaatggtcaatctcaagtc
cctcctggcttcaggtttcatccaactgaagaagagcttcttcagtactacttgaggaag
aaggtctcttacgagaagattgacctcgacgtgattcgtgacgttgatctcaacaagctc
gagccatgggacatacaagagaaatgcaaaataggaaccactccgcagaatgattggtac
ttcttcagccacaaagacaagaagtaccccacaggaacgcgcaccaatcgcgcaactgct
gcagggttctggaaggccactggccgcgacaaagtcatctacagcaatggaaagaggatt
ggaatgagaaagactctggttttctacaaaggaagagcgccacatggccagaaatccgat
tggatcatgcatgaatacagactagacgacaacaacaccaccgacaccaatattgtgtcc
aatgtgatgggggatgctgctcaggaagaagggtgggtggtgtgcaggatattcaagaag
aagaaccatctgaaaaccctagacagccccttagcttcaggggaagatagaaggagccac
ttgttcgactcgtgcgacgagggagctttggagcaaatacttcagcaaatgggaaggggt
tgcaaggaggagagcagctatgaaggcaactacaacagctatggaaggtttacaaggccg
tatgagacaacagcggcgaacaatggcggcggatacaatgataggttcatgaagctcccg
agcctcgagagcccaaaatccgcaagcatggagaaccaccacaacaccaacaacaacaat
aacatgaatagtaataataataataatggtgataacaacgagaacaacaataataatggg
taccatcccataattccagtagagatgggcacggacaatgaagggacattcacaacgcac
caggtgagtggtggtgacccaaacaacaacaacaacaacagcaacattgttcatccattg
gaggtaggatcaggtggtggaggcctcaccaactgggctgcgctggaccgtttagttgca
tctcaactcaatggccaaaccgatgcctctaggcaactaggatgtgctttcaacgacccc
accatgtattgcaccagcgtcgaccatcatgatcttcatcatcaaatccccaccctccga
tcctcatcaacgtccgctaacacacgaccttcccctgcacccgcattcatcaaccccccca
acacaggacttcaccagcgagattgacctttggaacttctcccgatccacgtcctcactg
ttggcatcctccgaaccactgtgccacgtgtccaacacgtcagtgtagcgagatcaataa
aataataatataaaaaaaaatatctcaagtccccccttttcctccatgttcgattgttaaa
tatagaaataattagcccctacttcatcacatatcatgtttaatatttaatattagttct
tcttctctcaagtctcagaggattattattacagactttaaatgtgataattctcctagt
atacattttttactttaattagcttatttcgactcaaaaaaataatattccattgacaatt
gtctcttgtaaatcttaatt

**SEQ ID NO: 358; GMNAM2 Glycine max NAM2 homologue GM02LC19289_SOYBEAN deduced protein sequence**

MPENMSISVNGQSQVPPGFRFHPTEEELLQYYLRKKVSYEKIDLDVIRDVDLNKLEPWDI
QEKCKIGTTPQNDWYFFSHKDKKYPTGTRTNRATAAGFWKATGRDKVIYSNGKRIGMRKT
LVFYKGRAPHGQKSDWIMHEYRLDDNNTTDTNIVSNVMGDAAQEEGWVVCRIFKKKNHLK
TLDSPLASGEDRRSHLFDSCDEGALEQILQQMGRGCKEESSYEGNYNSYGRFTRPYETTA
ANNGGGYNDRFMKLPSLESPKSASMENHHNTNNNNNMNSNNNNNGDNNENNNNNGYHPII
PVEMGTDNEGTFTTHQVSGGDPNNNNNNSNIVHPLEVGSGGGGLTNWAALDRLVASQLNG
QTDASRQLGCAFNDPTMYCTSVDHHDLHHQIPTLRSSSTSANTRPSPAPAFINPPTQDFT
SEIDLWNFSRSTSSLLASSEPLCHVSNTSV

FIGURE 31 (Continued)

## SEQ ID NO: 359; ZmNAM2 Zea mays NAM2 homologue ZM02LC45780_CORN

tgacaacctgttactggatcattgtgattttagcgcgaatagcacgagcgcaaaacgat
atcaggtacttacaattgtttgcaaagtgacgaagaacatacaaggtgacatgaccacaa
ttgtttgcagtgttgacacgagtgggctcacacaatagacaatacatatgtcgcacttcg
atcccgtcctcttaacaaccagagcacaggaccgcatttaccatggggtataccgtgtt
cgatatcaagaggccgcggtgcggtgctttgaccggctagttgtcggattgcatgctcct
gagcatgttcaggatggagaggaacaggttcaggatgtcgaggtacagcccaaccgacgc
ccagatgtactcgtcgtaggtgtggcgcttgatcaggttctcggtgtcgtacaggatgaa
gcctgagaagaccagagcccctagaccaccgaacaagcccaccgacacgggtcacagtgg
gaagaaaacctgcagcgcagaattccacacagaaccaaataaaagtttattgtgcaagca
gtggattgctcgtctggtaacaaagcatcattcgcggatatacagagcgcgtttctttca
gagcttgtcgtaggcattacctgaagaaagctagttaggacgaggatagtaagcgcggaa
gacaggataggccccaggtacccgaattccttgcccttctttgacgcccagaaagcatac
gcagtcagagaaaccaccacgccagccgtcagcactaaagcctccagaacgattttccct
tgggtgttagcacaagccacgccgatgctgaagctcaagcacaacgtgaacagacccagg
aaaacggaattgtgtgggtgcttgtgctgataatgatacaatgggatcatcaggatgaag
ggcaggacggcgagcacgagcgcgaggcccggggagtcggagagcgtggcgttgagggtg
gggtggagaacggtgagggcggagacggcggtggtgaggagcagctgcgcagcgaggatg
ccgtagaccttgcggacgaagccccatcggagggcgctctccccgcgcgagatccccggg
tacagcgtctccccggtcccggcctccaggtccaccaccgacgcctccaccttctccttc
atctccggcgcgcggcggtaccccgccggcgcgaggggctgcatctccgccaccgatgcc
atctctctctccgacgggggttgggtgcggtgcggtgcggggaaggggaaacccacgcgt
ccgcccacgcgtccgcccacgcgtccgcccacgcgtccgcccacgcgtccgcccacgcgt
ccgcccacgcgtccgcccacgcgtccgcggacgcgtgggatcgtcttcctcccctcacat
cctctggcctctggtcctccaccgtcctgctagccagagctgctcttgtacgcgcagctg
gcctctgtgcatatcaccagcacaccgcgcaggggaaggaaattaacaagagaaaaggca
aggagagcaggcaggcaaggaagctgcagaagccaaggaaggagaaggaggatcatcaat
gagcatctcggtgaacgggcagtcgtgcgtgccgccggggttccgcttccaccccacgga
ggaggagctgctcaactactacctccgcaagaaggtggcctcccaggagatcgacctcga
cgtcatccgcgacgtcgacctcaacaagctcgagccatgggacatccaagagaaatgcaa
gatcgggtcgggtccccagaacgactggtacttcttcagccacaaggacaagaagtaccc
gacggggacgcgcaccaaccgcgccacggccgccgggttctggaaggccaccggccgcga
caaggccatctacaacgccgtcaagcgcatcggcatgcgcaagacgctcgtcttctacaa
gggccgcgcgccgcacggccagaagtccgactggatcatgcacgagtaccgcctcgacga
ccccgctgctgctgctgctgctggatccggtgatgccgtggccaacgacgacgcagccgc
cacggctgctgctgctgccgccgcgtcgtcggacggcgggcaggaggacggctgggtggt
gtgcagggtgttcaagaagaagcaccaccacaaggagtcaggtgggggcgggggcaacaa
gcacggcagcagtaacagcgagcatgggcacggcggcgccggcaaggcatcggctgcggc
tgcggctgcggcgcaccagcaccagcaccatggaggcctgcagtactcctccagcgacga
ggcgctggaccagatcctgcagtacatgggcaggtcgtgcaagcaggagcacgagctggt
gtcgccggcgccggcgccgccgggacgggcggcggcgtccaggtacctccggcccatcga
gaccgttctgggcgggcacgcgttcatgaagcttcccgcgctcgagagcccgtcccagcg
ccaagcccaccaccaggagcgcgcgagtacgccgccgccggctgggacgacgacgacgcg
ctggaccgcctcgccgcctacgaccacctcaacggcctctccaacgacgacgcgtccaag
aacatggccgcgttcttcgacgtcgagcctagcgccgccgccgccgccgccgtggacggc

FIGURE 31 (Continued)

gacctgtggagcctcgcacggtccgtgtcggcgctgcacgcggacttgaccatgaacaac
gtctagcttctgggtcccgaccaacaacaacagggccccgaatccccgatacacatccat
atggcgtgtgcacgcatgcatgcatgcattgcatgccgcgcgcaccactaaccactcgac
cagcatgcatgcatacgtgcgcaccccaccggcgcccgcggccgctagtgcgtcgtgctc
ccgtagtgcgtgcatgcatggccgccgcacgtacgtattgcacgctcgctcgcgcgtacg
tacgtacgtgcgcgaataagctagctagccctaggatcggaaacatatgtatgcatccat
tgcatggccggatatacatcaggagctgctgccgctggtgtatgtccgccgccgtccaaa
ctccaaagtgagctgagatcgatcgatcgagctcgctcgctccaggtgtgcagtacgtac
gtacgtaagcgcctgtgtatgtgtaagcagacagcgtggtagtagctctagctagtagaa
catacttacacacactatatactgcataccgtttgtacctatcatatatagattactact
gtattattgcaaaccgctttgggggttttaaaaaaaaaaaaaaaa

**SEQ ID NO: 360; ZmNAM2 Zea mays NAM2 homologue ZM02LC45780_CORN, deduced protein sequence**

MSISVNGQSCVPPGFRFHPTEEELLNYYLRKKVASQEIDLDVIRDVDLNKLEPWDIQEKC
KIGSGPQNDWYFFSHKDKKYPTGTRTNRATAAGFWKATGRDKAIYNAVKRIGMRKTLVFY
KGRAPHGQKSDWIMHEYRLDDPAAAAAAGSGDAVANDDAAATAAAAAAASSDGGQEDGWV
VCRVFKKKHHHKESGGGGGNKHGSSNSEHGHGGAGKASAAAAAAAHQHQHHGGLQYSSSD
EALDQILQYMGRSCKQEHELVSPAPAPPGRAAASRYLRPIETVLGGHAFMKLPALESPSQ
RQAHHQERASTPPPAGTTTTRWTASPPTTTSTASPTTTRPRTWPRSSTSSLAPPPPPPWT
ATCGASHGPCRRCTRT

**SEQ ID NO: 361, GmNAM2 Glycine max NAM2 homologue 50450638_SOYBEAN**

ggcattggatcatttgtggcttgtaccgaactctgaatgccggagatgaagatgagcata
tgtgtgaatggagaatcccaagtccctccaggcttccggtttcatccaaccgaagaggaa
ctgttgcagtactatttgaggaagaaggtgtccaacgagaagatcgacctcgacgtgatt
cgcgatgttgatctcaacagactcgaaccatgggacatacaagagatgtgcaaaatagga
agcagcccacaaaacgattggtacttgttcagccacaaggacaagaagtaccccacggga
agccgcaccaaccgcgccatcattgttgggttctggaaggccacggggcgcgacaaggtc
atatatagcaacgggaagataattggaatgagaaaaacattggttttctacaaagggcgt
gcccccaatggccaaaagtctgattggatcatgcatgaatacaggctagacgacattaat
aacaccaatgagatggaacacgggtgggtggtctgtagagttttcaagaagaagaatgtc
cctctcaaaaccctagatagcccaaaatccatgaccatgaatatgtttacagaaagcgac
tactgtggaggcttcactaactgggaatcccttgatcagcttgtggcatcacaactgaat
ggacaaactgagacatattgcagtgatttgcaattccccgcatttctatcatcctcatac
attgctacaacaacacacattgctcccacaatacaggattaccccagctacgacattgac
ctgtggaaccactgtgcttcgcgcgtgtccaacactccaaatgtaacactctaatcaaat
ccttcttttaatttcttcccatgtcagattattactactattaaatcatccctaaaatat
tcattttcaaaaaaaaaaaaaaa

FIGURE 31 (Continued)

**SEQ ID NO: 362; GmNAM2 Glycine max NAM2 homologue 50450638_SOYBEAN, deduced protein sequence**

MPEMKMSICVNGESQVPPGFRFHPTEEELLQYYLRKKVSNEKIDLDVIRDVDLNRLEPWD
IQEMCKIGSSPQNDWYLFSHKDKKYPTGSRTNRAIIVGFWKATGRDKVIYSNGKIIGMRK
TLVFYKGRAPNGQKSDWIMHEYRLDDINNTNEMEHGWVVCRVFKKKNVPLKTLDSPKSMT
MNMFTESDYCGGFTNWESLDQLVASQLNGQTETYCSDLQFPAFLSSSYIATTTHIAPTIQ
DYPSYDIDLWNHCASRVSNTPNVTL

**SEQ ID NO: 363; ZmNAM2 Zea mays NAM2 homologue ZM06LC18271_CORN**

ggtcgtctaaaaaagcagcagctgctcttctcttctgctagcagctttatttgcctgcct
ccctaccggcgagggaggtacgtgcgccgatcgagaagcagcagcagcaattaataacta
aacatggatcaggcggcggaggagtcttgtgttcccccaggcttcaggttccaccctacg
gaggaagaactggtgggctactaccttgccaggaaggtggcctcccagaggatcgacctc
gacatcatccaggaggtggatctctacaggatagagccatgggatctgcaagagcggtgc
aagccgcagcacgccggcggtgggcacgacgagcagacacaggagtggtacttcttcagc
tacaaggaccgcaagtaccccagcgggacgaggacgaaccgcgccacggcggccggcttc
tggaaggccaccggcagggacaaaccggtgctgtcgtcgtccaccaggaccaccaggtgt
agcagggtcatcggcatgaggaagacgctggtgttctacaagggcagggcacccaacggc
aggaagagcgactggatcatgcacgagtaccgactccaatccaacgagcacgcaccggcg
caggaggaaggctgggtggtgtgccgcgctttccagaagcctatccccaaccagcggccc
ttcttccccaccagctacgccggctactacgaccacaacctctcaacgacggcacggctg
cacgtagacggcgaccgccatttcctggcgggctcatcagcagcagcagcagcactgcta
cagcagccaccagggcttgcaggcagcagcttcccgcagctgtactccgacgacgacttg
gagtccaagaagcagctgttgagtatcccgccgctggagagccccactgccatggcctgc
tgttccgacgccggcggctacgcgcagagaagcagctacgatgaacatgagatgatgatg
atgatgatccagcagggaggaggaggaggagagcaagctgcagccgccatcgactggaac
tttctggacagcctgctgtccgcgacgtcgcaactccatgggccctcgggatccttgttg
cagtgacctccggccatcgtcgatcgatccatctttctttgatccagcggagaacattat
tcattcatcggcattatacaatatatttattagttgcatacacacacagagtatacgtat
atgctaataattcagatgtgttcatacacacttccattgtcatgtgtagttcgttgcgac
ttacattacagactgccttagatgattcaggtatactgtatacaacatgatgcacagtat
agtaggagtaagtgcaccagtctcagcagctggtagctagctagctatgtaaaattcctc
cttaattacagctatgtaacatatatatacagttattattacttactgatatgatctatc
tatcaaaaaaaaaaa

**SEQ ID NO: 364; ZmNAM2 Zea mays NAM2 homologue ZM06LC18271_CORN, deduced protein sequence**

MDQAAEESCVPPGFRFHPTEEELVGYYLARKVASQRIDLDIIQEVDLYRIEPWDLQERCK
PQHAGGGHDEQTQEWYFFSYKDRKYPSGTRTNRATAAGFWKATGRDKPVLSSSTRTTRCS
RVIGMRKTLVFYKGRAPNGRKSDWIMHEYRLQSNEHAPAQEEGWVVCRAFQKPIPNQRPF
FPTSYAGYYDHNLSTTARLHVDGDRHFLAGSSAAAAALLQQPPGLAGSSFPQLYSDDDLE
SKKQLLSIPPLESPTAMACCSDAGGYAQRSSYDEHEMMMMMIQQGGGGGEQAAAAIDWNF
LDSLLSATSQLHGPSGSLLQ

FIGURE 31 (Continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003007699 A
- EP 2005003297 A
- US 5811238 A
- US 6395547 A
- WO 2004070039 A
- WO 2004065596 A
- US 4962028 A
- WO 0114572 A
- WO 9514098 A
- WO 9412015 A
- US 5565350 A, Kmiec
- WO 9322443 A, Zarling
- WO 0015815 A
- EP 1198985 A1
- US 5164310 A


### Non-patent literature cited in the description


- **WANG et al.** *Planta,* 2003, vol. 218, 1-14
- **RIECHMANN et al.** *Science,* 2000, vol. 290, 2105-2109
- **NAKANO et al.** *Plant Physiology,* 2006, vol. 140, 411-432
- **WANG et al.** *Plant Molecular Biology,* 2004, vol. 58, 183-192
- **TANG et al.** *Plant Cell Rep.,* 2007, vol. 26, 115-124
- **YI et al.** *Plant Physiol.,* 2004, vol. 136, 2862-2874
- **JUNG et al.** *Planta,* 26 August 2006
- **NAKANO et al.** *Plant Physiol.,* 2006, vol. 140, 411-432
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **NITZ et al.** *Plant Sci,* 2001, vol. 161 (2), 337-346
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70

- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **RABBANI et al.** *Plant Physiol,* 2003, vol. 133, 1755-1767
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- Current Protocols in Molecular Biology
- **OOKA et al.** *DNA Research,* 2003, vol. 10, 239-247
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **SAMBROOK.** Molecular Cloning: a laboratory manual. Cold Spring Harbor Laboratory Press, 2001
- Current Protocols in Molecular Biology. Current Protocols, vol. 1, 2

- **R.D.D. CROY.** Plant Molecular Biology Labfax. BIOS Scientific Publications Ltd (UK, 1993
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659

SEQUENCE LISTING

<110> CropDesign N.V.
Crop Functional Genomics Center

<120> Plants having enhanced yield-related traits and a method for making the same

<130> PF58338-EP3

<160> 364

<170> PatentIn version 3.3

<210> 1
<211> 1753
<212> DNA
<213> Oryza sativa

<400> 1
```
atccagatgc gcttgcactt gcacttgcat gcagttcatt gctctagcta taggctatag     60

ctactataca ctcatgagaa tctgagattg ggctgaaaat tgatgagcat cgatctgaag    120

aagacatata tatagctgat gatgatatag ctttttctgc atatgcttgt tttgatctgc    180

agccaatcga tcgaggcata gagagtcacc aggcaatcaa attccaacca tccatcagct    240

agatatatat acagcaaaga tcacgatact cctagctagc tagctagaag aattgatcgg    300

tcgatcggag gaaatagcaa tggatcggca tgaggaggag gcaggagagt ctccatgtgt    360

gccgccgggg ttcaggtttc acccgacgga ggaggagctg gtgggctact acctcgccag    420

gaaggtggcc tcccagaaga tcgatctcga catcatccag gagctcgatc tctacaggat    480

cgagccatgg gatctgcaag agcgttgcaa gtacggtggg catggcggcg atgagcagac    540

ggagtggtac ttcttcagct acaaggaccg caagtacccc agcgggacga ggaccaaccg    600

cgccacggcg gcgggcttct ggaaggccac cggccgcgac aagccggtgc tctcgtcgcc    660

gtcgacgagg gtgatcggga tgaggaagac gctggtgttc tacaagggtc gcgcccccaa    720

cggccggaag accgactgga tcatccacga gtaccgcctc caatcaaacg aacacgcccc    780

tactcaggag gaaggttggg tggtttgccg cgcgtttcag aagccgatgc ccaaccagca    840

gcagcacagg ctgtcctacg gctgcatccc cggcagctac ggcgccggag cctacgccgc    900

cgtccccgac aactacagct tgctgctgca ccacgacaac cctagcttcg ccgggcggcc    960

attgatgagc gccgctgcct cagctctctt cgccaacaac aacaataaca gcgtcgtcga   1020

ccacagcaac attctgagtt cagagtccaa gcttcacttc tccgacatga tgccgcctct   1080

ggagagcccc accatcgtcg acggcgaggg ctacgtctcg caggctagca gctgcgtcga   1140

cgtcgatcag caagccggca tcgtcgactg gaacctgctc accagcttgc tgccgccgcc   1200

ggcgcatcag ctcttccacc acctgccttc cgcttctagc tccaagaaca gcaacaacat   1260

ttcttcgtca ggcttcatcg atgaccgaga ctgatcgatc gatccagatc gattattatc   1320

aattgacaat tcattcatca tatatatgca tgaattctta caaatacgca caattaaacg   1380
```

```
atcgagtgta ctattaatta gttgattact ccgtctccat gatgtataca ttaatttgac   1440

cactggccat catcaggcca tctgcatgct tactatctag cttatgtcag tacacggttg   1500

ttagttcatt cttaattaat tagctactag tgtcagtgtg tgtatctatc ggttgttcgt   1560

cttttgacct cattgctgag agaggtttgt aactgatgat gattaagtta gctagcattt   1620

aattaggtgc tatatataca catatatatg cactatcact atatacatca tatatatgta   1680

tacatatatc gttgtctgct taatgtgtac accacctctc tgcagtatat atagtattga   1740

tcaattaatt tgt                                                      1753


<210>  2
<211>  324
<212>  PRT
<213>  Oryza sativa

<400>  2

Met Asp Arg His Glu Glu Glu Ala Gly Glu Ser Pro Cys Val Pro Pro
1               5                   10                  15


Gly Phe Arg Phe His Pro Thr Glu Glu Glu Leu Val Gly Tyr Tyr Leu
            20                  25                  30


Ala Arg Lys Val Ala Ser Gln Lys Ile Asp Leu Asp Ile Ile Gln Glu
        35                  40                  45


Leu Asp Leu Tyr Arg Ile Glu Pro Trp Asp Leu Gln Glu Arg Cys Lys
    50                  55                  60


Tyr Gly Gly His Gly Gly Asp Glu Gln Thr Glu Trp Tyr Phe Phe Ser
65                  70                  75                  80


Tyr Lys Asp Arg Lys Tyr Pro Ser Gly Thr Arg Thr Asn Arg Ala Thr
                85                  90                  95


Ala Ala Gly Phe Trp Lys Ala Thr Gly Arg Asp Lys Pro Val Leu Ser
            100                 105                 110


Ser Pro Ser Thr Arg Val Ile Gly Met Arg Lys Thr Leu Val Phe Tyr
        115                 120                 125


Lys Gly Arg Ala Pro Asn Gly Arg Lys Thr Asp Trp Ile Ile His Glu
    130                 135                 140


Tyr Arg Leu Gln Ser Asn Glu His Ala Pro Thr Gln Glu Glu Gly Trp
145                 150                 155                 160


Val Val Cys Arg Ala Phe Gln Lys Pro Met Pro Asn Gln Gln Gln His
                165                 170                 175
```

Arg Leu Ser Tyr Gly Cys Ile Pro Gly Ser Tyr Gly Ala Gly Ala Tyr
180 185 190

Ala Ala Val Pro Asp Asn Tyr Ser Leu Leu Leu His His Asp Asn Pro
195 200 205

Ser Phe Ala Gly Arg Pro Leu Met Ser Ala Ala Ala Ser Ala Leu Phe
210 215 220

Ala Asn Asn Asn Asn Asn Ser Val Val Asp His Ser Asn Ile Leu Ser
225 230 235 240

Ser Glu Ser Lys Leu His Phe Ser Asp Met Met Pro Pro Leu Glu Ser
245 250 255

Pro Thr Ile Val Asp Gly Glu Gly Tyr Val Ser Gln Ala Ser Ser Cys
260 265 270

Val Asp Val Asp Gln Gln Ala Gly Ile Val Asp Trp Asn Leu Leu Thr
275 280 285

Ser Leu Leu Pro Pro Pro Ala His Gln Leu Phe His His Leu Pro Ser
290 295 300

Ala Ser Ser Ser Lys Asn Ser Asn Asn Ile Ser Ser Ser Gly Phe Ile
305 310 315 320

Asp Asp Arg Asp

<210> 3
<211> 1346
<212> DNA
<213> Oryza sativa

<400> 3
aatttgcttc tcatctttct tctctactcc tctcaaatac aagagctcac actgctccct 60

agcttccttc ttcatccctt gaactagttg tcagctactg accagcctga agatagatgc 120

tagtttgtgt agagaattcg atcttctgag gcgtatggtg atcatggagt catgtgtgcc 180

tcctgggttt aggttccacc ccaccgacga ggagctcgtc ggctactacc tccggaagaa 240

ggtggcctcg cagaagatcg acctcgacgt catccgcgac gtcgacctct accgcatcga 300

gccatgggat ctccaagagc attgcaggat agggtacgag gagcagagcg agtggtactt 360

cttcagctac aaggacagga agtacccgac ggggacgagg acgaacaggg cgacgatgac 420

ggggttctgg aaggcgacgg ggagggacaa ggcggtgcgt gagaggagca ggctcatcgg 480

gatgaggaag acgctcgtct tctacaaggg cagggcaccc aacggccaca agaccgactg 540

gattgtccac gagtaccgcc tcgagtccga cgagaacgcc ccgcctcagg aagaaggctg 600

ggtggtgtgc cgcgcgttca agaagcgaac catgcagccg ccgcggagct ccatcggagc 660

atgggaggcg agctactcct accacgaccc cgccgtcttc gtcggcggcg gggaacactt 720

caagcaagag gccgcggccg agctggacgg cgtcgccgcc gccgccggag ctaacgcctt 780

cctgcggtac tccacccgcc tggccgagct cccgcagctg gagagcccgc cgctgccaag 840

ccaggggagc caggcggcct cggccgtcgt cgacggcgag gaagataacg ccgattctag 900

caggcgccct ggcggcggcg gcggcgccgc cgccgcggtg accacggact ggagggcgtt 960

cgacaagttc gtcgcgtccc agctcagccc cgaggagcag cacacctgcc gggccaccga 1020

cgacgacgac atggcagcgc tgctgctcct cgacggcggc gggcaggagg acgacgccgg 1080

gaggtggctg ggctccgccg ggttgctgag cgccgtggcg gccgacgcga cgacggactg 1140

cggcctcggc accagctgcg tgcctggcga catcaactga ttcagggcca agccgatcga 1200

gctctcactt ctccttagca taaagtgtga gatatctacg aattgtatgg atggctatat 1260

atacgtacgc ctatacatat gtagctggtc aaagcatcgt ttcagtttca gtttcagttc 1320

ttggaagtca atggatgttg accatt 1346

<210> 4
<211> 341
<212> PRT
<213> Oryza sativa

<400> 4

Met Val Ile Met Glu Ser Cys Val Pro Pro Gly Phe Arg Phe His Pro
1 5 10 15

Thr Asp Glu Glu Leu Val Gly Tyr Tyr Leu Arg Lys Lys Val Ala Ser
20 25 30

Gln Lys Ile Asp Leu Asp Val Ile Arg Asp Val Asp Leu Tyr Arg Ile
35 40 45

Glu Pro Trp Asp Leu Gln Glu His Cys Arg Ile Gly Tyr Glu Glu Gln
50 55 60

Ser Glu Trp Tyr Phe Phe Ser Tyr Lys Asp Arg Lys Tyr Pro Thr Gly
65 70 75 80

Thr Arg Thr Asn Arg Ala Thr Met Thr Gly Phe Trp Lys Ala Thr Gly
85 90 95

Arg Asp Lys Ala Val Arg Glu Arg Ser Arg Leu Ile Gly Met Arg Lys
100 105 110

Thr Leu Val Phe Tyr Lys Gly Arg Ala Pro Asn Gly His Lys Thr Asp
115 120 125

Trp Ile Val His Glu Tyr Arg Leu Glu Ser Asp Glu Asn Ala Pro Pro
130 135 140

Gln Glu Glu Gly Trp Val Val Cys Arg Ala Phe Lys Lys Arg Thr Met
145 150 155 160

Gln Pro Pro Arg Ser Ser Ile Gly Ala Trp Glu Ala Ser Tyr Ser Tyr
165 170 175

His Asp Pro Ala Val Phe Val Gly Gly Gly Glu His Phe Lys Gln Glu
180 185 190

Ala Ala Ala Glu Leu Asp Gly Val Ala Ala Ala Ala Gly Ala Asn Ala
195 200 205

Phe Leu Arg Tyr Ser Thr Arg Leu Ala Glu Leu Pro Gln Leu Glu Ser
210 215 220

Pro Pro Leu Pro Ser Gln Gly Ser Gln Ala Ala Ser Ala Val Val Asp
225 230 235 240

Gly Glu Glu Asp Asn Ala Asp Ser Ser Arg Arg Pro Gly Gly Gly Gly
245 250 255

Gly Ala Ala Ala Ala Val Thr Thr Asp Trp Arg Ala Phe Asp Lys Phe
260 265 270

Val Ala Ser Gln Leu Ser Pro Glu Glu Gln His Thr Cys Arg Ala Thr
275 280 285

Asp Asp Asp Asp Met Ala Ala Leu Leu Leu Leu Asp Gly Gly Gly Gln
290 295 300

Glu Asp Asp Ala Gly Arg Trp Leu Gly Ser Ala Gly Leu Leu Ser Ala
305 310 315 320

Val Ala Ala Asp Ala Thr Thr Asp Cys Gly Leu Gly Thr Ser Cys Val
325 330 335

Pro Gly Asp Ile Asn
340

<210> 5
<211> 1101
<212> DNA
<213> Oryza sativa

<400> 5
atggaatcat gcgtccctcc tgggttcagg ttccacccca ccgacgagga gctcgtcggc 60

tactacctcc gcaagaaggt cgcctcccag aagatcgacc tcgacgtcat ccgcgacatc 120

gatctctacc gcatcgaacc ctgggatctc caagaacatt gtgggatcgg gtacgatgag 180

caaagcgagt ggtacttctt cagctacaag gacaggaagt acccgacggg gacgaggacg 240

aacagggcga caatggcggg gttctggaag gcgacgggga gggacaaggc ggtgcacgac 300

aagagcaggc tcatcggcat gaggaagaca ctcgtcttct ataagggcag ggcgcctaat 360

ggccagaaga ccgactggat catgcacgag taccgcctcg agaccgacga gaacgcgccg 420

ccacaggaag aaggatgggt ggtgtgccga gcattcaaga agaggacggc gtatccggcg 480

aggagcatgg tggagacgtg ggactactcc ttgcacgagc gcaacatcat gagcgccgcg 540

gcggcggcgg cgttcgccga tccgagcgcg gcgtacgcgc agatgaggcg gcagcacagg 600

agcgggcggt tcaagcagga ggcggagctg gacggcgccg ccacagccct cctccactac 660

tccagccacc tcgccgagct gccgcagctc gagagcccct ccgcggctgc ggcgccgctc 720

cagcccaacc cgagccagct ggccaccgcc ggcgaggacg acgactgcaa gggcgataat 780

ggcggtagga gggccaagaa agcccgcgcc gccggcgaca aggtggcgac gaccacggac 840

tggagagcgc tcgacaagtt cgtcgcgtcg cagcttagcc ccggggagtg tggcagcatg 900

gaggcaacgg cggaagccgc ggcggcggca gtcgccggtg tgagctcgcc gctggaccac 960

ggcgatgacg acatggcagc attgctgttt ctcaacagcg acgagagaga cgaggtcgac 1020

aggtggacgg ggttgctcgg ctccggcgcc ggcgcgagcg gcgtcgacgg cgacctcgga 1080

atctgtgtgt ttgacaaatg a 1101

<210> 6
<211> 366
<212> PRT
<213> Oryza sativa

<400> 6

Met Glu Ser Cys Val Pro Pro Gly Phe Arg Phe His Pro Thr Asp Glu
1 5 10 15

Glu Leu Val Gly Tyr Tyr Leu Arg Lys Lys Val Ala Ser Gln Lys Ile
20 25 30

Asp Leu Asp Val Ile Arg Asp Ile Asp Leu Tyr Arg Ile Glu Pro Trp
35 40 45

Asp Leu Gln Glu His Cys Gly Ile Gly Tyr Asp Glu Gln Ser Glu Trp
50 55 60

Tyr Phe Phe Ser Tyr Lys Asp Arg Lys Tyr Pro Thr Gly Thr Arg Thr
65 70 75 80

```
Asn Arg Ala Thr Met Ala Gly Phe Trp Lys Ala Thr Gly Arg Asp Lys
                85                  90                  95

Ala Val His Asp Lys Ser Arg Leu Ile Gly Met Arg Lys Thr Leu Val
            100                 105                 110

Phe Tyr Lys Gly Arg Ala Pro Asn Gly Gln Lys Thr Asp Trp Ile Met
        115                 120                 125

His Glu Tyr Arg Leu Glu Thr Asp Glu Asn Ala Pro Pro Gln Glu Glu
    130                 135                 140

Gly Trp Val Val Cys Arg Ala Phe Lys Lys Arg Thr Ala Tyr Pro Ala
145                 150                 155                 160

Arg Ser Met Val Glu Thr Trp Asp Tyr Ser Leu His Glu Arg Asn Ile
                165                 170                 175

Met Ser Ala Ala Ala Ala Ala Ala Phe Ala Asp Pro Ser Ala Ala Tyr
            180                 185                 190

Ala Gln Met Arg Arg Gln His Arg Ser Gly Arg Phe Lys Gln Glu Ala
        195                 200                 205

Glu Leu Asp Gly Ala Ala Thr Ala Leu Leu His Tyr Ser Ser His Leu
    210                 215                 220

Ala Glu Leu Pro Gln Leu Glu Ser Pro Ser Ala Ala Ala Ala Pro Leu
225                 230                 235                 240

Gln Pro Asn Pro Ser Gln Leu Ala Thr Ala Gly Glu Asp Asp Asp Cys
                245                 250                 255

Lys Gly Asp Asn Gly Gly Arg Arg Ala Lys Lys Ala Arg Ala Ala Gly
            260                 265                 270

Asp Lys Val Ala Thr Thr Thr Asp Trp Arg Ala Leu Asp Lys Phe Val
        275                 280                 285

Ala Ser Gln Leu Ser Pro Gly Glu Cys Gly Ser Met Glu Ala Thr Ala
    290                 295                 300

Glu Ala Ala Ala Ala Ala Val Ala Gly Val Ser Ser Pro Leu Asp His
305                 310                 315                 320

Gly Asp Asp Asp Met Ala Ala Leu Leu Phe Leu Asn Ser Asp Glu Arg
                325                 330                 335
```

Asp Glu Val Asp Arg Trp Thr Gly Leu Leu Gly Ser Gly Ala Gly Ala
            340                 345                 350

Ser Gly Val Asp Gly Asp Leu Gly Ile Cys Val Phe Asp Lys
        355                 360                 365

<210> 7
<211> 1113
<212> DNA
<213> Oryza sativa

<400> 7
atggacactt tctcccatgt gccacctggt ttccgctttc accctactga cgaggagctc 60

gtcgattact acctgaggaa gaaggtggca tcgaagaaaa tcgacctcga cgttataaaa 120

gacgtcgacc tgtacaaaat cgagccctgg gatctccaag agaagtgcaa gattggcatg 180

gaagaacaga atgactggta cttcttcagc cacaaagaca aaaagtaccc gacgggcacc 240

cgcaccaacc gggccacggg cgccggcttc tggaaggcga cggggaggga caagcccatc 300

tacgcccgca gctgcctcgt cgggatgagg aagacactcg tcttctacaa gggccgtgcc 360

cccaatggcc agaagtcgga ctggatcatg cacgagtacc gcctcgagac caacgaaaac 420

ggcaccacac ctgaggaagg atgggtggtc tgccgggtgt tcaagaagcg ggtggcgacg 480

gtgcggagaa tggccgacgg atcaccgtgc tggttcgatg accacggcgc cgtcggtgcg 540

ttcatgccgg acctcagctc gccgaggcag ctactgccgc accaccacca ccaccacccg 600

ggctcgtcgg cggcgctgta ccacggccac caccaccagc agctgcagca gatgtacggt 660

cactgcaagc cggagctgga gtaccaccac ctcctgccgc aggaggcctt cctgcagcat 720

cttcctcagc tggagagccc caagccgccg ccgccgcctc ctgcggcggc ggcctacatt 780

ggcggccacc tcggatcgtc gtcgtccacc gcccttacta ctcacgacga cgaagcctcc 840

ggctccgccg cgcagcagca gccgccgtcg ttggaggctg tttacatggc cggcgccggc 900

gtcggcatcg gcgtcgacgc gtcggtgacg gactggaggc tactggacaa gttcgtcgcg 960

tctcagctgc tcagcaagga gtccatgtcc agctacggat cccatccggc tcaggtgttt 1020

caggctgcag acggtggcaa gcatgaagag gctttggact acgcttcgac gtcggccggc 1080

tccggcggcg gcgaggctga tctgtggaaa tag 1113

<210> 8
<211> 370
<212> PRT
<213> Oryza sativa

<400> 8

Met Asp Thr Phe Ser His Val Pro Pro Gly Phe Arg Phe His Pro Thr
1               5                   10                  15

```
Asp Glu Glu Leu Val Asp Tyr Tyr Leu Arg Lys Lys Val Ala Ser Lys
            20                  25                  30

Lys Ile Asp Leu Asp Val Ile Lys Asp Val Asp Leu Tyr Lys Ile Glu
        35                  40                  45

Pro Trp Asp Leu Gln Glu Lys Cys Lys Ile Gly Met Glu Glu Gln Asn
    50                  55                  60

Asp Trp Tyr Phe Phe Ser His Lys Asp Lys Lys Tyr Pro Thr Gly Thr
65                  70                  75                  80

Arg Thr Asn Arg Ala Thr Gly Ala Gly Phe Trp Lys Ala Thr Gly Arg
                85                  90                  95

Asp Lys Pro Ile Tyr Ala Arg Ser Cys Leu Val Gly Met Arg Lys Thr
            100                 105                 110

Leu Val Phe Tyr Lys Gly Arg Ala Pro Asn Gly Gln Lys Ser Asp Trp
        115                 120                 125

Ile Met His Glu Tyr Arg Leu Glu Thr Asn Glu Asn Gly Thr Thr Pro
    130                 135                 140

Glu Glu Gly Trp Val Val Cys Arg Val Phe Lys Lys Arg Val Ala Thr
145                 150                 155                 160

Val Arg Arg Met Ala Asp Gly Ser Pro Cys Trp Phe Asp Asp His Gly
                165                 170                 175

Ala Val Gly Ala Phe Met Pro Asp Leu Ser Ser Pro Arg Gln Leu Leu
            180                 185                 190

Pro His His His His His His Pro Gly Ser Ser Ala Ala Leu Tyr His
        195                 200                 205

Gly His His His Gln Gln Leu Gln Gln Met Tyr Gly His Cys Lys Pro
    210                 215                 220

Glu Leu Glu Tyr His His Leu Leu Pro Gln Glu Ala Phe Leu Gln His
225                 230                 235                 240

Leu Pro Gln Leu Glu Ser Pro Lys Pro Pro Pro Pro Pro Pro Ala Ala
                245                 250                 255

Ala Ala Tyr Ile Gly Gly His Leu Gly Ser Ser Ser Ser Thr Ala Leu
            260                 265                 270

Thr Thr His Asp Asp Glu Ala Ser Gly Ser Ala Ala Gln Gln Gln Pro
```

275 280 285

Pro Ser Leu Glu Ala Val Tyr Met Ala Gly Ala Gly Val Gly Ile Gly
290 295 300

Val Asp Ala Ser Val Thr Asp Trp Arg Leu Leu Asp Lys Phe Val Ala
305 310 315 320

Ser Gln Leu Leu Ser Lys Glu Ser Met Ser Ser Tyr Gly Ser His Pro
325 330 335

Ala Gln Val Phe Gln Ala Ala Asp Gly Gly Lys His Glu Glu Ala Leu
340 345 350

Asp Tyr Ala Ser Thr Ser Ala Gly Ser Gly Gly Gly Glu Ala Asp Leu
355 360 365

Trp Lys
370

<210> 9
<211> 1131
<212> DNA
<213> Oryza sativa

<400> 9
atggaagtgg aagcaaggat acacctcctg ttgatcaagc tagtcttcta taggagtgca 60

gaagagcgag aatcagcctc tgttcatatt aacttgctac cagttcggga gctatacttt 120

gatccgcgga agaagatgga cgcattctcc catgtcccgc caggttttcg tttccaccct 180

actgacgagg aactcgtgga ttactacctt aggaagaagg tagcactgaa gaagatagac 240

ttggacgtca taaaagatat tgatctgtac aaaattgagc cttgggacct acaagaacaa 300

tgcaagattg gaaacgagga gcagaacgag tggtacttct tcagccacaa ggacaagaag 360

tacccgacgg gcacacgcac caacagggcg accactgccg gcttttggaa ggccaccggg 420

agggacaagc cgatctatgt caagaactgc cttgttggga tgaggaagac gttggttttc 480

tacaggggcc gggctcccaa cggacagaag tcggactgga tcatgcacga gtatcgcttg 540

gagaccaacg aatacggagc tccccaagag gaaggatggg tggtctgcag ggtgttcaag 600

aagcgagtcg cggcggtgca aagggcggcc ggcgacggcg gcgactcacc tttctggttc 660

aacgagcacg tcgcgttcat ggcgccggcg ccaggcctcg actcgccgta ccatggccac 720

cgccagagcc acccttgcaa gctggaggta gagtatcacc accacctcct tcctcaggag 780

gcggcgccgt tcatgcacct cccaaggctg gagagcccca agctaccggc cgccgacatc 840

attgtcgcca ccgcagcgtc gtccgctctc cagccgtgcg gccacacgac ggcgcagcag 900

ctgcagctgc agatcgagcc ggtctacgtg acggccgatg cgtcggcagc agattggagg 960

```
gatcttgaca agctggtggc gtctcagttc ggccacggcg actcgactgc gaaggagccc   1020

agttactgca atccggtgca ggtgtttcag gtcgagggga agcaggagga tagcttggat   1080

tacgtgtcca cgtctgcctc ctgcggaggg gaggaggatt tgtggaaata g            1131


<210>  10
<211>  376
<212>  PRT
<213>  Oryza sativa

<400>  10

Met Glu Val Glu Ala Arg Ile His Leu Leu Leu Ile Lys Leu Val Phe
1               5                   10                  15

Tyr Arg Ser Ala Glu Glu Arg Glu Ser Ala Ser Val His Ile Asn Leu
            20                  25                  30

Leu Pro Val Arg Glu Leu Tyr Phe Asp Pro Arg Lys Lys Met Asp Ala
        35                  40                  45

Phe Ser His Val Pro Pro Gly Phe Arg Phe His Pro Thr Asp Glu Glu
    50                  55                  60

Leu Val Asp Tyr Tyr Leu Arg Lys Lys Val Ala Leu Lys Lys Ile Asp
65                  70                  75                  80

Leu Asp Val Ile Lys Asp Ile Asp Leu Tyr Lys Ile Glu Pro Trp Asp
                85                  90                  95

Leu Gln Glu Gln Cys Lys Ile Gly Asn Glu Glu Gln Asn Glu Trp Tyr
            100                 105                 110

Phe Phe Ser His Lys Asp Lys Lys Tyr Pro Thr Gly Thr Arg Thr Asn
        115                 120                 125

Arg Ala Thr Thr Ala Gly Phe Trp Lys Ala Thr Gly Arg Asp Lys Pro
    130                 135                 140

Ile Tyr Val Lys Asn Cys Leu Val Gly Met Arg Lys Thr Leu Val Phe
145                 150                 155                 160

Tyr Arg Gly Arg Ala Pro Asn Gly Gln Lys Ser Asp Trp Ile Met His
                165                 170                 175

Glu Tyr Arg Leu Glu Thr Asn Glu Tyr Gly Ala Pro Gln Glu Glu Gly
            180                 185                 190

Trp Val Val Cys Arg Val Phe Lys Lys Arg Val Ala Ala Val Gln Arg
        195                 200                 205
```

Ala Ala Gly Asp Gly Gly Asp Ser Pro Phe Trp Phe Asn Glu His Val
210 215 220

Ala Phe Met Ala Pro Ala Pro Gly Leu Asp Ser Pro Tyr His Gly His
225 230 235 240

Arg Gln Ser His Pro Cys Lys Leu Glu Val Glu Tyr His His His Leu
245 250 255

Leu Pro Gln Glu Ala Ala Pro Phe Met His Leu Pro Arg Leu Glu Ser
260 265 270

Pro Lys Leu Pro Ala Ala Asp Ile Ile Val Ala Thr Ala Ala Ser Ser
275 280 285

Ala Leu Gln Pro Cys Gly His Thr Thr Ala Gln Gln Leu Gln Leu Gln
290 295 300

Ile Glu Pro Val Tyr Val Thr Ala Asp Ala Ser Ala Ala Asp Trp Arg
305 310 315 320

Asp Leu Asp Lys Leu Val Ala Ser Gln Phe Gly His Gly Asp Ser Thr
325 330 335

Ala Lys Glu Pro Ser Tyr Cys Asn Pro Val Gln Val Phe Gln Val Glu
340 345 350

Gly Lys Gln Glu Asp Ser Leu Asp Tyr Val Ser Thr Ser Ala Ser Cys
355 360 365

Gly Gly Glu Glu Asp Leu Trp Lys
370 375

<210> 11
<211> 942
<212> DNA
<213> Medicago truncatula

<400> 11
atgatggagt catgtgtccc acctggattt cggttccacc caacggatgg agagcttgtt 60

ggttattatc taagaaagaa agtagcttct cacaagattg atcttgatgt tatcaaagag 120

atcgatctat atcgtattga accctgggat ctccaagaga gatgtagaat tgggaatgag 180

gagcaacatg agtggtattt ttttagtcac aaagataaga aatatccaac agggacgaga 240

acgaatagag ctacaatggc agggttctgg aaagcaaccg gaagagataa gtcggtgtat 300

gaacgaacaa aactgattgg aatgaggaag acactcgttt tctacaaagg aagagcacct 360

aatggacaga aaactgattg gatcatgcat gaatataggc ttgaaacagt tgaaaatgca 420

```
cctccacagg caagtgaaga aggttgggtt gtgtgcaaag cattcaagaa aaaaacaagt    480

gtccaagcaa aaacaaatga aagatgggat ccaagccact tacatgatga acaaacaagt    540

agcatcatct caagggtgga ccctattgac ctcatcttaa gacaaccaca gaggatttca    600

gctcaaaatt tcatgtacaa acaagagata gaagcagaag cagatacctt attaaggttc    660

atgcattcag aacaattggt acctcttcct caactacaaa gtccctcttt aacaaagagg    720

caaaactcaa tgccaataat atcagagaaa gtgactgatt ggagggatct tgacaagttt    780

gtggcttctc agttgagtca agaagatcat aggcatgaaa caagctcaga catgtcattg    840

ttcctacttc agagtagtaa ggatgatgaa gagaacaagt taagctcatt tttaactaca    900

aggtcagatt gtgacattgg aatatgtgta tttgaaaatt aa                        942


<210>  12
<211>  313
<212>  PRT
<213>  Medicago truncatula

<400>  12

Met Met Glu Ser Cys Val Pro Pro Gly Phe Arg Phe His Pro Thr Asp
1               5                   10                  15

Gly Glu Leu Val Gly Tyr Tyr Leu Arg Lys Lys Val Ala Ser His Lys
            20                  25                  30

Ile Asp Leu Asp Val Ile Lys Glu Ile Asp Leu Tyr Arg Ile Glu Pro
        35                  40                  45

Trp Asp Leu Gln Glu Arg Cys Arg Ile Gly Asn Glu Glu Gln His Glu
    50                  55                  60

Trp Tyr Phe Phe Ser His Lys Asp Lys Lys Tyr Pro Thr Gly Thr Arg
65                  70                  75                  80

Thr Asn Arg Ala Thr Met Ala Gly Phe Trp Lys Ala Thr Gly Arg Asp
                85                  90                  95

Lys Ser Val Tyr Glu Arg Thr Lys Leu Ile Gly Met Arg Lys Thr Leu
            100                 105                 110

Val Phe Tyr Lys Gly Arg Ala Pro Asn Gly Gln Lys Thr Asp Trp Ile
        115                 120                 125

Met His Glu Tyr Arg Leu Glu Thr Val Glu Asn Ala Pro Pro Gln Ala
    130                 135                 140

Ser Glu Glu Gly Trp Val Val Cys Lys Ala Phe Lys Lys Lys Thr Ser
145                 150                 155                 160
```

Val Gln Ala Lys Thr Asn Glu Arg Trp Asp Pro Ser His Leu His Asp
165 170 175

Glu Gln Thr Ser Ser Ile Ile Ser Arg Val Asp Pro Ile Asp Leu Ile
180 185 190

Leu Arg Gln Pro Gln Arg Ile Ser Ala Gln Asn Phe Met Tyr Lys Gln
195 200 205

Glu Ile Glu Ala Glu Ala Asp Thr Leu Leu Arg Phe Met His Ser Glu
210 215 220

Gln Leu Val Pro Leu Pro Gln Leu Gln Ser Pro Ser Leu Thr Lys Arg
225 230 235 240

Gln Asn Ser Met Pro Ile Ile Ser Glu Lys Val Thr Asp Trp Arg Asp
245 250 255

Leu Asp Lys Phe Val Ala Ser Gln Leu Ser Gln Glu Asp His Arg His
260 265 270

Glu Thr Ser Ser Asp Met Ser Leu Phe Leu Leu Gln Ser Ser Lys Asp
275 280 285

Asp Glu Glu Asn Lys Leu Ser Ser Phe Leu Thr Thr Arg Ser Asp Cys
290 295 300

Asp Ile Gly Ile Cys Val Phe Glu Asn
305 310

<210> 13
<211> 1414
<212> DNA
<213> Arabidopsis thaliana

<400> 13
atgaattcat tttcccacgt ccctccgggt tttagatttc acccgacaga tgaagaactt 60

gtagactact acctgaggaa aaaagtcgca tcgaagagaa tagaaattga tttcataaag 120

gacattgatc tttacaagat tgagccatgg gaccttcaag agttgtgcaa aattgggcat 180

gaagagcaga gtgattggta cttctttagc cataaagaca agaagtatcc cacagggact 240

cgaaccaata gagcaacaaa agcagggttt tggaaagcca ccggaagaga taaggctatc 300

tatttgaggc atagtctaat tggcatgagg aaaacacttg tgttttacaa gggaagagcc 360

ccaaatggac aaaagtctga ttggatcatg cacgaatacc gcttagaaac cgatgaaaac 420

ggaactcctc aggaagaagg atgggttgtg tgtagggttt tcaagaagag attggctgca 480

gttagacgaa tgggagatta cgactcatcc ccttcacatt ggtacgatga tcaactttct 540

tttatggcct ccgagctcga gacaaacggt caacgacgga ttctccccaa tcatcatcag 600

cagcagcagc acgagcacca acaacatatg ccatatggcc tcaatgcatc tgcttacgct 660

ctcaacaacc ctaacttgca atgcaagcaa gagctagaac tacactacaa ccacctggta 720

caacgaaatc atcttcttga tgaatctcat ttatcgttcc tccaacttcc tcaactagaa 780

agccctaaga ttcaacaaga taacagtaat tgcaactctc ttccttatgg aacaagcaac 840

atcgataata actcgagcca taatgctaac ttgcagcaat caaatatcgc gcatgaggaa 900

caattgaatc aaggaaatca gaacttcagc tctctataca tgaacagcgg caacgagcaa 960

gtgatggacc aagtcacaga ctggagagtt ctcgataaat ttgttgcttc tcagctaagc 1020

aacgaggagg ctgccacagc ttctgcatct atacagaata atgccaagga cacaagcaat 1080

gctgagtacc aagttgatga agaaaaagat ccgaaaaggg cttcagacat gggagaagaa 1140

tatactgctt ctacttcttc gagttgtcag attgatctat ggaagtgagc tgaaagagaa 1200

gacatataaa tgcatatata catatatata tatacgtaca cacgaacact aatcaagtgt 1260

agatgatgat gatggtacag atttatattt gctttgattg attcttacta cattattgaa 1320

cttatgtcat atgcatatat acattgcgta tctatgcata tttatacttg tactcaatat 1380

gattaaccat atataaactc taatctaaat gtaa 1414

<210> 14
<211> 395
<212> PRT
<213> Arabidopsis thaliana

<400> 14

Met Asn Ser Phe Ser His Val Pro Pro Gly Phe Arg Phe His Pro Thr
1 5 10 15

Asp Glu Glu Leu Val Asp Tyr Tyr Leu Arg Lys Lys Val Ala Ser Lys
20 25 30

Arg Ile Glu Ile Asp Phe Ile Lys Asp Ile Asp Leu Tyr Lys Ile Glu
35 40 45

Pro Trp Asp Leu Gln Glu Leu Cys Lys Ile Gly His Glu Glu Gln Ser
50 55 60

Asp Trp Tyr Phe Phe Ser His Lys Asp Lys Lys Tyr Pro Thr Gly Thr
65 70 75 80

Arg Thr Asn Arg Ala Thr Lys Ala Gly Phe Trp Lys Ala Thr Gly Arg
85 90 95

Asp Lys Ala Ile Tyr Leu Arg His Ser Leu Ile Gly Met Arg Lys Thr
100 105 110

```
Leu Val Phe Tyr Lys Gly Arg Ala Pro Asn Gly Gln Lys Ser Asp Trp
        115                 120                 125

Ile Met His Glu Tyr Arg Leu Glu Thr Asp Glu Asn Gly Thr Pro Gln
    130                 135                 140

Glu Glu Gly Trp Val Val Cys Arg Val Phe Lys Lys Arg Leu Ala Ala
145                 150                 155                 160

Val Arg Arg Met Gly Asp Tyr Asp Ser Ser Pro Ser His Trp Tyr Asp
                165                 170                 175

Asp Gln Leu Ser Phe Met Ala Ser Glu Leu Glu Thr Asn Gly Gln Arg
            180                 185                 190

Arg Ile Leu Pro Asn His His Gln Gln Gln Gln His Glu His Gln Gln
        195                 200                 205

His Met Pro Tyr Gly Leu Asn Ala Ser Ala Tyr Ala Leu Asn Asn Pro
    210                 215                 220

Asn Leu Gln Cys Lys Gln Glu Leu Glu Leu His Tyr Asn His Leu Val
225                 230                 235                 240

Gln Arg Asn His Leu Leu Asp Glu Ser His Leu Ser Phe Leu Gln Leu
                245                 250                 255

Pro Gln Leu Glu Ser Pro Lys Ile Gln Gln Asp Asn Ser Asn Cys Asn
            260                 265                 270

Ser Leu Pro Tyr Gly Thr Ser Asn Ile Asp Asn Asn Ser Ser His Asn
        275                 280                 285

Ala Asn Leu Gln Gln Ser Asn Ile Ala His Glu Glu Gln Leu Asn Gln
    290                 295                 300

Gly Asn Gln Asn Phe Ser Ser Leu Tyr Met Asn Ser Gly Asn Glu Gln
305                 310                 315                 320

Val Met Asp Gln Val Thr Asp Trp Arg Val Leu Asp Lys Phe Val Ala
                325                 330                 335

Ser Gln Leu Ser Asn Glu Glu Ala Ala Thr Ala Ser Ala Ser Ile Gln
            340                 345                 350

Asn Asn Ala Lys Asp Thr Ser Asn Ala Glu Tyr Gln Val Asp Glu Glu
        355                 360                 365
```

Lys Asp Pro Lys Arg Ala Ser Asp Met Gly Glu Glu Tyr Thr Ala Ser
370 375 380

Thr Ser Ser Ser Cys Gln Ile Asp Leu Trp Lys
385 390 395

<210> 15
<211> 1185
<212> DNA
<213> Arabidopsis thaliana

<400> 15
atgaattcgt tttcacaagt acctcctggc ttcagatttc atcctactga tgaagaactt 60

gtagactact acttgaggaa aaaagttgca tcaaagagaa tagaaatcga tatcatcaag 120

gatgttgatc tttacaagat tgagccatgt gatcttcaag agttatgcaa gataggaaac 180

gaagagcaga gcgaatggta cttctttagt cataaagaca agaagtatcc cacgggaact 240

cgaaccaata gagccacgaa agcaggattt tggaaagcca ctggaagaga caaggctata 300

tatataagac atagtcttat cggtatgagg aaaacacttg tgttttacaa aggaagagcc 360

ccaaatggtc agaaatccga ttggatcatg cacgaatatc gcttagaaac aagtgaaaat 420

ggaacccctc aggaagaagg atgggtagta tgtagggtat tcaagaagaa attggcagcg 480

acagtgagga aaatgggaga ttaccattca tcaccatcgc agcattggta cgatgatcag 540

ctctctttta tggcctccga gatcatttct agttctccac gacagtttct tcccaatcat 600

cattataacc gccaccatca ccagcagaca ttgccttgtg gcctcaatgc attcaacaac 660

aacaatccta acttgcaatg caagcaagag ctcgagttac attacaatca aatggtacaa 720

catcaacaac aaaaccatca tcttcgtgaa tctatgtttc tccagcttcc tcagctcgaa 780

agccctacca gtaattgcaa ttctgacaac aacaataaca caagaaatat tagtaacttg 840

cagaaatcat caaatatatc tcatgaggaa caattgcaac aagggaatca aagtttcagc 900

tctctgtatt acgatcaagg agtagagcaa atgactactg actggagagt tctcgataaa 960

tttgttgctt cacagcttag caatgatgaa gaggctgcag ccgtggtttc ttcttcttct 1020

catcaaaaca acgtcaagat tgacacgaga aacacgggtt atcatgtgat agatgaggga 1080

ataaatttgc cggagaatga ttctgaaagg gttgttgaaa tgggagaaga gtattcaaat 1140

gctcatgctg cttctacttc ttcaagttgt cagattgatc tctag 1185

<210> 16
<211> 394
<212> PRT
<213> Arabidopsis thaliana

<400> 16

Met Asn Ser Phe Ser Gln Val Pro Pro Gly Phe Arg Phe His Pro Thr
1 5 10 15

```
Asp Glu Glu Leu Val Asp Tyr Tyr Leu Arg Lys Lys Val Ala Ser Lys
            20                  25                  30

Arg Ile Glu Ile Asp Ile Ile Lys Asp Val Asp Leu Tyr Lys Ile Glu
        35                  40                  45

Pro Cys Asp Leu Gln Glu Leu Cys Lys Ile Gly Asn Glu Glu Gln Ser
    50                  55                  60

Glu Trp Tyr Phe Phe Ser His Lys Asp Lys Lys Tyr Pro Thr Gly Thr
65                  70                  75                  80

Arg Thr Asn Arg Ala Thr Lys Ala Gly Phe Trp Lys Ala Thr Gly Arg
                85                  90                  95

Asp Lys Ala Ile Tyr Ile Arg His Ser Leu Ile Gly Met Arg Lys Thr
            100                 105                 110

Leu Val Phe Tyr Lys Gly Arg Ala Pro Asn Gly Gln Lys Ser Asp Trp
        115                 120                 125

Ile Met His Glu Tyr Arg Leu Glu Thr Ser Glu Asn Gly Thr Pro Gln
    130                 135                 140

Glu Glu Gly Trp Val Val Cys Arg Val Phe Lys Lys Lys Leu Ala Ala
145                 150                 155                 160

Thr Val Arg Lys Met Gly Asp Tyr His Ser Ser Pro Ser Gln His Trp
                165                 170                 175

Tyr Asp Asp Gln Leu Ser Phe Met Ala Ser Glu Ile Ile Ser Ser Ser
            180                 185                 190

Pro Arg Gln Phe Leu Pro Asn His His Tyr Asn Arg His His His Gln
        195                 200                 205

Gln Thr Leu Pro Cys Gly Leu Asn Ala Phe Asn Asn Asn Asn Pro Asn
    210                 215                 220

Leu Gln Cys Lys Gln Glu Leu Glu Leu His Tyr Asn Gln Met Val Gln
225                 230                 235                 240

His Gln Gln Gln Asn His His Leu Arg Glu Ser Met Phe Leu Gln Leu
                245                 250                 255

Pro Gln Leu Glu Ser Pro Thr Ser Asn Cys Asn Ser Asp Asn Asn Asn
            260                 265                 270
```

Asn Thr Arg Asn Ile Ser Asn Leu Gln Lys Ser Ser Asn Ile Ser His
275 280 285

Glu Glu Gln Leu Gln Gln Gly Asn Gln Ser Phe Ser Ser Leu Tyr Tyr
290 295 300

Asp Gln Gly Val Glu Gln Met Thr Thr Asp Trp Arg Val Leu Asp Lys
305 310 315 320

Phe Val Ala Ser Gln Leu Ser Asn Asp Glu Glu Ala Ala Ala Val Val
325 330 335

Ser Ser Ser Ser His Gln Asn Asn Val Lys Ile Asp Thr Arg Asn Thr
340 345 350

Gly Tyr His Val Ile Asp Glu Gly Ile Asn Leu Pro Glu Asn Asp Ser
355 360 365

Glu Arg Val Val Glu Met Gly Glu Glu Tyr Ser Asn Ala His Ala Ala
370 375 380

Ser Thr Ser Ser Ser Cys Gln Ile Asp Leu
385 390

<210> 17
<211> 1095
<212> DNA
<213> Oryza sativa

<400> 17
atggatggat ctagtatgag cagcagcagc acgcagcagc aggcgcaggt tcctcctgga 60

tttcgtttcc acccgacgga cgaagagctg gtggattact accttcggaa gaaggtggcc 120

gcaagaagga ttgatctcaa tgtcatcaag gacgtcgatc tctacaagat tgagccatgg 180

gatctgcaag agcgttgccg gatcaatggc gggtcggcgg cggaggagca gaacgaatgg 240

tacttcttca gccacaagga caagaagtac ccgacgggga cgaggaccaa ccgtgcgacg 300

gcggccgggt tctggaaggc gacggggcga gacaagccca tctacgccac caagcagcac 360

agcctcctcg tgggcatgag gaagacgctc gtctactacc gcggccgcgc ccccaacggc 420

cacaagtccg actggatcat gcacgagtac cgcctcgaga ccaccgagac ggccccgccg 480

caggaagaag ggtgggtggt atgccgggtg ttcaagaaga gattacccac aacaagaaga 540

gattcagacc atgacgcacc ttgcggcagc tggtacgttg atgaagatgc accgggcgcc 600

ttcatgtctc cgatgatgat caccagatca tcaatattgc gcccacacca acaccacgcc 660

ggcatcacgc tgcaagagca acacctccac accacttaca agcacagaga cctcactact 720

aagattcagc agctccaagt ccctgcggca ggccatcatc tcctcaacac catgccccat 780

gacctggaga gttctacttc ctccttccat tctcttttgg tctcacctga tcaccaccaa 840

atcaacatgc accatgctca ggctgatccc ttctttgacg acatgcatgc agtcgatcaa 900

gccactacca ctgactggag agttcttgac aagtttgttg cctctcagct tagcaatgat 960

gctacaaaca agcctgcgga tcattatact gatgaaggag acattcttca ggtcagtgac 1020

aagcagcaag aggtggcagc cgccgattat gcgtccacat caacgtcaag cagccaaatt 1080

gatccatgga agtga 1095

<210> 18
<211> 364
<212> PRT
<213> Oryza sativa

<400> 18

Met Asp Gly Ser Ser Met Ser Ser Ser Ser Thr Gln Gln Gln Ala Gln
1 5 10 15

Val Pro Pro Gly Phe Arg Phe His Pro Thr Asp Glu Glu Leu Val Asp
20 25 30

Tyr Tyr Leu Arg Lys Lys Val Ala Ala Arg Arg Ile Asp Leu Asn Val
35 40 45

Ile Lys Asp Val Asp Leu Tyr Lys Ile Glu Pro Trp Asp Leu Gln Glu
50 55 60

Arg Cys Arg Ile Asn Gly Gly Ser Ala Ala Glu Glu Gln Asn Glu Trp
65 70 75 80

Tyr Phe Phe Ser His Lys Asp Lys Lys Tyr Pro Thr Gly Thr Arg Thr
85 90 95

Asn Arg Ala Thr Ala Ala Gly Phe Trp Lys Ala Thr Gly Arg Asp Lys
100 105 110

Pro Ile Tyr Ala Thr Lys Gln His Ser Leu Leu Val Gly Met Arg Lys
115 120 125

Thr Leu Val Tyr Tyr Arg Gly Arg Ala Pro Asn Gly His Lys Ser Asp
130 135 140

Trp Ile Met His Glu Tyr Arg Leu Glu Thr Thr Glu Thr Ala Pro Pro
145 150 155 160

Gln Glu Glu Gly Trp Val Val Cys Arg Val Phe Lys Lys Arg Leu Pro
165 170 175

Thr Thr Arg Arg Asp Ser Asp His Asp Ala Pro Cys Gly Ser Trp Tyr
180 185 190

```
Val Asp Glu Asp Ala Pro Gly Ala Phe Met Ser Pro Met Met Ile Thr
        195                 200                 205

Arg Ser Ser Ile Leu Arg Pro His Gln His His Ala Gly Ile Thr Leu
    210                 215                 220

Gln Glu Gln His Leu His Thr Thr Tyr Lys His Arg Asp Leu Thr Thr
225                 230                 235                 240

Lys Ile Gln Gln Leu Gln Val Pro Ala Ala Gly His His Leu Leu Asn
                245                 250                 255

Thr Met Pro His Asp Leu Glu Ser Ser Thr Ser Ser Phe His Ser Leu
            260                 265                 270

Leu Val Ser Pro Asp His His Gln Ile Asn Met His His Ala Gln Ala
        275                 280                 285

Asp Pro Phe Phe Asp Asp Met His Ala Val Asp Gln Ala Thr Thr Thr
    290                 295                 300

Asp Trp Arg Val Leu Asp Lys Phe Val Ala Ser Gln Leu Ser Asn Asp
305                 310                 315                 320

Ala Thr Asn Lys Pro Ala Asp His Tyr Thr Asp Glu Gly Asp Ile Leu
                325                 330                 335

Gln Val Ser Asp Lys Gln Gln Glu Val Ala Ala Ala Asp Tyr Ala Ser
            340                 345                 350

Thr Ser Thr Ser Ser Ser Gln Ile Asp Pro Trp Lys
        355                 360


<210>  19
<211>  1098
<212>  DNA
<213>  Arabidopsis thaliana

<400>  19
atggagccaa tggaatcttg tagcgttcct ccaggattta ggttccatcc gacggacgaa      60

gagcttgtcg ggtactatct aaggaagaaa atcgcatcgc aaaagattga tctcgacgtc     120

atcagagaca tcgatctcta cagaatagaa ccatgggatc tacaagaaca atgtcgaatc     180

ggttatgagg aacaaaatga atggtatttt tttagtcaca aggacaagaa atatccaacg     240

gggacaagaa ctaatagagc gaccatggct ggattttgga aagccacggg aagagacaaa     300

gctgtttacg acaaaacaaa actaattggt atgaggaaaa cacttgtgtt ctacaaagga     360

cgtgcaccta atggcaagaa atccgattgg atcatgcatg agtaccggct cgagtcagat     420
```

gagaatgcac cgcccccagga agaaggatgg gtggtttgta gagcattcaa aaaaagagct 480

acagggcaag ccaagaacac ggaaacttgg agctcaagtt acttttacga tgaagttgca 540

ccgaatggag ttaactcggt tatggacccc attgattaca tatctaagca gcaacataac 600

atttttggga aaggtttgat gtgtaagcaa gaactagaag gaatggttga tggtataaac 660

tatatacaat cgaatcaatt cattcagctc ccacaactcc aaagcccttc tctcccgctg 720

atgaaaagac cttcaagctc gatgtccata acatcaatgg ataacaatta caactataaa 780

ctcccattag cggatgaaga aagcttcgag tcattcataa gaggagagga tagaaggaag 840

aagaaaaagc aagtaatgat gacgggaaat tggagagagt tagacaagtt tgttgcttca 900

caacttatga gccaagaaga caatggaact tcaagtttcg caggtcatca tatagttaat 960

gaagataaaa acaacaatga tgtggagatg gattcgtcaa tgtttttgag cgaaagagaa 1020

gaagaaaaca ggttcgtcag tgaattcttg agtacaaact cggattatga tattgggatt 1080

tgcgtatttg ataattga 1098

<210> 20
<211> 365
<212> PRT
<213> Arabidopsis thaliana

<400> 20

Met Glu Pro Met Glu Ser Cys Ser Val Pro Pro Gly Phe Arg Phe His
1 5 10 15

Pro Thr Asp Glu Glu Leu Val Gly Tyr Tyr Leu Arg Lys Lys Ile Ala
20 25 30

Ser Gln Lys Ile Asp Leu Asp Val Ile Arg Asp Ile Asp Leu Tyr Arg
35 40 45

Ile Glu Pro Trp Asp Leu Gln Glu Gln Cys Arg Ile Gly Tyr Glu Glu
50 55 60

Gln Asn Glu Trp Tyr Phe Phe Ser His Lys Asp Lys Lys Tyr Pro Thr
65 70 75 80

Gly Thr Arg Thr Asn Arg Ala Thr Met Ala Gly Phe Trp Lys Ala Thr
85 90 95

Gly Arg Asp Lys Ala Val Tyr Asp Lys Thr Lys Leu Ile Gly Met Arg
100 105 110

Lys Thr Leu Val Phe Tyr Lys Gly Arg Ala Pro Asn Gly Lys Lys Ser
115 120 125

```
Asp Trp Ile Met His Glu Tyr Arg Leu Glu Ser Asp Glu Asn Ala Pro
    130                 135                 140

Pro Gln Glu Glu Gly Trp Val Val Cys Arg Ala Phe Lys Lys Arg Ala
145                 150                 155                 160

Thr Gly Gln Ala Lys Asn Thr Glu Thr Trp Ser Ser Ser Tyr Phe Tyr
                165                 170                 175

Asp Glu Val Ala Pro Asn Gly Val Asn Ser Val Met Asp Pro Ile Asp
            180                 185                 190

Tyr Ile Ser Lys Gln Gln His Asn Ile Phe Gly Lys Gly Leu Met Cys
        195                 200                 205

Lys Gln Glu Leu Glu Gly Met Val Asp Gly Ile Asn Tyr Ile Gln Ser
    210                 215                 220

Asn Gln Phe Ile Gln Leu Pro Gln Leu Gln Ser Pro Ser Leu Pro Leu
225                 230                 235                 240

Met Lys Arg Pro Ser Ser Ser Met Ser Ile Thr Ser Met Asp Asn Asn
                245                 250                 255

Tyr Asn Tyr Lys Leu Pro Leu Ala Asp Glu Glu Ser Phe Glu Ser Phe
            260                 265                 270

Ile Arg Gly Glu Asp Arg Arg Lys Lys Lys Lys Gln Val Met Met Thr
        275                 280                 285

Gly Asn Trp Arg Glu Leu Asp Lys Phe Val Ala Ser Gln Leu Met Ser
    290                 295                 300

Gln Glu Asp Asn Gly Thr Ser Ser Phe Ala Gly His His Ile Val Asn
305                 310                 315                 320

Glu Asp Lys Asn Asn Asn Asp Val Glu Met Asp Ser Ser Met Phe Leu
                325                 330                 335

Ser Glu Arg Glu Glu Glu Asn Arg Phe Val Ser Glu Phe Leu Ser Thr
            340                 345                 350

Asn Ser Asp Tyr Asp Ile Gly Ile Cys Val Phe Asp Asn
        355                 360                 365

<210>  21
<211>  1465
<212>  DNA
<213>  Arabidopsis thaliana
```

<400> 21
ctttctcttc acattcaccg aaacttataa ccaagatcaa tatcagttct ctctctcgat 60

gaacatcatc ttctacacag ccatctttct ttgacttctt cttcttcttc ttaatataac 120

ggctcgtttc ttttgtttcc aaacgagtaa atagtgtcct atacacatat ctataattcc 180

acaggttgaa gaaaagaaat aatggaatcg gtggatcaat catgtagtgt tcctccggga 240

ttcagattcc atccaacaga tgaagagctc gttggttact atttaaggaa aaaagttgca 300

tcgcaaaaga tcgatcttga tgtcataaga gatattgatc tctacagaat cgaaccatgg 360

gatttacaag agagctgccg aatcggatat gaggaaagaa atgaatggta tttcttcagc 420

cacaaagata agaaatatcc aacaggaaca agaacaaaca gagcgaccat ggctgggttt 480

tggaaagcca cgggccgaga caaggctgtt tacgacaagt caaaactgat tggtatgaga 540

aaaacacttg tgttctacaa aggaagagcc cctaatggcc aaaaaaccga ttggatcatg 600

catgaatacc ggctagagtc agatgagaat gcacctcctc aggaagaagg gtgggtggtt 660

tgtagagcgt tcaagaaaaa gccaatgacc gggcaagcca agaacacaga aacttggagc 720

tcaagttact tttacgacga attaccgagt ggagtacgct cagttacgga gcctcttaac 780

tacgtatcta agcagaaaca aaacgttttt gcacaagatt taatgttcaa gcaagaacta 840

gaaggatcag atatcggttt aaacttcatc cactgcgatc aattcattca acttccgcag 900

cttgaaagcc cttcactccc tcttaccaaa agaccagtga gcttgacgtc aattacatca 960

ttggagaaga ataaaaatat ctacaaaaga catttaatag aagaggatgt gagcttcaat 1020

gcgctaataa gtagtggaaa taaagataag aagaagaaga aaacatcagt gatgacgacg 1080

gattggagag cactcgataa atttgttgct tctcaactta tgagccaaga agatggagtt 1140

tcgggttttg gaggtcatca tgaagaagat aacaataaaa tcggtcatta caataacgaa 1200

gagagcaata acaagggatc agtagagact gcttcttcca cgttattgag tgatagagaa 1260

gaagagaaca gattcatcag tggattattg tgttccaact tggactatga cttatatagg 1320

gatttacatg tttgataaaa gataacacta taatatggtg cgtaacgttt gctatatagg 1380

tacgtagaat ttattgatac agtataacat atataaagat gtgtgaaaga tttgttttct 1440

tctactatat atactttttc gtgaa 1465

<210> 22
<211> 377
<212> PRT
<213> Arabidopsis thaliana

<400> 22

Met Glu Ser Val Asp Gln Ser Cys Ser Val Pro Pro Gly Phe Arg Phe
1 5 10 15

His Pro Thr Asp Glu Glu Leu Val Gly Tyr Tyr Leu Arg Lys Lys Val
20 25 30

```
Ala Ser Gln Lys Ile Asp Leu Asp Val Ile Arg Asp Ile Asp Leu Tyr
        35                  40                  45

Arg Ile Glu Pro Trp Asp Leu Gln Glu Ser Cys Arg Ile Gly Tyr Glu
    50                  55                  60

Glu Arg Asn Glu Trp Tyr Phe Phe Ser His Lys Asp Lys Lys Tyr Pro
65                  70                  75                  80

Thr Gly Thr Arg Thr Asn Arg Ala Thr Met Ala Gly Phe Trp Lys Ala
                85                  90                  95

Thr Gly Arg Asp Lys Ala Val Tyr Asp Lys Ser Lys Leu Ile Gly Met
            100                 105                 110

Arg Lys Thr Leu Val Phe Tyr Lys Gly Arg Ala Pro Asn Gly Gln Lys
        115                 120                 125

Thr Asp Trp Ile Met His Glu Tyr Arg Leu Glu Ser Asp Glu Asn Ala
    130                 135                 140

Pro Pro Gln Glu Glu Gly Trp Val Val Cys Arg Ala Phe Lys Lys Lys
145                 150                 155                 160

Pro Met Thr Gly Gln Ala Lys Asn Thr Glu Thr Trp Ser Ser Ser Tyr
                165                 170                 175

Phe Tyr Asp Glu Leu Pro Ser Gly Val Arg Ser Val Thr Glu Pro Leu
            180                 185                 190

Asn Tyr Val Ser Lys Gln Lys Gln Asn Val Phe Ala Gln Asp Leu Met
        195                 200                 205

Phe Lys Gln Glu Leu Glu Gly Ser Asp Ile Gly Leu Asn Phe Ile His
    210                 215                 220

Cys Asp Gln Phe Ile Gln Leu Pro Gln Leu Glu Ser Pro Ser Leu Pro
225                 230                 235                 240

Leu Thr Lys Arg Pro Val Ser Leu Thr Ser Ile Thr Ser Leu Glu Lys
                245                 250                 255

Asn Lys Asn Ile Tyr Lys Arg His Leu Ile Glu Glu Asp Val Ser Phe
            260                 265                 270

Asn Ala Leu Ile Ser Ser Gly Asn Lys Asp Lys Lys Lys Lys Lys Thr
        275                 280                 285
```

```
Ser Val Met Thr Thr Asp Trp Arg Ala Leu Asp Lys Phe Val Ala Ser
    290                 295                 300

Gln Leu Met Ser Gln Glu Asp Gly Val Ser Gly Phe Gly Gly His His
305                 310                 315                 320

Glu Glu Asp Asn Asn Lys Ile Gly His Tyr Asn Asn Glu Glu Ser Asn
                325                 330                 335

Asn Lys Gly Ser Val Glu Thr Ala Ser Ser Thr Leu Leu Ser Asp Arg
            340                 345                 350

Glu Glu Glu Asn Arg Phe Ile Ser Gly Leu Leu Cys Ser Asn Leu Asp
        355                 360                 365

Tyr Asp Leu Tyr Arg Asp Leu His Val
    370                 375


<210>  23
<211>  1101
<212>  DNA
<213>  Oryza sativa

<400>  23
atggaatcat gcgtccctcc tgggttcagg ttccacccca ccgacgagga gctcgtcggc      60

tactacctcc gcaagaaggt cgcctcccag aagatcgacc tcgacgtcat ccgcgacatc     120

gatctctacc gcatcgaacc ctgggatctc caagaacatt gtgggatcgg gtacgatgag     180

caaagcgagt ggtacttctt cagctacaag gacaggaagt acccgacggg gacgaggacg     240

aacagggcga caatggcggg gttctggaag gcgacgggga gggacaaggc ggtgcacgac     300

aagagcaggc tcatcggcat gaggaagaca ctcgtcttct ataagggcag ggcgcctaat     360

ggccagaaga ccgactggat catgcacgag taccgcctcg agaccgacga gaacgcgccg     420

ccacaggaag aaggatgggt ggtgtgccga gcattcaaga agaggacggc gtatccggcg     480

aggagcatgg tggagacgtg ggactactcc ttgcacgagc gcaacatcat gagcgccgcg     540

gcggcggcgg cgttcgccga tccgagcgcg gcgtacgcgc agatgaggcg gcagcacagg     600

agcgggcggt tcaagcagga ggcggagctg gacggcgccg ccacagccct cctccactac     660

tccagccacc tcgccgagct gccgcagctc gagagcccct ccgcggctgc ggcgccgctc     720

cagcccaacc cgagccagct ggccaccgcc ggcgaggacg acgactgcaa gggcgataat     780

ggcggtagga gggccaagaa agcccgcgcc gccggcgaca aggtggcgac gaccacggac     840

tggagagcgc tcgacaagtt cgtcgcgtcg cagcttagcc ccggggagtg tggcagcatg     900

gaggcaacgg cggaagccgc ggcggcggca gtcgccggtg tgagctcgcc gctggaccac     960

ggcgatgacg acatggcagc attgctgttt ctcaacagcg acgagagaga cgaggtcgac    1020
```

```
aggtggacgg ggttgctcgg ctccggcgcc ggcgcgagcg gcgtcgacgg cgacctcgga   1080

atctgtgtgt ttgacaaatg a                                              1101


<210>  24
<211>  366
<212>  PRT
<213>  Oryza sativa

<400>  24

Met Glu Ser Cys Val Pro Pro Gly Phe Arg Phe His Pro Thr Asp Glu
1               5                   10                  15

Glu Leu Val Gly Tyr Tyr Leu Arg Lys Lys Val Ala Ser Gln Lys Ile
            20                  25                  30

Asp Leu Asp Val Ile Arg Asp Ile Asp Leu Tyr Arg Ile Glu Pro Trp
        35                  40                  45

Asp Leu Gln Glu His Cys Gly Ile Gly Tyr Asp Glu Gln Ser Glu Trp
    50                  55                  60

Tyr Phe Phe Ser Tyr Lys Asp Arg Lys Tyr Pro Thr Gly Thr Arg Thr
65                  70                  75                  80

Asn Arg Ala Thr Met Ala Gly Phe Trp Lys Ala Thr Gly Arg Asp Lys
                85                  90                  95

Ala Val His Asp Lys Ser Arg Leu Ile Gly Met Arg Lys Thr Leu Val
            100                 105                 110

Phe Tyr Lys Gly Arg Ala Pro Asn Gly Gln Lys Thr Asp Trp Ile Met
        115                 120                 125

His Glu Tyr Arg Leu Glu Thr Asp Glu Asn Ala Pro Pro Gln Glu Glu
    130                 135                 140

Gly Trp Val Val Cys Arg Ala Phe Lys Lys Arg Thr Ala Tyr Pro Ala
145                 150                 155                 160

Arg Ser Met Val Glu Thr Trp Asp Tyr Ser Leu His Glu Arg Asn Ile
                165                 170                 175

Met Ser Ala Ala Ala Ala Ala Ala Phe Ala Asp Pro Ser Ala Ala Tyr
            180                 185                 190

Ala Gln Met Arg Arg Gln His Arg Ser Gly Arg Phe Lys Gln Glu Ala
        195                 200                 205

Glu Leu Asp Gly Ala Ala Thr Ala Leu Leu His Tyr Ser Ser His Leu
```

210 215 220

Ala Glu Leu Pro Gln Leu Glu Ser Pro Ser Ala Ala Ala Ala Pro Leu
225 230 235 240

Gln Pro Asn Pro Ser Gln Leu Ala Thr Ala Gly Glu Asp Asp Asp Cys
245 250 255

Lys Gly Asp Asn Gly Gly Arg Arg Ala Lys Lys Ala Arg Ala Ala Gly
260 265 270

Asp Lys Val Ala Thr Thr Thr Asp Trp Arg Ala Leu Asp Lys Phe Val
275 280 285

Ala Ser Gln Leu Ser Pro Gly Glu Cys Gly Ser Met Glu Ala Thr Ala
290 295 300

Glu Ala Ala Ala Ala Ala Val Ala Gly Val Ser Ser Pro Leu Asp His
305 310 315 320

Gly Asp Asp Asp Met Ala Ala Leu Leu Phe Leu Asn Ser Asp Glu Arg
325 330 335

Asp Glu Val Asp Arg Trp Thr Gly Leu Leu Gly Ser Gly Ala Gly Ala
340 345 350

Ser Gly Val Asp Gly Asp Leu Gly Ile Cys Val Phe Asp Lys
355 360 365

<210> 25
<211> 1047
<212> DNA
<213> Arabidopsis thaliana

<400> 25
atggaaagtc tcgcacacat tcctcccggt tatcgattcc atccgaccga tgaagaactc 60

gttgactatt atctcaagaa caaagttgca ttcccgggaa tgcaagttga tgttatcaaa 120

gatgttgatc tctacaaaat cgagccatgg gacatccaag agttatgtgg aagagggaca 180

ggagaagaga gggaatggta tttctttagc cacaaggaca agaaatatcc aactgggaca 240

cgaaccaata gagcaacggg ctccggattt tggaaagcaa cgggtcgaga caaggccatt 300

tactcaaagc aagagcttgt tgggatgagg aagactcttg tcttttacaa aggtagggcc 360

ccaaatggtc agaaatctga ttggataatg cacgaatacc gtcttgagac cgatgaaaat 420

ggaccgcctc atgaggaagg atgggtggtt tgtcgcgctt tcaagaagaa gctaaccacg 480

atgaactaca acaatccaag aacaatgatg ggatcatcat caggccaaga atctaactgg 540

ttcacgcagc aaatggatgt ggggaatggt aattactatc atcttcctga tctagagagt 600

```
ccgagaatgt ttcaaggctc atcatcatca tcactatcat cattacatca gaatgatcaa     660
gacccttatg gtgtcgtact cagcactatt aacgcaaccc caactacaat aatgcaacga     720
gatgatggtc atgtgattac caatgatgat gatcatatga tcatgatgaa cacaagtact     780
ggtgatcatc atcaatcagg attactagtc aatgatgatc ataatgatca agtaatggat     840
tggcaaacgc ttgacaagtt tgttgcttct cagctaatca tgagccaaga agaggaagaa     900
gttaacaaag atccatcaga taattcttcg aatgaaacat ttcatcatct ctctgaagag     960
caagctgcaa caatggtttc gatgaatgct tcttcctctt cttctccatg ttccttctac    1020
tcttgggctc aaaatacaca cacgtaa                                         1047
```

<210> 26
<211> 348
<212> PRT
<213> Arabidopsis thaliana

<400> 26

```
Met Glu Ser Leu Ala His Ile Pro Pro Gly Tyr Arg Phe His Pro Thr
1               5                   10                  15

Asp Glu Glu Leu Val Asp Tyr Tyr Leu Lys Asn Lys Val Ala Phe Pro
            20                  25                  30

Gly Met Gln Val Asp Val Ile Lys Asp Val Asp Leu Tyr Lys Ile Glu
        35                  40                  45

Pro Trp Asp Ile Gln Glu Leu Cys Gly Arg Gly Thr Gly Glu Glu Arg
    50                  55                  60

Glu Trp Tyr Phe Phe Ser His Lys Asp Lys Lys Tyr Pro Thr Gly Thr
65                  70                  75                  80

Arg Thr Asn Arg Ala Thr Gly Ser Gly Phe Trp Lys Ala Thr Gly Arg
                85                  90                  95

Asp Lys Ala Ile Tyr Ser Lys Gln Glu Leu Val Gly Met Arg Lys Thr
            100                 105                 110

Leu Val Phe Tyr Lys Gly Arg Ala Pro Asn Gly Gln Lys Ser Asp Trp
        115                 120                 125

Ile Met His Glu Tyr Arg Leu Glu Thr Asp Glu Asn Gly Pro Pro His
    130                 135                 140

Glu Glu Gly Trp Val Val Cys Arg Ala Phe Lys Lys Lys Leu Thr Thr
145                 150                 155                 160

Met Asn Tyr Asn Asn Pro Arg Thr Met Met Gly Ser Ser Ser Gly Gln
```

165 170 175

Glu Ser Asn Trp Phe Thr Gln Gln Met Asp Val Gly Asn Gly Asn Tyr
180 185 190

Tyr His Leu Pro Asp Leu Glu Ser Pro Arg Met Phe Gln Gly Ser Ser
195 200 205

Ser Ser Ser Leu Ser Ser Leu His Gln Asn Asp Gln Asp Pro Tyr Gly
210 215 220

Val Val Leu Ser Thr Ile Asn Ala Thr Pro Thr Thr Ile Met Gln Arg
225 230 235 240

Asp Asp Gly His Val Ile Thr Asn Asp Asp Asp His Met Ile Met Met
245 250 255

Asn Thr Ser Thr Gly Asp His His Gln Ser Gly Leu Leu Val Asn Asp
260 265 270

Asp His Asn Asp Gln Val Met Asp Trp Gln Thr Leu Asp Lys Phe Val
275 280 285

Ala Ser Gln Leu Ile Met Ser Gln Glu Glu Glu Glu Val Asn Lys Asp
290 295 300

Pro Ser Asp Asn Ser Ser Asn Glu Thr Phe His His Leu Ser Glu Glu
305 310 315 320

Gln Ala Ala Thr Met Val Ser Met Asn Ala Ser Ser Ser Ser Ser Pro
325 330 335

Cys Ser Phe Tyr Ser Trp Ala Gln Asn Thr His Thr
340 345

<210> 27
<211> 879
<212> DNA
<213> Arabidopsis thaliana

<400> 27
atgatgaagg ttgatcaaga ttattcgtgt agtataccgc ctggatttag gtttcatccg 60

acagatgaag aacttgtcgg atattatctc aagaagaaaa tcgcctccca gaggattgat 120

ctcgacgtta tcagagaaat tgatctttac aagatcgaac catgggatct acaagagaga 180

tgtaggatag ggtacgagga gcaaacggag tggtatttct tcagccatag agacaagaag 240

tatccgactg ggactaggac aaaccgagcc accgtggccg gtttctggaa agcaacgggc 300

cgggacaagg cggtttacct caactccaaa cttatcggta tgagaaaaac gcttgtcttt 360

```
taccgaggtc gagcgcctaa tggccaaaag tccgattgga tcattcacga atactacagc    420

ctcgagtcac accagaactc tcctccacag gaagaaggat gggtagtgtg tagagcattt    480

aagaaacgaa cgaccatccc aacaaaaagg aggcaacttt gggatccgaa ctgcttattc    540

tacgacgacg ccactctctt ggaacctctc gacaagcgag ccagacataa tcctgatttt    600

accgccacac cgttcaagca agaactactc tccgaggcca gtcacgtcca ggatggagat    660

ttcggatcta tgtaccttca atgcatcgat gatgatcaat tctcccagct tcctcagctc    720

gagagcccct ctcttccgtc ggaaataact ccccatagta ctactttttc tgagaacagt    780

agccggaaag atgacatgag ctccgagaag aggatcactg actggagata tctagataag    840

ttcgtggcgt ctcaattttt gatgagtgga gaagactaa                           879


<210>  28
<211>  292
<212>  PRT
<213>  Arabidopsis thaliana

<400>  28

Met Met Lys Val Asp Gln Asp Tyr Ser Cys Ser Ile Pro Pro Gly Phe
1               5                   10                  15


Arg Phe His Pro Thr Asp Glu Glu Leu Val Gly Tyr Tyr Leu Lys Lys
            20                  25                  30


Lys Ile Ala Ser Gln Arg Ile Asp Leu Asp Val Ile Arg Glu Ile Asp
        35                  40                  45


Leu Tyr Lys Ile Glu Pro Trp Asp Leu Gln Glu Arg Cys Arg Ile Gly
    50                  55                  60


Tyr Glu Glu Gln Thr Glu Trp Tyr Phe Phe Ser His Arg Asp Lys Lys
65                  70                  75                  80


Tyr Pro Thr Gly Thr Arg Thr Asn Arg Ala Thr Val Ala Gly Phe Trp
                85                  90                  95


Lys Ala Thr Gly Arg Asp Lys Ala Val Tyr Leu Asn Ser Lys Leu Ile
            100                 105                 110


Gly Met Arg Lys Thr Leu Val Phe Tyr Arg Gly Arg Ala Pro Asn Gly
        115                 120                 125


Gln Lys Ser Asp Trp Ile Ile His Glu Tyr Tyr Ser Leu Glu Ser His
    130                 135                 140


Gln Asn Ser Pro Pro Gln Glu Glu Gly Trp Val Val Cys Arg Ala Phe
145                 150                 155                 160
```

Lys Lys Arg Thr Thr Ile Pro Thr Lys Arg Arg Gln Leu Trp Asp Pro
165 170 175

Asn Cys Leu Phe Tyr Asp Asp Ala Thr Leu Leu Glu Pro Leu Asp Lys
180 185 190

Arg Ala Arg His Asn Pro Asp Phe Thr Ala Thr Pro Phe Lys Gln Glu
195 200 205

Leu Leu Ser Glu Ala Ser His Val Gln Asp Gly Asp Phe Gly Ser Met
210 215 220

Tyr Leu Gln Cys Ile Asp Asp Asp Gln Phe Ser Gln Leu Pro Gln Leu
225 230 235 240

Glu Ser Pro Ser Leu Pro Ser Glu Ile Thr Pro His Ser Thr Thr Phe
245 250 255

Ser Glu Asn Ser Ser Arg Lys Asp Asp Met Ser Ser Glu Lys Arg Ile
260 265 270

Thr Asp Trp Arg Tyr Leu Asp Lys Phe Val Ala Ser Gln Phe Leu Met
275 280 285

Ser Gly Glu Asp
290

<210> 29
<211> 927
<212> DNA
<213> Oryza sativa

<400> 29
ccatcttaat tttttacttc tttacttaat tatctgtcct actatatatg ttaattcatg 60

gatattaata tggaatatgg atgcaggagg aaggttgggt ggtttgccgc gcgtttcaga 120

agccgatgcc caaccagcag cagcacaggc tgtcctacgg ctgcatcccc ggcagctacg 180

gcgccggagc ctacgccgcc gtccccgaca actacagctt gctgctgcac cacgacaacc 240

ctagcttcgc cgggcggcca ttgatgagcg ccgctgcctc agctctcttc gccaacaaca 300

acaataacag cgtcgtcgac cacagcaaca ttctgagttc agagtccaag cttcacttct 360

ccgacatgat gccgcctctg gagagcccca ccatcgtcga cggcgagggc tacgtctcgc 420

aggctagcag ctgcgtcgac gtcgatcagc aagccggcat cgtcgactgg aacctgctca 480

ccagcttgct gccgccgccg gcgcatcagc tcttccacca cctgccttcc gcttcgggag 540

ttattacaag tagagtgaga ggagacggcg cggcggcggc ggcggcggcg ggatggagag 600

ggagaaccgg gtggagacca tctcccggct ggcccagtgg cgcatcgaca ccttcggccc 660

ctcctcctac cgccgctccg attccttcaa gatcggcatc tggaactggt tcccttccca 720

atcccaatcc cccactactc agagatcacc gatttgccct atcaaatcaa atcatccgcc 780

aattcgatcc gaatccgtgc aggtacctat cggtggagaa ggctcgatat gtatacgtgg 840

ggctgttccc ggagcccggg cgggtggcca aggaacggcc cccgctcgcc cgcttccttc 900

tccgagcttg ctggtccggc cccaatg 927

<210> 30
<211> 282
<212> PRT
<213> Oryza sativa

<400> 30

Met Gln Glu Glu Gly Trp Val Val Cys Arg Ala Phe Gln Lys Pro Met
1 5 10 15

Pro Asn Gln Gln Gln His Arg Leu Ser Tyr Gly Cys Ile Pro Gly Ser
20 25 30

Tyr Gly Ala Gly Ala Tyr Ala Ala Val Pro Asp Asn Tyr Ser Leu Leu
35 40 45

Leu His His Asp Asn Pro Ser Phe Ala Gly Arg Pro Leu Met Ser Ala
50 55 60

Ala Ala Ser Ala Leu Phe Ala Asn Asn Asn Asn Asn Ser Val Val Asp
65 70 75 80

His Ser Asn Ile Leu Ser Ser Glu Ser Lys Leu His Phe Ser Asp Met
85 90 95

Met Pro Pro Leu Glu Ser Pro Thr Ile Val Asp Gly Glu Gly Tyr Val
100 105 110

Ser Gln Ala Ser Ser Cys Val Asp Val Asp Gln Gln Ala Gly Ile Val
115 120 125

Asp Trp Asn Leu Leu Thr Ser Leu Leu Pro Pro Pro Ala His Gln Leu
130 135 140

Phe His His Leu Pro Ser Ala Ser Gly Val Ile Thr Ser Arg Val Arg
145 150 155 160

Gly Asp Gly Ala Ala Ala Ala Ala Ala Ala Gly Trp Arg Gly Arg Thr
165 170 175

Gly Trp Arg Pro Ser Pro Gly Trp Pro Ser Gly Ala Ser Thr Pro Ser
180 185 190

```
Ala Pro Pro Pro Thr Ala Ala Pro Ile Pro Ser Arg Ser Ala Ser Gly
        195                 200                 205

Thr Gly Ser Leu Pro Asn Pro Asn Pro Pro Leu Leu Arg Asp His Arg
    210                 215                 220

Phe Ala Leu Ser Asn Gln Ile Ile Arg Gln Phe Asp Pro Asn Pro Cys
225                 230                 235                 240

Arg Tyr Leu Ser Val Glu Lys Ala Arg Tyr Val Tyr Val Gly Leu Phe
                245                 250                 255

Pro Glu Pro Gly Arg Val Ala Lys Glu Arg Pro Pro Leu Ala Arg Phe
            260                 265                 270

Leu Leu Arg Ala Cys Trp Ser Gly Pro Asn
        275                 280
```

<210> 31
<211> 1168
<212> DNA
<213> Arabidopsis thaliana

<400> 31

```
atcttgtttc ttactcattt ctctaacaat tttccaaatt aaatacgttt ataggatcat     60
cgtggatgga taatataatg caatcgtcaa tgccaccggg attccgattt catccgacag    120
aggaagagct tgtgggttat tacctagata ggaagatcaa ttcaatgaag agtgctttag    180
atgtcattgt agagattgat ctctacaaaa tggagccatg ggatatacaa gcgaggtgta    240
aactagggta tgaagagcaa aacgagtggt acttctttag tcataaggac aggaagtacc    300
ctaccgggac taggaccaac cgagccactg cggctgggtt ctggaaagcc acgggtagag    360
acaaggcggt actatcaaaa aacagtgtca tcggaatgcg gaagacactt gtctactaca    420
agggtcgagc tcctaatgga agaaagtccg attggatcat gcacgaatac cgtctccaaa    480
actccgagct tgccccggtt caggaggaag gctgggtggt gtgtcgagca tttaggaagc    540
caattccaaa ccagaggcca ttagggtacg agccatggca gaaccagctc taccacgtcg    600
aaagtagtaa caactactca tcttcagtga caatgaacac gagtcatcat atcggtgcat    660
cttcatcaag tcataacctt aatcaaatgc tcatgagcaa taaccactac aatcctaata    720
atacatcctc atcgatgcat caatatggca acattgagct cccgcagttg gacagcccga    780
gcttgtcgcc tagtttaggg acgaataaag atcagaacga gagtttcgag caagaagaag    840
agaagagctt taactgtgtg gattggagaa cactagatac cttgcttgag acacaagtca    900
tacatccgca taaccctaat attcttatgt tcgaaacgca gtcgtataat ccggcgccaa    960
gcttcccttc catgcatcaa agctataatg aggtcgaagc taatattcat cattctcttg   1020
```

```
gatgcttccc tgactcgtaa ttaaaaaaaa acacacttct atatattgat ttgtattcta   1080

tgatttaatt gttccatctg gaaaataaca tttataactg tctatttgta aaagaagttt   1140

gtgtttatta cgtagaaatt tgttgttg                                       1168


<210>  32
<211>  324
<212>  PRT
<213>  Arabidopsis thaliana

<400>  32

Met Asp Asn Ile Met Gln Ser Ser Met Pro Pro Gly Phe Arg Phe His
1               5                   10                  15


Pro Thr Glu Glu Glu Leu Val Gly Tyr Tyr Leu Asp Arg Lys Ile Asn
            20                  25                  30


Ser Met Lys Ser Ala Leu Asp Val Ile Val Glu Ile Asp Leu Tyr Lys
        35                  40                  45


Met Glu Pro Trp Asp Ile Gln Ala Arg Cys Lys Leu Gly Tyr Glu Glu
    50                  55                  60


Gln Asn Glu Trp Tyr Phe Phe Ser His Lys Asp Arg Lys Tyr Pro Thr
65                  70                  75                  80


Gly Thr Arg Thr Asn Arg Ala Thr Ala Ala Gly Phe Trp Lys Ala Thr
                85                  90                  95


Gly Arg Asp Lys Ala Val Leu Ser Lys Asn Ser Val Ile Gly Met Arg
            100                 105                 110


Lys Thr Leu Val Tyr Tyr Lys Gly Arg Ala Pro Asn Gly Arg Lys Ser
        115                 120                 125


Asp Trp Ile Met His Glu Tyr Arg Leu Gln Asn Ser Glu Leu Ala Pro
    130                 135                 140


Val Gln Glu Glu Gly Trp Val Val Cys Arg Ala Phe Arg Lys Pro Ile
145                 150                 155                 160


Pro Asn Gln Arg Pro Leu Gly Tyr Glu Pro Trp Gln Asn Gln Leu Tyr
                165                 170                 175


His Val Glu Ser Ser Asn Asn Tyr Ser Ser Ser Val Thr Met Asn Thr
            180                 185                 190


Ser His His Ile Gly Ala Ser Ser Ser Ser His Asn Leu Asn Gln Met
        195                 200                 205
```

Leu Met Ser Asn Asn His Tyr Asn Pro Asn Asn Thr Ser Ser Ser Met
    210                 215                 220

His Gln Tyr Gly Asn Ile Glu Leu Pro Gln Leu Asp Ser Pro Ser Leu
225                 230                 235                 240

Ser Pro Ser Leu Gly Thr Asn Lys Asp Gln Asn Glu Ser Phe Glu Gln
                245                 250                 255

Glu Glu Glu Lys Ser Phe Asn Cys Val Asp Trp Arg Thr Leu Asp Thr
            260                 265                 270

Leu Leu Glu Thr Gln Val Ile His Pro His Asn Pro Asn Ile Leu Met
        275                 280                 285

Phe Glu Thr Gln Ser Tyr Asn Pro Ala Pro Ser Phe Pro Ser Met His
    290                 295                 300

Gln Ser Tyr Asn Glu Val Glu Ala Asn Ile His His Ser Leu Gly Cys
305                 310                 315                 320

Phe Pro Asp Ser

<210> 33
<211> 549
<212> DNA
<213> Oryza sativa

<400> 33
atgcagccgc cgcggagctc catcggagca tgggaggcga gctactccta ccacgacccc 60

gccgtcttcg tcggcggcgg ggaacacttc aagcaagagg ccgcggccga gctggacggc 120

gtcgccgccg ccgccggagc taacgccttc ctgcggtact ccacccgcct ggccgagctc 180

ccgcagctgg agagcccgcc gctgccaagc caggggagcc aggcggcctc ggccgtcgtc 240

gacggcgagg aagataacgc cgattctagc aggcgccctg gcggcggcgg cggcgccgcc 300

gccgcggtga ccacggactg gagggcgttc gacaagttcg tcgcgtccca gctcagcccc 360

gaggagcagc acacctgccg ggccaccgac gacgacgaca tggcagcgct gctgctcctc 420

gacggcggcg ggcaggagga cgacgccggg aggtggctgg gctccgccgg gttgctgagc 480

gccgtggcgg ccgacgcgac gacggactgc ggcctcggca ccagctgcgt gcctggcgac 540

atcaactga 549

<210> 34
<211> 182
<212> PRT
<213> Oryza sativa

<400> 34

Met Gln Pro Pro Arg Ser Ser Ile Gly Ala Trp Glu Ala Ser Tyr Ser
1               5                   10                  15

Tyr His Asp Pro Ala Val Phe Val Gly Gly Gly Glu His Phe Lys Gln
            20                  25                  30

Glu Ala Ala Ala Glu Leu Asp Gly Val Ala Ala Ala Ala Gly Ala Asn
        35                  40                  45

Ala Phe Leu Arg Tyr Ser Thr Arg Leu Ala Glu Leu Pro Gln Leu Glu
    50                  55                  60

Ser Pro Pro Leu Pro Ser Gln Gly Ser Gln Ala Ala Ser Ala Val Val
65                  70                  75                  80

Asp Gly Glu Glu Asp Asn Ala Asp Ser Ser Arg Arg Pro Gly Gly Gly
                85                  90                  95

Gly Gly Ala Ala Ala Ala Val Thr Thr Asp Trp Arg Ala Phe Asp Lys
            100                 105                 110

Phe Val Ala Ser Gln Leu Ser Pro Glu Glu Gln His Thr Cys Arg Ala
        115                 120                 125

Thr Asp Asp Asp Asp Met Ala Ala Leu Leu Leu Leu Asp Gly Gly Gly
    130                 135                 140

Gln Glu Asp Asp Ala Gly Arg Trp Leu Gly Ser Ala Gly Leu Leu Ser
145                 150                 155                 160

Ala Val Ala Ala Asp Ala Thr Thr Asp Cys Gly Leu Gly Thr Ser Cys
                165                 170                 175

Val Pro Gly Asp Ile Asn
            180

<210> 35
<211> 1074
<212> DNA
<213> Zinnia elegans

<400> 35
atggacacaa tgaatcaatc atcatgtact gtccctccag gttttcgttt tcatcctacc 60

gatgaagaac ttgttggtta ctatctcaga aagaaggttg catctcaaaa gattgatctt 120

gatgtcatta aagacatcga tctttatcga atcgaaccat gggatcttat agagagatgt 180

cggatcggat acgaggagca aaatgagtgg tatttcttca gtcacaagga taaaaagtat 240

ccaacaggga caaggacaaa tagggcaact atggcaggtt tttggaaggc aacgggtaga 300

gacaaagctg tttacgaaaa actaaggctc ataggaatga gaaaaaccct agttttctac 360

aaaggaaggg caccaaatgg ccagaaaacc gattggatca tgcacgagta caggctcgaa 420

tctgaagaaa atggacctcc tcaggaagaa ggatgggtag tatgtcgagc attcaagaaa 480

cgtgcaaccg gacaaacaag aacaacacca gcatgggaat caaattactt cttagatgaa 540

tcaagcctcc ttacatccac aatggatgat tacaccacaa ttcaaccttc aaatattcct 600

ttgacatcaa gtttcatgtg caagcaagag ttggaagcat cagaaaattt gaactttatt 660

cattgtgatc agtttattca acttccacac ctcgaaagcc catccctcca atcgataaaa 720

cggcctataa gctccatact atatgaacat gatcaacaag aagacgatca aatcacaaaa 780

agaatcacaa acaacgttag gagtaaagat gaagtgaggg attggaggga tcttgataag 840

tttgtagctt ctcaattgag ccaagaggaa gagattcgat gtgtcgaaga agggttttca 900

acgaatttcc aagagaacat tgatcattct actagtttga gtcatatgtt taattatcaa 960

gatggtattg aagaatacag tggttgtgac aaagggggca agttaaatgg attcttgagt 1020

tcaacaagct cagatcattt tgatgttgga atttgcatat ttgataaatt atga 1074

<210> 36
<211> 357
<212> PRT
<213> Zinnia elegans

<400> 36

Met Asp Thr Met Asn Gln Ser Ser Cys Thr Val Pro Pro Gly Phe Arg
1 5 10 15

Phe His Pro Thr Asp Glu Glu Leu Val Gly Tyr Tyr Leu Arg Lys Lys
20 25 30

Val Ala Ser Gln Lys Ile Asp Leu Asp Val Ile Lys Asp Ile Asp Leu
35 40 45

Tyr Arg Ile Glu Pro Trp Asp Leu Ile Glu Arg Cys Arg Ile Gly Tyr
50 55 60

Glu Glu Gln Asn Glu Trp Tyr Phe Phe Ser His Lys Asp Lys Lys Tyr
65 70 75 80

Pro Thr Gly Thr Arg Thr Asn Arg Ala Thr Met Ala Gly Phe Trp Lys
85 90 95

Ala Thr Gly Arg Asp Lys Ala Val Tyr Glu Lys Leu Arg Leu Ile Gly
100 105 110

Met Arg Lys Thr Leu Val Phe Tyr Lys Gly Arg Ala Pro Asn Gly Gln
115 120 125

```
Lys Thr Asp Trp Ile Met His Glu Tyr Arg Leu Glu Ser Glu Glu Asn
    130                 135                 140

Gly Pro Pro Gln Glu Glu Gly Trp Val Val Cys Arg Ala Phe Lys Lys
145                 150                 155                 160

Arg Ala Thr Gly Gln Thr Arg Thr Thr Pro Ala Trp Glu Ser Asn Tyr
                165                 170                 175

Phe Leu Asp Glu Ser Ser Leu Leu Thr Ser Thr Met Asp Asp Tyr Thr
            180                 185                 190

Thr Ile Gln Pro Ser Asn Ile Pro Leu Thr Ser Ser Phe Met Cys Lys
        195                 200                 205

Gln Glu Leu Glu Ala Ser Glu Asn Leu Asn Phe Ile His Cys Asp Gln
    210                 215                 220

Phe Ile Gln Leu Pro His Leu Glu Ser Pro Ser Leu Gln Ser Ile Lys
225                 230                 235                 240

Arg Pro Ile Ser Ser Ile Leu Tyr Glu His Asp Gln Gln Glu Asp Asp
                245                 250                 255

Gln Ile Thr Lys Arg Ile Thr Asn Asn Val Arg Ser Lys Asp Glu Val
            260                 265                 270

Arg Asp Trp Arg Asp Leu Asp Lys Phe Val Ala Ser Gln Leu Ser Gln
        275                 280                 285

Glu Glu Glu Ile Arg Cys Val Glu Glu Gly Phe Ser Thr Asn Phe Gln
    290                 295                 300

Glu Asn Ile Asp His Ser Thr Ser Leu Ser His Met Phe Asn Tyr Gln
305                 310                 315                 320

Asp Gly Ile Glu Glu Tyr Ser Gly Cys Asp Lys Gly Gly Lys Leu Asn
                325                 330                 335

Gly Phe Leu Ser Ser Thr Ser Ser Asp His Phe Asp Val Gly Ile Cys
            340                 345                 350

Ile Phe Asp Lys Leu
        355
```

<210> 37
<211> 1283
<212> DNA

<213> Zea mays

<400> 37
atgcatccag gtgttggacc attgtcggtg ccaccaggct tccgcttcca tccgactgat 60

gaggagctcc tctactacta cctgaggaaa aaggttgctt atgagcccat agatcttgat 120

gtcataaggg agattgatct caataagctt gagccctggg atctcaaaga aagatgcaaa 180

ataggcactg ggcctcaaaa cgagtggtac ttcttcagcc acaaggacaa gaagtactca 240

acgggaacga gaacgaaccg tgccacgacg gcgggattct ggaaggcgac tggccgagac 300

aaggccatct tccttggcag cgccaggagg atcggcttga gaaagaccct ggtgttctat 360

atcggcaggg cgccgcacgg gaagaagact gagtggatca tgcacgagta ccgccttgat 420

gaagagaacg ttgaaattca ggaagatgga tgggtggtat gtagggtttt caagaagaag 480

aactatgagc agagaggcca caacatggct gagatggcgg cactggacga cgatgagctc 540

cagcctttca cggttcctgt cgtccctgct ggcactagct ccctgccaac aacagaccac 600

aagaacaacc ctcacctcat gcaatatgac ttcccttctt tcgacccttc catgcagctc 660

ccacagctga tgagcgccga ccagcccgtg ccaaccctat tccccagcca tcctggcgtc 720

gccacggcca tgagctcatc aatcgacgtt gagtgctcgc agaacctgct gacgatgctg 780

acatccaacg gcagcgacgg gatgctccac gtccgcgctg gtggtgctgg agctggtgtc 840

gaccgcttcg ctggcacgac agattggtcg atcctagaca agctcctcgc ctcgcaccag 900

aacctcggac agctcttcca cggcaaggtc accgcagcat ctgcctcccc aatggctcca 960

taccatcagc agctcatgga actcgggtgg ctcgtcgtcg tcgtcgtcct tgcagaggct 1020

tccactgcag tacctcagcg gcgagacgac cgatctcctc aggttcccca agtaattatt 1080

ggatcgacct caagtttagc taccttgttg agcagtgacc atatcaggat tttggttgac 1140

tactaggctg cttaattcgg cctacttcgt atggagttta gtgcttttaa atgtatatat 1200

ggtgcaactg tttggggcat gcaggcatgg aatgccatcg atctaccatg gttgcttgct 1260

tggccaaaaa aaaaaaaaaa aaa 1283

<210> 38
<211> 381
<212> PRT
<213> Zea mays

<400> 38

Met His Pro Gly Val Gly Pro Leu Ser Val Pro Pro Gly Phe Arg Phe
1 5 10 15

His Pro Thr Asp Glu Glu Leu Leu Tyr Tyr Tyr Leu Arg Lys Lys Val
20 25 30

Ala Tyr Glu Pro Ile Asp Leu Asp Val Ile Arg Glu Ile Asp Leu Asn
35 40 45

```
Lys Leu Glu Pro Trp Asp Leu Lys Glu Arg Cys Lys Ile Gly Thr Gly
    50                  55                  60

Pro Gln Asn Glu Trp Tyr Phe Phe Ser His Lys Asp Lys Lys Tyr Ser
65                  70                  75                  80

Thr Gly Thr Arg Thr Asn Arg Ala Thr Thr Ala Gly Phe Trp Lys Ala
                85                  90                  95

Thr Gly Arg Asp Lys Ala Ile Phe Leu Gly Ser Ala Arg Arg Ile Gly
            100                 105                 110

Leu Arg Lys Thr Leu Val Phe Tyr Ile Gly Arg Ala Pro His Gly Lys
        115                 120                 125

Lys Thr Glu Trp Ile Met His Glu Tyr Arg Leu Asp Glu Glu Asn Val
    130                 135                 140

Glu Ile Gln Glu Asp Gly Trp Val Val Cys Arg Val Phe Lys Lys Lys
145                 150                 155                 160

Asn Tyr Glu Gln Arg Gly His Asn Met Ala Glu Met Ala Ala Leu Asp
                165                 170                 175

Asp Asp Glu Leu Gln Pro Phe Thr Val Pro Val Val Pro Ala Gly Thr
            180                 185                 190

Ser Ser Leu Pro Thr Thr Asp His Lys Asn Asn Pro His Leu Met Gln
        195                 200                 205

Tyr Asp Phe Pro Ser Phe Asp Pro Ser Met Gln Leu Pro Gln Leu Met
    210                 215                 220

Ser Ala Asp Gln Pro Val Pro Thr Leu Phe Pro Ser His Pro Gly Val
225                 230                 235                 240

Ala Thr Ala Met Ser Ser Ser Ile Asp Val Glu Cys Ser Gln Asn Leu
                245                 250                 255

Leu Thr Met Leu Thr Ser Asn Gly Ser Asp Gly Met Leu His Val Arg
            260                 265                 270

Ala Gly Gly Ala Gly Ala Gly Val Asp Arg Phe Ala Gly Thr Thr Asp
        275                 280                 285

Trp Ser Ile Leu Asp Lys Leu Leu Ala Ser His Gln Asn Leu Gly Gln
    290                 295                 300
```

Leu Phe His Gly Lys Val Thr Ala Ala Ser Ala Ser Pro Met Ala Pro
305                 310                 315                 320

Tyr His Gln Gln Leu Met Glu Leu Gly Trp Leu Val Val Val Val Val
                325                 330                 335

Leu Ala Glu Ala Ser Thr Ala Val Pro Gln Arg Arg Asp Asp Arg Ser
            340                 345                 350

Pro Gln Val Pro Gln Val Ile Ile Gly Ser Thr Ser Ser Leu Ala Thr
        355                 360                 365

Leu Leu Ser Ser Asp His Ile Arg Ile Leu Val Asp Tyr
    370                 375                 380

<210> 39
<211> 2193
<212> DNA
<213> Oryza sativa

<400> 39
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctcctcctc ccatctataa attcctcccc ccttttcccc tctctatata 1020

ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140

cacctcctcc tcacagggta tgtgcccttc ggttgttctt ggatttattg ttctaggttg 1200

tgtagtacgg gcgttgatgt taggaaaggg gatctgtatc tgtgatgatt cctgttcttg 1260

gatttgggat agaggggttc ttgatgttgc atgttatcgg ttcggtttga ttagtagtat 1320

ggttttcaat cgtctggaga gctctatgga aatgaaatgg tttagggtac ggaatcttgc 1380

gattttgtga gtaccttttg tttgaggtaa aatcagagca ccggtgattt tgcttggtgt 1440

aataaaagta cggttgtttg gtcctcgatt ctggtagtga tgcttctcga tttgacgaag 1500

ctatcctttg tttattccct attgaacaaa aataatccaa ctttgaagac ggtcccgttg 1560

atgagattga atgattgatt cttaagcctg tccaaaattt cgcagctggc ttgtttagat 1620

acagtagtcc ccatcacgaa attcatggaa acagttataa tcctcaggaa caggggattc 1680

cctgttcttc cgatttgctt tagtcccaga atttttttc ccaaatatct taaaaagtca 1740

ctttctggtt cagttcaatg aattgattgc tacaaataat gcttttatag cgttatccta 1800

gctgtagttc agttaatagg taataccccct atagtttagt caggagaaga acttatccga 1860

tttctgatct ccatttttaa ttatatgaaa tgaactgtag cataagcagt attcatttgg 1920

attatttttt ttattagctc tcacccttc attattctga gctgaaagtc tggcatgaac 1980

tgtcctcaat tttgttttca aattcacatc gattatctat gcattatcct cttgtatcta 2040

cctgtagaag tttctttttg gttattcctt gactgcttga ttacagaaag aaatttatga 2100

agctgtaatc gggatagtta tactgcttgt tcttatgatt catttccttt gtgcagttct 2160

tggtgtagct tgccactttc accagcaaag ttc 2193

<210> 40
<211> 1179
<212> DNA
<213> Oryza sativa

<400> 40
ttgcagttgt gaccaagtaa gctgagcatg cccttaactt cacctagaaa aaagtatact 60

tggcttaact gctagtaaga catttcagaa ctgagactgg tgtacgcatt tcatgcaagc 120

cattaccact ttacctgaca ttttggacag agattagaaa tagtttcgta ctacctgcaa 180

gttgcaactt gaaaagtgaa atttgttcct tgctaatata ttggcgtgta attcttttat 240

gcgttagcgt aaaaagttga aatttgggtc aagttactgg tcagattaac cagtaactgg 300

ttaaagttga aagatggtct tttagtaatg gagggagtac tacactatcc tcagctgatt 360

taaatcttat tccgtcggtg gtgatttcgt caatctccca acttagtttt tcaatatatt 420

cataggatag agtgtgcata tgtgtgttta tagggatgag tctacgcgcc ttatgaacac 480

ctacttttgt actgtatttg tcaatgaaaa gaaaatctta ccaatgctgc gatgctgaca 540

ccaagaagag gcgatgaaaa gtgcaacgga tatcgtgcca cgtcggttgc caagtcagca 600

cagacccaat gggcctttcc tacgtgtctc ggccacagcc agtcgtttac cgcacgttca 660

catgggcacg aactcgcgtc atcttcccac gcaaaacgac agatctgccc tatctggtcc 720

cacccatcag tggcccacac ctcccatgct gcattatttg cgactcccat cccgtcctcc 780

acgcccaaac accgcacacg ggtcgcgata gccacgaccc aatcacacaa cgccacgtca 840

ccatatgtta cgggcagcca tgcgcagaag atcccgcgac gtcgctgtcc cccgtgtcgg 900

ttacgaaaaa atatcccacc acgtgtcgct ttcacaggac aatatctcga aggaaaaaaa 960

tcgtagcgga aaatccgagg cacgagctgc gattggctgg gaggcgtcca gcgtggtggg 1020

gggcccaccc ccttatcctt agcccgtggc gctcctcgct cctcgggtcc gtgtataaat 1080

accctccgga actcactctt gctggtcacc aacacgaagc aaaaggacac cagaaacata 1140

gtacacttga gctcactcca aactcaaaca ctcacacca 1179

<210> 41
<211> 11
<212> PRT
<213> Artificial sequence

<220>
<223> Motif I

<220>
<221> VARIANT
<222> (1)..(1)
<223> /replace = "Pro" /replace = "Arg" /replace = "Gly"

<220>
<221> VARIANT
<222> (2)..(2)
<223> /replace = "Ser" /replace = "Met"

<220>
<221> VARIANT
<222> (3)..(3)
<223> /replace = "Ala"

<220>
<221> VARIANT
<222> (4)..(4)
<223> /replace = "Ile" /replace = "Val"

<220>
<221> VARIANT
<222> (6)..(6)
<223> /replace = "Val" /replace = "Phe"

<220>
<221> VARIANT
<222> (8)..(8)
<223> /replace = "Val" /replace = "Arg" /replace = "Lys"

<220>
<221> VARIANT
<222> (9)..(9)
<223> /replace = "Asp"

<220>
<221> VARIANT

<222> (10)..(10)
<223> /replace = "Ile" /replace = "Val"

<400> 41

Lys Ile Asp Leu Asp Ile Ile Gln Glu Leu Asp
1 5 10

<210> 42
<211> 14
<212> PRT
<213> Artificial sequence

<220>
<223> Motif II

<220>
<221> VARIANT
<222> (2)..(2)
<223> /replace = "Arg"

<220>
<221> VARIANT
<222> (3)..(3)
<223> /replace = "Leu" /replace = "Ile"

<220>
<221> UNSURE
<222> (5)..(7)
<223> Xaa can be any naturally occurring amino acid or a gap

<220>
<221> VARIANT
<222> (8)..(8)
<223> /replace = "Tyr" /replace = "Asn"

<220>
<221> VARIANT
<222> (9)..(9)
<223> /replace = "Glu"

<220>
<221> VARIANT
<222> (11)..(11)
<223> /replace = "Arg"

<220>
<221> VARIANT
<222> (12)..(12)
<223> /replace = "Asn" /replace = "Ser"

<400> 42

Cys Lys Tyr Gly Xaa Xaa Xaa Gly Asp Glu Gly Thr Glu Trp
1 5 10

<210> 43
<211> 11
<212> PRT
<213> Artificial sequence

<220>

<223> Motif III

<220>
<221> VARIANT
<222> (7)..(7)
<223> /replace = "Val"

<220>
<221> UNSURE
<222> (9)..(9)
<223> Xaa can be any naturally occurring amino acid, preferably Gln, Arg or Lys

<220>
<221> UNSURE
<222> (11)..(11)
<223> Xaa can be any naturally occurring amino acid, preferably Pro, Arg or Lys

<400> 43

Gly Trp Val Val Cys Arg Ala Phe Xaa Lys Xaa
1 5 10

<210> 44
<211> 30
<212> DNA
<213> Artificial sequence

<220>
<223> primer: adapter primer

<400> 44
aagcagtggt atcaacgcag agtacgcggg 30

<210> 45
<211> 26
<212> DNA
<213> Artificial sequence

<220>
<223> primer: OsNAM2-2

<400> 45
ctctccagag gcggcatcat gtcgga 26

<210> 46
<211> 18
<212> DNA
<213> Artificial sequence

<220>
<223> primer: OsNAM2-1

<400> 46
tgatcgggat gaggaaga 18

<210> 47
<211> 21
<212> DNA

<213> Artificial sequence

<220>
<223> primer: OsNAM2-3

<400> 47
gatcagtctc ggtcatcgat g 21

<210> 48
<211> 53
<212> DNA
<213> Artificial sequence

<220>
<223> primer: prm08733

<400> 48
ggggacaagt ttgtacaaaa aagcaggctt aaacaatgga tcggcatgag gag 53

<210> 49
<211> 48
<212> DNA
<213> Artificial sequence

<220>
<223> primer: prm08734

<400> 49
ggggaccact ttgtacaaga aagctgggtt cgatcgatca gtctcggt 48

<210> 50
<211> 1016
<212> DNA
<213> Oryza sativa

<400> 50
tcacaaccca caacattttc aaacaacgca aagcagtagc agcagcgaga agcaagcaag 60

aagcgatggg gatggggatg aggagggaga gggacgcgga ggcggagctg aacctgccgc 120

cggggttcag gttccacccc acggacgacg agctggtgga gcactacctg tgcaggaagg 180

cggcggggca gcgcctgccg gtgccgatca tcgccgaggt ggatctctac aagttcgacc 240

cgtgggatct gcccgagcgc gcgctgttcg gcgccaggga gtggtacttc ttcaccccgc 300

gggatcgcaa gtatcctaat gggtcacgcc ccaaccgcgc cgccggcaac gggtactgga 360

aggccaccgg cgccgacaag cccgtcgcgc cgcgggggcg cacgcttggg atcaagaagg 420

cgctcgtgtt ctacgccggc aaggcgccgc gaggggtcaa gactgattgg atcatgcatg 480

agtaccggct cgccgatgct ggccgcgccg ccgcgggcgc caagaaggga tctctcaggt 540

tggatgattg ggtgctgtgt cggctgtaca acaagaagaa cgagtgggag aagatgcagc 600

aggggaagga ggtgaaggag gaggcgtccg acatggttac gtcgcagtcg cactcgcaca 660

cccactcgtg ggcgagacg cgcacgccgg agtcggagat cgtggacaac gaccccttcc 720

cggagctgga ctcgttcccg gcgttccagc ctgcgccgcc gccggcgacg gcgatgatgg 780

```
tgcccaagaa agaatcgatg gacgacgcca ccgcggccgc cgccgccgcc gccaccatcc     840

ccaggaacaa cagcagcctg ttcgtggacc tgagctacga cgatatccag ggcatgtaca     900

gcggcctcga catgctgccg ccgggcgacg acttctactc gtcgctcttc gcgtcgccgc     960

gggtgaaggg gacgacgcca cgcgccggcg ccggcatggg catggtcccg ttctga        1016


<210>  51
<211>  316
<212>  PRT
<213>  Oryza sativa

<400>  51

Met Gly Met Gly Met Arg Arg Glu Arg Asp Ala Glu Ala Glu Leu Asn
1               5                   10                  15


Leu Pro Pro Gly Phe Arg Phe His Pro Thr Asp Asp Glu Leu Val Glu
            20                  25                  30


His Tyr Leu Cys Arg Lys Ala Ala Gly Gln Arg Leu Pro Val Pro Ile
        35                  40                  45


Ile Ala Glu Val Asp Leu Tyr Lys Phe Asp Pro Trp Asp Leu Pro Glu
    50                  55                  60


Arg Ala Leu Phe Gly Ala Arg Glu Trp Tyr Phe Phe Thr Pro Arg Asp
65                  70                  75                  80


Arg Lys Tyr Pro Asn Gly Ser Arg Pro Asn Arg Ala Ala Gly Asn Gly
                85                  90                  95


Tyr Trp Lys Ala Thr Gly Ala Asp Lys Pro Val Ala Pro Arg Gly Arg
            100                 105                 110


Thr Leu Gly Ile Lys Lys Ala Leu Val Phe Tyr Ala Gly Lys Ala Pro
        115                 120                 125


Arg Gly Val Lys Thr Asp Trp Ile Met His Glu Tyr Arg Leu Ala Asp
    130                 135                 140


Ala Gly Arg Ala Ala Ala Gly Ala Lys Lys Gly Ser Leu Arg Leu Asp
145                 150                 155                 160


Asp Trp Val Leu Cys Arg Leu Tyr Asn Lys Lys Asn Glu Trp Glu Lys
                165                 170                 175


Met Gln Gln Gly Lys Glu Val Lys Glu Glu Ala Ser Asp Met Val Thr
            180                 185                 190


Ser Gln Ser His Ser His Thr His Ser Trp Gly Glu Thr Arg Thr Pro
```

195 200 205

Glu Ser Glu Ile Val Asp Asn Asp Pro Phe Pro Glu Leu Asp Ser Phe
210 215 220

Pro Ala Phe Gln Pro Ala Pro Pro Pro Ala Thr Ala Met Met Val Pro
225 230 235 240

Lys Lys Glu Ser Met Asp Asp Ala Thr Ala Ala Ala Ala Ala Ala Ala
245 250 255

Thr Ile Pro Arg Asn Asn Ser Ser Leu Phe Val Asp Leu Ser Tyr Asp
260 265 270

Asp Ile Gln Gly Met Tyr Ser Gly Leu Asp Met Leu Pro Pro Gly Asp
275 280 285

Asp Phe Tyr Ser Ser Leu Phe Ala Ser Pro Arg Val Lys Gly Thr Thr
290 295 300

Pro Arg Ala Gly Ala Gly Met Gly Met Val Pro Phe
305 310 315

<210> 52
<211> 1423
<212> DNA
<213> Oryza sativa

<400> 52
tcgacccacg cgtccgctct tcccaacact agtaggataa agccacagag agagcagtag 60

tagtagcgag ctcgccggag aacggacgat caccggagaa gggggagaga gatgagcggc 120

ggtcaggacc tgcagctgcc gccggggttc cggttccacc cgacggacga ggagctggtg 180

atgcactacc tctgccgccg ctgcgccggc ctccccatcg ccgtccccat catcgccgag 240

atcgacctct acaagttcga tccatggcag cttccccgga tggcgctgta cggagagaag 300

gagtggtact tcttctcccc gcgagaccgc aagtacccga acgggtcgcg gccgaaccgc 360

gccgccgggt cggggtactg gaaggcgacc ggcgccgaca agccggtggg ctcgccgaag 420

ccggtggcga tcaagaaggc cctcgtcttc tacgccggca aggcgcccaa gggcgagaag 480

accaactgga tcatgcacga gtaccgcctc gccgacgtcg accgctccgc ccgcaagaag 540

aacagcctca ggttggatga ttgggtgctg tgccggattt acaacaagaa gggcgggctg 600

gagaagccgc cggccgcggc ggtggcggcg gcggggatgg tgagcagcgg cggcggcgtc 660

cagaggaagc cgatggtggg ggtgaacgcg gcggtgagct ccccgccgga gcagaagccg 720

gtggtggcgg ggccggcgtt cccggacctg gcggcgtact acgaccggcc gtcggactcg 780

atgccgcggc tgcacgccga ctcgagctgc tcggagcagg tgctgtcgcc ggagttcgcg 840

tgcgaggtgc agagccagcc caagatcagc gagtgggagc gcaccttcgc caccgtcggg 900

cccatcaacc ccgccgcctc catcctcgac cccgccggct ccggcggcct cggcggcctc 960

ggcggcggcg gcagcgaccc cctcctccag gacatcctca tgtactgggg caagccattc 1020

tagacgacca aaaaaaaaaa aaaacaaccg cattggcagc aatggtgtca ctgaacaccg 1080

tgcaggctag ctagcttcat ggccggtgaa ctttgactca ggcgagccgc cggagttgac 1140

tcaaagataa ttaaaagaag tgttttaagt ggattggatt ggattagaca gaggagatga 1200

ggactcgaga aaggcggcga tgagaccgtg gttgggggga ccctggcctg gactgaacga 1260

cgacgaggca gcagcagaaa gatggtgcaa ttgcatcggg tggcatgtca gtgtgtgtgt 1320

atagtggcat gtacatagta catggtgatt gattcggtat acaggggggct agctttcctg 1380

tttctgttta aaaaaaaaaa aaaaaaaaaa aaaaaaaaaa aaa 1423

<210> 53
<211> 303
<212> PRT
<213> Oryza sativa

<400> 53

Met Ser Gly Gly Gln Asp Leu Gln Leu Pro Pro Gly Phe Arg Phe His
1 5 10 15

Pro Thr Asp Glu Glu Leu Val Met His Tyr Leu Cys Arg Arg Cys Ala
20 25 30

Gly Leu Pro Ile Ala Val Pro Ile Ile Ala Glu Ile Asp Leu Tyr Lys
35 40 45

Phe Asp Pro Trp Gln Leu Pro Arg Met Ala Leu Tyr Gly Glu Lys Glu
50 55 60

Trp Tyr Phe Phe Ser Pro Arg Asp Arg Lys Tyr Pro Asn Gly Ser Arg
65 70 75 80

Pro Asn Arg Ala Ala Gly Ser Gly Tyr Trp Lys Ala Thr Gly Ala Asp
85 90 95

Lys Pro Val Gly Ser Pro Lys Pro Val Ala Ile Lys Lys Ala Leu Val
100 105 110

Phe Tyr Ala Gly Lys Ala Pro Lys Gly Glu Lys Thr Asn Trp Ile Met
115 120 125

His Glu Tyr Arg Leu Ala Asp Val Asp Arg Ser Ala Arg Lys Lys Asn
130 135 140

Ser Leu Arg Leu Asp Asp Trp Val Leu Cys Arg Ile Tyr Asn Lys Lys

145 150 155 160

Gly Gly Leu Glu Lys Pro Pro Ala Ala Ala Val Ala Ala Ala Gly Met
165 170 175

Val Ser Ser Gly Gly Gly Val Gln Arg Lys Pro Met Val Gly Val Asn
180 185 190

Ala Ala Val Ser Ser Pro Pro Glu Gln Lys Pro Val Val Ala Gly Pro
195 200 205

Ala Phe Pro Asp Leu Ala Ala Tyr Tyr Asp Arg Pro Ser Asp Ser Met
210 215 220

Pro Arg Leu His Ala Asp Ser Ser Cys Ser Glu Gln Val Leu Ser Pro
225 230 235 240

Glu Phe Ala Cys Glu Val Gln Ser Gln Pro Lys Ile Ser Glu Trp Glu
245 250 255

Arg Thr Phe Ala Thr Val Gly Pro Ile Asn Pro Ala Ala Ser Ile Leu
260 265 270

Asp Pro Ala Gly Ser Gly Gly Leu Gly Gly Leu Gly Gly Gly Gly Ser
275 280 285

Asp Pro Leu Leu Gln Asp Ile Leu Met Tyr Trp Gly Lys Pro Phe
290 295 300

<210> 54
<211> 1751
<212> DNA
<213> Oryza sativa

<400> 54
cccctcgagg tcgacccacg cgtccgcttc cattagcgac taatccagcc ttcgttaagg 60

gtgattagaa tttgattaat ttcaaggcct cagatcggag attgaatgga gtgcggtggt 120

gcgctgcagc tgccgccggg gttcaggttc cacccgacgg acgacgagct ggtgatgtac 180

tacctgtgcc gcaagtgcgg cggcctcccc ctcgccgccc cggtgatcgc cgaggtggac 240

ctctacaagt tcaacccatg ggatctcccc gagagggcga tgggagggga gaaggagtgg 300

tacttcttct cgccgcggga ccggaagtac ccgaacgggc agcggccgaa cagggcggcg 360

gggacggggt actggaaggc gacgggggcg gacaagccgg tggggtcgcc gcgggcggtg 420

gcgatcaaga aggcgctcgt cttctacgcc gggaagccgc ccaagggcgt caagaccaac 480

tggatcatgc acgagtaccg cctcgccgac gtcgaccgct ccgccgccgc ccgcaagctc 540

tccaagtcct cccacaacgc cctcaggttg gatgattggg tgttgtgccg aatctacaac 600

aagaagggag tgatcgagag gtacgacacg gtggacgccg gcgaggacgt gaagccggcg 660
gcggcggcgg cggcggcgaa gggtggtcgc atcggcggcg gcggcggcgc ggcggcgatg 720
aaggtggagc tctccgacta tgggttctac gaccaggagc cggagtcgga gatgctgtgc 780
ttcgaccggt cggggtcggc ggaccgcgac tcgatgccgc ggctgcacac cgactccagc 840
gggtcggagc acgtgctgtc gccgtcgccg tcgccggacg acttccccgg cggcggcgac 900
cacgactacg ccgagagcca gcccagcggc ggatgcggcg ggtggcccgg cgtcgactgg 960
gccgccgtcg gcgacgatgg cttcgtcatc gacagctccc tcttcgagct gccctcgccg 1020
gcggcgttct cccgcgccgc cggcgacggc gccgccttcg gcgacatgtt cacgtacctg 1080
cagaagccgt tctaattaac ggaacgtgac gcctctgcaa acgctaatta gctcactaac 1140
cactgtcagg tcgatcgtgt aattctttta ccagtctagg gtacagccaa aaaaccaaaa 1200
attaaccgtg ctcgatcgat cgatcgatcc atggatcgat gatcgcctcc accggcagat 1260
caaaatcgaa gcaaaaaatc agggagaatt cttggtactt ccagtacctc tcgtcgacac 1320
cacgtgtcgg gttttctgca ccgcggatcc tcgctggccg catcgtaacg gcgagcgagg 1380
ttaaaaatag tggagaattc aagaacacat ccctgatttt tgtttctgcc ggatggggag 1440
taccgtgagc atggtagaaa tgactggtaa aattttcgtt gatgataggt agccagagtc 1500
gatgtaacca gtcgattttt tttttttttg gttttagttg acatgccttc ctgatagatt 1560
cagaacaaac aatagagaag agaggagaaa aaaacacaga ggagaaattc agagaccaaa 1620
gttcaaagtt ttggaatttt caggggcgcc agcagctgat ggtattgtca gtcgtgtcta 1680
ggttcgtaga ggaaattaaa atgtagaaga acgggtactt cagttcactt caaaaaaaaa 1740
aaaaaaaaaa a 1751

<210> 55
<211> 329
<212> PRT
<213> Oryza sativa

<400> 55

Met Glu Cys Gly Gly Ala Leu Gln Leu Pro Pro Gly Phe Arg Phe His
1 5 10 15

Pro Thr Asp Asp Glu Leu Val Met Tyr Tyr Leu Cys Arg Lys Cys Gly
20 25 30

Gly Leu Pro Leu Ala Ala Pro Val Ile Ala Glu Val Asp Leu Tyr Lys
35 40 45

Phe Asn Pro Trp Asp Leu Pro Glu Arg Ala Met Gly Gly Glu Lys Glu
50 55 60

Trp Tyr Phe Phe Ser Pro Arg Asp Arg Lys Tyr Pro Asn Gly Gln Arg

65 70 75 80

Pro Asn Arg Ala Ala Gly Thr Gly Tyr Trp Lys Ala Thr Gly Ala Asp
85 90 95

Lys Pro Val Gly Ser Pro Arg Ala Val Ala Ile Lys Lys Ala Leu Val
100 105 110

Phe Tyr Ala Gly Lys Pro Pro Lys Gly Val Lys Thr Asn Trp Ile Met
115 120 125

His Glu Tyr Arg Leu Ala Asp Val Asp Arg Ser Ala Ala Ala Arg Lys
130 135 140

Leu Ser Lys Ser Ser His Asn Ala Leu Arg Leu Asp Asp Trp Val Leu
145 150 155 160

Cys Arg Ile Tyr Asn Lys Lys Gly Val Ile Glu Arg Tyr Asp Thr Val
165 170 175

Asp Ala Gly Glu Asp Val Lys Pro Ala Ala Ala Ala Ala Ala Ala Lys
180 185 190

Gly Gly Arg Ile Gly Gly Gly Gly Gly Ala Ala Ala Met Lys Val Glu
195 200 205

Leu Ser Asp Tyr Gly Phe Tyr Asp Gln Glu Pro Glu Ser Glu Met Leu
210 215 220

Cys Phe Asp Arg Ser Gly Ser Ala Asp Arg Asp Ser Met Pro Arg Leu
225 230 235 240

His Thr Asp Ser Ser Gly Ser Glu His Val Leu Ser Pro Ser Pro Ser
245 250 255

Pro Asp Asp Phe Pro Gly Gly Gly Asp His Asp Tyr Ala Glu Ser Gln
260 265 270

Pro Ser Gly Gly Cys Gly Gly Trp Pro Gly Val Asp Trp Ala Ala Val
275 280 285

Gly Asp Asp Gly Phe Val Ile Asp Ser Ser Leu Phe Glu Leu Pro Ser
290 295 300

Pro Ala Ala Phe Ser Arg Ala Ala Gly Asp Gly Ala Ala Phe Gly Asp
305 310 315 320

Met Phe Thr Tyr Leu Gln Lys Pro Phe
325

<210> 56
<211> 1336
<212> DNA
<213> Oryza sativa

<400> 56
atcctccaca agagaaacta ggatcttcaa gtatagctag cattgcgccc tcatttcatc 60

catggcaatg cagctgtcct tgcctgtctt gccaacgggt tttcgtttcc atccaaccga 120

tgaggaacta gtcatcaact acctccagag gcgtgctacc gggctatcgt gccccatccc 180

cataatcgcg gatgtcgaaa tatacaactt caacccgtgg gagcttccat ccatggctct 240

atttggggaa catgagtggt actttttac actacgcgac cacaggtacc ccaatagtgt 300

gcgccctagt cgctcagcgg cgtcaggctt ttggaaggcc accggcactg acaagcccgt 360

ccaagttgct aacatgcaaa gcactcctgt agctatgaag aaggcacttg tattctatgt 420

tggccgcccg cccatggaaa ccaagactac atggatcatg catgagtacc gtctcacaaa 480

cacgggaggg tccactgcct cccacccatc cttgtcttca tccaccgcac acccttctgt 540

gaagctagac gagtgggtgc tatgcaagat cttcaacaag tccccagagc cggacaacac 600

cgcaccacca tcaaacgtcg tctcacggtt acagtgctcg ccgccgctgc cgccgccggc 660

ggcaccgccc ggcaactacc cgccgctgcc ggtgggtgcg acgaacgacg gcggcgtgtt 720

cgcctgcgcc ggcgacatgc tcttcaccat ccaagagcac caggagggaa caccatcgat 780

gctgccgccg atccctaacc ttgagccacc ggcggcaaca attgggaatt cctctctcaa 840

tggcactgct gctgctgctg ctgctgctga tggccatggc cgccttgagg aagaggacac 900

cagcgcctac accttcaccg accaggaaat ggagcagatg ctcatggacc tgatggacca 960

ggatttcttt ggcaatgatc aaccccagga gtgaactgtg tggtggagtg attttcatat 1020

cattccatag ctctgatctg tgctggagag tgaatttagt atctattgta ccagatagaa 1080

taaaaccaga aaagaaataa aagaaggcca aagaaaaaag caaaaagaaa attgtataat 1140

tactaggttt ggatgccgtc tgtagaacct tgtgtgctat gtaacttttt tctttgtatt 1200

ttggcatttt catcatattg tgtcataaat aaagcagggc tttcttttgt agtttgccat 1260

ctcaatcttg caccgtgatg ttataaaaaa acaaaattaa ttattgtgct acactgtact 1320

tttggctgtg ttcggc 1336

<210> 57
<211> 310
<212> PRT
<213> Oryza sativa

<400> 57

Met Ala Met Gln Leu Ser Leu Pro Val Leu Pro Thr Gly Phe Arg Phe
1 5 10 15

```
His Pro Thr Asp Glu Glu Leu Val Ile Asn Tyr Leu Gln Arg Arg Ala
            20                  25                  30

Thr Gly Leu Ser Cys Pro Ile Pro Ile Ile Ala Asp Val Glu Ile Tyr
        35                  40                  45

Asn Phe Asn Pro Trp Glu Leu Pro Ser Met Ala Leu Phe Gly Glu His
    50                  55                  60

Glu Trp Tyr Phe Phe Thr Leu Arg Asp His Arg Tyr Pro Asn Ser Val
65                  70                  75                  80

Arg Pro Ser Arg Ser Ala Ala Ser Gly Phe Trp Lys Ala Thr Gly Thr
                85                  90                  95

Asp Lys Pro Val Gln Val Ala Asn Met Gln Ser Thr Pro Val Ala Met
            100                 105                 110

Lys Lys Ala Leu Val Phe Tyr Val Gly Arg Pro Pro Met Glu Thr Lys
        115                 120                 125

Thr Thr Trp Ile Met His Glu Tyr Arg Leu Thr Asn Thr Gly Gly Ser
    130                 135                 140

Thr Ala Ser His Pro Ser Leu Ser Ser Ser Thr Ala His Pro Ser Val
145                 150                 155                 160

Lys Leu Asp Glu Trp Val Leu Cys Lys Ile Phe Asn Lys Ser Pro Glu
                165                 170                 175

Pro Asp Asn Thr Ala Pro Pro Ser Asn Val Val Ser Arg Leu Gln Cys
            180                 185                 190

Ser Pro Pro Leu Pro Pro Pro Ala Ala Pro Pro Gly Asn Tyr Pro Pro
        195                 200                 205

Leu Pro Val Gly Ala Thr Asn Asp Gly Gly Val Phe Ala Cys Ala Gly
    210                 215                 220

Asp Met Leu Phe Thr Ile Gln Glu His Gln Glu Gly Thr Pro Ser Met
225                 230                 235                 240

Leu Pro Pro Ile Pro Asn Leu Glu Pro Pro Ala Ala Thr Ile Gly Asn
                245                 250                 255

Ser Ser Leu Asn Gly Thr Ala Ala Ala Ala Ala Ala Ala Asp Gly His
            260                 265                 270
```

Gly Arg Leu Glu Glu Glu Asp Thr Ser Ala Tyr Thr Phe Thr Asp Gln
        275                 280                 285

Glu Met Glu Gln Met Leu Met Asp Leu Met Asp Gln Asp Phe Phe Gly
    290                 295                 300

Asn Asp Gln Pro Gln Glu
305                 310

<210> 58
<211> 1176
<212> DNA
<213> Oryza sativa

<400> 58
atgccgagca gcggcggcgc catgcctgcc cttccaccag gcttccgctt ccacccccacc 60

gacgaggagc tcatcgttca ctacctcatg aaccaggccg cctccgtcaa gtgccccgtg 120

ccaatcatcg ccgaggtcaa catctacaag tgcaacccat gggaccttcc tggtaaggct 180

ttgttcggcg agaacgaatg gtacttcttc agcccgaggg accgcaagta ccccaacggc 240

gctcgcccca accgcgccgc cggctcgggg tactggaagg ccaccggcac cgacaagtcc 300

atcctctcca ctccgaccag cgacaacatc ggcgtcaaga aggccctcgt cttctacaag 360

ggcaagcctc ccaagggcgt caagaccgac tggatcatgc acgagtaccg tctcaccggc 420

acatcagcta acagcaccac caccacaaag cagcgtagag cgtcatccat gaccatgagg 480

ctggacgact gggtgctgtg cagaatccac aagaagagca acgacttcaa ttcctctgac 540

caacacgacc aagaacccga ggaatcaacc gtcgaacagc ttgaagacat ccatgacaac 600

aactcctctg aacaacctcc agctccagct gacatgaaca accaacagtc agatttccag 660

cccatgacgg cgatgagcat gagcaagtca tgctccctca ccgatctcct caacaccatc 720

gactgcgccg cgctctcgca gtttctcctc gacggctcat ccgacgccat cgctgagcct 780

cctgctcctc ccagcccccct aatatacaca acacctcatc caaattacca aacactaaac 840

tataacatta acagcaacag cagcatgcca cacgccttcg agtcacgcct agatcatcac 900

gatggttacg ttaacaatta taatgttaat ggcctgagga ggaagagaat gatggcgtgt 960

agtgcaactt cctttgatga tggcagcagc agcaatgact ttgtgcatgc cgttgtcaag 1020

aaaccgcagc tgctgccaag tgattcgagg ggtagtggtt ttggaggagg ttactgcaac 1080

cagcagcttt cagagactgc gactggcttt cagtttcaga acggcaatct gctgagccat 1140

ccatttcctc tgaacaatca tctgcagatg cagtag 1176

<210> 59
<211> 391
<212> PRT
<213> Oryza sativa

<400> 59

Met Pro Ser Ser Gly Gly Ala Met Pro Ala Leu Pro Pro Gly Phe Arg
1               5                   10                  15

Phe His Pro Thr Asp Glu Glu Leu Ile Val His Tyr Leu Met Asn Gln
            20                  25                  30

Ala Ala Ser Val Lys Cys Pro Val Pro Ile Ile Ala Glu Val Asn Ile
        35                  40                  45

Tyr Lys Cys Asn Pro Trp Asp Leu Pro Gly Lys Ala Leu Phe Gly Glu
    50                  55                  60

Asn Glu Trp Tyr Phe Phe Ser Pro Arg Asp Arg Lys Tyr Pro Asn Gly
65                  70                  75                  80

Ala Arg Pro Asn Arg Ala Ala Gly Ser Gly Tyr Trp Lys Ala Thr Gly
                85                  90                  95

Thr Asp Lys Ser Ile Leu Ser Thr Pro Thr Ser Asp Asn Ile Gly Val
            100                 105                 110

Lys Lys Ala Leu Val Phe Tyr Lys Gly Lys Pro Pro Lys Gly Val Lys
        115                 120                 125

Thr Asp Trp Ile Met His Glu Tyr Arg Leu Thr Gly Thr Ser Ala Asn
    130                 135                 140

Ser Thr Thr Thr Thr Lys Gln Arg Arg Ala Ser Ser Met Thr Met Arg
145                 150                 155                 160

Leu Asp Asp Trp Val Leu Cys Arg Ile His Lys Lys Ser Asn Asp Phe
                165                 170                 175

Asn Ser Ser Asp Gln His Asp Gln Glu Pro Glu Glu Ser Thr Val Glu
            180                 185                 190

Gln Leu Glu Asp Ile His Asp Asn Asn Ser Ser Glu Gln Pro Pro Ala
        195                 200                 205

Pro Ala Asp Met Asn Asn Gln Gln Ser Asp Phe Gln Pro Met Thr Ala
    210                 215                 220

Met Ser Met Ser Lys Ser Cys Ser Leu Thr Asp Leu Leu Asn Thr Ile
225                 230                 235                 240

Asp Cys Ala Ala Leu Ser Gln Phe Leu Leu Asp Gly Ser Ser Asp Ala
                245                 250                 255

```
Ile Ala Glu Pro Pro Ala Pro Pro Ser Pro Leu Ile Tyr Thr Thr Pro
            260                 265                 270

His Pro Asn Tyr Gln Thr Leu Asn Tyr Asn Ile Asn Ser Asn Ser Ser
        275                 280                 285

Met Pro His Ala Phe Glu Ser Arg Leu Asp His His Asp Gly Tyr Val
    290                 295                 300

Asn Asn Tyr Asn Val Asn Gly Leu Arg Arg Lys Arg Met Met Ala Cys
305                 310                 315                 320

Ser Ala Thr Ser Phe Asp Asp Gly Ser Ser Ser Asn Asp Phe Val His
                325                 330                 335

Ala Val Val Lys Lys Pro Gln Leu Leu Pro Ser Asp Ser Arg Gly Ser
            340                 345                 350

Gly Phe Gly Gly Gly Tyr Cys Asn Gln Gln Leu Ser Glu Thr Ala Thr
        355                 360                 365

Gly Phe Gln Phe Gln Asn Gly Asn Leu Leu Ser His Pro Phe Pro Leu
    370                 375                 380

Asn Asn His Leu Gln Met Gln
385                 390

<210>  60
<211>  1450
<212>  DNA
<213>  Triticum aestivum

<400>  60
acgaggcact cgccccaatc tcgaacaacg gccgtcggat ccatcatcat cggcagcgga      60

gcgattccat cgaccagctt tctacagacg gacatgggga tgccggccgt gaggaggagg     120

gagagggacg cggaggcgga gctcaacctg ccccccggct tccgcttcca ccccaccgac     180

gacgagctcg tcgagcacta cctctgccgc aaggcggcgg ggcagcgcct cccggtcccc     240

atcatcgccg aggtcgacct ctaccgcttc gacccctggg cgctccctga ccgcgccctc     300

ttcggcaccc gcgagtggta cttcttcacc ccccgcgacc gcaagtaccc caacggctcc     360

cgacccaacc gcgccgccgg caacggctac tggaaggcca ccggcgccga caagcccgtc     420

gcgccccgcg gcgggcggac catggggatc aagaaggccc tcgtgtttta cgcgggcaag     480

gcgcctaagg gggtcaagac cgactggatc atgcatgagt atcgcctcgc cgacgccggc     540

cgcgccgccg ccagcaagaa gggctcgctc aggctggacg actgggtgct ctgccggctc     600

tacaacaaga agaacgagtg ggagaagatg cagctgcagc agcaagggga agaggagacg     660

atgatggagc ccaaggcgga gaacacggcc tccgacatgg tggtcacctc gcactcccac     720
```

tcgcagtccc agtcgcactc gcactcgtgg ggcgaggcgc gcacgccgga gtcggagatc 780

gtcgacaacg acccgtcgct gttccagcag gcgacggcgg cggcgttcca ggcccagagc 840

cccgcggccg ccgcggcgca ccaggagatg atggccacgc tgatggtgcc caagaaggag 900

gcggcggacg aggccggcag gaacgacctc ttcgtcgacc tcagctacga cgacatccag 960

agcatgtaca acggcctcga catgatgccg cccggggacg acctgctcta ctcctccctc 1020

ttcgcctccc cccgtgtccg cgggagccag cccggcgccg gcggcatgcc ggccccgttc 1080

taagcagagc aaagacagag accgtcagag accccgagat cgacagaagt ggaatggacg 1140

acttcttggc cgcgagcgag cgagaccgcg gtgcagtgta aatacagcat aggaaacggg 1200

agggagggag gtggcgtcgt gtcgagagaa gccggcgtcg tgccggggcg tgccgttgta 1260

catggagagc ccggcgccgg ggcctggccg gctgggttga ttcttttgtt cttactacct 1320

tgtactctca atgcggatgt agctctttct tctcactcct cactagtaga atcgaccgac 1380

cgaatacata tgtagctctg ttctttcctt ctcttcagta gaaatcaacc aaattttgct 1440

gttatgctgc 1450

<210> 61
<211> 329
<212> PRT
<213> Triticum aestivum

<400> 61

Met Gly Met Pro Ala Val Arg Arg Arg Glu Arg Asp Ala Glu Ala Glu
1 5 10 15

Leu Asn Leu Pro Pro Gly Phe Arg Phe His Pro Thr Asp Asp Glu Leu
20 25 30

Val Glu His Tyr Leu Cys Arg Lys Ala Ala Gly Gln Arg Leu Pro Val
35 40 45

Pro Ile Ile Ala Glu Val Asp Leu Tyr Arg Phe Asp Pro Trp Ala Leu
50 55 60

Pro Asp Arg Ala Leu Phe Gly Thr Arg Glu Trp Tyr Phe Phe Thr Pro
65 70 75 80

Arg Asp Arg Lys Tyr Pro Asn Gly Ser Arg Pro Asn Arg Ala Ala Gly
85 90 95

Asn Gly Tyr Trp Lys Ala Thr Gly Ala Asp Lys Pro Val Ala Pro Arg
100 105 110

Gly Gly Arg Thr Met Gly Ile Lys Lys Ala Leu Val Phe Tyr Ala Gly
115 120 125

Lys Ala Pro Lys Gly Val Lys Thr Asp Trp Ile Met His Glu Tyr Arg
    130                 135                 140

Leu Ala Asp Ala Gly Arg Ala Ala Ala Ser Lys Lys Gly Ser Leu Arg
145                 150                 155                 160

Leu Asp Asp Trp Val Leu Cys Arg Leu Tyr Asn Lys Lys Asn Glu Trp
                165                 170                 175

Glu Lys Met Gln Leu Gln Gln Gln Gly Glu Glu Glu Thr Met Met Glu
            180                 185                 190

Pro Lys Ala Glu Asn Thr Ala Ser Asp Met Val Val Thr Ser His Ser
        195                 200                 205

His Ser Gln Ser Gln Ser His Ser His Ser Trp Gly Glu Ala Arg Thr
    210                 215                 220

Pro Glu Ser Glu Ile Val Asp Asn Asp Pro Ser Leu Phe Gln Gln Ala
225                 230                 235                 240

Thr Ala Ala Ala Phe Gln Ala Gln Ser Pro Ala Ala Ala Ala Ala His
                245                 250                 255

Gln Glu Met Met Ala Thr Leu Met Val Pro Lys Lys Glu Ala Ala Asp
            260                 265                 270

Glu Ala Gly Arg Asn Asp Leu Phe Val Asp Leu Ser Tyr Asp Asp Ile
        275                 280                 285

Gln Ser Met Tyr Asn Gly Leu Asp Met Met Pro Pro Gly Asp Asp Leu
    290                 295                 300

Leu Tyr Ser Ser Leu Phe Ala Ser Pro Arg Val Arg Gly Ser Gln Pro
305                 310                 315                 320

Gly Ala Gly Gly Met Pro Ala Pro Phe
                325

<210>  62
<211>  1141
<212>  DNA
<213>  Zea mays

<220>
<221>  variation
<222>  (1084)..(1084)
<223>  /replace="t" /replace="c" /replace="g"

<400> 62
gcacgaggaa cccgcccaca ggacaggaca cacagagccg agccacccca cccaccatcg 60

gcgaccagca gccagccgcg ggagcgagct gtttaaagac accgagtcgg agtcggacgg 120

aggactggca ggcacaaccg aagccaccgc ttctagttct cgggttcatc gccagcaatc 180

cagaccacat aatgggacag ccggtgacga ggaggaggga gagggacgcg gaggcggagc 240

tggacctgcc gccggggttc cggttccacc ccacagacga cgagctggtg gagcactacc 300

tgtgccgcaa ggcggcgggg cagcgcctcc ccgtgcccat catcgccgag gtggacctgt 360

acaggttcga cccgtgggac ctgccggagc gcgcgctctt cggggcccgg gagtggtact 420

tcttcacgcc cagggaccgc aagtacccca acggctcccg ccccaaccgc gccgccggcg 480

acgggtactg gaaggccacc ggcgccgaca agcccgtcgc gccgcgcgcc gccgccgccg 540

acgcccgcac gctcgggatc aagaaggcgc tcgtcttcta cgccggcaag gcgccgcgcg 600

gggtcaagac agactggatc atgcacgagt acaggctcgc tgacgccggc ggacgcgcca 660

agaaggggtc gctcaggttg gatgactggg tgctgtgccg gctgtacaac aagaagaacg 720

agtgggagaa gatgcggctg ggaaaggggg cagccgccgg cgcagtcaaa gaggaggagg 780

ccatggacat gagcacctcc cactcgcaga tcacccactc gtggggcgag acgcgcacgc 840

cggagtcgga gatcgtggac aacgacctgc cgttcccgga tccggcgatg atggtgccca 900

agaaggagcg ggtggacgac ggcggcagcg ccaggaccag cgacctgttc gtggatctca 960

gctacgacga catccaaggc atgtacagcg gcctcgacat gctgccgccg gccggcgagg 1020

acttgtactc ctcgctcttc gcgtcgccca gggtcagggg gaaccagccc accggacccg 1080

cggagttggg ccccttctga gtgagctcag gcgaagatgg aggcatggag atcaggagag 1140

a 1141

<210> 63
<211> 302
<212> PRT
<213> Zea mays

<220>
<221> UNSURE
<222> (298)..(298)

<400> 63

Met Gly Gln Pro Val Thr Arg Arg Arg Glu Arg Asp Ala Glu Ala Glu
1 5 10 15

Leu Asp Leu Pro Pro Gly Phe Arg Phe His Pro Thr Asp Asp Glu Leu
20 25 30

Val Glu His Tyr Leu Cys Arg Lys Ala Ala Gly Gln Arg Leu Pro Val
35 40 45

```
Pro Ile Ile Ala Glu Val Asp Leu Tyr Arg Phe Asp Pro Trp Asp Leu
    50                  55                  60

Pro Glu Arg Ala Leu Phe Gly Ala Arg Glu Trp Tyr Phe Phe Thr Pro
65                  70                  75                  80

Arg Asp Arg Lys Tyr Pro Asn Gly Ser Arg Pro Asn Arg Ala Ala Gly
                85                  90                  95

Asp Gly Tyr Trp Lys Ala Thr Gly Ala Asp Lys Pro Val Ala Pro Arg
            100                 105                 110

Ala Ala Ala Ala Asp Ala Arg Thr Leu Gly Ile Lys Lys Ala Leu Val
        115                 120                 125

Phe Tyr Ala Gly Lys Ala Pro Arg Gly Val Lys Thr Asp Trp Ile Met
    130                 135                 140

His Glu Tyr Arg Leu Ala Asp Ala Gly Gly Arg Ala Lys Lys Gly Ser
145                 150                 155                 160

Leu Arg Leu Asp Asp Trp Val Leu Cys Arg Leu Tyr Asn Lys Lys Asn
                165                 170                 175

Glu Trp Glu Lys Met Arg Leu Gly Lys Gly Ala Ala Ala Gly Ala Val
            180                 185                 190

Lys Glu Glu Glu Ala Met Asp Met Ser Thr Ser His Ser Gln Ile Thr
        195                 200                 205

His Ser Trp Gly Glu Thr Arg Thr Pro Glu Ser Glu Ile Val Asp Asn
    210                 215                 220

Asp Leu Pro Phe Pro Asp Pro Ala Met Met Val Pro Lys Lys Glu Arg
225                 230                 235                 240

Val Asp Asp Gly Gly Ser Ala Arg Thr Ser Asp Leu Phe Val Asp Leu
                245                 250                 255

Ser Tyr Asp Asp Ile Gln Gly Met Tyr Ser Gly Leu Asp Met Leu Pro
            260                 265                 270

Pro Ala Gly Glu Asp Leu Tyr Ser Ser Leu Phe Ala Ser Pro Arg Val
        275                 280                 285

Arg Gly Asn Gln Pro Thr Gly Pro Ala Xaa Leu Gly Pro Phe
    290                 295                 300
```

<210> 64
<211> 1090
<212> DNA
<213> Triticum aestivum

<400> 64
aattcggcac gagacagtcc accacgcacg tgcagcagca ccagcgcccg agaatcccat 60

tcccatcgac ggagaagaag aagtgaagaa acaatggtga tggcagcggc ggagcggcgg 120

gacgcggagg cggagctgaa cctgccgccg gggttccggt tccacccgac ggacgaggag 180

ctggtggcgg actacctctg cgcgcgcgcg gccggccgcg cgccgccggt gcccatcatc 240

gccgagctcg acctctaccg gttcgacccg tgggagctcc cggagcgggc gctcttcggg 300

gcgcgggagt ggtacttctt cacgccgcgg gaccgcaagt accccaacgg ctcccgcccc 360

aaccgggccg ccgggggcgg ctactggaag gccaccggcg ccgacaggcc cgtggcgcgc 420

gcgggcagga ccgtcgggat caagaaggcg ctcgtcttct accacggcag gccgtcggcg 480

ggggtcaaga cggactggat catgcacgag taccgcctcg ccggcgccga cggacgcgcc 540

gccaagaacg gcggcacgct caggcttgac gaatgggtgc tctgccgcct atacaacaag 600

aagaaccagt gggagaagat gcagcggcag cggcaggagg aggaggcggc ggccaaggct 660

gcggcgtcac agtcggtctc ctggggtgag acgcggacgc cggagtccga cgtcgacaac 720

gatccgttcc cggagctgga ctcgctgccg gagttccaga cggcaaacgc gtcaatactg 780

cccaaggagg aggtgcagga gctgggcaac gacgactggc tcatggggat cagcctcgac 840

gacctgcagg gccccggctc cctgatgctg ccctgggacg actcctacgc cgcctcgttc 900

ctgtcgccgg tggccacgat gaagatggag caggacgtca gcccattctt cttctgagct 960

ctcaatactc tcacggtcgc actgttgtgt gcggcgtaac tgtagatagt tcacatttgt 1020

tcaggattta tttgtaacgt tgcttctttt atacgatact ctcttccttt ctaaaaaaaa 1080

aaaaaaaaaa 1090

<210> 65
<211> 287
<212> PRT
<213> Triticum aestivum

<400> 65

Met Val Met Ala Ala Ala Glu Arg Arg Asp Ala Glu Ala Glu Leu Asn
1 5 10 15

Leu Pro Pro Gly Phe Arg Phe His Pro Thr Asp Glu Glu Leu Val Ala
20 25 30

Asp Tyr Leu Cys Ala Arg Ala Ala Gly Arg Ala Pro Pro Val Pro Ile
35 40 45

Ile Ala Glu Leu Asp Leu Tyr Arg Phe Asp Pro Trp Glu Leu Pro Glu

50 55 60

Arg Ala Leu Phe Gly Ala Arg Glu Trp Tyr Phe Phe Thr Pro Arg Asp
65 70 75 80

Arg Lys Tyr Pro Asn Gly Ser Arg Pro Asn Arg Ala Ala Gly Gly Gly
85 90 95

Tyr Trp Lys Ala Thr Gly Ala Asp Arg Pro Val Ala Arg Ala Gly Arg
100 105 110

Thr Val Gly Ile Lys Lys Ala Leu Val Phe Tyr His Gly Arg Pro Ser
115 120 125

Ala Gly Val Lys Thr Asp Trp Ile Met His Glu Tyr Arg Leu Ala Gly
130 135 140

Ala Asp Gly Arg Ala Ala Lys Asn Gly Gly Thr Leu Arg Leu Asp Glu
145 150 155 160

Trp Val Leu Cys Arg Leu Tyr Asn Lys Lys Asn Gln Trp Glu Lys Met
165 170 175

Gln Arg Gln Arg Gln Glu Glu Glu Ala Ala Ala Lys Ala Ala Ala Ser
180 185 190

Gln Ser Val Ser Trp Gly Glu Thr Arg Thr Pro Glu Ser Asp Val Asp
195 200 205

Asn Asp Pro Phe Pro Glu Leu Asp Ser Leu Pro Glu Phe Gln Thr Ala
210 215 220

Asn Ala Ser Ile Leu Pro Lys Glu Glu Val Gln Glu Leu Gly Asn Asp
225 230 235 240

Asp Trp Leu Met Gly Ile Ser Leu Asp Asp Leu Gln Gly Pro Gly Ser
245 250 255

Leu Met Leu Pro Trp Asp Asp Ser Tyr Ala Ala Ser Phe Leu Ser Pro
260 265 270

Val Ala Thr Met Lys Met Glu Gln Asp Val Ser Pro Phe Phe Phe
275 280 285

<210> 66
<211> 1255
<212> DNA
<213> Oryza sativa

<400> 66

```
catccaacaa cccaccaccc tcattccctc aagtcccaag atcgaacacc tcgtgtccat      60
ggcggcggcg aagcggcgag tgcgcgacgc ggaggcggac ctgaacctcc cgccgggctt     120
ccgcttccac cccaccgacg aggagctggt ggcgcactac ctctgcccgc gcgccgcggg     180
ccgcgccgcc ccggtcccca tcatcgccga gctcgacctc taccgccacg acccatggga     240
cctcccccac cgcgccctct tcggccgccg cgagtggtac ttcttcaccc cgcgcgaccg     300
caagtacccc aacggctccc gccccaaccg cgccgccgcc tcgggctact ggaaggccac     360
cggcgccgac aagcccgtgc tgcacaacgg caggacggcc gggatcaaga aggcgctcgt     420
gttctaccac ggcaagcccc cccgcggcgt caagacggag tggatcatgc acgagtaccg     480
cctcgccaag aagggcggcg ccgccgccgc cgcgggcgcg ggcgcgctca ggctggatga     540
ctgggtgctg tgccggctgt acaacaagaa gaacgagtgg gagaagatgc agagcaggaa     600
ggaggaggag gaggccatgg cggcggcgca gtcgtggggg gagacgcgga cgccggagtc     660
ggaggtcgtc gacagcgacg cgttcccgga gatggactac tcgctgccgg cggcgtcgtt     720
cgacgacgcc ctgctgccca aggaggaggc gcgcgacgac gactggctca tggggatgag     780
cctcgacgac ctccagggcc tcggctcgct gctgcaggcc gacgacctct ccatgctcgc     840
gccgccgccg gcggcgaaga cggagccgct cggcgcgcca ttcttctgag ctctctctct     900
ctctctctct ctctctctct ctgtgactgc accactgtat ataaattcag agttttcaga     960
catgttcagt attcagagtt ctcaggcaag ttcagaattc agagatggtt aaggattagt    1020
ggcttatgag cagtatgata tgcaggttag tttctagttt agcagtgtta ctccagattg    1080
gagatttgat taatgatttg attctaatta atgtagtata ctgagtgtta ctcatcatca    1140
cagattcatc agagctgtgt caagtgacaa ttcttttttt ttttatttcc tggatcaagt    1200
tcaccatgtt gtgctcaaga tttgtggata aatgcacatc catccttgaa ttggc         1255
```

```
<210>  67
<211>  276
<212>  PRT
<213>  Oryza sativa

<400>  67

Met Ala Ala Ala Lys Arg Arg Val Arg Asp Ala Glu Ala Asp Leu Asn
1               5                   10                  15

Leu Pro Pro Gly Phe Arg Phe His Pro Thr Asp Glu Glu Leu Val Ala
            20                  25                  30

His Tyr Leu Cys Pro Arg Ala Ala Gly Arg Ala Ala Pro Val Pro Ile
        35                  40                  45

Ile Ala Glu Leu Asp Leu Tyr Arg His Asp Pro Trp Asp Leu Pro His
    50                  55                  60
```

Arg Ala Leu Phe Gly Arg Arg Glu Trp Tyr Phe Phe Thr Pro Arg Asp
65 70 75 80

Arg Lys Tyr Pro Asn Gly Ser Arg Pro Asn Arg Ala Ala Ala Ser Gly
85 90 95

Tyr Trp Lys Ala Thr Gly Ala Asp Lys Pro Val Leu His Asn Gly Arg
100 105 110

Thr Ala Gly Ile Lys Lys Ala Leu Val Phe Tyr His Gly Lys Pro Pro
115 120 125

Arg Gly Val Lys Thr Glu Trp Ile Met His Glu Tyr Arg Leu Ala Lys
130 135 140

Lys Gly Gly Ala Ala Ala Ala Ala Gly Ala Gly Ala Leu Arg Leu Asp
145 150 155 160

Asp Trp Val Leu Cys Arg Leu Tyr Asn Lys Lys Asn Glu Trp Glu Lys
165 170 175

Met Gln Ser Arg Lys Glu Glu Glu Glu Ala Met Ala Ala Ala Gln Ser
180 185 190

Trp Gly Glu Thr Arg Thr Pro Glu Ser Glu Val Val Asp Ser Asp Ala
195 200 205

Phe Pro Glu Met Asp Tyr Ser Leu Pro Ala Ala Ser Phe Asp Asp Ala
210 215 220

Leu Leu Pro Lys Glu Glu Ala Arg Asp Asp Asp Trp Leu Met Gly Met
225 230 235 240

Ser Leu Asp Asp Leu Gln Gly Leu Gly Ser Leu Leu Gln Ala Asp Asp
245 250 255

Leu Ser Met Leu Ala Pro Pro Pro Ala Ala Lys Thr Glu Pro Leu Gly
260 265 270

Ala Pro Phe Phe
275

<210> 68
<211> 1683
<212> DNA
<213> Elaeis guineensis

<400> 68
aggcagaaat ctcaccgccg cctcagccat ttctaaagct tggtacaagg aacggtaatc 60

ctccaggcaa tcgagttgcg aggacgctga caatggggag caggagaaga gatgctgagg 120

cggagctcaa cctgccgccg ggattccggt tccaccccgac cgacgaggag ctcgtcgttc 180

attacctctg caagaaggtg gcctgccagc gcctgcccgt gcccatcatc gccgaggtcg 240

atctctacaa atatgaccca tgggatctgc cagagaaggc gttgttcggg ctaaaagagt 300

ggtatttctt cacccctcgt gatcgaaagt acccgaatgg ctcgaggcct aacagggccg 360

ccgggagggg gtactggaag gcgaccggtg ctgataaacc cgtgaccccg aaggggagca 420

gtaggcctct cgggatcaag aaagccttgg tgttttactc tgggaaggca ccaagaggag 480

tgaagactga ttggatcatg catgagtaca ggcttgccga cacgaaccga gcagccaaca 540

agaagggaag cctaaggctt gatgactggg ttctttgccg gttgtacaac aagaagaaca 600

cctgggagaa gatgcagcaa caaaaggagg cagcatcgtt tggagagacg atggattctg 660

tggatgacac aggatcggac agcttcagaa caccccgaatc ggacatagat aacgatgtca 720

tgttcccgga ctttgacgat atggctcagg ttggagctca cccacctcaa gctttcaatg 780

gaatgcaagg agtacgagtc aacaatataa ctgggtatca gccagcaata gagcagcgtc 840

caaaagagga gaatgactgg ttcacggacc tgaatctgga tgacttccac agtacatgca 900

tggcttttgg atcaacacct gctctcgata tgtcggacca tgattactac tactcaaacc 960

ttccacaact gagatcaaac cagagtgacc tgctaccctt ctaaagctac gaatcccagc 1020

tgtatataag tgaaaatagg caggaggtga caatctagtg gtagaactct gtaggattct 1080

tttgtttcca ccaattttat ataggtaaat ccattcataa gcaagggaca gtcgctgatg 1140

tacatatgat aatattattt tgtggtagac cttctgtttg aattagtttt acaaattttg 1200

agcattgtct agtggtagaa tatcttcgtt tcgattacag aaacttgcct cagcaacagg 1260

aaacatattc cacacacaca cacacacaaa aaaagaaaag cagcagcagg aatcagaata 1320

agttatagat ggtgggataa tcttaagtga cagtatataa cactgcgcac aaaaaggtgg 1380

acagtgagaa ctcaaaggag aatcagccgt ggcatgcggt catagttatt cgttcagaga 1440

gacagtttat agcatctgct tgggatcttt gtttagtgcg acacaaccaa cagtaggaac 1500

aaagaatatc tgtggacatc cacaagtcca gggtagtatc gaggaaacaa gcaacgttgt 1560

gtcgatttga caagagtgac cctccaaatg ttaagttttc tggaagaggg gaaagaagtg 1620

gaagcataag cgcatgcata agcaagcaca tcaccagcga aagcaaaaaa aaaaaaaaaa 1680

aaa 1683

<210> 69
<211> 303
<212> PRT
<213> Elaeis guineensis

<400> 69

Met Gly Ser Arg Arg Arg Asp Ala Glu Ala Glu Leu Asn Leu Pro Pro

```
1               5                   10                  15

Gly Phe Arg Phe His Pro Thr Asp Glu Glu Leu Val Val His Tyr Leu
            20                  25                  30

Cys Lys Lys Val Ala Cys Gln Arg Leu Pro Val Pro Ile Ile Ala Glu
        35                  40                  45

Val Asp Leu Tyr Lys Tyr Asp Pro Trp Asp Leu Pro Glu Lys Ala Leu
    50                  55                  60

Phe Gly Leu Lys Glu Trp Tyr Phe Phe Thr Pro Arg Asp Arg Lys Tyr
65                  70                  75                  80

Pro Asn Gly Ser Arg Pro Asn Arg Ala Ala Gly Arg Gly Tyr Trp Lys
                85                  90                  95

Ala Thr Gly Ala Asp Lys Pro Val Thr Pro Lys Gly Ser Ser Arg Pro
            100                 105                 110

Leu Gly Ile Lys Lys Ala Leu Val Phe Tyr Ser Gly Lys Ala Pro Arg
        115                 120                 125

Gly Val Lys Thr Asp Trp Ile Met His Glu Tyr Arg Leu Ala Asp Thr
    130                 135                 140

Asn Arg Ala Ala Asn Lys Lys Gly Ser Leu Arg Leu Asp Asp Trp Val
145                 150                 155                 160

Leu Cys Arg Leu Tyr Asn Lys Lys Asn Thr Trp Glu Lys Met Gln Gln
                165                 170                 175

Gln Lys Glu Ala Ala Ser Phe Gly Glu Thr Met Asp Ser Val Asp Asp
            180                 185                 190

Thr Gly Ser Asp Ser Phe Arg Thr Pro Glu Ser Asp Ile Asp Asn Asp
        195                 200                 205

Val Met Phe Pro Asp Phe Asp Asp Met Ala Gln Val Gly Ala His Pro
    210                 215                 220

Pro Gln Ala Phe Asn Gly Met Gln Gly Val Arg Val Asn Asn Ile Thr
225                 230                 235                 240

Gly Tyr Gln Pro Ala Ile Glu Gln Arg Pro Lys Glu Glu Asn Asp Trp
                245                 250                 255

Phe Thr Asp Leu Asn Leu Asp Asp Phe His Ser Thr Cys Met Ala Phe
            260                 265                 270
```

Gly Ser Thr Pro Ala Leu Asp Met Ser Asp His Asp Tyr Tyr Tyr Ser
275 280 285

Asn Leu Pro Gln Leu Arg Ser Asn Gln Ser Asp Leu Leu Pro Phe
290 295 300

<210> 70
<211> 1264
<212> DNA
<213> Oryza sativa

<400> 70
gcagcatcaa caaaggcagc agcagcagca gcagcagcag cagcgttggt ggtggtggtg 60

tgtcatcacc aacattttca cgagaggaga aggatggaaa tggcggcggc ggttgggggc 120

agcgggagga gggacgcgga ggcggagctg aacctgccgc cgggcttccg tttccacccg 180

accgacgagg agctcgtggt gcactacctc tgccgcaagg ttgcccggca gccgctcccc 240

gtcccaatca tcgccgaggt cgacctctac aagctcgacc cctgggatct ccctgagaag 300

gcgttgttcg ggaggaaaga gtggtacttc ttcacgccga gggaccggaa gtacccgaac 360

gggtcgaggc cgaaccgcgc agcggggaga gggtactgga aggcgacggg agccgacaag 420

ccggtggcgc ccaaggggag cgcgaggacg gtggggatca agaaggcgct cgtgttctac 480

tccgggaagg cgccgagggg ggtcaagacg gactggatca tgcacgagta ccgcctcgcc 540

gacgccgacc gcgccccggg cggcaagaag ggctcacaga agctggacga gtgggtgctg 600

tgccggctgt acaacaagaa gaacaactgg gagaaggtga agctggagca gcaggacgtg 660

gcctccgtgg cggcggcggc gccgcgcaac caccaccatc agaacggcga ggtcatggac 720

gcggcggcgg ctgacaccat gtccgacagc ttccagacgc acgactccga catcgacaac 780

gcctccgccg gcctgcggca cggtggctgc ggcggcggcg gcttcggcga cgtggcgccg 840

ccgaggaatg ggttcgtgac ggtgaaggag gacaacgact ggttcaccgg cctcaacttc 900

gacgagctgc agccgccgta catgatgaac ctgcagcaca tgcagatgca gatggtgaat 960

ccggcggcgc cagggcacga cggcggctac ttgcagtcca tcagctcgcc gcagatgaag 1020

atgtggcaga caatcctgcc accattctga gatggatgga gcaagaaaaa ggttgctgta 1080

gataaagggc ggaaatagga gtgatggcta gaaaattatt agatttacta gaacgaaaat 1140

gattagaaat ctggcaagca tgattctgca aatgtggtgg tagatgcttg cagtatgtaa 1200

ttcatttgtt cagtatatgc atttggtaat ctgcaaaaca aaaaaaaaaa aaaaaaaaaa 1260

aaaa 1264

<210> 71
<211> 316
<212> PRT
<213> Oryza sativa

<400> 71

Met Ala Ala Ala Val Gly Gly Ser Gly Arg Arg Asp Ala Glu Ala Glu
1 5 10 15

Leu Asn Leu Pro Pro Gly Phe Arg Phe His Pro Thr Asp Glu Glu Leu
20 25 30

Val Val His Tyr Leu Cys Arg Lys Val Ala Arg Gln Pro Leu Pro Val
35 40 45

Pro Ile Ile Ala Glu Val Asp Leu Tyr Lys Leu Asp Pro Trp Asp Leu
50 55 60

Pro Glu Lys Ala Leu Phe Gly Arg Lys Glu Trp Tyr Phe Phe Thr Pro
65 70 75 80

Arg Asp Arg Lys Tyr Pro Asn Gly Ser Arg Pro Asn Arg Ala Ala Gly
85 90 95

Arg Gly Tyr Trp Lys Ala Thr Gly Ala Asp Lys Pro Val Ala Pro Lys
100 105 110

Gly Ser Ala Arg Thr Val Gly Ile Lys Lys Ala Leu Val Phe Tyr Ser
115 120 125

Gly Lys Ala Pro Arg Gly Val Lys Thr Asp Trp Ile Met His Glu Tyr
130 135 140

Arg Leu Ala Asp Ala Asp Arg Ala Pro Gly Gly Lys Lys Gly Ser Gln
145 150 155 160

Lys Leu Asp Glu Trp Val Leu Cys Arg Leu Tyr Asn Lys Lys Asn Asn
165 170 175

Trp Glu Lys Val Lys Leu Glu Gln Gln Asp Val Ala Ser Val Ala Ala
180 185 190

Ala Ala Pro Arg Asn His His His Gln Asn Gly Glu Val Met Asp Ala
195 200 205

Ala Ala Ala Asp Thr Met Ser Asp Ser Phe Gln Thr His Asp Ser Asp
210 215 220

Ile Asp Asn Ala Ser Ala Gly Leu Arg His Gly Gly Cys Gly Gly Gly
225 230 235 240

Gly Phe Gly Asp Val Ala Pro Pro Arg Asn Gly Phe Val Thr Val Lys
245 250 255

Glu Asp Asn Asp Trp Phe Thr Gly Leu Asn Phe Asp Glu Leu Gln Pro
260 265 270

Pro Tyr Met Met Asn Leu Gln His Met Gln Met Gln Met Val Asn Pro
275 280 285

Ala Ala Pro Gly His Asp Gly Gly Tyr Leu Gln Ser Ile Ser Ser Pro
290 295 300

Gln Met Lys Met Trp Gln Thr Ile Leu Pro Pro Phe
305 310 315

<210> 72
<211> 1129
<212> DNA
<213> Glycine max

<400> 72
ttccagagtt ggtgttagtc ttggtgtagt gtagctaggg agggatatat atctgagtaa 60

gatggcatca gagcttgaat tgcccccagg cttcagattc catccaacgg acgaggagct 120

ggtgttgcac tatctctgcc gcaaatgcgc gtcgcagcca atcgccgttc ccatcatcgc 180

cgaaatcgac ctctacaaat acgacccctg ggacttaccc ggattggcta cttatggaga 240

gaaagagtgg tacttctttt caccacggga ccggaaatac ccaaacggtt cgaggccgaa 300

ccgggcggct ggcaccggtt actggaaggc aaccggggcg gataagccca ttggtcagcc 360

caaaccggtt gggattaaaa aagctttggt gttttacgca gggaaagctc ctaaagggga 420

caaaagcaat tggatcatgc acgagtatcg tctcgcagac gtagatcgct ccgttcgcaa 480

aaagaacacc ctaaggttgg atgattgggt gctttgccgt atttacaaca agaagggcac 540

gatcgagaaa ctgcaaccaa gcagcgatgt tgctcatagc cgaaatatcg aatcctcgga 600

gatcgaagac aggaagccgg agattctgaa aagcggagga ggttgtcttc cgccgcctgc 660

gccggtgcct gcgccgccgc aagcgacggc gaagacggat tacatgtact tcgacccgtc 720

ggattcaatc ccgaagctgc acacggactc gagctgttcg gagcaggtgg tatcgccggg 780

attcgcgagc gaggtgcaaa gcgagcccaa gtggaacgag tgggagaaaa gcctcgaatt 840

tccatttaat tacgtggatg ccactctcaa caacagcttc atggcccaat tccagggcaa 900

taatcagatg ttgtcgccgc tgcaggacat gttcatgtac tggcccaaca agtccttttg 960

agcacatcat gatggtttta aattacgatc cacaattgct cttaagattt tctgactcac 1020

catgcgacca caatcgtaat ttaaaaccat gtcacataaa atccacgcgc gccgcccatt 1080

cgcatttgca cggtagcacg agactcaagg tccatgagtg tgcctgtaa 1129

<210> 73
<211> 299

<212> PRT
<213> Glycine max

<400> 73

Met Ala Ser Glu Leu Glu Leu Pro Pro Gly Phe Arg Phe His Pro Thr
1 5 10 15

Asp Glu Glu Leu Val Leu His Tyr Leu Cys Arg Lys Cys Ala Ser Gln
20 25 30

Pro Ile Ala Val Pro Ile Ile Ala Glu Ile Asp Leu Tyr Lys Tyr Asp
35 40 45

Pro Trp Asp Leu Pro Gly Leu Ala Thr Tyr Gly Glu Lys Glu Trp Tyr
50 55 60

Phe Phe Ser Pro Arg Asp Arg Lys Tyr Pro Asn Gly Ser Arg Pro Asn
65 70 75 80

Arg Ala Ala Gly Thr Gly Tyr Trp Lys Ala Thr Gly Ala Asp Lys Pro
85 90 95

Ile Gly Gln Pro Lys Pro Val Gly Ile Lys Lys Ala Leu Val Phe Tyr
100 105 110

Ala Gly Lys Ala Pro Lys Gly Asp Lys Ser Asn Trp Ile Met His Glu
115 120 125

Tyr Arg Leu Ala Asp Val Asp Arg Ser Val Arg Lys Lys Asn Thr Leu
130 135 140

Arg Leu Asp Asp Trp Val Leu Cys Arg Ile Tyr Asn Lys Lys Gly Thr
145 150 155 160

Ile Glu Lys Leu Gln Pro Ser Ser Asp Val Ala His Ser Arg Asn Ile
165 170 175

Glu Ser Ser Glu Ile Glu Asp Arg Lys Pro Glu Ile Leu Lys Ser Gly
180 185 190

Gly Gly Cys Leu Pro Pro Pro Ala Pro Val Pro Ala Pro Pro Gln Ala
195 200 205

Thr Ala Lys Thr Asp Tyr Met Tyr Phe Asp Pro Ser Asp Ser Ile Pro
210 215 220

Lys Leu His Thr Asp Ser Ser Cys Ser Glu Gln Val Val Ser Pro Gly
225 230 235 240

Phe Ala Ser Glu Val Gln Ser Glu Pro Lys Trp Asn Glu Trp Glu Lys
245 250 255

Ser Leu Glu Phe Pro Phe Asn Tyr Val Asp Ala Thr Leu Asn Asn Ser
260 265 270

Phe Met Ala Gln Phe Gln Gly Asn Asn Gln Met Leu Ser Pro Leu Gln
275 280 285

Asp Met Phe Met Tyr Trp Pro Asn Lys Ser Phe
290 295

<210> 74
<211> 1304
<212> DNA
<213> Lycopersicon esculentum

<400> 74
ggcacgagca aagcaggagc aggagcagca acaaacagag agaagaaaac agaggaagat 60

aagaggaaaa tttatcgaat tcgaatcgag agaaaagggg aagtgaagtt gcgaagagtg 120

agaatttcaa aggaaatgaa caaaggagca aacggaaatc agcaattgga gttaccggcg 180

ggattcagat tccatccgac agacgacgaa ttggtgcagc actatctctg caggaaatgc 240

gccggacagt cgattgctgt atcaattata gctgaaattg atctttacaa gtttgatcca 300

tggcagttgc ctgagaaggc tttgtacggt gaaaaagagt ggtatttttt ctcaccaagg 360

gatagaaaat atccgaacgg ttcacggccg aaccgagcag caggaaccgg ttattggaag 420

gcaaccggag ctgataaacc ggtgggaaaa cccaaaacct tagggataaa gaaggcactt 480

gtgttctatg ccggaaaagc acccagaggt ataaaaacaa attggattat gcacgagtac 540

cgcctcgcca acgtggaccg ctctgctggc aagaacaata acttgaggct tgatgattgg 600

gtattgtgtc gaatatacaa caagaaaggc acacttgaga agcattacaa tgtggacaac 660

aaggaaacta caagctttgg agaatttgat gaagaaataa aaccaaaaat attgcccaca 720

caattagcac cgatgccacc acggcctcga tcgacaccag caaacgacta cttttatttc 780

gagtcatcag agtcgatgac tagaatgcac acgacaaact cgagctctgg ctcagagcat 840

gtcttgtcgc catgtgacaa ggaggttcag agcgcgccca aatgggacga agaccacaga 900

aacacccttg attttcagct aaactatttg gatggtttac taaatgaacc atttgaaacc 960

caaatgcagc agcaaatttg caactttgac cagttcaaca atttccaaga catgttccta 1020

tacatgcaaa aaccttacta aaattgtata aattcattgg atctaaattg agtgtgatcc 1080

atgacatttt ctttgttctt tggtggtgta ggtcaacttt ttattaagta gtttagagaa 1140

gtacaaaatg ctagtcaaat ttggtgggct acagcacaaa tgagccttga taagcatagc 1200

caaagagtcg tatagaaggg cttattatta ttgtaaggta tgtaaaaaca aatgaaaatt 1260

tgttaatatc aagttatcat tcttcaaaaa aaaaaaaaaa aaaa 1304

<210> 75
<211> 301
<212> PRT
<213> Lycopersicon esculentum

<400> 75

Met Asn Lys Gly Ala Asn Gly Asn Gln Gln Leu Glu Leu Pro Ala Gly
1               5                   10                  15

Phe Arg Phe His Pro Thr Asp Asp Glu Leu Val Gln His Tyr Leu Cys
            20                  25                  30

Arg Lys Cys Ala Gly Gln Ser Ile Ala Val Ser Ile Ile Ala Glu Ile
        35                  40                  45

Asp Leu Tyr Lys Phe Asp Pro Trp Gln Leu Pro Glu Lys Ala Leu Tyr
    50                  55                  60

Gly Glu Lys Glu Trp Tyr Phe Phe Ser Pro Arg Asp Arg Lys Tyr Pro
65                  70                  75                  80

Asn Gly Ser Arg Pro Asn Arg Ala Ala Gly Thr Gly Tyr Trp Lys Ala
                85                  90                  95

Thr Gly Ala Asp Lys Pro Val Gly Lys Pro Lys Thr Leu Gly Ile Lys
            100                 105                 110

Lys Ala Leu Val Phe Tyr Ala Gly Lys Ala Pro Arg Gly Ile Lys Thr
        115                 120                 125

Asn Trp Ile Met His Glu Tyr Arg Leu Ala Asn Val Asp Arg Ser Ala
    130                 135                 140

Gly Lys Asn Asn Asn Leu Arg Leu Asp Asp Trp Val Leu Cys Arg Ile
145                 150                 155                 160

Tyr Asn Lys Lys Gly Thr Leu Glu Lys His Tyr Asn Val Asp Asn Lys
                165                 170                 175

Glu Thr Thr Ser Phe Gly Glu Phe Asp Glu Glu Ile Lys Pro Lys Ile
            180                 185                 190

Leu Pro Thr Gln Leu Ala Pro Met Pro Pro Arg Pro Arg Ser Thr Pro
        195                 200                 205

Ala Asn Asp Tyr Phe Tyr Phe Glu Ser Ser Glu Ser Met Thr Arg Met
    210                 215                 220

His Thr Thr Asn Ser Ser Ser Gly Ser Glu His Val Leu Ser Pro Cys
225 230 235 240

Asp Lys Glu Val Gln Ser Ala Pro Lys Trp Asp Glu Asp His Arg Asn
245 250 255

Thr Leu Asp Phe Gln Leu Asn Tyr Leu Asp Gly Leu Leu Asn Glu Pro
260 265 270

Phe Glu Thr Gln Met Gln Gln Gln Ile Cys Asn Phe Asp Gln Phe Asn
275 280 285

Asn Phe Gln Asp Met Phe Leu Tyr Met Gln Lys Pro Tyr
290 295 300

<210> 76
<211> 1060
<212> DNA
<213> Brassica napus

<400> 76
cgaagaaaag agaagacttt tgaaaaaaaa aatgaaagca gggctaaact taccagcagg 60

attccgattc cacccaacgg acgaagagct tgtgcaattc tacctttgcc ggaaatgcgc 120

atcggaggag atctcagctc cggtcatcgc cgaaatcgat ctctacaagt tcaacccctg 180

ggagcttcct gagatgtctc tgtacggaga gaaagagtgg tacttcttct cgccacgaga 240

ccggaaatac ccaaacggtt cgcgtcctaa ccgggcggcg ggaaccggtt attggaaagc 300

caccggagct gataaaccga tcggtaaacc gaagacgctg ggtattaaga aagcgctcgt 360

gttctacgca gggaaagctc ccaaagggat taagacgaat tggattatgc acgagtatcg 420

cctcgctaat gtggacagat cagcttctgt taacaaaaag aacaaccttc gacttgatga 480

ttgggtgtta tgtcgaatct acaacaagaa agggaccatg gagaagtact accctgctga 540

tgagaaaccg atgaccgtga cggcggcttc atcgcctttc gatgcgtcgg actcgactta 600

cccgacgttg caagaggatg actcgagcag ctcggggggt cgcgtggtgt cgccggatgc 660

gcgggaggtg cagagcgagc ctaaatgggg ggagtttgag aatgcttttg atgcttccat 720

gttcggtggt ggctccatgg acttgctgca gagtgaagat tttgtgcctc agttcttgta 780

ccagccttct tatgatttca actcctggca ggaggatccg ccggagcaga aaccgttctt 840

gaattggagt tttgctccac aggggtgaaa aaggaagaga gtgaaaggtt ttttggtctg 900

tgttgtgata tgtgttagag agaagttctc aatcatcttt tgtttcttag tagtgagaga 960

aagattgtag agtgttgata gtttcttagc atcttcaatg tttcattggc aggtgaattc 1020

ttgttatttc atcagaaatt tatatgaaaa attatacctt 1060

<210> 77
<211> 278

<212> PRT
<213> Brassica napus

<400> 77

```
Met Lys Ala Gly Leu Asn Leu Pro Ala Gly Phe Arg Phe His Pro Thr
1               5                   10                  15

Asp Glu Glu Leu Val Gln Phe Tyr Leu Cys Arg Lys Cys Ala Ser Glu
            20                  25                  30

Glu Ile Ser Ala Pro Val Ile Ala Glu Ile Asp Leu Tyr Lys Phe Asn
        35                  40                  45

Pro Trp Glu Leu Pro Glu Met Ser Leu Tyr Gly Glu Lys Glu Trp Tyr
    50                  55                  60

Phe Phe Ser Pro Arg Asp Arg Lys Tyr Pro Asn Gly Ser Arg Pro Asn
65                  70                  75                  80

Arg Ala Ala Gly Thr Gly Tyr Trp Lys Ala Thr Gly Ala Asp Lys Pro
                85                  90                  95

Ile Gly Lys Pro Lys Thr Leu Gly Ile Lys Lys Ala Leu Val Phe Tyr
            100                 105                 110

Ala Gly Lys Ala Pro Lys Gly Ile Lys Thr Asn Trp Ile Met His Glu
        115                 120                 125

Tyr Arg Leu Ala Asn Val Asp Arg Ser Ala Ser Val Asn Lys Lys Asn
    130                 135                 140

Asn Leu Arg Leu Asp Asp Trp Val Leu Cys Arg Ile Tyr Asn Lys Lys
145                 150                 155                 160

Gly Thr Met Glu Lys Tyr Tyr Pro Ala Asp Glu Lys Pro Met Thr Val
                165                 170                 175

Thr Ala Ala Ser Ser Pro Phe Asp Ala Ser Asp Ser Thr Tyr Pro Thr
            180                 185                 190

Leu Gln Glu Asp Asp Ser Ser Ser Ser Gly Gly Arg Val Val Ser Pro
        195                 200                 205

Asp Ala Arg Glu Val Gln Ser Glu Pro Lys Trp Gly Glu Phe Glu Asn
    210                 215                 220

Ala Phe Asp Ala Ser Met Phe Gly Gly Gly Ser Met Asp Leu Leu Gln
225                 230                 235                 240
```

Ser Glu Asp Phe Val Pro Gln Phe Leu Tyr Gln Pro Ser Tyr Asp Phe
245 250 255

Asn Ser Trp Gln Glu Asp Pro Pro Glu Gln Lys Pro Phe Leu Asn Trp
260 265 270

Ser Phe Ala Pro Gln Gly
275

<210> 78
<211> 969
<212> DNA
<213> Oryza sativa

<400> 78
atgagcggcg gcggcgaagg ggcggcggcg gcggagaggc aggagctgca gctgccgccg 60

gggttcaggt tccacccgac ggacgaggag ctggtgatgc actacctctg ccggcggtgc 120

gccggcctcc ccatcgccgt ccccatcatc gccgaggtcg acctctacaa gttcgatcca 180

tggcatctcc caagaatggc gctgtacggc gagaaggagt ggtacttctt ctcccctcgg 240

gaccgcaagt acccgaacgg gtcgcggccg aaccgcgccg ccgggtccgg gtactggaag 300

gccaccggcg ccgacaagcc ggtgggcacg ccgaggccgg tggccatcaa gaaggcgctc 360

gtcttctacg ccggcaaggc gcccaagggc gacaagacca actggatcat gcacgagtac 420

cgcctcgccg acgtcgaccg ctccgcccgc aagaagaaca ccctccggct agatgattgg 480

gtcctgtgcc gaatctacaa caagaaaggc ggcgtggaga agccgagcgg cggcggcggc 540

ggcgaacgtt cgaatatgat gagccacggg gagaccgcgt cggcgggctc gccgccggag 600

cagaagccgg ccgtgctgcc gccgccgcca ccgccgtacg cggcggcggc gccgttctcg 660

gagctggcgg cgttctacga cgtgcggccg tcggactcgg tgccgcgggc gcacggcgcg 720

gactcgagct gctcggagca cgtgctgacg acgtcggcgt cgtccggcgg cgtcgtcgag 780

cggccggagg tgcagagcca gcccaagatc gccgagtggg agcgcacgtt cgccggcgcc 840

gccgccccgg ctggcgccgt cagcacggcc ggaccgattc tgggccagct cgaccccgcc 900

gccgccgtcg ccggcggcgg cgacccgctc ctccaggaca tcctcatgta ctggggcaag 960

ccgttctga 969

<210> 79
<211> 322
<212> PRT
<213> Oryza sativa

<400> 79

Met Ser Gly Gly Gly Glu Gly Ala Ala Ala Ala Glu Arg Gln Glu Leu
1 5 10 15

Gln Leu Pro Pro Gly Phe Arg Phe His Pro Thr Asp Glu Glu Leu Val

```
            20                  25                  30

Met His Tyr Leu Cys Arg Arg Cys Ala Gly Leu Pro Ile Ala Val Pro
        35                  40                  45

Ile Ile Ala Glu Val Asp Leu Tyr Lys Phe Asp Pro Trp His Leu Pro
    50                  55                  60

Arg Met Ala Leu Tyr Gly Glu Lys Glu Trp Tyr Phe Phe Ser Pro Arg
65                  70                  75                  80

Asp Arg Lys Tyr Pro Asn Gly Ser Arg Pro Asn Arg Ala Ala Gly Ser
                85                  90                  95

Gly Tyr Trp Lys Ala Thr Gly Ala Asp Lys Pro Val Gly Thr Pro Arg
            100                 105                 110

Pro Val Ala Ile Lys Lys Ala Leu Val Phe Tyr Ala Gly Lys Ala Pro
        115                 120                 125

Lys Gly Asp Lys Thr Asn Trp Ile Met His Glu Tyr Arg Leu Ala Asp
    130                 135                 140

Val Asp Arg Ser Ala Arg Lys Lys Asn Thr Leu Arg Leu Asp Asp Trp
145                 150                 155                 160

Val Leu Cys Arg Ile Tyr Asn Lys Lys Gly Gly Val Glu Lys Pro Ser
                165                 170                 175

Gly Gly Gly Gly Gly Glu Arg Ser Asn Met Met Ser His Gly Glu Thr
            180                 185                 190

Ala Ser Ala Gly Ser Pro Pro Glu Gln Lys Pro Ala Val Leu Pro Pro
        195                 200                 205

Pro Pro Pro Pro Tyr Ala Ala Ala Ala Pro Phe Ser Glu Leu Ala Ala
    210                 215                 220

Phe Tyr Asp Val Arg Pro Ser Asp Ser Val Pro Arg Ala His Gly Ala
225                 230                 235                 240

Asp Ser Ser Cys Ser Glu His Val Leu Thr Thr Ser Ala Ser Ser Gly
                245                 250                 255

Gly Val Val Glu Arg Pro Glu Val Gln Ser Gln Pro Lys Ile Ala Glu
            260                 265                 270

Trp Glu Arg Thr Phe Ala Gly Ala Ala Ala Pro Ala Gly Ala Val Ser
        275                 280                 285
```

```
Thr Ala Gly Pro Ile Leu Gly Gln Leu Asp Pro Ala Ala Ala Val Ala
    290                 295                 300

Gly Gly Gly Asp Pro Leu Leu Gln Asp Ile Leu Met Tyr Trp Gly Lys
305                 310                 315                 320

Pro Phe
```

<210> 80
<211> 1295
<212> DNA
<213> Solanum tuberosum

<400> 80

```
ggtcaaacaa ctgaaactaa caaagcagca gcagcagcaa caaacagaga agacaacaga     60
ggaagataaa caaaggaaat taagagggag atttatcgaa tcgaaaggga aagggaagtt    120
acgaagagtg agaatttgaa ggaaatgaac aaaggagcaa ccggaaatca gcaattggag    180
ttaccggcgg gattcagatt ccatccgaca gacgacgaat tggtgcagca ttatctctgc    240
aggaaatgtg ccggacagcc aattgcagta tcaattataa ctgaaattga tctttacaag    300
tttgatccat ggcagttgcc tgaaaaggct ttgtacggtg aaaaggagtg gtattttttc    360
tcaccaaggg atagaaaata tcctaacggt tcacggccga accgagcagc aggaaccggt    420
tattggaagg caaccggagc agataaaccg gtgggaaaac ccaaaacatt agggataaag    480
aaggcacttg tgttttatgc tggaaaagca cctagaggaa taaaaaccaa ttggattatg    540
catgagtacc ggctcgccaa tgtggaccgg tctgctggca agaacaataa cttgaggctt    600
gatgattggg tattgtgtcg aatttacaac aagaaaggca cacttgagaa gcattacaat    660
gtggacaaca aggaaactgc aagctttgga gaatttgatg aagaaataaa accaaaaata    720
ttgcccacac aattagcaca gatgccacca cggccccgat cgacaccgac aaacgactac    780
ttccatttcg aatcatcgga gtcgatgact agaatgcaca ccacaaactc gagctctggc    840
tcagagcatg tcttgtcgcc atgtgacaag gaggttcaga gcgcgcccaa atgggacgaa    900
gaccacagaa acacccttga ttttcagcta aattatctgg atggtttact aaatgaacca    960
tttgaaaccc aaatgcagca gcaaagttgc aactttgacc agttcaacaa tttccaagac   1020
atgttctttt acatgcaaaa gccttactaa aattgtataa attcattgga tctaaattga   1080
ttgtgatcca tgacattttc tttgttcttt ggtggtgtag gtcaactttt attaattagt   1140
ttagaaaagt acaaaatgca agtcaaattt ggtgggctgt aatgagcctt tgataagcat   1200
agccaaagag tcatatagaa gggcttatta atgttattat tgtaaggtac atgtaaaaca   1260
aatgaaaatt tgttaatatc aagttatcat tcttc                              1295
```

<210> 81
<211> 301
<212> PRT
<213> Solanum tuberosum

<400> 81

Met Asn Lys Gly Ala Thr Gly Asn Gln Gln Leu Glu Leu Pro Ala Gly
1 5 10 15

Phe Arg Phe His Pro Thr Asp Asp Glu Leu Val Gln His Tyr Leu Cys
20 25 30

Arg Lys Cys Ala Gly Gln Pro Ile Ala Val Ser Ile Ile Thr Glu Ile
35 40 45

Asp Leu Tyr Lys Phe Asp Pro Trp Gln Leu Pro Glu Lys Ala Leu Tyr
50 55 60

Gly Glu Lys Glu Trp Tyr Phe Phe Ser Pro Arg Asp Arg Lys Tyr Pro
65 70 75 80

Asn Gly Ser Arg Pro Asn Arg Ala Ala Gly Thr Gly Tyr Trp Lys Ala
85 90 95

Thr Gly Ala Asp Lys Pro Val Gly Lys Pro Lys Thr Leu Gly Ile Lys
100 105 110

Lys Ala Leu Val Phe Tyr Ala Gly Lys Ala Pro Arg Gly Ile Lys Thr
115 120 125

Asn Trp Ile Met His Glu Tyr Arg Leu Ala Asn Val Asp Arg Ser Ala
130 135 140

Gly Lys Asn Asn Asn Leu Arg Leu Asp Asp Trp Val Leu Cys Arg Ile
145 150 155 160

Tyr Asn Lys Lys Gly Thr Leu Glu Lys His Tyr Asn Val Asp Asn Lys
165 170 175

Glu Thr Ala Ser Phe Gly Glu Phe Asp Glu Glu Ile Lys Pro Lys Ile
180 185 190

Leu Pro Thr Gln Leu Ala Gln Met Pro Pro Arg Pro Arg Ser Thr Pro
195 200 205

Thr Asn Asp Tyr Phe His Phe Glu Ser Ser Glu Ser Met Thr Arg Met
210 215 220

His Thr Thr Asn Ser Ser Ser Gly Ser Glu His Val Leu Ser Pro Cys
225 230 235 240

Asp Lys Glu Val Gln Ser Ala Pro Lys Trp Asp Glu Asp His Arg Asn
245 250 255

Thr Leu Asp Phe Gln Leu Asn Tyr Leu Asp Gly Leu Leu Asn Glu Pro
260 265 270

Phe Glu Thr Gln Met Gln Gln Gln Ser Cys Asn Phe Asp Gln Phe Asn
275 280 285

Asn Phe Gln Asp Met Phe Phe Tyr Met Gln Lys Pro Tyr
290 295 300

<210> 82
<211> 1447
<212> DNA
<213> Arabidopsis thaliana

<400> 82
cgattacaat cttgagcaga cgaagaaaac ccaaaaaaaa tccagatcca gctgaaattg 60

aattttcaac caacgaagaa gagatttttc caagagcaac agacaagaag aagagaatga 120

agtcggagct aaatttacca gctgggttcc gattccatcc aacggacgag gagcttgtga 180

aattctactt gtgccggaaa tgtgcttccg agcagatctc ggctccggtt atcgccgaga 240

ttgatctcta caagttcaat ccttgggagc ttccagagat gtctctgtac ggagagaaag 300

agtggtactt cttctcacct agagatcgga aatacccaaa cggttcgcgt cctaaccggg 360

cagcaggaac cggttattgg aaagctaccg gagcagataa accgattggt aaaccgaaga 420

cgttgggtat caagaaagca ctcgtcttct acgcagggaa agctccaaaa gggattaaga 480

ccaattggat aatgcatgag tatcgtctcg ctaatgttga tagatcagct tctgttaaca 540

aaaagaacaa cctacgactt gatgattggg ttttatgtcg aatatacaac aagaaaggaa 600

ccatggagaa gtatttcccc gcggatgaga agccgaggac cacgacaatg gctgaacagt 660

catcatcacc ttttgataca tcagactcga cttacccgac attgcaagag gatgattcca 720

gcagctcagg tggtcacggt cacgtggtgt caccggatgt tttggaggtt cagagcgagc 780

ctaaatgggg agagcttgag gatgctttgg aagcttttga tacttcaatg tttggtagtt 840

ccatggagtt gttgcagcct gacgcttttg tccctcagtt cttgtatcag tctgattatt 900

tcacttcctt ccaggatccg cctgagcaga aaccattctt gaattggagt tttgctccac 960

aggggtaaaa acggaagaga ccaaaaaagg tgtttgctag tagtactgtg atgtgccaga 1020

gagaagagtc tcatctcaac tcatccctgg ctcttagtag taaaagaaga ttgtagaatg 1080

ttaatagctt ttagcatcaa tgtctcatta gcaggcacat tcttgttctt tcatgagaag 1140

tttatatgaa aactaaaaat ttatattcaa attcttcaag atgttgcact tatgtagata 1200

ctgatattaa ataacaacct aacctttatg agatatcatg ctctccggaa gcattttttg 1260

atgagtatca tcagctgaat cgaggcgatt tcctcacagc tgttattcct atgtctctga 1320

gacttttgat tctcgttttc ttgacctgag gtttcttttt ggaagacttc tctctttcct 1380

tctgttcaag ctgtgtggag ggtttttcag atccattatt cccacgagga gtcttcattt 1440

tgggatc 1447

<210> 83
<211> 283
<212> PRT
<213> Arabidopsis thaliana

<400> 83

Met Lys Ser Glu Leu Asn Leu Pro Ala Gly Phe Arg Phe His Pro Thr
1 5 10 15

Asp Glu Glu Leu Val Lys Phe Tyr Leu Cys Arg Lys Cys Ala Ser Glu
20 25 30

Gln Ile Ser Ala Pro Val Ile Ala Glu Ile Asp Leu Tyr Lys Phe Asn
35 40 45

Pro Trp Glu Leu Pro Glu Met Ser Leu Tyr Gly Glu Lys Glu Trp Tyr
50 55 60

Phe Phe Ser Pro Arg Asp Arg Lys Tyr Pro Asn Gly Ser Arg Pro Asn
65 70 75 80

Arg Ala Ala Gly Thr Gly Tyr Trp Lys Ala Thr Gly Ala Asp Lys Pro
85 90 95

Ile Gly Lys Pro Lys Thr Leu Gly Ile Lys Lys Ala Leu Val Phe Tyr
100 105 110

Ala Gly Lys Ala Pro Lys Gly Ile Lys Thr Asn Trp Ile Met His Glu
115 120 125

Tyr Arg Leu Ala Asn Val Asp Arg Ser Ala Ser Val Asn Lys Lys Asn
130 135 140

Asn Leu Arg Leu Asp Asp Trp Val Leu Cys Arg Ile Tyr Asn Lys Lys
145 150 155 160

Gly Thr Met Glu Lys Tyr Phe Pro Ala Asp Glu Lys Pro Arg Thr Thr
165 170 175

Thr Met Ala Glu Gln Ser Ser Ser Pro Phe Asp Thr Ser Asp Ser Thr
180 185 190

```
Tyr Pro Thr Leu Gln Glu Asp Asp Ser Ser Ser Ser Gly Gly His Gly
        195                 200                 205

His Val Val Ser Pro Asp Val Leu Glu Val Gln Ser Glu Pro Lys Trp
    210                 215                 220

Gly Glu Leu Glu Asp Ala Leu Glu Ala Phe Asp Thr Ser Met Phe Gly
225                 230                 235                 240

Ser Ser Met Glu Leu Leu Gln Pro Asp Ala Phe Val Pro Gln Phe Leu
                245                 250                 255

Tyr Gln Ser Asp Tyr Phe Thr Ser Phe Gln Asp Pro Pro Glu Gln Lys
            260                 265                 270

Pro Phe Leu Asn Trp Ser Phe Ala Pro Gln Gly
        275                 280


<210>  84
<211>  1211
<212>  DNA
<213>  Petunia x hybrida


<220>
<221>  misc_feature
<222>  (1006)..(1006)
<223>  n is a, c, g, or t

<220>
<221>  misc_feature
<222>  (1131)..(1131)
<223>  n is a, c, g, or t

<220>
<221>  misc_feature
<222>  (1154)..(1154)
<223>  n is a, c, g, or t

<220>
<221>  misc_feature
<222>  (1157)..(1157)
<223>  n is a, c, g, or t

<220>
<221>  misc_feature
<222>  (1187)..(1187)
<223>  n is a, c, g, or t

<220>
<221>  misc_feature
<222>  (1194)..(1194)
<223>  n is a, c, g, or t

<220>
<221>  misc_feature
<222>  (1199)..(1199)
<223>  n is a, c, g, or t
```

<400> 84

cccattctca gtatctagac acccaaaaat aaaaataaaa agatttatta ttttttcatc 60

aagaaattcg ttagaaaaat caaaaaatga caacagcaga attgcaatta cctccagggt 120

ttagatttca cccaactgat gaagaacttg tgatgcacta tttatgtaga aaatgtgctt 180

cacaaccaat tgctgtacca attattgctg aaattgatct ctacaaatat gacccatggg 240

atcttcctga tttggcattg tatggggaga aagaatggta tttcttttca cctagggacc 300

gaaagtatcc gaacggttca cgaccaaatc gagcagctgg aacaggttat tggaaagcca 360

ctggagctga taaaccaatt ggacatccta aggcagttgg aattaaaaag gcattggtgt 420

tttacgctgg caaggctccg aaaggcgaga aaacaaattg gattatgcac gaatacagac 480

ttgctgatgt tgatcgatct gctcgtaaga ataacaatag cttaaggcta gatgattggg 540

ttttgtgccg aatctacaac aaaaagggct caattgaaaa gaatcaactc aataacaaga 600

aaataatgaa tactagctat atggatatga cagtatcaag tgaagaagac cgaaagccag 660

agattctacc accgctacca cctcagcctg cgccgcaaca gcaacaagtt tataacgatt 720

ttttctacct ggatccatca gattcagtac caaaaatcca ctcagattca agctgctcgg 780

aacatgtggt ttcaccagag ttcacttgtg agagagaagt tcagagcgaa gccaagttaa 840

gtgagtggga aaaagctgcc cttgatcttc catttaatta catggatgcc actactggag 900

ctaccacact ggataatagc cttttaggtt cacagtttca gagcagttat cagatgtcgc 960

cattgcagga tatgttcatg cacctgcaca aacctttttta aggtcnaaag tgaaggaatc 1020

aatcatttta cggccgcatg tttgttaaaa tgagactaag ttaaagttcc cgtgacttgc 1080

gcacgttgcg ggctgtttat aggaattagt taccatcctt ttgggtttcc ncattgggca 1140

atttgtttaa aacnacnttt ttgtgttata tagttttaat aatgttnccc cctnaattna 1200

tttaaaaaaa a 1211

<210> 85
<211> 304
<212> PRT
<213> Petunia x hybrida

<400> 85

Met Thr Thr Ala Glu Leu Gln Leu Pro Pro Gly Phe Arg Phe His Pro
1 5 10 15

Thr Asp Glu Glu Leu Val Met His Tyr Leu Cys Arg Lys Cys Ala Ser
20 25 30

Gln Pro Ile Ala Val Pro Ile Ile Ala Glu Ile Asp Leu Tyr Lys Tyr
35 40 45

Asp Pro Trp Asp Leu Pro Asp Leu Ala Leu Tyr Gly Glu Lys Glu Trp
50 55 60

```
Tyr Phe Phe Ser Pro Arg Asp Arg Lys Tyr Pro Asn Gly Ser Arg Pro
65                  70                  75                  80

Asn Arg Ala Ala Gly Thr Gly Tyr Trp Lys Ala Thr Gly Ala Asp Lys
                85                  90                  95

Pro Ile Gly His Pro Lys Ala Val Gly Ile Lys Lys Ala Leu Val Phe
            100                 105                 110

Tyr Ala Gly Lys Ala Pro Lys Gly Glu Lys Thr Asn Trp Ile Met His
        115                 120                 125

Glu Tyr Arg Leu Ala Asp Val Asp Arg Ser Ala Arg Lys Asn Asn Asn
    130                 135                 140

Ser Leu Arg Leu Asp Asp Trp Val Leu Cys Arg Ile Tyr Asn Lys Lys
145                 150                 155                 160

Gly Ser Ile Glu Lys Asn Gln Leu Asn Asn Lys Lys Ile Met Asn Thr
                165                 170                 175

Ser Tyr Met Asp Met Thr Val Ser Ser Glu Glu Asp Arg Lys Pro Glu
            180                 185                 190

Ile Leu Pro Pro Leu Pro Pro Gln Pro Ala Pro Gln Gln Gln Gln Val
        195                 200                 205

Tyr Asn Asp Phe Phe Tyr Leu Asp Pro Ser Asp Ser Val Pro Lys Ile
    210                 215                 220

His Ser Asp Ser Ser Cys Ser Glu His Val Val Ser Pro Glu Phe Thr
225                 230                 235                 240

Cys Glu Arg Glu Val Gln Ser Glu Ala Lys Leu Ser Glu Trp Glu Lys
                245                 250                 255

Ala Ala Leu Asp Leu Pro Phe Asn Tyr Met Asp Ala Thr Thr Gly Ala
            260                 265                 270

Thr Thr Leu Asp Asn Ser Leu Leu Gly Ser Gln Phe Gln Ser Ser Tyr
        275                 280                 285

Gln Met Ser Pro Leu Gln Asp Met Phe Met His Leu His Lys Pro Phe
    290                 295                 300
```

<210> 86
<211> 1295
<212> DNA

<213>  Prunus mume

<400>  86
acacacagca cagtcgaatc aaacgacgcc gttcagaatg tcctcctccg atttacagct     60

gccgcccggc ttcaggttcc acccgacgga cgaagagctc gtgaagcact acctctgccg    120

caaatgccag tcgcagccga tttcggtccc gataatcgcc gaaatcgatc tctacaaata    180

caacccctgg gacctccctg gtttggcgtt gtacggtgag aaagagtggt atttcttttc    240

gccgagggac cggaagtatc cgaatggttc gaggccgaac cgggcggccg ggagcggcta    300

ttggaaggcg accggagcgg ataagccgat cgggagcccg aagccagtcg ggattaagaa    360

ggccctggtt ttctacgccg gaaaagctcc gagaggagag aagaccaatt ggattatgca    420

cgagtatcgc ctcgccgacg tcgaccgctc gcctcgcaaa aagagcagca gcctaaggtt    480

ggacgattgg gttctgtgtc gcatatacaa caaaaagggc accgtcgaga aacagcagca    540

gcagcaacaa cagcaacaac agcaaacgac gagtcgtatg agtagcggct cggaattcga    600

ggacaggaag ccggagaatc tggcgtgtcc tccgccgccg gctgcggtag cccccactcg    660

cccgaacgac tacgtgtact tcgacacgtc ggactctgtg ccgaggctgc acacggactc    720

aagctgctcg gagcacgtgg tgtcgccgga gttcacttgc gaggtgcaga gcgagcccaa    780

gtggaaggag tgggagaagg ccctcgattt taactacaat tacatggaca ccagtgtaga    840

gaacgggttc gggccgcagt tccagagcgc taatcagatg tcgccgctgc aggatatttt    900

catgtaccta cagaagccgt attgattttg atgaagcggg gcctacaatt gcattgctat    960

gagtcgccgc atcggacggc aacgggagac ccaggtcgat gagagtgcgt tttcgatgct   1020

gtgacaaggg aaagggaagg gggatttgcg tcgcggggtc caccatttgg gcccaaggct   1080

gggtgatttg ggagcgttga tataaagatg atatatatag atggaaaaaa gatgatgggg   1140

ctggttttgg aagatagaaa tatgaagaag tttggttttt aatgtgtaca gcatgtatgt   1200

atagatataa ataggtaatg ttgttcacat gatgattttg aagggccgtc taaatcgtaa   1260

tatttgttct ttgtgaaaaa aaaaaaaaaa aaaaa                               1295

<210>  87
<211>  295
<212>  PRT
<213>  Prunus mume

<400>  87

Met Ser Ser Ser Asp Leu Gln Leu Pro Pro Gly Phe Arg Phe His Pro
1               5                   10                  15

Thr Asp Glu Glu Leu Val Lys His Tyr Leu Cys Arg Lys Cys Gln Ser
            20                  25                  30

Gln Pro Ile Ser Val Pro Ile Ile Ala Glu Ile Asp Leu Tyr Lys Tyr
        35                  40                  45

```
Asn Pro Trp Asp Leu Pro Gly Leu Ala Leu Tyr Gly Glu Lys Glu Trp
    50                  55                  60

Tyr Phe Phe Ser Pro Arg Asp Arg Lys Tyr Pro Asn Gly Ser Arg Pro
65                  70                  75                  80

Asn Arg Ala Ala Gly Ser Gly Tyr Trp Lys Ala Thr Gly Ala Asp Lys
                85                  90                  95

Pro Ile Gly Ser Pro Lys Pro Val Gly Ile Lys Lys Ala Leu Val Phe
            100                 105                 110

Tyr Ala Gly Lys Ala Pro Arg Gly Glu Lys Thr Asn Trp Ile Met His
        115                 120                 125

Glu Tyr Arg Leu Ala Asp Val Asp Arg Ser Pro Arg Lys Lys Ser Ser
    130                 135                 140

Ser Leu Arg Leu Asp Asp Trp Val Leu Cys Arg Ile Tyr Asn Lys Lys
145                 150                 155                 160

Gly Thr Val Glu Lys Gln Gln Gln Gln Gln Gln Gln Gln Gln Gln Gln
                165                 170                 175

Thr Thr Ser Arg Met Ser Ser Gly Ser Glu Phe Glu Asp Arg Lys Pro
            180                 185                 190

Glu Asn Leu Ala Cys Pro Pro Pro Pro Ala Ala Val Ala Pro Thr Arg
        195                 200                 205

Pro Asn Asp Tyr Val Tyr Phe Asp Thr Ser Asp Ser Val Pro Arg Leu
    210                 215                 220

His Thr Asp Ser Ser Cys Ser Glu His Val Val Ser Pro Glu Phe Thr
225                 230                 235                 240

Cys Glu Val Gln Ser Glu Pro Lys Trp Lys Glu Trp Glu Lys Ala Leu
                245                 250                 255

Asp Phe Asn Tyr Asn Tyr Met Asp Thr Ser Val Glu Asn Gly Phe Gly
            260                 265                 270

Pro Gln Phe Gln Ser Ala Asn Gln Met Ser Pro Leu Gln Asp Ile Phe
        275                 280                 285

Met Tyr Leu Gln Lys Pro Tyr
    290                 295
```

<210> 88
<211> 1211
<212> DNA
<213> Brassica napus

<400> 88
atttcattag gattatatta ctagagacag atcctcgaat agaaaagaaa atgtcagaac 60

tacagctgcc tccaggattc cgatttcacc caaccgacga agagctggtg atgcactatc 120

tctgccgcaa atgcgcctct cagtccatcg ccgtccccat catcgctgag atcgatctct 180

acaaatatga tccttgggag cttcccggtt tagccttgta cggtgagaag gaatggtatt 240

tcttctctcc aagagacaga aaatatccga atggttctcg gccgaaccgg tcagctggtt 300

cgggttactg gaaagctact ggagctgata aaccgatcgg acttcctaaa ccggttggga 360

ttaagaaggc tctggttttc tacgccggga aagctccaaa gggtgagaaa accaattgga 420

tcatgcatga gtaccgtctt gccgacgttg accgatcatc ggctgctcgc aagaagaaga 480

atagtctcag gctggatgat tgggttcttt gtcggattta caacaaaaaa ggagccatcg 540

agaagcgagg accaccacca actccggttg tttacggcga cgaggtcgtg gaggagaagc 600

cgaggctgtc ggagatgggc atgcctcctc cgccggtgat gccgaatgat ttcgtgtact 660

ttgacacgtc ggactcggtg ccaaagctgc atacgacgga gtcgagctgc tcggagcagg 720

tggtgtcgcc ggagttcacg agcgaggttc agagcgagcc caagtggaag gattggtcgg 780

gtgagaaaag tagccttgat tttgggttta attacataga tgccaccgcg ttcgggggag 840

gaggagggag caatcagctg tttccgctac aggacatgtt catgtacaac atgcccaagc 900

cttattaggt gaaggcaaac gctcgtctat gggtatcacg agatcggtta cggttacggt 960

cggtcaatga gtgtgccgcc attagatatt tattaccatg atgtttgttg ttcagtgtta 1020

cttttatttt ctaagcggtc agattacggg aaatctctaa tcggatggcg gtcgatgtgt 1080

cattgtacta gtgttgtttg gtagaagaat ccacatgtct tttcttttt gggctttagt 1140

ggggcataaa agtcaaaagc taaggatatt tgttattatc tccttcgtaa taatcaatta 1200

aatatttctt g 1211

<210> 89
<211> 285
<212> PRT
<213> Brassica napus

<400> 89

Met Ser Glu Leu Gln Leu Pro Pro Gly Phe Arg Phe His Pro Thr Asp
1               5                   10                  15

Glu Glu Leu Val Met His Tyr Leu Cys Arg Lys Cys Ala Ser Gln Ser
            20                  25                  30

```
Ile Ala Val Pro Ile Ile Ala Glu Ile Asp Leu Tyr Lys Tyr Asp Pro
        35                  40                  45

Trp Glu Leu Pro Gly Leu Ala Leu Tyr Gly Glu Lys Glu Trp Tyr Phe
    50                  55                  60

Phe Ser Pro Arg Asp Arg Lys Tyr Pro Asn Gly Ser Arg Pro Asn Arg
65                  70                  75                  80

Ser Ala Gly Ser Gly Tyr Trp Lys Ala Thr Gly Ala Asp Lys Pro Ile
                85                  90                  95

Gly Leu Pro Lys Pro Val Gly Ile Lys Lys Ala Leu Val Phe Tyr Ala
            100                 105                 110

Gly Lys Ala Pro Lys Gly Glu Lys Thr Asn Trp Ile Met His Glu Tyr
        115                 120                 125

Arg Leu Ala Asp Val Asp Arg Ser Ser Ala Ala Arg Lys Lys Lys Asn
    130                 135                 140

Ser Leu Arg Leu Asp Asp Trp Val Leu Cys Arg Ile Tyr Asn Lys Lys
145                 150                 155                 160

Gly Ala Ile Glu Lys Arg Gly Pro Pro Pro Thr Pro Val Val Tyr Gly
                165                 170                 175

Asp Glu Val Val Glu Glu Lys Pro Arg Leu Ser Glu Met Gly Met Pro
            180                 185                 190

Pro Pro Pro Val Met Pro Asn Asp Phe Val Tyr Phe Asp Thr Ser Asp
        195                 200                 205

Ser Val Pro Lys Leu His Thr Thr Glu Ser Ser Cys Ser Glu Gln Val
    210                 215                 220

Val Ser Pro Glu Phe Thr Ser Glu Val Gln Ser Glu Pro Lys Trp Lys
225                 230                 235                 240

Asp Trp Ser Gly Glu Lys Ser Ser Leu Asp Phe Gly Phe Asn Tyr Ile
                245                 250                 255

Asp Ala Thr Ala Phe Gly Gly Gly Gly Gly Ser Asn Gln Leu Phe Pro
            260                 265                 270

Leu Gln Asp Met Phe Met Tyr Asn Met Pro Lys Pro Tyr
        275                 280                 285
```

<210> 90

<211> 1317
<212> DNA
<213> Arabidopsis thaliana

<400> 90
atggactttg ctctcttctc ctcgatttca atctttgaga taaaccacaa agatcctccg 60

attcgaaggt ttataaaaac tcaaaatcga atcttatcca caagaaaaca acaaggtact 120

tttccaaaaa tgaaggcgga gttgaatttg ccggcgggat tccgatttca tccgacggac 180

gaagagcttg tcaagttcta tctttgccgg agatgtgcgt cagaaccgat taacgttccg 240

gttatcgcag agattgactt gtacaaattc aatccatggg agcttccaga aatggcgttg 300

tacggtgaga aagaatggta cttcttctcg catagagacc ggaaataccc aaacgggtcg 360

agaccaaacc gggcagctgg aaccggttat tggaaagcga ctggagctga taaaccgatc 420

ggaaaaccga agacgttagg gattaagaaa gcactcgtct tctacgcagg aaaagctccg 480

aaagggatta aaacgaattg gattatgcac gagtatcgtc tcgctaatgt cgatcgatct 540

gcttctacca acaagaagaa caacttaaga cttgatgatt gggttttgtg tcggatatac 600

aataagaaag gaacaatgga gaagtattta ccggcggcgg ctgagaaacc gacggaaaag 660

atgagtacgt cggactcaag atgctcaagt cacgtgattt caccggacgt cacgtgttct 720

gataactggg aggttgagag tgagcccaaa tggattaatc tggaagacgc gttagaggca 780

tttaatgatg acacgtccat gtttagttcc attggtttgt tgcaaaatga cgcctttgtt 840

cctcagtttc agtaccagtc ctccgatttc gtcgattcgt ttcaggaccc gttcgagcag 900

aaaccgttct tgaattggaa ttttgctcct caagggtaaa aataatcggc aaaaagttga 960

agcttttcag agtcttcgat caccggcatt gtgtcggatc ctgacccgga gaccaagtcg 1020

ggtcatacga ttacataatc gggttattga gatttccaca tttggatttc cgagactaac 1080

caacttaacg gattctgggg taattggggg gttttgcaca ggtgaatcac actgagtcag 1140

caagtttcga ttttttggtt ttgttttgta atgattgatt aaatgtctaa agatatcacg 1200

aagtagatac agaagaactg taaaagcaat tgtgaccaac cattatgaat catatatata 1260

ttcaatgaag catgagctta tttcttcttt aatgaagcaa tgtacatttt tctccaa 1317

<210> 91
<211> 312
<212> PRT
<213> Arabidopsis thaliana

<400> 91

Met Asp Phe Ala Leu Phe Ser Ser Ile Ser Ile Phe Glu Ile Asn His
1 5 10 15

Lys Asp Pro Pro Ile Arg Arg Phe Ile Lys Thr Gln Asn Arg Ile Leu
20 25 30

Ser Thr Arg Lys Gln Gln Gly Thr Phe Pro Lys Met Lys Ala Glu Leu
35 40 45

Asn Leu Pro Ala Gly Phe Arg Phe His Pro Thr Asp Glu Glu Leu Val
50 55 60

Lys Phe Tyr Leu Cys Arg Arg Cys Ala Ser Glu Pro Ile Asn Val Pro
65 70 75 80

Val Ile Ala Glu Ile Asp Leu Tyr Lys Phe Asn Pro Trp Glu Leu Pro
85 90 95

Glu Met Ala Leu Tyr Gly Glu Lys Glu Trp Tyr Phe Phe Ser His Arg
100 105 110

Asp Arg Lys Tyr Pro Asn Gly Ser Arg Pro Asn Arg Ala Ala Gly Thr
115 120 125

Gly Tyr Trp Lys Ala Thr Gly Ala Asp Lys Pro Ile Gly Lys Pro Lys
130 135 140

Thr Leu Gly Ile Lys Lys Ala Leu Val Phe Tyr Ala Gly Lys Ala Pro
145 150 155 160

Lys Gly Ile Lys Thr Asn Trp Ile Met His Glu Tyr Arg Leu Ala Asn
165 170 175

Val Asp Arg Ser Ala Ser Thr Asn Lys Lys Asn Asn Leu Arg Leu Asp
180 185 190

Asp Trp Val Leu Cys Arg Ile Tyr Asn Lys Lys Gly Thr Met Glu Lys
195 200 205

Tyr Leu Pro Ala Ala Ala Glu Lys Pro Thr Glu Lys Met Ser Thr Ser
210 215 220

Asp Ser Arg Cys Ser Ser His Val Ile Ser Pro Asp Val Thr Cys Ser
225 230 235 240

Asp Asn Trp Glu Val Glu Ser Glu Pro Lys Trp Ile Asn Leu Glu Asp
245 250 255

Ala Leu Glu Ala Phe Asn Asp Asp Thr Ser Met Phe Ser Ser Ile Gly
260 265 270

Leu Leu Gln Asn Asp Ala Phe Val Pro Gln Phe Gln Tyr Gln Ser Ser
275 280 285

Asp Phe Val Asp Ser Phe Gln Asp Pro Phe Glu Gln Lys Pro Phe Leu

290 295 300

Asn Trp Asn Phe Ala Pro Gln Gly
305 310

<210> 92
<211> 1340
<212> DNA
<213> Capsicum annuum

<400> 92
gcaggcaaac acacaaagca acagctgcaa acagcaaaag aaacacaacg gagatttatt 60

cagacgcctt tgatcttcag ataagaaaca ggcaagttac caagagtgat atttattgag 120

acgtccttga ttttagctgg gaaacaggca agttttacaa agagaatgat caaaggaatc 180

gttggaaatc agcaattggg gttacctgca ggatttcgat ttcaccctac tgatgaagag 240

ttggtgcagc attatttgtg caggaaatgt gcaggacaga gtatttctgt ttcgattata 300

gctgaaattg atctttacaa gtttgatcct tggcagttgc ctgaaaaggc attatacggt 360

gaaaaagagt ggtatttttt ctcccaagag gatagaaaat acccgaacgg ttcaaggccg 420

aaccgggcag caggaaccgg ttattggaaa gcgaccggag cagataaacc ggtgggaaaa 480

ccgaaaacat tagggataaa aaaggcactt gtattttatg ctggaaaagc accaagaggt 540

ataaagacta attggattat gcatgagtat cgacttgcta atgtggacag atctgctgga 600

aagagtaata acttgaggct tgatgattgg gtattgtgtc gaatatacaa caagaagggg 660

cacactttga gaagtattac aatgtgggcc agcaagagag gtgagagttt tggggaattt 720

gaggatgaaa taaaaccaaa aatatttcca acacaactag caccggctcc ggggcagtgg 780

cccccacgac cccaatcaac ccccgcaagc gactacttca actttgaaac atccgagtcg 840

atgactacta ctaggatgca cacgacaaac tcgagctctg gctcagagca tgtcttgtcg 900

tcatgtgaca aggaggttca gagtgcacct aaatgggacg accttggaac cgccgccctt 960

gatttccaga taaattattt ggatggtttg ctgaacgacc cttttgaaat ccaaatgcag 1020

cagcaaaatt gcaacattga ccagttcaac actttccagg acatgttcct atacatgcaa 1080

aaaccttagt agaattgtat aaatcatgtg attcaaattg attttgatcc atgacatttt 1140

gttggttctt tggtggtgta ggtcaacttt ttattgatta gattagaaaa gtagaaatgc 1200

tattcaaatt tggtgggcta tagctcaaat gagccttcgc tagatagcca aaggattatg 1260

tagaaggctt attgtaaggt acaggtaaaa caaatgaaaa tttgttaata tcaatttatc 1320

attcttcaaa aaaaaaaaaa 1340

<210> 93
<211> 307
<212> PRT
<213> Capsicum annuum

<400> 93

Met Ile Lys Gly Ile Val Gly Asn Gln Gln Leu Gly Leu Pro Ala Gly
1 5 10 15

Phe Arg Phe His Pro Thr Asp Glu Glu Leu Val Gln His Tyr Leu Cys
20 25 30

Arg Lys Cys Ala Gly Gln Ser Ile Ser Val Ser Ile Ile Ala Glu Ile
35 40 45

Asp Leu Tyr Lys Phe Asp Pro Trp Gln Leu Pro Glu Lys Ala Leu Tyr
50 55 60

Gly Glu Lys Glu Trp Tyr Phe Phe Ser Gln Glu Asp Arg Lys Tyr Pro
65 70 75 80

Asn Gly Ser Arg Pro Asn Arg Ala Ala Gly Thr Gly Tyr Trp Lys Ala
85 90 95

Thr Gly Ala Asp Lys Pro Val Gly Lys Pro Lys Thr Leu Gly Ile Lys
100 105 110

Lys Ala Leu Val Phe Tyr Ala Gly Lys Ala Pro Arg Gly Ile Lys Thr
115 120 125

Asn Trp Ile Met His Glu Tyr Arg Leu Ala Asn Val Asp Arg Ser Ala
130 135 140

Gly Lys Ser Asn Asn Leu Arg Leu Asp Asp Trp Val Leu Cys Arg Ile
145 150 155 160

Tyr Asn Lys Lys Gly His Thr Leu Arg Ser Ile Thr Met Trp Ala Ser
165 170 175

Lys Arg Gly Glu Ser Phe Gly Glu Phe Glu Asp Glu Ile Lys Pro Lys
180 185 190

Ile Phe Pro Thr Gln Leu Ala Pro Ala Pro Gly Gln Trp Pro Pro Arg
195 200 205

Pro Gln Ser Thr Pro Ala Ser Asp Tyr Phe Asn Phe Glu Thr Ser Glu
210 215 220

Ser Met Thr Thr Thr Arg Met His Thr Thr Asn Ser Ser Ser Gly Ser
225 230 235 240

Glu His Val Leu Ser Ser Cys Asp Lys Glu Val Gln Ser Ala Pro Lys
245 250 255

```
Trp Asp Asp Leu Gly Thr Ala Ala Leu Asp Phe Gln Ile Asn Tyr Leu
            260                 265                 270

Asp Gly Leu Leu Asn Asp Pro Phe Glu Ile Gln Met Gln Gln Gln Asn
        275                 280                 285

Cys Asn Ile Asp Gln Phe Asn Thr Phe Gln Asp Met Phe Leu Tyr Met
    290                 295                 300

Gln Lys Pro
305
```

<210> 94
<211> 1316
<212> DNA
<213> Arabidopsis thaliana

<400> 94

```
aagtttcaaa aacgccaagt ttcagaggta gagagagaga tactacaatt gattaggata      60

ggatcaggat catcctcaac aacctcctcc taattcctcc tccattcata gtaacaataa     120

tattaagaaa gagggtaaac tatgtcagaa ttattacagt tgcctccagg tttccgattt     180

caccctaccg atgaagagct tgtcatgcac tatctctgcc gcaaatgtgc ctctcagtcc     240

atcgccgttc cgatcatcgc tgagatcgat ctctacaaat acgatccatg ggagcttcct     300

ggtttagcct tgtatggtga gaaggaatgg tacttcttct ctcccaggga cagaaaatat     360

cccaacggtt cgcgtcctaa ccggtccgct ggttctggtt actggaaagc taccggagct     420

gataaaccga tcggactacc taaaccggtc ggaattaaga aagctcttgt tttctacgcc     480

ggcaaagctc caaagggaga gaaaaccaat tggatcatgc acgagtaccg tctcgccgac     540

gttgaccggt ccgttcgcaa gaagaagaat agtctcaggc tggatgattg ggttctctgc     600

cggatttaca acaaaaaagg agctaccgag aggcggggac caccgcctcc ggttgtttac     660

ggcgacgaaa tcatggagga gaagccgaag gtgacggaga tggttatgcc tccgccgccg     720

caacagacaa gtgagttcgc gtatttcgac acgtcggatt cggtgccgaa gctgcatact     780

acggattcga gttgctcgga gcaggtggtg tcgccggagt tcacgagcga ggttcagagc     840

gagcccaagt ggaaagattg gtcggccgta agtaatgaca ataacaatac ccttgatttt     900

gggtttaatt acattgatgc caccgtggat aacgcgtttg gaggaggagg gagtagtaat     960

cagatgtttc cgctacagga tatgttcatg tacatgcaga agccttacta gaagggaatt    1020

cctttcctgc cgccgaaacg caacgcaaaa cgaccctcgt tttgcgttt atggcaacac     1080

gagaccgttt tatatggtca atgagtgtgc cgattcggcc attagatttc tgttcagtct    1140

tcgtttattc tatagaccgt ccgatttcag atcatcccta atcggacggt ggtcgttgga    1200

tgtatcagta gtgtattact gtgttaggta gaagaaaatc cacttgttct taaattggca    1260
```

taaaagtcag aagctaatat ttatatgtgc cgcaatcaat ttaatatttt ctgtct 1316

<210> 95
<211> 289
<212> PRT
<213> Arabidopsis thaliana

<400> 95

Met Ser Glu Leu Leu Gln Leu Pro Pro Gly Phe Arg Phe His Pro Thr
1 5 10 15

Asp Glu Glu Leu Val Met His Tyr Leu Cys Arg Lys Cys Ala Ser Gln
20 25 30

Ser Ile Ala Val Pro Ile Ile Ala Glu Ile Asp Leu Tyr Lys Tyr Asp
35 40 45

Pro Trp Glu Leu Pro Gly Leu Ala Leu Tyr Gly Glu Lys Glu Trp Tyr
50 55 60

Phe Phe Ser Pro Arg Asp Arg Lys Tyr Pro Asn Gly Ser Arg Pro Asn
65 70 75 80

Arg Ser Ala Gly Ser Gly Tyr Trp Lys Ala Thr Gly Ala Asp Lys Pro
85 90 95

Ile Gly Leu Pro Lys Pro Val Gly Ile Lys Lys Ala Leu Val Phe Tyr
100 105 110

Ala Gly Lys Ala Pro Lys Gly Glu Lys Thr Asn Trp Ile Met His Glu
115 120 125

Tyr Arg Leu Ala Asp Val Asp Arg Ser Val Arg Lys Lys Lys Asn Ser
130 135 140

Leu Arg Leu Asp Asp Trp Val Leu Cys Arg Ile Tyr Asn Lys Lys Gly
145 150 155 160

Ala Thr Glu Arg Arg Gly Pro Pro Pro Pro Val Val Tyr Gly Asp Glu
165 170 175

Ile Met Glu Glu Lys Pro Lys Val Thr Glu Met Val Met Pro Pro Pro
180 185 190

Pro Gln Gln Thr Ser Glu Phe Ala Tyr Phe Asp Thr Ser Asp Ser Val
195 200 205

Pro Lys Leu His Thr Thr Asp Ser Ser Cys Ser Glu Gln Val Val Ser
210 215 220

Pro Glu Phe Thr Ser Glu Val Gln Ser Glu Pro Lys Trp Lys Asp Trp
225 230 235 240

Ser Ala Val Ser Asn Asp Asn Asn Asn Thr Leu Asp Phe Gly Phe Asn
245 250 255

Tyr Ile Asp Ala Thr Val Asp Asn Ala Phe Gly Gly Gly Gly Ser Ser
260 265 270

Asn Gln Met Phe Pro Leu Gln Asp Met Phe Met Tyr Met Gln Lys Pro
275 280 285

Tyr

<210> 96
<211> 1423
<212> DNA
<213> Lycopersicon esculentum

<400> 96
ctaaattcct tcttgtttat cattttctct cttcccaaaa aaaaaatccc aaaatttaat 60

cataatacaa ttcgaattta tcaacctcgt actacgtaca tatttttgtt ggtacgtaaa 120

atactgaatt caggtcaact caaacatcgt aaattgtgat ttctttatgg aaagtacgga 180

ttcatcaacc gggacacgtc atcagcctca actcccaccg ggtttcgat tccacccgac 240

ggacgaagaa ctcatcgtcc actacctcaa aaaacgagtc gccggcgctc cgattccggt 300

ggatattatt ggtgaaattg atctttataa gtttgatcca tgggaactcc ctgctaaggc 360

aatattcgga gagcaagaat ggttcttttt tagtccaaga gatagaaaat atcctaacgg 420

ggcgaggcca aatcgggctg caacatcggg ttattggaag gctaccggaa ccgacaagcc 480

ggtttttact tccggtggaa cacaaaaggt tggggtaaaa aaggcgctcg tttttttacgg 540

cggtaaacca ccaaaagggg taaaaactaa ttggatcatg catgaataca gagttgtaga 600

aaataaaaca aataacaagc cacttggttg tgataatatt gttgccaaca aaaaaggatc 660

tttgaggcta gatgattggg ttttatgtcg aatttacaag aagaataaca cacaaaggtc 720

catagatgat ttgcatgata tgttgggatc gataccacaa aatgtaccaa attcaatatt 780

acaaggaata aagccttcaa actatggtac aatattgctc gaaaatgaat cgaatatgta 840

cgatggaatt atgaataaca cgaacgatat tatcaacaat aataatagat ccattccaca 900

aatatcgtca aagagaacga tgcatggagg tttgtattgg aataacgacg aagcaacaac 960

aacaacaaca actattgata ggaaccattc tccaaataca aaaaggttcc ttgttgagaa 1020

caacgaggac gatggactta acatgaataa tatttcgcga attacaaatc atgaacaaag 1080

tagctccatt gccaatttcc tgagccagtt tcctcaaaat ccttcgattc aacaacaaca 1140

```
acaacaacaa gaagaagtat tgggatctct taatgatggg gtcgtctttc gacaacctta   1200

taatcaagtt actggcatga attggtactc ttaaagatat aaaaaggcaa aaaatagtta   1260

gccctgtaaa atcaatcgat caatcaatca tagatatatt atatatggat ttcgttatat   1320

tttactttta gttagaatta atatatagaa tatcttctat ctcacattaa caaataagaa   1380

catttataac aaaaaaaaaa aaaaaaaaaa aaaaaaaaaa aaa                     1423


<210>  97
<211>  355
<212>  PRT
<213>  Lycopersicon esculentum

<400>  97

Met Glu Ser Thr Asp Ser Ser Thr Gly Thr Arg His Gln Pro Gln Leu
1               5                   10                  15

Pro Pro Gly Phe Arg Phe His Pro Thr Asp Glu Glu Leu Ile Val His
            20                  25                  30

Tyr Leu Lys Lys Arg Val Ala Gly Ala Pro Ile Pro Val Asp Ile Ile
        35                  40                  45

Gly Glu Ile Asp Leu Tyr Lys Phe Asp Pro Trp Glu Leu Pro Ala Lys
    50                  55                  60

Ala Ile Phe Gly Glu Gln Glu Trp Phe Phe Phe Ser Pro Arg Asp Arg
65                  70                  75                  80

Lys Tyr Pro Asn Gly Ala Arg Pro Asn Arg Ala Ala Thr Ser Gly Tyr
                85                  90                  95

Trp Lys Ala Thr Gly Thr Asp Lys Pro Val Phe Thr Ser Gly Gly Thr
            100                 105                 110

Gln Lys Val Gly Val Lys Lys Ala Leu Val Phe Tyr Gly Gly Lys Pro
        115                 120                 125

Pro Lys Gly Val Lys Thr Asn Trp Ile Met His Glu Tyr Arg Val Val
    130                 135                 140

Glu Asn Lys Thr Asn Asn Lys Pro Leu Gly Cys Asp Asn Ile Val Ala
145                 150                 155                 160

Asn Lys Lys Gly Ser Leu Arg Leu Asp Asp Trp Val Leu Cys Arg Ile
                165                 170                 175

Tyr Lys Lys Asn Asn Thr Gln Arg Ser Ile Asp Asp Leu His Asp Met
            180                 185                 190
```

Leu Gly Ser Ile Pro Gln Asn Val Pro Asn Ser Ile Leu Gln Gly Ile
195 200 205

Lys Pro Ser Asn Tyr Gly Thr Ile Leu Leu Glu Asn Glu Ser Asn Met
210 215 220

Tyr Asp Gly Ile Met Asn Asn Thr Asn Asp Ile Ile Asn Asn Asn Asn
225 230 235 240

Arg Ser Ile Pro Gln Ile Ser Ser Lys Arg Thr Met His Gly Gly Leu
245 250 255

Tyr Trp Asn Asn Asp Glu Ala Thr Thr Thr Thr Thr Thr Ile Asp Arg
260 265 270

Asn His Ser Pro Asn Thr Lys Arg Phe Leu Val Glu Asn Asn Glu Asp
275 280 285

Asp Gly Leu Asn Met Asn Asn Ile Ser Arg Ile Thr Asn His Glu Gln
290 295 300

Ser Ser Ser Ile Ala Asn Phe Leu Ser Gln Phe Pro Gln Asn Pro Ser
305 310 315 320

Ile Gln Gln Gln Gln Gln Gln Gln Glu Glu Val Leu Gly Ser Leu Asn
325 330 335

Asp Gly Val Val Phe Arg Gln Pro Tyr Asn Gln Val Thr Gly Met Asn
340 345 350

Trp Tyr Ser
355

<210> 98
<211> 1691
<212> DNA
<213> Arabidopsis thaliana

<400> 98
aaaagatcga cgaacataaa aacaaaaaca tataatttgg gtttttagag ttcgaaactt 60

gaaatctttt ttttttggt tgctgaggaa tcgaagtaga agagtataaa tgggtgttag 120

agagaaagat ccgttagccc agttgagttt gccaccaggt tttagatttt atccgacaga 180

tgaagagctt cttgttcagt atctatgtcg gaaagttgca ggctatcatt tctctctcca 240

ggtcatcgga gacatcgatc tctacaagtt cgatccttgg gatttgccaa gtaaggcttt 300

gtttggagag aaggaatggt atttctttag cccaagagat cggaaatatc cgaacgggtc 360

aagacccaat agagtagccg ggtcgggtta ttggaaagca acgggtactg acaaaattat 420

cacggcggat ggtcgtcgtg tcgggattaa aaaagctctg gtcttttacg ccggaaaagc 480

tcccaaaggc actaaaacca actggattat gcacgagtat cgcttaatag aacattctcg 540

tagccatgga agctccaagt tggatgattg ggtgttgtgt cgaatttaca agaaaacatc 600

tggatctcag agacaagctg ttactcctgt tcaagcttgt cgtgaagagc atagcacgaa 660

tgggtcgtca tcgtcttctt catcacagct tgacgacgtt cttgattcgt tcccggagat 720

aaaagaccag tcttttaatc ttcctcggat gaattcgctc aggacgattc ttaacgggaa 780

ctttgattgg gctagcttgg caggtcttaa tccaattcca gagctagctc cgaccaatgg 840

attaccgagt tacggtggtt acgatgcgtt tcgagcggcg gaaggtgagg cggagagtgg 900

gcatgtgaat cggcagcaga actcgagcgg gttgactcag agtttcgggt acagctcgag 960

tgggtttggt gtttcgggtc aaacattcga gtttaggcaa tgagagagat gtgaagttac 1020

tgatgggtga aaaaagtaaa aaaaaaactt ggagatagta gagtggcaat tgatgtaaat 1080

aatagggatt tatatggggc ttttaccgat tcggtgaggc ttaggattcc acaaaggaaa 1140

aaggctcgac tggggactag tttgatccaa cttgacggcc cacaaatgtg taatgtttct 1200

caacggagag aaaaataaat ggttaccaat atttttacac cggttatgtg ttgataccat 1260

ttgcacaaaa cggtttaagt tctgaattga ataacttaac acccaaaatt tggtaaaaag 1320

cttaatacaa aaagttcaaa ggcattttcg cttactgcga cactcggagt tgcagagtac 1380

tctacgtaac agactctgtt catagagttt gcggcaccgg tggtgggaca ttcttgcgga 1440

cacagtatgt acttttggaa gcatcgttcc taagggttgc tgcagtaaac tccttgtgac 1500

ttggctcgca agaaacataa catggccgca agaaaaaaca gcattaacga agaggtgttt 1560

gagatcttca tgtctagttt attcttcctg ttttattatg tgtcgactgg tgtcaaaaga 1620

aacgtgttga ttgtgaattt gtagacgcca ctctgtaaat ttatttaaaa taaactattt 1680

tacttcacgt t 1691

<210> 99
<211> 297
<212> PRT
<213> Arabidopsis thaliana

<400> 99

Met Gly Val Arg Glu Lys Asp Pro Leu Ala Gln Leu Ser Leu Pro Pro
1 5 10 15

Gly Phe Arg Phe Tyr Pro Thr Asp Glu Glu Leu Leu Val Gln Tyr Leu
20 25 30

Cys Arg Lys Val Ala Gly Tyr His Phe Ser Leu Gln Val Ile Gly Asp
35 40 45

Ile Asp Leu Tyr Lys Phe Asp Pro Trp Asp Leu Pro Ser Lys Ala Leu

```
        50                  55                  60

Phe Gly Glu Lys Glu Trp Tyr Phe Phe Ser Pro Arg Asp Arg Lys Tyr
65                  70                  75                  80

Pro Asn Gly Ser Arg Pro Asn Arg Val Ala Gly Ser Gly Tyr Trp Lys
                85                  90                  95

Ala Thr Gly Thr Asp Lys Ile Ile Thr Ala Asp Gly Arg Arg Val Gly
            100                 105                 110

Ile Lys Lys Ala Leu Val Phe Tyr Ala Gly Lys Ala Pro Lys Gly Thr
        115                 120                 125

Lys Thr Asn Trp Ile Met His Glu Tyr Arg Leu Ile Glu His Ser Arg
    130                 135                 140

Ser His Gly Ser Ser Lys Leu Asp Asp Trp Val Leu Cys Arg Ile Tyr
145                 150                 155                 160

Lys Lys Thr Ser Gly Ser Gln Arg Gln Ala Val Thr Pro Val Gln Ala
                165                 170                 175

Cys Arg Glu Glu His Ser Thr Asn Gly Ser Ser Ser Ser Ser Ser Ser
            180                 185                 190

Gln Leu Asp Asp Val Leu Asp Ser Phe Pro Glu Ile Lys Asp Gln Ser
        195                 200                 205

Phe Asn Leu Pro Arg Met Asn Ser Leu Arg Thr Ile Leu Asn Gly Asn
    210                 215                 220

Phe Asp Trp Ala Ser Leu Ala Gly Leu Asn Pro Ile Pro Glu Leu Ala
225                 230                 235                 240

Pro Thr Asn Gly Leu Pro Ser Tyr Gly Gly Tyr Asp Ala Phe Arg Ala
                245                 250                 255

Ala Glu Gly Glu Ala Glu Ser Gly His Val Asn Arg Gln Gln Asn Ser
            260                 265                 270

Ser Gly Leu Thr Gln Ser Phe Gly Tyr Ser Ser Ser Gly Phe Gly Val
        275                 280                 285

Ser Gly Gln Thr Phe Glu Phe Arg Gln
    290                 295
```

<210> 100
<211> 1335

<212> DNA
<213> Triticum aestivum

<400> 100
attgattccg ttcccacctg ctcaccgtcg ccatgccaat gggcagcagc agcgccgcca 60

tgcccgccct ccctcccggc ttccggttcc accccaccga cgaggagctc atcgtccact 120

acctcggcag gcaggccgcg tccatgccca gccccgtgcc catcatcgcc gaggtcaaca 180

tctacaagtg caacccatgg gacctccccg gcaaggcctt gttcggggag aatgagtggt 240

acttcttcag cccccgggat cgcaagtacc ccaacggcgc gcgccccaac cgcgccgccg 300

ggtccggcta ctggaaggcc accggcaccg acaaggccat cctgtccacg ccggccaacg 360

agagcatcgg cgtcaagaag gcgctcgtct tctaccgggg caagccgccc aagggcgtca 420

agaccgactg gatcatgcac gagtaccgcc tcaccgccgc cgacaaccgg accaccaagc 480

gcagaggatc ctccatgagg ctggatgact gggtgctgtg taggatccac aagaagtgca 540

acaacttgca caacttctcc tcctctgacc aggaacagga gcacgagcag gagagctcca 600

ccaccgtgga ggactcgcac aacaaccaca ccgtgtcgtc gcccaagtcg gaggccttcg 660

acggcgacgg cgacgaccag ctgcagctgc agcagttccg ccccatggcg atcgccaagt 720

cgtgctccct caccgacctg ctcaacaccg tcgactacgc cgcgctctcg cacctcctcc 780

tcgacggcgc cggcgccggc gcctcgtcgt cggacgccgg agcagactac cagctgccgc 840

cggaaaaccc gctcatctac tcgcagcctc catggcaaca aacgctacac tataataaca 900

ataacaatgg ctacgtgaac atcgacacca tcgacgtgcc tcagataccc gaggcccgtg 960

tagatgacta cggcatgaat ggcgataggt acaacggcat gaagaggaag aggtccagcg 1020

gcagtttgta ctgcagccag ctgcagctcc cggcggatca gtacagcggc atgctgatcc 1080

atccgttcct cagccagcag ctgcacatgt gaagaaccag agagcttgga tcgaagagat 1140

catcattgtt ccatttgaac ttgctgtaga tcgaggggat cccccgctgt ggcagatagg 1200

cagggatcta gcttgcttgc tgtgtcgtga ccgaccgacc gataagaacg gacaaatttc 1260

agaattcttg gtgtagcaca aaagctgtaa attacggggg tttattgtga aataaataaa 1320

tccagtatta ttaag 1335

<210> 101
<211> 359
<212> PRT
<213> Triticum aestivum

<400> 101

Met Pro Met Gly Ser Ser Ser Ala Ala Met Pro Ala Leu Pro Pro Gly
1 5 10 15

Phe Arg Phe His Pro Thr Asp Glu Glu Leu Ile Val His Tyr Leu Gly
20 25 30

```
Arg Gln Ala Ala Ser Met Pro Ser Pro Val Pro Ile Ile Ala Glu Val
        35                  40                  45

Asn Ile Tyr Lys Cys Asn Pro Trp Asp Leu Pro Gly Lys Ala Leu Phe
    50                  55                  60

Gly Glu Asn Glu Trp Tyr Phe Phe Ser Pro Arg Asp Arg Lys Tyr Pro
65                  70                  75                  80

Asn Gly Ala Arg Pro Asn Arg Ala Ala Gly Ser Gly Tyr Trp Lys Ala
                85                  90                  95

Thr Gly Thr Asp Lys Ala Ile Leu Ser Thr Pro Ala Asn Glu Ser Ile
            100                 105                 110

Gly Val Lys Lys Ala Leu Val Phe Tyr Arg Gly Lys Pro Pro Lys Gly
        115                 120                 125

Val Lys Thr Asp Trp Ile Met His Glu Tyr Arg Leu Thr Ala Ala Asp
    130                 135                 140

Asn Arg Thr Thr Lys Arg Arg Gly Ser Ser Met Arg Leu Asp Asp Trp
145                 150                 155                 160

Val Leu Cys Arg Ile His Lys Lys Cys Asn Asn Leu His Asn Phe Ser
                165                 170                 175

Ser Ser Asp Gln Glu Gln Glu His Glu Gln Glu Ser Ser Thr Thr Val
            180                 185                 190

Glu Asp Ser His Asn Asn His Thr Val Ser Ser Pro Lys Ser Glu Ala
        195                 200                 205

Phe Asp Gly Asp Gly Asp Asp Gln Leu Gln Leu Gln Gln Phe Arg Pro
    210                 215                 220

Met Ala Ile Ala Lys Ser Cys Ser Leu Thr Asp Leu Leu Asn Thr Val
225                 230                 235                 240

Asp Tyr Ala Ala Leu Ser His Leu Leu Leu Asp Gly Ala Gly Ala Gly
                245                 250                 255

Ala Ser Ser Ser Asp Ala Gly Ala Asp Tyr Gln Leu Pro Pro Glu Asn
            260                 265                 270

Pro Leu Ile Tyr Ser Gln Pro Pro Trp Gln Gln Thr Leu His Tyr Asn
        275                 280                 285
```

Asn Asn Asn Asn Gly Tyr Val Asn Ile Asp Thr Ile Asp Val Pro Gln
    290                 295                 300

Ile Pro Glu Ala Arg Val Asp Asp Tyr Gly Met Asn Gly Asp Arg Tyr
305                 310                 315                 320

Asn Gly Met Lys Arg Lys Arg Ser Ser Gly Ser Leu Tyr Cys Ser Gln
                325                 330                 335

Leu Gln Leu Pro Ala Asp Gln Tyr Ser Gly Met Leu Ile His Pro Phe
            340                 345                 350

Leu Ser Gln Gln Leu His Met
        355

<210> 102
<211> 1423
<212> DNA
<213> Triticum aestivum

<400> 102
gcagcatttt tatgcagtag ccatcttctc ctcctcctcc ccccgccttc cagctagcca 60

cctagctcac tatcacatca tccagcagcc cacaccaact catattgatt ccgttcccac 120

ctgctcgcca tcgccagcca tgccaatggg cagcagcgcc gccatgcccg ccctccctcc 180

cggcttccgg ttccacccca ccgacgagga gctcatcgtc cactacctcc gcaggcaggc 240

cgcgtccatg cccagccccg tgcccatcat cgccgaggtc aacatctaca agtgcaaccc 300

atgggacctc cccggcaaag ctttgttcgg ggagaatgag tggtacttct tcagcccccg 360

ggatcgcaag taccccaacg gcgcgcgccc gaaccgcgcc gccgggtccg gctactggaa 420

ggccaccggc accgacaagg ccatcctgtc cacgccggcc aacgagagca tcggggtcaa 480

gaaggcgctc gtgttctaca ggggcaagcc gcccaagggc gtcaagaccg actggatcat 540

gcacgagtac cgcctcaccg cagccgacaa ccggaccacc aagcgcagag gatcctccat 600

gaggctggat gactgggtgc tgtgtaggat ccacaagaag tgcggcaact tgcccaactt 660

ctcctcctct gaccaggaac aggagcatga gcaggagagc tccaccgtgg aggactcgca 720

gaacaaccac accgtgtcgt cgcccaagtc cgaggccttc gacggcgacg gcgacgacca 780

cctccagttg cagcagttcc gccccatggc gatcgccaag tcgtgctccc tcaccgacct 840

gctcaacacc gtcgactacg ccgcgctctc gcacctcctc ctcgacggcg ccggcgcctc 900

gtcgtcggac gccggagcag actaccagct gcctcccgaa aacccactca tctactcgca 960

gcctccatgg caacaaacgc tacactataa taacaacaac ggctacgtga acaacgagac 1020

catcgacgtg cctcagctac ccgaggcgcg cgtagatgac tacggcatga atggcgataa 1080

gtataacggc atgaagagga agagatccag cggcagcttg tactgcagcc agctgcagct 1140

cccagcggat cagtacagcg gcatgctgat ccatccgttc ctcagccagc agctgcacat 1200

gtgaagaacc agagagcttg ggtcgaagag atcatcattg ttccatttga acttgctgta 1260

gatcgagagg atccccccgct gtggcagata ggcagggatc tagctagctt gctgtgtcgt 1320

gaacgaccga ccgataagaa cggacaaatt tcagaattct tggtgtagca caaagctgta 1380

aattacgggg gtttattgtg aaataaatta atccagtatt atc 1423

<210> 103
<211> 354
<212> PRT
<213> Triticum aestivum

<400> 103

Met Pro Met Gly Ser Ser Ala Ala Met Pro Ala Leu Pro Pro Gly Phe
1 5 10 15

Arg Phe His Pro Thr Asp Glu Glu Leu Ile Val His Tyr Leu Arg Arg
20 25 30

Gln Ala Ala Ser Met Pro Ser Pro Val Pro Ile Ile Ala Glu Val Asn
35 40 45

Ile Tyr Lys Cys Asn Pro Trp Asp Leu Pro Gly Lys Ala Leu Phe Gly
50 55 60

Glu Asn Glu Trp Tyr Phe Phe Ser Pro Arg Asp Arg Lys Tyr Pro Asn
65 70 75 80

Gly Ala Arg Pro Asn Arg Ala Ala Gly Ser Gly Tyr Trp Lys Ala Thr
85 90 95

Gly Thr Asp Lys Ala Ile Leu Ser Thr Pro Ala Asn Glu Ser Ile Gly
100 105 110

Val Lys Lys Ala Leu Val Phe Tyr Arg Gly Lys Pro Pro Lys Gly Val
115 120 125

Lys Thr Asp Trp Ile Met His Glu Tyr Arg Leu Thr Ala Ala Asp Asn
130 135 140

Arg Thr Thr Lys Arg Arg Gly Ser Ser Met Arg Leu Asp Asp Trp Val
145 150 155 160

Leu Cys Arg Ile His Lys Lys Cys Gly Asn Leu Pro Asn Phe Ser Ser
165 170 175

Ser Asp Gln Glu Gln Glu His Glu Gln Glu Ser Ser Thr Val Glu Asp
180 185 190

```
Ser Gln Asn Asn His Thr Val Ser Ser Pro Lys Ser Glu Ala Phe Asp
        195                 200                 205

Gly Asp Gly Asp Asp His Leu Gln Leu Gln Gln Phe Arg Pro Met Ala
    210                 215                 220

Ile Ala Lys Ser Cys Ser Leu Thr Asp Leu Leu Asn Thr Val Asp Tyr
225                 230                 235                 240

Ala Ala Leu Ser His Leu Leu Leu Asp Gly Ala Gly Ala Ser Ser Ser
                245                 250                 255

Asp Ala Gly Ala Asp Tyr Gln Leu Pro Pro Glu Asn Pro Leu Ile Tyr
            260                 265                 270

Ser Gln Pro Pro Trp Gln Gln Thr Leu His Tyr Asn Asn Asn Asn Gly
        275                 280                 285

Tyr Val Asn Asn Glu Thr Ile Asp Val Pro Gln Leu Pro Glu Ala Arg
    290                 295                 300

Val Asp Asp Tyr Gly Met Asn Gly Asp Lys Tyr Asn Gly Met Lys Arg
305                 310                 315                 320

Lys Arg Ser Ser Gly Ser Leu Tyr Cys Ser Gln Leu Gln Leu Pro Ala
                325                 330                 335

Asp Gln Tyr Ser Gly Met Leu Ile His Pro Phe Leu Ser Gln Gln Leu
            340                 345                 350

His Met


<210>  104
<211>  891
<212>  DNA
<213>  Saccharum officinarum

<400>  104
atgagcggcg gcggtcagga tctgcagctg ccgccggggt tccggttcca cccgacggac      60

gaggagctgg tgatgcacta cctctgccgc cgctgcgcca gcctgcccat cgccgtcccc     120

atcatcgccg agatcgacct ctacaagttc gatccatggc agctccccag gatggcgctg     180

tacggcgaga aggagtggta cttcttctcc ccgcgggacc gcaagtaccc gaacgggtcc     240

aggcccaacc gcgccgccgg gtccggctac tggaaggcca ccggcgccga caagcccgtg     300

ggcacgccca agccgctcgc catcaagaag gcgctcgtct tctacgccgg caaggcgccc     360

aagggcgaga agaccaactg gatcatgcac gagtaccgcc tcgccgacgt cgaccgctcc     420

gcccgcaaga agaacagcct caggttggat gactgggtcc tgtgccgcat ctacaacaag     480
```

aagggagggc tggagaagcc ggcggcggcg tcctccggcg accacaagcc gatggtgttc 540

gccgcgggcg cggtgagctc cccgccggag cagaagccgt tcgtggcgac gccgggcggg 600

ctgccccccg ccgcgttcac ggcggacctg gcggcgtact acgaccggcc gtcggactcg 660

atgccgcggc tgcacgcgga ctccagctgc tcggagcagg tgctgtcgcc ggagcagctg 720

gcgtgcgacc gggaggtgca gagccagccc aagatcagcg agtgggagcg caccttcgcc 780

tccgaccccg tcaaccccgc tggctccatg ctcgtcgacc ccgtcgtcgg tggccacgcc 840

ggcgacccgc tgctgcagga catcctcatg tactggggca agccgttcta g 891

<210> 105
<211> 296
<212> PRT
<213> Saccharum officinarum

<400> 105

Met Ser Gly Gly Gly Gln Asp Leu Gln Leu Pro Pro Gly Phe Arg Phe
1 5 10 15

His Pro Thr Asp Glu Glu Leu Val Met His Tyr Leu Cys Arg Arg Cys
20 25 30

Ala Ser Leu Pro Ile Ala Val Pro Ile Ile Ala Glu Ile Asp Leu Tyr
35 40 45

Lys Phe Asp Pro Trp Gln Leu Pro Arg Met Ala Leu Tyr Gly Glu Lys
50 55 60

Glu Trp Tyr Phe Phe Ser Pro Arg Asp Arg Lys Tyr Pro Asn Gly Ser
65 70 75 80

Arg Pro Asn Arg Ala Ala Gly Ser Gly Tyr Trp Lys Ala Thr Gly Ala
85 90 95

Asp Lys Pro Val Gly Thr Pro Lys Pro Leu Ala Ile Lys Lys Ala Leu
100 105 110

Val Phe Tyr Ala Gly Lys Ala Pro Lys Gly Glu Lys Thr Asn Trp Ile
115 120 125

Met His Glu Tyr Arg Leu Ala Asp Val Asp Arg Ser Ala Arg Lys Lys
130 135 140

Asn Ser Leu Arg Leu Asp Asp Trp Val Leu Cys Arg Ile Tyr Asn Lys
145 150 155 160

Lys Gly Gly Leu Glu Lys Pro Ala Ala Ala Ser Ser Gly Asp His Lys
165 170 175

```
Pro Met Val Phe Ala Ala Gly Ala Val Ser Ser Pro Pro Glu Gln Lys
            180                 185                 190

Pro Phe Val Ala Thr Pro Gly Gly Leu Pro Pro Ala Ala Phe Thr Ala
        195                 200                 205

Asp Leu Ala Ala Tyr Tyr Asp Arg Pro Ser Asp Ser Met Pro Arg Leu
    210                 215                 220

His Ala Asp Ser Ser Cys Ser Glu Gln Val Leu Ser Pro Glu Gln Leu
225                 230                 235                 240

Ala Cys Asp Arg Glu Val Gln Ser Gln Pro Lys Ile Ser Glu Trp Glu
                245                 250                 255

Arg Thr Phe Ala Ser Asp Pro Val Asn Pro Ala Gly Ser Met Leu Val
            260                 265                 270

Asp Pro Val Val Gly Gly His Ala Gly Asp Pro Leu Leu Gln Asp Ile
        275                 280                 285

Leu Met Tyr Trp Gly Lys Pro Phe
    290                 295
```

<210>  106
<211>  1817
<212>  DNA
<213>  Oryza sativa

<400>  106

```
aactagctta gctagacagc gtgtgttggt ggtggtggtg gtgtgtgtgt gtgtgaggag      60
ggtgatcgat cgatcgagcg atggagagcc cggactcgtc gtccggctcg gcgccaccgc     120
gagtactacg gcggcaacag cagcagccgg gctcggcgcc ggagctgccg ccggggttcc     180
ggttccaccc gacggacgag gagctggtgg tgcactacct caagaagaag gccgcctccg     240
ttccgctccc cgtcaccatc atcgccgagg tcgatctcta caagttcgat ccctgggatc     300
tccccgagaa ggcgaacttc ggggagcagg agtggtattt cttcagcccg agggaccaca     360
agtacccgaa cggggcgcgg ccgaaccggg cggcgacgtc ggggtactgg aaggccaccg     420
gcaccgacaa gcccatcatg tcgtcgggga gcacccgcga gaaggtcggc gtgaagaagg     480
cgctcgtgtt ctaccggggc aagccaccca agggcgtcaa gactaactgg atcatgcacg     540
agtaccgtct cacggacacg tctagctccg ccgccgccgt cgctacgacc aggcggccgc     600
cgccgcccat caccggcggt agcaagggcg ccgtctctct caggctggat gactgggtgc     660
tgtgccgcat atacaagaag acgaacaagg ccggtgcggg gcagaggagc atggagtgcg     720
aggactccgt ggaggacgcg gtggccgcgt acgcgccgtc gtcgcagcag catgccacgg     780
```

ctgctgctgg catggccggt tcggacggcg ccggaggagt tgctgcagcg cacggcggcg 840

actacagttc actgctccat cacgacagcc acgaggacac cttcctcgta aacggcctgc 900

tcaccgcgga ggacgccgcc ggcctctcga ccggcgccag ctctctcagc cagctcgccg 960

cggcggcgag ggcggcggcg acaccgtgcg acgccaccaa gcagcttctt gctccgtctc 1020

caaccccatt caactggttc gaggcattcc ttccacgggc caaggagttt cctagtgggc 1080

taagcaggag tagcagagac atcggcgaca tgtcgctgtc atcgacggtg gacaggagcc 1140

tgtctgaggc tggcgccgtg gccattgaca ccggcgacgc cgccaatggc gcaaacacta 1200

tgcctgcatt tatcaatcct ctcggcgtgc agggtgcaac ctaccaacaa caccaagcca 1260

tcatgggtgc ctcgttgcca tcggagtcag cagcagcagc agccgcctgc aatttccagc 1320

atccgttcca actctccagg gtgaattggg attcctgaat aaaaggtgcc ggcattatgc 1380

atacacatat atgcacatgc atacatgcat ggcttttcaa gtagtagcac aacattacta 1440

gtaattaatc acactaatgt gtgtggcagt cgcattcagt gagcactgat cgagtagttg 1500

catgaacaca acactaatgc atgcactaca tatatatggc cgcattgctc ctagggcacg 1560

tacctgtacg aactaggtaa gtgacctaac cagctagatc atgttcagaa taaggagaag 1620

aagccgcatg caaacacgta gatcatatgg ctttgccttc acatgcgttt gcatcatata 1680

tatcgcattc agaaagtagt gctgcagcta acacatagat atggtgttag cttcatgcgt 1740

ttgtaccata tatagcggat gcagaaagta gctagttgca ctgttcatca ttatcccatg 1800

agatatgaac tttatat 1817

<210> 107
<211> 425
<212> PRT
<213> Oryza sativa

<400> 107

Met Glu Ser Pro Asp Ser Ser Ser Gly Ser Ala Pro Pro Arg Val Leu
1 5 10 15

Arg Arg Gln Gln Gln Gln Pro Gly Ser Ala Pro Glu Leu Pro Pro Gly
20 25 30

Phe Arg Phe His Pro Thr Asp Glu Glu Leu Val Val His Tyr Leu Lys
35 40 45

Lys Lys Ala Ala Ser Val Pro Leu Pro Val Thr Ile Ile Ala Glu Val
50 55 60

Asp Leu Tyr Lys Phe Asp Pro Trp Asp Leu Pro Glu Lys Ala Asn Phe
65 70 75 80

Gly Glu Gln Glu Trp Tyr Phe Phe Ser Pro Arg Asp His Lys Tyr Pro
85 90 95

Asn Gly Ala Arg Pro Asn Arg Ala Ala Thr Ser Gly Tyr Trp Lys Ala
100 105 110

Thr Gly Thr Asp Lys Pro Ile Met Ser Ser Gly Ser Thr Arg Glu Lys
115 120 125

Val Gly Val Lys Lys Ala Leu Val Phe Tyr Arg Gly Lys Pro Pro Lys
130 135 140

Gly Val Lys Thr Asn Trp Ile Met His Glu Tyr Arg Leu Thr Asp Thr
145 150 155 160

Ser Ser Ser Ala Ala Ala Val Ala Thr Thr Arg Arg Pro Pro Pro Pro
165 170 175

Ile Thr Gly Gly Ser Lys Gly Ala Val Ser Leu Arg Leu Asp Asp Trp
180 185 190

Val Leu Cys Arg Ile Tyr Lys Lys Thr Asn Lys Ala Gly Ala Gly Gln
195 200 205

Arg Ser Met Glu Cys Glu Asp Ser Val Glu Asp Ala Val Ala Ala Tyr
210 215 220

Ala Pro Ser Ser Gln Gln His Ala Thr Ala Ala Ala Gly Met Ala Gly
225 230 235 240

Ser Asp Gly Ala Gly Gly Val Ala Ala Ala His Gly Gly Asp Tyr Ser
245 250 255

Ser Leu Leu His His Asp Ser His Glu Asp Thr Phe Leu Val Asn Gly
260 265 270

Leu Leu Thr Ala Glu Asp Ala Ala Gly Leu Ser Thr Gly Ala Ser Ser
275 280 285

Leu Ser Gln Leu Ala Ala Ala Ala Arg Ala Ala Ala Thr Pro Cys Asp
290 295 300

Ala Thr Lys Gln Leu Leu Ala Pro Ser Pro Thr Pro Phe Asn Trp Phe
305 310 315 320

Glu Ala Phe Leu Pro Arg Ala Lys Glu Phe Pro Ser Gly Leu Ser Arg
325 330 335

Ser Ser Arg Asp Ile Gly Asp Met Ser Leu Ser Ser Thr Val Asp Arg

340 345 350

Ser Leu Ser Glu Ala Gly Ala Val Ala Ile Asp Thr Gly Asp Ala Ala
355 360 365

Asn Gly Ala Asn Thr Met Pro Ala Phe Ile Asn Pro Leu Gly Val Gln
370 375 380

Gly Ala Thr Tyr Gln Gln His Gln Ala Ile Met Gly Ala Ser Leu Pro
385 390 395 400

Ser Glu Ser Ala Ala Ala Ala Ala Ala Cys Asn Phe Gln His Pro Phe
405 410 415

Gln Leu Ser Arg Val Asn Trp Asp Ser
420 425

<210> 108
<211> 1218
<212> DNA
<213> Medicago truncatula

<400> 108
atgggaaccc cacagaccaa tttgccacca ggttttaggt tccaccctac tgatgcagaa 60

ctcattcttc actaccttag gaaaaaaatt gcctccattc ccttgcctgt ttccatcatt 120

gctgaagttg atatttataa gttggatcct tgggatttac cagctaaggc ttcatttggt 180

gagaaagaat ggtacttttt tagtcctaga gatagaaagt acccaaacgg tgcaaggcca 240

aacagggcag ctgcttcagg gtattggaaa gccactggca ctgataagac catagtggca 300

tcattgccat gtggaggagg aagatcacaa gagaacatta ttggtgtcaa aaaggctctt 360

gttttttaca aaggaaaacc cccaaagggt attaagacta attggatcat gcatgaatat 420

cgtcttgttg acaacaataa acctattaag ctcaaagatt cttccatgag gttggatgat 480

tgggtgctat gccgaattta caagaaatca aaatgtgcac taacttcaac agaatcatca 540

gaaactatgg taggtgaagt ggaacatgca gaagagacac aattccaaga aaccctattt 600

ccaatcacca aaaacacaac ctcacctctt caaaacacac tcatgtctca aaaatcagtg 660

tcctttttcaa acctattaga tgctatggac tactccatgt taagcagctt cttatctgaa 720

aattccaaca acccatcagg aattggaaca agttcaggtt tcaacactga aaattttaac 780

caacaacaat cttcccatat caacacttgc aacaattaca tgtctcagaa gaatcctcaa 840

tccaacactt taaagcacca gctttcaaac gtagatgaag acatgttata cccatcaaag 900

aagtacttga gttcctcttg caattttcct aacatcaatt ctcagtatga aaactacctt 960

atgaagcaat ctttgatgaa tcaacaattg cttttaggtc ctcatcatca atatcaaggc 1020

taatccaaaa taccacaaaa attaagtggt accaaaaaaa aaaagattat gaaatagaaa 1080

agttaagtaa gatttctaga agttgggccc accaaattac accaatgtca tcattaagta 1140

aggattgcta atttaatagt agtgcacaaa atatagtgtg ttctattttg tccattttaa 1200

ttttttttct aggaattg 1218

<210> 109
<211> 340
<212> PRT
<213> Medicago truncatula

<400> 109

Met Gly Thr Pro Gln Thr Asn Leu Pro Pro Gly Phe Arg Phe His Pro
1               5                   10                  15

Thr Asp Ala Glu Leu Ile Leu His Tyr Leu Arg Lys Lys Ile Ala Ser
            20                  25                  30

Ile Pro Leu Pro Val Ser Ile Ile Ala Glu Val Asp Ile Tyr Lys Leu
        35                  40                  45

Asp Pro Trp Asp Leu Pro Ala Lys Ala Ser Phe Gly Glu Lys Glu Trp
    50                  55                  60

Tyr Phe Phe Ser Pro Arg Asp Arg Lys Tyr Pro Asn Gly Ala Arg Pro
65                  70                  75                  80

Asn Arg Ala Ala Ala Ser Gly Tyr Trp Lys Ala Thr Gly Thr Asp Lys
                85                  90                  95

Thr Ile Val Ala Ser Leu Pro Cys Gly Gly Gly Arg Ser Gln Glu Asn
            100                 105                 110

Ile Ile Gly Val Lys Lys Ala Leu Val Phe Tyr Lys Gly Lys Pro Pro
        115                 120                 125

Lys Gly Ile Lys Thr Asn Trp Ile Met His Glu Tyr Arg Leu Val Asp
    130                 135                 140

Asn Asn Lys Pro Ile Lys Leu Lys Asp Ser Ser Met Arg Leu Asp Asp
145                 150                 155                 160

Trp Val Leu Cys Arg Ile Tyr Lys Lys Ser Lys Cys Ala Leu Thr Ser
                165                 170                 175

Thr Glu Ser Ser Glu Thr Met Val Gly Glu Val Glu His Ala Glu Glu
            180                 185                 190

Thr Gln Phe Gln Glu Thr Leu Phe Pro Ile Thr Lys Asn Thr Thr Ser
        195                 200                 205

```
Pro Leu Gln Asn Thr Leu Met Ser Gln Lys Ser Val Ser Phe Ser Asn
    210                 215                 220

Leu Leu Asp Ala Met Asp Tyr Ser Met Leu Ser Ser Phe Leu Ser Glu
225                 230                 235                 240

Asn Ser Asn Asn Pro Ser Gly Ile Gly Thr Ser Ser Gly Phe Asn Thr
                245                 250                 255

Glu Asn Phe Asn Gln Gln Gln Ser Ser His Ile Asn Thr Cys Asn Asn
            260                 265                 270

Tyr Met Ser Gln Lys Asn Pro Gln Ser Asn Thr Leu Lys His Gln Leu
        275                 280                 285

Ser Asn Val Asp Glu Asp Met Leu Tyr Pro Ser Lys Lys Tyr Leu Ser
    290                 295                 300

Ser Ser Cys Asn Phe Pro Asn Ile Asn Ser Gln Tyr Glu Asn Tyr Leu
305                 310                 315                 320

Met Lys Gln Ser Leu Met Asn Gln Gln Leu Leu Leu Gly Pro His His
                325                 330                 335

Gln Tyr Gln Gly
            340


<210>  110
<211>  6
<212>  PRT
<213>  Artificial sequence

<220>
<223>  motif I


<220>
<221>  VARIANT
<222>  (1)..(1)
<223>  /replace="Pro" /replace="Ser" /replace="Asn"

<220>
<221>  VARIANT
<222>  (2)..(2)
<223>  /replace="Leu" /replace="Ile" /replace="Ala"

<220>
<221>  VARIANT
<222>  (3)..(3)
<223>  /replace="Ser" /replace="Asp" /replace="Val" /replace="Gln"

<220>
<221>  VARIANT
<222>  (4)..(4)
<223>  /replace="Ile"
```

<220>
<221> VARIANT
<222> (6)..(6)
<223> /replace="Thr" /replace="Gly"

<400> 110

Ala Val Pro Val Ile Ala
1 5

<210> 111
<211> 6
<212> PRT
<213> Artificial sequence

<220>
<223> motif II

<220>
<221> VARIANT
<222> (2)..(2)
<223> /replace="Ser"

<220>
<221> VARIANT
<222> (3)..(3)
<223> /replace="Gln" /replace="Ala" /replace="Val"

<220>
<221> VARIANT
<222> (6)..(6)
<223> /replace="Ser"

<400> 111

Asn Gly Ser Arg Pro Asn
1 5

<210> 112
<211> 7
<212> PRT
<213> Artificial sequence

<220>
<223> motif III

<220>
<221> VARIANT
<222> (1)..(1)
<223> /replace="Tyr"

<220>
<221> VARIANT
<222> (2)..(2)
<223> /replace="Lys"

<220>
<221> VARIANT
<222> (3)..(3)
<223> /replace="Ile"

<220>
<221> VARIANT
<222> (4)..(4)
<223> /replace="His" /replace="Phe"

<220>
<221> VARIANT
<222> (5)..(5)
<223> /replace="Lys"

<220>
<221> VARIANT
<222> (7)..(7)
<223> /replace="Asn" /replace="Cys" /replace="Ser" /replace="Thr"

<400> 112

Cys Arg Leu Tyr Asn Lys Lys
1 5

<210> 113
<211> 7
<212> PRT
<213> Artificial sequence

<220>
<223> motif IV

<220>
<221> VARIANT
<222> (2)..(2)
<223> /replace="Gln" /replace="Thr"

<220>
<221> VARIANT
<222> (6)..(6)
<223> /replace="Val"

<220>
<221> VARIANT
<222> (7)..(7)
<223> /replace="Arg" /replace="Lys"

<400> 113

Asn Glu Trp Glu Lys Met Gln
1 5

<210> 114
<211> 10
<212> PRT
<213> Artificial sequence

<220>
<223> motif V

<220>
<221> VARIANT
<222> (4)..(4)
<223> /replace="Ala"

```
<220>
<221>  VARIANT
<222>  (10)..(10)
<223>  /replace="Asp"

<400>  114

Trp Gly Glu Thr Arg Thr Pro Glu Ser Glu
1               5                   10


<210>  115
<211>  10
<212>  PRT
<213>  Artificial sequence

<220>
<223>  motif VI


<220>
<221>  VARIANT
<222>  (1)..(1)
<223>  /replace="Leu"

<220>
<221>  VARIANT
<222>  (4)..(4)
<223>  /replace="Glu"

<220>
<221>  VARIANT
<222>  (6)..(6)
<223>  /replace="Arg" /replace="Ala" /replace="Val"

<220>
<221>  VARIANT
<222>  (7)..(7)
<223>  /replace="Val" /replace="Ala" /replace="Gln" /replace="Arg"

<220>
<221>  VARIANT
<222>  (8)..(8)
<223>  /replace="Glu"

<220>
<221>  VARIANT
<222>  (9)..(9)
<223>  /replace="Glu" /replace="Leu"

<220>
<221>  VARIANT
<222>  (10)..(10)
<223>  /replace="Gly" /replace="Asp"

<400>  115

Val Pro Lys Lys Glu Ser Met Asp Asp Ala
1               5                   10


<210>  116
<211>  10
<212>  PRT
```

<213> Artificial sequence

<220>
<223> motif VII

<220>
<221> VARIANT
<222> (2)..(2)
<223> /replace="Tyr"

<220>
<221> VARIANT
<222> (5)..(5)
<223> /replace="Ile"

<220>
<221> VARIANT
<222> (7)..(7)
<223> /replace="Ser"

<220>
<221> VARIANT
<222> (8)..(8)
<223> /replace="Met" /replace="Pro"

<220>
<221> VARIANT
<222> (9)..(9)
<223> /replace="Tyr"

<220>
<221> VARIANT
<222> (10)..(10)
<223> /replace="Asn"

<400> 116

Ser Leu Asp Asp Leu Gln Gly Leu Gly Ser
1               5                   10

<210> 117
<211> 14
<212> PRT
<213> Artificial sequence

<220>
<223> motif VIII

<220>
<221> VARIANT
<222> (3)..(3)
<223> /replace="Val" /replace="Ile"

<220>
<221> VARIANT
<222> (5)..(5)
<223> /replace="Lys"

<220>
<221> VARIANT
<222> (6)..(6)
<223> /replace="Ile" /replace="Ala"

<220>
<221> VARIANT
<222> (8)..(8)
<223> /replace="Ala" /replace="Ser"

<220>
<221> VARIANT
<222> (9)..(9)
<223> /replace="Glu"

<220>
<221> VARIANT
<222> (12)..(12)
<223> /replace="Gly"

<400> 117

Asp Ser Met Pro Arg Leu His Thr Asp Ser Ser Cys Ser Glu
1 5 10

<210> 118
<211> 20
<212> DNA
<213> Artificial sequence

<220>
<223> primer

<400> 118
ccaacactag taggataaag 20

<210> 119
<211> 20
<212> DNA
<213> Artificial sequence

<220>
<223> primer

<400> 119
accactgggc taattaatta 20

<210> 120
<211> 20
<212> DNA
<213> Artificial sequence

<220>
<223> primer

<400> 120
ggtcgaccca cgcgtccgct 20

<210> 121
<211> 20
<212> DNA
<213> Artificial sequence

<220>
<223> primer

<400>  121
aactgaagta cccgttctta                                                  20

<210>  122
<211>  20
<212>  DNA
<213>  Artificial sequence

<220>
<223>  primer

<400>  122
atcctccaca agagaaacta                                                  20

<210>  123
<211>  20
<212>  DNA
<213>  Artificial sequence

<220>
<223>  primer

<400>  123
agtacagtgt agcacaataa                                                  20

<210>  124
<211>  52
<212>  DNA
<213>  Artificial sequence

<220>
<223>  primer

<400>  124
ggggacaagt ttgtacaaaa aagcaggctt aaacaatggg gatggggatg ag              52

<210>  125
<211>  47
<212>  DNA
<213>  Artificial sequence

<220>
<223>  primer

<400>  125
ggggaccact ttgtacaaga aagctgggtt cgatcaccac ctgttcg                    47

<210>  126
<211>  51
<212>  DNA
<213>  Artificial sequence

<220>
<223>  primer

<400>  126
ggggacaagt ttgtacaaaa aagcaggctt aaacaatgga gtgcggtggt g               51

<210> 127
<211> 50
<212> DNA
<213> Artificial sequence

<220>
<223> primer

<400> 127
ggggaccact ttgtacaaga aagctgggtg tcacgttccg ttaattagaa 50

<210> 128
<211> 48
<212> DNA
<213> Artificial sequence

<220>
<223> primer

<400> 128
ggggacaagt ttgtacaaaa aagcaggctt aaacaatgcc gagcagcg 48

<210> 129
<211> 48
<212> DNA
<213> Artificial sequence

<220>
<223> primer

<400> 129
ggggaccact ttgtacaaga aagctgggtc tactgcatct gcagatga 48

<210> 130
<211> 48
<212> DNA
<213> Artificial sequence

<220>
<223> primer

<400> 130
ggggaccact ttgtacaaga aagctgggtc tactgcatct gcagatga 48

<210> 131
<211> 1100
<212> DNA
<213> Oryza sativa

<400> 131
catgtgcggc ggcgccatcc tctccgacct catcccgccg ccgcggcggg tcaccgccgg 60

cgacctctgg ctggagaaga ccaagaagca gcagcagcag aagaagaaga acaagggcgc 120

gaggaggctg ccactgcgcc aagaggagga ggatgatttc gaggccgact tcgaggagtt 180

cgaggtggat tccggcgagt gggaggtgga gtccgacgcc gacgaggcca agccgctcgc 240

cgcgccccgg agcggcttcg ctaaaggtgg attgaaaaac actactgttg ctggtgctga 300

tgggcctgca gcaaggtctg ctaaaaggaa gagaaagaac caattcaggg gtatccgcca 360

gcggccatgg ggcaaatggg ctgcggaaat cagagatcct cgcaaaggtg tccgcgtctg 420

gcttggcacc ttcaactctc ctgaggaagc tgccagagct tatgatgctg aagcacgaag 480

gattcgaggc aagaaggcca aggtcaattt cccagatggg gctccagtgg cttctcagag 540

gagtcatgct gagccctcct ccatgaacat gcctgctttc agcatcgaag agaagccggc 600

cgtcatgtca gcaggcaaca aaaccatgta caacacaaat gcttatgcct accctgctgt 660

tgagtacacc ttacaggagc catttgtgca gattcagaat gtctcatttg ttcctgcaat 720

gaacgcgatt gaggatactt tcgtgaacct gtcctctgat caagggagca actcctttgg 780

ttgctcggac tttagccagg agaatgatat caagacccct gacataactt ccatgcttgc 840

accgaccatg acaggtgttg atgactccgc attcctccag aacaatgcca gtgatgcaat 900

ggtacctcct gtgatgggga atgctagcat tgatcttgct gacctggagc cgtacatgaa 960

atttctgatc gatggtggtt cggatgagtc gattgacacc cttctgagct ctgatggatc 1020

tcaggatgtg gccagtagca tggacctttg gagcttcgat gacatgcccg tgtcggccga 1080

gttctactga ggggtttggg 1100

<210> 132
<211> 362
<212> PRT
<213> Oryza sativa

<400> 132

Met Cys Gly Gly Ala Ile Leu Ser Asp Leu Ile Pro Pro Pro Arg Arg
1 5 10 15

Val Thr Ala Gly Asp Leu Trp Leu Glu Lys Thr Lys Lys Gln Gln Gln
20 25 30

Gln Lys Lys Lys Asn Lys Gly Ala Arg Arg Leu Pro Leu Arg Gln Glu
35 40 45

Glu Glu Asp Asp Phe Glu Ala Asp Phe Glu Glu Phe Glu Val Asp Ser
50 55 60

Gly Glu Trp Glu Val Glu Ser Asp Ala Asp Glu Ala Lys Pro Leu Ala
65 70 75 80

Ala Pro Arg Ser Gly Phe Ala Lys Gly Gly Leu Lys Asn Thr Thr Val
85 90 95

Ala Gly Ala Asp Gly Pro Ala Ala Arg Ser Ala Lys Arg Lys Arg Lys
100 105 110

Asn Gln Phe Arg Gly Ile Arg Gln Arg Pro Trp Gly Lys Trp Ala Ala
115 120 125

```
Glu Ile Arg Asp Pro Arg Lys Gly Val Arg Val Trp Leu Gly Thr Phe
    130                 135                 140

Asn Ser Pro Glu Glu Ala Ala Arg Ala Tyr Asp Ala Glu Ala Arg Arg
145                 150                 155                 160

Ile Arg Gly Lys Lys Ala Lys Val Asn Phe Pro Asp Gly Ala Pro Val
                165                 170                 175

Ala Ser Gln Arg Ser His Ala Glu Pro Ser Ser Met Asn Met Pro Ala
            180                 185                 190

Phe Ser Ile Glu Glu Lys Pro Ala Val Met Ser Ala Gly Asn Lys Thr
        195                 200                 205

Met Tyr Asn Thr Asn Ala Tyr Ala Tyr Pro Ala Val Glu Tyr Thr Leu
    210                 215                 220

Gln Glu Pro Phe Val Gln Ile Gln Asn Val Ser Phe Val Pro Ala Met
225                 230                 235                 240

Asn Ala Ile Glu Asp Thr Phe Val Asn Leu Ser Ser Asp Gln Gly Ser
                245                 250                 255

Asn Ser Phe Gly Cys Ser Asp Phe Ser Gln Glu Asn Asp Ile Lys Thr
            260                 265                 270

Pro Asp Ile Thr Ser Met Leu Ala Pro Thr Met Thr Gly Val Asp Asp
        275                 280                 285

Ser Ala Phe Leu Gln Asn Asn Ala Ser Asp Ala Met Val Pro Pro Val
    290                 295                 300

Met Gly Asn Ala Ser Ile Asp Leu Ala Asp Leu Glu Pro Tyr Met Lys
305                 310                 315                 320

Phe Leu Ile Asp Gly Gly Ser Asp Glu Ser Ile Asp Thr Leu Leu Ser
                325                 330                 335

Ser Asp Gly Ser Gln Asp Val Ala Ser Ser Met Asp Leu Trp Ser Phe
            340                 345                 350

Asp Asp Met Pro Val Ser Ala Glu Phe Tyr
        355                 360
```

<210>  133
<211>  13
<212>  PRT

<213> Artificial sequence

<220>
<223> motif 1

<220>
<221> VARIANT
<222> (1)..(1)
<223> /replace = "Ile" /replace = "Val" /replace = "Leu" /replace = "Lys"

<220>
<221> VARIANT
<222> (2)..(2)
<223> /replace = "Lys" /replace = "Gln" /replace = "Asp" /replace = "Glu" /replace = "Ala"

<220>
<221> VARIANT
<222> (3)..(3)
<223> /replace = "Leu"

<220>
<221> VARIANT
<222> (4)..(4)
<223> /replace = "Tyr" /replace = "His"

<220>
<221> VARIANT
<222> (6)..(6)
<223> /replace = "Ser" /replace = "Arg" /replace = "Lys" /replace = "Thr" /replace = "Asp" /replace = "Gly" /replace = "His" /replace = "Leu" /replace = "Asn"

<220>
<221> VARIANT
<222> (7)..(7)
<223> /replace = "Thr" /replace = "Arg" /replace = "Asn" /replace = "Gly"

<220>
<221> VARIANT
<222> (9)..(9)
<223> /replace = "Arg" /replace = "Glu"

<220>
<221> VARIANT
<222> (10)..(10)
<223> /replace = "Leu" /replace = "Pro" /replace = "Thr"

<220>
<221> VARIANT
<222> (13)..(13)
<223> /replace = "Val"

<400> 133

```
Arg Arg Ile Arg Gly Ala Lys Ala Lys Val Asn Phe Pro
1               5                   10
```

<210> 134
<211> 9
<212> PRT

<213> Artificial sequence

<220>
<223> motif 2

<220>
<221> VARIANT
<222> (5)..(5)
<223> /replace = "Ser"

<220>
<221> VARIANT
<222> (6)..(6)
<223> /replace = "Val" /replace = "Leu"

<220>
<221> VARIANT
<222> (7)..(7)
<223> /replace = "Leu"

<220>
<221> VARIANT
<222> (8)..(8)
<223> /replace = "His" /replace = "Ala" /replace = "Tyr" /replace = "Gly" /replace = "Pro" /replace = "Geu"

<220>
<221> VARIANT
<222> (9)..(9)
<223> /replace = "Gly" /replace = "His" /replace = "Tyr" /replace = "Glu" /replace = "Gln" /replace = "Asn"

<400> 134

Met Cys Gly Gly Ala Ile Ile Ser Asp
1 5

<210> 135
<211> 15
<212> PRT
<213> Artificial sequence

<220>
<223> motif 3

<220>
<221> VARIANT
<222> (1)..(1)
<223> /replace = "Asn" /replace = "Arg" /replace = "Ser" /replace = "His" /replace = "Met" /replace = "Gln' /replace = "Pro" /replace = "Ala" /replace = "Glu"

<220>
<221> VARIANT
<222> (2)..(2)
<223> /replace = "Lys" /replace = "His" /replace = "Ala" /replace = "Gly" /replace = "Glu" /replace = "Val" /replace = "Pro" /replace = "Met"

<220>
<221> VARIANT
<222> (3)..(3)

<223> /replace = "Lys" /replace = "Ala" /replace = "Arg" /replace = "Ser" /replace = "Gln" /replace = "Thr" /replace = "Val" /replace = "Gly" /replace = "His" /replace = "Pro"

<220>
<221> VARIANT
<222> (4)..(4)
<223> /replace = "Lys" /replace = "Gln" /replace = "Ser" /replace = "Gly"

<220>
<221> VARIANT
<222> (5)..(5)
<223> /replace = "Gly" /replace = "Pro" /replace = "Ser" /replace = "Arg" /replace = "Thr" /replace = "Ala" /replace = "Asn"

<220>
<221> VARIANT
<222> (6)..(6)
<223> /replace = "Asn" /replace = "Arg" /replace = "Ser" /replace = "Ala" /replace = "Tyr" /replace = "His" /replace = "Gly"

<220>
<221> VARIANT
<222> (7)..(7)
<223> /replace = "Gln" /replace = "His" /replace = "Val" /replace = "Arg" /replace = "Lys" /replace = "Ala" /replace = "Pro" /replace = "Gly"

<220>
<221> VARIANT
<222> (8)..(8)
<223> /replace = "Phe"

<220>
<221> VARIANT
<222> (9)..(9)
<223> /replace = "Trp" /replace = "Lys" /replace = "Lys" /replace = "Leu" /replace = "Met"

<220>
<221> VARIANT
<222> (11)..(11)
<223> /replace = "Val"

<220>
<221> VARIANT
<222> (12)..(12)
<223> /replace = "Gln" /replace = "His"

<220>
<221> VARIANT
<222> (13)..(13)
<223> /replace = "Gln" /replace = "Trp" /replace = "Lys"

<220>
<221> VARIANT
<222> (15)..(15)
<223> /replace = "Lys" /replace = "Thr"

<400> 135

```
Lys Arg Glu Arg Lys Thr Leu Tyr Arg Gly Ile Arg Arg Arg Pro
1               5                   10                  15
```

```
<210>  136
<211>  17
<212>  PRT
<213>  Artificial sequence

<220>
<223>  motif 4


<220>
<221>  VARIANT
<222>  (3)..(3)
<223>  /replace = "Thr" /replace = "Glu" /replace = "Val"

<220>
<221>  VARIANT
<222>  (4)..(4)
<223>  /replace = "Ser"

<220>
<221>  VARIANT
<222>  (9)..(9)
<223>  /replace = "Asp" /replace = "Glu" /replace = "Ser" /replace =
       "Asn"

<220>
<221>  VARIANT
<222>  (10)..(10)
<223>  /replace = "Ser"

<220>
<221>  VARIANT
<222>  (12)..(12)
<223>  /replace = "Glu"

<220>
<221>  VARIANT
<222>  (13)..(13)
<223>  /replace = "Tyr" /replace = "Leu"

<220>
<221>  VARIANT
<222>  (14)..(14)
<223>  /replace = "Ser"

<220>
<221>  VARIANT
<222>  (15)..(15)
<223>  /replace = "Gln" /replace = "Leu"

<220>
<221>  VARIANT
<222>  (16)..(16)
<223>  /replace = "Gly" /replace = "Ser"

<220>
<221>  VARIANT
<222>  (17)..(17)
<223>  /replace = "Glu" /replace = "Asp"

<400>  136

Ser Asp Gln Gly Ser Asn Ser Phe Gly Cys Ser Asp Phe Gly Trp Glu
1               5                   10                  15
```

Asn

<210> 137
<211> 7
<212> PRT
<213> Artificial sequence

<220>
<223> motif 5

<220>
<221> VARIANT
<222> (1)..(1)
<223> /replace = "Ile" /replace = "Phe" /replace = "Met"

<220>
<221> VARIANT
<222> (3)..(3)
<223> /replace = "Thr" /replace = "Asn" /replace = "Met"

<220>
<221> VARIANT
<222> (4)..(4)
<223> /replace = "Tyr" /replace = "Leu" /replace = "Ile"

<220>
<221> VARIANT
<222> (5)..(5)
<223> /replace = "Glu" /replace = "Gln" /replace = "Gly"

<220>
<221> VARIANT
<222> (6)..(6)
<223> /replace = "Asp" /replace = "His" /replace = "Glu"

<220>
<221> VARIANT
<222> (7)..(7)
<223> /replace = "Tyr" /replace = "Phe" /replace = "Ser" /replace = "Met" /replace = "Leu" /replace = "Val" /replace = "Asp" /replace = "His" /replace = "Asn" /replace = "Glu" /replace = "Gly"

<400> 137

Leu Trp Ser Phe Asp Asn Ile
1               5

<210> 138
<211> 12
<212> PRT
<213> Artificial sequence

<220>
<223> motif 6

<220>
<221> VARIANT
<222> (1)..(1)

<223> /replace = "Glu" /replace = "Ser"

<220>
<221> VARIANT
<222> (2)..(2)
<223> /replace = "Ala" /replace = "Trp" /replace = "Asp"

<220>
<221> VARIANT
<222> (3)..(3)
<223> /replace = "Ala"

<220>
<221> VARIANT
<222> (4)..(4)
<223> /replace = "Asp" /replace = "Leu"

<220>
<221> VARIANT
<222> (5)..(5)
<223> /replace = "Ala" /replace = "Gly" /replace = "Glu"

<220>
<221> VARIANT
<222> (6)..(6)
<223> /replace = "Gly" /replace = "Leu"

<220>
<221> VARIANT
<222> (7)..(7)
<223> /replace = "Glu" /replace = "Arg" /replace = "Gly" /replace = "Gln" /replace = "Trp"

<220>
<221> VARIANT
<222> (8)..(8)
<223> /replace = "Gly" /replace = "Val" /replace = "Asp" /replace = "Arg"

<220>
<221> VARIANT
<222> (10)..(10)
<223> /replace = "Lys" /replace = "Val" /replace = "Tyr" /replace = "Gly" /replace = "Asp" /replace = "Leu" /replace = "Ile"

<220>
<221> VARIANT
<222> (11)..(11)
<223> /replace = "Arg" /replace = "Asp" /replace = "Ser" /replace = "Asn" /replace = "Ala" /replace = "Glu"

<220>
<221> VARIANT
<222> (12)..(12)
<223> /replace = "Gly" /replace = "Thr" /replace = "Glu" /replace = "Arg" /replace = "Ala" /replace = "Tyr" /replace = "Leu" /replace = "Leu" /replace = "Phe"

<400> 138

```
Asp Phe Glu Ala Asp Phe Asn Glu Phe Glu Val Asp
1               5                   10
```

<210> 139

<211> 884
<212> DNA
<213> Oryza sativa

<400> 139
gactcgtcag cttagagcac cgcaagcgcg cagcagcagc aaagatgtgt ggcggcgcga 60

tcatttccga cttcatcccg cagcgggaag cccaccgcgc ggccaccggc agcaagcgtg 120

ccctctgcgc ctccgacttc tggccgtcgg cgtcgcagga agccgccgac ttcgaccacc 180

tcaccgcccc ctgcaccttc acccccgacc aagcggcaga ggagccgacc aagaagcggg 240

agcggaagac gctgtaccgt ggcatcaggc ggcggccgtg gggaagtgg gcggcggaga 300

tccgcgaccc ggcgaagggc gcgcgcgtct ggctcggcac cttcgccacc gccgaggcgg 360

cggcccgcgc ctacgaccgc gccgcccgcc gcatccgcgg ggccaaggcc aaggtcaact 420

tccccaacga ggacccgcca ctcgacgacc cggccgccga cggccacagc cacggcggcg 480

ccgccatccc gtgcagggag ttcatggact acgacgccgt catggcgggc ttcttccacc 540

agccctacgt cgtcgccgac ggcgtgccgg ccgtgccggc ggaggaggcg cccacggtgg 600

cgtacgtgca ccaccacctg ccgccgcagc cgcagcagga cgcggggctg gagctctgga 660

gctttgataa catccacacg gccgtgccga tgtgagatcg atctgatgat cattcagatc 720

gatgctagct catgtgtttt taattatatc ttcacagcag aaacaaaaaa aagttcaatt 780

cagtttattt tgttgttata cgttttaaaa tgtttcatta ggttttcatg ttataggagt 840

gatttatcgg attgaactct caagtgaccg acttctgagt tctt 884

<210> 140
<211> 216
<212> PRT
<213> Oryza sativa

<400> 140

Met Cys Gly Gly Ala Ile Ile Ser Asp Phe Ile Pro Gln Arg Glu Ala
1 5 10 15

His Arg Ala Ala Thr Gly Ser Lys Arg Ala Leu Cys Ala Ser Asp Phe
20 25 30

Trp Pro Ser Ala Ser Gln Glu Ala Ala Asp Phe Asp His Leu Thr Ala
35 40 45

Pro Cys Thr Phe Thr Pro Asp Gln Ala Ala Glu Glu Pro Thr Lys Lys
50 55 60

Arg Glu Arg Lys Thr Leu Tyr Arg Gly Ile Arg Arg Arg Pro Trp Gly
65 70 75 80

Lys Trp Ala Ala Glu Ile Arg Asp Pro Ala Lys Gly Ala Arg Val Trp
85 90 95

Leu Gly Thr Phe Ala Thr Ala Glu Ala Ala Ala Arg Ala Tyr Asp Arg
100 105 110

Ala Ala Arg Arg Ile Arg Gly Ala Lys Ala Lys Val Asn Phe Pro Asn
115 120 125

Glu Asp Pro Pro Leu Asp Asp Pro Ala Ala Asp Gly His Ser His Gly
130 135 140

Gly Ala Ala Ile Pro Cys Arg Glu Phe Met Asp Tyr Asp Ala Val Met
145 150 155 160

Ala Gly Phe Phe His Gln Pro Tyr Val Val Ala Asp Gly Val Pro Ala
165 170 175

Val Pro Ala Glu Glu Ala Pro Thr Val Ala Tyr Val His His His Leu
180 185 190

Pro Pro Gln Pro Gln Gln Asp Ala Gly Leu Glu Leu Trp Ser Phe Asp
195 200 205

Asn Ile His Thr Ala Val Pro Met
210 215

<210> 141
<211> 1403
<212> DNA
<213> Triticum aestivum

<400> 141
ctctccaccc gcggccccac gtgctcccag ccatgtgcgg cggcgccatc ctctccgaca 60

tcatcccgcc gccgcgccgg gccaccggcg gcaacgtctg gcgggcggac aagaagagga 120

gggccaggcc cgacgccgcc gcggggaggc cccgccgcgt gcccgaggag gagttccagg 180

aggaggaggg cgacgcggag ttcgaggccg acttcgaggg gttcgtggag gcggaggagg 240

agtccgacgg cgaggccaag cccttccccg tccgcaggac cggcttctcc ggagatggac 300

tgaaggcaac tgctgctggt gatgatgact gtgcctcagg gtctgctaaa aggaagagaa 360

agagccagtt caggggcatc cgccgccgcc cttggggtaa atgggctgct gaaataagag 420

atcctcgcaa gggtgtccgt gtctggcttg gcacttacaa ctctgctgag gaagctgcca 480

gagcctatga tgttgaagcc cgcagaattc gtggcaagaa ggcaaaggtc aatttcccag 540

aagaagctcc catggatcct cagcaacgct gcgctacctc tgtgaaggtg cccgagttca 600

acaccgaaca gaagccagta ctcaacacca tgggcaacac agatgtgtat tcctgccctg 660

ctgttgacta caccttaaat cagcaatttg tgcagcctca gaacatgtcg tttgtgccta 720

cagtgaatgc agttgaggct cctttcatga atttttcctc tgaccagggg agcaactcct 780

```
ttagttgctc agacttcagc tgggagaatg atatcaagac ccctgacata acttctgtgc     840
ttgcatccat tcccacctca actgaggtca atgaatctgc atttctccag aacaatggca     900
ttaattcaac ggtacctcct gtgatgggtg atgctaatgt tgatcttgcc gacttggagc     960
catacatgaa gttcctgatg gacgatggtt cagatgagtc aattgacagc attctaagct    1020
gtgatgtacc gcaggacgtt gtcggcaaca tgggcctttg gacctttgat gacatgccct    1080
tgtctgctgg tttctactga gggaatcgag gtcgctgggt gcctgtatat atagacaaag    1140
gaataagtat tctggacatc aacaagtgct tgtgtctggt gcctctagaa tcgagcagta    1200
gcgacgtcag tctatggtta tgtctagctt aaatggtcag gagacctaag tcttttgcaa    1260
tagacctctg tcttgtgccc ccagactata ttatatctat atatgaaacc agtatgtgat    1320
gggaactgct tattttgtat tcctgtttct accttattgt aattgctaca agtggctgta    1380
aacctttttaa ctttgaaaaa aaa                                            1403
```

<210> 142
<211> 355
<212> PRT
<213> Triticum aestivum

<400> 142

```
Met Cys Gly Gly Ala Ile Leu Ser Asp Ile Ile Pro Pro Pro Arg Arg
1               5                   10                  15

Ala Thr Gly Gly Asn Val Trp Arg Ala Asp Lys Lys Arg Arg Ala Arg
            20                  25                  30

Pro Asp Ala Ala Ala Gly Arg Pro Arg Arg Val Pro Glu Glu Glu Phe
        35                  40                  45

Gln Glu Glu Glu Gly Asp Ala Glu Phe Glu Ala Asp Phe Glu Gly Phe
    50                  55                  60

Val Glu Ala Glu Glu Glu Ser Asp Gly Glu Ala Lys Pro Phe Pro Val
65                  70                  75                  80

Arg Arg Thr Gly Phe Ser Gly Asp Gly Leu Lys Ala Thr Ala Ala Gly
                85                  90                  95

Asp Asp Asp Cys Ala Ser Gly Ser Ala Lys Arg Lys Arg Lys Ser Gln
            100                 105                 110

Phe Arg Gly Ile Arg Arg Arg Pro Trp Gly Lys Trp Ala Ala Glu Ile
        115                 120                 125

Arg Asp Pro Arg Lys Gly Val Arg Val Trp Leu Gly Thr Tyr Asn Ser
    130                 135                 140
```

```
Ala Glu Glu Ala Ala Arg Ala Tyr Asp Val Glu Ala Arg Arg Ile Arg
145                 150                 155                 160

Gly Lys Lys Ala Lys Val Asn Phe Pro Glu Glu Ala Pro Met Asp Pro
                165                 170                 175

Gln Gln Arg Cys Ala Thr Ser Val Lys Val Pro Glu Phe Asn Thr Glu
            180                 185                 190

Gln Lys Pro Val Leu Asn Thr Met Gly Asn Thr Asp Val Tyr Ser Cys
        195                 200                 205

Pro Ala Val Asp Tyr Thr Leu Asn Gln Gln Phe Val Gln Pro Gln Asn
    210                 215                 220

Met Ser Phe Val Pro Thr Val Asn Ala Val Glu Ala Pro Phe Met Asn
225                 230                 235                 240

Phe Ser Ser Asp Gln Gly Ser Asn Ser Phe Ser Cys Ser Asp Phe Ser
                245                 250                 255

Trp Glu Asn Asp Ile Lys Thr Pro Asp Ile Thr Ser Val Leu Ala Ser
            260                 265                 270

Ile Pro Thr Ser Thr Glu Val Asn Glu Ser Ala Phe Leu Gln Asn Asn
        275                 280                 285

Gly Ile Asn Ser Thr Val Pro Pro Val Met Gly Asp Ala Asn Val Asp
    290                 295                 300

Leu Ala Asp Leu Glu Pro Tyr Met Lys Phe Leu Met Asp Asp Gly Ser
305                 310                 315                 320

Asp Glu Ser Ile Asp Ser Ile Leu Ser Cys Asp Val Pro Gln Asp Val
                325                 330                 335

Val Gly Asn Met Gly Leu Trp Thr Phe Asp Asp Met Pro Leu Ser Ala
            340                 345                 350

Gly Phe Tyr
        355

<210>  143
<211>  908
<212>  DNA
<213>  Oryza sativa

<400>  143
atccaaatcc aacgtccgcc taaagaaaaa cacgcacaca cttcttctgc ttccctcccc      60
```

ttgtttccgc tccacttccc ttccaagcaa gaaaaccgag gcttcagctt agctaggacc 120

gaccgatccg atgtgcggcg gtgcaatcat ctacgactac atcccggcgc gccgccggtt 180

gtgcgcctcc gacttctggc ccgacgccga cgactccgac ccccacaccc ccgctcccga 240

gaaaccgccg cgcgcgaaga gggagcggaa gaaccagtac cgcgggatca ggcagcggcc 300

gtgggggaag tgggcggcgg agatccgcga cccggtgaag gggtgcgcg tctggctcgg 360

cacctacccg accgccgagg ccgccgcgcg ggcctacgac cgcgccgcgc gccgcatcag 420

gggcgccaag gcgaaggtca acttccccaa cgacttcggc gccgcccccg cgccggccgc 480

ggcggcggcg aaggccgtcc ctcgcgtcgc gcccacgccg gccgtgctcc cgccgcccaa 540

gatggaggcg gtgtccgagg gcgccggcgc ctgctcctcc gacgaggtca aggagctgtc 600

cgaggagctg ctcgcgtacg agaactacat gagcttcctc ggcatcccct acatggaggg 660

cggcgccgcc tccgccgccg gcgccgagga agccgcggcg cccgccgggc tctggacctt 720

cgaagactac gagctgccgt cgctagcgct ctagtaaaaa tgtcacaata aaaatgacat 780

gtatgtgaaa aaatttttcc ctacccgtag taagtaacca gtgtcttttt ttaccgtact 840

ctatgcgtta tttttgttga aattaatcca ttgttgaagt tgtaactgtg catggtcggc 900

gccagtct 908

<210> 144
<211> 207
<212> PRT
<213> Oryza sativa

<400> 144

Met Cys Gly Gly Ala Ile Ile Tyr Asp Tyr Ile Pro Ala Arg Arg Arg
1 5 10 15

Leu Cys Ala Ser Asp Phe Trp Pro Asp Ala Asp Asp Ser Asp Pro His
20 25 30

Thr Pro Ala Pro Glu Lys Pro Pro Arg Ala Lys Arg Glu Arg Lys Asn
35 40 45

Gln Tyr Arg Gly Ile Arg Gln Arg Pro Trp Gly Lys Trp Ala Ala Glu
50 55 60

Ile Arg Asp Pro Val Lys Gly Val Arg Val Trp Leu Gly Thr Tyr Pro
65 70 75 80

Thr Ala Glu Ala Ala Ala Arg Ala Tyr Asp Arg Ala Ala Arg Arg Ile
85 90 95

Arg Gly Ala Lys Ala Lys Val Asn Phe Pro Asn Asp Phe Gly Ala Ala
100 105 110

Pro Ala Pro Ala Ala Ala Ala Ala Lys Ala Val Pro Arg Val Ala Pro
115 120 125

Thr Pro Ala Val Leu Pro Pro Pro Lys Met Glu Ala Val Ser Glu Gly
130 135 140

Ala Gly Ala Cys Ser Ser Asp Glu Val Lys Glu Leu Ser Glu Glu Leu
145 150 155 160

Leu Ala Tyr Glu Asn Tyr Met Ser Phe Leu Gly Ile Pro Tyr Met Glu
165 170 175

Gly Gly Ala Ala Ser Ala Ala Gly Ala Glu Glu Ala Ala Ala Pro Ala
180 185 190

Gly Leu Trp Thr Phe Glu Asp Tyr Glu Leu Pro Ser Leu Ala Leu
195 200 205

<210> 145
<211> 1574
<212> DNA
<213> Oryza sativa

<400> 145
aaaggcattc gcaacacaca cttgaagaaa aaaaacacga cgaacacgtt aaaaaaaggt 60

cgaagagaag tgggagaccc aaaccgcgaa caatgtgtgg aggcgccatc ctcgccgagt 120

tcatcccggc gccgtcgcgc gccgcggcgg cgaccaagcg ggtgaccgcc agccacctgt 180

ggccggccgg ctccaagaac gccgcccgcg gcaagagcaa gagcaagagg cagcagagga 240

gcttcgccga cgtcgacgac ttcgaggccg ccttcgagca gttcgacgat gactccgact 300

tcgacgacgc ggaggaagaa gacgaaggac acttcgtgtt cgcgtccaaa tctcgtgtcg 360

tcgccgggca cgacgggcgc gcggcggcga gggcggcgag caagaagaag cgggggcggc 420

acttccgagg catccggcag cggccatggg ggaagtgggc ggcggagatc cgcgacccgc 480

acaagggcac gcgcgtctgg ctcggcacgt tcaacacccc ggaggaggcc gcacgcgcct 540

acgacgtcga ggcgcgccgc ctccgcggca gcaaggccaa ggtcaacttc cccgccacgc 600

ccgccgccgc gcgcccacgc cgcggcaaca cgagagccac cgccgtgcca ccgccggcga 660

cagcacccgc cgccgccccg ccgcgcggac tgaagcgaga attctcgccg cctgctgaga 720

ccgcgctacc tttcttcacc aacggcttcg tcgacctgac gaccgccgcg gcgccgccac 780

cggccatgat gatgacgagc tccttcaccg acagcgtcgc cacgtcggag tccggcggga 840

gccccgccaa gaaggcgagg tccgacgacg tcgactcgtc cgagggcagc gtcggcggcg 900

gcagcgacac gctgggtttc accgacgagc tggagttcga cccgttcatg ctgttccagc 960

tcccctactc cgacggctac gagtccatcg acagcctctt cgccgccggc gacgccaaca 1020

```
gcgcgaacac cgacatgaac gccggcgtca acctgtggag cttcgacgac ttcccaatcg   1080
acggcgccct tttctgatgt actcctccat tgatgcagtt tgtgcactca attgttaatc   1140
acgtcgtcgt tgatgaatgt ttaaccggag gatgatgggg tgcagctgat atcatggctt   1200
gcttgattac cgaattatat tgcggtgttt gtgtttgtca attagattct gaatctgtac   1260
tactgccgat cttatgatca aaactatata ttaagtttat gtactcttaa tttgtgggct   1320
acttttacgg ggtcttcgta ctgctggtca aatagtccta tgaaatcgat catgtcggca   1380
atatcgtcgt caattatcgt ggtcgtggtt gttaatgatg tcaaataagt ctatgaaaac   1440
ctggtcctct tggtgtttat gtaccactga tcgatccatc atttgatctc tgtcatgttg   1500
agatggatcg tgtaagaata tcataatttg ctggattcag tccagtcgta atgtattttg   1560
gtttgtacaa ctgc                                                     1574
```

```
<210>  146
<211>  334
<212>  PRT
<213>  Oryza sativa

<400>  146

Met Cys Gly Gly Ala Ile Leu Ala Glu Phe Ile Pro Ala Pro Ser Arg
1               5                   10                  15

Ala Ala Ala Ala Thr Lys Arg Val Thr Ala Ser His Leu Trp Pro Ala
            20                  25                  30

Gly Ser Lys Asn Ala Ala Arg Gly Lys Ser Lys Ser Lys Arg Gln Gln
        35                  40                  45

Arg Ser Phe Ala Asp Val Asp Asp Phe Glu Ala Ala Phe Glu Gln Phe
    50                  55                  60

Asp Asp Asp Ser Asp Phe Asp Asp Ala Glu Glu Glu Asp Glu Gly His
65                  70                  75                  80

Phe Val Phe Ala Ser Lys Ser Arg Val Val Ala Gly His Asp Gly Arg
                85                  90                  95

Ala Ala Ala Arg Ala Ala Ser Lys Lys Lys Arg Gly Arg His Phe Arg
            100                 105                 110

Gly Ile Arg Gln Arg Pro Trp Gly Lys Trp Ala Ala Glu Ile Arg Asp
        115                 120                 125

Pro His Lys Gly Thr Arg Val Trp Leu Gly Thr Phe Asn Thr Pro Glu
    130                 135                 140
```

```
Glu Ala Ala Arg Ala Tyr Asp Val Glu Ala Arg Arg Leu Arg Gly Ser
145                 150                 155                 160

Lys Ala Lys Val Asn Phe Pro Ala Thr Pro Ala Ala Ala Arg Pro Arg
                165                 170                 175

Arg Gly Asn Thr Arg Ala Thr Ala Val Pro Pro Pro Ala Thr Ala Pro
            180                 185                 190

Ala Ala Ala Pro Pro Arg Gly Leu Lys Arg Glu Phe Ser Pro Pro Ala
        195                 200                 205

Glu Thr Ala Leu Pro Phe Phe Thr Asn Gly Phe Val Asp Leu Thr Thr
    210                 215                 220

Ala Ala Ala Pro Pro Pro Ala Met Met Met Thr Ser Ser Phe Thr Asp
225                 230                 235                 240

Ser Val Ala Thr Ser Glu Ser Gly Gly Ser Pro Ala Lys Lys Ala Arg
                245                 250                 255

Ser Asp Asp Val Asp Ser Ser Glu Gly Ser Val Gly Gly Gly Ser Asp
            260                 265                 270

Thr Leu Gly Phe Thr Asp Glu Leu Glu Phe Asp Pro Phe Met Leu Phe
        275                 280                 285

Gln Leu Pro Tyr Ser Asp Gly Tyr Glu Ser Ile Asp Ser Leu Phe Ala
    290                 295                 300

Ala Gly Asp Ala Asn Ser Ala Asn Thr Asp Met Asn Ala Gly Val Asn
305                 310                 315                 320

Leu Trp Ser Phe Asp Asp Phe Pro Ile Asp Gly Ala Leu Phe
                325                 330
```

```
<210>  147
<211>  1451
<212>  DNA
<213>  Triticum aestivum

<400>  147
ctttcctttc gcttagctct ccacccgcgg ccccacgtgc tcccagccat gtgcggcggc      60

gccatcctct ccgacatcat cccgccgccg cgccggcccg ccggcggccg cctctggcag     120

gcggacagga agaagaggag ggccgggccc cgccgcgtgc ccgaggagga gcccgaggag     180

gaggcggagg agggcgacga ggacttcgag gccgacttcg aggggttcgt ggacgaggag     240

tccgacggcg aggtcaagcc cttccccgcc cgcaggagcg gcttctccgg agatggattg     300

aaggcaactg ctgctggtga atatgactgt gcctcagggt ctgctaaaag gaagagaaag     360
```

aaccagttca ggggcatccg ccgccgccct tggggtaaat gggctgctga aataagagat 420

cctcgcaagg gtgtccgtgt ctggcttggt acttacaact ccgctgagga agctgccaga 480

gcctatgatg ttgaagcccg cagaattcgt ggcaagaagg caaaggtcaa tttcccagaa 540

gaagctccta tggctcctca gcaacgctgc gctactgctg tgaaggtgcc cgagttcaac 600

accgaacaga agccggtact caacaccatg ggcaacgcag atgtgtattc ctgctctgct 660

gttgactaca ccttaaatca gcaatttgtg cagcctcaga acatgtcgtt tgtgcctaca 720

gtgaatgcag ttgaggcccc tttcatgaat ttttcctctg accagggtag caactccttt 780

agttgctcag acttcagctg ggagaatgat atcaagaccc ctgacataac ttctgtgctt 840

gcatccattc ccacctcaac agaggtcaat gaatctgcat ttctccagaa caatggcatc 900

aattcaacgg tacctcctgt gatgggtgat gctaatgttg atcttgccga cttggagcca 960

tacatgaagt tcctgatgga cgatggttca gatgagtcaa ttgacagcat tctaagctgt 1020

gatgtacccc aggatgtggt cggcaacatg ggcctttgga cctttgatga catgcccttg 1080

tctgctggtt tctactgagg gaatcgaggt cgctgggtgc ctgtatatat agacaaaggg 1140

aataagtatt caggacatca acaagtgctt gtgtctggtg cctctagaat tgagcagtag 1200

cgatgtcagt ctatggttat gtctagctta aatggtcagg tgactgaggt cttttgcaat 1260

agacctctgt cttgtgcccc cagactatac ttatatctat atatgagacc agtatgtgat 1320

ggggaactgc ttattttgta ttcatgtttc taccttattg taactgctac aagaggctgt 1380

aaacctttta aatttgaagc cagtgtttgt tctgttgtgc ttaaaaaaaa aaaaaaaaaa 1440

aaagtatctg c 1451

<210> 148
<211> 349
<212> PRT
<213> Triticum aestivum

<400> 148

Met Cys Gly Gly Ala Ile Leu Ser Asp Ile Ile Pro Pro Pro Arg Arg
1 5 10 15

Pro Ala Gly Gly Arg Leu Trp Gln Ala Asp Arg Lys Lys Arg Arg Ala
20 25 30

Gly Pro Arg Arg Val Pro Glu Glu Glu Pro Glu Glu Glu Ala Glu Glu
35 40 45

Gly Asp Glu Asp Phe Glu Ala Asp Phe Glu Gly Phe Val Asp Glu Glu
50 55 60

Ser Asp Gly Glu Val Lys Pro Phe Pro Ala Arg Arg Ser Gly Phe Ser
65 70 75 80

Gly Asp Gly Leu Lys Ala Thr Ala Ala Gly Glu Tyr Asp Cys Ala Ser
85 90 95

Gly Ser Ala Lys Arg Lys Arg Lys Asn Gln Phe Arg Gly Ile Arg Arg
100 105 110

Arg Pro Trp Gly Lys Trp Ala Ala Glu Ile Arg Asp Pro Arg Lys Gly
115 120 125

Val Arg Val Trp Leu Gly Thr Tyr Asn Ser Ala Glu Glu Ala Ala Arg
130 135 140

Ala Tyr Asp Val Glu Ala Arg Arg Ile Arg Gly Lys Lys Ala Lys Val
145 150 155 160

Asn Phe Pro Glu Glu Ala Pro Met Ala Pro Gln Gln Arg Cys Ala Thr
165 170 175

Ala Val Lys Val Pro Glu Phe Asn Thr Glu Gln Lys Pro Val Leu Asn
180 185 190

Thr Met Gly Asn Ala Asp Val Tyr Ser Cys Ser Ala Val Asp Tyr Thr
195 200 205

Leu Asn Gln Gln Phe Val Gln Pro Gln Asn Met Ser Phe Val Pro Thr
210 215 220

Val Asn Ala Val Glu Ala Pro Phe Met Asn Phe Ser Ser Asp Gln Gly
225 230 235 240

Ser Asn Ser Phe Ser Cys Ser Asp Phe Ser Trp Glu Asn Asp Ile Lys
245 250 255

Thr Pro Asp Ile Thr Ser Val Leu Ala Ser Ile Pro Thr Ser Thr Glu
260 265 270

Val Asn Glu Ser Ala Phe Leu Gln Asn Asn Gly Ile Asn Ser Thr Val
275 280 285

Pro Pro Val Met Gly Asp Ala Asn Val Asp Leu Ala Asp Leu Glu Pro
290 295 300

Tyr Met Lys Phe Leu Met Asp Asp Gly Ser Asp Glu Ser Ile Asp Ser
305 310 315 320

Ile Leu Ser Cys Asp Val Pro Gln Asp Val Val Gly Asn Met Gly Leu
325 330 335

```
Trp Thr Phe Asp Asp Met Pro Leu Ser Ala Gly Phe Tyr
            340                 345


<210>  149
<211>  602
<212>  DNA
<213>  Arabidopsis thaliana

<400>  149
gttctagttt ggagttggaa gcgtaaaaaa caaaaaacaa aaatgtgtgg gggagctatc      60

atttctgatt tcatctggtc gaaatctgag tcagaaccga gtcaactcgg ctctgttagc     120

agcaggaaga agcgtaaacc cgtctcagtg agtgaagaaa gagatgggaa acgagagagg     180

aagaatctgt acagagggat aaggcagagg ccatggggca aatgggcagc ggagattcgt     240

gacccgagca aaggtgtacg tgtctggctt ggcacattca aaaccgccga cgaagctgct     300

cgagcctacg acgttgctgc catcaaaatc cgtggccgga aagccaaact gaatttccca     360

aacactcaag tagaagaaga agccgatact aaaccagggg ggaatcaaaa tgagctgatt     420

tcggaaaacc aagtagagag cttatcggag gacctgatgg cattggagga ttacatgaga     480

ttctatcaga ttccggttgc cgacgaccaa tcggcgaccg atattggaaa tttatggagc     540

tatcaagact ccaattaaat ctcttatttc ccggccggtt tgctcactca ttaatatgct     600

gc                                                                    602


<210>  150
<211>  171
<212>  PRT
<213>  Arabidopsis thaliana

<400>  150

Met Cys Gly Gly Ala Ile Ile Ser Asp Phe Ile Trp Ser Lys Ser Glu
1               5                   10                  15


Ser Glu Pro Ser Gln Leu Gly Ser Val Ser Ser Arg Lys Lys Arg Lys
            20                  25                  30


Pro Val Ser Val Ser Glu Glu Arg Asp Gly Lys Arg Glu Arg Lys Asn
        35                  40                  45


Leu Tyr Arg Gly Ile Arg Gln Arg Pro Trp Gly Lys Trp Ala Ala Glu
    50                  55                  60


Ile Arg Asp Pro Ser Lys Gly Val Arg Val Trp Leu Gly Thr Phe Lys
65                  70                  75                  80


Thr Ala Asp Glu Ala Ala Arg Ala Tyr Asp Val Ala Ala Ile Lys Ile
                85                  90                  95
```

Arg Gly Arg Lys Ala Lys Leu Asn Phe Pro Asn Thr Gln Val Glu Glu
100 105 110

Glu Ala Asp Thr Lys Pro Gly Gly Asn Gln Asn Glu Leu Ile Ser Glu
115 120 125

Asn Gln Val Glu Ser Leu Ser Glu Asp Leu Met Ala Leu Glu Asp Tyr
130 135 140

Met Arg Phe Tyr Gln Ile Pro Val Ala Asp Asp Gln Ser Ala Thr Asp
145 150 155 160

Ile Gly Asn Leu Trp Ser Tyr Gln Asp Ser Asn
165 170

<210> 151
<211> 1686
<212> DNA
<213> Oryza sativa

<400> 151
tctctccaat attttgtcga accgattgag ggaaaaaaaa agagaaaaag aaaagaaaaa 60

aaagaagaag aagagagaac tcgaactctc gcctaccatt tcgccccccct ccgcaagcaa 120

tccaccactg catcggcggc gagggctcac cggcggcgag ccatgtgcgg cggcgccatc 180

atccaccacc tgaaggggca cccggagggg tcgcgccggg cgacggaggg gctcctgtgg 240

cccgagaaga agaagcccag gtggggcggc ggcgggaggc gccacttcgg ggggttcgtg 300

gaggaggacg acgaggactt cgaggccgac ttcgaggagt tcgaggtgga ctccggggac 360

tcggatttgg agctcgggga ggaggacgac gatgacgtcg tcgagatcaa gccggccgcc 420

ttcaagaggg ccctctccag agataacttg agcaccatta ccactgccgg atttgatggt 480

cctgctgcaa agtctgccaa aagaaagaga aagaaccaat tcaggggcat ccgccagcgc 540

ccttggggta agtgggctgc tgaaatcaga gatcctcgca agggtgttcg tgtctggctt 600

ggcactttca acagtgctga agaagctgca agagcttatg atgctgaagc acgcaggatt 660

cgtggcaaga aggccaaggt gaattttcca gaggctccaa caactgctca gaagcgtcgt 720

gctggctcca ccactgctaa agcacccaag tcaagtgtgg aacagaagcc tactgtcaaa 780

ccagcattca acaatcttgc caatgcaaat gcgtttgtct acccatctgc taacttcact 840

tcaaacaagc cgtttgttca gcctgataac atgccatttg ttcctgcaat gaactctgct 900

gctcctattg aggaccctat catcaactct gaccagggaa gcaactcatt tggctgctct 960

gactttggct gggagaatga taccaagaca ccagatatta catcaattgc tcccatttca 1020

accatagctg aagtcgatga atctgcattc attaagagca gtaccaaccc aatggtccct 1080

cctgttatgg agaacagtgc tgttgatctg cctgatttag aaccctacat gaggttcctt 1140

ctggatgatg gtgctggtga ctcaattgat agccttctca acctggatgg atcacaggat 1200

gttgtcagca acatggacct ctggagcttt gatgacatgc ccgttagcga tttctattga 1260

ggaattcgaa gtcttctagt cggagcatgt acacagggaa aaaaaaggaa taaatactat 1320

tggagattgg gaggcacctg catggcacct tgggggtagc atatcgttat gtttagctta 1380

gatgcaaaag gctgcatcct gaaactcttt ggtgattgga cctgttccct atccgctgtc 1440

tatgtatggc agccatctat gagactcaag aactgctttt ttttttccgt tctagttttc 1500

tgtcgttgct acctgtatat ggctatgaac atcgtgaatc catggccatt atgttttaat 1560

ctatgttgtt gactgctctt tctttcggtc tttgctgtgc tgcgctatgt tggtgataac 1620

tagctaccat attgattttc tgtacataat tcatttgttg aatccgaaat taaatagtgc 1680

attgac 1686

<210> 152
<211> 365
<212> PRT
<213> Oryza sativa

<400> 152

Met Cys Gly Gly Ala Ile Ile His His Leu Lys Gly His Pro Glu Gly
1 5 10 15

Ser Arg Arg Ala Thr Glu Gly Leu Leu Trp Pro Glu Lys Lys Lys Pro
20 25 30

Arg Trp Gly Gly Gly Gly Arg Arg His Phe Gly Gly Phe Val Glu Glu
35 40 45

Asp Asp Glu Asp Phe Glu Ala Asp Phe Glu Glu Phe Glu Val Asp Ser
50 55 60

Gly Asp Ser Asp Leu Glu Leu Gly Glu Glu Asp Asp Asp Asp Val Val
65 70 75 80

Glu Ile Lys Pro Ala Ala Phe Lys Arg Ala Leu Ser Arg Asp Asn Leu
85 90 95

Ser Thr Ile Thr Thr Ala Gly Phe Asp Gly Pro Ala Ala Lys Ser Ala
100 105 110

Lys Arg Lys Arg Lys Asn Gln Phe Arg Gly Ile Arg Gln Arg Pro Trp
115 120 125

Gly Lys Trp Ala Ala Glu Ile Arg Asp Pro Arg Lys Gly Val Arg Val
130 135 140

Trp Leu Gly Thr Phe Asn Ser Ala Glu Glu Ala Ala Arg Ala Tyr Asp
145 150 155 160

Ala Glu Ala Arg Arg Ile Arg Gly Lys Lys Ala Lys Val Asn Phe Pro
                165                 170                 175

Glu Ala Pro Thr Thr Ala Gln Lys Arg Arg Ala Gly Ser Thr Thr Ala
            180                 185                 190

Lys Ala Pro Lys Ser Ser Val Glu Gln Lys Pro Thr Val Lys Pro Ala
        195                 200                 205

Phe Asn Asn Leu Ala Asn Ala Asn Ala Phe Val Tyr Pro Ser Ala Asn
    210                 215                 220

Phe Thr Ser Asn Lys Pro Phe Val Gln Pro Asp Asn Met Pro Phe Val
225                 230                 235                 240

Pro Ala Met Asn Ser Ala Ala Pro Ile Glu Asp Pro Ile Ile Asn Ser
                245                 250                 255

Asp Gln Gly Ser Asn Ser Phe Gly Cys Ser Asp Phe Gly Trp Glu Asn
            260                 265                 270

Asp Thr Lys Thr Pro Asp Ile Thr Ser Ile Ala Pro Ile Ser Thr Ile
        275                 280                 285

Ala Glu Val Asp Glu Ser Ala Phe Ile Lys Ser Ser Thr Asn Pro Met
    290                 295                 300

Val Pro Pro Val Met Glu Asn Ser Ala Val Asp Leu Pro Asp Leu Glu
305                 310                 315                 320

Pro Tyr Met Arg Phe Leu Leu Asp Asp Gly Ala Gly Asp Ser Ile Asp
                325                 330                 335

Ser Leu Leu Asn Leu Asp Gly Ser Gln Asp Val Val Ser Asn Met Asp
            340                 345                 350

Leu Trp Ser Phe Asp Asp Met Pro Val Ser Asp Phe Tyr
        355                 360                 365

<210> 153
<211> 1613
<212> DNA
<213> Oryza sativa

<400> 153
ctgtagctta ttagcggcca tgtgcggcgg agcaatcatc tccgggttca tcccgccgtc 60

ggccgctgcg gcggcggcgg cggcggcggc ggcggccaag aagcagcagg gcaggagggt 120

cacggccgac gtgctgtggc cggggatgct gcggaagggg aaggcggggg cggcggagga 180

```
ggactttgag gccgacttcc gcgagttcga gcgtggcatg agcgacgacg aggcggaggg     240
gggcggcggc gaggaggagg aggaggagga ggacgacgtg gtcgtggagg tccccccgcc     300
ggcgacggcg aggttcgtcg tccgtgccgc ggccaaggcg gcgcccccaa ctgcagatgg     360
gatgttgact acaaagcttg tccaacatga tggacctact gctagatcgg caaagcgcaa     420
gaggaagaat cagtacaggg ggatccgcca gcgtccctgg ggcaaatggg cagctgaaat     480
ccgagacccc agcaagggtg tccgtgtttg gcttggaaca tataacactg ctgaggaggc     540
agctagggca tatgacgctg aagcccgcaa gatccgtggc aagaaagcca aggtcaactt     600
tcctgatgaa ccagctgttg ctcagaagct ctccctgaag caaaacgctg ccaagcaaga     660
gaaactagct ccacctctga agtcctgtgg cgatgatgct ttctttcagc taaacagttc     720
agacaatgat ttgtttgcaa tgcttgcaaa ggtgcctgca aagccggcag agcctgttga     780
tctcatgcct ccagtcaaac ctcttgcttc cactgagaca ttcgagatga acatgctctc     840
tgatacgagc agcaactcat ttggctcttc agactttggt tgggaggatg acaccctgac     900
cccagactac acttcagtct ttgttcctaa tgctgccatg ccagcatatg gtgaacctgc     960
ttacctgaca ggtggagcgc caaagagaat gaggaacaac tatggtatcg ccgtgcccca    1020
gggaaatggc atgcctaatc tcgcacaaaa catgcccacc ttcgatcccg agatgaagta    1080
tttgccatta ccttatgttg agagcagctc agatgaatca atggacaacc ttctgcaaaa    1140
tgatgctaca caagacgggg caagcaacga gggcatctgg agccttgatg agctgctcat    1200
ggcagctggt gcctactgag gagacgaaat gttctggtca gtgtggtctg tcactagcaa    1260
accatgtaag gcactccaca ctacctacta ttttgatttc ttgtagatac attttcatgt    1320
ttgaatgtgt atggccaaga tgaagagctg gtgatgtctg ctatgttttg tagagggatg    1380
ctaccaaagt aatgctagaa tattacaagc tgctatcagc tgtactctct atgacaatgt    1440
ttatactatg tttgctgtat ggtgtggtct atgatttgaa gtatgtcgga gactaattca    1500
aataatgccc ttgggccatg gtgctgtgcc ttggtaaatg gtgctttatt taagggttat    1560
attaggttgg tgcctcagta attgccgttt tctaccaaaa aaaaaaaaaa aaa           1613
```

<210> 154
<211> 396
<212> PRT
<213> Oryza sativa

<400> 154

```
Met Cys Gly Gly Ala Ile Ile Ser Gly Phe Ile Pro Pro Ser Ala Ala
1               5                   10                  15

Ala Ala Ala Ala Ala Ala Val Ala Lys Lys Gln Gln Gly Arg Arg Val
            20                  25                  30
```

Thr Ala Asp Val Leu Trp Pro Gly Met Leu Arg Lys Gly Lys Ala Ala
35 40 45

Ala Ala Glu Glu Asp Phe Glu Ala Asp Phe Arg Glu Phe Glu Arg Gly
50 55 60

Met Ser Asp Asp Glu Ala Glu Gly Gly Gly Gly Glu Glu Glu Glu Asp
65 70 75 80

Asp Asp Asp Val Val Val Val Val Pro Pro Pro Ala Ala Ala Arg Phe
85 90 95

Val Val Arg Ala Ala Ala Lys Ala Ala Pro Pro Thr Ala Asp Gly Met
100 105 110

Leu Thr Thr Lys Leu Val Gln His Asp Gly Pro Thr Ala Arg Ser Ala
115 120 125

Lys His Lys Arg Lys Asn Gln Tyr Arg Gly Ile Arg Gln Arg Pro Trp
130 135 140

Gly Lys Trp Ala Ala Glu Ile Arg Asp Pro Ser Lys Gly Val Arg Val
145 150 155 160

Trp Leu Gly Thr Tyr Asn Thr Ala Glu Glu Ala Ala Arg Ala Tyr Asp
165 170 175

Ala Glu Ala Arg Lys Ile Arg Gly Lys Lys Ala Lys Val Asn Phe Pro
180 185 190

Asp Glu Pro Ala Val Ala Gln Lys Leu Ser Leu Lys Gln Asn Ala Ala
195 200 205

Lys Gln Glu Lys Leu Ala Pro Pro Leu Lys Thr Cys Gly Asp Asp Ala
210 215 220

Phe Phe Gln Leu Asn Ser Ser Asp Asn Asp Leu Phe Ala Met Leu Ala
225 230 235 240

Lys Val Pro Ala Lys Pro Ala Glu Pro Val Asp Leu Met Pro Pro Val
245 250 255

Lys Pro Leu Ala Ser Thr Glu Thr Phe Glu Met Asn Met Leu Ser Asp
260 265 270

Thr Ser Ser Asn Ser Phe Gly Ser Ser Asp Phe Gly Trp Glu Asp Asp
275 280 285

Thr Leu Thr Pro Asp Tyr Thr Ser Val Phe Val Pro Asn Ala Ala Met

290 295 300

Pro Ala Tyr Gly Glu Pro Ala Tyr Leu Thr Gly Gly Ala Pro Lys Arg
305 310 315 320

Met Arg Asn Asn Tyr Gly Ile Ala Val Pro Gln Gly Asn Gly Met Pro
325 330 335

Asn Leu Ala Gln Asn Met Pro Thr Phe Asp Pro Glu Met Lys Tyr Leu
340 345 350

Pro Leu Pro Tyr Val Glu Ser Ser Ser Asp Glu Ser Met Asp Asn Leu
355 360 365

Leu Gln Asn Asp Ala Thr Gln Asp Gly Ala Ser Asn Glu Gly Ile Trp
370 375 380

Ser Leu Asp Glu Leu Leu Met Ala Ala Gly Ala Tyr
385 390 395

<210> 155
<211> 1068
<212> DNA
<213> Triticum aestivum

<400> 155
atgtgcggcg gcgccatcct ctccgacatc atcccgccgc cgcgccgggc caccggcggc 60

aacgtctggc gggcggacaa gaagaggagg gccaggcccg acgccgccgc ggggaggccc 120

cgccgcgtgc ccgaggagga gttccaggag gaggagagcg acgcggagtt cgaggccgac 180

ttcgaggggt tcgtggaggc ggaggaggag tccgacggcg aggccaagcc cttccccgtc 240

cgcaggagcg gcttctccgg agatggattg aaggcaactg ctgctggtga tgatgactgt 300

gcttcagggt ctgctaaaag gaagagaaag aaccagttca ggggcatccg ccgccgccct 360

tggggtaaat gggctgctga aataagagat cctcgcaagg gtgtccgtgc ctggcttggc 420

acttacaact ctgctgagga agctgtcaga gcctatgatg ttgaagcccg cagaattcgt 480

ggcaagaagg caaagtcaat ttcccagaag aagctcccat ggctcctcag caaacgctgc 540

gctacctctg tgaaggtgcc cgagttcaac accgaacaga agccagtact caacaccatg 600

ggcaacgcag atgtgtattc ctgccctgct gttgactaca ccttaaatca gcaatttgtg 660

cagcctcaga acatgtcgtt tgtgcctaca gtgaatgcag ttgaggctcc tttcatgaat 720

ttttcctctg accaggggag caactccttt agttgctcag acttcagctg ggagaatgat 780

atcaagaccc ctgacataac ttctgtgctt gcatccattc ccacctcaac tgaggtcaat 840

gaatctgcat ttctccagaa caatggcatt aattcaacgg tacctcctgt gatgggtgat 900

gctaatgttg atcttgccta cttggagccg tacatgaagt tcctgatgga cgatggttca 960

```
gatgagtcaa ttgacagcat tctaagctgt gatgtaccgc aggacgttgt cggcaacatg   1020

ggcctttgga cctttgatga catgcccttg tctgctggtt tctactga                 1068


<210>  156
<211>  355
<212>  PRT
<213>  Triticum aestivum

<400>  156

Met Cys Gly Gly Ala Ile Leu Ser Asp Ile Ile Pro Pro Pro Arg Arg
1               5                   10                  15

Ala Thr Gly Gly Asn Val Trp Arg Ala Asp Lys Lys Arg Arg Ala Arg
            20                  25                  30

Pro Asp Ala Ala Ala Gly Arg Pro Arg Arg Val Pro Glu Glu Glu Phe
        35                  40                  45

Gln Glu Glu Glu Ser Asp Ala Glu Phe Glu Ala Asp Phe Glu Gly Phe
    50                  55                  60

Val Glu Ala Glu Glu Glu Ser Asp Gly Glu Ala Lys Pro Phe Pro Val
65                  70                  75                  80

Arg Arg Ser Gly Phe Ser Gly Asp Gly Leu Lys Ala Thr Ala Ala Gly
                85                  90                  95

Asp Asp Asp Cys Ala Ser Gly Ser Ala Lys Arg Lys Arg Lys Asn Gln
            100                 105                 110

Phe Arg Gly Ile Arg Arg Arg Pro Trp Gly Lys Trp Ala Ala Glu Ile
        115                 120                 125

Arg Asp Pro Arg Lys Gly Val Arg Ala Trp Leu Gly Thr Tyr Asn Ser
    130                 135                 140

Ala Glu Glu Ala Val Arg Ala Tyr Asp Val Glu Ala Arg Arg Ile Arg
145                 150                 155                 160

Gly Lys Lys Ala Lys Ser Ile Ser Gln Lys Lys Leu Pro Trp Leu Leu
                165                 170                 175

Ser Lys Arg Cys Ala Thr Ser Val Lys Val Pro Glu Phe Asn Thr Glu
            180                 185                 190

Gln Lys Pro Val Leu Asn Thr Met Gly Asn Ala Asp Val Tyr Ser Cys
        195                 200                 205

Pro Ala Val Asp Tyr Thr Leu Asn Gln Gln Phe Val Gln Pro Gln Asn
```

210 215 220

Met Ser Phe Val Pro Thr Val Asn Ala Val Glu Ala Pro Phe Met Asn
225 230 235 240

Phe Ser Ser Asp Gln Gly Ser Asn Ser Phe Ser Cys Ser Asp Phe Ser
245 250 255

Trp Glu Asn Asp Ile Lys Thr Pro Asp Ile Thr Ser Val Leu Ala Ser
260 265 270

Ile Pro Thr Ser Thr Glu Val Asn Glu Ser Ala Phe Leu Gln Asn Asn
275 280 285

Gly Ile Asn Ser Thr Val Pro Pro Val Met Gly Asp Ala Asn Val Asp
290 295 300

Leu Ala Tyr Leu Glu Pro Tyr Met Lys Phe Leu Met Asp Asp Gly Ser
305 310 315 320

Asp Glu Ser Ile Asp Ser Ile Leu Ser Cys Asp Val Pro Gln Asp Val
325 330 335

Val Gly Asn Met Gly Leu Trp Thr Phe Asp Asp Met Pro Leu Ser Ala
340 345 350

Gly Phe Tyr
355

<210> 157
<211> 999
<212> DNA
<213> Triticum aestivum

<400> 157
atgtgcggcg ggcgccatcc tctccgacat catcccgccg cggggaggcc ctgccgtgcg 60

cctgaggagg agttccagga ggaggagggc gacgcggagt tcgaggccga cttcgagggg 120

ttcgtggagg cggaggagga gtccgacggc gaggccaagc ccttccccgt ccgcaggagc 180

ggcttctccg gagatggatt gaaggcaact gctgctggtg atgatgactg tgcctcaggg 240

tctgctaaaa ggaagagaaa gaaccagttc aggggcgtcc gccgccgccc ttggggtaaa 300

tgggctgctg aaataagaga tcctcgcaag ggtgtccgtg tctggcttgg tacttacaac 360

tccgctgagg aagctgccag agcctatggt gttgaggccc gcagaattcg tggcaagaag 420

gcaaaggtca atttcccaga agaagctcct atggctcctc agcaacgctg cgctactgct 480

gtgaaggtgc ccgagttcaa caccgaacag aagccggtac tcaacaccat gggcaacgca 540

gatgtgtatt cctgctctgc tgttgactac accttaaatc agcaatttgt gcagcctcag 600

aacatgtcgt ttgtgcctac agtgaatgca gttgaggccc ctttcatgaa tttttcctct 660

gaccagggta gcaactcctt tagttgctca gacctcagct gggagaatga tatcaagacc 720

cctgacataa cttctgtgct tgcatccatt cccacctcaa cagaggtcaa tgaatctgca 780

tttctccaga acaatggcat caattcaacg gtacctcctg tgatgggtga tgctaatgtt 840

gatcttgccg acttggagcc atacatgaag ttcctgatgg acgatggttc agatgagtca 900

attgacagca ttctaagctg tgatgtaccc caggatgtgg tcggcaacat gggcctttgg 960

acctttgatg acatgccctt gtctgctggt ttctactga 999

<210> 158
<211> 332
<212> PRT
<213> Triticum aestivum

<400> 158

Met Cys Gly Gly Arg His Pro Leu Arg His His Pro Ala Ala Gly Arg
1 5 10 15

Pro Cys Arg Ala Pro Glu Glu Glu Phe Gln Glu Glu Glu Gly Asp Ala
20 25 30

Glu Phe Glu Ala Asp Phe Glu Gly Phe Val Glu Ala Glu Glu Glu Ser
35 40 45

Asp Gly Glu Ala Lys Pro Phe Pro Val Arg Arg Ser Gly Phe Ser Gly
50 55 60

Asp Gly Leu Lys Ala Thr Ala Ala Gly Asp Asp Asp Cys Ala Ser Gly
65 70 75 80

Ser Ala Lys Arg Lys Arg Lys Asn Gln Phe Arg Gly Val Arg Arg Arg
85 90 95

Pro Trp Gly Lys Trp Ala Ala Glu Ile Arg Asp Pro Arg Lys Gly Val
100 105 110

Arg Val Trp Leu Gly Thr Tyr Asn Ser Ala Glu Glu Ala Ala Arg Ala
115 120 125

Tyr Gly Val Glu Ala Arg Arg Ile Arg Gly Lys Lys Ala Lys Val Asn
130 135 140

Phe Pro Glu Glu Ala Pro Met Ala Pro Gln Gln Arg Cys Ala Thr Ala
145 150 155 160

Val Lys Val Pro Glu Phe Asn Thr Glu Gln Lys Pro Val Leu Asn Thr
165 170 175

Met Gly Asn Ala Asp Val Tyr Ser Cys Ser Ala Val Asp Tyr Thr Leu
180 185 190

Asn Gln Gln Phe Val Gln Pro Gln Asn Met Ser Phe Val Pro Thr Val
195 200 205

Asn Ala Val Glu Ala Pro Phe Met Asn Phe Ser Ser Asp Gln Gly Ser
210 215 220

Asn Ser Phe Ser Cys Ser Asp Leu Ser Trp Glu Asn Asp Ile Lys Thr
225 230 235 240

Pro Asp Ile Thr Ser Val Leu Ala Ser Ile Pro Thr Ser Thr Glu Val
245 250 255

Asn Glu Ser Ala Phe Leu Gln Asn Asn Gly Ile Asn Ser Thr Val Pro
260 265 270

Pro Val Met Gly Asp Ala Asn Val Asp Leu Ala Asp Leu Glu Pro Tyr
275 280 285

Met Lys Phe Leu Met Asp Asp Gly Ser Asp Glu Ser Ile Asp Ser Ile
290 295 300

Leu Ser Cys Asp Val Pro Gln Asp Val Val Gly Asn Met Gly Leu Trp
305 310 315 320

Thr Phe Asp Asp Met Pro Leu Ser Ala Gly Phe Tyr
325 330

<210> 159
<211> 1452
<212> DNA
<213> Arabidopsis thaliana

<400> 159
aaaacaacaa agcaaagcgt tgaagagaga agaagaagca aagatataac ccccaaaagt 60

atcaattagt ttccattttc gccgctaaga ttctgttttc gaacatttac accctcaaga 120

atcgccgcca tgtgtggagg agctataata tccgatttca ttccaccgcc gaggtctcgc 180

cgtgttacta gcgagtttat ttggccggat ctgaagaaga atttgaaagg atcgaagaaa 240

agctcgaaga atcgttcgaa tttcttcgat tttgacgctg agttcgaagc tgatttccaa 300

ggtttcaaag atgattcgtc tatcgattgc gatgatgatt tcgacgtcgg tgatgttttc 360

gccgatgtga aaccattcgt tttcacttcg actccaaaac ccgccgtctc cgccgctgcg 420

gaaggttcag tttttggtaa gaaagttact ggcttggatg gggacgctga gaaatctgca 480

aataggaaga ggaagaatca gtaccgaggg attaggcaac gtccttgggg aaaatgggct 540

gctgagatac gtgatccaag ggaaggtgct agaatctggc ttggaacgtt caagacagct 600

gaggaagctg ctagagctta cgatgctgca gcgcggagaa tccgtggatc taaagctaag 660

gtgaatttcc ctgaagaaaa catgaaggct aattctcaga aacgctctgt gaaggctaat 720

cttcagaaac cagtggctaa acctaaccct aacccaagtc cagctttggt tcagaactcg 780

aacatctcct ttgaaaatat gtgtttcatg gaggagaaac accaagtgag caacaacaac 840

aacaaccagt ttgggatgac aaactccgtt gatgctggat gtaatgggta tcagtatttc 900

agctctgacc agggtagtaa ttctttcgat tgttcggagt ttggttggag cgatcaagct 960

ccgataactc ccgacatctc ttctgcggtt atcaacaaca acaactcagc tctgttcttt 1020

gaggaagcca atccagctaa gaagctcaag tctatggatt tcgagacacc ttacaacaac 1080

actgaatggg acgcttcact ggatttcctc aacgaagatg ctgtaacgac tcaggacaat 1140

ggtgcaaacc ctatggacct atggagtatt gatgaaattc attccatgat tggaggagtc 1200

ttctgaagag atccagtttc atgagttcct gtttgcattg cgagaagcca tgagcctcta 1260

tcttgagggt agttgtgatg aagttaagta gaggcttatt tttaggggtt gtggtagttt 1320

ttgttttagt gaatcttttg aattcgtttg tgttttgttt ttgttacttt atgccccaaa 1380

actcctttaa catttgtcat aatgtgtttg aacctctcat ctgtttaatc aaataaatct 1440

tctttgtatg ct 1452

<210> 160
<211> 358
<212> PRT
<213> Arabidopsis thaliana

<400> 160

Met Cys Gly Gly Ala Ile Ile Ser Asp Phe Ile Pro Pro Pro Arg Ser
1 5 10 15

Arg Arg Val Thr Ser Glu Phe Ile Trp Pro Asp Leu Lys Lys Asn Leu
20 25 30

Lys Gly Ser Lys Lys Ser Ser Lys Asn Arg Ser Asn Phe Phe Asp Phe
35 40 45

Asp Ala Glu Phe Glu Ala Asp Phe Gln Gly Phe Lys Asp Asp Ser Ser
50 55 60

Ile Asp Cys Asp Asp Asp Phe Asp Val Gly Asp Val Phe Ala Asp Val
65 70 75 80

Lys Pro Phe Val Phe Thr Ser Thr Pro Lys Pro Ala Val Ser Ala Ala
85 90 95

Ala Glu Gly Ser Val Phe Gly Lys Lys Val Thr Gly Leu Asp Gly Asp

```
            100                 105                 110

Ala Glu Lys Ser Ala Asn Arg Lys Arg Lys Asn Gln Tyr Arg Gly Ile
        115                 120                 125

Arg Gln Arg Pro Trp Gly Lys Trp Ala Ala Glu Ile Arg Asp Pro Arg
    130                 135                 140

Glu Gly Ala Arg Ile Trp Leu Gly Thr Phe Lys Thr Ala Glu Glu Ala
145                 150                 155                 160

Ala Arg Ala Tyr Asp Ala Ala Ala Arg Arg Ile Arg Gly Ser Lys Ala
                165                 170                 175

Lys Val Asn Phe Pro Glu Glu Asn Met Lys Ala Asn Ser Gln Lys Arg
            180                 185                 190

Ser Val Lys Ala Asn Leu Gln Lys Pro Val Ala Lys Pro Asn Pro Asn
        195                 200                 205

Pro Ser Pro Ala Leu Val Gln Asn Ser Asn Ile Ser Phe Glu Asn Met
    210                 215                 220

Cys Phe Met Glu Glu Lys His Gln Val Ser Asn Asn Asn Asn Asn Gln
225                 230                 235                 240

Phe Gly Met Thr Asn Ser Val Asp Ala Gly Cys Asn Gly Tyr Gln Tyr
                245                 250                 255

Phe Ser Ser Asp Gln Gly Ser Asn Ser Phe Asp Cys Ser Glu Phe Gly
            260                 265                 270

Trp Ser Asp Gln Ala Pro Ile Thr Pro Asp Ile Ser Ser Ala Val Ile
        275                 280                 285

Asn Asn Asn Asn Ser Ala Leu Phe Phe Glu Glu Ala Asn Pro Ala Lys
    290                 295                 300

Lys Leu Lys Ser Met Asp Phe Glu Thr Pro Tyr Asn Asn Thr Glu Trp
305                 310                 315                 320

Asp Ala Ser Leu Asp Phe Leu Asn Glu Asp Ala Val Thr Thr Gln Asp
                325                 330                 335

Asn Gly Ala Asn Pro Met Asp Leu Trp Ser Ile Asp Glu Ile His Ser
            340                 345                 350

Met Ile Gly Gly Val Phe
        355
```

<210> 161
<211> 1568
<212> DNA
<213> Arabidopsis thaliana

<400> 161
aaaaacaaca aagcaaagcg ttgaagagag aagaagaagc aaagatataa cccccaaaag 60

tatcaattag tttccatttt cgccgctaag attctgtttt cgaacattta caccctcaag 120

aatcgccgcc atgtgtggag gagctataat atccgatttc attccaccgc cgaggtctcg 180

ccgtgttact agcgagttta tttggccgga tctgaagaag aatttgaaag gatcgaagaa 240

aagctcgaag aatcgttcga atttcttcga ttttgacgct gagttcgaag ctgatttcca 300

aggtttcaaa gatgattcgt ctatcgattg cgatgatgat ttcgacgtcg gtgatgtttt 360

cgccgatgtg aaaccattcg ttttcacttc gactccaaaa cccgccgtct ccgccgctgc 420

ggaaggttca gtttttggta agaaagttac tggcttggat ggggaagctg agaaatctgc 480

aaataggaag aggaagaatc agtaccgagg gattaggcaa cgtccttggg gaaaatgggc 540

tgctgagata cgtgatccaa gggaaggtgc tagaatctgg cttggaacgt tcaagacagc 600

tgaggaagct gctagagctt acgatgctgc agcgcggaga atccgtggat ctaaagctaa 660

ggtgaatttc cctgaagaaa acctgaaggc taattctcag aaacgctctg tgaaggctaa 720

tcttcagaaa ccagtggcta aacctaaccc taacccaagt ccagctttgg ttcagaactc 780

gaacatctcc tttgaaaata tgtgtttcat ggaggagaaa caccaagtga gcaacaacaa 840

caacaaccag tttgggatga caaactccgt tgatgctgga tgtaatgggt atcagtattt 900

cagctctgac cagggtagta attctttcga ttgttcggag tttggttgga gcgatcaagc 960

tccgataact cccgacatct cttctgcggt tatcaacaac aacaactcag ctctgttctt 1020

tgaggaagcc aatccagcta agaagctcaa gtctatggat ttcgagacac cttacaacaa 1080

cactgaatgg gacgcttcac tggatttcct caacgaagat gctgtaacga ctcaggacaa 1140

tggtgcaaac cctatggacc tatggagtat tgatgaaatt cattccatga ttggaggagt 1200

cttctgaaga gatccagttt catgtaaata aggctgcatg tttgtgagtt tcccgcatcg 1260

ttcgtttatc aacctccaaa actttctaat gtctgttact tgcatcttct tctgctgtct 1320

ctgtctgtct ctctcaggag ttcctgtttg cattgcgaga agccatgagc ctctatcttg 1380

agggtagttg tgatgaagtt aagtagaggc ttattttag gggttgtggt agtttttgtt 1440

ttagtgaatc ttttgaattc gtttgtgttt tgtttttgtt actttatgcc ccaaaactcc 1500

tttaacattt gtcataatgt gtttgaacct cctcatctgt ttaatcaaat aaatcttctt 1560

tgtatgct 1568

<210> 162
<211> 358

<212> PRT
<213> Arabidopsis thaliana

<400> 162

Met Cys Gly Gly Ala Ile Ile Ser Asp Phe Ile Pro Pro Pro Arg Ser
1 5 10 15

Arg Arg Val Thr Ser Glu Phe Ile Trp Pro Asp Leu Lys Lys Asn Leu
20 25 30

Lys Gly Ser Lys Lys Ser Ser Lys Asn Arg Ser Asn Phe Phe Asp Phe
35 40 45

Asp Ala Glu Phe Glu Ala Asp Phe Gln Gly Phe Lys Asp Asp Ser Ser
50 55 60

Ile Asp Cys Asp Asp Asp Phe Asp Val Gly Asp Val Phe Ala Asp Val
65 70 75 80

Lys Pro Phe Val Phe Thr Ser Thr Pro Lys Pro Ala Val Ser Ala Ala
85 90 95

Ala Glu Gly Ser Val Phe Gly Lys Lys Val Thr Gly Leu Asp Gly Glu
100 105 110

Ala Glu Lys Ser Ala Asn Arg Lys Arg Lys Asn Gln Tyr Arg Gly Ile
115 120 125

Arg Gln Arg Pro Trp Gly Lys Trp Ala Ala Glu Ile Arg Asp Pro Arg
130 135 140

Glu Gly Ala Arg Ile Trp Leu Gly Thr Phe Lys Thr Ala Glu Glu Ala
145 150 155 160

Ala Arg Ala Tyr Asp Ala Ala Ala Arg Arg Ile Arg Gly Ser Lys Ala
165 170 175

Lys Val Asn Phe Pro Glu Glu Asn Leu Lys Ala Asn Ser Gln Lys Arg
180 185 190

Ser Val Lys Ala Asn Leu Gln Lys Pro Val Ala Lys Pro Asn Pro Asn
195 200 205

Pro Ser Pro Ala Leu Val Gln Asn Ser Asn Ile Ser Phe Glu Asn Met
210 215 220

Cys Phe Met Glu Glu Lys His Gln Val Ser Asn Asn Asn Asn Asn Gln
225 230 235 240

Phe Gly Met Thr Asn Ser Val Asp Ala Gly Cys Asn Gly Tyr Gln Tyr
245 250 255

Phe Ser Ser Asp Gln Gly Ser Asn Ser Phe Asp Cys Ser Glu Phe Gly
260 265 270

Trp Ser Asp Gln Ala Pro Ile Thr Pro Asp Ile Ser Ser Ala Val Ile
275 280 285

Asn Asn Asn Asn Ser Ala Leu Phe Phe Glu Glu Ala Asn Pro Ala Lys
290 295 300

Lys Leu Lys Ser Met Asp Phe Glu Thr Pro Tyr Asn Asn Thr Glu Trp
305 310 315 320

Asp Ala Ser Leu Asp Phe Leu Asn Glu Asp Ala Val Thr Thr Gln Asp
325 330 335

Asn Gly Ala Asn Pro Met Asp Leu Trp Ser Ile Asp Glu Ile His Ser
340 345 350

Met Ile Gly Gly Val Phe
355

<210> 163
<211> 1644
<212> DNA
<213> Arabidopsis thaliana

<400> 163
ggagagtaac atcgagacaa agaagaaaaa ctaaaaaaga gaaccccaaa gaatcgaata 60

tttattattt cgcccccgaag attctatttc tgatcattta cacccctaaa aagagtagag 120

ctttcgtgaa gccaccatgt gtggaggagc tataatctcc gatttcatac ctccgccgag 180

gtccctccgc gtcactaacg agtttatctg gccggatctg aaaaacaaag tgaaagcttc 240

aaagaagaga tcgaataagc gatccgattt cttcgatctt gacgatgatt tcgaagctga 300

tttccaaggg tttaaggatg actcggcttt tgactgcgaa gacgatgatg atgtcttcgt 360

caatgttaag cctttcgtct tcaccgcaac tactaagccc gtagcttccg ctttcgtctc 420

cactggtata tatttggtag gttcagcata tgccaagaaa actgtagagt ccgctgagca 480

agctgagaaa tcttctaaga ggaagaggaa gaatcaatac cgagggatta ggcagcgtcc 540

ttggggaaaa tgggctgcgg agatccgtga tccgagaaaa ggctcccgag aatggcttgg 600

aacattcgac actgctgagg aagcagcaag agcttatgat gctgcagcac gcagaatccg 660

tggcacgaaa gctaaggtga attttcccga ggagaagaac cctagcgtcg tatcccagaa 720

acgtcctagt gctaagacta ataatcttca gaaatcagtg gctaaaccaa acaaaagcgt 780

aactttggtt cagcagccaa cacatctgag tcagcagtac tgcaacaact cctttgacaa 840

ctcttttggt gatatgagtt tcatggaaga gaagcctcag atgtacaaca atcagtttgg 900

gttaacaaac tcgttcgatg ctggaggtaa caatggatac cagtatttca gttccgatca 960

gggcagtaac tccttcgact gttctgagtt cgggtggagt gatcacggcc ctaaaacacc 1020

cgagatctct tcaatgcttg tcaataacaa cgaagcatca tttgttgaag aaaccaatgc 1080

agccaagaag ctcaaaccaa actctgatga gtcagacgat ctgatggcat accttgacaa 1140

cgccttgtgg gacaccccac tagaagtgga agccatgctt ggcgcagatg ctggtgctgt 1200

gactcaggaa gaggaaaacc cagtggagct atggagctta gatgagatca atttcatgct 1260

ggaaggagac ttttgaagtg atcgatggtt ccttagtttg taaataaagc tgtgttggat 1320

tttgctgttg gggatggta caagtcacac ctcaagctct atgcattggt atctcatgag 1380

cctctcttcc atagagagtt tctcttttaa ttttgtcgaa ataaaaaagg tgtgatgaag 1440

taaatagagg tataataata tctatctatt aagtcttgtt ttgttctttc atttttgtat 1500

ttcttttcta tttaaaagac agtttattag tcttctgagc tctcttttg atctttgtta 1560

tagcgtatca tcaccctcga aagtgtaatg ttttgtaccc ccaaacttgt ttagcattat 1620

aataaagtct ctttggaact tctc 1644

<210> 164
<211> 379
<212> PRT
<213> Arabidopsis thaliana

<400> 164

Met Cys Gly Gly Ala Ile Ile Ser Asp Phe Ile Pro Pro Pro Arg Ser
1 5 10 15

Leu Arg Val Thr Asn Glu Phe Ile Trp Pro Asp Leu Lys Asn Lys Val
20 25 30

Lys Ala Ser Lys Lys Arg Ser Asn Lys Arg Ser Asp Phe Phe Asp Leu
35 40 45

Asp Asp Asp Phe Glu Ala Asp Phe Gln Gly Phe Lys Asp Asp Ser Ala
50 55 60

Phe Asp Cys Glu Asp Asp Asp Asp Val Phe Val Asn Val Lys Pro Phe
65 70 75 80

Val Phe Thr Ala Thr Thr Lys Pro Val Ala Ser Ala Phe Val Ser Thr
85 90 95

Gly Ile Tyr Leu Val Gly Ser Ala Tyr Ala Lys Lys Thr Val Glu Ser
100 105 110

Ala Glu Gln Ala Glu Lys Ser Ser Lys Arg Lys Arg Lys Asn Gln Tyr
115 120 125

Arg Gly Ile Arg Gln Arg Pro Trp Gly Lys Trp Ala Ala Glu Ile Arg
130 135 140

Asp Pro Arg Lys Gly Ser Arg Glu Trp Leu Gly Thr Phe Asp Thr Ala
145 150 155 160

Glu Glu Ala Ala Arg Ala Tyr Asp Ala Ala Ala Arg Arg Ile Arg Gly
165 170 175

Thr Lys Ala Lys Val Asn Phe Pro Glu Glu Lys Asn Pro Ser Val Val
180 185 190

Ser Gln Lys Arg Pro Ser Ala Lys Thr Asn Asn Leu Gln Lys Ser Val
195 200 205

Ala Lys Pro Asn Lys Ser Val Thr Leu Val Gln Gln Pro Thr His Leu
210 215 220

Ser Gln Gln Tyr Cys Asn Asn Ser Phe Asp Asn Ser Phe Gly Asp Met
225 230 235 240

Ser Phe Met Glu Glu Lys Pro Gln Met Tyr Asn Asn Gln Phe Gly Leu
245 250 255

Thr Asn Ser Phe Asp Ala Gly Gly Asn Asn Gly Tyr Gln Tyr Phe Ser
260 265 270

Ser Asp Gln Gly Ser Asn Ser Phe Asp Cys Ser Glu Phe Gly Trp Ser
275 280 285

Asp His Gly Pro Lys Thr Pro Glu Ile Ser Ser Met Leu Val Asn Asn
290 295 300

Asn Glu Ala Ser Phe Val Glu Glu Thr Asn Ala Ala Lys Lys Leu Lys
305 310 315 320

Pro Asn Ser Asp Glu Ser Asp Asp Leu Met Ala Tyr Leu Asp Asn Ala
325 330 335

Leu Trp Asp Thr Pro Leu Glu Val Glu Ala Met Leu Gly Ala Asp Ala
340 345 350

Gly Ala Val Thr Gln Glu Glu Glu Asn Pro Val Glu Leu Trp Ser Leu
355 360 365

Asp Glu Ile Asn Phe Met Leu Glu Gly Asp Phe

370 375

<210> 165
<211> 1632
<212> DNA
<213> Arabidopsis thaliana

<400> 165
ggagagtaac atcgagacaa agaagaaaaa ctaaaaaaga gaaccccaaa gaatcgaata 60

tttattattt cgccccgaag attctatttc tgatcattta cacccctaaa aagagtagag 120

ctttcgtgaa gccaccatgt gtggaggagc tataatctcc gatttcatac ctccgccgag 180

gtccctccgc gtcactaacg agtttatctg gccggatctg aaaaacaaag tgaaagcttc 240

aaagaagaga tcgaataagc gatccgattt cttcgatctt gacgatgatt tcgaagctga 300

tttccaaggg tttaaggatg actcggcttt tgactgcgaa gacgatgatg atgtcttcgt 360

caatgttaag cctttcgtct tcaccgcaac tactaagccc gtagcttccg ctttcgtctc 420

cactgtaggt tcagcatatg ccaagaaaac tgtagagtcc gctgagcaag ctgagaaatc 480

ttctaagagg aagaggaaga atcaataccg agggattagg cagcgtcctt ggggaaaatg 540

ggctgcggag atccgtgatc cgagaaaagg ctcccgagaa tggcttggaa cattcgacac 600

tgctgaggaa gcagcaagag cttatgatgc tgcagcacgc agaatccgtg gcacgaaagc 660

taaggtgaat tttcccgagg agaagaaccc tagcgtcgta tcccagaaac gtcctagtgc 720

taagactaat aatcttcaga aatcagtggc taaaccaaac aaaagcgtaa ctttggttca 780

gcagccaaca catctgagtc agcagtactg caacaactcc tttgacaact cttttggtga 840

tatgagtttc atggaagaga agcctcagat gtacaacaat cagtttgggt taacaaactc 900

gttcgatgct ggaggtaaca atggatacca gtatttcagt tccgatcagg gcagtaactc 960

cttcgactgt tctgagttcg ggtggagtga tcacggccct aaaacacccg agatctcttc 1020

aatgcttgtc aataacaacg aagcatcatt tgttgaagaa accaatgcag ccaagaagct 1080

caaaccaaac tctgatgagt cagacgatct gatggcatac cttgacaacg ccttgtggga 1140

caccccacta gaagtggaag ccatgcttgg cgcagatgct ggtgctgtga ctcaggaaga 1200

ggaaaaccca gtggagctat ggagcttaga tgagatcaat ttcatgctgg aaggagactt 1260

ttgaagtgat cgatggttcc ttagtttgta aataaagctg tgttggattt tgctgttggg 1320

ggatggtaca agtcacacct caagctctat gcattggtat ctcatgagcc tctcttccat 1380

agagagtttc tcttttaatt ttgtcgaaat aaaaaaggtg tgatgaagta aatagaggta 1440

taataatatc tatctattaa gtcttgtttt gttctttcat ttttgtattt cttttctatt 1500

taaaagacag tttattagtc ttctgagctc tctttttgat ctttgttata gcgtatcatc 1560

accctcgaaa gtgtaatgtt ttgtaccccc aaacttgttt agcattataa taaagtctct 1620

ttggaacttc tc 1632

<210> 166
<211> 375
<212> PRT
<213> Arabidopsis thaliana

<400> 166

Met Cys Gly Gly Ala Ile Ile Ser Asp Phe Ile Pro Pro Pro Arg Ser
1 5 10 15

Leu Arg Val Thr Asn Glu Phe Ile Trp Pro Asp Leu Lys Asn Lys Val
20 25 30

Lys Ala Ser Lys Lys Arg Ser Asn Lys Arg Ser Asp Phe Phe Asp Leu
35 40 45

Asp Asp Asp Phe Glu Ala Asp Phe Gln Gly Phe Lys Asp Asp Ser Ala
50 55 60

Phe Asp Cys Glu Asp Asp Asp Asp Val Phe Val Asn Val Lys Pro Phe
65 70 75 80

Val Phe Thr Ala Thr Thr Lys Pro Val Ala Ser Ala Phe Val Ser Thr
85 90 95

Val Gly Ser Ala Tyr Ala Lys Lys Thr Val Glu Ser Ala Glu Gln Ala
100 105 110

Glu Lys Ser Ser Lys Arg Lys Arg Lys Asn Gln Tyr Arg Gly Ile Arg
115 120 125

Gln Arg Pro Trp Gly Lys Trp Ala Ala Glu Ile Arg Asp Pro Arg Lys
130 135 140

Gly Ser Arg Glu Trp Leu Gly Thr Phe Asp Thr Ala Glu Glu Ala Ala
145 150 155 160

Arg Ala Tyr Asp Ala Ala Ala Arg Arg Ile Arg Gly Thr Lys Ala Lys
165 170 175

Val Asn Phe Pro Glu Glu Lys Asn Pro Ser Val Val Ser Gln Lys Arg
180 185 190

Pro Ser Ala Lys Thr Asn Asn Leu Gln Lys Ser Val Ala Lys Pro Asn
195 200 205

Lys Ser Val Thr Leu Val Gln Gln Pro Thr His Leu Ser Gln Gln Tyr
210 215 220

Cys Asn Asn Ser Phe Asp Asn Ser Phe Gly Asp Met Ser Phe Met Glu

```
225                 230                 235                 240

Glu Lys Pro Gln Met Tyr Asn Asn Gln Phe Gly Leu Thr Asn Ser Phe
                245                 250                 255

Asp Ala Gly Gly Asn Asn Gly Tyr Gln Tyr Phe Ser Ser Asp Gln Gly
            260                 265                 270

Ser Asn Ser Phe Asp Cys Ser Glu Phe Gly Trp Ser Asp His Gly Pro
        275                 280                 285

Lys Thr Pro Glu Ile Ser Ser Met Leu Val Asn Asn Asn Glu Ala Ser
    290                 295                 300

Phe Val Glu Glu Thr Asn Ala Ala Lys Lys Leu Lys Pro Asn Ser Asp
305                 310                 315                 320

Glu Ser Asp Asp Leu Met Ala Tyr Leu Asp Asn Ala Leu Trp Asp Thr
                325                 330                 335

Pro Leu Glu Val Glu Ala Met Leu Gly Ala Asp Ala Gly Ala Val Thr
            340                 345                 350

Gln Glu Glu Glu Asn Pro Val Glu Leu Trp Ser Leu Asp Glu Ile Asn
        355                 360                 365

Phe Met Leu Glu Gly Asp Phe
    370                 375


<210>  167
<211>  1629
<212>  DNA
<213>  Arabidopsis thaliana

<400>  167
ggagagtaac atcgagacaa agaagaaaaa ctaaaaaaga gaaccccaaa gaatcgaata      60

tttattattt cgccccgaag attctatttc tgatcattta caccccctaaa aagagtagag    120

ctttcgtgaa gccaccatgt gtggaggagc tataatctcc gatttcatac ctccgccgag     180

gtccctccgc gtcactaacg agtttatctg gccggatctg aaaaacaaag tgaaagcttc     240

aaagaagaga tcgaataagc gatccgattt cttcgatctt gacgatgatt tcgaagctga     300

tttccaaggg tttaaggatg actcggcttt tgactgcgaa gacgatgatg atgtcttcgt     360

caatgttaag cctttcgtct tcaccgcaac tactaagccc gtagcttccg ctttcgtctc     420

cactggttca gcatatgcca agaaaactgt agagtccgct gagcaagctg agaaatcttc     480

taagaggaag aggaagaatc aataccgagg gattaggcag cgtccttggg gaaaatgggc     540

tgcggagatc cgtgatccga gaaaaggctc ccgagaatgg cttggaacat tcgacactgc     600
```

tgaggaagca gcaagagctt atgatgctgc agcacgcaga atccgtggca cgaaagctaa 660

ggtgaatttt cccgaggaga agaaccctag cgtcgtatcc cagaaacgtc ctagtgctaa 720

gactaataat cttcagaaat cagtggctaa accaaacaaa agcgtaactt tggttcagca 780

gccaacacat ctgagtcagc agtactgcaa caactccttt gacaactctt ttggtgatat 840

gagtttcatg gaagagaagc ctcagatgta caacaatcag tttgggttaa caaactcgtt 900

cgatgctgga ggtaacaatg gataccagta tttcagttcc gatcagggca gtaactcctt 960

cgactgttct gagttcgggt ggagtgatca cggccctaaa acacccgaga tctcttcaat 1020

gcttgtcaat aacaacgaag catcatttgt tgaagaaacc aatgcagcca agaagctcaa 1080

accaaactct gatgagtcag acgatctgat ggcatacctt gacaacgcct tgtgggacac 1140

cccactagaa gtggaagcca tgcttggcgc agatgctggt gctgtgactc aggaagagga 1200

aaacccagtg gagctatgga gcttagatga gatcaatttc atgctggaag gagacttttg 1260

aagtgatcga tggttcctta gtttgtaaat aaagctgtgt tggattttgc tgttggggga 1320

tggtacaagt cacacctcaa gctctatgca ttggtatctc atgagcctct cttccataga 1380

gagtttctct tttaattttg tcgaaataaa aaaggtgtga tgaagtaaat agaggtataa 1440

taatatctat ctattaagtc ttgttttgtt ctttcatttt tgtatttctt ttctatttaa 1500

aagacagttt attagtcttc tgagctctct ttttgatctt tgttatagcg tatcatcacc 1560

ctcgaaagtg taatgttttg tacccccaaa cttgtttagc attataataa agtctctttg 1620

gaacttctc 1629

<210> 168
<211> 374
<212> PRT
<213> Arabidopsis thaliana

<400> 168

Met Cys Gly Gly Ala Ile Ile Ser Asp Phe Ile Pro Pro Pro Arg Ser
1 5 10 15

Leu Arg Val Thr Asn Glu Phe Ile Trp Pro Asp Leu Lys Asn Lys Val
20 25 30

Lys Ala Ser Lys Lys Arg Ser Asn Lys Arg Ser Asp Phe Phe Asp Leu
35 40 45

Asp Asp Asp Phe Glu Ala Asp Phe Gln Gly Phe Lys Asp Asp Ser Ala
50 55 60

Phe Asp Cys Glu Asp Asp Asp Asp Val Phe Val Asn Val Lys Pro Phe
65 70 75 80

Val Phe Thr Ala Thr Thr Lys Pro Val Ala Ser Ala Phe Val Ser Thr

85 90 95

Gly Ser Ala Tyr Ala Lys Lys Thr Val Glu Ser Ala Glu Gln Ala Glu
100 105 110

Lys Ser Ser Lys Arg Lys Arg Lys Asn Gln Tyr Arg Gly Ile Arg Gln
115 120 125

Arg Pro Trp Gly Lys Trp Ala Ala Glu Ile Arg Asp Pro Arg Lys Gly
130 135 140

Ser Arg Glu Trp Leu Gly Thr Phe Asp Thr Ala Glu Glu Ala Ala Arg
145 150 155 160

Ala Tyr Asp Ala Ala Ala Arg Arg Ile Arg Gly Thr Lys Ala Lys Val
165 170 175

Asn Phe Pro Glu Glu Lys Asn Pro Ser Val Val Ser Gln Lys Arg Pro
180 185 190

Ser Ala Lys Thr Asn Asn Leu Gln Lys Ser Val Ala Lys Pro Asn Lys
195 200 205

Ser Val Thr Leu Val Gln Gln Pro Thr His Leu Ser Gln Gln Tyr Cys
210 215 220

Asn Asn Ser Phe Asp Asn Ser Phe Gly Asp Met Ser Phe Met Glu Glu
225 230 235 240

Lys Pro Gln Met Tyr Asn Asn Gln Phe Gly Leu Thr Asn Ser Phe Asp
245 250 255

Ala Gly Gly Asn Asn Gly Tyr Gln Tyr Phe Ser Ser Asp Gln Gly Ser
260 265 270

Asn Ser Phe Asp Cys Ser Glu Phe Gly Trp Ser Asp His Gly Pro Lys
275 280 285

Thr Pro Glu Ile Ser Ser Met Leu Val Asn Asn Asn Glu Ala Ser Phe
290 295 300

Val Glu Glu Thr Asn Ala Ala Lys Lys Leu Lys Pro Asn Ser Asp Glu
305 310 315 320

Ser Asp Asp Leu Met Ala Tyr Leu Asp Asn Ala Leu Trp Asp Thr Pro
325 330 335

Leu Glu Val Glu Ala Met Leu Gly Ala Asp Ala Gly Ala Val Thr Gln
340 345 350

Glu Glu Glu Asn Pro Val Glu Leu Trp Ser Leu Asp Glu Ile Asn Phe
355 360 365

Met Leu Glu Gly Asp Phe
370

<210> 169
<211> 1071
<212> DNA
<213> Arabidopsis thaliana

<400> 169
ataaaggcat ttcagctcca ccgtaggaaa ctttctcttg aaagaaaccc acagcaacaa 60

acagagaaaa tgtgtggcgg tgctattatt tccgattatg cccctctcgt caccaaggcc 120

aagggccgta aactcacggc tgaggaactc tggtcagagc tcgatgcttc cgccgccgac 180

gacttctggg gtttctattc cacctccaaa ctccatccca ccaaccaagt taacgtgaaa 240

gaggaggcag tgaagaagga gcaggcaaca gagccgggga aacggaggaa gaggaagaat 300

gtttatagag ggatacgtaa gcgtccatgg ggaaaatggg cggctgagat tcgagatcca 360

cgaaaaggtg ttagagtttg gcttggtacg ttcaacacgg cggaggaagc tgccatggct 420

tatgatgttg cggccaagca gatccgtggt gataaagcca agctcaactt cccagatctg 480

caccatcctc ctcctcctaa ttatactcct ccgccgtcat cgccacgatc aaccgatcag 540

cctccggcga agaaggtctg cgttgtctct cagagtgaga gcgagttaag tcagccgagt 600

ttcccggtgg agtgtatagg atttggaaat ggggacgagt ttcagaacct gagttacgga 660

tttgagccgg attatgatct gaaacagcag atatcgagct tggaatcgtt ccttgagctg 720

gacggtaaca cggcggagca accgagtcag cttgatgagt ccgtttccga ggtggatatg 780

tggatgcttg atgatgtcat tgcgtcgtat gagtaaaaga aaaaaaataa gtttaaaaaa 840

agttaaataa agtctgtaat atatatgtaa ccgccgttac ttttaaaagg tttttaccgt 900

cgcattggac tgctgatgat gtctgttgtg taatgtgtag aatgtgacca aatggacgtt 960

atattacggt ttgtggtatt attagtttct tagatggaaa aacttacatg tgtaaataag 1020

atttgtaatg taagacgaag tacttataac ttcttaactg ttttgtggta g 1071

<210> 170
<211> 248
<212> PRT
<213> Arabidopsis thaliana

<400> 170

Met Cys Gly Gly Ala Ile Ile Ser Asp Tyr Ala Pro Leu Val Thr Lys
1 5 10 15

Ala Lys Gly Arg Lys Leu Thr Ala Glu Glu Leu Trp Ser Glu Leu Asp

```
                20                  25                  30

Ala Ser Ala Ala Asp Asp Phe Trp Gly Phe Tyr Ser Thr Ser Lys Leu
        35                  40                  45

His Pro Thr Asn Gln Val Asn Val Lys Glu Glu Ala Val Lys Lys Glu
    50                  55                  60

Gln Ala Thr Glu Pro Gly Lys Arg Arg Lys Arg Lys Asn Val Tyr Arg
65                  70                  75                  80

Gly Ile Arg Lys Arg Pro Trp Gly Lys Trp Ala Ala Glu Ile Arg Asp
                85                  90                  95

Pro Arg Lys Gly Val Arg Val Trp Leu Gly Thr Phe Asn Thr Ala Glu
            100                 105                 110

Glu Ala Ala Met Ala Tyr Asp Val Ala Ala Lys Gln Ile Arg Gly Asp
        115                 120                 125

Lys Ala Lys Leu Asn Phe Pro Asp Leu His His Pro Pro Pro Pro Asn
    130                 135                 140

Tyr Thr Pro Pro Pro Ser Ser Pro Arg Ser Thr Asp Gln Pro Pro Ala
145                 150                 155                 160

Lys Lys Val Cys Val Val Ser Gln Ser Glu Ser Glu Leu Ser Gln Pro
                165                 170                 175

Ser Phe Pro Val Glu Cys Ile Gly Phe Gly Asn Gly Asp Glu Phe Gln
            180                 185                 190

Asn Leu Ser Tyr Gly Phe Glu Pro Asp Tyr Asp Leu Lys Gln Gln Ile
        195                 200                 205

Ser Ser Leu Glu Ser Phe Leu Glu Leu Asp Gly Asn Thr Ala Glu Gln
    210                 215                 220

Pro Ser Gln Leu Asp Glu Ser Val Ser Glu Val Asp Met Trp Met Leu
225                 230                 235                 240

Asp Asp Val Ile Ala Ser Tyr Glu
                245
```

<210> 171
<211> 1433
<212> DNA
<213> Oryza sativa

<400> 171

agactacaag ccattaaaca gagacgacca acgacttaac cacacttctt ctttgtgctg 60
tccaacctcc attgttgggt gtgaaagctt tggctcatct gccatgtgtg gaggatccat 120
tctcggcgac cttcacttgc cggtgcggcg gacagtgaac gccggtgacc tgtggggaga 180
cgccggcaag ggtagagatg gtggcgacgg cttgaagaag aggaagggga gttcttggga 240
tttcgatgtt gattgcgatg atgatgatga tgatgacttt gaggctgatt ttgaggagtt 300
tgaggatgac tatggcgatg atgatgatgt gggtttcggg gacgacgacc aagaatccga 360
catgaacggt ctcaagctcg ccggattcag caccacgaag ctcggcctcg gcggcagcag 420
gaagaggaag acgcgatacc gagggatccg gcagcggcca tgggggaaat gggcggcgga 480
gatcagggac ccccgcaagg gcgtccgcgt ctggctcggc acgttcggca ccgccgagga 540
ggccgccatg gcgtacgacg tcgaggcacg ccgcatccgc ggcaagaaag ccaaggtcaa 600
cttccccgac gccgccgccg ccgccccgaa gcggccacgg cgttcttcgg cgaagcattc 660
gccgcagcag cagaaggcca ggtcgtcgtc gtcgtcgccg gcgagcctga acgccagcga 720
cgccgtgtcc aagtccaaca acaaccgcgt cagctcggct gggagcagca ccgacgccac 780
cgccgccgcc atcgccatcg acgacggcgt caagctcgag ctgctctcgg agacggatcc 840
ttctccgccc atggccgccg ccgccgccgc gtggctcgac gcgttcgagc tgaacgatct 900
tgacggatca agatgcaagg acaacgcatt cgatcaccag attcacaagg tagaagcggc 960
tgtcgctgat gaattcgcgt tctacgacga tccgagctac atgcagctgg gttaccagct 1020
cgatcagggc aactcgtacg agaacatcga cgcgctcttc ggcggcgagg ccgtcaacat 1080
tggtggactc tggagcttcg acgacatgcc aatggagttc agagcttatt gagcatttga 1140
ttctatttag gagggagtga attatttggg aggaagaatc gatgttgtaa cttgtaaaat 1200
ctctgatgat gatctctgca tcatatgatc aatttgagtg cagttttgtt tttgtaaata 1260
cgaattcttt attatgatgt taggttctta atttgccctt cttcatcagg gatttgttca 1320
ggcttttgtt gtgatgactg attggacgag ggaaagattg cgtttttttgt tgtcatgttg 1380
ggtactctac tcttctgttc ctaaagttga taagtgccaa aatttgtgag agg 1433

<210> 172
<211> 342
<212> PRT
<213> Oryza sativa

<400> 172

Met Cys Gly Gly Ser Ile Leu Gly Asp Leu His Leu Pro Val Arg Arg
1 5 10 15

Thr Val Asn Ala Gly Asp Leu Trp Gly Asp Ala Gly Lys Gly Arg Asp
20 25 30

Gly Gly Asp Gly Leu Lys Lys Arg Lys Gly Ser Ser Trp Asp Phe Asp

35 40 45

Val Asp Cys Asp Asp Asp Asp Asp Asp Asp Phe Glu Ala Asp Phe Glu
50 55 60

Glu Phe Glu Asp Asp Tyr Gly Asp Asp Asp Asp Val Gly Phe Gly Asp
65 70 75 80

Asp Asp Gln Glu Ser Asp Met Asn Gly Leu Lys Leu Ala Gly Phe Ser
85 90 95

Thr Thr Lys Leu Gly Leu Gly Gly Ser Arg Lys Arg Lys Thr Arg Tyr
100 105 110

Arg Gly Ile Arg Gln Arg Pro Trp Gly Lys Trp Ala Ala Glu Ile Arg
115 120 125

Asp Pro Arg Lys Gly Val Arg Val Trp Leu Gly Thr Phe Gly Thr Ala
130 135 140

Glu Glu Ala Ala Met Ala Tyr Asp Val Glu Ala Arg Arg Ile Arg Gly
145 150 155 160

Lys Lys Ala Lys Val Asn Phe Pro Asp Ala Ala Ala Ala Ala Pro Lys
165 170 175

Arg Pro Arg Arg Ser Ser Ala Lys His Ser Pro Gln Gln Gln Lys Ala
180 185 190

Arg Ser Ser Ser Ser Ser Pro Ala Ser Leu Asn Ala Ser Asp Ala Val
195 200 205

Ser Lys Ser Asn Asn Asn Arg Val Ser Ser Ala Gly Ser Ser Thr Asp
210 215 220

Ala Thr Ala Ala Ala Ile Ala Ile Asp Asp Gly Val Lys Leu Glu Leu
225 230 235 240

Leu Ser Glu Thr Asp Pro Ser Pro Pro Met Ala Ala Ala Ala Ala Ala
245 250 255

Trp Leu Asp Ala Phe Glu Leu Asn Asp Leu Asp Gly Ser Arg Cys Lys
260 265 270

Asp Asn Ala Phe Asp His Gln Ile His Lys Val Glu Ala Ala Val Ala
275 280 285

Asp Glu Phe Ala Phe Tyr Asp Asp Pro Ser Tyr Met Gln Leu Gly Tyr
290 295 300

Gln Leu Asp Gln Gly Asn Ser Tyr Glu Asn Ile Asp Ala Leu Phe Gly
305                 310                 315                 320

Gly Glu Ala Val Asn Ile Gly Gly Leu Trp Ser Phe Asp Asp Met Pro
                325                 330                 335

Met Glu Phe Arg Ala Tyr
            340

<210>  173
<211>  749
<212>  DNA
<213>  Oryza sativa

<400>  173
ctcgcgccat ccaagcgccc gcctctatat atgcgcgccg ccaccatgtc cagctccggc      60

aactagctac tgcgaagtgc gaactgatca gatcgtcgag aggaaaccca agatccaacg     120

acgacatgtg tggcggcgcg attctggcta acatcatacc ggccaccccca cgccccccgca     180

agtgccgccc caccaccgcc accgccaccc ccaaggcgac gacaccgaac gtcgtcgtcg     240

tcgtcaacct cgtcgacaaa gaggccgagg tcagcgagag ctccggtgcc agcagcagcg     300

cgctgccgga cttctcgtgg cagggcatgt cggcgtcgtc cgacgacgac gccgcggcgc     360

agcaggcact cctcgacgcc gccggcggcg ccaagaagcg tccccggagc gagccccacg     420

tcacctccga cgacgaagtg ctcccggcgt cattcgacag tgacaacaac accgccgccg     480

ccggcctgct cccgctcgac gatcctttct tgttcggcga ccagttcggc gacctcaacg     540

gcggcgcgtt cgcctcgctc atggacgggc tgttcgccgc cggtgaagcg aacgtcgccg     600

gcgagagcgt ggggctctgg agcttcggcg acgactttct caacgcgtcg tactattagc     660

tagggcttcc atagttcttg tactttactt gtactgtact gtattgtact gattgttaac     720

gttgccataa agcaatcttg ctagtttgt                                       749

<210>  174
<211>  321
<212>  PRT
<213>  Oryza sativa

<400>  174

Met Cys Gly Gly Ala Ile Leu Ala Asn Ile Ile Pro Ala Thr Pro Pro
1               5                   10                  15

Arg Pro Ala Thr Ala Ala His Val Trp Pro Gly Gly Asp Gly Glu Lys
            20                  25                  30

Arg Arg Lys Val Gly Gly Gly Gly Cys Asp Asp Asp Phe Glu Ala Ala
        35                  40                  45

```
Phe Glu Arg Phe Gly Arg Glu Asp Ser Glu Met Glu Glu Glu Glu Val
    50                  55                  60

Glu Glu Val Val Val Gly Lys Lys Ala Ala Val Arg Arg Arg Arg Ala
65                  70                  75                  80

Thr Pro Ala Ala Gly Arg Arg Ala Arg Pro Ser Lys Tyr Trp Gly Val
                85                  90                  95

Arg Arg Arg Pro Trp Gly Lys Trp Ala Ala Glu Ile Arg Asp Pro Val
            100                 105                 110

Glu Gly Val Arg Val Trp Leu Gly Thr Phe Ala Thr Ala Glu Ala Ala
        115                 120                 125

Ala His Ala Tyr Asp Ala Ala Ala Arg Asp Leu Arg Gly Ala Thr Ala
    130                 135                 140

Lys Leu Asn Phe Pro Ser Ser Ser Ser Ser Thr Ala Ala Thr Pro Arg
145                 150                 155                 160

Pro Arg Lys Cys Arg Pro Thr Thr Ala Thr Ala Thr Pro Lys Ala Thr
                165                 170                 175

Thr Pro Asn Val Val Val Val Val Asn Leu Val Asp Lys Glu Ala Glu
            180                 185                 190

Val Ser Glu Ser Ser Gly Ala Ser Ser Ser Ala Leu Pro Asp Phe Ser
        195                 200                 205

Trp Gln Gly Met Ser Ala Ser Ser Asp Asp Asp Ala Ala Ala Gln Gln
    210                 215                 220

Ala Leu Leu Asp Ala Ala Gly Gly Ala Lys Lys Arg Pro Arg Ser Glu
225                 230                 235                 240

Pro His Val Thr Ser Asp Asp Glu Val Leu Pro Ala Ser Phe Asp Ser
                245                 250                 255

Asp Asn Asn Thr Ala Ala Ala Gly Leu Leu Pro Leu Asp Asp Pro Phe
            260                 265                 270

Leu Phe Gly Asp Gln Phe Gly Asp Leu Asn Gly Gly Ala Phe Ala Ser
        275                 280                 285

Leu Met Asp Gly Leu Phe Ala Ala Gly Glu Ala Asn Val Ala Gly Glu
    290                 295                 300
```

Ser Val Gly Leu Trp Ser Phe Gly Asp Asp Phe Leu Asn Ala Ser Tyr
305 310 315 320

Tyr

<210> 175
<211> 1192
<212> DNA
<213> Oryza sativa

<400> 175
tcatcacaag ctcacaagtt cacaacccaa cacccaaaag caaaagaaaa gcagcaacca 60

aagatgtgcg gcggagcgat ccttgcggag ctcataccga gcgcgccggc ggcgaggcgc 120

gtcacggcgg gccacgtctg gccgggcgac gccaacaagg ccaagaagaa gggcgcgcgc 180

gccgacgact tcgaggccgc gttccgcgac ttcgacaacg actccgatga cgaggagatg 240

atggtggagg aggcggagga ggaggaggcg acctccgagc acaagccgtt cgtcttccgc 300

gccaagaagg cggcggcggc ggcgtcgagc aggcgcagga agccggcgca gtacaggggc 360

gtgcggcgcc ggccgtgggg gaagtgggcg gcggagatcc gcgaccccgt caagggcatc 420

cgcgtctggc tcggcacctt caccaacgcc gaggccgccg cgctcgccta cgacgacgcc 480

gcgcgcgcca tccgcgggga cagggccaag ctcaacttcc cttccgctac cacccctgac 540

acccgcaagc gcggccgcgc caccgccgcc gccgccccgg ccgtcaaggc gaccccggtc 600

atcaacctcg tcgaggagga ggacgaggag gaggtcgccg ccgccatggc gtccatcaag 660

tacgagcccg agaccagcga gagctccgag tcgaacgccc tcccggactt ctcctggcag 720

ggcatgtcgg cctccgacga gttcgccgtc gccgcggcgg cgctgtcgct cgacagcgac 780

gacgacctcg ccaagaagcg tccgaggacc gagccggagg acaccaccga ctccggctcc 840

ggcgacgaca ccgacgcgct gttcgacgcg ctgctgttcg ccgaccagta caaccacttc 900

aacggcggcg cctacgagtc cctggacagc ctgttcagcg ccgacgccgt gcagaccacc 960

gccgccgccg ccgccgccga ccagggcatg ggctctggga gcttcgacga cggctgctgc 1020

ctcgtcgacg tcgaggccag cttgtccttc taggctctag ccgtcgtctc cggcgaccct 1080

gtgactcgat cttgtaccat gtcatgtaca tagaagagtg gttttgccaa gcaagcatgt 1140

gttcgtcctg gttcctttcg gtaatgaaaa attatgtgag gcttctcgtt ct 1192

<210> 176
<211> 329
<212> PRT
<213> Oryza sativa

<400> 176

Met Cys Gly Gly Ala Ile Leu Ala Glu Leu Ile Pro Ser Ala Pro Ala
1 5 10 15

```
Ala Arg Arg Val Thr Ala Gly His Val Trp Pro Gly Asp Ala Asn Lys
            20                  25                  30

Ala Lys Lys Lys Gly Ala Arg Ala Asp Asp Phe Glu Ala Ala Phe Arg
        35                  40                  45

Asp Phe Asp Asn Asp Ser Asp Asp Glu Glu Met Met Val Glu Glu Ala
    50                  55                  60

Glu Glu Glu Glu Ala Thr Ser Glu His Lys Pro Phe Val Phe Arg Ala
65                  70                  75                  80

Lys Lys Ala Ala Ala Ala Ala Ser Ser Arg Arg Arg Lys Pro Ala Gln
                85                  90                  95

Tyr Arg Gly Val Arg Arg Arg Pro Trp Gly Lys Trp Ala Ala Glu Ile
            100                 105                 110

Arg Asp Pro Val Lys Gly Ile Arg Val Trp Leu Gly Thr Phe Thr Asn
        115                 120                 125

Ala Glu Ala Ala Ala Leu Ala Tyr Asp Asp Ala Ala Arg Ala Ile Arg
    130                 135                 140

Gly Asp Arg Ala Lys Leu Asn Phe Pro Ser Ala Thr Thr Pro Asp Thr
145                 150                 155                 160

Arg Lys Arg Gly Arg Ala Thr Ala Ala Ala Ala Pro Ala Val Lys Ala
                165                 170                 175

Thr Pro Val Ile Asn Leu Val Glu Glu Glu Asp Glu Glu Glu Val Ala
            180                 185                 190

Ala Ala Met Ala Ser Ile Lys Tyr Glu Pro Glu Thr Ser Glu Ser Ser
        195                 200                 205

Glu Ser Asn Ala Leu Pro Asp Phe Ser Trp Gln Gly Met Ser Ala Ser
    210                 215                 220

Asp Glu Phe Ala Val Ala Ala Ala Ala Leu Ser Leu Asp Ser Asp Asp
225                 230                 235                 240

Asp Leu Ala Lys Lys Arg Pro Arg Thr Glu Pro Glu Asp Thr Thr Asp
                245                 250                 255

Ser Gly Ser Gly Asp Asp Thr Asp Ala Leu Phe Asp Ala Leu Leu Phe
            260                 265                 270
```

Ala Asp Gln Tyr Asn His Phe Asn Gly Gly Ala Tyr Glu Ser Leu Asp
275 280 285

Ser Leu Phe Ser Ala Asp Ala Val Gln Thr Thr Ala Ala Ala Ala Ala
290 295 300

Ala Asp Gln Gly Met Gly Leu Trp Ser Phe Asp Asp Gly Cys Cys Leu
305 310 315 320

Val Asp Val Glu Ala Ser Leu Ser Phe
325

<210> 177
<211> 789
<212> DNA
<213> Arabidopsis thaliana

<400> 177
atgtgcggag gagctgtaat ttccgattac atagcgccgg agaagattgc gagatcatct 60

ggaaagtctt cctggagaag taatggcgtc tttgactgct caatctacga tttcgatgga 120

aatttcgatg aattagagtc cgatgagcca tttgtcttct cctctactca caaacatcat 180

gcttcaggct cagcatcaga tgggaagaag aaacagagca gtcggtacaa aggaatcaga 240

agaaggcctt ggggaagatg ggcggctgag atacgtgatc caatcaaagg agttcgagtt 300

tggctcggga ctttcaacac agctgaagaa gctgcaagag cttatgatct tgaagctaag 360

agaatccgtg gagccaaagc taagctcaat ttccctaacg aatcctctgg aaagaggaaa 420

gccaaggcta agactgtgca acaggtagag gagaatcatg aggctgatct tgatgtggcg 480

gtggtaagct cagcgcctag tagtagctgt cttgatttct tgtgggagga gaataatccg 540

gacacgcttc tgattgatac acaatggctc gaagatatca tcatgggcga tgcgaataag 600

aaacatgaac ctaatgatag tgaagaagcc aacaacgttg atgcttctct gctttctgaa 660

gagcttcttg cttttgagaa ccagaccgaa tatttctcgc agatgccttt tacggaggga 720

aactgtgatt cctcaacgtc tctgagtagt ctctttgatg gaggcaatga catgggtcta 780

tggtcctga 789

<210> 178
<211> 262
<212> PRT
<213> Arabidopsis thaliana

<400> 178

Met Cys Gly Gly Ala Val Ile Ser Asp Tyr Ile Ala Pro Glu Lys Ile
1 5 10 15

Ala Arg Ser Ser Gly Lys Ser Ser Trp Arg Ser Asn Gly Val Phe Asp
20 25 30

```
Cys Ser Ile Tyr Asp Phe Asp Gly Asn Phe Asp Glu Leu Glu Ser Asp
        35                  40                  45

Glu Pro Phe Val Phe Ser Ser Thr His Lys His His Ala Ser Gly Ser
    50                  55                  60

Ala Ser Asp Gly Lys Lys Lys Gln Ser Ser Arg Tyr Lys Gly Ile Arg
65                  70                  75                  80

Arg Arg Pro Trp Gly Arg Trp Ala Ala Glu Ile Arg Asp Pro Ile Lys
                85                  90                  95

Gly Val Arg Val Trp Leu Gly Thr Phe Asn Thr Ala Glu Glu Ala Ala
            100                 105                 110

Arg Ala Tyr Asp Leu Glu Ala Lys Arg Ile Arg Gly Ala Lys Ala Lys
        115                 120                 125

Leu Asn Phe Pro Asn Glu Ser Ser Gly Lys Arg Lys Ala Lys Ala Lys
    130                 135                 140

Thr Val Gln Gln Val Glu Glu Asn His Glu Ala Asp Leu Asp Val Ala
145                 150                 155                 160

Val Val Ser Ser Ala Pro Ser Ser Ser Cys Leu Asp Phe Leu Trp Glu
                165                 170                 175

Glu Asn Asn Pro Asp Thr Leu Leu Ile Asp Thr Gln Trp Leu Glu Asp
            180                 185                 190

Ile Ile Met Gly Asp Ala Asn Lys Lys His Glu Pro Asn Asp Ser Glu
        195                 200                 205

Glu Ala Asn Asn Val Asp Ala Ser Leu Leu Ser Glu Glu Leu Leu Ala
    210                 215                 220

Phe Glu Asn Gln Thr Glu Tyr Phe Ser Gln Met Pro Phe Thr Glu Gly
225                 230                 235                 240

Asn Cys Asp Ser Ser Thr Ser Leu Ser Ser Leu Phe Asp Gly Gly Asn
                245                 250                 255

Asp Met Gly Leu Trp Ser
            260
```

<210> 179
<211> 1430
<212> DNA
<213> Oryza sativa

<400> 179
cccgcgtcgc gccatagcga cgtctctccc ggagaaagaa gagcgcgccg cgccatgtgc 60

ggcggtgcaa tcctcgccga tttcaccccg gcgagggtgc cccggcggct gaccgccgcc 120

gagctcctgc cggtgacccc gactcccccc gccgccgaga ggagaaccac ccggaagcgc 180

aagtccgacg tcgacttcga ggcggagttc gagcttttcg aggacgacga cgacgacgat 240

gagttcgagc tttccgacga tggcgacgag agtttggccg tgtcatgtgt gtcgtccccc 300

aagtcgaagg cagtaccttc gttttctttt tcgtcggatg tctcctcgag ctccaggccg 360

cggcggcgcg tggcggcggc ggcggccggt cgtcggaagg cgagcaagaa gagcaagtac 420

aggggcgtcc ggcgccggcc gtcggggagg ttcgcggcgg agatcaggga ccccaagaag 480

gggcggcgcg tgtggctcgg cacgtacggc agcgccgagg aggccgccat ggcctacgac 540

cgcgaggccc gccgcatccg cggcaagggc gcgaggctca acttcccccg cgacggcgat 600

ggctcccctc gccggagtaa cgaccggccc tgctggacca tcgacctcaa cctccccgcg 660

gcggccgtct ccggtgacga cgacgacgcc atggccgtcg acgccgcaga cgcagacgca 720

ggcagtgctg gccgtgcagc agcctatgca gatcaagaag cactgagcgc ggcaaagtgc 780

aagatcaagc agtgtcctcg cgacgaacag atggcgagcg ccacacctga gctcatggag 840

gaggacgcga gcagcagcag aaacatggtg cccctgtcca tggcgctgca gctgcagtat 900

gcggcgatga tcgccgaatg cgaccgcgag atggaggaga tcgccgccgt ggagagggac 960

ctcgagaggc gcaggaggca ggtgttcgag cgcagaggcc acctggtcag gcaggcctct 1020

cttctgctcg actgacgatc ttgctgaact gaactctgaa tgtttgagct tgtctgaagt 1080

ctgaactctg cactatgcca tatggtgttt caccttcact gtgaggctga catgtgggcc 1140

agacgtccgt ttgccttgct tgcttttgag ctgcaactgg gcgttgtgct gcaagcctgc 1200

aaagtgcctg tgtaaagttt gtgggattgt gtgtgtggat cttgtatcct tgtgactttg 1260

caagctttaa ttttgtgagg aacaatagta ttttagattg gtgtgtaaaa tatagaaata 1320

aataatagta gcaactaaat tttggttttc agctcttcat cagatggttt gtcatgggaa 1380

caaaatttca aacatgagca aattaaggtc atgttattat caattgtgat 1430

<210> 180
<211> 326
<212> PRT
<213> Oryza sativa

<400> 180

Met Cys Gly Gly Ala Ile Leu Ala Asp Phe Thr Pro Ala Arg Val Pro
1 5 10 15

Arg Arg Leu Thr Ala Ala Glu Leu Leu Pro Val Thr Pro Thr Pro Pro
20 25 30

```
Ala Ala Glu Arg Arg Thr Thr Arg Lys Arg Lys Ser Asp Val Asp Phe
        35                  40                  45

Glu Ala Glu Phe Glu Leu Phe Glu Asp Asp Asp Asp Asp Asp Glu Phe
    50                  55                  60

Glu Leu Ser Asp Asp Gly Asp Glu Ser Leu Ala Val Ser Cys Val Ser
65                  70                  75                  80

Ser Pro Lys Ser Lys Ala Val Pro Ser Phe Ser Phe Ser Ser Asp Val
                85                  90                  95

Ser Ser Ser Ser Arg Pro Arg Arg Arg Val Ala Ala Ala Ala Ala Gly
            100                 105                 110

Arg Arg Lys Ala Ser Lys Lys Ser Lys Tyr Arg Gly Val Arg Arg Arg
        115                 120                 125

Pro Ser Gly Arg Phe Ala Ala Glu Ile Arg Asp Pro Lys Lys Gly Arg
    130                 135                 140

Arg Val Trp Leu Gly Thr Tyr Gly Ser Ala Glu Glu Ala Ala Met Ala
145                 150                 155                 160

Tyr Asp Arg Glu Ala Arg Arg Ile Arg Gly Lys Gly Ala Arg Leu Asn
                165                 170                 175

Phe Pro Arg Asp Gly Asp Gly Ser Pro Arg Arg Ser Asn Asp Arg Pro
            180                 185                 190

Cys Trp Thr Ile Asp Leu Asn Leu Pro Ala Ala Ala Val Ser Gly Asp
        195                 200                 205

Asp Asp Asp Ala Met Ala Val Asp Ala Ala Asp Ala Asp Ala Gly Ser
    210                 215                 220

Ala Gly Arg Ala Ala Ala Tyr Ala Asp Gln Glu Ala Leu Ser Ala Ala
225                 230                 235                 240

Lys Cys Lys Ile Lys Gln Cys Pro Arg Asp Glu Gln Met Ala Ser Ala
                245                 250                 255

Thr Pro Glu Leu Met Glu Glu Asp Ala Ser Ser Ser Arg Asn Met Val
            260                 265                 270

Pro Leu Ser Met Ala Leu Gln Leu Gln Tyr Ala Ala Met Ile Ala Glu
        275                 280                 285
```

```
Cys Asp Arg Glu Met Glu Glu Ile Ala Ala Val Glu Arg Asp Leu Glu
    290                 295                 300

Arg Arg Arg Arg Gln Val Phe Glu Arg Arg Gly His Leu Val Arg Gln
305                 310                 315                 320

Ala Ser Leu Leu Leu Asp
                325
```

<210> 181
<211> 1049
<212> DNA
<213> Oryza sativa

<400> 181

```
agaagaaaag ccttgtgagt tggtaattga tagtagcaag acaatgtgtg ggggagcgat      60
catcgccgac ttcgtcccgc ccgccggcgc ccgccgcgcc gctgcctccg acatctccga     120
caacgccgtc ctctccgctg ccggtgccgg tgacgagtcg ttcgcggcgg ccaaggcgcc     180
ggcgccgggg aggaagacgg cgtaccgcgg catccggcgc cggccgtggg gacgctgggc     240
ggcggagatc cgcgacccga ggaagggcgc ccgcgtctgg ctcggcacct acgccaccgc     300
cgaggaggcc gcccgcgcct acgacgtcgc ggcgcgcgac atccgcggcg ccaaggccaa     360
gctcaacttc cccccgacca tcggcgccgc cgccgcgcca ccgccgccca agaagcgacg     420
caaagccgcc gccgcggcga accaccacca ccaccaccac cagcaggaga gctcaggctc     480
ctcgtcggcg tcgtcgctgc ctcccacccc gccgccggcc gccgagcacc agctccgcga     540
gtgcatgtcc gggctggagg cgttcttggg cctcgaggag gaggaggacg acggcggcgc     600
cggtgagcca tgggacgccg tcgacatgat gctcgagtag gctcgccggg aatggcagcg     660
ttgccatgct catgcatcca agcttattca tgcatgcagc agcaagatat acaactttag     720
tactactact atgttaatta ctacttactg cgtaggtaaa tgaaataaaa tggggttggt     780
taattagggt gttcttctgg gaatccttgg ctttgctaat tgctagtact actatgtact     840
ttggcgttaa gcattgcatt gccaggtgat gcatgtcatg taatcgtggt aattatatgg     900
ccgtcctcct agagctagct agcagattag atttactata taaagtggta gtagaaatat     960
atgtcctcct agagctaact agtgtcttgt aatgatttca gacttcttgg gcaacatgag    1020
caatgcattt cgacttccac tttacatct                                      1049
```

<210> 182
<211> 198
<212> PRT
<213> Oryza sativa

<400> 182

```
Met Cys Gly Gly Ala Ile Ile Ala Asp Phe Val Pro Pro Ala Gly Ala
1               5                   10                  15
```

Arg Arg Ala Ala Ala Ser Asp Ile Ser Asp Asn Ala Val Leu Ser Ala
20 25 30

Ala Gly Ala Gly Asp Glu Ser Phe Ala Ala Ala Lys Ala Pro Ala Pro
35 40 45

Gly Arg Lys Thr Ala Tyr Arg Gly Ile Arg Arg Arg Pro Trp Gly Arg
50 55 60

Trp Ala Ala Glu Ile Arg Asp Pro Arg Lys Gly Ala Arg Val Trp Leu
65 70 75 80

Gly Thr Tyr Ala Thr Ala Glu Glu Ala Ala Arg Ala Tyr Asp Val Ala
85 90 95

Ala Arg Asp Ile Arg Gly Ala Lys Ala Lys Leu Asn Phe Pro Pro Thr
100 105 110

Ile Gly Ala Ala Ala Ala Pro Pro Pro Pro Lys Lys Arg Arg Lys Ala
115 120 125

Ala Ala Ala Ala Asn His His His His His His Gln Gln Glu Ser Ser
130 135 140

Gly Ser Ser Ser Ala Ser Ser Leu Pro Pro Thr Pro Pro Pro Ala Ala
145 150 155 160

Glu His Gln Leu Arg Glu Cys Met Ser Gly Leu Glu Ala Phe Leu Gly
165 170 175

Leu Glu Glu Glu Glu Asp Asp Gly Gly Ala Gly Glu Pro Trp Asp Ala
180 185 190

Val Asp Met Met Leu Glu
195

<210> 183
<211> 1164
<212> DNA
<213> Oryza sativa

<400> 183
acctcgctct cctcgtcacc gcgatcgatc gatcgccgcc ggtttagatt tccttcctac 60

gcggaccgca cccacgcgct aggtttagct agctagtttg cttgcgcgca agcgtcgatc 120

gagctagcta gtgaagatag atatgtgcgg cggcgccatc ccgctgatca gcagccgcgg 180

ccccggcggc aagaggagcc tctccgccgc cgatgagctc tggccgccgc cgccgcagca 240

cgccagcgac gacccggccg agcaagcggc ggcggatgag gaggagcagg agcagcagcc 300

ggcggcgagg aggcagcggc gaggggagcg gaggacgctg taccggggca tccggcgcag 360

gccgtggggg aaatgggcgg cggagatccg cgacccggcc aagggcgccc gcgtctggct 420

cggcaccttc gccaccgccg aggctgccgc gcgggcctac gaccgcgccg cccgccgcat 480

ccgcggcacc aaggccaagg tcaacttccc caacgaggac aacgccttcg ccgccgcgcc 540

gccgccgtac cacctcgccg cctactacgg cgacgcctcc tccacctcct acctctaccc 600

gatggccatg acgcccgccg ccgccggact gagggagcag cagctgatga cgacgacggc 660

ggtggagtac agcgttaatg acgccgtcga cgtggccagc gtttacttcc agccgccgcc 720

gccggcggtt gcttacgagt tcagcgccgt cggcggtggc gccgtcgtcg tgccggtgtc 780

ggcggtggcg ccggcgatga cgtacggaca gagccaagag gtggcggctc cgctcatgtg 840

gaatttcgat gacatcacgg ccatgccaat gtgattgtct taccgtggag attaaggcat 900

ttaaaggctt atagataaca taaatttgcc atgtatgatc aatggttaat tcctattgct 960

caagggctta tattaagggt tacctacacg gcatggctag cttagcctac aatttatgtt 1020

tcttgcattt ctttctcttt ttttgagatg agctagtgtg ttatactatt tgttcatctt 1080

ttctggtagt ggtggataca ttttcgccgg ctagggttgg aatttgtaaa tagaagatgg 1140

tcaataataa gattgtttct ggct 1164

<210> 184
<211> 243
<212> PRT
<213> Oryza sativa

<400> 184

Met Cys Gly Gly Ala Ile Pro Leu Ile Ser Ser Arg Gly Pro Gly Gly
1 5 10 15

Lys Arg Ser Leu Ser Ala Ala Asp Glu Leu Trp Pro Pro Pro Pro Gln
20 25 30

His Ala Ser Asp Asp Pro Ala Glu Gln Ala Ala Ala Asp Glu Glu Glu
35 40 45

Gln Glu Gln Gln Pro Ala Ala Arg Arg Gln Arg Arg Gly Glu Arg Arg
50 55 60

Thr Leu Tyr Arg Gly Ile Arg Arg Arg Pro Trp Gly Lys Trp Ala Ala
65 70 75 80

Glu Ile Arg Asp Pro Ala Lys Gly Ala Arg Val Trp Leu Gly Thr Phe
85 90 95

Ala Thr Ala Glu Ala Ala Ala Arg Ala Tyr Asp Arg Ala Ala Arg Arg
100 105 110

Ile Arg Gly Thr Lys Ala Lys Val Asn Phe Pro Asn Glu Asp Asn Ala
115 120 125

Phe Ala Ala Ala Pro Pro Pro Tyr His Leu Ala Ala Tyr Tyr Gly Asp
130 135 140

Ala Ser Ser Thr Ser Tyr Leu Tyr Pro Met Ala Met Thr Pro Ala Ala
145 150 155 160

Ala Gly Leu Arg Glu Gln Gln Leu Met Thr Thr Thr Ala Val Glu Tyr
165 170 175

Ser Val Asn Asp Ala Val Asp Val Ala Ser Val Tyr Phe Gln Pro Pro
180 185 190

Pro Pro Ala Val Ala Tyr Glu Phe Ser Ala Val Gly Gly Gly Ala Val
195 200 205

Val Val Pro Val Ser Ala Val Ala Pro Ala Met Thr Tyr Gly Gln Ser
210 215 220

Gln Glu Val Ala Ala Pro Leu Met Trp Asn Phe Asp Asp Ile Thr Ala
225 230 235 240

Met Pro Met

<210> 185
<211> 1260
<212> DNA
<213> Oryza sativa

<400> 185
gtctcaaacc caatcaaact ccaaccaaac tcacctacct acccccaacc catccagagc 60

tagagctatg tgcggcggcg cgatcctcgc cgacctcata ccgtcgccgc gctccggcgg 120

ccacaccaaa aagaacaagc ggcggcggat cagcgacgac gaggacttcg aggccgcctt 180

cgaggagttc gacgccggcg acgacgactc cgactccgac tccgagtccg aggaggtaga 240

cgagtacgac gtcgtcgtcg acgacgacga cagcgaggac ggcgtggtgg ttcttccgcc 300

gccgccgccg ccgccgccgg tgattccaca tgagcgccat ggcgcgaggc ggttccgcgg 360

cgtgaggaag cggccgtggg ggaagtgggc ggcggagatc cgcgaccccg tgcgcggcgt 420

gcgcgtctgg ctcggtacct tccccaccgc cgagtccgcc gcgcgcgcct acgacgccgc 480

cgcccgccgc ctccgcggcg ccaaggccaa gcccaacttc ccctccgcgc cgccgccctc 540

ggctgctgct caccgccgca agaagcgccg cgcccacgcc gccacgcgct cgccgtcgtc 600

tccgcccgcc accagcgagg tcacggcggc gtccgcgtcc gcgtccagcg atgtccccgc 660

```
gccggcgttc gcttccttcg tcggcgagcc cgggcacggc ggcgccaagt cgatgccgac     720
gacgagccac acctcgcagc cagccccgcc ggcgacggtg gcgtccgaga acgtcgacga     780
cccggaggtg ttcgacccgt acgacgtcca cggcggcctc gcctcctact tcgccggcgg     840
cgcgtacgag tccctggaga gcctgttcgc gcacggcggc gacagcgccg ccgtcgacca     900
agcggcgagc gaccactggc cggcggcgct atggagcttc gcagacgacg gctcgttctg     960
cttctgatgc tgtcaaatca catcgccatt ggcggccatt gcaagccatg gcatggcacc    1020
tgatgatgct gatccagtga actggtcact cgttcttgat gcgttttcag tttcttgtac    1080
agtgtttttc cccagcaaca gtgaagtagt attcagttat tcactgttca gtcatgagtt    1140
catcgcaaaa tatgatgtaa gtatgtgtct tgagattgtt ttagcagtgg cttttgtttg    1200
tataatgtat ttctctgtaa ttaattaata gctgttttca gtgataaact aaattttctg    1260
```

```
<210>  186
<211>  322
<212>  PRT
<213>  Oryza sativa

<400>  186

Met Ser Gln Thr Gln Ser Asn Ser Asn Gln Thr His Leu Pro Thr Pro
1               5                   10                  15

Asn Pro Ser Arg Ala Arg Ala Met Cys Gly Gly Ala Ile Leu Ala Asp
            20                  25                  30

Leu Ile Pro Ser Pro Arg Ser Gly Gly His Thr Lys Lys Asn Lys Arg
        35                  40                  45

Arg Arg Ile Ser Asp Asp Glu Asp Phe Glu Ala Ala Phe Glu Glu Phe
    50                  55                  60

Asp Ala Gly Asp Asp Asp Ser Asp Ser Asp Ser Glu Ser Glu Glu Val
65                  70                  75                  80

Asp Glu Tyr Asp Val Val Val Asp Asp Asp Asp Ser Glu Asp Gly Val
                85                  90                  95

Val Val Leu Pro Pro Pro Pro Pro Pro Pro Pro Val Ile Pro His Glu
            100                 105                 110

Arg His Gly Ala Arg Arg Phe Arg Gly Val Arg Lys Arg Pro Trp Gly
        115                 120                 125

Lys Trp Ala Ala Glu Ile Arg Asp Pro Val Arg Gly Val Arg Val Trp
    130                 135                 140

Leu Gly Thr Phe Pro Thr Ala Glu Ser Ala Ala Arg Ala Tyr Asp Ala
```

145 150 155 160

Ala Ala Arg Arg Leu Arg Gly Ala Lys Ala Lys Pro Asn Phe Pro Ser
165 170 175

Ala Pro Pro Pro Ser Ala Ala Ala His Arg Arg Lys Lys Arg Arg Ala
180 185 190

His Ala Ala Thr Arg Ser Pro Ser Ser Pro Pro Ala Thr Ser Glu Val
195 200 205

Thr Ala Ala Ser Ala Ser Ala Ser Ser Asp Val Pro Ala Pro Ala Phe
210 215 220

Ala Ser Phe Val Gly Glu Pro Gly His Gly Gly Ala Lys Ser Met Pro
225 230 235 240

Thr Thr Ser His Thr Ser Gln Pro Ala Pro Pro Ala Thr Val Ala Ser
245 250 255

Glu Asn Val Asp Asp Pro Glu Val Phe Asp Pro Tyr Asp Val His Gly
260 265 270

Gly Leu Ala Ser Tyr Phe Ala Gly Gly Ala Tyr Glu Ser Leu Glu Ser
275 280 285

Leu Phe Ala His Gly Gly Asp Ser Ala Ala Val Asp Gln Ala Ala Ser
290 295 300

Asp His Trp Pro Ala Ala Leu Trp Ser Phe Ala Asp Asp Gly Ser Phe
305 310 315 320

Cys Phe

<210> 187
<211> 699
<212> DNA
<213> Oryza sativa

<400> 187
atgcgccgcc gcgtctcctc ctcctcctcc tcctcctcgt cctcgtcgcc ggcgaggcat 60

cacaaggcgc ggcgcagcag gaggaagctc gccgtcgacg aggactggga ggccgccttc 120

cgcgagttcc tctcccgcga ccacgacgac gacgacgacg accacgacgg tcagcatgtc 180

gttgttgcgc cgttgatccg tggtagtgac aagtgcgtcc acggccacga ggtggtggcg 240

tcgacggtcg gcggtggcgc aagcggcgga cgacgacgag ccgacgacga cgacggcgag 300

cggcggcggc ggcggcggag ggagaagcgg agctacccgt accgcggcat ccggcagcgg 360

ccgtggggga ggtgggcgtc ggagatccgc gaccccgtca agggcatccg cgtctggctc 420

ggcaccttcg acaccgccga gggcgccgcg cgcgcctacg acgacgaggt tcgccgcatc 480

tacggcggca acgccaagac caacttcccc ccatcgccgc ccacgccgcc gccgccggag 540

aagccagcgg cggagaggag cccctcgacg acgccgacga cgaccacgga ggactccggc 600

gactcgcgca tactcatcga gtgctgctcc gacgacctga tggacagcct cctcgccgcc 660

ttcgacatga ccaccggcga catgcgcttc tggagctaa 699

<210> 188
<211> 232
<212> PRT
<213> Oryza sativa

<400> 188

Met Arg Arg Arg Val Ser Ser Ser Ser Ser Ser Ser Ser Ser Ser Ser
1 5 10 15

Pro Ala Arg His His Lys Ala Arg Arg Ser Arg Arg Lys Leu Ala Val
20 25 30

Asp Glu Asp Trp Glu Ala Ala Phe Arg Glu Phe Leu Ser Arg Asp His
35 40 45

Asp Asp Asp Asp Asp Asp His Asp Gly Gln His Val Val Val Ala Pro
50 55 60

Leu Ile Arg Gly Ser Asp Lys Cys Val His Gly His Glu Val Val Ala
65 70 75 80

Ser Thr Val Gly Gly Gly Ala Ser Gly Gly Arg Arg Arg Ala Asp Asp
85 90 95

Asp Asp Gly Glu Arg Arg Arg Arg Arg Arg Arg Glu Lys Arg Ser Tyr
100 105 110

Pro Tyr Arg Gly Ile Arg Gln Arg Pro Trp Gly Arg Trp Ala Ser Glu
115 120 125

Ile Arg Asp Pro Val Lys Gly Ile Arg Val Trp Leu Gly Thr Phe Asp
130 135 140

Thr Ala Glu Gly Ala Ala Arg Ala Tyr Asp Asp Glu Val Arg Arg Ile
145 150 155 160

Tyr Gly Gly Asn Ala Lys Thr Asn Phe Pro Pro Ser Pro Pro Thr Pro
165 170 175

Pro Pro Pro Glu Lys Pro Ala Ala Glu Arg Ser Pro Ser Thr Thr Pro

180 185 190

Thr Thr Thr Thr Glu Asp Ser Gly Asp Ser Arg Ile Leu Ile Glu Cys
195 200 205

Cys Ser Asp Asp Leu Met Asp Ser Leu Leu Ala Ala Phe Asp Met Thr
210 215 220

Thr Gly Asp Met Arg Phe Trp Ser
225 230

<210> 189
<211> 1391
<212> DNA
<213> Lycopersicon esculentum

<400> 189
ttcaaattga gctttttctc cattaaaatt ctctctgcaa atttatagtt tttctttttt 60
cactttttga gaagaaatca aaagctatgt gtggtggtgc aattatctcc gatttggtac 120
ctcctagccg gatttctcgc cggttaaccg ctgattttct atggggtaca tccgatctga 180
acaagaagaa gaagaaccct agtaattacc actcaaagcc cttgaggtct aagtttattg 240
accttgaaga tgaatttgaa gctgactttc agcacttcaa ggataattct gatgatgatg 300
atgatgtgaa ggcatttggc cccaaatccg tgagatctgg tgattcaaac tgcgaagctg 360
acagatcctc caagagaaag aggaagaatc agtaccgggg gatcagacag cgtccttggg 420
gtaagtgggc agctgaaata cgtgatccaa ggaaaggtat tcgagtctgg cttggtactt 480
tcaattcagc cgaagaggca gccagagctt atgatgctga ggcgcgaagg atcagaggca 540
agaaagctaa ggtgaacttt cctgatgaag ctccagtgtc tgtttcaaga cgtgctatta 600
agcaaaatcc ccaaaaggca cttcgtgagg aaaccctgaa cacagttcag cccaacatga 660
cttatattag taacttggat ggtggatctg atgattcgtt cagtttttc gaagagaaac 720
cagcaaccaa gcagtacggc ttcgagaatg tgtcttttac tgctgtagat atgggactgg 780
gctcagtttc cccttcagct ggtacaaatg tttacttcag ctctgatgaa gcaagtaaca 840
cttttgactg ctctgatttc ggttgggctg aaccgtgtgc aaggactcca gagatctcat 900
ctgttctgtc ggaagttctg gaaaccaatg agactcattt tgatgatgat tccagaccag 960
agaaaaaact gaagtcctgt tccagcactt cattgacagt tgacggtaac actgtgaaca 1020
cgctatctga agagctatcg gcttttgaat cccagatgaa gttcttgcag atcccatatc 1080
tcgagggaaa ttgggatgca tcggttgatg ccttcctcaa tacaagtgca attcaggatg 1140
gtggaaacgc catggacctt tggtccttcg atgatgtacc ttctttaatg ggaggtgcct 1200
actaagctgc atacacatct tcccctgcta agttttgtaa ataacgcttc atttgagtga 1260
agtttgcgcc tgcgtttacg tttatcacca aactaaaaga ctatatatgt gttgtattaa 1320

tttattcaaa atttactcgt ttgatatatg taagtatgta tccttgtttt cataaaaaaa 1380

aaaaaaaaaa a 1391

<210> 190
<211> 372
<212> PRT
<213> Lycopersicon esculentum

<400> 190

Met Cys Gly Gly Ala Ile Ile Ser Asp Leu Val Pro Pro Ser Arg Ile
1 5 10 15

Ser Arg Arg Leu Thr Ala Asp Phe Leu Trp Gly Thr Ser Asp Leu Asn
20 25 30

Lys Lys Lys Lys Asn Pro Ser Asn Tyr His Ser Lys Pro Leu Arg Ser
35 40 45

Lys Phe Ile Asp Leu Glu Asp Glu Phe Glu Ala Asp Phe Gln His Phe
50 55 60

Lys Asp Asn Ser Asp Asp Asp Asp Asp Val Lys Ala Phe Gly Pro Lys
65 70 75 80

Ser Val Arg Ser Gly Asp Ser Asn Cys Glu Ala Asp Arg Ser Ser Lys
85 90 95

Arg Lys Arg Lys Asn Gln Tyr Arg Gly Ile Arg Gln Arg Pro Trp Gly
100 105 110

Lys Trp Ala Ala Glu Ile Arg Asp Pro Arg Lys Gly Ile Arg Val Trp
115 120 125

Leu Gly Thr Phe Asn Ser Ala Glu Glu Ala Ala Arg Ala Tyr Asp Ala
130 135 140

Glu Ala Arg Arg Ile Arg Gly Lys Lys Ala Lys Val Asn Phe Pro Asp
145 150 155 160

Glu Ala Pro Val Ser Val Ser Arg Arg Ala Ile Lys Gln Asn Pro Gln
165 170 175

Lys Ala Leu Arg Glu Glu Thr Leu Asn Thr Val Gln Pro Asn Met Thr
180 185 190

Tyr Ile Ser Asn Leu Asp Gly Gly Ser Asp Asp Ser Phe Ser Phe Phe
195 200 205

Glu Glu Lys Pro Ala Thr Lys Gln Tyr Gly Phe Glu Asn Val Ser Phe

210 215 220

Thr Ala Val Asp Met Gly Leu Gly Ser Val Ser Pro Ser Ala Gly Thr
225 230 235 240

Asn Val Tyr Phe Ser Ser Asp Glu Ala Ser Asn Thr Phe Asp Cys Ser
245 250 255

Asp Phe Gly Trp Ala Glu Pro Cys Ala Arg Thr Pro Glu Ile Ser Ser
260 265 270

Val Leu Ser Glu Val Leu Glu Thr Asn Glu Thr His Phe Asp Asp Asp
275 280 285

Ser Arg Pro Glu Lys Lys Leu Lys Ser Cys Ser Ser Thr Ser Leu Thr
290 295 300

Val Asp Gly Asn Thr Val Asn Thr Leu Ser Glu Glu Leu Ser Ala Phe
305 310 315 320

Glu Ser Gln Met Lys Phe Leu Gln Ile Pro Tyr Leu Glu Gly Asn Trp
325 330 335

Asp Ala Ser Val Asp Ala Phe Leu Asn Thr Ser Ala Ile Gln Asp Gly
340 345 350

Gly Asn Ala Met Asp Leu Trp Ser Phe Asp Asp Val Pro Ser Leu Met
355 360 365

Gly Gly Ala Tyr
370

<210> 191
<211> 1107
<212> DNA
<213> Oryza sativa

<400> 191
agatattcag acacacacct tcaataactt gcaaggataa ctcaaactac ttgaatcaga 60

tcagacaaaa acatcataag aatatcacga tgtgtggagg agcactgatc ccgaacgact 120

atggcgacaa gccgccgccg ccgccgtcgg agtcgtcgga gtgggacgcc acaacgaaga 180

tgaagaagaa gaagaagcgt ggtggcggcg gcgacgacga ctgggaggcc gccttccggg 240

agttcatcgc tggcgacgac gacgacgacg acggcggcgt ttccatgttc ccttctggtg 300

cagggacgat ggagacgacc acagaggtgg cgccggcggc ggcggtggtg gagaggccgc 360

ggcggcggcg aagggtgagg cggagctacc cgtaccgcgg cgtccggcag cggccgtggg 420

ggcggtgggc gtcggagatc cgcgaccccg tcaagggcgc ccgcgtctgg ctcggcacct 480

tcgacaccgc cgtcgaggcc gcgcgcgcct acgacgccga ggcgcgccgc atccacggcc 540

acaaggcaag gaccaacttc ccgcccgacg agcctccgct gccggcgcca tcgcaggcgc 600

cgttctgctt cctgctcgac gacgacgacg acgacgacgg cgtggcccgt ggaaacagcc 660

cggcgtcgtc gtcggcgccg gacagagcct ccgcttgcac gacgtcgtcg acggtggcgt 720

ccggcgagcg aggcgatgag ctcatactgc tggagtgctg ctccgacgac gtgatggaca 780

gcctcctcgc cggcttcgac gtgtccagcg aaccacgcag tgttttggga atggttaatt 840

agcagcgcgc acgcctgatt agatcggtac atgtgaagta caagagaagt agttactact 900

agctaggagc aaattaactt ggagtgcaag aataaaatat gcatgtctct gcactactac 960

tgtagtgtta gcaagtttgc tcattgttct gttgtatcct ttcatgtcca tttcagactt 1020

cccaatgcta cataaggatg tacatatgta tgatgttgtg tgccttgtta atcaatgaat 1080

gtaaatatct ttatattgct tttgtac 1107

<210> 192
<211> 250
<212> PRT
<213> Oryza sativa

<400> 192

Met Cys Gly Gly Ala Leu Ile Pro Asn Asp Tyr Gly Asp Lys Pro Pro
1               5                   10                  15

Pro Pro Pro Ser Glu Ser Ser Glu Trp Asp Ala Thr Thr Lys Met Lys
            20                  25                  30

Lys Lys Lys Lys Arg Gly Gly Gly Gly Asp Asp Asp Trp Glu Ala Ala
        35                  40                  45

Phe Arg Glu Phe Ile Ala Gly Asp Asp Asp Asp Asp Asp Gly Gly Val
    50                  55                  60

Ser Met Phe Pro Ser Gly Ala Gly Thr Met Glu Thr Thr Thr Glu Val
65                  70                  75                  80

Ala Pro Ala Ala Ala Val Val Glu Arg Pro Arg Arg Arg Arg Arg Val
                85                  90                  95

Arg Arg Ser Tyr Pro Tyr Arg Gly Val Arg Gln Arg Pro Trp Gly Arg
            100                 105                 110

Trp Ala Ser Glu Ile Arg Asp Pro Val Lys Gly Ala Arg Val Trp Leu
        115                 120                 125

Gly Thr Phe Asp Thr Ala Val Glu Ala Ala Arg Ala Tyr Asp Ala Glu
    130                 135                 140

Ala Arg Arg Ile His Gly His Lys Ala Arg Thr Asn Phe Pro Pro Asp
145 150 155 160

Glu Pro Pro Leu Pro Ala Pro Ser Gln Ala Pro Phe Cys Phe Leu Leu
165 170 175

Asp Asp Asp Asp Asp Asp Asp Gly Val Ala Arg Gly Asn Ser Pro Ala
180 185 190

Ser Ser Ser Ala Pro Asp Arg Ala Ser Ala Cys Thr Thr Ser Ser Thr
195 200 205

Val Ala Ser Gly Glu Arg Gly Asp Glu Leu Ile Leu Leu Glu Cys Cys
210 215 220

Ser Asp Asp Val Met Asp Ser Leu Leu Ala Gly Phe Asp Val Ser Ser
225 230 235 240

Glu Pro Arg Ser Val Leu Gly Met Val Asn
245 250

<210> 193
<211> 1552
<212> DNA
<213> Lycopersicon esculentum

<400> 193
gacaattata cattttccgt caaaacataa atcatgtgtg gtggttctat aatctccgat 60

tacatagacc ctagccggac ttctcgccgg ctcaccgccg agtttctatg ggtcgtttc 120

gatctcggta agaagcaaaa aaatcccaac aattatcact ctaaagctaa gcatttgcga 180

tctgaagttg ttgacgactt tgaagccgat tttcaggact tcaaagagtt atccgatgat 240

gaggatgttc aagtcgatgt caagccattt gccttctctg cttccaaaca ctctactggt 300

tccaaatctt tgaaaactgt tgattcagac aaggatgctg ctgctgataa atcctctaag 360

agaaagagga agaatcaata tagagggatc agacagagac cttggggtaa gtgggcagct 420

gaaatacgtg acccaaggaa aggggttcgg gtctggctgg gaaccttcaa tactgcagaa 480

gaagctgcca aagcttatga tattgaggcg aggaggatca gaggcaagaa ggctaaggta 540

aactttcctg atgaagctcc cgcccctgca tcaagacaca ctgttaaggt gaatcctcag 600

aaggtccttc ctgaggagag cctgtattca cttcagtccg actcagcaat catgaacagc 660

gtggaggatg accattatga ttcttttgga ttttttgaag agaaacccat gacaaaacag 720

tatggatatg agaatgggag cagtgcttct gcagatacgg gatttggttc gttcgtccct 780

tcagctggcg gtgatatcta cttcaactct gatgtaggaa gcaactcttt tgaatgctct 840

gattttggtt ggggagagcc atgctccagg actccagaga tatcatctgt tctgtcagct 900

```
gctattgaat gtaatgaagc tcaatttgtt gaagatgcca attctcagaa aaagttgaaa     960

tcatgcacca acaaccccgt agctgatgat ggaaaccccc gttactatgg tacctgaaga    1020

gcttccagct tttgaacctc agatgaattt ctttcatctc ccatatatgg agggaaattg    1080

ggatgcatca ggtggtaact tcctcaacac aagtgcaact caaaatggtg gtgaaaatgc    1140

tatggacctg tggtcctttg atgatgttcc ttctttaatg ggaggtatct tttaagtcaa    1200

catgccttga gttttgtaaa taaggcttca tgtgagtgat tttttgctgt tgtataatgt    1260

acttagtgca aatatttgat atgttaattt gatctccgtt aacttgttct tttaggtgtg    1320

tctggtggtg gaagaagaat gatccacaga gaaccatgat taagccatgg ataatgcact    1380

aagtagagca gtgcataggt aattaggttt aattaactac tagtagagat gttaaattcg    1440

agttttactt aaaaacaatt gggctgtatg gattatgaga attgatgaga cctcgatgct    1500

tgctataacg ttaccttttt agtgttgctt tcacaaaaaa aaaaaaaaaa aa            1552


<210>  194
<211>  327
<212>  PRT
<213>  Lycopersicon esculentum

<400>  194

Met Cys Gly Gly Ser Ile Ile Ser Asp Tyr Ile Asp Pro Ser Arg Thr
1               5                   10                  15


Ser Arg Arg Leu Thr Ala Glu Phe Leu Trp Gly Arg Phe Asp Leu Gly
            20                  25                  30


Lys Lys Gln Lys Asn Pro Asn Asn Tyr His Ser Lys Ala Lys His Leu
        35                  40                  45


Arg Ser Glu Val Val Asp Asp Phe Glu Ala Asp Phe Gln Asp Phe Lys
    50                  55                  60


Glu Leu Ser Asp Asp Glu Asp Val Gln Val Asp Val Lys Pro Phe Ala
65                  70                  75                  80


Phe Ser Ala Ser Lys His Ser Thr Gly Ser Lys Ser Leu Lys Thr Val
                85                  90                  95


Asp Ser Asp Lys Asp Ala Ala Ala Asp Lys Ser Ser Lys Arg Lys Arg
            100                 105                 110


Lys Asn Gln Tyr Arg Gly Ile Arg Gln Arg Pro Trp Gly Lys Trp Ala
        115                 120                 125


Ala Glu Ile Arg Asp Pro Arg Lys Gly Val Arg Val Trp Leu Gly Thr
    130                 135                 140
```

Phe Asn Thr Ala Glu Glu Ala Ala Lys Ala Tyr Asp Ile Glu Ala Arg
145 150 155 160

Arg Ile Arg Gly Lys Lys Ala Lys Val Asn Phe Pro Asp Glu Ala Pro
165 170 175

Ala Pro Ala Ser Arg His Thr Val Lys Val Asn Pro Gln Lys Val Leu
180 185 190

Pro Glu Glu Ser Leu Tyr Ser Leu Gln Ser Asp Ser Ala Ile Met Asn
195 200 205

Ser Val Glu Asp Asp His Tyr Asp Ser Phe Gly Phe Phe Glu Glu Lys
210 215 220

Pro Met Thr Lys Gln Tyr Gly Tyr Glu Asn Gly Ser Ser Ala Ser Ala
225 230 235 240

Asp Thr Gly Phe Gly Ser Phe Val Pro Ser Ala Gly Gly Asp Ile Tyr
245 250 255

Phe Asn Ser Asp Val Gly Ser Asn Ser Phe Glu Cys Ser Asp Phe Gly
260 265 270

Trp Gly Glu Pro Cys Ser Arg Thr Pro Glu Ile Ser Ser Val Leu Ser
275 280 285

Ala Ala Ile Glu Cys Asn Glu Ala Gln Phe Val Glu Asp Ala Asn Ser
290 295 300

Gln Lys Lys Leu Lys Ser Cys Thr Asn Asn Pro Val Ala Asp Asp Gly
305 310 315 320

Asn Pro Arg Tyr Tyr Gly Thr
325

<210> 195
<211> 993
<212> DNA
<213> Lycopersicon esculentum

<400> 195
tcatttctgt atcaatcaat attctttgtt tctgctgttt tgatgaaatc acactaagat 60

gtgtggtggt gcaattcttg ctgatatcat tcctcctcgt gaccgccgtt tgtcatccac 120

cgacctatgg ccgactgatt tctggccaat ttccacccaa aatgttcctc tcaaccccaa 180

acgagctcga ccctctacag gtggtgagca gatgaagaag aggcaaagga agaatcttta 240

cagagggata agacaacgtc catggggtaa atgggctgct gaaattcgtg acccgagaaa 300

```
aggggttagg gtttggttag gtactttcaa cactgctgaa gaagctgcaa gagcttatga     360
tagagaagct cgtaaaatca ggggtaagaa agctaaagtt aatttcccca atgaagatga     420
cgaccattac tgctacagtc atccagagcc ccctcccttg aacattgctt gtgatactac     480
tgttacttac aatcaagaat caaataactg ttaccccttt tactcaatcg agaacgttga     540
acctgttatg gaatttgcaa gttataatgg aattgaagat ggaggagagg agatggtgaa     600
aaatttgaat aacagggttg tagaggaaga ggagaaaaca gaggatgaag tgcagatact     660
ttctgatgag ctgatggctt atgagtcatt gatgaagttc tatgaaatac cgtatgttga     720
cgggcaatca gtggcggcga cggtgaatcc agcggcggag accgccgtgg gcggtggctc     780
gatggagctt tggagttttg atgatgttag tcgtctacaa ccaagttata atgtagttta     840
attattgttt tgtttaaact tttcataatt ttattttatc gaattaggaa gaattgagtt     900
tttataattt aatcaattgt gtaaaactat gtttctaatt cattaatatt atattggata     960
tgttgttttt aaaaaaaaaa aaaaaaaaaa aaa                                  993
```

```
<210>  196
<211>  260
<212>  PRT
<213>  Lycopersicon esculentum

<400>  196

Met Cys Gly Gly Ala Ile Leu Ala Asp Ile Ile Pro Pro Arg Asp Arg
1               5                   10                  15

Arg Leu Ser Ser Thr Asp Leu Trp Pro Thr Asp Phe Trp Pro Ile Ser
            20                  25                  30

Thr Gln Asn Val Pro Leu Asn Pro Lys Arg Ala Arg Pro Ser Thr Gly
        35                  40                  45

Gly Glu Gln Met Lys Lys Arg Gln Arg Lys Asn Leu Tyr Arg Gly Ile
    50                  55                  60

Arg Gln Arg Pro Trp Gly Lys Trp Ala Ala Glu Ile Arg Asp Pro Arg
65                  70                  75                  80

Lys Gly Val Arg Val Trp Leu Gly Thr Phe Asn Thr Ala Glu Glu Ala
                85                  90                  95

Ala Arg Ala Tyr Asp Arg Glu Ala Arg Lys Ile Arg Gly Lys Lys Ala
            100                 105                 110

Lys Val Asn Phe Pro Asn Glu Asp Asp Asp His Tyr Cys Tyr Ser His
        115                 120                 125

Pro Glu Pro Pro Pro Leu Asn Ile Ala Cys Asp Thr Thr Val Thr Tyr
```

130 135 140

Asn Gln Glu Ser Asn Asn Cys Tyr Pro Phe Tyr Ser Ile Glu Asn Val
145 150 155 160

Glu Pro Val Met Glu Phe Ala Ser Tyr Asn Gly Ile Glu Asp Gly Gly
165 170 175

Glu Glu Met Val Lys Asn Leu Asn Asn Arg Val Val Glu Glu Glu Glu
180 185 190

Lys Thr Glu Asp Glu Val Gln Ile Leu Ser Asp Glu Leu Met Ala Tyr
195 200 205

Glu Ser Leu Met Lys Phe Tyr Glu Ile Pro Tyr Val Asp Gly Gln Ser
210 215 220

Val Ala Ala Thr Val Asn Pro Ala Ala Glu Thr Ala Val Gly Gly Gly
225 230 235 240

Ser Met Glu Leu Trp Ser Phe Asp Asp Val Ser Arg Leu Gln Pro Ser
245 250 255

Tyr Asn Val Val
260

<210> 197
<211> 771
<212> DNA
<213> Arabidopsis thaliana

<400> 197
atggaaaaca gctacaccgt tgatggtcac cgtcttcaat attccgttcc gttaagctcc 60

atgcatgaaa ccagtcaaaa ctccgaaact tacggattat ccaaagagtc gccgttggtc 120

tgcatgccct tgttcgaaac caacactact tcattcgata tctcttctct ttctcgtttt 180

aacccaaaac cagaacccga aaacacgcat cgtgtcatgg acgattccat cgccgccgtc 240

gtgggcgaaa acgttctttt cggtgataaa aacaaagtct ctgatcactt gaccaaagaa 300

ggtggtgtga agcgggggcg gaagatgccg cagaagaccg gaggattcat gggagtgaga 360

aaacggccgt gggggagatg gtcggcggag ataagagaca ggatagggcg gtgcagacac 420

tggttaggaa cgttcgacac ggcggaagag gcagcgcgtg cgtatgacgc ggcggcgagg 480

aggcttagag ggaccaaagc caagaccaat ttcgtgattc ctccgctttt tcccaaggaa 540

atagctcagg ctcaggagga taataggatg aggcagaagc agaagaagaa gaagaagaaa 600

aaagtgagtg tgaggaagtg tgttaaagtc acatcggttg cacagttgtt cgatgatgcc 660

aattttataa attcttctag tattaaagga aatgtgatta gttctattga taatcttgaa 720

aaaatgggtc tagagcttga tttgagttta gggttgttgt ctaggaagtg a 771

<210> 198
<211> 256
<212> PRT
<213> Arabidopsis thaliana

<400> 198

Met Glu Asn Ser Tyr Thr Val Asp Gly His Arg Leu Gln Tyr Ser Val
1 5 10 15

Pro Leu Ser Ser Met His Glu Thr Ser Gln Asn Ser Glu Thr Tyr Gly
20 25 30

Leu Ser Lys Glu Ser Pro Leu Val Cys Met Pro Leu Phe Glu Thr Asn
35 40 45

Thr Thr Ser Phe Asp Ile Ser Ser Leu Phe Ser Phe Asn Pro Lys Pro
50 55 60

Glu Pro Glu Asn Thr His Arg Val Met Asp Asp Ser Ile Ala Ala Val
65 70 75 80

Val Gly Glu Asn Val Leu Phe Gly Asp Lys Asn Lys Val Ser Asp His
85 90 95

Leu Thr Lys Glu Gly Gly Val Lys Arg Gly Arg Lys Met Pro Gln Lys
100 105 110

Thr Gly Gly Phe Met Gly Val Arg Lys Arg Pro Trp Gly Arg Trp Ser
115 120 125

Ala Glu Ile Arg Asp Arg Ile Gly Arg Cys Arg His Trp Leu Gly Thr
130 135 140

Phe Asp Thr Ala Glu Glu Ala Ala Arg Ala Tyr Asp Ala Ala Ala Arg
145 150 155 160

Arg Leu Arg Gly Thr Lys Ala Lys Thr Asn Phe Val Ile Pro Pro Leu
165 170 175

Phe Pro Lys Glu Ile Ala Gln Ala Gln Glu Asp Asn Arg Met Arg Gln
180 185 190

Lys Gln Lys Lys Lys Lys Lys Lys Lys Val Ser Val Arg Lys Cys Val
195 200 205

Lys Val Thr Ser Val Ala Gln Leu Phe Asp Asp Ala Asn Phe Ile Asn
210 215 220

```
Ser Ser Ser Ile Lys Gly Asn Val Ile Ser Ser Ile Asp Asn Leu Glu
225                 230                 235                 240

Lys Met Gly Leu Glu Leu Asp Leu Ser Leu Gly Leu Leu Ser Arg Lys
                245                 250                 255
```

<210> 199
<211> 1413
<212> DNA
<213> Nicotiana tabacum

<400> 199

```
gaattcggca cgagaaaaaa gaaagaagtt tactccgtca aaaacgaaac tgatttctgc      60
ataaaacttt tctgctgaga gaaaacaaaa agcatgtgtg gtggtgctat aatctccgat     120
tacattgccc cgagccgaac ttctcgccgg ctcaccgccg agttgctatg gggccggtcc     180
gatctgagta ataagcaaaa aaatcctaac aattatcact ccaagccgtt gagatcccaa     240
gtagttgacc tagacgatga cttcgaggct gattttcagg actttaaaga tttctccgat     300
gacgaggatg ttcaagtcga tgtcaagcca tttgccttct ctgcttcgaa aaactctaat     360
gttgaaggct ccaaatctgt gaaaactgat gattcagaca aggatgctga tagatcctct     420
aagagaaaga ggaagaatca gtataggggg atcagacagc gaccttgggg taagtgggca     480
gctgaaatac gtgacccaag aaaagggtt cgggtgtggc tgggaacttt caatactgca     540
gaagaagctg ccagagctta tgatgttgag gctaggagga tcagaggcaa taaagctaag     600
gtaaactttc ccgatgaagc tccagtgcct gcctcgagac gtactgttaa ggtgaatcct     660
caaaaggtcc ttcctaagga gatcctggac tcggttcagc ccgactcgac tatcataaac     720
aacatggagg attgctgtta tgattctttg ggatttcttg aagagaaacc catgacgaag     780
cagtttggat gtgaggatgg gagcagtgct tctggagata cgggatttgg ctcatttgcc     840
ccttcagctg gtaccgatat ctacttcaac tctgatgttg gaagtaactc ttttgactgc     900
tctgattttg gttggggaga gccatgtgcc aggactccag agatatcatc cgttctgtca     960
gctgttattg aaagcaatga atctcaactt gttgaagatg ataccagtcc aatgaaaaaa    1020
ctgaaatcaa gccccattaa tccagtagct gatgatggaa ataccgcaaa caagctatct    1080
gaagagcttt cagcttttga aacccagatg aagttccttc agatccccta tctggaggga    1140
aattgggatg catcagttga tactttcctc aactcaagtg caactcagga tggtgataat    1200
gctatggact tatggtcctt tgatgatgtt ccttctttat tgggaggtgt cttttaagtc    1260
agcatgcctt gtctagtttt tgtaaataag gcttcatgtg agtgaacttt gctattgttt    1320
tgcctcaaag aaaggctctt tattatgtac agaagctttt tgaaatggta aatagtttaa    1380
tctctgttta aaaaaaaaaa aaaaaaaaaa aaa                                 1413
```

<210> 200

<211> 387
<212> PRT
<213> Nicotiana tabacum

<400> 200

```
Met Cys Gly Gly Ala Ile Ile Ser Asp Tyr Ile Ala Pro Ser Arg Thr
1               5                   10                  15

Ser Arg Arg Leu Thr Ala Glu Leu Leu Trp Gly Arg Ser Asp Leu Ser
            20                  25                  30

Asn Lys Gln Lys Asn Pro Asn Asn Tyr His Ser Lys Pro Leu Arg Ser
        35                  40                  45

Gln Val Val Asp Leu Asp Asp Asp Phe Glu Ala Asp Phe Gln Asp Phe
    50                  55                  60

Lys Asp Phe Ser Asp Asp Glu Asp Val Gln Val Asp Val Lys Pro Phe
65                  70                  75                  80

Ala Phe Ser Ala Ser Lys Asn Ser Asn Val Glu Gly Ser Lys Ser Val
                85                  90                  95

Lys Thr Asp Asp Ser Asp Lys Asp Ala Asp Arg Ser Ser Lys Arg Lys
            100                 105                 110

Arg Lys Asn Gln Tyr Arg Gly Ile Arg Gln Arg Pro Trp Gly Lys Trp
        115                 120                 125

Ala Ala Glu Ile Arg Asp Pro Arg Lys Gly Val Arg Val Trp Leu Gly
    130                 135                 140

Thr Phe Asn Thr Ala Glu Glu Ala Ala Arg Ala Tyr Asp Val Glu Ala
145                 150                 155                 160

Arg Arg Ile Arg Gly Asn Lys Ala Lys Val Asn Phe Pro Asp Glu Ala
                165                 170                 175

Pro Val Pro Ala Ser Arg Arg Thr Val Lys Val Asn Pro Gln Lys Val
            180                 185                 190

Leu Pro Lys Glu Ile Leu Asp Ser Val Gln Pro Asp Ser Thr Ile Ile
        195                 200                 205

Asn Asn Met Glu Asp Cys Cys Tyr Asp Ser Leu Gly Phe Leu Glu Glu
    210                 215                 220

Lys Pro Met Thr Lys Gln Phe Gly Cys Glu Asp Gly Ser Ser Ala Ser
225                 230                 235                 240
```

Gly Asp Thr Gly Phe Gly Ser Phe Ala Pro Ser Ala Gly Thr Asp Ile
245 250 255

Tyr Phe Asn Ser Asp Val Gly Ser Asn Ser Phe Asp Cys Ser Asp Phe
260 265 270

Gly Trp Gly Glu Pro Cys Ala Arg Thr Pro Glu Ile Ser Ser Val Leu
275 280 285

Ser Ala Val Ile Glu Ser Asn Glu Ser Gln Leu Val Glu Asp Asp Thr
290 295 300

Ser Pro Met Lys Lys Leu Lys Ser Ser Pro Ile Asn Pro Val Ala Asp
305 310 315 320

Asp Gly Asn Thr Ala Asn Lys Leu Ser Glu Glu Leu Ser Ala Phe Glu
325 330 335

Thr Gln Met Lys Phe Leu Gln Ile Pro Tyr Leu Glu Gly Asn Trp Asp
340 345 350

Ala Ser Val Asp Thr Phe Leu Asn Ser Ser Ala Thr Gln Asp Gly Asp
355 360 365

Asn Ala Met Asp Leu Trp Ser Phe Asp Asp Val Pro Ser Leu Leu Gly
370 375 380

Gly Val Phe
385

<210> 201
<211> 1605
<212> DNA
<213> Fagus sylvatica

<400> 201
caaacacact gaagaattaa gtcatttggg aatcaggttt cttggaaaaa cccctgccaa 60

agccccttca aggctctcag ctttgagtcc ccagatgtgt ggaggagcta taatctccga 120

ctttatagcg ccaaccgggt cgcggcggtt gacggcggat tatctctggg gcgatcggaa 180

aaaacccatt tcaggaaagc gattctcgaa gcctgtagtc gatttggacg acgaattcga 240

gctcgatttt cagggcttta aggacgagga ggagtctgat atcgacgagg aagaggtcct 300

tgtgcaagat gtcaagccct tcactttttc tgctcctcct agctctggat ctaagcctgt 360

aaaatccgtg gaattcaatg ggcaagctga gaaatctgca aagagaaaga ggaagaatca 420

gtatcggggg atccggcagc gcccatgggg taagtgggct gctgagattc gagacccaag 480

gaaaggggtc cgtgtctggc ttggaacttt taacactgca gaaaaagctg caagagctta 540

tgatgcagag gcacggagaa ttcgtggcaa gaaggctaag gtgaattttc ccgatgagac 600

tccccgtgct tctccaaagc gttcagttaa ggcaaatctg cagaagccac ttgccaaggc 660

aaacctgaac tctgtccagc ccaacctgaa ccaaaatttc aattttatga acaactctga 720

tcaggactat accatgggtt tgatggaaga gaaacctttc acaaaccagt atgggtatat 780

ggattccatc cctgccaatg cagatgttgg actaaaaccc tttgcttcca ataatactac 840

cccgtacttt aactcagatc aggggagtaa ctcgtttgat tgttctgact atggatgggg 900

agaacagggc tctaagactc cagaaatctc atctgttctt tcagctactt tagaagggga 960

tgaatctcag tttgtggagg atgctatgcc cacgaagaaa ttgaagtcag actctgggaa 1020

tgcagtgttc attgaaaata acactgcaaa gacactgtca gaggagctct cagcttttga 1080

gtcccagatg aactttcaga tgccatttct tgagggaagc tgggaatcca acatggaggc 1140

actgttcagt ggggacacaa ctcaggatgg taactcgatg gatctttgga gcttcgatga 1200

cctccccgtt atggctgggg gagttctgtg accgcaaaac tattttccgc atgcttgctg 1260

ttctagttta tgtataaata aggctaaata catgttagaa tggtttgtca ttctgtggag 1320

atggacatgc ctgtggtttc aaacaagctg aacactgaat gcttaagact ctatgaaggg 1380

atgtactgaa gtagtgttgt tctactgttg tatgagggag tacataggtc cctatttagg 1440

atccctttga gagactacct tgaagacatt gaattttggg attttgaatt tgatgttttt 1500

gtattgatga ttatgtgtga tgaaacctct gtcataaaaa tactaaacta aaaacctgat 1560

gtatgtcaac tgtgtttagt gtttgtttca aaaaaaaaaa aaaaa 1605

<210> 202
<211> 378
<212> PRT
<213> Fagus sylvatica

<400> 202

Met Cys Gly Gly Ala Ile Ile Ser Asp Phe Ile Ala Pro Thr Gly Ser
1 5 10 15

Arg Arg Leu Thr Ala Asp Tyr Leu Trp Gly Asp Arg Lys Lys Pro Ile
20 25 30

Ser Gly Lys Arg Phe Ser Lys Pro Val Val Asp Leu Asp Asp Glu Phe
35 40 45

Glu Leu Asp Phe Gln Gly Phe Lys Asp Glu Glu Glu Ser Asp Ile Asp
50 55 60

Glu Glu Glu Val Leu Val Gln Asp Val Lys Pro Phe Thr Phe Ser Ala
65 70 75 80

Pro Pro Ser Ser Gly Ser Lys Pro Val Lys Ser Val Glu Phe Asn Gly

85 90 95

Gln Ala Glu Lys Ser Ala Lys Arg Lys Arg Lys Asn Gln Tyr Arg Gly
100 105 110

Ile Arg Gln Arg Pro Trp Gly Lys Trp Ala Ala Glu Ile Arg Asp Pro
115 120 125

Arg Lys Gly Val Arg Val Trp Leu Gly Thr Phe Asn Thr Ala Glu Lys
130 135 140

Ala Ala Arg Ala Tyr Asp Ala Glu Ala Arg Arg Ile Arg Gly Lys Lys
145 150 155 160

Ala Lys Val Asn Phe Pro Asp Glu Thr Pro Arg Ala Ser Pro Lys Arg
165 170 175

Ser Val Lys Ala Asn Leu Gln Lys Pro Leu Ala Lys Ala Asn Leu Asn
180 185 190

Ser Val Gln Pro Asn Leu Asn Gln Asn Phe Asn Phe Met Asn Asn Ser
195 200 205

Asp Gln Asp Tyr Thr Met Gly Leu Met Glu Glu Lys Pro Phe Thr Asn
210 215 220

Gln Tyr Gly Tyr Met Asp Ser Ile Pro Ala Asn Ala Asp Val Gly Leu
225 230 235 240

Lys Pro Phe Ala Ser Asn Asn Thr Thr Pro Tyr Phe Asn Ser Asp Gln
245 250 255

Gly Ser Asn Ser Phe Asp Cys Ser Asp Tyr Gly Trp Gly Glu Gln Gly
260 265 270

Ser Lys Thr Pro Glu Ile Ser Ser Val Leu Ser Ala Thr Leu Glu Gly
275 280 285

Asp Glu Ser Gln Phe Val Glu Asp Ala Met Pro Thr Lys Lys Leu Lys
290 295 300

Ser Asp Ser Gly Asn Ala Val Phe Ile Glu Asn Asn Thr Ala Lys Thr
305 310 315 320

Leu Ser Glu Glu Leu Ser Ala Phe Glu Ser Gln Met Asn Phe Gln Met
325 330 335

Pro Phe Leu Glu Gly Ser Trp Glu Ser Asn Met Glu Ala Leu Phe Ser
340 345 350

Gly Asp Thr Thr Gln Asp Gly Asn Ser Met Asp Leu Trp Ser Phe Asp
355 360 365

Asp Leu Pro Val Met Ala Gly Gly Val Leu
370 375

<210> 203
<211> 1653
<212> DNA
<213> Fagus sylvatica

<400> 203
caacaatatt ttctctcaca aaaactaaac tctctgtatt tcccaaactt tccaaaaaaa 60

ccattttact tttcacccat ccgagcaaaa aaaacaaaga gaaaaaagaa acccctccaa 120

aaaagccaag accgccatgt gtggaggagc tataatctcc gactttatag cgccaaccgg 180

gtcgcggcgg ttgacggcgg attatctctg gggcgatcgg aaaaaaccca tttcaggaaa 240

gcgattctcg aagcctgtag tcgatttgga cgacgaattc gagctcgatt ttcagggctt 300

caaggacgag gaggagtctg atatcgacga ggaagaggtc cttgtgcaag atgtcaagcc 360

cttcactttt tctgctcctc ctagctctgg atctaagcct gtaaaatccg tggaattcaa 420

tgggcaagct gagaaatctg caaagagaaa gaggaagaat cagtatcggg ggatccggca 480

gcgcccatgg ggtaagtggg ctgctgagat tcgagaccca aggaaagggg tccgtgtctg 540

gctcggaact tttaacactg cagaagaagc tgcaagagct tatgatgcag aagcacggag 600

aattcgtggc aagaaaagca aagggaattt tccgatgaga cttcccgtgc caaagcgttc 660

agttaaggca aatctgcaga agccacttgc caaggcaaac ctgaactctg tccagcccaa 720

cctgaaccaa aatttcaatt ttatgaactc tgatcaggac tataccatgg gtttgatgga 780

agagaaacct ttcacgaacc agtatgggta tatggattcc atccctgtca atgcagatgt 840

tggactaaaa tcctttgctt ccaataatac tgccccatac tttaactcag atcaggggag 900

taactcgttt gattgttctg actatggatg gggagaacag ggctctaaga ctccagaaat 960

ctcatctgtt ctttcagcca ctttagaagg ggatgaatct cagtttgtgg aggatgctgt 1020

gcccacgaag aaattgaagt cagactctgg gaatgcagtg ttcattgaaa ataacactgc 1080

aaagacactg tcagaggagc tctcagcttt tgagtcccag atgaactttc agatgccatt 1140

tcttgaggga agctgggaat ccaacatgga ggcactgttc agtggggaca caactcagga 1200

tggtaactcg atggatcttt ggagcttcga tgacctcccc gttatggctg ggggagttct 1260

gtgagcaaac tattcccatg cttgctagtt tatgtaaata aggctacatg ttagaatggt 1320

ttgtcatctg tgatggacat gccgtttcaa acaagctgtg aacatgctta agactcttat 1380

gaaggatgta ctgaagtagt ctactgttgt atgaggagta cataggtatt taggatccct 1440

ttctcccttg aagacattga atttgggatt ttgaatttga tctttgtatt gatgattatg 1500

tgtgatctgt cataaaaata ctaaaaacct gatgtatgtc aactttagtg tttgtttcta 1560

tcttggttct tttacaaaaa gggccttcaa tttttgtact gtttatttgt ttttattggt 1620

ttttgatttt acctaaaaaa aaaaaaaaaa aaa 1653

<210> 204
<211> 375
<212> PRT
<213> Fagus sylvatica

<400> 204

Met Cys Gly Gly Ala Ile Ile Ser Asp Phe Ile Ala Pro Thr Gly Ser
1 5 10 15

Arg Arg Leu Thr Ala Asp Tyr Leu Trp Gly Asp Arg Lys Lys Pro Ile
20 25 30

Ser Gly Lys Arg Phe Ser Lys Pro Val Val Asp Leu Asp Asp Glu Phe
35 40 45

Glu Leu Asp Phe Gln Gly Phe Lys Asp Glu Glu Glu Ser Asp Ile Asp
50 55 60

Glu Glu Glu Val Leu Val Gln Asp Val Lys Pro Phe Thr Phe Ser Ala
65 70 75 80

Pro Pro Ser Ser Gly Ser Lys Pro Val Lys Ser Val Glu Phe Asn Gly
85 90 95

Gln Ala Glu Lys Ser Ala Lys Arg Lys Arg Lys Asn Gln Tyr Arg Gly
100 105 110

Ile Arg Gln Arg Pro Trp Gly Lys Trp Ala Ala Glu Ile Arg Asp Pro
115 120 125

Arg Lys Gly Val Arg Val Trp Leu Gly Thr Phe Asn Thr Ala Glu Glu
130 135 140

Ala Ala Arg Ala Tyr Asp Ala Glu Ala Arg Arg Ile Arg Gly Lys Lys
145 150 155 160

Ser Lys Gly Asn Phe Pro Met Arg Leu Pro Val Pro Lys Arg Ser Val
165 170 175

Lys Ala Asn Leu Gln Lys Pro Leu Ala Lys Ala Asn Leu Asn Ser Val
180 185 190

Gln Pro Asn Leu Asn Gln Asn Phe Asn Phe Met Asn Ser Asp Gln Asp
195 200 205

```
Tyr Thr Met Gly Leu Met Glu Glu Lys Pro Phe Thr Asn Gln Tyr Gly
    210                 215                 220

Tyr Met Asp Ser Ile Pro Val Asn Ala Asp Val Gly Leu Lys Ser Phe
225                 230                 235                 240

Ala Ser Asn Asn Thr Ala Pro Tyr Phe Asn Ser Asp Gln Gly Ser Asn
                245                 250                 255

Ser Phe Asp Cys Ser Asp Tyr Gly Trp Gly Glu Gln Gly Ser Lys Thr
            260                 265                 270

Pro Glu Ile Ser Ser Val Leu Ser Ala Thr Leu Glu Gly Asp Glu Ser
        275                 280                 285

Gln Phe Val Glu Asp Ala Val Pro Thr Lys Lys Leu Lys Ser Asp Ser
    290                 295                 300

Gly Asn Ala Val Phe Ile Glu Asn Asn Thr Ala Lys Thr Leu Ser Glu
305                 310                 315                 320

Glu Leu Ser Ala Phe Glu Ser Gln Met Asn Phe Gln Met Pro Phe Leu
                325                 330                 335

Glu Gly Ser Trp Glu Ser Asn Met Glu Ala Leu Phe Ser Gly Asp Thr
            340                 345                 350

Thr Gln Asp Gly Asn Ser Met Asp Leu Trp Ser Phe Asp Asp Leu Pro
        355                 360                 365

Val Met Ala Gly Gly Val Leu
    370                 375
```

<210> 205
<211> 1379
<212> DNA
<213> Capsicum annuum

<400> 205

```
acgcggggaa attaaacttt ctccattgaa attcactgcg aaaaaaaaac cctttcccag     60

ttataataca ctacttaaat tattagagaa aagaaaaagc tatgtgtggt ggtgcaatta    120

tctccgattt ggtacctcct agccggattt cccgccggct aaccgccgag ttgctatggg    180

gtaactctga tctgagcaaa aagaagaaaa atccagggaa ttattactca aagcctttga    240

acaggtctaa gtttattgac cttgatgagg aatttgaagc tgactttcag gacttcaagg    300

actatgccga tgacgatgtt gatgatgtta agcccttcgg ttccaaatct gtgaaatctg    360

gcgattcaag ctgcgatact gaaaaatctt ccaagagaaa gaggaagaat cagtaccggg    420
```

```
ggatcagaca gcgtccttgg ggtaagtggg cagctgaaat tcgtgatccg aggaaaggga     480
ttcgagtttg gcttggaact ttcaattctg cggaagaagc agctagagct tatgatgttg     540
aggcacgaag gatcagaggc aagaaggcta aggtgaactt tcctgatgga tctccagctt     600
ctgcttcaag acgtgctgtt aagccaaatc ctcaggaggc acttcgcgag gaaatcttga     660
acacagttca gccgaacaca acttatatca acaacttgga cggcggatct gatgattcgt     720
ttggcttttt cgaagagaaa ccagcagcaa agcagtatgg ctatgagaat gtttctttta     780
ctgctggaga tatgggactg ggttcaattt cccctttcaac tggtacaaca aatgtttact     840
tcagttctga tgaaggaagc aacacctttg actgctctga tttcggttgg ggtgaaccat     900
gtccgaggac tccagagatc tcatctgttc tgtcagaagt tctagaatgt aatggtactc     960
aatctgatga agatgctaga ccagagaaaa aactgaagtc gtgttccaac gcttccttgc    1020
cagatgagga taacactgtg cacacgctat ctgaagagct atcggctttt gaatcccaga    1080
tgaagttctt gcagatccca tatcttgagg gaaattggga tgcatcagtt gatgcctttg    1140
tcaacacagg cgcaattcag gatggcggaa atgcgatgga tctctggacc ttcgatgatg    1200
ttccttcttt aatgggaggt gtctactaag ccaacacgca ccttccctta ctaagttttg    1260
taaataaagc ttcatttgag tgaagtttgc agttatgttg tctccaaaca aaaaagacta    1320
tatatgtgtt gtattaaatt tatttcataa atttacttgt ttgatacaaa aaaaaaaaa     1379
```

```
<210>  206
<211>  375
<212>  PRT
<213>  Capsicum annuum

<400>  206

Met Cys Gly Gly Ala Ile Ile Ser Asp Leu Val Pro Pro Ser Arg Ile
1               5                   10                  15

Ser Arg Arg Leu Thr Ala Glu Leu Leu Trp Gly Asn Ser Asp Leu Ser
            20                  25                  30

Lys Lys Lys Lys Asn Pro Gly Asn Tyr Tyr Ser Lys Pro Leu Asn Arg
        35                  40                  45

Ser Lys Phe Ile Asp Leu Asp Glu Glu Phe Glu Ala Asp Phe Gln Asp
    50                  55                  60

Phe Lys Asp Tyr Ala Asp Asp Asp Val Asp Asp Val Lys Pro Phe Gly
65                  70                  75                  80

Ser Lys Ser Val Lys Ser Gly Asp Ser Ser Cys Asp Thr Glu Lys Ser
                85                  90                  95
```

```
Ser Lys Arg Lys Arg Lys Asn Gln Tyr Arg Gly Ile Arg Gln Arg Pro
            100                 105                 110

Trp Gly Lys Trp Ala Ala Glu Ile Arg Asp Pro Arg Lys Gly Ile Arg
        115                 120                 125

Val Trp Leu Gly Thr Phe Asn Ser Ala Glu Glu Ala Ala Arg Ala Tyr
    130                 135                 140

Asp Val Glu Ala Arg Arg Ile Arg Gly Lys Lys Ala Lys Val Asn Phe
145                 150                 155                 160

Pro Asp Gly Ser Pro Ala Ser Ala Ser Arg Arg Ala Val Lys Pro Asn
                165                 170                 175

Pro Gln Glu Ala Leu Arg Glu Glu Ile Leu Asn Thr Val Gln Pro Asn
            180                 185                 190

Thr Thr Tyr Ile Asn Asn Leu Asp Gly Gly Ser Asp Asp Ser Phe Gly
        195                 200                 205

Phe Phe Glu Glu Lys Pro Ala Ala Lys Gln Tyr Gly Tyr Glu Asn Val
    210                 215                 220

Ser Phe Thr Ala Gly Asp Met Gly Leu Gly Ser Ile Ser Pro Ser Thr
225                 230                 235                 240

Gly Thr Thr Asn Val Tyr Phe Ser Ser Asp Glu Gly Ser Asn Thr Phe
                245                 250                 255

Asp Cys Ser Asp Phe Gly Trp Gly Glu Pro Cys Pro Arg Thr Pro Glu
            260                 265                 270

Ile Ser Ser Val Leu Ser Glu Val Leu Glu Cys Asn Gly Thr Gln Ser
        275                 280                 285

Asp Glu Asp Ala Arg Pro Glu Lys Lys Leu Lys Ser Cys Ser Asn Ala
    290                 295                 300

Ser Leu Pro Asp Glu Asp Asn Thr Val His Thr Leu Ser Glu Glu Leu
305                 310                 315                 320

Ser Ala Phe Glu Ser Gln Met Lys Phe Leu Gln Ile Pro Tyr Leu Glu
                325                 330                 335

Gly Asn Trp Asp Ala Ser Val Asp Ala Phe Val Asn Thr Gly Ala Ile
            340                 345                 350

Gln Asp Gly Gly Asn Ala Met Asp Leu Trp Thr Phe Asp Asp Val Pro
```

355 360 365

Ser Leu Met Gly Gly Val Tyr
370 375

<210> 207
<211> 1329
<212> DNA
<213> Capsicum annuum

<400> 207
agttataata cactacttaa attattagag aaaagaaaaa gctatgtgtg gtggtgcaat 60

tatctccgat ttggtacctc ctagccggat ttcccgccgg ctaaccgccg agttgctatg 120

gggtaactct gatctgagca aaaagaagaa aaatccaggg aattattact caaagccttt 180

gaacaggtct aagtttattg accttgatga ggaatttgaa gctgactttc aggacttcaa 240

ggactatgcc gatgacgatg ttgatgatgt taagcccttc ggttccaaat ctgtgaaatc 300

tggcgattca agctgcgata ctgaaaaatc ttccaagaga aagaggaaga atcagtaccg 360

ggggatcaga cagcgtcctt ggggtaagtg ggcagctgaa attcgtgatc cgaggaaagg 420

gattcgagtt tggcttggaa ctttcaattc tgcggaagaa gcagctagag cttatgatgt 480

tgaggcacga aggatcagag gcaagaaggc taaggtgaac tttcctgatg gatctccagc 540

ttctgcttca agacgtgctg ttaagccaaa tcctcaggag gcacttcgcg aggaaatctt 600

gaacacagtt cagccgaaca caacttatat caacaacttg gacggcggat ctgatgattc 660

gtttggcttt ttcgaagaga aaccagcagc aaagcagtat ggctatgaga atgtttcttt 720

tactgctgga gatatgggac tgggttcaat ttccccttca actggtacaa caaatgttta 780

cttcagttct gatgaaggaa gcaacacctt tgactgctct gatttcggtt ggggtgaacc 840

atgtccgagg actccagaga tctcatctgt tctgtcagaa gttctagaat gtaatggtac 900

tcaatctgat gaagatgcta gaccagagaa aaaactgaag tcgtgttcca acgcttcctt 960

gccagatgag gataacactg tgcacacgct atctgaagag ctatcggctt ttgaatccca 1020

gatgaagttc ttgcagatcc catatcttga gggaaattgg gatgcatcag ttgatgcctt 1080

tgtcaacaca ggcgcaattc aggatggcgg aaatgcgatg gatctctggc cttcgatgat 1140

gttccttctt taatgggagg tgtctataag ccaacacgca ccttccctta ttaagttttg 1200

taaataaagc ttcatttgag tgaagtttgc agttatgttg tctccaaaca aaaaagacta 1260

tatatgtgtt gtattaaatt tatttcataa atttacttgt ttgatgtaaa aaaaaaaaaa 1320

aaaaaaaaa 1329

<210> 208
<211> 369
<212> PRT
<213> Capsicum annuum

<400> 208

```
Met Cys Gly Gly Ala Ile Ile Ser Asp Leu Val Pro Pro Ser Arg Ile
1               5                   10                  15

Ser Arg Arg Leu Thr Ala Glu Leu Leu Trp Gly Asn Ser Asp Leu Ser
            20                  25                  30

Lys Lys Lys Lys Asn Pro Gly Asn Tyr Tyr Ser Lys Pro Leu Asn Arg
        35                  40                  45

Ser Lys Phe Ile Asp Leu Asp Glu Glu Phe Glu Ala Asp Phe Gln Asp
    50                  55                  60

Phe Lys Asp Tyr Ala Asp Asp Asp Val Asp Asp Val Lys Pro Phe Gly
65                  70                  75                  80

Ser Lys Ser Val Lys Ser Gly Asp Ser Ser Cys Asp Thr Glu Lys Ser
                85                  90                  95

Ser Lys Arg Lys Arg Lys Asn Gln Tyr Arg Gly Ile Arg Gln Arg Pro
            100                 105                 110

Trp Gly Lys Trp Ala Ala Glu Ile Arg Asp Pro Arg Lys Gly Ile Arg
        115                 120                 125

Val Trp Leu Gly Thr Phe Asn Ser Ala Glu Glu Ala Ala Arg Ala Tyr
    130                 135                 140

Asp Val Glu Ala Arg Arg Ile Arg Gly Lys Lys Ala Lys Val Asn Phe
145                 150                 155                 160

Pro Asp Gly Ser Pro Ala Ser Ala Ser Arg Arg Ala Val Lys Pro Asn
                165                 170                 175

Pro Gln Glu Ala Leu Arg Glu Glu Ile Leu Asn Thr Val Gln Pro Asn
            180                 185                 190

Thr Thr Tyr Ile Asn Asn Leu Asp Gly Gly Ser Asp Asp Ser Phe Gly
        195                 200                 205

Phe Phe Glu Glu Lys Pro Ala Ala Lys Gln Tyr Gly Tyr Glu Asn Val
    210                 215                 220

Ser Phe Thr Ala Gly Asp Met Gly Leu Gly Ser Ile Ser Pro Ser Thr
225                 230                 235                 240

Gly Thr Thr Asn Val Tyr Phe Ser Ser Asp Glu Gly Ser Asn Thr Phe
                245                 250                 255
```

Asp Cys Ser Asp Phe Gly Trp Gly Glu Pro Cys Pro Arg Thr Pro Glu
260 265 270

Ile Ser Ser Val Leu Ser Glu Val Leu Glu Cys Asn Gly Thr Gln Ser
275 280 285

Asp Glu Asp Ala Arg Pro Glu Lys Lys Leu Lys Ser Cys Ser Asn Ala
290 295 300

Ser Leu Pro Asp Glu Asp Asn Thr Val His Thr Leu Ser Glu Glu Leu
305 310 315 320

Ser Ala Phe Glu Ser Gln Met Lys Phe Leu Gln Ile Pro Tyr Leu Glu
325 330 335

Gly Asn Trp Asp Ala Ser Val Asp Ala Phe Val Asn Thr Gly Ala Ile
340 345 350

Gln Asp Gly Gly Asn Ala Met Asp Leu Trp Pro Ser Met Met Phe Leu
355 360 365

Leu

<210> 209
<211> 1032
<212> DNA
<213> Capsicum annuum

<400> 209
attctcaaaa ataatttttc atttctgatt caatcttgtt gtttctttca ttttgaaaaa 60

aaaaagaaga agaagaagag tactttaact acactgcaaa atgtgtggtg gagcaattct 120

tgctgatatc attcctcgtc gtgaccgtcg tctgtcatcc acagacttat ggtcaatttg 180

ttctgatgat ttctggccaa attcttcatt ttccaagcca ttttccaccc aaaatgtttc 240

ccctgcaaag cccaaacgaa ctcaaccctc tgcaggtaat gagcaaatcc agaaagccaa 300

gaaaaggcaa aggaagaatc tatacagggg aatccgccag cgtccatggg gtaaatgggc 360

agctgaaatt cgtgacccaa gaaaaggggt cagagtctgg ttaggtactt tcaacactgc 420

tgaagaagct gctagagctt atgacaaaga agctcgtaaa atccggggag agaaagctaa 480

agttaatttc ccaaatgaag atgatcacta cagttatcca gagcctcctc ctctcgctgc 540

ttacaataat actacttttt ataataattg ctatgcgttc gagaacaatg aacccgttat 600

cgaatatgca atagctaaca acaatgatca aaatgggctg attaaagagg tggaaaatat 660

gaatggaagg gtcgtggagg aggaggaaaa aacagagatt caagtgcaga aactctctga 720

ggaactgatg gcttatgagt cattgatgaa gttttatgag atcccttatg ttgatgggca 780

atcagtggcg gcgatggcga atccagcggc ggaggctgtc ttgggtggtg gcttgatgga 840

gctttggagt tttgatgatg ttagtcgtca acaacctagt tataatgtag tttgattatt 900

gtttgtttac attgttgtat gtttttattt ttcggtttag gagaatgggt ttccttgtaa 960

ttctcaatgt ttaagcaatt ggtaaaacta ttgtctctaa aaaaaaaaaa aaaaaaaaaa 1020

aaaaaaaaaa aa 1032

<210> 210
<211> 264
<212> PRT
<213> Capsicum annuum

<400> 210

Met Cys Gly Gly Ala Ile Leu Ala Asp Ile Ile Pro Arg Arg Asp Arg
1 5 10 15

Arg Leu Ser Ser Thr Asp Leu Trp Ser Ile Cys Ser Asp Asp Phe Trp
20 25 30

Pro Asn Ser Ser Phe Ser Lys Pro Phe Ser Thr Gln Asn Val Ser Pro
35 40 45

Ala Lys Pro Lys Arg Thr Gln Pro Ser Ala Gly Asn Glu Gln Ile Gln
50 55 60

Lys Ala Lys Lys Arg Gln Arg Lys Asn Leu Tyr Arg Gly Ile Arg Gln
65 70 75 80

Arg Pro Trp Gly Lys Trp Ala Ala Glu Ile Arg Asp Pro Arg Lys Gly
85 90 95

Val Arg Val Trp Leu Gly Thr Phe Asn Thr Ala Glu Glu Ala Ala Arg
100 105 110

Ala Tyr Asp Lys Glu Ala Arg Lys Ile Arg Gly Glu Lys Ala Lys Val
115 120 125

Asn Phe Pro Asn Glu Asp Asp His Tyr Ser Tyr Pro Glu Pro Pro Pro
130 135 140

Leu Ala Ala Tyr Asn Asn Thr Thr Phe Tyr Asn Asn Cys Tyr Ala Phe
145 150 155 160

Glu Asn Asn Glu Pro Val Ile Glu Tyr Ala Ile Ala Asn Asn Asn Asp
165 170 175

Gln Asn Gly Leu Ile Lys Glu Val Glu Asn Met Asn Gly Arg Val Val
180 185 190

```
Glu Glu Glu Glu Lys Thr Glu Ile Gln Val Gln Lys Leu Ser Glu Glu
        195                 200                 205

Leu Met Ala Tyr Glu Ser Leu Met Lys Phe Tyr Glu Ile Pro Tyr Val
    210                 215                 220

Asp Gly Gln Ser Val Ala Ala Met Ala Asn Pro Ala Ala Glu Ala Val
225                 230                 235                 240

Leu Gly Gly Gly Leu Met Glu Leu Trp Ser Phe Asp Asp Val Ser Arg
                245                 250                 255

Gln Gln Pro Ser Tyr Asn Val Val
            260


<210>  211
<211>  1597
<212>  DNA
<213>  Gossypium hirsutum

<400>  211
aaaaaaccaa cccatttatt tcactaccca acaacccaaa agataaaagg aatcgtttac      60

ttgttcgtag caatttatcg tcatttcaag ctcaattctc tgaaaatttt tgtgctgaac     120

ttcttttttc tctttgtacc accatgtgtg gaggtgctat tatctccgat ttcataccgc     180

cgtcgcggtc gcgacgattg acggccgatt tcttgtggcc cgatctgaaa aaatccgggt     240

tgaagaaggg gtcgggtaag agatactcga agcccgtgat cgacttgggc gatgatttcg     300

agactgactt tcaggagttc aaagatgaag aatctgatat agatgattat gatgttgatg     360

atgttttggc tgatgtaaag ccctttgctt ttaacgctac aaagaaacct gcttctgctg     420

tctctcatgg ttcgaactct gaaaaatcca tgcaattcaa tggtcaagct gagaaatgtg     480

cgaaaagaaa gaggaagaac cagtatcgtg gaatccggca gcgcccatgg ggtaaatggg     540

ctgctgagat ccgtgaccca aggaaagggg ttagggtctg gttaggaact ttcaatactg     600

ctgaagaagc tgcgagagct tatgatgctg aggcacggag aattcgtggt aagaaagcta     660

aggtgaactt ccctaacgag actccgcgta cctctccaaa gcatgcagtc aagacaaatt     720

ctcagaaacc actttccaag tcaaattcga gccctgttca gccaaatctc aaccagaatt     780

acaattactt gaaccagcct gagcaggaat actttgatac catgggtttc gtagaagaga     840

agccatcggt caatcagttc gcatacgtgg accctgttcc tacgtctata gatgctggat     900

ttaatcaatc agataatgcc cccttgtact tcaattcaga ccagggaagt aactcgatca     960

attgttccga ctatggctgg ggagaacagg gtgccaaaac tcctgaaata tcatccattc    1020

tggaagcttc tgtagagggt gatgagtttc ttgaggatgc taaccctagc aagaagctga    1080

aaccaagttc cgacaatgtt atgcctgccg aagacaactc cgcgaagacc ttgtcggacg    1140
```

agctgttggc tttggacaac cagatgaaat acttccaaat gccgccattt attgaaggaa 1200

actgggacgc cactattgat gctttcctca atggagatgc aacacaggat ggtggaaacc 1260

cgatggatct ttggaacttt gatgatttcc ctaccatggc ggagggtgtt ttctgagcga 1320

actttccata ataactagtg tttgtaaata aagcaacatg aatttggtca aaatctgttg 1380

tgaagttgaa gtaaaaacca agctatatgc atgcttaagc cttgcctgca ctgctttcag 1440

aggtttttag tatgtacccc ttttttatgt gttttttttgt agactttgga ctaaatttta 1500

aatttgagtg actgtataag taactgtgtc tgaatttgtc tatgtttgaa tactgaaaaa 1560

catatgaatg ttttaaaccc aaaaaaaaaa aaaaaaa 1597

<210> 212
<211> 390
<212> PRT
<213> Gossypium hirsutum

<400> 212

Met Cys Gly Gly Ala Ile Ile Ser Asp Phe Ile Pro Pro Ser Arg Ser
1 5 10 15

Arg Arg Leu Thr Ala Asp Phe Leu Trp Pro Asp Leu Lys Lys Ser Gly
20 25 30

Leu Lys Lys Gly Ser Gly Lys Arg Tyr Ser Lys Pro Val Ile Asp Leu
35 40 45

Gly Asp Asp Phe Glu Thr Asp Phe Gln Glu Phe Lys Asp Glu Glu Ser
50 55 60

Asp Ile Asp Asp Tyr Asp Val Asp Asp Val Leu Ala Asp Val Lys Pro
65 70 75 80

Phe Ala Phe Asn Ala Thr Lys Lys Pro Ala Ser Ala Val Ser His Gly
85 90 95

Ser Asn Ser Glu Lys Ser Met Gln Phe Asn Gly Gln Ala Glu Lys Cys
100 105 110

Ala Lys Arg Lys Arg Lys Asn Gln Tyr Arg Gly Ile Arg Gln Arg Pro
115 120 125

Trp Gly Lys Trp Ala Ala Glu Ile Arg Asp Pro Arg Lys Gly Val Arg
130 135 140

Val Trp Leu Gly Thr Phe Asn Thr Ala Glu Glu Ala Ala Arg Ala Tyr
145 150 155 160

Asp Ala Glu Ala Arg Arg Ile Arg Gly Lys Lys Ala Lys Val Asn Phe

```
                165                 170                 175

Pro Asn Glu Thr Pro Arg Thr Ser Pro Lys His Ala Val Lys Thr Asn
            180                 185                 190

Ser Gln Lys Pro Leu Ser Lys Ser Asn Ser Ser Pro Val Gln Pro Asn
        195                 200                 205

Leu Asn Gln Asn Tyr Asn Tyr Leu Asn Gln Pro Glu Gln Glu Tyr Phe
    210                 215                 220

Asp Thr Met Gly Phe Val Glu Glu Lys Pro Ser Val Asn Gln Phe Ala
225                 230                 235                 240

Tyr Val Asp Pro Val Pro Thr Ser Ile Asp Ala Gly Phe Asn Gln Ser
                245                 250                 255

Asp Asn Ala Pro Leu Tyr Phe Asn Ser Asp Gln Gly Ser Asn Ser Ile
            260                 265                 270

Asn Cys Ser Asp Tyr Gly Trp Gly Glu Gln Gly Ala Lys Thr Pro Glu
        275                 280                 285

Ile Ser Ser Ile Leu Glu Ala Ser Val Glu Gly Asp Glu Phe Leu Glu
    290                 295                 300

Asp Ala Asn Pro Ser Lys Lys Leu Lys Pro Ser Ser Asp Asn Val Met
305                 310                 315                 320

Pro Ala Glu Asp Asn Ser Ala Lys Thr Leu Ser Asp Glu Leu Leu Ala
                325                 330                 335

Leu Asp Asn Gln Met Lys Tyr Phe Gln Met Pro Pro Phe Ile Glu Gly
            340                 345                 350

Asn Trp Asp Ala Thr Ile Asp Ala Phe Leu Asn Gly Asp Ala Thr Gln
        355                 360                 365

Asp Gly Gly Asn Pro Met Asp Leu Trp Asn Phe Asp Asp Phe Pro Thr
    370                 375                 380

Met Ala Glu Gly Val Phe
385                 390
```

<210> 213
<211> 1403
<212> DNA
<213> Gossypium barbadense

<400> 213

ttgagaatcg atgcccggat taataatttc tgggatgtag tcactaaaaa ggcctttctc 60
atgattttct tgcagttaaa ggtcttaaat tatatttcct tgtgacagtt atgttgtgat 120
ttgtagtttt tcatggatga gtaatgttta tttatggttc atgtatccgt acgatattaa 180
atttttcttt ttgtgctcta tcattgaagg ttcgaactct gaaaagtcca tgcagttcga 240
tggtcaagct gagaaatgtg cgaaaagaaa gaggaagaac cagtatcgtg gaatccggca 300
gcgcccatgg ggtaaatggg ctgctgagat ccgtgaccca aggaaagggg ttagggtctg 360
gttaggaact ttcaatactg ctgaagaagc tgcgagagct tatgatgctg aggcacggag 420
aattcgtggt aagaaagcta aggtgaactt ccctaacgag actccgcgta cctctccaaa 480
gcatgcagtc aagacaaatt ctcagaaacc actttccaag tcgaatttga gccctgttca 540
gctaaatctc gaccagaatt acaattactt gagccagcct gagcaggaat acttcgatac 600
catgggtttc gtagaagaga agccactggt caatcagttt gcatatgtgg accctgttcc 660
tacgtctata gatgctggat ctaatcaatc agataatgcc cccttgtact tcaattcgga 720
ccagggaagt aactccatca attgttccga ctatggctgg ggagaacagg gtgccagaac 780
tcctgaaata tcatccattc ttgaagcttc tgtagtgggt gaagagtttc ttgaggatgc 840
taaccctagc aagaagctga aaccaagttc tgacaatgtt atgcctgccg aagacaactc 900
cgcgaagacc ttgtcggacg agctgttggc tttggacaac cagatgaaat acttccaaat 960
gccgccattt attgaaggaa actgggacgc cactattgat gctttcctca atggagatgc 1020
aacacaggat ggtggaaacc cgatggatct ttggaacttt gatgatttcc ctaccatggc 1080
ggagggtgtt ttctgagcga actttccata ataactagtg tttgtaaata aagcaacatg 1140
aatttggtca aaatctgttg tgaagttgaa gtaaaaacca agctatatgc atgcttaagc 1200
cttgcctgca ctgctttcag aggtttttag tatgtacccc ttttttatgt gttttttttgt 1260
agactttgga ctaaatttta aatttgagtg actgtataag taattgtgtc tgaatttgtt 1320
tatgtttgaa tactgaaaaa catatgaatg ttttaaactc tgctatttgt ttctcccaaa 1380
aaaaaaaaaa aaaaaaaaaa aaa 1403

<210> 214
<211> 319
<212> PRT
<213> Gossypium barbadense

<400> 214

Met Ser Asn Val Tyr Leu Trp Phe Met Tyr Pro Tyr Asp Ile Lys Phe
1 5 10 15

Phe Phe Leu Cys Ser Ile Ile Glu Gly Ser Asn Ser Glu Lys Ser Met
20 25 30

Gln Phe Asp Gly Gln Ala Glu Lys Cys Ala Lys Arg Lys Arg Lys Asn

35 40 45

Gln Tyr Arg Gly Ile Arg Gln Arg Pro Trp Gly Lys Trp Ala Ala Glu
50 55 60

Ile Arg Asp Pro Arg Lys Gly Val Arg Val Trp Leu Gly Thr Phe Asn
65 70 75 80

Thr Ala Glu Glu Ala Ala Arg Ala Tyr Asp Ala Glu Ala Arg Arg Ile
85 90 95

Arg Gly Lys Lys Ala Lys Val Asn Phe Pro Asn Glu Thr Pro Arg Thr
100 105 110

Ser Pro Lys His Ala Val Lys Thr Asn Ser Gln Lys Pro Leu Ser Lys
115 120 125

Ser Asn Leu Ser Pro Val Gln Leu Asn Leu Asp Gln Asn Tyr Asn Tyr
130 135 140

Leu Ser Gln Pro Glu Gln Glu Tyr Phe Asp Thr Met Gly Phe Val Glu
145 150 155 160

Glu Lys Pro Leu Val Asn Gln Phe Ala Tyr Val Asp Pro Val Pro Thr
165 170 175

Ser Ile Asp Ala Gly Ser Asn Gln Ser Asp Asn Ala Pro Leu Tyr Phe
180 185 190

Asn Ser Asp Gln Gly Ser Asn Ser Ile Asn Cys Ser Asp Tyr Gly Trp
195 200 205

Gly Glu Gln Gly Ala Arg Thr Pro Glu Ile Ser Ser Ile Leu Glu Ala
210 215 220

Ser Val Val Gly Glu Glu Phe Leu Glu Asp Ala Asn Pro Ser Lys Lys
225 230 235 240

Leu Lys Pro Ser Ser Asp Asn Val Met Pro Ala Glu Asp Asn Ser Ala
245 250 255

Lys Thr Leu Ser Asp Glu Leu Leu Ala Leu Asp Asn Gln Met Lys Tyr
260 265 270

Phe Gln Met Pro Pro Phe Ile Glu Gly Asn Trp Asp Ala Thr Ile Asp
275 280 285

Ala Phe Leu Asn Gly Asp Ala Thr Gln Asp Gly Gly Asn Pro Met Asp
290 295 300

```
Leu Trp Asn Phe Asp Asp Phe Pro Thr Met Ala Glu Gly Val Phe
305                 310                 315


<210>  215
<211>  1538
<212>  DNA
<213>  Gossypium hirsutum

<400>  215
cggcacgagg agagagagag aactagtctc gtgccgggaa aatttacaat taaaaaaatt      60

aattttgccc tctaaatttc tctaaaaaat ctcctaaatc ccatacttta aatccaattc     120

gccatgtgtg gaggtgcgat tatctccgat ttcattccgc ctgcgcggtc gcgactggtg     180

acggcgaatt atttgtggcc ggatctgaaa aaatccggct ctaaaaagcg gtcgggtaga     240

aagcactcga agaagccggc ggtcggcttt gaagatgact tcgaggctga ttttcaggtg     300

tttaaggatg aggattctga tgtcgatgac ttcaacgatg atgttgatga tgttttggct     360

gatgttaagt cctttgcttt ttctgctaca aagaaaccct ctcctgctgt ttctcatggt     420

tcaaactcca taaaatctgt ggaattcagt ggtcaagctg agaaatctgc aaaaagaaag     480

aggaagaacc agtatcgcgg aattcgccag cgtccgtggg gtaaatgggc tgctgagatc     540

cgtgatccta ggaaaggggt tagggtgtgg cttggaactt ttaatactgc tgaggaagct     600

gcacgagctt atgatgctga ggcactgaga attcggggta agaaagcaaa ggtgaacttc     660

cctgatgaga ctccacgtac cactccaaag catgctgtta aaatgaattc tcagaaacct     720

ctttccgggt caaatttgag ttctgttcaa tcaagtctca acccagattt cagttactcg     780

aacaaacttg agcagggcta ctatgatacc gtgggtttca ttgaagagaa gtcactaatg     840

gatcagtttg catatgcaga ccctgttaga gctgctgtag atgatgggtt aaaacccttt     900

gctgaccctg agaataccgc ttcgtttttt atctcagatc cagggagtaa caactttgac     960

agttccgacc ttgtctgggg cgaccagggt gccaagactc ctgaaatatc atcctctctt    1020

gaacctactc tagaggtcaa cgagtttctg gacaacgcaa accctacgaa gaagttgaaa    1080

ctgagcttga ataatgtcat gcctactgga ggagacaatt cggtaaagag tttatccgat    1140

gagctattag ctatggacaa tcaagtgaac tactttcaga caccatttat tgacgagaac    1200

tggaatgttt cgatggatga cttccttaat ggagatgcaa ctcaggttgg cggaaacgaa    1260

atgggttttt ggagctttga tgactttcct tccatagatg ggggtatttt ctgaacaaac    1320

tctccttact tattatccgt ttccgtacat aaggcatcgt gttagcgagt ttcgaccctc    1380

tggcaactgc ttgactattg tttctatctt gatatctcta acaccgaaaa attcaaattt    1440

aaatttcgag tgactgttag aacttacaac attgattatg tgtgatttat gatgaaaatt    1500

tgaaactcct atgaatgttt aaccaaaaaa aaaaaaaa                           1538
```

<210> 216
<211> 396
<212> PRT
<213> Gossypium hirsutum

<400> 216

```
Met Cys Gly Gly Ala Ile Ile Ser Asp Phe Ile Pro Pro Ala Arg Ser
1               5                   10                  15

Arg Leu Val Thr Ala Asn Tyr Leu Trp Pro Asp Leu Lys Lys Ser Gly
            20                  25                  30

Ser Lys Lys Arg Ser Gly Arg Lys His Ser Lys Lys Pro Ala Val Gly
        35                  40                  45

Phe Glu Asp Asp Phe Glu Ala Asp Phe Gln Val Phe Lys Asp Glu Asp
    50                  55                  60

Ser Asp Val Asp Asp Phe Asn Asp Asp Val Asp Asp Val Leu Ala Asp
65                  70                  75                  80

Val Lys Ser Phe Ala Phe Ser Ala Thr Lys Lys Pro Ser Pro Ala Val
                85                  90                  95

Ser His Gly Ser Asn Ser Ile Lys Ser Val Glu Phe Ser Gly Gln Ala
            100                 105                 110

Glu Lys Ser Ala Lys Arg Lys Arg Lys Asn Gln Tyr Arg Gly Ile Arg
        115                 120                 125

Gln Arg Pro Trp Gly Lys Trp Ala Ala Glu Ile Arg Asp Pro Arg Lys
    130                 135                 140

Gly Val Arg Val Trp Leu Gly Thr Phe Asn Thr Ala Glu Glu Ala Ala
145                 150                 155                 160

Arg Ala Tyr Asp Ala Glu Ala Leu Arg Ile Arg Gly Lys Lys Ala Lys
                165                 170                 175

Val Asn Phe Pro Asp Glu Thr Pro Arg Thr Thr Pro Lys His Ala Val
            180                 185                 190

Lys Met Asn Ser Gln Lys Pro Leu Ser Gly Ser Asn Leu Ser Ser Val
        195                 200                 205

Gln Ser Ser Leu Asn Pro Asp Phe Ser Tyr Ser Asn Lys Leu Glu Gln
    210                 215                 220

Gly Tyr Tyr Asp Thr Val Gly Phe Ile Glu Glu Lys Ser Leu Met Asp
225                 230                 235                 240
```

Gln Phe Ala Tyr Ala Asp Pro Val Arg Ala Ala Val Asp Asp Gly Leu
245 250 255

Lys Pro Phe Ala Asp Pro Glu Asn Thr Ala Ser Phe Phe Ile Ser Asp
260 265 270

Pro Gly Ser Asn Asn Phe Asp Ser Ser Asp Leu Val Trp Gly Asp Gln
275 280 285

Gly Ala Lys Thr Pro Glu Ile Ser Ser Ser Leu Glu Pro Thr Leu Glu
290 295 300

Val Asn Glu Phe Leu Asp Asn Ala Asn Pro Thr Lys Lys Leu Lys Leu
305 310 315 320

Ser Leu Asn Asn Val Met Pro Thr Gly Gly Asp Asn Ser Val Lys Ser
325 330 335

Leu Ser Asp Glu Leu Leu Ala Met Asp Asn Gln Val Asn Tyr Phe Gln
340 345 350

Thr Pro Phe Ile Asp Glu Asn Trp Asn Val Ser Met Asp Asp Phe Leu
355 360 365

Asn Gly Asp Ala Thr Gln Val Gly Gly Asn Glu Met Gly Phe Trp Ser
370 375 380

Phe Asp Asp Phe Pro Ser Ile Asp Gly Gly Ile Phe
385 390 395

<210> 217
<211> 995
<212> DNA
<213> Gossypium hirsutum

<220>
<221> misc_feature
<222> (919)..(919)
<223> n is a, c, g, or t

<400> 217
atgtgtggag gtgcaattat ttccgatttc gtcgccgtga aacatgaccc gaaattgacc 60

ggcgatgacc tttggtctga aattgacata ttctccgacc tcctaggttt cgacaacaat 120

ggcaaaagct ttgtcaatca tcagtttcat aacaacaaca agaagctgat caatccaaaa 180

gacaatcaac ttaacaaaga gagaagcgag acgattcaga agacaagccg agtcactaaa 240

aatgttgaaa agactcagcg aactcggaaa aacttttacc gaggaataag acaaaggcca 300

tggggcaaat gggcagctga gattagagac cctcaaaaag gcgtccgagt ttggctcggt 360

acccataaca cagccgaaga agcggctcga gcctacgatg aagccgccaa gcgtatccgt 420

ggtgataaag ccaagctcaa cttccctcaa acgcccacca aaaatcaagc agcttcacca 480

gctcttactc agcttcctcc tgctaagaaa aggtgcatcg ttcccgagtt aactcaaccg 540

agcttccaga ctgactcacc accttatcct atgggtcttg ggtctggaag aagtgaagat 600

tataagccga ttgaagtggt ggagagtgag ttggagttga aagaacaaat ctggagcctg 660

gaatcatttc tgggtttgga gactaatcat gagactatga ctcaactgag tggtaacgga 720

gggactgact cactggagct ttggacactt gatgacctcg taactcagta taagcaacgg 780

cgctaacatg ttcatcagtg ggggcgaaaa ttaaataata gttagcgtta ataaatgttt 840

tttgtgttaa ttagggtttt tttatttata ctatcatgca ttatatatat atatagatga 900

gaataaagga ttaacgctnt gctcttgcat ggattagtta gcgttaatca atgttagtgt 960

gtaattaggg tttttattat gttaaaaaaa aaaaa 995

<210> 218
<211> 261
<212> PRT
<213> Gossypium hirsutum

<400> 218

Met Cys Gly Gly Ala Ile Ile Ser Asp Phe Val Ala Val Lys His Asp
1 5 10 15

Pro Lys Leu Thr Gly Asp Asp Leu Trp Ser Glu Ile Asp Ile Phe Ser
20 25 30

Asp Leu Leu Gly Phe Asp Asn Asn Gly Lys Ser Phe Val Asn His Gln
35 40 45

Phe His Asn Asn Asn Lys Lys Leu Ile Asn Pro Lys Asp Asn Gln Leu
50 55 60

Asn Lys Glu Arg Ser Glu Thr Ile Gln Lys Thr Ser Arg Val Thr Lys
65 70 75 80

Asn Val Glu Lys Thr Gln Arg Thr Arg Lys Asn Phe Tyr Arg Gly Ile
85 90 95

Arg Gln Arg Pro Trp Gly Lys Trp Ala Ala Glu Ile Arg Asp Pro Gln
100 105 110

Lys Gly Val Arg Val Trp Leu Gly Thr His Asn Thr Ala Glu Glu Ala
115 120 125

Ala Arg Ala Tyr Asp Glu Ala Ala Lys Arg Ile Arg Gly Asp Lys Ala
130 135 140

Lys Leu Asn Phe Pro Gln Thr Pro Thr Lys Asn Gln Ala Ala Ser Pro
145                 150                 155                 160

Ala Leu Thr Gln Leu Pro Pro Ala Lys Lys Arg Cys Ile Val Pro Glu
                165                 170                 175

Leu Thr Gln Pro Ser Phe Gln Thr Asp Ser Pro Pro Tyr Pro Met Gly
            180                 185                 190

Leu Gly Ser Gly Arg Ser Glu Asp Tyr Lys Pro Ile Glu Val Val Glu
        195                 200                 205

Ser Glu Leu Glu Leu Lys Glu Gln Ile Trp Ser Leu Glu Ser Phe Leu
    210                 215                 220

Gly Leu Glu Thr Asn His Glu Thr Met Thr Gln Leu Ser Gly Asn Gly
225                 230                 235                 240

Gly Thr Asp Ser Leu Glu Leu Trp Thr Leu Asp Asp Leu Val Thr Gln
                245                 250                 255

Tyr Lys Gln Arg Arg
            260

<210>  219
<211>  1042
<212>  DNA
<213>  Gossypium hirsutum

<400>  219
atgtgtggag gtgcaattat ttccgatttc attgccgtga aacgcggtcg gaagttaacc    60

gccgaggatc tttggtctga acttgacaca ttctccgacc tgttgggtct ggattacgga    120

aatggaaaag agtcttcttt cactcagtcc gacaacacca aggccggttc caaagccaag    180

aaccttgaaa aagtggcaaa cgagacgact cagaagacaa gccgagggag agagaaagaa    240

gggaagactc agcgaactcg aaagaacatt tacagaggaa tcaggcaaag gccatggggg    300

aaatggcgg ctgagataag agatcctcac aaaggtgtcc gagtctggct cggtacatac    360

aacacggctg aagaagcggc tcgagcctac gatgaagccg ccaagcgcat ccgtggggag    420

aaagccaagc tcaacttccc tcaaactcca cacctaactc agcctcctgc taagaaaagg    480

tgtatgatgg ctcctgagtt gactccgccg agttctgaaa caaagagccc accaacccca    540

caacttttta tgggttttgg ttacgagaat ggagtttaca gaccgagtga agcaatggaa    600

agcgagatgg agctgaagga gcaaatctcg agcctggagt cgttcctggg tttggagcct    660

gatgagacaa caactgagtt gagtggaagt gctgagcctg actcagtgga cctttggatg    720

cttgatgacc ttgtgacaca tcatcaacaa cagcctcagc tcttttatta gaaattaaaa    780

```
agttttaatt agggtaatgt tatgtttgat atgattatgc ttaagacttt aatgtttgta    840

ttaatgaagg gtaaatagtt gtgacaataa aaagattggc cacccagttt taatttatat    900

ttatttctaa tttgggtgac atgtaaattg gtttcgagaa acattgaagt acccataatg    960

ttagttcatg tatcagatat agtaaacact ttggattaat aaaaaaacct cctaatattt   1020

tatttgtaaa aaaaaaaaaa aa                                            1042


<210>  220
<211>  256
<212>  PRT
<213>  Gossypium hirsutum

<400>  220

Met Cys Gly Gly Ala Ile Ile Ser Asp Phe Ile Ala Val Lys Arg Gly
1               5                   10                  15

Arg Lys Leu Thr Ala Glu Asp Leu Trp Ser Glu Leu Asp Thr Phe Ser
            20                  25                  30

Asp Leu Leu Gly Leu Asp Tyr Gly Asn Gly Lys Glu Ser Ser Phe Thr
        35                  40                  45

Gln Ser Asp Asn Thr Lys Ala Gly Ser Lys Ala Lys Asn Leu Glu Lys
    50                  55                  60

Val Ala Asn Glu Thr Thr Gln Lys Thr Ser Arg Gly Arg Glu Lys Glu
65                  70                  75                  80

Gly Lys Thr Gln Arg Thr Arg Lys Asn Ile Tyr Arg Gly Ile Arg Gln
                85                  90                  95

Arg Pro Trp Gly Lys Trp Ala Ala Glu Ile Arg Asp Pro His Lys Gly
            100                 105                 110

Val Arg Val Trp Leu Gly Thr Tyr Asn Thr Ala Glu Glu Ala Ala Arg
        115                 120                 125

Ala Tyr Asp Glu Ala Ala Lys Arg Ile Arg Gly Glu Lys Ala Lys Leu
    130                 135                 140

Asn Phe Pro Gln Thr Pro His Leu Thr Gln Pro Pro Ala Lys Lys Arg
145                 150                 155                 160

Cys Met Met Ala Pro Glu Leu Thr Pro Pro Ser Ser Glu Thr Lys Ser
                165                 170                 175

Pro Pro Thr Pro Gln Leu Phe Met Gly Phe Gly Tyr Glu Asn Gly Val
            180                 185                 190
```

```
Tyr Arg Pro Ser Glu Ala Met Glu Ser Glu Met Glu Leu Lys Glu Gln
        195                 200                 205

Ile Ser Ser Leu Glu Ser Phe Leu Gly Leu Glu Pro Asp Glu Thr Thr
    210                 215                 220

Thr Glu Leu Ser Gly Ser Ala Glu Pro Asp Ser Val Asp Leu Trp Met
225                 230                 235                 240

Leu Asp Asp Leu Val Thr His His Gln Gln Gln Pro Gln Leu Phe Tyr
                245                 250                 255


<210>  221
<211>  1143
<212>  DNA
<213>  Gossypium barbadense

<400>  221
ccatctgaaa aaaaaacagg ttttctttta agataacacc caaaaatgtg tggaggtgca      60

attatttccg atttcgtcgc ccccaaatat gaccggaaat tgaccggcga tgacctttgg     120

tctgaacttg acatattctc cgacctccta ggtttcgaca acaatggcaa aagctttgtc     180

aatcatcagt ttcataacaa caacaacaac aagctcatta atccaaaaga caatcaactt     240

aacaaagaga gaagcgagac gattcagaag acaagccaag tcactaaaaa ggttgaaaag     300

actcagcgaa ctcggaaaaa cttttacaga ggaataagac aaagaccatg gggcaaatgg     360

gcagctgaga ttagagaccc tcaaaaaggc gttcgagttt ggctcggtac ctataacaca     420

gccgaagaag cgactcgagc ctacgatgaa gcagccaagc gtatccgtgg tgataaagcc     480

aagctcaact tccctcaaac gcccaccaaa aatcaagcag cttcaccagc tcttactctg     540

cttcctcctg ctaagaaaag gtgcatcgtt cccggagtta actcaaccga gtttaccaga     600

ctggactcac cacctatatc cctatgggtt cttggggtat gagaagaagt gaaagattat     660

aagccgattg aagtggtgga gagtgagttg gagttgaaag aacaaatctg gagccttgga     720

atcatccctg ggtttggaga ctaaccatga gactatgact cagctgagtg gtaacggtgg     780

gactgaccca ctggagcttt ggacacttga tgaccccgta acccagtata agcaacggcg     840

ctaacatgtt cattagtggg ggcgaaaatt aaataatagt tagcattaat caatgttttt     900

gtgttactta gggttttttt atttatgcta tcatgcatta tatatatata tgagaataaa     960

ggattaacgc tttgctcttg catggattag ttagcgttaa tcaatatttg tgtttagggt    1020

tttttattat gttgttatat atgtatatgc ttatgacttt catgtttgta tcaatgcatt    1080

agttgcatta tatatataag aataaaggat ttaacaaaaa aaaaaaaata aaaaaaaaaa    1140

aaa                                                                  1143
```

<210> 222
<211> 198
<212> PRT
<213> Gossypium barbadense

<400> 222

Met Cys Gly Gly Ala Ile Ile Ser Asp Phe Val Ala Pro Lys Tyr Asp
1 5 10 15

Arg Lys Leu Thr Gly Asp Asp Leu Trp Ser Glu Leu Asp Ile Phe Ser
20 25 30

Asp Leu Leu Gly Phe Asp Asn Asn Gly Lys Ser Phe Val Asn His Gln
35 40 45

Phe His Asn Asn Asn Asn Asn Lys Leu Ile Asn Pro Lys Asp Asn Gln
50 55 60

Leu Asn Lys Glu Arg Ser Glu Thr Ile Gln Lys Thr Ser Gln Val Thr
65 70 75 80

Lys Lys Val Glu Lys Thr Gln Arg Thr Arg Lys Asn Phe Tyr Arg Gly
85 90 95

Ile Arg Gln Arg Pro Trp Gly Lys Trp Ala Ala Glu Ile Arg Asp Pro
100 105 110

Gln Lys Gly Val Arg Val Trp Leu Gly Thr Tyr Asn Thr Ala Glu Glu
115 120 125

Ala Thr Arg Ala Tyr Asp Glu Ala Ala Lys Arg Ile Arg Gly Asp Lys
130 135 140

Ala Lys Leu Asn Phe Pro Gln Thr Pro Thr Lys Asn Gln Ala Ala Ser
145 150 155 160

Pro Ala Leu Thr Leu Leu Pro Pro Ala Lys Lys Arg Cys Ile Val Pro
165 170 175

Gly Val Asn Ser Thr Glu Phe Thr Arg Leu Asp Ser Pro Pro Ile Ser
180 185 190

Leu Trp Val Leu Gly Val
195

<210> 223
<211> 1079
<212> DNA
<213> Gossypium hirsutum

<400> 223

```
gcacgaggat ctaagaaccc gaaaagtaga gctttggttc tctgtctgct gcttctttct      60
tctcttggta cttcattatt tttcctttca cttccttttt gtgaaaaaac attgaaaccc     120
ttttcaagac gcaatagaag atgtgtggag gtgcaatcat ttccgatttc attgccgtga     180
aacgcggtcg gaagttaacc gccgaggatc tttggtctga acttgacaca ttctccgacc     240
tgttgggtct ggattacgga aatggaaaag accctttcac tcagttcgac aacaccaagg     300
ccggttccaa agccaagaac cttgaaaaag tgacaaacga gtcgactcag aagacaagcc     360
gggggagaga gaaagaaggg aagactcagc gaactcgaaa gaacatttac agaggaatca     420
ggcgaaggcc atgggggaaa tgggcggctg agataagaga tcctcacaaa ggtgtccgaa     480
tctggctcgg tacatacaac acggctgaag aagcggctcg agcctacgat gaagccgcca     540
agcgcatccg cggggacaaa gccaagctca acttccctca aactccacgc ctaactcagc     600
ctcctgctaa gaaaaggtgt atgatggctc ctgagttgac tccaccgagt tctgaaacca     660
agagcccacc aaccccacaa ccttttatgg gttttggtta cgagaatgga gtttacaggc     720
cgagtgaagc aatggaaagc gagatggagc tgaaggagca aatctcgagt ctggagtcct     780
tcctgggttt ggagcctgat gagatgacaa ctgagttgag tggaagcgct gagcctgagt     840
cagtgaacct ttggatgctt gatcaccttg tgacacatca tcaacaacag cctcagctct     900
tttattagaa attaaaaagt ttaaattagg gtaatgttat gtttgatatg attatgctta     960
agactttaat gtttgtatta atgaagggta aatagttgtg acaataaaaa gattggccac    1020
ccagttttaa tttatattta tttcctaaaa ttctaaaaaa aaaaaaaaaa aaaaaaaaa     1079
```

<210> 224
<211> 255
<212> PRT
<213> Gossypium hirsutum

<400> 224

```
Met Cys Gly Gly Ala Ile Ile Ser Asp Phe Ile Ala Val Lys Arg Gly
1               5                   10                  15

Arg Lys Leu Thr Ala Glu Asp Leu Trp Ser Glu Leu Asp Thr Phe Ser
            20                  25                  30

Asp Leu Leu Gly Leu Asp Tyr Gly Asn Gly Lys Asp Pro Phe Thr Gln
        35                  40                  45

Phe Asp Asn Thr Lys Ala Gly Ser Lys Ala Lys Asn Leu Glu Lys Val
    50                  55                  60

Thr Asn Glu Ser Thr Gln Lys Thr Ser Arg Gly Arg Glu Lys Glu Gly
65                  70                  75                  80

Lys Thr Gln Arg Thr Arg Lys Asn Ile Tyr Arg Gly Ile Arg Arg Arg
```

```
                85                  90                  95

Pro Trp Gly Lys Trp Ala Ala Glu Ile Arg Asp Pro His Lys Gly Val
            100                 105                 110

Arg Ile Trp Leu Gly Thr Tyr Asn Thr Ala Glu Glu Ala Ala Arg Ala
        115                 120                 125

Tyr Asp Glu Ala Ala Lys Arg Ile Arg Gly Asp Lys Ala Lys Leu Asn
    130                 135                 140

Phe Pro Gln Thr Pro Arg Leu Thr Gln Pro Pro Ala Lys Lys Arg Cys
145                 150                 155                 160

Met Met Ala Pro Glu Leu Thr Pro Pro Ser Ser Glu Thr Lys Ser Pro
                165                 170                 175

Pro Thr Pro Gln Pro Phe Met Gly Phe Gly Tyr Glu Asn Gly Val Tyr
            180                 185                 190

Arg Pro Ser Glu Ala Met Glu Ser Glu Met Glu Leu Lys Glu Gln Ile
        195                 200                 205

Ser Ser Leu Glu Ser Phe Leu Gly Leu Glu Pro Asp Glu Met Thr Thr
    210                 215                 220

Glu Leu Ser Gly Ser Ala Glu Pro Glu Ser Val Asn Leu Trp Met Leu
225                 230                 235                 240

Asp His Leu Val Thr His His Gln Gln Gln Pro Gln Leu Phe Tyr
                245                 250                 255

<210>  225
<211>  916
<212>  DNA
<213>  Gossypium hirsutum

<400>  225
atgagactat gcggccgctg ttacaagtcg acgaatcaac ttaacaaagg gagaagcgag      60

acgattcaga agacaagccg agtcactaaa aatgttgaaa agactcagcg aactcggaaa     120

aacttttacc gaggaataag acaaaggcca tggggcaaat gggcagctga gattagagac     180

cctcaaaaag gcgtccgagt ttggctcggt acccataaca cagccgaaga agcggctcga     240

gcctacgatg aagccgccaa gcgtatccgt ggtgataaag ccaagctcaa cttccctcaa     300

acgcccacca aaaatcaagc agcttcacca gctcttattc agcttcctcc tgctaagaaa     360

aggtgcatcg ttcccgagtt aactcaaccg agtttccaga ctgactcacc accttatcct     420

atgggtcttg ggtctggaag aagtgaagat tataagccga ttgaagtggt ggagagtgag     480
```

ttggagttga aagaacaaat ctggagcctg gagtcgttcc tgggtttgga gcctgatgag 540

acaacaactg agttgagtgg aagtgctgag cctgactcag tggacctttg gatgcttgat 600

gaccttgtga cacatcatca acaacagcct cagctctttt attagaaatt aaaaagtttt 660

aattagggta atgttatgtt tgatatgatt atgcttaaga ctttaatgtt tgtattaatg 720

aagggtaaat agttgtgaca ataaaaagat tggccaccca gttttaattt atatttattt 780

ctaatttggg tgacatgtaa attggtttcg agaaacattg aagtacccat aatgttagtt 840

catgtatcag atatagtaaa cactttggat taataaaaaa acctcctaat attttatttg 900

taaaaaaaaa aaaaaa 916

<210> 226
<211> 214
<212> PRT
<213> Gossypium hirsutum

<400> 226

Met Arg Leu Cys Gly Arg Cys Tyr Lys Ser Thr Asn Gln Leu Asn Lys
1 5 10 15

Gly Arg Ser Glu Thr Ile Gln Lys Thr Ser Arg Val Thr Lys Asn Val
20 25 30

Glu Lys Thr Gln Arg Thr Arg Lys Asn Phe Tyr Arg Gly Ile Arg Gln
35 40 45

Arg Pro Trp Gly Lys Trp Ala Ala Glu Ile Arg Asp Pro Gln Lys Gly
50 55 60

Val Arg Val Trp Leu Gly Thr His Asn Thr Ala Glu Glu Ala Ala Arg
65 70 75 80

Ala Tyr Asp Glu Ala Ala Lys Arg Ile Arg Gly Asp Lys Ala Lys Leu
85 90 95

Asn Phe Pro Gln Thr Pro Thr Lys Asn Gln Ala Ala Ser Pro Ala Leu
100 105 110

Ile Gln Leu Pro Pro Ala Lys Lys Arg Cys Ile Val Pro Glu Leu Thr
115 120 125

Gln Pro Ser Phe Gln Thr Asp Ser Pro Pro Tyr Pro Met Gly Leu Gly
130 135 140

Ser Gly Arg Ser Glu Asp Tyr Lys Pro Ile Glu Val Val Glu Ser Glu
145 150 155 160

Leu Glu Leu Lys Glu Gln Ile Trp Ser Leu Glu Ser Phe Leu Gly Leu

165 170 175

Glu Pro Asp Glu Thr Thr Thr Glu Leu Ser Gly Ser Ala Glu Pro Asp
180 185 190

Ser Val Asp Leu Trp Met Leu Asp Asp Leu Val Thr His His Gln Gln
195 200 205

Gln Pro Gln Leu Phe Tyr
210

<210> 227
<211> 1190
<212> DNA
<213> Gossypium hirsutum

<400> 227
ataaccgtcg gtctataaat cctagacccc aataccaaag ctttttccat ctgaaaaaaa 60

aacaggtttt cttttaagat aacacccaaa aatgtgtgga ggtgcaatta tttccgattt 120

cgtcgccccc aaatatgacc ggaaattgac cggcgatgac ctttggtctg aacttgacat 180

attctccgac ctcctaggtt tcgacaacaa tggcaaaagc tttgtcaatc atcagtttca 240

taacaacaac aacaacaagc tcattaatcc aaaagacaat caacttaaca aagagagaag 300

cgagacgatt cagaagacaa gccaagtcac taaaaaggtt gaaaagactc agcgaactcg 360

gaaaaacttt tacagaggaa taagacaaag accatggggc aaatgggcag ctgagattag 420

agaccctcaa aaaggcgtcc gagtttggct cggtacctat aacacagccg aagaagcggc 480

tcgagcctac tatgaagccg ccaagcgtat ccgtggtgat aaagccaagc tcaacttccc 540

tcaaacgccc accaaaaatc aagcagcttc accagctctt actcagcttc ctcctgctaa 600

gaaaaggtgc atcgttcccg agttaactca accgagtttc cagactgact caccacctta 660

tcctatgggt cttgggtatg gaagaagtga agattataag ccgattgaag tggtggagag 720

tgagttggag ttgaaagaac aaatctggag cctggaatca ttcctgggtt tggagactaa 780

ccatgagact atgactcagc tgagtggtaa cggtgggact gactcactgg agctttggac 840

acttgatgac ctcgtaactc agtataagca acggcgctaa catgttcatt agtgggggcg 900

aaaattaaat aatagttagc gttaatcaat gtttttgtgt tacttagggt ttttttattt 960

atgctatcat gcattatata tatatatatg agaataaagg attaacgctt tgctcttgca 1020

tggattagtt agcgttaatc aatatttgtg tgttaattag ggttttttat tatgttgtta 1080

tatatgtata tgcttaagga ctttcatgtt tgtatcaatg cattagttgc attatatata 1140

ttaagaataa agggatttta cacgttgcag tttgaaaaaa aaaaaaaaaa 1190

<210> 228
<211> 262
<212> PRT

<213> Gossypium hirsutum

<400> 228

Met Cys Gly Gly Ala Ile Ile Ser Asp Phe Val Ala Pro Lys Tyr Asp
1               5                   10                  15

Arg Lys Leu Thr Gly Asp Asp Leu Trp Ser Glu Leu Asp Ile Phe Ser
            20                  25                  30

Asp Leu Leu Gly Phe Asp Asn Asn Gly Lys Ser Phe Val Asn His Gln
        35                  40                  45

Phe His Asn Asn Asn Asn Asn Lys Leu Ile Asn Pro Lys Asp Asn Gln
    50                  55                  60

Leu Asn Lys Glu Arg Ser Glu Thr Ile Gln Lys Thr Ser Gln Val Thr
65                  70                  75                  80

Lys Lys Val Glu Lys Thr Gln Arg Thr Arg Lys Asn Phe Tyr Arg Gly
                85                  90                  95

Ile Arg Gln Arg Pro Trp Gly Lys Trp Ala Ala Glu Ile Arg Asp Pro
            100                 105                 110

Gln Lys Gly Val Arg Val Trp Leu Gly Thr Tyr Asn Thr Ala Glu Glu
        115                 120                 125

Ala Ala Arg Ala Tyr Tyr Glu Ala Ala Lys Arg Ile Arg Gly Asp Lys
    130                 135                 140

Ala Lys Leu Asn Phe Pro Gln Thr Pro Thr Lys Asn Gln Ala Ala Ser
145                 150                 155                 160

Pro Ala Leu Thr Gln Leu Pro Pro Ala Lys Lys Arg Cys Ile Val Pro
                165                 170                 175

Glu Leu Thr Gln Pro Ser Phe Gln Thr Asp Ser Pro Pro Tyr Pro Met
            180                 185                 190

Gly Leu Gly Tyr Gly Arg Ser Glu Asp Tyr Lys Pro Ile Glu Val Val
        195                 200                 205

Glu Ser Glu Leu Glu Leu Lys Glu Gln Ile Trp Ser Leu Glu Ser Phe
    210                 215                 220

Leu Gly Leu Glu Thr Asn His Glu Thr Met Thr Gln Leu Ser Gly Asn
225                 230                 235                 240

Gly Gly Thr Asp Ser Leu Glu Leu Trp Thr Leu Asp Asp Leu Val Thr

245 250 255

Gln Tyr Lys Gln Arg Arg
260

<210> 229
<211> 1460
<212> DNA
<213> Manihot esculenta

<400> 229
ctgcagcgac aaccaccatg tgtggcggtg ctatcatctc cgacttcatc cctgccaccg 60

ctgctggccg atcctcgcga cggttgacag cggactttct ctggcctgat ctaaagaagc 120

ccattgggaa aatcgttggt gaccttgacg atgatttcga ggctgatttc caggagttta 180

aggatgagtc tgatgtcgat gaggaagatg acgttttgtt tgatgtcaag cctttctctt 240

tctctgctac tgcttctcct cctcctcgca atcgcagtcc ttcacgtggt tctacagctg 300

taaagtctgt ggaatttaat gggctagctg aaaaatctgc aaagagaaag agaaaaaacc 360

agtacagagg aatccggcag cgcccatggg gaaaatgggc tgctgagatt cgtgacccca 420

ggaaaggggt gcgtgtctgg ctaggaacat tcaatactgc tgaagaagct gcaagagcgt 480

atgatgctga ggcacgtaga attcgtggca agaaagctaa agtgaacttt cctgatgaag 540

ctccacgtgc ttccccaaag cggacaatga aggcaaaccc tcagaaacca cttcctaaga 600

gaaatgctac tgagagtatg agttacttga acaatccaga tcaggactac ttcaatactt 660

tgggctctgt tgatgagaaa ccactagtga gccagtttga tttgatggac tcttttcctg 720

ccaatggaga tgctacagtc aaatctattc ctccatgtga taatgttccc acgtttttca 780

attctgatca gggaagcaac tcatttgaat gttccgactt tggatggggg gagcaggcct 840

caaagactcc tgaaatctca tctgttcttt cagctactcc agaaattgat gaatcacttt 900

tcatggatga tgctaaccct aaaaagaaga tgaagtctga ctctgaaaat gcagttccta 960

tagaagaaag caatggaaaa tctctatcag aggagttgtt ggcttttgac aaccagatga 1020

actttcagat gccttatctt gagggaagtt gggaggcttc acttgatggc ttccttaatg 1080

gagacgtgac tcaggatggt ggaaatccaa tggacctgtg gagctttgat gacctcccta 1140

atatggttgg gggagtttat tgagcaaaat cataattgcc ggttggcttc tgtaaataag 1200

gctacatgga tggttttctc tctgaaatgt attacaagca catctgaaca tgcttaatcc 1260

tttgaggaga gtgtcctagg gagcttttgg tacaagagtt gtagtaaata ggaatattta 1320

gtttcccttt tacattttga gacactggac ttgggttgtt caatttaata actgtaattg 1380

acaatgtgcg tgtgatttgt gtttaaaact gatataaatt ttatctctac tgttgttttt 1440

aaaaaaaaaa aaaaaaaaaa 1460

<210> 230

<211> 381
<212> PRT
<213> Manihot esculenta

<400> 230

```
Met Cys Gly Gly Ala Ile Ile Ser Asp Phe Ile Pro Ala Thr Ala Ala
1               5                   10                  15

Gly Arg Ser Ser Arg Arg Leu Thr Ala Asp Phe Leu Trp Pro Asp Leu
            20                  25                  30

Lys Lys Pro Ile Gly Lys Ile Val Gly Asp Leu Asp Asp Asp Phe Glu
        35                  40                  45

Ala Asp Phe Gln Glu Phe Lys Asp Glu Ser Asp Val Asp Glu Glu Asp
    50                  55                  60

Asp Val Leu Phe Asp Val Lys Pro Phe Ser Phe Ser Ala Thr Ala Ser
65                  70                  75                  80

Pro Pro Pro Arg Asn Arg Ser Pro Ser Arg Gly Ser Thr Ala Val Lys
                85                  90                  95

Ser Val Glu Phe Asn Gly Leu Ala Glu Lys Ser Ala Lys Arg Lys Arg
            100                 105                 110

Lys Asn Gln Tyr Arg Gly Ile Arg Gln Arg Pro Trp Gly Lys Trp Ala
        115                 120                 125

Ala Glu Ile Arg Asp Pro Arg Lys Gly Val Arg Val Trp Leu Gly Thr
    130                 135                 140

Phe Asn Thr Ala Glu Glu Ala Ala Arg Ala Tyr Asp Ala Glu Ala Arg
145                 150                 155                 160

Arg Ile Arg Gly Lys Lys Ala Lys Val Asn Phe Pro Asp Glu Ala Pro
                165                 170                 175

Arg Ala Ser Pro Lys Arg Thr Met Lys Ala Asn Pro Gln Lys Pro Leu
            180                 185                 190

Pro Lys Arg Asn Ala Thr Glu Ser Met Ser Tyr Leu Asn Asn Pro Asp
        195                 200                 205

Gln Asp Tyr Phe Asn Thr Leu Gly Ser Val Asp Glu Lys Pro Leu Val
    210                 215                 220

Ser Gln Phe Asp Leu Met Asp Ser Phe Pro Ala Asn Gly Asp Ala Thr
225                 230                 235                 240
```

```
Val Lys Ser Ile Pro Pro Cys Asp Asn Val Pro Thr Phe Phe Asn Ser
                245                 250                 255

Asp Gln Gly Ser Asn Ser Phe Glu Cys Ser Asp Phe Gly Trp Gly Glu
            260                 265                 270

Gln Ala Ser Lys Thr Pro Glu Ile Ser Ser Val Leu Ser Ala Thr Pro
        275                 280                 285

Glu Ile Asp Glu Ser Leu Phe Met Asp Asp Ala Asn Pro Lys Lys Lys
    290                 295                 300

Met Lys Ser Asp Ser Glu Asn Ala Val Pro Ile Glu Glu Ser Asn Gly
305                 310                 315                 320

Lys Ser Leu Ser Glu Glu Leu Leu Ala Phe Asp Asn Gln Met Asn Phe
                325                 330                 335

Gln Met Pro Tyr Leu Glu Gly Ser Trp Glu Ala Ser Leu Asp Gly Phe
            340                 345                 350

Leu Asn Gly Asp Val Thr Gln Asp Gly Gly Asn Pro Met Asp Leu Trp
        355                 360                 365

Ser Phe Asp Asp Leu Pro Asn Met Val Gly Gly Val Tyr
    370                 375                 380


<210>  231
<211>  1409
<212>  DNA
<213>  Populus alba x Populus tremula

<400>  231
ataatcacca tcaatcaaca tgtgtggcgg tgctatcatc tcaggcttca tcgctccgac      60

aaccaccgct cgatcttctc ggcggttgac ctcgggcttt gagtggcttg agccgaagaa     120

acccttcaac aacaagcact tgaagccagt tgttgctgat cccgaagatg attttgaggc     180

tgatcttcaa gagtttaagg acgagtctga tgtcgacgag gattatgatg tctttgctga     240

tgccaagcct tttgctttct ctgccagtgc ttctgaacct gctaaaaaac gtgggctccc     300

tcgtggttct actgctgtta aatctgctgg attcagtgga cttgctaaaa actcagcaaa     360

gaggaagaga aagaaccagt ttagaggaat taggcagcgt ccatggggaa aatgggctgc     420

tgagattcgt gatcccagga aaggggtacg tgtctggttg ggaacattca atactgcaga     480

agaagctgct agagcatatg attctgaggc acgtagaatt cgtggcaaga aagcgaaggt     540

gaacttccct gatgaagctc catgtgcttc agcaaggcat ccaattaagg aaaactcaca     600

gaaacgactt acaaaggcaa atttaagcca ggattttagt tacttgagca acccagaaac     660
```

ggattataat aatatgggct ttgtggaaga gaaaccacaa gtgagccagt ttggaataat 720

gaattctatc ccggtcaatg gagattctgg ggtgacgccc ttaactcctt ctgacaatgc 780

ttctatgtat ttcaattctg acaaggggag caactcattt gattgtgact ttgggtgggg 840

agaacaaggc gctgaaatct tgtctgttct tgcagcaact ccagaagttg atgaatccgt 900

ctttgtggaa gctaatccta agaagttgaa atcatacact gagtatgcag tgcctgttga 960

agagaggaat ggaaaatctc tgtccgaaga gttgctggct tttgacaatc agttgatgaa 1020

ccttcagatg ccagatcttg tgggtaactg ggaggcttct cttgatagct tccttaatgg 1080

agacacaact caggatggca caaacgcagt ggacttgtgg agcttcgaag acttcccctc 1140

catggttggg ggagtttatt gagccaactt ttctgtgctt gccaggtttt gtaaataata 1200

aggctacatg aatggttgtt tatctgagat gggaatggag tgcaacacag atgaacatgc 1260

ttagccttgg tgtgggagaa gcattctcag gagcattttc catataaaag tagtacatcg 1320

gtttcccgtt tcattacatt tggagtcact ggactttgga agttggattc gatgactgta 1380

atttgacaat gtggtgtgac ttatttaga 1409

<210> 232
<211> 380
<212> PRT
<213> Populus alba x Populus tremula

<400> 232

Met Cys Gly Gly Ala Ile Ile Ser Gly Phe Ile Ala Pro Thr Thr Thr
1 5 10 15

Ala Arg Ser Ser Arg Arg Leu Thr Ser Gly Phe Glu Trp Leu Glu Pro
20 25 30

Lys Lys Pro Phe Asn Asn Lys His Leu Lys Pro Val Val Ala Asp Pro
35 40 45

Glu Asp Asp Phe Glu Ala Asp Leu Gln Glu Phe Lys Asp Glu Ser Asp
50 55 60

Val Asp Glu Asp Tyr Asp Val Phe Ala Asp Ala Lys Pro Phe Ala Phe
65 70 75 80

Ser Ala Ser Ala Ser Glu Pro Ala Lys Lys Arg Gly Leu Pro Arg Gly
85 90 95

Ser Thr Ala Val Lys Ser Ala Gly Phe Ser Gly Leu Ala Lys Asn Ser
100 105 110

Ala Lys Arg Lys Arg Lys Asn Gln Phe Arg Gly Ile Arg Gln Arg Pro
115 120 125

```
Trp Gly Lys Trp Ala Ala Glu Ile Arg Asp Pro Arg Lys Gly Val Arg
    130                 135                 140

Val Trp Leu Gly Thr Phe Asn Thr Ala Glu Glu Ala Ala Arg Ala Tyr
145                 150                 155                 160

Asp Ser Glu Ala Arg Arg Ile Arg Gly Lys Lys Ala Lys Val Asn Phe
                165                 170                 175

Pro Asp Glu Ala Pro Cys Ala Ser Ala Arg His Pro Ile Lys Glu Asn
            180                 185                 190

Ser Gln Lys Arg Leu Thr Lys Ala Asn Leu Ser Gln Asp Phe Ser Tyr
        195                 200                 205

Leu Ser Asn Pro Glu Thr Asp Tyr Asn Asn Met Gly Phe Val Glu Glu
    210                 215                 220

Lys Pro Gln Val Ser Gln Phe Gly Ile Met Asn Ser Ile Pro Val Asn
225                 230                 235                 240

Gly Asp Ser Gly Val Thr Pro Leu Thr Pro Ser Asp Asn Ala Ser Met
                245                 250                 255

Tyr Phe Asn Ser Asp Lys Gly Ser Asn Ser Phe Asp Cys Asp Phe Gly
            260                 265                 270

Trp Gly Glu Gln Gly Ala Glu Ile Leu Ser Val Leu Ala Ala Thr Pro
        275                 280                 285

Glu Val Asp Glu Ser Val Phe Val Glu Ala Asn Pro Lys Lys Leu Lys
    290                 295                 300

Ser Tyr Thr Glu Tyr Ala Val Pro Val Glu Glu Arg Asn Gly Lys Ser
305                 310                 315                 320

Leu Ser Glu Glu Leu Leu Ala Phe Asp Asn Gln Leu Met Asn Leu Gln
                325                 330                 335

Met Pro Asp Leu Val Gly Asn Trp Glu Ala Ser Leu Asp Ser Phe Leu
            340                 345                 350

Asn Gly Asp Thr Thr Gln Asp Gly Thr Asn Ala Val Asp Leu Trp Ser
        355                 360                 365

Phe Glu Asp Phe Pro Ser Met Val Gly Gly Val Tyr
    370                 375                 380
```

<210> 233
<211> 1523
<212> DNA
<213> Trifolium pratense

<400> 233
gaatcaatca acaaaacaaa acccagtaaa aatttcaatc ctttttaaca aaatcttcaa 60

ttgggtataa gaaagtttca atctttttct tgttaagata aagacaaaga tctgacaaaa 120

acagaaagat gtgtggtggt gctattattt ccgacttcat tccagctgcg gcggcggtgg 180

gtggttcccg ccgtgtcact gctgatatct tgtggccaaa tttgaggaaa actggttcca 240

aaaaatcatc ttttttgctt gacgatgatt ttgaagctgg tttcagacag tttaaggatg 300

attctgattt cgatgaagac gaggatgaag atgacgatga agggttgttg gtcggtgtca 360

aaggttttac ctttgctgct tctaacaaca aatcttcaag gaacttctct cgtggctcgg 420

ctggtgcaaa atccgtggca tcgaaatcaa atgagcaagc tgaaaaggaa tcaaagagaa 480

agaggaagaa tcagtatagg ggtatccgcc aacgtccatg gggaaaatgg gcagctgaga 540

tccgtgatcc aaggaaagga gtccgtgttt ggctcggaac tttcaacact gctgaagaag 600

ctgcaagagc ttacgatgct gaagctagga gaatccgtgg caagaaagcc aaggtgaatt 660

tccccgatga ggctccaaat gcttcctcaa aacgtctgaa gacaaatcct gataaccagc 720

tgttgaacaa aaatctgaac tctttcaagc cgaacggaaa caacaaaatg ttcaatttca 780

gcgagaatat ggagaacttc tattctccta tggatcaggt tgaacagaaa ccattggtta 840

acaaccaata tggtgctgct gacattggag ccttcactgg aaacggagtt caccttgcac 900

ctgctgatgt taatgcttat ttcagttctg agcattcaag caactcgttt gattattctg 960

atttatgttg gggggaacaa ggcccgaaaa cccctgagat ttcctcggtg ttttctgctc 1020

ctctcgaagc tgaacctcag atgaacatgc agtctaacaa ctctcaggat atgctaccta 1080

tgcaagctga atctgcaaag acactctctg aggagcttgc agatatcgaa tcccagctga 1140

agttcttcga gaactcattc gatgataact ggagtgatgc ttcacttgcg tctttgctcg 1200

gtgctgatgt aactcaggat gctggaaaca caatgaacct ttggagcttt gatgacctgc 1260

cttccatcgc aggcggagtt ttctgaacaa ctttcgcctc accggttatg taaataaagc 1320

tacaagttag taactttcag tttacattcg gtttggttca ttttgttttt acaaggtgga 1380

taattaaatt gttttgtttt caggattgtt ggtttatttt tatattgttg atggaatcag 1440

aaacaagaac aagttaccag ctttaattaa gcgtggagtt ttattctctt gtggcaaaaa 1500

taaattattt ataggttttt att 1523

<210> 234
<211> 385
<212> PRT
<213> Trifolium pratense

<400> 234

```
Met Cys Gly Gly Ala Ile Ile Ser Asp Phe Ile Pro Ala Ala Ala Ala
1               5                   10                  15

Val Gly Gly Ser Arg Arg Val Thr Ala Asp Ile Leu Trp Pro Asn Leu
            20                  25                  30

Arg Lys Thr Gly Ser Lys Lys Ser Ser Phe Leu Leu Asp Asp Asp Phe
        35                  40                  45

Glu Ala Gly Phe Arg Gln Phe Lys Asp Asp Ser Asp Phe Asp Glu Asp
    50                  55                  60

Glu Asp Glu Asp Asp Asp Glu Gly Leu Leu Val Gly Val Lys Gly Phe
65                  70                  75                  80

Thr Phe Ala Ala Ser Asn Asn Lys Ser Ser Arg Asn Phe Ser Arg Gly
                85                  90                  95

Ser Ala Gly Ala Lys Ser Val Ala Ser Lys Ser Asn Glu Gln Ala Glu
            100                 105                 110

Lys Glu Ser Lys Arg Lys Arg Lys Asn Gln Tyr Arg Gly Ile Arg Gln
        115                 120                 125

Arg Pro Trp Gly Lys Trp Ala Ala Glu Ile Arg Asp Pro Arg Lys Gly
    130                 135                 140

Val Arg Val Trp Leu Gly Thr Phe Asn Thr Ala Glu Glu Ala Ala Arg
145                 150                 155                 160

Ala Tyr Asp Ala Glu Ala Arg Arg Ile Arg Gly Lys Lys Ala Lys Val
                165                 170                 175

Asn Phe Pro Asp Glu Ala Pro Asn Ala Ser Ser Lys Arg Leu Lys Thr
            180                 185                 190

Asn Pro Asp Asn Gln Leu Leu Asn Lys Asn Leu Asn Ser Phe Lys Pro
        195                 200                 205

Asn Gly Asn Asn Lys Met Phe Asn Phe Ser Glu Asn Met Glu Asn Phe
    210                 215                 220

Tyr Ser Pro Met Asp Gln Val Glu Gln Lys Pro Leu Val Asn Asn Gln
225                 230                 235                 240

Tyr Gly Ala Ala Asp Ile Gly Ala Phe Thr Gly Asn Gly Val His Leu
                245                 250                 255
```

Ala Pro Ala Asp Val Asn Ala Tyr Phe Ser Ser Glu His Ser Ser Asn
260 265 270

Ser Phe Asp Tyr Ser Asp Leu Cys Trp Gly Glu Gln Gly Pro Lys Thr
275 280 285

Pro Glu Ile Ser Ser Val Phe Ser Ala Pro Leu Glu Ala Glu Pro Gln
290 295 300

Met Asn Met Gln Ser Asn Asn Ser Gln Asp Met Leu Pro Met Gln Ala
305 310 315 320

Glu Ser Ala Lys Thr Leu Ser Glu Glu Leu Ala Asp Ile Glu Ser Gln
325 330 335

Leu Lys Phe Phe Glu Asn Ser Phe Asp Asp Asn Trp Ser Asp Ala Ser
340 345 350

Leu Ala Ser Leu Leu Gly Ala Asp Val Thr Gln Asp Ala Gly Asn Thr
355 360 365

Met Asn Leu Trp Ser Phe Asp Asp Leu Pro Ser Ile Ala Gly Gly Val
370 375 380

Phe
385

<210> 235
<211> 1539
<212> DNA
<213> Glycine max

<400> 235
attgagccaa agctttgtat tttctgcgat aattttccat tgggtggaag aaagtctcaa 60

cctttattcg aaagagcaag gatctgagtt gagttgagtg atcatgtgtg gtggtgcgat 120

tatctccgac ttcataccgg caggtcccgc cagcggggcg cggcgcgtga ccgccgacat 180

cctgtggccg agtttgagga agcgcttctc gaagccgctg ctggacgatg atttcgaggc 240

tgggttcaga gaattcaagg atgattcgga aatcgaggat gttgatgacg aggacgatga 300

agacgaggag gagttgaaga agaagccctt tgggttctct cgctccagca acaaggctgc 360

ttctaagcct ctctctcgtg gagcaacaac tgtgaaatct gtggaatcaa aggggcaagc 420

tgagaagtgt gccaagagaa agaggaagaa ccagtatcgc ggaatccgcc agcgtccatg 480

gggaaagtgg gctgctgaga ttcgcgaccc aagaaagggg gttcgtgttt ggcttggaac 540

tttcagcact gctgaagaag ctgcaagagc ttacgatgct gaagcaagga ggatccgtgg 600

caagaaagcc aaggtgaatt tccctgatga gccttcaggc gctgcttcct caaaacgtct 660

caaggcgaat ccagaggctc agccaatgaa gaaaaatctg aactctgtga agccgaaaat 720

aaaccagatg ttcaattttg gtgacaatct tgagggctac tacagcccta tagatcaggt 780

ggaacagaaa ccactggtta accagtatgt taaccgtgcc ccgtttgctg gaaatggagt 840

tcaagtctca cctgttactc catctgctga tgttactgct tacttcagct ctgagcattc 900

gagcaactcg tttgattatt ctgaccttgg atggggtgaa caagtcccca agacccccga 960

gatctcatcc ttgctttctg ctgctccttt ggagggtgct gctgatcagg ttcagaagac 1020

caacaactcg caggatgtgg tggctgcaca agatgattct gcaaaaaccc tttccgaaga 1080

gcttgcagac attgaatccc agctcaagtt ctttgagacc ccttcttttc ttgatgaagc 1140

ctgggctgat gctacattgg cgtctttgct cggcggagac gcaactcatg acgccgccgg 1200

aaaccctatg aacctttgga gcttcgacga cctgccttcc atggcaggag tcttctgaac 1260

accctttatc tccccttta tgtaaataaa gctacaagaa ttgtgatcgt gatgttggtg 1320

atggagtcca cagccaagaa acctgcttaa agcttatgtg gagtttattt tatcttgtag 1380

ctaatgcagt agtataggac tatatatagg tttttattat agggtatcct tttgtgaact 1440

caaagacctc gttttcaggg gattttctgt ttgatgtcct taaggattat caattatgtt 1500

atatatggtc ttggataaaa ataaaaaaaa aaaaaaaaa 1539

<210> 236
<211> 384
<212> PRT
<213> Glycine max

<400> 236

Met Cys Gly Gly Ala Ile Ile Ser Asp Phe Ile Pro Ala Gly Pro Ala
1 5 10 15

Ser Gly Ala Arg Arg Val Thr Ala Asp Ile Leu Trp Pro Ser Leu Arg
20 25 30

Lys Arg Phe Ser Lys Pro Leu Leu Asp Asp Asp Phe Glu Ala Gly Phe
35 40 45

Arg Glu Phe Lys Asp Asp Ser Glu Ile Glu Asp Val Asp Asp Glu Asp
50 55 60

Asp Glu Asp Glu Glu Glu Leu Lys Lys Lys Pro Phe Gly Phe Ser Arg
65 70 75 80

Ser Ser Asn Lys Ala Ala Ser Lys Pro Leu Ser Arg Gly Ala Thr Thr
85 90 95

Val Lys Ser Val Glu Ser Lys Gly Gln Ala Glu Lys Cys Ala Lys Arg
100 105 110

Lys Arg Lys Asn Gln Tyr Arg Gly Ile Arg Gln Arg Pro Trp Gly Lys
115 120 125

Trp Ala Ala Glu Ile Arg Asp Pro Arg Lys Gly Val Arg Val Trp Leu
130 135 140

Gly Thr Phe Ser Thr Ala Glu Glu Ala Ala Arg Ala Tyr Asp Ala Glu
145 150 155 160

Ala Arg Arg Ile Arg Gly Lys Lys Ala Lys Val Asn Phe Pro Asp Glu
165 170 175

Pro Ser Gly Ala Ala Ser Ser Lys Arg Leu Lys Ala Asn Pro Glu Ala
180 185 190

Gln Pro Met Lys Lys Asn Leu Asn Ser Val Lys Pro Lys Ile Asn Gln
195 200 205

Met Phe Asn Phe Gly Asp Asn Leu Glu Gly Tyr Tyr Ser Pro Ile Asp
210 215 220

Gln Val Glu Gln Lys Pro Leu Val Asn Gln Tyr Val Asn Arg Ala Pro
225 230 235 240

Phe Ala Gly Asn Gly Val Gln Val Ser Pro Val Thr Pro Ser Ala Asp
245 250 255

Val Thr Ala Tyr Phe Ser Ser Glu His Ser Ser Asn Ser Phe Asp Tyr
260 265 270

Ser Asp Leu Gly Trp Gly Glu Gln Val Pro Lys Thr Pro Glu Ile Ser
275 280 285

Ser Leu Leu Ser Ala Ala Pro Leu Glu Gly Ala Ala Asp Gln Val Gln
290 295 300

Lys Thr Asn Asn Ser Gln Asp Val Val Ala Ala Gln Asp Asp Ser Ala
305 310 315 320

Lys Thr Leu Ser Glu Glu Leu Ala Asp Ile Glu Ser Gln Leu Lys Phe
325 330 335

Phe Glu Thr Pro Ser Phe Leu Asp Glu Ala Trp Ala Asp Ala Thr Leu
340 345 350

Ala Ser Leu Leu Gly Gly Asp Ala Thr His Asp Ala Ala Gly Asn Pro
355 360 365

Met Asn Leu Trp Ser Phe Asp Asp Leu Pro Ser Met Ala Gly Val Phe

370 375 380

<210> 237
<211> 458
<212> DNA
<213> Narcissus pseudonarcissus

<400> 237
atgtgtggag gagcaataat ctctgatttc atacctccat caaggtcaag gaagctcact 60

gccgattact tgtggcccaa tctcaacaag gggaaggaca agaagaagag ctttgggttt 120

gaagatgact ttgaagctga cttcaatgag tttgaagatg agtctgagga gggggaatct 180

gaagctgttg atgtcaaacc ctttaagttt ggggctaaag ctggcttttc tagagaggga 240

tcgacttatc tgaaacccat cgaactcaat ggagctgctg aacgatcatc caaaaggaag 300

aggaagaacc aatacagagg tatccgtcag cgtccatggg gtaaatgggc agctgagata 360

agagatccta acaaaggagt tcgtgtttgg ctgggaactt ttaacacagc tgaagaagct 420

gcgagagcct atgatgatga agcccgtagg atccgtgg 458

<210> 238
<211> 153
<212> PRT
<213> Narcissus pseudonarcissus

<400> 238

Met Cys Gly Gly Ala Ile Ile Ser Asp Phe Ile Pro Pro Ser Arg Ser
1 5 10 15

Arg Lys Leu Thr Ala Asp Tyr Leu Trp Pro Asn Leu Asn Lys Gly Lys
20 25 30

Asp Lys Lys Lys Ser Phe Gly Phe Glu Asp Asp Phe Glu Ala Asp Phe
35 40 45

Asn Glu Phe Glu Asp Glu Ser Glu Glu Gly Glu Ser Glu Ala Val Asp
50 55 60

Val Lys Pro Phe Lys Phe Gly Ala Lys Ala Gly Phe Ser Arg Glu Gly
65 70 75 80

Ser Thr Tyr Leu Lys Pro Ile Glu Leu Asn Gly Ala Ala Glu Arg Ser
85 90 95

Ser Lys Arg Lys Arg Lys Asn Gln Tyr Arg Gly Ile Arg Gln Arg Pro
100 105 110

Trp Gly Lys Trp Ala Ala Glu Ile Arg Asp Pro Asn Lys Gly Val Arg
115 120 125

Val Trp Leu Gly Thr Phe Asn Thr Ala Glu Glu Ala Ala Arg Ala Tyr
130 135 140

Asp Asp Glu Ala Arg Arg Ile Arg Gly
145 150

<210> 239
<211> 823
<212> DNA
<213> Arabidopsis thaliana

<400> 239
agtaatttag cagcaacaaa cagagaaaat gtgtggcggt gctattattt ccgattatgc 60

ccctctcgtc accaaggcca agggccgtaa gctcacggct gaggaactct ggtcagagct 120

cgatgcttcc gccgccgacg acttctgggg tttctattcc acctccaaac tccatcccac 180

caaccaagtt aacgtgaaag aggaggaggc ggtgaagaag gagcaggcaa cagagccggg 240

gaaacggagg aagaggaaga atgtttatag agggatacgt aagcgtccat ggggaaaatg 300

ggcggcggag attcgagatc cacgaaaagg tgtcagagtt tggcttggta cgttcaacac 360

ggcggaggaa gctgccatgg cttatgatgt agcggcgaag cagatccgtg gtgagaaagc 420

caagctcaac ttcccagatc tggatcatca tccttctact cctccgccat cgtctacttc 480

actaagatta tccgatcagc caccggcgaa gaaggtttgc gttgtctctc agagcgagtt 540

agctcagccg agtttcccgg tggagtgtgt tggatttgga aagggggaag agtttcagaa 600

cctgatgtac ggatttgaac cggattatga tttgaaacag cagatatcga gcttggagtc 660

gttccttgag cttgacggta ccacggcgga gcaaccgagt caactagatg agtctgtttg 720

cgatgtggat atgtggatgc ttgatgatgt cattgcgtcg tatgagtaaa aggaaaaaaa 780

aacagaagat aatgtaatat gtaaaaaaaa aaactaaatt act 823

<210> 240
<211> 246
<212> PRT
<213> Arabidopsis thaliana

<400> 240

Met Cys Gly Gly Ala Ile Ile Ser Asp Tyr Ala Pro Leu Val Thr Lys
1 5 10 15

Ala Lys Gly Arg Lys Leu Thr Ala Glu Glu Leu Trp Ser Glu Leu Asp
20 25 30

Ala Ser Ala Ala Asp Asp Phe Trp Gly Phe Tyr Ser Thr Ser Lys Leu
35 40 45

His Pro Thr Asn Gln Val Asn Val Lys Glu Glu Glu Ala Val Lys Lys
50 55 60

```
Glu Gln Ala Thr Glu Pro Gly Lys Arg Arg Lys Arg Lys Asn Val Tyr
65                  70                  75                  80

Arg Gly Ile Arg Lys Arg Pro Trp Gly Lys Trp Ala Ala Glu Ile Arg
                85                  90                  95

Asp Pro Arg Lys Gly Val Arg Val Trp Leu Gly Thr Phe Asn Thr Ala
            100                 105                 110

Glu Glu Ala Ala Met Ala Tyr Asp Val Ala Ala Lys Gln Ile Arg Gly
        115                 120                 125

Glu Lys Ala Lys Leu Asn Phe Pro Asp Leu Asp His His Pro Ser Thr
    130                 135                 140

Pro Pro Pro Ser Ser Thr Ser Leu Arg Leu Ser Asp Gln Pro Pro Ala
145                 150                 155                 160

Lys Lys Val Cys Val Val Ser Gln Ser Glu Leu Ala Gln Pro Ser Phe
                165                 170                 175

Pro Val Glu Cys Val Gly Phe Gly Lys Gly Glu Glu Phe Gln Asn Leu
            180                 185                 190

Met Tyr Gly Phe Glu Pro Asp Tyr Asp Leu Lys Gln Gln Ile Ser Ser
        195                 200                 205

Leu Glu Ser Phe Leu Glu Leu Asp Gly Thr Thr Ala Glu Gln Pro Ser
    210                 215                 220

Gln Leu Asp Glu Ser Val Cys Asp Val Asp Met Trp Met Leu Asp Asp
225                 230                 235                 240

Val Ile Ala Ser Tyr Glu
                245
```

<210> 241
<211> 918
<212> DNA
<213> Medicago truncatula

<400> 241

```
atgtgtggtg gtgctatcat cgctgacttc attcctcgcc gtgacggccg ccgtctcacc      60

gcctcggagc tctggcctaa ctcatttggc caacagattg actcctcaaa cttcggtttt     120

tctcatactg ctgctgatca acaaccaccc agcactctca aaagatcaca gcctcccaaa     180

gttaatgaac gagttgagaa gccactgaaa aaacagagga agaatctcta tagaggaatt     240

cgacagcgtc catggggaaa atgggctgca gagattcgtg atccaagaaa aggagttcgt     300
```

```
gtttggcttg gtacattcaa cactgctgaa gaagctgcta gagcttacga caaagaagct    360
cgaaaaatcc gtggcaaaaa agctaaggtt aattttccta acgaagatga tgaatatacc    420
attcatgcta ctcgtcgcta caataataat cctccaccga ttcgaccaca gaagcttcct    480
ctatatcatc aacaacacta tcagaagaat ctgaacttgg aatttggtta tgatctgaac    540
caaactgaca caattcatgc tatcaatact ggttctggtg gtgatgagaa ttctttattt    600
gggtctggtt cagtttcaga ggttggtttt tctgtgatgg agttcaatgg tggttccaat    660
cagaatgaat tcggttattt tggtggtgtt gtgaatgaac atgaaaaaga gaaagagaaa    720
gtggtagaac aagaaacaca tgttgttgaa caagctgaag ctgaattagc aaaaaatgag    780
gtacaagaat tgtctgatga attgttggca tacgaggatt acatgaagtt ttatcagatt    840
tactatgatg gacaatcagt gatgccacct aataatgttc aggaacatgt ggttggagat    900
ttatggagct ttgattga                                                  918

<210>  242
<211>  305
<212>  PRT
<213>  Medicago truncatula

<400>  242

Met Cys Gly Gly Ala Ile Ile Ala Asp Phe Ile Pro Arg Arg Asp Gly
1               5                   10                  15

Arg Arg Leu Thr Ala Ser Glu Leu Trp Pro Asn Ser Phe Gly Gln Gln
            20                  25                  30

Ile Asp Ser Ser Asn Phe Gly Phe Ser His Thr Ala Ala Asp Gln Gln
        35                  40                  45

Pro Pro Ser Thr Leu Lys Arg Ser Gln Pro Pro Lys Val Asn Glu Arg
    50                  55                  60

Val Glu Lys Pro Leu Lys Lys Gln Arg Lys Asn Leu Tyr Arg Gly Ile
65                  70                  75                  80

Arg Gln Arg Pro Trp Gly Lys Trp Ala Ala Glu Ile Arg Asp Pro Arg
                85                  90                  95

Lys Gly Val Arg Val Trp Leu Gly Thr Phe Asn Thr Ala Glu Glu Ala
            100                 105                 110

Ala Arg Ala Tyr Asp Lys Glu Ala Arg Lys Ile Arg Gly Lys Lys Ala
        115                 120                 125

Lys Val Asn Phe Pro Asn Glu Asp Asp Glu Tyr Thr Ile His Ala Thr
    130                 135                 140
```

Arg Arg Tyr Asn Asn Asn Pro Pro Pro Ile Arg Pro Gln Lys Leu Pro
145                 150                 155                 160

Leu Tyr His Gln Gln His Tyr Gln Lys Asn Leu Asn Leu Glu Phe Gly
                165                 170                 175

Tyr Asp Leu Asn Gln Thr Asp Thr Ile His Ala Ile Asn Thr Gly Ser
            180                 185                 190

Gly Gly Asp Glu Asn Ser Leu Phe Gly Ser Gly Ser Val Ser Glu Val
        195                 200                 205

Gly Phe Ser Val Met Glu Phe Asn Gly Gly Ser Asn Gln Asn Glu Phe
    210                 215                 220

Gly Tyr Phe Gly Gly Val Val Asn Glu His Glu Lys Glu Lys Glu Lys
225                 230                 235                 240

Val Val Glu Gln Glu Thr His Val Val Glu Gln Ala Glu Ala Glu Leu
                245                 250                 255

Ala Lys Asn Glu Val Gln Glu Leu Ser Asp Glu Leu Leu Ala Tyr Glu
            260                 265                 270

Asp Tyr Met Lys Phe Tyr Gln Ile Tyr Tyr Asp Gly Gln Ser Val Met
        275                 280                 285

Pro Pro Asn Asn Val Gln Glu His Val Val Gly Asp Leu Trp Ser Phe
    290                 295                 300

Asp
305

<210> 243
<211> 723
<212> DNA
<213> Medicago truncatula

<400> 243
atgtcacccc ctaaaccgta tataaaccaa ctcaaatctc tgactttccc ttcaacttca 60

aaatcttcta aaccttcaaa aactcagttt ccatacatga actacgcctt ctccactacc 120

accctcacct ccgatcaaga actctcagtc atcgtcgctg ccctaaccaa cgtagtctcc 180

ggttccacct ccaccgtcgg cttagatcga atagttcaac cggtgaacat agaaacctgt 240

cgggaatgca acatagcagg atgtttagga tgcaatttct tccctgaaga gaaaaaacaa 300

aaacaaaaac aaaaacaaaa gagagctaag aataataagt acagaggagt gaggcagaga 360

ccatggggaa aatgggctgc tgagattcgt gacccgagac gtgcggttcg tgtttggctt 420

ggaaccttta ccacggcgga ggaagctgct agagcttacg acaatgccgc tatcgagttc 480

cgcgggccga gagccaagct taattttcca cttgttgatg aatctcttaa gcgtactgtt 540

gaggatccag aattggttgt tcatgtaaag gatgaagaaa tgcaaattga gacaacgatg 600

ggatttggga ataatacgac tgaatgtgat ttttgggata gtattgggga agaagctgat 660

tttcaacaac ttatgaggtt tatggattct tctcattcta gaacaggaaa cacttttaat 720

tag 723

<210> 244
<211> 240
<212> PRT
<213> Medicago truncatula

<400> 244

Met Ser Pro Pro Lys Pro Tyr Ile Asn Gln Leu Lys Ser Leu Thr Phe
1 5 10 15

Pro Ser Thr Ser Lys Ser Ser Lys Pro Ser Lys Thr Gln Phe Pro Tyr
20 25 30

Met Asn Tyr Ala Phe Ser Thr Thr Thr Leu Thr Ser Asp Gln Glu Leu
35 40 45

Ser Val Ile Val Ala Ala Leu Thr Asn Val Val Ser Gly Ser Thr Ser
50 55 60

Thr Val Gly Leu Asp Arg Ile Val Gln Pro Val Asn Ile Glu Thr Cys
65 70 75 80

Arg Glu Cys Asn Ile Ala Gly Cys Leu Gly Cys Asn Phe Phe Pro Glu
85 90 95

Glu Lys Lys Gln Lys Gln Lys Gln Lys Gln Lys Arg Ala Lys Asn Asn
100 105 110

Lys Tyr Arg Gly Val Arg Gln Arg Pro Trp Gly Lys Trp Ala Ala Glu
115 120 125

Ile Arg Asp Pro Arg Arg Ala Val Arg Val Trp Leu Gly Thr Phe Thr
130 135 140

Thr Ala Glu Glu Ala Ala Arg Ala Tyr Asp Asn Ala Ala Ile Glu Phe
145 150 155 160

Arg Gly Pro Arg Ala Lys Leu Asn Phe Pro Leu Val Asp Glu Ser Leu
165 170 175

Lys Arg Thr Val Glu Asp Pro Glu Leu Val Val His Val Lys Asp Glu

180 185 190

Glu Met Gln Ile Glu Thr Thr Met Gly Phe Gly Asn Asn Thr Thr Glu
195 200 205

Cys Asp Phe Trp Asp Ser Ile Gly Glu Glu Ala Asp Phe Gln Gln Leu
210 215 220

Met Arg Phe Met Asp Ser Ser His Ser Arg Thr Gly Asn Thr Phe Asn
225 230 235 240

<210> 245
<211> 1138
<212> DNA
<213> Solanum tuberosum

<400> 245
attcggcacg agaccaaaac caaaaccaag cttctctctt gttataatat atatatatat 60

aaaaaaaact cgacctttgt acaaaccatt tttcatatct gtatcaatct ctttgtttct 120

gccattttga gtaaaagagt ttaaatcaca ctaaaatgtg tggtggtgca attctttatg 180

atattattcc tcgtgaccgc cgtttgtcat ccaccgactt atggccaagc tctgctgatt 240

tctggcaaac ttcttctttt tccaagccaa tttccaccca aaatgttcct cccaagccta 300

aacgagctca actctctaga ggtagtgagc agatgaagaa gaggcaaagg aagaatcttt 360

acaggggaat ccgacaacgt ccatggggta aatgggctgc tgaaattcgt gacccgagaa 420

aaagggttag ggtctggtta ggtactttca acactgctga agctgcaaga gcttatgata 480

gagaagctcg taaaatcagg ggaaagaaag ctaaagttaa tttccccaat gaagacgacg 540

accactacta cagtcatcca gagccgcctc ctttgaacat tgtttatgaa tcttatgata 600

ctactagtac ttacaatcaa gaatcaaata actgttaccc cttccactca atcgaaaaca 660

ctgaacctgt tatggaattc gcaattgcta acaaaaattc atctgggtct gcttataatg 720

gaattgaaga tcagaatgtg gaaggagaag agcagacggt gaaaaattca aataacagga 780

tcgtagagga agaggaaaaa acagaggatg aagtgcagat actttctgat gaactgatgg 840

cttatgagtc attgatgaag ttctatgaaa taccgtatgt tgacgggcaa tcagtggcgg 900

cgacggtgaa tccagcggcg gacaccgaag tgggcggtgg ctcgatggag ctttggagtt 960

ttgatgatgt tagtcgtcta caaccaagtt ataatgttag tttgattatt gttttgttta 1020

aattgttgca tctttttagt ttgctgaatt agaactaatt gagtttttgt aatttttaat 1080

caattgtgtt gaaactatat ttttahttca ttactctatt aaaaaaaaaa aaaaaaa 1138

<210> 246
<211> 298
<212> PRT
<213> Solanum tuberosum

<400> 246

Met Cys Gly Gly Ala Ile Leu Tyr Asp Ile Ile Pro Arg Asp Arg Arg
1 5 10 15

Leu Ser Ser Thr Asp Leu Trp Pro Ser Ser Ala Asp Phe Trp Gln Thr
20 25 30

Ser Ser Phe Ser Lys Pro Ile Ser Thr Gln Asn Val Pro Pro Lys Pro
35 40 45

Lys Arg Ala Gln Leu Ser Arg Gly Ser Glu Gln Met Lys Lys Arg Gln
50 55 60

Arg Lys Asn Leu Tyr Arg Gly Ile Arg Gln Arg Pro Trp Gly Lys Trp
65 70 75 80

Ala Ala Glu Ile Arg Asp Pro Arg Lys Arg Val Arg Val Trp Leu Gly
85 90 95

Thr Phe Asn Thr Ala Glu Ala Ala Arg Ala Tyr Asp Arg Glu Ala Arg
100 105 110

Lys Ile Arg Gly Lys Lys Ala Lys Val Asn Phe Pro Asn Glu Asp Asp
115 120 125

Asp His Tyr Tyr Ser His Pro Glu Pro Pro Pro Leu Asn Ile Val Tyr
130 135 140

Glu Ser Tyr Asp Thr Thr Ser Thr Tyr Asn Gln Glu Ser Asn Asn Cys
145 150 155 160

Tyr Pro Phe His Ser Ile Glu Asn Thr Glu Pro Val Met Glu Phe Ala
165 170 175

Ile Ala Asn Lys Asn Ser Ser Gly Ser Ala Tyr Asn Gly Ile Glu Asp
180 185 190

Gln Asn Val Glu Gly Glu Glu Gln Thr Val Lys Asn Ser Asn Asn Arg
195 200 205

Ile Val Glu Glu Glu Glu Lys Thr Glu Asp Glu Val Gln Ile Leu Ser
210 215 220

Asp Glu Leu Met Ala Tyr Glu Ser Leu Met Lys Phe Tyr Glu Ile Pro
225 230 235 240

Tyr Val Asp Gly Gln Ser Val Ala Ala Thr Val Asn Pro Ala Ala Asp
245 250 255

Thr Glu Val Gly Gly Gly Ser Met Glu Leu Trp Ser Phe Asp Asp Val
260 265 270

Ser Arg Leu Gln Pro Ser Tyr Asn Val Ser Leu Ile Ile Val Leu Phe
275 280 285

Lys Leu Leu His Leu Phe Ser Leu Leu Asn
290 295

<210> 247
<211> 1132
<212> DNA
<213> Solanum tuberosum

<400> 247
ctttcactca aaaaaaaaa ggaaaaaatt aagagtaaca aaagatgtgt ggaggtgcca 60

taatctccga ttatgagccc gccggaaact tctaccggaa actctctgct cgtgacctgt 120

gggctgagct ggaccctatc tccgactact ggtcctcttc ctcctcatcc tcaactgtcg 180

aaaaccctta ttccgctcag tcgccggtga ctcactccgt cgataagcct aagaaatcag 240

attccggcaa atctaatcaa ctcaaaaaag gtaataagac tgtgaaggtt gagaaggaga 300

agagtactgg accaaggcag agaaagaaca agtacagagg aataaggcag agaccatggg 360

gaaaatgggc tgctgagatt cgcgatcctc agaagggtgt ccgtgtttgg cttggtacat 420

tcaacacagc agaggatgct gccagagcct atgatgaggc tgctaagcgc attcgtggta 480

acaaggccaa actcaacttc cctgccccat caccacctgc taagcgacag tgcactagca 540

ctgtcgctgc tgatcctcca ccagcactac tccttgagag ttctaacata atatcttata 600

acaattctcc tttaatgaac ttcggatatg atgttcagag ccaaactccc tactacccaa 660

tggaaatgcc cgttgctagt gatgattatg aactcaagga acagatttcc aacttggaat 720

cgttcctgga attggagcca gcagattcat ctgatcagtt ttcagggatc gtcgatcctg 780

atcctcttaa tgtttttctg atggaggatt ttgcttcaac tcagcatcag ttctattgat 840

cctgagttgt ttggtgagtg atgagtgact agtttattag cttttggctg tagtagtagt 900

aatagagaaa aaagtacata tgatatgata ataataagtt gcgtgcctta gcctgcaatt 960

gtaatagtat caatgtttgt tgtcttgtgt tgtttatgct ttctaaatct tggatttacc 1020

ttataatgtt tggtcatttg gtgtatgtat tgtaactata tatggagtac tttattacta 1080

aaaaaaaaaa aaaaaaaaaa aaaaaaaaaa aaaaaaaaaa aaaaaaaaaa aa 1132

<210> 248
<211> 264
<212> PRT
<213> Solanum tuberosum

<400> 248

Met Cys Gly Gly Ala Ile Ile Ser Asp Tyr Glu Pro Ala Gly Asn Phe
1               5                   10                  15

Tyr Arg Lys Leu Ser Ala Arg Asp Leu Trp Ala Glu Leu Asp Pro Ile
            20                  25                  30

Ser Asp Tyr Trp Ser Ser Ser Ser Ser Ser Ser Thr Val Glu Asn Pro
        35                  40                  45

Tyr Ser Ala Gln Ser Pro Val Thr His Ser Val Asp Lys Pro Lys Lys
    50                  55                  60

Ser Asp Ser Gly Lys Ser Asn Gln Leu Lys Lys Gly Asn Lys Thr Val
65                  70                  75                  80

Lys Val Glu Lys Glu Lys Ser Thr Gly Pro Arg Gln Arg Lys Asn Lys
                85                  90                  95

Tyr Arg Gly Ile Arg Gln Arg Pro Trp Gly Lys Trp Ala Ala Glu Ile
            100                 105                 110

Arg Asp Pro Gln Lys Gly Val Arg Val Trp Leu Gly Thr Phe Asn Thr
        115                 120                 125

Ala Glu Asp Ala Ala Arg Ala Tyr Asp Glu Ala Ala Lys Arg Ile Arg
    130                 135                 140

Gly Asn Lys Ala Lys Leu Asn Phe Pro Ala Pro Ser Pro Pro Ala Lys
145                 150                 155                 160

Arg Gln Cys Thr Ser Thr Val Ala Ala Asp Pro Pro Pro Ala Leu Leu
                165                 170                 175

Leu Glu Ser Ser Asn Ile Ile Ser Tyr Asn Asn Ser Pro Leu Met Asn
            180                 185                 190

Phe Gly Tyr Asp Val Gln Ser Gln Thr Pro Tyr Tyr Pro Met Glu Met
        195                 200                 205

Pro Val Ala Ser Asp Asp Tyr Glu Leu Lys Glu Gln Ile Ser Asn Leu
    210                 215                 220

Glu Ser Phe Leu Glu Leu Glu Pro Ala Asp Ser Ser Asp Gln Phe Ser
225                 230                 235                 240

Gly Ile Val Asp Pro Asp Pro Leu Asn Val Phe Leu Met Glu Asp Phe
                245                 250                 255

Ala Ser Thr Gln His Gln Phe Tyr

260

<210> 249
<211> 1259
<212> DNA
<213> Coffea canephora

<400> 249
aagtaccgca aatttgtaag cttagatcat caaaaatgtg tggcggtgca atcctcgaag 60

gcctcatccc gccccgcaac aacaacgacg gcactcgccg tctctcctcc gctgacctct 120

ggccctccgc tgctgatttt tggcccaact ctcaccttgc caagcccagc cgctccaacc 180

caactgatgc cccccacaaa gccacgcacg aaacttgtca cgatccacat gatgaagctg 240

gtggaaagca gcaagtcctt cccaagggtg ccaagaggca gaggaaaaac ttatacaggg 300

ggatcaggca gcgaccatgg gggaaatggg ctgctgagat tcgagaccct aggaaaggcg 360

tgagagtctg gttgggcact ttcaacactg ccgaagaagc tgccagagcc tacgacaaag 420

aagctcgaaa aatcagaggc aagaaagcca aggttaactt cccaaacgag gattcgacca 480

gttatcccac atgcatccct tcgcaaaccc agtaccaaca ggtacacatc cccagccctc 540

ccactttctg tggtccctcc tcctccaatt gcaagttcaa tcagctccgt gttgggtcgt 600

ctgattgcag tgccagtgac tgctatcacg cgaacagcat cgatgattgt gcccgtttca 660

ccaatatgat gggttttcga aatccaagcg aagaggtttc tggttctggt agttcggata 720

atgagtgttt tcttggccac cttaaacaag tagccaaggc ggcggcggag gaggaggcgg 780

cttcggttat aactgaaaat gatacgaaag acaataacaa agcagctgaa agcgaggagt 840

accaagtgga aaagctgtct gaggagctgc tggcctatga gtccttcatg aagttctatc 900

agattcctta tatagatggg cagtccgcag ctgcatcggc caaccctgct caagagctgg 960

caacttctat tcagctttgg agctttgatg atgtctcacc cgccaatcgc ccaatgcctc 1020

tgtaaccgcc aatcgcccaa gttcgatggt cttttttttg ggagtcccaa ttgcgttttg 1080

gtgcctagtt tttgtaggtt ttgctatgta attgacatta acttcttaaa acattgtagg 1140

aaattgttgt taggttgcag gttggtactc ttgaccggct tgtttagtta aaatacctat 1200

atgtaggttg gcactttgta attactgttt ttgtggacaa aaaaaaaaaa aaaaaaaaa 1259

<210> 250
<211> 329
<212> PRT
<213> Coffea canephora

<400> 250

Met Cys Gly Gly Ala Ile Leu Glu Gly Leu Ile Pro Pro Arg Asn Asn
1 5 10 15

Asn Asp Gly Thr Arg Arg Leu Ser Ser Ala Asp Leu Trp Pro Ser Ala
20 25 30

Ala Asp Phe Trp Pro Asn Ser His Leu Ala Lys Pro Ser Arg Ser Asn
35 40 45

Pro Thr Asp Ala Pro His Lys Ala Thr His Glu Thr Cys His Asp Pro
50 55 60

His Asp Glu Ala Gly Gly Lys Gln Gln Val Leu Pro Lys Gly Ala Lys
65 70 75 80

Arg Gln Arg Lys Asn Leu Tyr Arg Gly Ile Arg Gln Arg Pro Trp Gly
85 90 95

Lys Trp Ala Ala Glu Ile Arg Asp Pro Arg Lys Gly Val Arg Val Trp
100 105 110

Leu Gly Thr Phe Asn Thr Ala Glu Glu Ala Ala Arg Ala Tyr Asp Lys
115 120 125

Glu Ala Arg Lys Ile Arg Gly Lys Lys Ala Lys Val Asn Phe Pro Asn
130 135 140

Glu Asp Ser Thr Ser Tyr Pro Thr Cys Ile Pro Ser Gln Thr Gln Tyr
145 150 155 160

Gln Gln Val His Ile Pro Ser Pro Pro Thr Phe Cys Gly Pro Ser Ser
165 170 175

Ser Asn Cys Lys Phe Asn Gln Leu Arg Val Gly Ser Ser Asp Cys Ser
180 185 190

Ala Ser Asp Cys Tyr His Ala Asn Ser Ile Asp Asp Cys Ala Arg Phe
195 200 205

Thr Asn Met Met Gly Phe Arg Asn Pro Ser Glu Glu Val Ser Gly Ser
210 215 220

Gly Ser Ser Asp Asn Glu Cys Phe Leu Gly His Leu Lys Gln Val Ala
225 230 235 240

Lys Ala Ala Ala Glu Glu Glu Ala Ala Ser Val Ile Thr Glu Asn Asp
245 250 255

Thr Lys Asp Asn Asn Lys Ala Ala Glu Ser Glu Glu Tyr Gln Val Glu
260 265 270

Lys Leu Ser Glu Glu Leu Leu Ala Tyr Glu Ser Phe Met Lys Phe Tyr
275 280 285

```
Gln Ile Pro Tyr Ile Asp Gly Gln Ser Ala Ala Ala Ser Ala Asn Pro
    290                 295                 300

Ala Gln Glu Leu Ala Thr Ser Ile Gln Leu Trp Ser Phe Asp Asp Val
305                 310                 315                 320

Ser Pro Ala Asn Arg Pro Met Pro Leu
                325
```

<210> 251
<211> 1394
<212> DNA
<213> Hordeum vulgare

<400> 251

```
caggaagaga aaacaatgtg tggcggcgcc atcctagcgc agctgatccc gccgtcggcg      60
ggccgtccgt cgaagcaggc ggcagcgggc ggccgggccc cgcccacgag ctccaagaag     120
ggcggcgtga gcaagagccg ccacagcagc accccagatg ccgacgacga cgtcttcgag     180
gccgccttcg aggacttcga tgaccacttc gacctgcggg cggaggagga cggcggcgac     240
gaccatgtcg tctttgcatc caagcctgcc ttctctccac gtccggccta cgacggtggc     300
cgcgcggcgc atgcggcgag caggaagaag cgcaccggcc acctccatgg catccggcag     360
cggccgtggg gcaagtgggc ggcggagatc cgcgacccgc acaagggcac ccgcgtctgg     420
ctcggcacgt tcgacacggc cgatgatgcc gcccgggcct acgacgtcgc cgcccgtcgc     480
ctccgtggca gcaaggccaa ggtcaacttc cccgacgcgg ccaggaccgg ggctcgcccg     540
cgccgcgcca gccgtagaac cgcgcagaaa ccgcaatgcc cccctgcgcg gacgacggcg     600
tactctgcca ccgcagcagc acgcgcacag ccggagcagg acgctatgat ggtcaaaccc     660
gagctgatgg agtttttcaa cgtggacgcc atcgtccacc tgaccactgc cgtcgccgcg     720
ctaccgcctg tcacggcgag caccttcgcc gacacgatgc cgagggtcga cgaggactct     780
tctgtgggga gcggcggcgg cgccatgctg ggttcgccg acgagcttgg gttcgatccg      840
ttcatgatgt tccagctacc ctgctcggac atgtacgaat ccgccgacag catcttcgcc     900
ggagacgctg tcatcccgga tgccctcagc gtggacagtg gcatggacgc cgtcagcctc     960
tggagcttcg acgagttccc catggacagc gccattttct gacgctttcc gtgtgatgca    1020
ctgcactctg ttggttgtaa gaatctccac ctggcctcta cgtagttcct tgtaaatgcc    1080
cgcgcacaga accttgctca gaccagattc tgtttcttgg ccaggaacga aaggaagggt    1140
tgctgccgat gcatgattgc ttcctcgatg aacgcagatt cgaaatgtat tctactgttt    1200
gagtttcttg ttcgtcacac actgtaccaa actgtattgt accctatcat aatttctgct    1260
cggtacctct ctgtactgct ggtaccaaac tgtattgtac tctgtcatga tctgtactag    1320
tctttggtac tgctggtcaa tagtcctacg aattgatcaa aaaaaaaaaa aaaaaaaaaa    1380
```

aaaaaaaaaa aaaa 1394

<210> 252
<211> 328
<212> PRT
<213> Hordeum vulgare

<400> 252

Met Cys Gly Gly Ala Ile Leu Ala Gln Leu Ile Pro Pro Ser Ala Gly
1 5 10 15

Arg Pro Ser Lys Gln Ala Ala Ala Gly Gly Arg Ala Pro Pro Thr Ser
20 25 30

Ser Lys Lys Gly Gly Val Ser Lys Ser Arg His Ser Ser Thr Pro Asp
35 40 45

Ala Asp Asp Asp Val Phe Glu Ala Ala Phe Glu Asp Phe Asp Asp His
50 55 60

Phe Asp Leu Arg Ala Glu Glu Asp Gly Gly Asp Asp His Val Val Phe
65 70 75 80

Ala Ser Lys Pro Ala Phe Ser Pro Arg Pro Ala Tyr Asp Gly Gly Arg
85 90 95

Ala Ala His Ala Ala Ser Arg Lys Lys Arg Thr Gly His Leu His Gly
100 105 110

Ile Arg Gln Arg Pro Trp Gly Lys Trp Ala Ala Glu Ile Arg Asp Pro
115 120 125

His Lys Gly Thr Arg Val Trp Leu Gly Thr Phe Asp Thr Ala Asp Asp
130 135 140

Ala Ala Arg Ala Tyr Asp Val Ala Ala Arg Arg Leu Arg Gly Ser Lys
145 150 155 160

Ala Lys Val Asn Phe Pro Asp Ala Ala Arg Thr Gly Ala Arg Pro Arg
165 170 175

Arg Ala Ser Arg Arg Thr Ala Gln Lys Pro Gln Cys Pro Pro Ala Arg
180 185 190

Thr Thr Ala Tyr Ser Ala Thr Ala Ala Ala Arg Ala Gln Pro Glu Gln
195 200 205

Asp Ala Met Met Val Lys Pro Glu Leu Met Glu Phe Phe Asn Val Asp
210 215 220

Ala Ile Val His Leu Thr Thr Ala Val Ala Ala Leu Pro Pro Val Thr
225 230 235 240

Ala Ser Thr Phe Ala Asp Thr Met Pro Arg Val Asp Glu Asp Ser Ser
245 250 255

Val Gly Ser Gly Gly Gly Ala Met Leu Gly Phe Ala Asp Glu Leu Gly
260 265 270

Phe Asp Pro Phe Met Met Phe Gln Leu Pro Cys Ser Asp Met Tyr Glu
275 280 285

Ser Ala Asp Ser Ile Phe Ala Gly Asp Ala Val Ile Pro Asp Ala Leu
290 295 300

Ser Val Asp Ser Gly Met Asp Ala Val Ser Leu Trp Ser Phe Asp Glu
305 310 315 320

Phe Pro Met Asp Ser Ala Ile Phe
325

<210> 253
<211> 985
<212> DNA
<213> Cucumis melo

<400> 253
aattctctaa tcatgtgtgg cggcgccatt atcgccgact taatccctcg ccgtgacggc 60

caacgcgtta ccgcttccga catttggcct aactcctcct tcttccattt caacaaaatt 120

cgctccgatc aagtttcaac tcccctcaaa cgaacccccc tcccggcctc ttccgaggct 180

tcgaagccca agaagaggca gaggaagaat ctttacagag gaattcgtca gcgcccgtgg 240

ggaaaatggg cggctgagat tcgtgacccc aggaagggga tccgtgtctg gcttgggact 300

ttcaatactg ctgaggaagc cgctcgagct tacgatcgag aagctcgcaa gattcgtgga 360

aagaaagcta aggtcaattt ccctaatgaa gatgatgcat attccattca agctcccatt 420

cctcaatttc accctcatct ttacactgtc cctgagaatt ccgaattccc ctacgatctc 480

aaccaaatcg gtgatttcac cggcactcac tttcccgtgg cgattgagga acaatccggc 540

tctgggtcgg aggattctta ttctccaccg aaaagattcg gtgtgaaaga ggctgaagat 600

cagaagccgg agcagaaggt tagcgttatt gccgcggcgg aggaagagaa cgaggtgcaa 660

aagctttctg aggaactaat ggcgtatgag aactacatga agttctacca aattccatac 720

ctcgacggtc aatcgacggt gacgaatccg gctgaagaac aagtggtggg cgatctctgg 780

agcttcgacg acgaggatgg gcttcacggc tctgtttcgt cgtctgaatt atgatgattg 840

atgatccaat aacttctcca tatctcaaac cggtttcctc cattttctat tgttgttttg 900

ttatttaatt ccagaatgtg ttttatgtaa ttttctgtcg ttgaacgatg tcaatttgtt 960

gatatgaaaa aaaaaaaaaa aaaaa 985

<210> 254
<211> 273
<212> PRT
<213> Cucumis melo

<400> 254

Met Cys Gly Gly Ala Ile Ile Ala Asp Leu Ile Pro Arg Arg Asp Gly
1 5 10 15

Gln Arg Val Thr Ala Ser Asp Ile Trp Pro Asn Ser Ser Phe Phe His
20 25 30

Phe Asn Lys Ile Arg Ser Asp Gln Val Ser Thr Pro Leu Lys Arg Thr
35 40 45

Pro Leu Pro Ala Ser Ser Glu Ala Ser Lys Pro Lys Lys Arg Gln Arg
50 55 60

Lys Asn Leu Tyr Arg Gly Ile Arg Gln Arg Pro Trp Gly Lys Trp Ala
65 70 75 80

Ala Glu Ile Arg Asp Pro Arg Lys Gly Ile Arg Val Trp Leu Gly Thr
85 90 95

Phe Asn Thr Ala Glu Glu Ala Ala Arg Ala Tyr Asp Arg Glu Ala Arg
100 105 110

Lys Ile Arg Gly Lys Lys Ala Lys Val Asn Phe Pro Asn Glu Asp Asp
115 120 125

Ala Tyr Ser Ile Gln Ala Pro Ile Pro Gln Phe His Pro His Leu Tyr
130 135 140

Thr Val Pro Glu Asn Ser Glu Phe Pro Tyr Asp Leu Asn Gln Ile Gly
145 150 155 160

Asp Phe Thr Gly Thr His Phe Pro Val Ala Ile Glu Glu Gln Ser Gly
165 170 175

Ser Gly Ser Glu Asp Ser Tyr Ser Pro Pro Lys Arg Phe Gly Val Lys
180 185 190

Glu Ala Glu Asp Gln Lys Pro Glu Gln Lys Val Ser Val Ile Ala Ala
195 200 205

Ala Glu Glu Glu Asn Glu Val Gln Lys Leu Ser Glu Glu Leu Met Ala

210 215 220

Tyr Glu Asn Tyr Met Lys Phe Tyr Gln Ile Pro Tyr Leu Asp Gly Gln
225 230 235 240

Ser Thr Val Thr Asn Pro Ala Glu Glu Gln Val Val Gly Asp Leu Trp
245 250 255

Ser Phe Asp Asp Glu Asp Gly Leu His Gly Ser Val Ser Ser Ser Glu
260 265 270

Leu

<210> 255
<211> 1010
<212> DNA
<213> Malus x domestica

<400> 255
ctgagagggc tcttttttct ctctaagttt ctacaagtgg caatataaac atgtgtggtg 60
gtgctatcat ttccgacttc atcgccgtca agcgcgccct gaagctgacg gcggaggacc 120
tctggtcaga tcttgacacc atctctgacc tccttggcat agactactcc aacagcatca 180
acaaacagcc ggagaatcac aaggtggtcc aaaagccgaa accatctatc accaaagtag 240
tgacaagtga tgagaagccc aagcaggcga gcgggtctgc tgctgccgca aaaggcaaga 300
gagtgagaaa aaacgtgtac agaggaataa ggcagaggcc gtggggcaaa tgggcggctg 360
agattcgcga cccctacaaa gccgtccggg tctggctcgg cacctatgac accgctgagg 420
aagccgcccg cgcttacgat gaagccgccg tgcgcatccg cggggacaag gccaagctca 480
actttgccca accaccatcc tcttcaccgc ttccatctct ggcgccggat acgccgccgc 540
cgacaaagag gcggtgcatt gttgctgagt caactcgggt ggagccgact caaccgagtt 600
tccagaccgg ttcttactat tatgatccat tatatcacgg cggtggtggt ggggaaatgt 660
atgctaagaa agaggtggcg ggtggggacg aggtggtggt agagcagctg agtcaggtgg 720
tgagtggaag cggagagtcg gactcgttgt acctgtggat gctggatgac ctggtggcat 780
atcagcaaca agggcagctt ctgtattaag gcagcggaat tgttctagct aaatatttgt 840
atggctagga attaaaaaca tattaattaa agggtatgta tgtttaattt acgtgtgtga 900
aatgcgagtg ttgagtatgt ctatgactgg gtttgtgtaa cgtgtgttgt ttgctacggt 960
gtttatgttt aatagtaact gcttttgtct ttgaaaaaaa aaaaaaaaaa 1010

<210> 256
<211> 252
<212> PRT
<213> Malus x domestica

<400> 256

Met Cys Gly Gly Ala Ile Ile Ser Asp Phe Ile Ala Val Lys Arg Ala
1 5 10 15

Leu Lys Leu Thr Ala Glu Asp Leu Trp Ser Asp Leu Asp Thr Ile Ser
20 25 30

Asp Leu Leu Gly Ile Asp Tyr Ser Asn Ser Ile Asn Lys Gln Pro Glu
35 40 45

Asn His Lys Val Val Gln Lys Pro Lys Pro Ser Ile Thr Lys Val Val
50 55 60

Thr Ser Asp Glu Lys Pro Lys Gln Ala Ser Gly Ser Ala Ala Ala Ala
65 70 75 80

Lys Gly Lys Arg Val Arg Lys Asn Val Tyr Arg Gly Ile Arg Gln Arg
85 90 95

Pro Trp Gly Lys Trp Ala Ala Glu Ile Arg Asp Pro Tyr Lys Ala Val
100 105 110

Arg Val Trp Leu Gly Thr Tyr Asp Thr Ala Glu Glu Ala Ala Arg Ala
115 120 125

Tyr Asp Glu Ala Ala Val Arg Ile Arg Gly Asp Lys Ala Lys Leu Asn
130 135 140

Phe Ala Gln Pro Pro Ser Ser Ser Pro Leu Pro Ser Leu Ala Pro Asp
145 150 155 160

Thr Pro Pro Pro Thr Lys Arg Arg Cys Ile Val Ala Glu Ser Thr Arg
165 170 175

Val Glu Pro Thr Gln Pro Ser Phe Gln Thr Gly Ser Tyr Tyr Tyr Asp
180 185 190

Pro Leu Tyr His Gly Gly Gly Gly Gly Glu Met Tyr Ala Lys Lys Glu
195 200 205

Val Ala Gly Gly Asp Glu Val Val Val Glu Gln Leu Ser Gln Val Val
210 215 220

Ser Gly Ser Gly Glu Ser Asp Ser Leu Tyr Leu Trp Met Leu Asp Asp
225 230 235 240

Leu Val Ala Tyr Gln Gln Gln Gly Gln Leu Leu Tyr
245 250

<210> 257
<211> 1354
<212> DNA
<213> Oryza sativa

<220>
<221> misc_feature
<222> (37)..(37)
<223> n is a, c, g, or t

<220>
<221> misc_feature
<222> (1324)..(1324)
<223> n is a, c, g, or t

<400> 257
ggcacgcgcc tcctcggaat cgccacgaga cccttgnagc cagataaaaa aggaaaaaaa 60

gagagtcgta gttcgcctct tcttcctcct ctcgttctcg cggccatatc gaattcctgc 120

agcccggggg atccccgaat tcagctcagt aaatctgttg attgctcgtt tcaaatcatc 180

atcggtgttt cgtttcttga tttcttgatc catccatggc gacgacagtg gattggtgtg 240

gccgtggcag caatcttcct gctgcgatgt atgacatggt ggtggatagc aaggagctaa 300

tgggcgcgct tgctccgtcc atggtgtcct tctcctaccc gtgttccgag cagagcgcga 360

gctcgcttct tgctggcgcc aactacctga ctcccgcgca ggtgctccat gtccaggcgc 420

agctacagcg cctgcgccgc ccgggcgcgg cgagcggctg cctcgccgcg gcgccgccgc 480

tgccgatgaa gcggcatggc gcggtggcgg tggcggcggc ggcggcggcg gcgcgggcgc 540

cggtcaagct gtaccgcggc gtcaggcagc ggcattgggg caagtgggtg gcggagatcc 600

gcctgccgcg caaccgcacc cgcctctggc tgggcacctt cgacaccgcc gaggaggccg 660

cgctggcgta cgacagcgcc gcgttccgcc tccgcggcga gtccgcgagg ctcaacttcc 720

ccgagctccg ccgcggcggc gcccacctcg gcccgccgct ccacgccgcg gtcgacgcca 780

agctccacgc catctgccac gggatggacc tgccccaacc ccaaccccaa acccagagca 840

atgcgacgac gacgacgatg tcgacgacgg cgacgaacac cccaagcccc ttcttctcct 900

cggagagccc ggtggtcaag agcgagcccg tctgctccgc ctccgagagc tcttcctcgg 960

ccgacggcga cgtgtcatcg acgggctcct ccgacgtcgt cccggagatg cagctgctcg 1020

acttctcgga ggcgccatgg gacgagtccg agagcttcct gctgcacaag tacccgtcgc 1080

tggagatcga ctgggacgcc gatcctttcc tgatgcagtg gctgaatctt cttcctctcc 1140

ctgttcttga ctccagctct tggtgtcctg aacattttca caactcagag agtgatgctc 1200

taccttgatt agctacatgt taatctaggt tttgggtttg atcatgccat cggattctgt 1260

agcatcaacc cctggtctgc ttggtttttt caagtggcat tgcacaggaa ggaggtctca 1320

gagntttggt aattgcgagt tgtgcaaccc tgca 1354

<210> 258
<211> 330
<212> PRT
<213> Oryza sativa

<400> 258

```
Met Ala Thr Thr Val Asp Trp Cys Gly Arg Gly Ser Asn Leu Pro Ala
1               5                   10                  15

Ala Met Tyr Asp Met Val Val Asp Ser Lys Glu Leu Met Gly Ala Leu
            20                  25                  30

Ala Pro Ser Met Val Ser Phe Ser Tyr Pro Cys Ser Glu Gln Ser Ala
        35                  40                  45

Ser Ser Leu Leu Ala Gly Ala Asn Tyr Leu Thr Pro Ala Gln Val Leu
    50                  55                  60

His Val Gln Ala Gln Leu Gln Arg Leu Arg Arg Pro Gly Ala Ala Ser
65                  70                  75                  80

Gly Cys Leu Ala Ala Ala Pro Pro Leu Pro Met Lys Arg His Gly Ala
                85                  90                  95

Val Ala Val Ala Ala Ala Ala Ala Ala Ala Arg Ala Pro Val Lys Leu
            100                 105                 110

Tyr Arg Gly Val Arg Gln Arg His Trp Gly Lys Trp Val Ala Glu Ile
        115                 120                 125

Arg Leu Pro Arg Asn Arg Thr Arg Leu Trp Leu Gly Thr Phe Asp Thr
    130                 135                 140

Ala Glu Glu Ala Ala Leu Ala Tyr Asp Ser Ala Ala Phe Arg Leu Arg
145                 150                 155                 160

Gly Glu Ser Ala Arg Leu Asn Phe Pro Glu Leu Arg Arg Gly Gly Ala
                165                 170                 175

His Leu Gly Pro Pro Leu His Ala Ala Val Asp Ala Lys Leu His Ala
            180                 185                 190

Ile Cys His Gly Met Asp Leu Pro Gln Pro Gln Pro Gln Thr Gln Ser
        195                 200                 205

Asn Ala Thr Thr Thr Thr Met Ser Thr Thr Ala Thr Asn Thr Pro Ser
    210                 215                 220

Pro Phe Phe Ser Ser Glu Ser Pro Val Val Lys Ser Glu Pro Val Cys
225                 230                 235                 240
```

Ser Ala Ser Glu Ser Ser Ser Ser Ala Asp Gly Asp Val Ser Ser Thr
245 250 255

Gly Ser Ser Asp Val Val Pro Glu Met Gln Leu Leu Asp Phe Ser Glu
260 265 270

Ala Pro Trp Asp Glu Ser Glu Ser Phe Leu Leu His Lys Tyr Pro Ser
275 280 285

Leu Glu Ile Asp Trp Asp Ala Asp Pro Phe Leu Met Gln Trp Leu Asn
290 295 300

Leu Leu Pro Leu Pro Val Leu Asp Ser Ser Ser Trp Cys Pro Glu His
305 310 315 320

Phe His Asn Ser Glu Ser Asp Ala Leu Pro
325 330

<210> 259
<211> 962
<212> DNA
<213> Oryza sativa

<400> 259
ggcttaaaca atggcgacga cagtggattg gtgtggccgt ggcagcaatc ttcctgctgc 60

gatgtatgac atggtggtgg atagcaagga gctaatgggc gcgcttgctc cgtccatggt 120

gtccttctcc tacccgtgtt ccgagcagag cgcgagctcg cttcttgctg gcgccaacta 180

cctgactccc gcgcaggtgc tccatgtcca ggcgcagcta cagcgcctgc gccgcccggg 240

cgcggcgagc ggctgcctcg ccgcggcgcc gccgctgccg atgaagcggc atggcgcggt 300

ggcggtggcg gcggcggcgg cggcgcgggc gccggtcaag ctgtaccgcg gcgtcaggca 360

gcggcattgg ggcaagtggg tggcggagat ccgcctgccg cgcaaccgca cccgcctctg 420

gctgggcacc ttcgacaccg ccgaggaggc cgcgctggcg tacgacagcg ccgcgttccg 480

cctccgcggc gagtccgcga ggctcaactt ccccgagctc cgccgcggcg gcgcccacct 540

cggcccgccg ctccacgccg cggtcgacgc caagctccac gccatctgcc acgggatgga 600

cctgccccaa ccccaacccc aaacccagag caatgcgacg acgacgacga tgtcgacgac 660

ggcgacgaac accccaagcc ccttcttctc ctcggagagc ccggtggtca agagcgagcc 720

cgtctgctcc gcctccgaga gctcttcctc ggccgacggc gacgtgtcat cgacgggctc 780

ctccgacgtc gtcccggaga tgcagctgct cgacttctcg gaggcgccat gggacgagtc 840

cgagagcttc ctgctgcaca agtacccgtc gctggagatc gactgggacg cgatcctttc 900

ctgatgcagt ggctgaatct tcttcctctc cctgttcttg aacccagctt tcttgtacaa 960

ag 962

<210> 260
<211> 297
<212> PRT
<213> Oryza sativa

<400> 260

```
Met Ala Thr Thr Val Asp Trp Cys Gly Arg Gly Ser Asn Leu Pro Ala
1               5                   10                  15

Ala Met Tyr Asp Met Val Val Asp Ser Lys Glu Leu Met Gly Ala Leu
            20                  25                  30

Ala Pro Ser Met Val Ser Phe Ser Tyr Pro Cys Ser Glu Gln Ser Ala
        35                  40                  45

Ser Ser Leu Leu Ala Gly Ala Asn Tyr Leu Thr Pro Ala Gln Val Leu
    50                  55                  60

His Val Gln Ala Gln Leu Gln Arg Leu Arg Arg Pro Gly Ala Ala Ser
65                  70                  75                  80

Gly Cys Leu Ala Ala Ala Pro Pro Leu Pro Met Lys Arg His Gly Ala
                85                  90                  95

Val Ala Val Ala Ala Ala Ala Ala Ala Arg Ala Pro Val Lys Leu Tyr
            100                 105                 110

Arg Gly Val Arg Gln Arg His Trp Gly Lys Trp Val Ala Glu Ile Arg
        115                 120                 125

Leu Pro Arg Asn Arg Thr Arg Leu Trp Leu Gly Thr Phe Asp Thr Ala
    130                 135                 140

Glu Glu Ala Ala Leu Ala Tyr Asp Ser Ala Ala Phe Arg Leu Arg Gly
145                 150                 155                 160

Glu Ser Ala Arg Leu Asn Phe Pro Glu Leu Arg Arg Gly Gly Ala His
                165                 170                 175

Leu Gly Pro Pro Leu His Ala Ala Val Asp Ala Lys Leu His Ala Ile
            180                 185                 190

Cys His Gly Met Asp Leu Pro Gln Pro Gln Pro Gln Thr Gln Ser Asn
        195                 200                 205

Ala Thr Thr Thr Thr Met Ser Thr Thr Ala Thr Asn Thr Pro Ser Pro
    210                 215                 220
```

Phe Phe Ser Ser Glu Ser Pro Val Val Lys Ser Glu Pro Val Cys Ser
225 230 235 240

Ala Ser Glu Ser Ser Ser Ser Ala Asp Gly Asp Val Ser Ser Thr Gly
245 250 255

Ser Ser Asp Val Val Pro Glu Met Gln Leu Leu Asp Phe Ser Glu Ala
260 265 270

Pro Trp Asp Glu Ser Glu Ser Phe Leu Leu His Lys Tyr Pro Ser Leu
275 280 285

Glu Ile Asp Trp Asp Ala Ile Leu Ser
290 295

<210> 261
<211> 894
<212> DNA
<213> Oryza sativa

<400> 261
atggcgacga cagtggattg gtgtggccgt ggcagcaatc ttcctgctgc gatgtatgac 60

atggtggtgg atagcaagga gctaatgggc gcgcttgctc cgtccatggt gtccttctcc 120

tacccgtgtt ccgagcagag cgcgagctcg cttcttgctg gcgccaacta cctgactccc 180

gcgcaggtgc tccatgtcca ggcgcagcta cagcgcctgc gccgcccggg cgcggcgagc 240

ggctgcctcg ccgcggcgcc gccgctgccg atgaagcggc atggcgcggt ggcggtggcg 300

gcggcggcgg cggcgcgggc gccggtcaag ctgtaccgcg gcgtcaggca gcggcattgg 360

ggcaagtggg tggcggagat ccgcctgccg cgcaaccgca cccgcctctg gctgggcacc 420

ttcgacaccg ccgaggaggc cgcgctggcg tacgacagcg ccgcgttccg cctccgcggc 480

gagtccgcga ggctcaactt ccccgagctc cgccgcggcg gcgcccacct cggcccgccg 540

ctccacgccg cggtcgacgc caagctccac gccatctgcc acgggatgga cctgccccaa 600

ccccaacccc aaacccagag caatgcgacg acgacgacga tgtcgacgac ggcgacgaac 660

accccaagcc ccttcttctc ctcggagagc ccggtggtca agagcgagcc cgtctgctcc 720

gcctccgaga gctcttcctc ggccgacggc gacgtgtcat cgacgggctc ctccgacgtc 780

gtcccggaga tgcagctgct cgacttctcg gaggcgccat gggacgagtc cgagagcttc 840

ctgctgcaca agtacccgtc gctggagatc gactgggacg cgatcctttc ctga 894

<210> 262
<211> 297
<212> PRT
<213> Oryza sativa

<400> 262

Met Ala Thr Thr Val Asp Trp Cys Gly Arg Gly Ser Asn Leu Pro Ala

1 5 10 15

Ala Met Tyr Asp Met Val Val Asp Ser Lys Glu Leu Met Gly Ala Leu
20 25 30

Ala Pro Ser Met Val Ser Phe Ser Tyr Pro Cys Ser Glu Gln Ser Ala
35 40 45

Ser Ser Leu Leu Ala Gly Ala Asn Tyr Leu Thr Pro Ala Gln Val Leu
50 55 60

His Val Gln Ala Gln Leu Gln Arg Leu Arg Arg Pro Gly Ala Ala Ser
65 70 75 80

Gly Cys Leu Ala Ala Ala Pro Pro Leu Pro Met Lys Arg His Gly Ala
85 90 95

Val Ala Val Ala Ala Ala Ala Ala Ala Arg Ala Pro Val Lys Leu Tyr
100 105 110

Arg Gly Val Arg Gln Arg His Trp Gly Lys Trp Val Ala Glu Ile Arg
115 120 125

Leu Pro Arg Asn Arg Thr Arg Leu Trp Leu Gly Thr Phe Asp Thr Ala
130 135 140

Glu Glu Ala Ala Leu Ala Tyr Asp Ser Ala Ala Phe Arg Leu Arg Gly
145 150 155 160

Glu Ser Ala Arg Leu Asn Phe Pro Glu Leu Arg Arg Gly Gly Ala His
165 170 175

Leu Gly Pro Pro Leu His Ala Ala Val Asp Ala Lys Leu His Ala Ile
180 185 190

Cys His Gly Met Asp Leu Pro Gln Pro Gln Pro Gln Thr Gln Ser Asn
195 200 205

Ala Thr Thr Thr Thr Met Ser Thr Thr Ala Thr Asn Thr Pro Ser Pro
210 215 220

Phe Phe Ser Ser Glu Ser Pro Val Val Lys Ser Glu Pro Val Cys Ser
225 230 235 240

Ala Ser Glu Ser Ser Ser Ser Ala Asp Gly Asp Val Ser Ser Thr Gly
245 250 255

Ser Ser Asp Val Val Pro Glu Met Gln Leu Leu Asp Phe Ser Glu Ala
260 265 270

Pro Trp Asp Glu Ser Glu Ser Phe Leu Leu His Lys Tyr Pro Ser Leu
        275                 280                 285

Glu Ile Asp Trp Asp Ala Ile Leu Ser
    290                 295

<210> 263
<211> 929
<212> DNA
<213> Triticum aestivum

<400> 263
gctctcctcg gcttcccttc ctccaaatcg tcggcgtttc ttgatcgacg agggcatggc 60

gacgacggtg gactggcgca gctataggcc cgatcttcct gccgcgatgt atcacatggt 120

ggacagcagg gaccaggtaa tgcacgcgtt tgctccggcg acggcgcagg gcgcggctcc 180

gaccatctcg ttcgccttcc cctgccccgg cgcggagcag agcgccggcc tgcttcgtgg 240

cgccagctac ctcactcccg cgcaaatcct ccagctccag tcgcagctgc accacgtgcg 300

ccgggcgccg ggcgcggcca tggccgcggt ggggcagccg atgaagcggc acggcgtggc 360

agcgctcccg gcgcggccgg cgaccaagct ttaccgcggc gtaaggcagc ggcattgggg 420

gaagtgggtc gccgagatcc gcctgccccg caaccgcacc cgcctctggc tcggcacctt 480

cgacaccgcc gacgaggccg cgctggccta cgacgccgcc gccttccggc tccgcggcga 540

gtcctccagg ctcaacttcc ccgagctcag gcgcggcggc gagcaccacg gcccgccgct 600

cgacgccgcg atcgacgcca agctccgctc catctgccac ggggaagaca tgccccagag 660

ccagagcgat gagacgccgg cgccgacgac gacactgacg ccgatttctt tcccggacgt 720

caagagcgag ccagtctgct ccgtctccga gagctcgtcg tcggctgacg gcgaggtgtc 780

ctcgtgctcc gacgtcgtcc cggagatgca gcttcttgat ttctcggagg ctccatggga 840

cgagttcctg ctgcgcaagt acccgtcgct cgagatcgac tgggacgcga tcctttcttg 900

aatcaagttc atgctcgatc cacagctcg 929

<210> 264
<211> 281
<212> PRT
<213> Triticum aestivum

<400> 264

Met Ala Thr Thr Val Asp Trp Arg Ser Tyr Arg Pro Asp Leu Pro Ala
1               5                   10                  15

Ala Met Tyr His Met Val Asp Ser Arg Asp Gln Val Met His Ala Phe
            20                  25                  30

Ala Pro Ala Thr Ala Gln Gly Ala Ala Pro Thr Ile Ser Phe Ala Phe

```
            35                  40                  45

Pro Cys Pro Gly Ala Glu Gln Ser Ala Gly Leu Leu Arg Gly Ala Ser
    50                  55                  60

Tyr Leu Thr Pro Ala Gln Ile Leu Gln Leu Gln Ser Gln Leu His His
65                  70                  75                  80

Val Arg Arg Ala Pro Gly Ala Ala Met Ala Ala Val Gly Gln Pro Met
                85                  90                  95

Lys Arg His Gly Val Ala Ala Leu Pro Ala Arg Pro Ala Thr Lys Leu
            100                 105                 110

Tyr Arg Gly Val Arg Gln Arg His Trp Gly Lys Trp Val Ala Glu Ile
        115                 120                 125

Arg Leu Pro Arg Asn Arg Thr Arg Leu Trp Leu Gly Thr Phe Asp Thr
    130                 135                 140

Ala Asp Glu Ala Ala Leu Ala Tyr Asp Ala Ala Ala Phe Arg Leu Arg
145                 150                 155                 160

Gly Glu Ser Ser Arg Leu Asn Phe Pro Glu Leu Arg Arg Gly Gly Glu
                165                 170                 175

His His Gly Pro Pro Leu Asp Ala Ala Ile Asp Ala Lys Leu Arg Ser
            180                 185                 190

Ile Cys His Gly Glu Asp Met Pro Gln Ser Gln Ser Asp Glu Thr Pro
        195                 200                 205

Ala Pro Thr Thr Thr Leu Thr Pro Ile Ser Phe Pro Asp Val Lys Ser
    210                 215                 220

Glu Pro Val Cys Ser Val Ser Glu Ser Ser Ser Ser Ala Asp Gly Glu
225                 230                 235                 240

Val Ser Ser Cys Ser Asp Val Val Pro Glu Met Gln Leu Leu Asp Phe
                245                 250                 255

Ser Glu Ala Pro Trp Asp Glu Phe Leu Leu Arg Lys Tyr Pro Ser Leu
            260                 265                 270

Glu Ile Asp Trp Asp Ala Ile Leu Ser
        275                 280

<210>  265
<211>  999
```

<212> DNA
<213> Triticum aestivum

<400> 265
gctctcctcg gcttctcttc ctccaaatcg tcggcgtttc ttgatcgaca gggcatggcg 60

acgacggtgg actggcgcag ctataggccc gatcttcctg cggcgatgta ccgcatggtg 120

gacagcagag accaggtaat gcacgcgttc gctccgccga cggcgcaggg cgcggctccg 180

accatctcgt tcgccttccc atgccccggc gcggatcaga gcgccggcct gcttcgtggc 240

gccacctacc tcactcccgc gcaaatcctc cagctccagt cgcagctcca ccacgtgcgc 300

cgggcgccgg gcgcgcccat ggccgcggtg gggcagccga tgaagcggca cggcgtggcg 360

gcgctcccgg cgcggccggc gaccaagctg taccgcggcg tgcggcagcg gcattggggg 420

aagtgggtcg cggagatccg cctgccccgc aaccgcaccc gcctctggct cggcaccttc 480

gacaccgccg acgaggccgc gctggcctac gacgccgccg ccttccggct ccgcggcgag 540

tccgccaggc tcaacttccc cgagctcagg cgcggcggcg agcaccacgg cccgccgctc 600

gatgccgcga tcgacgccaa gctccgctcc atctgccacg ggaagacat gccgcagagc 660

cagagcaatg agacgccggc gccgacgccg acactgacgc cgatttcttt cccggacgtc 720

aagagcgagc cagtctgctc cgtctccgag agctcttcgt cggctgacgg cgaggtgtcc 780

tcgtgctccg acgtcgtccc ggagatgcag cttcttgatt tctcggaggc tccatgggac 840

gagtccctgc tgcgcaagta cccgtcgctc gagatcgact gggacgcgat ccttccttga 900

atcaagttca tgctcgatcc acagctcgtc caaagtgatt acttcggctt ccacttaggc 960

tcgatcgagt atacgcgtgt agcatcaacc cctccctgc 999

<210> 266
<211> 281
<212> PRT
<213> Triticum aestivum

<400> 266

Met Ala Thr Thr Val Asp Trp Arg Ser Tyr Arg Pro Asp Leu Pro Ala
1 5 10 15

Ala Met Tyr Arg Met Val Asp Ser Arg Asp Gln Val Met His Ala Phe
20 25 30

Ala Pro Pro Thr Ala Gln Gly Ala Ala Pro Thr Ile Ser Phe Ala Phe
35 40 45

Pro Cys Pro Gly Ala Asp Gln Ser Ala Gly Leu Leu Arg Gly Ala Thr
50 55 60

Tyr Leu Thr Pro Ala Gln Ile Leu Gln Leu Gln Ser Gln Leu His His
65 70 75 80

Val Arg Arg Ala Pro Gly Ala Pro Met Ala Ala Val Gly Gln Pro Met
85 90 95

Lys Arg His Gly Val Ala Ala Leu Pro Ala Arg Pro Ala Thr Lys Leu
100 105 110

Tyr Arg Gly Val Arg Gln Arg His Trp Gly Lys Trp Val Ala Glu Ile
115 120 125

Arg Leu Pro Arg Asn Arg Thr Arg Leu Trp Leu Gly Thr Phe Asp Thr
130 135 140

Ala Asp Glu Ala Ala Leu Ala Tyr Asp Ala Ala Ala Phe Arg Leu Arg
145 150 155 160

Gly Glu Ser Ala Arg Leu Asn Phe Pro Glu Leu Arg Arg Gly Gly Glu
165 170 175

His His Gly Pro Pro Leu Asp Ala Ala Ile Asp Ala Lys Leu Arg Ser
180 185 190

Ile Cys His Gly Glu Asp Met Pro Gln Ser Gln Ser Asn Glu Thr Pro
195 200 205

Ala Pro Thr Pro Thr Leu Thr Pro Ile Ser Phe Pro Asp Val Lys Ser
210 215 220

Glu Pro Val Cys Ser Val Ser Glu Ser Ser Ser Ser Ala Asp Gly Glu
225 230 235 240

Val Ser Ser Cys Ser Asp Val Val Pro Glu Met Gln Leu Leu Asp Phe
245 250 255

Ser Glu Ala Pro Trp Asp Glu Ser Leu Leu Arg Lys Tyr Pro Ser Leu
260 265 270

Glu Ile Asp Trp Asp Ala Ile Leu Pro
275 280

<210> 267
<211> 847
<212> DNA
<213> Triticum aestivum

<400> 267
tcctccagct ccagtcgcag ctccaccacg tgcgccgggc gccgggcgcg gccatggcag 60

tggcggggca gcccatgaag cggcacggcg ttgcggcgct cccggcgcag ccggcggcca 120

agctgtaccg cggcgtgcgg cagcggcatt gggggaagtg ggtcgccgag atccgcctgc 180

```
cccgcaaccg caccccgcctc tggctcggca ccttcgacac cgccgacgag gccgcgctgg    240
cctacgacgc cgccgccttc cggctccgcg gcgagtccgc caggctcaac ttccccgagc    300
tcaggcgcgg cggcgagcac cacggcccgc cgctcgacgc cgcgatcgac gccaagctcc    360
gctccatctg ccacggggag gacctgccgc agagccagag caatgcgacg ccggcgccga    420
cgccgaccct gacgccgagc tctttcccgg acgtcaagag cgagccaggc tgctccgtct    480
ccgagagctc gtcgtcggcc gacggcgagg tgtcctcgtg ctccgacgtc gtcccggaga    540
tgcagcttct tgatttctcg gaggctccat gggacgagtc cctgctgcgc aagtacccgt    600
cgctcgagat cgactgggac gcgatccttt cttgaaccga gttcatgccc gatccacagc    660
tcgttcaaag tgattgcttc tgctttcgtt aggctcttag ataggttatt atgggtttga    720
tcaagtattc ttgtgtaaca tcaatccctg ctctgcttgg tttttgaatg gcattgccag    780
gaaggaggtt ttatgtagaa attttaagta tgtcatgtag tttgtgattc attttttttt    840
ttttttt                                                               847
```

<210> 268
<211> 193
<212> PRT
<213> Triticum aestivum

<400> 268

```
Met Ala Val Ala Gly Gln Pro Met Lys Arg His Gly Val Ala Ala Leu
1               5                   10                  15

Pro Ala Gln Pro Ala Ala Lys Leu Tyr Arg Gly Val Arg Gln Arg His
            20                  25                  30

Trp Gly Lys Trp Val Ala Glu Ile Arg Leu Pro Arg Asn Arg Thr Arg
        35                  40                  45

Leu Trp Leu Gly Thr Phe Asp Thr Ala Asp Glu Ala Ala Leu Ala Tyr
    50                  55                  60

Asp Ala Ala Ala Phe Arg Leu Arg Gly Glu Ser Ala Arg Leu Asn Phe
65                  70                  75                  80

Pro Glu Leu Arg Arg Gly Gly Glu His His Gly Pro Pro Leu Asp Ala
                85                  90                  95

Ala Ile Asp Ala Lys Leu Arg Ser Ile Cys His Gly Glu Asp Leu Pro
            100                 105                 110

Gln Ser Gln Ser Asn Ala Thr Pro Ala Pro Thr Pro Thr Leu Thr Pro
        115                 120                 125

Ser Ser Phe Pro Asp Val Lys Ser Glu Pro Gly Cys Ser Val Ser Glu
```

130 135 140

Ser Ser Ser Ser Ala Asp Gly Glu Val Ser Ser Cys Ser Asp Val Val
145 150 155 160

Pro Glu Met Gln Leu Leu Asp Phe Ser Glu Ala Pro Trp Asp Glu Ser
165 170 175

Leu Leu Arg Lys Tyr Pro Ser Leu Glu Ile Asp Trp Asp Ala Ile Leu
180 185 190

Ser

<210> 269
<211> 1654
<212> DNA
<213> Oryza sativa

<400> 269
tttttctctt ctggattctt gccgattttt aggcttcctt tgcttttcta aggccagaaa 60

gggttgtttt gcaataccct tctagttcat cctctcaaat ttttcttctt tctattcaaa 120

agctctttct ttctttgcac cctttgtttc tctactttta ttgaggaaca gggctgattt 180

ttcacttctt gcatccaaaa agcacccctt ttttctttcc tgtttttaag atttccatac 240

ccttgccatc ttcatcttct acctccatcc agtcctcatg gccgcagcaa tagacatgta 300

caagtataac actagcacac accagatcgc atcctcggat caggagctca tgaaagcgct 360

cgaacctttt attaggagcg cttcttcttc ctccgcttcc tccccctgcc accactacta 420

ctcttcttct ccttccatga gccaagattc ttacatgccc accccatctt atcccacttc 480

ctctatcaca accgccgccg ccaccaccac ctcgtctttc tcgcagctac ctccgctgta 540

ctcttcgcag tatcatgctg cttcacctgc ggcgtcggcg acgaacgggc cgatggggct 600

gacccacctg ggcccagccc agatccagca gatccaggcc cagttcttgg cccagcagca 660

gcagcagagg gccctggccg gcgccttcct tcggccgcgt ggccagccga tgaagcagtc 720

cgggtcgccg ccgcgcgcgg ggccgttcgc ggcggtcgcc ggggcggcgc agtcgaagct 780

ctaccgcgga gtgcggcagc gccactgggg gaagtgggtg gcggagatcc gcctcccgaa 840

gaaccggacg cggctgtggc tcggcacctt cgacaccgcc gaggacgccg cgctcgccta 900

cgacaaggcc gccttccgcc tccgcggcga cctcgcgcgg ctcaacttcc ccaccctccg 960

ccgcggcggc gcccacctcg ccggcccgct ccacgcctcc gtcgacgcca agctcaccgc 1020

catctgccag tccctcgcca cgagctcgtc caagaacacc cccgccgagt cagcggcctc 1080

cgcggcggag ccggagtccc ccaagtgctc ggcgtcgacg gaaggggagg actcggtgtc 1140

cgccggctcc cctcctccgc ccacgccgct gtcgcccccg gtgccggaga tggagaagct 1200

ggacttcacg gaggcgccat gggacgagtc ggagacattc cacctgcgca agtacccgtc 1260

ctgggagatc gactgggact caatcctctc ataaacaagc agaagcagct actactagtc 1320

tattactagt actagtagta gtcttcgtca agctagagtc actcaactca actagctgtg 1380

taatcttctc tgaattccgt ggcttccatg gctcggtggc attttagacg tcggccatgg 1440

ctgctgcgag tagcagtaac tagtcagtac tcagtagtag taaggtcgtt ggtattacgt 1500

cgtcgtgcaa gtgtcgttgg tgtactcagt gatctgatct cctggttgag ctgccggttg 1560

ttttttcac ggcgcggccg gtcgagaatt aagctgtaat cccttgttac atgttggaaa 1620

ttcagtagct tatgtaacta tatgtacctt tctc 1654

<210> 270
<211> 338
<212> PRT
<213> Oryza sativa

<400> 270

Met Ala Ala Ala Ile Asp Met Tyr Lys Tyr Asn Thr Ser Thr His Gln
1               5                   10                  15

Ile Ala Ser Ser Asp Gln Glu Leu Met Lys Ala Leu Glu Pro Phe Ile
            20                  25                  30

Arg Ser Ala Ser Ser Ser Ser Ala Ser Ser Pro Cys His His Tyr Tyr
        35                  40                  45

Ser Ser Ser Pro Ser Met Ser Gln Asp Ser Tyr Met Pro Thr Pro Ser
    50                  55                  60

Tyr Pro Thr Ser Ser Ile Thr Thr Ala Ala Ala Thr Thr Thr Ser Ser
65                  70                  75                  80

Phe Ser Gln Leu Pro Pro Leu Tyr Ser Ser Gln Tyr His Ala Ala Ser
                85                  90                  95

Pro Ala Ala Ser Ala Thr Asn Gly Pro Met Gly Leu Thr His Leu Gly
            100                 105                 110

Pro Ala Gln Ile Gln Gln Ile Gln Ala Gln Phe Leu Ala Gln Gln Gln
        115                 120                 125

Gln Gln Arg Ala Leu Ala Gly Ala Phe Leu Arg Pro Arg Gly Gln Pro
    130                 135                 140

Met Lys Gln Ser Gly Ser Pro Pro Arg Ala Gly Pro Phe Ala Ala Val
145                 150                 155                 160

Ala Gly Ala Ala Gln Ser Lys Leu Tyr Arg Gly Val Arg Gln Arg His

165 170 175

Trp Gly Lys Trp Val Ala Glu Ile Arg Leu Pro Lys Asn Arg Thr Arg
180 185 190

Leu Trp Leu Gly Thr Phe Asp Thr Ala Glu Asp Ala Ala Leu Ala Tyr
195 200 205

Asp Lys Ala Ala Phe Arg Leu Arg Gly Asp Leu Ala Arg Leu Asn Phe
210 215 220

Pro Thr Leu Arg Arg Gly Gly Ala His Leu Ala Gly Pro Leu His Ala
225 230 235 240

Ser Val Asp Ala Lys Leu Thr Ala Ile Cys Gln Ser Leu Ala Thr Ser
245 250 255

Ser Ser Lys Asn Thr Pro Ala Glu Ser Ala Ala Ser Ala Ala Glu Pro
260 265 270

Glu Ser Pro Lys Cys Ser Ala Ser Thr Glu Gly Glu Asp Ser Val Ser
275 280 285

Ala Gly Ser Pro Pro Pro Pro Thr Pro Leu Ser Pro Pro Val Pro Glu
290 295 300

Met Glu Lys Leu Asp Phe Thr Glu Ala Pro Trp Asp Glu Ser Glu Thr
305 310 315 320

Phe His Leu Arg Lys Tyr Pro Ser Trp Glu Ile Asp Trp Asp Ser Ile
325 330 335

Leu Ser

<210> 271
<211> 834
<212> DNA
<213> Arabidopsis thaliana

<400> 271
atggctttaa acatgaatgc ttacgtagac gagttcatgg aagctcttga accattcatg 60

aaggtaactt catcttcttc tacttcgaat tcatcaaatc caaaaccatt aactcctaat 120

ttcatcccta ataatgacca agtcttaccg gtatctaacc aaaccggtcc gattgggcta 180

aaccagctca ctccaacaca aatcctccaa attcagacag agttacatct ccggcaaaac 240

caatctcgtc gtcgcgctgg tagtcatctt ctcaccgcta aaccaacctc aatgaagaaa 300

atcgacgtag caactaaacc ggttaaacta taccgaggcg taagacagag gcaatggggt 360

aaatgggtag ctgagattcg gctacctaaa aaccgaaccc ggttatggct cggtacgttc 420

gaaacggctc aagaagctgc attagcttac gatcaagcag ctcataagat cagaggagac 480

aacgctcgtc tcaatttccc agacattgtt cgtcaaggac actataaaca gatattgtct 540

ccgtctatca acgcaaagat cgaatccatc tgcaatagtt ctgatcttcc actgcctcag 600

atcgagaaac agaacaaaac agaggaggtg ctctctggtt tttccaaacc ggagaaagaa 660

ccggaatttg ggagatata cggatgcgga tactcgggct catctcctga gtcggatata 720

acgttgttgg atttctcaag cgactgtgtg aaagaagatg agagtttctt gatgggtttg 780

cacaagtatc cttctttgga gattgattgg gacgctatag agaaactctt cgga 834

<210> 272
<211> 278
<212> PRT
<213> Arabidopsis thaliana

<400> 272

Met Ala Leu Asn Met Asn Ala Tyr Val Asp Glu Phe Met Glu Ala Leu
1 5 10 15

Glu Pro Phe Met Lys Val Thr Ser Ser Ser Ser Thr Ser Asn Ser Ser
20 25 30

Asn Pro Lys Pro Leu Thr Pro Asn Phe Ile Pro Asn Asn Asp Gln Val
35 40 45

Leu Pro Val Ser Asn Gln Thr Gly Pro Ile Gly Leu Asn Gln Leu Thr
50 55 60

Pro Thr Gln Ile Leu Gln Ile Gln Thr Glu Leu His Leu Arg Gln Asn
65 70 75 80

Gln Ser Arg Arg Arg Ala Gly Ser His Leu Leu Thr Ala Lys Pro Thr
85 90 95

Ser Met Lys Lys Ile Asp Val Ala Thr Lys Pro Val Lys Leu Tyr Arg
100 105 110

Gly Val Arg Gln Arg Gln Trp Gly Lys Trp Val Ala Glu Ile Arg Leu
115 120 125

Pro Lys Asn Arg Thr Arg Leu Trp Leu Gly Thr Phe Glu Thr Ala Gln
130 135 140

Glu Ala Ala Leu Ala Tyr Asp Gln Ala Ala His Lys Ile Arg Gly Asp
145 150 155 160

Asn Ala Arg Leu Asn Phe Pro Asp Ile Val Arg Gln Gly His Tyr Lys

165 170 175

Gln Ile Leu Ser Pro Ser Ile Asn Ala Lys Ile Glu Ser Ile Cys Asn
180 185 190

Ser Ser Asp Leu Pro Leu Pro Gln Ile Glu Lys Gln Asn Lys Thr Glu
195 200 205

Glu Val Leu Ser Gly Phe Ser Lys Pro Glu Lys Glu Pro Glu Phe Gly
210 215 220

Glu Ile Tyr Gly Cys Gly Tyr Ser Gly Ser Ser Pro Glu Ser Asp Ile
225 230 235 240

Thr Leu Leu Asp Phe Ser Ser Asp Cys Val Lys Glu Asp Glu Ser Phe
245 250 255

Leu Met Gly Leu His Lys Tyr Pro Ser Leu Glu Ile Asp Trp Asp Ala
260 265 270

Ile Glu Lys Leu Phe Gly
275

<210> 273
<211> 1083
<212> DNA
<213> Oryza sativa

<400> 273
atggcggcca tagatttgta caagtatcag ctgagctcct cgtcctcgtc ttcttcctcg 60
gatcaggagc tcatgaaagc gctcgaacct tttatcagga gcgcttcacc cacctccacc 120
tccacctcca cgccattgtt ctactcctct agctccatct ccaccaccac caccactccc 180
ttctcctact cgtctccatt gccgcaagaa tcgtactacc tccctgcttc ttcgtcctac 240
gccgccattg ttccacctcc gacgacgacg acgaacacca ccacctcctt ctcggagctc 300
cctcccctgc ctccctcctc ctcgtcgttc gcctcgccgg cgaatgctgc ggcggtgggg 360
ctggctcacc ttggcccgga gcagatccag cagattcagg tgcagttctt gatgcagcag 420
cagctgcagc agcgggggat ggcggcgtcg gcgtcggcgt cggcggcggc gtcgtacctt 480
ggcccgcggg cgcagcccat gaagcaggcc ggggcggcgg cggcggcggc ggccggcggc 540
aagatgtacc gcggcgtgcg gcagcggcac tgggggaagt gggtggcgga gatccggctg 600
ccgaagaacc ggacgcggct gtggctgggc acgttcgaca ccgccgagga cgcggcgctc 660
gcctacgaca aggcggcgtt ccgcctccgc ggcgacgccg cgcgcctcaa cttccccacg 720
ctccgccgcg gcggcgccca cctcgccggc ccgctccacg cctccatcga cgccaagctc 780
accgccatct gccacagcct cgccgccgcg ccgcccgcct cctccaagaa agccgccgcc 840

gccgccgctc acccggactc gcccaaaggc tccgcgtcga cgacgaccac gacgtcggag 900

ggagacgagt cggcgatctc cgcctgttcg cctcctctcc cgccgccgcc accgccgccg 960

ccggcggcgc tccccgagat ggcaaacctt gacttcacgg aggcgccatg ggacgaatcc 1020

gacgccttcc acctctacaa gtgtccgtca tgggagatcg actgggactc catcctctcg 1080

tga 1083

<210> 274
<211> 360
<212> PRT
<213> Oryza sativa

<400> 274

Met Ala Ala Ile Asp Leu Tyr Lys Tyr Gln Leu Ser Ser Ser Ser Ser
1               5                   10                  15

Ser Ser Ser Ser Asp Gln Glu Leu Met Lys Ala Leu Glu Pro Phe Ile
            20                  25                  30

Arg Ser Ala Ser Pro Thr Ser Thr Ser Thr Ser Thr Pro Leu Phe Tyr
        35                  40                  45

Ser Ser Ser Ser Ile Ser Thr Thr Thr Thr Thr Pro Phe Ser Tyr Ser
    50                  55                  60

Ser Pro Leu Pro Gln Glu Ser Tyr Tyr Leu Pro Ala Ser Ser Ser Tyr
65                  70                  75                  80

Ala Ala Ile Val Pro Pro Pro Thr Thr Thr Thr Asn Thr Thr Thr Ser
                85                  90                  95

Phe Ser Glu Leu Pro Pro Leu Pro Pro Ser Ser Ser Ser Phe Ala Ser
            100                 105                 110

Pro Ala Asn Ala Ala Ala Val Gly Leu Ala His Leu Gly Pro Glu Gln
        115                 120                 125

Ile Gln Gln Ile Gln Val Gln Phe Leu Met Gln Gln Gln Leu Gln Gln
    130                 135                 140

Arg Gly Met Ala Ala Ser Ala Ser Ala Ser Ala Ala Ala Ser Tyr Leu
145                 150                 155                 160

Gly Pro Arg Ala Gln Pro Met Lys Gln Ala Gly Ala Ala Ala Ala Ala
                165                 170                 175

Ala Ala Gly Gly Lys Met Tyr Arg Gly Val Arg Gln Arg His Trp Gly
            180                 185                 190

Lys Trp Val Ala Glu Ile Arg Leu Pro Lys Asn Arg Thr Arg Leu Trp
195 200 205

Leu Gly Thr Phe Asp Thr Ala Glu Asp Ala Ala Leu Ala Tyr Asp Lys
210 215 220

Ala Ala Phe Arg Leu Arg Gly Asp Ala Ala Arg Leu Asn Phe Pro Thr
225 230 235 240

Leu Arg Arg Gly Gly Ala His Leu Ala Gly Pro Leu His Ala Ser Ile
245 250 255

Asp Ala Lys Leu Thr Ala Ile Cys His Ser Leu Ala Ala Ala Pro Pro
260 265 270

Ala Ser Ser Lys Lys Ala Ala Ala Ala Ala Ala His Pro Asp Ser Pro
275 280 285

Lys Gly Ser Ala Ser Thr Thr Thr Thr Thr Ser Glu Gly Asp Glu Ser
290 295 300

Ala Ile Ser Ala Cys Ser Pro Pro Leu Pro Pro Pro Pro Pro Pro Pro
305 310 315 320

Pro Ala Ala Leu Pro Glu Met Ala Asn Leu Asp Phe Thr Glu Ala Pro
325 330 335

Trp Asp Glu Ser Asp Ala Phe His Leu Tyr Lys Cys Pro Ser Trp Glu
340 345 350

Ile Asp Trp Asp Ser Ile Leu Ser
355 360

<210> 275
<211> 1076
<212> DNA
<213> Asparagus officinalis

<400> 275
ccacctttga tcactcctcg ccatcaaact ccagtctttt gagcatggac cagcctgatc 60

tgagccaagt tgggccagtt gggctgaccc aactgagccc acttcaaatc cagcagatcc 120

aagctcaaat ccagctcaac caccaacacc agctaatggt ctccagaacc ctgcaagcta 180

gaaattacca taacaccctc ggtgtaaagc cccagcccat gaagctccag gcctcggtcg 240

ctgcacctca gtcaaaaccc acgaagctct acagaggggt gaggcaaagg cactggggta 300

aatgggttgc agagatcaga ctgccgaaga atcgaaccag gctttggctt ggaacctttg 360

acactgctga agaagctgcc ttggcctatg acaaggctgc ttacaaattg agaggggact 420

```
acgcgaggct caatttccct aacctgaagc atacccatct ggctggcaac cctctgcact    480

cgtctgttga tgccaagctc gaagcaattt gccagaccct ggagagccct gggaagaaga    540

aggttagcac cgggtcgaag cctgagccgg ttgtttcatc gccgactgtt gagaccgagg    600

aggagtcttc ctcttcttct tcggtgacgg gggcgacgaa ttctccggtt tcagagatcg    660

agagcttgga tttcaatgag gtgccgtggg atgagactga ggactttgtg ttgaggaaat    720

acccatccta tgagattgat tgggattcta tattgtcttc agcttagtag tgtctggttt    780

gtgcttgttg ttgttagatt tgggggtctt tagtggctgt tgcaaatgga gtttttggca    840

tttgcgtagc cttgttcagg gatttttagt ttagtttatt tttttttctt tcttttagag    900

tgtaagaact catggcctct gctgattatt tttgcttggc gaggccgttg aggggttaag    960

tgtactacta ttgtcagttc caggtgtaac tcttctctgt tgcagctttg taagtatgag   1020

tgtatcttgt gtttaattaa ggtatatttg tagcttttga aaaaaaaaaa aaaaaa       1076
```

<210> 276
<211> 240
<212> PRT
<213> Asparagus officinalis

<400> 276

```
Met Asp Gln Pro Asp Leu Ser Gln Val Gly Pro Val Gly Leu Thr Gln
1               5                   10                  15

Leu Ser Pro Leu Gln Ile Gln Gln Ile Gln Ala Gln Ile Gln Leu Asn
            20                  25                  30

His Gln His Gln Leu Met Val Ser Arg Thr Leu Gln Ala Arg Asn Tyr
        35                  40                  45

His Asn Thr Leu Gly Val Lys Pro Gln Pro Met Lys Leu Gln Ala Ser
    50                  55                  60

Val Ala Ala Pro Gln Ser Lys Pro Thr Lys Leu Tyr Arg Gly Val Arg
65                  70                  75                  80

Gln Arg His Trp Gly Lys Trp Val Ala Glu Ile Arg Leu Pro Lys Asn
                85                  90                  95

Arg Thr Arg Leu Trp Leu Gly Thr Phe Asp Thr Ala Glu Glu Ala Ala
            100                 105                 110

Leu Ala Tyr Asp Lys Ala Ala Tyr Lys Leu Arg Gly Asp Tyr Ala Arg
        115                 120                 125

Leu Asn Phe Pro Asn Leu Lys His Thr His Leu Ala Gly Asn Pro Leu
    130                 135                 140
```

```
His Ser Ser Val Asp Ala Lys Leu Glu Ala Ile Cys Gln Thr Leu Glu
145                 150                 155                 160

Ser Pro Gly Lys Lys Lys Val Ser Thr Gly Ser Lys Pro Glu Pro Val
                165                 170                 175

Val Ser Ser Pro Thr Val Glu Thr Glu Glu Glu Ser Ser Ser Ser Ser
            180                 185                 190

Ser Val Thr Gly Ala Thr Asn Ser Pro Val Ser Glu Ile Glu Ser Leu
        195                 200                 205

Asp Phe Asn Glu Val Pro Trp Asp Glu Thr Glu Asp Phe Val Leu Arg
    210                 215                 220

Lys Tyr Pro Ser Tyr Glu Ile Asp Trp Asp Ser Ile Leu Ser Ser Ala
225                 230                 235                 240


<210>  277
<211>  1013
<212>  DNA
<213>  Aloe vera

<400>  277
aaatccaatc tttcgaccgc cgacctgagc cagctgtgcc cgataggttt gaatcacctg      60

agctcgctcc agatccaaca aattcaagcc cagatccagc tcaaccagca gcagcagctc     120

atgatatcga gagccatgca agcaaggaac tataacctcg ggtgagatc tcagcccatg      180

aagctcgccg ccgctgcagc gccgcctcag ccgaagccga cgaagctcta caggggcgtg     240

aggcagcggc actggggcaa gtgggtggcc gagatcagac taccgaagaa caggacaagg     300

ctctggctcg gcacttttga cacagccgag gaggccgcct tggcctacga caaggccgcc     360

tacaagctga gggggacta cgccaggctc aacttccccc acctgaagca caccggtgcc      420

cacctggctc ccggcggccc cctgcactcc tctgtggacg ccaagctgca ggccatctgc     480

cagagcttgg agcagaataa gagctcaaac tcaaactcat caaagaaaga gaagaggggg     540

gatgcagtag aagaaaaatc tgacaaggtg gtggttgttg ttgccgaggg cgaggagtct     600

tgctcatctt cttcgatgaa cacggggagt gcgagctcgc catcgtcgga gatagagagc     660

ctggacttca cggaggtgcc atgggatgag tctgaggact ttgtgctgag gaaataccct     720

tcatgggaga ttgattggga tgctatactg tcttaatgtt ttggtagtcg ttgtgttttg     780

tggtttgttt ctagggtttt tagtcgttgg tggctggttg caaatggagt tttggcatt      840

tgcacagcct tttagagctc aaggtctttg ctggttattt tttcttggca aaggactggg     900

tggggggagt agttctgttt cagatttcag gttgttgtta ttgtcatctt tgtagtagct     960

ttgtattata atcagtttat ttgtgttcca gttcttaaaa aaaaaaaaaa aaa           1013
```

<210> 278
<211> 211
<212> PRT
<213> Aloe vera

<400> 278

```
Met Ile Ser Arg Ala Met Gln Ala Arg Asn Tyr Asn Leu Gly Val Arg
1               5                   10                  15

Ser Gln Pro Met Lys Leu Ala Ala Ala Ala Ala Pro Pro Gln Pro Lys
            20                  25                  30

Pro Thr Lys Leu Tyr Arg Gly Val Arg Gln Arg His Trp Gly Lys Trp
        35                  40                  45

Val Ala Glu Ile Arg Leu Pro Lys Asn Arg Thr Arg Leu Trp Leu Gly
    50                  55                  60

Thr Phe Asp Thr Ala Glu Glu Ala Ala Leu Ala Tyr Asp Lys Ala Ala
65                  70                  75                  80

Tyr Lys Leu Arg Gly Asp Tyr Ala Arg Leu Asn Phe Pro His Leu Lys
                85                  90                  95

His Thr Gly Ala His Leu Ala Pro Gly Gly Pro Leu His Ser Ser Val
            100                 105                 110

Asp Ala Lys Leu Gln Ala Ile Cys Gln Ser Leu Glu Gln Asn Lys Ser
        115                 120                 125

Ser Asn Ser Asn Ser Ser Lys Lys Glu Lys Arg Gly Asp Ala Val Glu
    130                 135                 140

Glu Lys Ser Asp Lys Val Val Val Val Val Ala Glu Gly Glu Glu Ser
145                 150                 155                 160

Cys Ser Ser Ser Ser Met Asn Thr Gly Ser Ala Ser Ser Pro Ser Ser
                165                 170                 175

Glu Ile Glu Ser Leu Asp Phe Thr Glu Val Pro Trp Asp Glu Ser Glu
            180                 185                 190

Asp Phe Val Leu Arg Lys Tyr Pro Ser Trp Glu Ile Asp Trp Asp Ala
        195                 200                 205

Ile Leu Ser
    210
```

<210> 279

<211> 1493
<212> DNA
<213> Gossypium hirsutum

<400> 279
acctctttta atccgttctg gatttaggac ctgttcttca agttttggta agaaacaggt 60

taatttttta ttaaaatttg gtcctcatct tatttctagt tttagtgtaa tggcagctgc 120

aatggatttc attagtattg gagtagatca atcagatcta tatgggggag aattgatgga 180

agctctcgaa ccttttatga aaagtgtttc ctcctcttcc ccttcccctt ccccttcccc 240

ttccccttct tctcttcctt ctacttctta cctttctttc tcttcttccg aaacacaacc 300

taatttttac ccagatagct gttgttaccc ttaccctaca ccaatggact cagtatcatg 360

tccccaacag cctcaaactg gttcaacaat tgggctaaat agcttgacac aagctcagat 420

ccatcagatc cagcttcaat tccacctcca caacaaccaa ccaagctacc tttgccaaag 480

tccccaaccc aacaccatca gcgccaatag taacccgatg gttagctttc tttgccctaa 540

acctgtccca atgaaacacg tgggtgcgcc atccaaaccc accaaacttt acagaggagt 600

taggcaacgc cactggggaa aatgggtcgc tgagatccgg ttacctagaa accggacacg 660

tctttggtta ggcacttttg acactgctga ggaagcagcc ttggcttatg acaaagcagc 720

ttataaacta agaggtgact ttgcgagact caacttccct aaccttcgtc accacggttc 780

ccacgtaggt gactacaagc ctttgccttc ctctgttgac gctaagcttc aagccatttg 840

tgagagcttg gtacaaaacc ctaagcaagg gagcaagaag aaatcatcca aggttacggc 900

agacaccaaa agcaggaaca acaaaaaatc cgacatggca gagccaaagc ccgaggagaa 960

tacagcgaaa gtggagaact cctcatcttt gtctacggtt caatccgaga gtgagggttc 1020

ggctgtatct tcacctttat cagatcttac attctcggat ttcgacgaac aaccatggcc 1080

ggaagtcgtt tcttcttcgg aaacttttat gttgtccaag taccettcag agatcgattg 1140

ggattccatt ctaaaagcct gaagggaaaa acccaagttt cgttttctgt gtaacagtct 1200

tttgttgttt aggagtttcc tttcgtgtat tttttttgt aaagctagct gcaatggagt 1260

ttttggcagt tgcagtggca tgtattttag gttattaaga gtctgttaat gagtgtaagt 1320

gcttgtgttc ataagaattt gtgattttct ccatgctggt cagctcgttt ttttacttgc 1380

tgaaccaaaa ggtgaaattt gttagtgtct ataaaaaaag tttatagctt ttgtattttg 1440

tacgaattaa atgttattta tgttgttgta ttctctggta tcgtgttttc ttc 1493

<210> 280
<211> 350
<212> PRT
<213> Gossypium hirsutum

<400> 280

Met Ala Ala Ala Met Asp Phe Ile Ser Ile Gly Val Asp Gln Ser Asp
1 5 10 15

```
Leu Tyr Gly Gly Glu Leu Met Glu Ala Leu Glu Pro Phe Met Lys Ser
            20                  25                  30

Val Ser Ser Ser Ser Pro Ser Pro Ser Pro Ser Pro Ser Pro Ser Ser
        35                  40                  45

Leu Pro Ser Thr Ser Tyr Leu Ser Phe Ser Ser Ser Glu Thr Gln Pro
    50                  55                  60

Asn Phe Tyr Pro Asp Ser Cys Cys Tyr Pro Tyr Pro Thr Pro Met Asp
65                  70                  75                  80

Ser Val Ser Cys Pro Gln Gln Pro Gln Thr Gly Ser Thr Ile Gly Leu
                85                  90                  95

Asn Ser Leu Thr Gln Ala Gln Ile His Gln Ile Gln Leu Gln Phe His
            100                 105                 110

Leu His Asn Asn Gln Pro Ser Tyr Leu Cys Gln Ser Pro Gln Pro Asn
        115                 120                 125

Thr Ile Ser Ala Asn Ser Asn Pro Met Val Ser Phe Leu Cys Pro Lys
    130                 135                 140

Pro Val Pro Met Lys His Val Gly Ala Pro Ser Lys Pro Thr Lys Leu
145                 150                 155                 160

Tyr Arg Gly Val Arg Gln Arg His Trp Gly Lys Trp Val Ala Glu Ile
                165                 170                 175

Arg Leu Pro Arg Asn Arg Thr Arg Leu Trp Leu Gly Thr Phe Asp Thr
            180                 185                 190

Ala Glu Glu Ala Ala Leu Ala Tyr Asp Lys Ala Ala Tyr Lys Leu Arg
        195                 200                 205

Gly Asp Phe Ala Arg Leu Asn Phe Pro Asn Leu Arg His His Gly Ser
    210                 215                 220

His Val Gly Asp Tyr Lys Pro Leu Pro Ser Ser Val Asp Ala Lys Leu
225                 230                 235                 240

Gln Ala Ile Cys Glu Ser Leu Val Gln Asn Pro Lys Gln Gly Ser Lys
                245                 250                 255

Lys Lys Ser Ser Lys Val Thr Ala Asp Thr Lys Ser Arg Asn Asn Lys
            260                 265                 270
```

Lys Ser Asp Met Ala Glu Pro Lys Pro Glu Glu Asn Thr Ala Lys Val
275 280 285

Glu Asn Ser Ser Ser Leu Ser Thr Val Gln Ser Glu Ser Glu Gly Ser
290 295 300

Ala Val Ser Ser Pro Leu Ser Asp Leu Thr Phe Ser Asp Phe Asp Glu
305 310 315 320

Gln Pro Trp Pro Glu Val Val Ser Ser Ser Glu Thr Phe Met Leu Ser
325 330 335

Lys Tyr Pro Ser Glu Ile Asp Trp Asp Ser Ile Leu Lys Ala
340 345 350

<210> 281
<211> 993
<212> DNA
<213> Arabidopsis thaliana

<400> 281
atgaatttgt acacttgtag cagatcgttt caagactctg gtggtgaact catggacgcg 60

cttgtacctt ttatcaaaag cgtttccgat tctccttctt cttcttctgc agcgtctgcg 120

tctgcgtttc ttcacccctc tgcgttttct ctccctcctc tccccggtta ttacccggat 180

tcaacgttct tgacccaacc gttttcatac gggtcggatc ttcaacaaac cgggtcatta 240

atcggactca acaacctctc ttcttctcag atccaccaga tccagtctca gatccatcat 300

cctcttcctc cgacgcatca caacaacaac aactctttct cgaatcttct cagcccaaag 360

ccgttactga tgaagcaatc tggagtcgct ggatcttgtt tcgcttacgg ttcaggtgtt 420

ccttcgaagc cgacgaagct ttacagaggt gtgaggcaac gtcactgggg aaaatgggtg 480

gctgagatcc gtttgccgag aaatcggact cgtctctggc ttgggacttt tgacacggcg 540

gaggaagctg cgttggccta tgataaggcg gcgtacaagc tgcgcggcga tttcgcccgg 600

cttaacttcc ctaacctacg tcataacgga tctcacatcg gaggcgattt cggtgaatat 660

aaacctcttc actcctcagt cgacgctaag cttgaagcta tttgtaaaag catggcggag 720

actcagaaac aggacaaatc gacgaaatca tcgaagaaac gtgagaagaa ggtttcgtcg 780

ccagatctat cggagaaagt gaaggcggag gagaattcgg tttcgatcgg tggatctcca 840

ccggtgacgg agtttgaaga gtccaccgct ggatcttcgc cgttgtcgga cttgacgttc 900

gctgacccgg aggagccgcc gcagtggaac gagacgttct cgttggagaa gtatccgtcg 960

tacgagatcg attgggattc gattctagct tag 993

<210> 282
<211> 330
<212> PRT

<213> Arabidopsis thaliana

<400> 282

Met Asn Leu Tyr Thr Cys Ser Arg Ser Phe Gln Asp Ser Gly Gly Glu
1 5 10 15

Leu Met Asp Ala Leu Val Pro Phe Ile Lys Ser Val Ser Asp Ser Pro
20 25 30

Ser Ser Ser Ser Ala Ala Ser Ala Ser Ala Phe Leu His Pro Ser Ala
35 40 45

Phe Ser Leu Pro Pro Leu Pro Gly Tyr Tyr Pro Asp Ser Thr Phe Leu
50 55 60

Thr Gln Pro Phe Ser Tyr Gly Ser Asp Leu Gln Gln Thr Gly Ser Leu
65 70 75 80

Ile Gly Leu Asn Asn Leu Ser Ser Ser Gln Ile His Gln Ile Gln Ser
85 90 95

Gln Ile His His Pro Leu Pro Pro Thr His His Asn Asn Asn Asn Ser
100 105 110

Phe Ser Asn Leu Leu Ser Pro Lys Pro Leu Leu Met Lys Gln Ser Gly
115 120 125

Val Ala Gly Ser Cys Phe Ala Tyr Gly Ser Gly Val Pro Ser Lys Pro
130 135 140

Thr Lys Leu Tyr Arg Gly Val Arg Gln Arg His Trp Gly Lys Trp Val
145 150 155 160

Ala Glu Ile Arg Leu Pro Arg Asn Arg Thr Arg Leu Trp Leu Gly Thr
165 170 175

Phe Asp Thr Ala Glu Glu Ala Ala Leu Ala Tyr Asp Lys Ala Ala Tyr
180 185 190

Lys Leu Arg Gly Asp Phe Ala Arg Leu Asn Phe Pro Asn Leu Arg His
195 200 205

Asn Gly Ser His Ile Gly Gly Asp Phe Gly Glu Tyr Lys Pro Leu His
210 215 220

Ser Ser Val Asp Ala Lys Leu Glu Ala Ile Cys Lys Ser Met Ala Glu
225 230 235 240

Thr Gln Lys Gln Asp Lys Ser Thr Lys Ser Ser Lys Lys Arg Glu Lys

245 250 255

Lys Val Ser Ser Pro Asp Leu Ser Glu Lys Val Lys Ala Glu Glu Asn
260 265 270

Ser Val Ser Ile Gly Gly Ser Pro Pro Val Thr Glu Phe Glu Glu Ser
275 280 285

Thr Ala Gly Ser Ser Pro Leu Ser Asp Leu Thr Phe Ala Asp Pro Glu
290 295 300

Glu Pro Pro Gln Trp Asn Glu Thr Phe Ser Leu Glu Lys Tyr Pro Ser
305 310 315 320

Tyr Glu Ile Asp Trp Asp Ser Ile Leu Ala
325 330

<210> 283
<211> 1825
<212> DNA
<213> Arabidopsis thaliana

<400> 283
atcggtgagg ttgagagtaa ttcactacac acacacaaaa aataaattga gtgcctcccc 60

caaaaacaaa attggtagat aacgagcaat tgtttttttt cagatttgat cctgaatttt 120

tacatttttt tttttgcaat ctccccctaa tctgttgttt ctcgcttctt cttctgttaa 180

tcatctgtct ttcaaaaaga aagaaaaaag aaaaattcga tttctgggtt tgtttttgtc 240

atacagaaaa aaatcaagct tatgaatttg tgtttaattt tttgttttaa tttgaaaggc 300

aggtttttc agaacgagat cgtttttca aatttcttct gattttacct cttttttct 360

tcttagattt tagtgaatcg agggtgaaat ttttgattcc ctcttttcgg atctacacag 420

aggttgctta tttcaaacct tttagatcca tttttttta attttctcgg aaaaatccct 480

gtttctttac ttttttataa gtctcaggtt caatttttc ggattcaaat ttttatttta 540

aatggcagct gctatgaatt tgtacacttg tagcagatcg tttcaagact ctggtggtga 600

actcatggac gcgcttgtac cttttatcaa aagcgtttcc gattctcctt cttcttcttc 660

tgcagcgtct gcgtctgcgt ttcttcaccc ctctgcgttt tctctccctc ctctccccgg 720

ttattacccg gattcaacgt tcttgaccca accgttttca tacgggtcgg atcttcaaca 780

aaccgggtca ttaatcggac tcaacaacct ctcttcttct cagatccacc agatccagtc 840

tcagatccat catcctcttc ctccgacgca tcacaacaac aacaactctt tctcgaatct 900

tctcagccca aagccgttac tgatgaagca atctggagtc gctggatctt gtttcgctta 960

cggttcaggt gttccttcga agccgacgaa gctttacaga ggtgtgaggc aacgtcactg 1020

ggaaaaatgg gtggctgaga tccgtttgcc gagaaatcgg actcgtctct ggcttgggac 1080

ttttgacacg gcggaggaag ctgcgttggc ctatgataag gcggcgtaca agctgcgcgg 1140

cgatttcgcc cggcttaact tccctaacct acgtcataac ggatctcaca tcggaggcga 1200

tttcggtgaa tataaacctc ttcactcctc agtcgacgct aagcttgaag ctatttgtaa 1260

aagcatggcg gagactcaga aacaggacaa atcgacgaaa tcatcgaaga aacgtgagaa 1320

gaaggtttcg tcgccagatc tatcggagaa agtgaaggcg gaggagaatt cggtttcgat 1380

cggtggatct ccaccggtga cggagtttga agagtccacc gctggatctt cgccgttgtc 1440

ggacttgacg ttcgctgacc cggaggagcc gccgcagtgg aacgagacgt tctcgttgga 1500

gaagtatccg tcgtacgaga tcgattggga ttcgattcta gcttaggggc aaaataggaa 1560

attcagccgc ttgcaatgga gtttttgtga aattgcatga ctggcccaag agtaattaat 1620

taaatatgga ttagtgttaa atttcgtatg ttaatatttg tattatggtt tgtattagtc 1680

tctctgtgtc ggtccagctt gcggtttttt gtcaggctcg accatgccac agttttcatt 1740

ttatgtaatc ttttttcttt ttgtcttatg taatttgtag cttcagtttc ttcatctata 1800

atgcaatttt attatgatta tgtaa 1825

<210> 284
<211> 334
<212> PRT
<213> Arabidopsis thaliana

<400> 284

Met Ala Ala Ala Met Asn Leu Tyr Thr Cys Ser Arg Ser Phe Gln Asp
1 5 10 15

Ser Gly Gly Glu Leu Met Asp Ala Leu Val Pro Phe Ile Lys Ser Val
20 25 30

Ser Asp Ser Pro Ser Ser Ser Ser Ala Ala Ser Ala Ser Ala Phe Leu
35 40 45

His Pro Ser Ala Phe Ser Leu Pro Pro Leu Pro Gly Tyr Tyr Pro Asp
50 55 60

Ser Thr Phe Leu Thr Gln Pro Phe Ser Tyr Gly Ser Asp Leu Gln Gln
65 70 75 80

Thr Gly Ser Leu Ile Gly Leu Asn Asn Leu Ser Ser Ser Gln Ile His
85 90 95

Gln Ile Gln Ser Gln Ile His His Pro Leu Pro Pro Thr His His Asn
100 105 110

Asn Asn Asn Ser Phe Ser Asn Leu Leu Ser Pro Lys Pro Leu Leu Met
115 120 125

Lys Gln Ser Gly Val Ala Gly Ser Cys Phe Ala Tyr Gly Ser Gly Val
130 135 140

Pro Ser Lys Pro Thr Lys Leu Tyr Arg Gly Val Arg Gln Arg His Trp
145 150 155 160

Gly Lys Trp Val Ala Glu Ile Arg Leu Pro Arg Asn Arg Thr Arg Leu
165 170 175

Trp Leu Gly Thr Phe Asp Thr Ala Glu Glu Ala Ala Leu Ala Tyr Asp
180 185 190

Lys Ala Ala Tyr Lys Leu Arg Gly Asp Phe Ala Arg Leu Asn Phe Pro
195 200 205

Asn Leu Arg His Asn Gly Ser His Ile Gly Gly Asp Phe Gly Glu Tyr
210 215 220

Lys Pro Leu His Ser Ser Val Asp Ala Lys Leu Glu Ala Ile Cys Lys
225 230 235 240

Ser Met Ala Glu Thr Gln Lys Gln Asp Lys Ser Thr Lys Ser Ser Lys
245 250 255

Lys Arg Glu Lys Lys Val Ser Ser Pro Asp Leu Ser Glu Lys Val Lys
260 265 270

Ala Glu Glu Asn Ser Val Ser Ile Gly Gly Ser Pro Pro Val Thr Glu
275 280 285

Phe Glu Glu Ser Thr Ala Gly Ser Ser Pro Leu Ser Asp Leu Thr Phe
290 295 300

Ala Asp Pro Glu Glu Pro Pro Gln Trp Asn Glu Thr Phe Ser Leu Glu
305 310 315 320

Lys Tyr Pro Ser Tyr Glu Ile Asp Trp Asp Ser Ile Leu Ala
325 330

<210> 285
<211> 1224
<212> DNA
<213> Arabidopsis thaliana

<400> 285
aaaaaacaaa tatttagaaa caaaaaatgc tataattctt ccttttcttt gttctttcag 60

agattttgat ttctattcaa tcgactaaga gggtgtgttt ttaatccctc tctgttttaa 120

ttttgtttcc tagtctttaa agatccatgg cagccataga tatgttcaat agcaacacag 180

```
atccttttca agaagagctc atgaaagcac ttcaacctta taccaccaac actgattctt    240

cttctcctac gtattcaaac acagtcttcg gtttcaatca aaccacatct ctcggtctaa    300

accagctcac accttaccaa atccaccaaa tccaaaacca gcttaaccag agacgtaaca    360

taatctctcc aaatctagcc ccaaagcctg tcccaatgaa gaacatgacc gctcagaaac    420

tctatagagg agttagacaa aggcactggg gaaaatgggt agctgagatc cgtttaccca    480

agaaccggac ccgactctgg cttggaactt tcgacacagc tgaagaagca gccatggctt    540

atgacctagc tgcttacaag ctaagaggcg agttcgcgag acttaatttc ccacagttca    600

gacacgagga tggatactac ggaggaggta gctgtttcaa tcctcttcat tcctctgtcg    660

acgcaaagct ccaagagatt tgtcagagct tgagaaaaac agaggatatt gacctcccct    720

gttctgaaac agagcttttc ccgccaaaaa cagagtatca agaaagtgaa tatgggttct    780

tgagatctga tgagaattcg ttttcagatg agtctcatgt ggaatcttct tcgccggaat    840

ctggtattac tacgttcttg gacttttcgg attctggatt tgatgagatt gggagtttcg    900

ggctggagaa gtttccttct gtggagattg attgggatgc gattagcaaa ttgtccgaat    960

cttaaacaaa gcaaagagaa gactttttct tttaggagtt tgtctttcaa tttcagtgtc   1020

ttatattaat ctctctgcaa ctgaaatttt taacagttgc ggagagaatc gtctctaggg   1080

tttgtttctc ttcctccatg ttttggtctg actggttaat gtcttttttt ttttttgaa   1140

ctttcaaaaa cctttgtcat tgaccaatcg gagaagtttc catgtattct ctcatcttta   1200

aatttctaat gaagaatgta aatt                                          1224
```

```
<210>  286
<211>  272
<212>  PRT
<213>  Arabidopsis thaliana

<400>  286

Met Ala Ala Ile Asp Met Phe Asn Ser Asn Thr Asp Pro Phe Gln Glu
1               5                   10                  15

Glu Leu Met Lys Ala Leu Gln Pro Tyr Thr Thr Asn Thr Asp Ser Ser
            20                  25                  30

Ser Pro Thr Tyr Ser Asn Thr Val Phe Gly Phe Asn Gln Thr Thr Ser
        35                  40                  45

Leu Gly Leu Asn Gln Leu Thr Pro Tyr Gln Ile His Gln Ile Gln Asn
    50                  55                  60

Gln Leu Asn Gln Arg Arg Asn Ile Ile Ser Pro Asn Leu Ala Pro Lys
65                  70                  75                  80

Pro Val Pro Met Lys Asn Met Thr Ala Gln Lys Leu Tyr Arg Gly Val
```

85 90 95

Arg Gln Arg His Trp Gly Lys Trp Val Ala Glu Ile Arg Leu Pro Lys
100 105 110

Asn Arg Thr Arg Leu Trp Leu Gly Thr Phe Asp Thr Ala Glu Glu Ala
115 120 125

Ala Met Ala Tyr Asp Leu Ala Ala Tyr Lys Leu Arg Gly Glu Phe Ala
130 135 140

Arg Leu Asn Phe Pro Gln Phe Arg His Glu Asp Gly Tyr Tyr Gly Gly
145 150 155 160

Gly Ser Cys Phe Asn Pro Leu His Ser Ser Val Asp Ala Lys Leu Gln
165 170 175

Glu Ile Cys Gln Ser Leu Arg Lys Thr Glu Asp Ile Asp Leu Pro Cys
180 185 190

Ser Glu Thr Glu Leu Phe Pro Pro Lys Thr Glu Tyr Gln Glu Ser Glu
195 200 205

Tyr Gly Phe Leu Arg Ser Asp Glu Asn Ser Phe Ser Asp Glu Ser His
210 215 220

Val Glu Ser Ser Ser Pro Glu Ser Gly Ile Thr Thr Phe Leu Asp Phe
225 230 235 240

Ser Asp Ser Gly Phe Asp Glu Ile Gly Ser Phe Gly Leu Glu Lys Phe
245 250 255

Pro Ser Val Glu Ile Asp Trp Asp Ala Ile Ser Lys Leu Ser Glu Ser
260 265 270

<210> 287
<211> 966
<212> DNA
<213> Oryza sativa

<400> 287
atggatcgcc aatggcgttg cctcgctgct gcggcttcta ctagtggtaa tagtaagctt 60

cctccgctgc cgatggcgct gggcggcgcc gtggagtacg ggcagctcgt ccatggcgcg 120

gcggcgcccg tggcgccgtt cttcgtggac gccgagcagc agagcctgtc gccggcgacg 180

gccatggttc ttggcgccgg gtggtataac tataaccttg tcacgccgtc gcaggcggcg 240

cagctgcacc accggctgcg acgggcggtg ggcgcggcgc cgtgctcgat gaagcggtgc 300

ggtggcatgg cggcggcggc ggcggggcgg cttgcgctgg tggggccggc gccggtgcag 360

gcgaagctgt accgcggcgt gcggcagcgg cactggggga aatgggtggc ggagatccgg 420

ctgccgcgga accggacgcg gctgtggctg ggcacgtacg acaccgccga ggacgccgcg 480

ctcgcctacg acggtgccgc gttccggctg cgcggcgacg ccgcccgcct caacttcccc 540

gagctccggc gcggagggcg gcaccacgcg ccggcgctca gcgcgtccgt cgacgccaag 600

atcctccaag ccaccaccac caccacggcg gacaccgccg ccgccgcagc accggcgtcg 660

acgaacacca cgccgccgcc gtcgccgcgc gtcgtcaaga ccgagccggg ctgctgctcc 720

gtctccgaag cctccacgac gacgacggcc gacgccgccg acgtctcgtc cacagggtct 780

tccccatccc cgacctcatc gaatcaggcc gccaccgcca cgccgccggc gccgcggccg 840

ccgccgccgt tgccggagac gattcagcag ctggacttca cggaggcgcc atgggacgag 900

gccgacggct tcgcgctgcg ccggtatccg tcgtgggaga tcgactggga cgccatcctc 960

tcctga 966

<210> 288
<211> 321
<212> PRT
<213> Oryza sativa

<400> 288

Met Asp Arg Gln Trp Arg Cys Leu Ala Ala Ala Ala Ser Thr Ser Gly
1 5 10 15

Asn Ser Lys Leu Pro Pro Leu Pro Met Ala Leu Gly Gly Ala Val Glu
20 25 30

Tyr Gly Gln Leu Val His Gly Ala Ala Ala Pro Val Ala Pro Phe Phe
35 40 45

Val Asp Ala Glu Gln Gln Ser Leu Ser Pro Ala Thr Ala Met Val Leu
50 55 60

Gly Ala Gly Trp Tyr Asn Tyr Asn Leu Val Thr Pro Ser Gln Ala Ala
65 70 75 80

Gln Leu His His Arg Leu Arg Arg Ala Val Gly Ala Ala Pro Cys Ser
85 90 95

Met Lys Arg Cys Gly Gly Met Ala Ala Ala Ala Ala Gly Arg Leu Ala
100 105 110

Leu Val Gly Pro Ala Pro Val Gln Ala Lys Leu Tyr Arg Gly Val Arg
115 120 125

Gln Arg His Trp Gly Lys Trp Val Ala Glu Ile Arg Leu Pro Arg Asn
130 135 140

Arg Thr Arg Leu Trp Leu Gly Thr Tyr Asp Thr Ala Glu Asp Ala Ala
145 150 155 160

Leu Ala Tyr Asp Gly Ala Ala Phe Arg Leu Arg Gly Asp Ala Ala Arg
165 170 175

Leu Asn Phe Pro Glu Leu Arg Arg Gly Gly Arg His His Ala Pro Ala
180 185 190

Leu Ser Ala Ser Val Asp Ala Lys Ile Leu Gln Ala Thr Thr Thr Thr
195 200 205

Thr Ala Asp Thr Ala Ala Ala Ala Ala Pro Ala Ser Thr Asn Thr Thr
210 215 220

Pro Pro Pro Ser Pro Arg Val Val Lys Thr Glu Pro Gly Cys Cys Ser
225 230 235 240

Val Ser Glu Ala Ser Thr Thr Thr Thr Ala Asp Ala Ala Asp Val Ser
245 250 255

Ser Thr Gly Ser Ser Pro Ser Pro Thr Ser Ser Asn Gln Ala Ala Thr
260 265 270

Ala Thr Pro Pro Ala Pro Arg Pro Pro Pro Pro Leu Pro Glu Thr Ile
275 280 285

Gln Gln Leu Asp Phe Thr Glu Ala Pro Trp Asp Glu Ala Asp Gly Phe
290 295 300

Ala Leu Arg Arg Tyr Pro Ser Trp Glu Ile Asp Trp Asp Ala Ile Leu
305 310 315 320

Ser

<210> 289
<211> 1384
<212> DNA
<213> Broussonetia papyrifera

<400> 289
caccacacca gttttctctg atccctttag agaagagctc atgaaagcac ttgaaccttt 60

tatgaaaagt gctcataata atatcaacaa caacatttca ccaatctctt cttcttcctc 120

ttcttcttac ctttctagtg agcccaactt ttaccctgaa tctgaaactt gctcaccttc 180

aactacccaa atgttttcca gtgggttgtt ctccagcttt aaccacatgg gttctgagca 240

gactggttct ttaggcttaa accagctcac ccaatcccag attctccaaa ttcaagccca 300

aatctacttc caacaacaac agcaacagca acaaaatcta ctcatgacca ccataactcc 360

gccccaaaac agcctcaact acctcggtcc gagggcggtt ccgatgaaga acgtcggcgc 420

caattcaaag cccaacaaac tctacagagg agtgaggcag aggcattggg gcaaatgggt 480

cgccgagatc agactcccca agaaccggac ccgcctctgg ctgggcacct tcgacaccgc 540

cgaggaagcc gccttggcct acgacaaggc ggcgtacaag ctccgcgggg acttcgcccg 600

cctcaacttc ccccacctcc gccacgaagg cgcccacgtc tctggcgagt tcggcgagta 660

caagcccctc cattcctccg tcgacgccaa gcttcaggcg atttgccaaa gcctggcgaa 720

ttcgcagaag caggggagtg ccaaggaggc ttgttccgag ccggaggtga agccagtcat 780

cgagcccaag atggcgtccg ataattctcc gaaaggcgaa ttggaggtgt cgtcttcgtc 840

gttgtcgtcg tcgtcgtcgt tgtcgttgtc gttgtcgttg tcgtcgccgt tgtcggatga 900

gtcttcggcg ggtcatcct cgccggagtc cgatgtcacg ttgttggact tctcggattc 960

tcattgggat gggaatgaga attttgggct agggaagtac ccttcagtgg agatcgactg 1020

ggatgctctg tgatcgtaat aaatgttggt tatgttgtca ttttctcttt tttatttttc 1080

ctgcgttatt agttgttttt aaggtttttt tttttagttg tacaagcacg gcttctgcga 1140

tggaattttt aacatggcag ggtttagct cagtttttta aatttaggaa gggtcagtaa 1200

gtcagtcagt cagtcagatg tttttatgtt gtaatatttg atgtcgtttg atatctccta 1260

tgttggtcag ctggtagttt ttttccttgc taggcctggc catgggagat ctctctgggt 1320

tgtatttact actttttgaa tgtaattagg caagtctact ttatataaaa aaaaaaaaaa 1380

aaaa 1384

<210> 290
<211> 330
<212> PRT
<213> Broussonetia papyrifera

<400> 290

Met Lys Ala Leu Glu Pro Phe Met Lys Ser Ala His Asn Asn Ile Asn
1 5 10 15

Asn Asn Ile Ser Pro Ile Ser Ser Ser Ser Ser Ser Ser Tyr Leu Ser
20 25 30

Ser Glu Pro Asn Phe Tyr Pro Glu Ser Glu Thr Cys Ser Pro Ser Thr
35 40 45

Thr Gln Met Phe Ser Ser Gly Leu Phe Ser Ser Phe Asn His Met Gly
50 55 60

Ser Glu Gln Thr Gly Ser Leu Gly Leu Asn Gln Leu Thr Gln Ser Gln
65 70 75 80

Ile Leu Gln Ile Gln Ala Gln Ile Tyr Phe Gln Gln Gln Gln Gln Gln
85 90 95

Gln Gln Asn Leu Leu Met Thr Thr Ile Thr Pro Pro Gln Asn Ser Leu
100 105 110

Asn Tyr Leu Gly Pro Arg Ala Val Pro Met Lys Asn Val Gly Ala Asn
115 120 125

Ser Lys Pro Asn Lys Leu Tyr Arg Gly Val Arg Gln Arg His Trp Gly
130 135 140

Lys Trp Val Ala Glu Ile Arg Leu Pro Lys Asn Arg Thr Arg Leu Trp
145 150 155 160

Leu Gly Thr Phe Asp Thr Ala Glu Glu Ala Ala Leu Ala Tyr Asp Lys
165 170 175

Ala Ala Tyr Lys Leu Arg Gly Asp Phe Ala Arg Leu Asn Phe Pro His
180 185 190

Leu Arg His Glu Gly Ala His Val Ser Gly Glu Phe Gly Glu Tyr Lys
195 200 205

Pro Leu His Ser Ser Val Asp Ala Lys Leu Gln Ala Ile Cys Gln Ser
210 215 220

Leu Ala Asn Ser Gln Lys Gln Gly Ser Ala Lys Glu Ala Cys Ser Glu
225 230 235 240

Pro Glu Val Lys Pro Val Ile Glu Pro Lys Met Ala Ser Asp Asn Ser
245 250 255

Pro Lys Gly Glu Leu Glu Val Ser Ser Ser Ser Leu Ser Ser Ser Ser
260 265 270

Ser Leu Ser Leu Ser Leu Ser Leu Ser Ser Pro Leu Ser Asp Glu Ser
275 280 285

Ser Ala Gly Ser Ser Ser Pro Glu Ser Asp Val Thr Leu Leu Asp Phe
290 295 300

Ser Asp Ser His Trp Asp Gly Asn Glu Asn Phe Gly Leu Gly Lys Tyr
305 310 315 320

Pro Ser Val Glu Ile Asp Trp Asp Ala Leu
325 330

<210> 291
<211> 993
<212> DNA
<213> Jatropha curcas

<400> 291
ggactcagat agttgctcca catcgactgc ccttccattt tcaaatgggt tctcgatcca 60

agaccccaac cgtcttcagc aacctacttg ttcaatcggg ctatgtctta ctccaaccca 120

gatccaccag atccagaccc aaatccatta ccagaatcaa aatggattca atttccagaa 180

tttccacacc caaaaccaac atggcttaac tttttaggtc cgaaacccgt gcccatgaag 240

caggtgggtt caccaccaaa acccactaag ctctacagag gagtaaggca gcgacattgg 300

ggcaaatggg ttgccgagat ccgactaccc aagaaccgta cacgactctg gcttggtact 360

tttgacacag cagaagaagc cgctttagct tatgacaaag cggcgtacaa actccgtggc 420

gacttcgcga gacttaactt ccctaacctc cgccaccaag ggtcccacat tgaaggcagc 480

ttcggcgagt ataagcctct ccattcctcg gtcgatgcga aactgcaagc tatttgtcaa 540

agcttagcag aatcgcagaa acaaggagga aaagcagaga agcaatcaaa ctcgtcagcg 600

aaaaagaaga cttcggtggg gactactcca gcgacggcgg agaaggttaa ggaagctaag 660

gcaccgcaac aggttgttcc ggacaagtgt tgcaaggtcg agacaccatc gtcagtgttg 720

acagaaagtg aagcctctgg cggatcttca ccgttgtcgg atcttacgtt tccggatcta 780

gaagaggcac cattggatgt tgattctgga aattttaatt tggagaagta cccatcttat 840

gaaattgatt gggcttctct tttatcttct tagatttggt tatgttatgt tatttatctt 900

ttatctttat tttgcagtta tgtttagttc ttaggcgtgt ggttgctgca atgagttttt 960

ggcagttgca gagccaaaaa aaaaaaaaaa aaa 993

<210> 292
<211> 256
<212> PRT
<213> Jatropha curcas

<400> 292

Met Ser Tyr Ser Asn Pro Asp Pro Pro Asp Pro Asp Pro Asn Pro Leu
1 5 10 15

Pro Glu Ser Lys Trp Ile Gln Phe Pro Glu Phe Pro His Pro Lys Pro
20 25 30

Thr Trp Leu Asn Phe Leu Gly Pro Lys Pro Val Pro Met Lys Gln Val
35 40 45

Gly Ser Pro Pro Lys Pro Thr Lys Leu Tyr Arg Gly Val Arg Gln Arg
50 55 60

His Trp Gly Lys Trp Val Ala Glu Ile Arg Leu Pro Lys Asn Arg Thr

65 70 75 80

Arg Leu Trp Leu Gly Thr Phe Asp Thr Ala Glu Glu Ala Ala Leu Ala
85 90 95

Tyr Asp Lys Ala Ala Tyr Lys Leu Arg Gly Asp Phe Ala Arg Leu Asn
100 105 110

Phe Pro Asn Leu Arg His Gln Gly Ser His Ile Glu Gly Ser Phe Gly
115 120 125

Glu Tyr Lys Pro Leu His Ser Ser Val Asp Ala Lys Leu Gln Ala Ile
130 135 140

Cys Gln Ser Leu Ala Glu Ser Gln Lys Gln Gly Gly Lys Ala Glu Lys
145 150 155 160

Gln Ser Asn Ser Ser Ala Lys Lys Lys Thr Ser Val Gly Thr Thr Pro
165 170 175

Ala Thr Ala Glu Lys Val Lys Glu Ala Lys Ala Pro Gln Gln Val Val
180 185 190

Pro Asp Lys Cys Cys Lys Val Glu Thr Pro Ser Ser Val Leu Thr Glu
195 200 205

Ser Glu Ala Ser Gly Gly Ser Ser Pro Leu Ser Asp Leu Thr Phe Pro
210 215 220

Asp Leu Glu Glu Ala Pro Leu Asp Val Asp Ser Gly Asn Phe Asn Leu
225 230 235 240

Glu Lys Tyr Pro Ser Tyr Glu Ile Asp Trp Ala Ser Leu Leu Ser Ser
245 250 255

<210> 293
<211> 969
<212> DNA
<213> Arabidopsis thaliana

<400> 293
tcttcctccg acgcatcaca acaacaacaa ctctttctcg aatcttctca gcccaaagcc 60

gttactgatg aagcaatctg gagtcgctgg atcttgtttc gcttacggtt caggtgttcc 120

ttcgaagccg acgaagcttt acagaggtgt gaggcaacgt cactggggaa aatgggtggc 180

tgagatccgt ttgccgagaa atcggactcg tctctggctt gggacttttg acacggcgga 240

ggaagctgcg ttggcctatg ataaggcggc gtacaagctg cgcggcgatt tcgcccggct 300

taacttccct aacctacgtc ataacggatt tcacatcgga ggcgatttcg gtgaatataa 360

acctcttcac tcctcagtcg acgctaagct tgaagctatt tgtaaaagca tggcggagac 420

tcagaaacag gacaaatcga cgaaatcatc gaagaaacgt gagaagaagg tttcgtcgcc 480

agatctatcg gagaaagtga aggcggagga gaattcggtt tcgatcggtg gatctccacc 540

ggtgacggag tttgaagagt ccaccgctgg atcttcgccg ttgtcggact tgacgttcgc 600

tgacccggag gagccgccgc agtggaacga gacgttctcg ttggagaagt atccgtcgta 660

cgagatcgat tgggattcga ttctagctta ggggcaaaat aggaaattca gccgcttgca 720

atggagtttt tgtgaaattg catgactggc ccaagagtaa ttaattaaat atggattagt 780

gttaaatttc gtatgttaat atttgtatta tggtttgtat tagtctctct gtgtcggtcc 840

agcttgcggt tttttgtcag gctcgaccat gccacagttt tcattttatg taatcttttt 900

ttcttttgtc ttatgtaatt tgtagcttca gtttcttcat ctataatgca attttattat 960

gattatgtg 969

<210> 294
<211> 229
<212> PRT
<213> Arabidopsis thaliana

<400> 294

Leu Pro Pro Thr His His Asn Asn Asn Asn Ser Phe Ser Asn Leu Leu
1 5 10 15

Ser Pro Lys Pro Leu Leu Met Lys Gln Ser Gly Val Ala Gly Ser Cys
20 25 30

Phe Ala Tyr Gly Ser Gly Val Pro Ser Lys Pro Thr Lys Leu Tyr Arg
35 40 45

Gly Val Arg Gln Arg His Trp Gly Lys Trp Val Ala Glu Ile Arg Leu
50 55 60

Pro Arg Asn Arg Thr Arg Leu Trp Leu Gly Thr Phe Asp Thr Ala Glu
65 70 75 80

Glu Ala Ala Leu Ala Tyr Asp Lys Ala Ala Tyr Lys Leu Arg Gly Asp
85 90 95

Phe Ala Arg Leu Asn Phe Pro Asn Leu Arg His Asn Gly Phe His Ile
100 105 110

Gly Gly Asp Phe Gly Glu Tyr Lys Pro Leu His Ser Ser Val Asp Ala
115 120 125

Lys Leu Glu Ala Ile Cys Lys Ser Met Ala Glu Thr Gln Lys Gln Asp
130 135 140

Lys Ser Thr Lys Ser Ser Lys Lys Arg Glu Lys Lys Val Ser Ser Pro
145 150 155 160

Asp Leu Ser Glu Lys Val Lys Ala Glu Glu Asn Ser Val Ser Ile Gly
165 170 175

Gly Ser Pro Pro Val Thr Glu Phe Glu Glu Ser Thr Ala Gly Ser Ser
180 185 190

Pro Leu Ser Asp Leu Thr Phe Ala Asp Pro Glu Glu Pro Pro Gln Trp
195 200 205

Asn Glu Thr Phe Ser Leu Glu Lys Tyr Pro Ser Tyr Glu Ile Asp Trp
210 215 220

Asp Ser Ile Leu Ala
225

<210> 295
<211> 1407
<212> DNA
<213> Oryza sativa

<400> 295
cttggttagg ttgcttgcag agagcgagag acccagggcc ttttagatcc agggctccca 60
gatctgctgt ttttctttca tcttctttgt gttctagtgt taggttggta gccaagaggg 120
gttctttttc cacacctcac ttgctagatc taccaccaaa aagccatctg tttttttact 180
gtggctgtcg atttctccct ccttcctgcc tgttcttgat tttggattcg gaaccaggca 240
aatctttgtt actactgttt tagtatcaga aaaaaaaatc ccaaaaaaaa gagtgagtag 300
atggctgcag ctatagatct gtcaggggag gagctgatga gagcactcga gccttttatc 360
cgagatgcct ccggctcgcc tccggtttgt tcccagttta gtcccacctc gccattctcc 420
ttcccgcacg cgttcgcgta cggtggcggg ctggcccagc agccagagct cagcccggcc 480
cagatgcact acatccaggc ccgcctccac ctccagcggc aggcggcgca ggccggcccg 540
ctcggcccgc gcgcgcagcc gatgaaggcg tcgtcgtcgt cggcttcggc ggcgggggcg 600
gcggcgacgc cgccgcggcc gcagaagctg taccgcggcg tgcggcagcg gcactggggg 660
aagtgggtgg cggagatccg cctcccgcgg aaccgcacgc ggctctggct cggcaccttc 720
gacacagccg aggaggcggc gcttgcctac gaccaggcgg cgtaccgcct ccgcggcgac 780
gcggcgcggc tcaacttccc cgacaacgcc gcctcccgcg gcccgctcca cgcctccgtc 840
gacgccaagc tccagacgct ctgccagaac atcgccgccg ccaagaacgc caagaagtcc 900
tctgtctccg cctccgccgc cgcaacatcc tccgcgccca ccagcaactg ctcgtcgccg 960
tcctccgacg acgcgtcgtc ctgcctggag tccgccgact cgtcgccctc cctgtcgccg 1020

tcctccgccg ccaccacggc cgagacgccg gcgaccgtgc cggagatgca gcagctcgac 1080

ttcagcgagg cgccgtggga cgaggccgcc gccttcgccc tcaccaagta cccgtcctac 1140

gagatcgact gggactccct cctcgccgcc aattaacacc accaccactt cttggctagt 1200

actcacgccg tcgttagcct aatcgtcgcc gccgccgccg ccgtgcagat gggattttag 1260

acattctgca ccgcacggac gggcatggat tagcagtttt atagtcccta atccttttgg 1320

tttggttcgt ttgtgtacgt agatcgatct ctctccggac ttgtttcctc tgtagatgct 1380

agggttggtt ggtgttcttc ctcaacc 1407

<210> 296
<211> 291
<212> PRT
<213> Oryza sativa

<400> 296

Met Ala Ala Ala Ile Asp Leu Ser Gly Glu Glu Leu Met Arg Ala Leu
1 5 10 15

Glu Pro Phe Ile Arg Asp Ala Ser Gly Ser Pro Pro Val Cys Ser Gln
20 25 30

Phe Ser Pro Thr Ser Pro Phe Ser Phe Pro His Ala Phe Ala Tyr Gly
35 40 45

Gly Gly Leu Ala Gln Gln Pro Glu Leu Ser Pro Ala Gln Met His Tyr
50 55 60

Ile Gln Ala Arg Leu His Leu Gln Arg Gln Ala Ala Gln Ala Gly Pro
65 70 75 80

Leu Gly Pro Arg Ala Gln Pro Met Lys Ala Ser Ser Ser Ser Ala Ser
85 90 95

Ala Ala Gly Ala Ala Ala Thr Pro Pro Arg Pro Gln Lys Leu Tyr Arg
100 105 110

Gly Val Arg Gln Arg His Trp Gly Lys Trp Val Ala Glu Ile Arg Leu
115 120 125

Pro Arg Asn Arg Thr Arg Leu Trp Leu Gly Thr Phe Asp Thr Ala Glu
130 135 140

Glu Ala Ala Leu Ala Tyr Asp Gln Ala Ala Tyr Arg Leu Arg Gly Asp
145 150 155 160

Ala Ala Arg Leu Asn Phe Pro Asp Asn Ala Ala Ser Arg Gly Pro Leu
165 170 175

```
His Ala Ser Val Asp Ala Lys Leu Gln Thr Leu Cys Gln Asn Ile Ala
            180                 185                 190

Ala Ala Lys Asn Ala Lys Lys Ser Ser Val Ser Ala Ser Ala Ala Ala
        195                 200                 205

Thr Ser Ser Ala Pro Thr Ser Asn Cys Ser Ser Pro Ser Ser Asp Asp
    210                 215                 220

Ala Ser Ser Cys Leu Glu Ser Ala Asp Ser Ser Pro Ser Leu Ser Pro
225                 230                 235                 240

Ser Ser Ala Ala Thr Thr Ala Glu Thr Pro Ala Thr Val Pro Glu Met
                245                 250                 255

Gln Gln Leu Asp Phe Ser Glu Ala Pro Trp Asp Glu Ala Ala Ala Phe
            260                 265                 270

Ala Leu Thr Lys Tyr Pro Ser Tyr Glu Ile Asp Trp Asp Ser Leu Leu
        275                 280                 285

Ala Ala Asn
    290


<210>  297
<211>  1657
<212>  DNA
<213>  Oryza sativa

<400>  297
aacccaaacg acggagaccc actactccga gccatcaaga acacacacac atccagaaag      60

cctaatccaa tccaaaagcc ctaatcaccc agactccatc tctgtgaggt ttgagagagg     120

taggtgagcc gtagatctga gcgagttctt gggtcttcca gcaggtagat cacttcatct     180

gaggatcaat cttttttttt ttcctttgtt tttgagcttc tgttggtgta caggacagag     240

agttccagag ccttttagtt tctggtgttc tgatctgttc ttggtgtaag attattggtc     300

tgatttggta gccaagaggg ttaatttttt ccacacctcc ttgtgctagt tagcttagct     360

tatacccccc ttgtaaagtg attagtagat ctagaacttc tcttttcgtc tgccagttct     420

tggattttgg aaagaacagg tggtttgtta ttcagatttt taggttagaa aaaatccaca     480

aaaaaaaaga tattcgatgg cagctgctat agaaggaaat ctgatgcggg cgctgggaga     540

ggctccgtcg ccgcagatgc agaagatcgc gccgccgccg tttcatcccg gcttgccgcc     600

ggcgccggcg aacttctcct cggccggagt ccacgggttc cactacatgg gcccggccca     660

gctcagcccg gcccagatcc agcgcgtcca ggcccaactc cacatgcagc ggcaggccca     720

gtcggggctc ggcccgcggg cccagcccat gaagcccgct tcggcggctg ctccggcggc     780
```

ggcggcggcg cgggcgcaga agctgtaccg cggcgtgcgg cagcggcact ggggcaagtg 840

ggtggcggag atccggctgc cgcgcaaccg caccaggctc tggctcggca ccttcgacac 900

cgccgaggag gcggcgctca cctacgacca ggccgcgtac cgcctccgcg gcgacgcggc 960

gcggctcaac ttcccggaca acgccgcgtc gcggggcccg ctcgacgccg ccgtggacgc 1020

caagctccag gccatctgcg acaccatcgc cgcgtccaag aacgcctcat ccaggtccag 1080

gggcggcgcc ggcagggcca tgcccatcaa cgcgcccctg gtcgccgcgg cgtcgtcgtc 1140

ctccggctcc gaccactccg gcggcggcga cgacggcggc tcggagacgt cgtcgtcgtc 1200

tgcggcggcg tcgccgctgg cggagatgga gcagctggac ttcagcgagg tgccgtggga 1260

cgaggcggag ggttcgcgc tcaccaagta cccgtcgtac gagatcgact gggactcgct 1320

gctcaacaac aataactagc tcttctcttc catagcccgc cattgccggc cggcgcagat 1380

gaggttttag gcattctgcg cggcggcgag gcgatggatt atatgttcgt tcttgtgtag 1440

atccttttct ttttgtgttg gttttttagg ttccggaggc tgctcgccgg cgtcgttttt 1500

gtgtccggcg tctccgatgt ctagtaagca gtgactcgtt agtgtagtag ttgtacaact 1560

ctacaaatgt acaaatactt gtgcatgtag gttgttgtaa tcaattgttg gtaactacct 1620

gtaatagaac tactgtgaat taactgtatt aatgtgc 1657

<210> 298
<211> 280
<212> PRT
<213> Oryza sativa

<400> 298

Met Ala Ala Ala Ile Glu Gly Asn Leu Met Arg Ala Leu Gly Glu Ala
1 5 10 15

Pro Ser Pro Gln Met Gln Lys Ile Ala Pro Pro Pro Phe His Pro Gly
20 25 30

Leu Pro Pro Ala Pro Ala Asn Phe Ser Ser Ala Gly Val His Gly Phe
35 40 45

His Tyr Met Gly Pro Ala Gln Leu Ser Pro Ala Gln Ile Gln Arg Val
50 55 60

Gln Ala Gln Leu His Met Gln Arg Gln Ala Gln Ser Gly Leu Gly Pro
65 70 75 80

Arg Ala Gln Pro Met Lys Pro Ala Ser Ala Ala Ala Pro Ala Ala Ala
85 90 95

Ala Ala Arg Ala Gln Lys Leu Tyr Arg Gly Val Arg Gln Arg His Trp
100 105 110

Gly Lys Trp Val Ala Glu Ile Arg Leu Pro Arg Asn Arg Thr Arg Leu
        115                 120                 125

Trp Leu Gly Thr Phe Asp Thr Ala Glu Glu Ala Ala Leu Thr Tyr Asp
    130                 135                 140

Gln Ala Ala Tyr Arg Leu Arg Gly Asp Ala Ala Arg Leu Asn Phe Pro
145                 150                 155                 160

Asp Asn Ala Ala Ser Arg Gly Pro Leu Asp Ala Ala Val Asp Ala Lys
                165                 170                 175

Leu Gln Ala Ile Cys Asp Thr Ile Ala Ala Ser Lys Asn Ala Ser Ser
            180                 185                 190

Arg Ser Arg Gly Gly Ala Gly Arg Ala Met Pro Ile Asn Ala Pro Leu
        195                 200                 205

Val Ala Ala Ala Ser Ser Ser Ser Gly Ser Asp His Ser Gly Gly Gly
    210                 215                 220

Asp Asp Gly Gly Ser Glu Thr Ser Ser Ser Ala Ala Ala Ser Pro
225                 230                 235                 240

Leu Ala Glu Met Glu Gln Leu Asp Phe Ser Glu Val Pro Trp Asp Glu
                245                 250                 255

Ala Glu Gly Phe Ala Leu Thr Lys Tyr Pro Ser Tyr Glu Ile Asp Trp
            260                 265                 270

Asp Ser Leu Leu Asn Asn Asn Asn
        275                 280

<210> 299
<211> 835
<212> DNA
<213> Lycopersicon esculentum

<400> 299
gtttgttcca cttccacaga gatgaattcc caaatctttt caactgggtt ttctgggtat 60

ggaatggagc aacagggttc aattgggctg aatcagttaa ccccaattca gatccagcaa 120

attcaagctc aaatcaactt tcaaaaccaa caacaacagc agcagcagca gatgatgtta 180

cagactgccc atcatgcttc caccatgaat ttcttggctc caaagccggt tccaatgaag 240

caatctgggt cgccaccaaa acccacgaag ctctacagag gtgttagaca acgccactgg 300

ggtaagtggg tcgctgagat ccgtttgcct aagaaccgaa cccgcctttg gcttggtaca 360

tttgacaccg ctgaagaagc tgctctggct tacgacaagg cggcgtatat gcttcgtggc 420

gactttgctc gactgaactt ccctcaactc cgccacaacg gcaacctaat cggcggcgac 480

tttggtgaat acaatccatt gcattcctca gttgatgcta agctaaagga catatgccaa 540

agcttggcac aggggaagag cattgactct aagaagaaga aaaccaaagg gttgtcggcg 600

gagaaagcgg cggtggtgaa gatggaggaa gaggagagca aaacagcaga agttggatcc 660

gaaagtgacg ggtcccattc cggttccggt ggatcatcgc cggtgaccga actgatattc 720

ccggagttca ctgaggaaga gccaacttgg gacatgtcag aaaatttttt gttgcagaag 780

tatccatctc atgaaattga ttgggcctct ctataaaatt agaaaataat taaga 835

<210> 300
<211> 264
<212> PRT
<213> Lycopersicon esculentum

<400> 300

Met Asn Ser Gln Ile Phe Ser Thr Gly Phe Ser Gly Tyr Gly Met Glu
1 5 10 15

Gln Gln Gly Ser Ile Gly Leu Asn Gln Leu Thr Pro Ile Gln Ile Gln
20 25 30

Gln Ile Gln Ala Gln Ile Asn Phe Gln Asn Gln Gln Gln Gln Gln Gln
35 40 45

Gln Gln Met Met Leu Gln Thr Ala His His Ala Ser Thr Met Asn Phe
50 55 60

Leu Ala Pro Lys Pro Val Pro Met Lys Gln Ser Gly Ser Pro Pro Lys
65 70 75 80

Pro Thr Lys Leu Tyr Arg Gly Val Arg Gln Arg His Trp Gly Lys Trp
85 90 95

Val Ala Glu Ile Arg Leu Pro Lys Asn Arg Thr Arg Leu Trp Leu Gly
100 105 110

Thr Phe Asp Thr Ala Glu Glu Ala Ala Leu Ala Tyr Asp Lys Ala Ala
115 120 125

Tyr Met Leu Arg Gly Asp Phe Ala Arg Leu Asn Phe Pro Gln Leu Arg
130 135 140

His Asn Gly Asn Leu Ile Gly Gly Asp Phe Gly Glu Tyr Asn Pro Leu
145 150 155 160

His Ser Ser Val Asp Ala Lys Leu Lys Asp Ile Cys Gln Ser Leu Ala
165 170 175

```
Gln Gly Lys Ser Ile Asp Ser Lys Lys Lys Lys Thr Lys Gly Leu Ser
            180                 185                 190

Ala Glu Lys Ala Ala Val Val Lys Met Glu Glu Glu Glu Ser Lys Thr
        195                 200                 205

Ala Glu Val Gly Ser Glu Ser Asp Gly Ser His Ser Gly Ser Gly Gly
    210                 215                 220

Ser Ser Pro Val Thr Glu Leu Ile Phe Pro Glu Phe Thr Glu Glu Glu
225                 230                 235                 240

Pro Thr Trp Asp Met Ser Glu Asn Phe Leu Leu Gln Lys Tyr Pro Ser
                245                 250                 255

His Glu Ile Asp Trp Ala Ser Leu
            260
```

```
<210>  301
<211>  1042
<212>  DNA
<213>  Zea mays

<400>  301
cacgagcaat ccccttcaac aaacgcaccg cactccacgg cagccagaaa acacatccca      60

cggggcccag acccggcgac ccacctgagc ccggcgcaga tgcagttcat ccaggcccag     120

ctccacctgc agcggaaccc ggggctgggc ccgcgggcgc agcccatgaa gcccgccgtc     180

ccagtgccgc cggcgccggc gccgcagcgg cctgtgaagc tgtaccgcgg cgtgcggcag     240

cgtcactggg gcaagtgggt ggccgagatc cggctccccc ggaaccgcac ccgcctgtgg     300

ctcgggacct tcgacaccgc cgagcaggca gcgctggcct acgaccaggc ggcgtaccgc     360

ctccgcgggg acgcggcgcg gctcaacttc cccgacaacg cggagtccag ggcgccgctc     420

gaccccgccg tggacgccaa gctgcaggcc atctgcgcca ccatcgccgc cgcgtcgtcg     480

tcatccaaga attccaaggc caagagcaag gcgatgccaa tcaacgcgtc cgttctggaa     540

gcggcagcgg cgtctccgag caacagctcc tccgacgaag gttccggctc cgggttcggg     600

tcggacgacg agatgtcctc gtcttccccg acgccggtgg tggcgccgcc ggtggcggac     660

atgggacagt tggatttcag cgaggttccg tgggacgagg acgagagctt cgtgctccgc     720

aagtacccgt cctacgagat cgactgggac gcgctgctct ccaactagtc gcccttcgcc     780

gacagatgtg ctgttgtagt tcagtagtgg cagtatctct ggccgccgca gatgaggttt     840

taggcaatct gcaggccgcc ggcccatgtg tattaagtag gttttgctca gttgttggcc     900

ccggacttcg ccggcgtttt tgtgaccggc gtccccgagt gcactgcatt ggtgtactgg     960

tctgtctgta aaaaaaaatg gatctgtgta cttctatagt gtgtattcaa ccattgttct    1020
```

taaaaaaaaa aaaaaaaaaa aa 1042

<210> 302
<211> 222
<212> PRT
<213> Zea mays

<400> 302

Met Gln Phe Ile Gln Ala Gln Leu His Leu Gln Arg Asn Pro Gly Leu
1 5 10 15

Gly Pro Arg Ala Gln Pro Met Lys Pro Ala Val Pro Val Pro Pro Ala
20 25 30

Pro Ala Pro Gln Arg Pro Val Lys Leu Tyr Arg Gly Val Arg Gln Arg
35 40 45

His Trp Gly Lys Trp Val Ala Glu Ile Arg Leu Pro Arg Asn Arg Thr
50 55 60

Arg Leu Trp Leu Gly Thr Phe Asp Thr Ala Glu Gln Ala Ala Leu Ala
65 70 75 80

Tyr Asp Gln Ala Ala Tyr Arg Leu Arg Gly Asp Ala Ala Arg Leu Asn
85 90 95

Phe Pro Asp Asn Ala Glu Ser Arg Ala Pro Leu Asp Pro Ala Val Asp
100 105 110

Ala Lys Leu Gln Ala Ile Cys Ala Thr Ile Ala Ala Ala Ser Ser Ser
115 120 125

Ser Lys Asn Ser Lys Ala Lys Ser Lys Ala Met Pro Ile Asn Ala Ser
130 135 140

Val Leu Glu Ala Ala Ala Ala Ser Pro Ser Asn Ser Ser Ser Asp Glu
145 150 155 160

Gly Ser Gly Ser Gly Phe Gly Ser Asp Asp Glu Met Ser Ser Ser Ser
165 170 175

Pro Thr Pro Val Val Ala Pro Pro Val Ala Asp Met Gly Gln Leu Asp
180 185 190

Phe Ser Glu Val Pro Trp Asp Glu Asp Glu Ser Phe Val Leu Arg Lys
195 200 205

Tyr Pro Ser Tyr Glu Ile Asp Trp Asp Ala Leu Leu Ser Asn
210 215 220

<210> 303
<211> 926
<212> DNA
<213> Oryza sativa

<400> 303
ggaaagaaca ggtggtttgt tattcagatt tttaggttag aaaaaatcca caaaaaaaaa 60

gatattcgat ggcagctgct atagaaggaa atctgatgcg ggcgctggga gaggctccgt 120

cgccgcagat gcagaagatc gcgccgccgc cgtttcatcc cggcttgccg ccggcgccgg 180

cgaacttctc ctcggccgga gtccacgggt tccactacat gggcccggcc cagctcagcc 240

cggcccagat ccagcgcgtc caggcccaac tccacatgca gcggcaggcc cagtcggggc 300

tcggcccgcg ggcccagccc atgaagcccg cttcggcggc tgctccggcg gcggcggcgg 360

cgcgggcgca gaagctgtac cgcggcgtgc ggcagcggca ctggggcaag tgggtggcgg 420

agatccggct gccgcgcaac caccccaggc tctggctcgg caccttcgac accgccgagg 480

aggcggcgct cacctacggc caggccgcgt accgcctccg cggcgacgcg gcgcggctca 540

acttcccgga caacgccgcg tcgcggggcc cgctcgacgc cgccgtggac gccaagctcc 600

aggccatctg cgacaccatc gccgcgtcca agaacgcctc atccaggtcc aggggcggcg 660

ccggcagggc catgcccatc aatgcgcccc tggtcgccgc ggcgtcgtcg tcctccggct 720

ccgaccactc cggcggcggc gacgacggcg gctcggagac gtcgtcgtcg tctgcggcgg 780

cgtcgccgct ggcggagatg gagcagctgg acttcagcga ggtgccgtgg gacgaggcgg 840

aggggttcgc gctcaccaag tacccgtcgt acgagatcga ctgggactcg ctgctcaaca 900

acaataacta gctcttctct ccatag 926

<210> 304
<211> 280
<212> PRT
<213> Oryza sativa

<400> 304

Met Ala Ala Ala Ile Glu Gly Asn Leu Met Arg Ala Leu Gly Glu Ala
1 5 10 15

Pro Ser Pro Gln Met Gln Lys Ile Ala Pro Pro Pro Phe His Pro Gly
20 25 30

Leu Pro Pro Ala Pro Ala Asn Phe Ser Ser Ala Gly Val His Gly Phe
35 40 45

His Tyr Met Gly Pro Ala Gln Leu Ser Pro Ala Gln Ile Gln Arg Val
50 55 60

Gln Ala Gln Leu His Met Gln Arg Gln Ala Gln Ser Gly Leu Gly Pro
65 70 75 80

Arg Ala Gln Pro Met Lys Pro Ala Ser Ala Ala Ala Pro Ala Ala Ala
85 90 95

Ala Ala Arg Ala Gln Lys Leu Tyr Arg Gly Val Arg Gln Arg His Trp
100 105 110

Gly Lys Trp Val Ala Glu Ile Arg Leu Pro Arg Asn His Pro Arg Leu
115 120 125

Trp Leu Gly Thr Phe Asp Thr Ala Glu Glu Ala Ala Leu Thr Tyr Gly
130 135 140

Gln Ala Ala Tyr Arg Leu Arg Gly Asp Ala Ala Arg Leu Asn Phe Pro
145 150 155 160

Asp Asn Ala Ala Ser Arg Gly Pro Leu Asp Ala Ala Val Asp Ala Lys
165 170 175

Leu Gln Ala Ile Cys Asp Thr Ile Ala Ala Ser Lys Asn Ala Ser Ser
180 185 190

Arg Ser Arg Gly Gly Ala Gly Arg Ala Met Pro Ile Asn Ala Pro Leu
195 200 205

Val Ala Ala Ala Ser Ser Ser Ser Gly Ser Asp His Ser Gly Gly Gly
210 215 220

Asp Asp Gly Gly Ser Glu Thr Ser Ser Ser Ser Ala Ala Ala Ser Pro
225 230 235 240

Leu Ala Glu Met Glu Gln Leu Asp Phe Ser Glu Val Pro Trp Asp Glu
245 250 255

Ala Glu Gly Phe Ala Leu Thr Lys Tyr Pro Ser Tyr Glu Ile Asp Trp
260 265 270

Asp Ser Leu Leu Asn Asn Asn Asn
275 280

<210> 305
<211> 1434
<212> DNA
<213> Atriplex hortensis

<400> 305
aatttactta cccccaagcc cagccccaac aaatgagcta ctcataccca cctccacttc 60

ctagtaatac ccttaacaac tttctatctc ccaagcctgt gaccatgaaa acaactggtg 120

ggccgcccaa gcccactaag ctttatcgtg gggttaggca acgtcactgg ggtaaatggg 180

ttgctgagat ccgtttaccc aagaacagga cccggctttg gttgggtacc tttgatacag 240

ctgaggaagc tgctttggct tacgacaagg cggcttacaa gctgagaggt gactttgcga 300

ggttgaattt tcctaatctc cgccatgaag ggtcccacat cggtggcgaa ttcggcgaat 360

acaaacccct tcattcctcc gtaaacgcaa aactcgaagc catttgcgag agtctagcca 420

aacaggggaa tgaaaaacag gggaaatcag gaaagtccaa gaagaaagat gttgctaata 480

ataacaataa tacttcatct tcgtcatctt caagctgttg tacaacagct actgctgcgg 540

ccgatgcacc gcagcagcag cagcggatgc cggaaaacgg cggcgatgta aaaaccgaga 600

gcacctccga tagtgaggtg gggtccggtg gatcgtcgcc gttgtcggat ttgacattcg 660

gagataatga agaaatggga tcggagaatt tcttgttgga gtcatgccca tctcatgaga 720

ttgattggga tgctatatta tcatctgaat cttaataatt tcatttgtgg tcttaagtat 780

gggttaataa ggagtattaa ttaaattaaa ttagggagtt ataaaatgtg tcatcataat 840

ataagtcatg taatgttgtg taaatttttt cttttttttt ttttaatttt aattaaaatt 900

agtaggttgg tagtgggtag tttcagggag gagtgatctc tgcaatgaag tttttggaaa 960

ttgtagggac tccatatttg gtcttctttg gtgaggcaag tgttttttttg atcttgcctt 1020

tgatcaattg aagagtagtt tttttttctt attgtttatt ttatgtttat agtagaaaaa 1080

attttaggat tgtaatttga tgatcatttt tagtgaagta ttattattaa tatttttatt 1140

agtataaaaa aaaattggac tgttatcaag gtaagtgctt attttgactt ccatatttgt 1200

atgttgctct ctttctttga ttctttcata tttctagtga gatctgaact ataattaata 1260

tggattaaac cttaaattac tagtggtttg tgtaacagga tatgcttgat gttcctgttt 1320

tatacataat cggagctttt gtccctgcaa caatgattgc agtgctctat tattttgatc 1380

acagtgtggc atctcaactt gctcagcagc gacagcaacg gaattcggcc ctcg 1434

<210> 306
<211> 240
<212> PRT
<213> Atriplex hortensis

<400> 306

Met Ser Tyr Ser Tyr Pro Pro Pro Leu Pro Ser Asn Thr Leu Asn Asn
1 5 10 15

Phe Leu Ser Pro Lys Pro Val Thr Met Lys Thr Thr Gly Gly Pro Pro
20 25 30

Lys Pro Thr Lys Leu Tyr Arg Gly Val Arg Gln Arg His Trp Gly Lys
35 40 45

Trp Val Ala Glu Ile Arg Leu Pro Lys Asn Arg Thr Arg Leu Trp Leu
50 55 60

Gly Thr Phe Asp Thr Ala Glu Glu Ala Ala Leu Ala Tyr Asp Lys Ala
65 70 75 80

Ala Tyr Lys Leu Arg Gly Asp Phe Ala Arg Leu Asn Phe Pro Asn Leu
85 90 95

Arg His Glu Gly Ser His Ile Gly Gly Glu Phe Gly Glu Tyr Lys Pro
100 105 110

Leu His Ser Ser Val Asn Ala Lys Leu Glu Ala Ile Cys Glu Ser Leu
115 120 125

Ala Lys Gln Gly Asn Glu Lys Gln Gly Lys Ser Gly Lys Ser Lys Lys
130 135 140

Lys Asp Val Ala Asn Asn Asn Asn Asn Thr Ser Ser Ser Ser Ser Ser
145 150 155 160

Ser Cys Cys Thr Thr Ala Thr Ala Ala Ala Asp Ala Pro Gln Gln Gln
165 170 175

Gln Arg Met Pro Glu Asn Gly Gly Asp Val Lys Thr Glu Ser Thr Ser
180 185 190

Asp Ser Glu Val Gly Ser Gly Gly Ser Ser Pro Leu Ser Asp Leu Thr
195 200 205

Phe Gly Asp Asn Glu Glu Met Gly Ser Glu Asn Phe Leu Leu Glu Ser
210 215 220

Cys Pro Ser His Glu Ile Asp Trp Asp Ala Ile Leu Ser Ser Glu Ser
225 230 235 240

<210> 307
<211> 1507
<212> DNA
<213> Arabidopsis thaliana

<400> 307
aacaagagcc aagctaatga gataagtgta tcggtgaggc tgagagttat tcacttacaa 60

aaaaaaaaaa aaacttgagt gtaaccaaaa aaaaaagttg atatactttc tggttttctc 120

cttaactttt attctttaca aatccatccc ccttagatct gtttatttcc cgctactttg 180

attcatttct gttagtaatc tgtctttcgt atagaagaaa actgatttct tggtttgtat 240

tttcttaaag agatcaatct ttttttattt ttgatcttct tgtgtttttt tttctttgta 300

gaattaatcg tttgtgaggg tattttttta attccctcct ctcagaaatc tacacagagg 360

ttttttattt tataaacctc tttttcgatt ttcttgaaaa caaaaaatcc tgttctttac 420

```
ttttttaca agaacaaggg aaaaaaattt ctttttatta gaaatgacaa cttctatgga    480
tttttacagt aacaaaacgt ttcaacaatc tgatccattc ggtggtgaat taatggaagc   540
gcttttacct tttatcaaaa gcccttccaa cgattcatcc gcgtttgcgt tctctctacc   600
cgctccaatt tcatacgggt cggatctcca ctcattttct caccatctta gtcctaaacc   660
ggtctcaatg aaacaaaccg gtacttccgc ggctaaaccg acgaagctat acagaggagt   720
gagacaacgt cactggggaa aatgggtggc tgagattcgt ttaccgagga atcgaactcg   780
actttggctc ggaacattcg acacggcgga ggaagctgct ttagcttatg acaaggcggc   840
gtataagctc cgaggagatt ttgcgcggct taatttccct gatctccgtc ataacgacga   900
gtatcaacct cttcaatcat cagtcgacgc taagcttgaa gctatttgtc aaaacttagc   960
tgagacgacg cagaaacagg tgagatcaac gaagaagtct tcttctcgga aacgttcatc  1020
aaccgtcgca gtgaaactac cggaggagga ctactctagc gccggatctt cgccgctgtt  1080
aacggagagt tatggatctg gtggatcttc ttcgccgttg tcggagctga cgtttggtga  1140
tacggaggag gagattcagc cgccgtggaa cgagaacgcg ttggagaagt atccgtcgta  1200
cgagatcgat tgggattcga ttcttcagtg ttcgagtctt gtaaattaga tgttgccata  1260
ggggtatttt agggacttta gagctctctg cgatggagtt tttggtcatt gcagagattt  1320
tattattatt aaggggggttt gttatgttaa tatcaaataa gtttatctac tttgatgtta  1380
attagtgtta atctctgcgt cggtccaagc tgttttttttt tggcatgctt cgaccgtgtg  1440
agatttctta tgtaattttt gtagttcctt gattttctta gttcaagtta aattggcaca  1500
aaagaac                                                             1507
```

<210> 308
<211> 261
<212> PRT
<213> Arabidopsis thaliana

<400> 308

```
Met Thr Thr Ser Met Asp Phe Tyr Ser Asn Lys Thr Phe Gln Gln Ser
1               5                   10                  15

Asp Pro Phe Gly Gly Glu Leu Met Glu Ala Leu Leu Pro Phe Ile Lys
            20                  25                  30

Ser Pro Ser Asn Asp Ser Ser Ala Phe Ala Phe Ser Leu Pro Ala Pro
        35                  40                  45

Ile Ser Tyr Gly Ser Asp Leu His Ser Phe Ser His His Leu Ser Pro
    50                  55                  60

Lys Pro Val Ser Met Lys Gln Thr Gly Thr Ser Ala Ala Lys Pro Thr
65                  70                  75                  80
```

Lys Leu Tyr Arg Gly Val Arg Gln Arg His Trp Gly Lys Trp Val Ala
85 90 95

Glu Ile Arg Leu Pro Arg Asn Arg Thr Arg Leu Trp Leu Gly Thr Phe
100 105 110

Asp Thr Ala Glu Glu Ala Ala Leu Ala Tyr Asp Lys Ala Ala Tyr Lys
115 120 125

Leu Arg Gly Asp Phe Ala Arg Leu Asn Phe Pro Asp Leu Arg His Asn
130 135 140

Asp Glu Tyr Gln Pro Leu Gln Ser Ser Val Asp Ala Lys Leu Glu Ala
145 150 155 160

Ile Cys Gln Asn Leu Ala Glu Thr Thr Gln Lys Gln Val Arg Ser Thr
165 170 175

Lys Lys Ser Ser Ser Arg Lys Arg Ser Ser Thr Val Ala Val Lys Leu
180 185 190

Pro Glu Glu Asp Tyr Ser Ser Ala Gly Ser Ser Pro Leu Leu Thr Glu
195 200 205

Ser Tyr Gly Ser Gly Gly Ser Ser Ser Pro Leu Ser Glu Leu Thr Phe
210 215 220

Gly Asp Thr Glu Glu Glu Ile Gln Pro Pro Trp Asn Glu Asn Ala Leu
225 230 235 240

Glu Lys Tyr Pro Ser Tyr Glu Ile Asp Trp Asp Ser Ile Leu Gln Cys
245 250 255

Ser Ser Leu Val Asn
260

<210> 309
<211> 1296
<212> DNA
<213> Arabidopsis thaliana

<400> 309
ataacgagag attttttata tataaaaaat aaaggaagca cgagttcacc ttaaaaattt 60

gagtttctct tttcttgacc ctgaagtttc gttttgatca atttcaccca tttctttgtt 120

cgtcgtcttt tctttcaaaa gggtttcatc tttgtattat aaaaatccaa actttatatc 180

ttcacaagca gcaaagatct ctatttcaat cctctcatgg aaactgcttc tctttctttc 240

cctgtcccaa acacgagctt cggtgtaaac aaatctatgc ctctcggtct aaaccagctc 300

acaccatacc aaattcatca gattcagaac cagcttaacc atagacgtag cacaatctca 360

aacctctctc caaaccgtat cagaatgaag aacttaacac cttctacctc caaaaccaag 420

aatctctaca gaggcgtaag gcaaaggcac tgggggaaat gggtcgctga gatccgtttg 480

cccaagaacc ggacccgtct ctggctcggg acattcgaaa ccgccgaaaa agccgcctta 540

gcttacgacc aagctgcttt tcagctccgt ggagatatcg cgaagcttaa cttcccaaac 600

ctcatacacg aagacatgaa tcctctccct tcctctgttg ataccaagct tcaagctatc 660

tgcaaaagtt tgagaaaaac agaggaaatt tgctctgttt ctgatcaaac aaaggagtac 720

tctgtttact ctgtttcaga taaaacagag cttttcctgc caaaagcaga gcttttcttg 780

cctaaaagag agcatttgga gacaaatgag ctctctaatg agtcaccgag aagcgatgag 840

acctcgttgt tggatgagtc gcaggcggaa tattcatcgt cggataaaac attcctggat 900

ttctcggata ctgagtttga agagattgga agtttcgggc ttcggaagtt tccttcggtg 960

gagattgatt gggatgcaat tagtaaactg gccaattctt aaagtcttct attttatatt 1020

aatcttctgt gcaactgaaa ttttcaacaa gttgcagatg agtttattag gtttgaatct 1080

gtcctagttt tctttgttgg tcgtatagag ttttactatg tagctaatct ccatgtttgg 1140

tttggcttgg taagttttta aggtcctcgt catcaacaaa ccggagaaag tttatgtatt 1200

caaaagctat gtattatatc ctcacatcta cttatgagag tgagatcaaa gtttgtaaaa 1260

tgaagtcttg atttcttctt ttagttctct cttttt 1296

<210> 310
<211> 261
<212> PRT
<213> Arabidopsis thaliana

<400> 310

Met Glu Thr Ala Ser Leu Ser Phe Pro Val Pro Asn Thr Ser Phe Gly
1 5 10 15

Val Asn Lys Ser Met Pro Leu Gly Leu Asn Gln Leu Thr Pro Tyr Gln
20 25 30

Ile His Gln Ile Gln Asn Gln Leu Asn His Arg Arg Ser Thr Ile Ser
35 40 45

Asn Leu Ser Pro Asn Arg Ile Arg Met Lys Asn Leu Thr Pro Ser Thr
50 55 60

Ser Lys Thr Lys Asn Leu Tyr Arg Gly Val Arg Gln Arg His Trp Gly
65 70 75 80

Lys Trp Val Ala Glu Ile Arg Leu Pro Lys Asn Arg Thr Arg Leu Trp
85 90 95

Leu Gly Thr Phe Glu Thr Ala Glu Lys Ala Ala Leu Ala Tyr Asp Gln
100 105 110

Ala Ala Phe Gln Leu Arg Gly Asp Ile Ala Lys Leu Asn Phe Pro Asn
115 120 125

Leu Ile His Glu Asp Met Asn Pro Leu Pro Ser Ser Val Asp Thr Lys
130 135 140

Leu Gln Ala Ile Cys Lys Ser Leu Arg Lys Thr Glu Glu Ile Cys Ser
145 150 155 160

Val Ser Asp Gln Thr Lys Glu Tyr Ser Val Tyr Ser Val Ser Asp Lys
165 170 175

Thr Glu Leu Phe Leu Pro Lys Ala Glu Leu Phe Leu Pro Lys Arg Glu
180 185 190

His Leu Glu Thr Asn Glu Leu Ser Asn Glu Ser Pro Arg Ser Asp Glu
195 200 205

Thr Ser Leu Leu Asp Glu Ser Gln Ala Glu Tyr Ser Ser Ser Asp Lys
210 215 220

Thr Phe Leu Asp Phe Ser Asp Thr Glu Phe Glu Glu Ile Gly Ser Phe
225 230 235 240

Gly Leu Arg Lys Phe Pro Ser Val Glu Ile Asp Trp Asp Ala Ile Ser
245 250 255

Lys Leu Ala Asn Ser
260

<210> 311
<211> 1296
<212> DNA
<213> Glycine max

<400> 311
ataacgagag attttttata tataaaaaat aaaggaagca cgagttcacc ttaaaaattt 60

gagtttctct tttcttgacc ctgaagtttc gttttgatca atttcaccca tttctttgtt 120

cgtcgtcttt tctttcaaaa gggtttcatc tttgtattat aaaaatccaa actttatatc 180

ttcacaagca gcaaagatct ctatttcaat cctctcatgg aaactgcttc tctttctttc 240

cctgtcccaa acacgagctt cggtgtaaac aaatctatgc ctctcggtct aaaccagctc 300

acaccatacc aaattcatca gattcagaac cagcttaacc atagacgtag cacaatctca 360

aacctctctc caaaccgtat cagaatgaag aacttaacac cttctacctc caaaaccaag 420

aatctctaca gaggcgtaag gcaaaggcac tgggggaaat gggtcgctga gatccgtttg 480

cccaagaacc ggacccgtct ctggctcggg acattcgaaa ccgccgaaaa agccgcctta 540

gcttacgacc aagctgcttt tcagctccgt ggagatatcg cgaagcttaa cttgccaaac 600

ctcatacacg aagacatgaa tcctctccct tcctctgttg ataccaagct tcaagctatc 660

tgcaaaagtt tgagaaaaac agaggaaatt tgctctgttt ctgatcaaac aaaggagtac 720

tctgtttact ctgtttcaga taaaacagag cttttcctgc caaaagcaga gcttttcttg 780

cctaaaagag agcatttgga gacaaatgag ctctctaatg agtcaccgag aagcgatgag 840

acctcgttgt tggatgagtc gcaggcggaa tattcatcgt cggataaaac attcctggat 900

ttctcggata ctgagtttga agagattgga agtttcgggc ttcggaagtt tccttcggtg 960

gagattgatt gggatgcaat tagtaaactg gccaattctt aaagtcttct attttatatt 1020

aatcttctgt gcaactgaaa ttttcaacaa gttgcagatg agtttattag gtttgaatct 1080

gtcctagttt tcttgttggt cgtatagagt tttactatgt agctaatctc catgtttggt 1140

ttggcttggt aagtttttaa ggtcctcgtc atcaacaaac cggagaaagt ttatgtattc 1200

aaaagctatg tattatatcc tcacatctac ttatgagagt gagatcaaag tttgtaaaat 1260

gaagtcttga tttcttcttt tagttctctc tttttc 1296

<210> 312
<211> 272
<212> PRT
<213> Glycine max

<400> 312

Met Ala Ala Leu Met Asp Phe Tyr Ser Ser Ser Thr Glu Phe Gln Leu
1 5 10 15

His Ser Asp Pro Phe Arg Gly Glu Leu Met Glu Val Leu Glu Pro Phe
20 25 30

Met Lys Ser Pro Phe Ser Thr Pro Ser Pro Ser Asn Ser Cys Phe Leu
35 40 45

Ser Thr Ser Tyr Ser Pro Ser Pro Asn Asn Tyr Ser Pro Ser Leu Tyr
50 55 60

Ser Asn Gly Leu Ser Ser Ile Pro Asn Thr Thr Gln Asn Leu Ile Gly
65 70 75 80

Phe Gly Gln Gly Gln Pro Thr Ser Leu Val Gly Leu Asn His Leu Thr
85 90 95

Pro Ser Gln Ile Ser Gln Ile Gln Ala Gln Ile Gln Ile Gln Asn His
100 105 110

Ser Asn Thr Leu Ser Phe Leu Gly Pro Lys Pro Ile Pro Met Lys His
115 120 125

Val Gly Met Pro Pro Lys Pro Thr Lys Leu Tyr Arg Gly Val Arg Gln
130 135 140

Arg His Trp Gly Lys Trp Val Ala Glu Ile Arg Leu Pro Lys Asn Arg
145 150 155 160

Thr Arg Leu Trp Leu Gly Thr Phe Asp Thr Ala Glu Glu Ala Ala Leu
165 170 175

Ala Tyr Asp Lys Ala Ala Tyr Lys Leu Arg Gly Asp Phe Ala Arg Leu
180 185 190

Asn Phe Pro Asn Leu Arg His Gln Gly Ser Ser Val Gly Gly Asp Phe
195 200 205

Gly Glu Tyr Lys Pro Leu His Ser Ala Val Asp Ala Lys Leu Gln Ala
210 215 220

Ile Cys Glu Gly Leu Ala Glu Leu Gln Lys Gln Gly Lys Thr Glu Lys
225 230 235 240

Pro Pro Arg Lys Thr Arg Ser Lys Leu Ala Ser Pro Pro Glu Asn Asp
245 250 255

Asn Asn Asn Asp Asn Asn Ser Cys Lys Val Glu Ala Ala Pro Pro Leu
260 265 270

<210> 313
<211> 977
<212> DNA
<213> Arabidopsis thaliana

<400> 313
gtacattttt ttttgtattt caggaaactc cgatggcgga tctcttcggt ggtggccacg 60

gcggcgagct tatggaagca cttcaacctt tttacaaaag tgcttccacg tctgcttcaa 120

atcctgcgtt tgcgtcctca aacgatgcgt ttgcgtctgc cccaaacgac ctattttctt 180

cttcttctta ctataatcct catgcatctt tattcccttc acattccaca acctcttacc 240

cggatattta ttctggatcc atgacctatc catcttcatt cgggtcggat cttcaacaac 300

ccgaaaacta ccaatctcag ttccattacc aaaacactat cacttacact caccaagaca 360

acaacacttg catgcttaac ttcattgagc cgagccaacc gggttttatg acccaaccgg 420

gtccgagttc gggttcggtt tcaaaaccgg ctaagctcta tagaggagtg aggcaaagac 480

attggggaaa atgggtcgcg gagatccgtt tacccaggaa ccgaacccga ctttggctcg 540

gaacattcga cacggctgaa gaagccgcgt tggcttatga tcgcgccgcg tttaagcttc 600

gtggtgactc ggctcggctt aacttcccag ctctccgata ccaaaccggc tcgtctccgt 660

ctgataccgg cgaatatggt cctattcaag ctgccgtaga cgctaaacta gaagccatat 720

tagctgagcc gaagaatcag ccgggcaaaa cggagaggac gtcgaggaaa cgagctaaag 780

ccgcggcttc ttcagctgag cagccgtcag cgccacaaca acattccggg tcgggtgaaa 840

gtgatgggtc gggttcaccg acttcggatg ttatggtgca ggagatgtgc caagagccag 900

agatgccatg gaatgaaaat ttcatgctcg gcaagtgtcc ttcttatgag atagattggg 960

cttcaatttt atcgtga 977

<210> 314
<211> 314
<212> PRT
<213> Arabidopsis thaliana

<400> 314

Met Ala Asp Leu Phe Gly Gly Gly His Gly Gly Glu Leu Met Glu Ala
1 5 10 15

Leu Gln Pro Phe Tyr Lys Ser Ala Ser Thr Ser Ala Ser Asn Pro Ala
20 25 30

Phe Ala Ser Ser Asn Asp Ala Phe Ala Ser Ala Pro Asn Asp Leu Phe
35 40 45

Ser Ser Ser Ser Tyr Tyr Asn Pro His Ala Ser Leu Phe Pro Ser His
50 55 60

Ser Thr Thr Ser Tyr Pro Asp Ile Tyr Ser Gly Ser Met Thr Tyr Pro
65 70 75 80

Ser Ser Phe Gly Ser Asp Leu Gln Gln Pro Glu Asn Tyr Gln Ser Gln
85 90 95

Phe His Tyr Gln Asn Thr Ile Thr Tyr Thr His Gln Asp Asn Asn Thr
100 105 110

Cys Met Leu Asn Phe Ile Glu Pro Ser Gln Pro Gly Phe Met Thr Gln
115 120 125

Pro Gly Pro Ser Ser Gly Ser Val Ser Lys Pro Ala Lys Leu Tyr Arg
130 135 140

Gly Val Arg Gln Arg His Trp Gly Lys Trp Val Ala Glu Ile Arg Leu
145 150 155 160

Pro Arg Asn Arg Thr Arg Leu Trp Leu Gly Thr Phe Asp Thr Ala Glu
165 170 175

Glu Ala Ala Leu Ala Tyr Asp Arg Ala Ala Phe Lys Leu Arg Gly Asp
180 185 190

Ser Ala Arg Leu Asn Phe Pro Ala Leu Arg Tyr Gln Thr Gly Ser Ser
195 200 205

Pro Ser Asp Thr Gly Glu Tyr Gly Pro Ile Gln Ala Ala Val Asp Ala
210 215 220

Lys Leu Glu Ala Ile Leu Ala Glu Pro Lys Asn Gln Pro Gly Lys Thr
225 230 235 240

Glu Arg Thr Ser Arg Lys Arg Ala Lys Ala Ala Ala Ser Ser Ala Glu
245 250 255

Gln Pro Ser Ala Pro Gln Gln His Ser Gly Ser Gly Glu Ser Asp Gly
260 265 270

Ser Gly Ser Pro Thr Ser Asp Val Met Val Gln Glu Met Cys Gln Glu
275 280 285

Pro Glu Met Pro Trp Asn Glu Asn Phe Met Leu Gly Lys Cys Pro Ser
290 295 300

Tyr Glu Ile Asp Trp Ala Ser Ile Leu Ser
305 310

<210> 315
<211> 1291
<212> DNA
<213> Arabidopsis thaliana

<400> 315
aacaaatctc tctgtttctc ccgctcttgc tctgttttct caaagacaaa agaggacatc 60

gtcgttgact cctcttctct atggctactg ctaagaacaa gggaaaatca atcagggtcc 120

ttggtaccag tgaagcagag aaaaaggatg agatggagtt ggaggaggag ttccagttta 180

gtagcggcaa gtataaagat tcgggtcctg gctcggacat gtggttagga gatgcttcct 240

ctacgtctcc aagaagtctt aggaagacta gaacctttga ccgacataat ccctatctcg 300

tatcttctta tgctactcct cagccgccaa caacaactac atgctctgtc tcttttccct 360

tttacctccc tccagcgatt caaaatcaac aacgattttt acacccgaat gacccttcag 420

gacaaagaca gcaacaaatg atctcgtttg atcctcaaca acaggtgcaa ccatatgttg 480

cacaacagca gcaacaacaa caacatctat tgcagtactg gagagacatt ctgaagctga 540

gtccgagcgg aagaatgatg atgatgaaca tgttaagaca agaaagcgat ctgccactga 600

```
cgaggccacc ggttcaaccc ttcagcgcca ccaagctata tagaggtgtc aggcaacgcc     660

actggggaaa atgggttgcc gagatccgta agccacgaaa caggacacgt ctctggctag     720

ggacattcga tacagcagaa gaagccgcca tggcctacga ccgcgaggcc ttcaagttga     780

ggggagagac cgctaggctc aatttccctg aactttttct caataaacaa gagccaactc     840

ccgtgcatca gaaacaatgt gagacgggga ctactagtga agactcaagc agaagaggag     900

aggatgattc gagcacggca ttggcagtag gaggggtgag tgaggagacg ggttgggctg     960

aggcatggtt caatgcaatt ccagaggaat ggggacctgg aagccctcta tgggatgatt    1020

accactttcc catttctaac cataaggacg atcttgacgc cacacaaaac tcttcttctg    1080

atacaattta ggacctttgt tagaatatag atatgcttag ttgtatgact gatctagctt    1140

gtgttttttt tttgggtgga gacagttttt gtcatcttcc acattttaga ttctattttc    1200

gaccatcatt tttttcttga tcggtgacta tgaatctaat ggggtcaatc attttcacat    1260

ataaaactta agtatttggt gtttgtactt c                                   1291


<210>  316
<211>  336
<212>  PRT
<213>  Arabidopsis thaliana

<400>  316

Met Ala Thr Ala Lys Asn Lys Gly Lys Ser Ile Arg Val Leu Gly Thr
1               5                   10                  15

Ser Glu Ala Glu Lys Lys Asp Glu Met Glu Leu Glu Glu Glu Phe Gln
            20                  25                  30

Phe Ser Ser Gly Lys Tyr Lys Asp Ser Gly Pro Gly Ser Asp Met Trp
        35                  40                  45

Leu Gly Asp Ala Ser Ser Thr Ser Pro Arg Ser Leu Arg Lys Thr Arg
    50                  55                  60

Thr Phe Asp Arg His Asn Pro Tyr Leu Val Ser Ser Tyr Ala Thr Pro
65                  70                  75                  80

Gln Pro Pro Thr Thr Thr Thr Cys Ser Val Ser Phe Pro Phe Tyr Leu
                85                  90                  95

Pro Pro Ala Ile Gln Asn Gln Gln Arg Phe Leu His Pro Asn Asp Pro
            100                 105                 110

Ser Gly Gln Arg Gln Gln Gln Met Ile Ser Phe Asp Pro Gln Gln Gln
        115                 120                 125
```

Val Gln Pro Tyr Val Ala Gln Gln Gln Gln Gln Gln Gln His Leu Leu
130 135 140

Gln Tyr Trp Arg Asp Ile Leu Lys Leu Ser Pro Ser Gly Arg Met Met
145 150 155 160

Met Met Asn Met Leu Arg Gln Glu Ser Asp Leu Pro Leu Thr Arg Pro
165 170 175

Pro Val Gln Pro Phe Ser Ala Thr Lys Leu Tyr Arg Gly Val Arg Gln
180 185 190

Arg His Trp Gly Lys Trp Val Ala Glu Ile Arg Lys Pro Arg Asn Arg
195 200 205

Thr Arg Leu Trp Leu Gly Thr Phe Asp Thr Ala Glu Glu Ala Ala Met
210 215 220

Ala Tyr Asp Arg Glu Ala Phe Lys Leu Arg Gly Glu Thr Ala Arg Leu
225 230 235 240

Asn Phe Pro Glu Leu Phe Leu Asn Lys Gln Glu Pro Thr Pro Val His
245 250 255

Gln Lys Gln Cys Glu Thr Gly Thr Thr Ser Glu Asp Ser Ser Arg Arg
260 265 270

Gly Glu Asp Asp Ser Ser Thr Ala Leu Ala Val Gly Gly Val Ser Glu
275 280 285

Glu Thr Gly Trp Ala Glu Ala Trp Phe Asn Ala Ile Pro Glu Glu Trp
290 295 300

Gly Pro Gly Ser Pro Leu Trp Asp Asp Tyr His Phe Pro Ile Ser Asn
305 310 315 320

His Lys Asp Asp Leu Asp Ala Thr Gln Asn Ser Ser Ser Asp Thr Ile
325 330 335

<210> 317
<211> 1405
<212> DNA
<213> Arabidopsis thaliana

<400> 317
atgatcacac caatacacac acaacactcc ttaattctcg tatatataaa catttattct 60

ccacctattt tgtcaaaatt aagaacaggc ttcattctct ggacaaacac tcaaaaaaca 120

aacaaaaaaa ggaacatgga agatcagttt cctaaaatag aaactagctt catgcacgac 180

aagctcttgt cttctggaat ctacgggttc ttgagttctt cgacgccgcc acaacttctc 240

```
ggtgttccaa tatttttgga aggtatgaaa tctcctcttc ttcctgcttc ttcgactccg     300
agctactttg tgtcgcctca tgatcatgag ctcacatctt ctattcatcc atctccggta     360
gcttctgttc cttggaactt tctagaatct tttcctcagt ctcaacatcc tgatcatcat     420
ccttctaaac ctccaaacct tactttgttc cttaaagaac caaagctact agaactttct     480
caatccgaaa gcaacatgag cccttaccat aaatacatcc caaactcctt ttatcaatca     540
gaccaaaaca gaaacgaatg ggtagagatc aataaaactc taaccaacta tccctcgaaa     600
ggttttggaa actattggct aagtaccacc aagactcaac ccatgaagtc aaaaacaaga     660
aaggttgttc agacgacgac cccaacaaaa ctgtatagag gagtgagaca aagacactgg     720
ggcaaatggg tcgcagagat taggcttcca aggaacagaa cccgtgtttg gctcggcact     780
tttgaaaccg ctgagcaagc agcaatggct tacgatacag cagcttatat ccttcgtggc     840
gaattcgcac acctcaactt tcctgatctt aaacaccagc tcaagtccgg ttctttgcga     900
tgcatgatcg cctcactttt ggagtccaag attcaacaga tctcatcttc ccaagtaagt     960
aactctcctt ctcctcctcc tccaaaagtg ggaacaccgg agcaaaagaa tcatcacatg    1020
aagatggagt caggagaaga cgtgatgatg aagaaacaga aaagccataa ggaagtgatg    1080
gaaggagatg gtgtacaatt gagtaggatg ccttctttgg atatggatct catttgggat    1140
gctctctcat ttcctcattc ttcttgactt caaattaata tttgtcaaac ttattttact    1200
tacttctacc ctttttttata tcaaaagttt ccaccaaaga aagaaattca tattatgatg    1260
ccaagattgg tttgcatttg gggttgaaca cattgtaatt cttcttacga ccacataatc    1320
aagtggttct cctttttttg tctgctaatt aattgttctt ttctttgcgg ttactagtaa    1380
ccataaaata gaaaagtgtt tgttt                                          1405
```

```
<210>  318
<211>  388
<212>  PRT
<213>  Arabidopsis thaliana

<400>  318

Met Ile Thr Pro Ile His Thr Gln His Ser Leu Ile Leu Val Tyr Ile
1               5                   10                  15

Asn Ile Tyr Ser Pro Pro Ile Leu Ser Lys Leu Arg Thr Gly Phe Ile
            20                  25                  30

Leu Trp Thr Asn Thr Gln Lys Thr Asn Lys Lys Arg Asn Met Glu Asp
        35                  40                  45

Gln Phe Pro Lys Ile Glu Thr Ser Phe Met His Asp Lys Leu Leu Ser
    50                  55                  60
```

Ser Gly Ile Tyr Gly Phe Leu Ser Ser Ser Thr Pro Pro Gln Leu Leu
65 70 75 80

Gly Val Pro Ile Phe Leu Glu Gly Met Lys Ser Pro Leu Leu Pro Ala
85 90 95

Ser Ser Thr Pro Ser Tyr Phe Val Ser Pro His Asp His Glu Leu Thr
100 105 110

Ser Ser Ile His Pro Ser Pro Val Ala Ser Val Pro Trp Asn Phe Leu
115 120 125

Glu Ser Phe Pro Gln Ser Gln His Pro Asp His His Pro Ser Lys Pro
130 135 140

Pro Asn Leu Thr Leu Phe Leu Lys Glu Pro Lys Leu Leu Glu Leu Ser
145 150 155 160

Gln Ser Glu Ser Asn Met Ser Pro Tyr His Lys Tyr Ile Pro Asn Ser
165 170 175

Phe Tyr Gln Ser Asp Gln Asn Arg Asn Glu Trp Val Glu Ile Asn Lys
180 185 190

Thr Leu Thr Asn Tyr Pro Ser Lys Gly Phe Gly Asn Tyr Trp Leu Ser
195 200 205

Thr Thr Lys Thr Gln Pro Met Lys Ser Lys Thr Arg Lys Val Val Gln
210 215 220

Thr Thr Thr Pro Thr Lys Leu Tyr Arg Gly Val Arg Gln Arg His Trp
225 230 235 240

Gly Lys Trp Val Ala Glu Ile Arg Leu Pro Arg Asn Arg Thr Arg Val
245 250 255

Trp Leu Gly Thr Phe Glu Thr Ala Glu Gln Ala Ala Met Ala Tyr Asp
260 265 270

Thr Ala Ala Tyr Ile Leu Arg Gly Glu Phe Ala His Leu Asn Phe Pro
275 280 285

Asp Leu Lys His Gln Leu Lys Ser Gly Ser Leu Arg Cys Met Ile Ala
290 295 300

Ser Leu Leu Glu Ser Lys Ile Gln Gln Ile Ser Ser Ser Gln Val Ser
305 310 315 320

Asn Ser Pro Ser Pro Pro Pro Pro Lys Val Gly Thr Pro Glu Gln Lys

325 330 335

Asn His His Met Lys Met Glu Ser Gly Glu Asp Val Met Met Lys Lys
340 345 350

Gln Lys Ser His Lys Glu Val Met Glu Gly Asp Gly Val Gln Leu Ser
355 360 365

Arg Met Pro Ser Leu Asp Met Asp Leu Ile Trp Asp Ala Leu Ser Phe
370 375 380

Pro His Ser Ser
385

<210> 319
<211> 1422
<212> DNA
<213> Oryza sativa

<400> 319
atggacgcgg tggacagagg cggaggcgga ggcggtggag gagcgcgcgg gcacgggcgg 60
agatggaagg ggaagggagt gagcgcggcg atctcctcct ccgcggccga gacgcagcag 120
ccggtgccag ttttagaaga cgcgccggcg gccgcagcat tgctccgtcc ccagaagaag 180
atccgcagcc ccgatcgtcg cctccagcgc tccatctcct cgctgtcgtc ggcccctgct 240
tcccccgact cgtcctctgt ctccaacccc atgtctcccc cggcgatgtc cttgccgaat 300
cagccgccat cgtcgcgaca tatatttccg ttcgcgtacg atccgtctcc gggcgcggcg 360
gcaccaaggc tcctcccgct gctgcagtac tccagcttgt acccgcagcc tctgctgccg 420
cagcagcaat caccccttgca gaatcagcaa atgatatcgt tcggcagtag ccagcagcag 480
cagcagcagc agccgcagtt tggggcggcg tctcctctgt tcccgccgca gttcttgccg 540
ccggaggagc agcagcgcct gctgctgcgc tactggagcg aggcgctgaa cctgagcccc 600
ccaggcgtgc gcggcggcgc cctgccgccg tcgctgtacc agcacctgct gcgcgcgcct 660
gggccgccca aactgtaccg cggcgtgcgg cagcgccact gggggaagtg ggtggcggag 720
atccgcctgc cgcggaaccg caccaggctg tggctcggca cgttcgacac cgccgaggac 780
gcagccatgg cgtacgaccg cgaggccttc aagctccgcg gcgagaacgc gcggctcaac 840
ttccccgacc tcttccttgg caaaggccgc accggcggga gcggacgcac cagcgccagc 900
gccgcggcct cctgctcctc ctcctcctct tcggctccgc ctacaccgga cgagagccac 960
acgcagcaag ctcagccgca gccgcagcag cctacagaag agtcatccaa cactgaaccg 1020
aagcctctgc tcttcgtagc agagcaggac ggcattccag aacccgagct gaatcctcag 1080
ctccagacag ctgaacaaca tggcagcgac ggcaacacgg ccatgttcca gccgtcggtg 1140
acgtccggcg gcatttgggg tccggccgac gaggcgtggt tcagcgcttg gggccagga 1200

agctccgtct gggactacga catggacagc gcccacggcc tcctcctcca gtctcgcttg 1260

gccggtgagc agaccggcat ggactacgcc tacaccgcgc cggaagtcct cgtggcaccg 1320

gtgccggcgg cagggacagc catggccact gccgcttcct cctctcttcc tcctcgtcct 1380

ccccctcctt gccacagtcc aaccttcgca tggaaggact aa 1422

<210> 320
<211> 473
<212> PRT
<213> Oryza sativa

<400> 320

Met Asp Ala Val Asp Arg Gly Gly Gly Gly Gly Gly Gly Gly Ala Arg
1 5 10 15

Gly His Gly Arg Arg Trp Lys Gly Lys Gly Val Ser Ala Ala Ile Ser
20 25 30

Ser Ser Ala Ala Glu Thr Gln Gln Pro Val Pro Val Leu Glu Asp Ala
35 40 45

Pro Ala Ala Ala Ala Leu Leu Arg Pro Gln Lys Lys Ile Arg Ser Pro
50 55 60

Asp Arg Arg Leu Gln Arg Ser Ile Ser Ser Leu Ser Ser Ala Pro Ala
65 70 75 80

Ser Pro Asp Ser Ser Ser Val Ser Asn Pro Met Ser Pro Pro Ala Met
85 90 95

Ser Leu Pro Asn Gln Pro Pro Ser Ser Arg His Ile Phe Pro Phe Ala
100 105 110

Tyr Asp Pro Ser Pro Gly Ala Ala Ala Pro Arg Leu Leu Pro Leu Leu
115 120 125

Gln Tyr Ser Ser Leu Tyr Pro Gln Pro Leu Leu Pro Gln Gln Gln Ser
130 135 140

Pro Leu Gln Asn Gln Gln Met Ile Ser Phe Gly Ser Ser Gln Gln Gln
145 150 155 160

Gln Gln Gln Gln Pro Gln Phe Gly Ala Ala Ser Pro Leu Phe Pro Pro
165 170 175

Gln Phe Leu Pro Pro Glu Glu Gln Gln Arg Leu Leu Leu Arg Tyr Trp
180 185 190

Ser Glu Ala Leu Asn Leu Ser Pro Pro Gly Val Arg Gly Gly Ala Leu

```
        195                 200                 205

Pro Pro Ser Leu Tyr Gln His Leu Leu Arg Ala Pro Gly Pro Pro Lys
    210                 215                 220

Leu Tyr Arg Gly Val Arg Gln Arg His Trp Gly Lys Trp Val Ala Glu
225                 230                 235                 240

Ile Arg Leu Pro Arg Asn Arg Thr Arg Leu Trp Leu Gly Thr Phe Asp
                245                 250                 255

Thr Ala Glu Asp Ala Ala Met Ala Tyr Asp Arg Glu Ala Phe Lys Leu
            260                 265                 270

Arg Gly Glu Asn Ala Arg Leu Asn Phe Pro Asp Leu Phe Leu Gly Lys
        275                 280                 285

Gly Arg Thr Gly Gly Ser Gly Arg Thr Ser Ala Ser Ala Ala Ala Ser
    290                 295                 300

Cys Ser Ser Ser Ser Ser Ser Ala Pro Pro Thr Pro Asp Glu Ser His
305                 310                 315                 320

Thr Gln Gln Ala Gln Pro Gln Pro Gln Gln Pro Thr Glu Glu Ser Ser
                325                 330                 335

Asn Thr Glu Pro Lys Pro Leu Leu Phe Val Ala Glu Gln Asp Gly Ile
            340                 345                 350

Pro Glu Pro Glu Leu Asn Pro Gln Leu Gln Thr Ala Glu Gln His Gly
        355                 360                 365

Ser Asp Gly Asn Thr Ala Met Phe Gln Pro Ser Val Thr Ser Gly Gly
    370                 375                 380

Ile Trp Gly Pro Ala Asp Glu Ala Trp Phe Ser Ala Trp Gly Pro Gly
385                 390                 395                 400

Ser Ser Val Trp Asp Tyr Asp Met Asp Ser Ala His Gly Leu Leu Leu
                405                 410                 415

Gln Ser Arg Leu Ala Gly Glu Gln Thr Gly Met Asp Tyr Ala Tyr Thr
            420                 425                 430

Ala Pro Glu Val Leu Val Ala Pro Val Pro Ala Ala Gly Thr Ala Met
        435                 440                 445

Ala Thr Ala Ala Ser Ser Ser Leu Pro Pro Arg Pro Pro Pro Pro Cys
    450                 455                 460
```

His Ser Pro Thr Phe Ala Trp Lys Asp
465 470

<210> 321
<211> 1604
<212> DNA
<213> Oryza sativa

<400> 321
acgtttggct gcaccctgtc tcgcggctga tacgtttgca caggcgttca gatacagccg 60

atacaccggc ggcgctagct gcgtttcttc tccgcatgga cgctccgtcg tcgccctcgc 120

caccgtcggc gcgacacatc ttccccttcg cgtacgaggc ctccacgacg acggtgggtg 180

ggagcccaag gctccacccg ctgtcgtggc agcaatccag catgtcccag cccgcgtcgc 240

cacagcaaca gcagcagcag ccgttgcagc accagcagat gatatcgttc ggcgcgtcgc 300

ctccgtgctc gacgacgcag ttcgtcgtcc cggagaacgc gcagcagcag cagatgctgc 360

tgcggtactg gagcgaggcg ctgaacctga gcccgcgcgg tggccccggt ggcgtgccac 420

cgtggctgta ccagcagctg ctccgggtcc cgccaccgcc gcagaagctt taccgcggcg 480

tgcggcagag gcactggggg aagtgggtcg cggagatccg cctgccacgg aaccgcacgc 540

ggctgtggct tggcaccttc gacaccgccg aggacgccgc catggcgtac gaccgcgagg 600

ccttcaagct ccgcggcgag aacgcgcggc tcaactttcc ggaccggttc cttgggaagg 660

gccgcgccgg cgggaaaggc cgcaccagcg taagctcctc ggccgctgcc gccgcgtcgt 720

gctcgtcgtc gtcgctatcg ccaccggaga cacctgacga cgcaaacacg cagcaacaag 780

ctcctcagca gcgagaacag cgggacacgg caggagtgtc catggagaag aaacaaccac 840

agcctccagc tccaacctct cgtcaagaag ggtgctctgg cggcgacgca gctgcgccgt 900

acccggccga aatgcttcac gcgccggcgg cgtgcggcgg catgtgggtt gctcccgacg 960

agtcatggtt cagcacgtgg ggccctggca gctccttctg ggacgactac gacatggaca 1020

gcgctcgcgg cctcttcctc caccctcgct tcaccggtga cgaaactagc atggatcatt 1080

ccggcacgca agcaaccgtg ccggcagtgg cagcaacagc ggcagggatg agcatgccat 1140

gcgatgatgt tccggtaacc tcttcttctt cagatctccc tccccaaggg acacctcaga 1200

ctcctacctt catgtggaag gaggactaag catgcatgca cacagccacg cacctcgacg 1260

agacgactat ctcttccatc tcccatcgac ttcgacgatc tctttcatta ctcatcgact 1320

tcaaccacaa aggtatgatt agggtagaga tgcttgcaga ttgcagtttt aaagacaatt 1380

ttgcagcatc acacagctct acgtacaaca gccacaccta tatcaattct caagacttct 1440

aaaccacaag ggaacccctg ttctaggctg cgatttttag gtcagcttgt tggggtgacc 1500

acagtacaat tccctttatt tcgtatcttc ccaagattgt gtgtgtcaga tattcgttga 1560

actcgacttc aaaagatttg aagtattcat gttttctttt actc 1604

<210> 322
<211> 377
<212> PRT
<213> Oryza sativa

<400> 322

```
Met Asp Ala Pro Ser Ser Pro Ser Pro Pro Ser Ala Arg His Ile Phe
1               5                   10                  15

Pro Phe Ala Tyr Glu Ala Ser Thr Thr Thr Val Gly Gly Ser Pro Arg
            20                  25                  30

Leu His Pro Leu Ser Trp Gln Gln Ser Ser Met Ser Gln Pro Ala Ser
        35                  40                  45

Pro Gln Gln Gln Gln Gln Gln Pro Leu Gln His Gln Gln Met Ile Ser
    50                  55                  60

Phe Gly Ala Ser Pro Pro Cys Ser Thr Thr Gln Phe Val Val Pro Glu
65                  70                  75                  80

Asn Ala Gln Gln Gln Gln Met Leu Leu Arg Tyr Trp Ser Glu Ala Leu
                85                  90                  95

Asn Leu Ser Pro Arg Gly Gly Pro Gly Gly Val Pro Pro Trp Leu Tyr
            100                 105                 110

Gln Gln Leu Leu Arg Val Pro Pro Pro Pro Gln Lys Leu Tyr Arg Gly
        115                 120                 125

Val Arg Gln Arg His Trp Gly Lys Trp Val Ala Glu Ile Arg Leu Pro
    130                 135                 140

Arg Asn Arg Thr Arg Leu Trp Leu Gly Thr Phe Asp Thr Ala Glu Asp
145                 150                 155                 160

Ala Ala Met Ala Tyr Asp Arg Glu Ala Phe Lys Leu Arg Gly Glu Asn
                165                 170                 175

Ala Arg Leu Asn Phe Pro Asp Arg Phe Leu Gly Lys Gly Arg Ala Gly
            180                 185                 190

Gly Lys Gly Arg Thr Ser Val Ser Ser Ser Ala Ala Ala Ala Ala Ser
        195                 200                 205

Cys Ser Ser Ser Ser Leu Ser Pro Pro Glu Thr Pro Asp Asp Ala Asn
    210                 215                 220
```

Thr Gln Gln Gln Ala Pro Gln Gln Arg Glu Gln Arg Asp Thr Ala Gly
225 230 235 240

Val Ser Met Glu Lys Lys Gln Pro Gln Pro Pro Ala Pro Thr Ser Arg
245 250 255

Gln Glu Gly Cys Ser Gly Gly Asp Ala Ala Ala Pro Tyr Pro Ala Glu
260 265 270

Met Leu His Ala Pro Ala Ala Cys Gly Gly Met Trp Val Ala Pro Asp
275 280 285

Glu Ser Trp Phe Ser Thr Trp Gly Pro Gly Ser Ser Phe Trp Asp Asp
290 295 300

Tyr Asp Met Asp Ser Ala Arg Gly Leu Phe Leu His Pro Arg Phe Thr
305 310 315 320

Gly Asp Glu Thr Ser Met Asp His Ser Gly Thr Gln Ala Thr Val Pro
325 330 335

Ala Val Ala Ala Thr Ala Ala Gly Met Ser Met Pro Cys Asp Asp Val
340 345 350

Pro Val Thr Ser Ser Ser Ser Asp Leu Pro Pro Gln Gly Thr Pro Gln
355 360 365

Thr Pro Thr Phe Met Trp Lys Glu Asp
370 375

<210> 323
<211> 1401
<212> DNA
<213> Oryza sativa

<400> 323
atggacgctc cgagcggcga gagcggcggc ggcggcggcg gcggtggtgg caggaggtgg 60

aaggggaagg gggtgacgcc catacagccg cggcggcagc tggggacggt tttggaggac 120

tcgtcggcgg cattgctgcg gccgctcaag aagattgggc ggagccccga ccgcctcctc 180

cgctccgcat cgtcgctctc cacgtcctcg tcggctccgc cttcgcctcg ctcttcttcg 240

gcttccgatg ctccagtccg cgtcatctcg tcgtcgccgt cgtcgccctc gccaccgtcg 300

gcgcgacaca tcttcccctt cgcgtacgag gcctccacga cgacggtggg tgggagccca 360

aggctccacc cgctgtcgtg gcagcaatcc agcatgtccc agcccgcgtc gccacagcaa 420

cagcagcagc agccgttgca gcaccagcag atgatatcgt tcggcgcgtc gcctccgtgc 480

tcgacgacgc agttcgtcgt cccggagaac gcgcagcagc agcagatgct gctgcggtac 540

tggagcgagg cgctgaacct gagcccgcgc ggtggccccg gtggcgtgcc accgtggctg 600

taccagcagc tgctccgggt cccgccaccg ccgcagaagc tttaccgcgg cgtgcggcag 660

aggcactggg ggaagtgggt cgcggagatc cgcctgccac ggaaccgcac gcggctgtgg 720

cttggcacct tcgacaccgc cgaggacgcc gccatggcgt acgaccgcga ggccttcaag 780

ctccgcggcg agaacgcgcg gctcaacttt ccggaccggt tccttgggaa gggccgcgcc 840

ggcgggaaag gccgcaccag cgtaagctcc tcggccgctg ccgccgcgtc gtgctcgtcg 900

tcgtcgctat cgccaccgga gacacctgac gacgcaaaca cgcagcaaca agctcctcag 960

cagcgagaac agcgggacac ggcaggagtg tccatggaga agaaacaacc acagcctcca 1020

gctccaacct ctcgtcaaga agggtgctct ggcggcgacg cagctgcgcc gtacccggcc 1080

gaaatgcttc acgcgccggc ggcgtgcggc ggcatgtggg ttgctcccga cgagtcatgg 1140

ttcagcacgt ggggccctgg cagctccttc tgggacgact acgacatgga cagcgctcgc 1200

ggcctcttcc tccaccctcg cttcaccggt gacgaaacta gcatggatca ttccggcacg 1260

caagcaaccg tgccggcagt ggcagcaaca gcggcaggga tgagcatgcc atgcgatgat 1320

gttccggtaa cctcttcttc ttcagatctc cctccccaag ggacacctca gactcctacc 1380

ttcatgtgga aggaggacta a 1401

<210> 324
<211> 466
<212> PRT
<213> Oryza sativa

<400> 324

Met Asp Ala Pro Ser Gly Glu Ser Gly Gly Gly Gly Gly Gly Gly Gly
1 5 10 15

Gly Arg Arg Trp Lys Gly Lys Gly Val Thr Pro Ile Gln Pro Arg Arg
20 25 30

Gln Leu Gly Thr Val Leu Glu Asp Ser Ser Ala Ala Leu Leu Arg Pro
35 40 45

Leu Lys Lys Ile Gly Arg Ser Pro Asp Arg Leu Leu Arg Ser Ala Ser
50 55 60

Ser Leu Ser Thr Ser Ser Ser Ala Pro Pro Ser Pro Arg Ser Ser Ser
65 70 75 80

Ala Ser Asp Ala Pro Val Arg Val Ile Ser Ser Ser Pro Ser Ser Pro
85 90 95

Ser Pro Pro Ser Ala Arg His Ile Phe Pro Phe Ala Tyr Glu Ala Ser
100 105 110

```
Thr Thr Thr Val Gly Gly Ser Pro Arg Leu His Pro Leu Ser Trp Gln
        115                 120                 125

Gln Ser Ser Met Ser Gln Pro Ala Ser Pro Gln Gln Gln Gln Gln Gln
    130                 135                 140

Pro Leu Gln His Gln Gln Met Ile Ser Phe Gly Ala Ser Pro Pro Cys
145                 150                 155                 160

Ser Thr Thr Gln Phe Val Val Pro Glu Asn Ala Gln Gln Gln Gln Met
                165                 170                 175

Leu Leu Arg Tyr Trp Ser Glu Ala Leu Asn Leu Ser Pro Arg Gly Gly
            180                 185                 190

Pro Gly Gly Val Pro Pro Trp Leu Tyr Gln Gln Leu Leu Arg Val Pro
        195                 200                 205

Pro Pro Pro Gln Lys Leu Tyr Arg Gly Val Arg Gln Arg His Trp Gly
    210                 215                 220

Lys Trp Val Ala Glu Ile Arg Leu Pro Arg Asn Arg Thr Arg Leu Trp
225                 230                 235                 240

Leu Gly Thr Phe Asp Thr Ala Glu Asp Ala Ala Met Ala Tyr Asp Arg
                245                 250                 255

Glu Ala Phe Lys Leu Arg Gly Glu Asn Ala Arg Leu Asn Phe Pro Asp
            260                 265                 270

Arg Phe Leu Gly Lys Gly Arg Ala Gly Gly Lys Gly Arg Thr Ser Val
        275                 280                 285

Ser Ser Ser Ala Ala Ala Ala Ala Ser Cys Ser Ser Ser Ser Leu Ser
    290                 295                 300

Pro Pro Glu Thr Pro Asp Asp Ala Asn Thr Gln Gln Gln Ala Pro Gln
305                 310                 315                 320

Gln Arg Glu Gln Arg Asp Thr Ala Gly Val Ser Met Glu Lys Lys Gln
                325                 330                 335

Pro Gln Pro Pro Ala Pro Thr Ser Arg Gln Glu Gly Cys Ser Gly Gly
            340                 345                 350

Asp Ala Ala Ala Pro Tyr Pro Ala Glu Met Leu His Ala Pro Ala Ala
        355                 360                 365

Cys Gly Gly Met Trp Val Ala Pro Asp Glu Ser Trp Phe Ser Thr Trp
```

370 375 380

Gly Pro Gly Ser Ser Phe Trp Asp Asp Tyr Asp Met Asp Ser Ala Arg
385 390 395 400

Gly Leu Phe Leu His Pro Arg Phe Thr Gly Asp Glu Thr Ser Met Asp
405 410 415

His Ser Gly Thr Gln Ala Thr Val Pro Ala Val Ala Ala Thr Ala Ala
420 425 430

Gly Met Ser Met Pro Cys Asp Asp Val Pro Val Thr Ser Ser Ser Ser
435 440 445

Asp Leu Pro Pro Gln Gly Thr Pro Gln Thr Pro Thr Phe Met Trp Lys
450 455 460

Glu Asp
465

<210> 325
<211> 1528
<212> DNA
<213> Arabidopsis thaliana

<400> 325
attaaataga tctttaaaca aaaaaaccaa attctacttt tctttcaaaa acaatctctc 60
attatctcaa tcttactctt gttcaatctc ttttgaactt gaaaaaccat cattctatca 120
aatcaaacac aattgttagg gtttcttttt atcttatact ccacgaaacg tctggttgag 180
aaaaaatgga agaaagcaat gatatttttc agaacaattt cagtcctaaa atctcagaaa 240
tcagagcatc tctgtctcag atcatattag caggaggacc aaacacactc gactcaatct 300
tctcacttct cactccctcc tccgtagaat ccgccacaac atcattcaac actcacaatc 360
ctccgccacc gccgcagctc gggtcctccg tctatctccg ccaacgagat atcatcgaga 420
agttccacct tcagaacaga gcaatctcca ctcctcatcc tcctctgttt tcctcaacgt 480
acgatcatca tcaaacttcc gagctaatgc tccaagcggc ggccgggtct ccagccgccg 540
ctttcgccgc tgcgttagcg gctgggaggg tgacgaaaaa gaagaaactt tacagaggag 600
tgaggcagag acattgggga aaatgggttg cggagataag attgccgcaa aacagaatga 660
gggtttggtt aggtacttac gacacggcgg aagccgccgc ttacgcttac gatcgcgccg 720
cctataagct ccgtggagaa tacgctcgtc tcaattttcc taatctcaaa gacccgagtg 780
agctactcgg actcggagat tcctctaagc taatagctct caagaacgcc gtcgacggga 840
agatccaatc catttgccag agagtcagaa aagagagagc aaaaaagagc gtaaaagtga 900
gcaaaaactc gtcggccacg gcggattctt cgtgtttgtc atcgccggag attctgtcgt 960

cgtctccggt gacgacaact actactgcgg ttacttcaga ggatagttat tgggtttcac 1020

ctatgggttt gtgtaacagt gaaaatagtt ctccggtatc agtttcggtc cctagtgaag 1080

taccggcgac ggcggaggaa gaggcaatga tgggagtgga cactgatggg tttttattag 1140

cgaggatgcc atcgttcgat ccagagctga tttgggaagt tcttgccaat taatactaca 1200

caaagaatgt gaattttgat caaaattgca acaagaagaa gaaaaaaaaa agtttggaat 1260

taggacaaaa atgatgagaa aaattagggt tttaggtgtt aatttagggt ttaaagtaaa 1320

accggatctt ttgttaaaat cattcgaaag ttgttaaata atataattag ggtttctcaa 1380

atacaatgta aagcctcgtg gtttttgaaa gaggttgtga aagctccttt ttttgaattt 1440

ctgttttaga tcatcgaagg actttctttc tgattgtact tagaagaaca attatatgtt 1500

acagtgttaa tttaataaaa ttgagatt 1528

<210> 326
<211> 335
<212> PRT
<213> Arabidopsis thaliana

<400> 326

Met Glu Glu Ser Asn Asp Ile Phe Gln Asn Asn Phe Ser Pro Lys Ile
1 5 10 15

Ser Glu Ile Arg Ala Ser Leu Ser Gln Ile Ile Leu Ala Gly Gly Pro
20 25 30

Asn Thr Leu Asp Ser Ile Phe Ser Leu Leu Thr Pro Ser Ser Val Glu
35 40 45

Ser Ala Thr Thr Ser Phe Asn Thr His Asn Pro Pro Pro Pro Pro Gln
50 55 60

Leu Gly Ser Ser Val Tyr Leu Arg Gln Arg Asp Ile Ile Glu Lys Phe
65 70 75 80

His Leu Gln Asn Arg Ala Ile Ser Thr Pro His Pro Pro Leu Phe Ser
85 90 95

Ser Thr Tyr Asp His His Gln Thr Ser Glu Leu Met Leu Gln Ala Ala
100 105 110

Ala Gly Ser Pro Ala Ala Ala Phe Ala Ala Ala Leu Ala Ala Gly Arg
115 120 125

Val Thr Lys Lys Lys Lys Leu Tyr Arg Gly Val Arg Gln Arg His Trp
130 135 140

Gly Lys Trp Val Ala Glu Ile Arg Leu Pro Gln Asn Arg Met Arg Val

145 150 155 160

Trp Leu Gly Thr Tyr Asp Thr Ala Glu Ala Ala Ala Tyr Ala Tyr Asp
165 170 175

Arg Ala Ala Tyr Lys Leu Arg Gly Glu Tyr Ala Arg Leu Asn Phe Pro
180 185 190

Asn Leu Lys Asp Pro Ser Glu Leu Leu Gly Leu Gly Asp Ser Ser Lys
195 200 205

Leu Ile Ala Leu Lys Asn Ala Val Asp Gly Lys Ile Gln Ser Ile Cys
210 215 220

Gln Arg Val Arg Lys Glu Arg Ala Lys Lys Ser Val Lys Val Ser Lys
225 230 235 240

Asn Ser Ser Ala Thr Ala Asp Ser Ser Cys Leu Ser Ser Pro Glu Ile
245 250 255

Leu Ser Ser Ser Pro Val Thr Thr Thr Thr Thr Ala Val Thr Ser Glu
260 265 270

Asp Ser Tyr Trp Val Ser Pro Met Gly Leu Cys Asn Ser Glu Asn Ser
275 280 285

Ser Pro Val Ser Val Ser Val Pro Ser Glu Val Pro Ala Thr Ala Glu
290 295 300

Glu Glu Ala Met Met Gly Val Asp Thr Asp Gly Phe Leu Leu Ala Arg
305 310 315 320

Met Pro Ser Phe Asp Pro Glu Leu Ile Trp Glu Val Leu Ala Asn
325 330 335

<210> 327
<211> 843
<212> DNA
<213> Oryza sativa

<400> 327
atggcagctg ctatagaagg aaatctgatg cgggcgctgg gagaggctcc gtcgccgcag 60

atgcagaaga tcgcgccgcc gccgtttcat cccggcttgc cgccggcgcc ggcgaacttc 120

tcctcggccg gagtccacgg gttccactac atgggcccgg cccagctcag cccggcccag 180

atccagcgcg tccaggccca actccacatg cagcggcagg cccagtcggg gctcggcccg 240

cgggcccagc ccatgaagcc cgcttcggcg gctgctccgg cggcggcggc ggcgcgggcg 300

cagaagctgt accgcggcgt gcggcagcgg cactggggca agtgggtggc ggagatccgg 360

ctgccgcgca accgcaccag gctctggctc ggcaccttcg acaccgccga ggaggcggcg 420

ctcacctacg accaggccgc gtaccgcctc cgcggcgacg cggcgcggct caacttcccg 480

gacaacgccg cgtcgcgggg cccgctcgac gccgccgtgg acgccaagct ccaggccatc 540

tgcgacacca tcgccgcgtc caagaacgcc tcatccaggt ccaggggcgg cgccggcagg 600

gccatgccca tcaacgcgcc cctggtcgcc gcggcgtcgt cgtcctccgg ctccgaccac 660

tccggcggcg gcgacgacgg cggctcggag acgtcgtcgt cgtctgcggc ggcgtcgccg 720

ctggcggaga tggagcagct ggacttcagc gaggtgccgt gggacgaggc ggaggggttc 780

gcgctcacca agtacccgtc gtacgagatc gactgggact cgctgctcaa caacaataac 840

tag 843

<210> 328
<211> 280
<212> PRT
<213> Oryza sativa

<400> 328

Met Ala Ala Ala Ile Glu Gly Asn Leu Met Arg Ala Leu Gly Glu Ala
1 5 10 15

Pro Ser Pro Gln Met Gln Lys Ile Ala Pro Pro Pro Phe His Pro Gly
20 25 30

Leu Pro Pro Ala Pro Ala Asn Phe Ser Ser Ala Gly Val His Gly Phe
35 40 45

His Tyr Met Gly Pro Ala Gln Leu Ser Pro Ala Gln Ile Gln Arg Val
50 55 60

Gln Ala Gln Leu His Met Gln Arg Gln Ala Gln Ser Gly Leu Gly Pro
65 70 75 80

Arg Ala Gln Pro Met Lys Pro Ala Ser Ala Ala Ala Pro Ala Ala Ala
85 90 95

Ala Ala Arg Ala Gln Lys Leu Tyr Arg Gly Val Arg Gln Arg His Trp
100 105 110

Gly Lys Trp Val Ala Glu Ile Arg Leu Pro Arg Asn Arg Thr Arg Leu
115 120 125

Trp Leu Gly Thr Phe Asp Thr Ala Glu Glu Ala Ala Leu Thr Tyr Asp
130 135 140

Gln Ala Ala Tyr Arg Leu Arg Gly Asp Ala Ala Arg Leu Asn Phe Pro
145 150 155 160

Asp Asn Ala Ala Ser Arg Gly Pro Leu Asp Ala Ala Val Asp Ala Lys
165 170 175

Leu Gln Ala Ile Cys Asp Thr Ile Ala Ala Ser Lys Asn Ala Ser Ser
180 185 190

Arg Ser Arg Gly Gly Ala Gly Arg Ala Met Pro Ile Asn Ala Pro Leu
195 200 205

Val Ala Ala Ala Ser Ser Ser Ser Gly Ser Asp His Ser Gly Gly Gly
210 215 220

Asp Asp Gly Gly Ser Glu Thr Ser Ser Ser Ser Ala Ala Ala Ser Pro
225 230 235 240

Leu Ala Glu Met Glu Gln Leu Asp Phe Ser Glu Val Pro Trp Asp Glu
245 250 255

Ala Glu Gly Phe Ala Leu Thr Lys Tyr Pro Ser Tyr Glu Ile Asp Trp
260 265 270

Asp Ser Leu Leu Asn Asn Asn Asn
275 280

<210> 329
<211> 822
<212> DNA
<213> Oryza sativa

<400> 329
atggacgcca gcctccgcac actgcctccg gcgatcttcc ccggcgaggt ccgctccgcc 60

gtctcctccc tcctcctctc tcccggcggc agcgccctcg acaccgtgtt ctcccacctc 120

ccgccgcccg tcaccatccc gccgctcggc tccagcgtct actaccgcca gagcgagctc 180

ctgcgccact tcgccgcgtc gcaggcggcg caggcgcaga gcgccaccgc cgccgccagc 240

tcttcgtcgg ccgccgcggc gggtctcgac gatggcgcgc cgcggaagct gtaccgcggc 300

gtgcggcagc ggcagtgggg gaagtgggtg gccgagatca ggctgccgca gaaccgggtg 360

cgcgtgtggc tcggcaccta cgactcgccg gagaccgccg cgcacgccta cgaccgcgcc 420

gcgttcaagc tccgcggcga gtacgcgcgc ctcaacttcc ccggcgtcat ggacggccgc 480

gactgccccg acaacctgcg ccaactccgc gacgccgtcg acgccaagat ccaggccatc 540

cgcgtccgca tggcgcgcaa gcgcgcgcgc gcgcgccgcc agcgcgagga gagcaagaag 600

agccaacgcg ccgaggacgc gaaggcggcg acgccgtctc gccccgtggc ctccgagcgc 660

gccgcgtccg agacgacgac gacgacaacc acgacgtcgt cgtcgtacgg gtcaccggac 720

ggcgtgctct ccatgagcgc ggcgtccgtc gacggcgact gcccgctcga gcggatgccg 780

tcgttcgatc ccgagttgat ctgggagatg cttaacttct ag 822

<210> 330
<211> 273
<212> PRT
<213> Oryza sativa

<400> 330

Met Asp Ala Ser Leu Arg Thr Leu Pro Pro Ala Ile Phe Pro Gly Glu
1 5 10 15

Val Arg Ser Ala Val Ser Ser Leu Leu Leu Ser Pro Gly Gly Ser Ala
20 25 30

Leu Asp Thr Val Phe Ser His Leu Pro Pro Pro Val Thr Ile Pro Pro
35 40 45

Leu Gly Ser Ser Val Tyr Tyr Arg Gln Ser Glu Leu Leu Arg His Phe
50 55 60

Ala Ala Ser Gln Ala Ala Gln Ala Gln Ser Ala Thr Ala Ala Ala Ser
65 70 75 80

Ser Ser Ser Ala Ala Ala Ala Gly Leu Asp Asp Gly Ala Pro Arg Lys
85 90 95

Leu Tyr Arg Gly Val Arg Gln Arg Gln Trp Gly Lys Trp Val Ala Glu
100 105 110

Ile Arg Leu Pro Gln Asn Arg Val Arg Val Trp Leu Gly Thr Tyr Asp
115 120 125

Ser Pro Glu Thr Ala Ala His Ala Tyr Asp Arg Ala Ala Phe Lys Leu
130 135 140

Arg Gly Glu Tyr Ala Arg Leu Asn Phe Pro Gly Val Met Asp Gly Arg
145 150 155 160

Asp Cys Pro Asp Asn Leu Arg Gln Leu Arg Asp Ala Val Asp Ala Lys
165 170 175

Ile Gln Ala Ile Arg Val Arg Met Ala Arg Lys Arg Ala Arg Ala Arg
180 185 190

Arg Gln Arg Glu Glu Ser Lys Lys Ser Gln Arg Ala Glu Asp Ala Lys
195 200 205

Ala Ala Thr Pro Ser Arg Pro Val Ala Ser Glu Arg Ala Ala Ser Glu
210 215 220

Thr Thr Thr Thr Thr Thr Thr Thr Ser Ser Ser Tyr Gly Ser Pro Asp
225 230 235 240

Gly Val Leu Ser Met Ser Ala Ala Ser Val Asp Gly Asp Cys Pro Leu
245 250 255

Glu Arg Met Pro Ser Phe Asp Pro Glu Leu Ile Trp Glu Met Leu Asn
260 265 270

Phe

<210> 331
<211> 34
<212> PRT
<213> Artificial sequence

<220>
<223> C-terminal reqion of SEQ ID NO: 2

<400> 331

Pro Phe Leu Met Gln Trp Leu Asn Leu Leu Pro Leu Pro Val Leu Asp
1 5 10 15

Ser Ser Ser Trp Cys Pro Glu His Phe His Asn Ser Glu Ser Asp Ala
20 25 30

Leu Pro

<210> 332
<211> 56
<212> PRT
<213> Artificial sequence

<220>
<223> AP2 DNA-binding domain

<400> 332

Tyr Arg Gly Val Arg Gln Arg His Trp Gly Lys Trp Val Ala Glu Ile
1 5 10 15

Arg Leu Pro Arg Asn Arg Thr Arg Leu Trp Leu Gly Thr Phe Asp Thr
20 25 30

Ala Glu Glu Ala Ala Leu Ala Tyr Asp Ser Ala Ala Phe Arg Leu Arg
35 40 45

Gly Glu Ser Ala Arg Leu Asn Phe
50 55

<210> 333
<211> 17
<212> PRT
<213> Artificial sequence

<220>
<223> Motif I

<220>
<221> UNSURE
<222> (4)..(4)
<223> Xaa can be any naturally occurring amino acid

<220>
<221> UNSURE
<222> (6)..(6)
<223> Xaa can be any naturally occurring amino acid

<220>
<221> UNSURE
<222> (8)..(8)
<223> Xaa can be any naturally occurring amino acid

<220>
<221> UNSURE
<222> (10)..(10)
<223> Xaa can be any naturally occurring amino acid

<220>
<221> VARIANT
<222> (15)..(15)
<223> /replace ="Tyr"

<220>
<221> VARIANT
<222> (16)..(16)
<223> /replace ="Glu"

<220>
<221> VARIANT
<222> (17)..(17)
<223> /replace ="Ser"

<400> 333

Arg Leu Pro Xaa Asn Xaa Arg Xaa Arg Xaa Trp Leu Gly Thr Phe Asp
1 5 10 15

Thr

<210> 334
<211> 3
<212> PRT
<213> Artificial sequence

<220>
<223> Motif II

<220>
<221> VARIANT

<222> (3)..(3)
<223> /replace = "Glu"

<400> 334

Arg Gly Asp
1

<210> 335
<211> 24
<212> PRT
<213> Artificial sequence

<220>
<223> Motif III

<400> 335

Trp Asp Glu Ser Glu Ser Phe Leu Leu His Lys Tyr Pro Ser Leu Glu
1               5                   10                  15

Ile Asp Trp Asp Ala Ile Leu Ser
            20

<210> 336
<211> 17
<212> PRT
<213> Artificial sequence

<220>
<223> Motif IV

<400> 336

Gly Pro Pro Leu His Ala Ala Val Asp Ala Lys Leu His Ala Ile Cys
1               5                   10                  15

His

<210> 337
<211> 23
<212> PRT
<213> Artificial sequence

<220>
<223> Motif V

<400> 337

Gly Ala Asn Tyr Leu Thr Pro Ala Gln Val Leu His Val Gln Ala Gln
1               5                   10                  15

Leu Gln Arg Leu Arg Arg Pro
            20

<210> 338
<211> 28

<212> PRT
<213> Artificial sequence

<220>
<223> Motif V

<400> 338

Val Asp Ser Lys Glu Leu Met Gly Ala Leu Ala Pro Ser Met Val Ser
1 5 10 15

Phe Ser Tyr Pro Cys Ser Glu Gln Ser Ala Ser Ser
20 25

<210> 339
<211> 2194
<212> DNA
<213> Oryza sativa

<400> 339
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc cctttccccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 340
<211> 1264
<212> DNA
<213> Oryza sativa

<400> 340
tcgacgctac tcaagtggtg ggaggccacc gcatgttcca acgaagcgcc aaagaaagcc 60

ttgcagactc taatgctatt agtcgcctag gatatttgga atgaaaggaa ccgcagagtt 120

tttcagcacc aagagcttcc ggtggctagt ctgatagcca aaattaagga ggatgccaaa 180

acatgggtct tggcgggcgc gaaacacctt gataggtggc ttacctttta acatgttcgg 240

gccaaaggcc ttgagacggt aaagttttct atttgcgctt gcgcatgtac aattttattc 300

ctctattcaa tgaaattggt ggctcactgg ttcattaaaa aaaaaagaat ctagcctgtt 360

cgggaagaag aggattttgt tcgtgagaga gagagagaga gagagagaga gagagagaga 420

gaaggaggag gaggattttc aggcttcgca ttgcccaacc tctgcttctg ttggcccaag 480

aagaatccca ggcgcccatg ggctggcagt ttaccacgga cctacctagc ctaccttagc 540

tatctaagcg ggccgaccta gtagccacgt gcctagtgta gattaaagtt gccgggccag 600

caggaagcca cgctgcaatg gcatcttccc ctgtccttcg cgtacgtgaa aacaaaccca 660

ggtaagctta gaatcttctt gcccgttgga ctgggacacc caccaatccc accatgcccc 720

gatattcctc cggtctcggt tcatgtgatg tcctctcttg tgtgatcacg gagcaagcat 780

tcttaaacgg caaaagaaaa tcaccaactt gctcacgcag tcacgctgca ccgcgcgaag 840

cgacgcccga taggccaaga tcgcgagata aaataacaac caatgatcat aaggaaacaa 900

gcccgcgatg tgtcgtgtgc agcaatcttg gtcatttgcg ggatcgagtg cttcacagct 960

aaccaaatat tcggccgatg atttaacaca ttatcagcgt agatgtacgt acgatttgtt 1020

aattaatcta cgagccttgc tagggcaggt gttctgccag ccaatccaga tcgccctcgt 1080

atgcacgctc acatgatggc agggcagggt tcacatgagc tctaacggtc gattaattaa 1140

tcccggggct cgactataaa tacctcccta atcccatgat caaaaccatc tcaagcagcc 1200

taatcatctc cagctgatca agagctctta attagctagc tagtgattag ctgcgcttgt 1260

gatc 1264

<210> 341
<211> 1542
<212> DNA
<213> Hordeum vulgare

<400> 341
ccgcttcatt aaaaactgcc catttttcca gttccgacga ggcgcctccc cccctccccg 60

gcgacgacta ggacgaggca ccgccggcaa caaccatgtg cggcggcgcg atcctcaagg 120

acctcaaggt ccccgcggtg acgcggaagg tgacggaggc ggcgctgtgg cccgagaaga 180

agaagcccag gcaggccgac ggcggggccc ggcgcctcgg gctggtcgac ggcgaggagg 240

acttcgaggc cgacttcgag gagttcgagg ccgactccgg ggactccgac ctggagctcg 300

ggcgcggccg ggcggctgag aaggacgacg acgaggtcgt cgagatcaag ccctacgccg 360

ccgtcaagag gcccctctcc caagatgact tcagcatcat tactactgct ggttgtgatg 420

gtcctgcaca aaggtcagca aaaaggaaga gaaagaacca attcaggggt atccgtcagc 480

gcccctgggg taagtgggct gctgaaatca gagatcctag caaaggtgtc cgtgtttggc 540

ttggtacttt caacagtgct gaagaagctg caagagccta cgatgttgaa gcacgcagga 600

tccgtggcaa gaaggccaag gtaaactttc cagaggaacc aacagttcct cagaagcgcc 660

gtgttcgccc tgctcctctt aaagcaccca agctaagcgc atcacaggaa cctaccgtca 720

taccagcagt caacaacctt gccaacccaa atgctttcgt ctacccatct gctgactttg 780

catcaaacca gccacctgtt cagcctgata acgtgccatt tgttcctgca atgaagtttg 840

ctgcccctgt tgaagctcct gttatgaata tgtactctga ccagggaagc aactcttttg 900

gctgttctga cttgggctgg gactatgaga ccaagactcc agatatatca tccgttgctc 960

ccatttccac cattgctgaa ggagcagaat ctgcgcttat ccagagcaac acctacaact 1020

cagtggtgcc tcctgttatg gagaacaatg ctgtcgattt tgaaccttgg acgaggtttc 1080

ttatggatga tggcgtggat gagccgattg acagccttct gaactttggt gtgcctcagg 1140

atgtcattag caacatggac ctttggagct tcgatgacat gcccatctgt ggagaattat 1200

gctgagggat tcaaacccct gtatataaag acaaagggaa taagactatg ggagattggg 1260

aagcaccctg ttgtcaactt cggctagcat atgcttatgt ccaagcttag atgcaaaaaa 1320

gttgcatcct gagtctcttt tgtaatcaac ccttttccta gctgactgtc gatgaaccgt 1380

tgtcatttat gagtctgatg aaccgttgtc tttatctttt gtcaacttat atgtcgtcgc 1440

taccatttct gaatgtgaat ggcatggatc tatgtccagt ttgctttaaa aaaaaaaaaa 1500

aaaaaaaaaa aaaaaaaaaa aaaaaaaaaa aaaaaaaaaa aa 1542

<210> 342
<211> 369
<212> PRT
<213> Hordeum vulgare

<400> 342

Met Cys Gly Gly Ala Ile Leu Lys Asp Leu Lys Val Pro Ala Val Thr
1 5 10 15

Arg Lys Val Thr Glu Ala Ala Leu Trp Pro Glu Lys Lys Lys Pro Arg
20 25 30

Gln Ala Asp Gly Gly Ala Arg Arg Leu Gly Leu Val Asp Gly Glu Glu
35 40 45

Asp Phe Glu Ala Asp Phe Glu Glu Phe Glu Ala Asp Ser Gly Asp Ser
50 55 60

Asp Leu Glu Leu Gly Arg Gly Arg Ala Ala Glu Lys Asp Asp Asp Glu
65 70 75 80

Val Val Glu Ile Lys Pro Tyr Ala Ala Val Lys Arg Pro Leu Ser Gln
85 90 95

Asp Asp Phe Ser Ile Ile Thr Thr Ala Gly Cys Asp Gly Pro Ala Gln
100 105 110

Arg Ser Ala Lys Arg Lys Arg Lys Asn Gln Phe Arg Gly Ile Arg Gln
115 120 125

Arg Pro Trp Gly Lys Trp Ala Ala Glu Ile Arg Asp Pro Ser Lys Gly
130 135 140

Val Arg Val Trp Leu Gly Thr Phe Asn Ser Ala Glu Glu Ala Ala Arg
145 150 155 160

Ala Tyr Asp Val Glu Ala Arg Arg Ile Arg Gly Lys Lys Ala Lys Val
165 170 175

Asn Phe Pro Glu Glu Pro Thr Val Pro Gln Lys Arg Arg Val Arg Pro
180 185 190

```
Ala Pro Leu Lys Ala Pro Lys Leu Ser Ala Ser Gln Glu Pro Thr Val
        195                 200                 205

Ile Pro Ala Val Asn Asn Leu Ala Asn Pro Asn Ala Phe Val Tyr Pro
    210                 215                 220

Ser Ala Asp Phe Ala Ser Asn Gln Pro Pro Val Gln Pro Asp Asn Val
225                 230                 235                 240

Pro Phe Val Pro Ala Met Lys Phe Ala Ala Pro Val Glu Ala Pro Val
                245                 250                 255

Met Asn Met Tyr Ser Asp Gln Gly Ser Asn Ser Phe Gly Cys Ser Asp
            260                 265                 270

Leu Gly Trp Asp Tyr Glu Thr Lys Thr Pro Asp Ile Ser Ser Val Ala
        275                 280                 285

Pro Ile Ser Thr Ile Ala Glu Gly Ala Glu Ser Ala Leu Ile Gln Ser
    290                 295                 300

Asn Thr Tyr Asn Ser Val Val Pro Pro Val Met Glu Asn Asn Ala Val
305                 310                 315                 320

Asp Phe Glu Pro Trp Thr Arg Phe Leu Met Asp Asp Gly Val Asp Glu
                325                 330                 335

Pro Ile Asp Ser Leu Leu Asn Phe Gly Val Pro Gln Asp Val Ile Ser
            340                 345                 350

Asn Met Asp Leu Trp Ser Phe Asp Asp Met Pro Ile Cys Gly Glu Leu
        355                 360                 365

Cys


<210>  343
<211>  1489
<212>  DNA
<213>  Zea mays

<400>  343
caccatttcg ctcccgcaag caccgatttg tttactgctc tctccgacgc gcggcggcgg      60

agctccctac gacgactgaa ccatgtgcgg cggcgcgatc cttgccaacc tccgcgagcc     120

ggcgccgcgc cggctcacag agcgggacat ctggcagcag aagaagaagc ccaagagggg     180

cggcgccggc gggaggcgct cgttcgcggc ggaagacgat gaggacttcg aggccgactt     240

cgaggacttc gaagccgact ccagtgattc agatttggag ctcagggaag ggactgacga     300
```

cgacgtcatc gagatcaagc ccttcaccgc caagaggact ttctccagag atggcttaag 360
caccatgact actgctggtt agcaaggtca gccaaaagga agaggaagaa tcaatacagg 420
ggtatccgcc agcgcccttg gggtaagtgg gctgctgaga tcagagatcc ccagaagggc 480
gttcgtgttt ggcttggtac tttcaatagt ccggaggaag ctgcaagagc ttatgatgct 540
gaagcgcgca ggattcgtgg caagaaggcc aaggttaact ttcctgatgc accagcagtt 600
ggtcagaagt gccgttctag ttcagcttct gctaaagcac tcaagtcatg tgttgaacag 660
aagccaattg tcaaaacaga tatgaacatc cttgccaaca caaatgcacc cttctaccaa 720
tctgttaact acgcatccaa tttatttgtt cagcatggca atatgccatt tgttccagca 780
atgaactcta ctgtttcttt tgatgatcct atcatgaatc tgcactctga ccagggaagt 840
aactcccttg gctgctcaga cttgggctgg gagaatgata ccaagacacc agacatcaca 900
tccattgctc ccattcccac tattgctgaa ggcgatgagt ctgtatttgt caactccaat 960
tcaaacagct cgatggtgcc tcctgtcctg gagaacaatg ctgttgatct cactgatggg 1020
ctgacagatt tagaatccta tatgaggttt cttctggatg gcggtgcaag tgattcaatt 1080
gatagccttc tgaaccttga tggatcgcag gatgttggta gcaacatgga cctctggacc 1140
ttcgatgaca tgcccatcgc tggcgatttc ttctgaggaa tttgaagctt ggccatagga 1200
ctatgtacat agggactagg ggaataaaga ctgggagatt gggaagcacc tgcttggcac 1260
cttgggggta gcatatgctc gtgtctagct cagatgcaga agtcgcattc tgaaactctt 1320
tggttatcga cctgttccct attttaacta tctctgtatg agagccattt atgagactga 1380
accattttgt tttgctttct tttcgttgtt acaatatatg gtttaacatt atgatttatg 1440
gatatgtgcc aaatatggtg ctcaacagtg gtcaaacatg ccttttaga 1489

<210> 344
<211> 164
<212> PRT
<213> Zea mays

<400> 344

Met Asn Ile Leu Ala Asn Thr Asn Ala Pro Phe Tyr Gln Ser Val Asn
1 5 10 15

Tyr Ala Ser Asn Leu Phe Val Gln His Gly Asn Met Pro Phe Val Pro
20 25 30

Ala Met Asn Ser Thr Val Ser Phe Asp Asp Pro Ile Met Asn Leu His
35 40 45

Ser Asp Gln Gly Ser Asn Ser Leu Gly Cys Ser Asp Leu Gly Trp Glu
50 55 60

Asn Asp Thr Lys Thr Pro Asp Ile Thr Ser Ile Ala Pro Ile Pro Thr
65 70 75 80

Ile Ala Glu Gly Asp Glu Ser Val Phe Val Asn Ser Asn Ser Asn Ser
85 90 95

Ser Met Val Pro Pro Val Leu Glu Asn Asn Ala Val Asp Leu Thr Asp
100 105 110

Gly Leu Thr Asp Leu Glu Ser Tyr Met Arg Phe Leu Leu Asp Gly Gly
115 120 125

Ala Ser Asp Ser Ile Asp Ser Leu Leu Asn Leu Asp Gly Ser Gln Asp
130 135 140

Val Gly Ser Asn Met Asp Leu Trp Thr Phe Asp Asp Met Pro Ile Ala
145 150 155 160

Gly Asp Phe Phe

<210> 345
<211> 789
<212> DNA
<213> Triticum aestivum

<400> 345
gacgaggcac cgccggcaat catgtgcggc ggcgcgatcc tcaaggacct caaggtcccc 60

gcgccgacgc ggaaggtgac ggcggcggtg ctgtggcccg agaagaacaa gcccaagcgg 120

gccgacggcg gcgcccggcg cctcgcgggg ctcggccggc gcggggggct cgggctggac 180

gacggcgacg cggacttcga ggccgacttc gaggagttcg aggccgactc cggggactcc 240

gaccaggagc tcgggcgcgc cggggtggct gagaaggacg gcgacgacga ggtcgtcgag 300

accaagccct tcgccgccgt caagaggtcc ctctcccaag atgacttaag caccatgacc 360

actgctggtt ttgatggtcc tgcacaaagg tcagcaaaaa ggaagagaaa gaacgaattc 420

aggggtatcc gccagcgccc ctggggtaag tgggctgctg aaatcagaga tcctagcaag 480

ggtgtccgtg tttggcttgg taccttcaac agtgctgaag aagctgcaag agcttatgat 540

gttgaagcac gaaggatccg tggcaagaag gccaaggtta actttccaga ggaaccaaca 600

gttcctcaga agcgccgtgc ttgccctgct gctcctaaag ttcccaagtc aagcgcagca 660

caggaaccta ccgtcatacc agcagtcaac aaccttgcca acccaaatgc tttcgtctac 720

ccgcctgctg actttgcatc aaagcagcca cttgttcagc ctgacaacgt gccatatgtt 780

ccctgcaat 789

<210> 346
<211> 256

<212> PRT
<213> Triticum aestivum

<400> 346

Met Cys Gly Gly Ala Ile Leu Lys Asp Leu Lys Val Pro Ala Pro Thr
1               5                   10                  15

Arg Lys Val Thr Ala Ala Val Leu Trp Pro Glu Lys Asn Lys Pro Lys
            20                  25                  30

Arg Ala Asp Gly Gly Ala Arg Arg Leu Ala Gly Leu Gly Arg Arg Gly
        35                  40                  45

Gly Leu Gly Leu Asp Asp Gly Asp Ala Asp Phe Glu Ala Asp Phe Glu
    50                  55                  60

Glu Phe Glu Ala Asp Ser Gly Asp Ser Asp Gln Glu Leu Gly Arg Ala
65                  70                  75                  80

Gly Val Ala Glu Lys Asp Gly Asp Asp Glu Val Val Glu Thr Lys Pro
                85                  90                  95

Phe Ala Ala Val Lys Arg Ser Leu Ser Gln Asp Asp Leu Ser Thr Met
            100                 105                 110

Thr Thr Ala Gly Phe Asp Gly Pro Ala Gln Arg Ser Ala Lys Arg Lys
        115                 120                 125

Arg Lys Asn Glu Phe Arg Gly Ile Arg Gln Arg Pro Trp Gly Lys Trp
    130                 135                 140

Ala Ala Glu Ile Arg Asp Pro Ser Lys Gly Val Arg Val Trp Leu Gly
145                 150                 155                 160

Thr Phe Asn Ser Ala Glu Glu Ala Ala Arg Ala Tyr Asp Val Glu Ala
                165                 170                 175

Arg Arg Ile Arg Gly Lys Lys Ala Lys Val Asn Phe Pro Glu Glu Pro
            180                 185                 190

Thr Val Pro Gln Lys Arg Arg Ala Cys Pro Ala Ala Pro Lys Val Pro
        195                 200                 205

Lys Ser Ser Ala Ala Gln Glu Pro Thr Val Ile Pro Ala Val Asn Asn
    210                 215                 220

Leu Ala Asn Pro Asn Ala Phe Val Tyr Pro Pro Ala Asp Phe Ala Ser
225                 230                 235                 240

Lys Gln Pro Leu Val Gln Pro Asp Asn Val Pro Tyr Val Pro Cys Asn
245 250 255

<210> 347
<211> 1399
<212> DNA
<213> Triticum aestivum

<400> 347
gggacacaca cgcagatccg agctaaccac catcgacgag cgccagcgcc agcagccgag 60

ccggatcgac cttttctttt ttcttttaca cagcgggaca gagaaaggag tcaggcagcc 120

aaagccaccc accgctttta ccaaccgatc gtccgatcgg cgttgccgcc accgctagca 180

ttgtcggctt cagctccatc caaatccacc gccagcaagc aagccggcgc catgggtctg 240

ccgatgagga gggagaggga cgcggaggcg gagctcaacc tgccgccggg gttccggttc 300

cacccgacgg acgaggagct ggtggcgcac tacctctgcg cgcgcgccgc ggggcgccgc 360

ccgcccgtgc ccatcatcgc cgaggtcgac ctgtaccgct tcgacccctg ggacctgccg 420

gagcgcgccc tcttcgggcg ccgcgagtgg tacttcttca cgccgcggga ccgcaagtac 480

cccaacggct cccgccccaa ccgcgccgcc gggtcgggct actggaaggc cacgggcgcg 540

gacaagcccg tggagcacgg gggccggacg gcggggatca agaaggcgct cgtgttctac 600

cacggcaagc cgccgcgcgg ggtcaagacg gagtggatca tgcacgagta ccgcctcgcc 660

gaagccggcg gcgcccgcgc caagaagtcc ggcgccggca cgctcaggct ggatgactgg 720

gtgctgtgcc gcctgtacaa caagaagaac gagtgggaga agatgcagca gcagaaggag 780

aaggagagaa gaaggcgatg gagtcggagg cgtcgctctc gcactcccac tccgacacgc 840

ggacgccgga atcggagatc gacgacgacc cgttcccgga gctgggctct ctgccggcgt 900

tcgacgacat gggcccagcc ccagcagcag cagcccctgc cggcgtcgtc ctgcccaagg 960

aggaggtgga ggacttcggc gacctcggcg gcgacgactg gctcgcgggc atcaacctcg 1020

acgacctgca gatgccgggg gacgccgccg atttctacgg ctccatgctc gtctcgccga 1080

tggccgccaa gatggagccg gacggcggct tccccttctt ctgagatcct tcggcgactc 1140

cggggacgac cggatgcagc gcaacgcaaa ctgtgtatag ttcagatttt cttcatggaa 1200

ccaacaacaa caacaaaaaa agttctgcta atatcttgtt ctagtggtgt agtgcagaag 1260

tagcagcagg catgattgaa gttccattgt agtactgatg ttcatcgcca cttgatctgt 1320

gacaagtgac ggcattcttt cgtttcttgg ctgcaaattc ttcgttgttc agaatataat 1380

ttcagatggg taatgccaa 1399

<210> 348
<211> 343
<212> PRT
<213> Triticum aestivum

<400> 348

```
Met Gly Leu Pro Met Arg Arg Glu Arg Asp Ala Glu Ala Glu Leu Asn
1               5                   10                  15

Leu Pro Pro Gly Phe Arg Phe His Pro Thr Asp Glu Glu Leu Val Ala
            20                  25                  30

His Tyr Leu Cys Ala Arg Ala Ala Gly Arg Arg Pro Pro Val Pro Ile
        35                  40                  45

Ile Ala Glu Val Asp Leu Tyr Arg Phe Asp Pro Trp Asp Leu Pro Glu
    50                  55                  60

Arg Ala Leu Phe Gly Arg Arg Glu Trp Tyr Phe Phe Thr Pro Arg Asp
65                  70                  75                  80

Arg Lys Tyr Pro Asn Gly Ser Arg Pro Asn Arg Ala Ala Gly Ser Gly
                85                  90                  95

Tyr Trp Lys Ala Thr Gly Ala Asp Lys Pro Val Glu His Gly Gly Arg
            100                 105                 110

Thr Ala Gly Ile Lys Lys Ala Leu Val Phe Tyr His Gly Lys Pro Pro
        115                 120                 125

Arg Gly Val Lys Thr Glu Trp Ile Met His Glu Tyr Arg Leu Ala Glu
    130                 135                 140

Ala Gly Gly Ala Arg Ala Lys Lys Ser Gly Ala Gly Thr Leu Arg Leu
145                 150                 155                 160

Asp Asp Trp Val Leu Cys Arg Leu Tyr Asn Lys Lys Asn Glu Trp Glu
                165                 170                 175

Lys Met Gln Gln Gln Lys Glu Lys Glu Arg Arg Arg Arg Trp Ser Arg
            180                 185                 190

Arg Arg Arg Ser Arg Thr Pro Thr Pro Thr Arg Gly Arg Arg Asn Arg
        195                 200                 205

Arg Ser Thr Thr Thr Arg Ser Arg Ser Trp Ala Leu Cys Arg Arg Ser
    210                 215                 220

Thr Thr Trp Ala Gln Pro Gln Gln Gln Gln Pro Leu Pro Ala Ser Ser
225                 230                 235                 240

Cys Pro Arg Arg Arg Trp Arg Thr Ser Ala Thr Ser Ala Ala Thr Thr
                245                 250                 255
```

Gly Ser Arg Ala Ser Thr Ser Thr Thr Cys Arg Cys Arg Gly Thr Pro
260 265 270

Pro Ile Ser Thr Ala Pro Cys Ser Ser Arg Arg Trp Pro Pro Arg Trp
275 280 285

Ser Arg Thr Ala Ala Ser Pro Ser Ser Glu Ile Leu Arg Arg Leu Arg
290 295 300

Gly Arg Pro Asp Ala Ala Gln Arg Lys Leu Cys Ile Val Gln Ile Phe
305 310 315 320

Phe Met Glu Pro Thr Thr Thr Thr Lys Lys Val Leu Leu Ile Ser Cys
325 330 335

Ser Ser Gly Val Val Gln Lys
340

<210> 349
<211> 1262
<212> DNA
<213> Hordeum vulgare

<400> 349
tttcatatgc agtagccgtc ttctcctcct cctcctcctc ctgccttcca gctagctcac 60

tatcgcatca tccacaccac acctactact cataccgatt ccgttcccac ccgctcgcca 120

tcgccatgcc aatgggcagc agcagcagca gcgccgccat gccggccctc cctcccggct 180

tccgggtcca ccccaccgac gaggagctca tcgtccacta cctcggcagg caggccgcgt 240

ccatgcccag ccccgtgccc atcatcgccg aggtcaacat ctacaagtgc aacccatggg 300

acctccccgg caaggccttg cttggggaga acgagtggta cttcttcagc cccccgggatc 360

gcaagtaccc caacggcgcg cgcccgaacc gcgccgccgg gtccggctac tggaaggcca 420

ccggcaccga caagcccatc ccgcccaggg agagcggcgg caggaccgtc ggcatcaaga 480

aggcgctcgt cttctactcc ggcagggcgc ccaggggcgt caagaccgac tggatcatgc 540

acgagtaccg catcgcccaa gccgaccgca cacccggcaa gaagggatcc ctcaagctgg 600

acgaatgggt gctgtgccgg ctgtataaca agaagaacaa ctgggacaag gtcaaggtgg 660

agcaggacat ggctgtggtg cagggacaga atggggaggt catggacgcg ctggccaccg 720

acgccatgtc cgacagcttc cagacgcacg actcctcgga gatcgacaac gcctccggcc 780

tgcagcagca gcaccacggc ttcatggaca tggcgcagcg gcaggccagg gaaggcatgg 840

tgacggtcaa ggaggacagc gactggttca ccggcctgag tatggacgac ctgcagactt 900

gttacatgaa catggggcag atggtgaatc cagcggcgat gcctgtgcac gacggcagcg 960

gctatctgca gtcgatgaac tcgcctcaga tgatgagacc catgtggcaa acaatcttgc 1020

caccattctg agatggaaga agaaagatat ggtgtaaatt atgtttgaaa tagaagtgat 1080

tagacaaata cttactttga actagaaaga gatagaaatt tcccaggcat gattctgaag 1140

atgtggtggt aggcttgtag cagtatttat tctttggttt cgatctataa atttggtatt 1200

ctaaacaaac atgtaatttt attcccatat catctcaagt ctaagaaaaa aaaaaaaaaa 1260

aa 1262

<210> 350
<211> 301
<212> PRT
<213> Hordeum vulgare

<400> 350

Met Pro Met Gly Ser Ser Ser Ser Ser Ala Ala Met Pro Ala Leu Pro
1               5                   10                  15

Pro Gly Phe Arg Val His Pro Thr Asp Glu Glu Leu Ile Val His Tyr
            20                  25                  30

Leu Gly Arg Gln Ala Ala Ser Met Pro Ser Pro Val Pro Ile Ile Ala
        35                  40                  45

Glu Val Asn Ile Tyr Lys Cys Asn Pro Trp Asp Leu Pro Gly Lys Ala
    50                  55                  60

Leu Leu Gly Glu Asn Glu Trp Tyr Phe Phe Ser Pro Arg Asp Arg Lys
65                  70                  75                  80

Tyr Pro Asn Gly Ala Arg Pro Asn Arg Ala Ala Gly Ser Gly Tyr Trp
                85                  90                  95

Lys Ala Thr Gly Thr Asp Lys Pro Ile Pro Pro Arg Glu Ser Gly Gly
            100                 105                 110

Arg Thr Val Gly Ile Lys Lys Ala Leu Val Phe Tyr Ser Gly Arg Ala
        115                 120                 125

Pro Arg Gly Val Lys Thr Asp Trp Ile Met His Glu Tyr Arg Ile Ala
    130                 135                 140

Gln Ala Asp Arg Thr Pro Gly Lys Lys Gly Ser Leu Lys Leu Asp Glu
145                 150                 155                 160

Trp Val Leu Cys Arg Leu Tyr Asn Lys Lys Asn Asn Trp Asp Lys Val
                165                 170                 175

Lys Val Glu Gln Asp Met Ala Val Val Gln Gly Gln Asn Gly Glu Val
            180                 185                 190

Met Asp Ala Leu Ala Thr Asp Ala Met Ser Asp Ser Phe Gln Thr His
195 200 205

Asp Ser Ser Glu Ile Asp Asn Ala Ser Gly Leu Gln Gln Gln His His
210 215 220

Gly Phe Met Asp Met Ala Gln Arg Gln Ala Arg Glu Gly Met Val Thr
225 230 235 240

Val Lys Glu Asp Ser Asp Trp Phe Thr Gly Leu Ser Met Asp Asp Leu
245 250 255

Gln Thr Cys Tyr Met Asn Met Gly Gln Met Val Asn Pro Ala Ala Met
260 265 270

Pro Val His Asp Gly Ser Gly Tyr Leu Gln Ser Met Asn Ser Pro Gln
275 280 285

Met Met Arg Pro Met Trp Gln Thr Ile Leu Pro Pro Phe
290 295 300

<210> 351
<211> 1564
<212> DNA
<213> Triticum aestivum

<220>
<221> misc_feature
<222> (1552)..(1552)
<223> n is a, c, g, or t

<400> 351
gcagctagaa ctgcggcgag ctgatccagc tgagagaact acagcgaggt ttgttggatc 60

gccgacccga ccggagatga gcggcggaca ggagctgaat ctgccgccgg gcttccggtt 120

ccacccgacg gacgaggagc tggtgacgca ctacctctgc cgccgctgcg ccggcgcgcc 180

catcgccgtc cccatcatca ccgagatcga cctctacaag ttcgacccct ggcagctccc 240

aaagatggcg ctgtacggcg agaaggagtg gtacttcttc tccccgcggg accgcaagta 300

ccccaacggg tccaggccca accgggccgc cgggtccggc tactggaagg ccaccggggc 360

cgacaagccc gtgggcaccc ccaagccgct ggccatcaag aaggcgctcg tcttctacgc 420

cggcaaggcc cccaagggcg agaagaccaa ctggatcatg cacgagtacc gcctcgccga 480

cgtcgaccga tcccgccccg caagaagaac agcctcaggt tggatgattg ggtgctgtgc 540

cgcatctaca acaagaaggg cggcatggag aagccggcgg ccgtggaccg gaagccggcg 600

gccatgggcg gctacggggg tggccctggg gccatggcga gctccccgcc ggagcagaag 660

cccgtcatgg ggatgaacgc caacggcggc ggcggcggcg cgcagccgtt ccgggacttc 720

gcggcgtact acgaccggcc gtccgactcg atgccgcggc tgcacgcgga ctcgagctgc 780

```
tcggagcagg tgctgtcgcc ggagttcccg gcggggaggt gcagagccag cccaagatca     840
gcgagtggga gcgctcgttc gcctccggcg gcgaccccgt gaacccggcg gccggctcca     900
tgctcgagcc caacggcggc ttcggcggcg acccgctcct ccaggacatc ctcatgtact     960
ggggcaagcc gttctaggca gcgaagaaac cgatcggtcg gtcgaagcga gtacctccta    1020
tccttggcgt ttggggcgat gaaacgggcg agccgccatt gttgacctga tgaaggggag    1080
ataaatttaa gaagatatta gacgggagat aagacaaaat caggtgcttg atgacgacga    1140
cgacgacggc gaagatcgga aggtggcggc gatgataccg tgggtccccg gcctctcacc    1200
agcatgacat gtgaccgacg acgcccaaga tgcttcaaag cgttcgccgc attgcatcat    1260
cgggcgggcg gtcgtgcgct agcaagcatg catgtgcgtg tatatggatg ggtgtacata    1320
catggagatc atgattggtt cggtgcaggg gttgctgttt cttgattggt tagttgtaat    1380
attttttttt ttttgcgggt gagttgtaag ggtttattga taagttgata ccataccata    1440
ccagtgctag ccgccgtgcg tggtgctggc tagctgtagt ccgagtggta gtagtgtaac    1500
tgtaagccat tcatcaaatg aaactgaata tattatctgc cctcaaaaaa anaaaaaaaa    1560
aaaa                                                                 1564


<210>  352
<211>  199
<212>  PRT
<213>  Triticum aestivum

<400>  352

Met Ser Gly Gly Gln Glu Leu Asn Leu Pro Pro Gly Phe Arg Phe His
1               5                   10                  15

Pro Thr Asp Glu Glu Leu Val Thr His Tyr Leu Cys Arg Arg Cys Ala
            20                  25                  30

Gly Ala Pro Ile Ala Val Pro Ile Ile Thr Glu Ile Asp Leu Tyr Lys
        35                  40                  45

Phe Asp Pro Trp Gln Leu Pro Lys Met Ala Leu Tyr Gly Glu Lys Glu
    50                  55                  60

Trp Tyr Phe Phe Ser Pro Arg Asp Arg Lys Tyr Pro Asn Gly Ser Arg
65                  70                  75                  80

Pro Asn Arg Ala Ala Gly Ser Gly Tyr Trp Lys Ala Thr Gly Ala Asp
                85                  90                  95

Lys Pro Val Gly Thr Pro Lys Pro Leu Ala Ile Lys Lys Ala Leu Val
            100                 105                 110
```

Phe Tyr Ala Gly Lys Ala Pro Lys Gly Glu Lys Thr Asn Trp Ile Met
115 120 125

His Glu Tyr Arg Leu Ala Asp Val Asp Arg Ser Arg Pro Ala Arg Arg
130 135 140

Thr Ala Ser Gly Trp Met Ile Gly Cys Cys Ala Ala Ser Thr Thr Arg
145 150 155 160

Arg Ala Ala Trp Arg Ser Arg Arg Pro Trp Thr Gly Ser Arg Arg Pro
165 170 175

Trp Ala Ala Thr Gly Val Ala Leu Gly Pro Trp Arg Ala Pro Arg Arg
180 185 190

Ser Arg Ser Pro Ser Trp Gly
195

<210> 353
<211> 1373
<212> DNA
<213> Zea mays

<400> 353
cttgacgtct accagctgca taataacttg gttcagaaga agaagatgga cgctttcaca 60

catgttcctc ccggctttcg tttccaccct accgacgagg aactcgttga ctactacctt 120

aggaaaaagg tagcactgaa gaagatagat ttggatgtga taaaagatgt ggatttgtac 180

aagattgagc cttgggatct gcaagaaaag tgcaggattg gaagcgaaga gcagaacgag 240

tggtacttct tcagccacaa ggacaagaag tacccaaccg gcactcgcac caacagagcg 300

acgacggccg gtttttggaa ggccacgggg agagacaagc cgatctacgt gaagaactgc 360

ctcgtaggga tgaggaagac actggttttc tacaaaggcc gggcgcccaa tggacagaag 420

tcagactgga tcatgcacga gtatcgcctg gagaccaaca acaatggaat tccacatgag 480

gaaggatggg ttgtatgcag ggtgttcagg aagcgactcg cgactgtcca aagaatggtc 540

ggggactcgc cttactggtt caatgaccac gcggggttca tggcgccgga gctcggctca 600

ccgaggcagg cggcgcacca tcagcagaac gtcatgatgt accacaggca acagagcagc 660

tacagctacc cttgcaaggt ggagctggag taccaccacc tccttcctca ggagcacttc 720

ctgcagcagc tccctcagct ggagagcccc aagctccctg accttattgg ccaagtagac 780

actactctcc agccagcatg cggccttaca caggagcatg gtgcccctcg ctacaccatg 840

caggagcttc aggccgagcc tctctatctg gcgactggcg gggacacaga ttggcgagct 900

ctcgacaagt tcatggcgtc ccagcttagc cacggagaca tcacccctaa taaggagtca 960

gctaactact ccaatccggc actgcaggtg attcagcagt ctgaagagaa agaagaggcc 1020

ttggactacg tgtcaacgtc agcctcctgt ggaggggaca atgatttgtg gaaataacag 1080

cgtcaatggt aatacatatt cacatacgca ctaaatcact ggactagtgc attattccct 1140

cattacacag ttaataacat tgaggcgtgc tggatgtaat gtaacgtagt atataaagta 1200

gccaactgat tcatgtgtca tggtatagta gaatggtatg tgcataacaa agtgtaaccg 1260

agtaggtcac acgtccatgc ataagttggc ataatgtctg gtgttaataa taaggttagc 1320

taacagctga agctaaggag cttcagcacc tgatttgaca aaaaaaaaaa aaa 1373

<210> 354
<211> 343
<212> PRT
<213> Zea mays

<400> 354

Met Asp Ala Phe Thr His Val Pro Pro Gly Phe Arg Phe His Pro Thr
1 5 10 15

Asp Glu Glu Leu Val Asp Tyr Tyr Leu Arg Lys Lys Val Ala Leu Lys
20 25 30

Lys Ile Asp Leu Asp Val Ile Lys Asp Val Asp Leu Tyr Lys Ile Glu
35 40 45

Pro Trp Asp Leu Gln Glu Lys Cys Arg Ile Gly Ser Glu Glu Gln Asn
50 55 60

Glu Trp Tyr Phe Phe Ser His Lys Asp Lys Lys Tyr Pro Thr Gly Thr
65 70 75 80

Arg Thr Asn Arg Ala Thr Thr Ala Gly Phe Trp Lys Ala Thr Gly Arg
85 90 95

Asp Lys Pro Ile Tyr Val Lys Asn Cys Leu Val Gly Met Arg Lys Thr
100 105 110

Leu Val Phe Tyr Lys Gly Arg Ala Pro Asn Gly Gln Lys Ser Asp Trp
115 120 125

Ile Met His Glu Tyr Arg Leu Glu Thr Asn Asn Asn Gly Ile Pro His
130 135 140

Glu Glu Gly Trp Val Val Cys Arg Val Phe Arg Lys Arg Leu Ala Thr
145 150 155 160

Val Gln Arg Met Val Gly Asp Ser Pro Tyr Trp Phe Asn Asp His Ala
165 170 175

Gly Phe Met Ala Pro Glu Leu Gly Ser Pro Arg Gln Ala Ala His His
180 185 190

Gln Gln Asn Val Met Met Tyr His Arg Gln Gln Ser Ser Tyr Ser Tyr
195 200 205

Pro Cys Lys Val Glu Leu Glu Tyr His His Leu Leu Pro Gln Glu His
210 215 220

Phe Leu Gln Gln Leu Pro Gln Leu Glu Ser Pro Lys Leu Pro Asp Leu
225 230 235 240

Ile Gly Gln Val Asp Thr Thr Leu Gln Pro Ala Cys Gly Leu Thr Gln
245 250 255

Glu His Gly Ala Pro Arg Tyr Thr Met Gln Glu Leu Gln Ala Glu Pro
260 265 270

Leu Tyr Leu Ala Thr Gly Gly Asp Thr Asp Trp Arg Ala Leu Asp Lys
275 280 285

Phe Met Ala Ser Gln Leu Ser His Gly Asp Ile Thr Pro Asn Lys Glu
290 295 300

Ser Ala Asn Tyr Ser Asn Pro Ala Leu Gln Val Ile Gln Gln Ser Glu
305 310 315 320

Glu Lys Glu Glu Ala Leu Asp Tyr Val Ser Thr Ser Ala Ser Cys Gly
325 330 335

Gly Asp Asn Asp Leu Trp Lys
340

<210> 355
<211> 1361
<212> DNA
<213> Helianthus annuus

<400> 355
cttgcttttg tgttgatatg tctaaagaag aagtgaattt gtctgtgaat gtaaatggtc 60

agtctaaagt gcctccaggg tttcggttcc accctaccga agaagagctt cttcattact 120

acttaaggaa gaaagtcgcg tatgaaaaga tcgatcttga tgttattcgt gatattgatc 180

ttaacaagct cgaaccatgg gatatcaaag aaaagtgcag aattggatca accccgcaaa 240

acgattggta tttttttagc cacaaagaca agaaataccc gactggaacg cgtacaaatc 300

gtgcaactgc tgcaggtttt tggaaagcca ccggtcgaga taaggtcatt tatagtagca 360

tgagaagaat cggtatgagg aagacactcg tgttctataa aggaagagca cctcatggac 420

aaaagtcgga ttggattatg cacgaatata gactcgatga caacacgatt atctcccagg 480

attatgcctc aggttctaat ttatgtgatt ccactcaaga agacgggtgg gtcgtatgtc 540

```
gcgtttttaa aaagaaaaac taccataaag cggttgagag tccccaaagg tcatcatcag     600
caccttccat tgattcaaca gctcaaatgc aatctttaaa gaaagatgga actcaacttc     660
ttgcatacat caatggtact aaatcctgca agcaagaaac cgaatcactc gccaatatag     720
caaccaccca tgactatgac cctttgcaac aattcatcaa cccgataagc gacagattcc     780
tacacctccc aaggctccac aacacttcaa gtgacttgat cacgtcagcc acctttgatc     840
aagattcaat ctttccggcc cataacacca tgactcattt gctaacggaa gttgaacatt     900
ctaggaatca taaacatctt gaaaactggt cagatgtgga tcgactcgtg gcctcccaac     960
tcaatggcca accgcgatca tccaggcaga tgtacggttg ctacgacgaa cccaatgaag    1020
acatatgctt ctcacttcac catgatgatc agcaagaacc accacaacca tcatccatgg    1080
ctaaacccaa ccaagcagcc tatacaagcg agatagatat atggagcttt gcacaatctt    1140
catcacaatc atcatcaccg gatccatttt accatttgtc ggtatagttc acataagtaa    1200
tatacttttt actttatgga aaatacagat ggaataaact gttatatata cttggatgta    1260
tatatagtct ctttaattag ctgggtgggt tatggatctt gatgtgaagt gaaaggtcat    1320
gacttatgaa tgtattgtgt ttgatgcaaa aaaaaaaaaa a                        1361
```

```
<210>  356
<211>  389
<212>  PRT
<213>  Helianthus annuus

<400>  356

Met Ser Lys Glu Glu Val Asn Leu Ser Val Asn Val Asn Gly Gln Ser
1               5                   10                  15

Lys Val Pro Pro Gly Phe Arg Phe His Pro Thr Glu Glu Glu Leu Leu
            20                  25                  30

His Tyr Tyr Leu Arg Lys Lys Val Ala Tyr Glu Lys Ile Asp Leu Asp
        35                  40                  45

Val Ile Arg Asp Ile Asp Leu Asn Lys Leu Glu Pro Trp Asp Ile Lys
    50                  55                  60

Glu Lys Cys Arg Ile Gly Ser Thr Pro Gln Asn Asp Trp Tyr Phe Phe
65                  70                  75                  80

Ser His Lys Asp Lys Lys Tyr Pro Thr Gly Thr Arg Thr Asn Arg Ala
                85                  90                  95

Thr Ala Ala Gly Phe Trp Lys Ala Thr Gly Arg Asp Lys Val Ile Tyr
            100                 105                 110
```

```
Ser Ser Met Arg Arg Ile Gly Met Arg Lys Thr Leu Val Phe Tyr Lys
        115                 120                 125

Gly Arg Ala Pro His Gly Gln Lys Ser Asp Trp Ile Met His Glu Tyr
    130                 135                 140

Arg Leu Asp Asp Asn Thr Ile Ile Ser Gln Asp Tyr Ala Ser Gly Ser
145                 150                 155                 160

Asn Leu Cys Asp Ser Thr Gln Glu Asp Gly Trp Val Val Cys Arg Val
                165                 170                 175

Phe Lys Lys Lys Asn Tyr His Lys Ala Val Glu Ser Pro Gln Arg Ser
            180                 185                 190

Ser Ser Ala Pro Ser Ile Asp Ser Thr Ala Gln Met Gln Ser Leu Lys
        195                 200                 205

Lys Asp Gly Thr Gln Leu Leu Ala Tyr Ile Asn Gly Thr Lys Ser Cys
    210                 215                 220

Lys Gln Glu Thr Glu Ser Leu Ala Asn Ile Ala Thr Thr His Asp Tyr
225                 230                 235                 240

Asp Pro Leu Gln Gln Phe Ile Asn Pro Ile Ser Asp Arg Phe Leu His
                245                 250                 255

Leu Pro Arg Leu His Asn Thr Ser Ser Asp Leu Ile Thr Ser Ala Thr
            260                 265                 270

Phe Asp Gln Asp Ser Ile Phe Pro Ala His Asn Thr Met Thr His Leu
        275                 280                 285

Leu Thr Glu Val Glu His Ser Arg Asn His Lys His Leu Glu Asn Trp
    290                 295                 300

Ser Asp Val Asp Arg Leu Val Ala Ser Gln Leu Asn Gly Gln Pro Arg
305                 310                 315                 320

Ser Ser Arg Gln Met Tyr Gly Cys Tyr Asp Glu Pro Asn Glu Asp Ile
                325                 330                 335

Cys Phe Ser Leu His His Asp Asp Gln Gln Glu Pro Pro Gln Pro Ser
            340                 345                 350

Ser Met Ala Lys Pro Asn Gln Ala Ala Tyr Thr Ser Glu Ile Asp Ile
        355                 360                 365

Trp Ser Phe Ala Gln Ser Ser Ser Gln Ser Ser Ser Pro Asp Pro Phe
```

370 375 380

Tyr His Leu Ser Val
385

<210> 357
<211> 1820
<212> DNA
<213> Glycine max

<400> 357
tcacctcatc atctatcatt atatctttct ttcaattcct atcctcttcc ttgtagtgta 60

cccattttga atgtgttctc tctctctctc tctttcttta ggtccctggt gaatatctag 120

aaccactctc tagctagttt ctctcttctt gctagctagc tatcgccgat cactctcttg 180

gtttaaacca cctgaatgcc ggaaaacatg agcatatcag tgaatggtca atctcaagtc 240

cctcctggct tcaggtttca tccaactgaa gaagagcttc ttcagtacta cttgaggaag 300

aaggtctctt acgagaagat tgacctcgac gtgattcgtg acgttgatct caacaagctc 360

gagccatggg acatacaaga gaaatgcaaa ataggaacca ctccgcagaa tgattggtac 420

ttcttcagcc acaaagacaa gaagtacccc acaggaacgc gcaccaatcg cgcaactgct 480

gcagggttct ggaaggccac tggccgcgac aaagtcatct acagcaatgg aaagaggatt 540

ggaatgagaa agactctggt tttctacaaa ggaagagcgc cacatggcca gaaatccgat 600

tggatcatgc atgaatacag actagacgac aacaacacca ccgacaccaa tattgtgtcc 660

aatgtgatgg gggatgctgc tcaggaagaa gggtgggtgg tgtgcaggat attcaagaag 720

aagaaccatc tgaaaaccct agacagcccc ttagcttcag gggaagatag aaggagccac 780

ttgttcgact cgtgcgacga gggagctttg gagcaaatac ttcagcaaat gggaaggggt 840

tgcaaggagg agagcagcta tgaaggcaac tacaacagct atggaaggtt tacaaggccg 900

tatgagacaa cagcggcgaa caatggcggc ggatacaatg ataggttcat gaagctcccg 960

agcctcgaga gcccaaaatc cgcaagcatg gagaaccacc acaacaccaa caacaacaat 1020

aacatgaata gtaataataa taataatggt gataacaacg agaacaacaa taataatggg 1080

taccatccca taattccagt agagatgggc acggacaatg aagggacatt cacaacgcac 1140

caggtgagtg gtggtgaccc aaacaacaac aacaacaaca gcaacattgt tcatccattg 1200

gaggtaggat caggtggtgg aggcctcacc aactgggctg cgctggaccg tttagttgca 1260

tctcaactca atggccaaac cgatgcctct aggcaactag gatgtgcttt caacgacccc 1320

accatgtatt gcaccagcgt cgaccatcat gatcttcatc atcaaatccc caccctccga 1380

tcctcatcaa cgtccgctaa cacacgacct tcccctgcac ccgcattcat caacccccca 1440

acacaggact tcaccagcga gattgacctt tggaacttct cccgatccac gtcctcactg 1500

ttggcatcct ccgaaccact gtgccacgtg tccaacacgt cagtgtagcg agatcaataa 1560

aataataata taaaaaaaaa tatctcaagt ccccctttc ctccatgttc gattgttaaa 1620

tatagaaata attagcccct acttcatcac atatcatgtt taatatttaa tattagttct 1680

tcttctctca agtctcagag gattattatt acagacttta aatgtgataa ttctcctagt 1740

atacattttt actttaatta gcttatttcg actcaaaaaa ataatattcc attgacaatt 1800

gtctcttgta aatcttaatt 1820

<210> 358
<211> 450
<212> PRT
<213> Glycine max

<400> 358

Met Pro Glu Asn Met Ser Ile Ser Val Asn Gly Gln Ser Gln Val Pro
1 5 10 15

Pro Gly Phe Arg Phe His Pro Thr Glu Glu Glu Leu Leu Gln Tyr Tyr
20 25 30

Leu Arg Lys Lys Val Ser Tyr Glu Lys Ile Asp Leu Asp Val Ile Arg
35 40 45

Asp Val Asp Leu Asn Lys Leu Glu Pro Trp Asp Ile Gln Glu Lys Cys
50 55 60

Lys Ile Gly Thr Thr Pro Gln Asn Asp Trp Tyr Phe Phe Ser His Lys
65 70 75 80

Asp Lys Lys Tyr Pro Thr Gly Thr Arg Thr Asn Arg Ala Thr Ala Ala
85 90 95

Gly Phe Trp Lys Ala Thr Gly Arg Asp Lys Val Ile Tyr Ser Asn Gly
100 105 110

Lys Arg Ile Gly Met Arg Lys Thr Leu Val Phe Tyr Lys Gly Arg Ala
115 120 125

Pro His Gly Gln Lys Ser Asp Trp Ile Met His Glu Tyr Arg Leu Asp
130 135 140

Asp Asn Asn Thr Thr Asp Thr Asn Ile Val Ser Asn Val Met Gly Asp
145 150 155 160

Ala Ala Gln Glu Glu Gly Trp Val Val Cys Arg Ile Phe Lys Lys Lys
165 170 175

Asn His Leu Lys Thr Leu Asp Ser Pro Leu Ala Ser Gly Glu Asp Arg
180 185 190

Arg Ser His Leu Phe Asp Ser Cys Asp Glu Gly Ala Leu Glu Gln Ile
        195                 200                 205

Leu Gln Gln Met Gly Arg Gly Cys Lys Glu Glu Ser Ser Tyr Glu Gly
    210                 215                 220

Asn Tyr Asn Ser Tyr Gly Arg Phe Thr Arg Pro Tyr Glu Thr Thr Ala
225                 230                 235                 240

Ala Asn Asn Gly Gly Gly Tyr Asn Asp Arg Phe Met Lys Leu Pro Ser
                245                 250                 255

Leu Glu Ser Pro Lys Ser Ala Ser Met Glu Asn His His Asn Thr Asn
            260                 265                 270

Asn Asn Asn Asn Met Asn Ser Asn Asn Asn Asn Asn Gly Asp Asn Asn
        275                 280                 285

Glu Asn Asn Asn Asn Asn Gly Tyr His Pro Ile Ile Pro Val Glu Met
    290                 295                 300

Gly Thr Asp Asn Glu Gly Thr Phe Thr Thr His Gln Val Ser Gly Gly
305                 310                 315                 320

Asp Pro Asn Asn Asn Asn Asn Asn Ser Asn Ile Val His Pro Leu Glu
                325                 330                 335

Val Gly Ser Gly Gly Gly Gly Leu Thr Asn Trp Ala Ala Leu Asp Arg
            340                 345                 350

Leu Val Ala Ser Gln Leu Asn Gly Gln Thr Asp Ala Ser Arg Gln Leu
        355                 360                 365

Gly Cys Ala Phe Asn Asp Pro Thr Met Tyr Cys Thr Ser Val Asp His
    370                 375                 380

His Asp Leu His His Gln Ile Pro Thr Leu Arg Ser Ser Ser Thr Ser
385                 390                 395                 400

Ala Asn Thr Arg Pro Ser Pro Ala Pro Ala Phe Ile Asn Pro Pro Thr
                405                 410                 415

Gln Asp Phe Thr Ser Glu Ile Asp Leu Trp Asn Phe Ser Arg Ser Thr
            420                 425                 430

Ser Ser Leu Leu Ala Ser Ser Glu Pro Leu Cys His Val Ser Asn Thr
        435                 440                 445

Ser Val
    450

<210>  359
<211>  3287
<212>  DNA
<213>  Zea mays

<400>  359
tgacaacctg ttactggatc attgtgattt ttagcgcgaa tagcacgagc gcaaaacgat      60

atcaggtact tacaattgtt tgcaaagtga cgaagaacat acaaggtgac atgaccacaa     120

ttgtttgcag tgttgacacg agtgggctca cacaatagac aatacatatg tcgcacttcg     180

atcccgtcct cttaacaacc agagcacagg accgcattta ccatgggggt ataccgtgtt     240

cgatatcaag aggccgcggt gcggtgcttt gaccggctag ttgtcggatt gcatgctcct     300

gagcatgttc aggatggaga ggaacaggtt caggatgtcg aggtacagcc caaccgacgc     360

ccagatgtac tcgtcgtagg tgtggcgctt gatcaggttc tcggtgtcgt acaggatgaa     420

gcctgagaag accagagccc ctagaccacc gaacaagccc accgacacgg gtcacagtgg     480

gaagaaaacc tgcagcgcag aattccacac agaaccaaat aaaagtttat tgtgcaagca     540

gtggattgct cgtctggtaa caaagcatca ttcgcggata tacagagcgc gtttctttca     600

gagcttgtcg taggcattac ctgaagaaag ctagttagga cgaggatagt aagcgcggaa     660

gacaggatag gccccaggta cccgaattcc ttgcccttct ttgacgccca gaaagcatac     720

gcagtcagag aaaccaccac gccagccgtc agcactaaag cctccagaac gattttccct     780

tgggtgttag cacaagccac gccgatgctg aagctcaagc acaacgtgaa cagacccagg     840

aaaacggaat tgtgtgggtg cttgtgctga taatgataca atgggatcat caggatgaag     900

ggcaggacgg cgagcacgag cgcgaggccc ggggagtcgg agagcgtggc gttgagggtg     960

gggtggagaa cggtgagggc ggagacggcg gtggtgagga gcagctgcgc agcgaggatg    1020

ccgtagacct tgcggacgaa gccccatcgg agggcgctct ccccgcgcga gatccccggg    1080

tacagcgtct ccccggtccc ggcctccagg tccaccaccg acgcctccac cttctccttc    1140

atctccggcg cgcggcggta ccccgccggc gcgaggggct gcatctccgc caccgatgcc    1200

atctctctct ccgacggggg ttgggtgcgg tgcggtgcgg ggaaggggaa acccacgcgt    1260

ccgcccacgc gtccgcccac gcgtccgccc acgcgtccgc ccacgcgtcc gcccacgcgt    1320

ccgcccacgc gtccgcccac gcgtccgcgg acgcgtggga tcgtcttcct cccctcacat    1380

cctctggcct ctggtcctcc accgtcctgc tagccagagc tgctcttgta cgcgcagctg    1440

gcctctgtgc atatcaccag cacaccgcgc aggggaagga aattaacaag agaaaaggca    1500

aggagagcag gcaggcaagg aagctgcaga agccaaggaa ggagaaggag gatcatcaat    1560

gagcatctcg gtgaacgggc agtcgtgcgt gccgccgggg ttccgcttcc accccacgga    1620

ggaggagctg ctcaactact acctccgcaa gaaggtggcc tcccaggaga tcgacctcga    1680

cgtcatccgc gacgtcgacc tcaacaagct cgagccatgg gacatccaag agaaatgcaa 1740

gatcgggtcg ggtccccaga acgactggta cttcttcagc cacaaggaca agaagtaccc 1800

gacggggacg cgcaccaacc gcgccacggc cgccgggttc tggaaggcca ccggccgcga 1860

caaggccatc tacaacgccg tcaagcgcat cggcatgcgc aagacgctcg tcttctacaa 1920

gggccgcgcg ccgcacggcc agaagtccga ctggatcatg cacgagtacc gcctcgacga 1980

ccccgctgct gctgctgctg ctggatccgg tgatgccgtg gccaacgacg acgcagccgc 2040

cacggctgct gctgctgccg ccgcgtcgtc ggacggcggg caggaggacg gctgggtggt 2100

gtgcagggtg ttcaagaaga agcaccacca caaggagtca ggtgggggcg ggggcaacaa 2160

gcacggcagc agtaacagcg agcatgggca cggcggcgcc ggcaaggcat cggctgcggc 2220

tgcggctgcg gcgcaccagc accagcacca tggaggcctg cagtactcct ccagcgacga 2280

ggcgctggac cagatcctgc agtacatggg caggtcgtgc aagcaggagc acgagctggt 2340

gtcgccggcg ccggcgccgc cgggacgggc ggcggcgtcc aggtacctcc ggcccatcga 2400

gaccgttctg ggcgggcacg cgttcatgaa gcttcccgcg ctcgagagcc cgtcccagcg 2460

ccaagcccac caccaggagc gcgcgagtac gccgccgccg gctgggacga cgacgacgcg 2520

ctggaccgcc tcgccgccta cgaccacctc aacggcctct ccaacgacga cgcgtccaag 2580

aacatggccg cgttcttcga cgtcgagcct agcgccgccg ccgccgccgc cgtggacggc 2640

gacctgtgga gcctcgcacg gtccgtgtcg gcgctgcacg cggacttgac catgaacaac 2700

gtctagcttc tgggtcccga ccaacaacaa cagggccccg aatccccgat acacatccat 2760

atggcgtgtg cacgcatgca tgcatgcatt gcatgccgcg cgcaccacta accactcgac 2820

cagcatgcat gcatacgtgc gcaccccacc ggcgcccgcg gccgctagtg cgtcgtgctc 2880

ccgtagtgcg tgcatgcatg gccgccgcac gtacgtattg cacgctcgct cgcgcgtacg 2940

tacgtacgtg cgcgaataag ctagctagcc ctaggatcgg aaacatatgt atgcatccat 3000

tgcatggccg gatatacatc aggagctgct gccgctggtg tatgtccgcc gccgtccaaa 3060

ctccaaagtg agctgagatc gatcgatcga gctcgctcgc tccaggtgtg cagtacgtac 3120

gtacgtaagc gcctgtgtat gtgtaagcag acagcgtggt agtagctcta gctagtagaa 3180

catacttaca cacactatat actgcatacc gtttgtacct atcatatata gattactact 3240

gtattattgc aaaccgcttt gggggggtttt aaaaaaaaaa aaaaaaa 3287

<210> 360
<211> 376
<212> PRT
<213> Zea mays

<400> 360

Met Ser Ile Ser Val Asn Gly Gln Ser Cys Val Pro Pro Gly Phe Arg
1 5 10 15

Phe His Pro Thr Glu Glu Glu Leu Leu Asn Tyr Tyr Leu Arg Lys Lys
20 25 30

Val Ala Ser Gln Glu Ile Asp Leu Asp Val Ile Arg Asp Val Asp Leu
35 40 45

Asn Lys Leu Glu Pro Trp Asp Ile Gln Glu Lys Cys Lys Ile Gly Ser
50 55 60

Gly Pro Gln Asn Asp Trp Tyr Phe Phe Ser His Lys Asp Lys Lys Tyr
65 70 75 80

Pro Thr Gly Thr Arg Thr Asn Arg Ala Thr Ala Ala Gly Phe Trp Lys
85 90 95

Ala Thr Gly Arg Asp Lys Ala Ile Tyr Asn Ala Val Lys Arg Ile Gly
100 105 110

Met Arg Lys Thr Leu Val Phe Tyr Lys Gly Arg Ala Pro His Gly Gln
115 120 125

Lys Ser Asp Trp Ile Met His Glu Tyr Arg Leu Asp Asp Pro Ala Ala
130 135 140

Ala Ala Ala Ala Gly Ser Gly Asp Ala Val Ala Asn Asp Asp Ala Ala
145 150 155 160

Ala Thr Ala Ala Ala Ala Ala Ala Ala Ser Ser Asp Gly Gly Gln Glu
165 170 175

Asp Gly Trp Val Val Cys Arg Val Phe Lys Lys Lys His His His Lys
180 185 190

Glu Ser Gly Gly Gly Gly Gly Asn Lys His Gly Ser Ser Asn Ser Glu
195 200 205

His Gly His Gly Gly Ala Gly Lys Ala Ser Ala Ala Ala Ala Ala Ala
210 215 220

Ala His Gln His Gln His His Gly Gly Leu Gln Tyr Ser Ser Ser Asp
225 230 235 240

Glu Ala Leu Asp Gln Ile Leu Gln Tyr Met Gly Arg Ser Cys Lys Gln
245 250 255

Glu His Glu Leu Val Ser Pro Ala Pro Ala Pro Pro Gly Arg Ala Ala
260 265 270

Ala Ser Arg Tyr Leu Arg Pro Ile Glu Thr Val Leu Gly Gly His Ala
        275                 280                 285

Phe Met Lys Leu Pro Ala Leu Glu Ser Pro Ser Gln Arg Gln Ala His
    290                 295                 300

His Gln Glu Arg Ala Ser Thr Pro Pro Pro Ala Gly Thr Thr Thr Thr
305                 310                 315                 320

Arg Trp Thr Ala Ser Pro Pro Thr Thr Thr Ser Thr Ala Ser Pro Thr
                325                 330                 335

Thr Thr Arg Pro Arg Thr Trp Pro Arg Ser Ser Thr Ser Ser Leu Ala
            340                 345                 350

Pro Pro Pro Pro Pro Pro Trp Thr Ala Thr Cys Gly Ala Ser His Gly
        355                 360                 365

Pro Cys Arg Arg Cys Thr Arg Thr
    370                 375

<210> 361
<211> 923
<212> DNA
<213> Glycine max

<400> 361
ggcattggat catttgtggc ttgtaccgaa ctctgaatgc cggagatgaa gatgagcata 60

tgtgtgaatg gagaatccca agtccctcca ggcttccggt ttcatccaac cgaagaggaa 120

ctgttgcagt actatttgag gaagaaggtg tccaacgaga agatcgacct cgacgtgatt 180

cgcgatgttg atctcaacag actcgaacca tgggacatac aagagatgtg caaaatagga 240

agcagcccac aaaacgattg gtacttgttc agccacaagg acaagaagta ccccacggga 300

agccgcacca accgcgccat cattgttggg ttctggaagg ccacggggcg cgacaaggtc 360

atatatagca acgggaagat aattggaatg agaaaaacat tggttttcta caaagggcgt 420

gcccccaatg gccaaaagtc tgattggatc atgcatgaat acaggctaga cgacattaat 480

aacaccaatg agatggaaca cgggtgggtg gtctgtagag ttttcaagaa gaagaatgtc 540

cctctcaaaa ccctagatag cccaaaatcc atgaccatga atatgtttac agaaagcgac 600

tactgtggag gcttcactaa ctgggaatcc cttgatcagc ttgtggcatc acaactgaat 660

ggacaaactg agacatattg cagtgatttg caattccccg catttctatc atcctcatac 720

attgctacaa caacacacat tgctcccaca atacaggatt accccagcta cgacattgac 780

ctgtggaacc actgtgcttc gcgcgtgtcc aacactccaa atgtaacact ctaatcaaat 840

ccttctttta atttcttccc atgtcagatt attactacta ttaaatcatc cctaaaatat 900

tcattttcaa aaaaaaaaaa aaa 923

<210> 362
<211> 265
<212> PRT
<213> Glycine max

<400> 362

Met Pro Glu Met Lys Met Ser Ile Cys Val Asn Gly Glu Ser Gln Val
1 5 10 15

Pro Pro Gly Phe Arg Phe His Pro Thr Glu Glu Glu Leu Leu Gln Tyr
20 25 30

Tyr Leu Arg Lys Lys Val Ser Asn Glu Lys Ile Asp Leu Asp Val Ile
35 40 45

Arg Asp Val Asp Leu Asn Arg Leu Glu Pro Trp Asp Ile Gln Glu Met
50 55 60

Cys Lys Ile Gly Ser Ser Pro Gln Asn Asp Trp Tyr Leu Phe Ser His
65 70 75 80

Lys Asp Lys Lys Tyr Pro Thr Gly Ser Arg Thr Asn Arg Ala Ile Ile
85 90 95

Val Gly Phe Trp Lys Ala Thr Gly Arg Asp Lys Val Ile Tyr Ser Asn
100 105 110

Gly Lys Ile Ile Gly Met Arg Lys Thr Leu Val Phe Tyr Lys Gly Arg
115 120 125

Ala Pro Asn Gly Gln Lys Ser Asp Trp Ile Met His Glu Tyr Arg Leu
130 135 140

Asp Asp Ile Asn Asn Thr Asn Glu Met Glu His Gly Trp Val Val Cys
145 150 155 160

Arg Val Phe Lys Lys Lys Asn Val Pro Leu Lys Thr Leu Asp Ser Pro
165 170 175

Lys Ser Met Thr Met Asn Met Phe Thr Glu Ser Asp Tyr Cys Gly Gly
180 185 190

Phe Thr Asn Trp Glu Ser Leu Asp Gln Leu Val Ala Ser Gln Leu Asn
195 200 205

Gly Gln Thr Glu Thr Tyr Cys Ser Asp Leu Gln Phe Pro Ala Phe Leu
210 215 220

Ser Ser Ser Tyr Ile Ala Thr Thr Thr His Ile Ala Pro Thr Ile Gln
225 230 235 240

Asp Tyr Pro Ser Tyr Asp Ile Asp Leu Trp Asn His Cys Ala Ser Arg
245 250 255

Val Ser Asn Thr Pro Asn Val Thr Leu
260 265

<210> 363
<211> 1455
<212> DNA
<213> Zea mays

<400> 363
ggtcgtctaa aaaagcagca gctgctcttc tcttctgcta gcagctttat ttgcctgcct 60

ccctaccggc gagggaggta cgtgcgccga tcgagaagca gcagcagcaa ttaataacta 120

aacatggatc aggcggcgga ggagtcttgt gttcccccag gcttcaggtt ccaccctacg 180

gaggaagaac tggtgggcta ctaccttgcc aggaaggtgg cctcccagag gatcgacctc 240

gacatcatcc aggaggtgga tctctacagg atagagccat gggatctgca agagcggtgc 300

aagccgcagc acgccggcgg tgggcacgac gagcagacac aggagtggta cttcttcagc 360

tacaaggacc gcaagtaccc cagcgggacg aggacgaacc gcgccacggc ggccggcttc 420

tggaaggcca ccggcaggga caaaccggtg ctgtcgtcgt ccaccaggac caccaggtgt 480

agcagggtca tcggcatgag gaagacgctg gtgttctaca agggcagggc acccaacggc 540

aggaagagcg actggatcat gcacgagtac cgactccaat ccaacgagca cgcaccggcg 600

caggaggaag gctgggtggt gtgccgcgct ttccagaagc ctatccccaa ccagcggccc 660

ttcttcccca ccagctacgc cggctactac gaccacaacc tctcaacgac ggcacggctg 720

cacgtagacg gcgaccgcca tttcctggcg ggctcatcag cagcagcagc agcactgcta 780

cagcagccac cagggcttgc aggcagcagc ttcccgcagc tgtactccga cgacgacttg 840

gagtccaaga agcagctgtt gagtatcccg ccgctggaga gccccactgc catggcctgc 900

tgttccgacg ccggcggcta cgcgcagaga agcagctacg atgaacatga gatgatgatg 960

atgatgatcc agcagggagg aggaggagga gagcaagctg cagccgccat cgactggaac 1020

tttctggaca gcctgctgtc cgcgacgtcg caactccatg ggccctcggg atccttgttg 1080

cagtgacctc cggccatcgt cgatcgatcc atctttcttt gatccagcgg agaacattat 1140

tcattcatcg gcattataca atatatttat tagttgcata cacacacaga gtatacgtat 1200

atgctaataa ttcagatgtg ttcatacaca cttccattgt catgtgtagt tcgttgcgac 1260

ttacattaca gactgcctta gatgattcag gtatactgta tacaacatga tgcacagtat 1320

agtaggagta agtgcaccag tctcagcagc tggtagctag ctagctatgt aaaattcctc 1380

cttaattaca gctatgtaac atatatatac agttattatt acttactgat atgatctatc 1440

tatcaaaaaa aaaaa 1455

<210> 364
<211> 320
<212> PRT
<213> Zea mays

<400> 364

Met Asp Gln Ala Ala Glu Glu Ser Cys Val Pro Pro Gly Phe Arg Phe
1 5 10 15

His Pro Thr Glu Glu Glu Leu Val Gly Tyr Tyr Leu Ala Arg Lys Val
20 25 30

Ala Ser Gln Arg Ile Asp Leu Asp Ile Ile Gln Glu Val Asp Leu Tyr
35 40 45

Arg Ile Glu Pro Trp Asp Leu Gln Glu Arg Cys Lys Pro Gln His Ala
50 55 60

Gly Gly Gly His Asp Glu Gln Thr Gln Glu Trp Tyr Phe Phe Ser Tyr
65 70 75 80

Lys Asp Arg Lys Tyr Pro Ser Gly Thr Arg Thr Asn Arg Ala Thr Ala
85 90 95

Ala Gly Phe Trp Lys Ala Thr Gly Arg Asp Lys Pro Val Leu Ser Ser
100 105 110

Ser Thr Arg Thr Thr Arg Cys Ser Arg Val Ile Gly Met Arg Lys Thr
115 120 125

Leu Val Phe Tyr Lys Gly Arg Ala Pro Asn Gly Arg Lys Ser Asp Trp
130 135 140

Ile Met His Glu Tyr Arg Leu Gln Ser Asn Glu His Ala Pro Ala Gln
145 150 155 160

Glu Glu Gly Trp Val Val Cys Arg Ala Phe Gln Lys Pro Ile Pro Asn
165 170 175

Gln Arg Pro Phe Phe Pro Thr Ser Tyr Ala Gly Tyr Tyr Asp His Asn
180 185 190

Leu Ser Thr Thr Ala Arg Leu His Val Asp Gly Asp Arg His Phe Leu
195 200 205

Ala Gly Ser Ser Ala Ala Ala Ala Ala Leu Leu Gln Gln Pro Pro Gly

```
            210                 215                 220

        Leu Ala Gly Ser Ser Phe Pro Gln Leu Tyr Ser Asp Asp Asp Leu Glu
        225                 230                 235                 240

        Ser Lys Lys Gln Leu Leu Ser Ile Pro Pro Leu Glu Ser Pro Thr Ala
                        245                 250                 255

        Met Ala Cys Cys Ser Asp Ala Gly Gly Tyr Ala Gln Arg Ser Ser Tyr
                    260                 265                 270

        Asp Glu His Glu Met Met Met Met Met Ile Gln Gln Gly Gly Gly Gly
                275                 280                 285

        Gly Glu Gln Ala Ala Ala Ala Ile Asp Trp Asn Phe Leu Asp Ser Leu
            290                 295                 300

        Leu Ser Ala Thr Ser Gln Leu His Gly Pro Ser Gly Ser Leu Leu Gln
        305                 310                 315                 320
```